# EUROPÄISCHE PATENTSCHRIFT

(11) **EP 2 691 379 B1**
(45) Veröffentlichungstag und Bekanntmachung des Hinweises auf die Patenterteilung: **02.11.2016**
(21) Anmeldenummer: 12713928.5
(22) Anmeldetag: 26.03.2012
(51) Int. Cl.: C07D 261/04, C07D 413/12, C07D 417/12, A01N 43/80

(54) **HERBIZID UND FUNGIZID WIRKSAME 3-PHENYLISOXAZOLIN-5-CARBOXAMIDE UND 3- PHENYLISOXAZOLIN-5-THIOAMIDE**
HERBICIDALLY AND FUNGICIDALLY ACTIVE 3-PHENYLISOXAZOLINE-5-CARBOXAMIDES AND 3-PHENYLISOXAZOLINE-5-THIOAMIDES
3-PHÉNYLISOXAZOLINO-5-CARBOXAMIDES ET 3-PHÉNYLISOXAZOLINO-5-THIOAMIDES À ACTIVITÉ HERBICIDE ET FONGICIDE

(30) Priorität: 31.03.2011 EP 11160613
(43) Veröffentlichungstag der Anmeldung: 05.02.2014
(73) Patentinhaber: Bayer Intellectual Property GmbH, 40789 Monheim am Rhein (DE)
(72) Erfinder: WILLMS, Lothar, 65719 Hofheim (DE); SCHMITT, Monika H., 60318 Frankfurt (DE); FRENZEL, Thomas, 51061 Köln (DE); HAAF, Klaus Bernhard, 65779 Kelkheim (DE); HÄUSER-HAHN, Isolde, 51375 Leverkusen (DE); HEINEMANN, Ines, 65719 Hofheim (DE); GATZWEILER, Elmar, 61231 Bad Nauheim (DE); ROSINGER, Christopher Hugh, 65719 Hofheim (DE); DITTGEN, Jan, 60316 Frankfurt (DE); FEUCHT, Dieter, 65760 Eschborn (DE); HILLS, Martin Jeffrey, 65510 Idstein (DE); RINOLFI, Philippe, F-69380 Châtillon d´Azergues (FR); KEHNE, Heinz, 65719 Hofheim (DE)
(74) Vertreter: BIP Patents
(86) Internationale Anmeldenummer: PCT/EP2012/055323
(87) Internationale Veröffentlichungsnummer: WO 2012/130798

(56) Entgegenhaltungen:
- WO-A1-99/05130
- MIROSAA AW GUCMA ET AL: "Synthesis and biological activity of 3-substituted isoxazolecarboxamides", MONATSHEFTE FÜR CHEMIE - CHEMICAL MONTHLY ; AN INTERNATIONAL JOURNAL OF CHEMISTRY, SPRINGER-VERLAG, AU, Bd. 141, Nr. 4, 23. Februar 2010 (2010-02-23), Seiten 461-469, XP019853437, ISSN: 1434-4475 in der Anmeldung erwähnt

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Speziell betrifft sie substituierte 3-Phenylisoxazolin-5-carboxamide und 3-Phenylisoxazolin-5-thioamide, Verfahren zu ihrer Herstellung und ihre Verwendung als Herbizide.

WO1999/5014681 A1, WO1995/014680 A1, WO 2008/035315 A1,
WO2005/051931 A1 und WO2005/021515 A1 beschreiben unter anderen jeweils 3-Phenylisoxazolin-5-carbox-amide, die am Phenylring in 3- und 4-Position durch Alkoxy-Reste substiuiert sind. WO1998/057937 A1 beschreibt unter anderen jeweils solche Verbindungen, die am Phenylring in 4-Position durch einen Alkoxy-Rest substiuiert sind. WO2006/016237 A1 beschreibt unter anderen jeweils solche Verbindungen, die am Phenylring durch einen Amido-Rest substiuiert sind. Die in vorstehend genannten Dokumenten beschriebenen Verbindungen werden darin als pharmakologisch wirksam offenbart. In WO2005/021516 A1 werden die Verbindungen 3-({[3-(3-tert-Butylphenyl)-5-ethyl-4,5-dihydro-1,2-oxazol-5-yl]carbonyl}amino)-5-fluor-4-oxopentansäure und 3-({[3-(3-tert-Butylphenyl)-5-isopropyl-4,5-dihydro-1,2-oxazol-5-yl]carbonyl}amino)-5-fluor-4-oxopentansäure als pharmakologisch wirksam offenbart.

Aus DE 4026018 A1 und EP 520371 A2 und DE 4017665 sind 3-Phenylisoxazolin-5-carboxamide bekannt, die in 5-Position des Isoxazolin-Rings ein Wasserstoffatom tragen. Diese Verbindungen werden dort als agrochemisch wirksame Safener beschrieben, d.h. als Verbindungen, die die unerwünschte herbizide Wirkung von Herbiziden gegenüber Kulturpflanzen aufheben. Eine herbizide Wirkung dieser Verbindungen ist nicht offenbart.

Aus Monatshefte Chemie (2010) 141, 461 und Letters in Organic Chemistry (2010), 7, 502 sind ebenfalls 3-Phenylisoxazolin-5-carboxamide bekannt, die in 5-Position des Isoxazolin-Rings ein Wasserstoffatom tragen. Einige der genannten Verbindungen zeigen fungizide Wirkung. Keine der vorstehend genannten Schriften offenbart eine herbizide Wirkung solcher 3-Phenylisoxazolin-5-carboxamide. WO 99/05130 A1 offenbart herbizid wirksame 3-Phenylisoxazoline, die in 5-Position des Isoxazolinringes eine Oxy-, Thio oder Amino-Gruppe tragen.

Aufgabe der vorliegenden Erfindung ist die Bereitstellung herbizid wirksamer Verbindungen.

Es wurde gefunden, daß substituierte 3-Phenylisoxazolin-5-carboxamide und 3-Phenylisoxazolin-5-thioamide als Herbizide besonders gut geeignet sind. Ein Gegenstand der vorliegenden Erfindung sind 3-Phenylisoxazolin-5-carboxamide und 3-Phenylisoxazolin-5-thioamide der Formel (I) 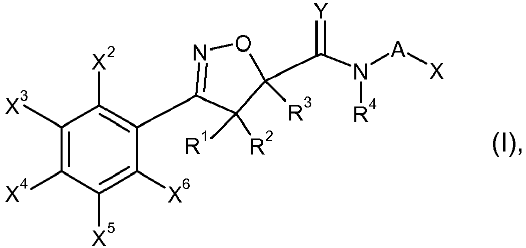 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵, oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 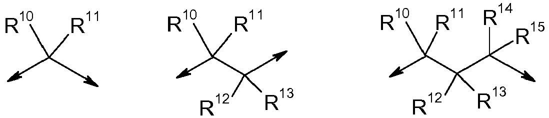 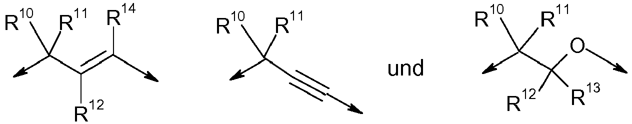 R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CP₂R⁸, NR⁶SO₂R₈, NR⁶SO₂NR₆R₈, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen fünf- oder sechsgliedrigen gesättigten, teilweise ungesättigten, vollständig ungesättigten oder aromatischen Ring, der aus r Kohlenstoffatomen, s Stickstoffatomen, n Schwefelatomen und n Sauerstoffatomen aufgebaut ist, und der durch n Reste aus der Gruppe bestehend aus R⁶, R^{6a}, R⁸ und R⁹ substituiert ist;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO²R⁸, NR⁶SO²R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₃-C₆)-Alkenyloxy oder (C₃-C₆)-Alkinyloxy,
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cy_{C}loalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
r bedeutet 1, 2, 3, 4 oder 5;
s bedeutet 0, 1, 2, 3 oder 4;
mit der Maßgabe, daß X³ und X⁴ nicht gleichzeitig substituiertes oder unsubstituiertes Alkoxy bedeuten.

Ein weiterer Gegenstand der Erfindung sind 3-Phenylisoxazolin-5-carboxamide und 3-Phenylisoxazolin-5-thioamide der Formel (I), oder deren Salze 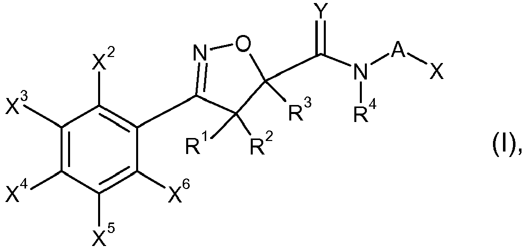 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy, oder
R¹ und R² bilden gemeinsam mit dem Kohlenstoffatom, an das sie gebunden sind, einen gesättigten, teilweise oder vollständig ungesättigten drei-, vier- oder
fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵, oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 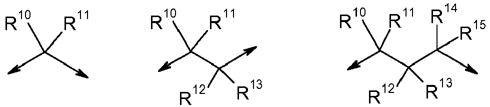 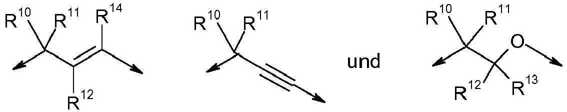 R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷,X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO²R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch n Reste aus der Gruppe bestehend aus R⁶, R^{6a}, R⁸ und R⁹ substituierten Ring aus der Gruppe bestehend aus 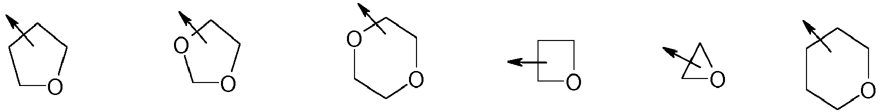 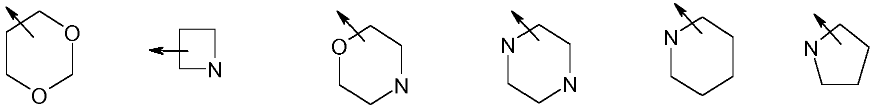 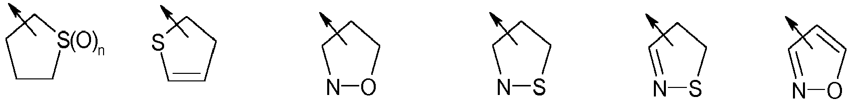 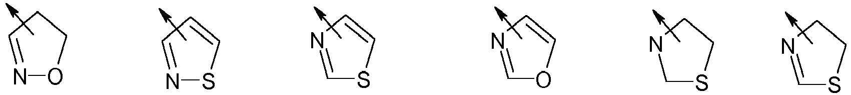 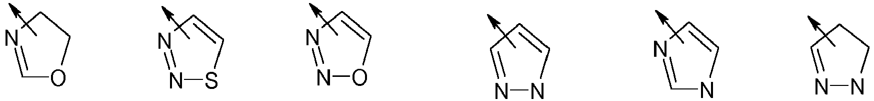 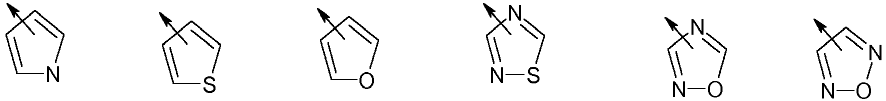 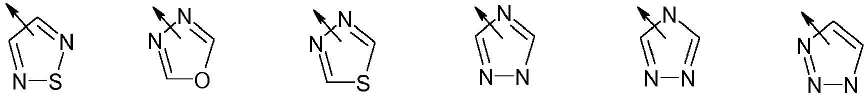 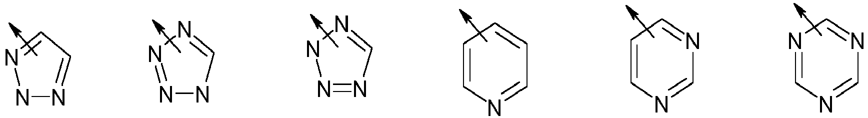 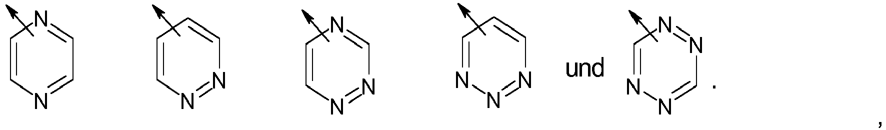 X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR₆R₈,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
mit der Maßgabe, daß X³ und X⁴ nicht gleichzeitig substituiertes oder unsubstituiertes Alkoxy bedeuten.

Alkyl bedeutet gesättigte, geradkettige oder verzweigte Kohlenwasserstoffreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit der jeweils angegebenen Anzahl von Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit der jeweils angegebenen Anzahl von Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Dimethylpropoxy, 1-Ethylpropoxy, Hexoxy, 1,1-Dimethylpropoxy, 1,2-Dimethyl-propoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome und/oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der Formel (I) umfaßt, jedoch nicht spezifisch definiert sind. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Je nach Art der oben definierten Substituenten weisen die Verbindungen der Formel (I) saure Eigenschaften auf und können mit anorganischen oder organischen Basen oder mit Metallionen Salze, gegebenenfalls auch innere Salze oder Addukte bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Ist eine Gruppe mehrfach durch Reste substituiert, so bedeutet dies, daß diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Bevorzugt sind 3-Phenylisoxazolin-5-carboxamide und 3-Phenyl-isoxazolin-5-thioamide der Formel (I), worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵, oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 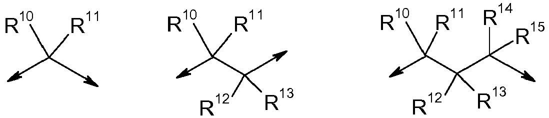 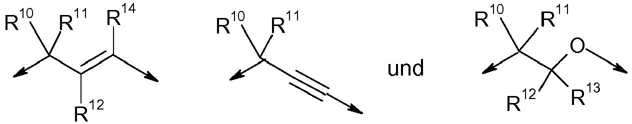 R¹⁰, R¹¹, R¹², R¹³, R¹⁴ und R¹⁵ bedeuten unabhängig voneinanderjeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷,X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO²R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch n Reste aus der Gruppe bestehend aus R⁶, R^{6a}, R⁸ und R⁹ substituierten Ring aus der Gruppe bestehend aus 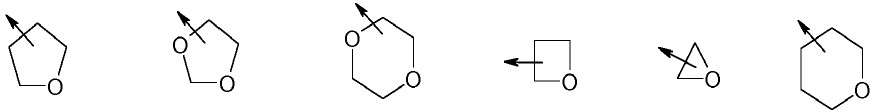 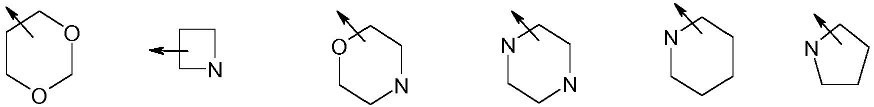 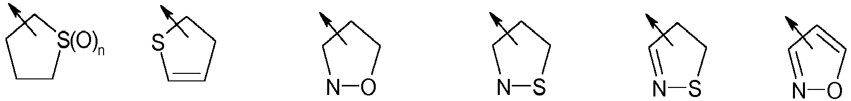 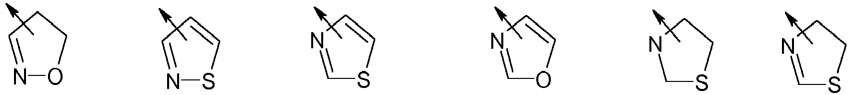 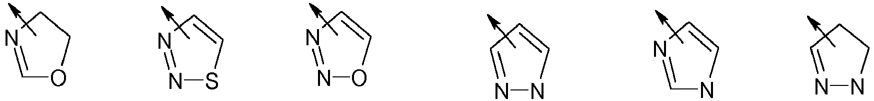 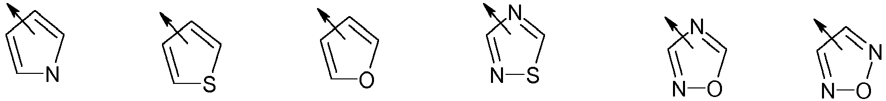 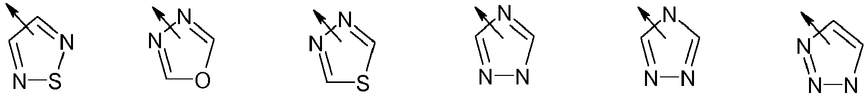 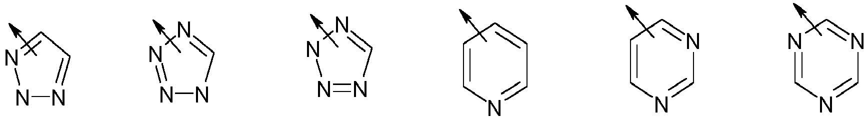 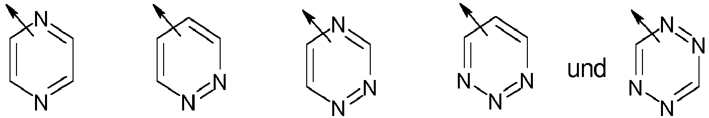 X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO²R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
mit der Maßgabe, daß X³ und X⁴ nicht gleichzeitig substituiertes oder unsubstituiertes Alkoxy bedeuten.

Besonders bevorzugt sind 3-Phenylisoxazolin-5-carboxamide und 3-Phenylisoxazolin-5-thioamide der Formel (I), worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl;
R³ bedeutet Fluor, Chlor, oder Cyano,
oder durch jeweils m Reste aus der Gruppe bestehend aus Fluor und Chlor substituiertes (C₁-C₆)-Alkyl, (C₃-C₆)-Cycloalkyl, (C₂-C₆)-Alkenyl oder (C₂-C₆)-Alkinyl, oder durch m Reste aus der Gruppe bestehend aus Fluor und Chlor substituiertes (C₁-C₆)-Alkylcarbonyl;
A bedeutet eine Bindung oder eine divalente Einheit aus der Gruppe bestehend aus CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH(CH₂OCH₃),CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ und CH₂CH₂CONHCH₂;
R⁴ bedeutet Wasserstoff oder (C₁-C₈)-Alkyl;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Cyano, Hydroxy, OR⁷, X¹, OX¹, NHX¹, S(O)ₙ R⁵, CO₂R⁸, CONR⁶R⁸, CONR⁸SO₂R⁵ und POR⁹R⁹ substituiertes (C₁-C₁₂)-Alkyl, (C₃-C₈)-Cycloalkyl, (C₂-C₁₂)-Alkenyl oder (C₂-C₁₂)-Alkinyl;
X¹ bedeutet einen durch n Reste aus der Gruppe bestehend aus R⁶, R^{6a}, R⁸ und R⁹ substituierten Ring aus der Gruppe bestehend aus 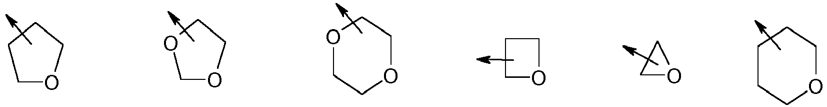 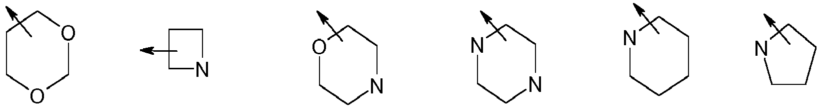 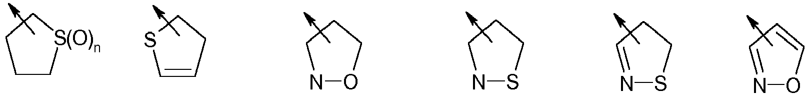 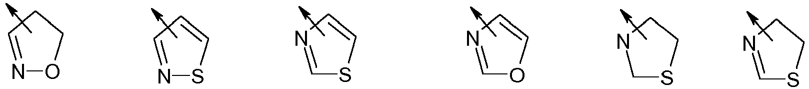 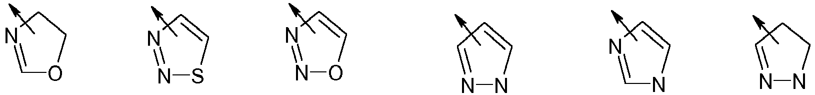 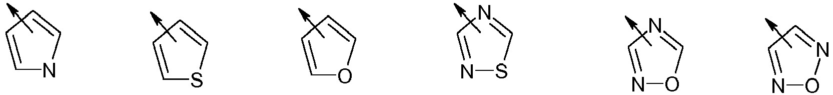 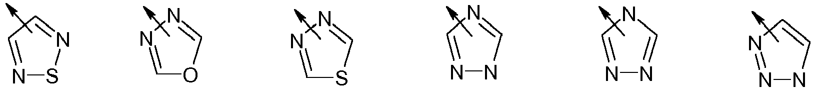 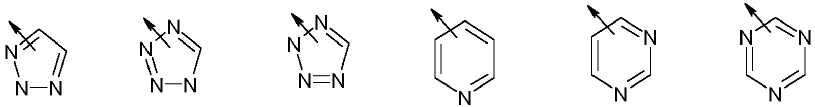 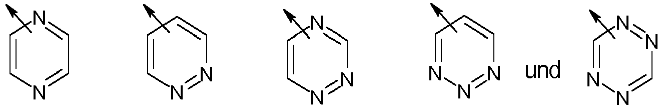 X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy;
X³ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
X⁵ bedeutet Wasserstoff oder X³;
R⁵ bedeutet Methyl oder Ethyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor und Chlor substituiertes (C₁-C₆)-Alkyl;
R⁸ bedeutet R⁷,
R⁹ bedeutet (C₁-C₃)-Alkoxy;
m bedeutet 0, 1, 2 oder 3;
n bedeutet 0, 1 oder 2;
mit der Maßgabe, daß X³ und X⁴ nicht gleichzeitig substituiertes oder unsubstituiertes Alkoxy bedeuten.

Die in oben genannten Dokumenten beschriebenen Verbindungen weisen nicht nur eine pharmakologische Wirkung auf, sondern überraschenderweise auch eine herbizide Wirkung im Falle der 3-Phenylisoxazolin-5-carboxamide und 3-Phenylisoxazolin-5-thioamide. Ein weiterer Gegenstand vorliegender Erfindung ist daher die Verwendung von 3-Phenylisoxazolin-5-carboxamide und 3-Phenylisoxazolin-5-thioamide der Formel (Ia) als Herbizide.

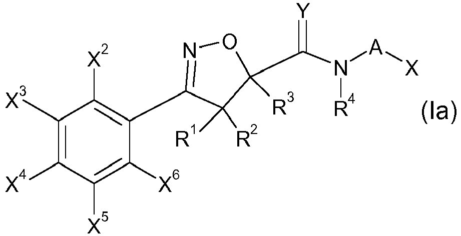

In Formel (Ia) haben die Reste und Indices folgende Bedeutung:
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
   oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
   oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
   oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano, Hydroxy,
   oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 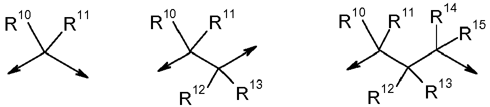 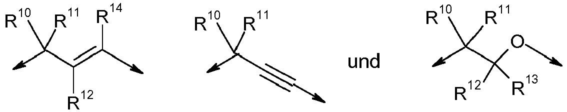
R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
   oder
   durch jeweils m Reste aus der Gruppe bestehend aus Fluor, Chlor, Brom, Iod, Cyano, Hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
   oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen fünf- oder sechsgliedrigen gesättigten, teilweise ungesättigten, vollständig ungesättigten oder aromatischen Ring, der aus r Kohlenstoffatomen, s Stickstoffatomen, n Schwefelatomen und n Sauerstoffatomen aufgebaut ist, und der durch n Reste aus der Gruppe bestehend aus R⁶, R^{6a}, R⁸ und R⁹ substituiert ist, oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, Chlor, Brom, Iod, Cyano, Nitro,
   oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
r bedeutet 1, 2, 3, 4 oder 5;
s bedeutet 0, 1, 2, 3 oder 4.

Ferner betrifft die vorliegende Erfindung die Verwendung von 3-Phenylisoxazolin-5-carboxamide und 3-Phenylisoxazolin-5-thioamide der Formel (Ia) zur Bekämpfung unerwünschter Pflanzen 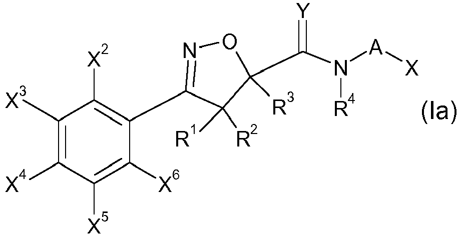 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano, Hydroxy,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 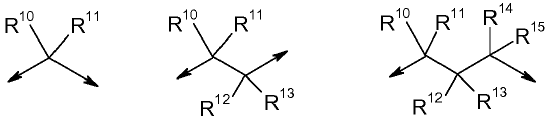 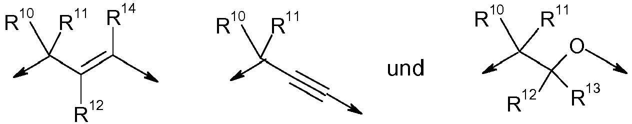 R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch n Reste aus der Gruppe bestehend aus R⁶, R^{6a} , R⁸ und R⁹ substituierten Ring aus der Gruppe bestehend aus 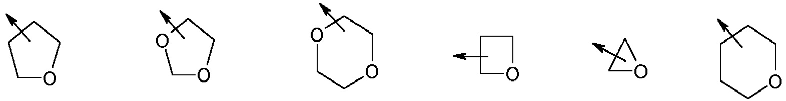 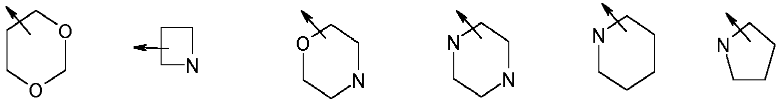 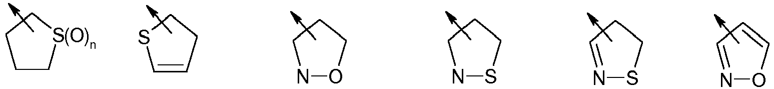 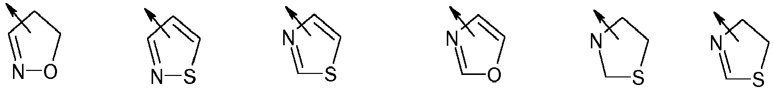 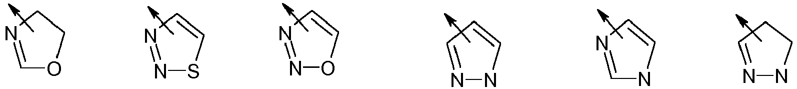 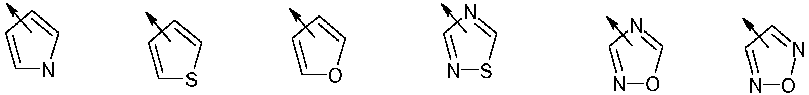 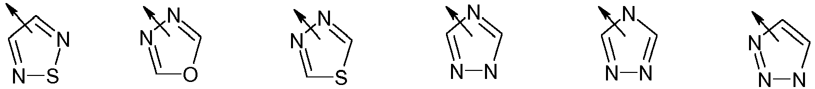 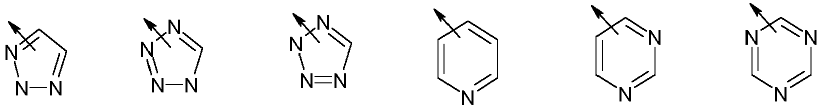 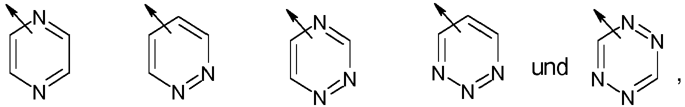 oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1; und
q bedeutet 3, 4 oder 5.

Bevorzugt als Herbizide sind 3-Phenylisoxazolin-5-carboxamide und 3-Phenylisoxazolin-5-thioamide der Formel (Ia) geeignet, worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano, Hydroxy,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 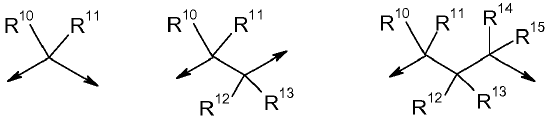 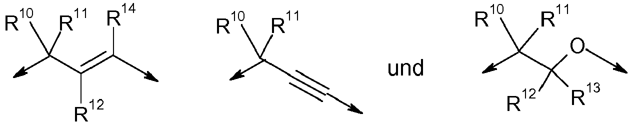 R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch n Reste aus der Gruppe bestehend aus R⁶, R^{6a} , R⁸ und R⁹ substituierten Ring aus der Gruppe bestehend aus 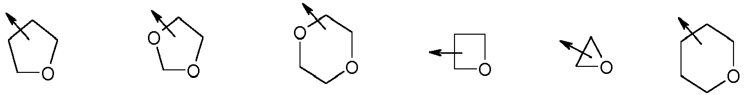 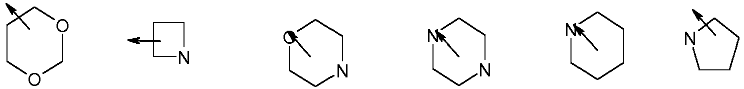 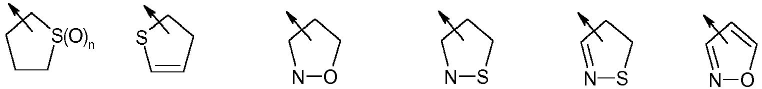 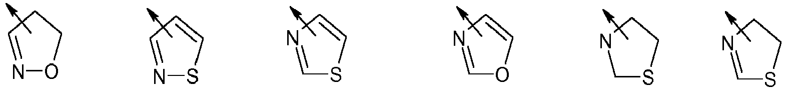 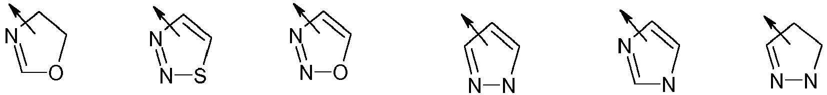 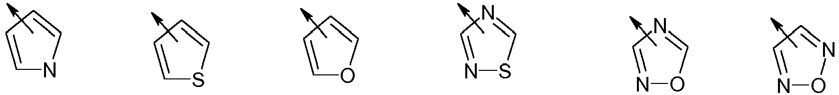 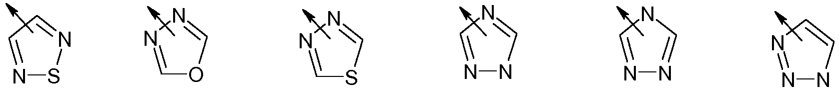 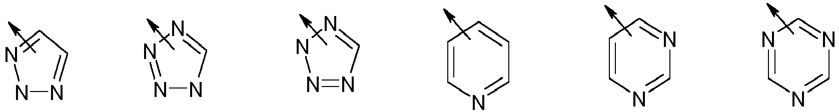 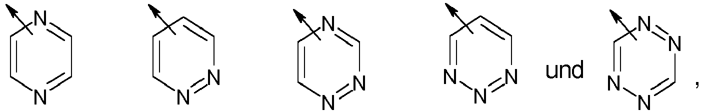 oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl,
X³ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
R⁹ bedeutet (C₁-C₃)-Alkyl oder (C₁-C₃)-Alkoxy;
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5.

Besonders bevorzugt als Herbizide sind 3-Phenylisoxazolin-5-carboxamide und 3-Phenylisoxazolin-5-thioamide der Formel (Ia) geeignet,
worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl;
R³ bedeutet Fluor, Chlor, oder Cyano,
oder durch jeweils m Reste aus der Gruppe bestehend aus Fluor und Chlor substituiertes (C₁-C₆)-Alkyl, (C₃-C₆)-Cycloalkyl, (C₂-C₆)-Alkenyl oder (C₂-C₆)-Alkinyl, oder durch m Reste aus der Gruppe bestehend aus Fluor und Chlor substituiertes (C₁-C₆)-Alkylcarbonyl;
R⁴ bedeutet Wasserstoff, Hydroxy oder (C₁-C₈)-Alkyl;
A bedeutet eine Bindung oder eine divalente Einheit aus der Gruppe bestehend aus CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C=C, CH(CF₃)CH₂, CH(CH₃)CH₂O, CH₂CH₂O, CH(cPr)CH₂O, CH(CH₂OCH₃), CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ und CH₂CH₂CONHCH₂;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Cyano, Hydroxy, X¹, OX¹, NHX¹, S(O)ₙR⁵, CO₂R⁸, CONR⁶R⁸, CONR⁸SO₂R⁵ und POR⁹R⁹ substituiertes (C₁-C₁₂)-Alkyl, (C₃-C₈)-Cycloalkyl, (C₂-C₁₂)-Alkenyl oder (C₂-C₁₂)-Alkinyl;
X¹ bedeutet einen durch n Reste aus der Gruppe bestehend aus R⁶, R^{6a}, R⁸ und R⁹ substituierten Ring aus der Gruppe bestehend aus 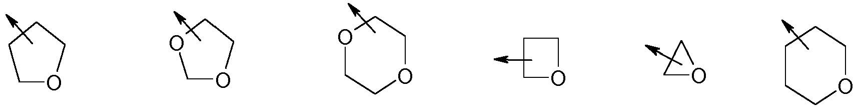 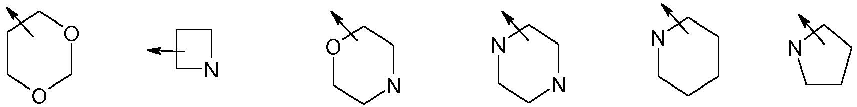 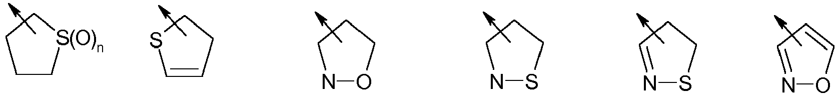 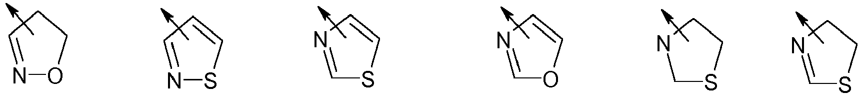 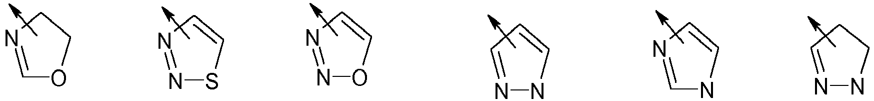 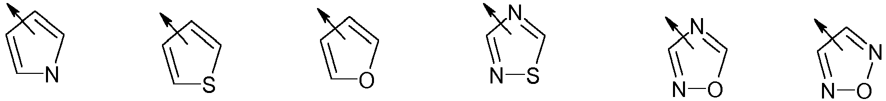 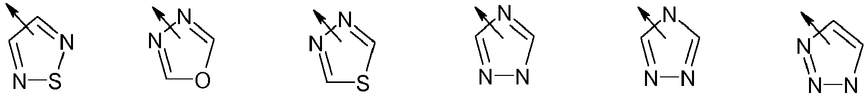 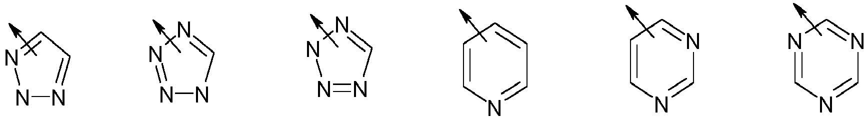 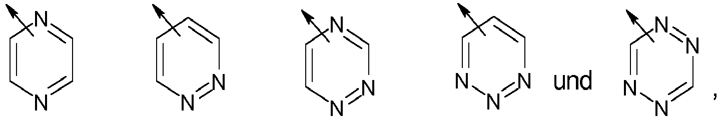 oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy,
X³ bedeutet Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy,
X⁵ bedeutet Wasserstoff oder X³;
R⁵ bedeutet Methyl oder Ethyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor und Chlor substituiertes (C₁-C₆)-Alkyl,
R⁸ bedeutet R⁷;
R⁹ bedeutet (C₁-C₃)-Alkoxy,
m bedeutet 0, 1, 2 oder 3;
n bedeutet 0, 1 oder 2.

Die erfindungsgemäßen Verbindungen können gemäß dem Fachmann an sich bekannten Reaktionen beispielsweise nach der in Schema 1 genannten Reaktionsfolge hergestellt werden.

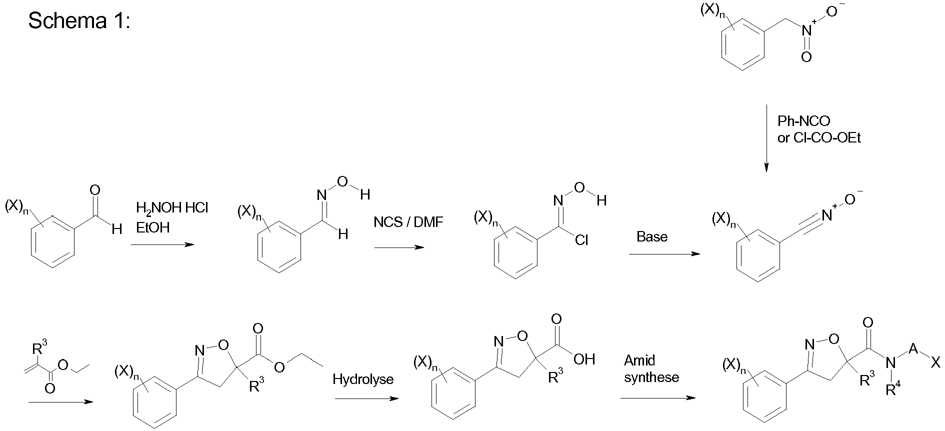

In Schema 1 und den nachfolgenden Schemata steht (X)ₙ für die Substituenten X², X³, X⁴, X⁵ und X⁶. Solche 1,3-dipolaren Cycloadditionen von Nitriloxiden mit geeigneten Dipolarophilen sind beispielsweise beschrieben in Reviews: 1,3 dipolar Cycloaddition Chemistry, Padwa, ed. Wiley, New York, 1984; Kanemasa and Tsuge, Heterocycles 1990, 30, 719.

Erfindungsgemäße Verbindungen, die in der 4- und 5- Position des Isoxazolinringsystems substituiert sind, lassen sich ebenfalls durch 1,3 dipolare Cycloaddition herstellen, indem geeignet 1,2-disubstituierte Olefine als Dipolarophile eingesetzt werden. Zumeist entstehen bei dieser Reaktion Diastereomeren-gemische, die durch Säulenchromatographie getrennt werden können. Optisch aktive Isoxazoline können durch chirale HPLC geeigneter Vorstufen oder Endstufen erhalten werden, ebenso auch durch enantioselektive Reakionen wie z.B. enzymatische Ester- oder Amidspaltungen oder durch den Einsatz von chiralen Hilfsreagenzien am Dipolarophiel wie von Olssen beschrieben, (J. Org.Chem. 1988, 53, 2468). Erfindungsgemäße Verbindungen können auch durch den Einsatz von käuflichen, geeignet substituierten Alkenen als Edukt hergestellt werden. So können geeignet substituierte Acrylsäureester oder Acrylsäureamide eingesetzt werden.

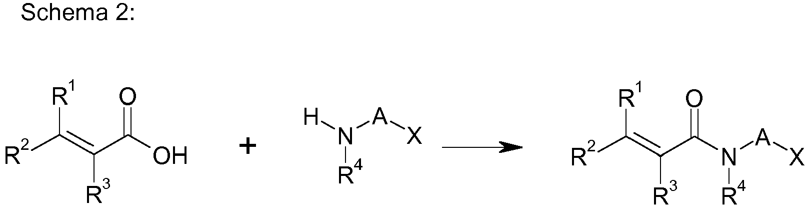

Zur Aktivierung der Acrylsäure bieten sich dabei Carbodiimide wie zum Beispiel EDCI an (Chen, F. M. F.; Benoiton, N. L. Synthesis 1979, 709). Zur Herstellung von Acrylsäureamiden siehe US2521902, JP60112746, J. of Polymer Science 1979, 17 (6), 1655. Geeignet substituierte Acrylsäureamide können in einer 1,3-Cycloadditionsreaktion mit Nitriloxiden zu den erfindungsgemäßen Verbindungen umgesetzt werden.

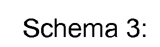

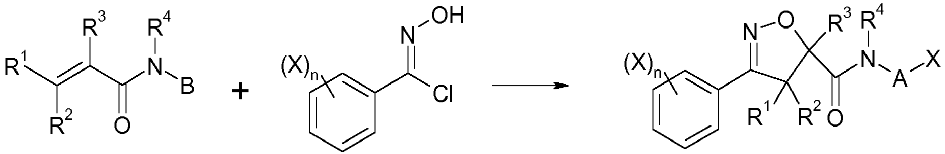

Transformationen der funktionellen Gruppen R³ sind sowohl auf der Stufe der Alkene als auch auf der Stufe der Isoxazoline möglich. In Schema 4 ist der Zugang zu verschiedenen R³-substituierten Isoxazolinen beschrieben.

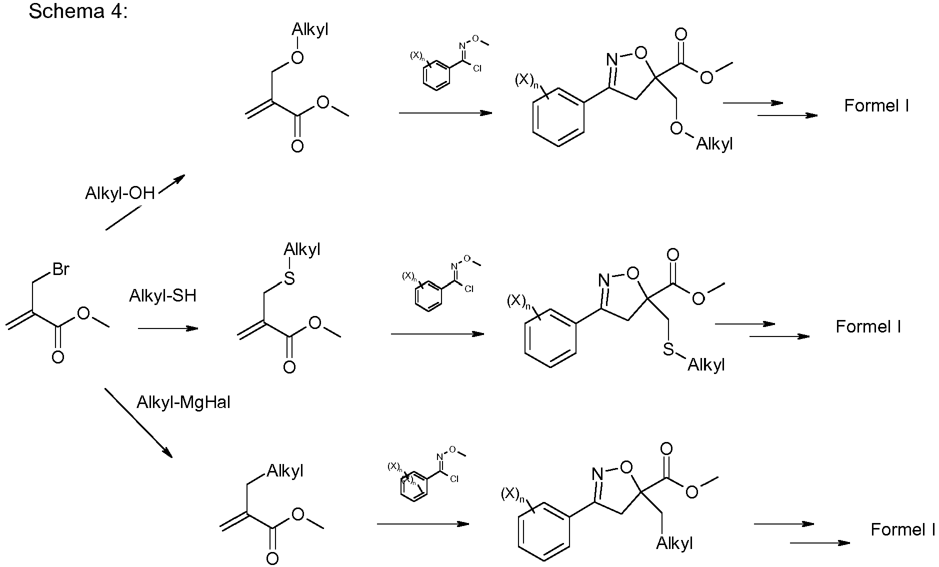

Käuflicher 2-Brommethylacrylsäuremethylester kann unter basischen Bedingungen mit Alkoholen zu 2-Alkoyxmethylacrylsäuremethylestern umgesetzt werden, die dann mit Chloroximen über die Nitriloxide zu 3-Phenyl-5-methoxycarbonylisoxazolinen reagieren. In analoger Weise sind Thioether zugänglich, welche anschließend zu den entsprechenden Sulfoxiden oder Sulfonen oxidiert werden können. Verschiedene 2-Alkylacrylsäureester können ausgehend von 2-Brommethyl-acrylsäuremethylester beispielsweise durch Umsetzung mit metallorganischen Reagentien hergestellt werden. Solche Methoden sind beispielsweise beschrieben in Metzger, Albrecht; Piller, Fabian M.; Knochel, Paul; Chemical Communications, 2008, 44, p. 5824 - 5826 und auch aus WO2006/33551 bekannt.

Erfindungsgemäße Verbindungen, die als Substituent R³ eine Cyano-Gruppe aufweisen, lassen sich analog unter Verwendung geeigneter Cyanacrylate wie zum Beispiel Ethyl-2-cyanacrylat aufbauen. Geeignet substituierte Crotonsäureester können zur Herstellung von R² und R³ disubstituierten Isoxazolinen genutzt werden. Crotonsäureester sind zum Teil käuflich und können auch beispielsweise aus 3-Bromacrylsäureethylester durch nucleophile Substitutionsreaktionen hergestellt werden. Solche Methoden sind beispielsweise beschrieben in Birkofer,L.; Hempel,K. Chem. Ber., 1963, 96, 1373; Tanoury, G.J.; Chen, M.; Dong, Y.; Forslund, R. E.; Magdziak, D.; Organic Letters, 2008, 10, 185.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automations-module beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasenunterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und der Verbindungen der Formel (Ia) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispiels-weise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolether-sulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylen-oxid-Kondensationsprodukte, Alkylpolyether, Sorbitanester wie z.B. Sorbitanfett-säureester oder Polyoxethylensorbitanester wie z.B. Polyoxyethylensorbitan-fettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasser-dispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bifenox, Bicyclopyrone, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dimefuron, Dikegulac-sodium, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacetmethyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-lndol-3-ylbuttersäure (IBA), lodosulfuron, Iodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäure-hydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methio-pyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Naprop-amide, Naptalam, NC-310, d.h. 4-(2,4-Dichlorobenzoyl)-1-methyl-5-benzyloxy-pyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonan-säure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl} oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl) phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuronmethyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

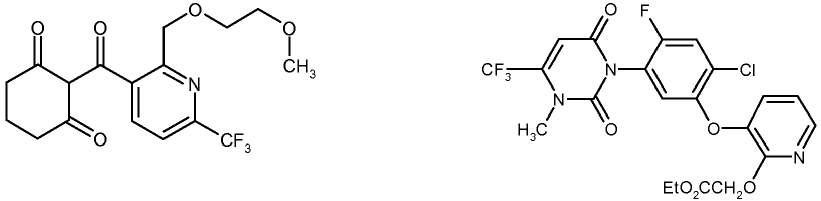

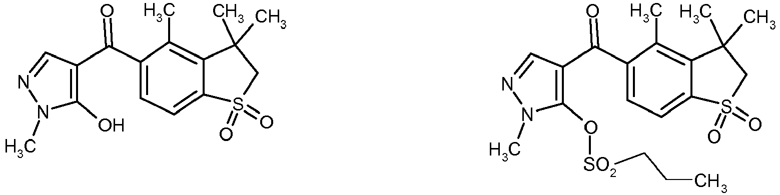

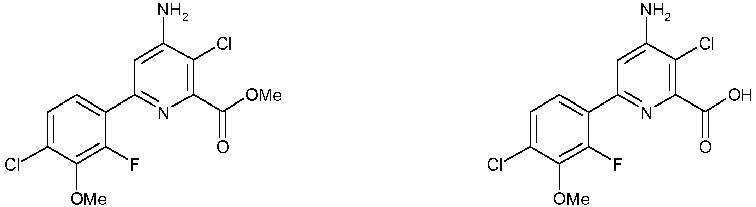

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung näher.

### A. Chemische Beispiele

### 1. Herstellung von Methyl-N-{[3-(3,5-dichlorphenyl)-5-methyl-4,5-dihydro-1,2-oxazol-5-yl]carbonyl}glycinat (Beispiel 6.454)

### Intermediat 1 : 3,5-Dichlorbenzaldehydoxim

23.82 g (342.8 mmol) Hydroxylaminhydrochlorid wurden mit 80 ml Ethanol versetzt. Nach Zugabe von 28.12 g (342.8 mmo) Natriumacetat tropfte man eine Lösung von 50.00 g (285.7 mmol) 3,5-Dichlorbenzaldehyd in 100 ml Ethanol innerhalb von 30 min zu, ließ für 2h rühren und anschließend über Nacht stehen. Die Reaktionsmischung wurde vollständig eingeengt, dann mit 500 ml CH₂Cl₂ versetzt und mit 400 ml Wasser gewaschen. Die Wasserphase wurde einmal mit 100 ml CH₂Cl₂ gewaschen, die organische Phase über Na₂SO₄ getrocknet, abfiltriert und eingeengt. Der Rückstand wurde ohne weitere Reinigung verwendet. Ausbeute: 56.50 g (98%) ¹H NMR (CDCl₃) : σ = 7.36 (s, 1 H, Ar-H), 7.47 (s, 2H, Ar-H), 7.63 (s br, 1 H, OH), 8.02 (s, 1 H, HC=NOH).

### Intermediat 2: 3,5-Dichlor-N-hydroxybenzolcarboximidoylchlorid

30.00 g (157.9 mmol) 3,5-Dichlorbenzaldehydoxim wurden in 379 ml 0.5M HCl in DMF vorgelegt und bei Raumtemperatur (RT) portionsweise mit 116.7 g (189.5 mmol) Oxone (Kaliumperoxomonosulfat) versetzt. Damit die Reaktionsmischung sich auf nicht mehr als 50°C Innentemperatur erwärmte, wurde mit einem Eisbad gekühlt. Nach 2h wurde die Reaktionslösung auf 1l Eiswasser gegossen und zweimal mit je 500 ml Ether extrahiert. Die vereinigten organischen Phasen wurden anschließend mit 400 ml 0.5M wässriger HCl und mit 200 ml gesättigter NaCl-Lösung gewaschen, über Na2SO4 getrocknet, abfiltriert und eingeengt. Der Rückstand wurde ohne weitere Reinigung verwendet. Ausbeute: 28.40 g (80%) ¹H NMR (CDCl₃) : σ = 7.43 (s, 1 H, Ar-H), 7.74 (s, 2H, Ar-H), 8.03 (s br, 1 H, OH).

### Intermediat 3: Ethyl-3-(3,5-dichlorphenyl)-5-methyl-4,5-dihydro-1,2-oxazol-5-carboxylat

5.00 g (22.3 mmol) wurden in 150 ml 2-Propanol gelöst und mit 12.71 g (111.4 mmol) Methacrylsäureethylester versetzt. Zu dieser Lösung wurden bei RT 9,36 g (111.4 mmol) NaHCO₃ gegeben und für 2h auf 40°C erwärmt. Anschließend filtrierte man vom Feststoff ab und engte das Filtrat am Rotationsverdampfer ein. Das Rohprodukt wurde durch eine Chromatographie (Kieselgel, Essigester/ n-Heptan 1:1) gereinigt. Ausbeute: 6.50 g (92%)
¹H NMR (CDCl₃) : σ = 1.34 (t, J = 8 Hz, 3H, CH₂CH₃), 1.72 (s, 3H, C-CH₃), 3.14 (d H_{A} von AB, J = 19 Hz, 1 H, N=C-CH_{A}H_{B}), 3.84 (d H_{B} von AB, J = 19 Hz, 1 H, N=C-CH_{A}H_{B}), 4.26 (q, J = 8 Hz, 2H, CH₂CH₃), 7.40 (s, 1 H, Ar-H), 7.54 (s, 2H, Ar-H).

### Intermediat 4: 3-(3,5-Dichlorphenyl)-5-methyl-4,5-dihydro-1,2-oxazol-5-carbonsäure

5.00 g (16.55 mmol) Ethyl-3-(3,5-dichlorphenyl)-5-methyl-4,5-dihydro-1,2-oxazol-5-carboxylat wurden in 50 ml Ethanol gelöst und anschließend mit 24.82 ml 1n NaOH versetzt. Nach 1 h rühren bei RT entfernte man das Lösemittel, setzte 100 ml Wasser zu und stellte die wässrige Phase durch Zugabe von 2n HCl auf pH 2 ein. Das entstandene Produkt wurde durch Extraktion mit 100 ml Essigsäureethylester, Trocknen der organischen Phase über Na2SO4, Filtration und Einengen erhalten. Ausbeute: 4.30 g (90%)
¹H NMR (CDCl₃) : σ = 1.78 (s, 3H, C-CH₃), 3.24 (d H_{A} von AB, J = 19 Hz, 1 H, N=C-CH_{A}H_{B}), 3.83 (d H_{B} von AB, J = 19 Hz, 1 H,N=C-CH_{A}H_{B}), 7.42 (s, 1 H, Ar-H), 7.53 (s, 2H, Ar-H).

### 2. Herstellung von Methyl-N-{[3-(3,5-dichlorphenyl)-5-methyl-4,5-dihydro-1,2-oxazol-5-yl]carbonyl}glycinat (Beispiel 6.454)

5.00 g(18.2 mmol) 3-(3,5-Dichlorphenyl)-5-methyl-4,5-dihydro-1,2-oxazol-5-carbonsäure wurden mit 100 ml CH₂Cl₂ und 70 mg Dimethylformamid versetzt. 3.47 g (27.4 mmol) Oxalylchlorid wurden langsam unter Rühren bei RT zugegeben. Nach 30 min wurde die Lösung eingeengt und der Rückstand (Intermediat 5) ohne weitere Reinigung eingesetzt (Rohausbeute 5.50 g). 4.72 g (37.6 mmol) Glycinmethylesterhydrochlorid wurden in 100 ml Dichlormethan zusammen mit 7.61 g (75.2 mmol) Triethylamin bei RT vorgelegt. Das oben beschriebene Rohprodukt wurde in wenig CH₂Cl₂ aufgenommen, langsam bei RT zugetropft und anschließend 30 min gerührt. Zur Aufarbeitung wurde mit 1 n HCL und anschließend mit gesättigter NaCl -Lösung extrahiert. Die organische Phase wurde über Na2SO4 getrocknet, filtriert und eingeengt. Nach Chromatographie über Kieselgel in Essigester: n-Heptan 1:1 erhielt man das Produkt. Ausbeute: 5.80 g (85%)
¹H NMR (CDCl₃) : σ = 1.75 (s, 3H, C-CH₃), 3.20 (d H_{A} von AB, J = 19 Hz, 1 H, N=C-CH_{A}H_{B}), 3.76 (s, 3H, O-CH₃), 3.78 (d H_{B} von AB, J = 19 Hz, 1 H, N=C-CH_{A}H_{B}), 4.04 (d Ab-spektrum, J_{AB} = 15Hz, 2H, CH₂-C=O), 7.26 (s br, 1 H, NH), 7.41 (s, 1 H, Ar-H), 7.53 (s, 2H, Ar-H).

### 3. Herstellung von N-{[3-(3,5-Dichlorphenyl)-5-methyl-4,5-dihydro-1,2-oxazol-5-yl]carbonyl}glycin (Beispiel 6.420)

Zu einer Lösung von 4.00 g (11.6 mmol) Methyl-N-{[3-(3,5-dichlorphenyl)-5-methyl-4,5-dihydro-1,2-oxazol-5-yl]carbonyl}glycinat in 100 ml Methanol wurden 23.2 ml 1n NaOH in Wasser getropft. Nach 30 min wurde das Lösemittel im Vakuum entfernt, der Rückstand in 50 ml Wasser aufgenommen und mit 2n HCl sauer gestellt. Das Produkt wurde durch mehrfache Extraktion mit Methylenchlorid, Einengen der organischen Phase, Trocknen über Na₂SO₄, Filtrieren und erneutem Einengen isoliert. Ausbeute: 3.80 g (94%)
¹H NMR (CDCl₃) : σ = 1.75 (s, 3H, C-CH₃), 3.21 (d H_{A} von AB, J = 19 Hz, 1 H, N=C-CH_{A}H_{B}), 3.79 (d H_{B} von AB, J = 19 Hz, 1 H, N=C-CH_{A}H_{B}), 4.08 (d Ab-spektrum, J_{AB} = 15Hz, 2H, CH₂-C=O), 7.35 (t br, J = 7 Hz, 1 H, NH), 7.41 (s, 1 H, Ar-H), 7.51 (s, 2H, Ar-H).

### 4. Herstellung von 3-(3,5-Dichlorphenyl)-5-methyl-N-{2-[(methylsulfonyl)amino]-2-oxoethyl}-4,5-dihydro-1,2-oxazol-5-carboxamid (Beispiel 6.339)

1.50 g (4.53 mmol) N-{[3-(3,5-dichlorphenyl)-5-methyl-4,5-dihydro-1,2-oxazol-5-yl]carbonyl}glycin wurden mit 50 ml CH₂Cl₂ und 40 mg Dimethylformamid versetzt. 0.862 g (6.79 mmol) Oxalylchlorid wurden langsam unter Rühren bei RT zugegeben. Nach 30 min wurde die Lösung am Rotationsverdampfer eingeengt und ohne weitere Reinigung eingesetzt (Rohausbeute 1.60 g). 400 mg dieses Rohproduktes (Intermediat 6) wurden in 5 ml CH₂Cl₂ gelöst und zu einer Lösung aus 108 mg (1.14 mmol) Methansulfonsäureamid, 14 mg (0.11 mmol) Dimethylaminopyridin, 231 mg (2.29 mmol) Triethylamin in 10 ml CH₂Cl₂ getropft. Nach 2h wurde das Lösemittel im Vakuum entfernt und das Rohprodukt über Kieselgel mit Essigsäureethylester /n-Heptan (1:1) chromatographiert. Ausbeute: 160 mg (34%)
¹H NMR (DMSO-D₆) : σ = 1.57 (s, 3H, C-CH₃), 3.18 (s, 3H, O₂S-CH₃), 3.42 (d H_{A} von AB, J = 19 Hz, 1 H, N=C-CH_{A}H_{B}), 3.72 (d H_{B} von AB, 1 H, J = 19 Hz, 1 H, N=C-CH_{A}H_{B}), 3.78 (AB-spektrum, J_{AB} = 15Hz, 2H, CH₂-C=O), 7.71 (s, 2H, Ar-H), 7.75 (s, 1 H, Ar-H), 8.24 (t br, J = 6 Hz, 1 H, NH).

### 5. Herstellung von N-tert-Butyl-3-(3,5-dichlorphenyl)-5-methyl-4,5-dihydro-1,2-oxazol-5-carboxamid (Beispiel 6.117)

5.00 g(18.2 mmol) 3-(3,5-Dichlorphenyl)-5-methyl-4,5-dihydro-1,2-oxazol-5-carbonsäure wurden mit 100 ml CH₂Cl₂ und 70 mg Dimethylformamid versetzt. 3.47 g (27.4 mmol) Oxalylchlorid wurden langsam unter Rühren bei RT zugegeben. Nach 30 min wurde die Lösung eingeengt und ohne weitere Reinigung eingesetzt (Rohausbeute 5.50 g). 200 mg (0,68 mmol) dieses Rohproduktes wurden in 3 ml CH₂Cl₂ aufgenommen und zu einer Lösung aus 50 mg (0,68 mmol) tert. Butylamin 69 mg, (0.68 mmol) Triethylamin und 10 mg Dimethylaminopyridin in 50 ml CH₂Cl₂ bei 0°C getropft. Zur Aufarbeitung wurde mit Wasser, mit gesättigter NaHCO₃-Lösung und erneut mit Wasser extrahiert. Nach Trocknen der organischen Phase, Filtrieren und Entfernen des Lösemittels im Vakuum, erhielt man das Produkt. Ausbeute: 100 mg (43%)
¹H NMR (CDCl₃) : σ = 1.37 (s, 9H, C-(CH₃)₃), 1.68 (s, 3H, CH₃), 3.14 (d H_{A} von AB, J = 18 Hz, 1 H, N=C-CH_{A}H_{B}), 3.76 (d H_{B} von AB, 1 H, J = 18 Hz, N=C-CH_{A}H_{B}), 6.61 (s br, 1 H, NH), 7.42 (s, 1 H, Ar-H), 7.53 (s, 2H, Ar-H).

### 6. Herstellung von 3-(3,5-Dichlorphenyl)-5-(hydroxymethyl)-4,5-dihydro-1,2-oxazol-5-carbonsäure-cyclopropylamid (Beispiel 10.042)

### Intermediat 7: Ethyl-3-(3,5-dichlorphenyl)-5-(hydroxymethyl)-4,5-dihydro-1,2-oxazol-5-carboxylat

3.00 g (15.8 mmol) 3,5-Dichlorbenzaldehydoxim wurden in 100 ml DMF zusammen mit 2.21g (16.6 mmol) N-Chlorsuccinimid bei RT 4h gerührt. Anschließend wurden 3.08 g (23.7 mmol) 2-(Hydroxymethyl)acrylsäureethylester und 2.40 g (23.7 mmol) Triethylamin zugegeben und 18h bei RT gerührt. Die Reaktionslösung wurde im Vakuum eingeengt und dann mit CH₂Cl₂ und Wasser versetzt. Die wässrige Phase wurde einmal mit CH₂Cl₂ nachextrahiert und die vereinigten organischen Phasen zweimal mit Wasser gewaschen, über Na2SO4 getrocknet, filtriert und eingeengt. Nach Chromatographie über Kieselgel mit n-Heptan : Essigsäureethylester (3:1) erhielt man das Produkt. Ausbeute: 3.10 g (62%)
¹H NMR (CDCl₃) : σ = 1.33 (t, J=8 Hz, 3H, CH₂-CH₃), 2.07 (m, 1 H, OH), 3.62 (AB-Spektrum, J_{AB} = 15 Hz, 2H, CH_{A}H_{B}-C=N), 4.28 (q, J=8Hz, 2H, CH₂-CH₃), 7.42 (s, 1 H, Ar-H), 7.56 (s, 2H, Ar-H).

### Intermediat 8: 3-(3,5-dichlorphenyl)-5-(hydroxymethyl)-4,5-dihydro-1,2-oxazol-5-carbonsäure

2.80 g (8.80 mmol) Ethyl-3-(3,5-dichlorphenyl)-5-(hydroxymethyl)-4,5-dihydro-1,2-oxazol-5-carboxylat wurden in 100 ml Methanol gelöst und mit einer Lösung aus 458 mg (11.4 mmol) NaOH in 20 ml Wasser versetzt. Nach 18 h Rühren bei RT entfernte man die Lösemittel im Vakuum, gab Wasser und Methylenchlorid zu, und trennte die organische Phase ab. Beim Ansäuren der wässrigen Phase schied sich das Produkt als weißer Niederschlag ab, der abfiltriert und getrocknet wurde. Ausbeute: 2.22 g (87 %)
¹H NMR (CDCl₃) : σ = 3.65 (AB-Spektrum, J_{AB} = 16 Hz, 2H, CH_{A}H_{B}-C=N), 4.02 (AB-Spektrum, J_{AB} = 12 Hz, 2H, CH_{A}H_{B}-OH), 7.43 (s, 1 H, Ar-H), 7.56 (s, 2H, Ar-H).

### 7. Herstellung von 3-(3,5-Dichlorphenyl)-5-(hydroxymethyl)-4,5-dihydro-1,2-oxazol-5-carbonsäure-cyclopropylamid (Beispiel 10.042)

400 mg (1.38 mmol) 3-(3,5-dichlorphenyl)-5-(hydroxymethyl)-4,5-dihydro-1,2-oxazol-5-carbonsäure wurde in 50 ml Methylenchlorid vorgelegt und mit 264 mg (1.38 mmol) N-[3-(dimethylamino)propyl]-N'-ethylcarbodiimide hydrochlorid, 10 mg DMAP als Katalysator, und 79 mg (1.38 mmol) Cyclopropylamin versetzt und 18h bei RT gerührt. Die Reaktionslösung wurde jeweils einmal mit wässriger NaHCO₃, 2n HCl und Wasser gewaschen. Anschließend trocknete man über MgSO₄, filtrierte und engte im Vakuum ein. Nach Trennung über RP - HPLC (Acetontril : Wasser) erhielt man das Produkt.
Ausbeute: 80 mg (18%)

¹H NMR (CDCl₃ / TMS) : σ = 0.55 (m, 2H, c-Pr-HH), 0.82 (m, 2H, c-Pr-HH),2.78 (m, 1 H, NH-CH), 3.58 (AB-Spektrum, J_{AB} = 19 Hz, 2H, CH_{A}H_{B}-C=N), 3.91 J_{AB} = 14 Hz, 2H, CH_{A}H_{B}-OH), 6.82 (s br, 1 H, NH), 7.45 (s, 1 H, Ar-H), 7.53 (s, 2H, Ar-H).

### 8. Herstellung von 5-Cyan-3-(3,5-difluorphenyl)-N-[(1-ethyl-3-methyl-1H-pyrazol-4-yl)methyl]-4,5-dihydro-1,2-oxazol-5-carboxamid (Bsp.: 4.074)

Intermediat 9: Ethyl-5-cyan-3-(3,5-difluorphenyl)-4,5-dihydro-1,2-oxazol-5-carboxylat Zu einer Lösung von 3,5-Difluorbenzaldehydoxim (1.00 g, 6.37 mmol) in DMF (60 ml) wurde NCS (850 mg, 6.37 mmol) gegeben und die Mischung für 1 h bei 40°C gerührt. Anschließend wurde das Heizen beendet und Ethyl-2-cyanacrylat (1.20 g, 9.55 mmol) und NaHCO₃ (561 mg, 6.68 mmol) zugegeben. Es wurde für 2 h bei RT gerührt und anschließend mit Wasser (400 ml) versetzt. Die Mischung wurde mit MTBE extrahiert (3 x 50 ml), die vereinten organischen Phasen mit verdünnter Schwefelsäure (0.5 M, 30 ml) gewaschen, über Na₂SO₄ getrocknet und eingeengt. Chromatographie des Rückstands über Kieselgel mit Heptan/EtOAc als Eluent lieferte 810 mg leicht verunreinigtes Ethyl-5-cyan-3-(3,5-difluorphenyl)-4,5-dihydro-1,2-oxazol-5-carboxylat, das ohne weitere Reinigung verwendet wurde.
¹H NMR (DMSO D₆) σ = 1.26 (t, 3H); 4.25-4.44 (m, 3H); 4.48 (d, 1 H); 7.41-7.51 (m, 3H).

Intermediat 10: 5-Cyan-3-(3,5-difluorphenyl)-4,5-dihydro-1,2-oxazol-5-carbonsäure Das Intermediat 9 wurde analog zu der Vorschrift Intermediat 4 zur Carbonsäure Intermediat 10 in THF als Reaktionslösemittel hydrolysiert. Ausbeute: 25%
¹H NMR (DMSO d6 / TMS) : σ = 4.23 (d,1 H, CHH); 4.41 (d,1 H, CHH); 7.40-7.53 (m, 3H, Ar-H).

### 9. Herstellung von 5-Cyan-3-(3,5-difluorphenyl)-N-[(1-ethyl-3-methyl-1H-pyrazol-4-yl)methyl]-4,5-dihydro-1,2-oxazol-5-carboxamid (Bsp.: 4.074)

50 mg (0.19 mmol) 5-Cyan-3-(3,5-difluorphenyl)-4,5-dihydro-1,2-oxazol-5-carbonsäure wurden in 4 ml CH₂Cl₂ vorgelegt und mit 57 mg (0.30 mmol) N-[3-(dimethylamino)propyl]-N'-ethylcarbodiimide hydrochlorid, 27 mg (0.20 mmol)1-Hydroxybenzotriazol und 30 mg (0.22 mmol) (1-ethyl-3-methyl-1 H-pyrazol-4-yl)methylamin versetzt und 18h bei RT gerührt. Die Lösung wurde jeweils einmal mit wässriger NaHCO₃, 2n HCl und Wasser gewaschen. Anschließend trocknete man über Na₂SO₄, filtrierte und engte im Vakuum ein. Nach Chromatographie über Kieselgel in Essigsäureethylester/ n-Heptan erhielt man das Produkt.
Ausbeute: 30 mg (23%)
¹H NMR (CDCl₃) : σ = 1.45 (t, 3H, CH₂CH₃); 2.21 (s, 3H, C-CH₃); 3.98 (d, 1 H, isoxazolinCHH); 4.07 (q, 2H, N-CH₂CH₃); 4.10 (d, 1 H, isoxazolinCHH); 4.23-4.41 (m, 2H, NH-CH₂), 6.76 (s br, 1 H, NH); 6.94 (m, 1 H, Ph-H); 7.17 (m, 2H, Ph-H); 7.30 (s, 1H, Pyrazol-H).

### 10. Herstellung von Ethyl-3-(3-cyclopropyl-5-fluorphenyl)-5-methyl-4,5-dihydro-1,2-oxazol-5-carboxylat

### Intermediat 11:

Eine Mischung aus Ethyl-3-(3-brom-5-fluorphenyl)-5-methyl-4,5-dihydro-1,2-oxazol-5-carboxylat (300 mg, 0.91 mmol), Cyclopropylboronsäure (312 mg, 3.64 mmol), Kaliumcarbonat (502 mg, 3.64 mmol), Wasser (0.70 ml, 38.86 mmol) und Toluol (6.3 ml) wurde für 5 min mittels Durchleiten von Stickstoff entgast. Anschließend wurden Tricyclohexylphosphan (38 mg, 0.14 mmol) und Palladium(II)acetat (20 mg, 0.02 mmol) zugegeben und die Reaktionsmischung für 30 min im Mikrowellenofen in einem verschlossenen Gefäß auf 130°C erhitzt. Nach Abkühlung wurde die Mischung mit Wasser (20 ml) versetzt und mit EtOAc (3 x 20 ml) extrahiert. Die vereinten organischen Phasen wurden über Natriumsulfat getrocknet und das Lösemittel im Vakuum entfernt. Nach Chromatographie des Rückstands über Kieselgel mit Heptan/EtOAc als Eluent wurden 130 mg (49%) Ethyl-3-(3-cyclopropyl-5-fluorphenyl)-5-methyl-4,5-dihydro-1,2-oxazol-5-carboxylat erhalten.
¹H NMR (CDCl₃ / TMS) : σ = 0.72 (m, 2H); 1.02 (m, 2H); 1.32 (t, 3H); 1.71 (s, 1 H); 1.91 (m, 1H); 3.16 (d, 1H); 3.84 (d, 1 H); 4.26 (m, 2H); 6.79 (m, 1 H); 7.12 (m, 1 H); 7.17 (m, 1H).

### Intermediat 12: Ethyl-3-(3-ethyl-5-fluorphenyl)-5-methyl-4,5-dihydro-1,2-oxazol-5-carboxylat

Ethyl-3-(3-brom-5-fluorphenyl)-5-methyl-4,5-dihydro-1,2-oxazol-5-carboxylat (1000 mg, 3.03 mmol), Kaliumcarbonat (1.67 g, 12.12 mmol) und Tetrakis(triphenyl-phosphan)palladium (350 mg, 0.30 mmol) wurden in DMF (10 ml) suspendiert. Anschließend wurde Triethylboran (1 M in Hexan, 4.24 ml, 4.24 mmol) zugegeben und die Reaktionsmischung im Mikrowellenofen für 30 min auf 150°C erhitzt. Nach Abkühlung wurde die Mischung mit Wasser (20 ml), Schwefelsäure (1 M, 5 ml) und CH₂Cl₂ (20 ml) versetzt und geschüttelt. Anschließend wurde die organische Phase über Na₂SO₄ getrocknet und das Lösemittel im Vakuum entfernt. Chromatographie des Rückstands über Kieselgel mit Heptan/EtOAc als Eluent lieferte 701 mg (83%) Ethyl-3-(3-ethyl-5-fluorphenyl)-5-methyl-4,5-dihydro-1,2-oxazol-5-carboxylat.
¹H NMR (CDCl₃) : σ = 1.25 (t, 3H); 1.32 (t, 3H); 1.71 (s, 3H); 2.67 (m, 2H); 3.18 (d, 1 H); 3.86 (d, 1 H); 4.26 (m, 2H); 6.96 (m, 1 H); 7.18 (m, 1 H); 7.27 (m, 1 H).

Intermediate 11 und 12 wurden analog zu Vorschrift Intermediat 10 verseift und analog den Vorschriften zu Beispiel 6.339 zu den erfindungsgemäßen Verbindungen umgesetzt.

### 11. Herstellung von 3-(3,5-Difluorphenyl)-5-methyl-N-(1H-pyrazol-4-ylmethyl)-4,5-dihydro-1,2-oxazol-5-carboxamid (Bsp.: 1.261)

Zu einer Lösung von 3-(3,5-Difluorphenyl)-5-methyl-4,5-dihydro-1,2-oxazol-5-carbonsäure (1.00 g, 4.15 mmol) in Dichlormethan (30 ml) wurden 1-(1H-Pyrazol-4-yl)methanaminhydrochlorid (609 mg, 4.56 mmol), N,N-Diisopropylethylamin (1.61 g, 12.44 mmol), HOBt (506 mg, 4.15 mmol) und EDCI (1.59 g, 8.29 mmol) gegeben und die Mischung für 16 h bei RT gerührt. Anschließend wurde mit H₂SO₄ (0.5 M, 10 ml) versetzt und mit CH₂Cl₂ (20 ml) verdünnt. Die wässrige Phase wurde mit CH₂Cl₂ extrahiert (2 x 20 ml), die vereinten organischen Phasen mit gesättigter NaHCO₃-Lösung gewaschen (30 ml), über Na₂SO₄ getrocknet und das Lösemittel entfernt. Es wurden 1.2 g 3-(3,5-Difluorphenyl)-5-methyl-N-(1H-pyrazol-4-ylmethyl)-4,5-dihydro-1,2-oxazol-5-carboxamid erhalten, das ohne weitere Reinigung verwendet wurde.
¹H NMR (CDCl₃) : σ = 1.72 (s, 3H); 3.17 (d, 1 H); 3.77 (d, 1 H); 4.28 (dd, 1 H); 4.41 (dd, 1 H); 6.87 (m, 1 H); 7.01 (brt, 1 H); 7.14 (m, 2H); 7.53 (brs, 2H).

### 12. Herstellung von N-{[1-(Cyanmethyl)-1H-pyrazol-4-yl]methyl}-3-(3,5-difluorphenyl)-5-methyl-4,5-dihydro-1,2-oxazol-5-carboxamid (Bsp.: 1.103)

Zu einer Lösung von 3-(3,5-Difluorphenyl)-5-methyl-N-(1H-pyrazol-4-ylmethyl)-4,5-dihydro-1,2-oxazol-5-carboxamid (200 mg, 0.62 mmol) in DMF (10 ml) bei 0°C wurde NaH (60 Gew.-% Suspension in Mineralöl, 30 mg, 0.75 mmol) gegeben und die Mischung für 15 min gerührt. Danach wurde auf RT erwärmt und Bromacetonitril (225 mg, 1.87 mmol) zugegeben. Es wurde für 2 h bei RT und anschließend für 4 h bei 60°C gerührt. Nach Abkühlung auf RT wurde Schwefelsäure (0.5 M, 10 ml) zugegeben und die Mischung mit EtOAc extrahiert (3 x 20 ml). Die vereinten organischen Phasen wurden über Na₂SO₄ getrocknet und das Lösemittel im Vakuum entfernt. Der Rückstand wurde über Kieselgel mit Heptan/EtOAc als Eluent chromatographiert und lieferte N-{[1-(Cyanmethyl)-1H-pyrazol-4-yl]methyl}-3-(3,5-difluorphenyl)-5-methyl-4,5-dihydro-1,2-oxazol-5-carboxamid (144 mg, 64%).
¹H NMR (CDCl₃) : σ = 1.72 (s, 3H); 3.19 (d, 1H), 3.76 (d, 1H), 4.23 (dd, 1H), 4.38 (dd, 1 H); 5.02 (s, 2H); 6.88 (m, 1 H); 7.04 (brt, 1 H); 7.16 (m, 2H); 7.49 (s, 1 H); 7.50 (s, 1 H).

### 12. Herstellung von N-[(3-Chlor-1-ethyl-1H-pyrazol-4-yl)methyl]-3-(3,5-dichlorphenyl)-5-methyl-4,5-dihydro-1,2-oxazol-5-carboxamid (Bsp.: 1.344)

(3-Amino-1-ethyl-1H-pyrazol-4-carbonitrilhydrochlorid) wurde analog Schmidt et al. Helvetiva Chimica Acta 1959, 42, 763 und 770 hergestellt und wie folgt weiter umgesetzt:

### Intermediat 15: 3-Chlor-1-ethyl-1 H-pyrazol-4-carbonitril

1.00 g, 7.34 mmol 3-Amino-1-ethyl-1H-pyrazol-4-carbonitrilhydrochlorid wurde in halbkonzentrierter Salzsäure (50 ml) gelöst, auf 0°C gekühlt und mit Natriumnitrit (532 mg, 7.71 mmol) versetzt. Die entstandene Mischung wurde für 45 Minuten bei 0°C gerührt und anschließend zu einer auf 60°C vorgewärmten Lösung von Kupfer(I)chlorid (727 mg, 7.34 mmol) in halbkonzentrierter Salzsäure getropft, wobei Gasentwicklung auftrat. Die Reaktionsmischung wurde für 2 Stunden bei 60°C nachgerührt, auf RT abgekühlt und mit CH₂Cl₂ extrahiert (3 x 50 ml). Die vereinten organischen Phasen wurden über Na₂SO₄ getrocknet, das Lösemittel entfernt und der Rückstand über Kieselgel mit Dichlormethan/Methanol als Eluent chromatographiert. Es wurden 630 mg (55%) 3-Chlor-1-ethyl-1H-pyrazol-4-carbonitril erhalten.
¹H NMR (DMSO-D₆ / TMS) : σ = 1.37 (t, 3H); 4.17 (q, 2H); 8.68 (s, 1 H).

### Intermediat 16: 1-(3-Chlor-1-ethyl-1 H-pyrazol-4-yl)methanamin

Zu einer Lösung von 3-Chlor-1-ethyl-1 H-pyrazol-4-carbonitril (620 mg, 3.99 mmol) in Methanol (40 ml) wurden konzentrierte Ammoniak-Lösung (20 ml) und Raney-Nickel (1.2 g, 40 Gew.-% Suspension in Wasser) gegeben und die Mischung anschließend unter heftigem Rühren für 16 Stunden unter einer Wasserstoff-Atmosphäre hydriert. Zur Aufarbeitung wurde die Reaktionsmischung über Celite filtriert und das Filtrat im Vakuum eingeengt. Es wurden 645 mg 1-(3-Chlor-1-ethyl-1 H-pyrazol-4-yl)methanamin erhalten, das ohne weitere Reinigung verwendet wurde.
¹H NMR (DMSO-D₆) : σ =] 1.33 (t, 3H); 3.48 (s, 2H); 4.04 (q, 2H); 7.67 (s, 1 H).

### 13. Herstellung von N-[(3-Chlor-1-ethyl-1H-pyrazol-4-yl)methyl]-3-(3,5-dichlorphenyl)-5-methyl-4,5-dihydro-1,2-oxazol-5-carboxamid (Bsp.: 1.344)

Intermediat 5 wurde analog zur Herstellung von Beispiel 6.454 mit dem Intermediat 16 zu den erfindungsgemäßen Verbindungen wie zum Beispiel 1.344 umgesetzt.

### 14. Herstellung von N-Cyclopropyl-3-(3-fluor-5-methylphenyl)-5-methyl-4,5-dihydro-1,2-oxazol-5-carbothioamid (Beispiel 6.084)

N-Cyclopropyl-3-(3-fluor-5-methylphenyl)-5-methyl-4,5-dihydro-1,2-oxazol-5-carboxamid (Beispiel 6.083) (41 mg, 0.15 mmol) und 4-Methoxyphenyldithio-phosphonsäureanhydrid (71 mg, 0.18 mmol) wurden in Toluol (10 ml) und THF (5 ml) gelöst und die Mischung für 4 h auf 100°C erhitzt. Anschließend wurde die Mischung auf RT abgekühlt und im Vakuum eingeengt. Chromatographie des Rückstands über Kieselgel mit Heptan/EtOAc als Eluent lieferte 32 mg (74%) N-Cyclopropyl-3-(3-fluor-5-methylphenyl)-5-methyl-4,5-dihydro-1,2-oxazol-5-carbothioamid.
¹H NMR (CDCl₃) : σ = 0.69 (m, 2H); 0.95 (m, 2H); 1.86 (s, 3H); 2.36 (s, 3H); 3.25 (m, 1 H), 3.37 (d, 1 H); 4.23 (d, 1 H); 6.93 (m, 1 H); 7.13-7.23 (m, 2H); 8.60 (brs, 1 H).

In Analogie zu der Herstellung der oben genannten Verbindungen und gemäß den allgemeinen Angaben zur Herstellung sind die in folgenden Tabellen genannten Verbindungen erhältlich. Die NMR-Daten der in diesen Tabellen offenbarten Beispiele werden entweder in klassischer Form (δ-Werte, Anzahl der H-Atome, Multiplettauf-spaltung) oder als sogenannte NMR-Peak-Listen aufgeführt. Beim NMR-Peak-Listenverfahren werden die NMR-Daten ausgewählter Beispiele in Form von NMR-Peaklisten notiert, wobei zu jedem Signalpeak erst der δ-Wert in ppm und dann die Signalintensität durch ein Leerzeichen getrennt aufgeführt wird. Die δ-Wert - Signalintensitäts - Zahlenpaare von verschiedenen Signalpeaks werden durch Semikolons voneinander getrennt aufgelistet. Leerzeichen getrennt aufgeführt wird. Die δ-Wert - Signalintensitäts - Zahlenpaare von verschiedenen Signalpeaks werden durch Semikolons voneinander getrennt aufgelistet.

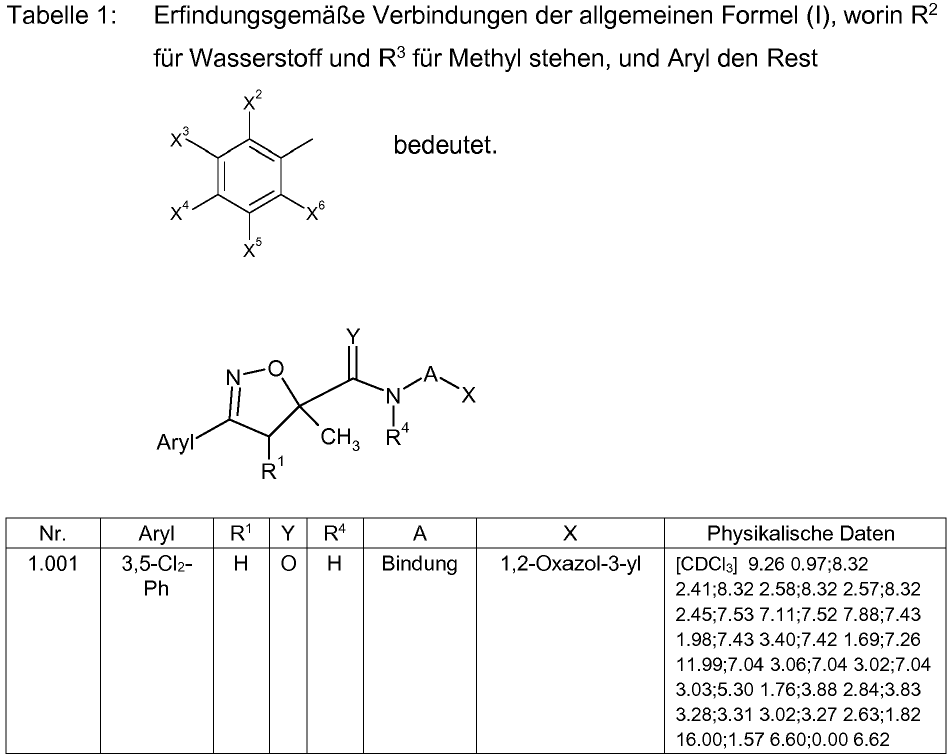

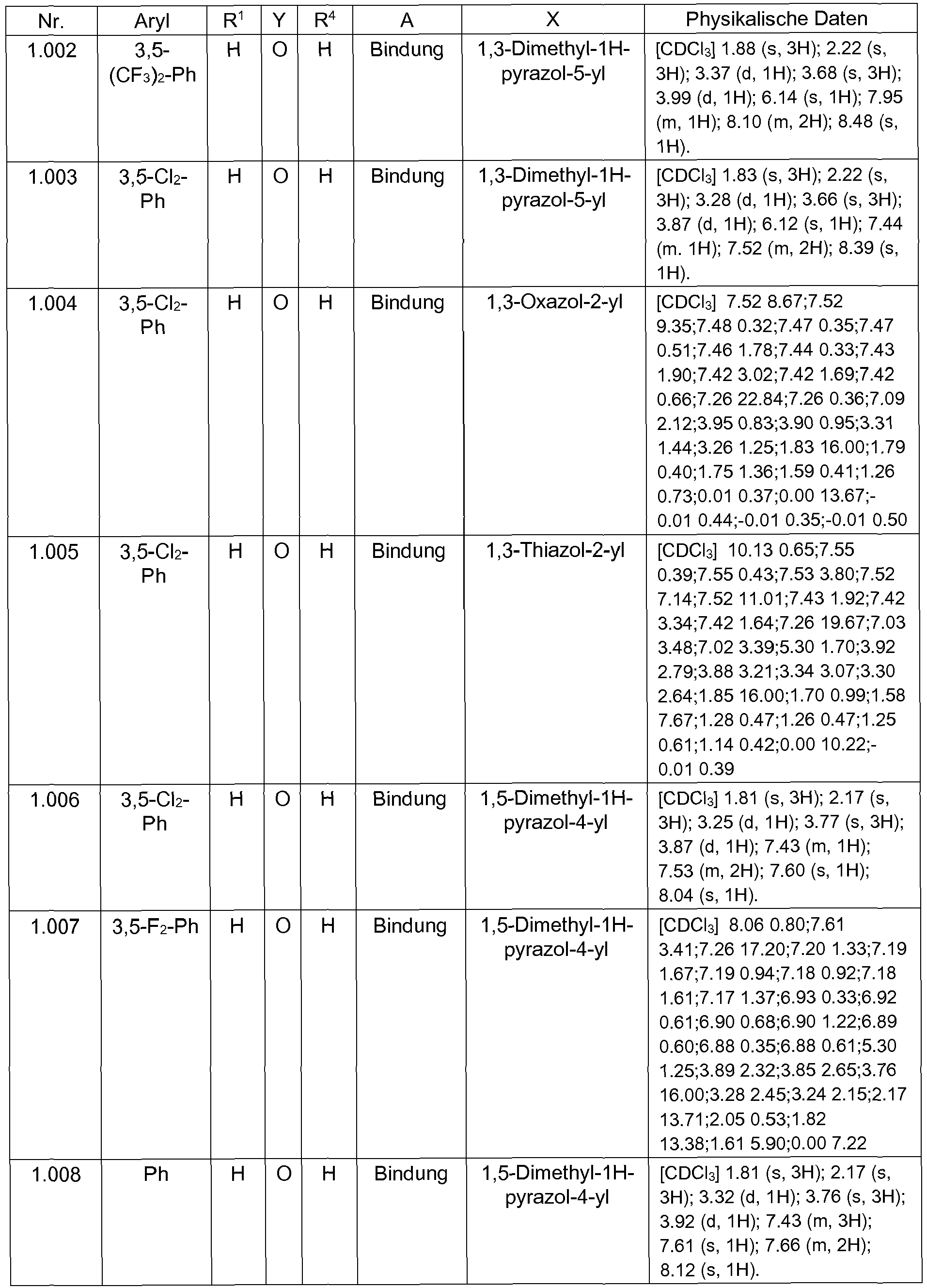

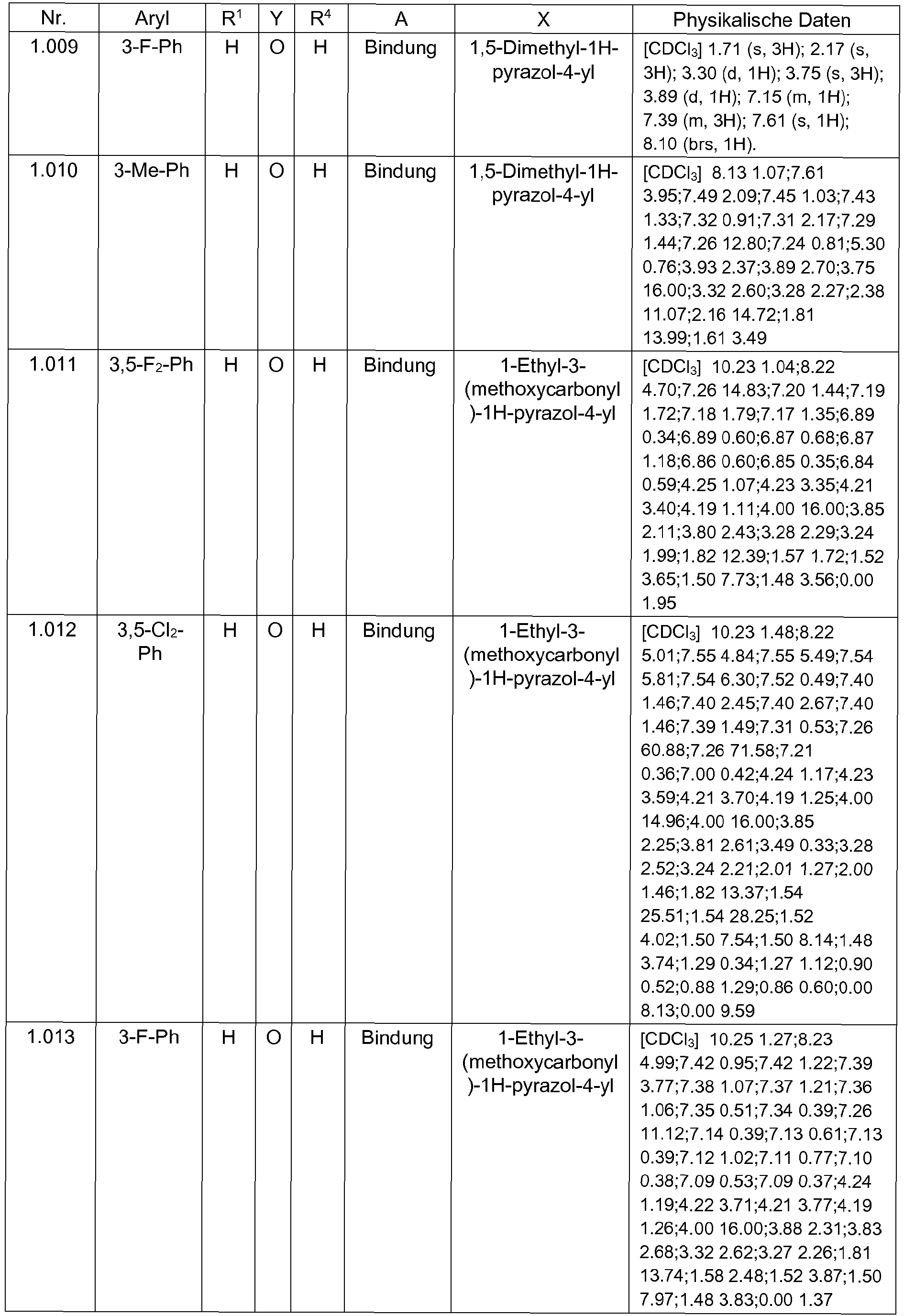

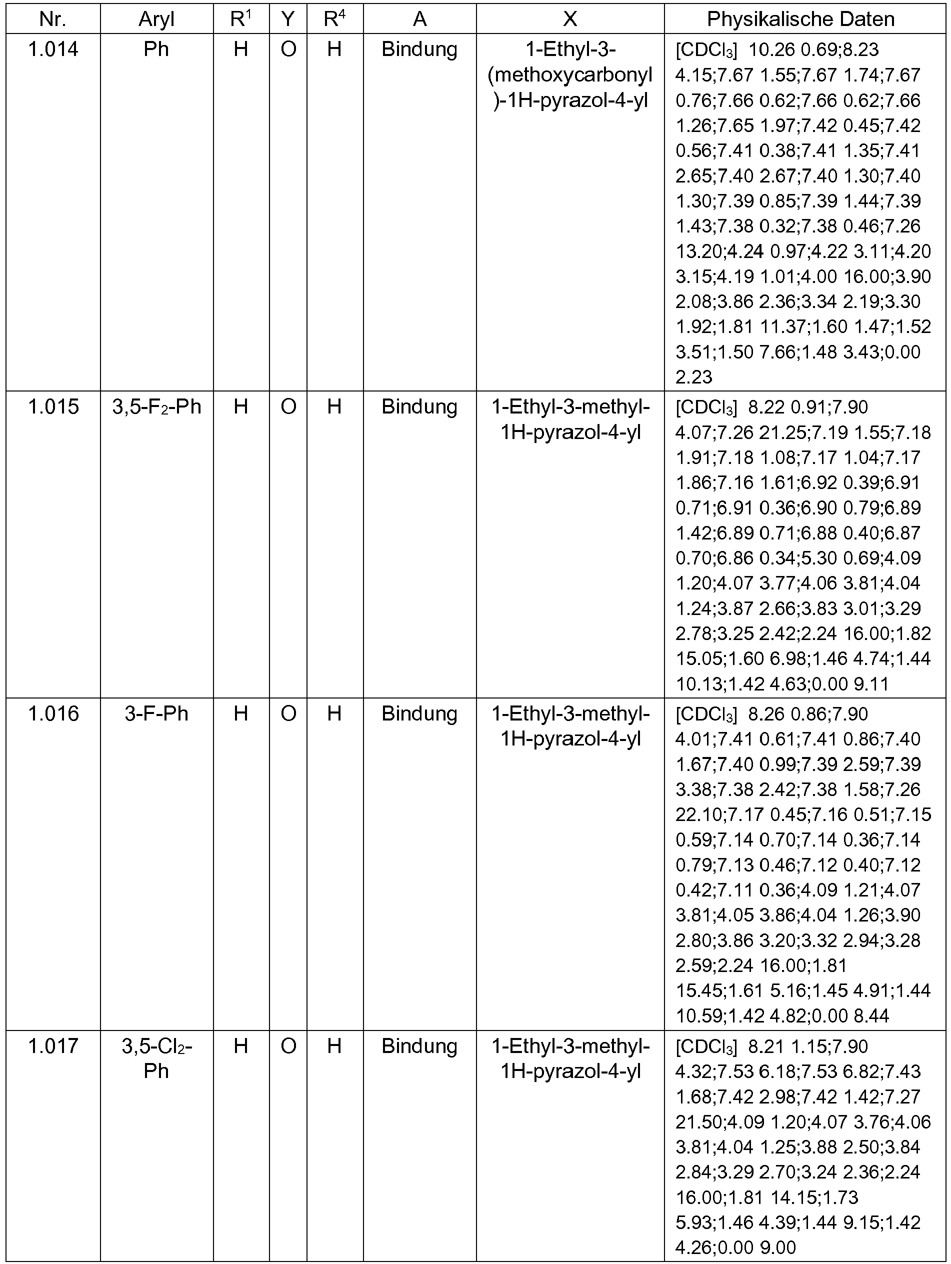

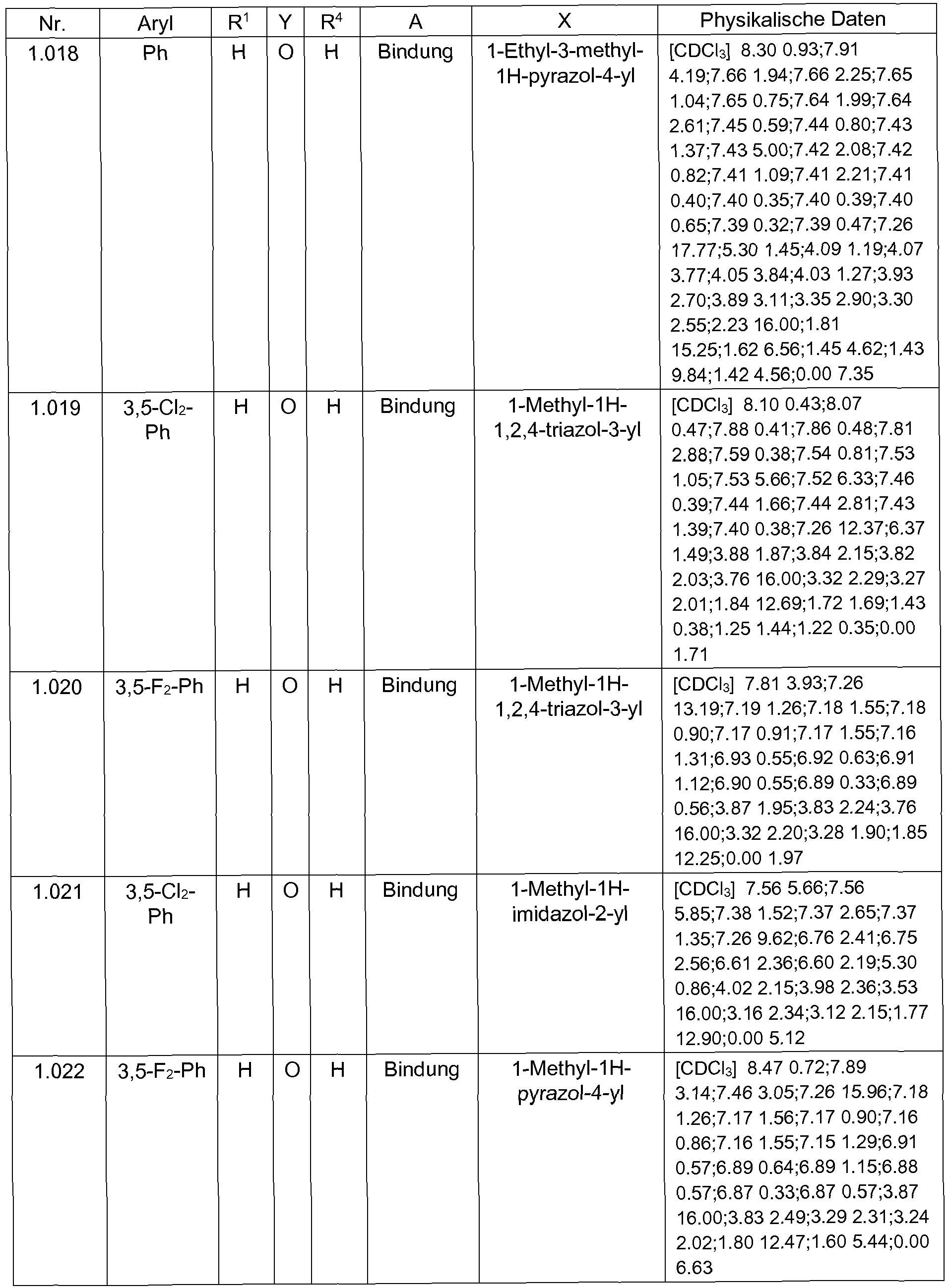

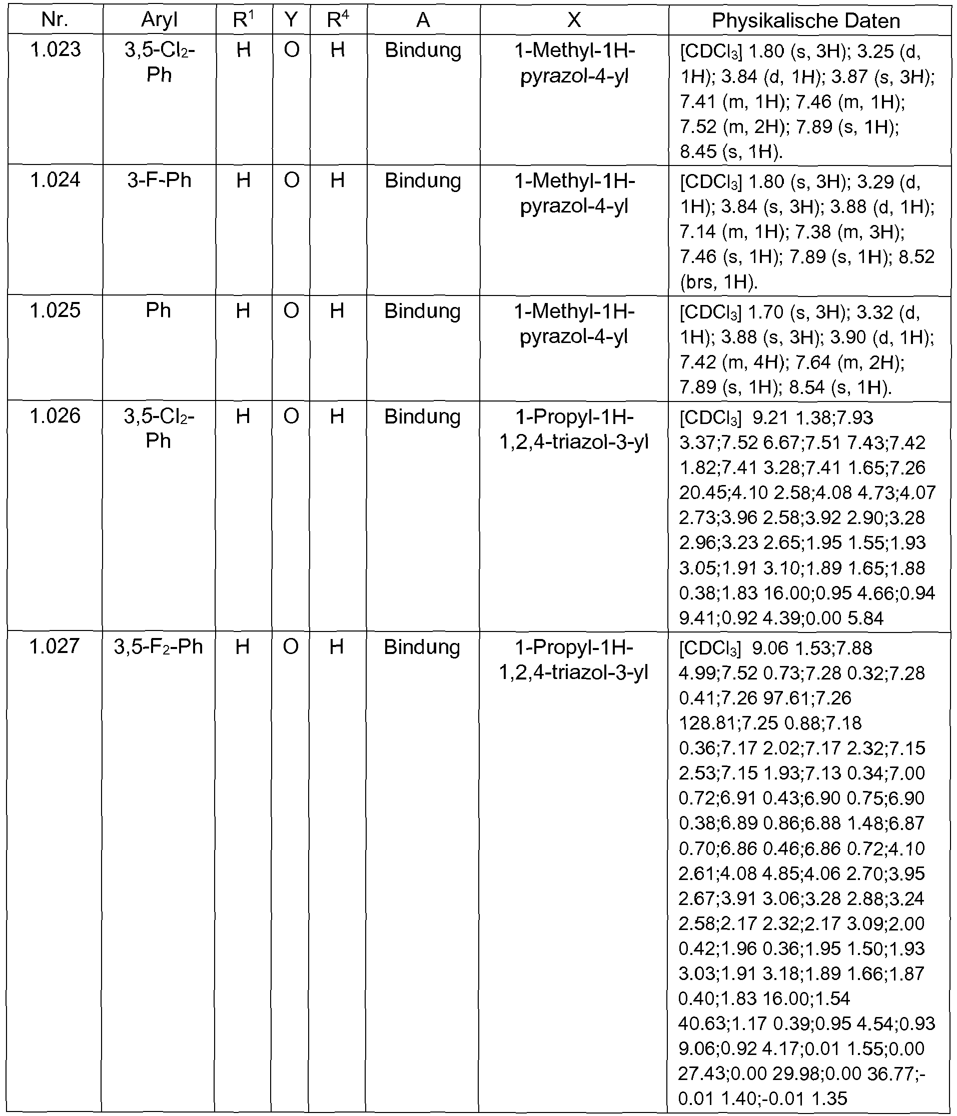

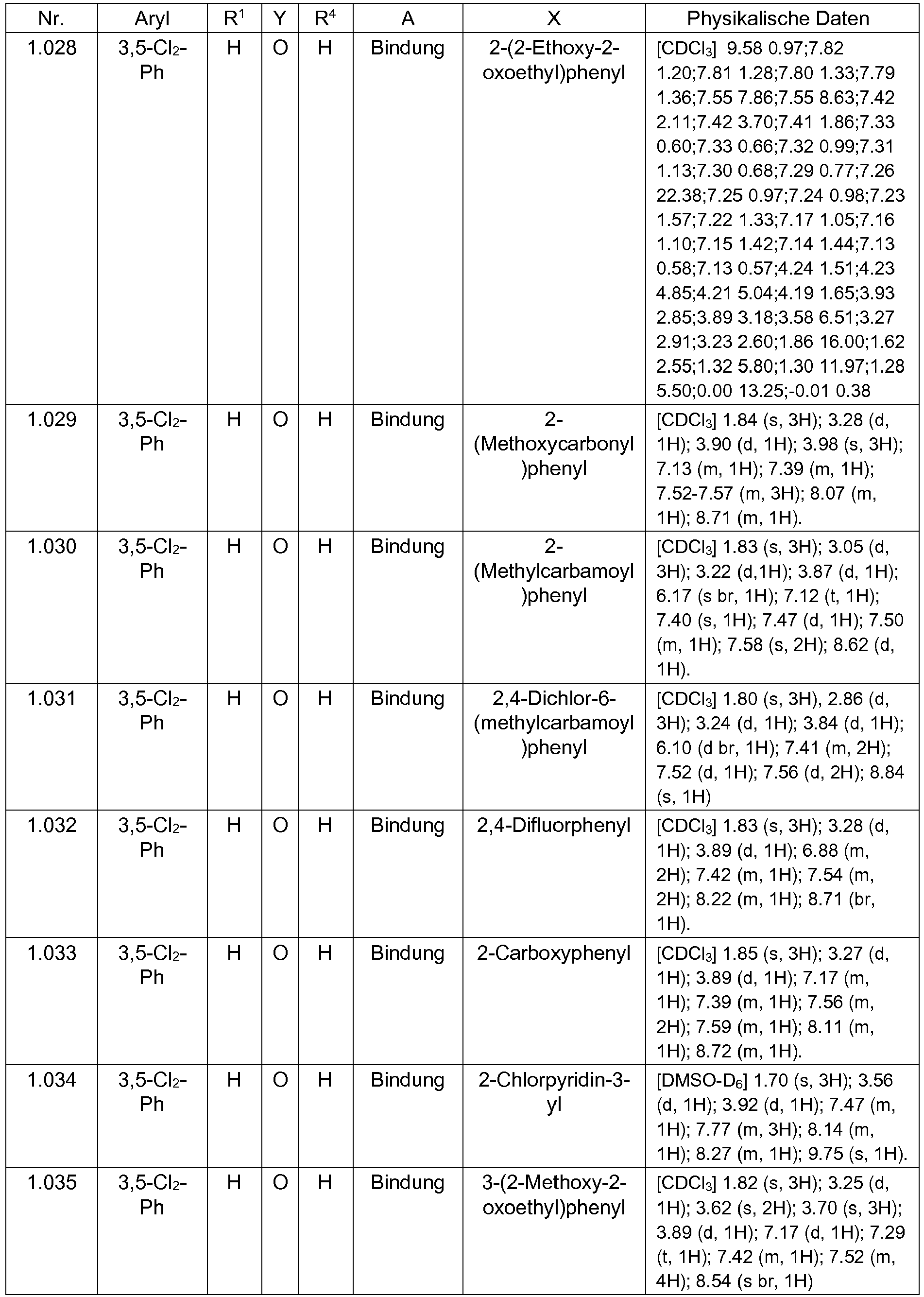

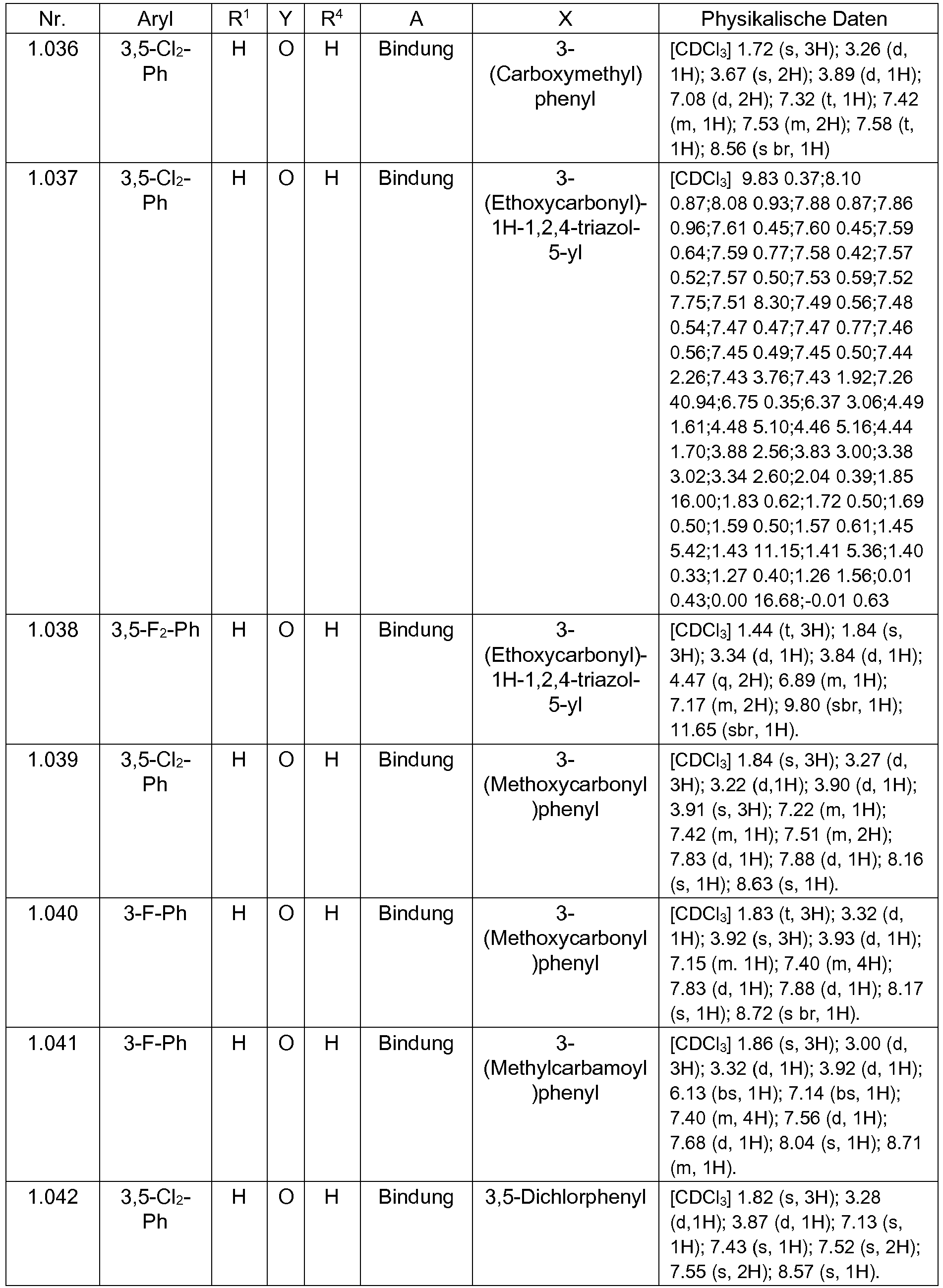

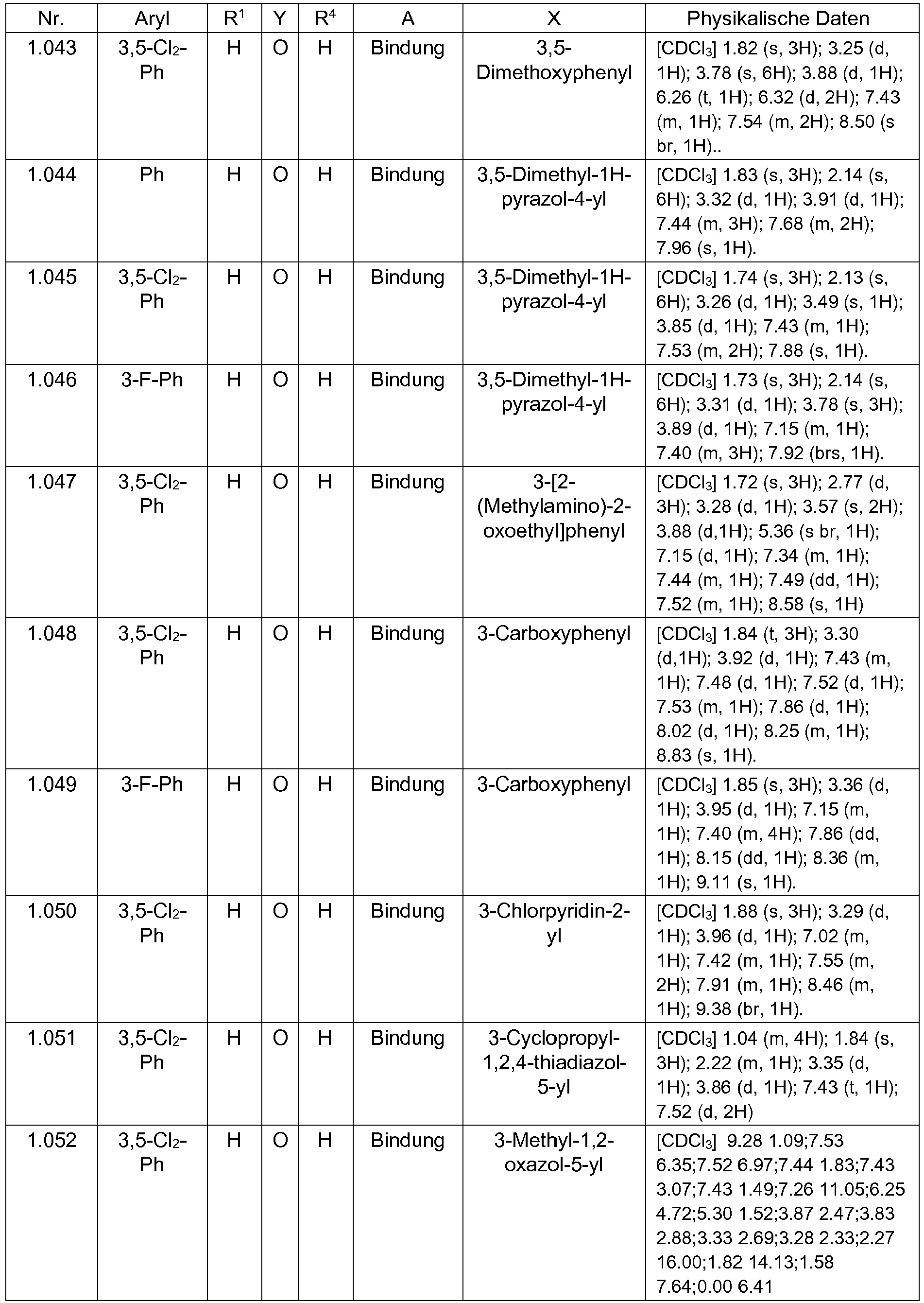

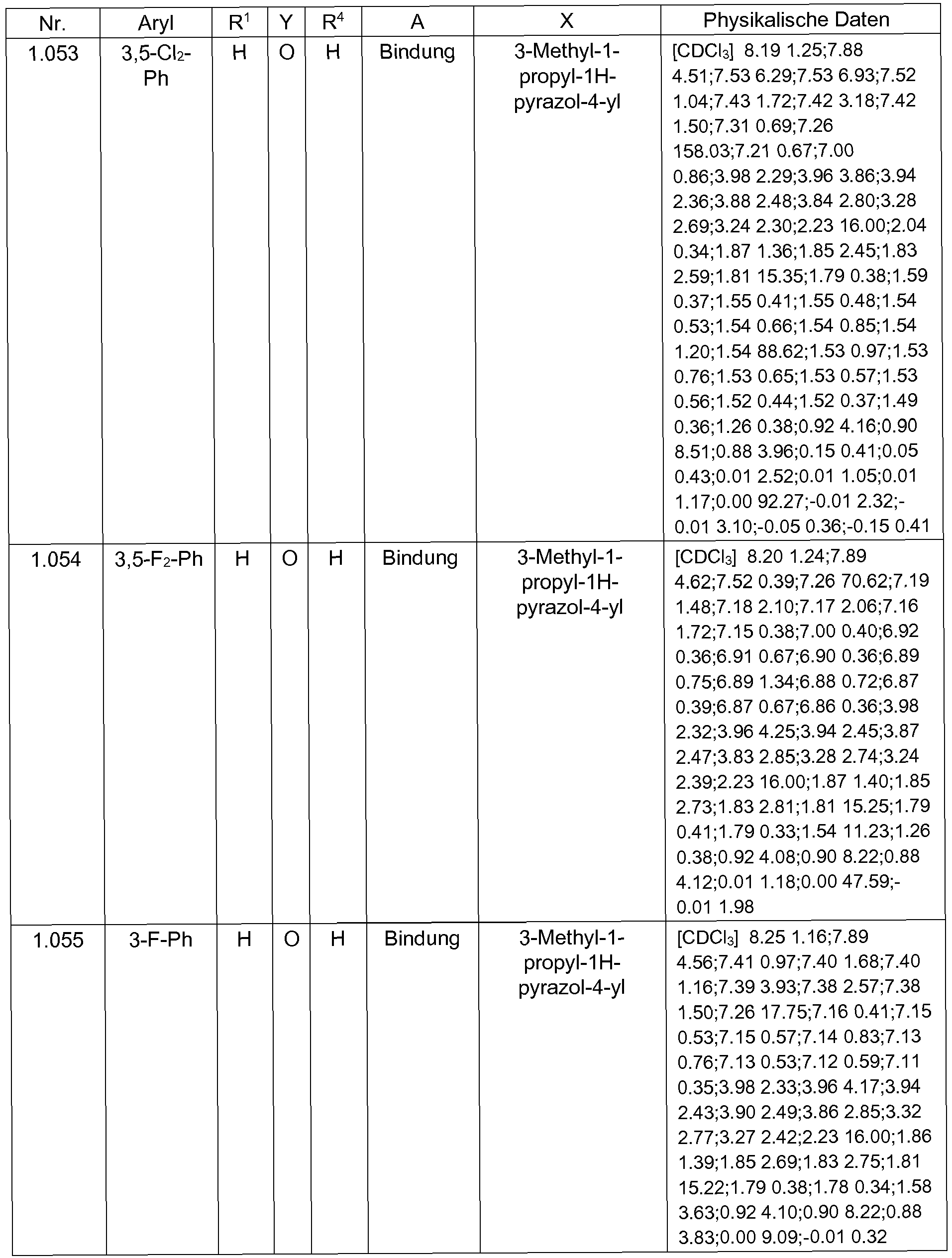

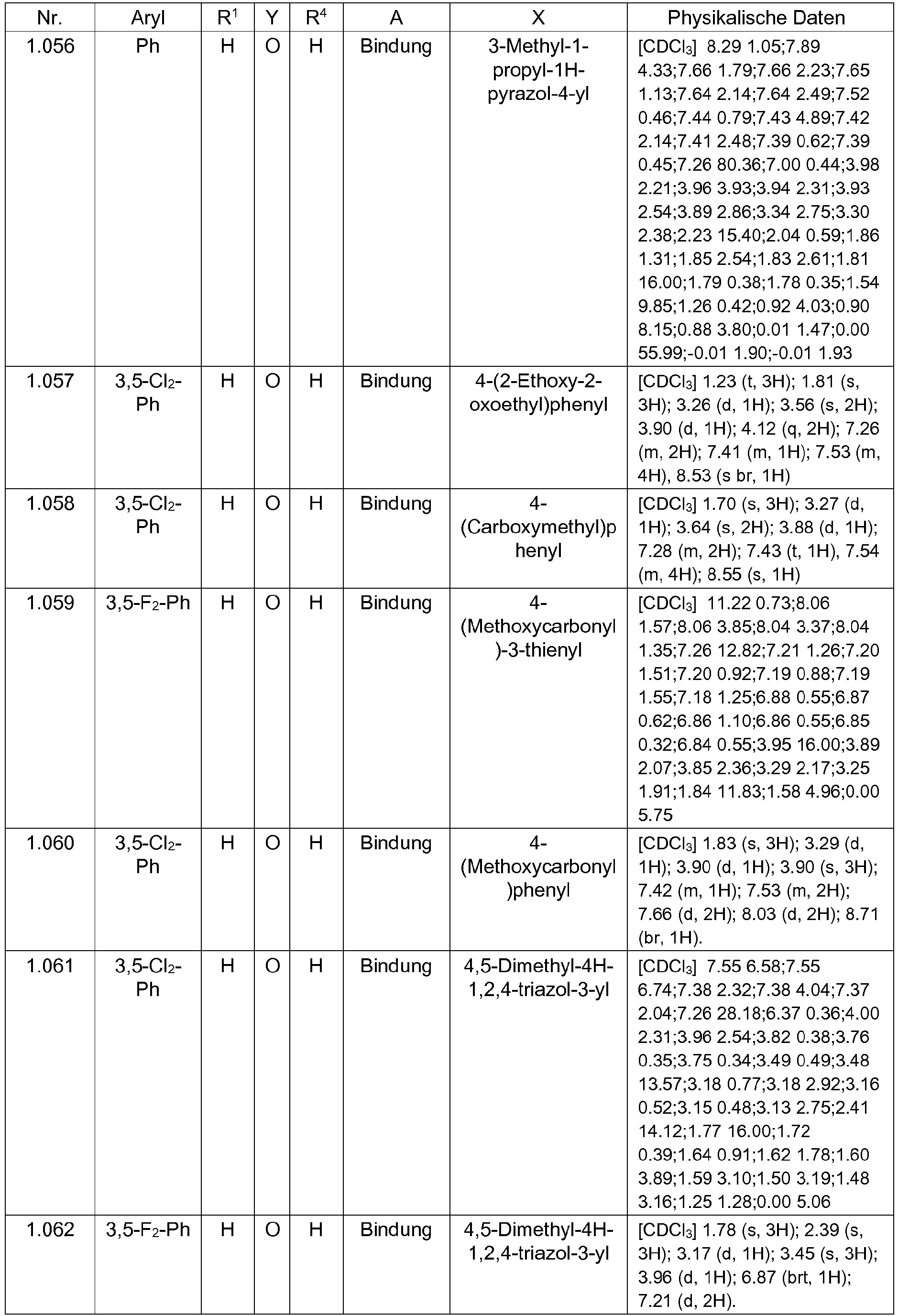

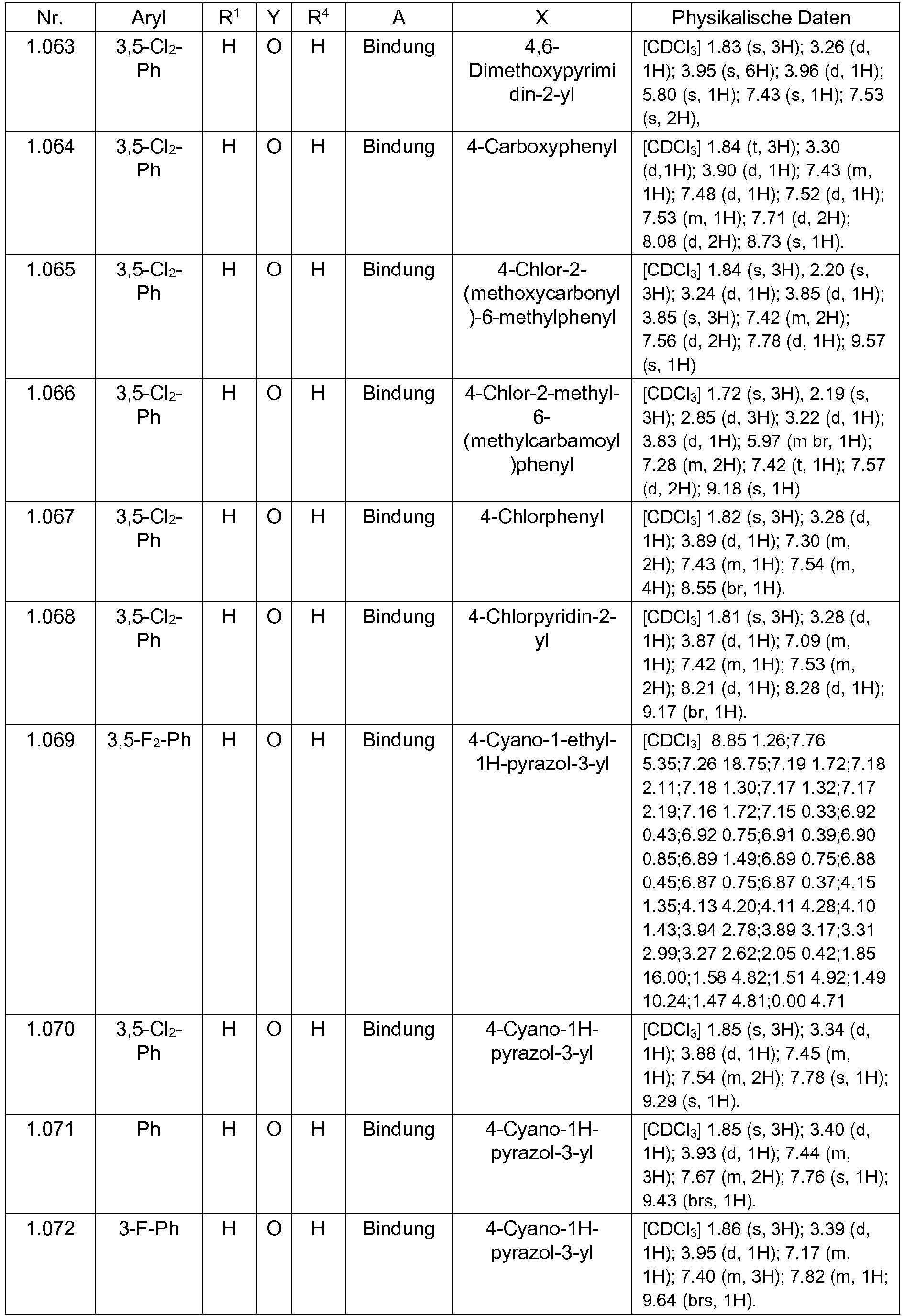

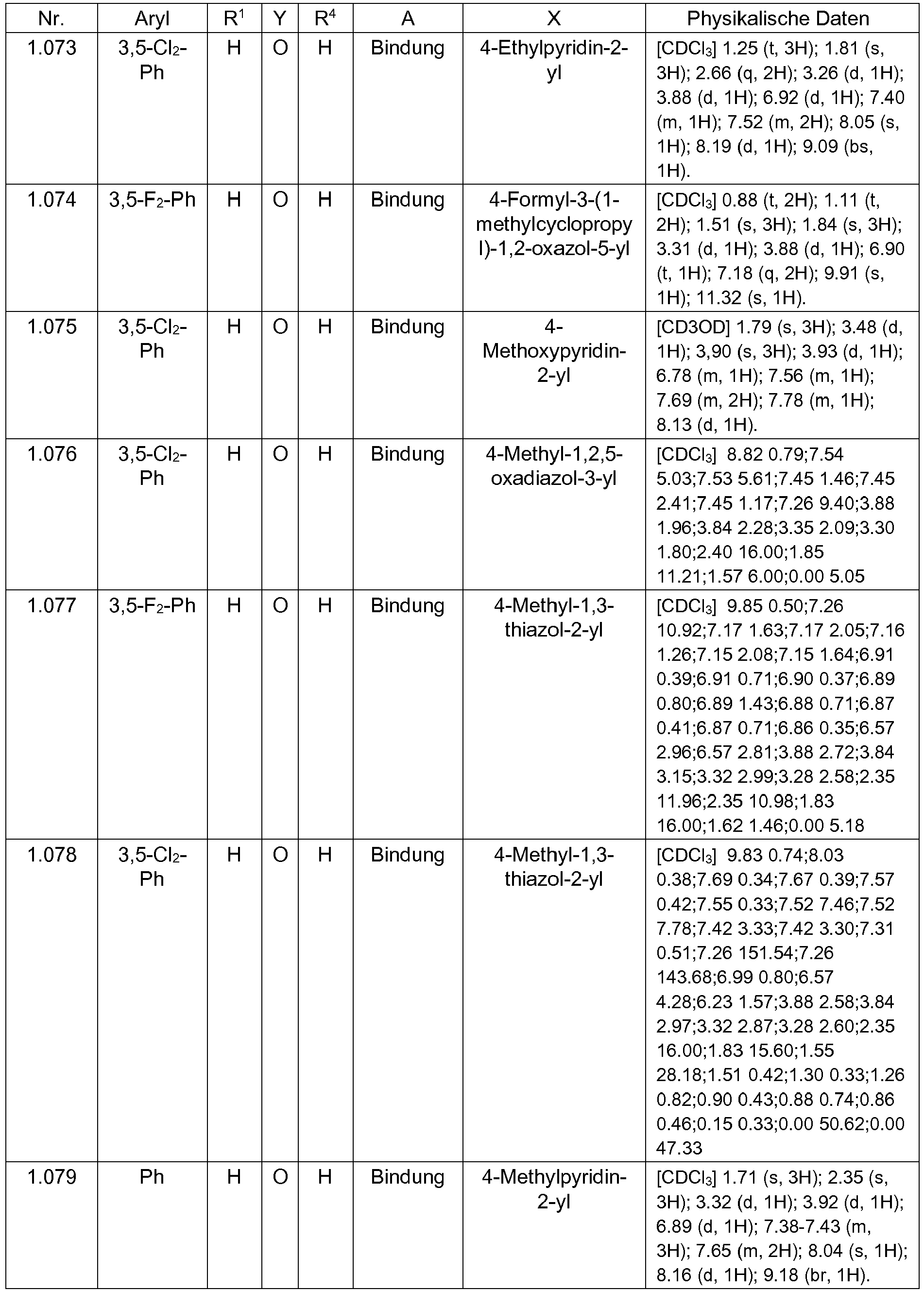

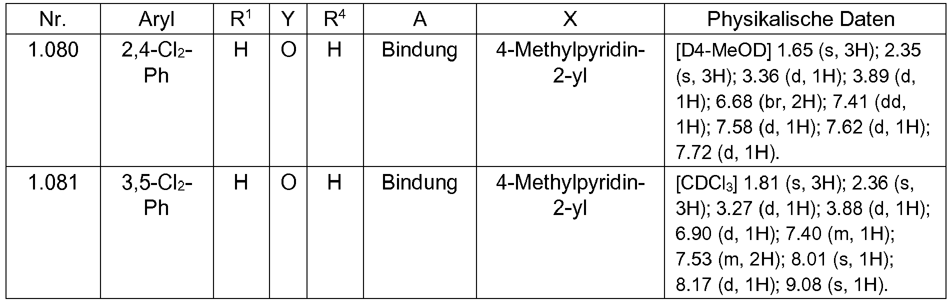

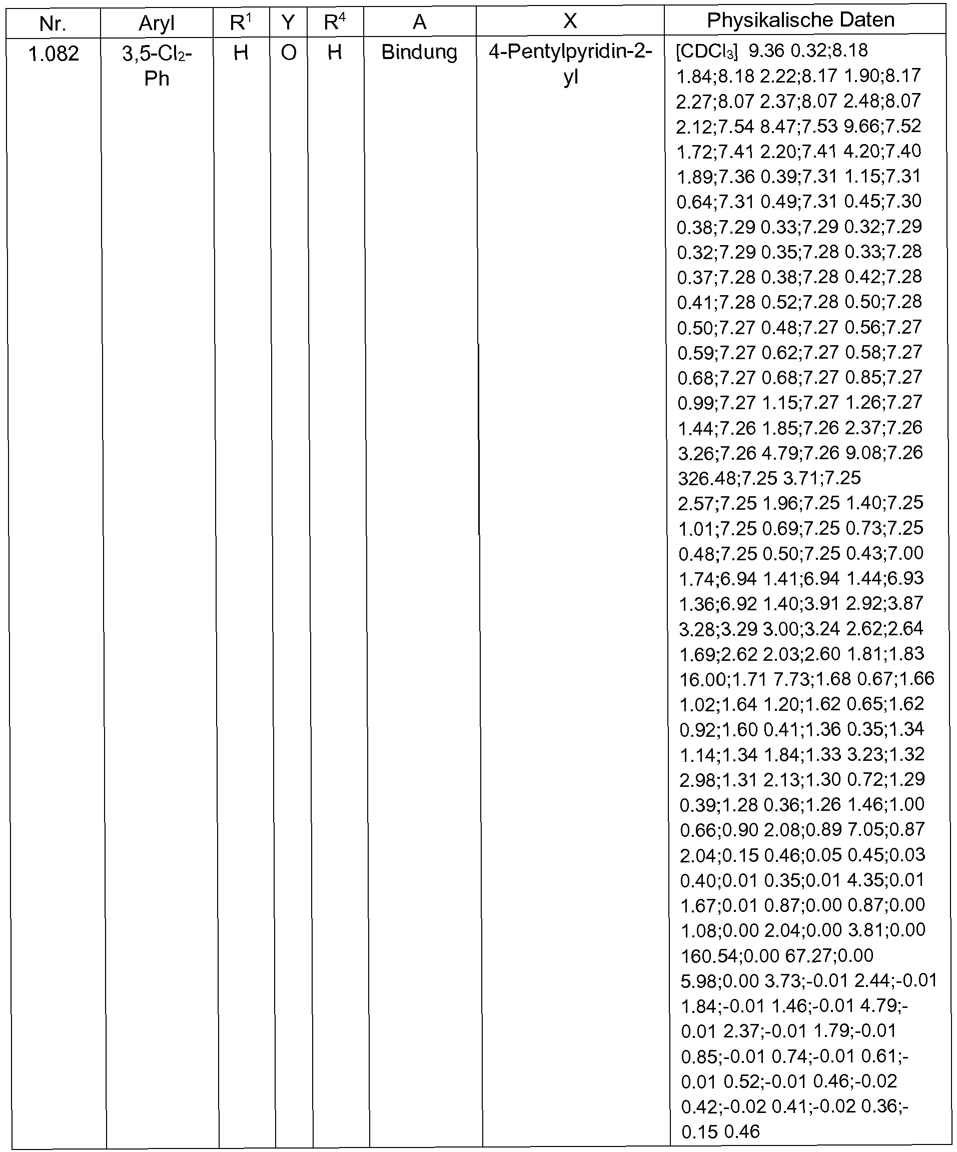

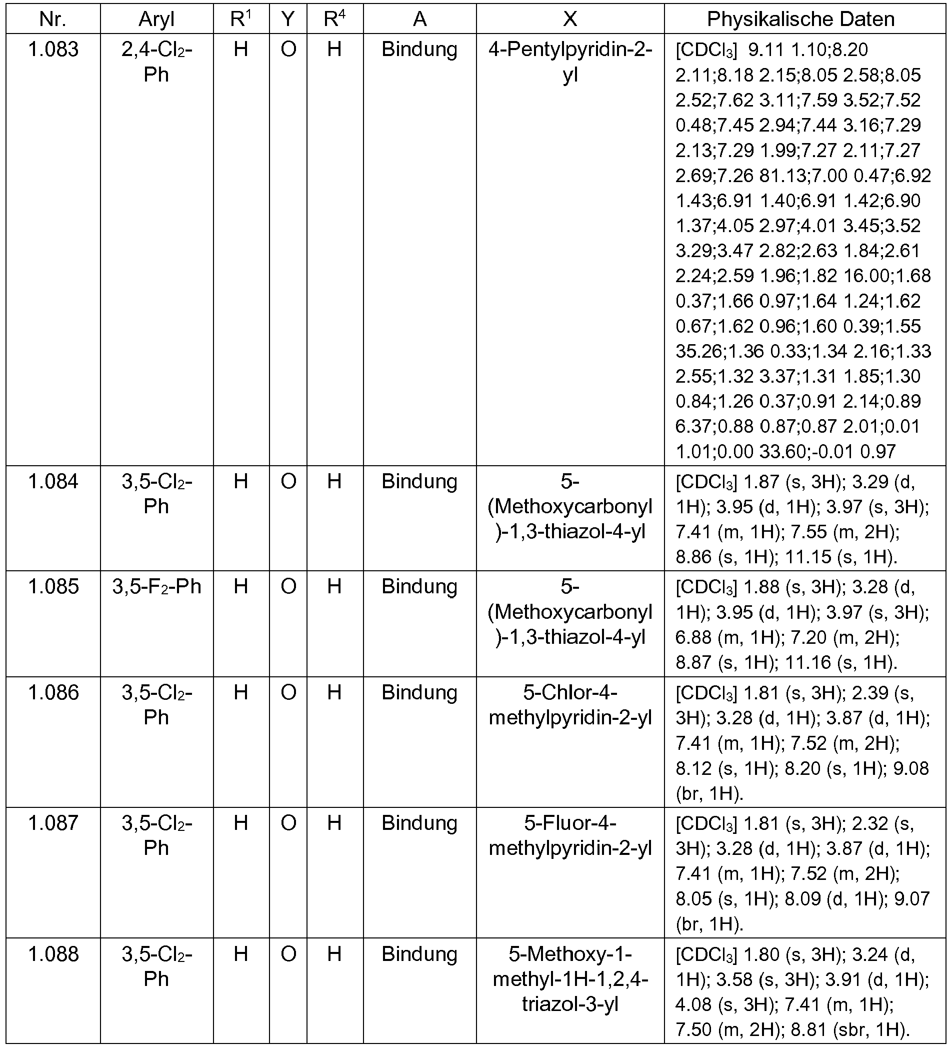

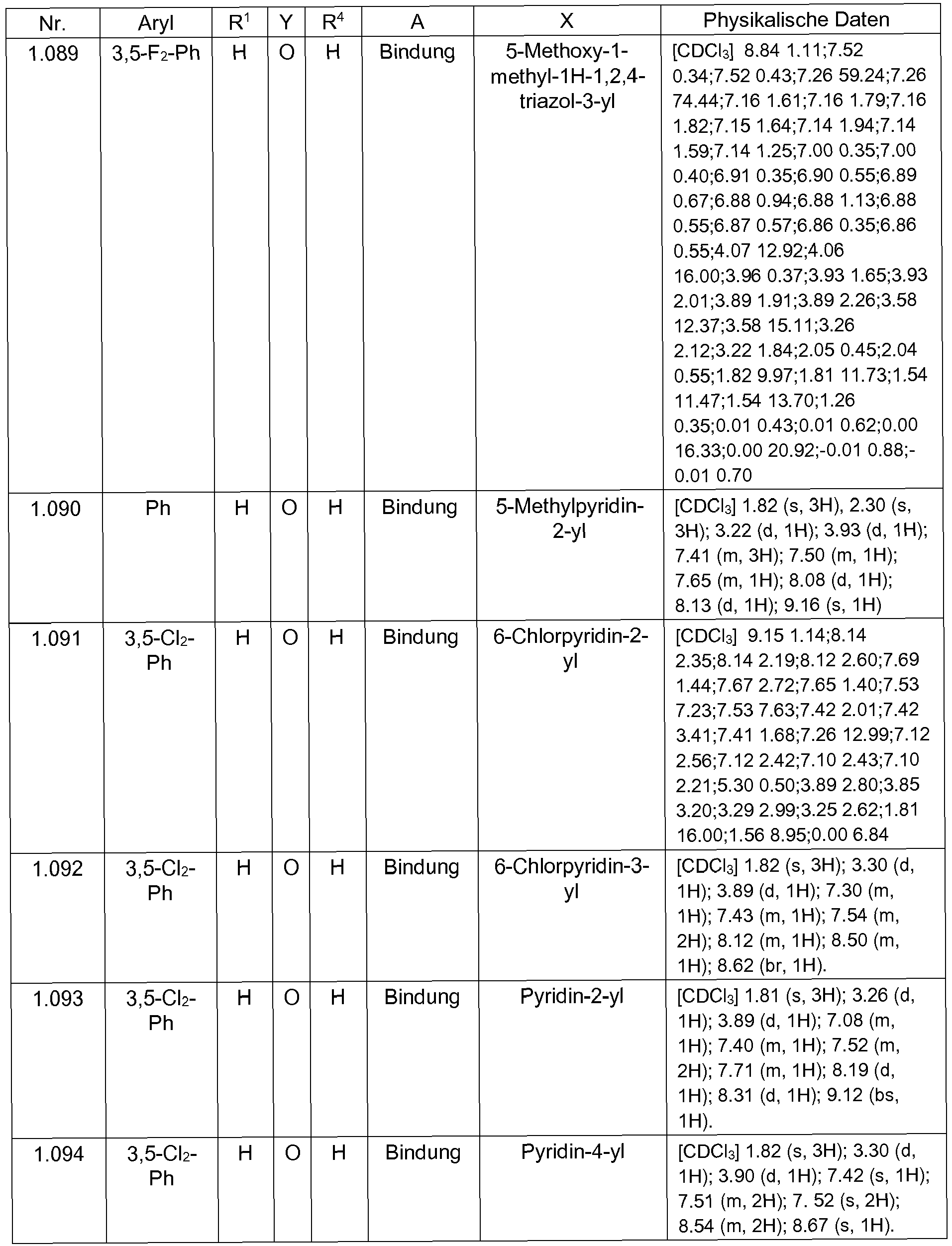

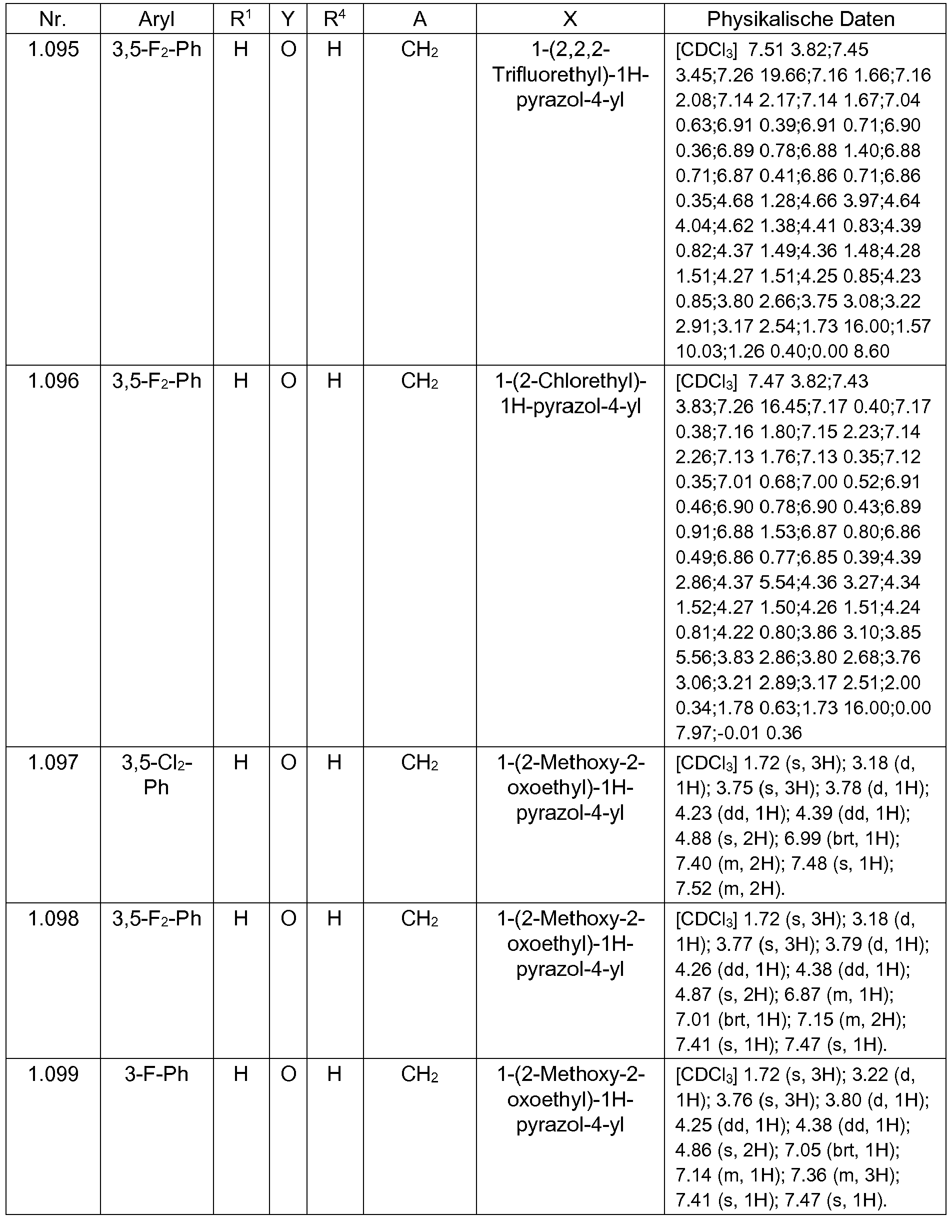

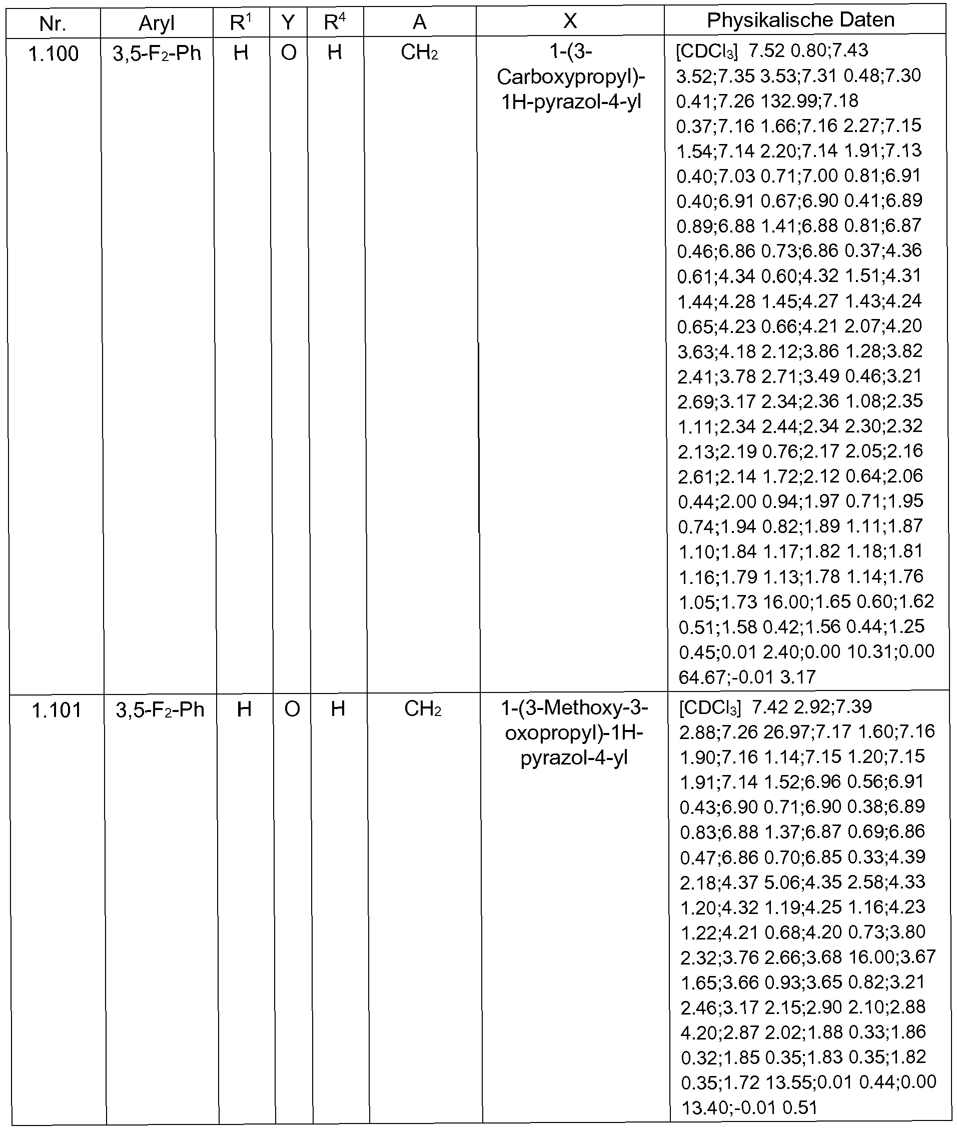

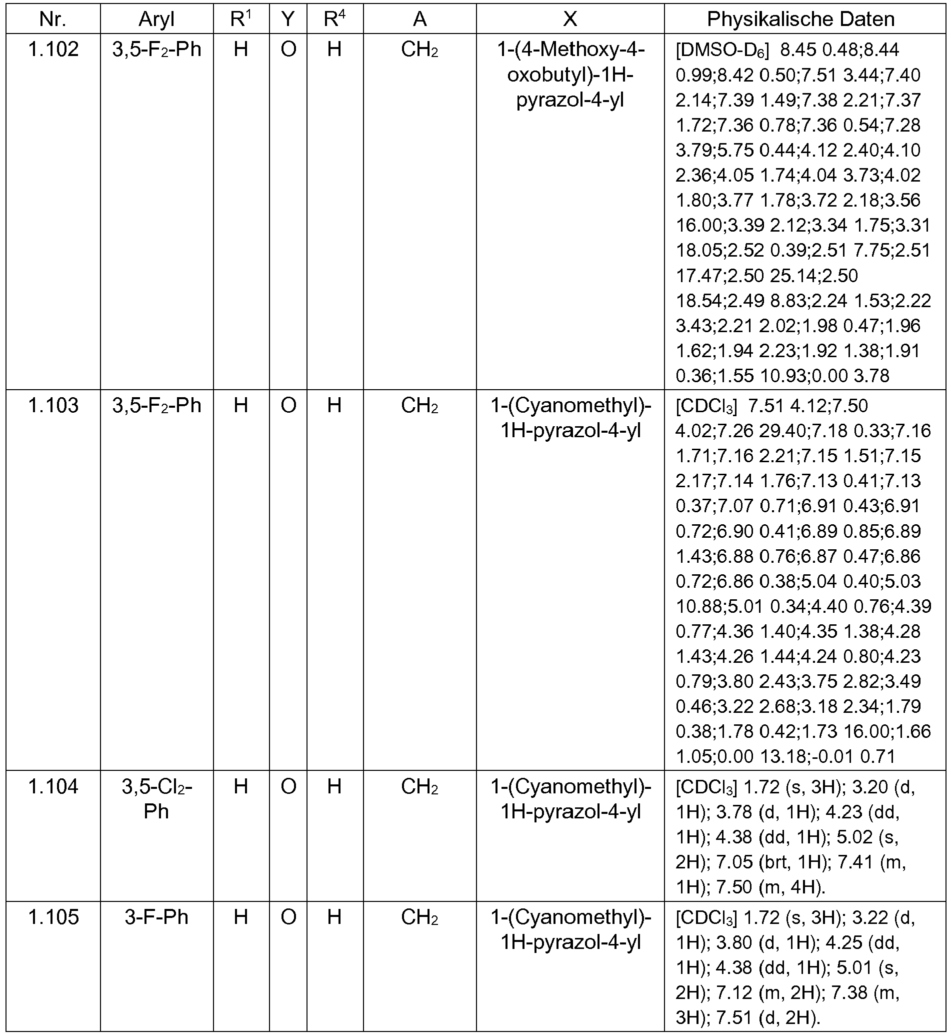

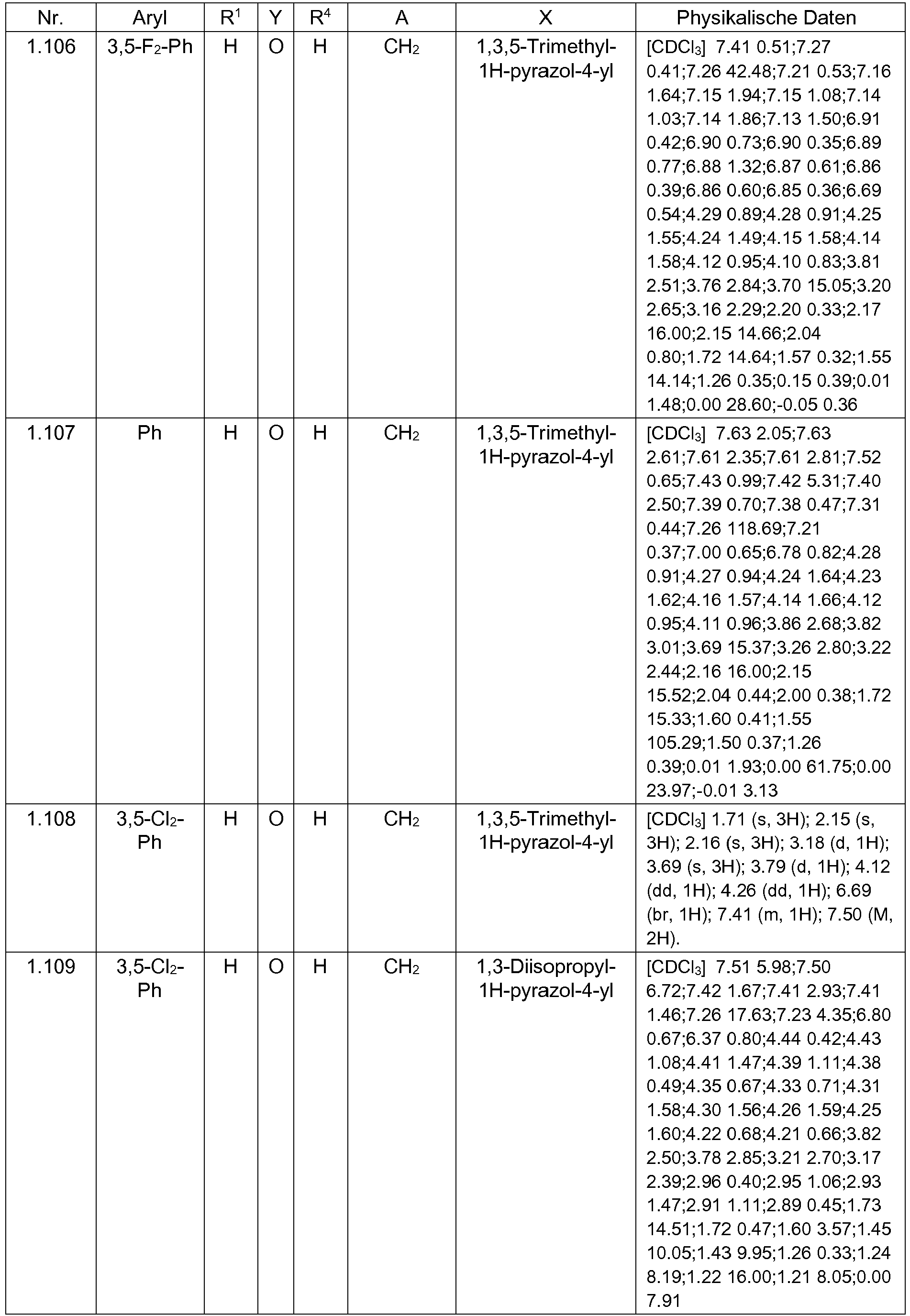

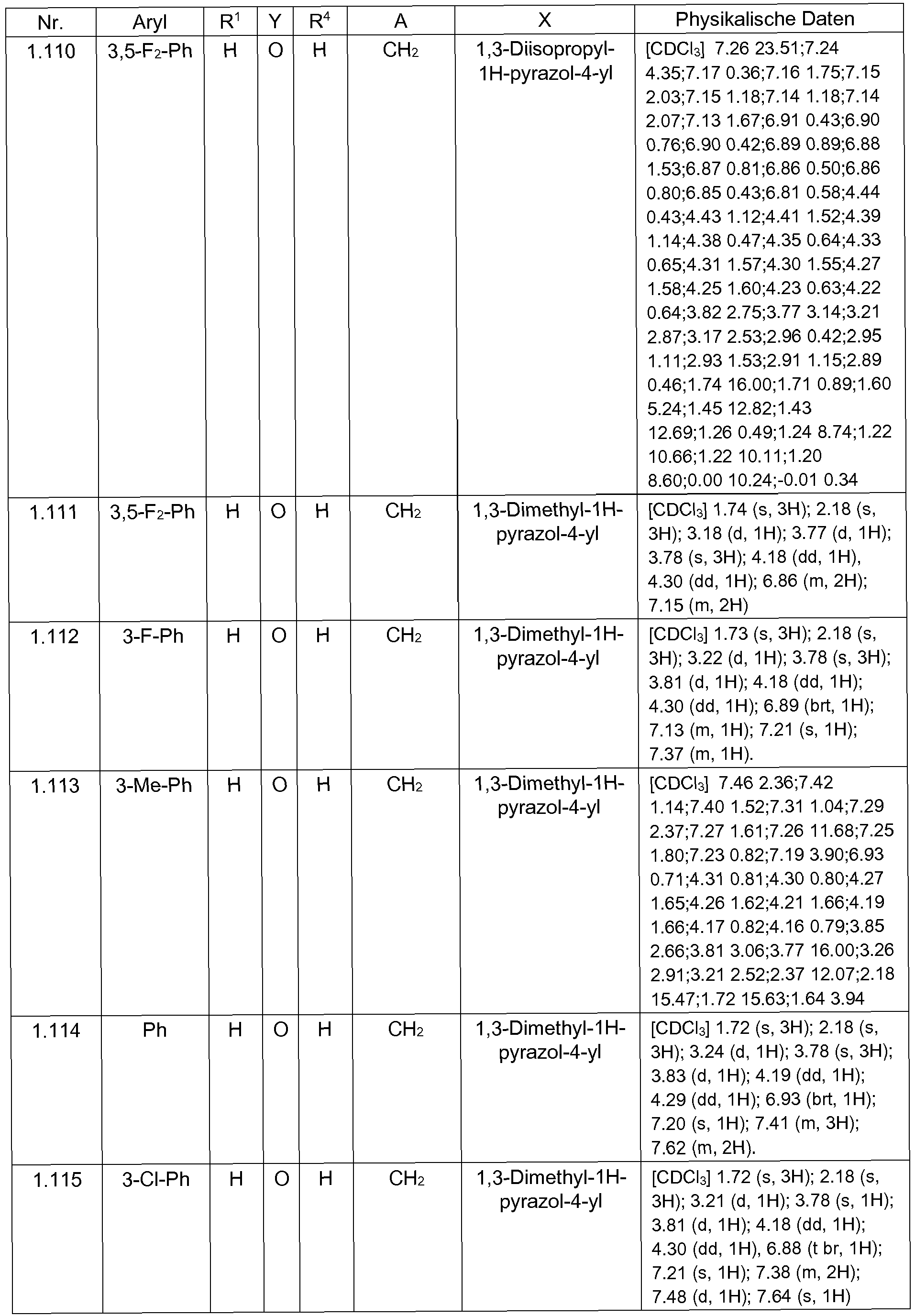

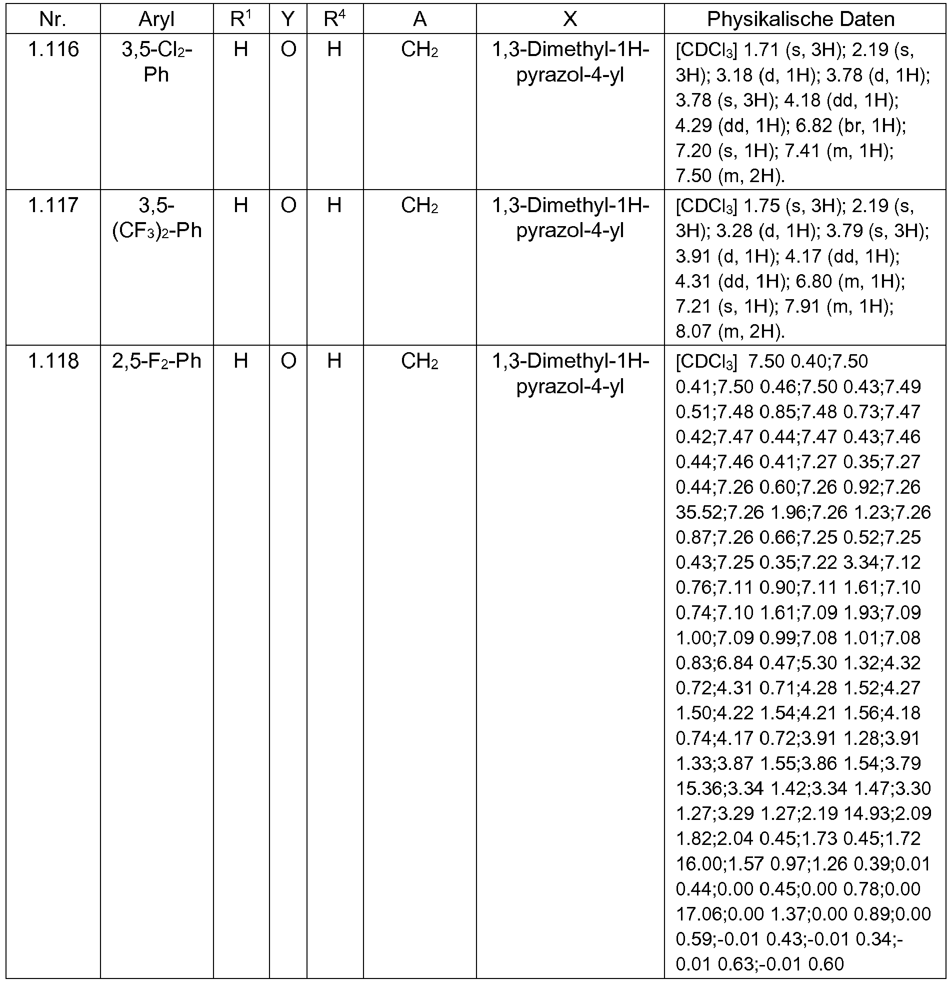

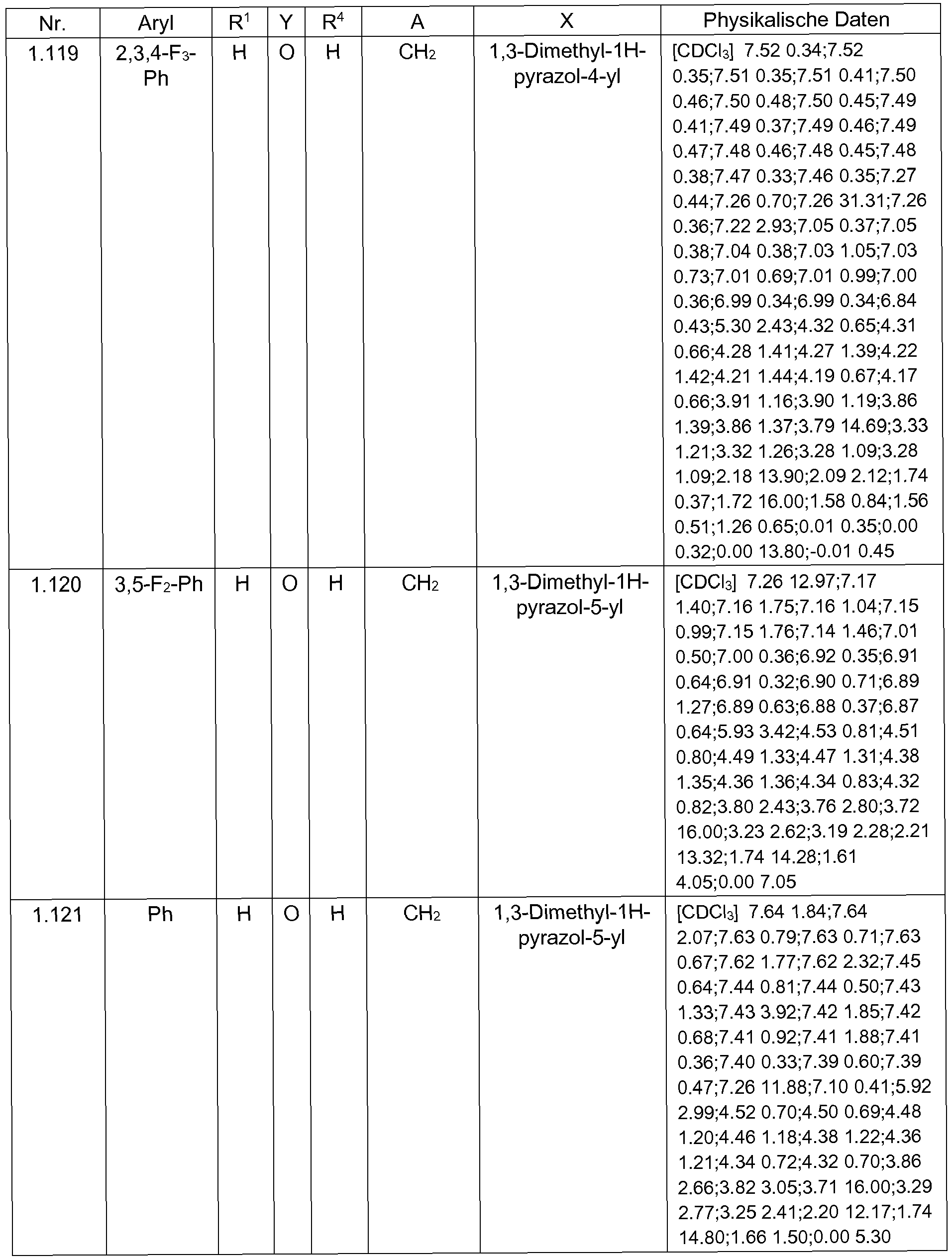

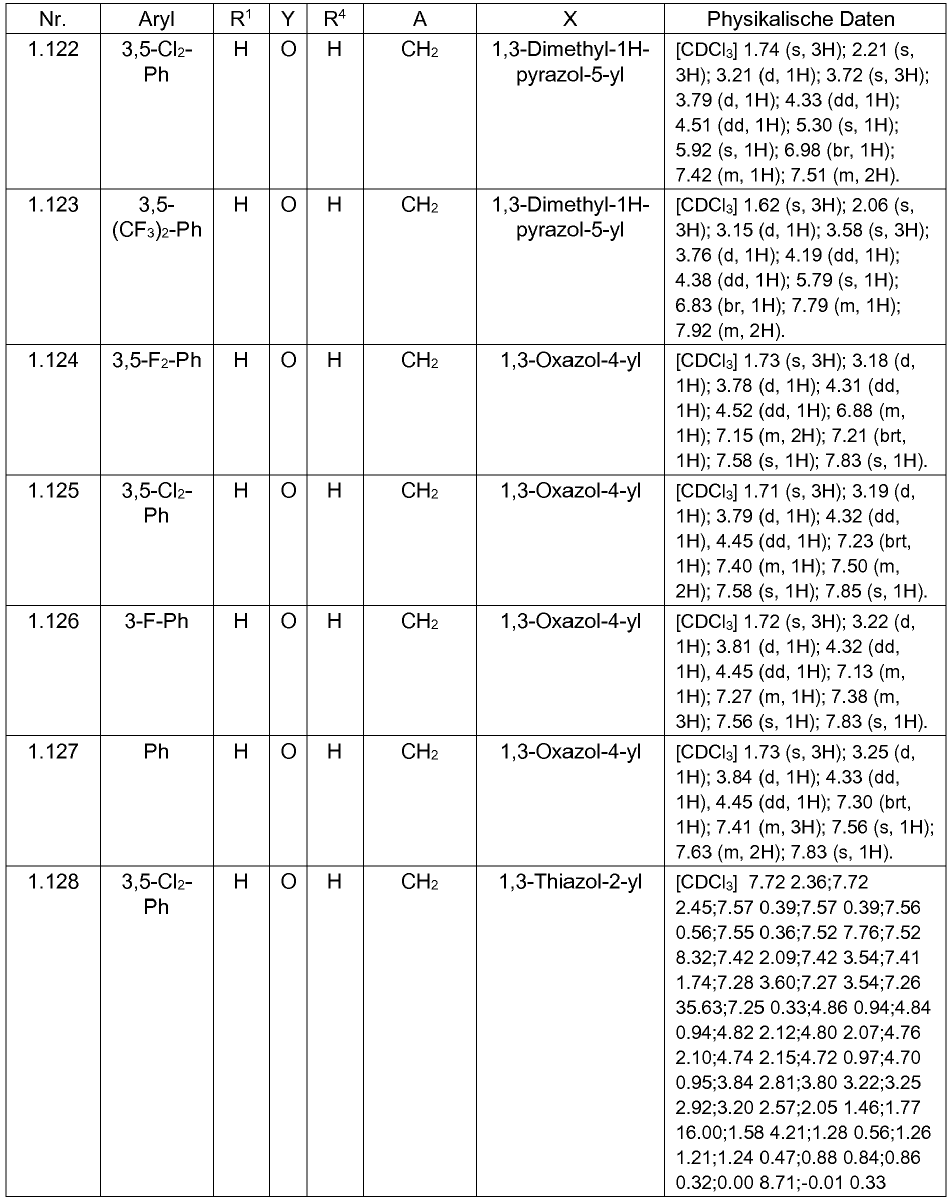

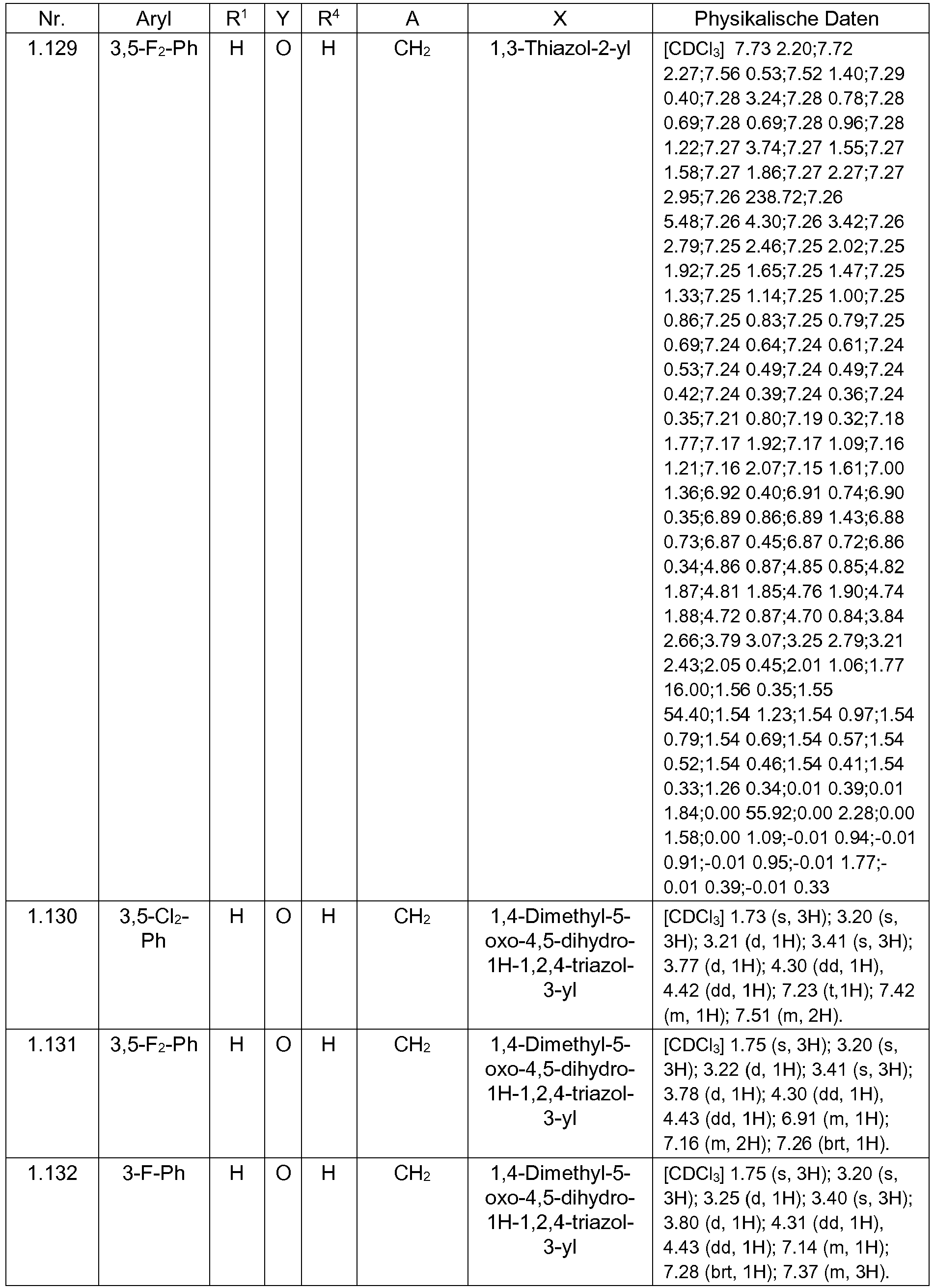

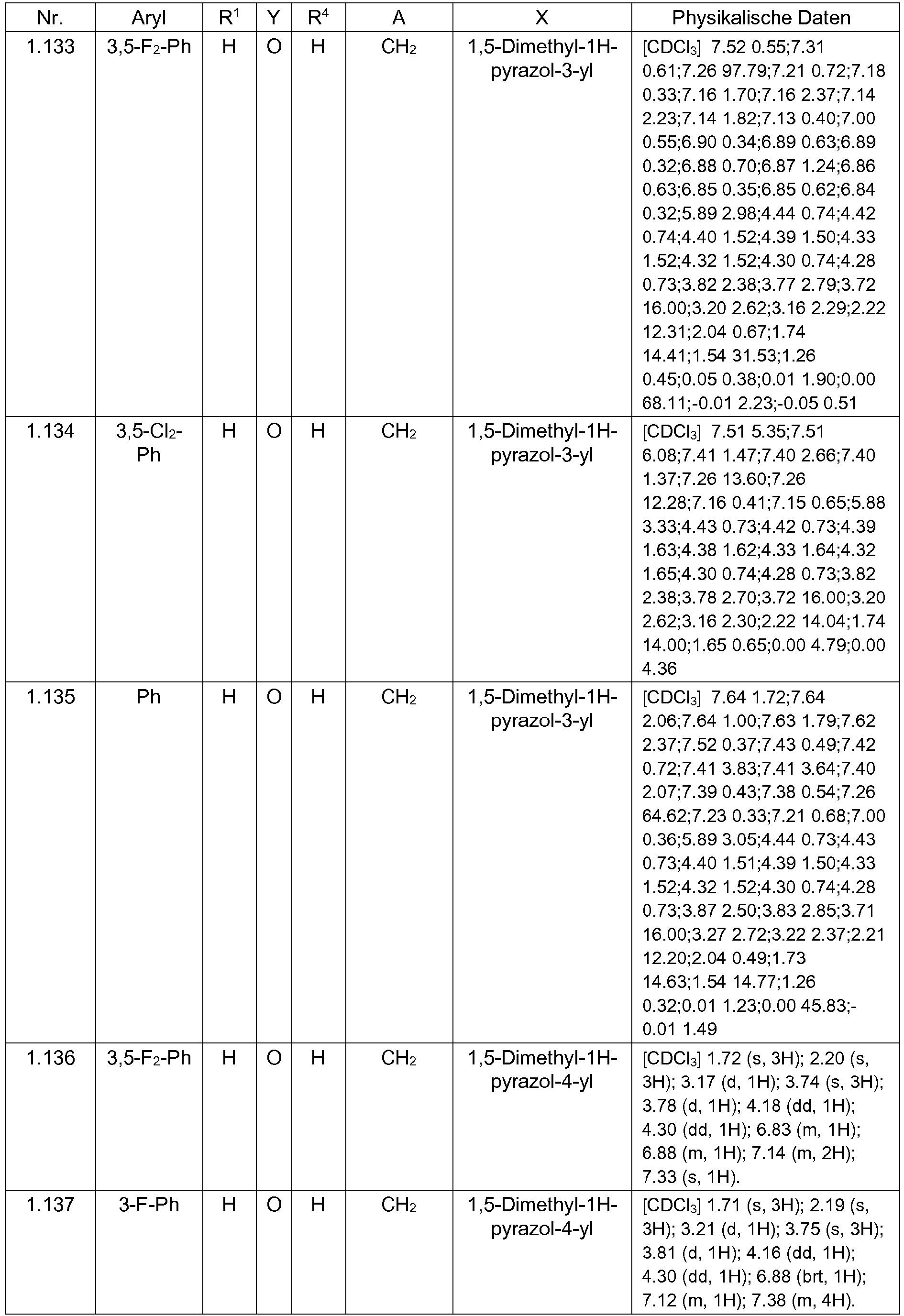

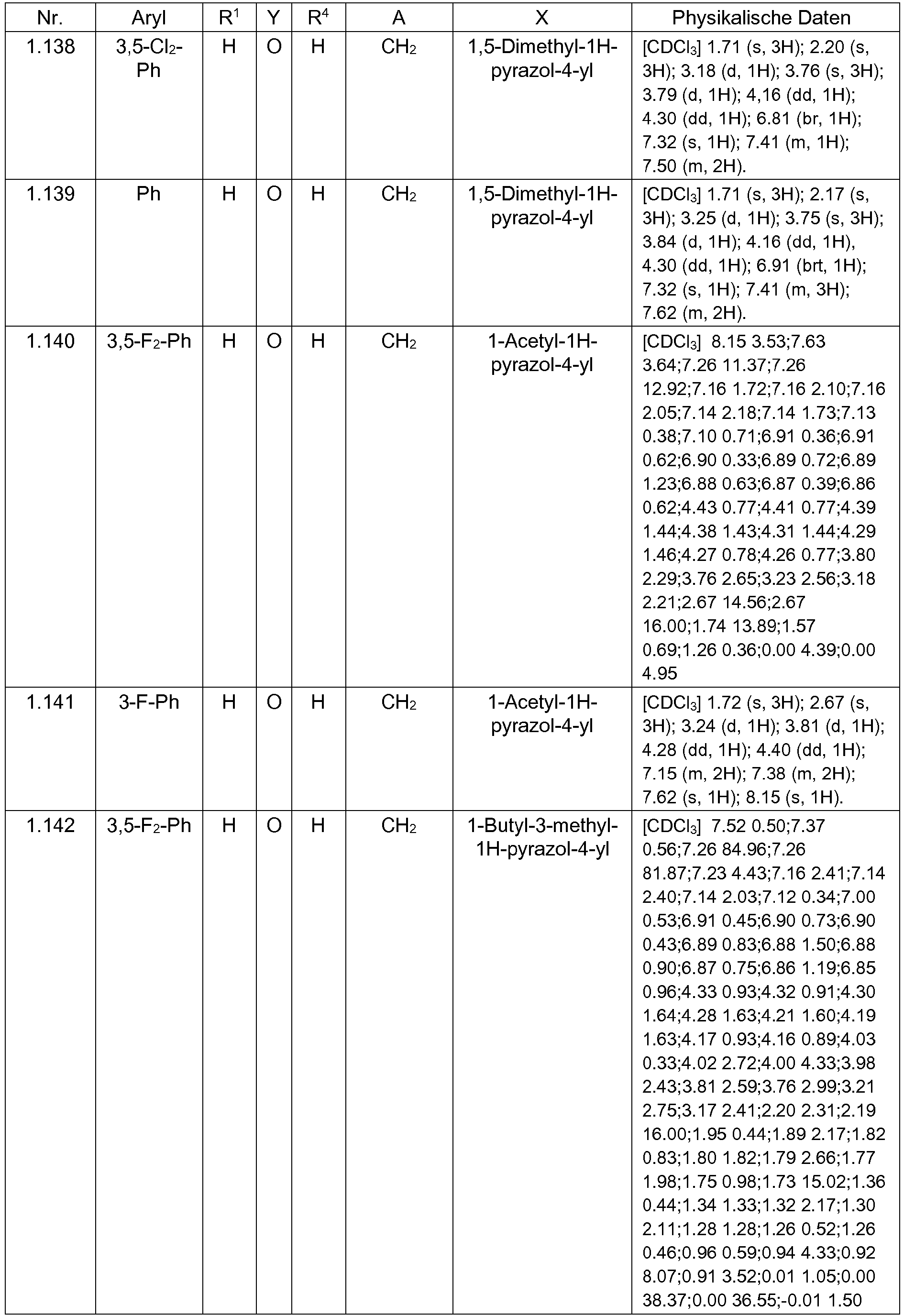

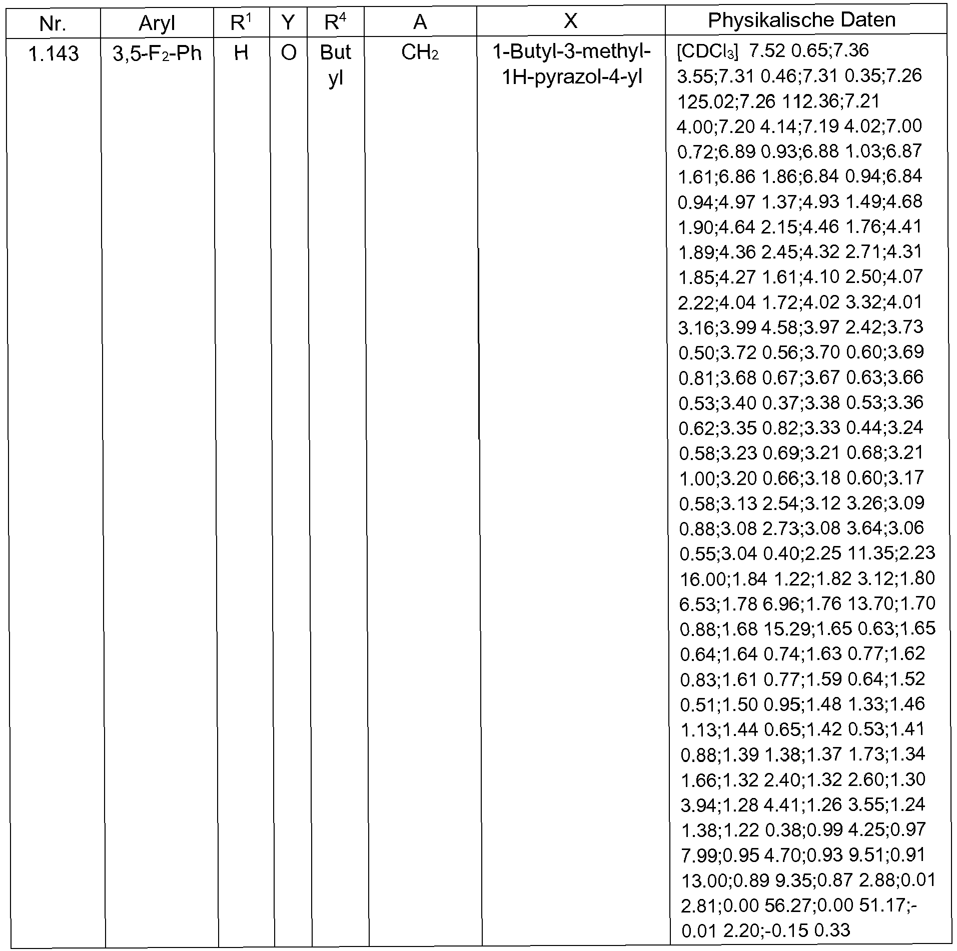

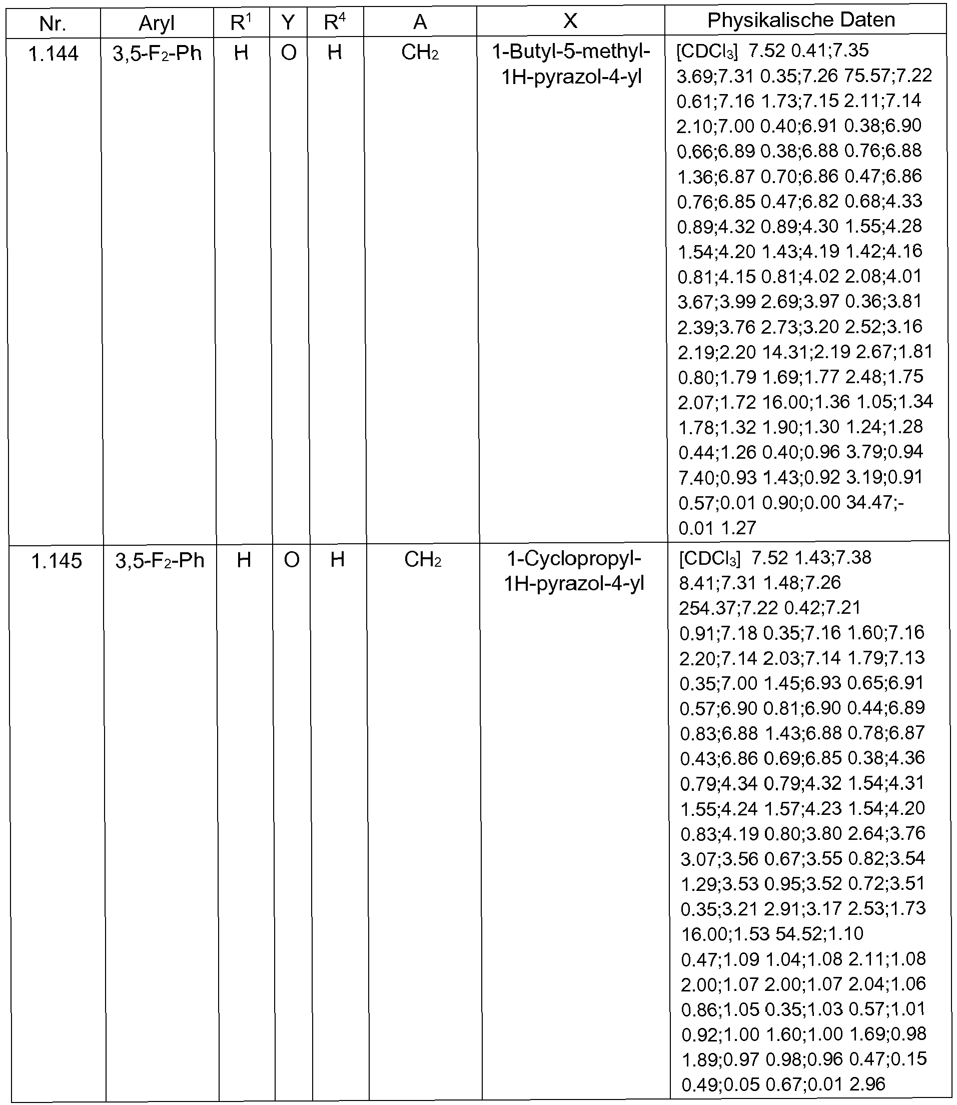

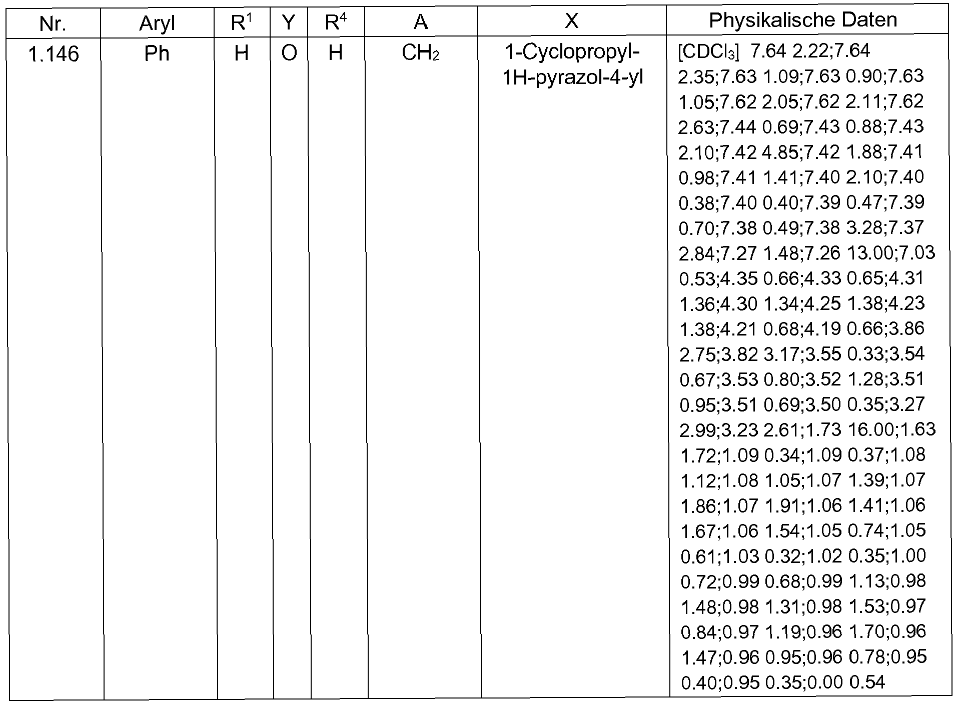

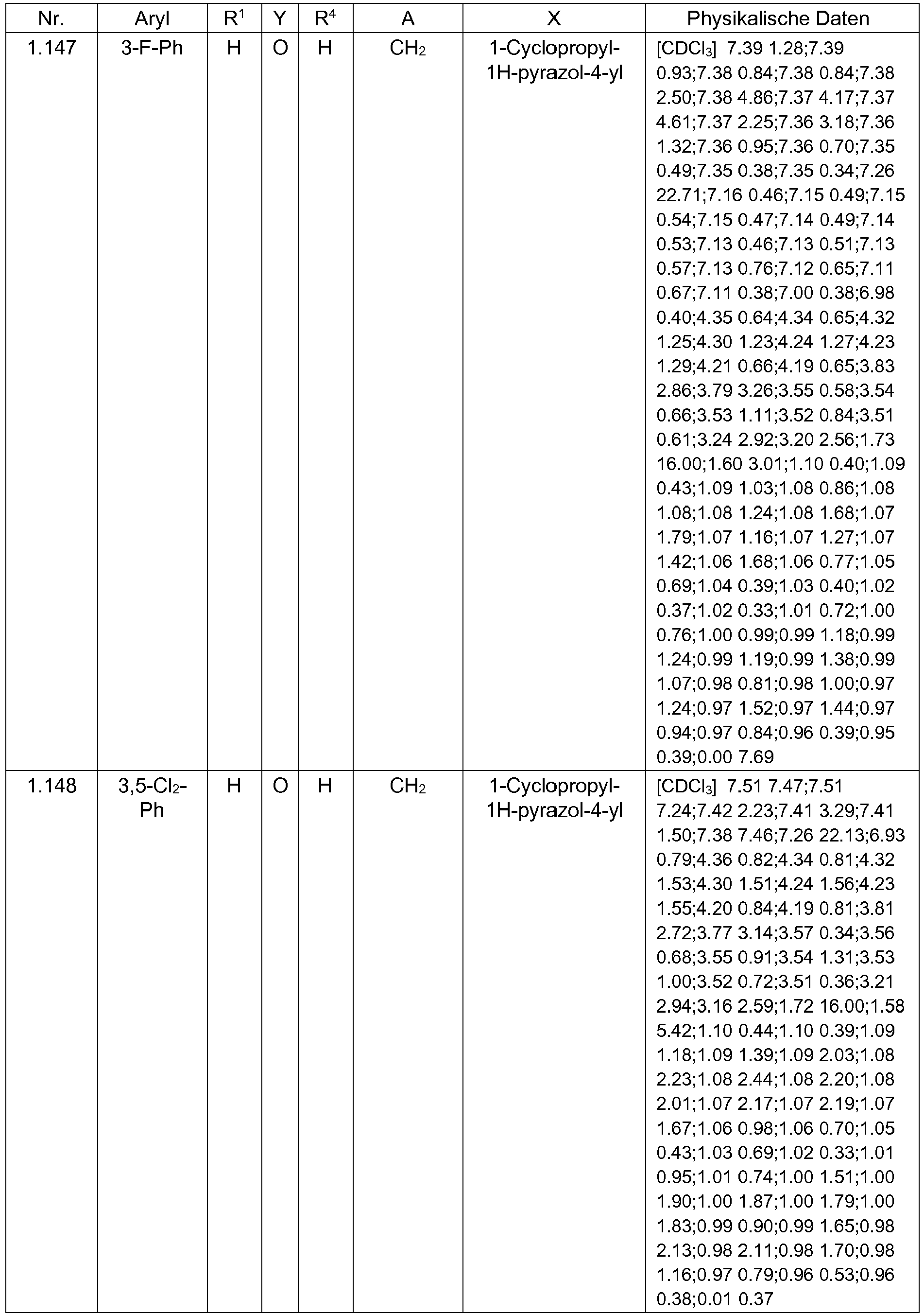

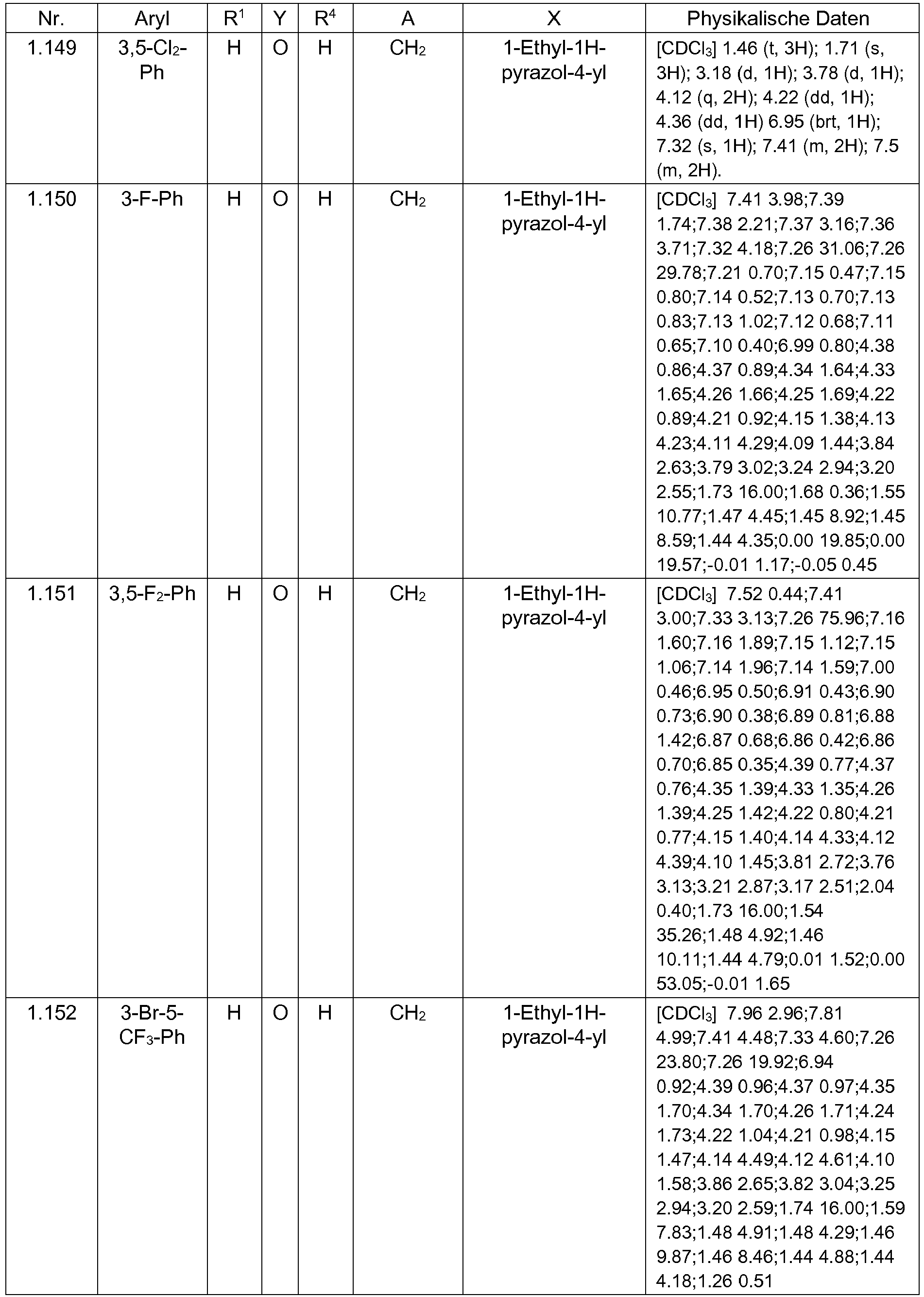

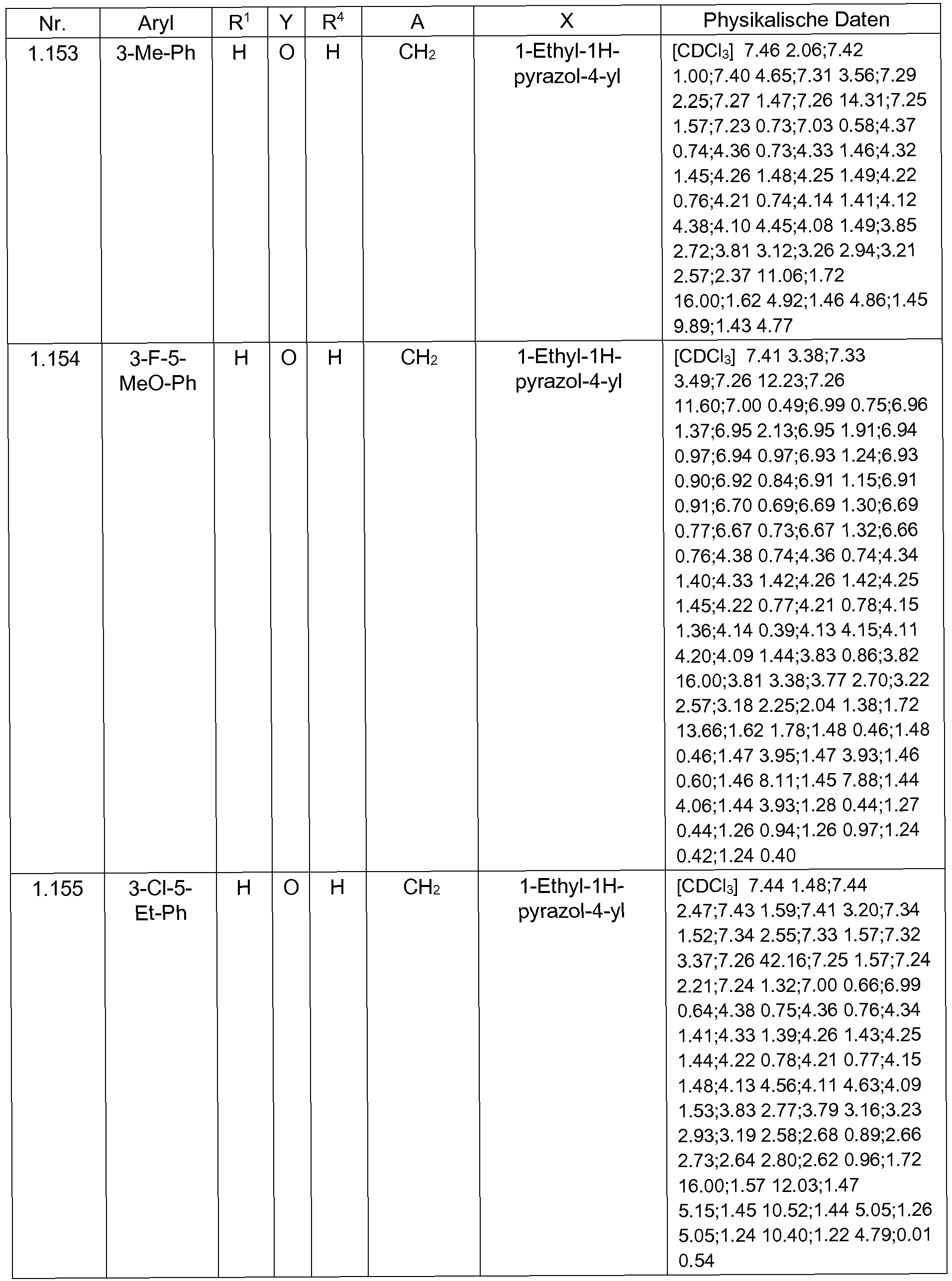

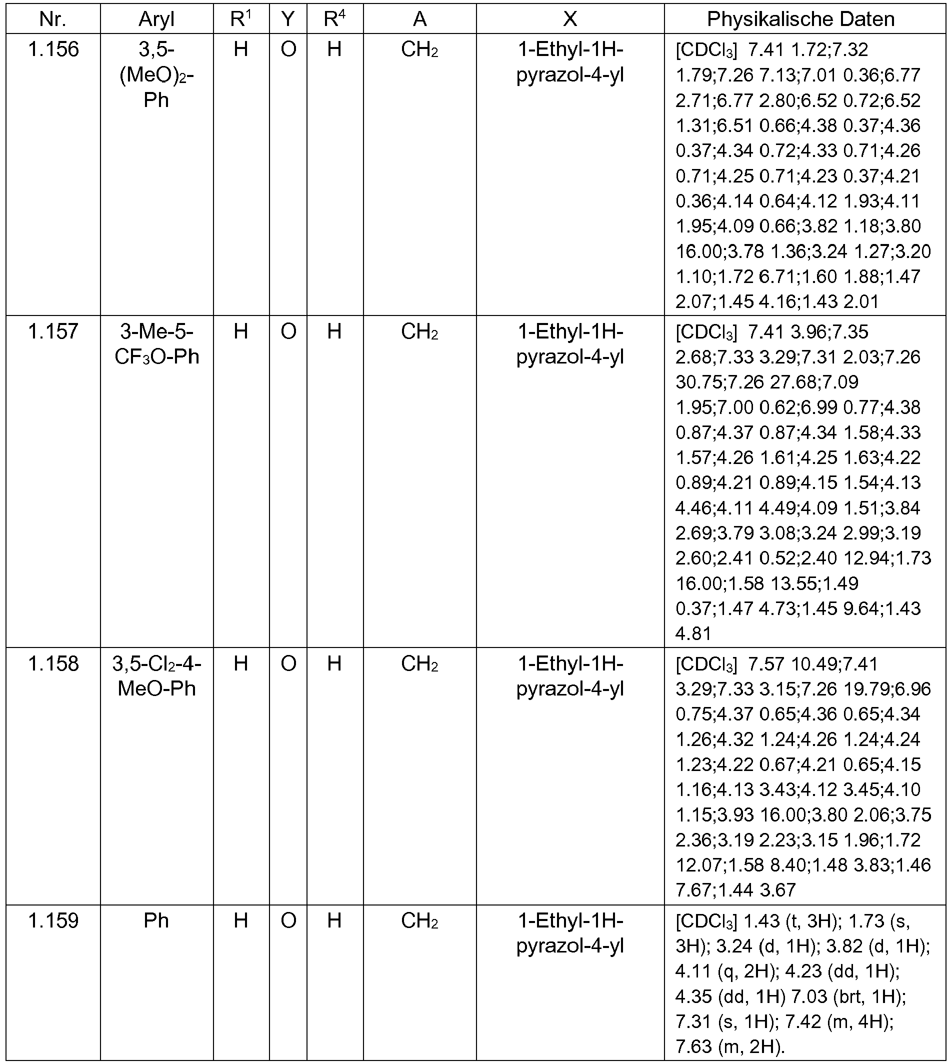

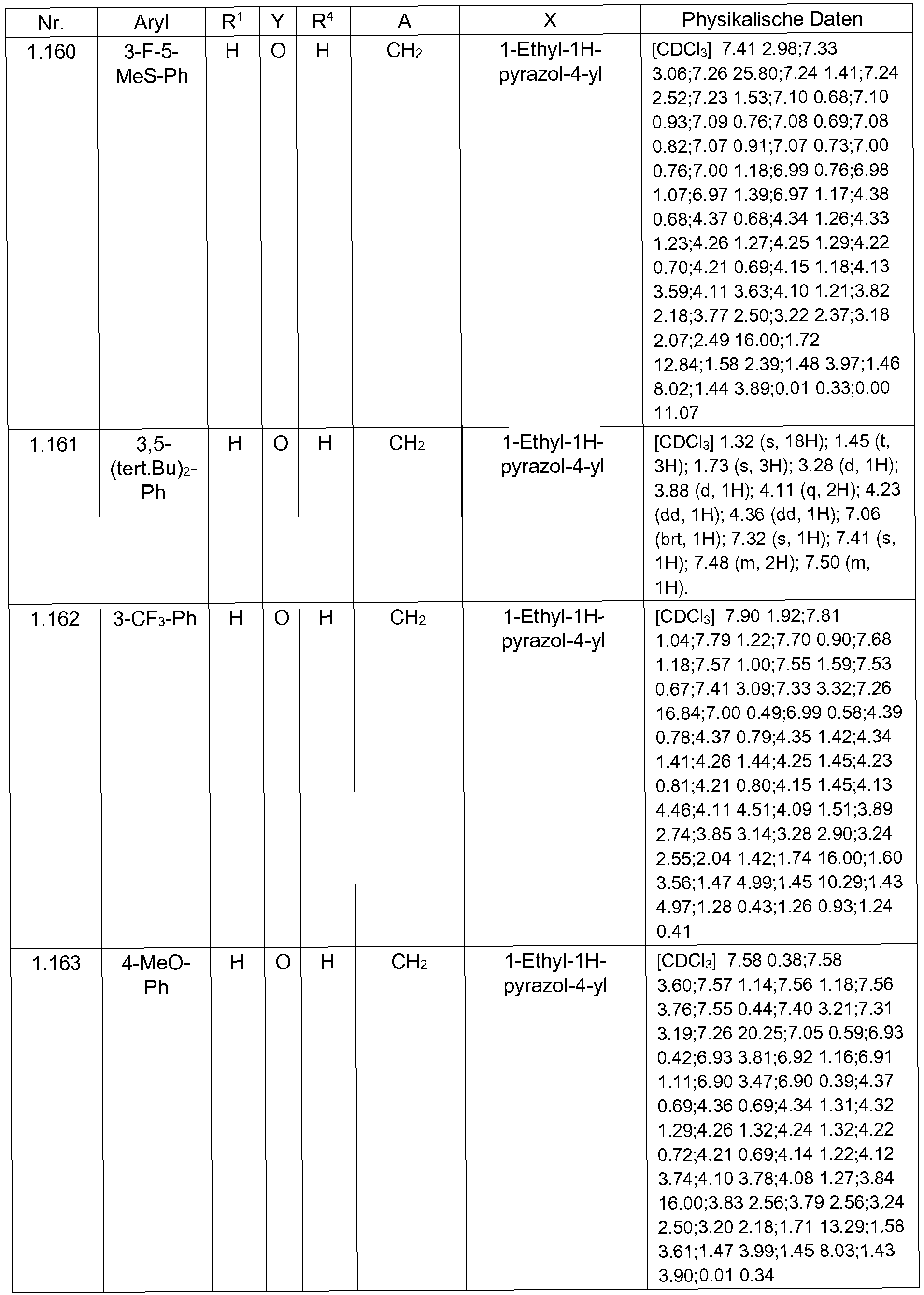

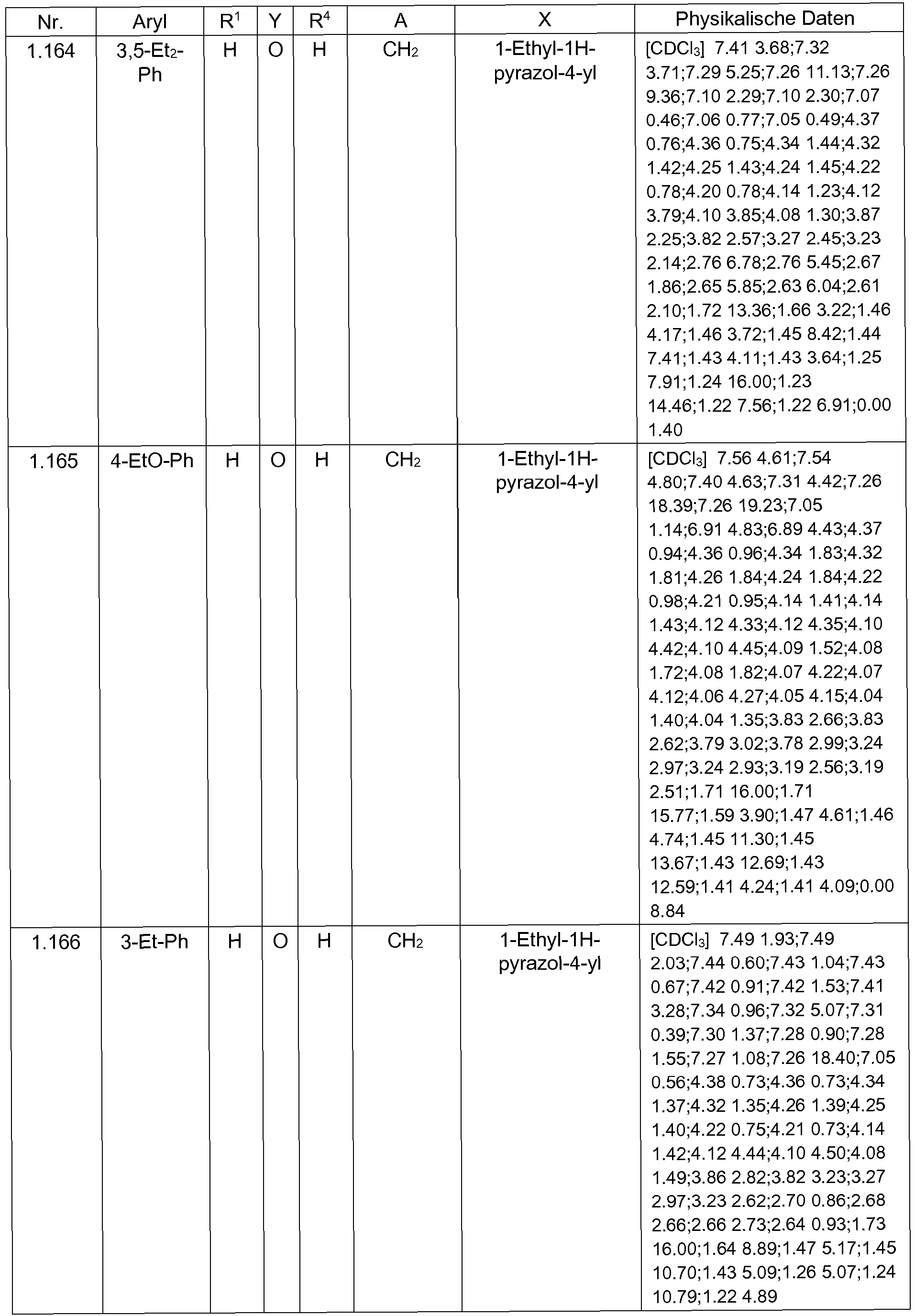

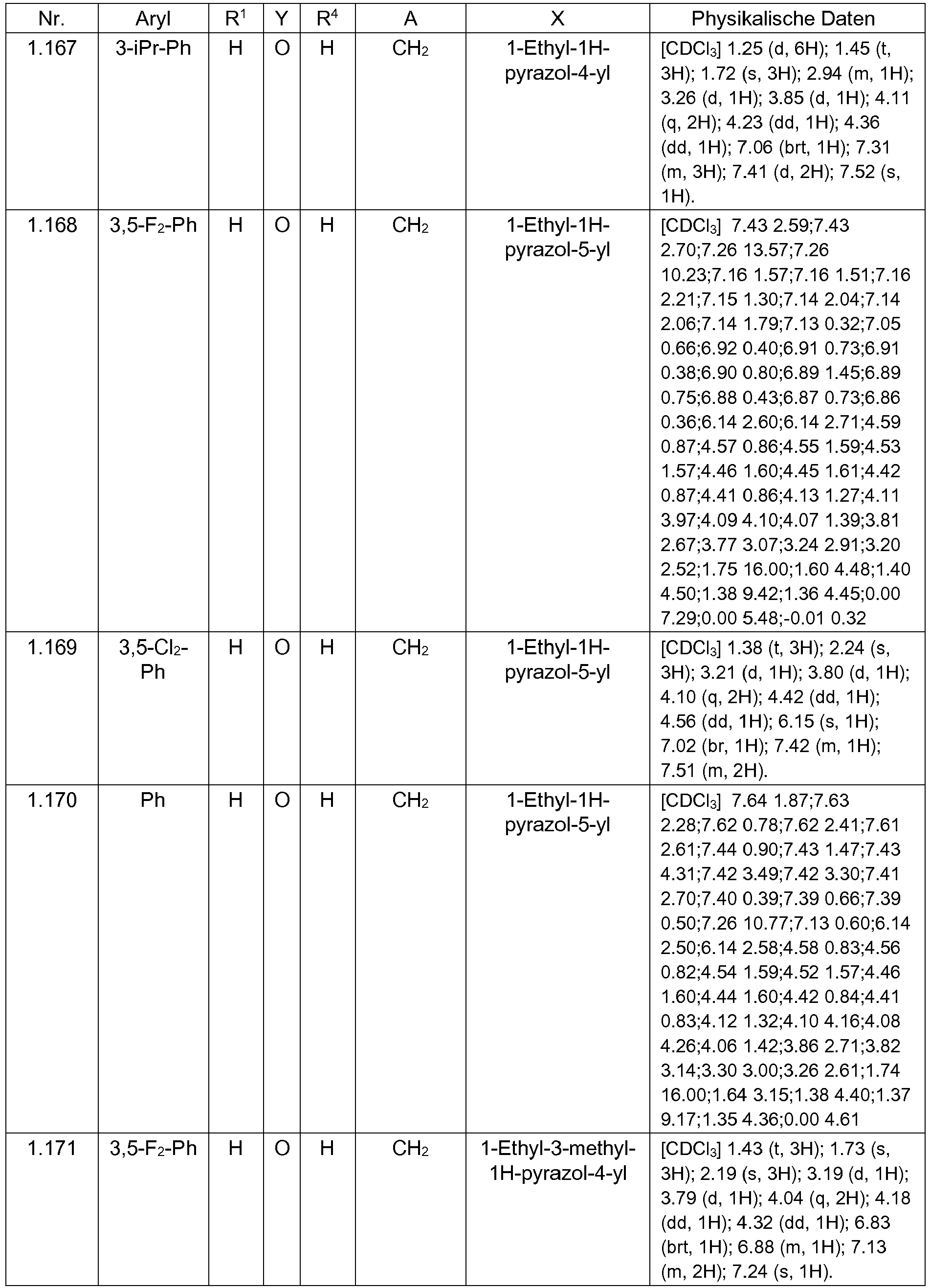

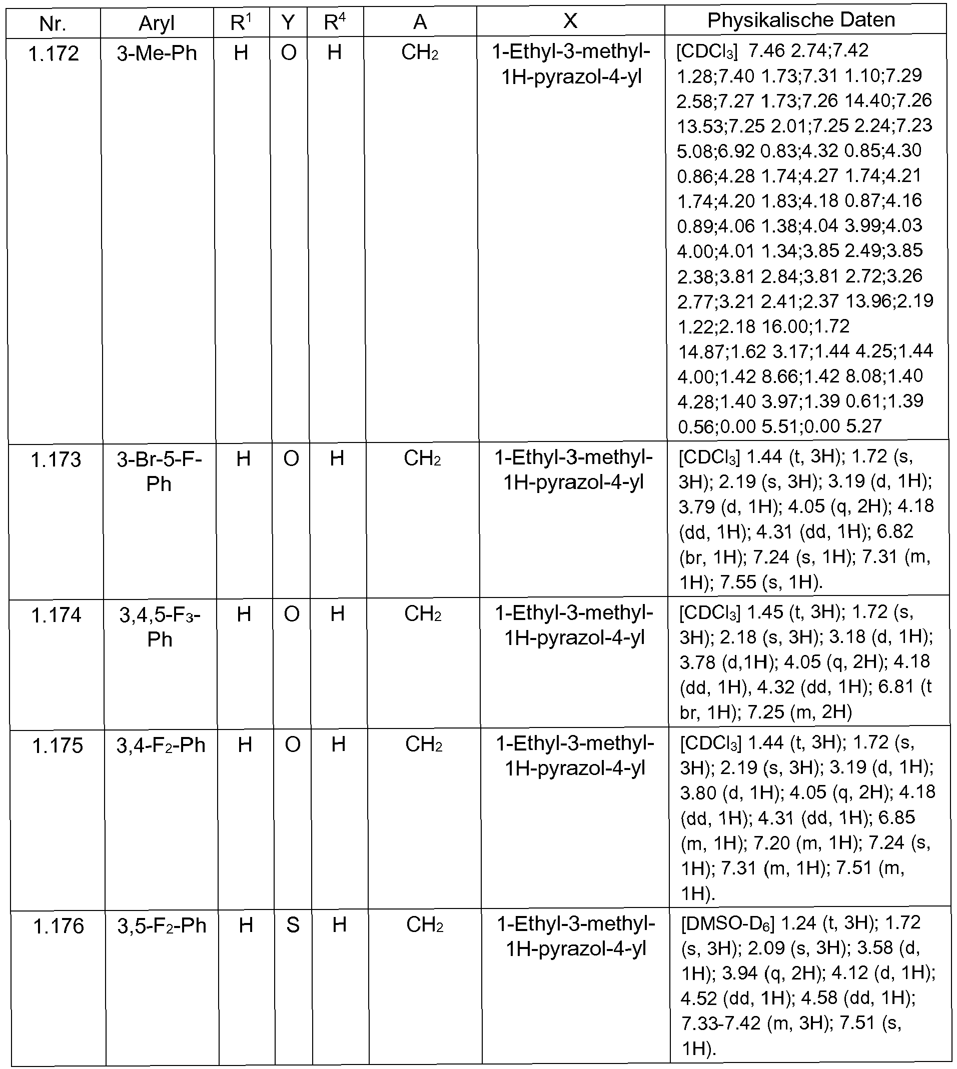

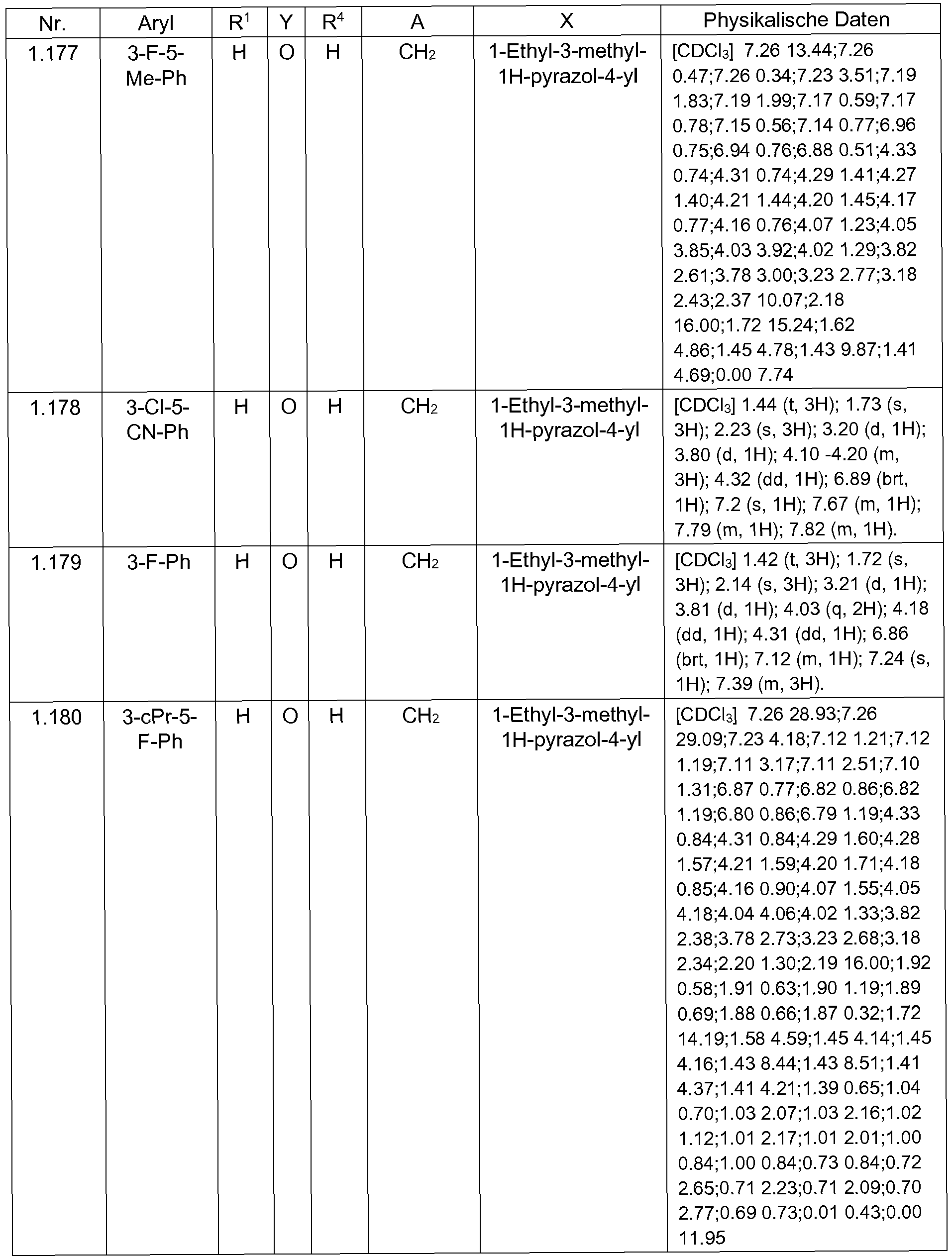

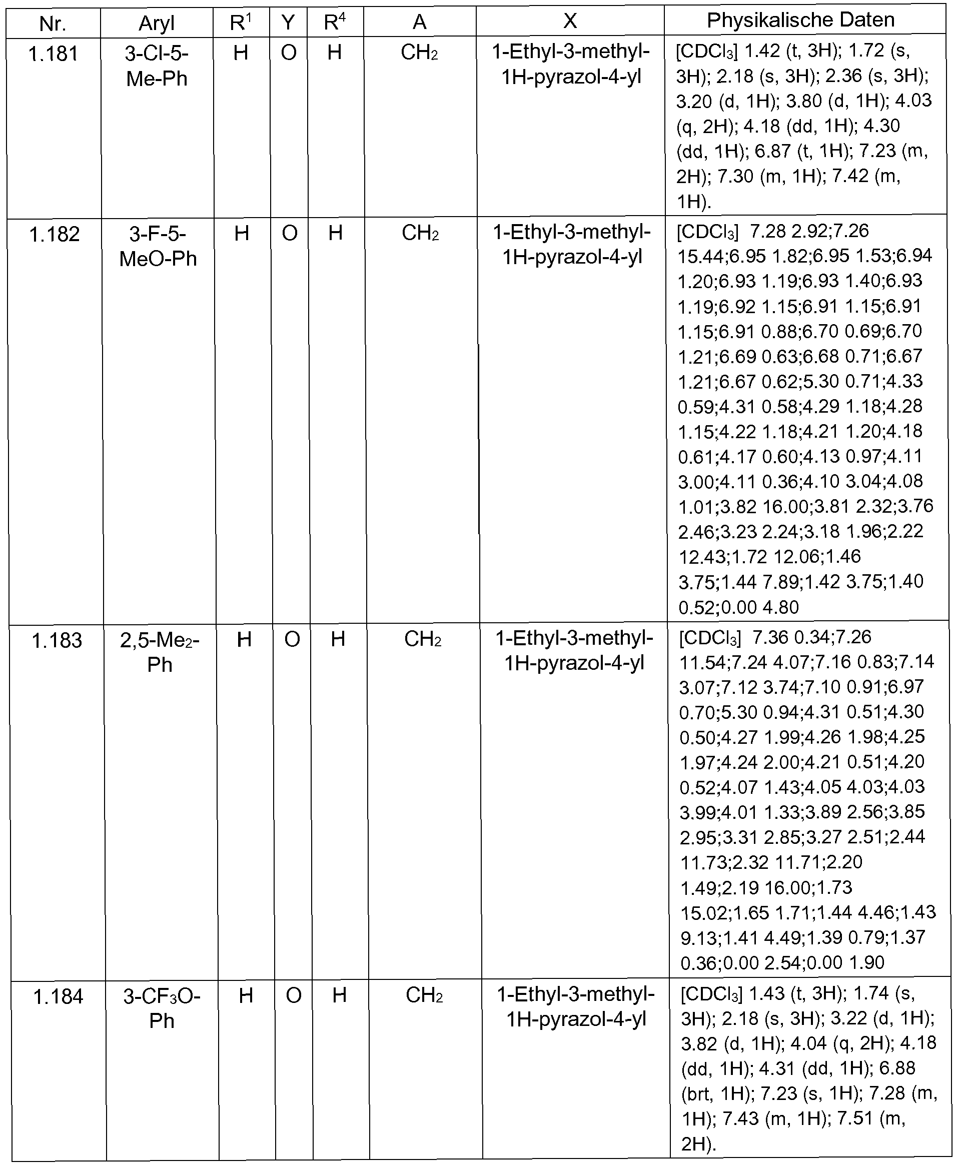

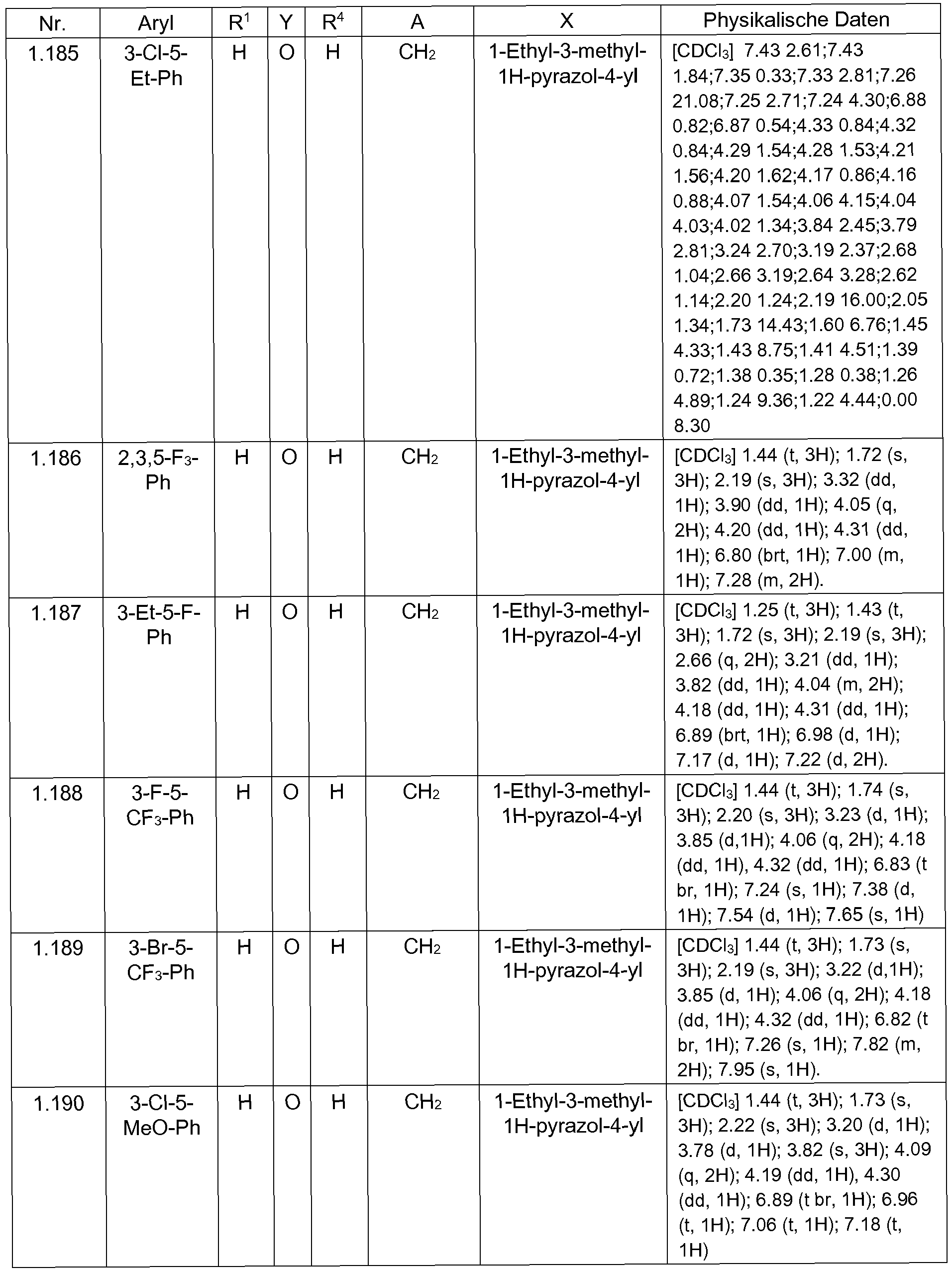

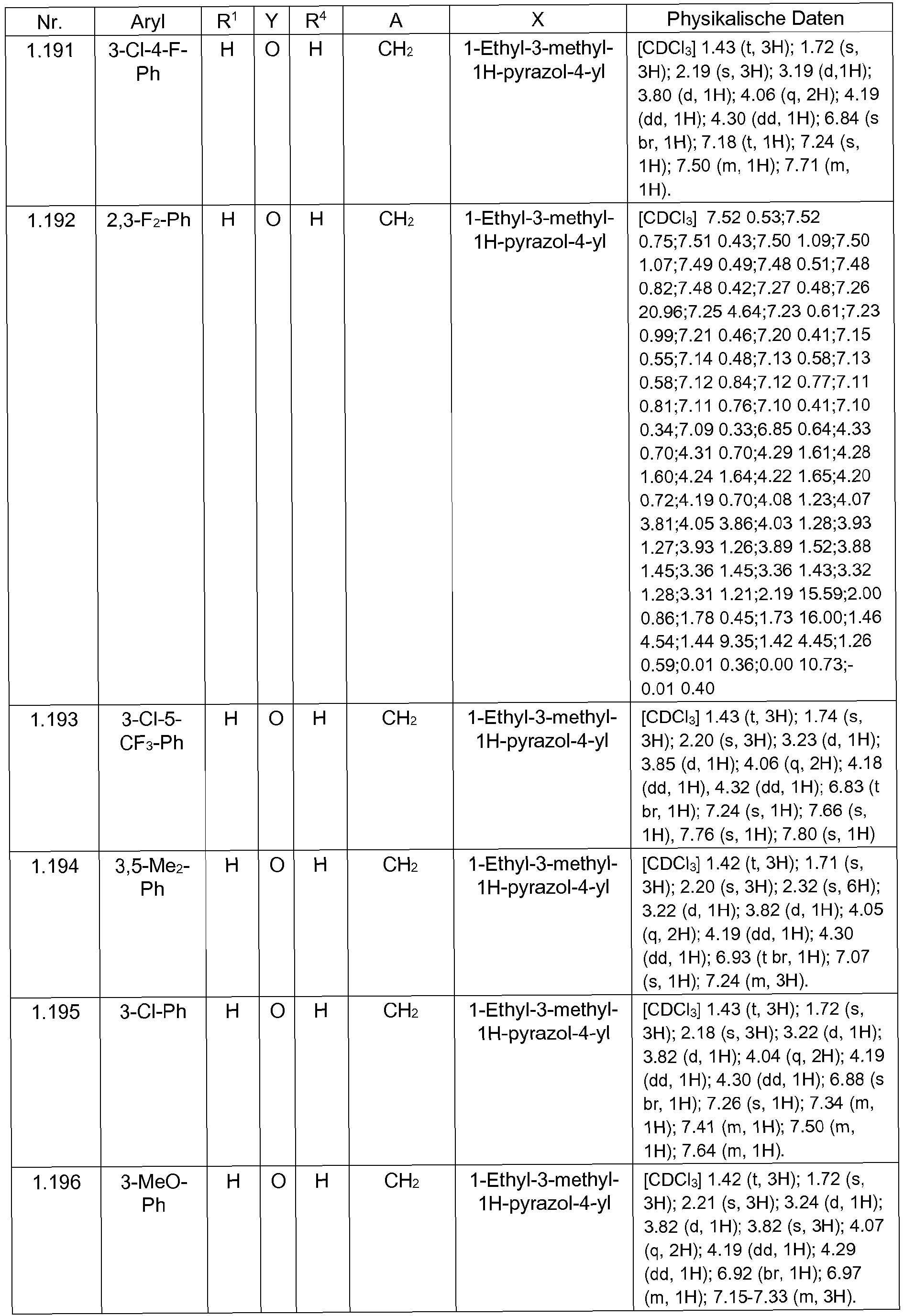

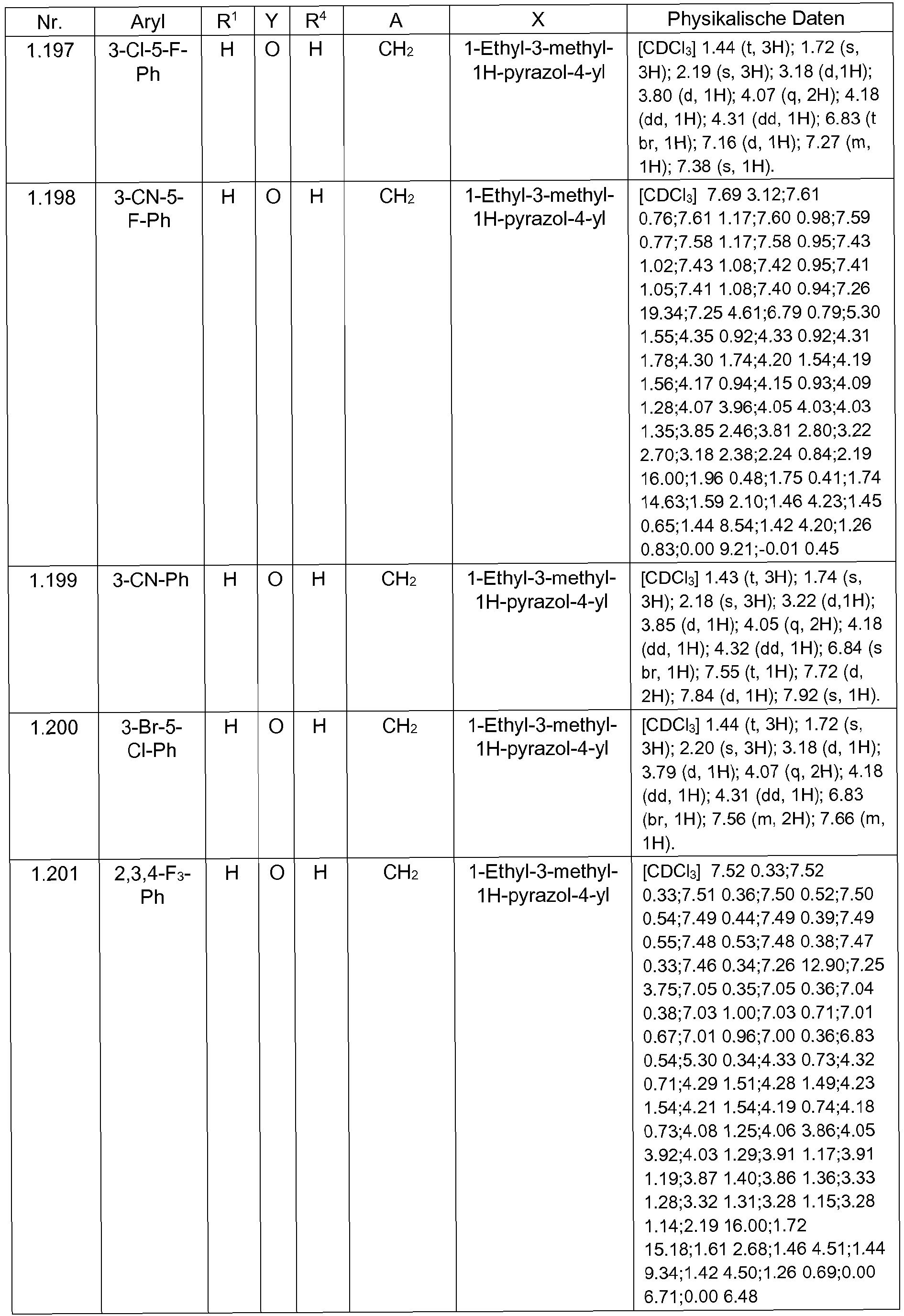

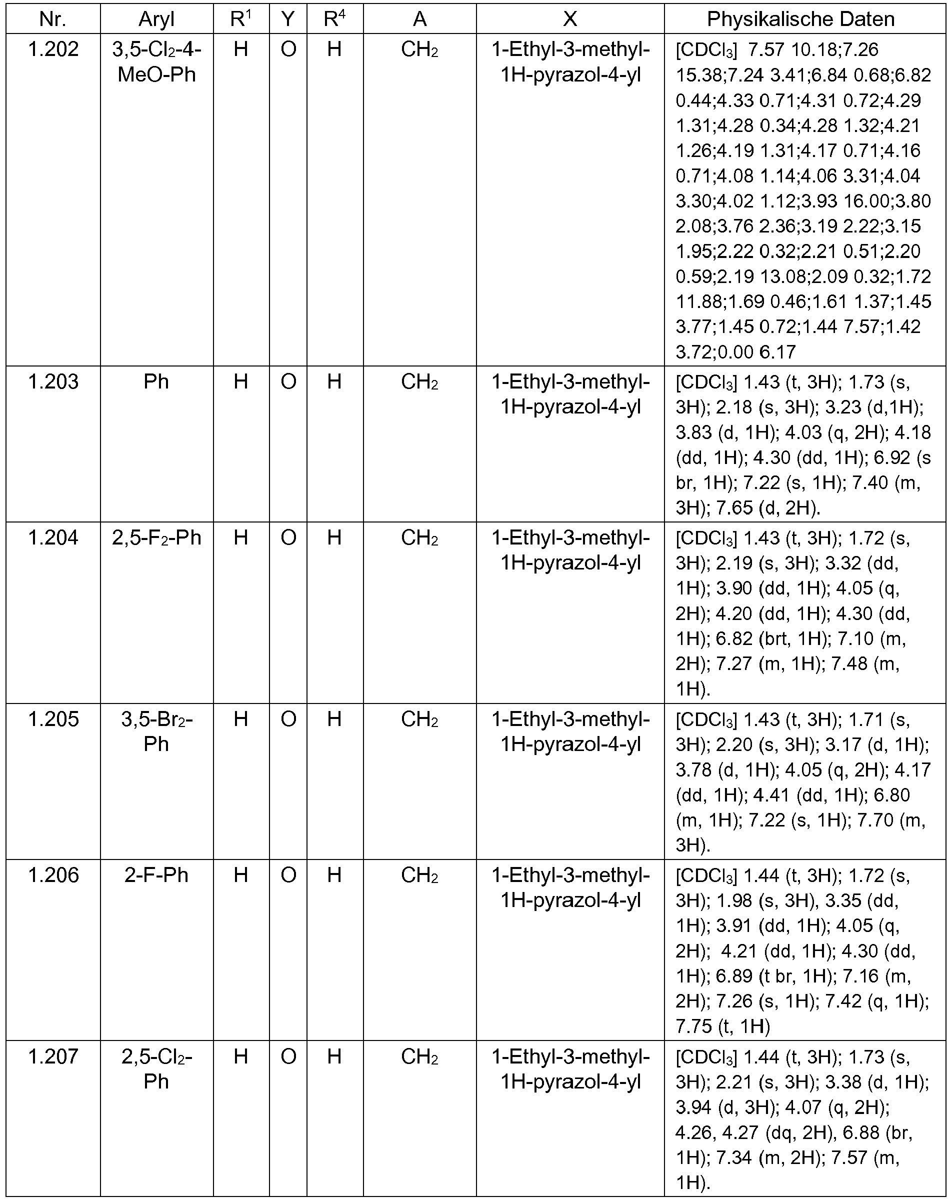

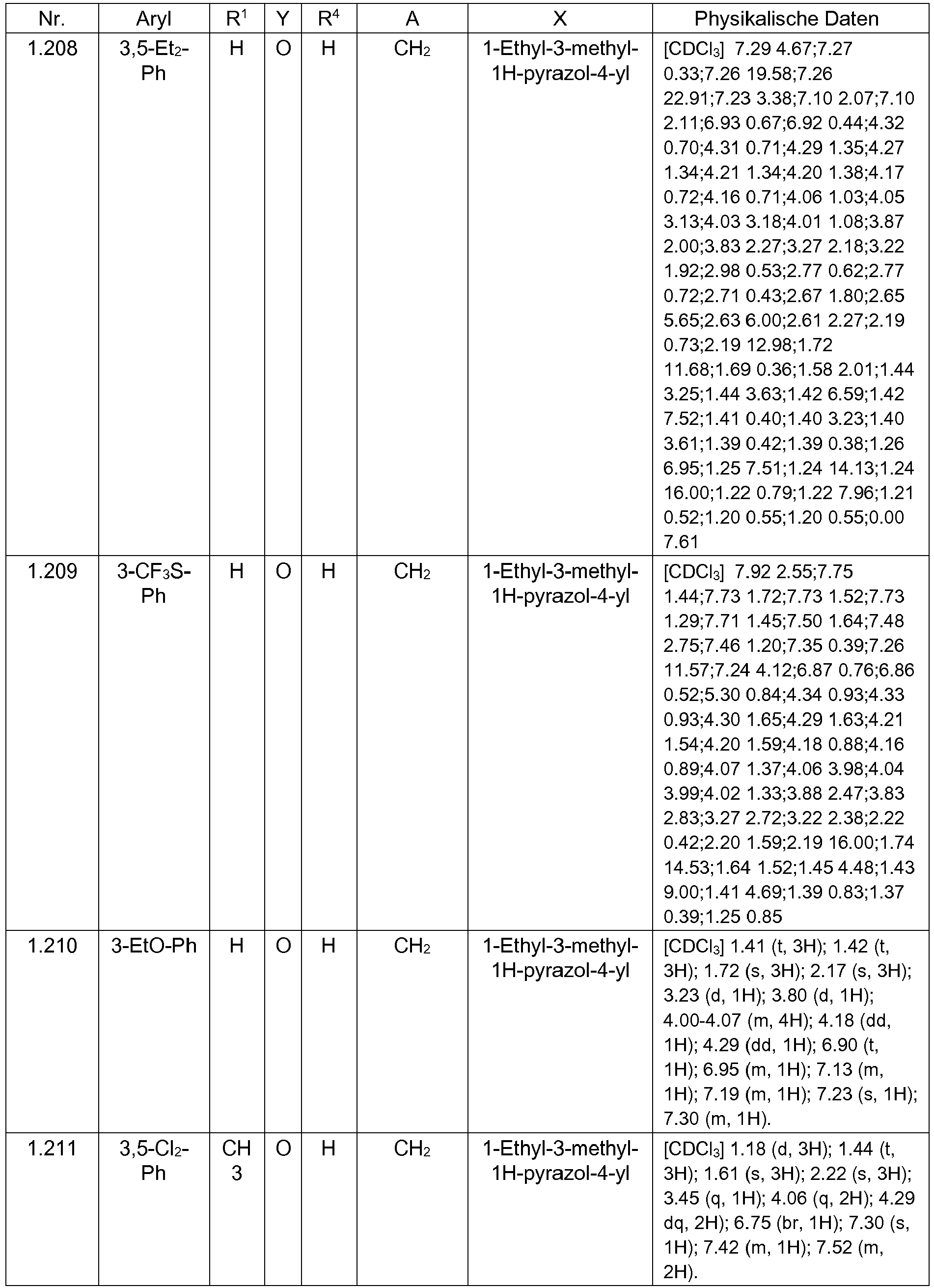

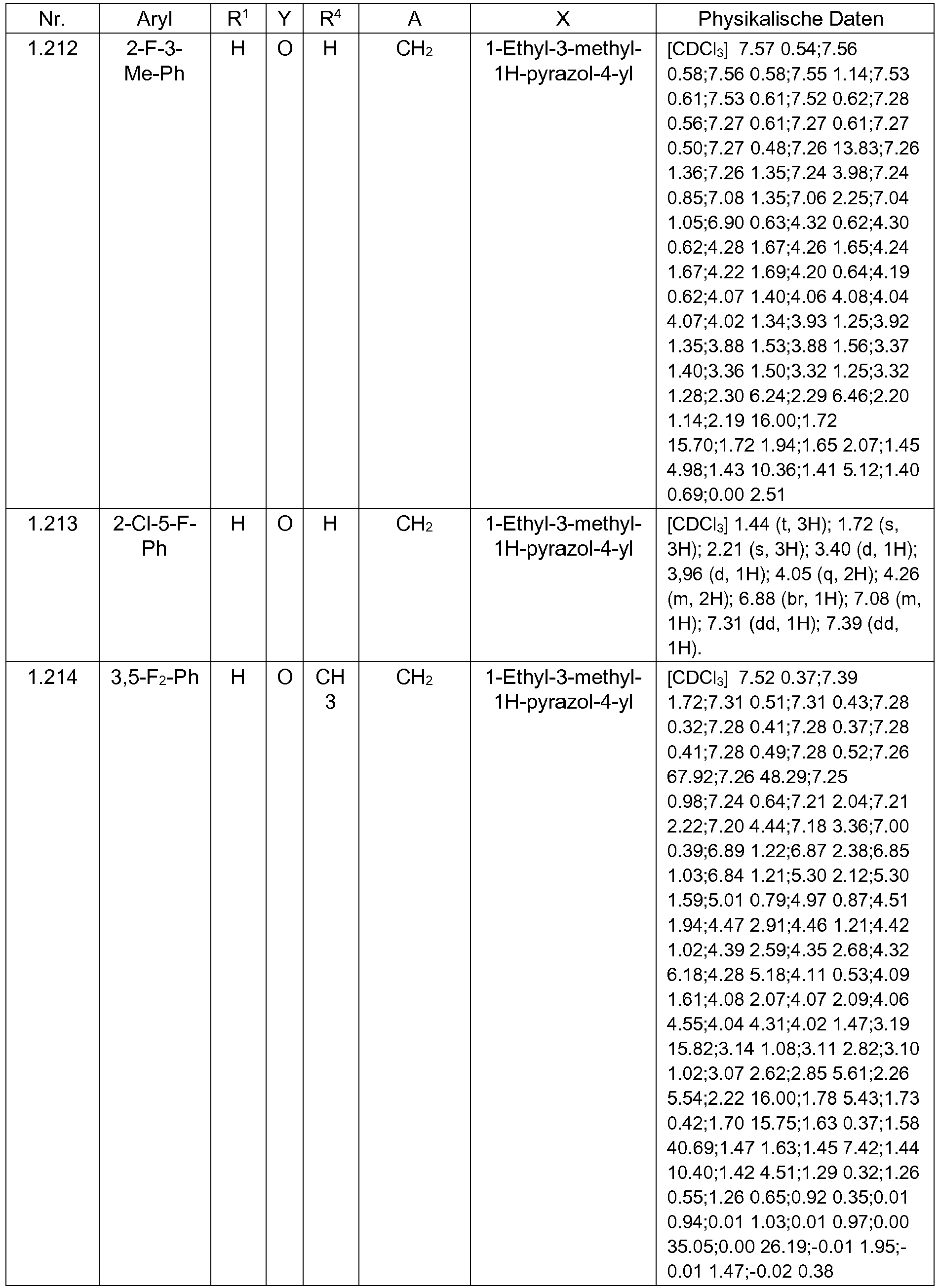

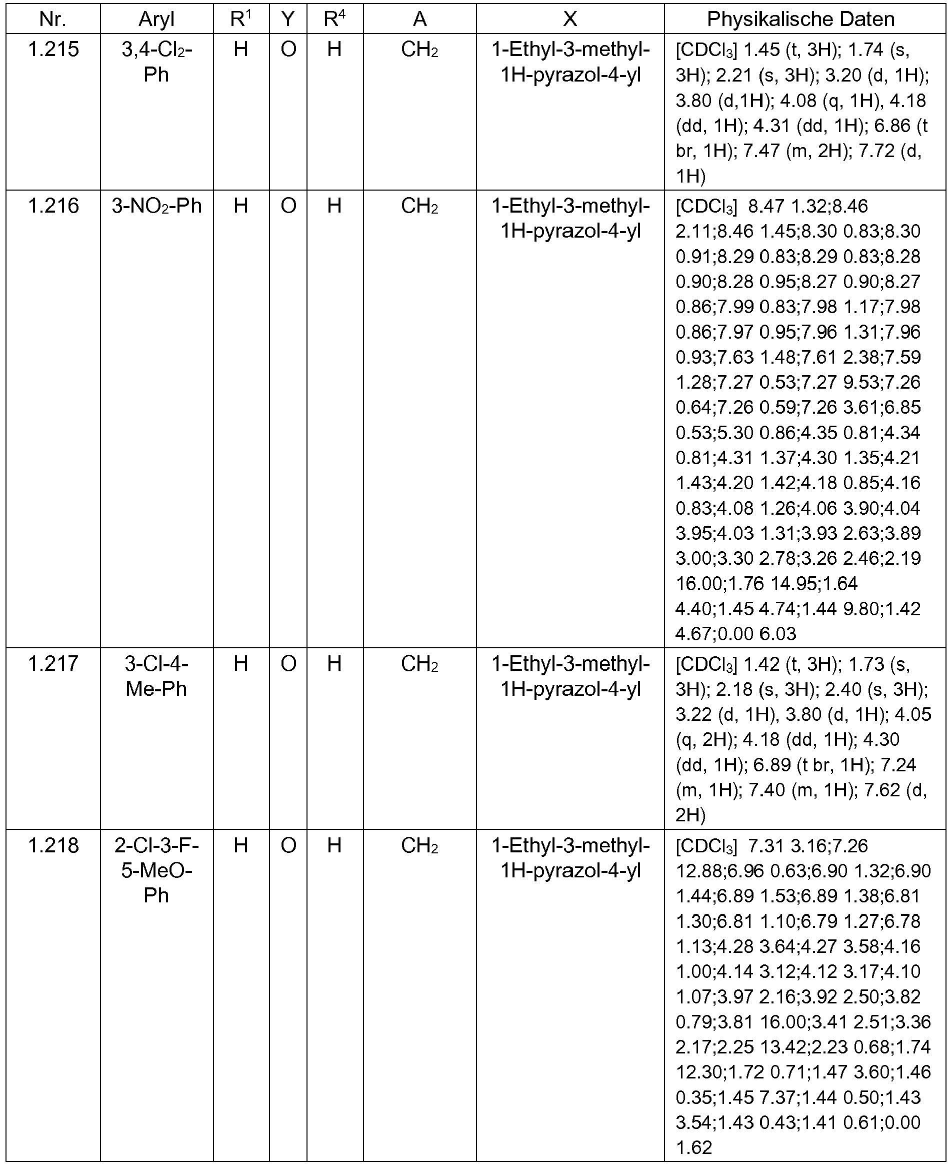

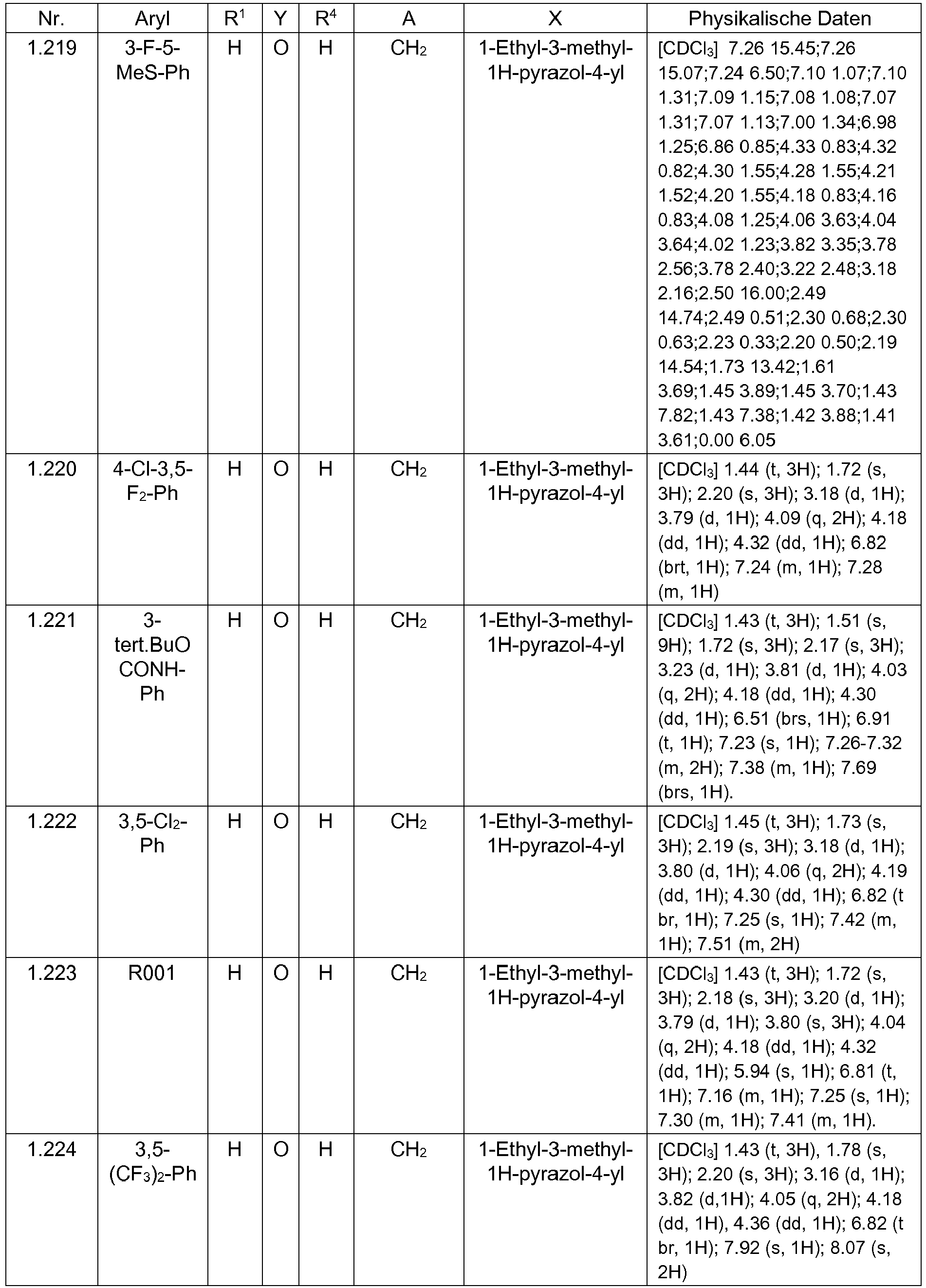

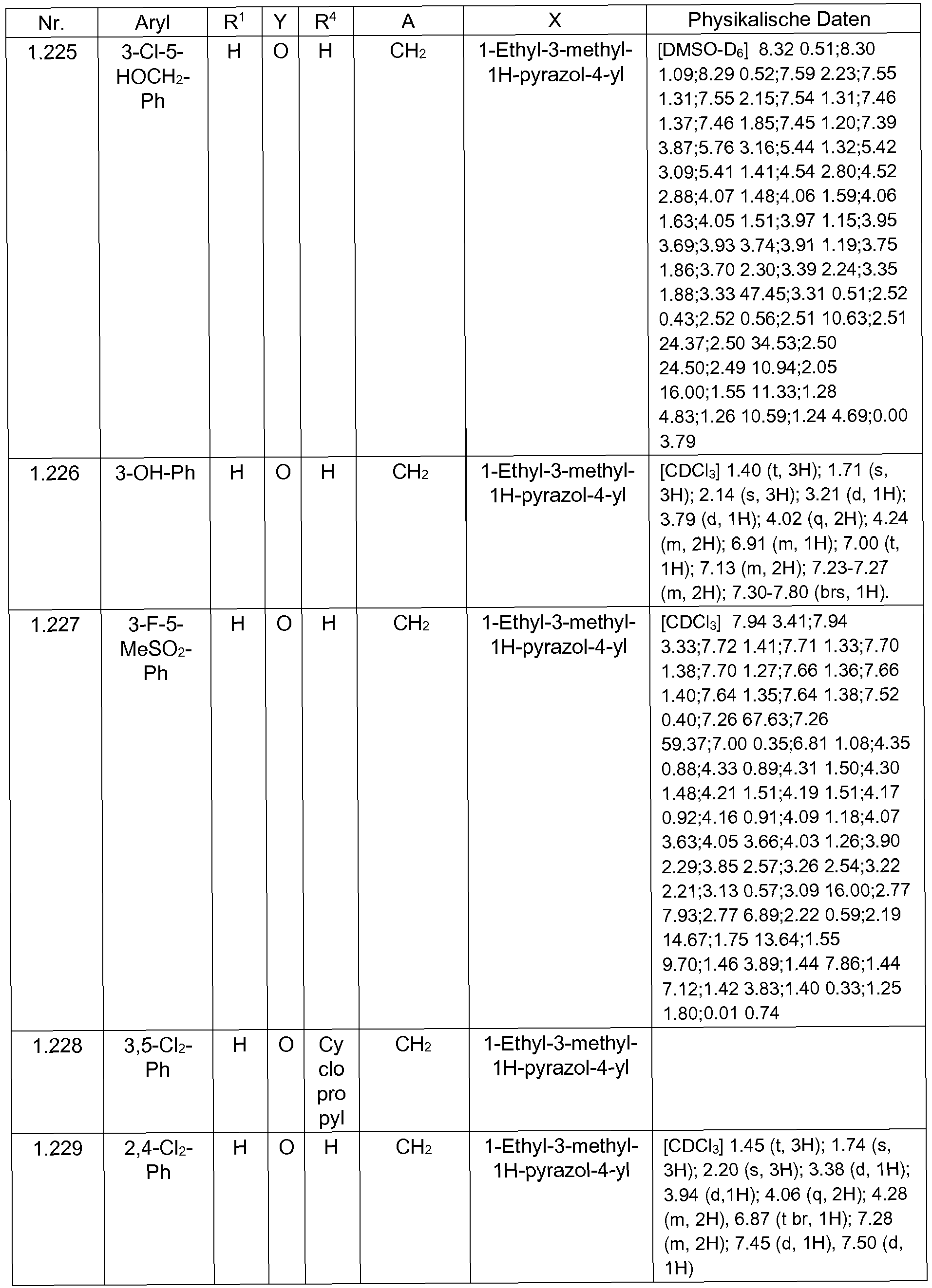

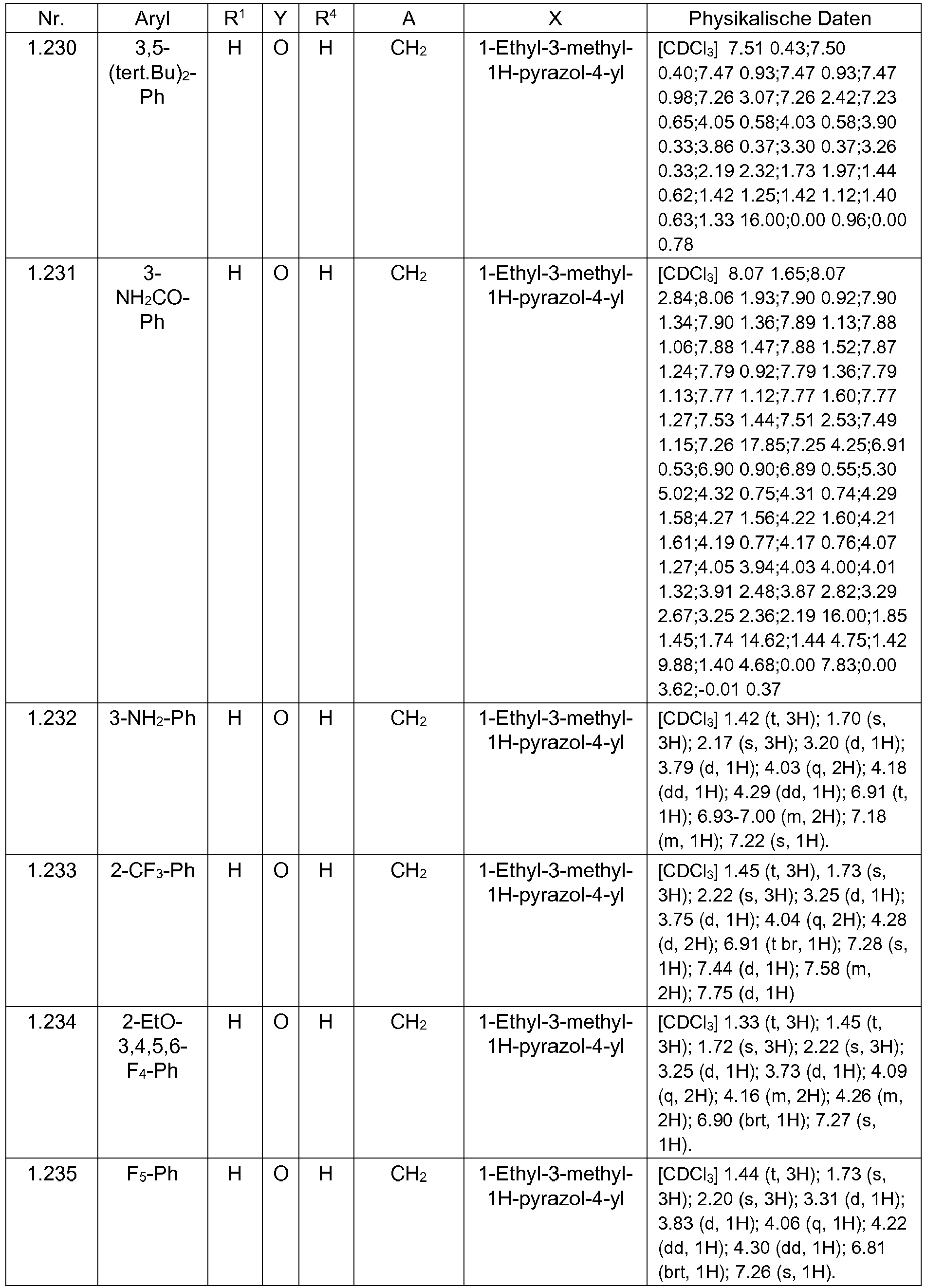

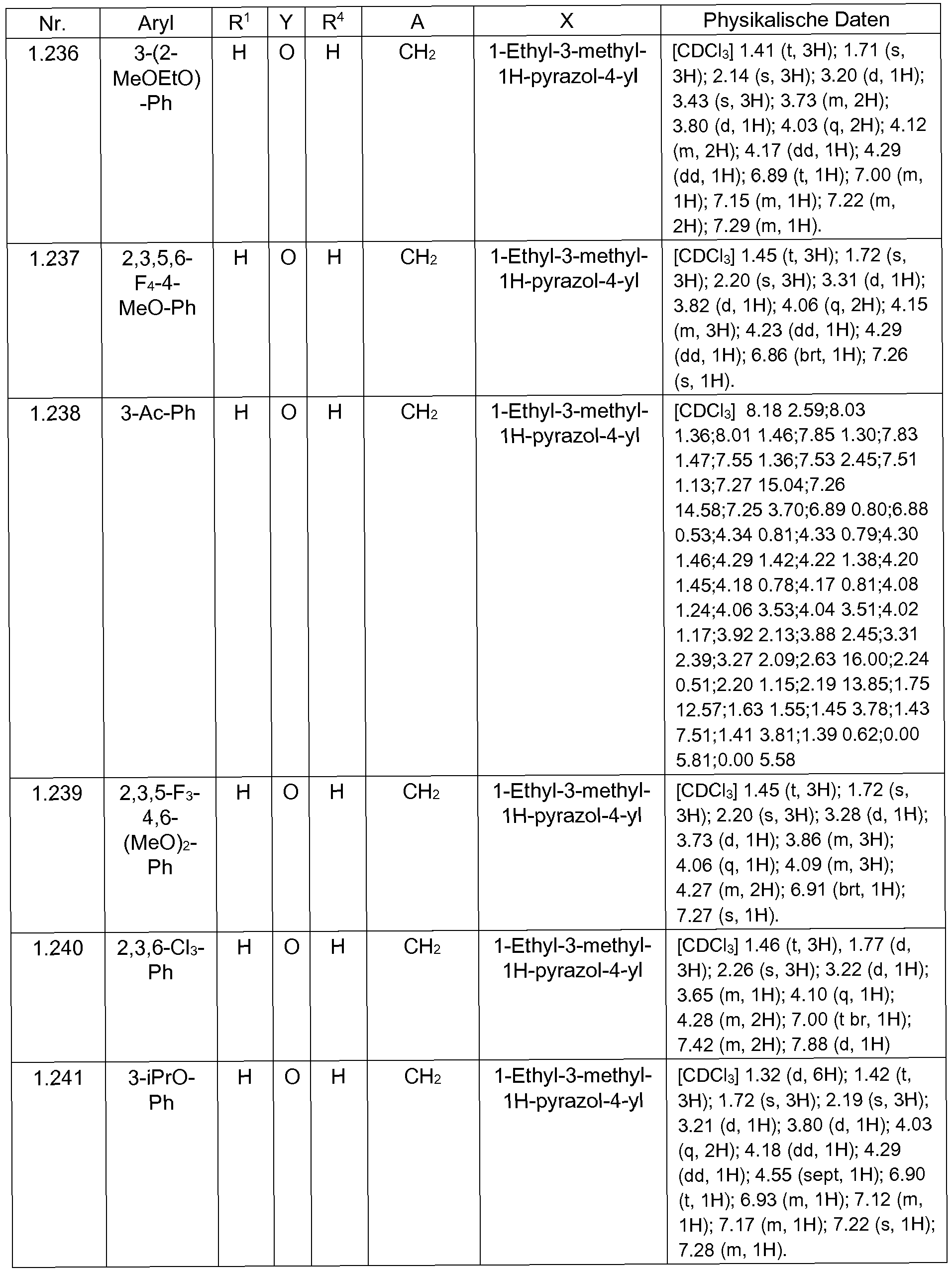

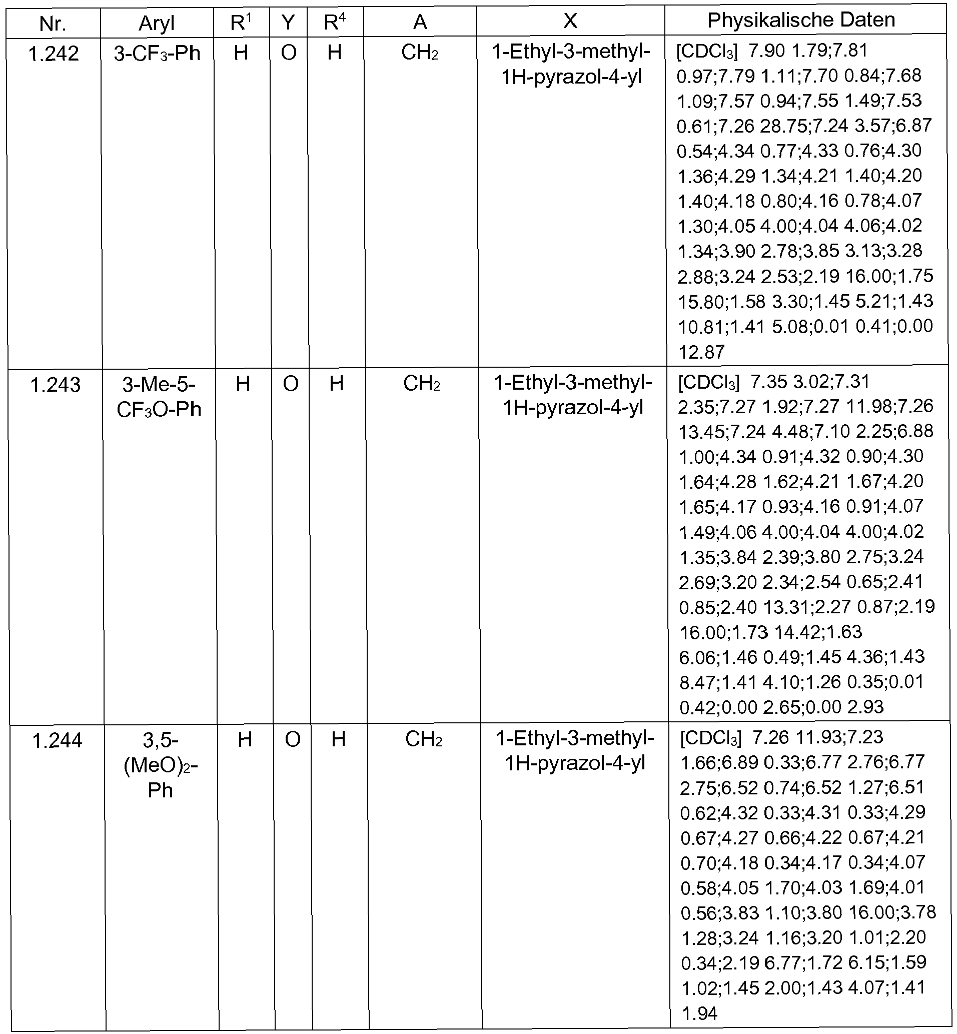

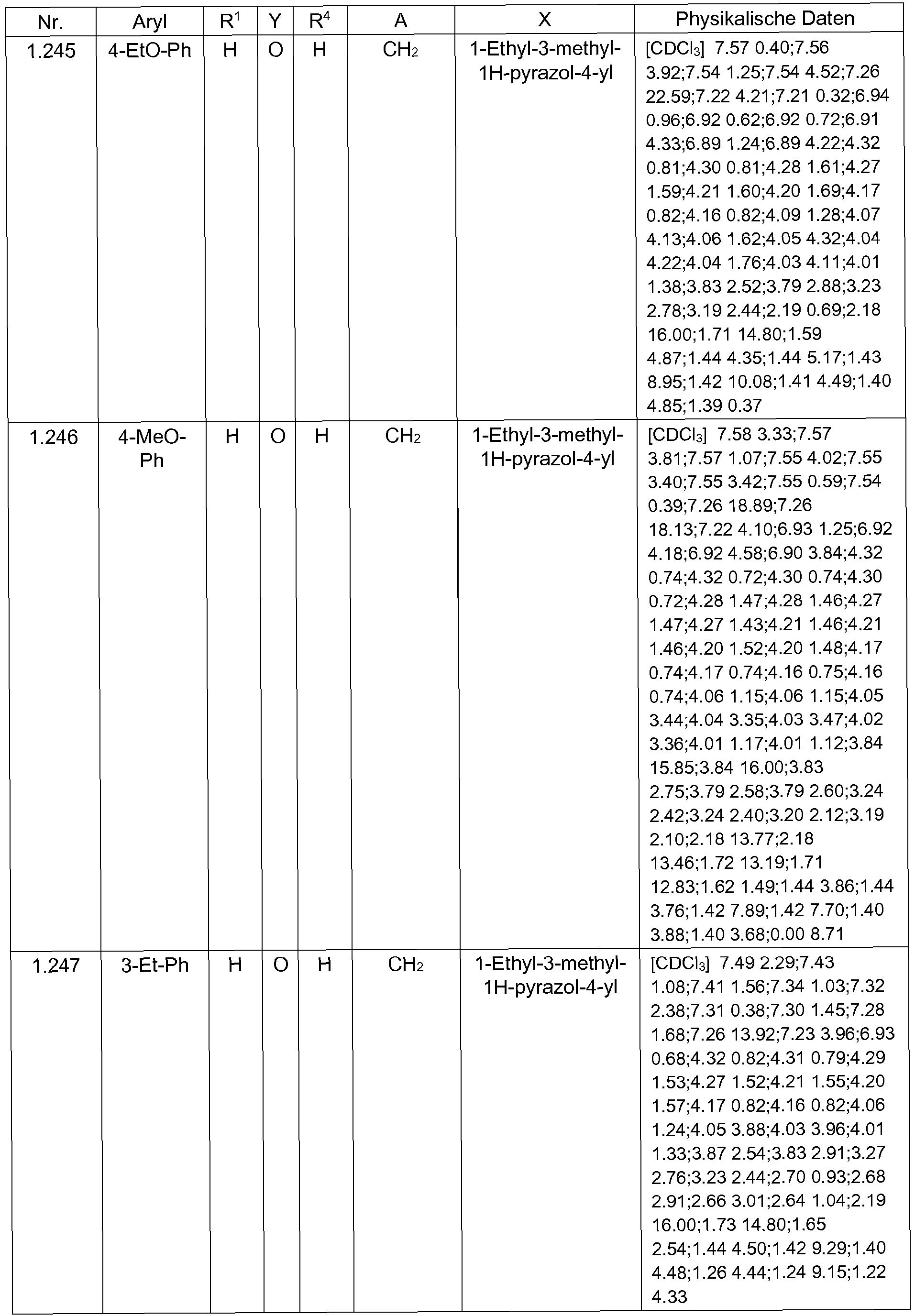

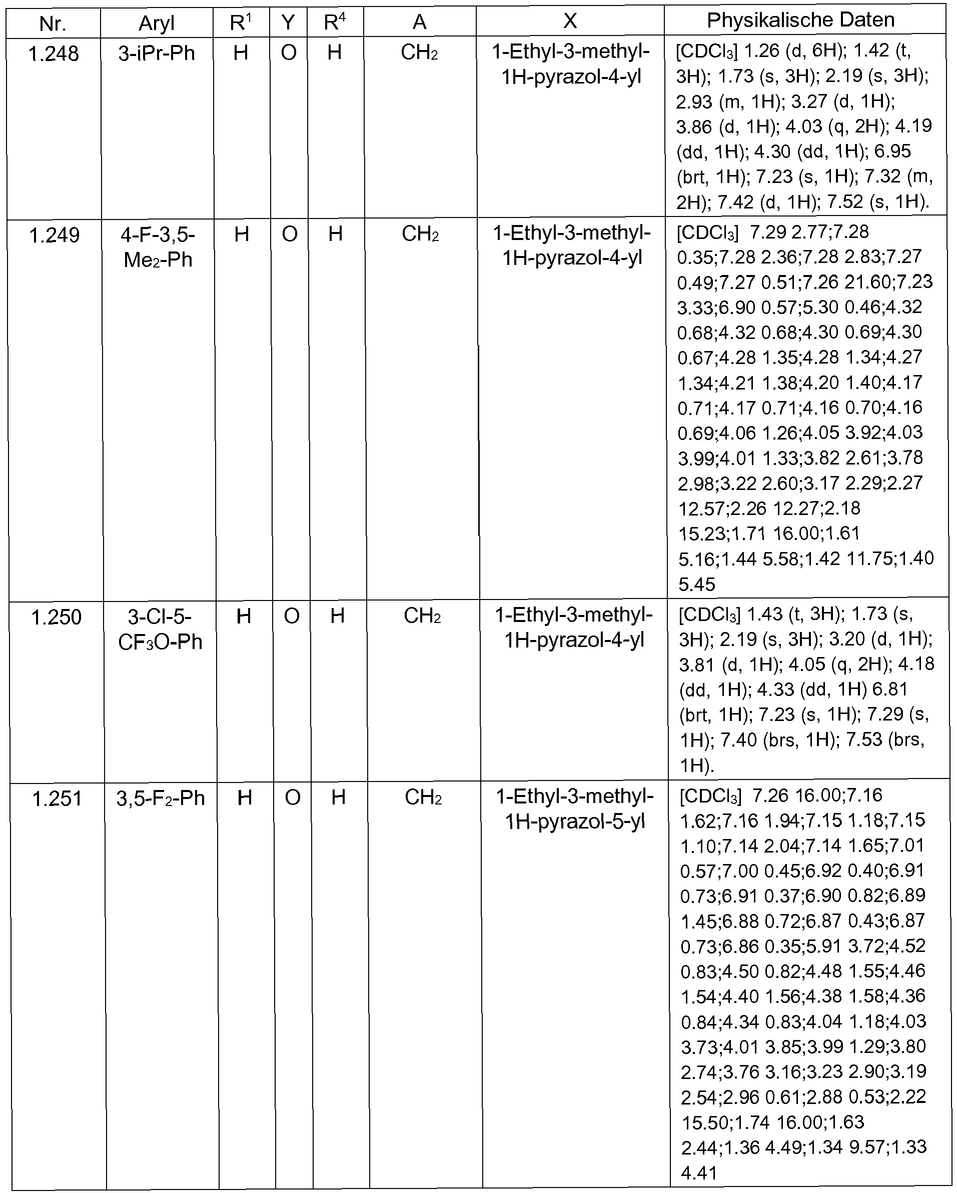

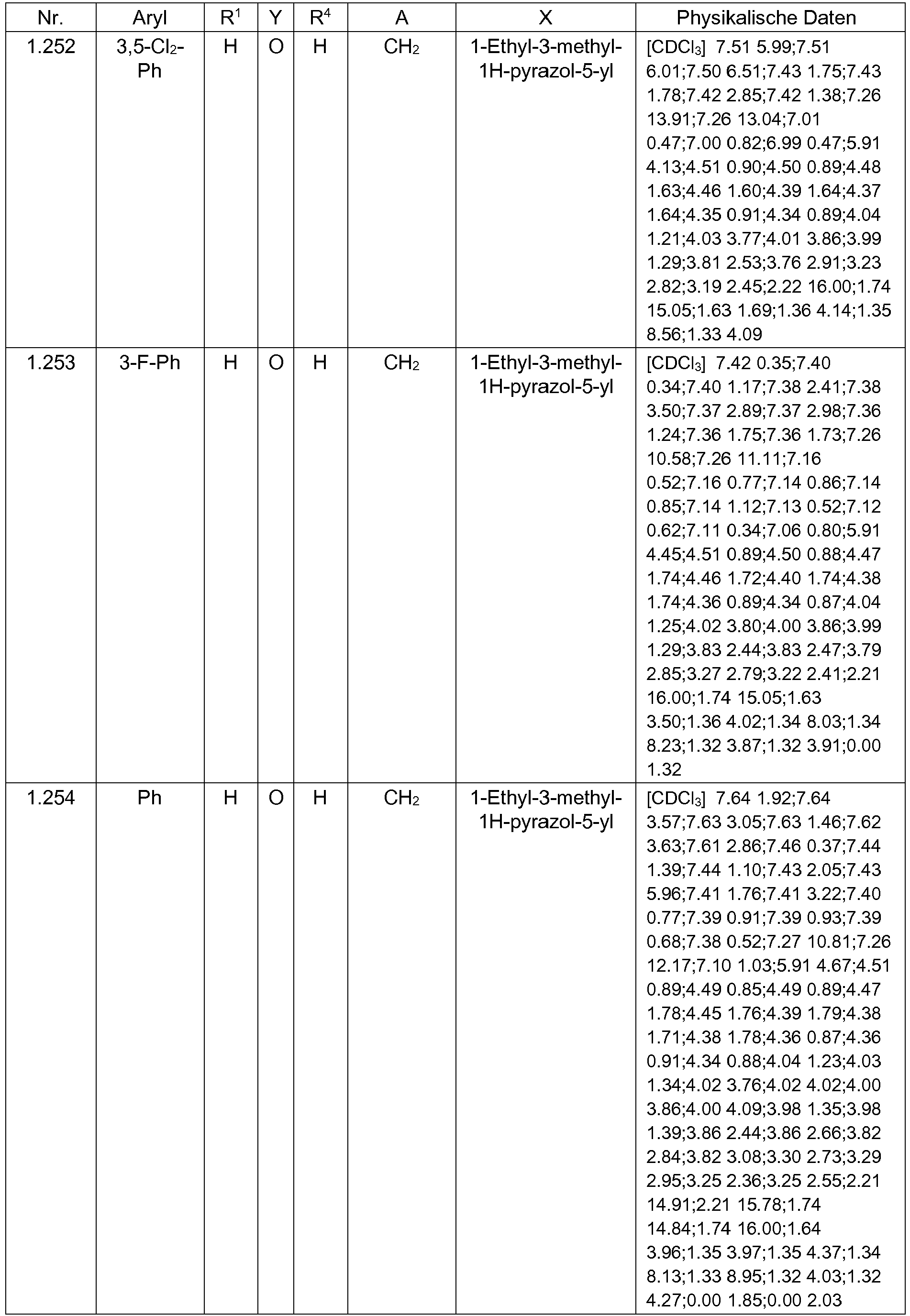

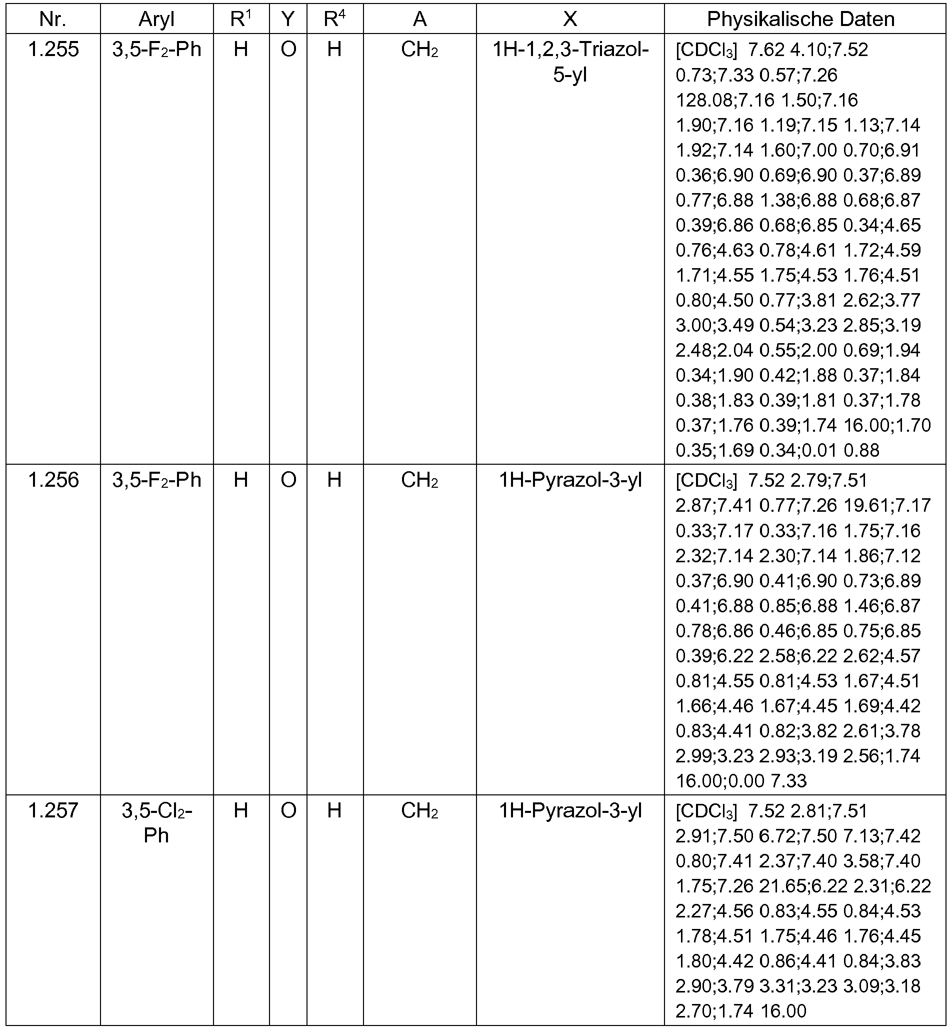

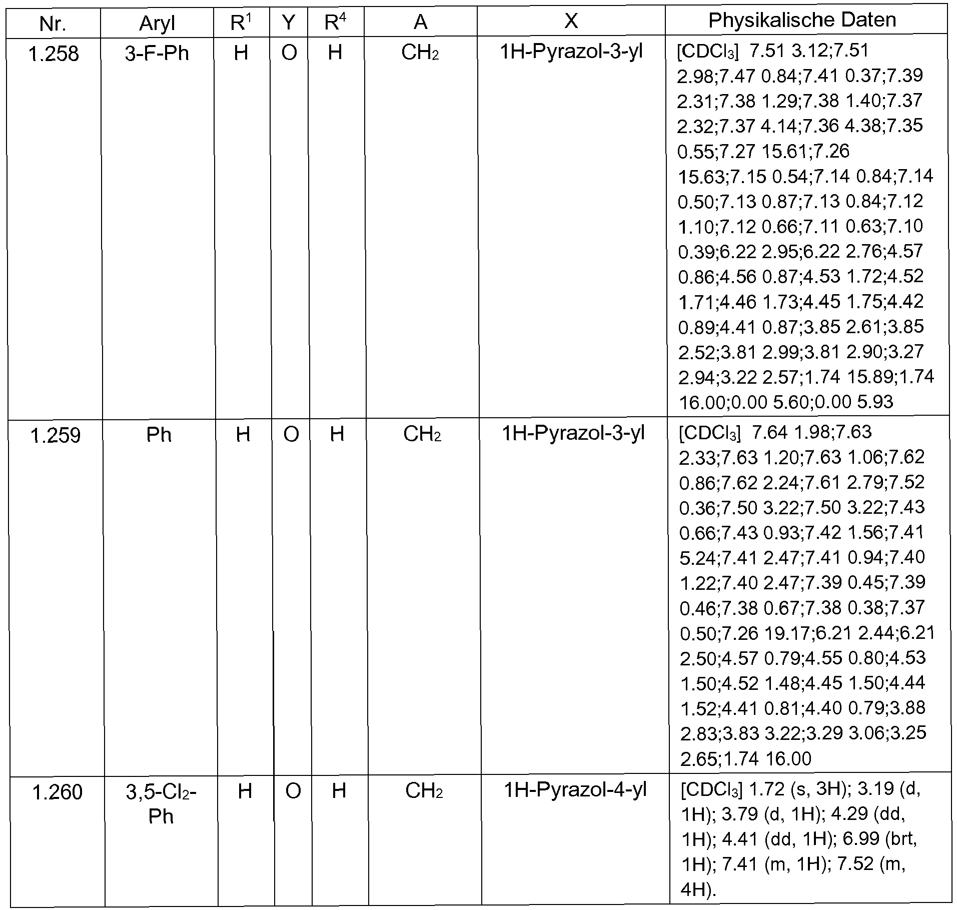

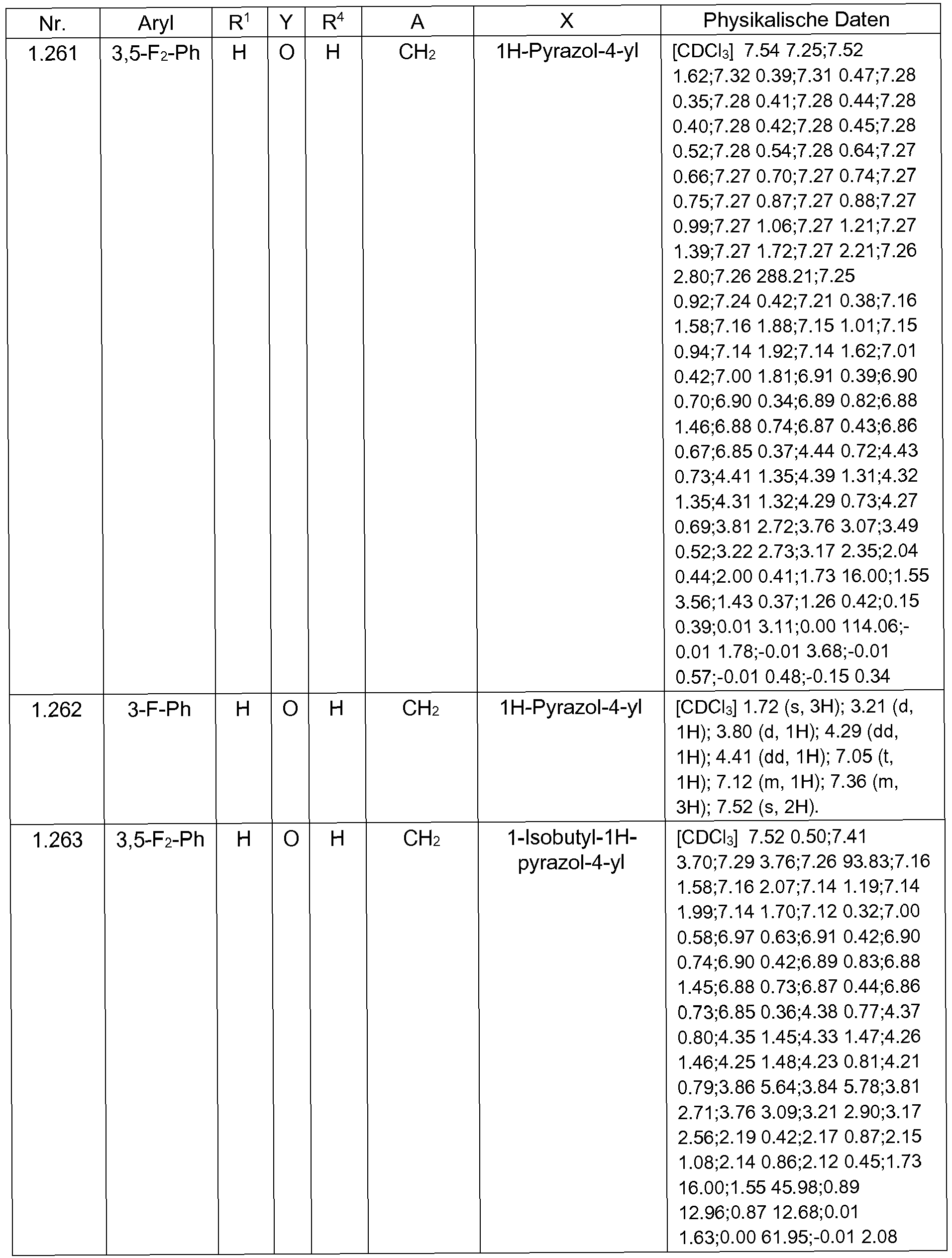

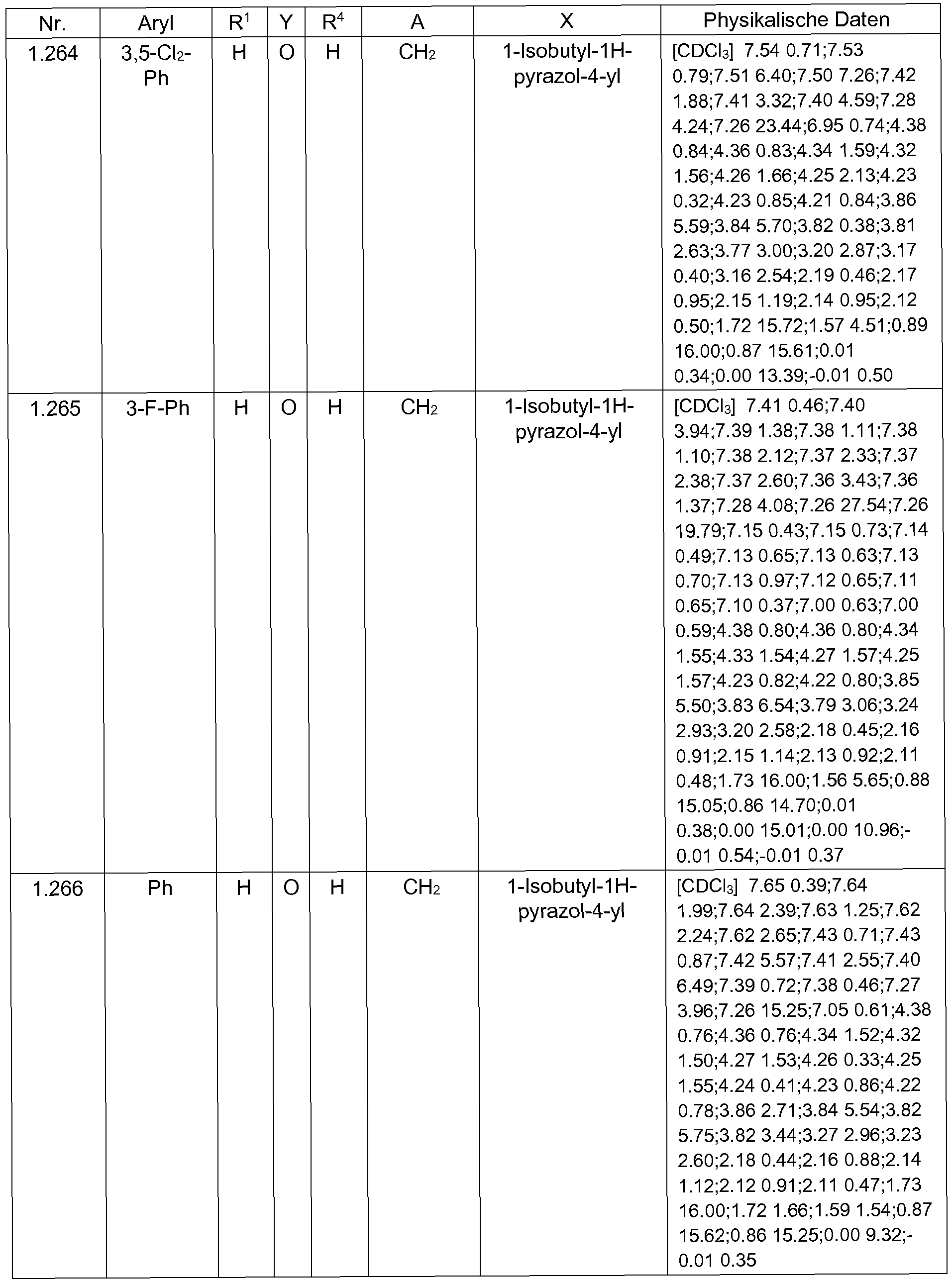

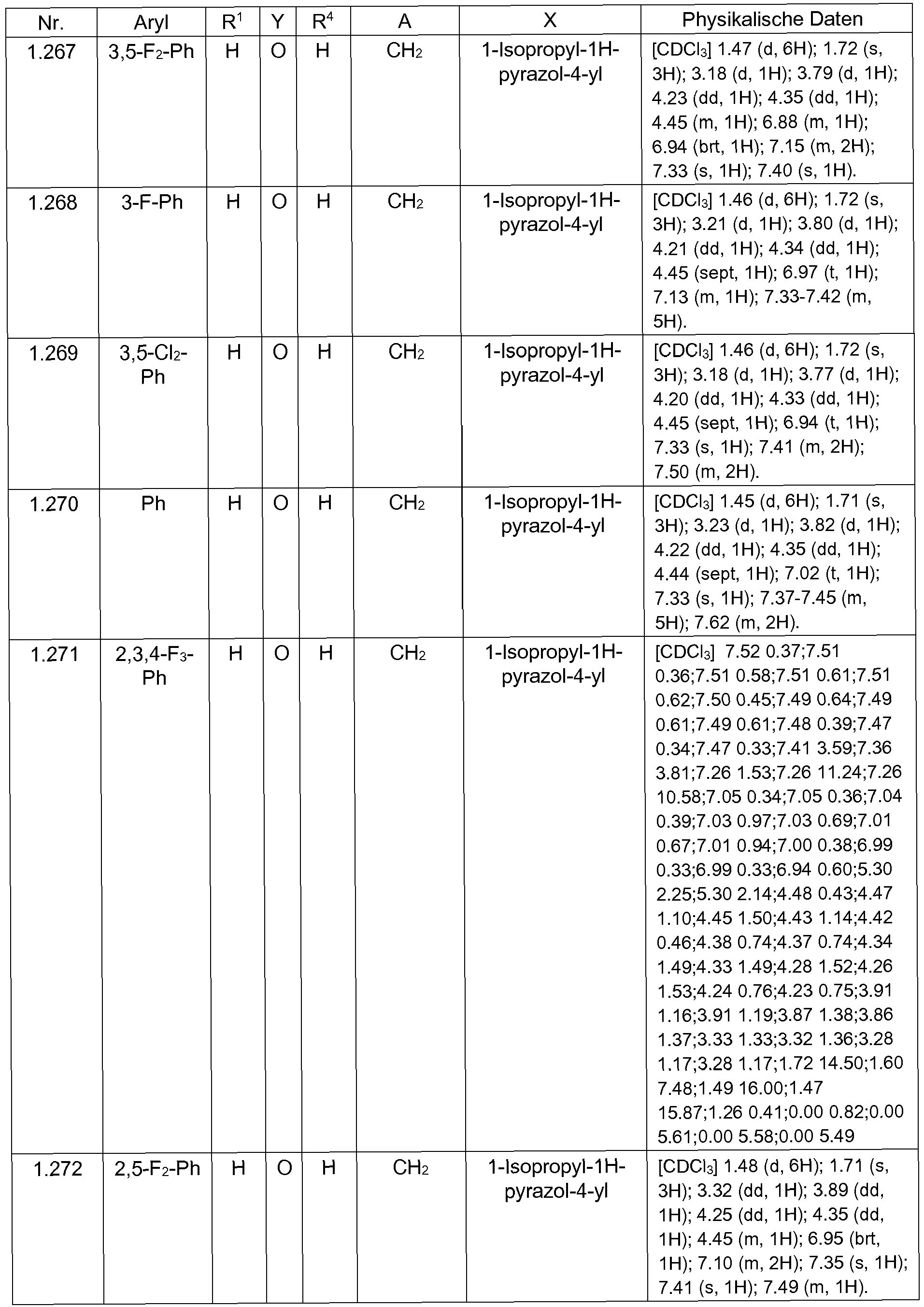

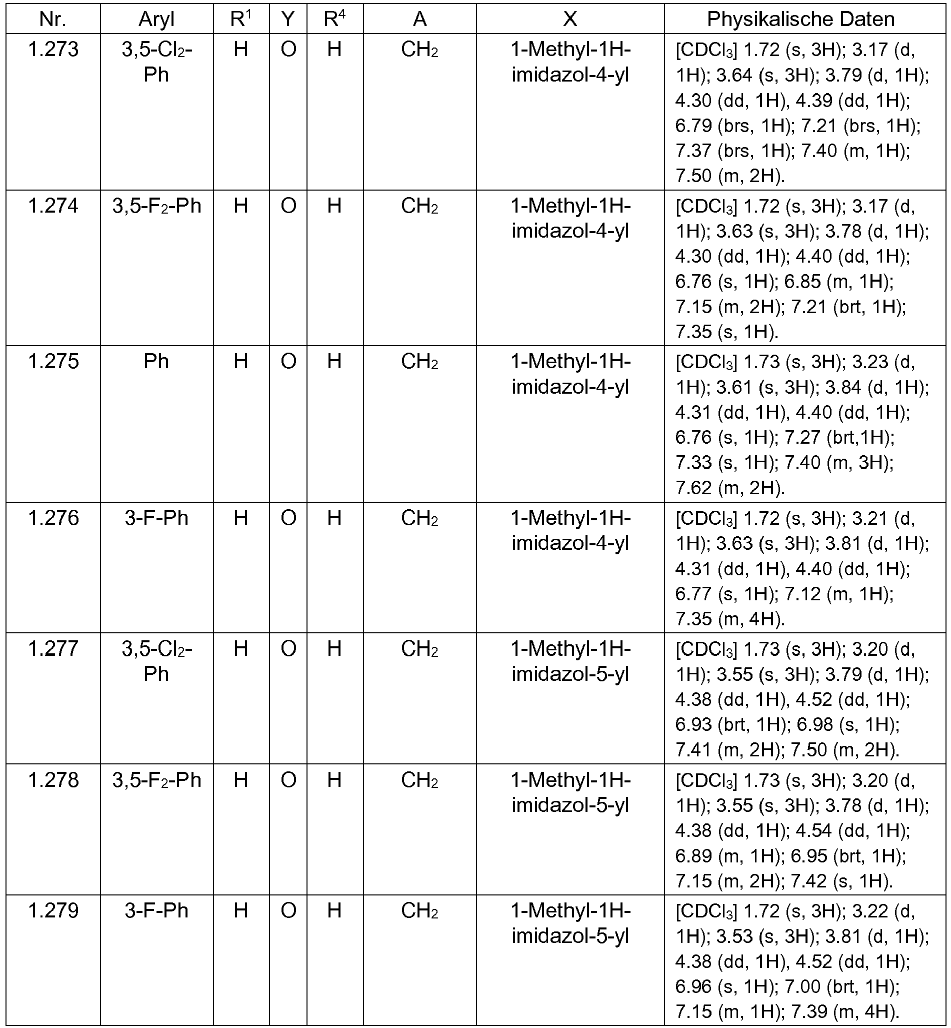

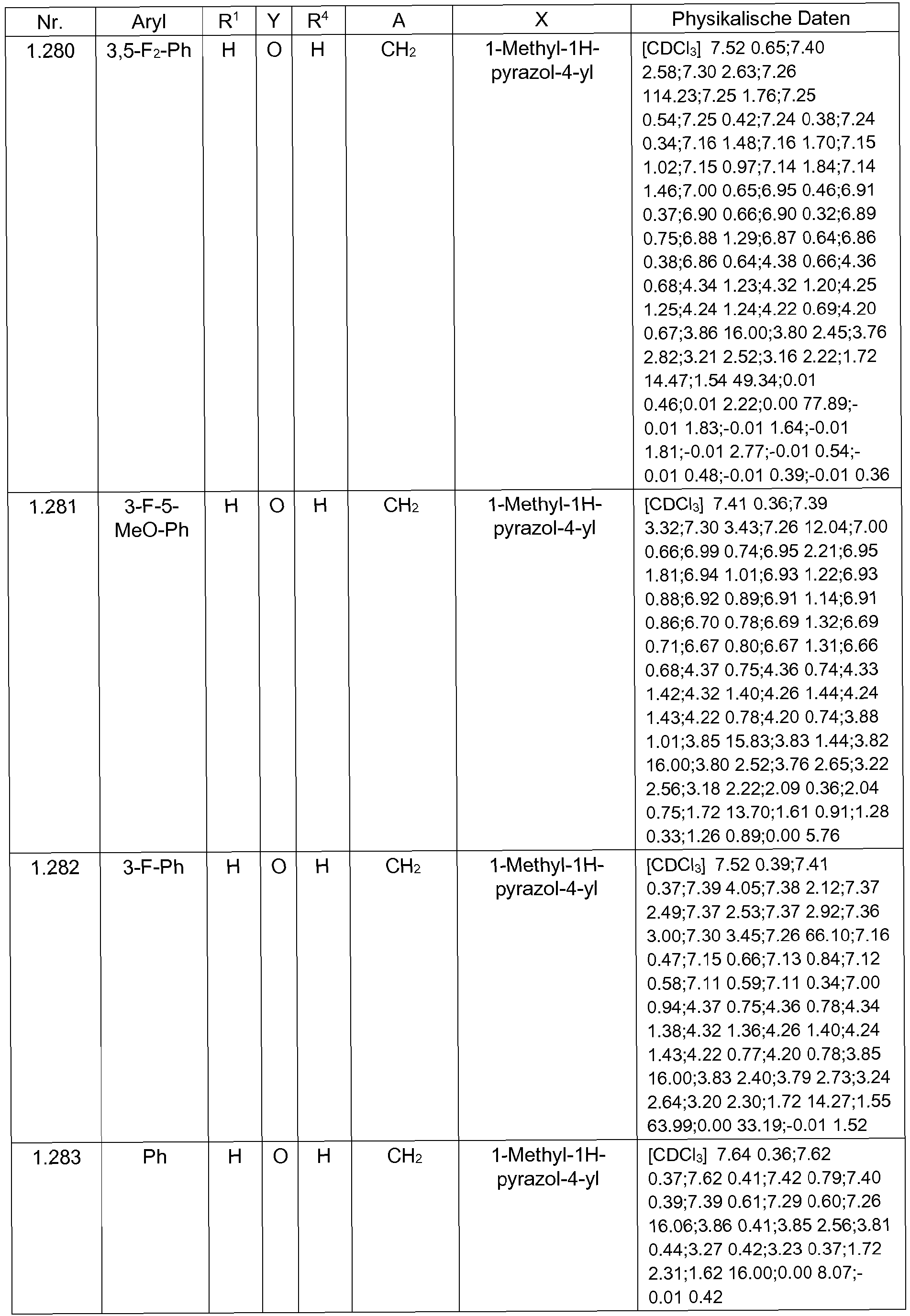

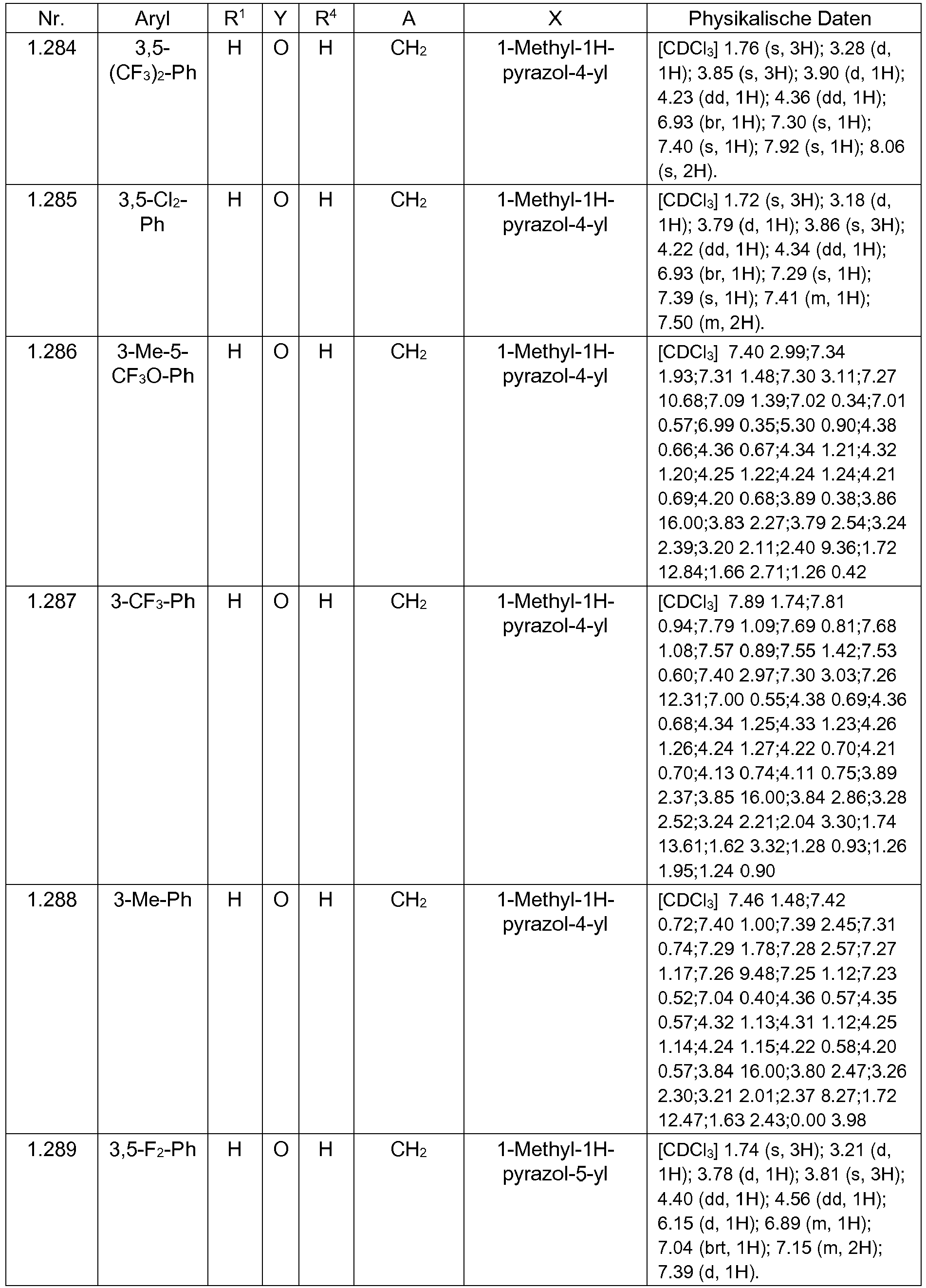

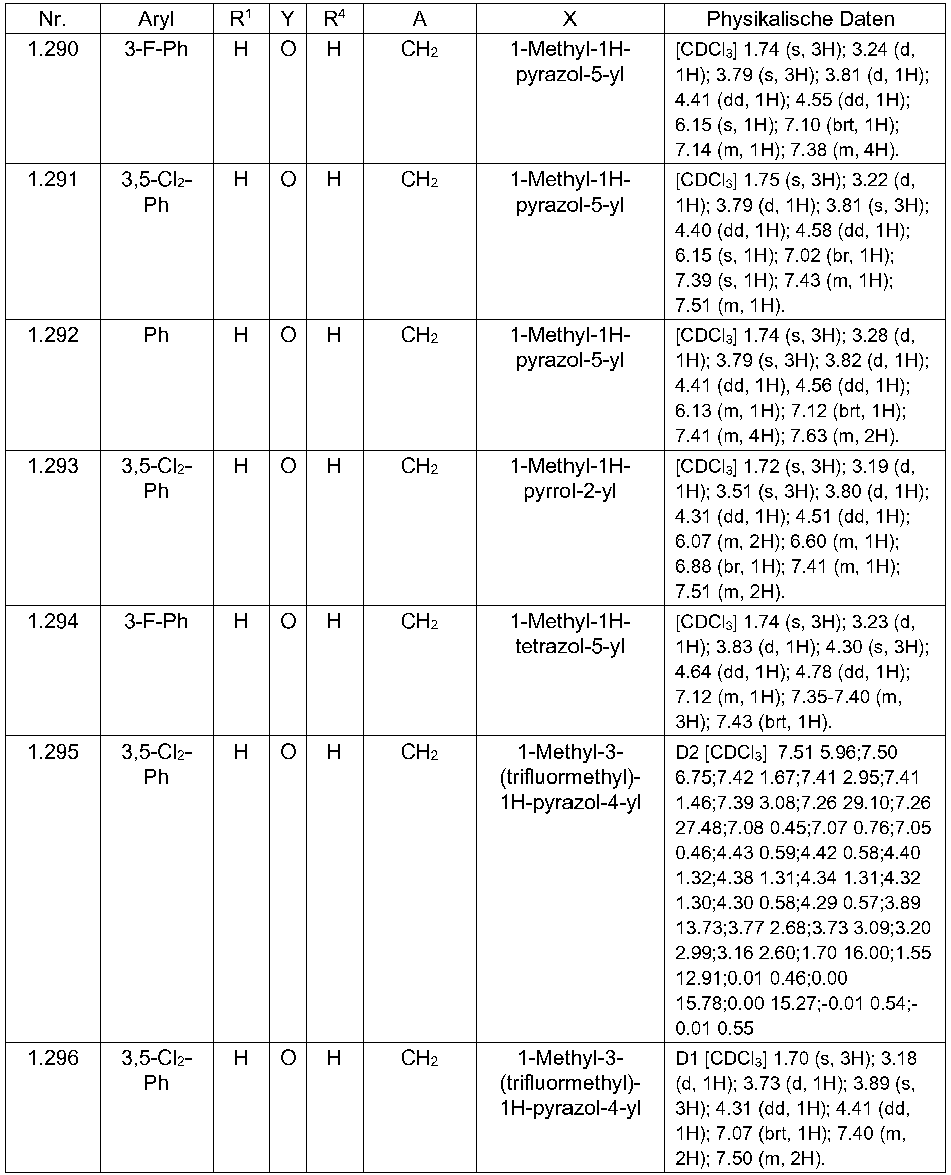

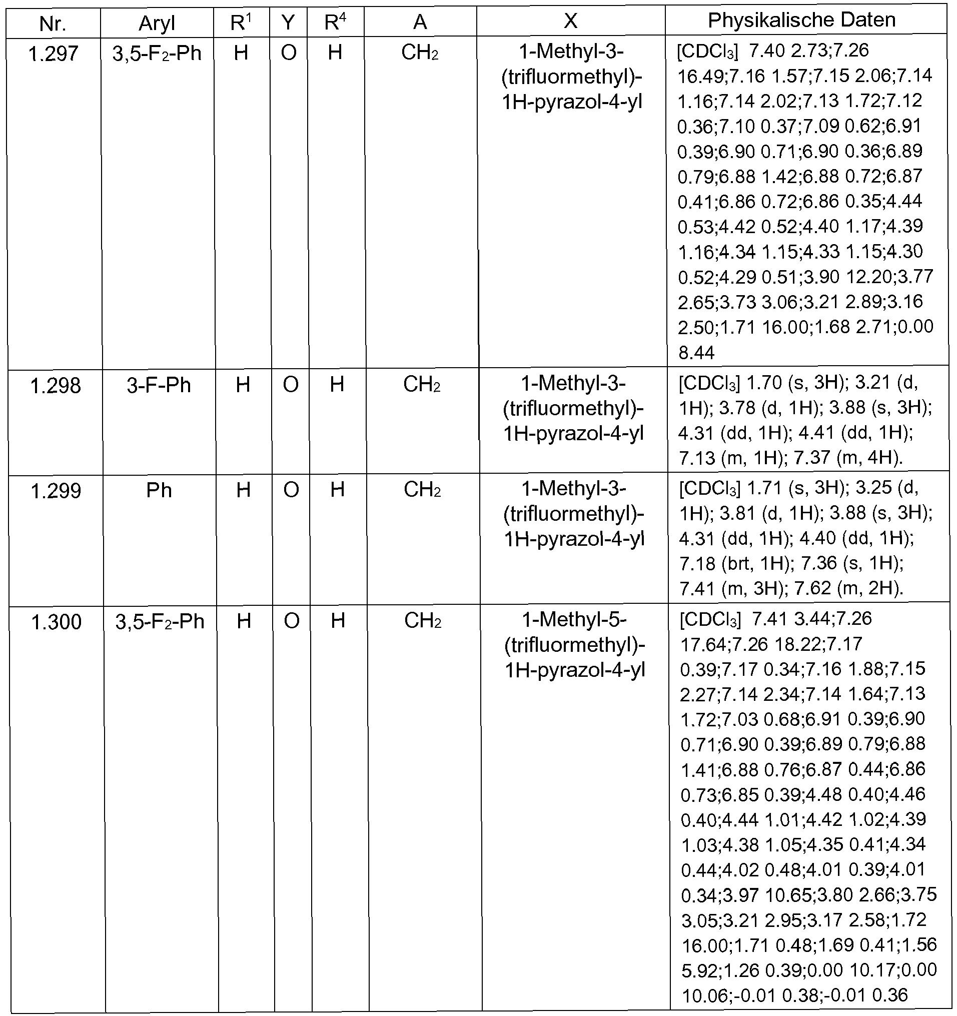

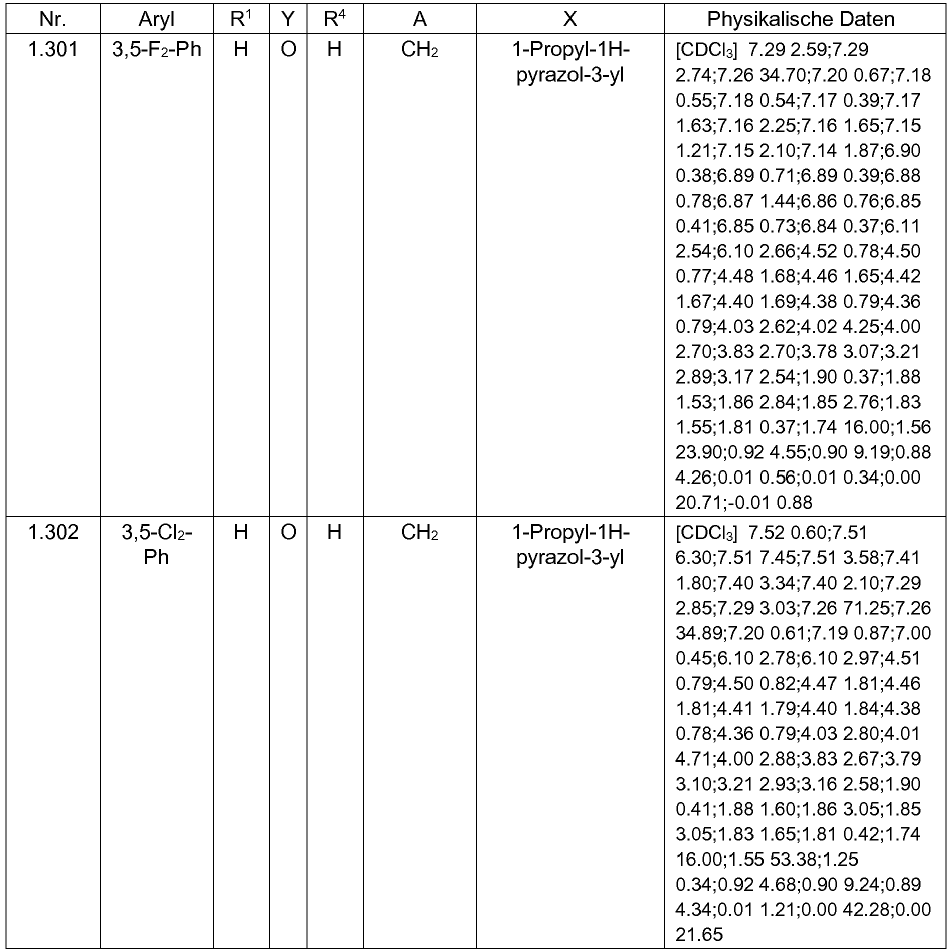

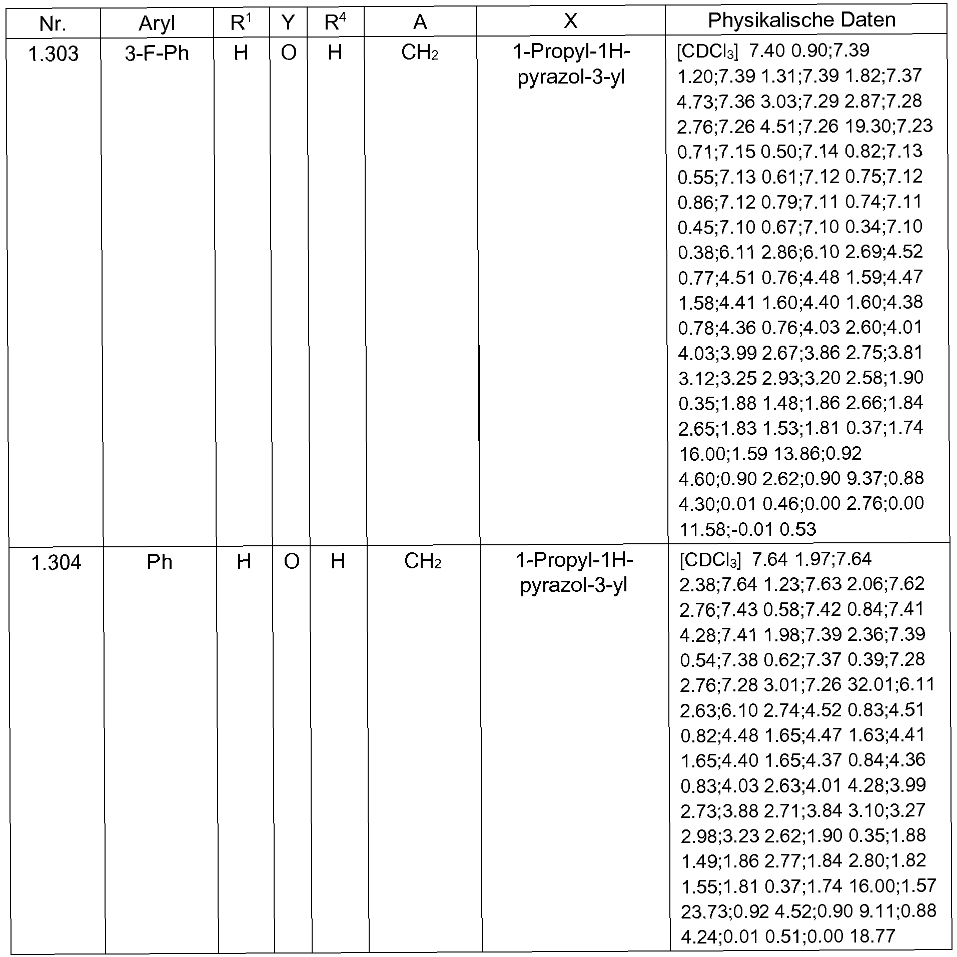

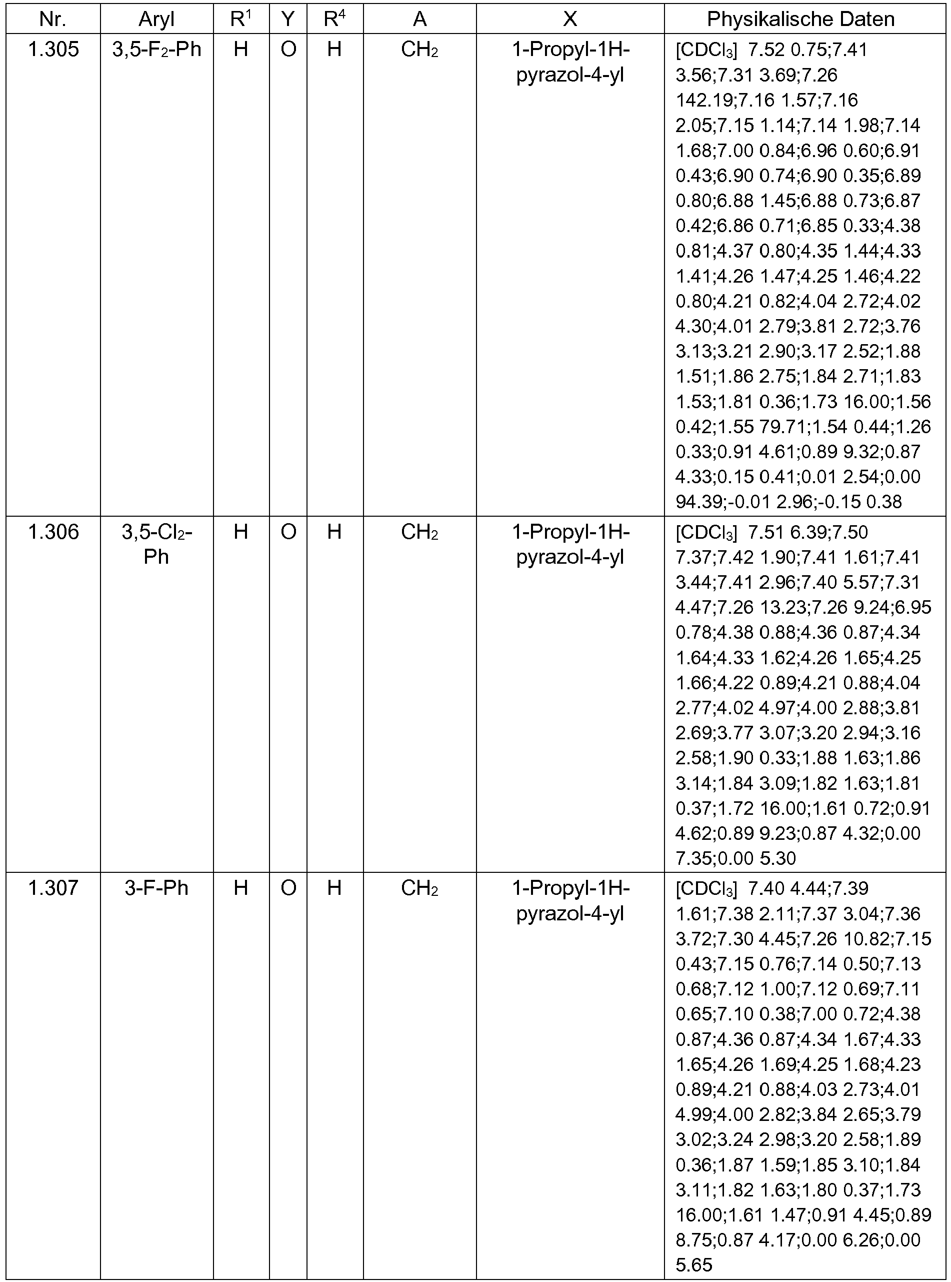

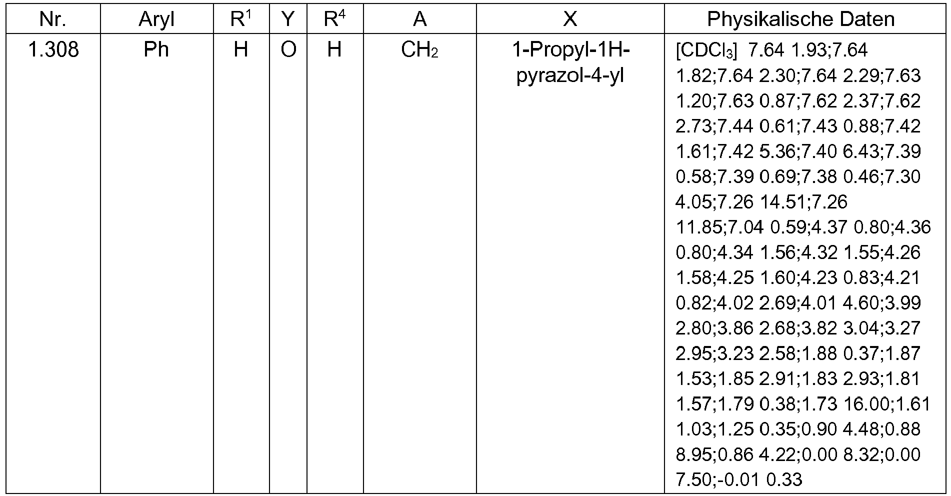

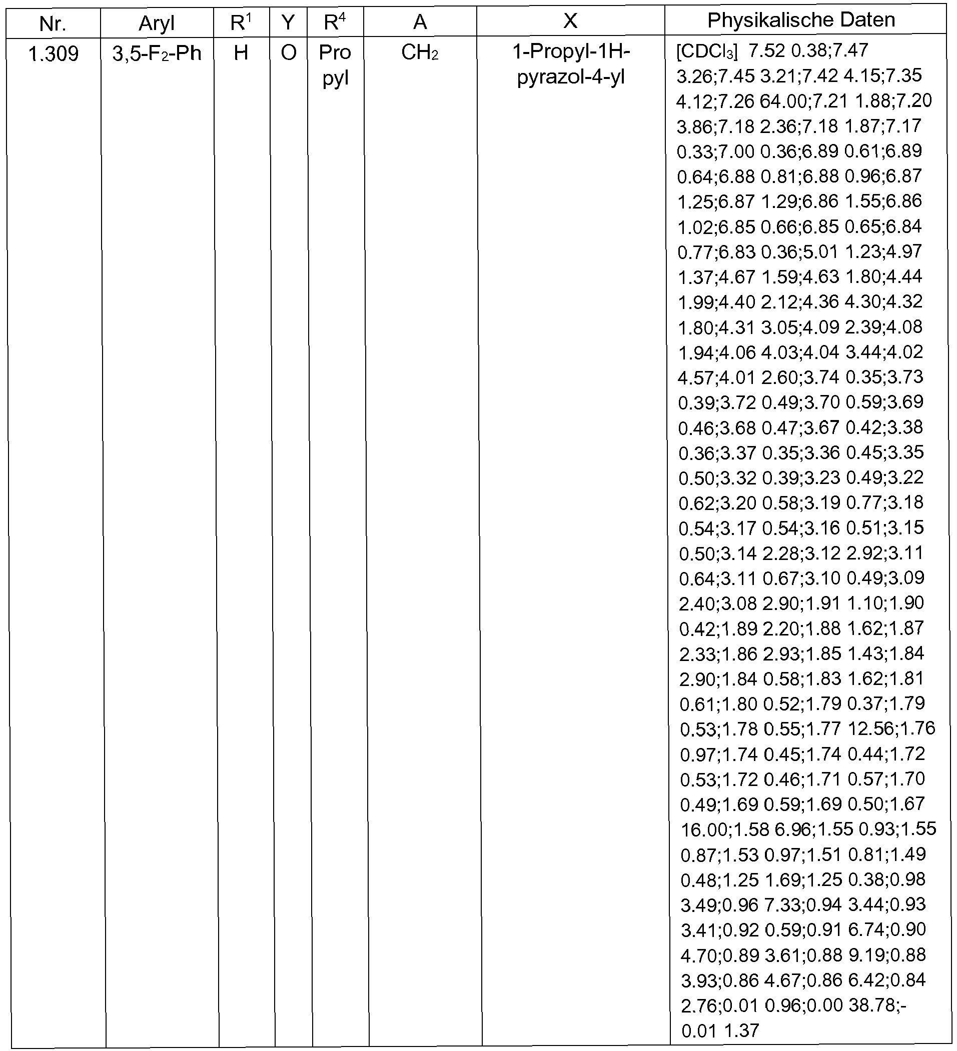

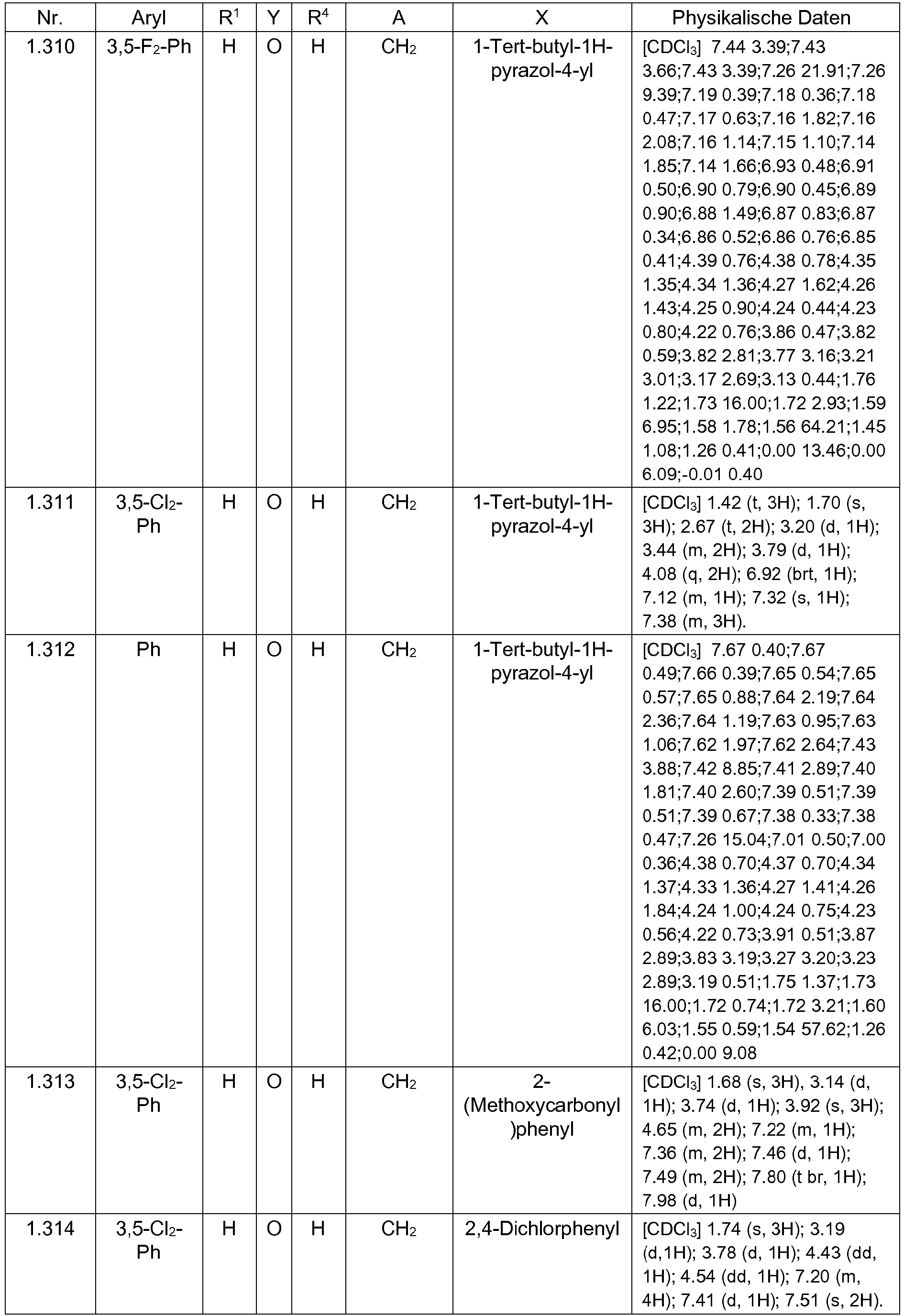

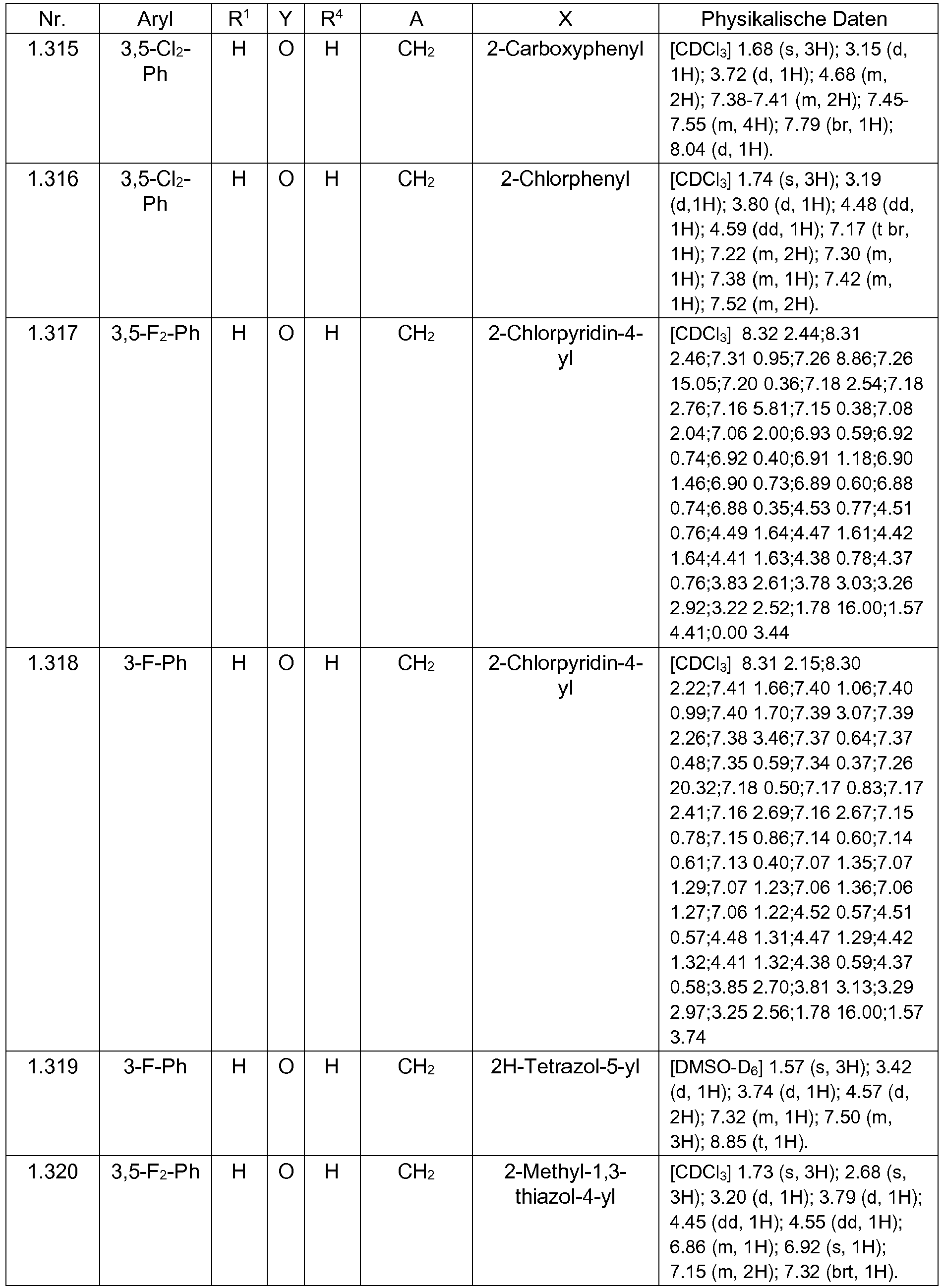

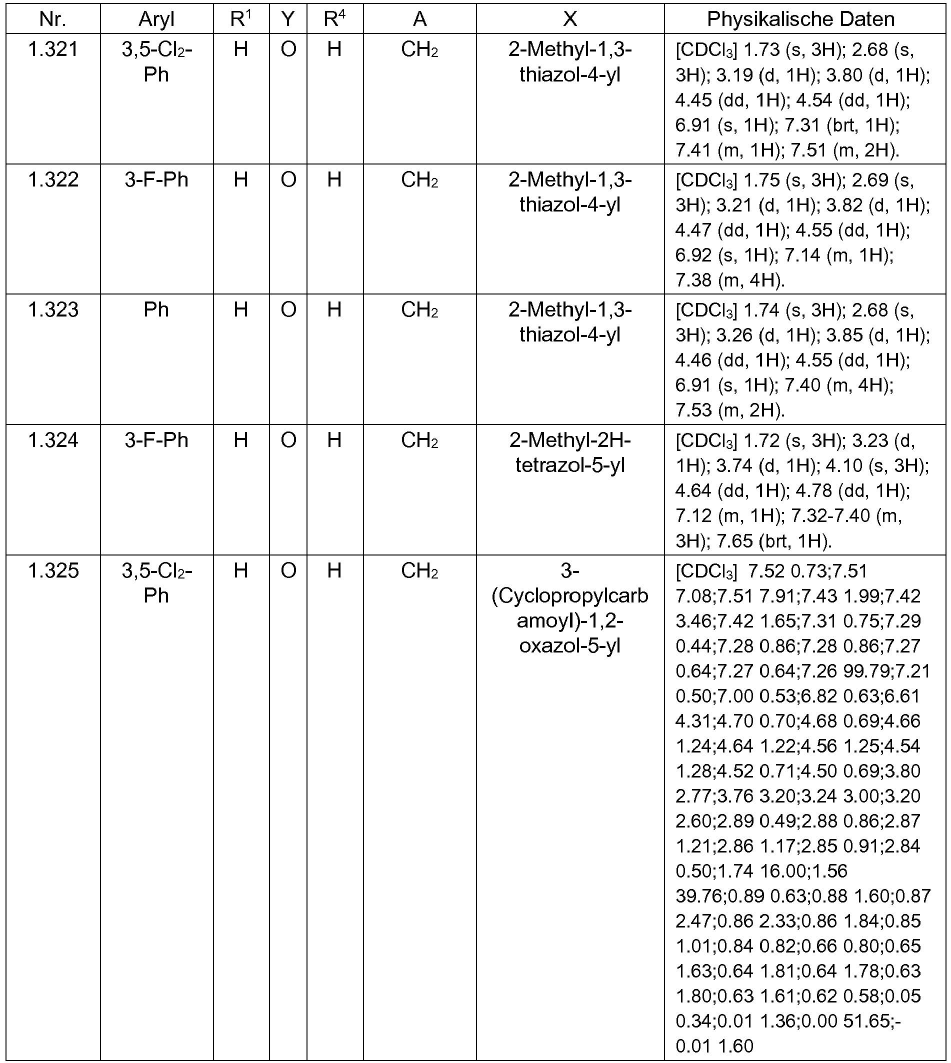

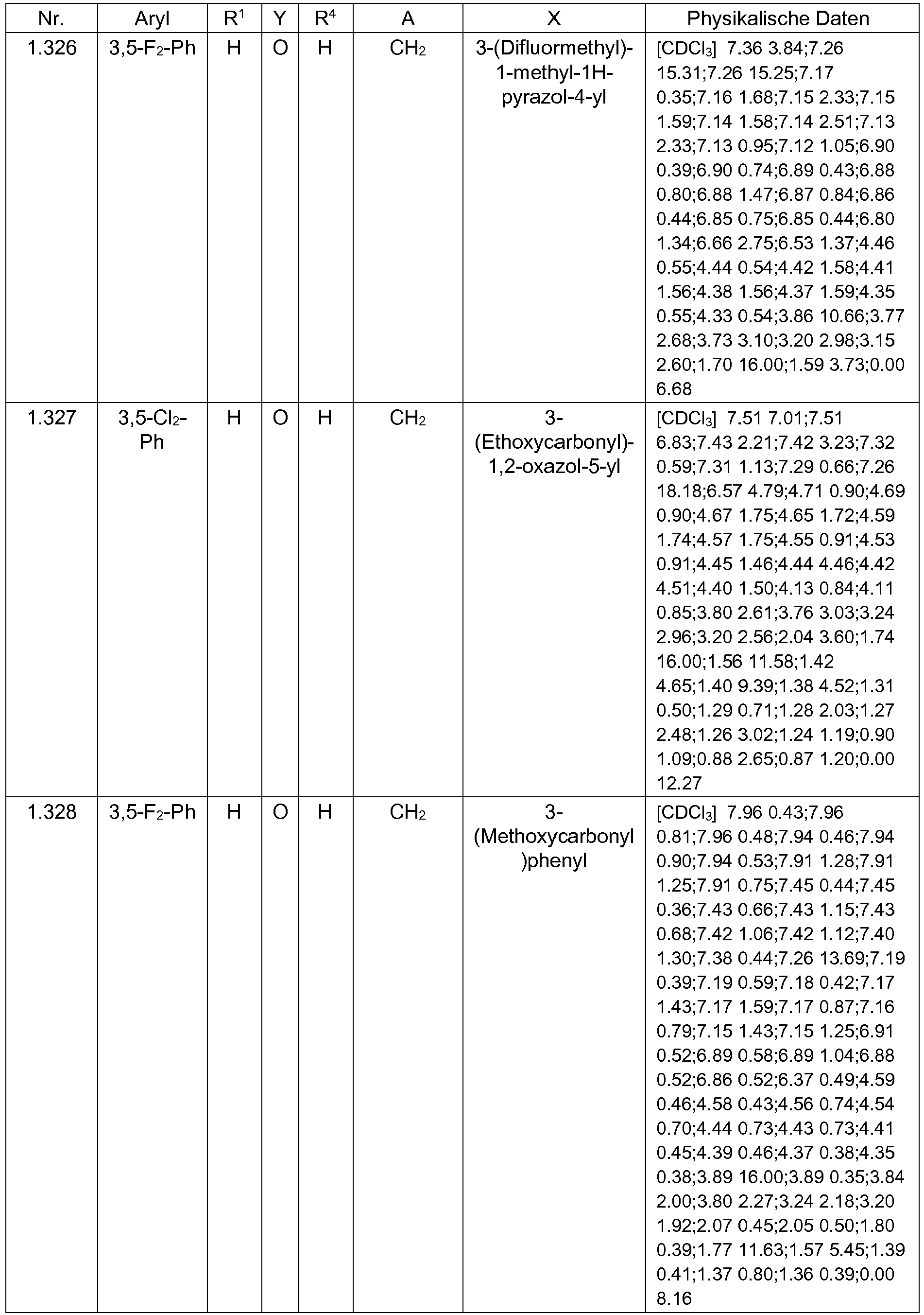

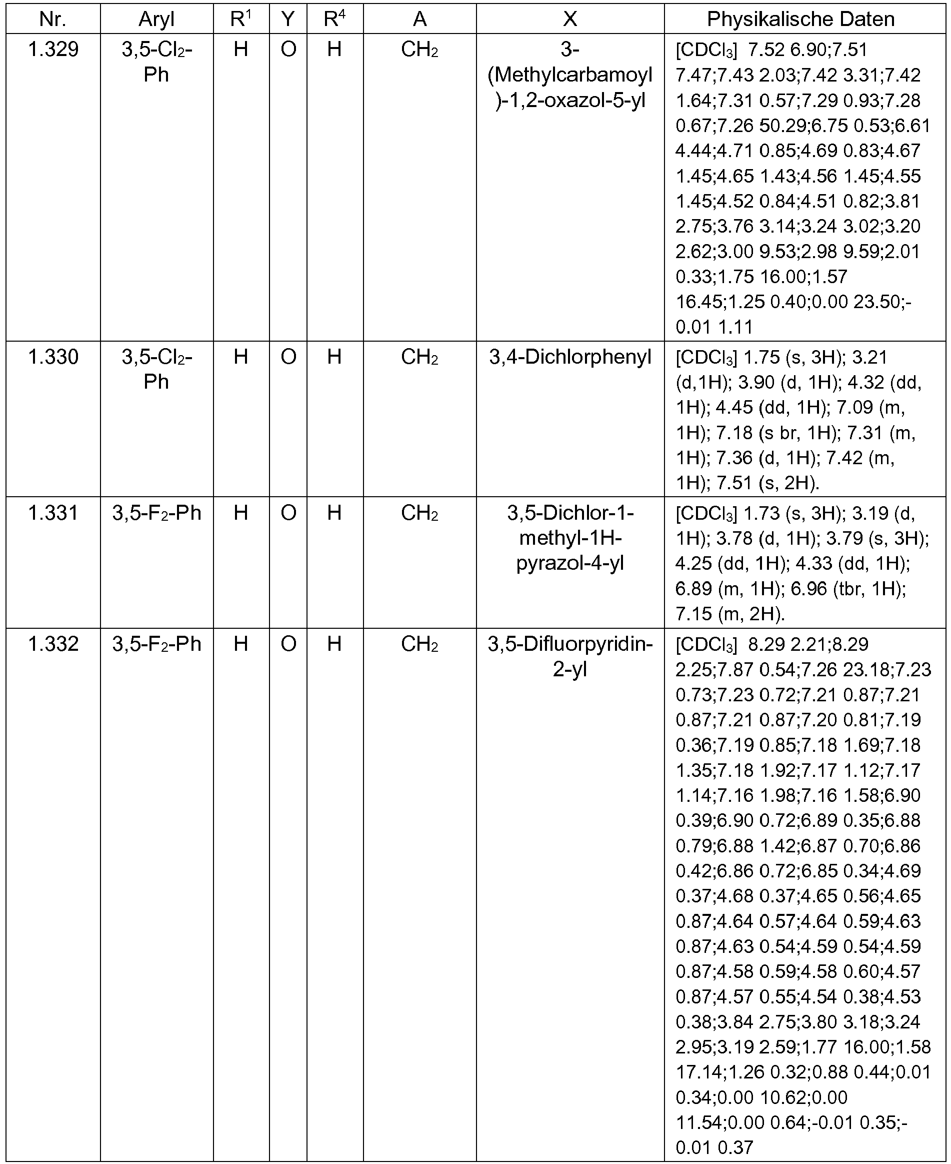

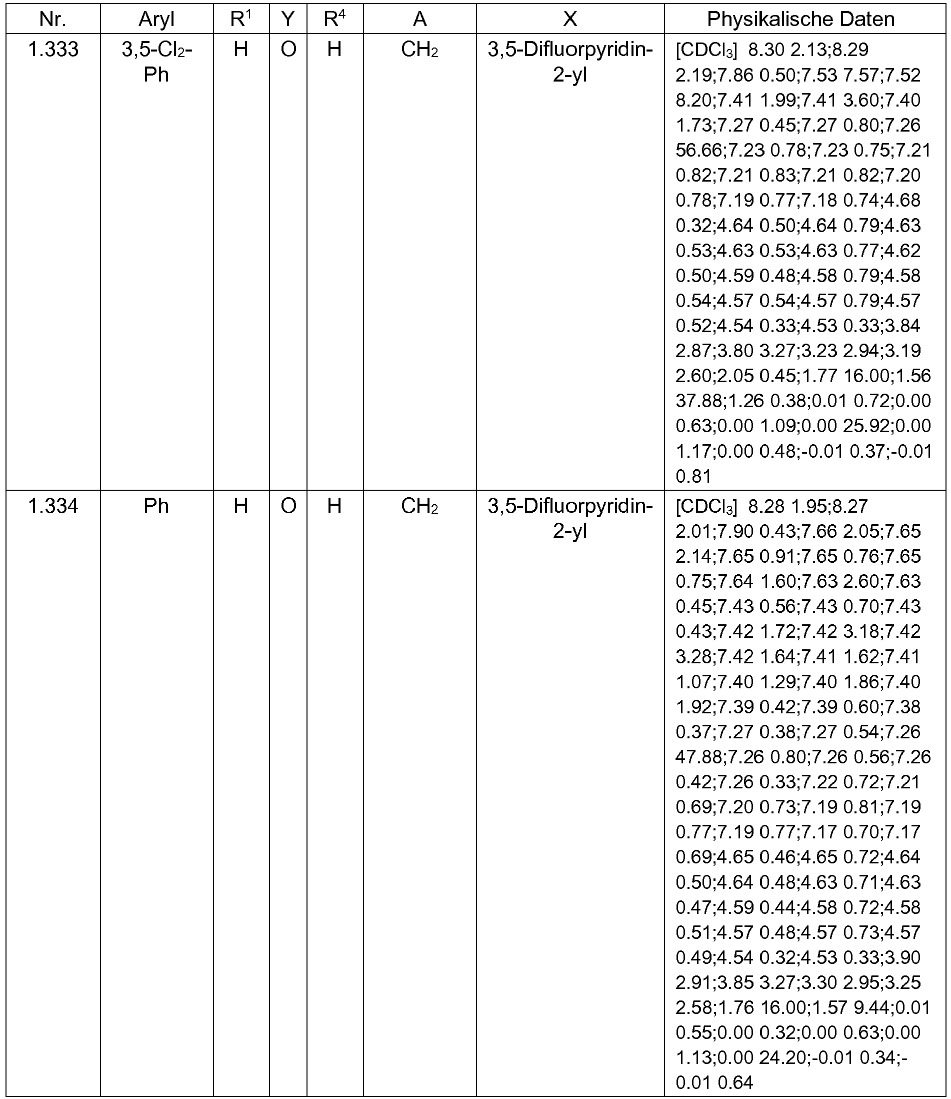

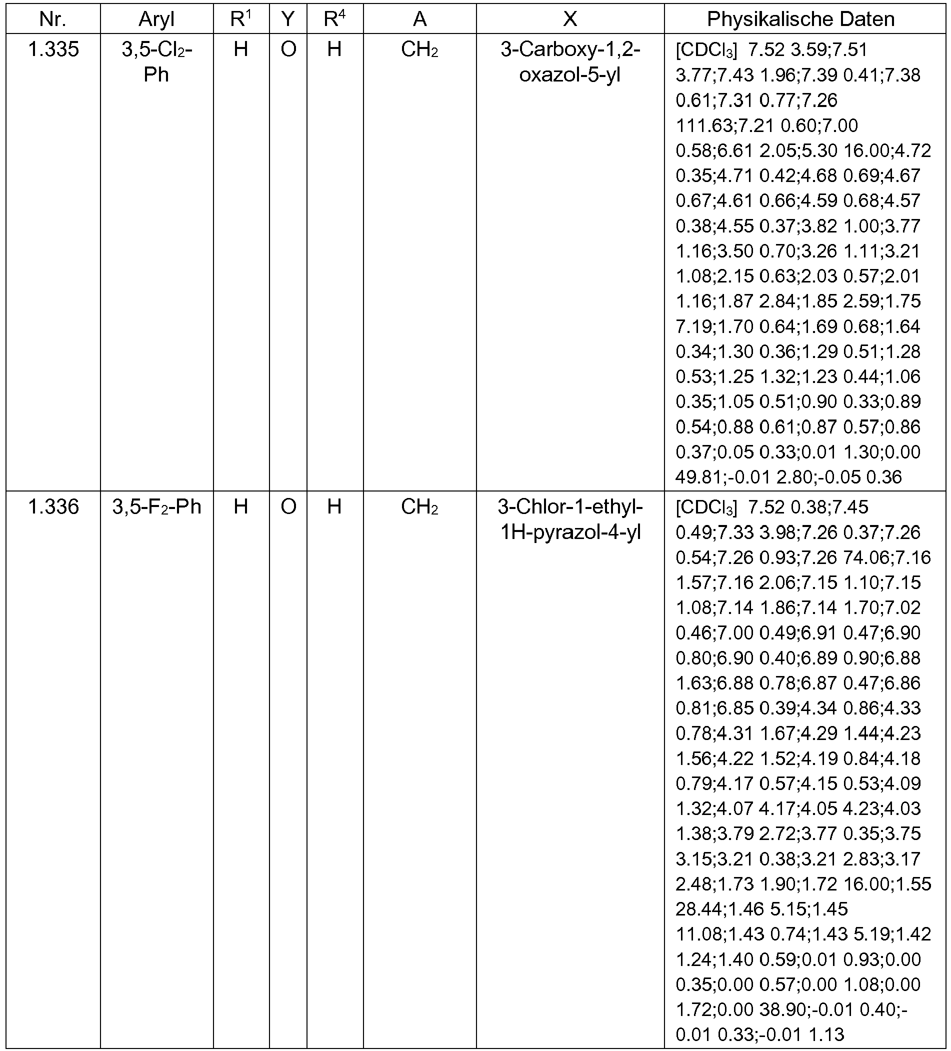

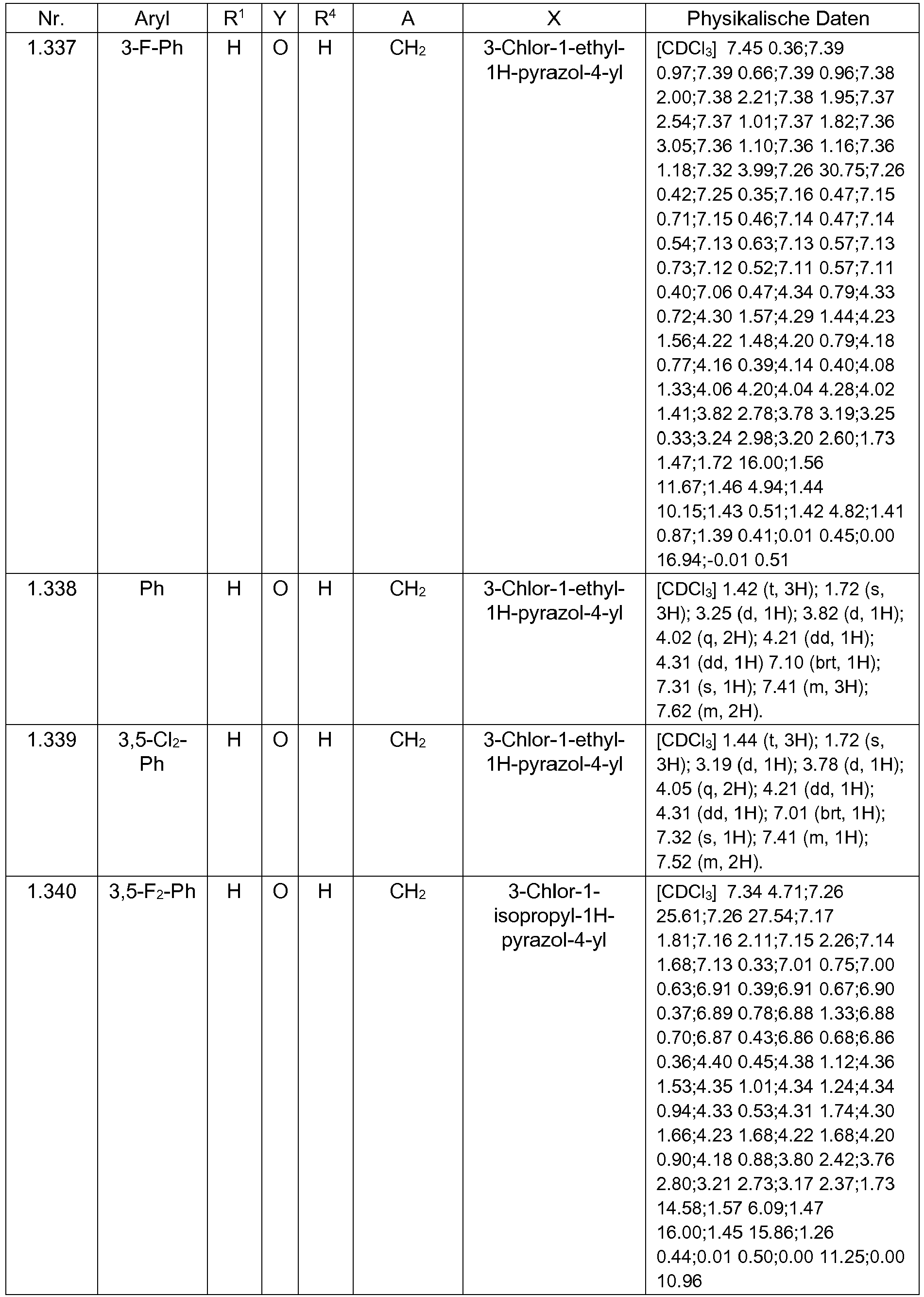

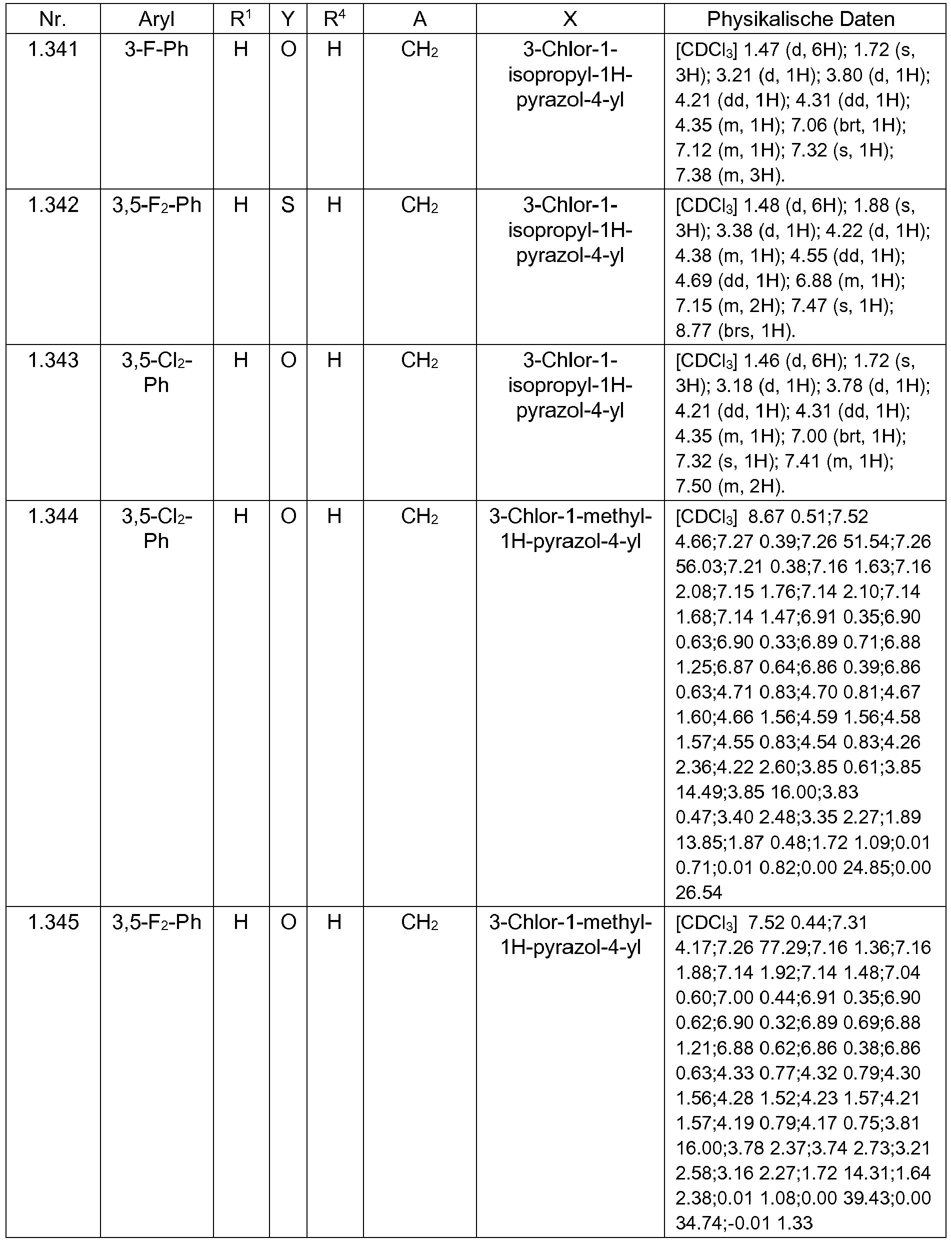

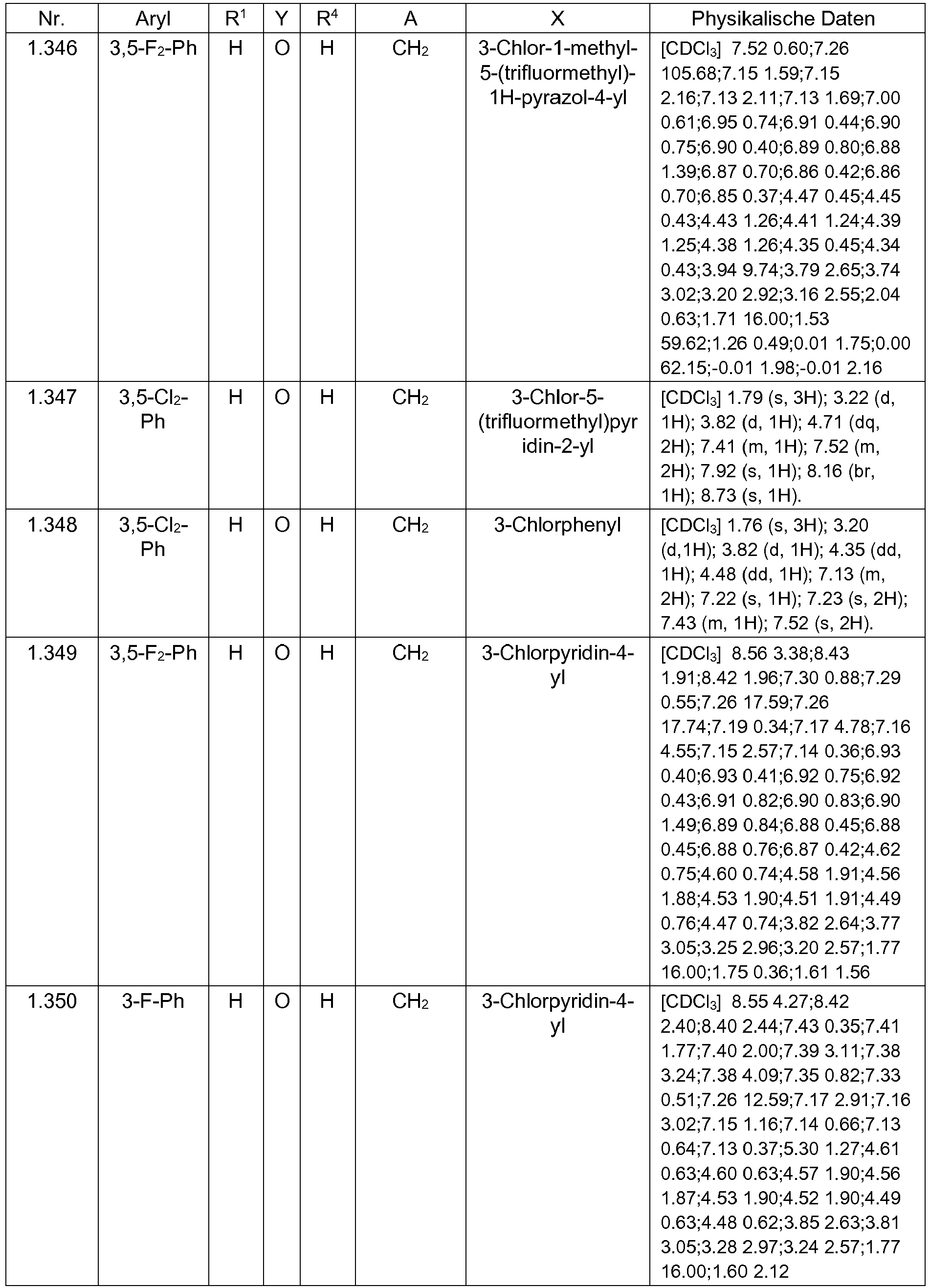

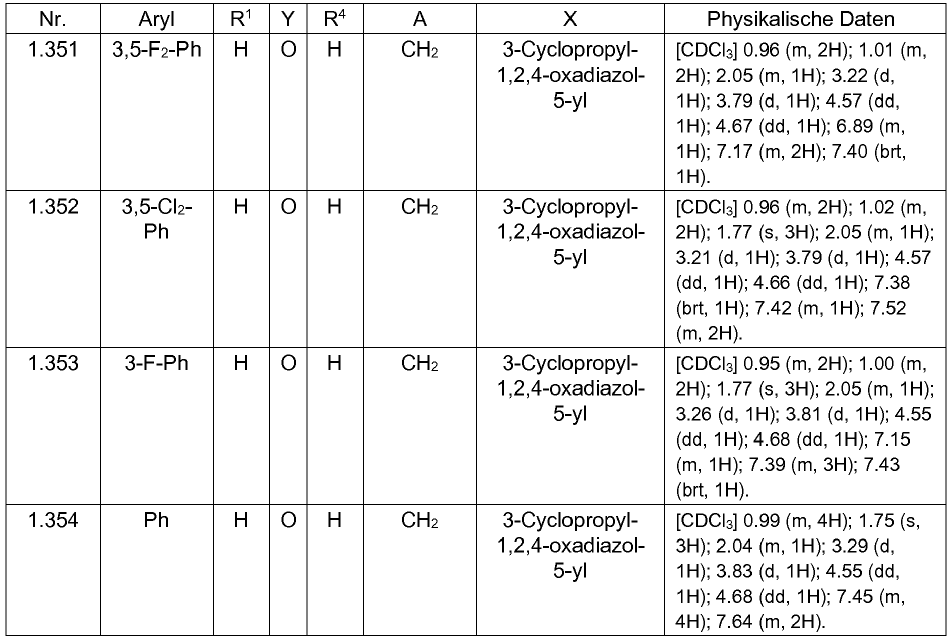

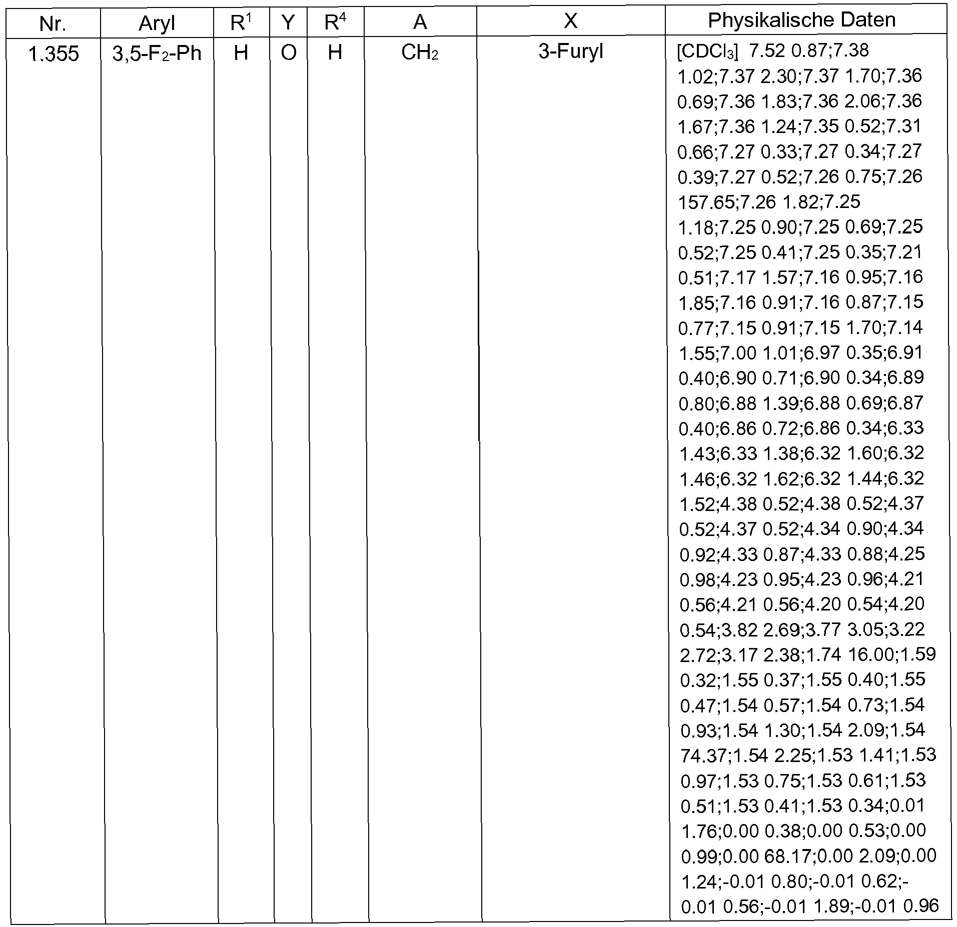

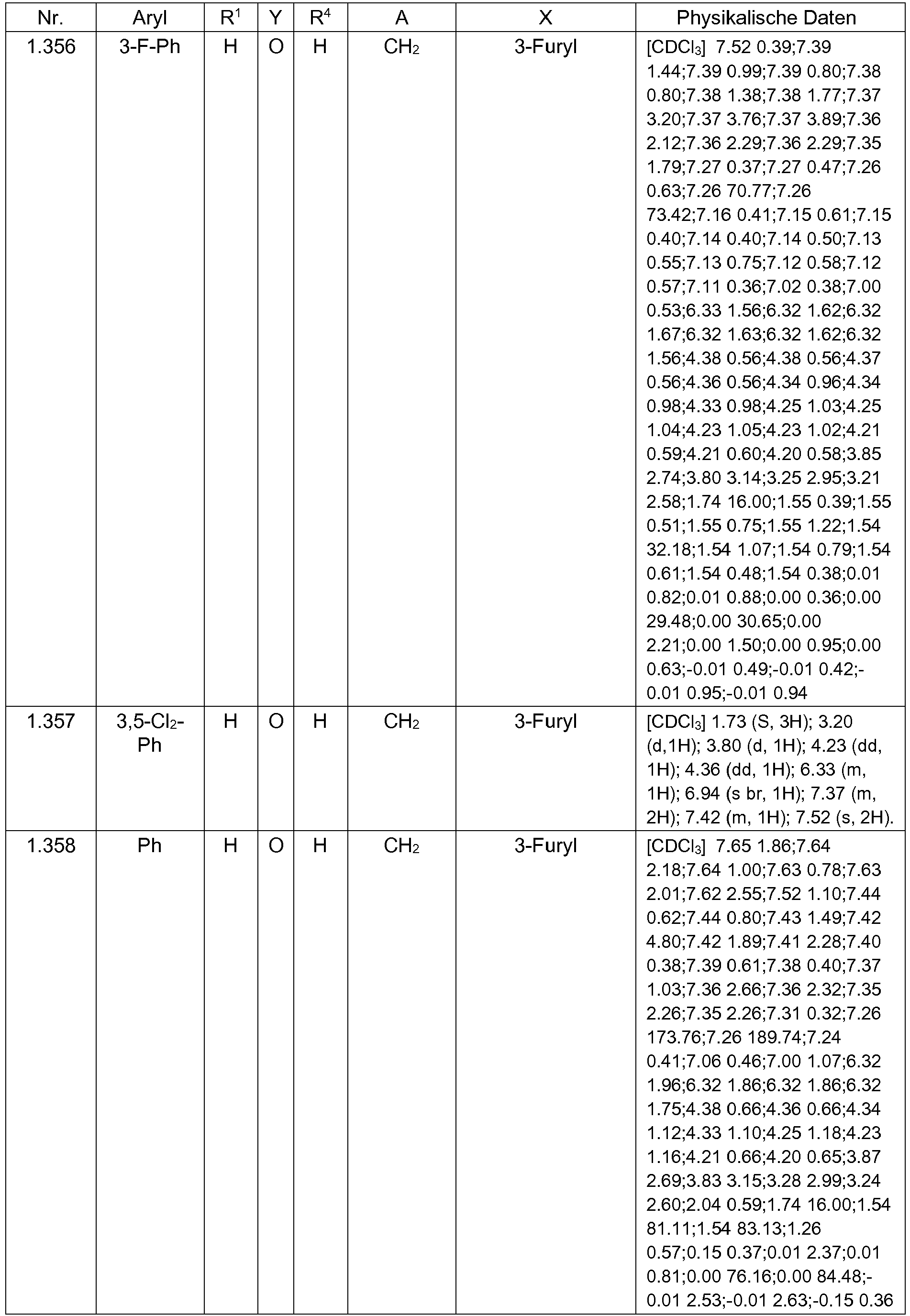

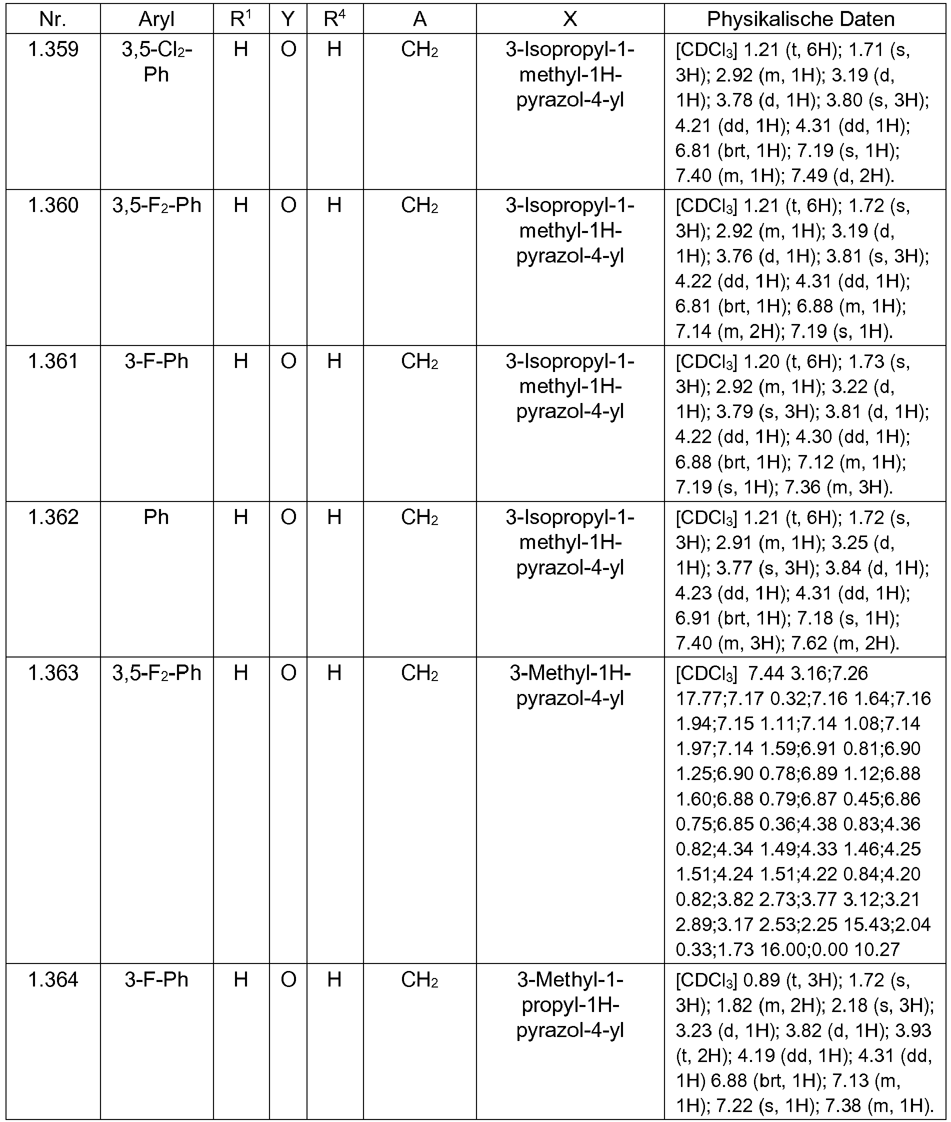

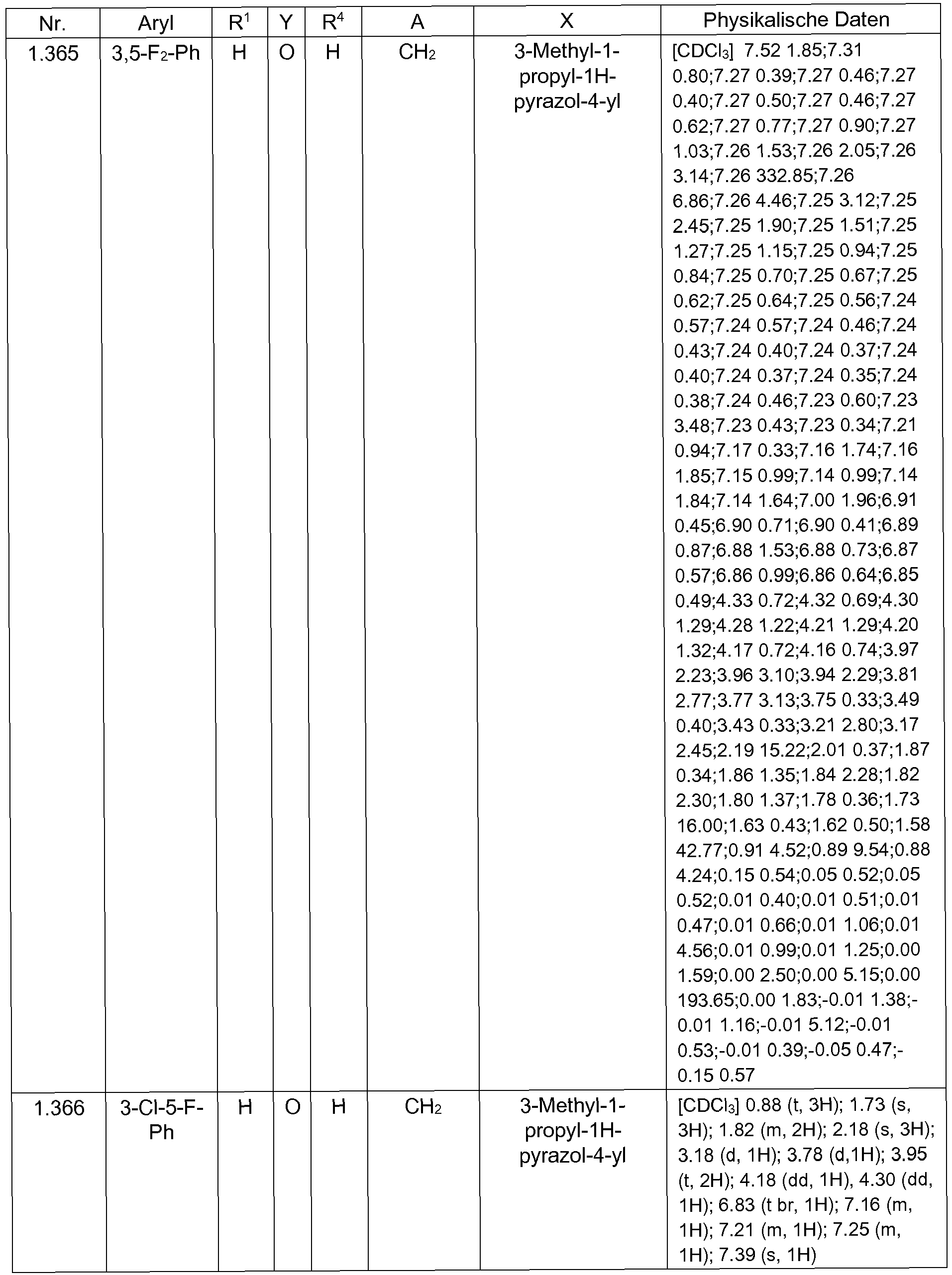

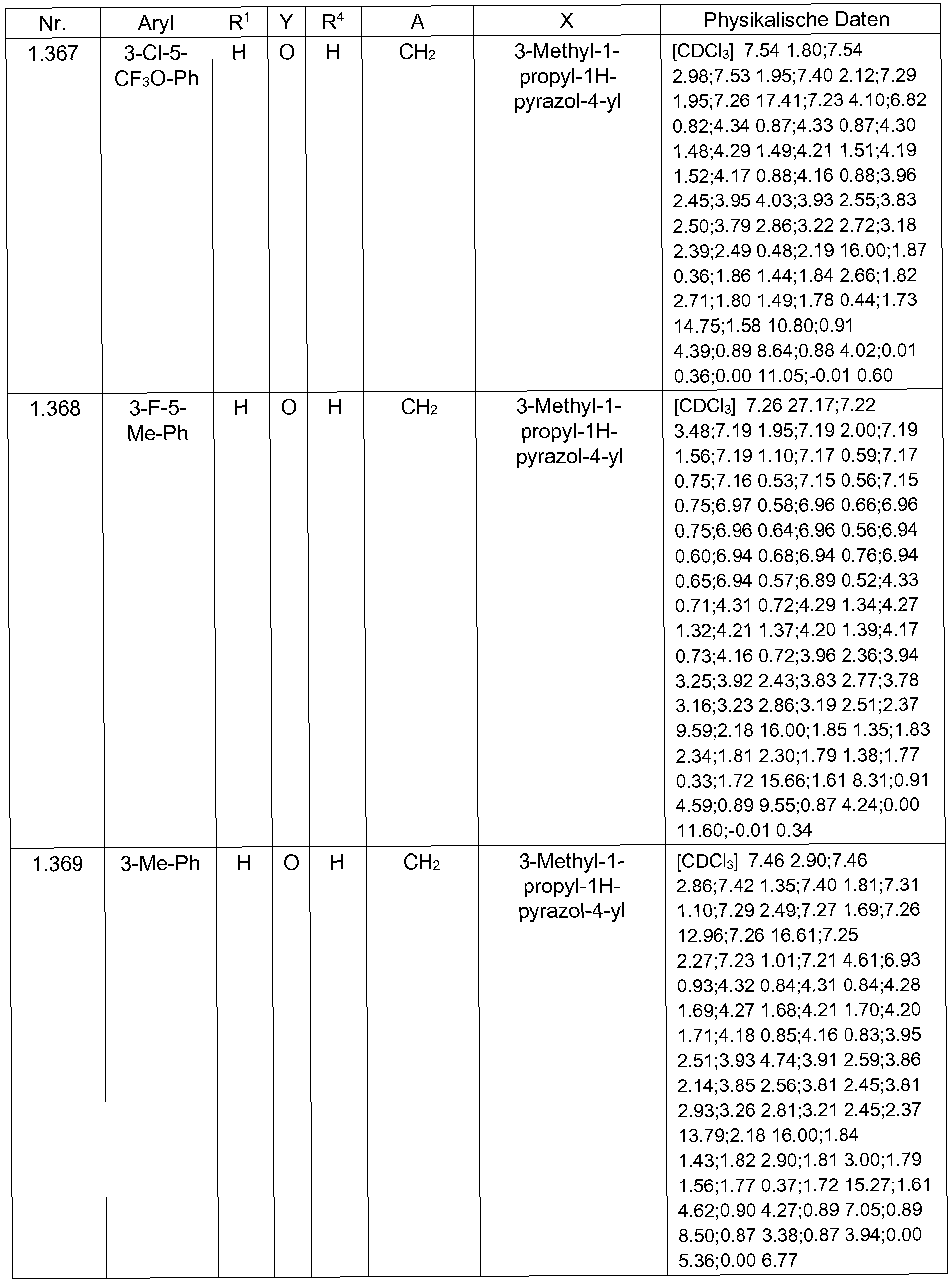

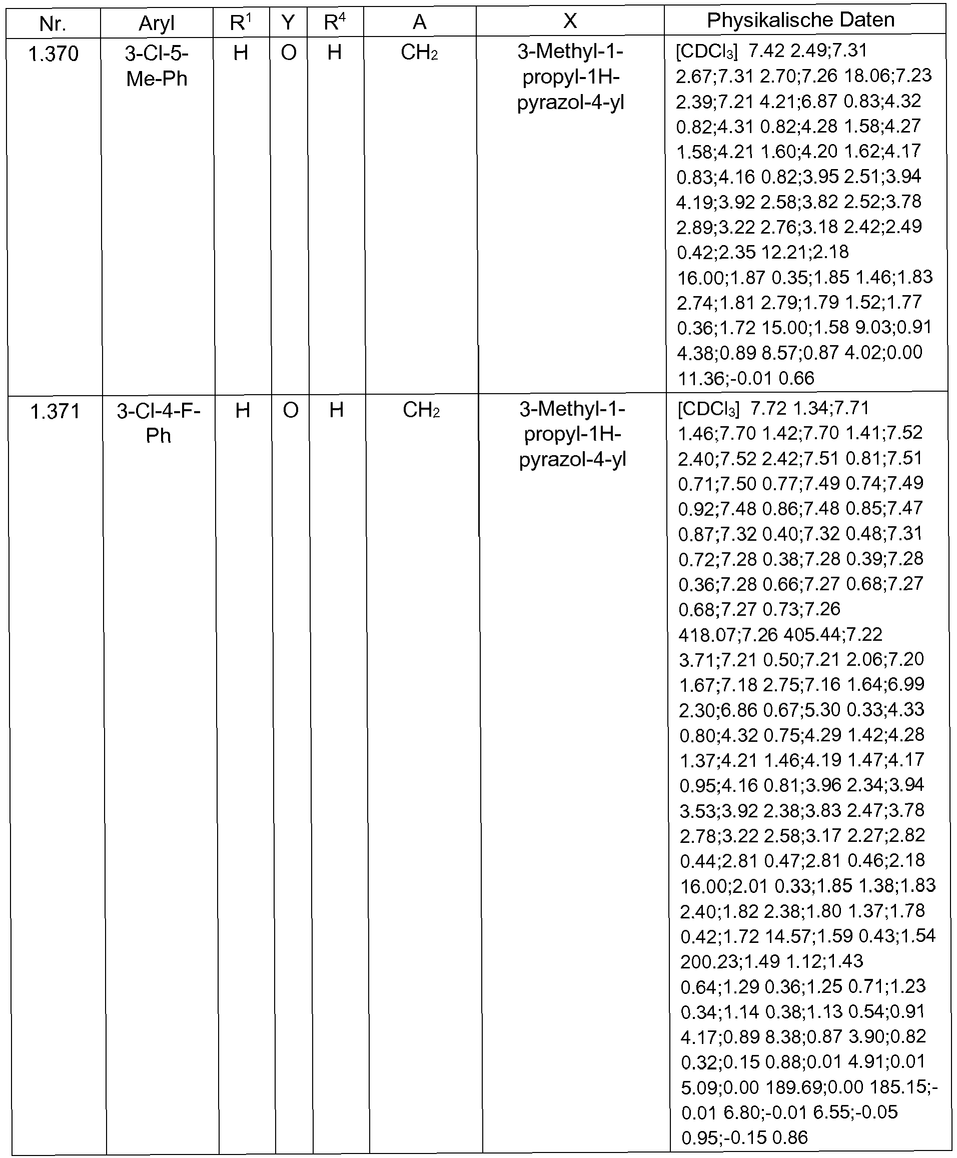

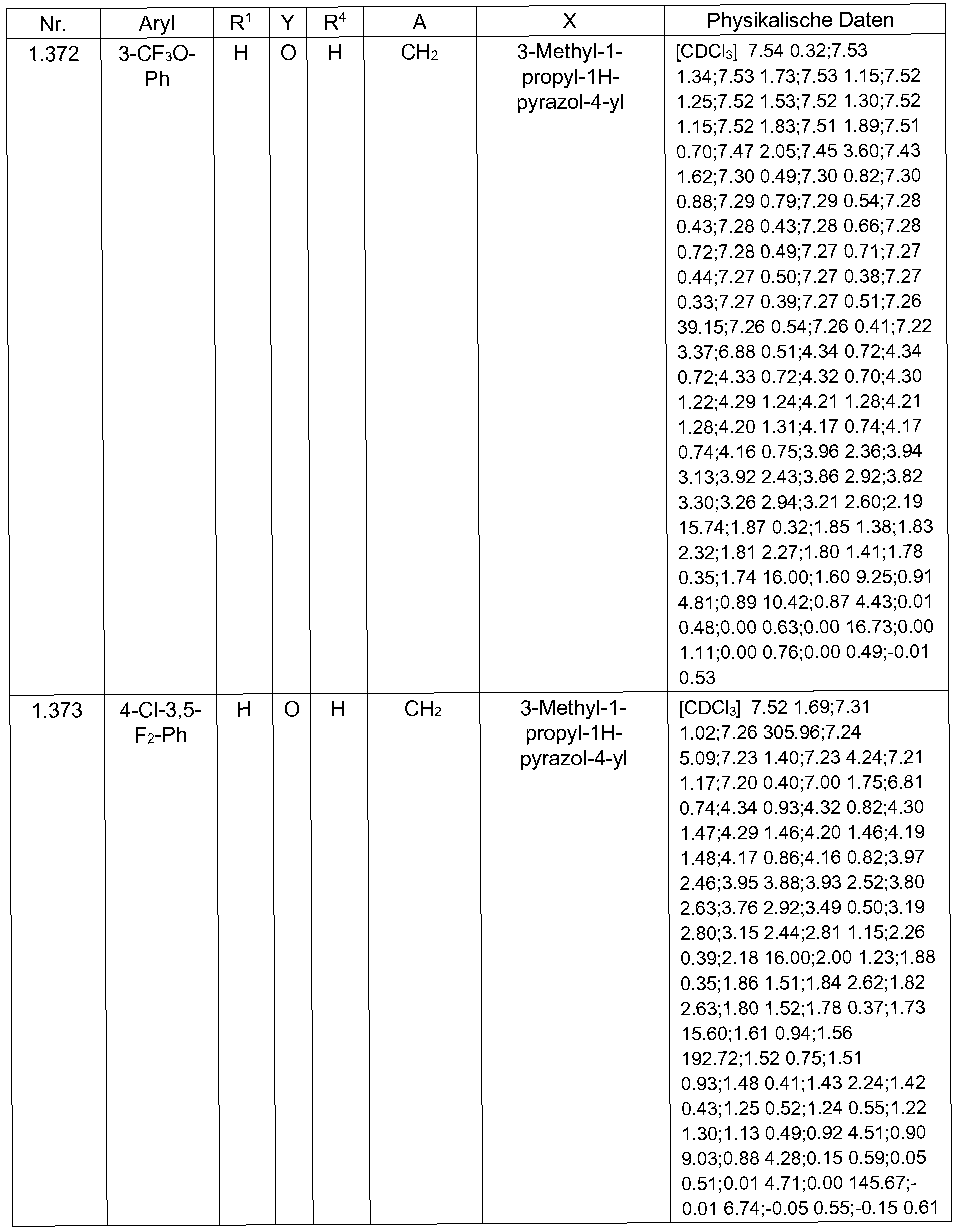

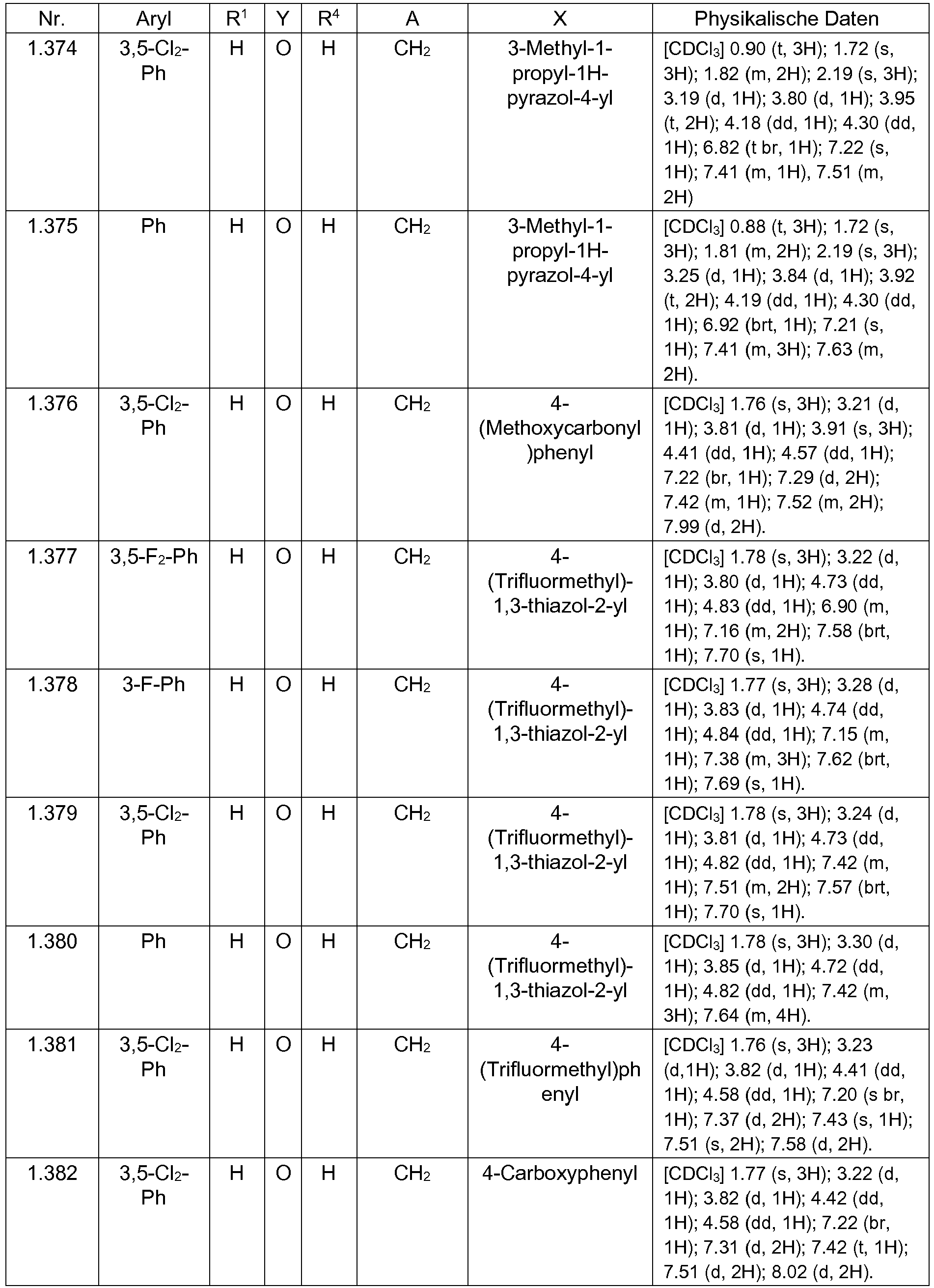

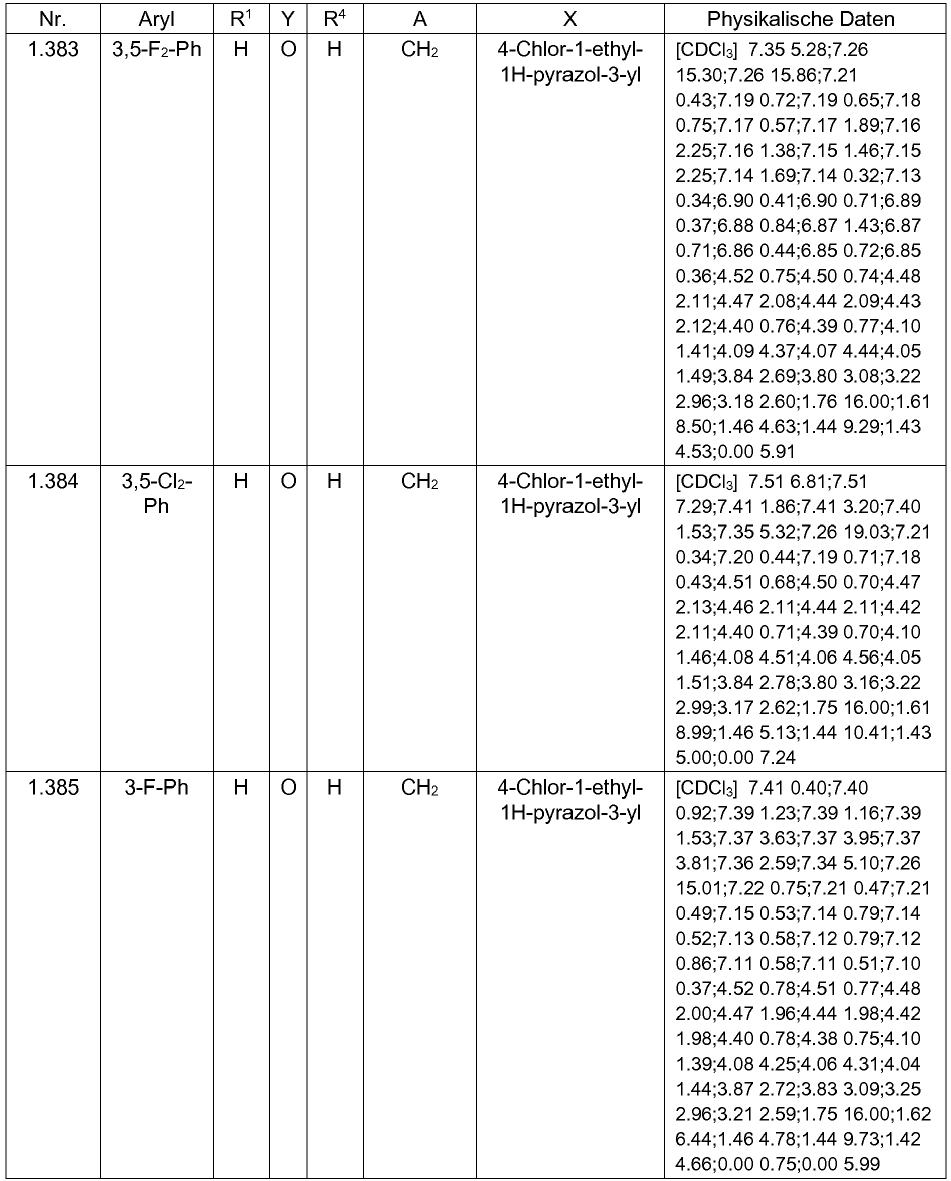

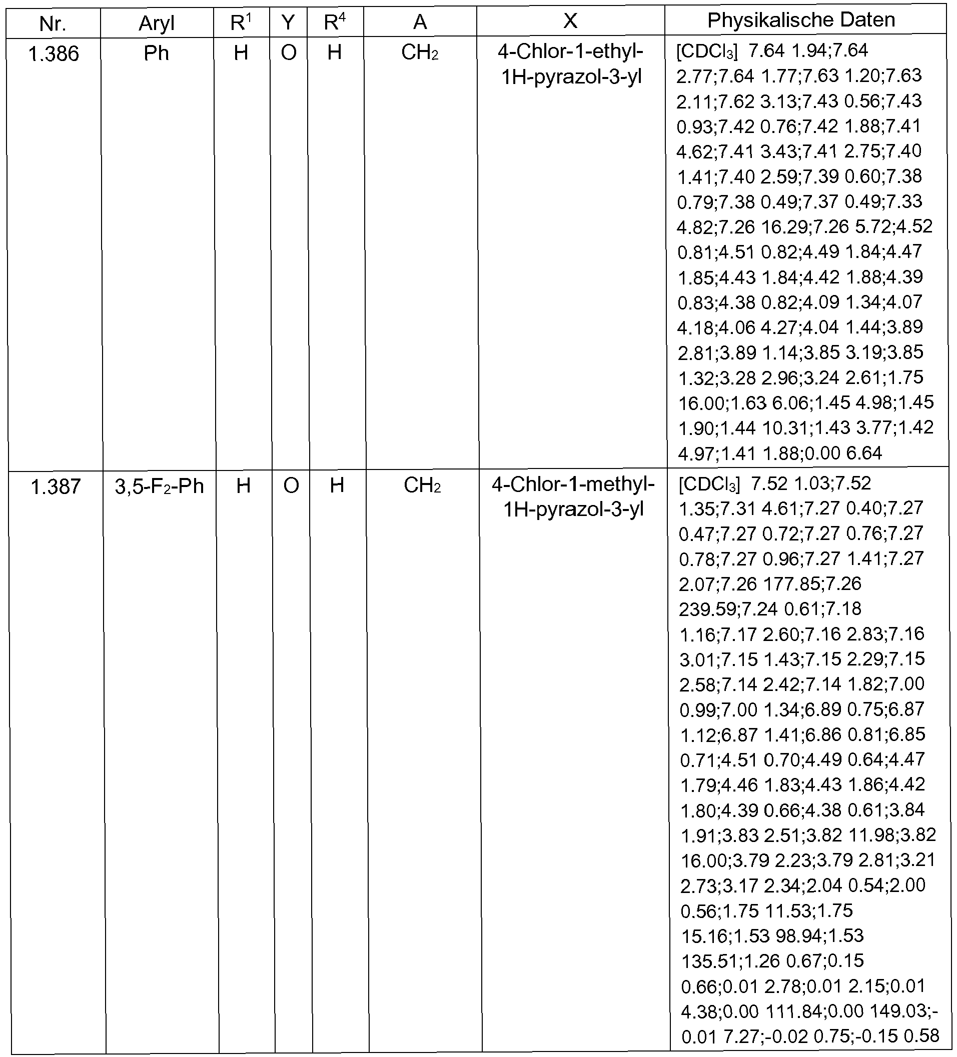

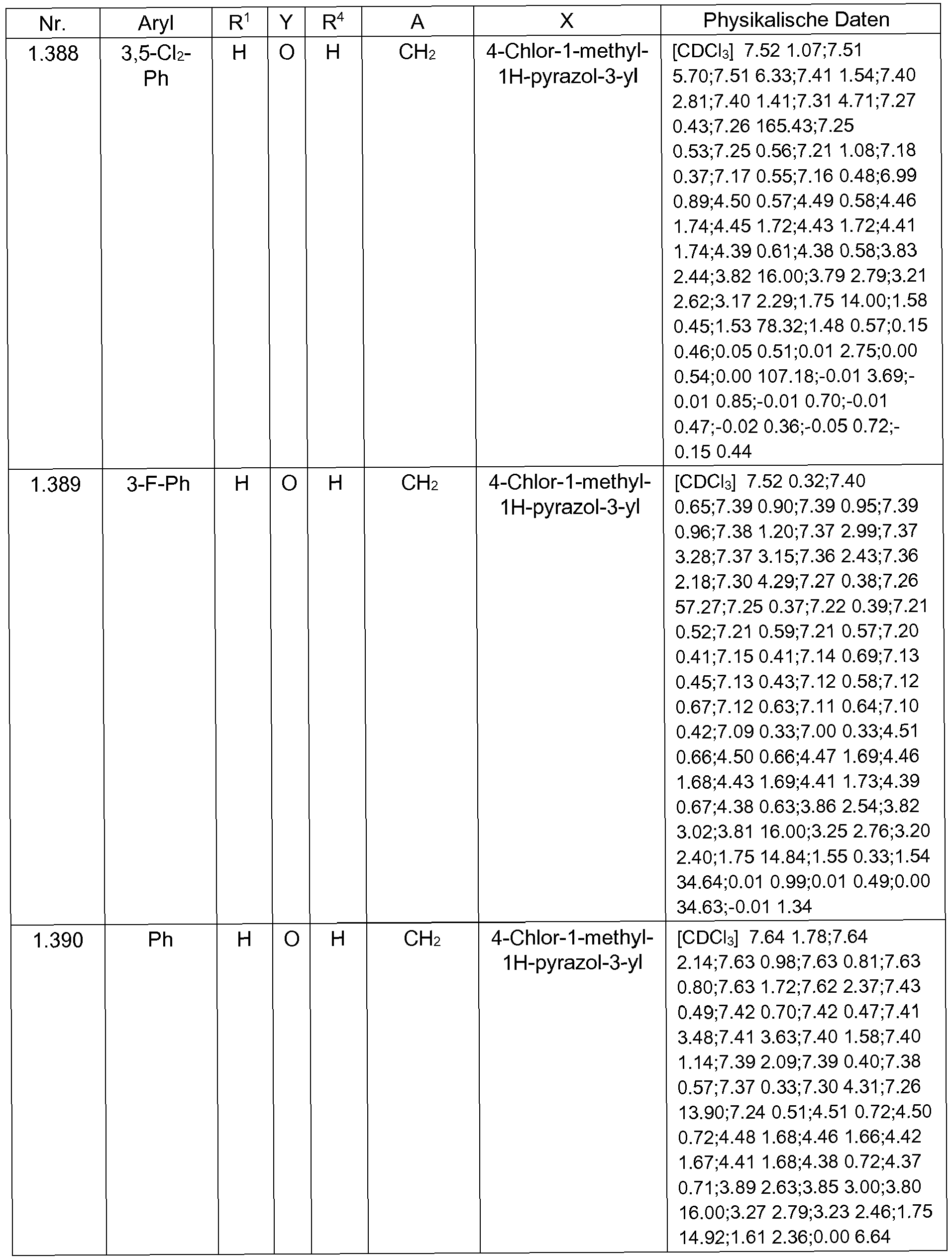

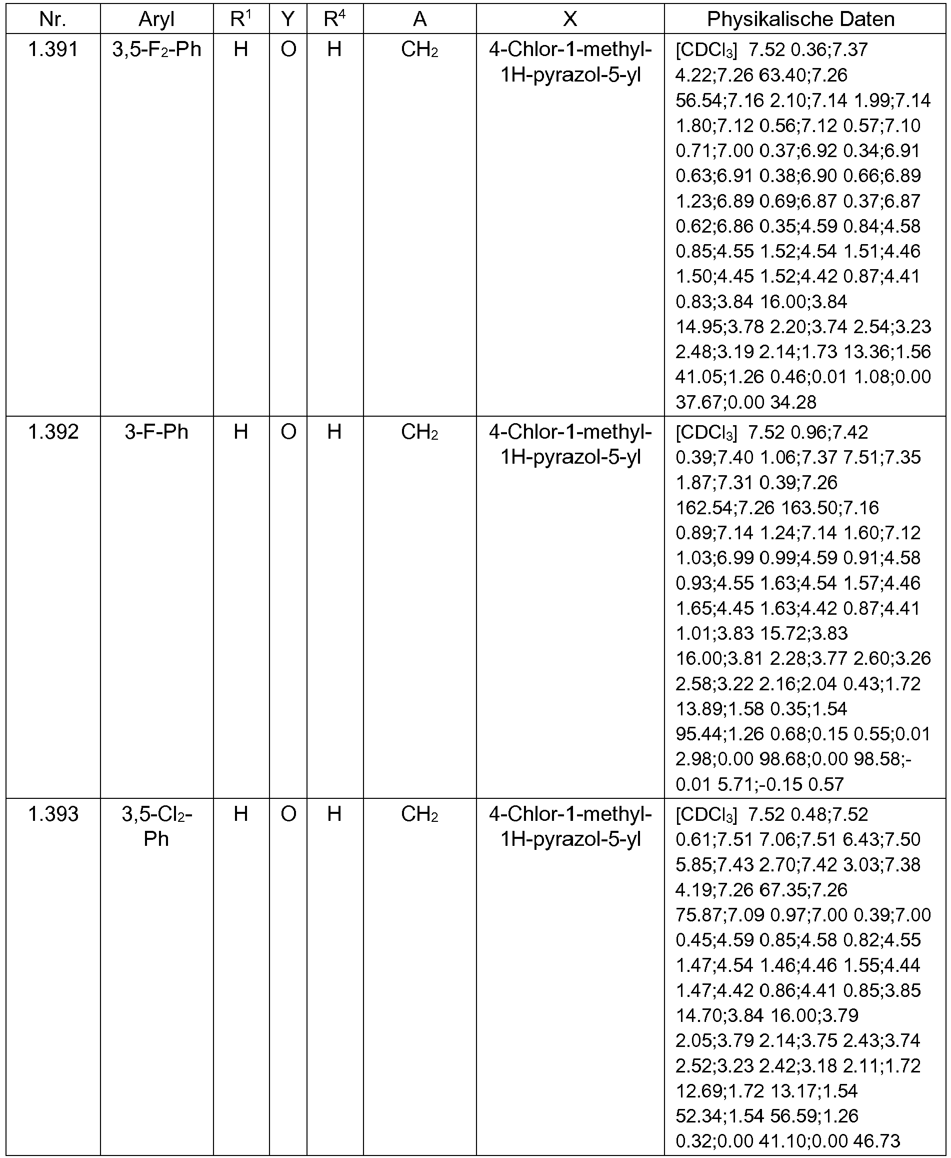

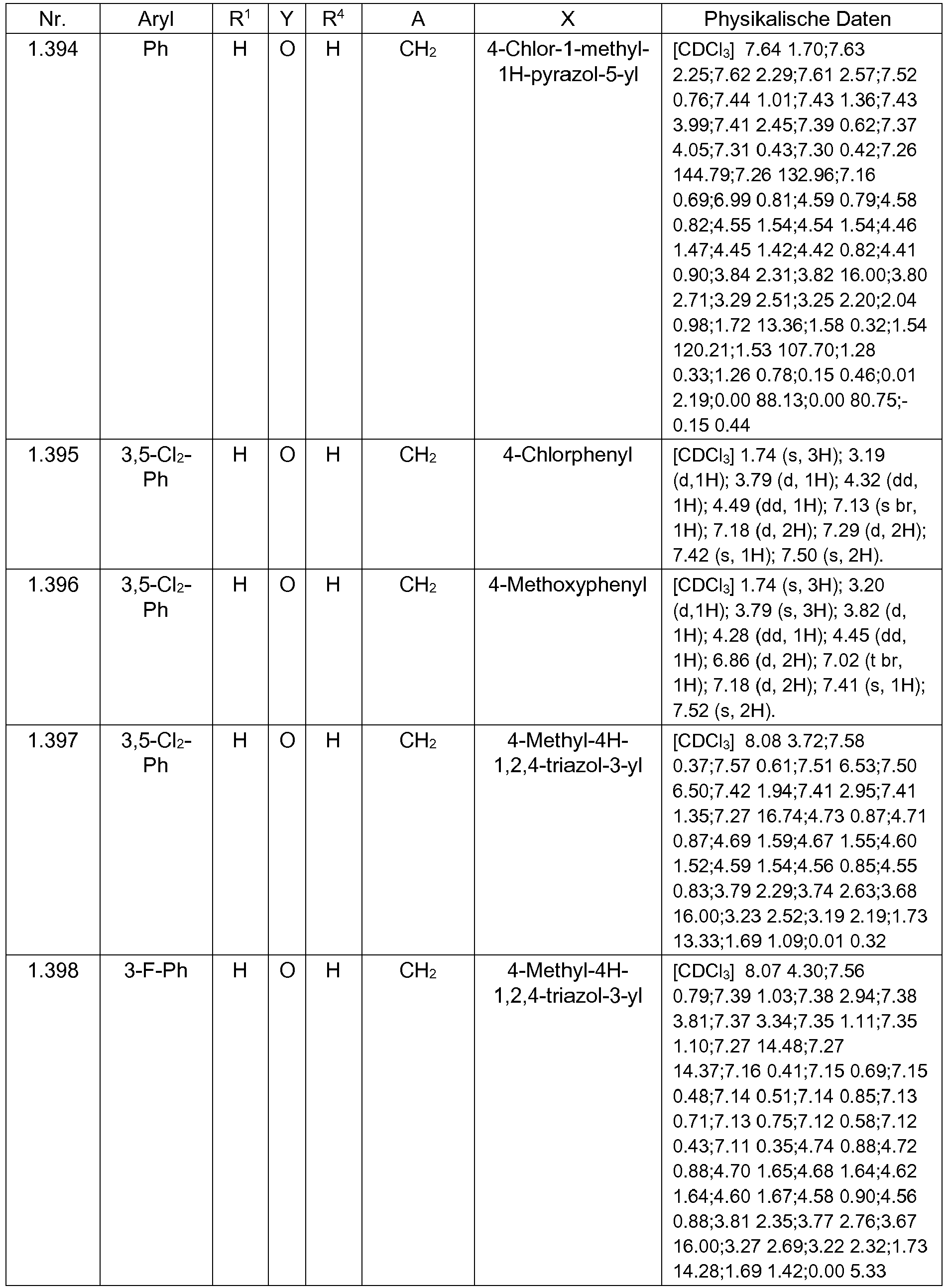

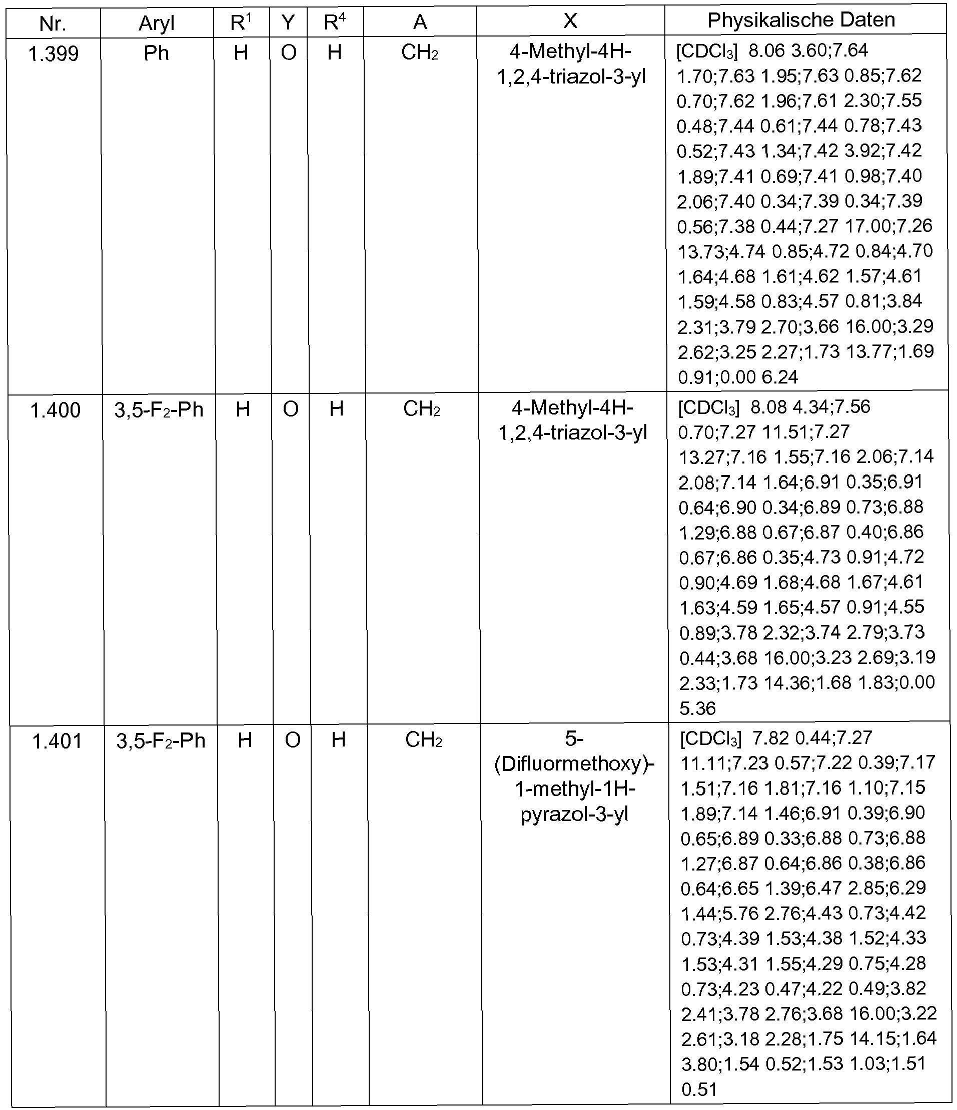

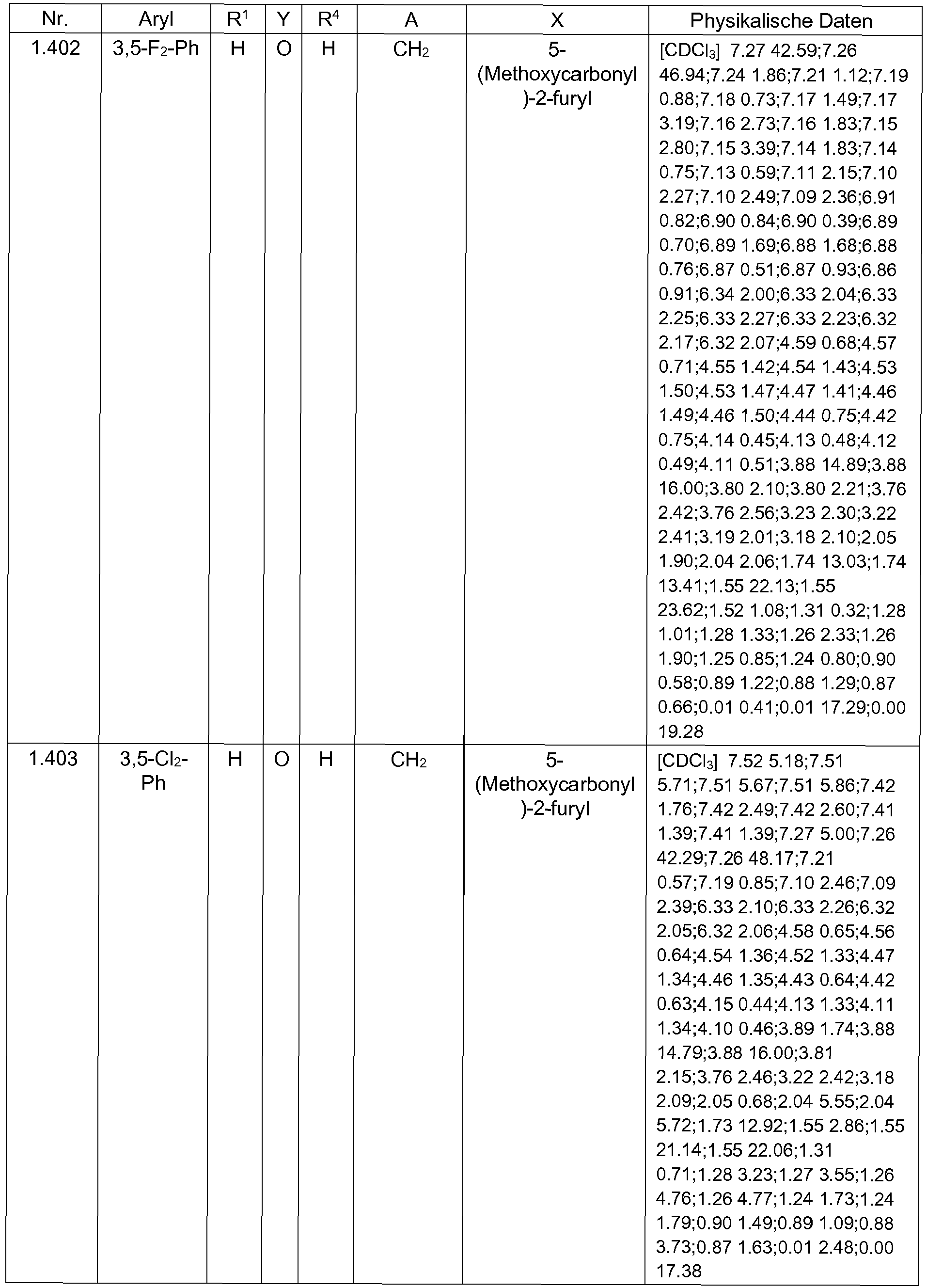

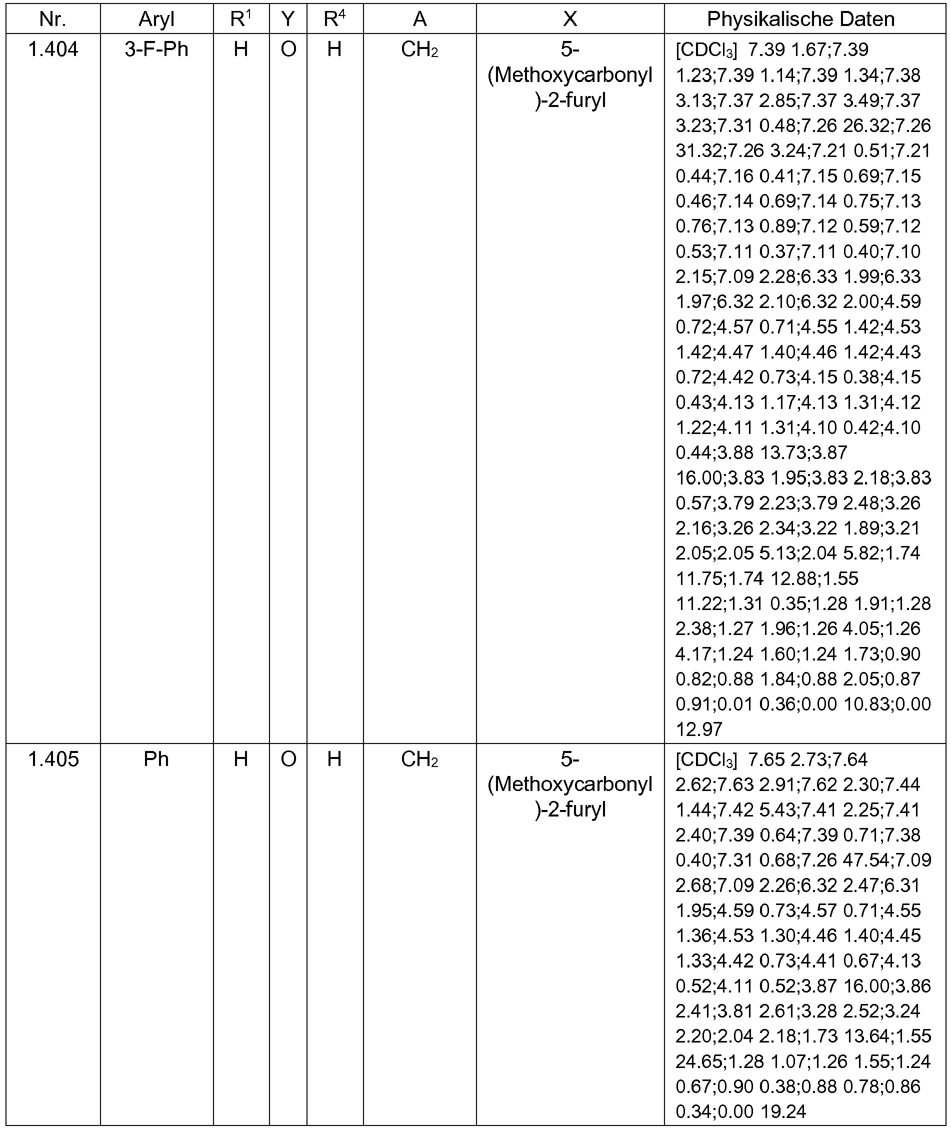

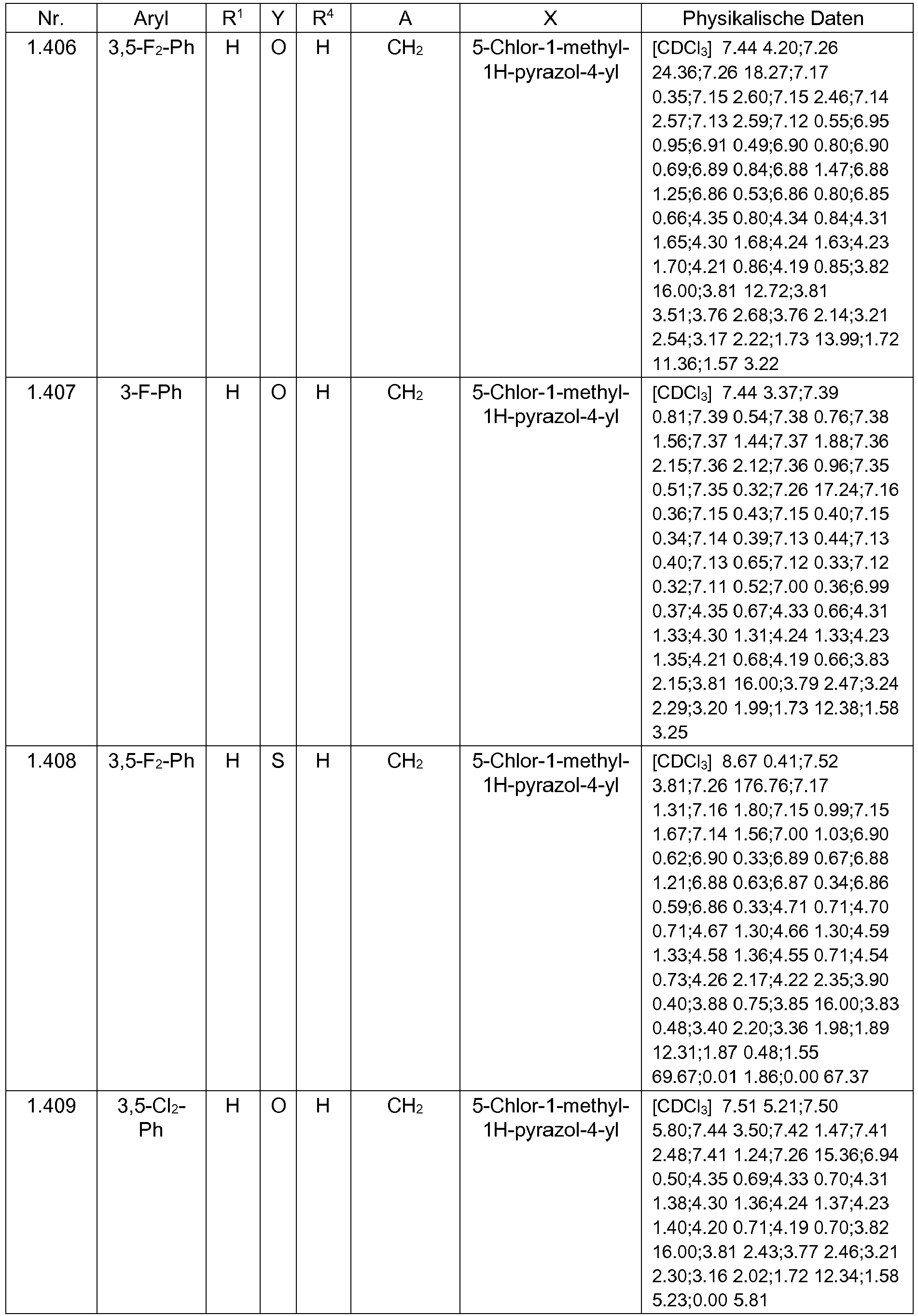

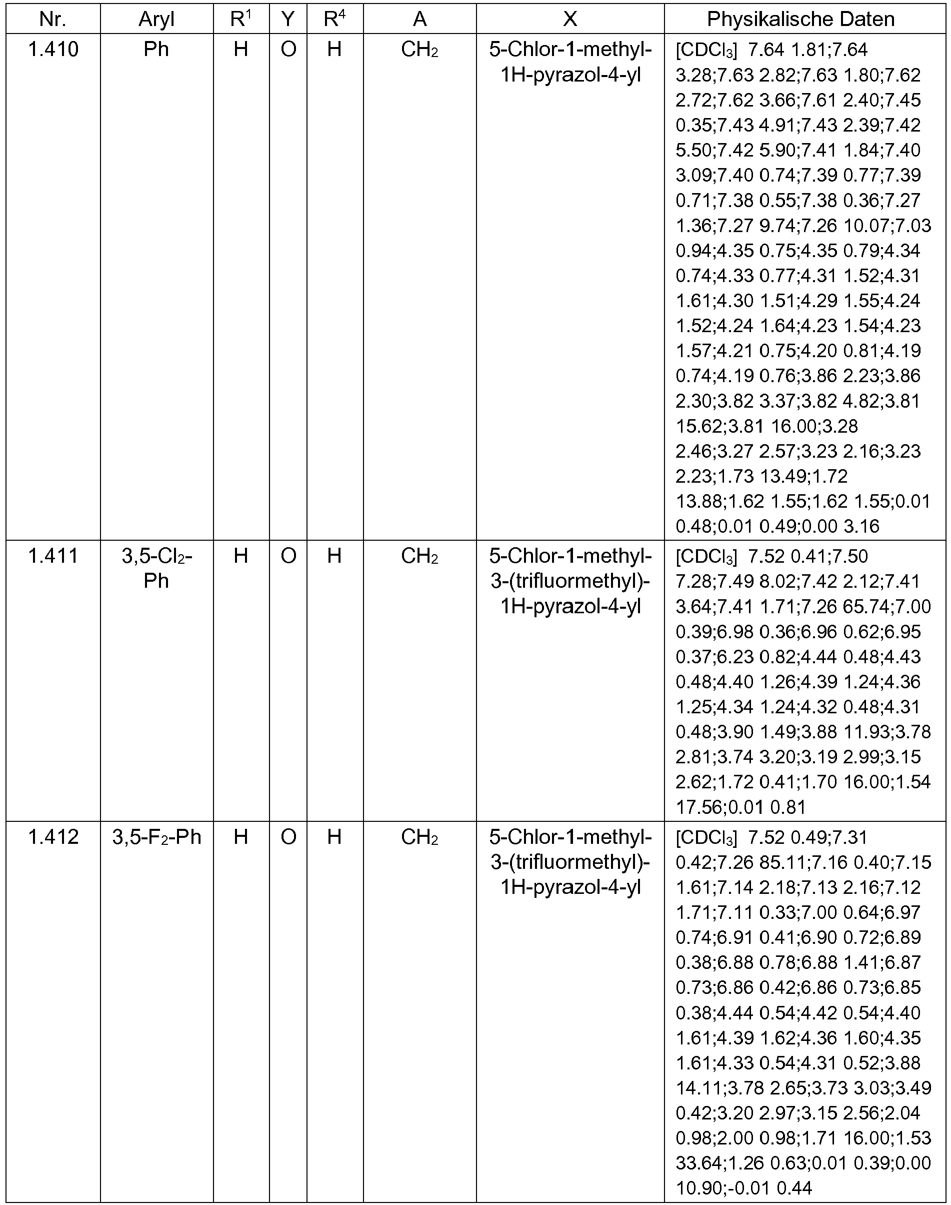

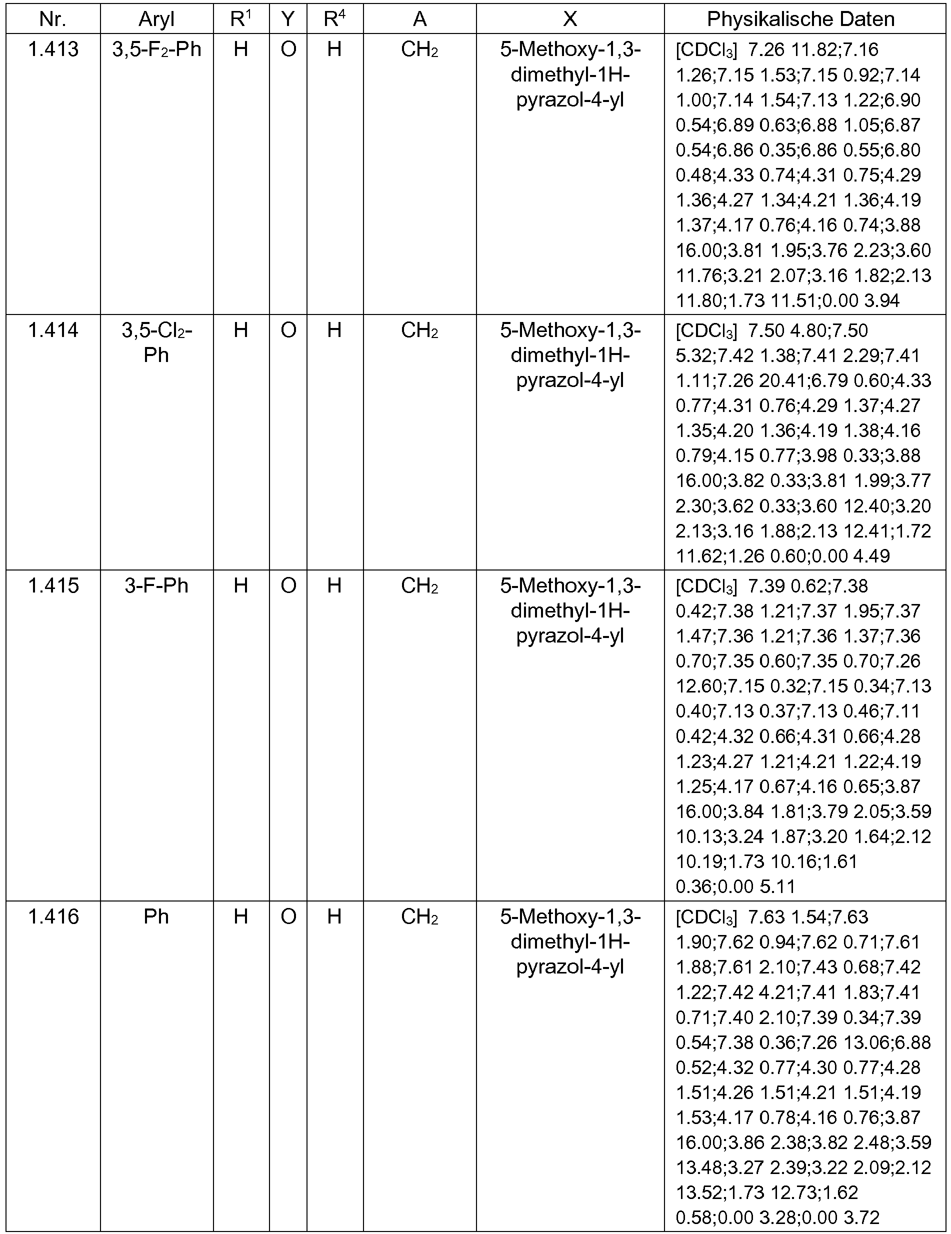

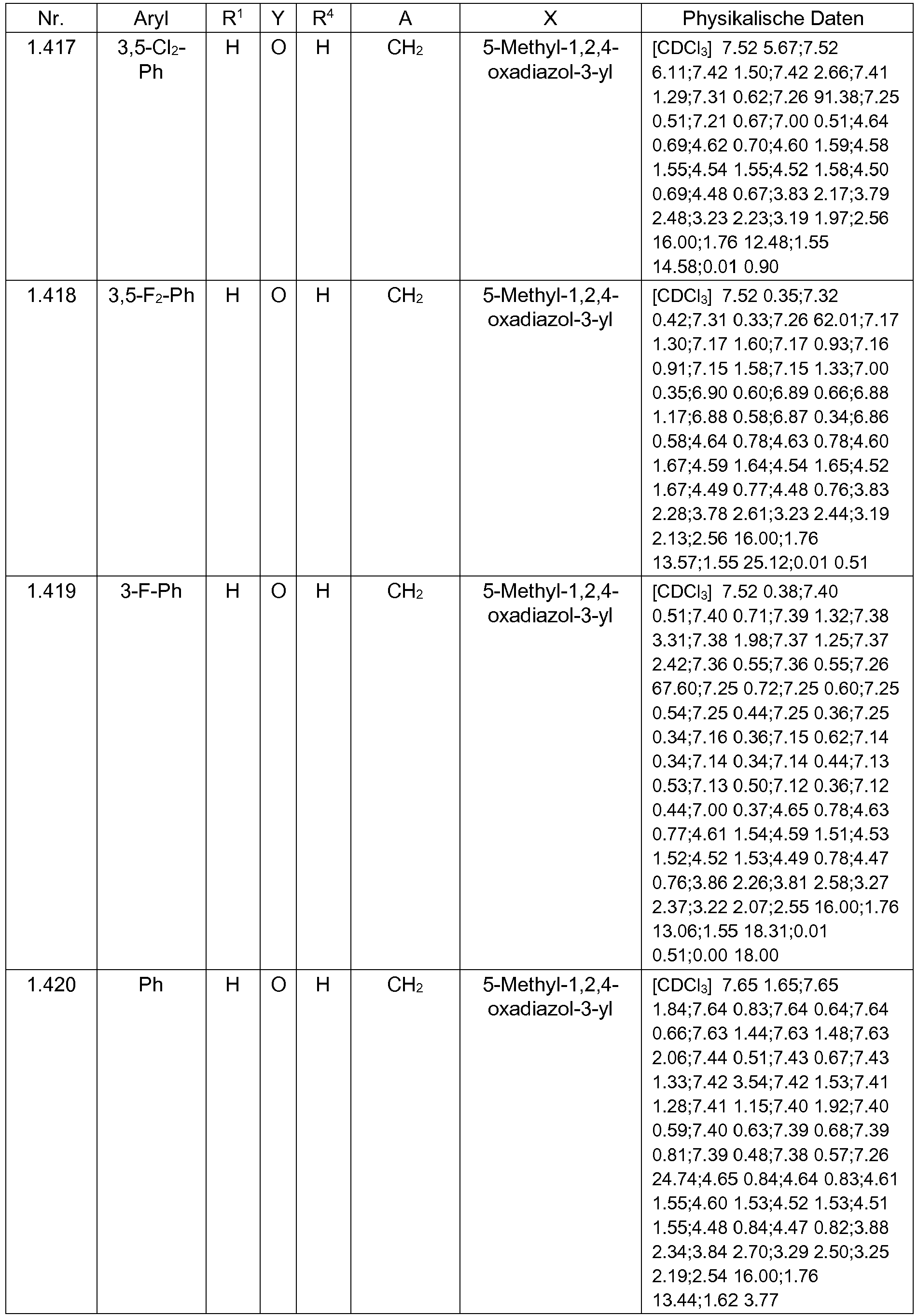

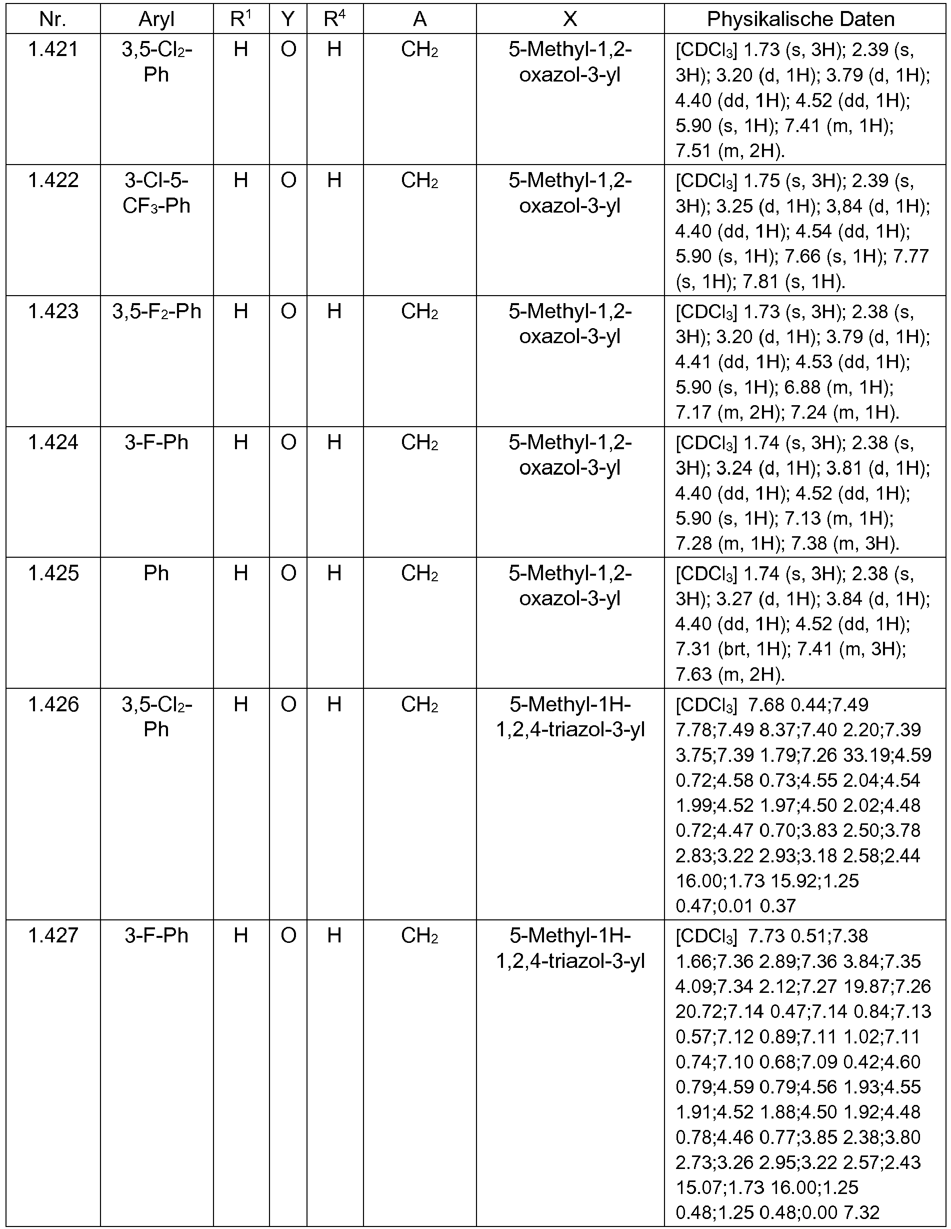

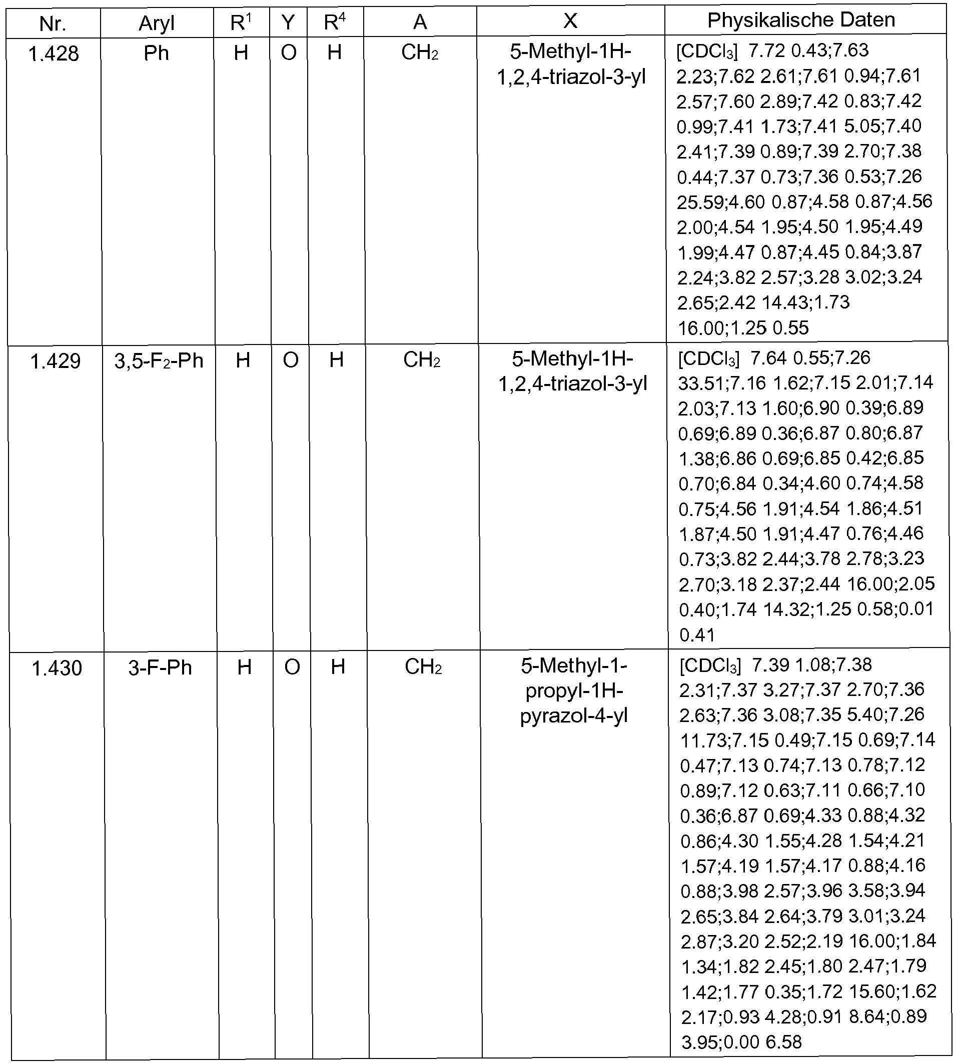

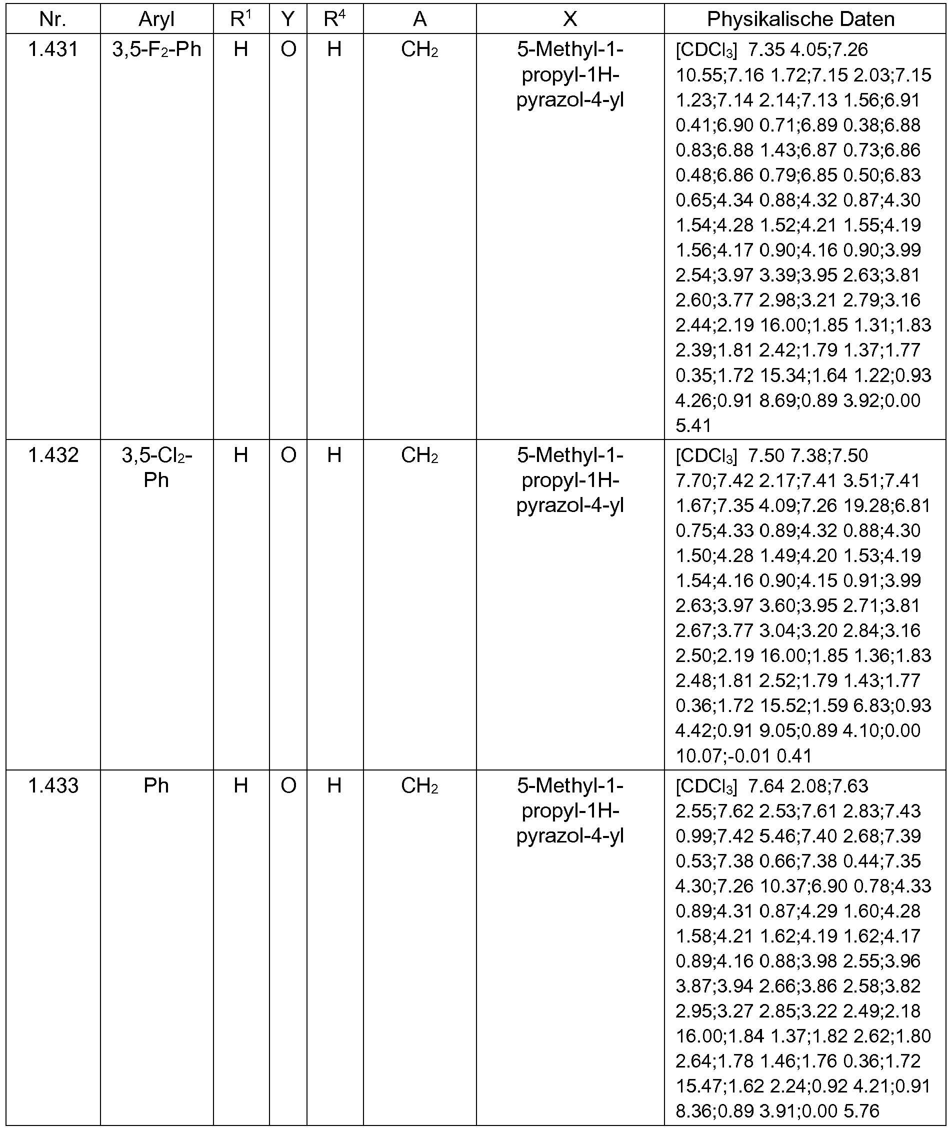

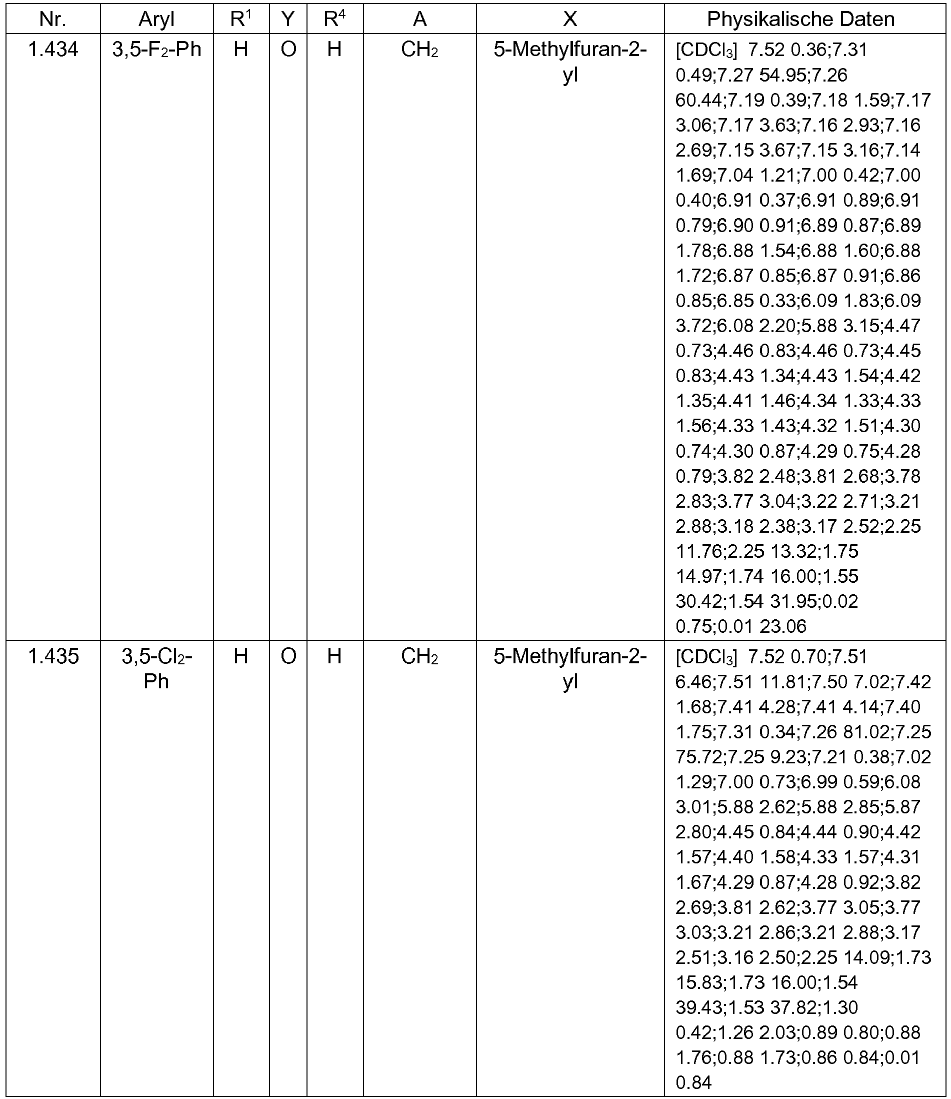

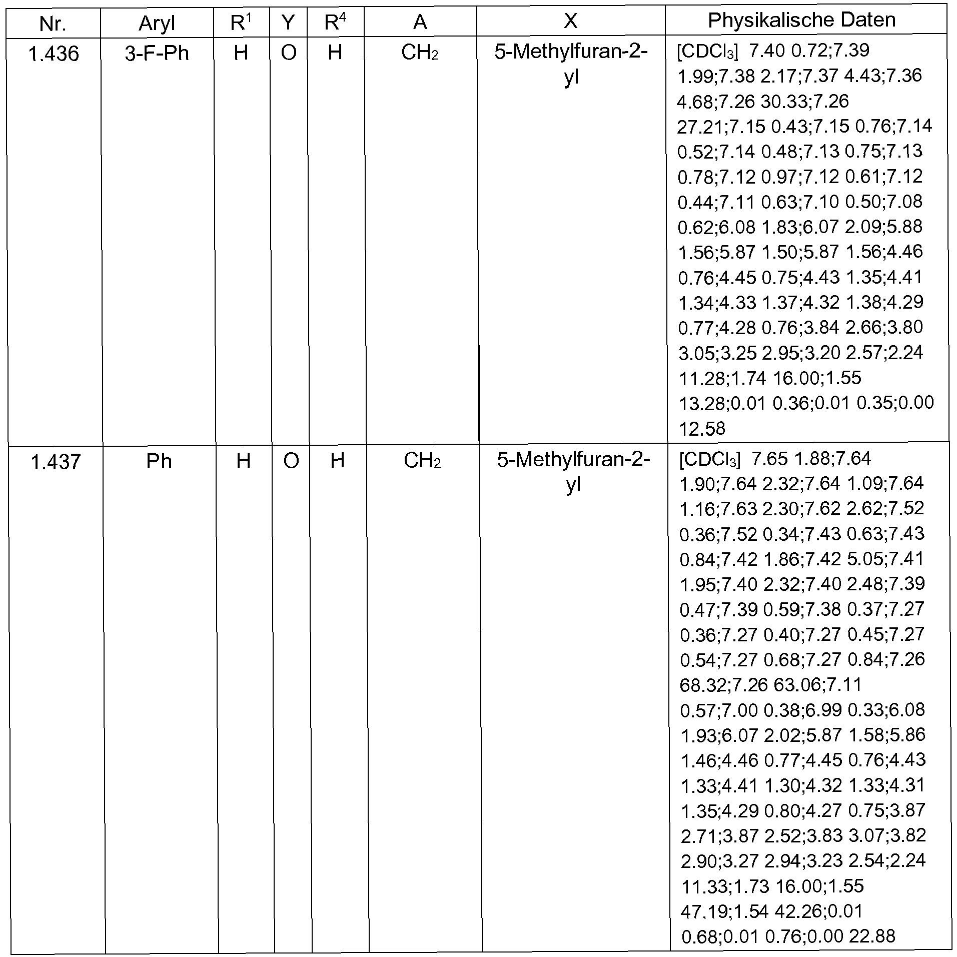

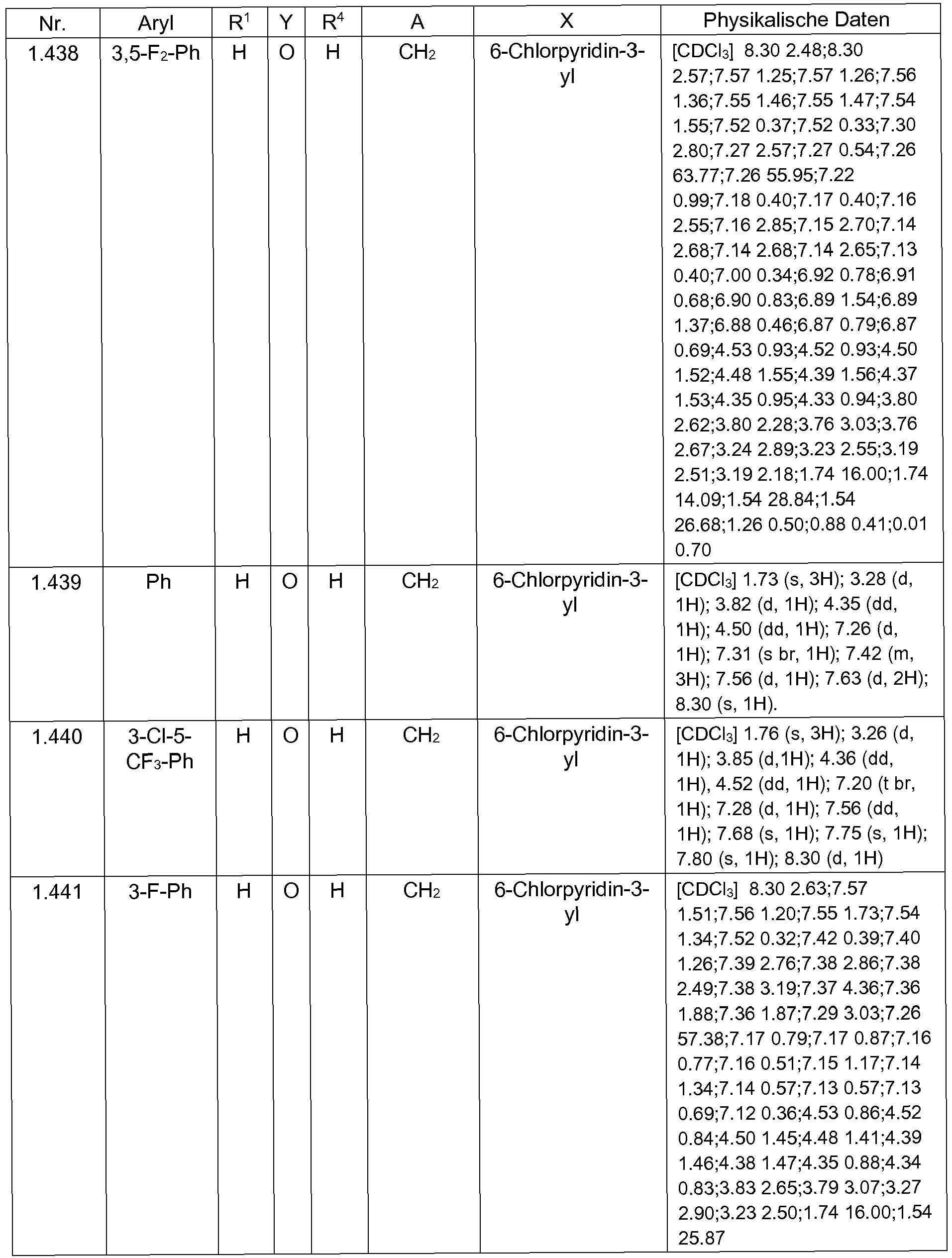

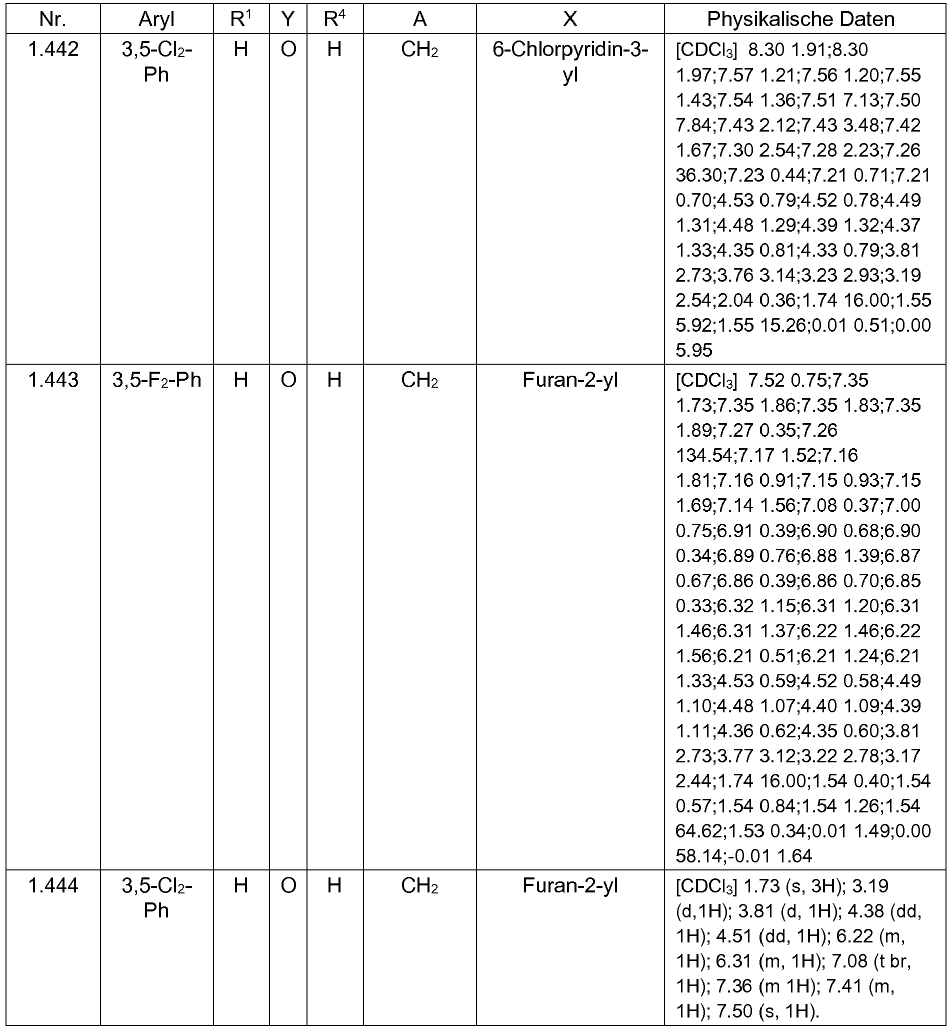

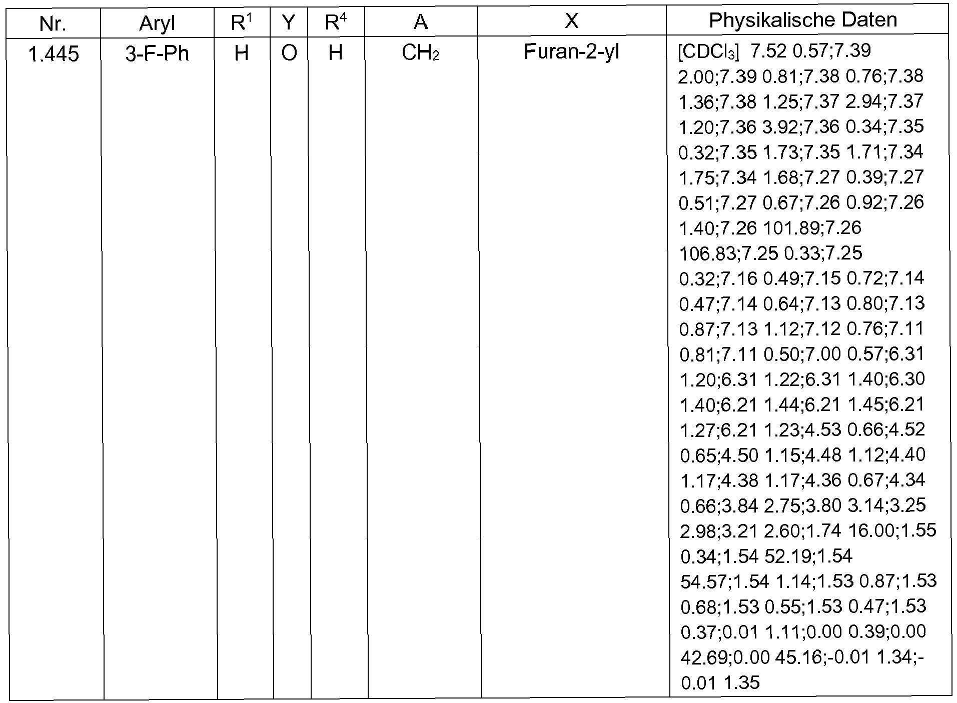

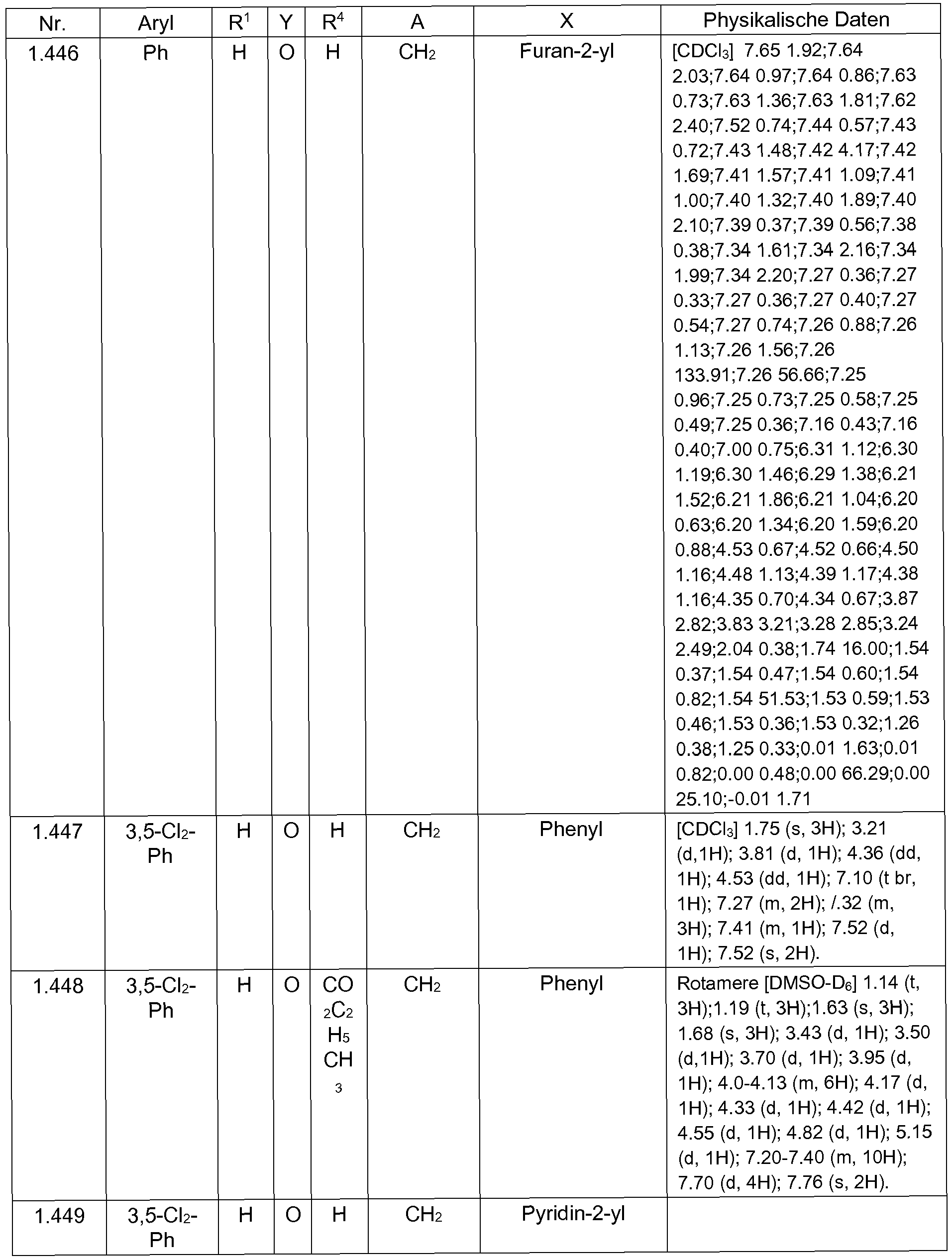

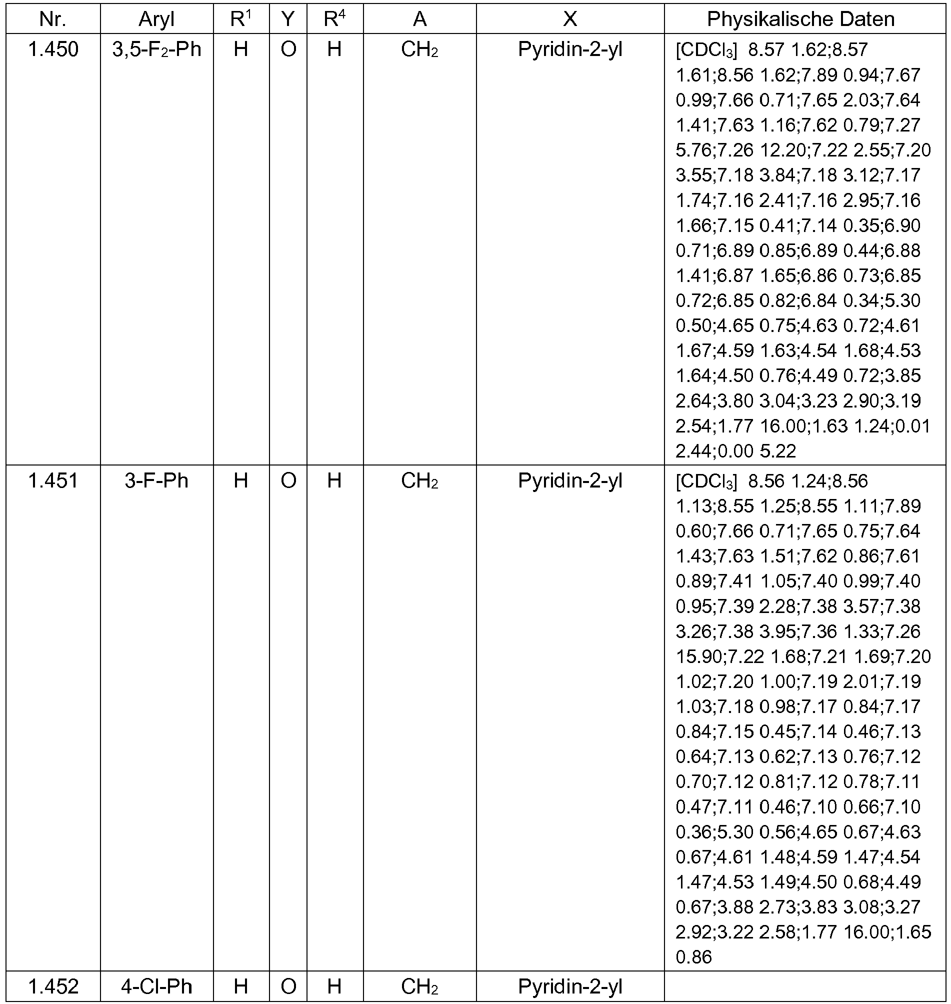

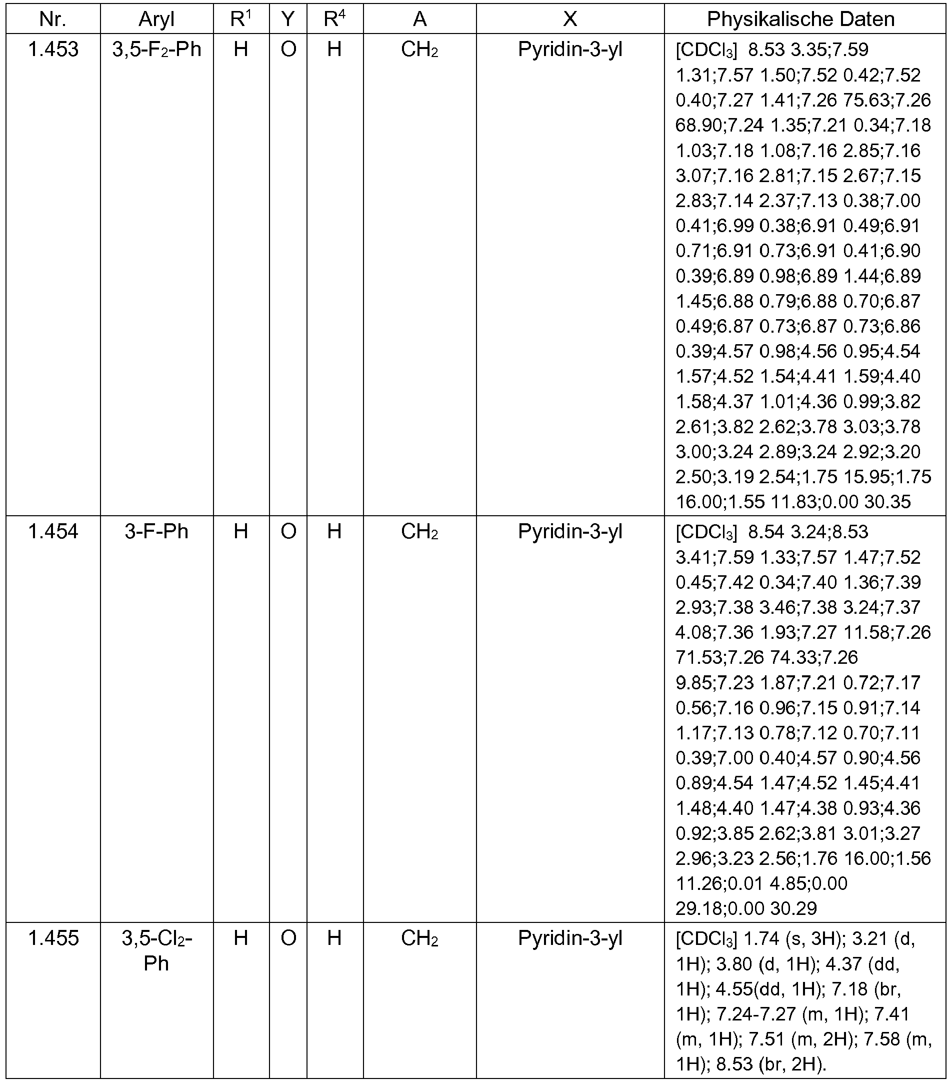

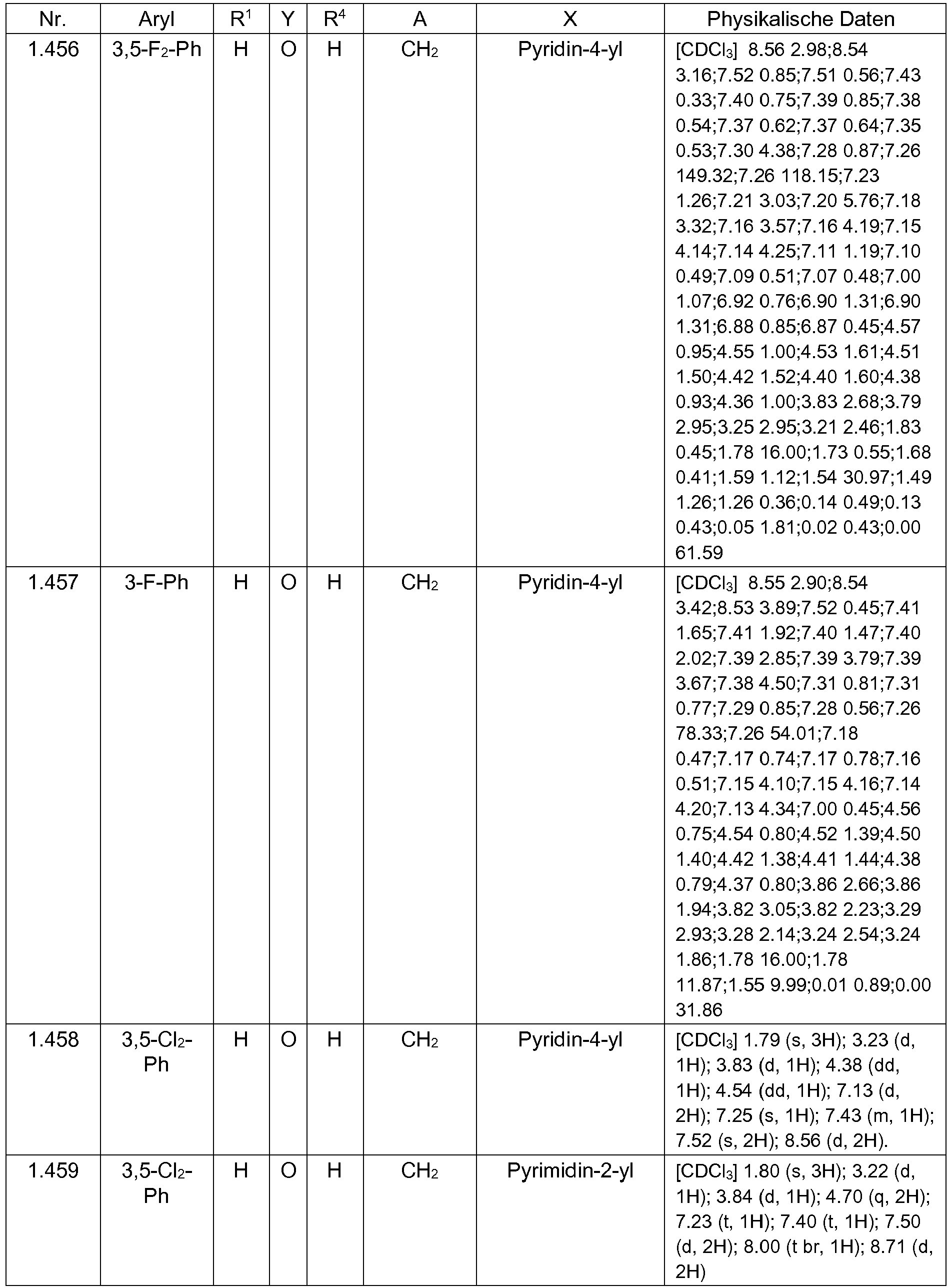

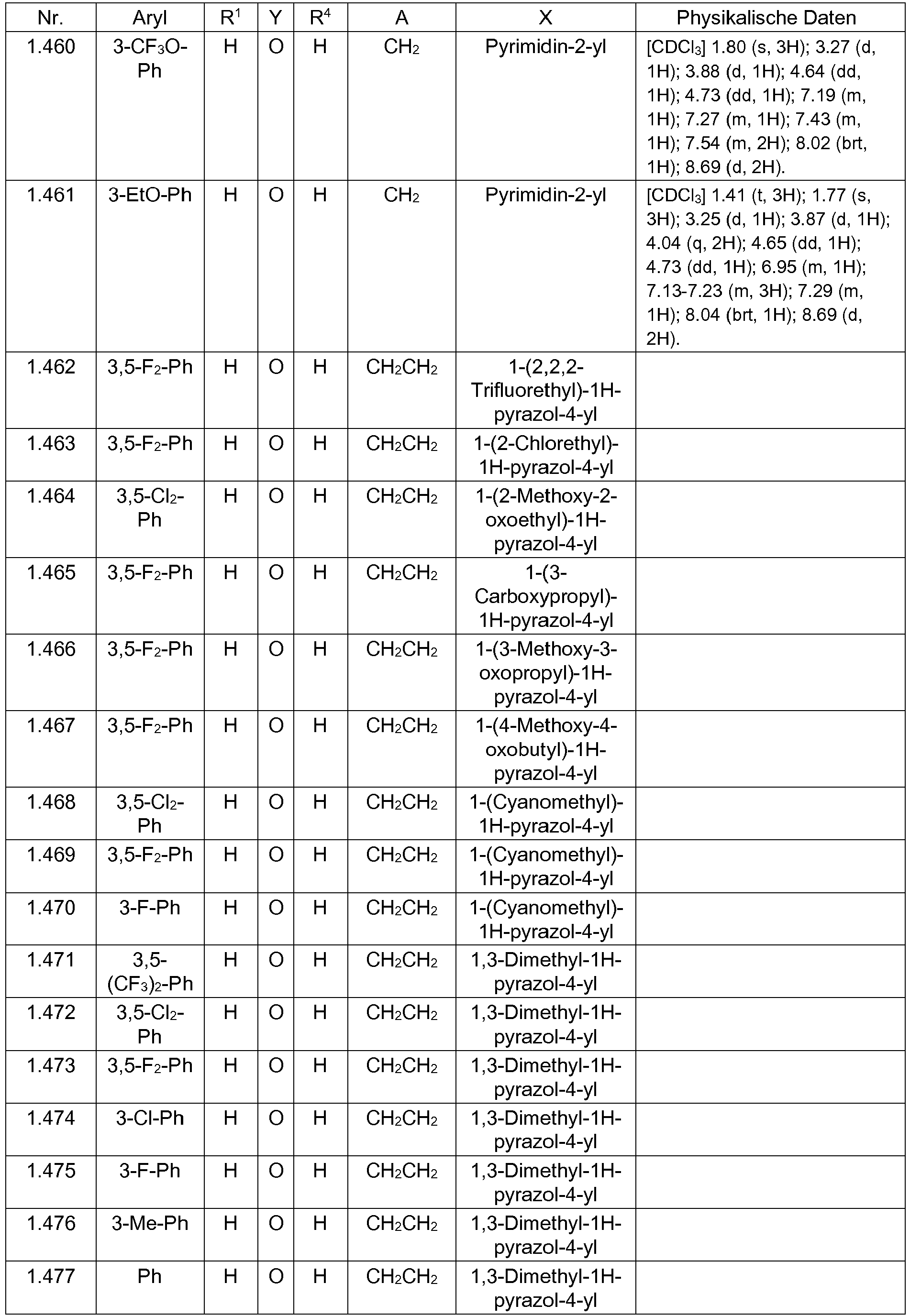

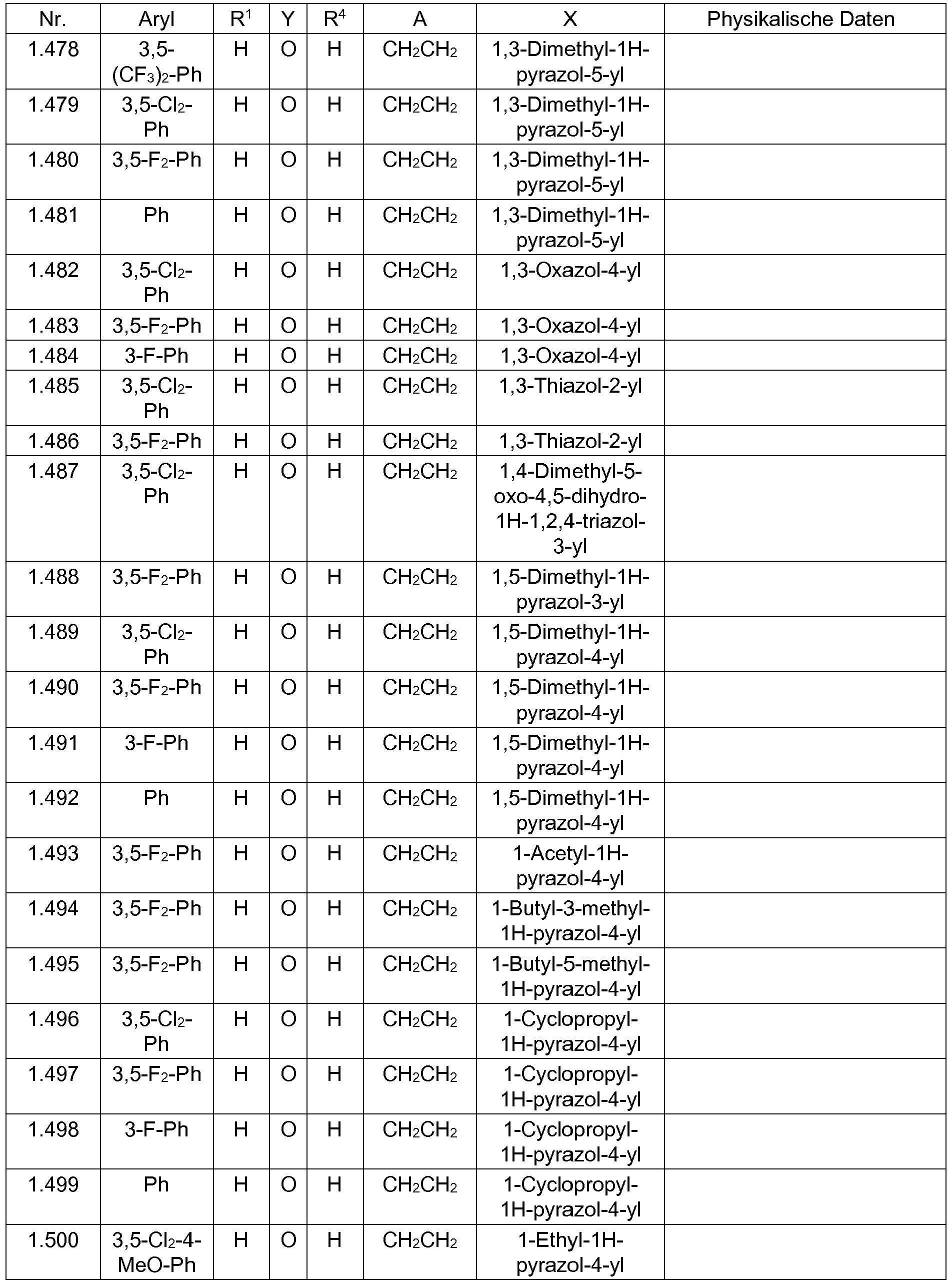

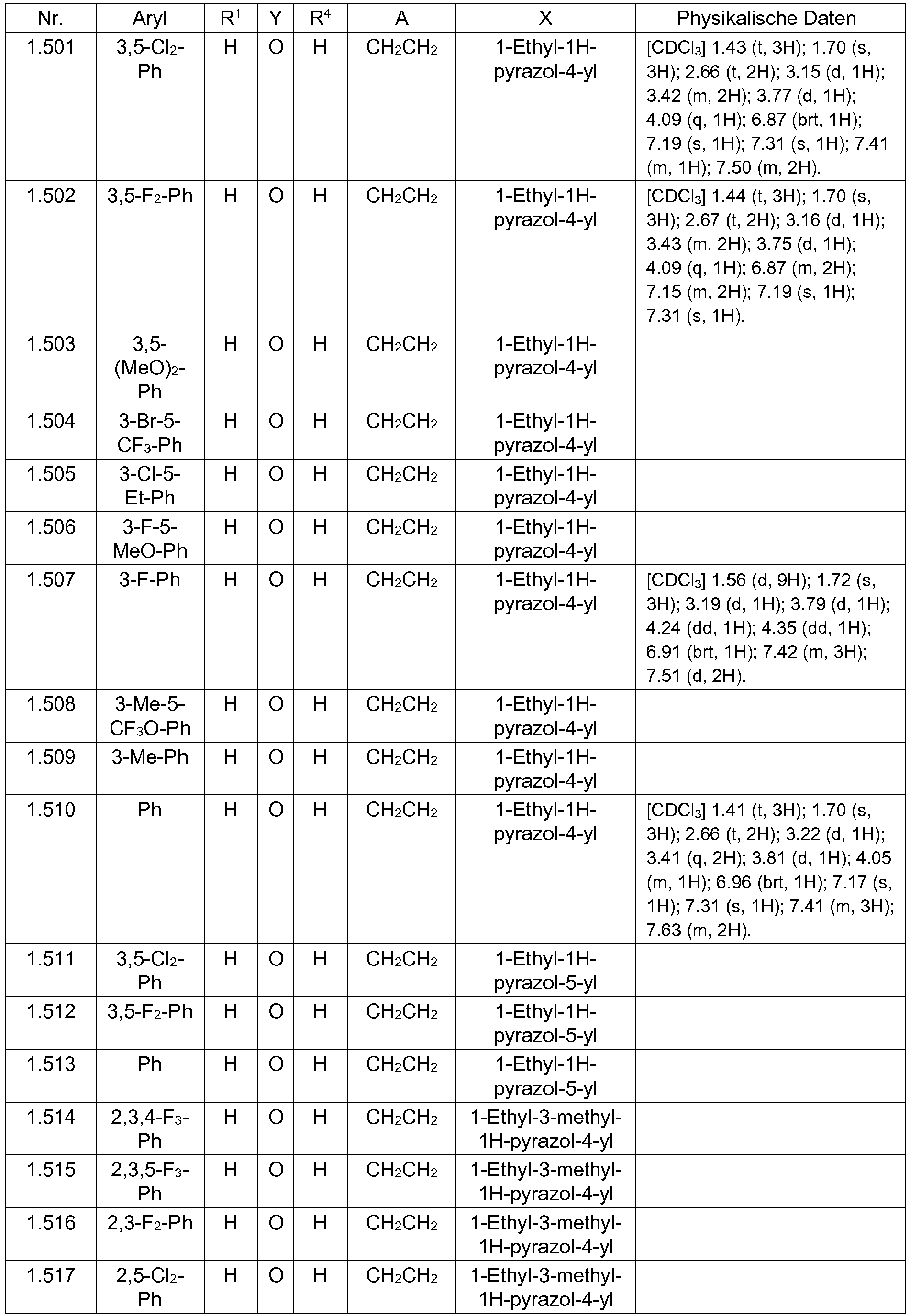

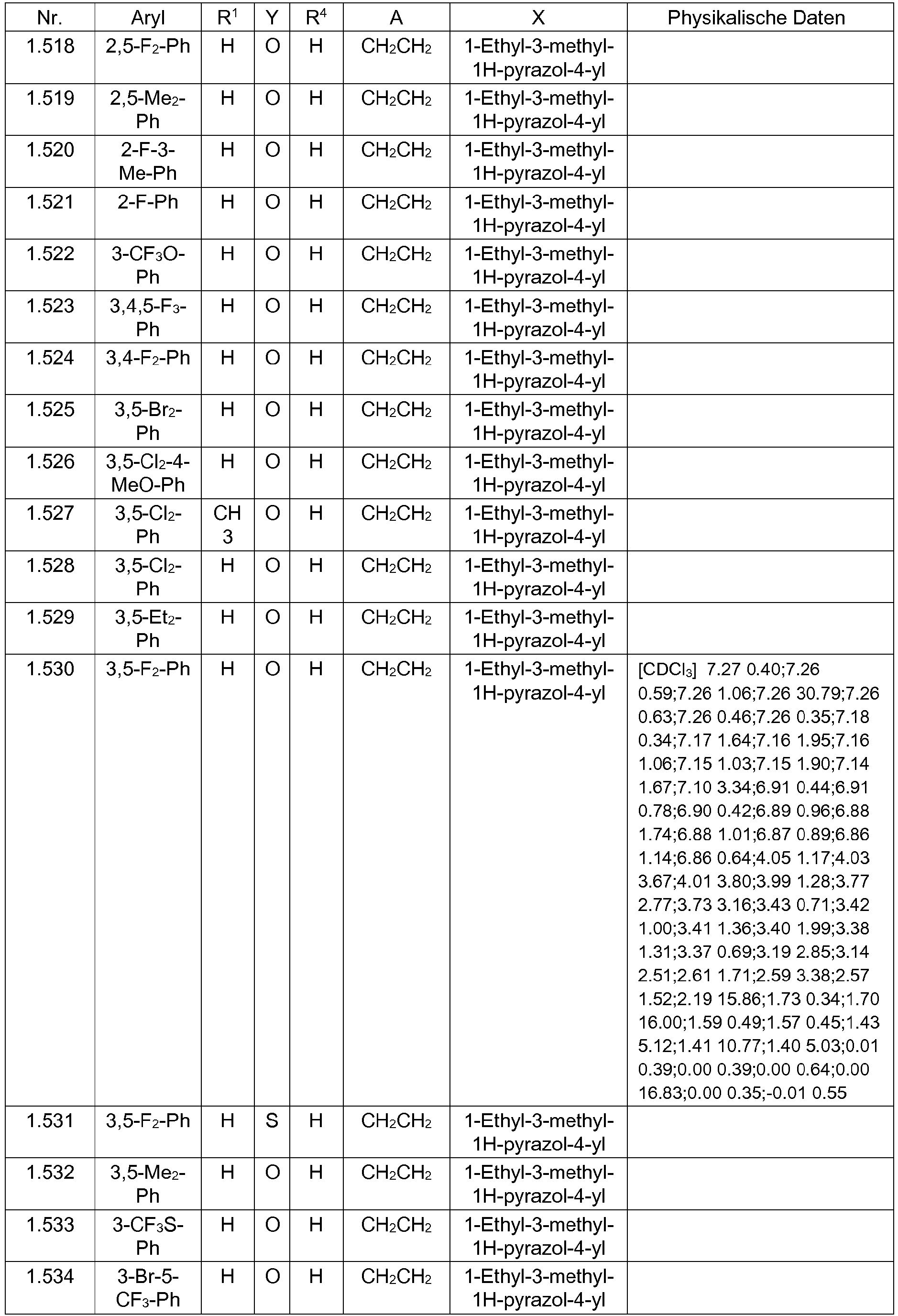

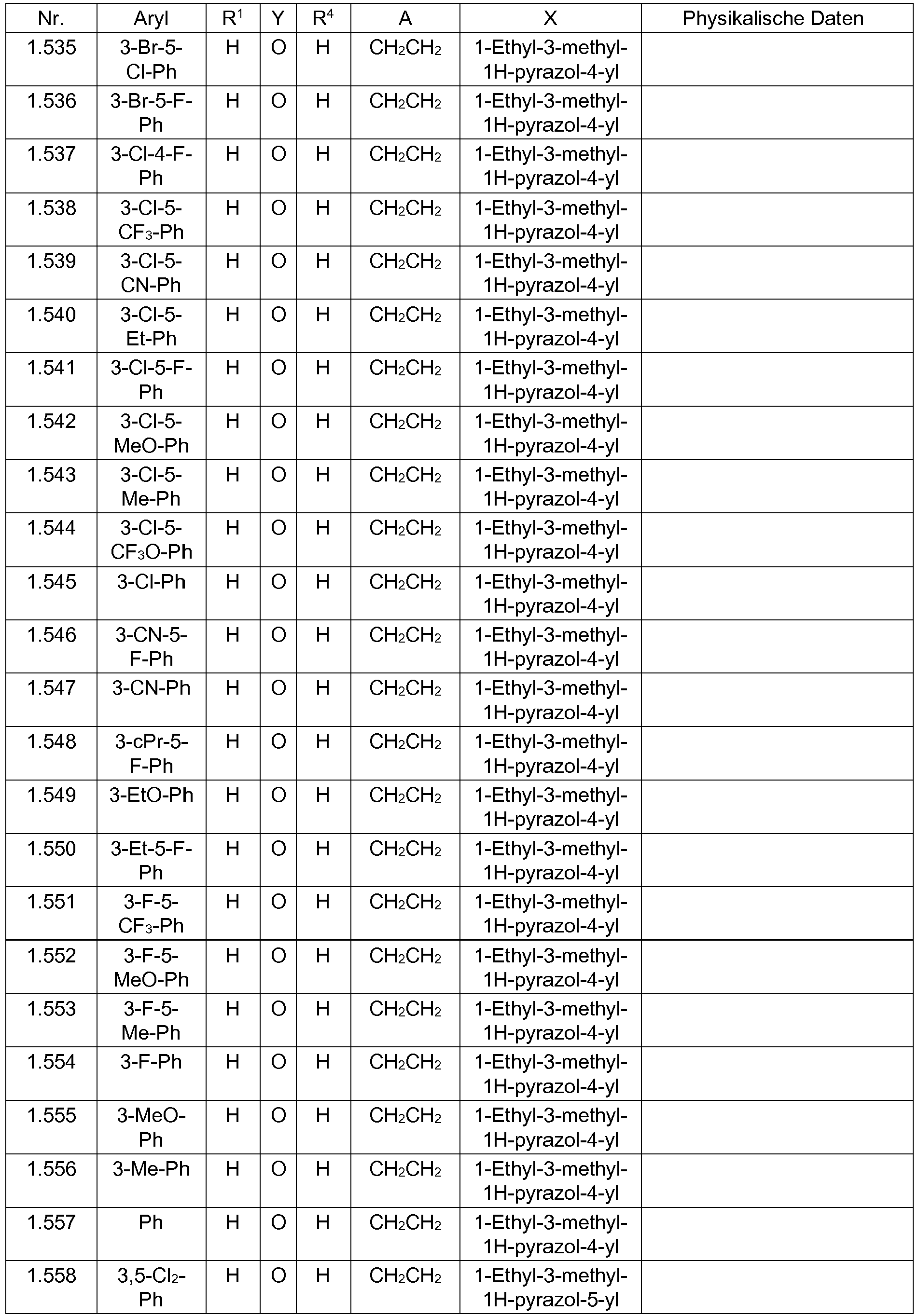

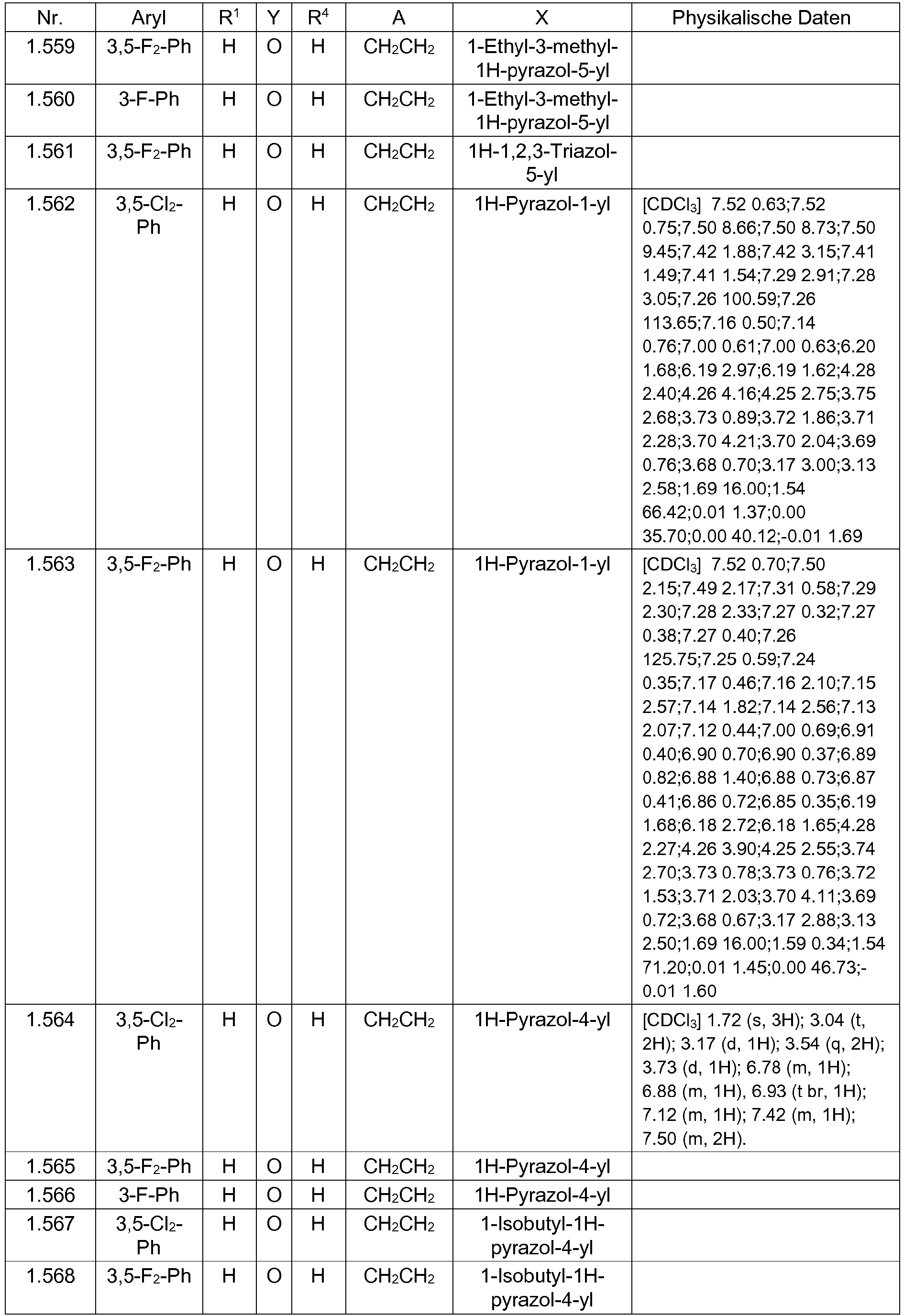

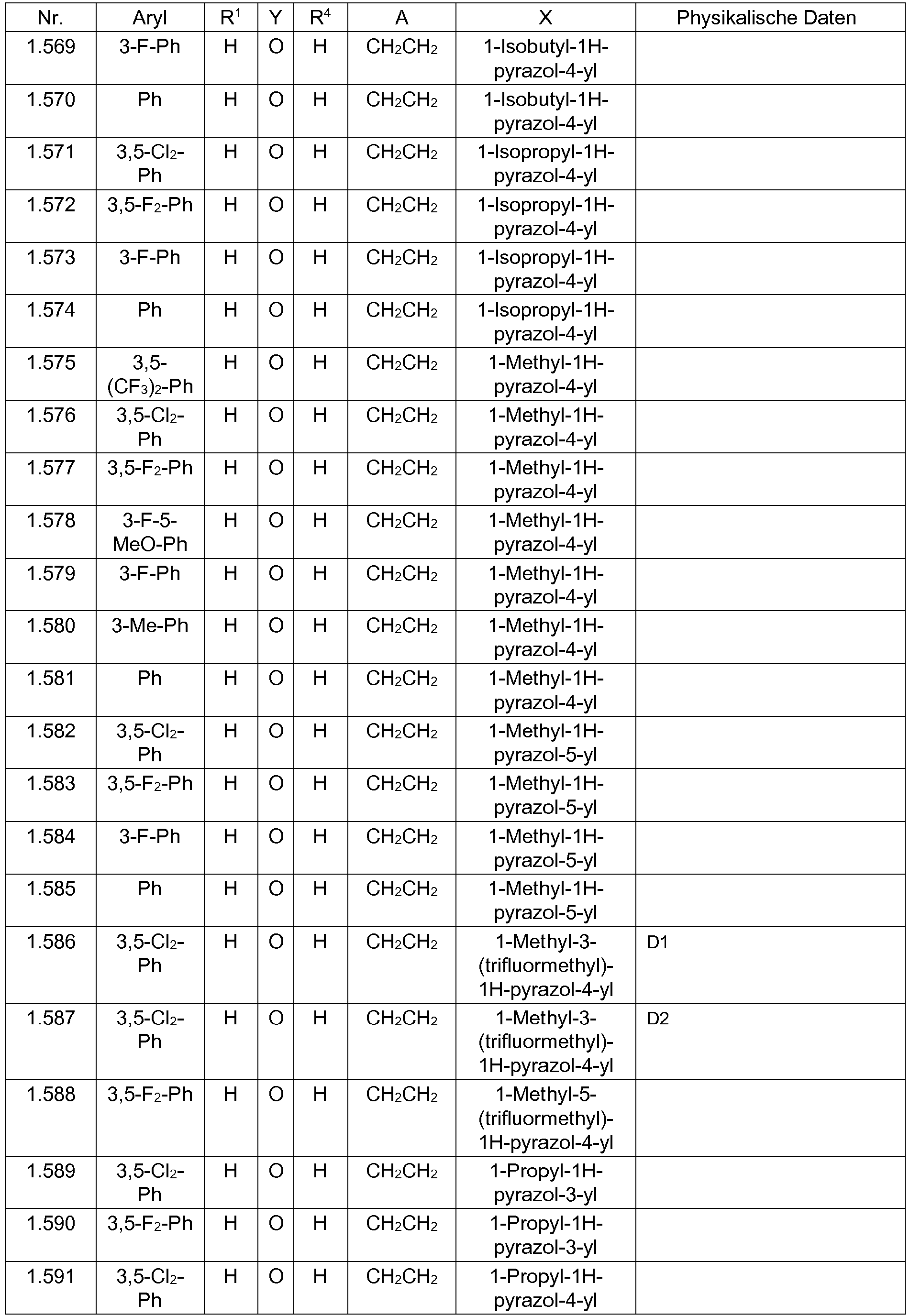

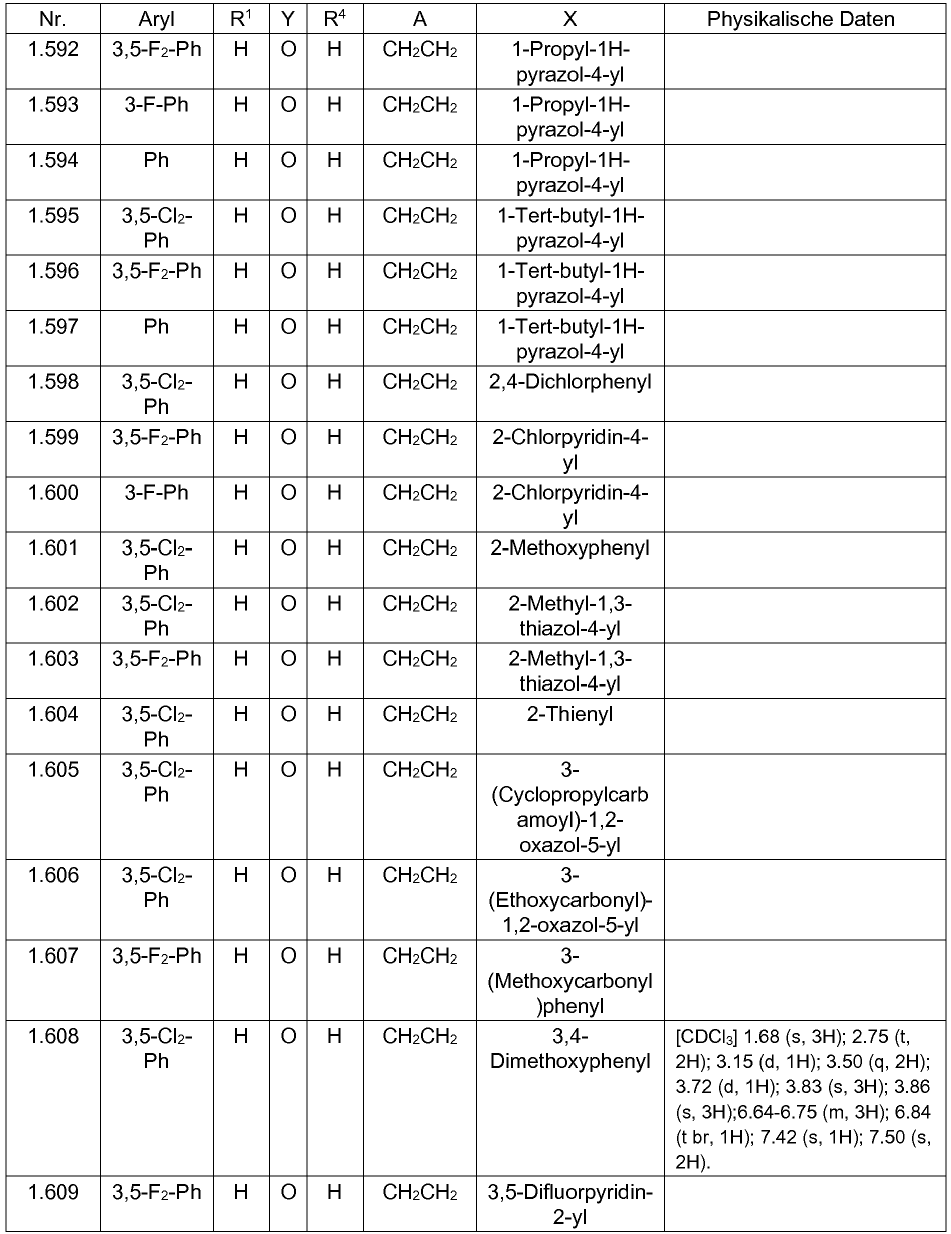

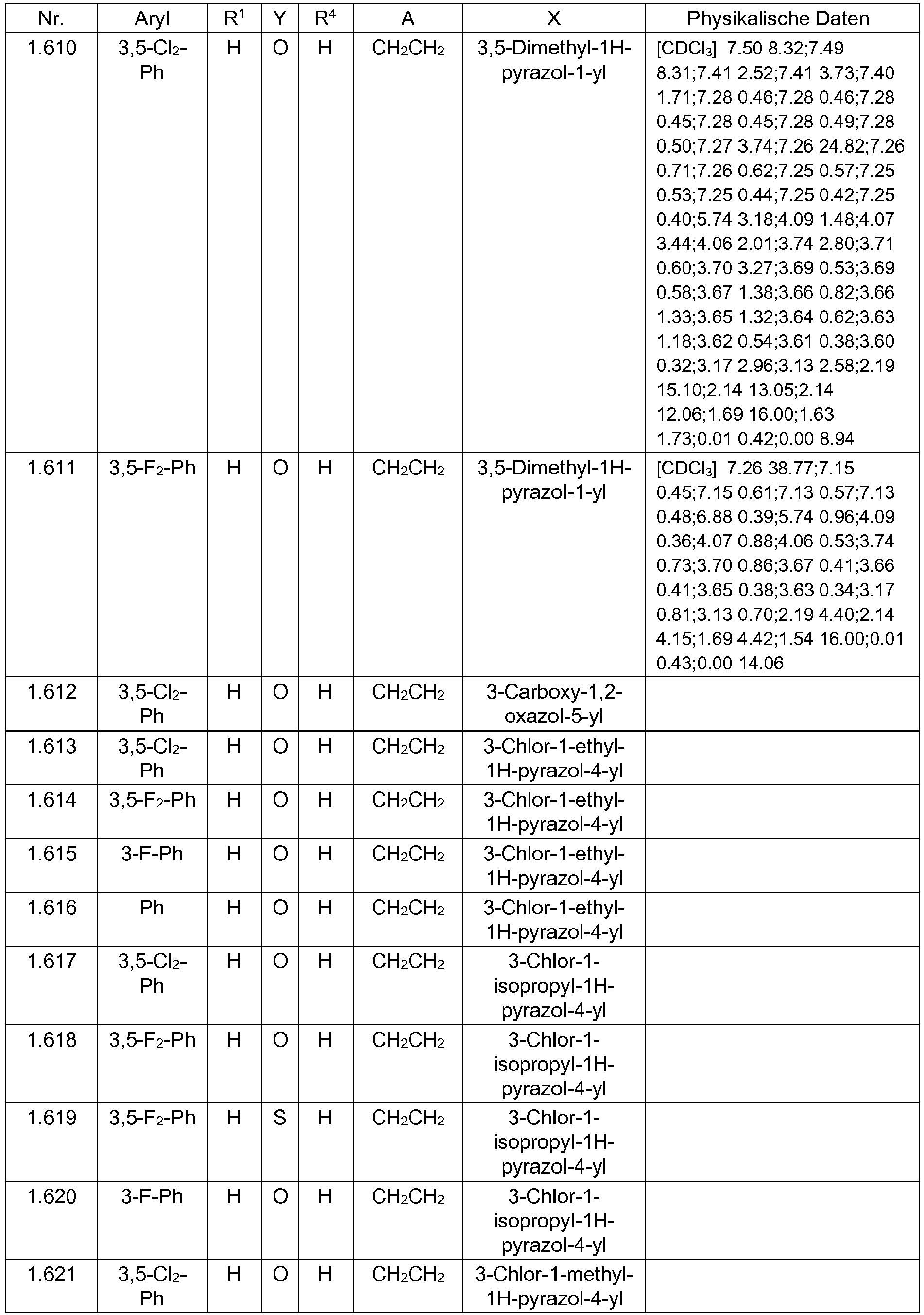

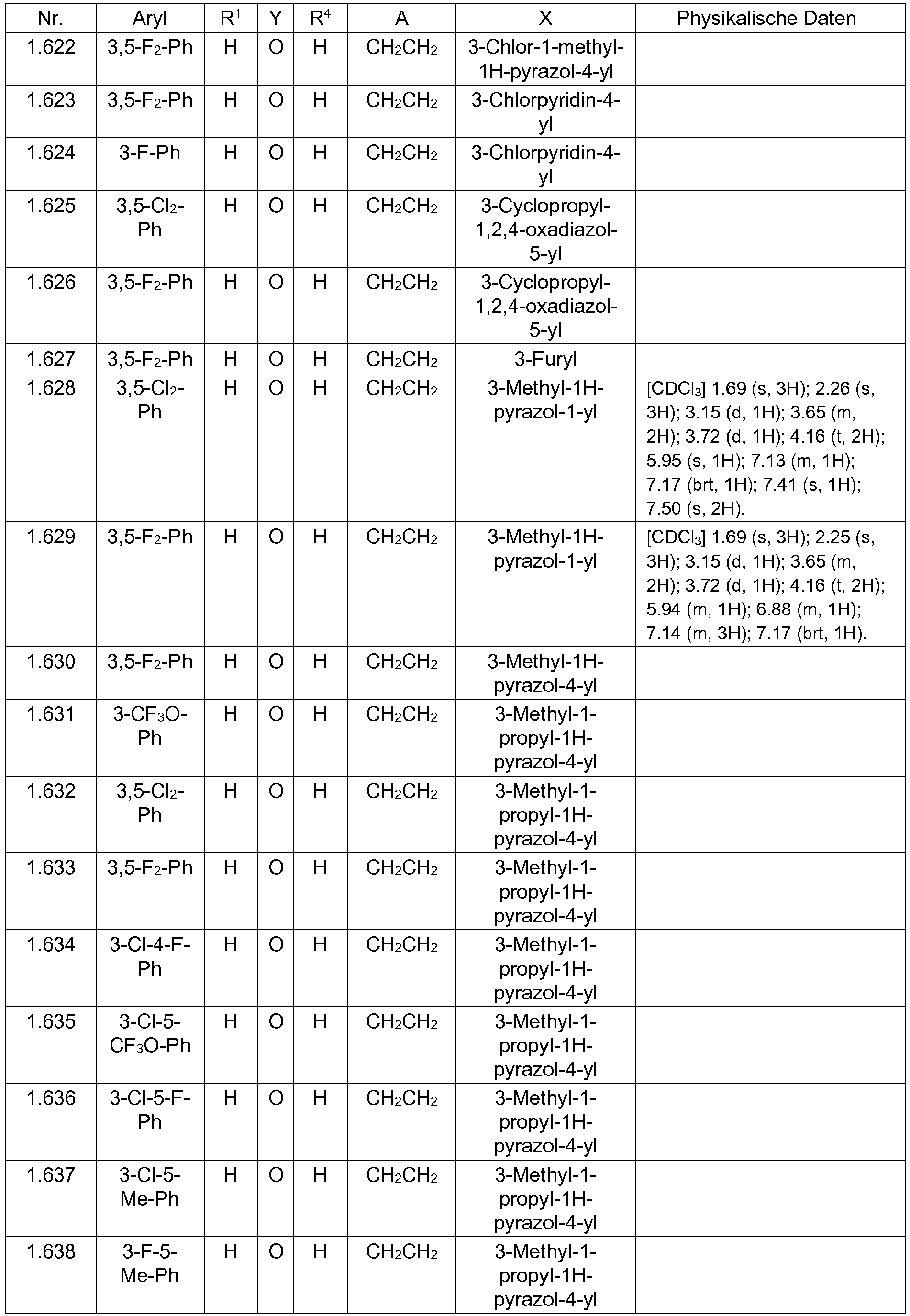

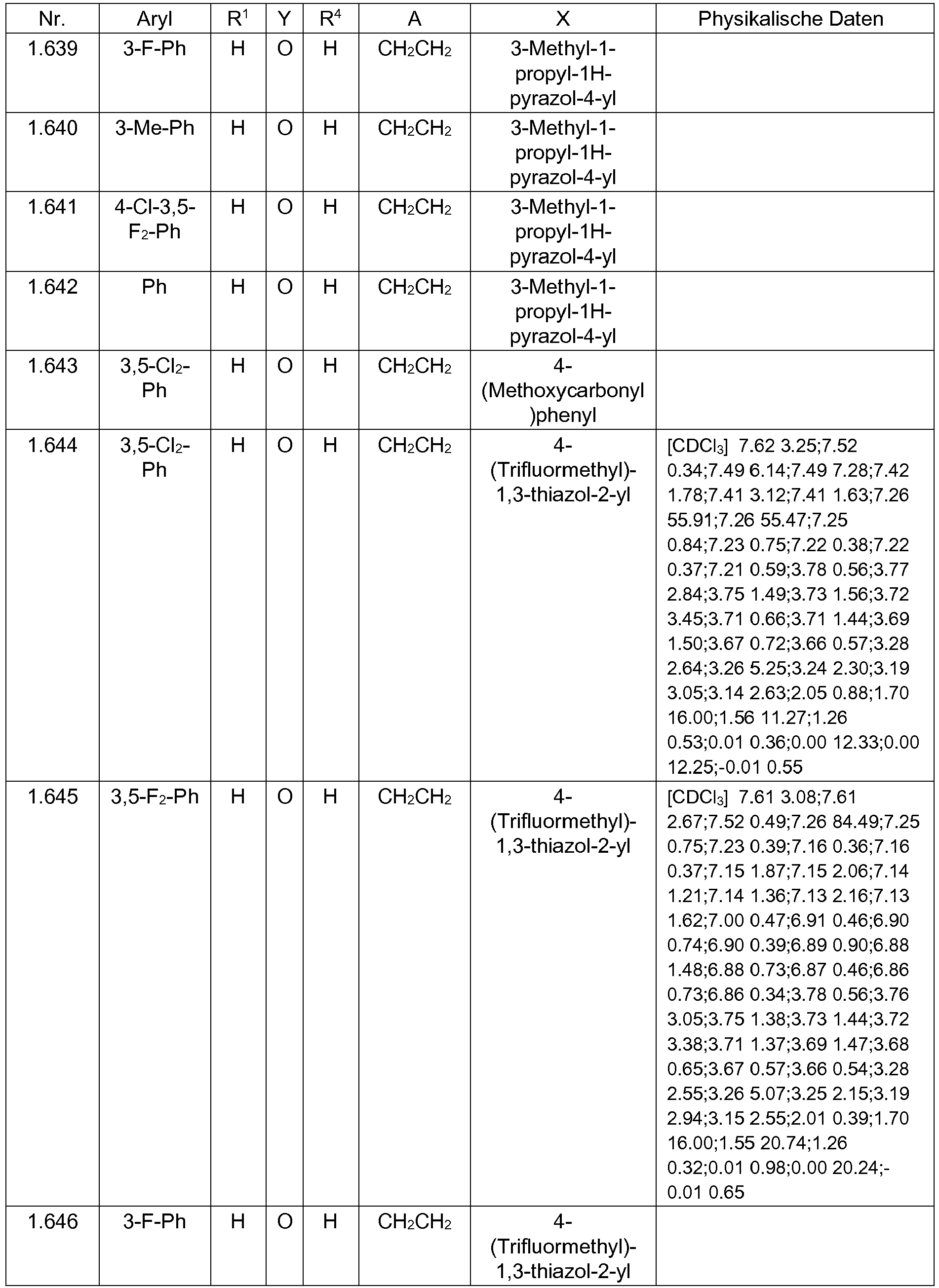

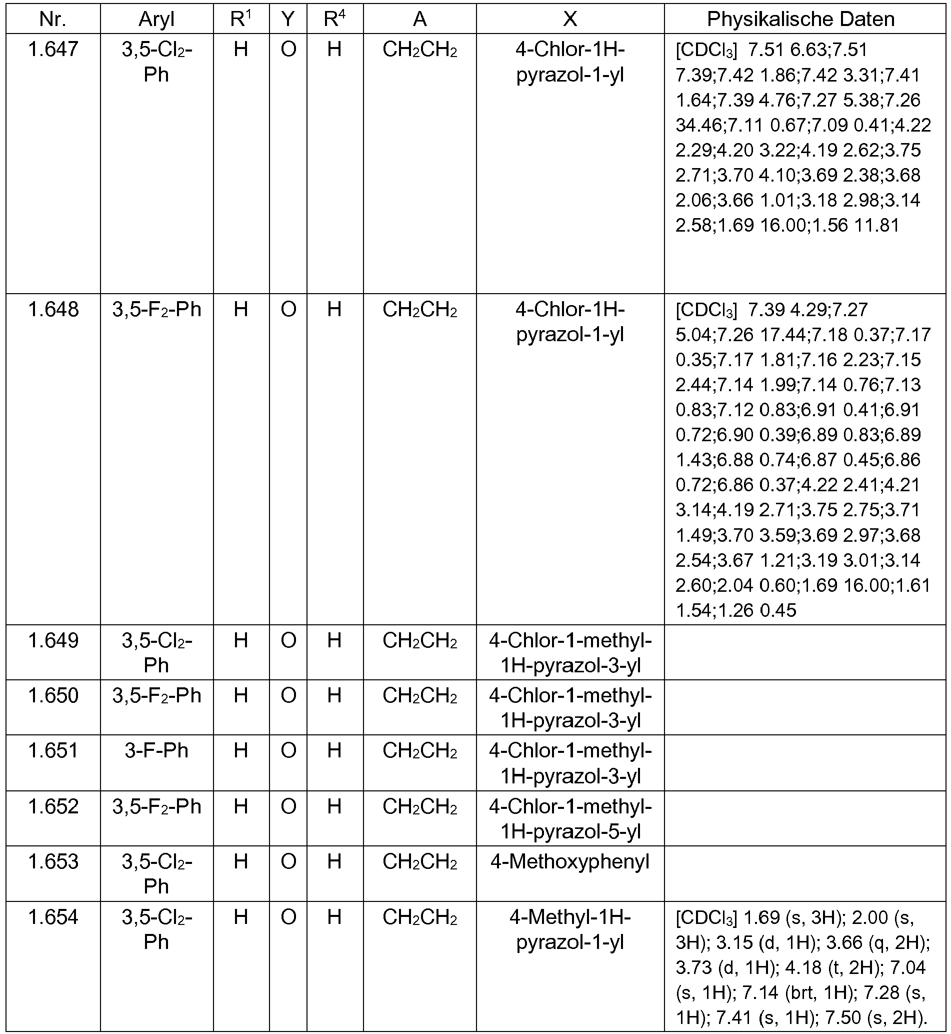

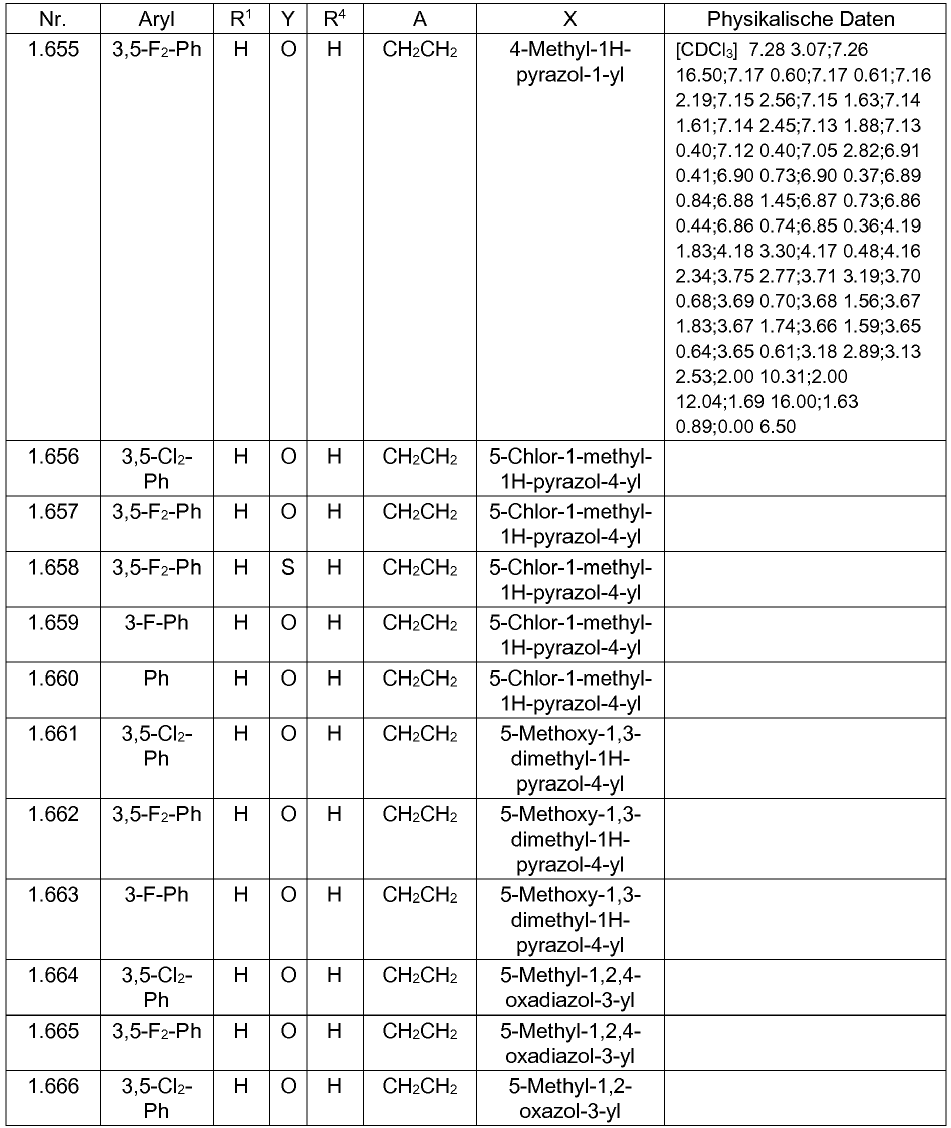

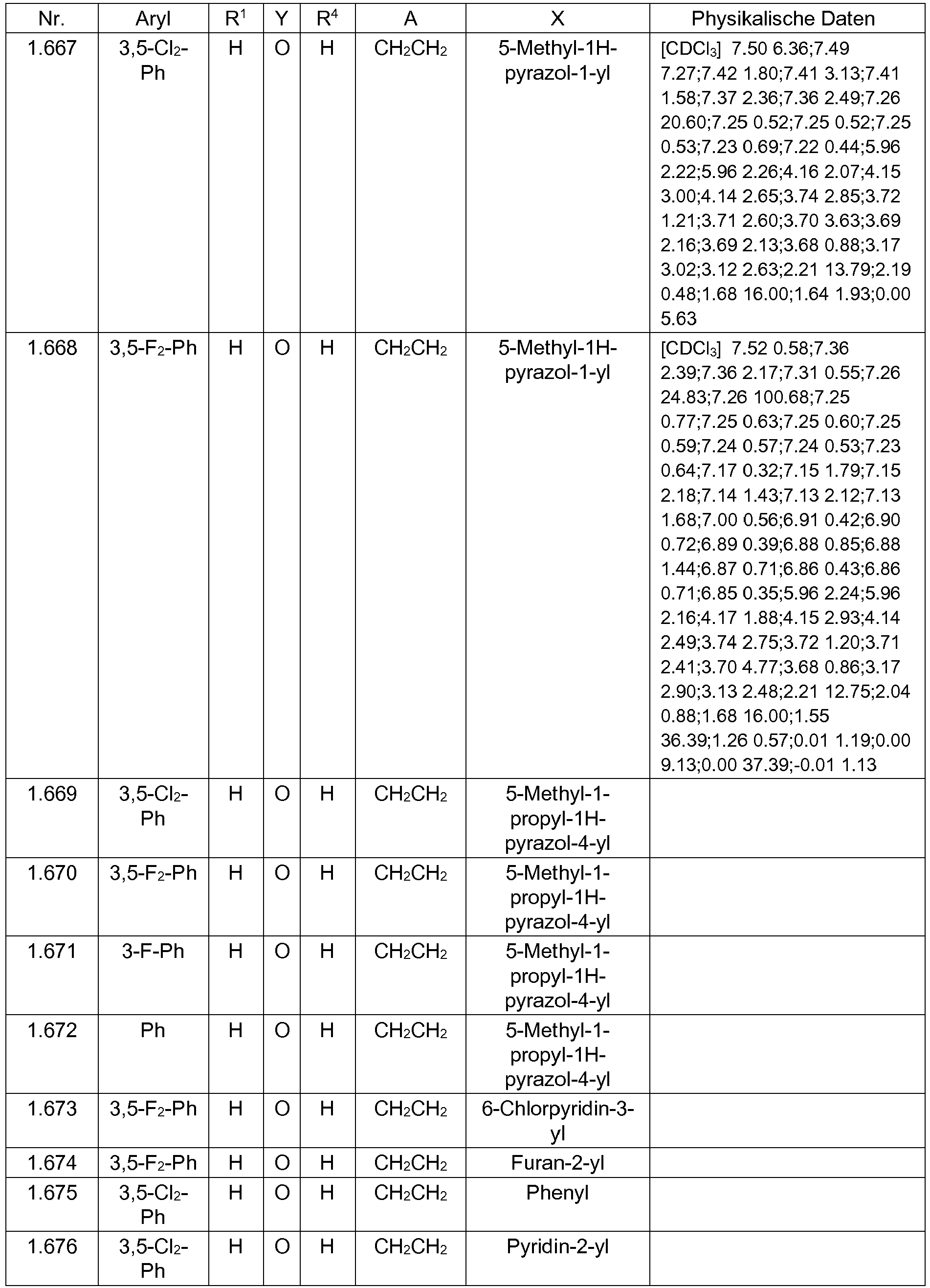

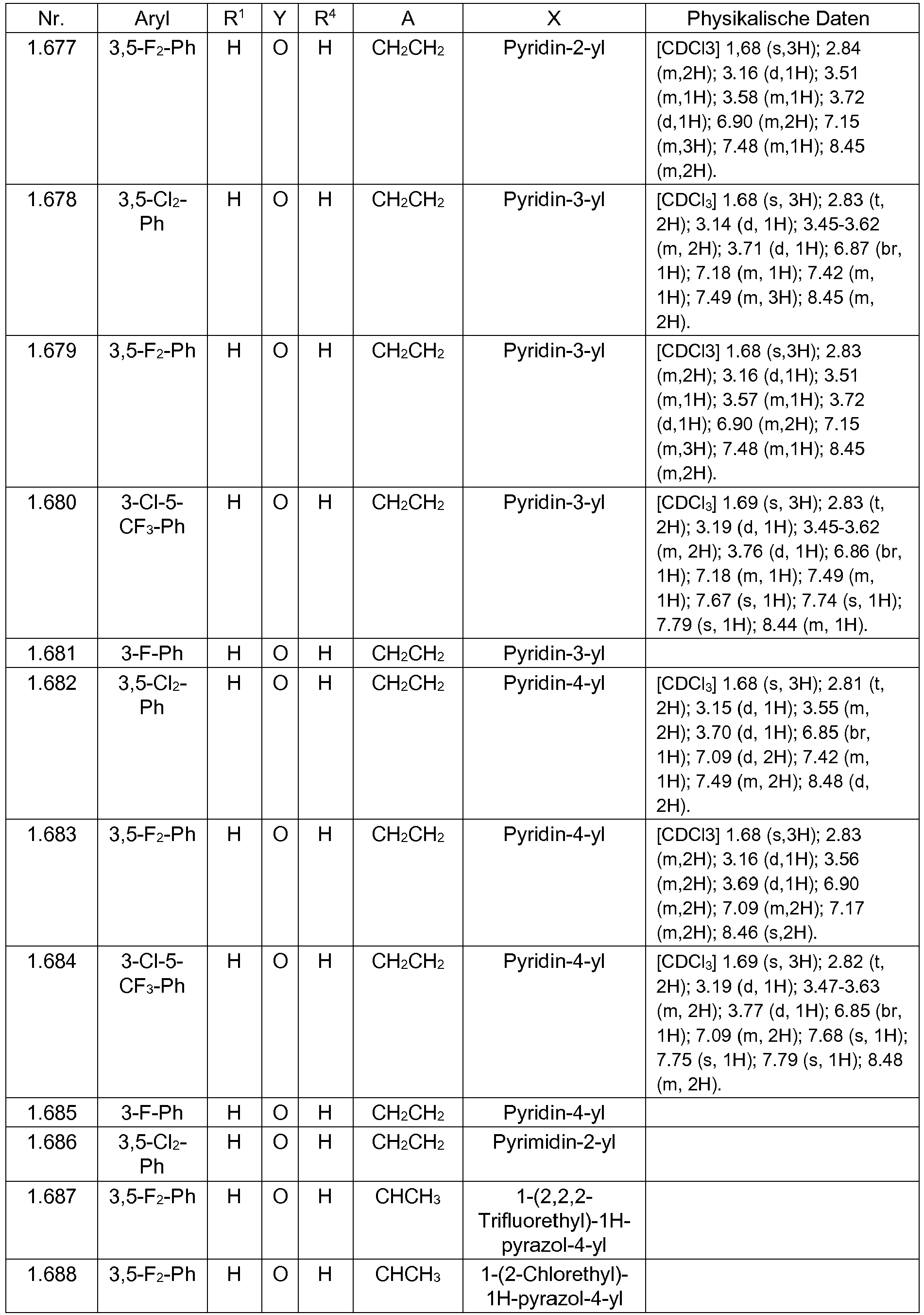

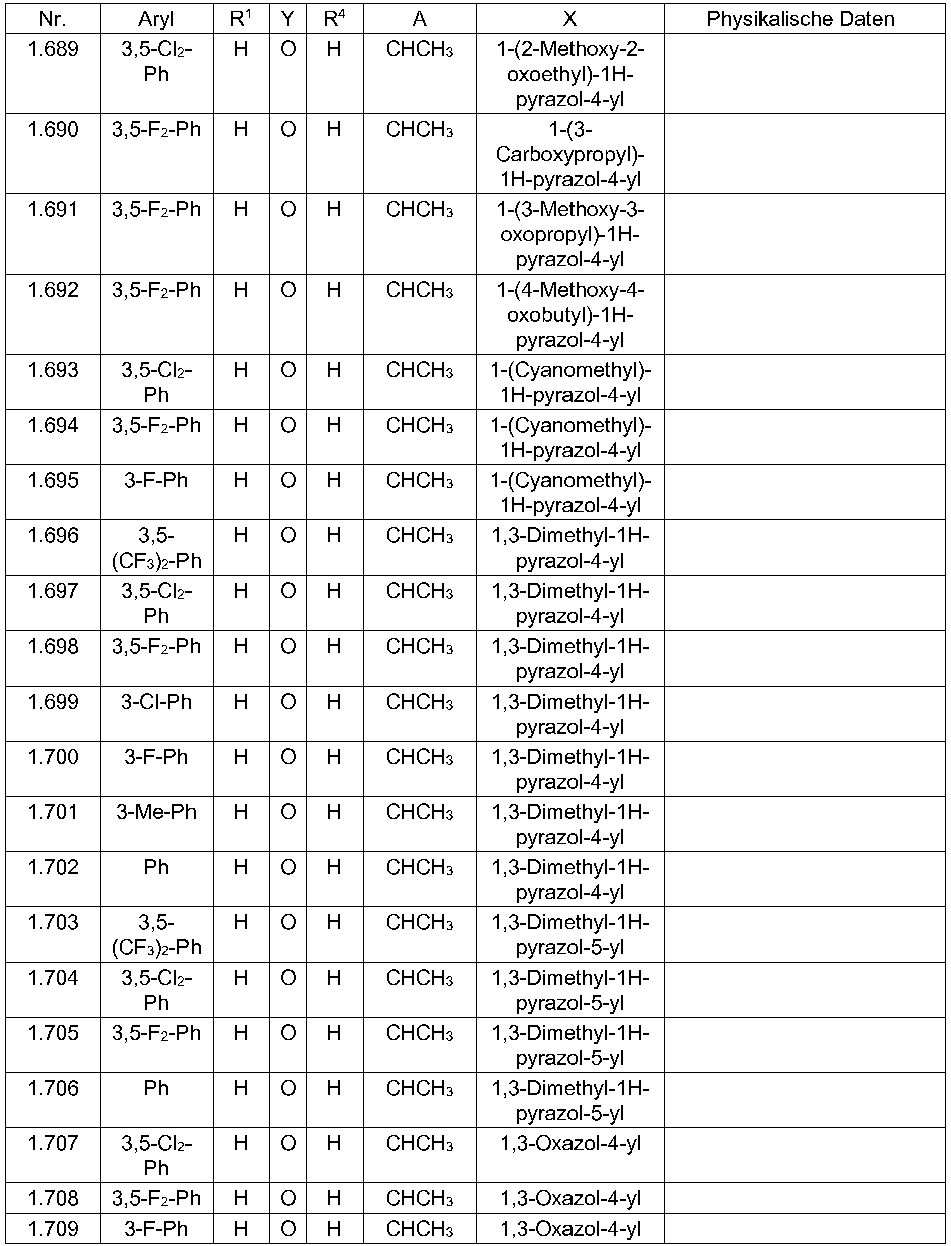

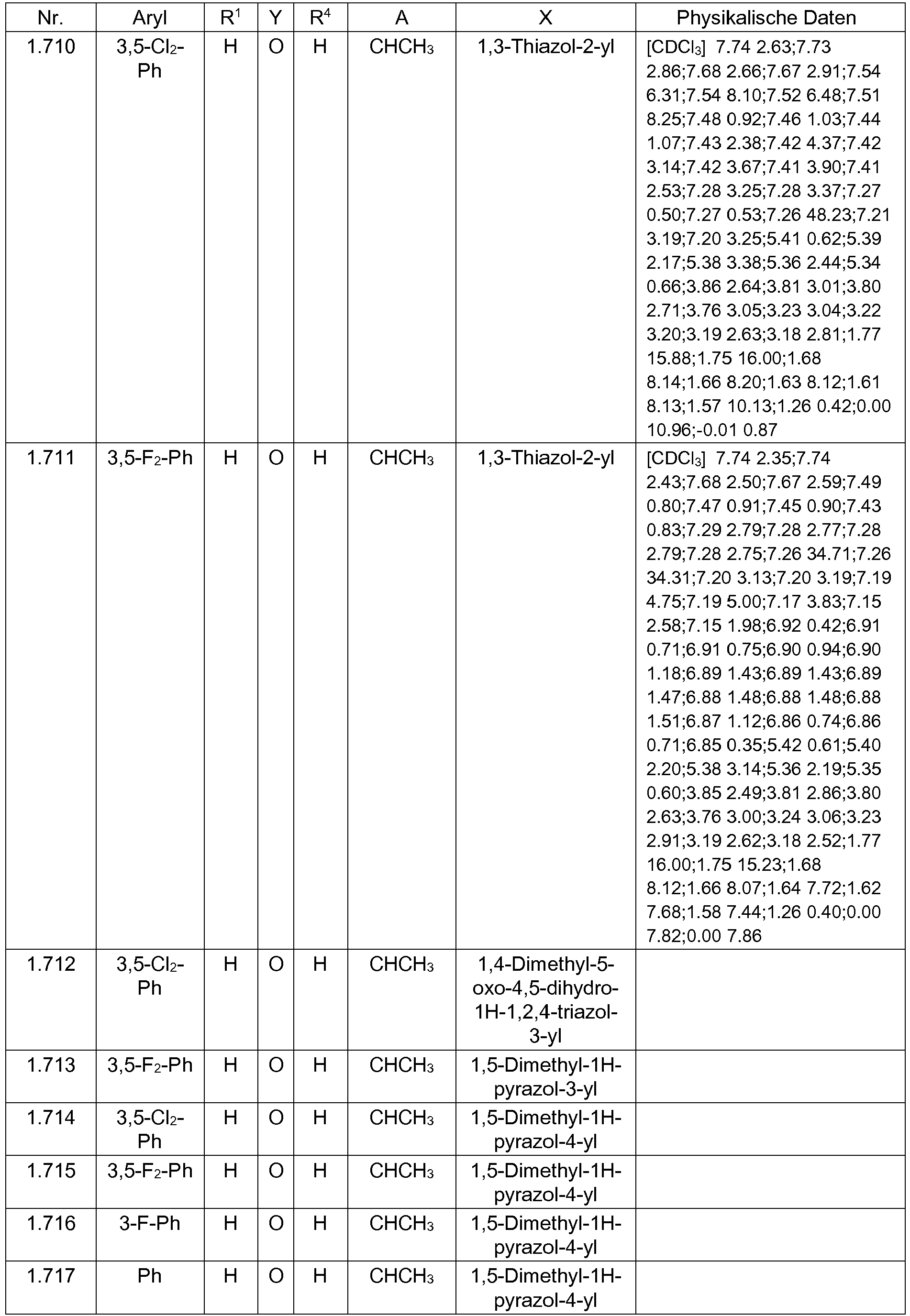

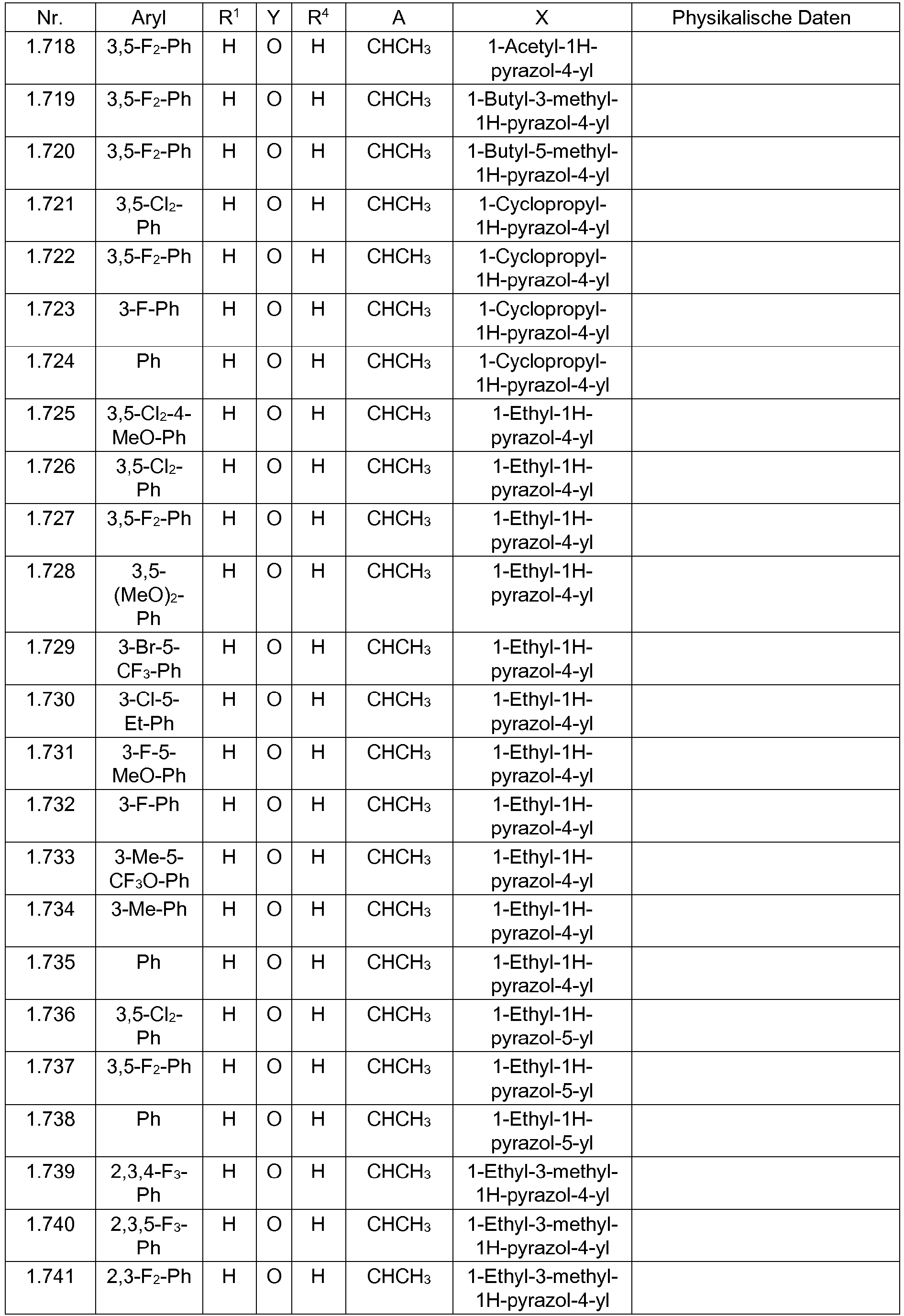

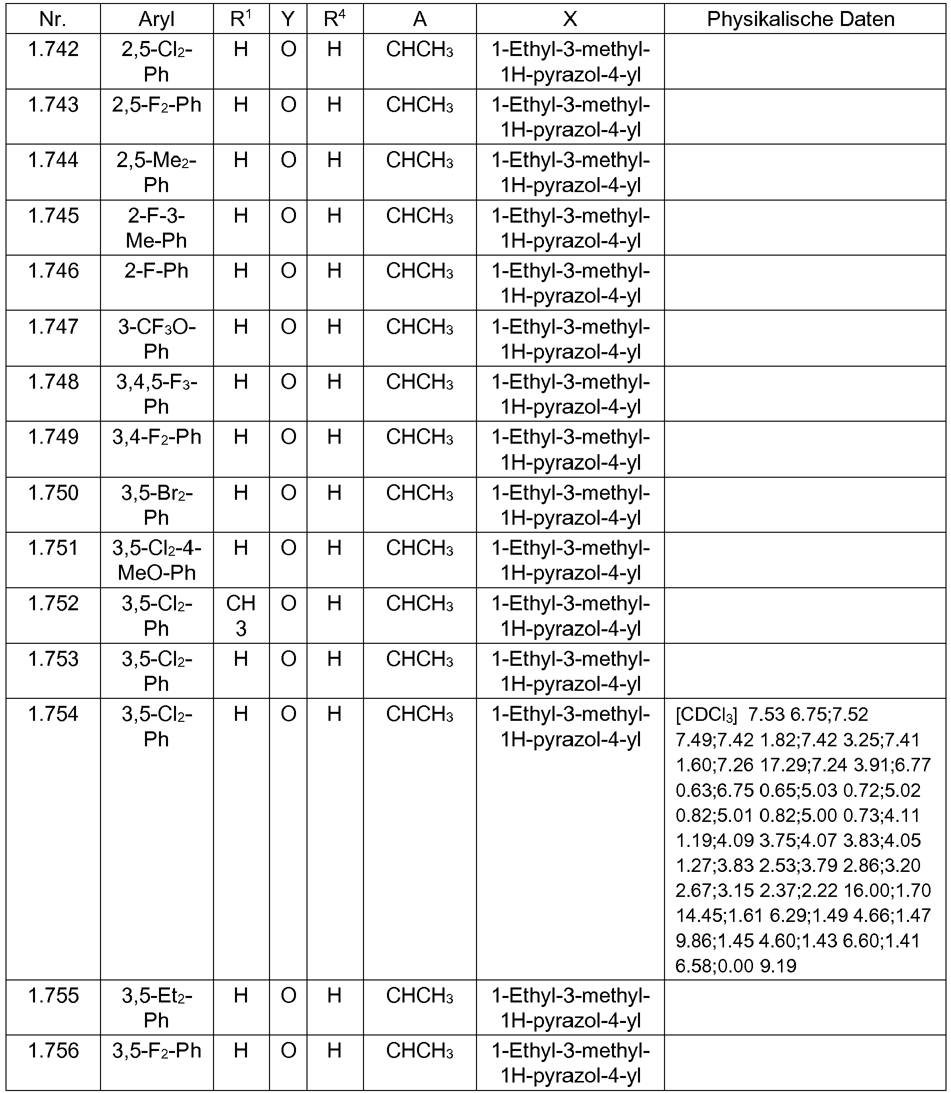

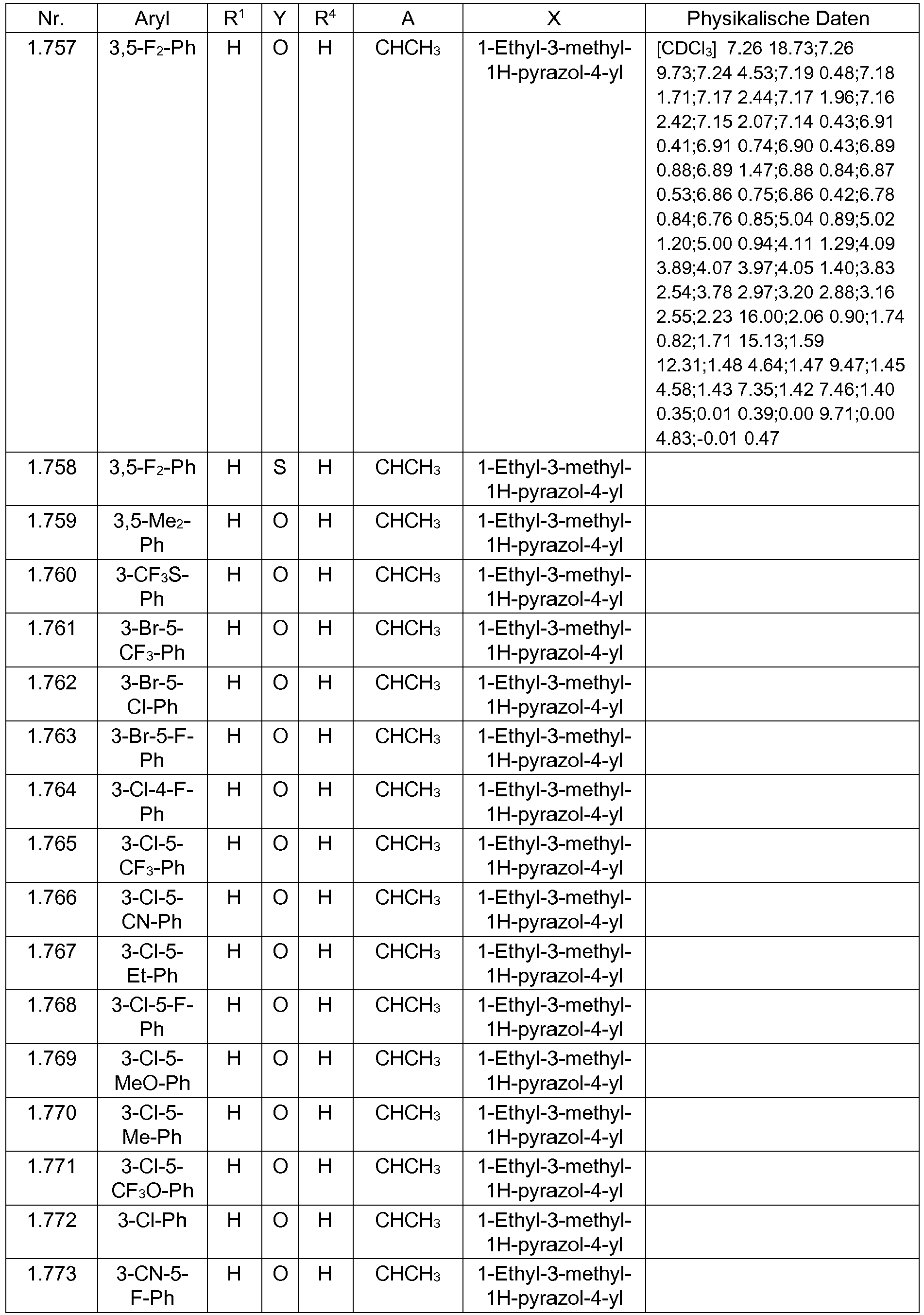

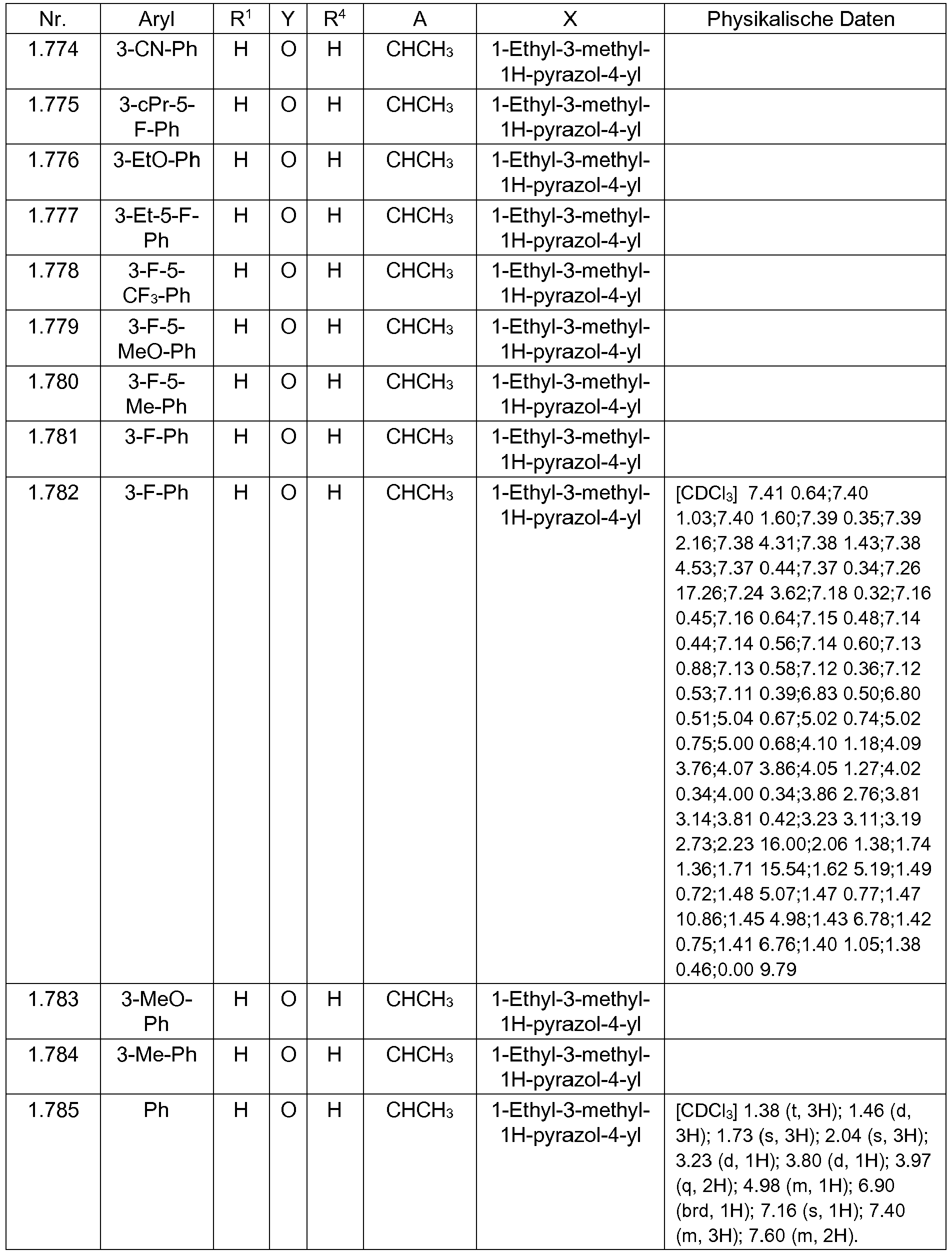

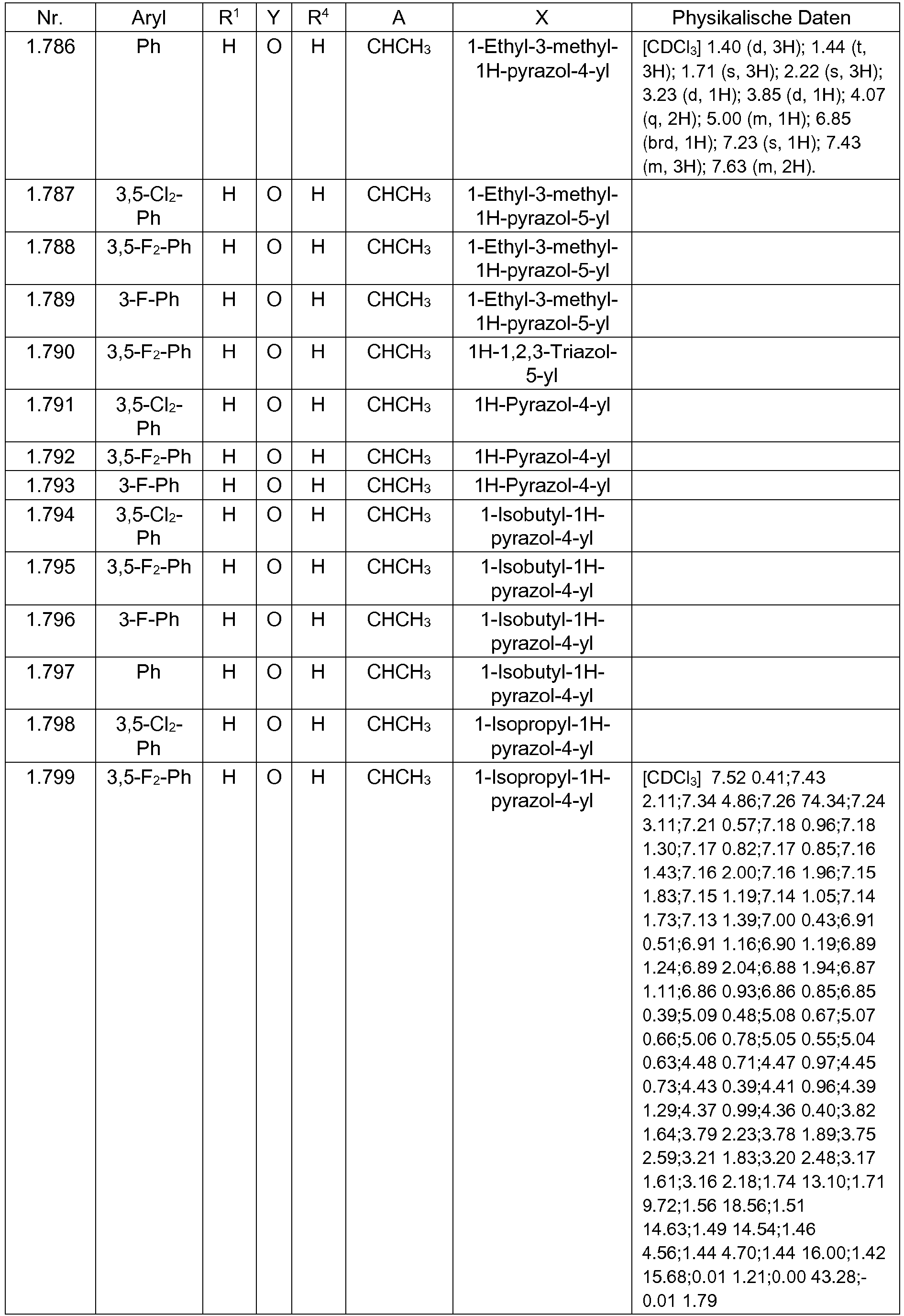

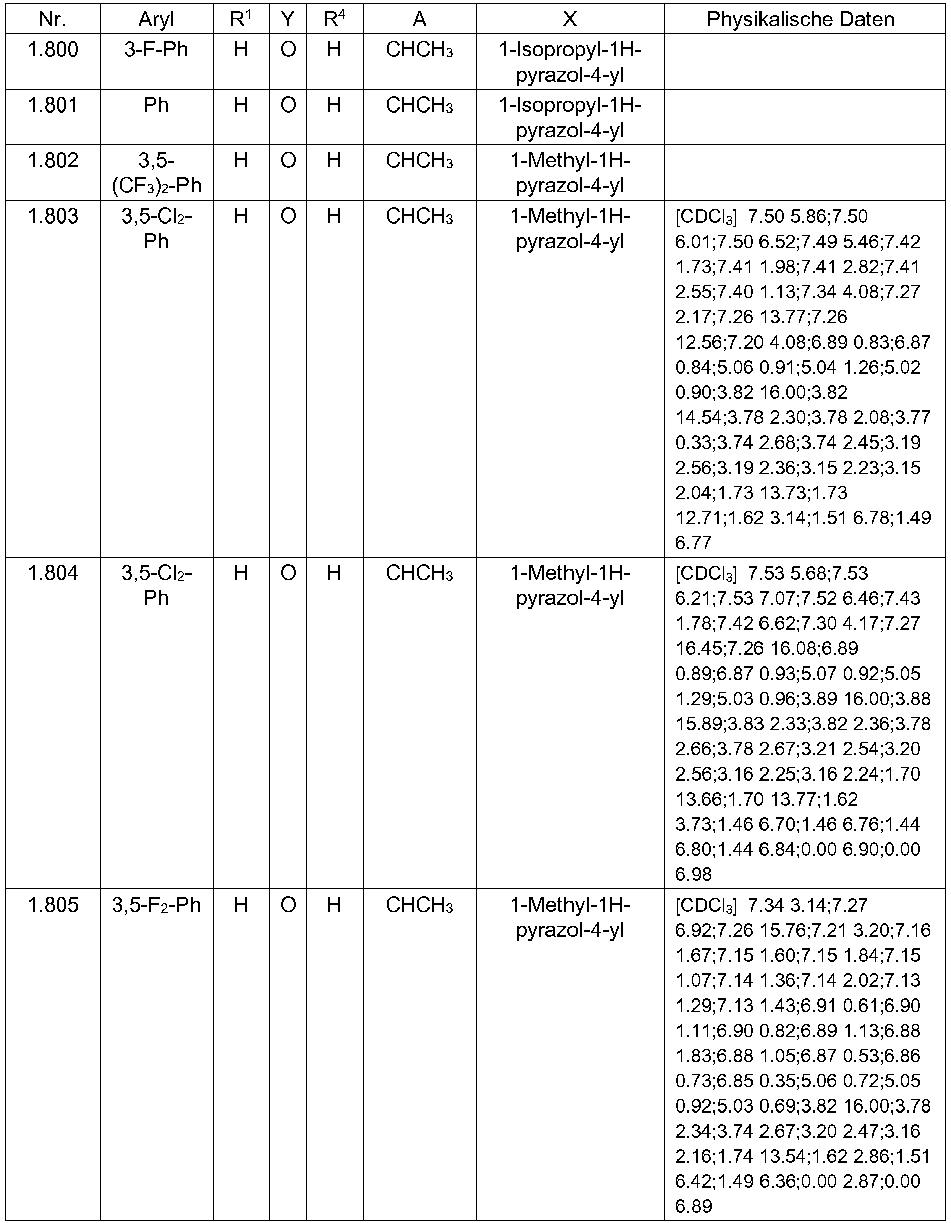

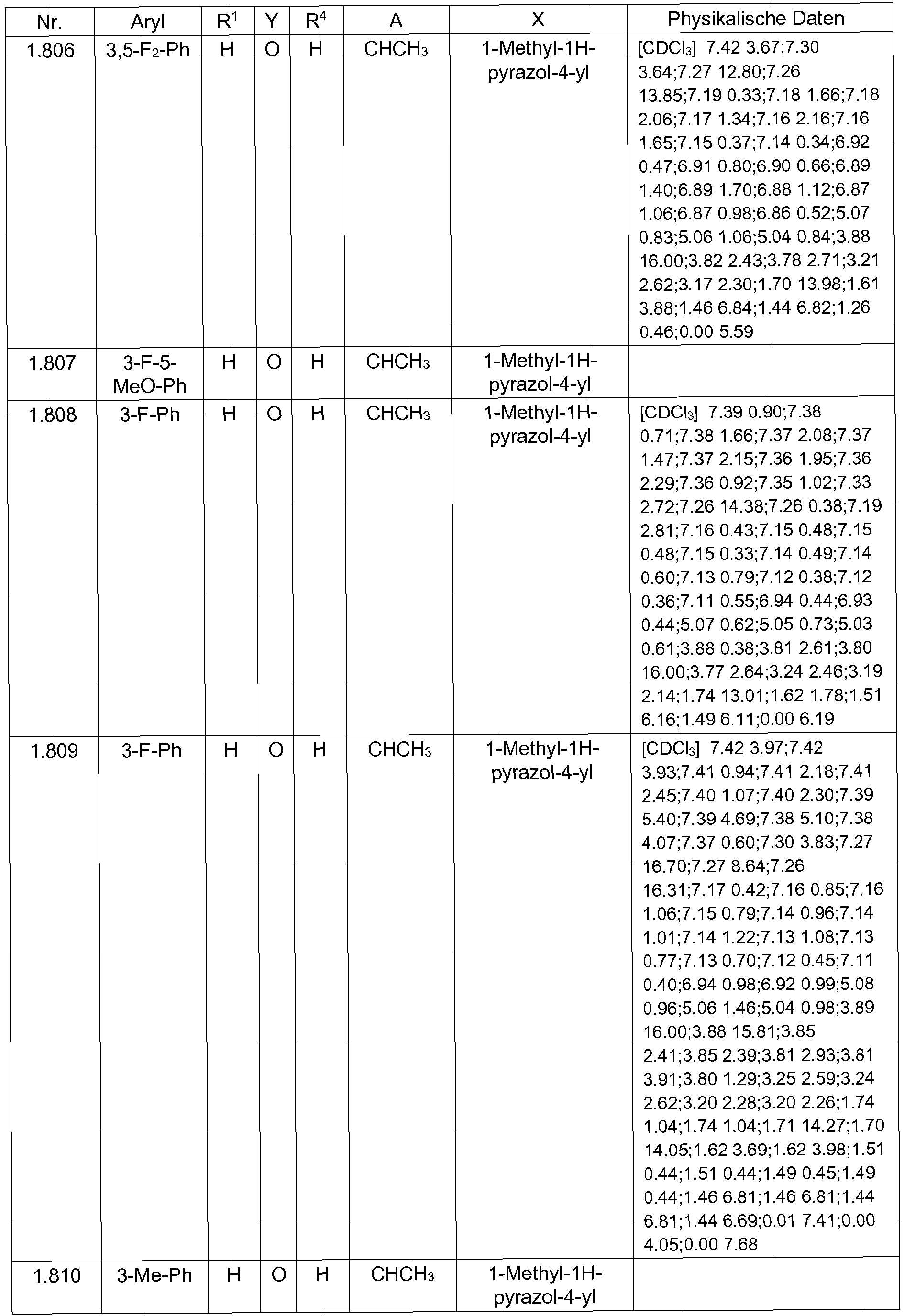

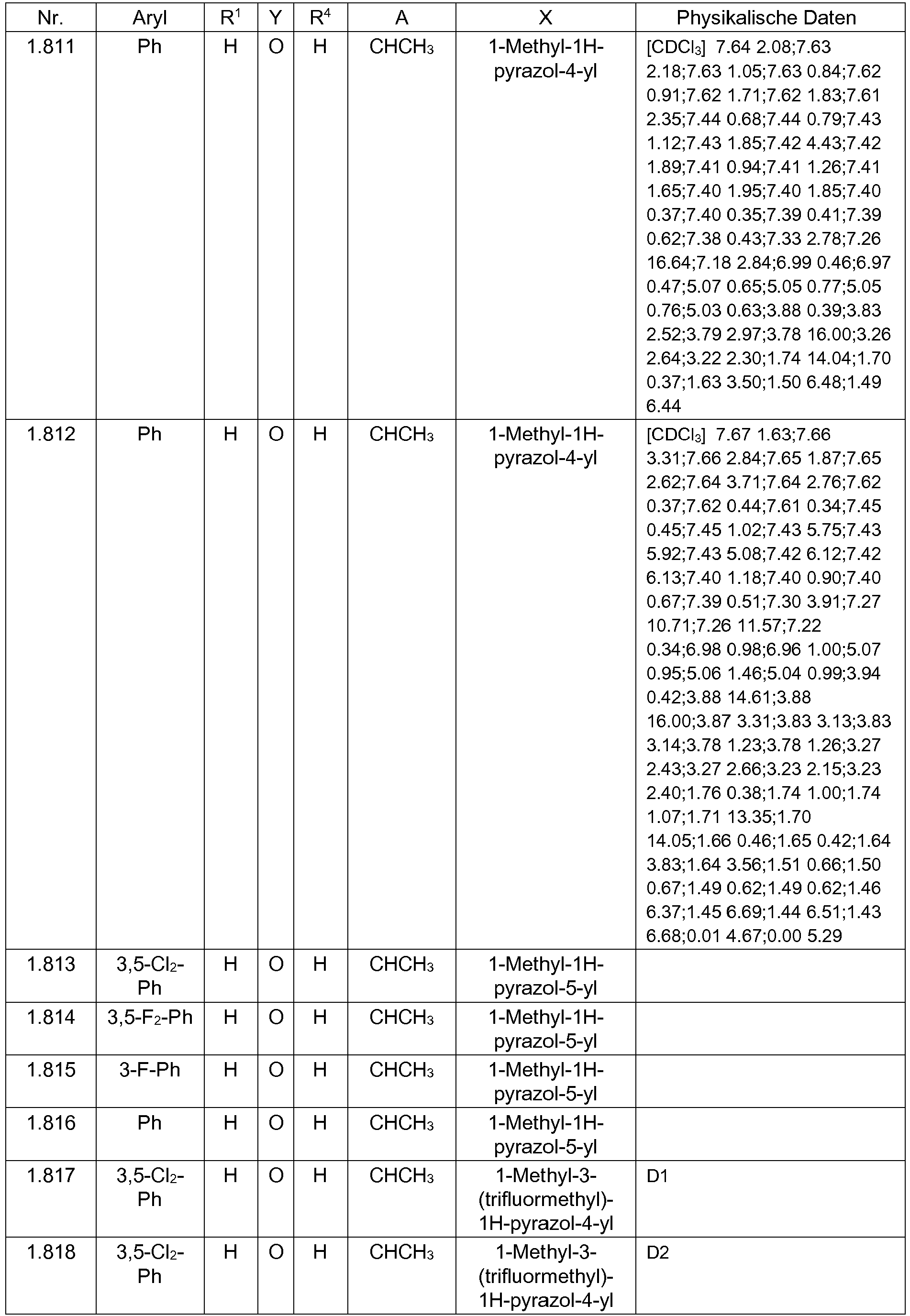

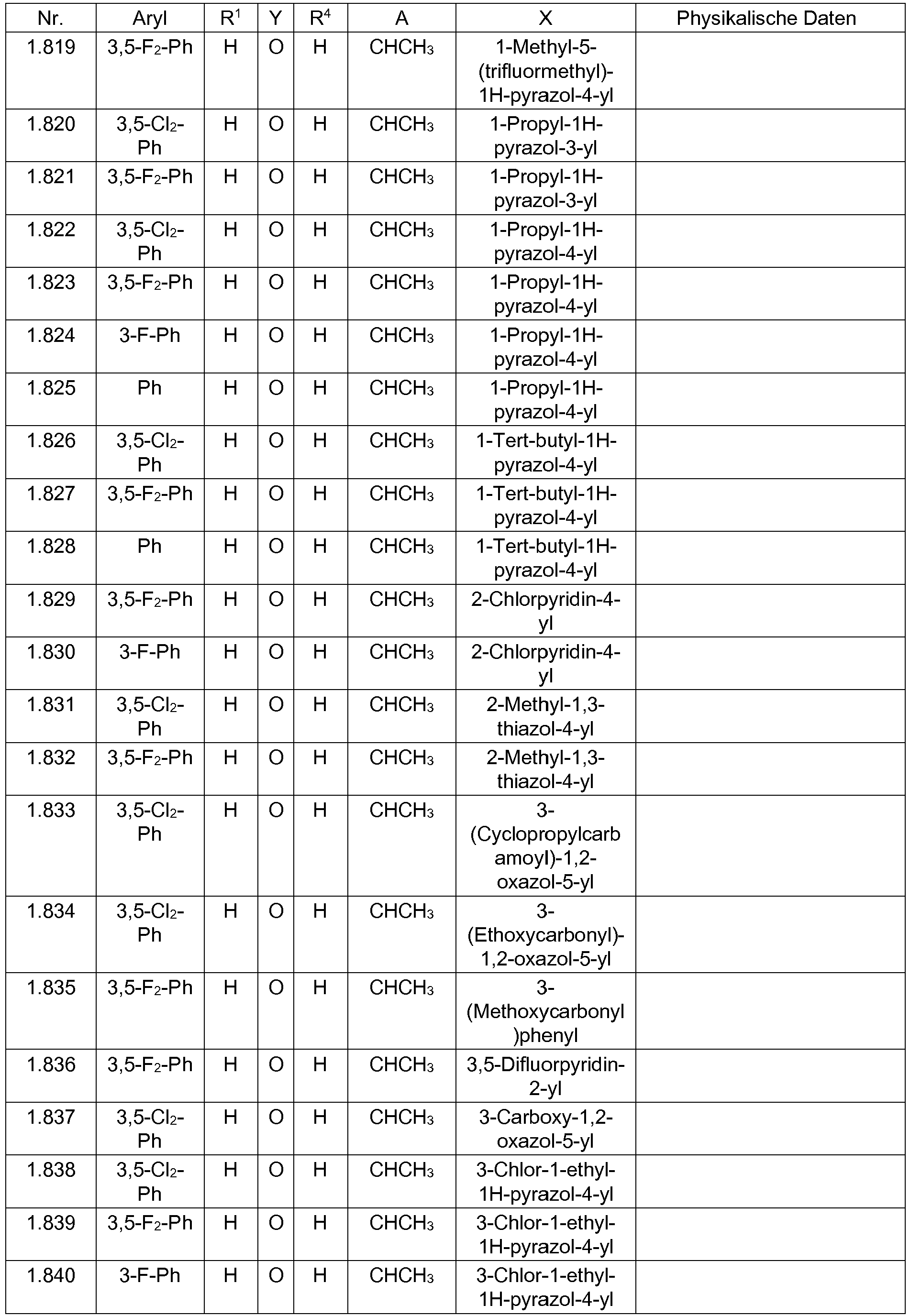

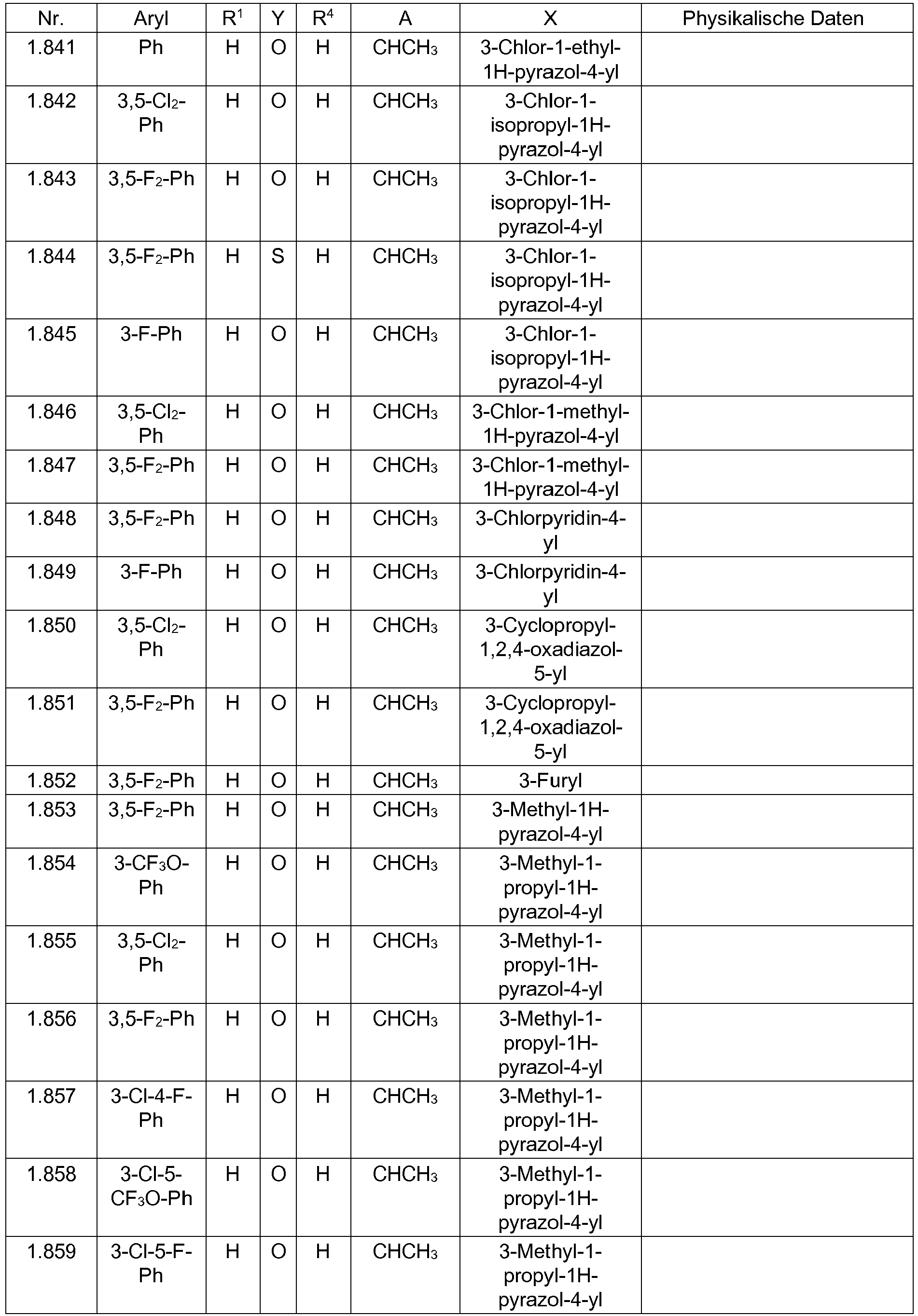

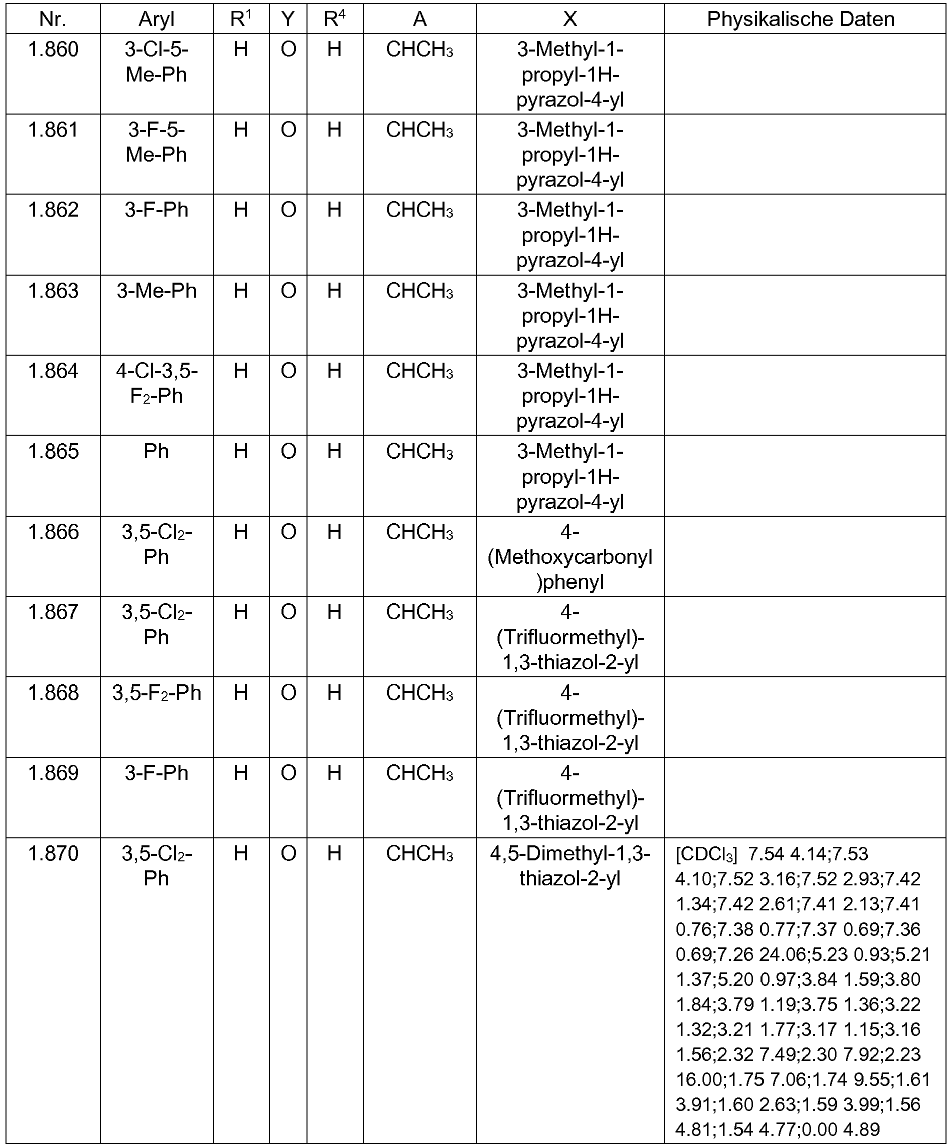

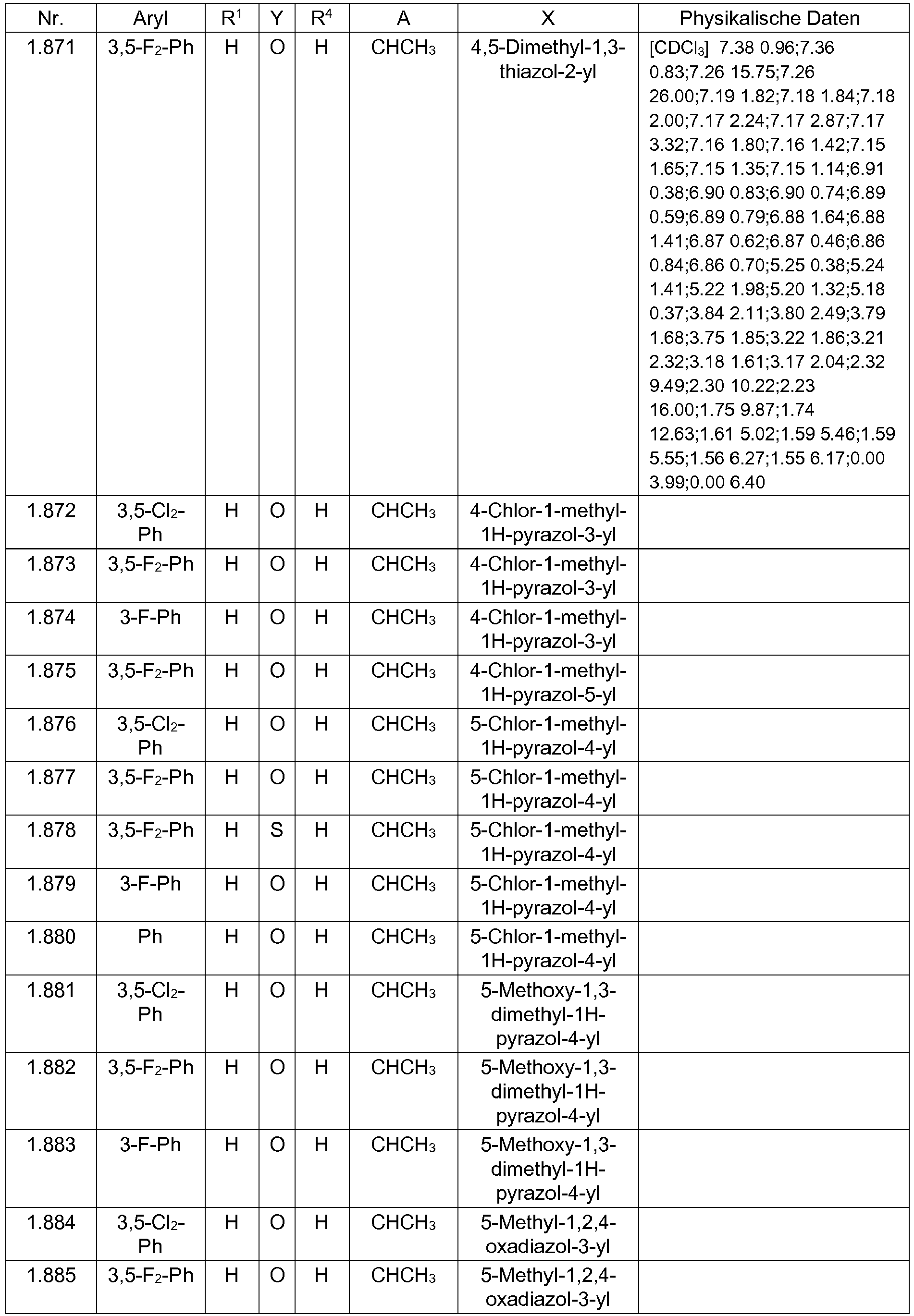

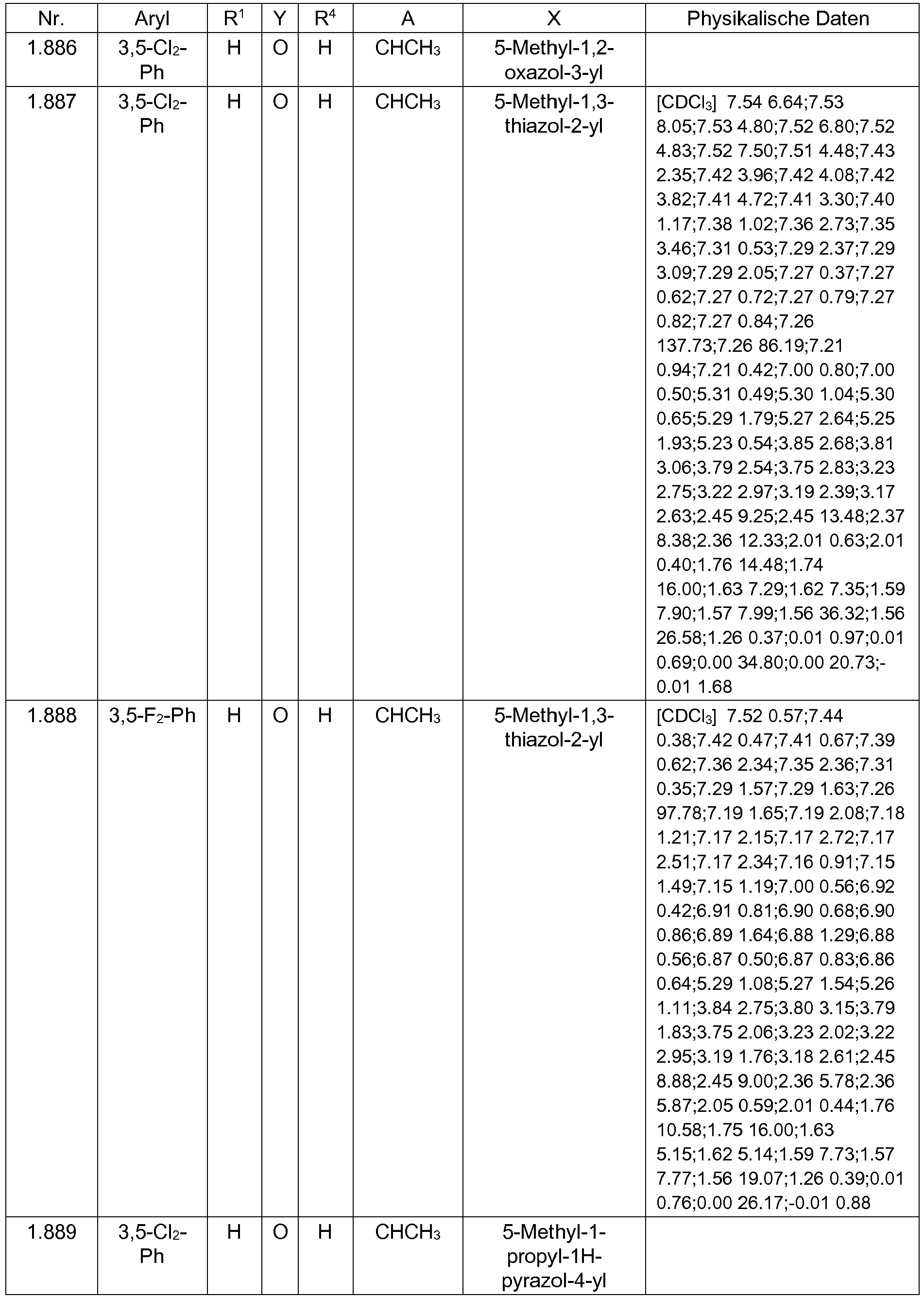

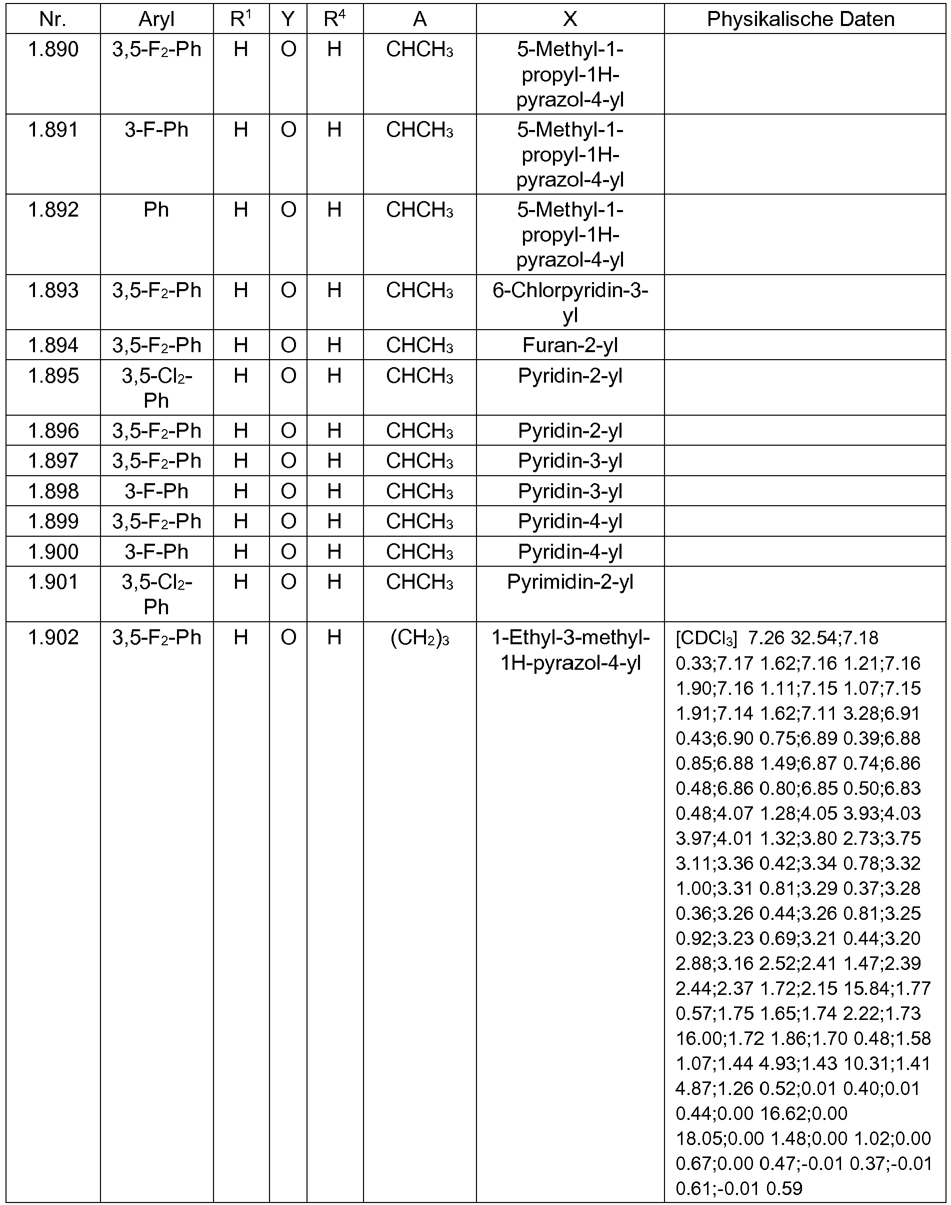

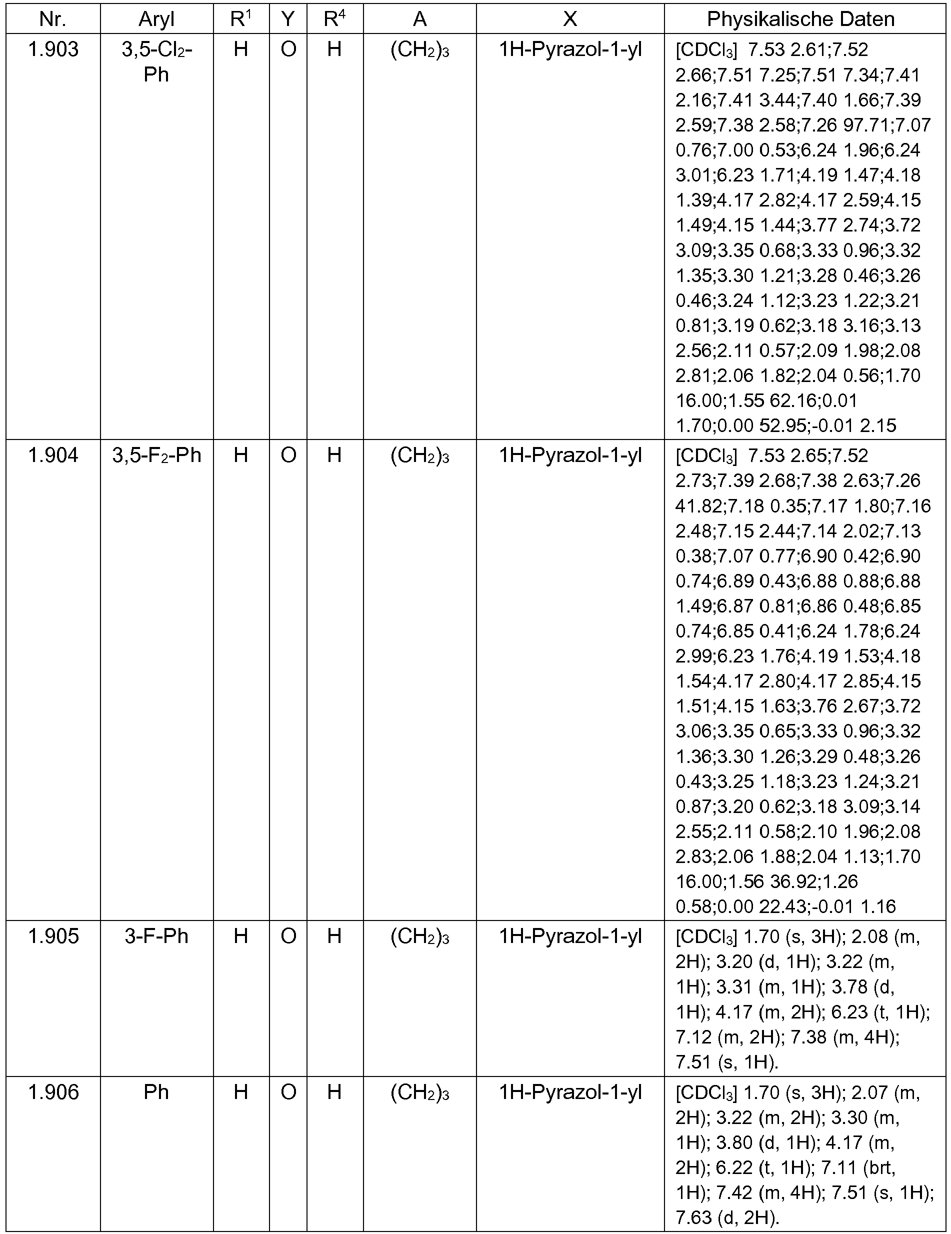

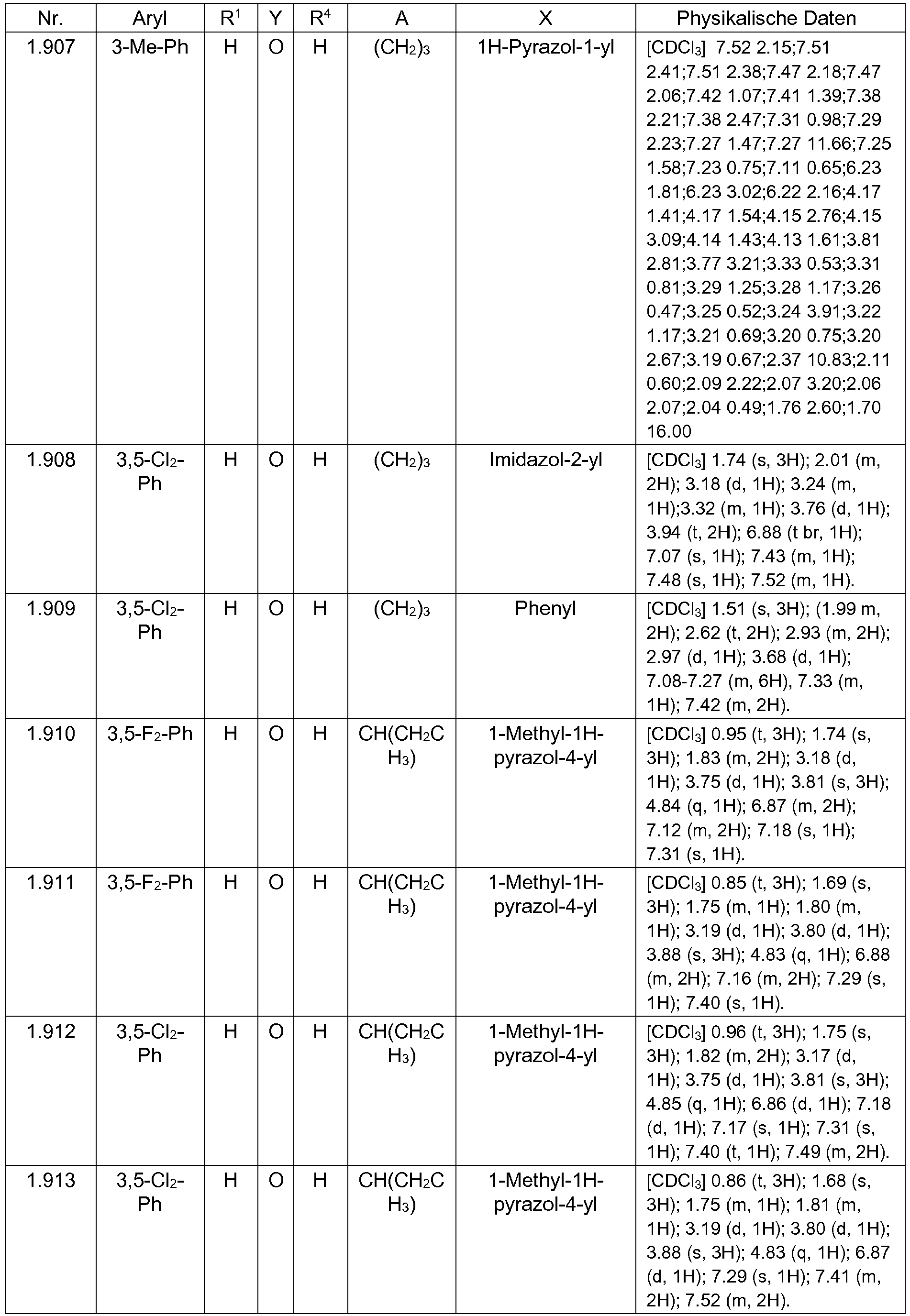

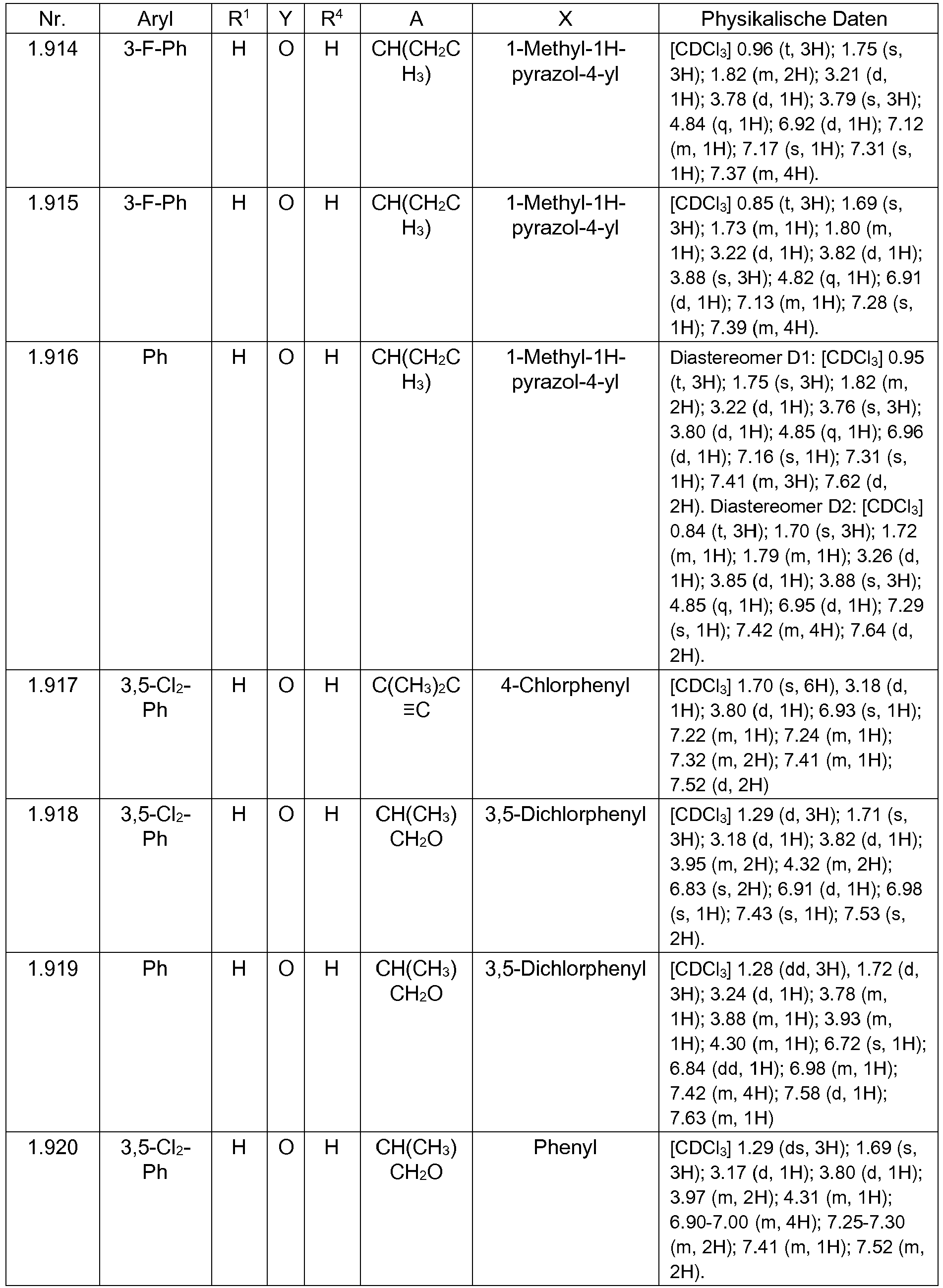

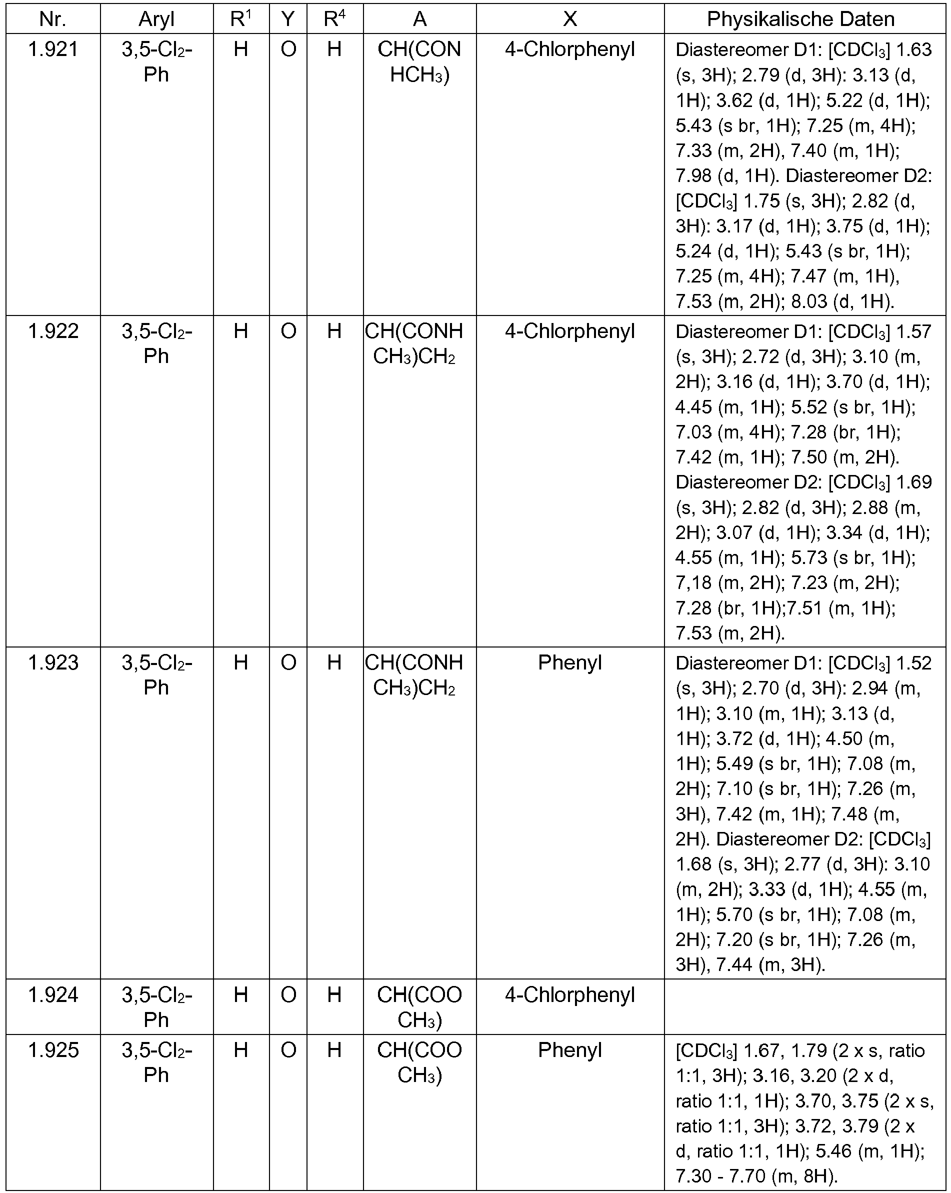

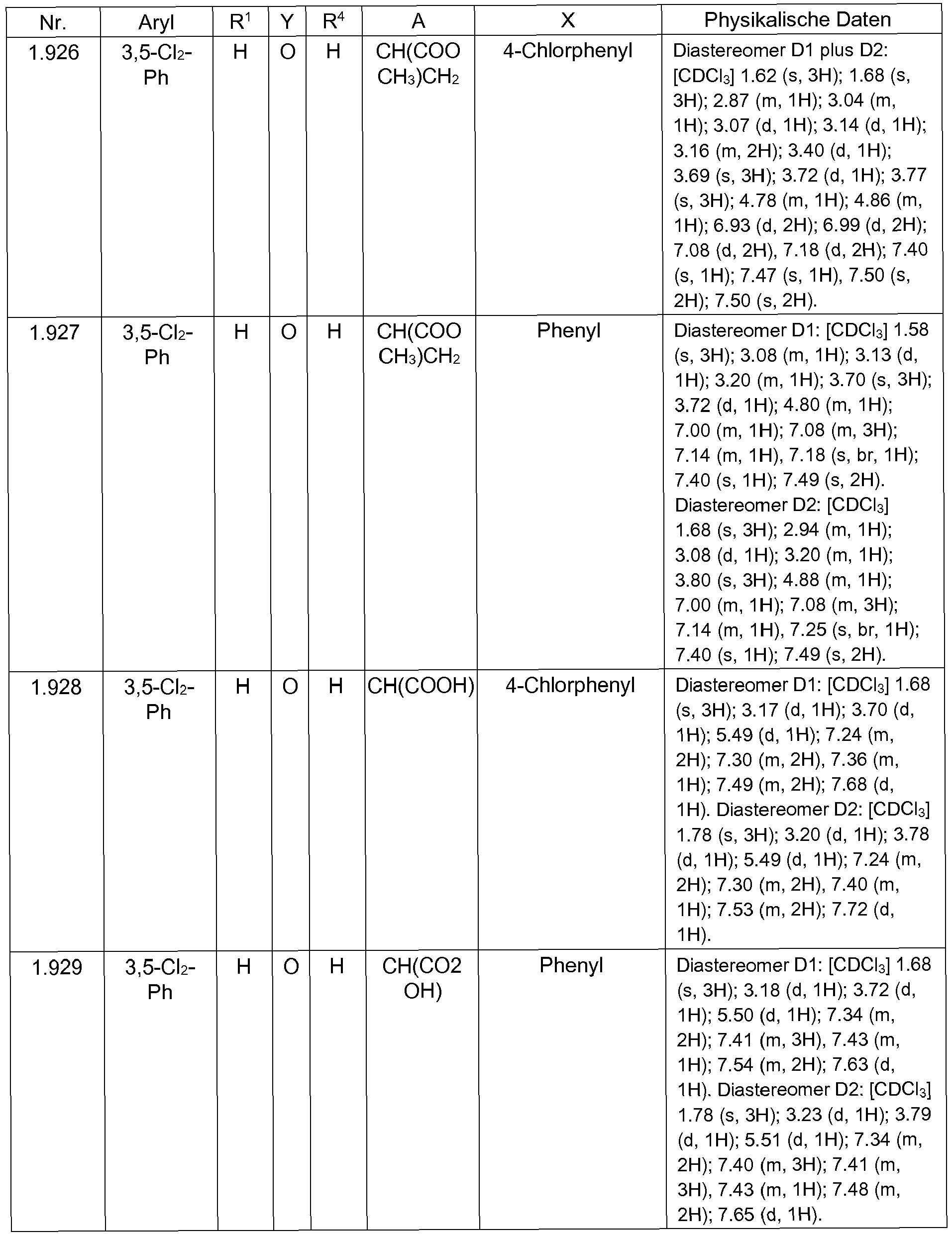

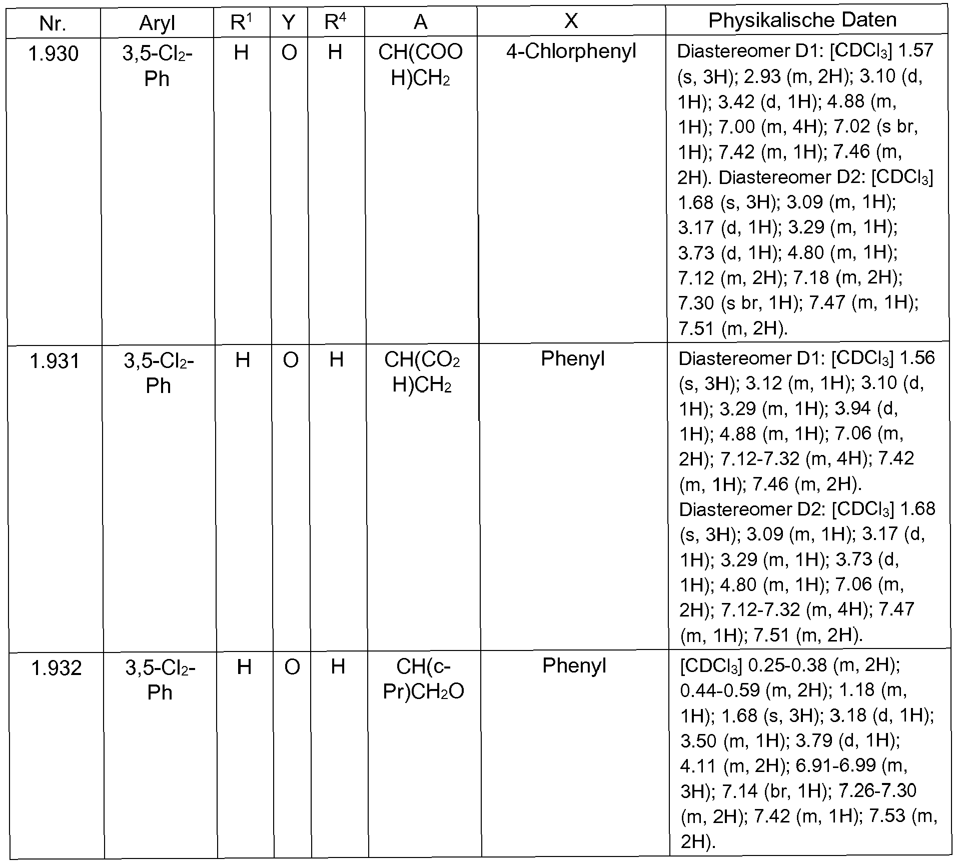

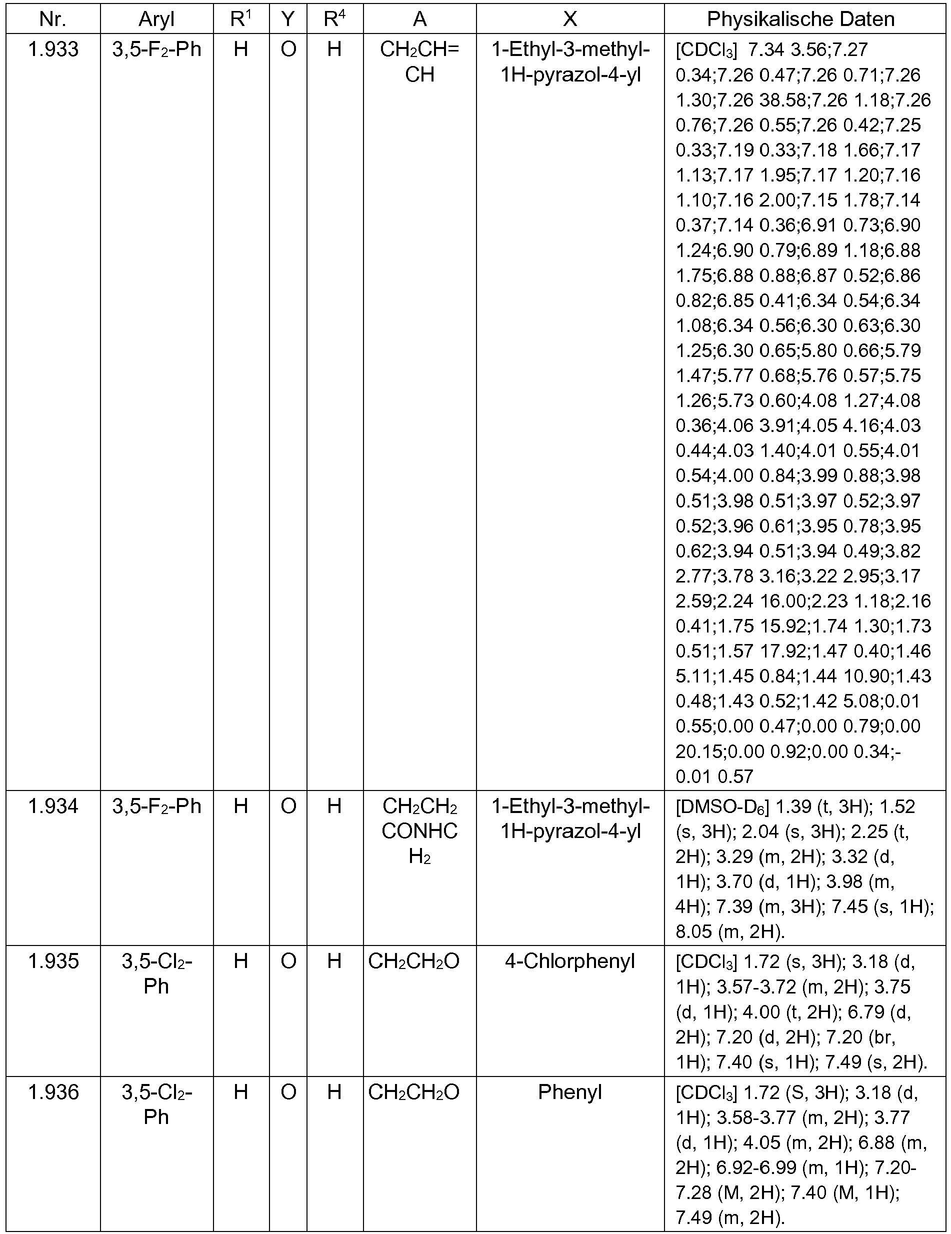

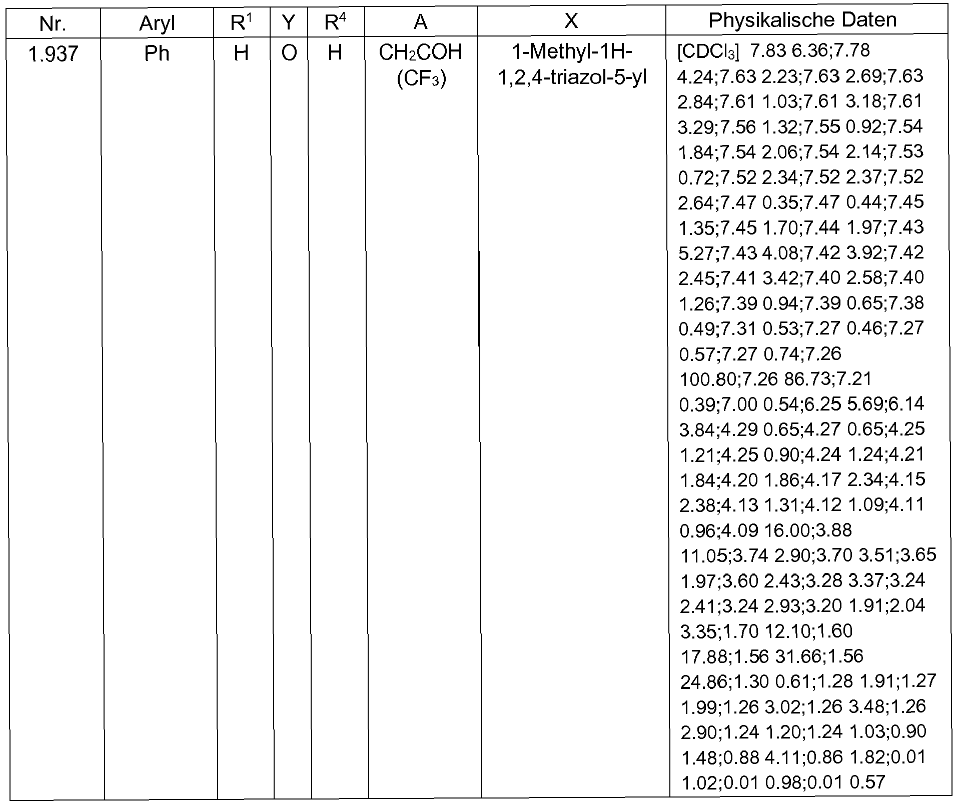

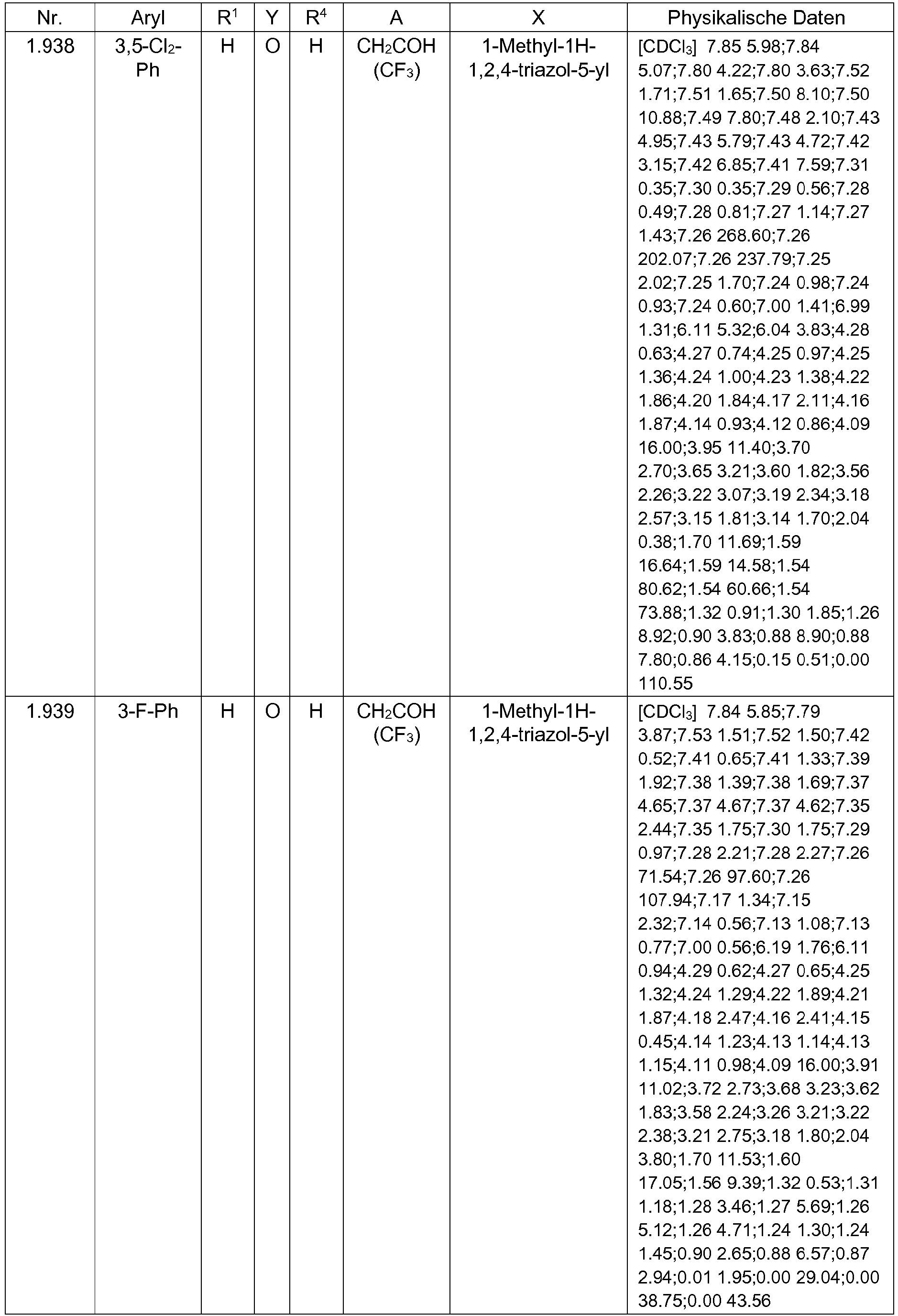

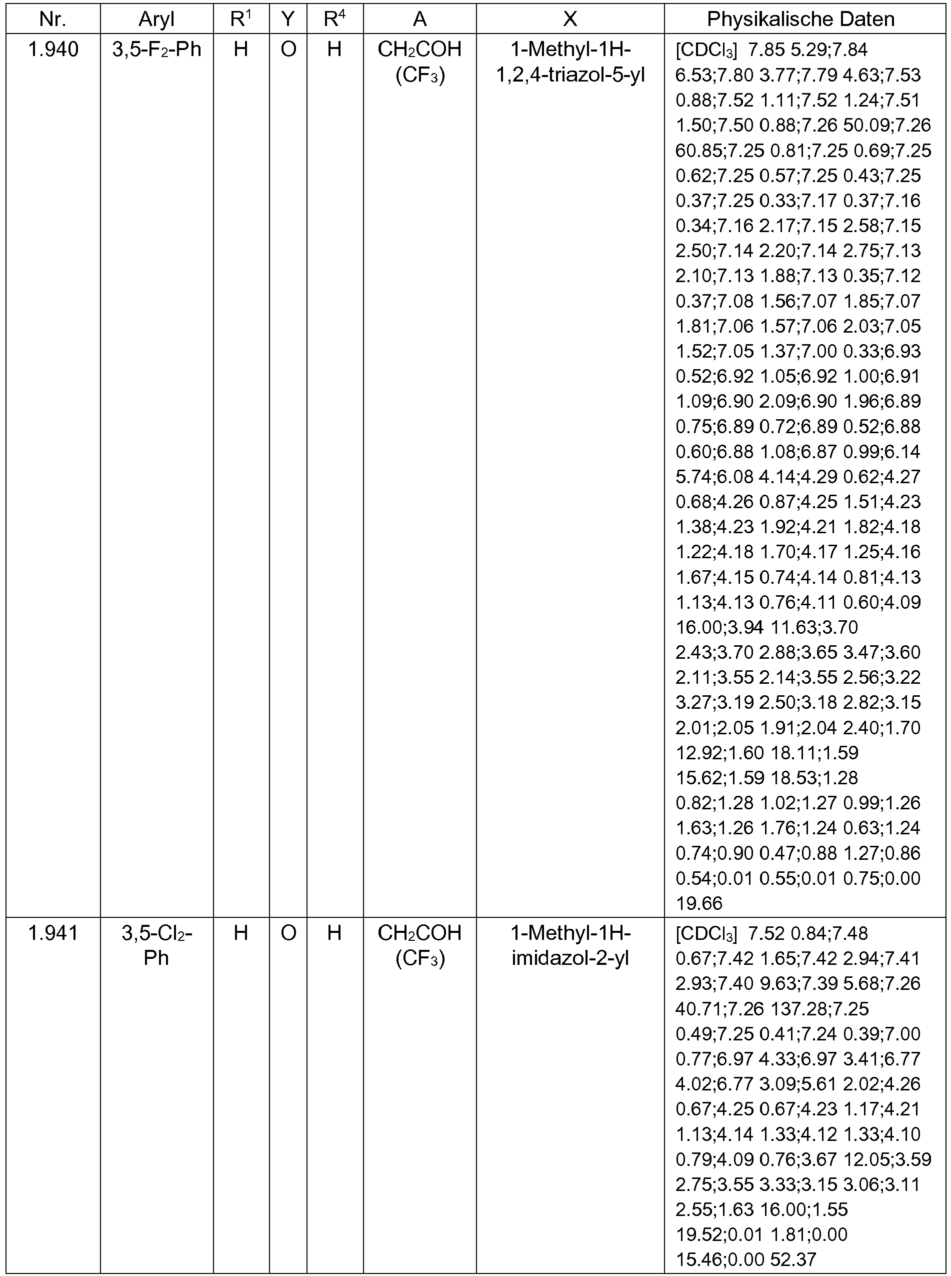

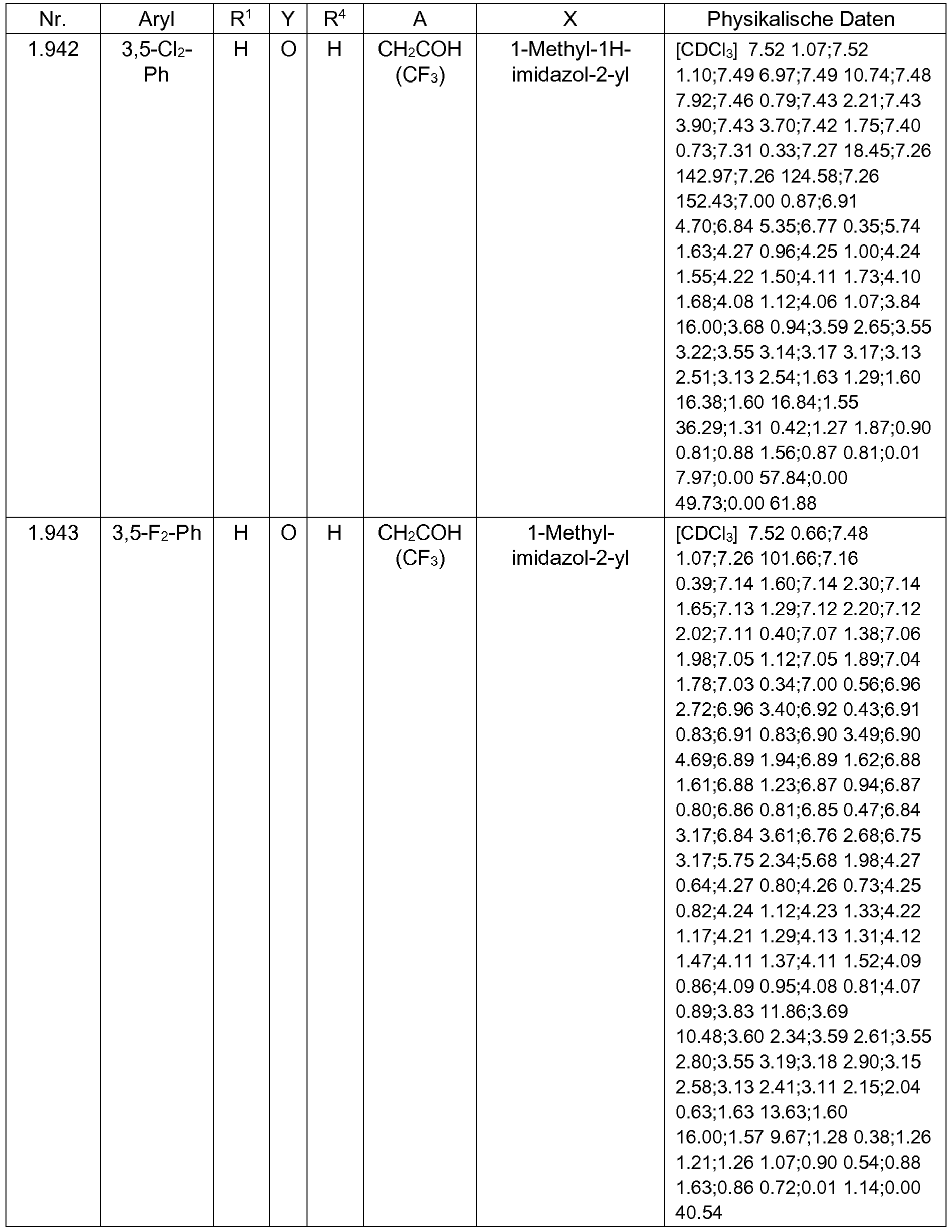

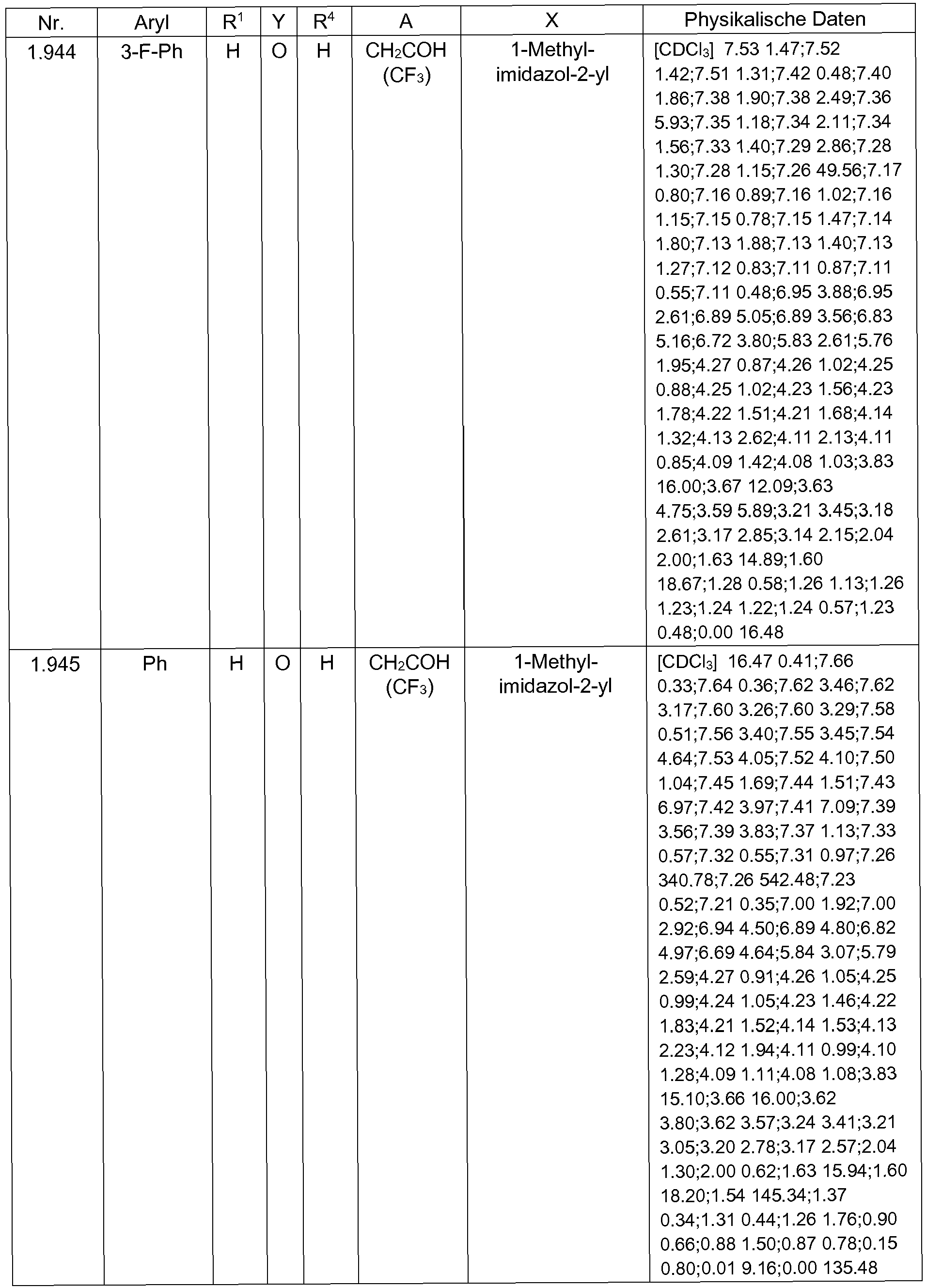

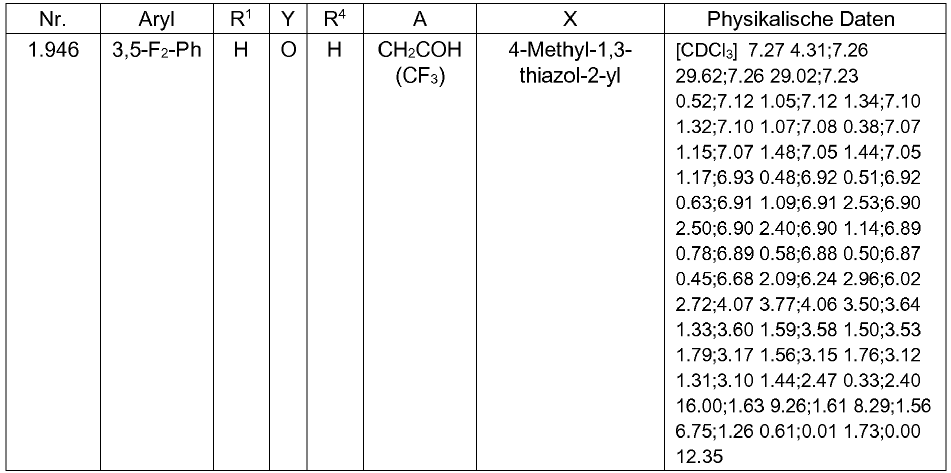

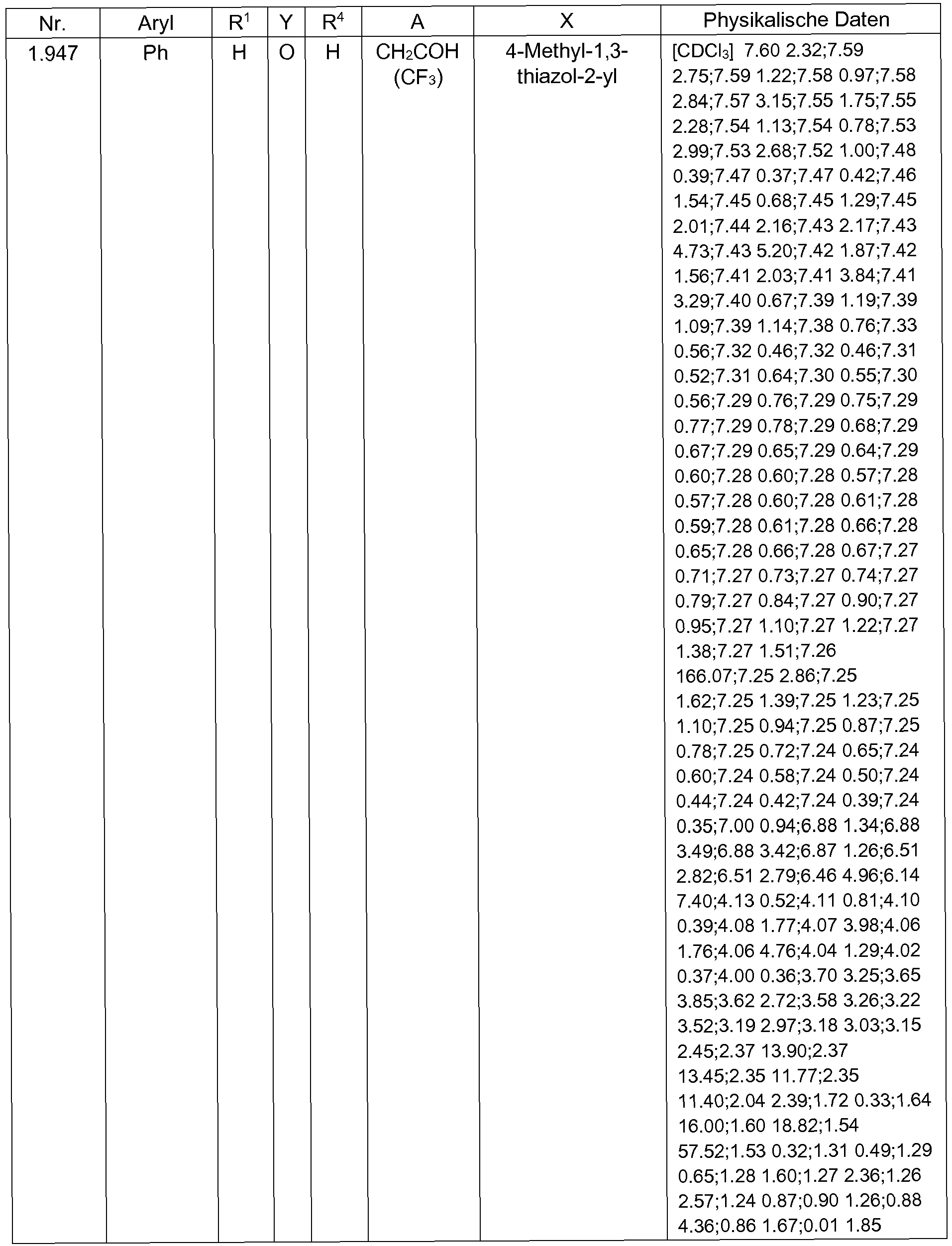

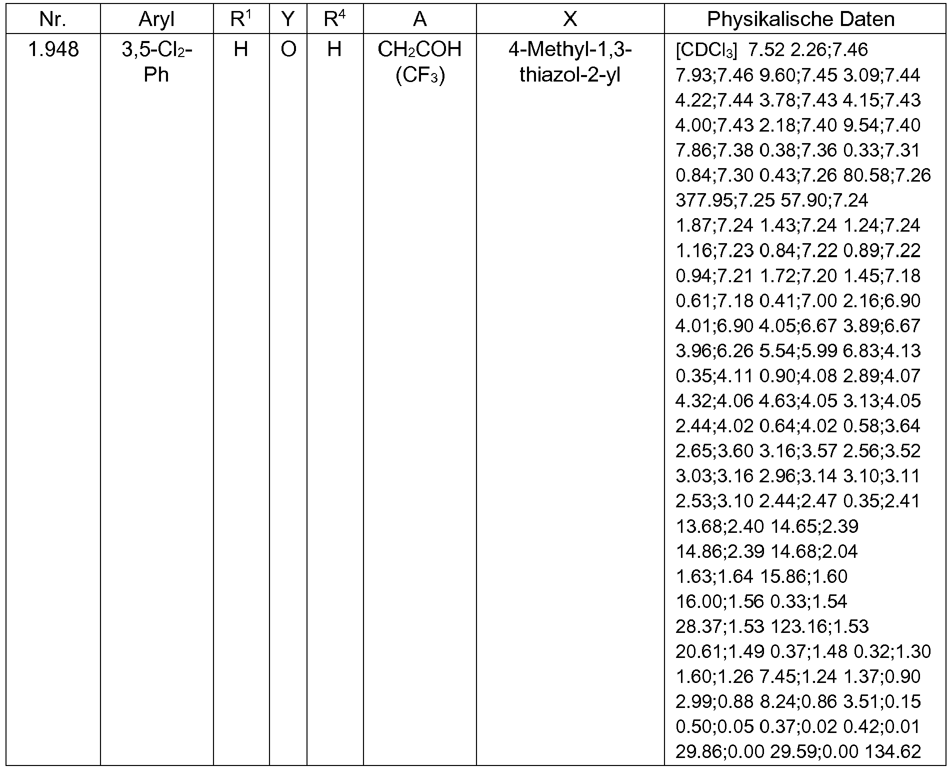

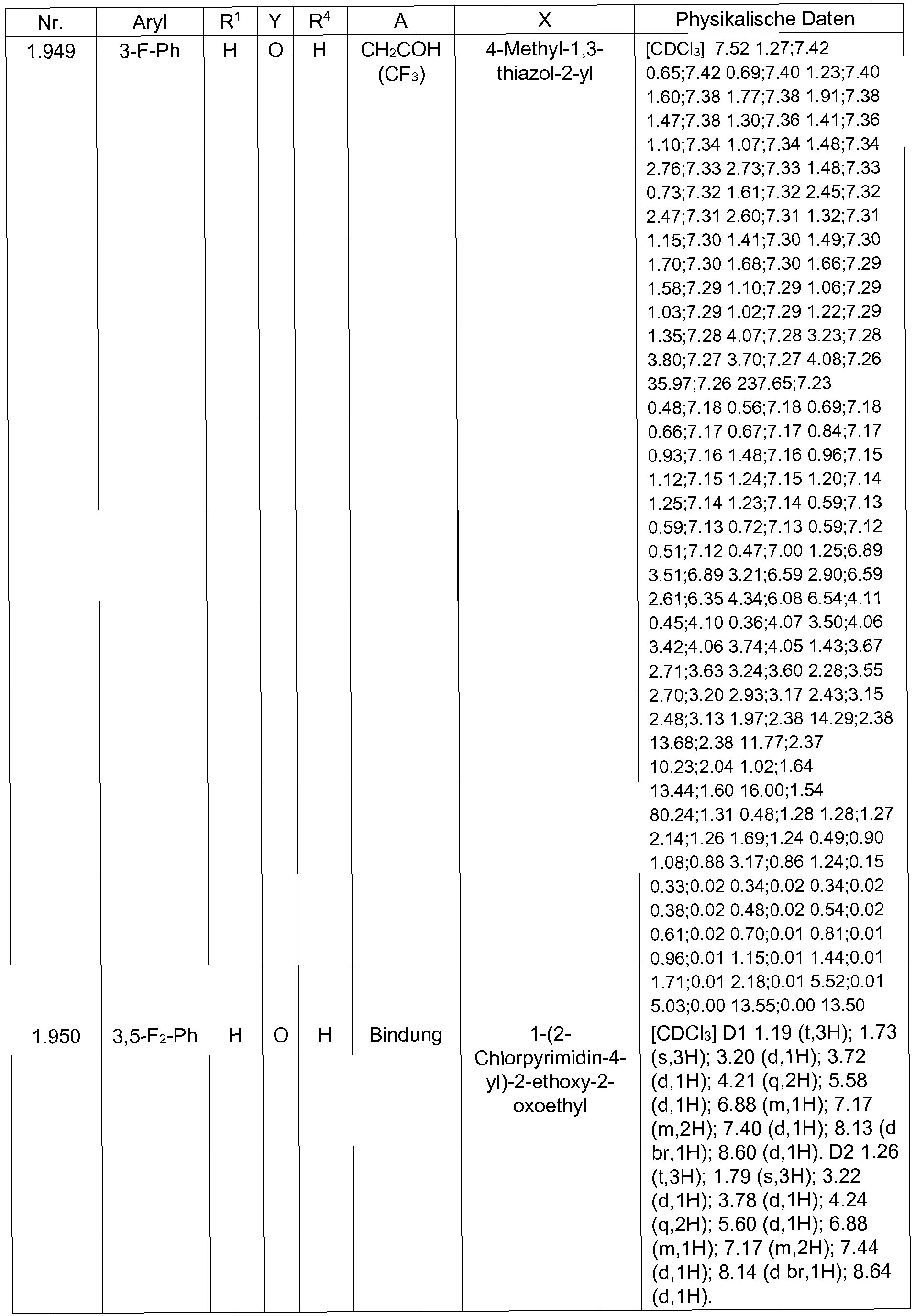

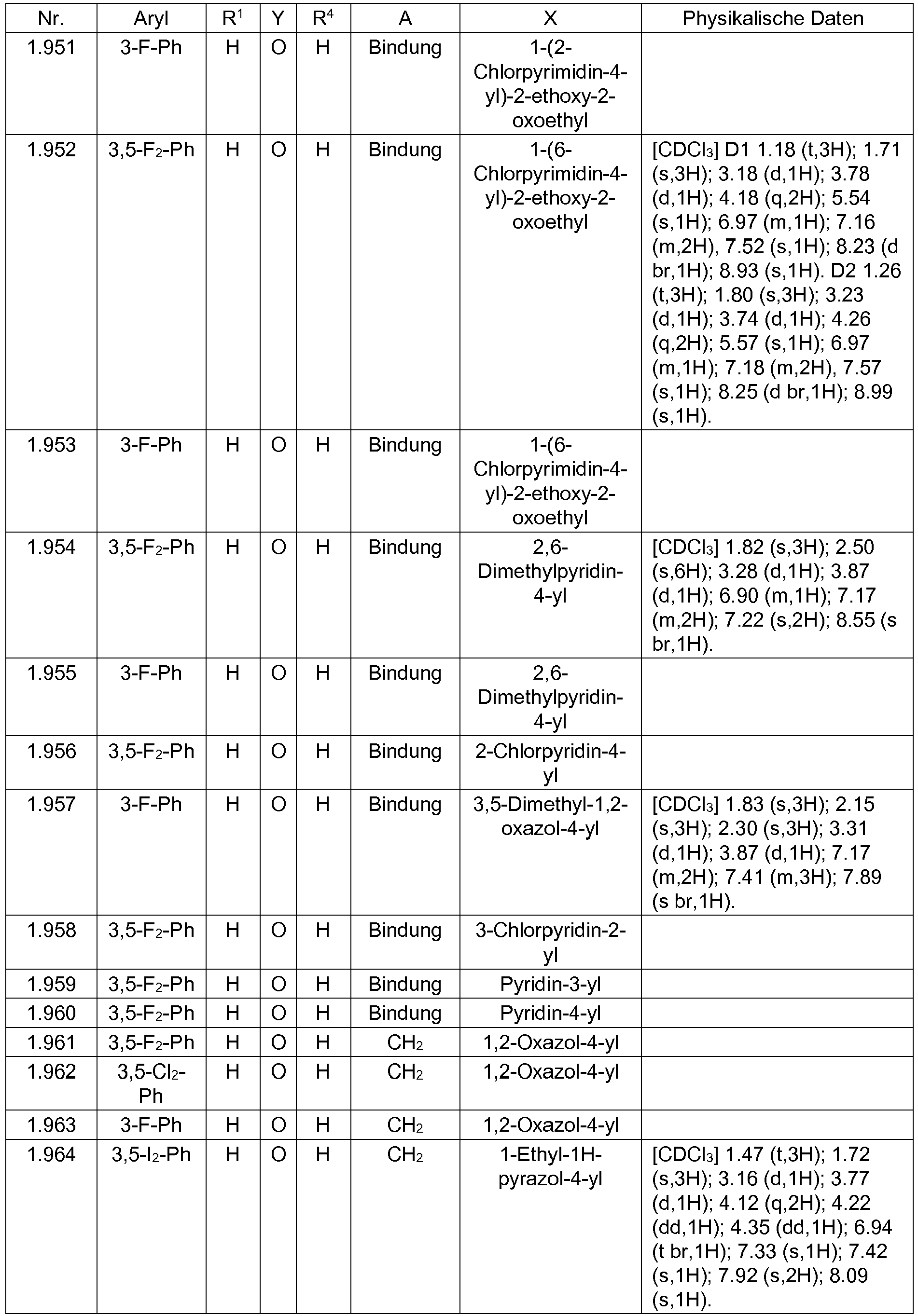

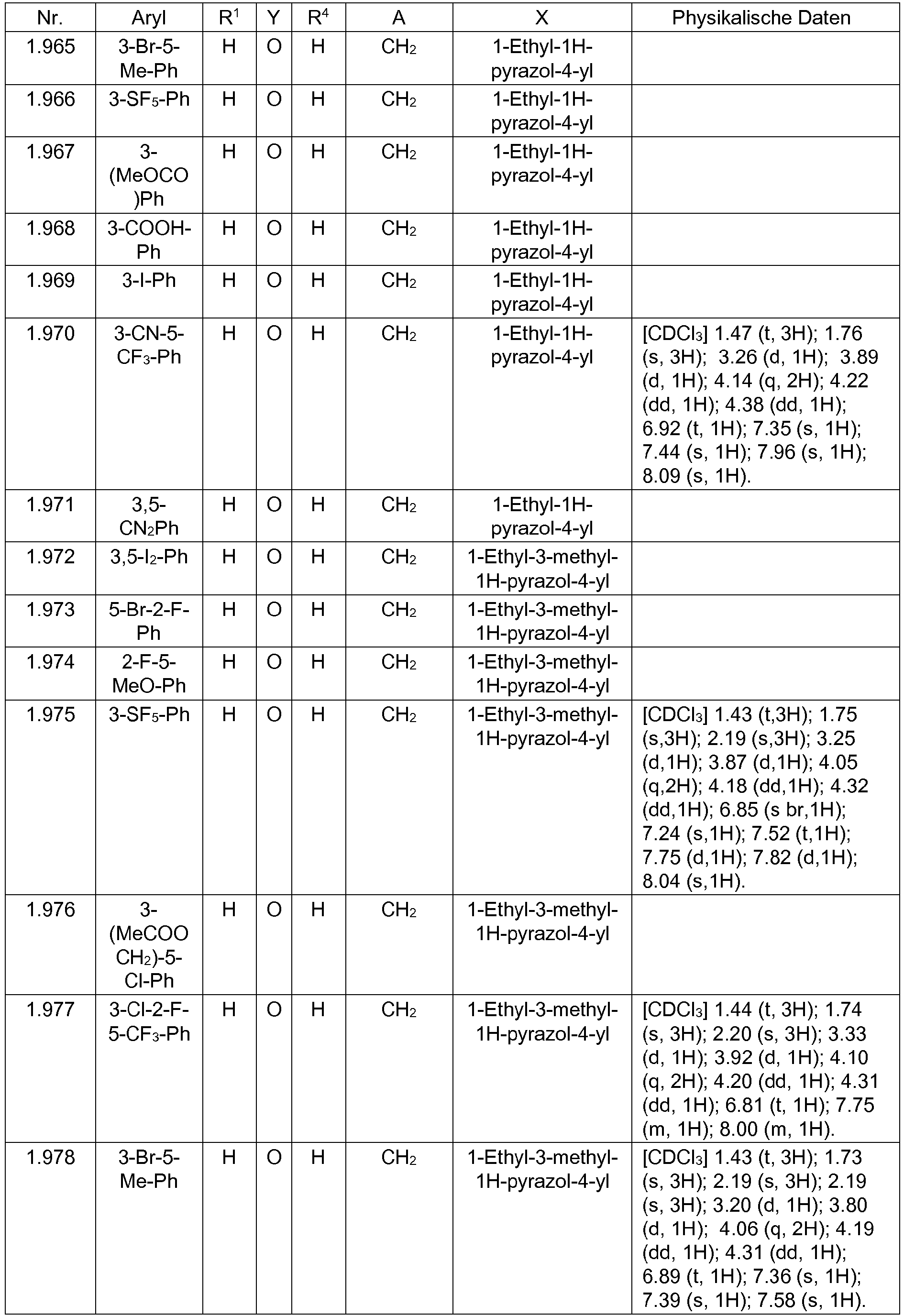

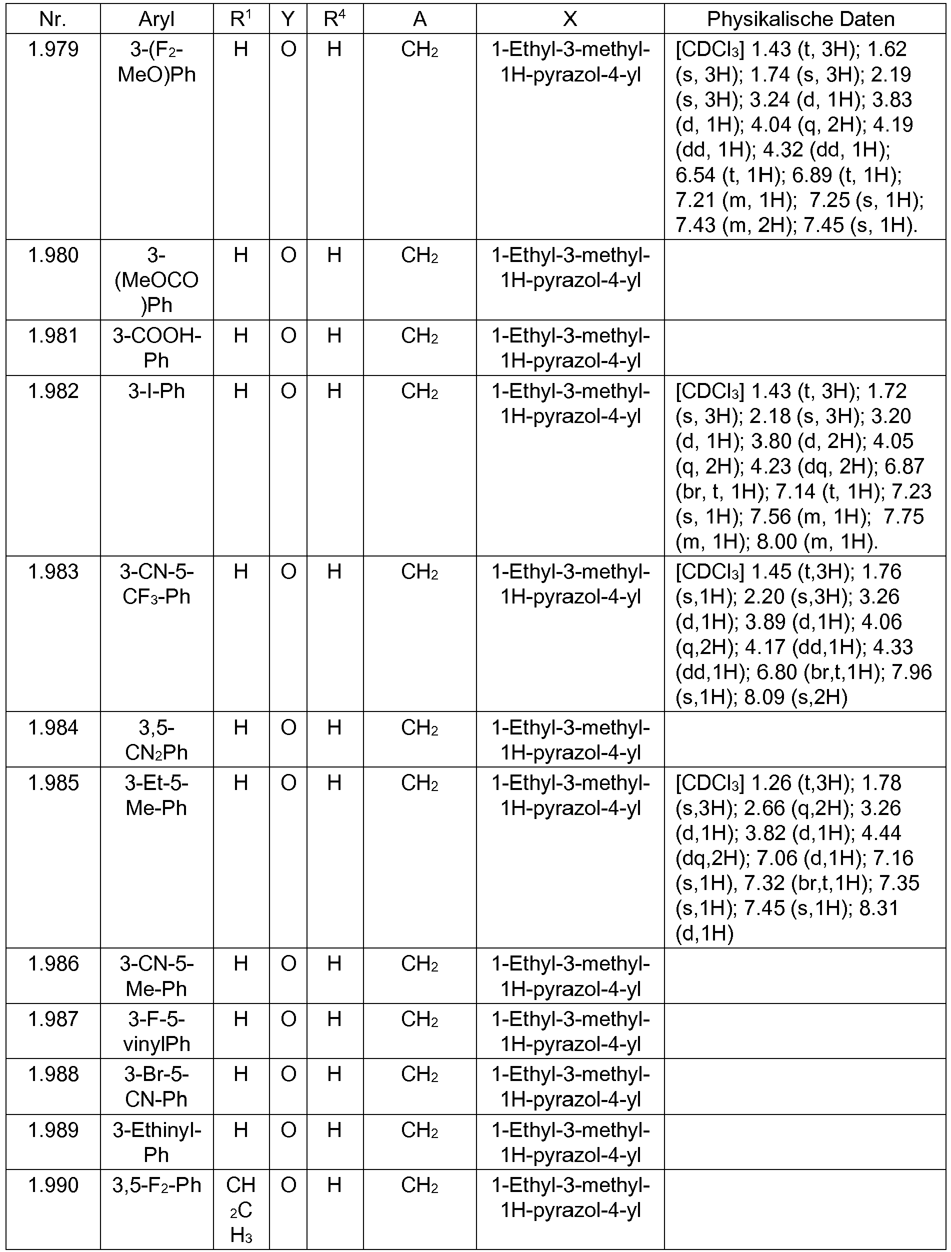

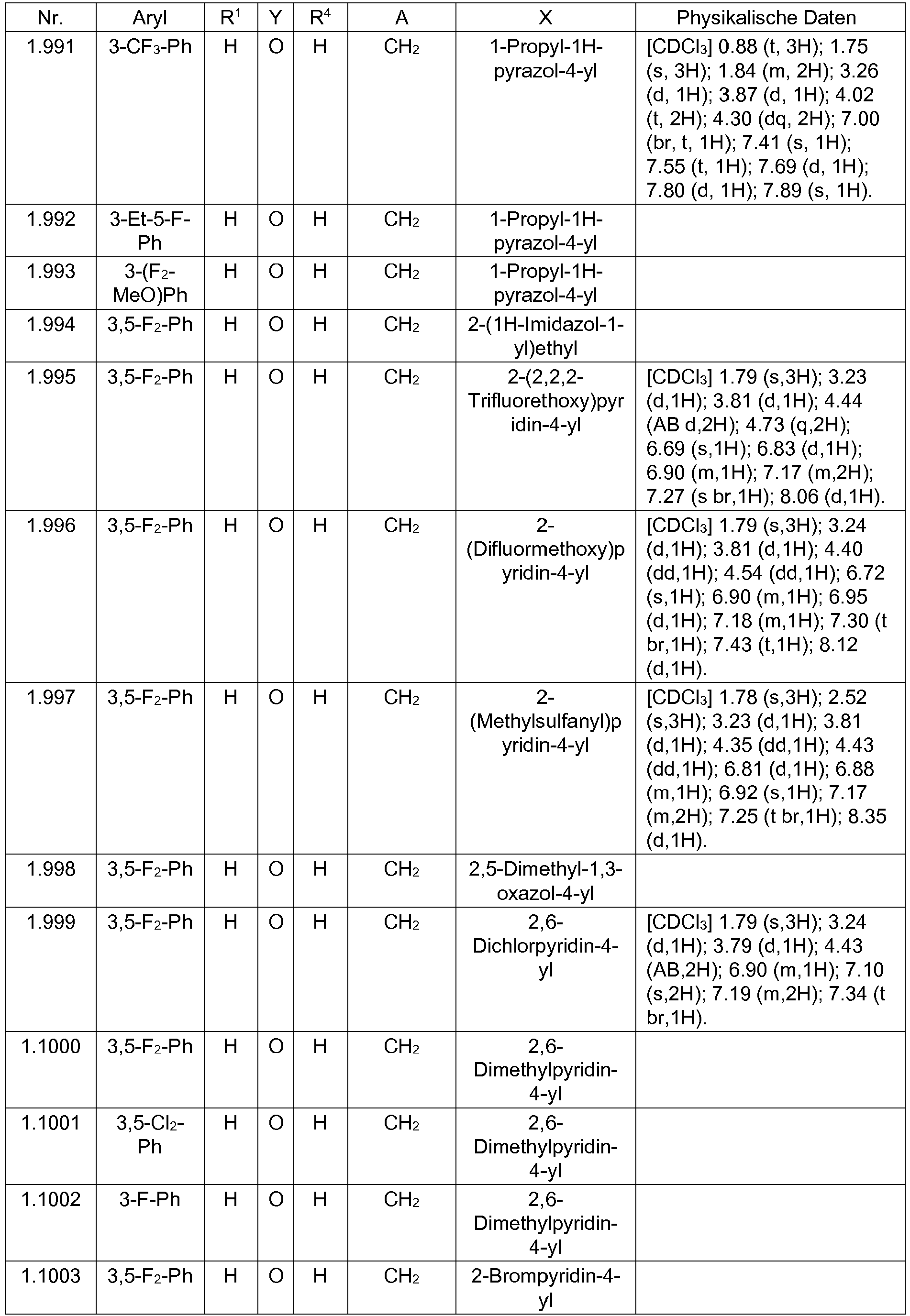

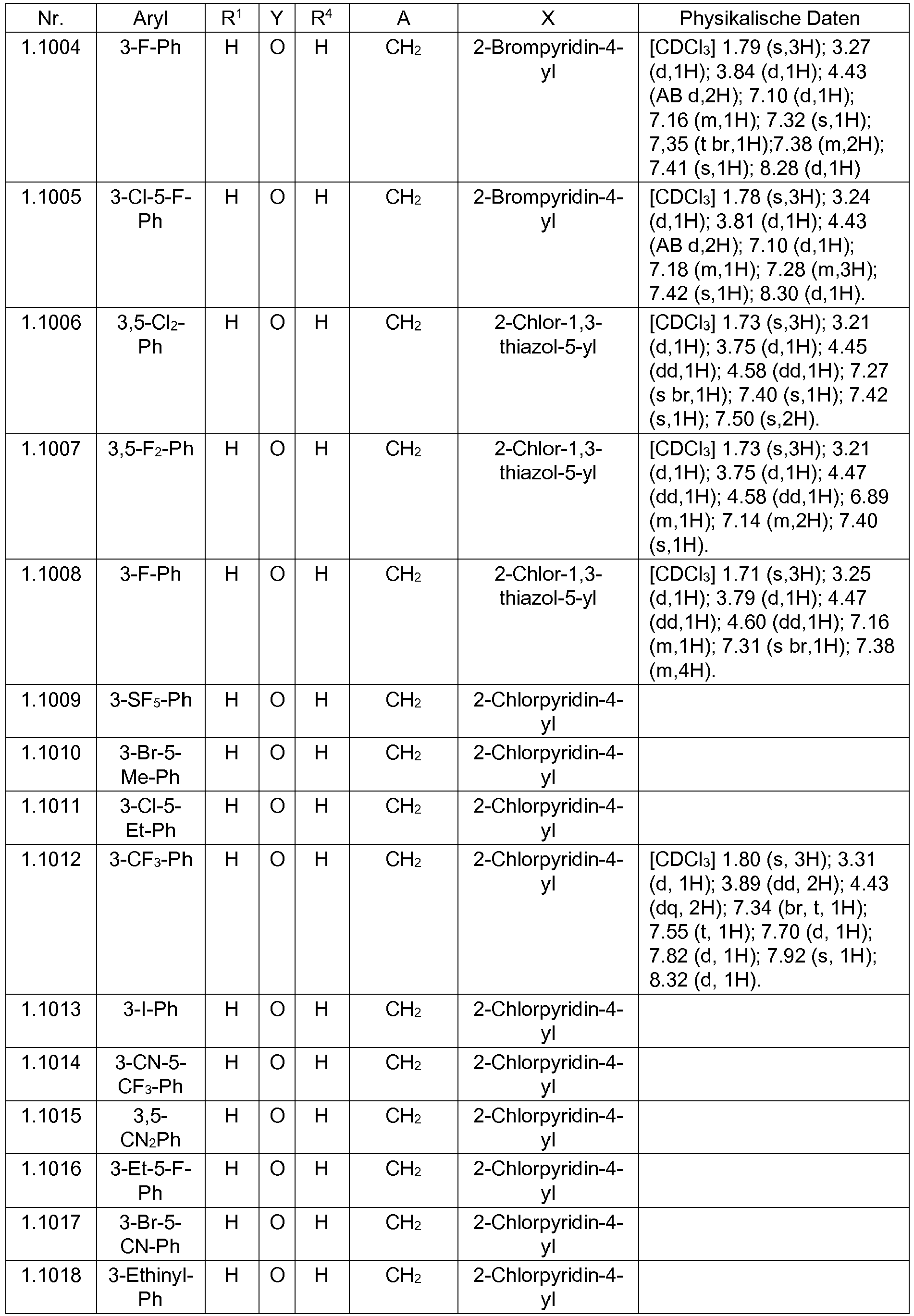

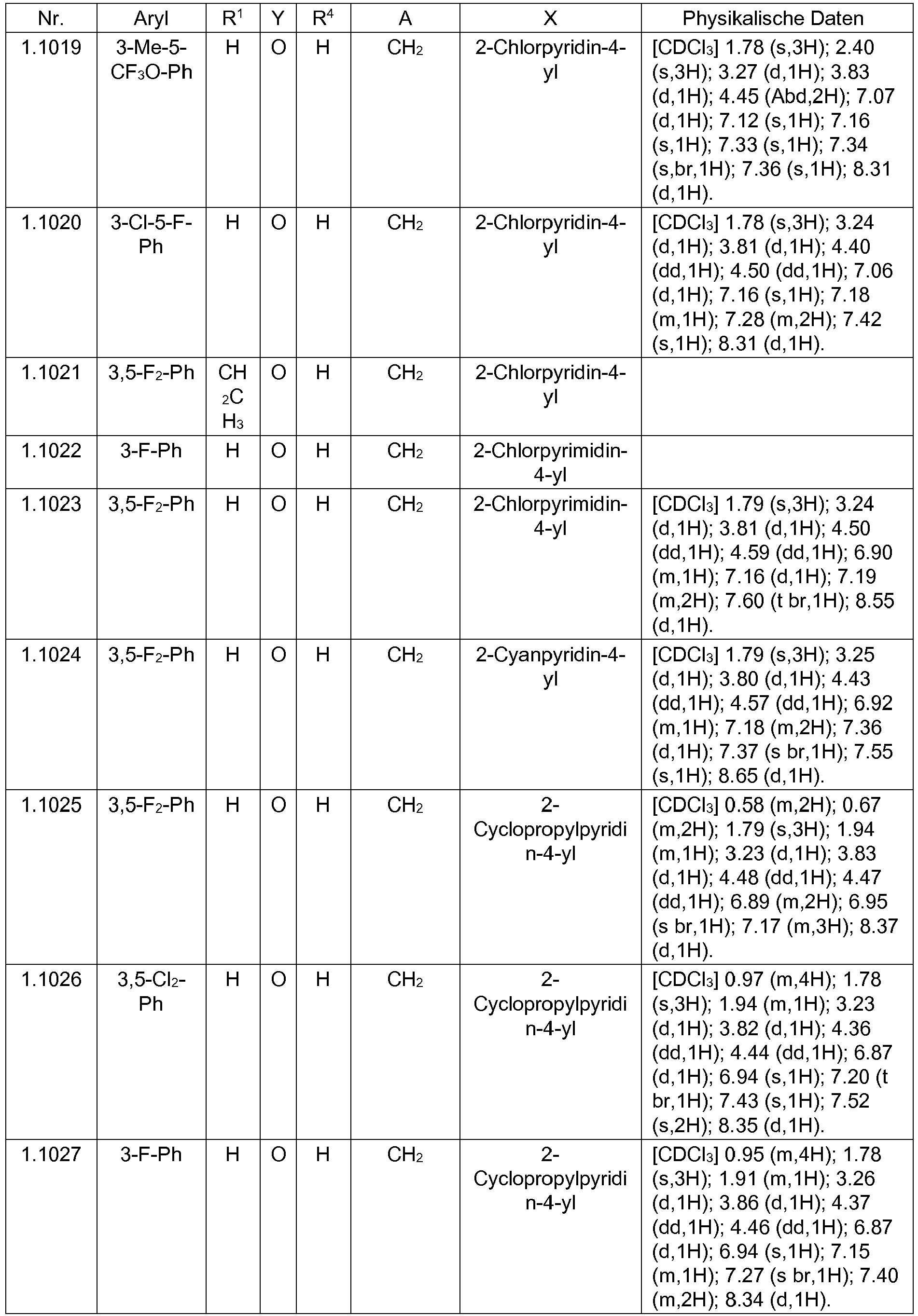

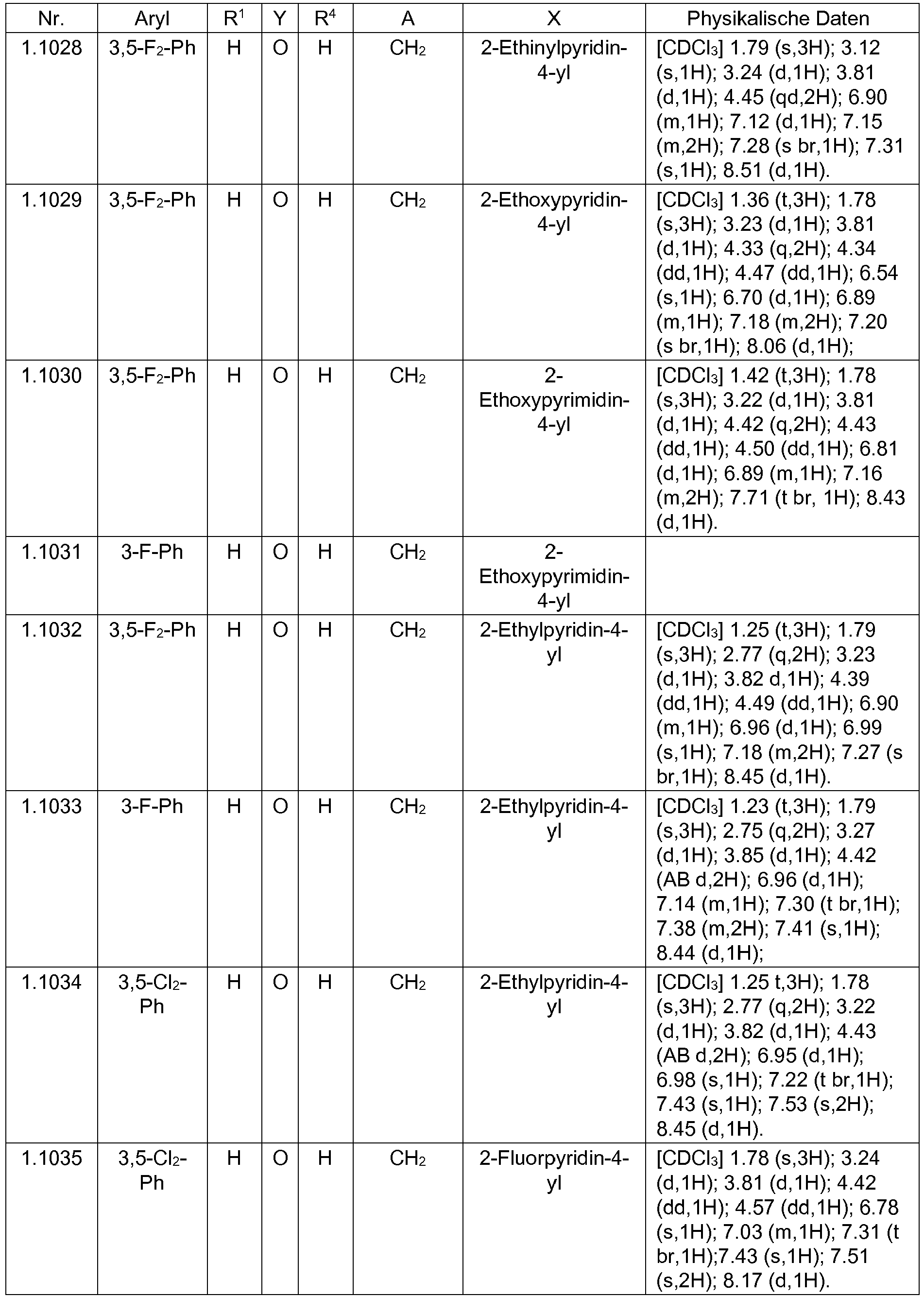

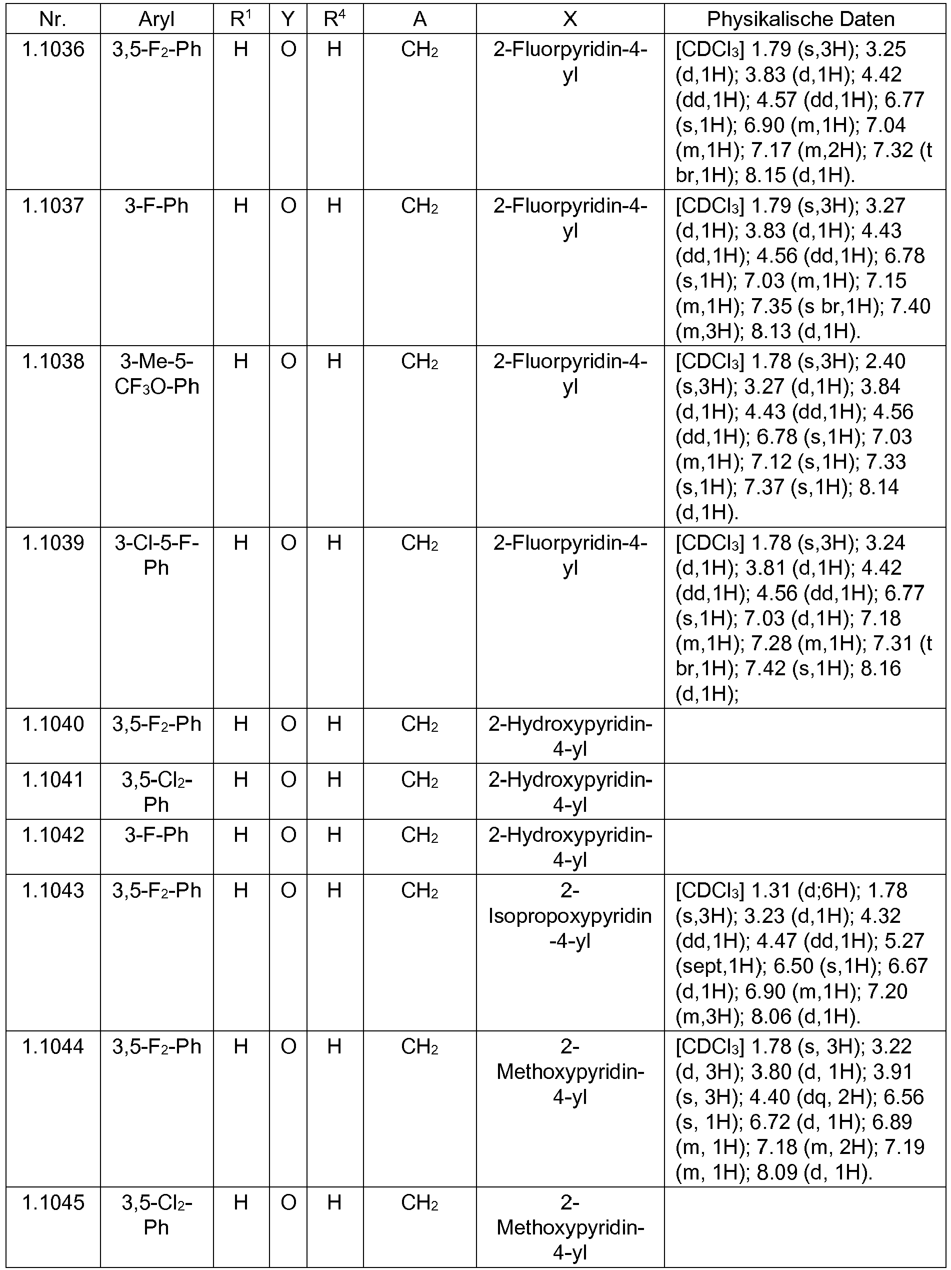

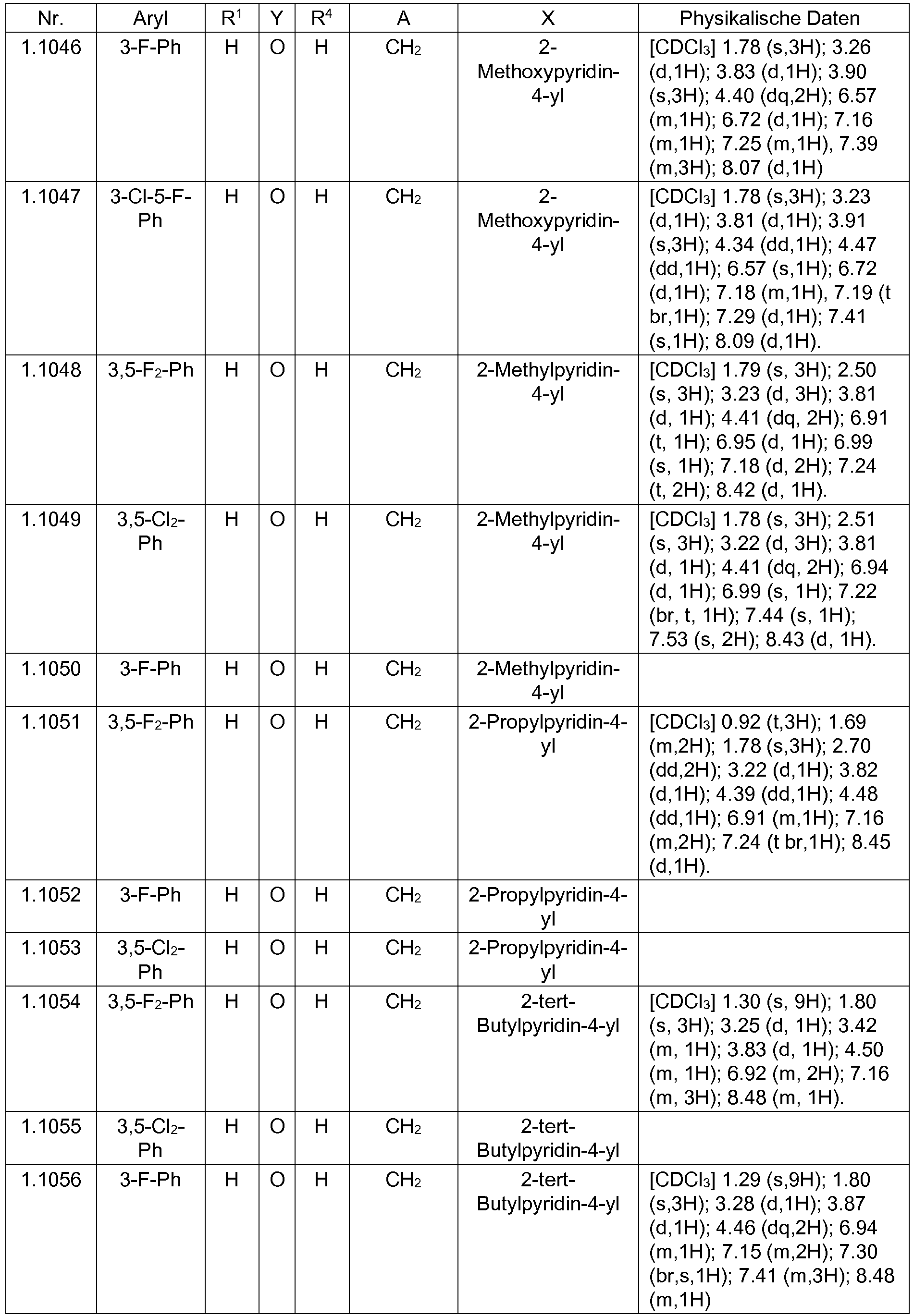

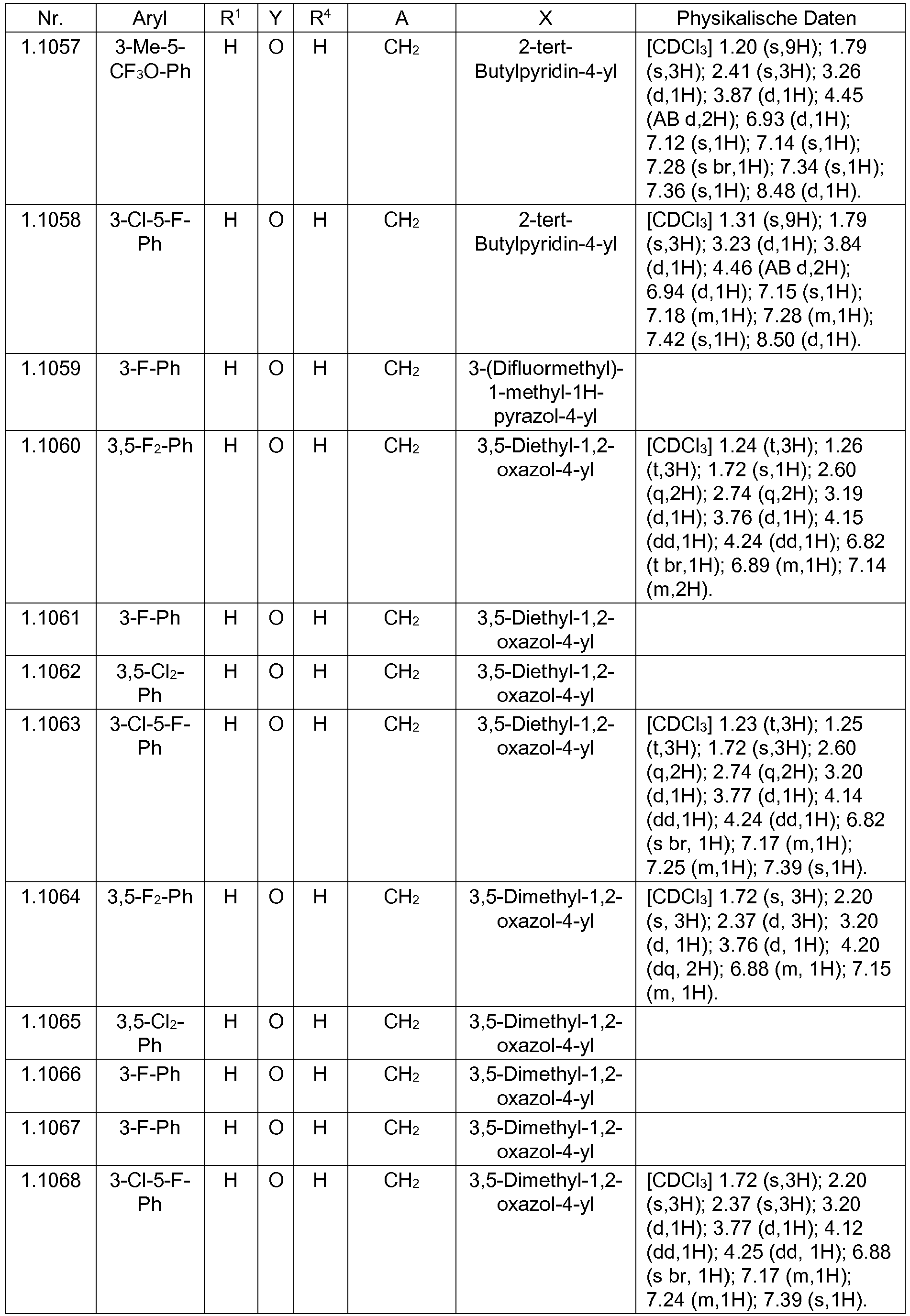

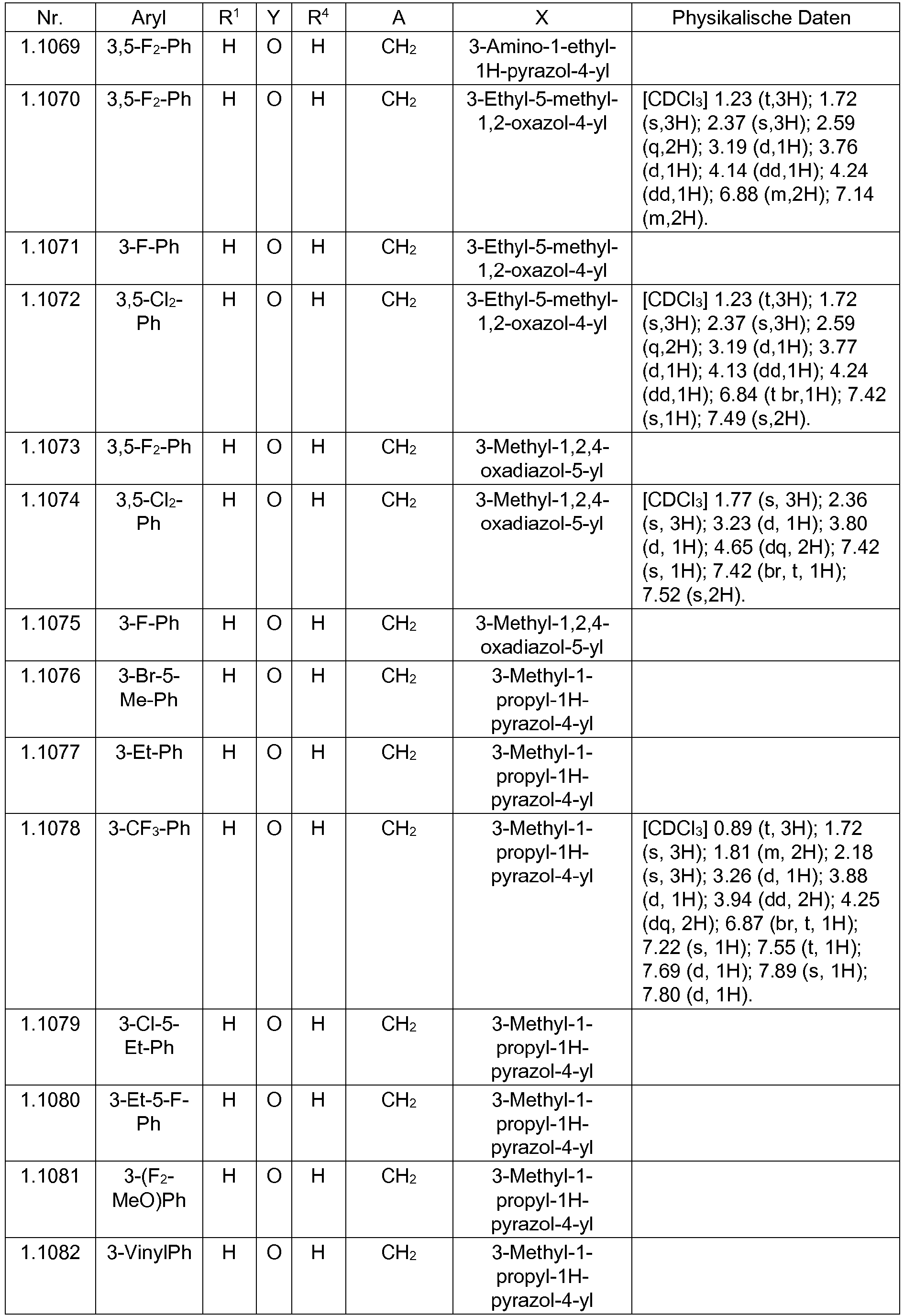

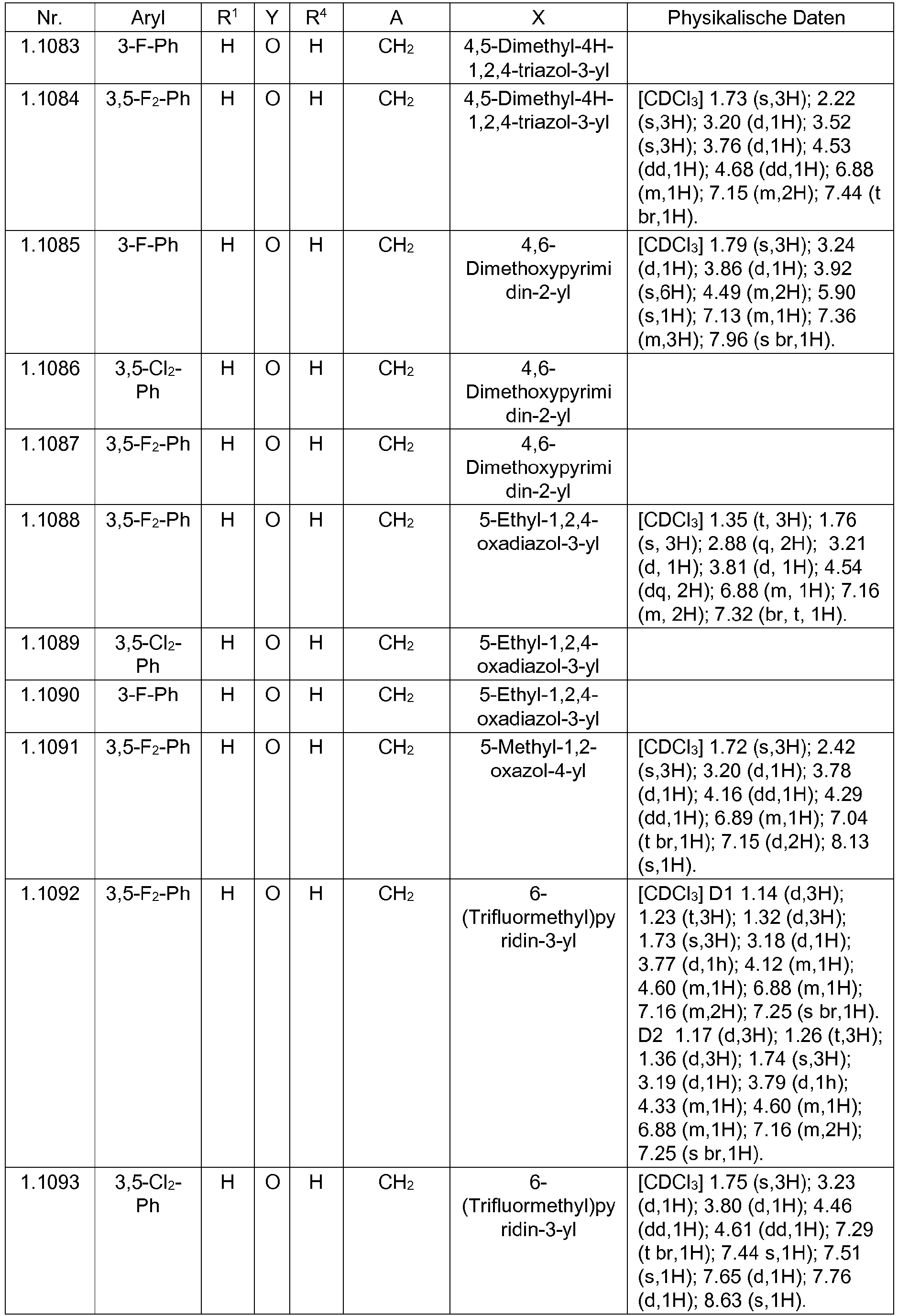

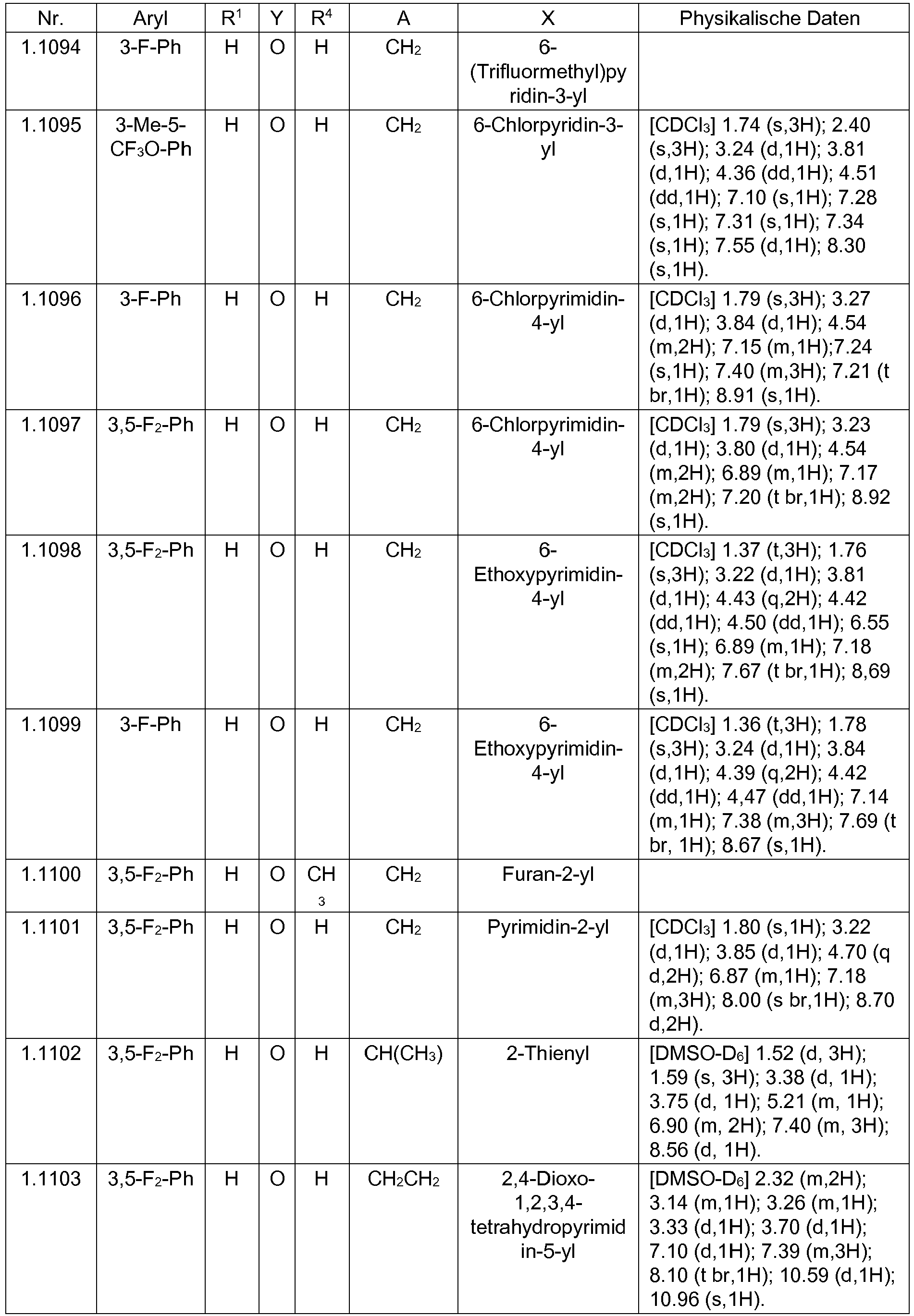

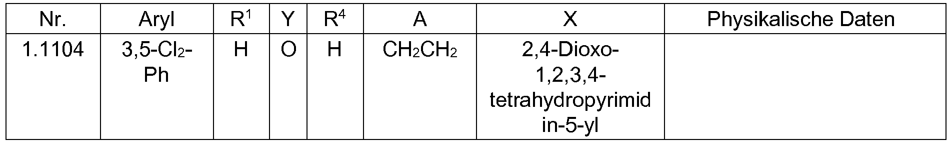

| Tabelle 2: Erfindungsgemäße Verbindungen der allgemeinen Formel (I), worin R² für Wasserstoff und R³ für Ethyl stehen, und Aryl den Rest 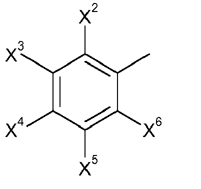 bedeutet. | | | | | | | |
|---|---|---|---|---|---|---|---|
| 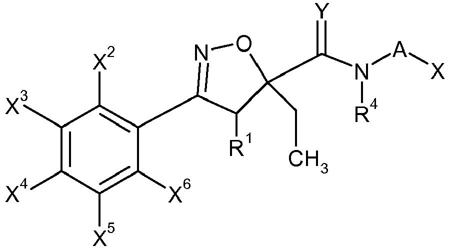 | | | | | | | |

| Nr. | Aryl | R¹ | Y | R⁴ | A | X | Physikalische Daten |
|---|---|---|---|---|---|---|---|
| 2.001 | 3,5-Cl₂-Ph | H | O | H | Bindung | 1,2-Oxazol-3-yl | |
| 2.002 | 3,5-(CF₃)₂-Ph | H | O | H | Bindung | 1,3-Dimethyl-1H-pyrazol-5-yl | |
| 2.003 | 3,5-Cl₂-Ph | H | O | H | Bindung | 1,3-Dimethyl-1H-pyrazol-5-yl | [CDCl₃] 1.09 (t, 3H); 2.06 (m, 1H); 2.25 (s, 3H); 2.25 (m, 1H); 3.33 (d, 1H); 3.71 (s, 3H); 3.78 (d, 1H); 6.18 (s, 1H); 7.44 (m, 1H); 7.55 (m, 2H); 8.45 (s, 1H). |
| 2.004 | 3,5-Cl₂-Ph | H | O | H | Bindung | 1,3-Oxazol-2-yl | |
| 2.005 | 3,5-Cl₂-Ph | H | O | H | Bindung | 1,3-Thiazol-2-yl | |
| 2.006 | 3,5-Cl₂-Ph | H | O | H | Bindung | 1,5-Dimethyl-1H-pyrazol-4-yl | |
| 2.007 | 3,5-F₂-Ph | H | O | H | Bindung | 1,5-Dimethyl-1H-pyrazol-4-yl | |
| 2.008 | 3-F-Ph | H | O | H | Bindung | 1,5-Dimethyl-1H-pyrazol-4-yl | |
| 2.009 | 3-Me-Ph | H | O | H | Bindung | 1,5-Dimethyl-1H-pyrazol-4-yl | |
| 2.010 | Ph | H | O | H | Bindung | 1,5-Dimethyl-1H-pyrazol-4-yl | |
| 2.011 | 3,5-Cl₂-Ph | H | O | H | Bindung | 1-Ethyl-3-(methoxycarbonyl)-1H-pyrazol-4-yl | |
| 2.012 | 3,5-F₂-Ph | H | O | H | Bindung | 1-Ethyl-3-(methoxycarbonyl)-1H-pyrazol-4-yl | |
| 2.013 | 3-F-Ph | H | O | H | Bindung | 1-Ethyl-3-(methoxycarbonyl)-1H-pyrazol-4-yl | |
| 2.014 | Ph | H | O | H | Bindung | 1-Ethyl-3-(methoxycarbonyl)-1H-pyrazol-4-yl | |
| 2.015 | 3,5-Cl₂-Ph | H | O | H | Bindung | 1-Ethyl-3-methyl-1 H-pyrazol-4-yl | |
| 2.016 | 3,5-F₂-Ph | H | O | H | Bindung | 1-Ethyl-3-methyl-1H-pyrazol-4-yl | |
| 2.017 | 3-F-Ph | H | O | H | Bindung | 1-Ethyl-3-methyl-1 H-pyrazol-4-yl | |
| 2.018 | Ph | H | O | H | Bindung | 1-Ethyl-3-methyl-1H-pyrazol-4-yl | |
| 2.019 | 3,5-Cl₂-Ph | H | O | H | Bindung | 1-Methyl-1H-1,2,4-triazol-3-yl | |
| 2.020 | 3,5-F₂-Ph | H | O | H | Bindung | 1-Methyl-1H-1,2,4-triazol-3-yl | |
| 2.021 | 3,5-Cl₂-Ph | H | O | H | Bindung | 1-Methyl-1H-imidazol-2-yl | |
| 2.022 | 3,5-Cl₂-Ph | H | O | H | Bindung | 1-Methyl-1H-pyrazol-4-yl | |
| 2.023 | 3,5-F₂-Ph | H | O | H | Bindung | 1-Methyl-1H-pyrazol-4-yl | |
| 2.024 | 3-F-Ph | H | O | H | Bindung | 1-Methyl-1H-pyrazol-4-yl | |
| 2.025 | Ph | H | O | H | Bindung | 1-Methyl-1H-pyrazol-4-yl | |
| 2.026 | 3,5-Cl₂-Ph | H | O | H | Bindung | 1-Propyl-1H-1,2,4-triazol-3-yl | |
| 2.027 | 3,5-F₂-Ph | H | O | H | Bindung | 1-Propyl-1H-1,2,4-triazol-3-yl | |
| 2.028 | 3,5-Cl₂-Ph | H | O | H | Bindung | 2-(2-Ethoxy-2-oxoethyl)phenyl | |
| 2.029 | 3,5-Cl₂-Ph | H | O | H | Bindung | 2-(Methoxycarbonyl)p henyl | |
| 2.030 | 3,5-Cl₂-Ph | H | O | H | Bindung | 2-(Methylcarbamoyl)p henyl | |
| 2.031 | 3,5-Cl₂-Ph | H | O | H | Bindung | 2,4-Dichlor-6-(methylcarbamoyl)p henyl | [CDCl₃] 1.09 (t, 3H), 2.05 (m, 1H); 2.26 (m, 1H); 3.31 (d, 1H); 3.78 (d, 1H); 7.42 (t, 1H); 7.55 (m, 2H); 7.64 (d, 1H); 8.18 (d, 1H); 9.03 (s br, 1H) |
| 2.032 | 3,5-Cl₂-Ph | H | O | H | Bindung | 2,4-Difluorphenyl | |
| 2.033 | 3,5-Cl₂-Ph | H | O | H | Bindung | 2-Carboxyphenyl | |
| 2.034 | 3,5-Cl₂-Ph | H | O | H | Bindung | 2-Chlorpyridin-3-yl | |
| 2.035 | 3,5-Cl₂-Ph | H | O | H | Bindung | 3-(2-Methoxy-2-oxoethyl)phenyl | |
| 2.036 | 3,5-Cl₂-Ph | H | O | H | Bindung | 3-(Carboxymethyl)ph enyl | |
| 2.037 | 3,5-Cl₂-Ph | H | O | H | Bindung | 3-(Ethoxycarbonyl)-1H-1,2,4-triazol-5-yl | |
| 2.038 | 3,5-Cl₂-Ph | H | O | H | Bindung | 3-(Ethoxycarbonyl)-1H-1,2,4-triazol-5-yl | |
| 2.039 | 3,5-F₂-Ph | H | O | H | Bindung | 3-(Methoxycarbonyl)p henyl | |
| 2.040 | 3,5-Cl₂-Ph | H | O | H | Bindung | 3-(Methoxycarbonyl)p henyl | |
| 2.041 | 3-F-Ph | H | O | H | Bindung | 3-(Methylcarbamoyl)p henyl | |
| 2.042 | 3-F-Ph | H | O | H | Bindung | 3,5-Dichlorphenyl | |
| 2.043 | 3,5-Cl₂-Ph | H | O | H | Bindung | 3,5-Dimethoxyphenyl | |
| 2.044 | 3,5-Cl₂-Ph | H | O | H | Bindung | 3,5-Dimethyl-1H-pyrazol-4-yl | |
| 2.045 | 3,5-Cl₂-Ph | H | O | H | Bindung | 3,5-Dimethyl-1H-pyrazol-4-yl | |
| 2.046 | 3-F-Ph | H | O | H | Bindung | 3,5-Dimethyl-1H-pyrazol-4-yl | |
| 2.047 | Ph | H | O | H | Bindung | 3-[2-(Methylamino)-2-oxoethyl]phenyl | |
| 2.048 | 3,5-Cl₂-Ph | H | O | H | Bindung | 3-Carboxyphenyl | |
| 2.049 | 3,5-Cl₂-Ph | H | O | H | Bindung | 3-Carboxyphenyl | |
| 2.050 | 3-F-Ph | H | O | H | Bindung | 3-Chlorpyridin-2-yl | |
| 2.051 | 3,5-Cl₂-Ph | H | O | H | Bindung | 3-Cyclopropyl-1,2,4-thiadiazol-5-yl | |
| 2.052 | 3,5-Cl₂-Ph | H | O | H | Bindung | 3-Methyl-1,2-oxazol-5-yl | |
| 2.053 | 3,5-Cl₂-Ph | H | O | H | Bindung | 3-Methyl-1-propyl-1 H-pyrazol-4-yl | |
| 2.054 | 3,5-Cl₂-Ph | H | O | H | Bindung | 3-Methyl-1-propyl-1 H-pyrazol-4-yl | |
| 2.055 | 3,5-F₂-Ph | H | O | H | Bindung | 3-Methyl-1-propyl-1 H-pyrazol-4-yl | |
| 2.056 | 3-F-Ph | H | O | H | Bindung | 3-Methyl-1-propyl-1 H-pyrazol-4-yl | |
| 2.057 | Ph | H | O | H | Bindung | 4-(2-Ethoxy-2-oxoethyl)phenyl | |
| 2.058 | 3,5-Cl₂-Ph | H | O | H | Bindung | 4-(Carboxymethyl)ph enyl | |
| 2.059 | 3,5-Cl₂-Ph | H | O | H | Bindung | 4-(Methoxycarbonyl)-3-thienyl | |
| 2.060 | 3,5-F₂-Ph | H | O | H | Bindung | 4-(Methoxycarbonyl)p henyl | |
| 2.061 | 3,5-Cl₂-Ph | H | O | H | Bindung | 4,5-Dimethyl-4H-1,2,4-triazol-3-yl | |
| 2.062 | 3,5-Cl₂-Ph | H | O | H | Bindung | 4,5-Dimethyl-4H-1,2,4-triazol-3-yl | |
| 2.063 | 3,5-F₂-Ph | H | O | H | Bindung | 4,6-Dimethoxypyrimidin -2-yl | |
| 2.064 | 3,5-Cl₂-Ph | H | O | H | Bindung | 4-Carboxyphenyl | |
| 2.065 | 3,5-Cl₂-Ph | H | O | H | Bindung | 4-Chlor-2-(methoxycarbonyl)-6-methylphenyl | |
| 2.066 | 3,5-Cl₂-Ph | H | O | H | Bindung | 4-Chlor-2-methyl-6-(methylcarbamoyl)p henyl | |
| 2.067 | 3,5-Cl₂-Ph | H | O | H | Bindung | 4-Chlorphenyl | |
| 2.068 | 3,5-Cl₂-Ph | H | O | H | Bindung | 4-Chlorpyridin-2-yl | |
| 2.069 | 3,5-Cl₂-Ph | H | O | H | Bindung | 4-Cyano-1-ethyl-1 H-pyrazol-3-yl | |
| 2.070 | 3,5-F₂-Ph | H | O | H | Bindung | 4-Cyano-1H-pyrazol-3-yl | |
| 2.071 | 3,5-Cl₂-Ph | H | O | H | Bindung | 4-Cyano-1H-pyrazol-3-yl | |
| 2.072 | 3-F-Ph | H | O | H | Bindung | 4-Cyano-1H-pyrazol-3-yl | |
| 2.073 | Ph | H | O | H | Bindung | 4-Ethylpyridin-2-yl | |
| 2.074 | 3,5-Cl₂-Ph | H | O | H | Bindung | 4-Formyl-3-(1-methylcyclopropyl)-1,2-oxazol-5-yl | |
| 2.075 | 3,5-F₂-Ph | H | O | H | Bindung | 4-Methoxypyridin-2-yl | |
| 2.076 | 3,5-Cl₂-Ph | H | O | H | Bindung | 4-Methyl-1,2,5-oxad iazol-3-yl | |
| 2.077 | 3,5-Cl₂-Ph | H | O | H | Bindung | 4-Methyl-1,3-thiazol-2-yl | |
| 2.078 | 3,5-Cl₂-Ph | H | O | H | Bindung | 4-Methyl-1,3-thiazol-2-yl | |
| 2.079 | 3,5-F₂-Ph | H | O | H | Bindung | 4-Methylpyridin-2-yl | |
| 2.080 | 2,4-Cl₂-Ph | H | O | H | Bindung | 4-Methylpyridin-2-yl | |
| 2.081 | 3,5-Cl₂-Ph | H | O | H | Bindung | 4-Methylpyridin-2-yl | |
| 2.082 | Ph | H | O | H | Bindung | 4-Pentylpyridin-2-yl | |
| 2.083 | 2,4-Cl₂-Ph | H | O | H | Bindung | 4-Pentylpyridin-2-yl | |
| 2.084 | 3,5-Cl₂-Ph | H | O | H | Bindung | 5-(Methoxycarbonyl)-1,3-thiazol-4-yl | |
| 2.085 | 3,5-F₂-Ph | H | O | H | Bindung | 5-(Methoxycarbonyl)-1,3-thiazol-4-yl | [CDCl₃] 12.07 0.33;9.10 2.14;8.19 4.59;8.17 4.58;8.05 5.85;7.52 3.19;7.37 0.38;7.36 0.32;7.36 0.73;7.31 0.95;7.31 0.90;7.31 1.11;7.26 548.67;7.24 1.58;7.24 0.98;7.23 0.78;7.23 0.73;7.23 0.65;7.22 0.62;7.21 1.91;7.21 1.09;7.21 1.36;7.20 1.05;7.19 3.92;7.19 4.53;7.18 2.84;7.17 3.42;7.17 4.97;7.17 3.74;7.16 1.03;7.15 0.81;7.00 3.05;6.91 3.32;6.91 3.01;6.90 4.21;6.90 3.61;6.88 1.90;6.87 3.10;6.87 1.69;6.86 0.98;6.85 1,56;3.81 4.95;3.76 5.93;3.56 0.48;3.49 1,12;3.32 5.31;3.28 4.47;2.63 3.62;2.61 4.88;2.59 4.00;2.29 0.44;2.27 1.41;2.26 1.87;2.24 2.47;2.22 2.17;2.20 0.72;2.09 0.64;2.07 2.02;2.06 2,39;2.04 2.06;2.02 1.58;2.01 1.87;1.67 0.86;1.65 2.18;1.63 3.04;1.62 2.35:1.60 1.71:1.59 0.44;1.54 410.81;1.51 1.09;1.50 1,75;1.45 0.40;1.44 0,42;1.36 0.93;1.34 4.63;1.33 6.03;1.32 7.24;1.31 4.50;1.30 2.21;1.28 0.80;1.26 2.31;1.23 0.37;1.22 0.52;1.21 0.33;1.18 0.44;1.10 7.40;1.08 16.00;1.06 6.92;1.03 0.33;0.93 0.40;0.90 4.64;0.89 13.24;0.87 4.36;0.84 0.58;0.83 0.58;0.81 0.50;0.15 1.05;0.10 0.41;0.06 0.71;0.05 0.62;0.05 0.64;0.02 0.41;0.02 0.41;0.02 0.49;0.02 0.41;0.02 0.54;0.02 0.55;0.02 0.72;0.01 0.98;0.01 1.15;0.01 1.46;0.01 11.29;0.00 298.63;-0.01 12.22;-0.01 1.79;-0.01 1.67;-0.02 1.42;-0.02 1.23;-0.02 0.96;-0.02 0.92;-0.02 0.64;-0.03 0.50;-0.03 0.43;-0.05 0.90;-0.05 0.92;-0.15 1.07 |
| 2.086 | 3,5-Cl₂-Ph | H | O | H | Bindung | 5-Chlor-4-methylpyridin-2-yl | |
| 2.087 | 3,5-F₂-Ph | H | O | H | Bindung | 5-Fluor-4-methylpyridin-2-yl | |
| 2.088 | 3,5-Cl₂-Ph | H | O | H | Bindung | 5-Methoxy-1-methyl-1H-1,2,4-triazol-3-yl | |
| 2.089 | 3,5-Cl₂-Ph | H | O | H | Bindung | 5-Methoxy-1-methyl-1H-1,2,4-triazol-3-yl | |
| 2.090 | 3,5-Cl₂-Ph | H | O | H | Bindung | 5-Methylpyridin-2-yl | |
| 2.091 | 3,5-F₂-Ph | H | O | H | Bindung | 6-Chlorpyridin-2-yl | |
| 2.092 | Ph | H | O | H | Bindung | 6-Chlorpyridin-3-yl | |
| 2.093 | 3,5-Cl₂-Ph | H | O | H | Bindung | Pyridin-2-yl | |
| 2.094 | 3,5-Cl₂-Ph | H | O | H | Bindung | Pyridin-4-yl | |
| 2.095 | 3,5-Cl₂-Ph | H | O | H | Bindung | 1-(2,2,2-Trifluorethyl)-1H-pyrazol-4-yl | |
| 2.096 | 3,5-Cl₂-Ph | H | O | H | Bindung | 1-(2-Chlorethyl)-1 H-pyrazol-4-yl | |
| 2.097 | 3,5-F₂-Ph | H | O | H | CH₂ | 1-(2-Methoxy-2-oxoethyl)-1H-pyrazol-4-yl | |
| 2.098 | 3,5-F₂-Ph | H | O | H | CH₂ | 1-(3-Carboxypropyl)-1H-pyrazol-4-yl | |
| 2.099 | 3,5-Cl₂-Ph | H | O | H | CH₂ | 1-(3-Methoxy-3-oxopropyl)-1H-pyrazol-4-yl | |
| 2.100 | 3,5-F₂-Ph | H | O | H | CH₂ | 1-(4-Methoxy-4-oxobutyl)-1H-pyrazol-4-yl | |
| 2.101 | 3,5-F₂-Ph | H | O | H | CH₂ | 1-(Cyanomethyl)-1 H-pyrazol-4-yl | |
| 2.102 | 3,5-F₂-Ph | H | O | H | CH₂ | 1-(Cyanomethyl)-1 H-pyrazol-4-yl | |
| 2.103 | 3,5-Cl₂-Ph | H | O | H | CH₂ | 1-(Cyanomethyl)-1 H-pyrazol-4-yl | |
| 2.104 | 3,5-F₂-Ph | H | O | H | CH₂ | 1,3-Dimethyl-1H-pyrazol-4-yl | |
| 2.105 | 3-F-Ph | H | O | H | CH₂ | 1,3-Dimethyl-1H-pyrazol-4-yl | |
| 2.106 | 3,5-(CF₃)₂-Ph | H | O | H | CH₂ | 1,3-Dimethyl-1H-pyrazol-4-yl | |
| 2.107 | 3,5-Cl₂-Ph | H | O | H | CH₂ | 1,3-Dimethyl-1H-pyrazol-4-yl | |
| 2.108 | 3,5-F₂-Ph | H | O | H | CH₂ | 1,3-Dimethyl-1H-pyrazol-4-yl | |
| 2.109 | 3-CI-Ph | H | O | H | CH₂ | 1,3-Dimethyl-1H-pyrazol-4-yl | |
| 2.110 | 3-F-Ph | H | O | H | CH₂ | 1,3-Dimethyl-1H-pyrazol-4-yl | |
| 2.111 | 3-Me-Ph | H | O | H | CH₂ | 1,3-Dimethyl-1H-pyrazol-5-yl | |
| 2.112 | Ph | H | O | H | CH₂ | 1,3-Dimethyl-1H-pyrazol-5-yl | |
| 2.113 | 3,5-(CF₃)₂-Ph | H | O | H | CH₂ | 1,3-Dimethyl-1H-pyrazol-5-yl | |
| 2.114 | 3,5-Cl₂-Ph | H | O | H | CH₂ | 1,3-Dimethyl-1H-pyrazol-5-yl | |
| 2.115 | 3,5-F₂-Ph | H | O | H | CH₂ | 1,3-Oxazol-4-yl | |
| 2.116 | Ph | H | O | H | CH₂ | 1,3-Oxazol-4-yl | |
| 2.117 | 3,5-Cl₂-Ph | H | O | H | CH₂ | 1,3-Oxazol-4-yl | |
| 2.118 | 3,5-F₂-Ph | H | O | H | CH₂ | 1,3-Thiazol-2-yl | |
| 2.119 | 3-F-Ph | H | O | H | CH₂ | 1,3-Thiazol-2-yl | |
| 2.120 | 3,5-Cl₂-Ph | H | O | H | CH₂ | 1,4-Dimethyl-5-oxo-4,5-dihydro-1H-1,2,4-triazol-3-yl | |
| 2.121 | 3,5-F₂-Ph | H | O | H | CH₂ | 1,5-Dimethyl-1H-pyrazol-3-yl | |
| 2.122 | 3,5-Cl₂-Ph | H | O | H | CH₂ | 1,5-Dimethyl-1H-pyrazol-4-yl | |
| 2.123 | 3,5-F₂-Ph | H | O | H | CH₂ | 1,5-Dimethyl-1H-pyrazol-4-yl | |
| 2.124 | 3,5-Cl₂-Ph | H | O | H | CH₂ | 1,5-Dimethyl-1H-pyrazol-4-yl | |
| 2.125 | 3,5-F₂-Ph | H | O | H | CH₂ | 1,5-Dimethyl-1H-pyrazol-4-yl | |
| 2.126 | 3-F-Ph | H | O | H | CH₂ | 1-Acetyl-1H-pyrazol-4-yl | |
| 2.127 | Ph | H | O | H | CH₂ | 1-Butyl-3-methyl-1 H-pyrazol-4-yl | |
| 2.128 | 3,5-F₂-Ph | H | O | H | CH₂ | 1-Butyl-5-methyl-1 H-pyrazol-4-yl | |
| 2.129 | 3,5-F₂-Ph | H | O | H | CH₂ | 1-Cyclopropyl-1H-pyrazol-4-yl | |
| 2.130 | 3,5-F₂-Ph | H | O | H | CH₂ | 1-Cyclopropyl-1H-pyrazol-4-yl | |
| 2.131 | 3,5-Cl₂-Ph | H | O | H | CH₂ | 1-Cyclopropyl-1H-pyrazol-4-yl | |
| 2.132 | 3,5-F₂-Ph | H | O | H | CH₂ | 1-Cyclopropyl-1H-pyrazol-4-yl | |
| 2.133 | 3-F-Ph | H | O | H | CH₂ | 1-Ethyl-1H-pyrazol-4-yl | |
| 2.134 | Ph | H | O | H | CH₂ | 1-Ethyl-1H-pyrazol-4-yl | |
| 2.135 | 3,5-Cl₂-4-MeO-Ph | H | O | H | CH₂ | 1-Ethyl-1H-pyrazol-4-yl | |
| 2.136 | 3,5-Cl₂-Ph | H | O | H | CH₂ | 1-Ethyl-1H-pyrazol-4-yl | [CDCl₃] 0.98 (t, 3H); 1.46 (t, 3H), 1.95 (m, 1H); 2.15 (m, 1H); 3.21 (d, 1H); 3.70 (d,1H); 4.12 (q, 2H); 4.21 (dd, 1H), 4.38 (dd, 1H); 6.98 (t br, 1H); 7.33 (s, 1H); 7.40 (m, 2H), 7.50 (d, 2H) |
| 2.137 | 3,5-F₂-Ph | H | O | H | CH₂ | 1-Ethyl-1H-pyrazol-4-yl | [CDCl3] 0.99 (t,3H); 1.46 (t,3H); 1.97 (m,1H); 2.16 (m,1H); 3.22 (d,1H); 3.69 (d,1H); 4.13 (q,2H); 4.22 (dd,1H); 4.41 (dd,1H); 6.88 (t,1H); 6.99 (s,1H); 7.15 (d,2H); 7.34 (s,1H); 7.42 (s,1H). |
| 2.138 | 3,5-(MeO)₂-Ph | H | O | H | CH₂ | 1-Ethyl-1H-pyrazol-4-yl | |
| 2.139 | 3-Br-5-CF₃-Ph | H | O | H | CH₂ | 1-Ethyl-1H-pyrazol-4-yl | |
| 2.140 | 3-CI-5-Et-Ph | H | O | H | CH₂ | 1-Ethyl-1H-pyrazol-4-yl | |
| 2.141 | 3-F-5-MeO-Ph | H | O | H | CH₂ | 1-Ethyl-1H-pyrazol-4-yl | |
| 2.142 | 3-F-Ph | H | O | H | CH₂ | 1-Ethyl-1H-pyrazol-4-yl | [CDCl3] 0.99 (t,3H); 1.45 (t,3H); 1.95 (m,1H); 2.17 (m,1H); 3.25 (d,1H); 3.73 (d,1H); 4.12 (q,2H); 4.22 (dd,1H); 4.39 (dd,1H); 7.03 (s br,1H); 7.13 (m,1H); 7.38 (m,3H). |
| 2.143 | 3-Me-5-CF₃O-Ph | H | O | H | CH₂ | 1-Ethyl-1H-pyrazol-4-yl | |
| 2.144 | 3-Me-Ph | H | O | H | CH₂ | 1-Ethyl-1H-pyrazol-5-yl | |
| 2.145 | Ph | H | O | H | CH₂ | 1-Ethyl-1H-pyrazol-5-yl | |
| 2.146 | 3,5-Cl₂-Ph | H | O | H | CH₂ | 1-Ethyl-1H-pyrazol-5-yl | |
| 2.147 | 3,5-F₂-Ph | H | O | H | CH₂ | 1-Ethyl-3-methyl-1H-pyrazol-4-yl | |
| 2.148 | Ph | H | O | H | CH₂ | 1-Ethyl-3-methyl-1 H-pyrazol-4-yl | |
| 2.149 | 2,3,4-F₃-Ph | H | O | H | CH₂ | 1-Ethyl-3-methyl-1 H-pyrazol-4-yl | |
| 2.150 | 2,3,5-F₃-Ph | H | O | H | CH₂ | 1-Ethyl-3-methyl-1 H-pyrazol-4-yl | |
| 2.151 | 2,3-F₂-Ph | H | O | H | CH₂ | 1-Ethyl-3-methyl-1H-pyrazol-4-yl | |
| 2.152 | 2,5-Cl₂-Ph | H | O | H | CH₂ | 1-Ethyl-3-methyl-1H-pyrazol-4-yl | |
| 2.153 | 2,5-F₂-Ph | H | O | H | CH₂ | 1-Ethyl-3-methyl-1H-pyrazol-4-yl | |
| 2.154 | 2,5-Me₂-Ph | H | O | H | CH₂ | 1-Ethyl-3-methyl-1H-pyrazol-4-yl | |
| 2.155 | 2-F-3-Me-Ph | H | O | H | CH₂ | 1-Ethyl-3-methyl-1H-pyrazol-4-yl | |
| 2.156 | 2-F-Ph | H | O | H | CH₂ | 1-Ethyl-3-methyl-1 H-pyrazol-4-yl | |
| 2.157 | 3-CF₃O-Ph | H | O | H | CH₂ | 1-Ethyl-3-methyl-1H-pyrazol-4-yl | |
| 2.158 | 3,4,5-F₃-Ph | H | O | H | CH₂ | 1-Ethyl-3-methyl-1H-pyrazol-4-yl | |
| 2.159 | 3,4-F₂-Ph | H | O | H | CH₂ | 1-Ethyl-3-methyl-1H-pyrazol-4-yl | [CDCl₃] 0.99 (t, 3H); 1.44 (t, 1H); 1.96 (m, 1H); 2.15 (m, 1H); 2.19 (s, 3H); 3.21 (d, 1H);3.70(d, 1H); 4.05 (q, 2H); 4.18 (dd, 1H); 4.33 (dd, 1H); 6.88 (brt, 1H); 7.27 (m, 3H); 7.51 (m,1H) |
| 2.160 | 3,5-Br₂-Ph | H | O | H | CH₂ | 1-Ethyl-3-methyl-1H-pyrazol-4-yl | [CDCl₃] 0.96 (t, 3H); 1.44 (t, 3H); 1.95 (m, 1H); 2.14 (m, 1H); 2.20 (s, 3H); 3.19 (d, 1H); 3.7 (d, 1H); 4.05 (q, 2H); 4.17 (dd, 1H); 4.32 (dd, 1H); 6.82 (m, 1H); 7.22 (s, 1H); 7.70 (m, 3H). |
| 2.161 | 3,5-Cl₂-4-MeO-Ph | H | O | H | CH₂ | 1-Ethyl-3-methyl-1H-pyrazol-4-yl | |
| 2.162 | 3,5-Cl₂-Ph | CH 3 | O | H | CH₂ | 1-Ethyl-3-methyl-1H-pyrazol-4-yl | |
| 2.163 | 3,5-Cl₂-Ph | H | O | H | CH₂ | 1-Ethyl-3-methyl-1H-pyrazol-4-yl | [CDCl₃] 1.00 (t, 3H); 1.44 (t, 3H), 1.95 (m, 1H); 2.14 (m, 1H); 2.20 (s, 3H); 3.21 (d, 1H); 3.70 (d,1H); 4.05 (q, 2H); 4.18 (dd, 1 H), 4.32 (dd, 1H); 6.84 (t br, 1H); 7.40 (t, 1H), 7.50 (d, 2H) |
| 2.164 | 3,5-Et₂-Ph | H | O | H | CH₂ | 1-Ethyl-3-methyl-1H-pyrazol-4-yl | |
| 2.165 | 3,5-F₂-Ph | H | O | H | CH₂ | 1-Ethyl-3-methyl-1H-pyrazol-4-yl | [CDCl₃] 7.52 0.89;7.35 0.37;7.31 0.43;7.26 160.13;7.25 4.77;7.18 0.34;7.17 1.62;7.16 2.10;7.15 2.04;7.14 1.71;7.00 0.86;6.91 0.39;6.90 0.72;6.90 0.39;6.88 0.85;6.88 1.46;6.87 0.81;6.86 0.73;6.86 1.08;6.85 0.82;5.30 0.47;4.36 0.91;4.34 0.85;4.32 1.43;4.31 1.41;4.20 1.45;4.19 1.46;4.17 0.92;4.15 0.90;4.08 1.25;4.08 0.48;4.06 3.96;4.04 3.96;4.02 1.30;3.72 2.57;3.68 3.09;3.24 2.60;3.20 2.24;2.81 0.72;2.21 1.43;2.19 16.00;2.17 1.00;2.15 1.19;2.13 1.04;2.12 0.33;1.98 0.98;1.96 1.23;1.94 0.96;1.93 0.76;1.54 61.65;1.45 4.71;1.43 10.25;1.42 4.74;1.39 0.78;1.38 0.35;1.22 0.47;1.13 0.44;1.01 3.65;0.99 7.73; 0.97 3.39;0.15 0.46;0.01 3.07;0.00 104.92;-0.01 3.60;-0.15 0.44 |
| 2.166 | 3,5-F₂-Ph | H | S | H | CH₂ | 1-Ethyl-3-methyl-1H-pyrazol-4-yl | |
| 2.167 | 3,5-Me₂-Ph | H | O | H | CH₂ | 1-Ethyl-3-methyl-1H-pyrazol-4-yl | |
| 2.168 | 3-CF₃S-Ph | H | O | H | CH₂ | 1-Ethyl-3-methyl-1H-pyrazol-4-yl | |
| 2.169 | 3-Br-5-CF₃-Ph | H | O | H | CH₂ | 1-Ethyl-3-methyl-1H-pyrazol-4-yl | |
| 2.170 | 3-Br-5-Cl-Ph | H | O | H | CH₂ | 1-Ethyl-3-methyl-1H-pyrazol-4-yl | |
| 2.171 | 3-Br-5-F-Ph | H | O | H | CH₂ | 1-Ethyl-3-methyl-1H-pyrazol-4-yl | |
| 2.172 | 3-Cl-4-F-Ph | H | O | H | CH₂ | 1-Ethyl-3-methyl-1H-pyrazol-4-yl | |
| 2.173 | 3-Cl-5-CF₃-Ph | H | O | H | CH₂ | 1-Ethyl-3-methyl-1H-pyrazol-4-yl | |
| 2.174 | 3-Cl-5-CN-Ph | H | O | H | CH₂ | 1-Ethyl-3-methyl-1H-pyrazol-4-yl | |
| 2.175 | 3-CI-5-Et-Ph | H | O | H | CH₂ | 1-Ethyl-3-methyl-1H-pyrazol-4-yl | |
| 2.176 | 3-CI-5-F-Ph | H | O | H | CH₂ | 1-Ethyl-3-methyl-1H-pyrazol-4-yl | [CDCl₃] 7.521.17;7.40 2.23;7.39 1.50;7.31 0.77;7.31 0.48;7.28 1.13;7.27 1.34;7.27 1.58;7.27 1.78;7.27 1.32; 7.27 1.34; 7.26 205.45;7.25 3.10;7.25 4.59; 7.21 0.88; 7.17 0.86;7.17 1.14;7.16 0.88;7.15 0.93;7.15 1.17;7.14 0.81;7.00 1.12;6.84 0.60;5.30 0.73;4.36 0.86;4.34 0.82;4.32 1.33;4.30 1.31;4.20 1.31;4.19 1.34;4.17 0.84;4.15 0.88;4.08 1.28;4.08 0.43;4.06 4.02;4.04 4.04;4.03 1.32;3.72 2.58;3.68 3.07;3.24 2.54;3.20 2.20;2.23 0.38;2.21 1.16;2.19 16.00;2.19 1.11;2.17 0.91;2.15 1.15;2.13 1.05;2.10 0.43;1.98 0.99;1.96 1.16;1.94 0.94;1.92 0.72;1.54 25.04;1.45 5.19;1.43 11.03;1.42 5.16;1.40 0.75;1.38 0.36;1.01 3.57;0.99 7.85;0.97 3.34;0.15 0.47;0.05 0.48;0.01 3.79;0.00 135.38;-0.01 4.15;-0.01 0.48;-0.05 0.54;-0.15 0.44 |
| 2.177 | 3-Cl-5-MeO-Ph | H | O | H | CH₂ | 1-Ethyl-3-methyl-1H-pyrazol-4-yl | |
| 2.178 | 3-Cl-5-Me-Ph | H | O | H | CH₂ | 1-Ethyl-3-methyl-1H-pyrazol-4-yl | |
| 2.179 | 3-Cl-5-CF₃O-Ph | H | O | H | CH₂ | 1-Ethyl-3-methyl-1H-pyrazol-4-yl | |
| 2.180 | 3-Cl-Ph | H | O | H | CH₂ | 1-Ethyl-3-methyl-1H-pyrazol-4-yl | |
| 2.181 | 3-CN-5-F-Ph | H | O | H | CH₂ | 1-Ethyl-3-methyl-1H-pyrazol-4-yl | |
| 2.182 | 3-CN-Ph | H | O | H | CH₂ | 1-Ethyl-3-methyl-1H-pyrazol-4-yl | |
| 2.183 | 3-cPr-5-F-Ph | H | O | H | CH₂ | 1-Ethyl-3-methyl-1H-pyrazol-4-yl | |
| 2.184 | 3-EtO-Ph | H | O | H | CH₂ | 1-Ethyl-3-methyl-1H-pyrazol-4-yl | |
| 2.185 | 3-Et-5-F-Ph | H | O | H | CH₂ | 1-Ethyl-3-methyl-1H-pyrazol-4-yl | |
| 2.186 | 3-F-5-CF₃-Ph | H | O | H | CH₂ | 1-Ethyl-3-methyl-1H-pyrazol-4-yl | |
| 2.187 | 3-F-5-MeO-Ph | H | O | H | CH₂ | 1-Ethyl-3-methyl-1H-pyrazol-4-yl | |
| 2.188 | 3-F-5-Me-Ph | H | O | H | CH₂ | 1-Ethyl-3-methyl-1H-pyrazol-4-yl | |
| 2.189 | 3-F-Ph | H | O | H | CH₂ | 1-Ethyl-3-methyl-1H-pyrazol-4-yl | |
| 2.190 | 3-MeO-Ph | H | O | H | CH₂ | 1-Ethyl-3-methyl-1H-pyrazol-4-yl | |
| 2.191 | 3-Me-Ph | H | O | H | CH₂ | 1-Ethyl-3-methyl-1H-pyrazol-5-yl | |
| 2.192 | Ph | H | O | H | CH₂ | 1-Ethyl-3-methyl-1H-pyrazol-5-yl | |
| 2.193 | 3,5-Cl₂-Ph | H | O | H | CH₂ | 1-Ethyl-3-methyl-1H-pyrazol-5-yl | |
| 2.194 | 3,5-F₂-Ph | H | O | H | CH₂ | 1H-1,2,3-Triazol-5-yl | |
| 2.195 | 3-F-Ph | H | O | H | CH₂ | 1H-Pyrazol-4-yl | |
| 2.196 | 3,5-F₂-Ph | H | O | H | CH₂ | 1H-Pyrazol-4-yl | |
| 2.197 | 3,5-Cl₂-Ph | H | O | H | CH₂ | 1H-Pyrazol-4-yl | |
| 2.198 | 3,5-F₂-Ph | H | O | H | CH₂ | 1-Isobutyl-1H-pyrazol-4-yl | |
| 2.199 | 3-F-Ph | H | O | H | CH₂ | 1-Isobutyl-1H-pyrazol-4-yl | |
| 2.200 | 3,5-Cl₂-Ph | H | O | H | CH₂ | 1-Isobutyl-1H-pyrazol-4-yl | |
| 2.201 | 3,5-F₂-Ph | H | O | H | CH₂ | 1-Isobutyl-1H-pyrazol-4-yl | |
| 2.202 | 3-F-Ph | H | O | H | CH₂ | 1-Isopropyl-1H-pyrazol-4-yl | |
| 2.203 | Ph | H | O | H | CH₂ | 1-Isopropyl-1H-pyrazol-4-yl | |
| 2.204 | 3,5-Cl₂-Ph | H | O | H | CH₂ | 1-Isopropyl-1H-pyrazol-4-yl | |
| 2.205 | 3,5-F₂-Ph | H | O | H | CH₂ | 1-Isopropyl-1H-pyrazol-4-yl | |
| 2.206 | 3-F-Ph | H | O | H | CH₂ | 1-Methyl-1H-pyrazol-4-yl | |
| 2.207 | Ph | H | O | H | CH₂ | 1-Methyl-1H-pyrazol-4-yl | |
| 2.208 | 3,5-(CF₃)₂-Ph | H | O | H | CH₂ | 1-Methyl-1H-pyrazol-4-yl | |
| 2.209 | 3,5-Cl₂-Ph | H | O | H | CH₂ | 1-Methyl-1H-pyrazol-4-yl | |
| 2.210 | 3,5-F₂-Ph | H | O | H | CH₂ | 1-Methyl-1H-pyrazol-4-yl | |
| 2.211 | 3-F-5-MeO-Ph | H | O | H | CH₂ | 1-Methyl-1H-pyrazol-4-yl | |
| 2.212 | 3-F-Ph | H | O | H | CH₂ | 1-Methyl-1H-pyrazol-4-yl | |
| 2.213 | 3-Me-Ph | H | O | H | CH₂ | 1-Methyl-1H-pyrazol-5-yl | |
| 2.214 | Ph | H | O | H | CH₂ | 1-Methyl-1H-pyrazol-5-yl | |
| 2.215 | 3,5-Cl₂-Ph | H | O | H | CH₂ | 1-Methyl-1H-pyrazol-5-yl | |
| 2.216 | 3,5-F₂-Ph | H | O | H | CH₂ | 1-Methyl-1H-pyrazol-5-yl | |
| 2.217 | 3-F-Ph | H | O | H | CH₂ | 1-Methyl-3-(trifluormethyl)-1H-pyrazol-4-yl | |
| 2.218 | Ph | H | O | H | CH₂ | 1-Methyl-3-(trifluormethyl)-1H-pyrazol-4-yl | |
| 2.219 | 3,5-Cl₂-Ph | H | O | H | CH₂ | 1-Methyl-5-(trifluormethyl)-1H-pyrazol-4-yl | D1 |
| 2.220 | 3,5-Cl₂-Ph | H | O | H | CH₂ | 1-Propyl-1H-pyrazol-3-yl | D2 |
| 2.221 | 3,5-F₂-Ph | H | O | H | CH₂ | 1-Propyl-1H-pyrazol-3-yl | |
| 2.222 | 3,5-Cl₂-Ph | H | O | H | CH₂ | 1-Propyl-1H-pyrazol-4-yl | |
| 2.223 | 3,5-F₂-Ph | H | O | H | CH₂ | 1-Propyl-1H-pyrazol-4-yl | |
| 2.224 | 3,5-Cl₂-Ph | H | O | H | CH₂ | 1-Propyl-1H-pyrazol-4-yl | |
| 2.225 | 3,5-F₂-Ph | H | O | H | CH₂ | 1-Propyl-1H-pyrazol-4-yl | |
| 2.226 | 3-F-Ph | H | O | H | CH₂ | 1-Tert-butyl-1H-pyrazol-4-yl | |
| 2.227 | Ph | H | O | H | CH₂ | 1-Tert-butyl-1H-pyrazol-4-yl | |
| 2.228 | 3,5-Cl₂-Ph | H | O | H | CH₂ | 1-Tert-butyl-1H-pyrazol-4-yl | |
| 2.229 | 3,5-F₂-Ph | H | O | H | CH₂ | 2-Chlorpyridin-4-yl | |
| 2.230 | Ph | H | O | H | CH₂ | 2-Chlorpyridin-4-yl | |
| 2.231 | 3,5-F₂-Ph | H | O | H | CH₂ | 2-Methyl-1,3-thiazol-4-yl | [CDCl3] 1.04 (t,3H); 2.03 (m,1H); 2.20 (m,1H); 3.28 (d,1H); 3.70 (d,1H); 4.38 (dd,1H); 4.54 (dd,1H); 6.91 (m,1H); 7.09 (d,1H); 7.16 (s,1H); 7.18 (s,2H); 7.34 (tbr,1H); 8.33 (d,1H). |
| 2.232 | 3-F-Ph | H | O | H | CH₂ | 2-Methyl-1,3-thiazol-4-yl | [CDCl3] 1.04 (t,3H); 2.01 (m,1H); 2.20 (m,1H); 3.30 (d,1H); 3.74 (d,1H); 4.38 (dd,1H); 4.53 (dd,1H); 7.05 (m,1 H); 7.14 (m,2H); 7.35 (s br,1H); 7.39 (m,3H); 8.32 (d,1H). |
| 2.233 | 3,5-Cl₂-Ph | H | O | H | CH₂ | 3-(Cyclopropylcarba moyl)-1,2-oxazol-5-yl | |
| 2.234 | 3,5-F₂-Ph | H | O | H | CH₂ | 3-(Ethoxycarbonyl)-1,2-oxazol-5-yl | |
| 2.235 | 3,5-Cl₂-Ph | H | O | H | CH₂ | 3-(Methoxycarbonyl)p henyl | |
| 2.236 | 3,5-Cl₂-Ph | H | O | H | CH₂ | 3,5-Difluorpyridin-2-yl | |
| 2.237 | 3,5-F₂-Ph | H | O | H | CH₂ | 3-Carboxy-1,2-oxazol-5-yl | |
| 2.238 | 3,5-F₂-Ph | H | O | H | CH₂ | 3-Chlor-1-ethyl-1H-pyrazol-4-yl | |
| 2.239 | 3,5-Cl₂-Ph | H | O | H | CH₂ | 3-Chlor-1-ethyl-1H-pyrazol-4-yl | |
| 2.240 | 3,5-Cl₂-Ph | H | O | H | CH₂ | 3-Chlor-1-ethyl-1H-pyrazol-4-yl | |
| 2.241 | 3,5-F₂-Ph | H | O | H | CH₂ | 3-Chlor-1-ethyl-1H-pyrazol-4-yl | |
| 2.242 | 3-F-Ph | H | O | H | CH₂ | 3-Chlor-1-isopropyl-1H-pyrazol-4-yl | |
| 2.243 | Ph | H | O | H | CH₂ | 3-Chlor-1-isopropyl-1H-pyrazol-4-yl | |
| 2.244 | 3,5-Cl₂-Ph | H | O | H | CH₂ | 3-Chlor-1-isopropyl-1H-pyrazol-4-yl | |
| 2.245 | 3,5-F₂-Ph | H | O | H | CH₂ | 3-Chlor-1-isopropyl-1H-pyrazol-4-yl | |
| 2.246 | 3,5-F₂-Ph | H | S | H | CH₂ | 3-Chlor-1-methyl-1H-pyrazol-4-yl | |
| 2.247 | 3-F-Ph | H | O | H | CH₂ | 3-Chlor-1-methyl-1 H-pyrazol-4-yl | |
| 2.248 | 3,5-Cl₂-Ph | H | O | H | CH₂ | 3-Chlorpyridin-4-yl | |
| 2.249 | 3,5-F₂-Ph | H | O | H | CH₂ | 3-Chlorpyridin-4-yl | |
| 2.250 | 3,5-F₂-Ph | H | O | H | CH₂ | 3-Cyclopropyl-1,2,4-oxadiazol-5-yl | |
| 2.251 | 3-F-Ph | H | O | H | CH₂ | 3-Cyclopropyl-1,2,4-oxadiazol-5-yl | |
| 2.252 | 3,5-Cl₂-Ph | H | O | H | CH₂ | 3-Furyl | |
| 2.253 | 3,5-F₂-Ph | H | O | H | CH₂ | 3-Methyl-1H-pyrazol-4-yl | |
| 2.254 | 3,5-F₂-Ph | H | O | H | CH₂ | 3-Methyl-1-propyl-1H-pyrazol-4-yl | |
| 2.255 | 3,5-F₂-Ph | H | O | H | CH₂ | 3-Methyl-1-propyl-1H-pyrazol-4-yl | |
| 2.256 | 3-CF₃O-Ph | H | O | H | CH₂ | 3-Methyl-1-propyl-1H-pyrazol-4-yl | |
| 2.257 | 3,5-Cl₂-Ph | H | O | H | CH₂ | 3-Methyl-1-propyl-1H-pyrazol-4-yl | [CDCl₃] 0.89 (t, 3H); 0.97 (t, 3H); 1.83 (m, 2H); 1.95 (m, 1H); 2.15 (m, 1H); 2.20 (s, 3H); 3.21 (d, 1H), 3.69 (d, 1H); 3.96 (t, 2H); 4.17 (dd, 1H); 4.32 (dd, 1H); 6.87 (t br, 1H); 7.24 (s, 1H); 7.42 (t, 1H); 7.50 (d, 2H) |
| 2.258 | 3,5-F₂-Ph | H | O | H | CH₂ | 3-Methyl-1-propyl-1H-pyrazol-4-yl | |
| 2.259 | 3-Cl-4-F-Ph | H | O | H | CH₂ | 3-Methyl-1-propyl-1H-pyrazol-4-yl | |
| 2.260 | 3-Cl-5-CF₃O-Ph | H | O | H | CH₂ | 3-Methyl-1-propyl-1 H-pyrazol-4-yl | |
| 2.261 | 3-Cl-5-F-Ph | H | O | H | CH₂ | 3-Methyl-1-propyl-1H-pyrazol-4-yl | |
| 2.262 | 3-Cl-5-Me-Ph | H | O | H | CH₂ | 3-Methyl-1-propyl-1H-pyrazol-4-yl | |
| 2.263 | 3-F-5-Me-Ph | H | O | H | CH₂ | 3-Methyl-1-propyl-1H-pyrazol-4-yl | |
| 2.264 | 3-F-Ph | H | O | H | CH₂ | 3-Methyl-1-propyl-1H-pyrazol-4-yl | |
| 2.265 | 3-Me-Ph | H | O | H | CH₂ | 3-Methyl-1-propyl-1 H-pyrazol-4-yl | |
| 2.266 | 4-Cl-3,5-F₂-Ph | H | O | H | CH₂ | 4-(Methoxycarbonyl)p henyl | |
| 2.267 | Ph | H | O | H | CH₂ | 4-(Trifluormethyl)-1,3-thiazol-2-yl | |
| 2.268 | 3,5-Cl₂-Ph | H | O | H | CH₂ | 4-(Trifluormethyl)-1,3-thiazol-2-yl | |
| 2.269 | 3,5-Cl₂-Ph | H | O | H | CH₂ | 4-(Trifluormethyl)-1,3-thiazol-2-yl | |
| 2.270 | 3,5-F₂-Ph | H | O | H | CH₂ | 4-Chlor-1-methyl-1H-pyrazol-3-yl | |
| 2.271 | 3-F-Ph | H | O | H | CH₂ | 4-Chlor-1-methyl-1H-pyrazol-3-yl | |
| 2.272 | 3,5-Cl₂-Ph | H | O | H | CH₂ | 4-Chlor-1-methyl-1H-pyrazol-3-yl | |
| 2.273 | 3,5-F₂-Ph | H | O | H | CH₂ | 4-Chlor-1-methyl-1H-pyrazol-5-yl | |
| 2.274 | 3-F-Ph | H | O | H | CH₂ | 5-Chlor-1-methyl-1H-pyrazol-4-yl | |
| 2.275 | 3,5-F₂-Ph | H | O | H | CH₂ | 5-Chlor-1-methyl-1H-pyrazol-4-yl | |
| 2.276 | 3,5-Cl₂-Ph | H | O | H | CH₂ | 5-Chlor-1-methyl-1H-pyrazol-4-yl | |
| 2.277 | 3,5-F₂-Ph | H | O | H | CH₂ | 5-Chlor-1-methyl-1H-pyrazol-4-yl | |
| 2.278 | 3,5-F₂-Ph | H | S | H | CH₂ | 5-Chlor-1-methyl-1H-pyrazol-4-yl | |
| 2.279 | 3-F-Ph | H | O | H | CH₂ | 5-Methoxy-1,3-dimethyl-1H-pyrazol-4-yl | |
| 2.280 | Ph | H | O | H | CH₂ | 5-Methoxy-1,3-dimethyl-1H-pyrazol-4-yl | |
| 2.281 | 3,5-Cl₂-Ph | H | O | H | CH₂ | 5-Methoxy-1,3-dimethyl-1H-pyrazol-4-yl | |
| 2.282 | 3,5-F₂-Ph | H | O | H | CH₂ | 5-Methyl-1,2,4-oxad iazol-3-yl | |
| 2.283 | 3-F-Ph | H | O | H | CH₂ | 5-Methyl-1,2,4-oxad iazol-3-yl | |
| 2.284 | 3,5-Cl₂-Ph | H | O | H | CH₂ | 5-Methyl-1,2-oxazol-3-yl | |
| 2.285 | 3,5-F₂-Ph | H | O | H | CH₂ | 5-Methyl-1-propyl-1H-pyrazol-4-yl | |
| 2.286 | 3,5-Cl₂-Ph | H | O | H | CH₂ | 5-Methyl-1-propyl-1H-pyrazol-4-yl | |
| 2.287 | 3,5-Cl₂-Ph | H | O | H | CH₂ | 5-Methyl-1-propyl-1H-pyrazol-4-yl | |
| 2.288 | 3,5-F₂-Ph | H | O | H | CH₂ | 5-Methyl-1-propyl-1H-pyrazol-4-yl | |
| 2.289 | 3-F-Ph | H | O | H | CH₂ | 6-Chlorpyridin-3-yl | |
| 2.290 | Ph | H | O | H | CH₂ | Furan-2-yl | |
| 2.291 | 3,5-F₂-Ph | H | O | H | CH₂ | Pyridin-2-yl | |
| 2.292 | 3,5-F₂-Ph | H | O | H | CH₂ | Pyridin-2-yl | |
| 2.293 | 3,5-Cl₂-Ph | H | O | H | CH₂ | Pyridin-3-yl | |
| 2.294 | 3,5-F₂-Ph | H | O | H | CH₂ | Pyridin-3-yl | |
| 2.295 | 3,5-F₂-Ph | H | O | H | CH₂ | Pyridin-4-yl | |
| 2.296 | 3-F-Ph | H | O | H | CH₂ | Pyridin-4-yl | |
| 2.297 | 3,5-F₂-Ph | H | O | H | CH₂ | Pyrimidin-2-yl | |
| 2.298 | 3-F-Ph | H | O | H | CH₂ | 1-(2,2,2-Trifluorethyl)-1H-pyrazol-4-yl | |
| 2.299 | 3,5-Cl₂-Ph | H | O | H | CH₂ | 1-(2-Chlorethyl)-1H-pyrazol-4-yl | |
| 2.300 | 3,5-F₂-Ph | H | O | H | CH₂CH₂ | 1-(2-Methoxy-2-oxoethyl)-1H-pyrazol-4-yl | |
| 2.301 | 3,5-F₂-Ph | H | O | H | CH₂CH₂ | 1-(3-Carboxypropyl)-1H-pyrazol-4-yl | |
| 2.302 | 3,5-Cl₂-Ph | H | O | H | CH₂CH₂ | 1-(3-Methoxy-3-oxopropyl)-1H-pyrazol-4-yl | |
| 2.303 | 3,5-F₂-Ph | H | O | H | CH₂CH₂ | 1-(4-Methoxy-4-oxobutyl)-1H-pyrazol-4-yl | |
| 2.304 | 3,5-F₂-Ph | H | O | H | CH₂CH₂ | 1-(Cyanomethyl)-1H-pyrazol-4-yl | |
| 2.305 | 3,5-F₂-Ph | H | O | H | CH₂CH₂ | 1-(Cyanomethyl)-1H-pyrazol-4-yl | |
| 2.306 | 3,5-F₂-Ph | H | O | H | CH₂CH₂ | 1-(Cyanomethyl)-1H-pyrazol-4-yl | |
| 2.307 | 3,5-Cl₂-Ph | H | O | H | CH₂CH₂ | 1,3-Dimethyl-1H-pyrazol-4-yl | |
| 2.308 | 3-F-Ph | H | O | H | CH₂CH₂ | 1,3-Dimethyl-1H-pyrazol-4-yl | |
| 2.309 | 3,5-F₂-Ph | H | O | H | CH₂CH₂ | 1,3-Dimethyl-1H-pyrazol-4-yl | |
| 2.310 | 3-F-Ph | H | O | H | CH₂CH₂ | 1,3-Dimethyl-1H-pyrazol-4-yl | |
| 2.311 | 3-Me-Ph | H | O | H | CH₂CH₂ | 1,3-Dimethyl-1H-pyrazol-4-yl | |
| 2.312 | Ph | H | O | H | CH₂CH₂ | 1,3-Dimethyl-1H-pyrazol-4-yl | |
| 2.313 | 3-Cl-Ph | H | O | H | CH₂CH₂ | 1,3-Dimethyl-1H-pyrazol-4-yl | |
| 2.314 | 3,5-Cl₂-Ph | H | O | H | CH₂CH₂ | 1,3-Dimethyl-1H-pyrazol-5-yl | |
| 2.315 | 3,5-(CF₃)₂-Ph | H | O | H | CH₂CH₂ | 1,3-Dimethyl-1H-pyrazol-5-yl | |
| 2.316 | 3,5-F₂-Ph | H | O | H | CH₂CH₂ | 1,3-Dimethyl-1H-pyrazol-5-yl | |
| 2.317 | Ph | H | O | H | CH₂CH₂ | 1,3-Dimethyl-1H-pyrazol-5-yl | |
| 2.318 | 3,5-Cl₂-Ph | H | O | H | CH₂CH₂ | 1,3-Oxazol-4-yl | |
| 2.319 | 3,5-(CF₃)₂-Ph | H | O | H | CH₂CH₂ | 1,3-Oxazol-4-yl | |
| 2.320 | 3,5-F₂-Ph | H | O | H | CH₂CH₂ | 1,3-Oxazol-4-yl | |
| 2.321 | 3,5-Cl₂-Ph | H | O | H | CH₂CH₂ | 1,3-Thiazol-2-yl | |
| 2.322 | 3-F-Ph | H | O | H | CH₂CH₂ | 1,3-Thiazol-2-yl | |
| 2.323 | 3,5-Cl₂-Ph | H | O | H | CH₂CH₂ | 1,4-Dimethyl-5-oxo-4,5-dihydro-1H-1,2,4-triazol-3-yl | |
| 2.324 | 3,5-F₂-Ph | H | O | H | CH₂CH₂ | 1,5-Dimethyl-1H-pyrazol-3-yl | |
| 2.325 | 3,5-Cl₂-Ph | H | O | H | CH₂CH₂ | 1,5-Dimethyl-1H-pyrazol-4-yl | |
| 2.326 | 3,5-F₂-Ph | H | O | H | CH₂CH₂ | 1,5-Dimethyl-1H-pyrazol-4-yl | |
| 2.327 | 3,5-F₂-Ph | H | O | H | CH₂CH₂ | 1,5-Dimethyl-1H-pyrazol-4-yl | |
| 2.328 | 3-F-Ph | H | O | H | CH₂CH₂ | 1,5-Dimethyl-1H-pyrazol-4-yl | |
| 2.329 | 3,5-Cl₂-Ph | H | O | H | CH₂CH₂ | 1-Acetyl-1H-pyrazol-4-yl | |
| 2.330 | Ph | H | O | H | CH₂CH₂ | 1-Butyl-3-methyl-1H-pyrazol-4-yl | |
| 2.331 | 3,5-F₂-Ph | H | O | H | CH₂CH₂ | 1-Butyl-5-methyl-1H-pyrazol-4-yl | |
| 2.332 | 3,5-F₂-Ph | H | O | H | CH₂CH₂ | 1-Cyclopropyl-1H-pyrazol-4-yl | |
| 2.333 | 3,5-F₂-Ph | H | O | H | CH₂CH₂ | 1-Cyclopropyl-1H-pyrazol-4-yl | |
| 2.334 | 3,5-F₂-Ph | H | O | H | CH₂CH₂ | 1-Cyclopropyl-1H-pyrazol-4-yl | |
| 2.335 | Ph | H | O | H | CH₂CH₂ | 1-Cyclopropyl-1H-pyrazol-4-yl | |
| 2.336 | 3-F-Ph | H | O | H | CH₂CH₂ | 1-Ethyl-1H-pyrazol-4-yl | |
| 2.337 | 3,5-Cl₂-Ph | H | O | H | CH₂CH₂ | 1-Ethyl-1H-pyrazol-4-yl | |
| 2.338 | 3,5-Cl₂-Ph | H | O | H | CH₂CH₂ | 1-Ethyl-1H-pyrazol-4-yl | |
| 2.339 | 3-F-Ph | H | O | H | CH₂CH₂ | 1-Ethyl-1H-pyrazol-4-yl | |
| 2.340 | 3,5-F₂-Ph | H | O | H | CH₂CH₂ | 1-Ethyl-1H-pyrazol-4-yl | |
| 2.341 | 3-Br-5-CF₃-Ph | H | O | H | CH₂CH₂ | 1-Ethyl-1H-pyrazol-4-yl | |
| 2.342 | 3-Me-Ph | H | O | H | CH₂CH₂ | 1-Ethyl-1H-pyrazol-4-yl | |
| 2.343 | 3-F-5-MeO-Ph | H | O | H | CH₂CH₂ | 1-Ethyl-1H-pyrazol-4-yl | |
| 2.344 | 3-CI-5-Et-Ph | H | O | H | CH₂CH₂ | 1-Ethyl-1H-pyrazol-4-yl | |
| 2.345 | 3,5-(MeO)₂-Ph | H | O | H | CH₂CH₂ | 1-Ethyl-1H-pyrazol-4-yl | |
| 2.346 | 3-Me-5-CF₃O-Ph | H | O | H | CH₂CH₂ | 1-Ethyl-1H-pyrazol-4-yl | |
| 2.347 | 3,5-Cl₂-4-MeO-Ph | H | O | H | CH₂CH₂ | 1-Ethyl-1H-pyrazol-5-yl | |
| 2.348 | Ph | H | O | H | CH₂CH₂ | 1-Ethyl-1H-pyrazol-5-yl | |
| 2.349 | 3,5-F₂-Ph | H | O | H | CH₂CH₂ | 1-Ethyl-1H-pyrazol-5-yl | |
| 2.350 | 3,5-Cl₂-Ph | H | O | H | CH₂CH₂ | 1-Ethyl-3-methyl-1H-pyrazol-4-yl | |
| 2.351 | Ph | H | O | H | CH₂CH₂ | 1-Ethyl-3-methyl-1H-pyrazol-4-yl | |
| 2.352 | 3,5-F₂-Ph | H | O | H | CH₂CH₂ | 1-Ethyl-3-methyl-1H-pyrazol-4-yl | |
| 2.353 | 3-Me-Ph | H | O | H | CH₂CH₂ | 1-Ethyl-3-methyl-1H-pyrazol-4-yl | |
| 2.354 | 3-Br-5-F-Ph | H | O | H | CH₂CH₂ | 1-Ethyl-3-methyl-1H-pyrazol-4-yl | |
| 2.355 | 3,4,5-F₃-Ph | H | O | H | CH₂CH₂ | 1-Ethyl-3-methyl-1H-pyrazol-4-yl | |
| 2.356 | 3,4-F₂-Ph | H | O | H | CH₂CH₂ | 1-Ethyl-3-methyl-1H-pyrazol-4-yl | |
| 2.357 | 3,5-F₂-Ph | H | S | H | CH₂CH₂ | 1-Ethyl-3-methyl-1H-pyrazol-4-yl | |
| 2.358 | 3-F-5-Me-Ph | H | O | H | CH₂CH₂ | 1-Ethyl-3-methyl-1H-pyrazol-4-yl | |
| 2.359 | 3-Cl-5-CN-Ph | H | O | H | CH₂CH₂ | 1-Ethyl-3-methyl-1H-pyrazol-4-yl | |
| 2.360 | 3-F-Ph | H | O | H | CH₂CH₂ | 1-Ethyl-3-methyl-1H-pyrazol-4-yl | |
| 2.361 | 3-cPr-5-F-Ph | H | O | H | CH₂CH₂ | 1-Ethyl-3-methyl-1H-pyrazol-4-yl | |
| 2.362 | 3-Cl-5-Me-Ph | H | O | H | CH₂CH₂ | 1-Ethyl-3-methyl-1H-pyrazol-4-yl | |
| 2.363 | 3-F-5-MeO-Ph | H | O | H | CH₂CH₂ | 1-Ethyl-3-methyl-1H-pyrazol-4-yl | |
| 2.364 | 2,5-Me₂-Ph | H | O | H | CH₂CH₂ | 1-Ethyl-3-methyl-1H-pyrazol-4-yl | |
| 2.365 | 3-CF₃O-Ph | H | O | H | CH₂CH₂ | 1-Ethyl-3-methyl-1H-pyrazol-4-yl | |
| 2.366 | 3-CI-5-Et-Ph | H | O | H | CH₂CH₂ | 1-Ethyl-3-methyl-1H-pyrazol-4-yl | |
| 2.367 | 2,3,5-F₃-Ph | H | O | H | CH₂CH₂ | 1-Ethyl-3-methyl-1H-pyrazol-4-yl | |
| 2.368 | 3-Et-5-F-Ph | H | O | H | CH₂CH₂ | 1-Ethyl-3-methyl-1H-pyrazol-4-yl | |
| 2.369 | 3-F-5-CF₃-Ph | H | O | H | CH₂CH₂ | 1-Ethyl-3-methyl-1H-pyrazol-4-yl | |
| 2.370 | 3-Br-5-CF₃-Ph | H | O | H | CH₂CH₂ | 1-Ethyl-3-methyl-1H-pyrazol-4-yl | |
| 2.371 | 3-Cl-5-MeO-Ph | H | O | H | CH₂CH₂ | 1-Ethyl-3-methyl-1H-pyrazol-4-yl | |
| 2.372 | 3-Cl-4-F-Ph | H | O | H | CH₂CH₂ | 1-Ethyl-3-methyl-1H-pyrazol-4-yl | |
| 2.373 | 2,3-F₂-Ph | H | O | H | CH₂CH₂ | 1-Ethyl-3-methyl-1H-pyrazol-4-yl | |
| 2.374 | 3-Cl-5-CF₃-Ph | H | O | H | CH₂CH₂ | 1-Ethyl-3-methyl-1H-pyrazol-4-yl | |
| 2.375 | 3,5-Me₂-Ph | H | O | H | CH₂CH₂ | 1-Ethyl-3-methyl-1H-pyrazol-4-yl | |
| 2.376 | 3-Cl-Ph | H | O | H | CH₂CH₂ | 1-Ethyl-3-methyl-1H-pyrazol-4-yl | |
| 2.377 | 3-MeO-Ph | H | O | H | CH₂CH₂ | 1-Ethyl-3-methyl-1H-pyrazol-4-yl | |
| 2.378 | 3-Cl-5-F-Ph | H | O | H | CH₂CH₂ | 1-Ethyl-3-methyl-1H-pyrazol-4-yl | |
| 2.379 | 3-CN-5-F-Ph | H | O | H | CH₂CH₂ | 1-Ethyl-3-methyl-1H-pyrazol-4-yl | |
| 2.380 | 3-CN-Ph | H | O | H | CH₂CH₂ | 1-Ethyl-3-methyl-1H-pyrazol-4-yl | |
| 2.381 | 3-Br-5-CI-Ph | H | O | H | CH₂CH₂ | 1-Ethyl-3-methyl-1H-pyrazol-4-yl | |
| 2.382 | 2,3,4-F₃-Ph | H | O | H | CH₂CH₂ | 1-Ethyl-3-methyl-1H-pyrazol-4-yl | |
| 2.383 | 3,5-Cl₂-4-MeO-Ph | H | O | H | CH₂CH₂ | 1-Ethyl-3-methyl-1H-pyrazol-4-yl | |
| 2.384 | Ph | H | O | H | CH₂CH₂ | 1-Ethyl-3-methyl-1H-pyrazol-4-yl | |
| 2.385 | 2,5-F₂-Ph | H | O | H | CH₂CH₂ | 1-Ethyl-3-methyl-1H-pyrazol-4-yl | |
| 2.386 | 3,5-Br₂-Ph | H | O | H | CH₂CH₂ | 1-Ethyl-3-methyl-1H-pyrazol-4-yl | |
| 2.387 | 2-F-Ph | H | O | H | CH₂CH₂ | 1-Ethyl-3-methyl-1H-pyrazol-4-yl | |
| 2.388 | 2,5-Cl₂-Ph | H | O | H | CH₂CH₂ | 1-Ethyl-3-methyl-1H-pyrazol-4-yl | |
| 2.389 | 3,5-Et₂-Ph | H | O | H | CH₂CH₂ | 1-Ethyl-3-methyl-1H-pyrazol-4-yl | |
| 2.390 | 3-CF₃S-Ph | H | O | H | CH₂CH₂ | 1-Ethyl-3-methyl-1H-pyrazol-4-yl | |
| 2.391 | 3-EtO-Ph | H | O | H | CH₂CH₂ | 1-Ethyl-3-methyl-1H-pyrazol-4-yl | |
| 2.392 | 3,5-Cl₂-Ph | CH 3 | O | H | CH₂CH₂ | 1-Ethyl-3-methyl-1H-pyrazol-4-yl | |
| 2.393 | 2-F-3-Me-Ph | H | O | H | CH₂CH₂ | 1-Ethyl-3-methyl-1H-pyrazol-5-yl | |
| 2.394 | 3-CI-5-CF₃O-Ph | H | O | H | CH₂CH₂ | 1-Ethyl-3-methyl-1H-pyrazol-5-yl | |
| 2.395 | 3,5-F₂-Ph | H | O | H | CH₂CH₂ | 1-Ethyl-3-methyl-1H-pyrazol-5-yl | |
| 2.396 | 3,5-Cl₂-Ph | H | O | H | CH₂CH₂ | 1H-1,2,3-Triazol-5-yl | |
| 2.397 | 3-F-Ph | H | O | H | CH₂CH₂ | 1H-Pyrazol-4-yl | |
| 2.398 | 3,5-F₂-Ph | H | O | H | CH₂CH₂ | 1H-Pyrazol-4-yl | |
| 2.399 | 3,5-Cl₂-Ph | H | O | H | CH₂CH₂ | 1H-Pyrazol-4-yl | |
| 2.400 | 3,5-F₂-Ph | H | O | H | CH₂CH₂ | 1-Isobutyl-1H-pyrazol-4-yl | |
| 2.401 | 3-F-Ph | H | O | H | CH₂CH₂ | 1-Isobutyl-1H-pyrazol-4-yl | |
| 2.402 | 3,5-F₂-Ph | H | O | H | CH₂CH₂ | 1-Isobutyl-1H-pyrazol-4-yl | |
| 2.403 | 3,5-Cl₂-Ph | H | O | H | CH₂CH₂ | 1-Isobutyl-1H-pyrazol-4-yl | |
| 2.404 | 3-F-Ph | H | O | H | CH₂CH₂ | 1-Isopropyl-1H-pyrazol-4-yl | |
| 2.405 | Ph | H | O | H | CH₂CH₂ | 1-Isopropyl-1H-pyrazol-4-yl | |
| 2.406 | 3,5-F₂-Ph | H | O | H | CH₂CH₂ | 1-Isopropyl-1H-pyrazol-4-yl | |
| 2.407 | 3-F-Ph | H | O | H | CH₂CH₂ | 1-Isopropyl-1H-pyrazol-4-yl | |
| 2.408 | 3,5-Cl₂-Ph | H | O | H | CH₂CH₂ | 1-Methyl-1H-pyrazol-4-yl | |
| 2.409 | Ph | H | O | H | CH₂CH₂ | 1-Methyl-1H-pyrazol-4-yl | |
| 2.410 | 3,5-F₂-Ph | H | O | H | CH₂CH₂ | 1-Methyl-1H-pyrazol-4-yl | |
| 2.411 | 3-F-5-MeO-Ph | H | O | H | CH₂CH₂ | 1-Methyl-1H-pyrazol-4-yl | |
| 2.412 | 3-F-Ph | H | O | H | CH₂CH₂ | 1-Methyl-1H-pyrazol-4-yl | |
| 2.413 | Ph | H | O | H | CH₂CH₂ | 1-Methyl-1H-pyrazol-4-yl | |
| 2.414 | 3,5-(CF₃)₂-Ph | H | O | H | CH₂CH₂ | 1-Methyl-1H-pyrazol-4-yl | |
| 2.415 | 3,5-Cl₂-Ph | H | O | H | CH₂CH₂ | 1-Methyl-1H-pyrazol-5-yl | |
| 2.416 | 3-Me-Ph | H | O | H | CH₂CH₂ | 1-Methyl-1H-pyrazol-5-yl | |
| 2.417 | 3,5-F₂-Ph | H | O | H | CH₂CH₂ | 1-Methyl-1H-pyrazol-5-yl | |
| 2.418 | 3-F-Ph | H | O | H | CH₂CH₂ | 1-Methyl-1H-pyrazol-5-yl | |
| 2.419 | 3,5-Cl₂-Ph | H | O | H | CH₂CH₂ | 1-Methyl-3-(trifluormethyl)-1H-pyrazol-4-yl | |
| 2.420 | Ph | H | O | H | CH₂CH₂ | 1-Methyl-3-(trifluormethyl)-1H-pyrazol-4-yl | |
| 2.421 | 3,5-Cl₂-Ph | H | O | H | CH₂CH₂ | 1-Methyl-5-(trifluormethyl)-1H-pyrazol-4-yl | D1 |
| 2.422 | 3,5-Cl₂-Ph | H | O | H | CH₂CH₂ | 1-Propyl-1H-pyrazol-3-yl | D2 |
| 2.423 | 3,5-F₂-Ph | H | O | H | CH₂CH₂ | 1-Propyl-1H-pyrazol-3-yl | |
| 2.424 | 3,5-F₂-Ph | H | O | H | CH₂CH₂ | 1-Propyl-1H-pyrazol-4-yl | |
| 2.425 | 3,5-Cl₂-Ph | H | O | H | CH₂CH₂ | 1-Propyl-1H-pyrazol-4-yl | |
| 2.426 | 3,5-F₂-Ph | H | O | H | CH₂CH₂ | 1-Propyl-1H-pyrazol-4-yl | |
| 2.427 | 3,5-Cl₂-Ph | H | O | H | CH₂CH₂ | 1-Propyl-1H-pyrazol-4-yl | |
| 2.428 | 3-F-Ph | H | O | H | CH₂CH₂ | 1-Tert-butyl-1H-pyrazol-4-yl | |
| 2.429 | Ph | H | O | H | CH₂CH₂ | 1-Tert-butyl-1H-pyrazol-4-yl | |
| 2.430 | 3,5-F₂-Ph | H | O | H | CH₂CH₂ | 1-Tert-butyl-1H-pyrazol-4-yl | |
| 2.431 | 3,5-Cl₂-Ph | H | O | H | CH₂CH₂ | 2-Chlorpyridin-4-yl | |
| 2.432 | Ph | H | O | H | CH₂CH₂ | 2-Chlorpyridin-4-yl | |
| 2.433 | 3,5-F₂-Ph | H | O | H | CH₂CH₂ | 2-Methyl-1,3-thiazol-4-yl | |
| 2.434 | 3-F-Ph | H | O | H | CH₂CH₂ | 2-Methyl-1,3-thiazol-4-yl | |
| 2.435 | 3,5-F₂-Ph | H | O | H | CH₂CH₂ | 3-(Cyclopropylcarba moyl)-1,2-oxazol-5-yl | |
| 2.436 | 3,5-Cl₂-Ph | H | O | H | CH₂CH₂ | 3-(Ethoxycarbonyl)-1,2-oxazol-5-yl | |
| 2.437 | 3,5-Cl₂-Ph | H | O | H | CH₂CH₂ | 3-(Methoxycarbonyl)p henyl | |
| 2.438 | 3,5-Cl₂-Ph | H | O | H | CH₂CH₂ | 3,5-Difluorpyridin-2-yl | |
| 2.439 | 3,5-F₂-Ph | H | O | H | CH₂CH₂ | 3-Carboxy-1,2-oxazol-5-yl | |
| 2.440 | 3,5-F₂-Ph | H | O | H | CH₂CH₂ | 3-Chlor-1-ethyl-1H-pyrazol-4-yl | |
| 2.441 | 3,5-Cl₂-Ph | H | O | H | CH₂CH₂ | 3-Chlor-1-ethyl-1H-pyrazol-4-yl | |
| 2.442 | 3,5-F₂-Ph | H | O | H | CH₂CH₂ | 3-Chlor-1-ethyl-1H-pyrazol-4-yl | |
| 2.443 | 3-F-Ph | H | O | H | CH₂CH₂ | 3-Chlor-1-ethyl-1H-pyrazol-4-yl | |
| 2.444 | Ph | H | O | H | CH₂CH₂ | 3-Chlor-1-isopropyl-1H-pyrazol-4-yl | |
| 2.445 | 3,5-Cl₂-Ph | H | O | H | CH₂CH₂ | 3-Chlor-1-isopropyl-1H-pyrazol-4-yl | |
| 2.446 | 3,5-F₂-Ph | H | O | H | CH₂CH₂ | 3-Chlor-1-isopropyl-1H-pyrazol-4-yl | |
| 2.447 | 3-F-Ph | H | O | H | CH₂CH₂ | 3-Chlor-1-isopropyl-1H-pyrazol-4-yl | |
| 2.448 | 3,5-F₂-Ph | H | S | H | CH₂CH₂ | 3-Chlor-1-methyl-1H-pyrazol-4-yl | |
| 2.449 | 3,5-Cl₂-Ph | H | O | H | CH₂CH₂ | 3-Chlor-1-methyl-1H-pyrazol-4-yl | |
| 2.450 | 3,5-Cl₂-Ph | H | O | H | CH₂CH₂ | 3-Chlorpyridin-4-yl | |
| 2.451 | 3,5-F₂-Ph | H | O | H | CH₂CH₂ | 3-Chlorpyridin-4-yl | |
| 2.452 | 3,5-F₂-Ph | H | O | H | CH₂CH₂ | 3-Cyclopropyl-1,2,4-oxadiazol-5-yl | |
| 2.453 | 3-F-Ph | H | O | H | CH₂CH₂ | 3-Cyclopropyl-1,2,4-oxadiazol-5-yl | |
| 2.454 | 3,5-F₂-Ph | H | O | H | CH₂CH₂ | 3-Furyl | |
| 2.455 | 3,5-Cl₂-Ph | H | O | H | CH₂CH₂ | 3-Methyl-1H-pyrazol-4-yl | |
| 2.456 | 3,5-F₂-Ph | H | O | H | CH₂CH₂ | 3-Methyl-1-propyl-1H-pyrazol-4-yl | |
| 2.457 | 3,5-F₂-Ph | H | O | H | CH₂CH₂ | 3-Methyl-1-propyl-1H-pyrazol-4-yl | |
| 2.458 | 3-F-Ph | H | O | H | CH₂CH₂ | 3-Methyl-1-propyl-1H-pyrazol-4-yl | |
| 2.459 | 3,5-F₂-Ph | H | O | H | CH₂CH₂ | 3-Methyl-1-propyl-1H-pyrazol-4-yl | |
| 2.460 | 3-Cl-5-F-Ph | H | O | H | CH₂CH₂ | 3-Methyl-1-propyl-1H-pyrazol-4-yl | |
| 2.461 | 3-Cl-5-CF₃O-Ph | H | O | H | CH₂CH₂ | 3-Methyl-1-propyl-1H-pyrazol-4-yl | |
| 2.462 | 3-F-5-Me-Ph | H | O | H | CH₂CH₂ | 3-Methyl-1-propyl-1H-pyrazol-4-yl | |
| 2.463 | 3-Me-Ph | H | O | H | CH₂CH₂ | 3-Methyl-1-propyl-1H-pyrazol-4-yl | |
| 2.464 | 3-Cl-5-Me-Ph | H | O | H | CH₂CH₂ | 3-Methyl-1-propyl-1H-pyrazol-4-yl | |
| 2.465 | 3-Cl-4-F-Ph | H | O | H | CH₂CH₂ | 3-Methyl-1-propyl-1H-pyrazol-4-yl | |
| 2.466 | 3-CF₃O-Ph | H | O | H | CH₂CH₂ | 3-Methyl-1-propyl-1H-pyrazol-4-yl | |
| 2.467 | 4-Cl-3,5-F₂-Ph | H | O | H | CH₂CH₂ | 3-Methyl-1-propyl-1H-pyrazol-4-yl | |
| 2.468 | 3,5-Cl₂-Ph | H | O | H | CH₂CH₂ | 4-(Methoxycarbonyl)p henyl | |
| 2.469 | Ph | H | O | H | CH₂CH₂ | 4-(Trifluormethyl)-1,3-thiazol-2-yl | |
| 2.470 | 3,5-Cl₂-Ph | H | O | H | CH₂CH₂ | 4-(Trifluormethyl)-1,3-thiazol-2-yl | |
| 2.471 | 3,5-F₂-Ph | H | O | H | CH₂CH₂ | 4-(Trifluormethyl)-1,3-thiazol-2-yl | |
| 2.472 | 3-F-Ph | H | O | H | CH₂CH₂ | 4-Chlor-1-methyl-1H-pyrazol-3-yl | |
| 2.473 | 3,5-Cl₂-Ph | H | O | H | CH₂CH₂ | 4-Chlor-1-methyl-1H-pyrazol-3-yl | |
| 2.474 | 3,5-F₂-Ph | H | O | H | CH₂CH₂ | 4-Chlor-1-methyl-1H-pyrazol-3-yl | |
| 2.475 | 3,5-Cl₂-Ph | H | O | H | CH₂CH₂ | 4-Chlor-1-methyl-1H-pyrazol-5-yl | |
| 2.476 | 3-F-Ph | H | O | H | CH₂CH₂ | 5-Chlor-1-methyl-1H-pyrazol-4-yl | |
| 2.477 | 3,5-F₂-Ph | H | O | H | CH₂CH₂ | 5-Chlor-1-methyl-1H-pyrazol-4-yl | |
| 2.478 | 3,5-F₂-Ph | H | O | H | CH₂CH₂ | 5-Chlor-1-methyl-1H-pyrazol-4-yl | |
| 2.479 | 3-F-Ph | H | O | H | CH₂CH₂ | 5-Chlor-1-methyl-1H-pyrazol-4-yl | |
| 2.480 | 3,5-F₂-Ph | H | S | H | CH₂CH₂ | 5-Chlor-1-methyl-1H-pyrazol-4-yl | |
| 2.481 | 3,5-Cl₂-Ph | H | O | H | CH₂CH₂ | 5-Methoxy-1,3-dimethyl-1H-pyrazol-4-yl | |
| 2.482 | Ph | H | O | H | CH₂CH₂ | 5-Methoxy-1,3-dimethyl-1H-pyrazol-4-yl | |
| 2.483 | 3,5-F₂-Ph | H | O | H | CH₂CH₂ | 5-Methoxy-1,3-dimethyl-1H-pyrazol-4-yl | |
| 2.484 | 3,5-Cl₂-Ph | H | O | H | CH₂CH₂ | 5-Methyl-1,2,4-oxad iazol-3-yl | |
| 2.485 | 3-F-Ph | H | O | H | CH₂CH₂ | 5-Methyl-1,2,4-oxadiazol-3-yl | |
| 2.486 | 3,5-Cl₂-Ph | H | O | H | CH₂CH₂ | 5-Methyl-1,2-oxazol-3-yl | |
| 2.487 | 3,5-F₂-Ph | H | O | H | CH₂CH₂ | 5-Methyl-1-propyl-1H-pyrazol-4-yl | |
| 2.488 | 3,5-Cl₂-Ph | H | O | H | CH₂CH₂ | 5-Methyl-1-propyl-1H-pyrazol-4-yl | |
| 2.489 | 3-F-Ph | H | O | H | CH₂CH₂ | 5-Methyl-1-propyl-1H-pyrazol-4-yl | |
| 2.490 | 3,5-F₂-Ph | H | O | H | CH₂CH₂ | 5-Methyl-1-propyl-1H-pyrazol-4-yl | |
| 2.491 | 3,5-Cl₂-Ph | H | O | H | CH₂CH₂ | 6-Chlorpyridin-3-yl | |
| 2.492 | Ph | H | O | H | CH₂CH₂ | Furan-2-yl | |
| 2.493 | 3,5-F₂-Ph | H | O | H | CH₂CH₂ | Pyridin-2-yl | |
| 2.494 | 3,5-F₂-Ph | H | O | H | CH₂CH₂ | Pyridin-2-yl | |
| 2.495 | 3,5-Cl₂-Ph | H | O | H | CH₂CH₂ | Pyridin-3-yl | |
| 2.496 | 3,5-F₂-Ph | H | O | H | CH₂CH₂ | Pyridin-3-yl | |
| 2.497 | 3,5-F₂-Ph | H | O | H | CH₂CH₂ | Pyridin-4-yl | |
| 2.498 | 3-F-Ph | H | O | H | CH₂CH₂ | Pyridin-4-yl | |
| 2.499 | 3,5-F₂-Ph | H | O | H | CH₂CH₂ | Pyrimidin-2-yl | |
| 2.500 | 3-F-Ph | H | O | H | CH₂CH₂ | 1-(2,2,2-Trifluorethyl)-1H-pyrazol-4-yl | |
| 2.501 | 3,5-Cl₂-Ph | H | O | H | CH₂CH₂ | 1-(2-Chlorethyl)-1H-pyrazol-4-yl | |
| 2.502 | 3,5-F₂-Ph | H | O | H | CHCH₃ | 1-(2-Methoxy-2-oxoethyl)-1H-pyrazol-4-yl | |
| 2.503 | 3,5-F₂-Ph | H | O | H | CHCH₃ | 1-(3-Carboxypropyl)-1H-pyrazol-4-yl | |
| 2.504 | 3,5-Cl₂-Ph | H | O | H | CHCH₃ | 1-(3-Methoxy-3-oxopropyl)-1H-pyrazol-4-yl | |
| 2.505 | 3,5-F₂-Ph | H | O | H | CHCH₃ | 1-(4-Methoxy-4-oxobutyl)-1H-pyrazol-4-yl | |
| 2.506 | 3,5-F₂-Ph | H | O | H | CHCH₃ | 1-(Cyanomethyl)-1H-pyrazol-4-yl | |
| 2.507 | 3,5-F₂-Ph | H | O | H | CHCH₃ | 1-(Cyanomethyl)-1H-pyrazol-4-yl | |
| 2.508 | 3,5-F₂-Ph | H | O | H | CHCH₃ | 1-(Cyanomethyl)-1H-pyrazol-4-yl | |
| 2.509 | 3,5-Cl₂-Ph | H | O | H | CHCH₃ | 1,3-Dimethyl-1H-pyrazol-4-yl | |
| 2.510 | 3-F-Ph | H | O | H | CHCH₃ | 1,3-Dimethyl-1H-pyrazol-4-yl | |
| 2.511 | 3,5-F₂-Ph | H | O | H | CHCH₃ | 1,3-Dimethyl-1H-pyrazol-4-yl | |
| 2.512 | 3-F-Ph | H | O | H | CHCH₃ | 1,3-Dimethyl-1H-pyrazol-4-yl | |
| 2.513 | 3-Me-Ph | H | O | H | CHCH₃ | 1,3-Dimethyl-1H-pyrazol-4-yl | |
| 2.514 | Ph | H | O | H | CHCH₃ | 1,3-Dimethyl-1H-pyrazol-4-yl | |
| 2.515 | 3-CI-Ph | H | O | H | CHCH₃ | 1,3-Dimethyl-1H-pyrazol-4-yl | |
| 2.516 | 3,5-Cl₂-Ph | H | O | H | CHCH₃ | 1,3-Dimethyl-1H-pyrazol-5-yl | |
| 2.517 | 3,5-(CF₃)₂-Ph | H | O | H | CHCH₃ | 1,3-Dimethyl-1H-pyrazol-5-yl | |
| 2.518 | 3,5-F₂-Ph | H | O | H | CHCH₃ | 1,3-Dimethyl-1H-pyrazol-5-yl | |
| 2.519 | Ph | H | O | H | CHCH₃ | 1,3-Dimethyl-1H-pyrazol-5-yl | |
| 2.520 | 3,5-Cl₂-Ph | H | O | H | CHCH₃ | 1,3-Oxazol-4-yl | |
| 2.521 | 3,5-(CF₃)₂-Ph | H | O | H | CHCH₃ | 1,3-Oxazol-4-yl | |
| 2.522 | 3,5-F₂-Ph | H | O | H | CHCH₃ | 1,3-Oxazol-4-yl | |
| 2.523 | 3,5-Cl₂-Ph | H | O | H | CHCH₃ | 1,3-Thiazol-2-yl | |
| 2.524 | 3-F-Ph | H | O | H | CHCH₃ | 1,3-Thiazol-2-yl | |
| 2.525 | 3,5-Cl₂-Ph | H | O | H | CHCH₃ | 1,4-Dimethyl-5-oxo-4,5-dihydro-1H-1,2,4-triazol-3-yl | |
| 2.526 | 3,5-F₂-Ph | H | O | H | CHCH₃ | 1,5-Dimethyl-1H-pyrazol-3-yl | |
| 2.527 | 3,5-Cl₂-Ph | H | O | H | CHCH₃ | 1,5-Dimethyl-1H-pyrazol-4-yl | |
| 2.528 | 3,5-F₂-Ph | H | O | H | CHCH₃ | 1,5-Dimethyl-1H-pyrazol-4-yl | |
| 2.529 | 3,5-F₂-Ph | H | O | H | CHCH₃ | 1,5-Dimethyl-1H-pyrazol-4-yl | |
| 2.530 | 3-F-Ph | H | O | H | CHCH₃ | 1,5-Dimethyl-1H-pyrazol-4-yl | |
| 2.531 | 3,5-Cl₂-Ph | H | O | H | CHCH₃ | 1-Acetyl-1H-pyrazol-4-yl | |
| 2.532 | Ph | H | O | H | CHCH₃ | 1-Butyl-3-methyl-1H-pyrazol-4-yl | |
| 2.533 | 3,5-F₂-Ph | H | O | H | CHCH₃ | 1-Butyl-5-methyl-1H-pyrazol-4-yl | |
| 2.534 | 3,5-F₂-Ph | H | O | H | CHCH₃ | 1-Cyclopropyl-1H-pyrazol-4-yl | |
| 2.535 | 3,5-F₂-Ph | H | O | H | CHCH₃ | 1-Cyclopropyl-1H-pyrazol-4-yl | |
| 2.536 | 3,5-F₂-Ph | H | O | H | CHCH₃ | 1-Cyclopropyl-1H-pyrazol-4-yl | |
| 2.537 | Ph | H | O | H | CHCH₃ | 1-Cyclopropyl-1H-pyrazol-4-yl | |
| 2.538 | 3-F-Ph | H | O | H | CHCH₃ | 1-Ethyl-1H-pyrazol-4-yl | |
| 2.539 | 3,5-Cl₂-Ph | H | O | H | CHCH₃ | 1-Ethyl-1H-pyrazol-4-yl | |
| 2.540 | 3,5-Cl₂-Ph | H | O | H | CHCH₃ | 1-Ethyl-1H-pyrazol-4-yl | |
| 2.541 | 3-F-Ph | H | O | H | CHCH₃ | 1-Ethyl-1H-pyrazol-4-yl | |
| 2.542 | 3,5-F₂-Ph | H | O | H | CHCH₃ | 1-Ethyl-1H-pyrazol-4-yl | |
| 2.543 | 3-Br-5-CF₃-Ph | H | O | H | CHCH₃ | 1-Ethyl-1H-pyrazol-4-yl | |
| 2.544 | 3-Me-Ph | H | O | H | CHCH₃ | 1-Ethyl-1H-pyrazol-4-yl | |
| 2.545 | 3-F-5-MeO-Ph | H | O | H | CHCH₃ | 1-Ethyl-1H-pyrazol-4-yl | |
| 2.546 | 3-CI-5-Et-Ph | H | O | H | CHCH₃ | 1-Ethyl-1H-pyrazol-4-yl | |
| 2.547 | 3,5-(MeO)₂-Ph | H | O | H | CHCH₃ | 1-Ethyl-1H-pyrazol-4-yl | |
| 2.548 | 3-Me-5-CF₃O-Ph | H | O | H | CHCH₃ | 1-Ethyl-1H-pyrazol-4-yl | |
| 2.549 | 3,5-Cl₂-4-MeO-Ph | H | O | H | CHCH₃ | 1-Ethyl-1H-pyrazol-5-yl | |
| 2.550 | Ph | H | O | H | CHCH₃ | 1-Ethyl-1H-pyrazol-5-yl | |
| 2.551 | 3,5-F₂-Ph | H | O | H | CHCH₃ | 1-Ethyl-1H-pyrazol-5-yl | |
| 2.552 | 3,5-Cl₂-Ph | H | O | H | CHCH₃ | 1-Ethyl-3-methyl-1H-pyrazol-4-yl | |
| 2.553 | Ph | H | O | H | CHCH₃ | 1-Ethyl-3-methyl-1H-pyrazol-4-yl | |
| 2.554 | 3,5-F₂-Ph | H | O | H | CHCH₃ | 1-Ethyl-3-methyl-1H-pyrazol-4-yl | |
| 2.555 | 3-Me-Ph | H | O | H | CHCH₃ | 1-Ethyl-3-methyl-1H-pyrazol-4-yl | |
| 2.556 | 3-Br-5-F-Ph | H | O | H | CHCH₃ | 1-Ethyl-3-methyl-1H-pyrazol-4-yl | |
| 2.557 | 3,4,5-F₃-Ph | H | O | H | CHCH₃ | 1-Ethyl-3-methyl-1H-pyrazol-4-yl | |
| 2.558 | 3,4-F₂-Ph | H | O | H | CHCH₃ | 1-Ethyl-3-methyl-1H-pyrazol-4-yl | |
| 2.559 | 3,5-F₂-Ph | H | S | H | CHCH₃ | 1-Ethyl-3-methyl-1H-pyrazol-4-yl | |
| 2.560 | 3-F-5-Me-Ph | H | O | H | CHCH₃ | 1-Ethyl-3-methyl-1H-pyrazol-4-yl | |
| 2.561 | 3-Cl-5-CN-Ph | H | O | H | CHCH₃ | 1-Ethyl-3-methyl-1H-pyrazol-4-yl | |
| 2.562 | 3-F-Ph | H | O | H | CHCH₃ | 1-Ethyl-3-methyl-1H-pyrazol-4-yl | |
| 2.563 | 3-cPr-5-F-Ph | H | O | H | CHCH₃ | 1-Ethyl-3-methyl-1H-pyrazol-4-yl | |
| 2.564 | 3-Cl-5-Me-Ph | H | O | H | CHCH₃ | 1-Ethyl-3-methyl-1H-pyrazol-4-yl | |
| 2.565 | 3-F-5-MeO-Ph | H | O | H | CHCH₃ | 1-Ethyl-3-methyl-1H-pyrazol-4-yl | |
| 2.566 | 2,5-Me₂-Ph | H | O | H | CHCH₃ | 1-Ethyl-3-methyl-1H-pyrazol-4-yl | |
| 2.567 | 3-CF₃O-Ph | H | O | H | CHCH₃ | 1-Ethyl-3-methyl-1H-pyrazol-4-yl | |
| 2.568 | 3-Cl-5-Et-Ph | H | O | H | CHCH₃ | 1-Ethyl-3-methyl-1H-pyrazol-4-yl | |
| 2.569 | 2,3,5-F₃-Ph | H | O | H | CHCH₃ | 1-Ethyl-3-methyl-1H-pyrazol-4-yl | |
| 2.570 | 3-Et-5-F-Ph | H | O | H | CHCH₃ | 1-Ethyl-3-methyl-1H-pyrazol-4-yl | |
| 2.571 | 3-F-5-CF₃-Ph | H | O | H | CHCH₃ | 1-Ethyl-3-methyl-1H-pyrazol-4-yl | |
| 2.572 | 3-Br-5-CF₃-Ph | H | O | H | CHCH₃ | 1-Ethyl-3-methyl-1H-pyrazol-4-yl | |
| 2.573 | 3-Cl-5-MeO-Ph | H | O | H | CHCH₃ | 1-Ethyl-3-methyl-1H-pyrazol-4-yl | |
| 2.574 | 3-Cl-4-F-Ph | H | O | H | CHCH₃ | 1-Ethyl-3-methyl-1H-pyrazol-4-yl | |
| 2.575 | 2,3-F₂-Ph | H | O | H | CHCH₃ | 1-Ethyl-3-methyl-1H-pyrazol-4-yl | |
| 2.576 | 3-Cl-5-CF₃-Ph | H | O | H | CHCH₃ | 1-Ethyl-3-methyl-1H-pyrazol-4-yl | |
| 2.577 | 3,5-Me₂-Ph | H | O | H | CHCH₃ | 1-Ethyl-3-methyl-1H-pyrazol-4-yl | |
| 2.578 | 3-Cl-Ph | H | O | H | CHCH₃ | 1-Ethyl-3-methyl-1H-pyrazol-4-yl | |
| 2.579 | 3-MeO-Ph | H | O | H | CHCH₃ | 1-Ethyl-3-methyl-1H-pyrazol-4-yl | |
| 2.580 | 3-Cl-5-F-Ph | H | O | H | CHCH₃ | 1-Ethyl-3-methyl-1H-pyrazol-4-yl | |
| 2.581 | 3-CN-5-F-Ph | H | O | H | CHCH₃ | 1-Ethyl-3-methyl-1H-pyrazol-4-yl | |
| 2.582 | 3-CN-Ph | H | O | H | CHCH₃ | 1-Ethyl-3-methyl-1H-pyrazol-4-yl | |
| 2.583 | 3-Br-5-Cl-Ph | H | O | H | CHCH₃ | 1-Ethyl-3-methyl-1H-pyrazol-4-yl | |
| 2.584 | 2,3,4-F₃-Ph | H | O | H | CHCH₃ | 1-Ethyl-3-methyl-1H-pyrazol-4-yl | |
| 2.585 | 3,5-Cl₂-4-MeO-Ph | H | O | H | CHCH₃ | 1-Ethyl-3-methyl-1H-pyrazol-4-yl | |
| 2.586 | Ph | H | O | H | CHCH₃ | 1-Ethyl-3-methyl-1H-pyrazol-4-yl | |
| 2.587 | 2,5-F₂-Ph | H | O | H | CHCH₃ | 1-Ethyl-3-methyl-1H-pyrazol-4-yl | |
| 2.588 | 3,5-Br₂-Ph | H | O | H | CHCH₃ | 1-Ethyl-3-methyl-1H-pyrazol-4-yl | |
| 2.589 | 2-F-Ph | H | O | H | CHCH₃ | 1-Ethyl-3-methyl-1H-pyrazol-4-yl | |
| 2.590 | 2,5-Cl₂-Ph | H | O | H | CHCH₃ | 1-Ethyl-3-methyl-1H-pyrazol-4-yl | |
| 2.591 | 3,5-Et₂-Ph | H | O | H | CHCH₃ | 1-Ethyl-3-methyl-1H-pyrazol-4-yl | |
| 2.592 | 3-CF₃S-Ph | H | O | H | CHCH₃ | 1-Ethyl-3-methyl-1H-pyrazol-4-yl | |
| 2.593 | 3-EtO-Ph | H | O | H | CHCH₃ | 1-Ethyl-3-methyl-1H-pyrazol-4-yl | |
| 2.594 | 3,5-Cl₂-Ph | CH 3 | O | H | CHCH₃ | 1-Ethyl-3-methyl-1H-pyrazol-4-yl | |
| 2.595 | 2-F-3-Me-Ph | H | O | H | CHCH₃ | 1-Ethyl-3-methyl-1H-pyrazol-5-yl | |
| 2.596 | 3-CI-5-CF₃O-Ph | H | O | H | CHCH₃ | 1-Ethyl-3-methyl-1H-pyrazol-5-yl | |
| 2.597 | 3,5-F₂-Ph | H | O | H | CHCH₃ | 1-Ethyl-3-methyl-1H-pyrazol-5-yl | |
| 2.598 | 3,5-Cl₂-Ph | H | O | H | CHCH₃ | 1H-1,2,3-Triazol-5-yl | |
| 2.599 | 3-F-Ph | H | O | H | CHCH₃ | 1H-Pyrazol-4-yl | |
| 2.600 | 3,5-F₂-Ph | H | O | H | CHCH₃ | 1H-Pyrazol-4-yl | |
| 2.601 | 3,5-Cl₂-Ph | H | O | H | CHCH₃ | 1H-Pyrazol-4-yl | |
| 2.602 | 3,5-F₂-Ph | H | O | H | CHCH₃ | 1-Isobutyl-1H-pyrazol-4-yl | |
| 2.603 | 3-F-Ph | H | O | H | CHCH₃ | 1-Isobutyl-1H-pyrazol-4-yl | |
| 2.604 | 3,5-F₂-Ph | H | O | H | CHCH₃ | 1-Isobutyl-1H-pyrazol-4-yl | |
| 2.605 | 3,5-Cl₂-Ph | H | O | H | CHCH₃ | 1-Isobutyl-1H-pyrazol-4-yl | |
| 2.606 | 3-F-Ph | H | O | H | CHCH₃ | 1-Isopropyl-1H-pyrazol-4-yl | |
| 2.607 | Ph | H | O | H | CHCH₃ | 1-Isopropyl-1H-pyrazol-4-yl | |
| 2.608 | 3,5-F₂-Ph | H | O | H | CHCH₃ | 1-Isopropyl-1H-pyrazol-4-yl | |
| 2.609 | 3-F-Ph | H | O | H | CHCH₃ | 1-Isopropyl-1H-pyrazol-4-yl | |
| 2.610 | 3,5-Cl₂-Ph | H | O | H | CHCH₃ | 1-Methyl-1H-pyrazol-4-yl | |
| 2.611 | Ph | H | O | H | CHCH₃ | 1-Methyl-1H-pyrazol-4-yl | |
| 2.612 | 3,5-F₂-Ph | H | O | H | CHCH₃ | 1-Methyl-1H-pyrazol-4-yl | |
| 2.613 | 3-F-5-MeO-Ph | H | O | H | CHCH₃ | 1-Methyl-1H-pyrazol-4-yl | |
| 2.614 | 3-F-Ph | H | O | H | CHCH₃ | 1-Methyl-1H-pyrazol-4-yl | |
| 2.615 | Ph | H | O | H | CHCH₃ | 1-Methyl-1H-pyrazol-4-yl | |
| 2.616 | 3,5-(CF₃)₂-Ph | H | O | H | CHCH₃ | 1-Methyl-1H-pyrazol-4-yl | |
| 2.617 | 3,5-Cl₂-Ph | H | O | H | CHCH₃ | 1-Methyl-1H-pyrazol-5-yl | |
| 2.618 | 3-Me-Ph | H | O | H | CHCH₃ | 1-Methyl-1H-pyrazol-5-yl | |
| 2.619 | 3,5-F₂-Ph | H | O | H | CHCH₃ | 1-Methyl-1H-pyrazol-5-yl | |
| 2.620 | 3-F-Ph | H | O | H | CHCH₃ | 1-Methyl-1H-pyrazol-5-yl | |
| 2.621 | 3,5-Cl₂-Ph | H | O | H | CHCH₃ | 1-Methyl-3-(trifluormethyl)-1H-pyrazol-4-yl | |
| 2.622 | Ph | H | O | H | CHCH₃ | 1-Methyl-3-(trifluormethyl)-1H-pyrazol-4-yl | |
| 2.623 | 3,5-Cl₂-Ph | H | O | H | CHCH₃ | 1-Methyl-5-(trifluormethyl)-1H-pyrazol-4-yl | D1 |
| 2.624 | 3,5-Cl₂-Ph | H | O | H | CHCH₃ | 1-Propyl-1H-pyrazol-3-yl | D2 |
| 2.625 | 3,5-F₂-Ph | H | O | H | CHCH₃ | 1-Propyl-1H-pyrazol-3-yl | |
| 2.626 | 3,5-F₂-Ph | H | O | H | CHCH₃ | 1-Propyl-1H-pyrazol-4-yl | |
| 2.627 | 3,5-Cl₂-Ph | H | O | H | CHCH₃ | 1-Propyl-1H-pyrazol-4-yl | |
| 2.628 | 3,5-F₂-Ph | H | O | H | CHCH₃ | 1-Propyl-1H-pyrazol-4-yl | |
| 2.629 | 3,5-Cl₂-Ph | H | O | H | CHCH₃ | 1-Propyl-1H-pyrazol-4-yl | |
| 2.630 | 3-F-Ph | H | O | H | CHCH₃ | 1-Tert-butyl-1H-pyrazol-4-yl | |
| 2.631 | Ph | H | O | H | CHCH₃ | 1-Tert-butyl-1H-pyrazol-4-yl | |
| 2.632 | 3,5-F₂-Ph | H | O | H | CHCH₃ | 1-Tert-butyl-1H-pyrazol-4-yl | |
| 2.633 | 3,5-Cl₂-Ph | H | O | H | CHCH₃ | 2-Chlorpyridin-4-yl | |
| 2.634 | Ph | H | O | H | CHCH₃ | 2-Chlorpyridin-4-yl | |
| 2.635 | 3,5-F₂-Ph | H | O | H | CHCH₃ | 2-Methyl-1,3-thiazol-4-yl | |
| 2.636 | 3-F-Ph | H | O | H | CHCH₃ | 2-Methyl-1,3-thiazol-4-yl | |
| 2.637 | 3,5-F₂-Ph | H | O | H | CHCH₃ | 3-(Cyclopropylcarba moyl)-1,2-oxazol-5-yl | |
| 2.638 | 3,5-Cl₂-Ph | H | O | H | CHCH₃ | 3-(Ethoxycarbonyl)-1,2-oxazol-5-yl | |
| 2.639 | 3,5-Cl₂-Ph | H | O | H | CHCH₃ | 3-(Methoxycarbonyl)p henyl | |
| 2.640 | 3,5-Cl₂-Ph | H | O | H | CHCH₃ | 3,5-Difluorpyridin-2-yl | |
| 2.641 | 3,5-F₂-Ph | H | O | H | CHCH₃ | 3-Carboxy-1,2-oxazol-5-yl | |
| 2.642 | 3,5-F₂-Ph | H | O | H | CHCH₃ | 3-Chlor-1-ethyl-1H-pyrazol-4-yl | |
| 2.643 | 3,5-Cl₂-Ph | H | O | H | CHCH₃ | 3-Chlor-1-ethyl-1H-pyrazol-4-yl | |
| 2.644 | 3,5-F₂-Ph | H | O | H | CHCH₃ | 3-Chlor-1-ethyl-1H-pyrazol-4-yl | |
| 2.645 | 3-F-Ph | H | O | H | CHCH₃ | 3-Chlor-1-ethyl-1H-pyrazol-4-yl | |
| 2.646 | Ph | H | O | H | CHCH₃ | 3-Chlor-1-isopropyl-1H-pyrazol-4-yl | |
| 2.647 | 3,5-Cl₂-Ph | H | O | H | CHCH₃ | 3-Chlor-1-isopropyl-1H-pyrazol-4-yl | |
| 2.648 | 3,5-F₂-Ph | H | O | H | CHCH₃ | 3-Chlor-1-isopropyl-1H-pyrazol-4-yl | |
| 2.649 | 3-F-Ph | H | O | H | CHCH₃ | 3-Chlor-1-isopropyl-1H-pyrazol-4-yl | |
| 2.650 | 3,5-F₂-Ph | H | S | H | CHCH₃ | 3-Chlor-1-methyl-1H-pyrazol-4-yl | |
| 2.651 | 3,5-Cl₂-Ph | H | O | H | CHCH₃ | 3-Chlor-1-methyl-1H-pyrazol-4-yl | |
| 2.652 | 3,5-Cl₂-Ph | H | O | H | CHCH₃ | 3-Chlorpyridin-4-yl | |
| 2.653 | 3,5-F₂-Ph | H | O | H | CHCH₃ | 3-Chlorpyridin-4-yl | |
| 2.654 | 3,5-F₂-Ph | H | O | H | CHCH₃ | 3-Cyclopropyl-1,2,4-oxadiazol-5-yl | |
| 2.655 | 3-F-Ph | H | O | H | CHCH₃ | 3-Cyclopropyl-1,2,4-oxadiazol-5-yl | |
| 2.656 | 3,5-F₂-Ph | H | O | H | CHCH₃ | 3-Furyl | |
| 2.657 | 3,5-Cl₂-Ph | H | O | H | CHCH₃ | 3-Methyl-1H-pyrazol-4-yl | |
| 2.658 | 3,5-F₂-Ph | H | O | H | CHCH₃ | 3-Methyl-1-propyl-1H-pyrazol-4-yl | |
| 2.659 | 3,5-F₂-Ph | H | O | H | CHCH₃ | 3-Methyl-1-propyl-1H-pyrazol-4-yl | |
| 2.660 | 3-F-Ph | H | O | H | CHCH₃ | 3-Methyl-1-propyl-1H-pyrazol-4-yl | |
| 2.661 | 3,5-F₂-Ph | H | O | H | CHCH₃ | 3-Methyl-1-propyl-1H-pyrazol-4-yl | |
| 2.662 | 3-CI-5-F-Ph | H | O | H | CHCH₃ | 3-Methyl-1-propyl-1H-pyrazol-4-yl | |
| 2.663 | 3-Cl-5-CF₃O-Ph | H | O | H | CHCH₃ | 3-Methyl-1-propyl-1H-pyrazol-4-yl | |
| 2.664 | 3-F-5-Me-Ph | H | O | H | CHCH₃ | 3-Methyl-1-propyl-1H-pyrazol-4-yl | |
| 2.665 | 3-Me-Ph | H | O | H | CHCH₃ | 3-Methyl-1-propyl-1H-pyrazol-4-yl | |
| 2.666 | 3-Cl-5-Me-Ph | H | O | H | CHCH₃ | 3-Methyl-1-propyl-1H-pyrazol-4-yl | |
| 2.667 | 3-Cl-4-F-Ph | H | O | H | CHCH₃ | 3-Methyl-1-propyl-1H-pyrazol-4-yl | |
| 2.668 | 3-CF₃O-Ph | H | O | H | CHCH₃ | 3-Methyl-1-propyl-1H-pyrazol-4-yl | |
| 2.669 | 4-Cl-3,5-F₂-Ph | H | O | H | CHCH₃ | 3-Methyl-1-propyl-1H-pyrazol-4-yl | |
| 2.670 | 3,5-Cl₂-Ph | H | O | H | CHCH₃ | 4-(Methoxycarbonyl)p henyl | |
| 2.671 | Ph | H | O | H | CHCH₃ | 4-(Trifluormethyl)-1,3-thiazol-2-yl | |
| 2.672 | 3,5-Cl₂-Ph | H | O | H | CHCH₃ | 4-(Trifluormethyl)-1,3-thiazol-2-yl | |
| 2.673 | 3,5-F₂-Ph | H | O | H | CHCH₃ | 4-(Trifluormethyl)-1,3-thiazol-2-yl | |
| 2.674 | 3-F-Ph | H | O | H | CHCH₃ | 4-Chlor-1-methyl-1H-pyrazol-3-yl | |
| 2.675 | 3,5-Cl₂-Ph | H | O | H | CHCH₃ | 4-Chlor-1-methyl-1H-pyrazol-3-yl | |
| 2.676 | 3,5-F₂-Ph | H | O | H | CHCH₃ | 4-Chlor-1-methyl-1H-pyrazol-3-yl | |
| 2.677 | 3,5-Cl₂-Ph | H | O | H | CHCH₃ | 4-Chlor-1-methyl-1H-pyrazol-5-yl | |
| 2.678 | 3-F-Ph | H | O | H | CHCH₃ | 5-Chlor-1-methyl-1H-pyrazol-4-yl | |
| 2.679 | 3,5-F₂-Ph | H | O | H | CHCH₃ | 5-Chlor-1-methyl-1H-pyrazol-4-yl | |
| 2.680 | 3,5-F₂-Ph | H | O | H | CHCH₃ | 5-Chlor-1-methyl-1H-pyrazol-4-yl | |
| 2.681 | 3-F-Ph | H | O | H | CHCH₃ | 5-Chlor-1-methyl-1H-pyrazol-4-yl | |
| 2.682 | 3,5-F₂-Ph | H | S | H | CHCH₃ | 5-Chlor-1-methyl-1H-pyrazol-4-yl | |
| 2.683 | 3,5-Cl₂-Ph | H | O | H | CHCH₃ | 5-Methoxy-1,3-dimethyl-1H-pyrazol-4-yl | |
| 2.684 | Ph | H | O | H | CHCH₃ | 5-Methoxy-1,3-dimethyl-1H-pyrazol-4-yl | |
| 2.685 | 3,5-F₂-Ph | H | O | H | CHCH₃ | 5-Methoxy-1,3-dimethyl-1H-pyrazol-4-yl | |
| 2.686 | 3,5-Cl₂-Ph | H | O | H | CHCH₃ | 5-Methyl-1,2,4-oxadiazol-3-yl | |
| 2.687 | 3-F-Ph | H | O | H | CHCH₃ | 5-Methyl-1,2,4-oxadiazol-3-yl | |
| 2.688 | 3,5-Cl₂-Ph | H | O | H | CHCH₃ | 5-Methyl-1,2-oxazol-3-yl | |
| 2.689 | 3,5-F₂-Ph | H | O | H | CHCH₃ | 5-Methyl-1-propyl-1H-pyrazol-4-yl | |
| 2.690 | 3,5-Cl₂-Ph | H | O | H | CHCH₃ | 5-Methyl-1-propyl-1H-pyrazol-4-yl | |
| 2.691 | 3-F-Ph | H | O | H | CHCH₃ | 5-Methyl-1-propyl-1H-pyrazol-4-yl | |
| 2.692 | 3,5-F₂-Ph | H | O | H | CHCH₃ | 5-Methyl-1-propyl-1H-pyrazol-4-yl | |
| 2.693 | 3,5-Cl₂-Ph | H | O | H | CHCH₃ | 6-Chlorpyridin-3-yl | |
| 2.694 | Ph | H | O | H | CHCH₃ | Furan-2-yl | |
| 2.695 | 3,5-F₂-Ph | H | O | H | CHCH₃ | Pyridin-2-yl | |
| 2.696 | 3,5-F₂-Ph | H | O | H | CHCH₃ | Pyridin-2-yl | |
| 2.697 | 3,5-Cl₂-Ph | H | O | H | CHCH₃ | Pyridin-3-yl | |
| 2.698 | 3,5-F₂-Ph | H | O | H | CHCH₃ | Pyridin-3-yl | |
| 2.699 | 3,5-F₂-Ph | H | O | H | CHCH₃ | Pyridin-4-yl | |
| 2.700 | 3-F-Ph | H | O | H | CHCH₃ | Pyridin-4-yl | |
| 2.701 | 3,5-F₂-Ph | H | O | H | CHCH₃ | Pyrimidin-2-yl | |
| 2.702 | 3-F-Ph | H | O | H | CHCH₃ | Pyridin-4-yl | |
| 2.703 | 3,5-Cl₂-Ph | H | O | H | CHCH₃ | Pyrimidin-2-yl | |
| 2.704 | 3-F-Ph | H | O | H | Bindung | 3,5-Dimethyl-1,2-oxazol-4-yl | |
| 2.705 | 3-F-Ph | H | O | H | CH₂ | 1,2-Oxazol-4-yl | |
| 2.706 | 3,5-F₂-Ph | H | O | H | CH₂ | 2,5-Dimethyl-1,3-oxazol-4-yl | |
| 2.707 | 3,5-F₂-Ph | H | O | H | CH₂ | 2-Fluorpyridin-4-yl | [CDCl₃] 1.04 (t,3H); 2.01 (m,1H); 2.20 (m,1H); 3.27 (d,1H); 3.71 (d,1H); 4.41 (dd,1H); 4.59 (dd,1H); 6.80 (s,1H); 6.90 (m,1H); 7.04 (d,1H); 7.16 (m,2H); 7.32 (t br,1H); 8.15 (d,1H). |
| 2.708 | 3-F-Ph | H | O | H | CH₂ | 2-Fluorpyridin-4-yl | [CDCl₃] 1.04 (t,3H);2.03 (m,1H); 2.20 (m,1H); 3.30 (d,1H); 3.74 (d,1H); 4.41 (dd,1H); 4.60 (dd,1H); 6.78 (s,1H); 7.05 (m,1H); 7.14 (m,1H); 7.33 (s br,1H); 7.51 (m,3H); 8.16 (d,1 H). |
| 2.709 | 3-F-Ph | H | O | H | CH₂ | 2-Methoxypyridin-4-yl | [COCl₃] 1.04 (t,3H); 2.00 (m,1H); 2.20 (m,1H); 3.29 (d,1H); 3.74 (d,1H); 3.90 (s,1H); 4.32 (dd,1H); 4.51 (dd,1H); 6.59 (s,1H); 6.73 (d,1H); 7.15 (m,1H); 3.38 (m,3H); 8.09 (d,1H); |
| 2.710 | 3,5-F₂-Ph | H | O | H | CH₂ | 2-Methoxypyridin-4-yl | [CDCl₃] 1.03 (t,3H); 2.01 (m,1H); 2.19 (m,1H); 3.26 (d,1H); 3.71 (d,1H); 3.90 (s,3H); 4.41 (AB d,2H); 6.58 (s,1H); 6.73 (d,1H); 6.89 (m,2H); 7.17 (m,2H); 7.18 (s br,1H); 8.08 (d,1 H). |
| 2.711 | 3,5-F₂-Ph | H | O | H | CH₂ | 3,5-Diethyl-1,2-oxazol-4-yl | |
| 2.712 | 3-F-Ph | H | O | H | CH₂ | 3,5-Dimethyl-1,2-oxazol-4-yl | |
| 2.713 | 3,5-F₂-Ph | H | O | H | CH₂ | 3,5-Dimethyl-1,2-oxazol-4-yl | [CDCl₃] 0.98 (t,3H); 1.95 (m,1H); 2.13 (m,1H); 2.21 (s,3H); 2.38 (s,3H); 3.23 (d,1H); 6.68 (d,1H); 4.11 (dd,1H); 4.26 (dd,1H); 6.88 (m,2H); 7.15 (m,2H). |
| 2.714 | 3,5-Cl₂-Ph | H | O | H | CH₂ | 3,5-Dimethyl-1,2-oxazol-4-yl | [CDCl₃] 0.97 (t,3H); 1.95 (m,1H); 2.12 (m,1H); 2.21 (s,3H); 2.38 (s,3H); 3.23 (d,1H); 3.68 (d,1H); 4.11 (dd,1H); 4.27 (dd,1H); 6.90 (t br,1H); 7.42 (s,1H); 7.51 (s,2H). |
| 2.715 | 3,5-F₂-Ph | H | O | H | CH₂ | 3-Chlor-5-(trifluormethyl)pyridin-2-yl | |
| 2.716 | 3,5-F₂-Ph | H | O | H | CH₂ | 3-Ethyl-5-methyl-1,2-oxazol-4-yl | |
| 2.717 | 3-F-Ph | H | O | H | CH₂ | 3-Ethyl-5-methyl-1,2-oxazol-4-yl | |
| 2.718 | 3-F-Ph | H | O | H | CH₂ | 6-(Trifluormethyl)pyridin-3-yl | [CDCl₃] 1.01 (t,3H); 1.99 (m,1H); 2.17 (m,1H); 3.29 (d,1H); 3.73 (d,1H); 4.45 (dd,1H); 4.65 (dd,1H); 7.15 (m,1H); 7.38 (m,3H); 7.63 (d,1H); 7.77 (d,1H); 8.65 (s,1H). |
| 2.719 | 3,5-F₂-Ph | H | O | H | CH₂CH₂ | 3-Chlor-5-(trifluormethyl)pyridin-2-yl | |

| Tabelle 3: Erfindungsgemäße Verbindungen der allgemeinen Formel (I), worin R² für Wasserstoff und R³ für Trifluormethyl stehen, und Aryl den Rest 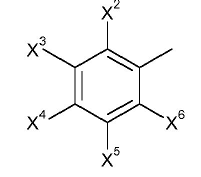 bedeutet. | | | | | | | |
|---|---|---|---|---|---|---|---|
| 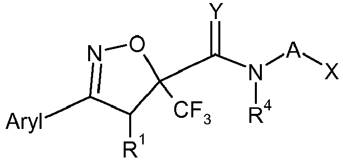 | | | | | | | |

| Nr. | Aryl | R¹ | Y | R⁴ | A | X | Physikalische Daten |
|---|---|---|---|---|---|---|---|
| 3.001 | 3,5-Cl₂-Ph | H | O | H | Bindung | 1,2-Oxazol-3-yl | |
| 3.002 | 3,5-(CF₃)₂-Ph | H | O | H | Bindung | 1,3-Dimethyl-1H-pyrazol-5-yl | |
| 3.003 | 3,5-Cl₂-Ph | H | O | H | Bindung | 1,3-Dimethyl-1H-pyrazol-5-yl | |
| 3.004 | 3,5-Cl₂-Ph | H | O | H | Bindung | 1,3-Oxazol-2-yl | |
| 3.005 | 3,5-Cl₂-Ph | H | O | H | Bindung | 1,3-Thiazol-2-yl | |
| 3.006 | 3,5-Cl₂-Ph | H | O | H | Bindung | 1,5-Dimethyl-1H-pyrazol-4-yl | |
| 3.007 | 3,5-F₂-Ph | H | O | H | Bindung | 1,5-Dimethyl-1H-pyrazol-4-yl | |
| 3.008 | Ph | H | O | H | Bindung | 1,5-Dimethyl-1H-pyrazol-4-yl | |
| 3.009 | 3-F-Ph | H | O | H | Bindung | 1,5-Dimethyl-1H-pyrazol-4-yl | |
| 3.010 | 3-Me-Ph | H | O | H | Bindung | 1,5-Dimethyl-1H-pyrazol-4-yl | |
| 3.011 | 3,5-F₂-Ph | H | O | H | Bindung | 1-Ethyl-3-(methoxycarbonyl)-1H-pyrazol-4-yl | |
| 3.012 | 3,5-Cl₂-Ph | H | O | H | Bindung | 1-Ethyl-3-(methoxycarbonyl)-1H-pyrazol-4-yl | |
| 3.013 | 3-F-Ph | H | O | H | Bindung | 1-Ethyl-3-(methoxycarbonyl)-1H-pyrazol-4-yl | |
| 3.014 | Ph | H | O | H | Bindung | 1-Ethyl-3-(methoxycarbonyl)-1H-pyrazol-4-yl | |
| 3.015 | 3,5-F₂-Ph | H | O | H | Bindung | 1-Ethyl-3-methyl-1H-pyrazol-4-yl | |
| 3.016 | 3-F-Ph | H | O | H | Bindung | 1-Ethyl-3-methyl-1H-pyrazol-4-yl | |
| 3.017 | 3,5-Cl₂-Ph | H | O | H | Bindung | 1-Ethyl-3-methyl-1H-pyrazol-4-yl | |
| 3.018 | Ph | H | O | H | Bindung | 1-Ethyl-3-methyl-1H-pyrazol-4-yl | |
| 3.019 | 3,5-Cl₂-Ph | H | O | H | Bindung | 1-Methyl-1H-1,2,4-triazol-3-yl | |
| 3.020 | 3,5-F₂-Ph | H | O | H | Bindung | 1-Methyl-1H-1,2,4-triazol-3-yl | |
| 3.021 | 3,5-Cl₂-Ph | H | O | H | Bindung | 1-Methyl-1H-imidazol-2-yl | |
| 3.022 | 3,5-F₂-Ph | H | O | H | Bindung | 1-Methyl-1H-pyrazol-4-yl | |
| 3.023 | 3,5-Cl₂-Ph | H | O | H | Bindung | 1-Methyl-1H-pyrazol-4-yl | |
| 3.024 | 3-F-Ph | H | O | H | Bindung | 1-Methyl-1H-pyrazol-4-yl | |
| 3.025 | Ph | H | O | H | Bindung | 1-Methyl-1H-pyrazol-4-yl | |
| 3.026 | 3,5-Cl₂-Ph | H | O | H | Bindung | 1-Propyl-1H-1,2,4-triazol-3-yl | |
| 3.027 | 3,5-F₂-Ph | H | O | H | Bindung | 1-Propyl-1H-1,2,4-triazol-3-yl | |
| 3.028 | 3,5-Cl₂-Ph | H | O | H | Bindung | 2-(2-Ethoxy-2-oxoethyl)phenyl | |
| 3.029 | 3,5-Cl₂-Ph | H | O | H | Bindung | 2-(Methoxycarbonyl)phenyl | |
| 3.030 | 3,5-Cl₂-Ph | H | O | H | Bindung | 2-(Methylcarbamoyl)phenyl | |
| 3.031 | 3,5-Cl₂-Ph | H | O | H | Bindung | 2,4-Dichlor-6-(methylcarbamoyl)phenyl | |
| 3.032 | 3,5-Cl₂-Ph | H | O | H | Bindung | 2,4-Difluorphenyl | |
| 3.033 | 3,5-Cl₂-Ph | H | O | H | Bindung | 2-Carboxyphenyl | |
| 3.034 | 3,5-Cl₂-Ph | H | O | H | Bindung | 2-Chlorpyridin-3-yl | |
| 3.035 | 3,5-Cl₂-Ph | H | O | H | Bindung | 3-(2-Methoxy-2-oxoethyl)phenyl | |
| 3.036 | 3,5-Cl₂-Ph | H | O | H | Bindung | 3-(Carboxymethyl)phenyl | |
| 3.037 | 3,5-Cl₂-Ph | H | O | H | Bindung | 3-(Ethoxycarbonyl)-1H-1,2,4-triazol-5-yl | |
| 3.038 | 3,5-F₂-Ph | H | O | H | Bindung | 3-(Ethoxycarbonyl)-1H-1,2,4-triazol-5-yl | |
| 3.039 | 3,5-Cl₂-Ph | H | O | H | Bindung | 3-(Methoxycarbonyl)phenyl | |
| 3.040 | 3-F-Ph | H | O | H | Bindung | 3-(Methoxycarbonyl)phenyl | |
| 3.041 | 3-F-Ph | H | O | H | Bindung | 3-(Methylcarbamoyl)phenyl | |
| 3.042 | 3,5-Cl₂-Ph | H | O | H | Bindung | 3,5-Dichlorphenyl | |
| 3.043 | 3,5-Cl₂-Ph | H | O | H | Bindung | 3,5-Dimethoxyphenyl | |
| 3.044 | Ph | H | O | H | Bindung | 3,5-Dimethyl-1H-pyrazol-4-yl | |
| 3.045 | 3,5-Cl₂-Ph | H | O | H | Bindung | 3,5-Dimethyl-1H-pyrazol-4-yl | |
| 3.046 | 3-F-Ph | H | O | H | Bindung | 3,5-Dimethyl-1H-pyrazol-4-yl | |
| 3.047 | 3,5-Cl₂-Ph | H | O | H | Bindung | 3-[2-(Methylamino)-2-oxoethyl]phenyl | |
| 3.048 | 3,5-Cl₂-Ph | H | O | H | Bindung | 3-Carboxyphenyl | |
| 3.049 | 3-F-Ph | H | O | H | Bindung | 3-Carboxyphenyl | |
| 3.050 | 3,5-Cl₂-Ph | H | O | H | Bindung | 3-Chlorpyridin-2-yl | |
| 3.051 | 3,5-Cl₂-Ph | H | O | H | Bindung | 3-Cyclopropyl-1,2,4-thiadiazol-5-yl | |
| 3.052 | 3,5-Cl₂-Ph | H | O | H | Bindung | 3-Methyl-1,2-oxazol-5-yl | |
| 3.053 | 3,5-Cl₂-Ph | H | O | H | Bindung | 3-Methyl-1-propyl-1H-pyrazol-4-yl | |
| 3.054 | 3,5-F₂-Ph | H | O | H | Bindung | 3-Methyl-1-propyl-1H-pyrazol-4-yl | |
| 3.055 | 3-F-Ph | H | O | H | Bindung | 3-Methyl-1-propyl-1 H-pyrazol-4-yl | |
| 3.056 | Ph | H | O | H | Bindung | 3-Methyl-1-propyl-1 H-pyrazol-4-yl | |
| 3.057 | 3,5-Cl₂-Ph | H | O | H | Bindung | 4-(2-Ethoxy-2-oxoethyl)phenyl | |
| 3.058 | 3,5-Cl₂-Ph | H | O | H | Bindung | 4-(Carboxymethyl)phenyl | |
| 3.059 | 3,5-F₂-Ph | H | O | H | Bindung | 4-(Methoxycarbonyl)-3-thienyl | |
| 3.060 | 3,5-Cl₂-Ph | H | O | H | Bindung | 4-(Methoxycarbonyl)phenyl | |
| 3.061 | 3,5-Cl₂-Ph | H | O | H | Bindung | 4,5-Dimethyl-4H-1,2,4-triazol-3-yl | |
| 3.062 | 3,5-F₂-Ph | H | O | H | Bindung | 4,5-Dimethyl-4H-1,2,4-triazol-3-yl | |
| 3.063 | 3,5-Cl₂-Ph | H | O | H | Bindung | 4,6-Dimethoxypyrimidin-2-yl | |
| 3.064 | 3,5-Cl₂-Ph | H | O | H | Bindung | 4-Carboxyphenyl | |
| 3.065 | 3,5-Cl₂-Ph | H | O | H | Bindung | 4-Chlor-2-(methoxycarbonyl)-6-methylphenyl | |
| 3.066 | 3,5-Cl₂-Ph | H | O | H | Bindung | 4-Chlor-2-methyl-6-(methylcarbamoyl)phenyl | |
| 3.067 | 3,5-Cl₂-Ph | H | O | H | Bindung | 4-Chlorphenyl | |
| 3.068 | 3,5-Cl₂-Ph | H | O | H | Bindung | 4-Chlorpyridin-2-yl | |
| 3.069 | 3,5-F₂-Ph | H | O | H | Bindung | 4-Cyano-1-ethyl-1H-pyrazol-3-yl | |
| 3.070 | 3,5-Cl₂-Ph | H | O | H | Bindung | 4-Cyano-1H-pyrazol-3-yl | |
| 3.071 | Ph | H | O | H | Bindung | 4-Cyano-1H-pyrazol-3-yl | |
| 3.072 | 3-F-Ph | H | O | H | Bindung | 4-Cyano-1H-pyrazol-3-yl | |
| 3.073 | 3,5-Cl₂-Ph | H | O | H | Bindung | 4-Ethylpyridin-2-yl | |
| 3.074 | 3,5-F₂-Ph | H | O | H | Bindung | 4-Formyl-3-(1-methylcyclopropyl)-1,2-oxazol-5-yl | |
| 3.075 | 3,5-Cl₂-Ph | H | O | H | Bindung | 4-Methoxypyridin-2-yl | |
| 3.076 | 3,5-Cl₂-Ph | H | O | H | Bindung | 4-Methyl-1,2,5-oxad iazol-3-yl | |
| 3.077 | 3,5-F₂-Ph | H | O | H | Bindung | 4-Methyl-1,3-thiazol-2-yl | |
| 3.078 | 3,5-Cl₂-Ph | H | O | H | Bindung | 4-Methyl-1,3-thiazol-2-yl | |
| 3.079 | Ph | H | O | H | Bindung | 4-Methylpyridin-2-yl | |
| 3.080 | 2,4-Cl₂-Ph | H | O | H | Bindung | 4-Methylpyridin-2-yl | |
| 3.081 | 3,5-Cl₂-Ph | H | O | H | Bindung | 4-Methylpyridin-2-yl | |
| 3.082 | 3,5-Cl₂-Ph | H | O | H | Bindung | 4-Pentylpyridin-2-yl | |
| 3.083 | 2,4-Cl₂-Ph | H | O | H | Bindung | 4-Pentylpyridin-2-yl | |
| 3.084 | 3,5-Cl₂-Ph | H | O | H | Bindung | 5-(Methoxycarbonyl)-1,3-thiazol-4-yl | |
| 3.085 | 3,5-F₂-Ph | H | O | H | Bindung | 5-(Methoxycarbonyl)-1,3-thiazol-4-yl | |
| 3.086 | 3,5-Cl₂-Ph | H | O | H | Bindung | 5-Chlor-4-methylpyridin-2-yl | |
| 3.087 | 3,5-Cl₂-Ph | H | O | H | Bindung | 5-Fluor-4-methylpyridin-2-yl | |
| 3.088 | 3,5-Cl₂-Ph | H | O | H | Bindung | 5-Methoxy-1-methyl-1H-1,2,4-triazol-3-yl | |
| 3.089 | 3,5-F₂-Ph | H | O | H | Bindung | 5-Methoxy-1-methyl-1H-1,2,4-triazol-3-yl | |
| 3.090 | Ph | H | O | H | Bindung | 5-Methylpyridin-2-yl | |
| 3.091 | 3,5-Cl₂-Ph | H | O | H | Bindung | 6-Chlorpyridin-2-yl | |
| 3.092 | 3,5-Cl₂-Ph | H | O | H | Bindung | 6-Chlorpyridin-3-yl | |
| 3.093 | 3,5-Cl₂-Ph | H | O | H | Bindung | Pyridin-2-yl | |
| 3.094 | 3,5-Cl₂-Ph | H | O | H | Bindung | Pyridin-4-yl | |
| 3.095 | 3,5-F₂-Ph | H | O | H | CH₂ | 1-(2,2,2-Trifluorethyl)-1H-pyrazol-4-yl | |
| 3.096 | 3,5-F₂-Ph | H | O | H | CH₂ | 1-(2-Chlorethyl)-1H-pyrazol-4-yl | |
| 3.097 | 3,5-Cl₂-Ph | H | O | H | CH₂ | 1-(2-Methoxy-2-oxoethyl)-1H-pyrazol-4-yl | |
| 3.098 | 3,5-F₂-Ph | H | O | H | CH₂ | 1-(3-Carboxypropyl)-1H-pyrazol-4-yl | |
| 3.099 | 3,5-F₂-Ph | H | O | H | CH₂ | 1-(3-Methoxy-3-oxopropyl)-1H-pyrazol-4-yl | |
| 3.100 | 3,5-F₂-Ph | H | O | H | CH₂ | 1-(4-Methoxy-4-oxobutyl)-1H-pyrazol-4-yl | |
| 3.101 | 3,5-Cl₂-Ph | H | O | H | CH₂ | 1-(Cyanomethyl)-1H-pyrazol-4-yl | |
| 3.102 | 3,5-F₂-Ph | H | O | H | CH₂ | 1-(Cyanomethyl)-1H-pyrazol-4-yl | |
| 3.103 | 3-F-Ph | H | O | H | CH₂ | 1-(Cyanomethyl)-1H-pyrazol-4-yl | |
| 3.104 | 3,5-(CF₃)₂-Ph | H | O | H | CH₂ | 1,3-Dimethyl-1H-pyrazol-4-yl | |
| 3.105 | 3,5-Cl₂-Ph | H | O | H | CH₂ | 1,3-Dimethyl-1H-pyrazol-4-yl | |
| 3.106 | 3,5-F₂-Ph | H | O | H | CH₂ | 1,3-Dimethyl-1H-pyrazol-4-yl | |
| 3.107 | 3-Cl-Ph | H | O | H | CH₂ | 1,3-Dimethyl-1H-pyrazol-4-yl | |
| 3.108 | 3-F-Ph | H | O | H | CH₂ | 1,3-Dimethyl-1H-pyrazol-4-yl | |
| 3.109 | 3-Me-Ph | H | O | H | CH₂ | 1,3-Dimethyl-1H-pyrazol-4-yl | |
| 3.110 | Ph | H | O | H | CH₂ | 1,3-Dimethyl-1H-pyrazol-4-yl | |
| 3.111 | 3,5-(CF₃)₂-Ph | H | O | H | CH₂ | 1,3-Dimethyl-1H-pyrazol-5-yl | |
| 3.112 | 3,5-Cl₂-Ph | H | O | H | CH₂ | 1,3-Dimethyl-1H-pyrazol-5-yl | |
| 3.113 | 3,5-F₂-Ph | H | O | H | CH₂ | 1,3-Dimethyl-1H-pyrazol-5-yl | |
| 3.114 | Ph | H | O | H | CH₂ | 1,3-Dimethyl-1H-pyrazol-5-yl | |
| 3.115 | 3,5-Cl₂-Ph | H | O | H | CH₂ | 1,3-Oxazol-4-yl | |
| 3.116 | 3,5-F₂-Ph | H | O | H | CH₂ | 1,3-Oxazol-4-yl | |
| 3.117 | 3-F-Ph | H | O | H | CH₂ | 1,3-Oxazol-4-yl | |
| 3.118 | 3,5-Cl₂-Ph | H | O | H | CH₂ | 1,3-Thiazol-2-yl | |
| 3.119 | 3,5-F₂-Ph | H | O | H | CH₂ | 1,3-Thiazol-2-yl | |
| 3.120 | 3,5-Cl₂-Ph | H | O | H | CH₂ | 1,4-Dimethyl-5-oxo-4,5-dihydro-1H-1,2,4-triazol-3-yl | |
| 3.121 | 3,5-F₂-Ph | H | O | H | CH₂ | 1,5-Dimethyl-1H-pyrazol-3-yl | |
| 3.122 | 3,5-Cl₂-Ph | H | O | H | CH₂ | 1,5-Dimethyl-1H-pyrazol-4-yl | |
| 3.123 | 3,5-F₂-Ph | H | O | H | CH₂ | 1,5-Dimethyl-1H-pyrazol-4-yl | |
| 3.124 | 3-F-Ph | H | O | H | CH₂ | 1,5-Dimethyl-1H-pyrazol-4-yl | |
| 3.125 | Ph | H | O | H | CH₂ | 1,5-Dimethyl-1H-pyrazol-4-yl | |
| 3.126 | 3,5-F₂-Ph | H | O | H | CH₂ | 1-Acetyl-1 H-pyrazol-4-yl | |
| 3.127 | 3,5-F₂-Ph | H | O | H | CH₂ | 1-Butyl-3-methyl-1H-pyrazol-4-yl | |
| 3.128 | 3,5-F₂-Ph | H | O | H | CH₂ | 1-Butyl-5-methyl-1H-pyrazol-4-yl | |
| 3.129 | 3,5-Cl₂-Ph | H | O | H | CH₂ | 1-Cyclopropyl-1H-pyrazol-4-yl | |
| 3.130 | 3,5-F₂-Ph | H | O | H | CH₂ | 1-Cyclopropyl-1H-pyrazol-4-yl | |
| 3.131 | 3-F-Ph | H | O | H | CH₂ | 1-Cyclopropyl-1H-pyrazol-4-yl | |
| 3.132 | Ph | H | O | H | CH₂ | 1-Cyclopropyl-1H-pyrazol-4-yl | |
| 3.133 | 3,5-Cl₂-4-MeO-Ph | H | O | H | CH₂ | 1-Ethyl-1H-pyrazol-4-yl | |
| 3.134 | 3,5-Cl₂-Ph | H | O | H | CH₂ | 1-Ethyl-1H-pyrazol-4-yl | |
| 3.135 | 3,5-F₂-Ph | H | O | H | CH₂ | 1-Ethyl-1H-pyrazol-4-yl | |
| 3.136 | 3,5-(MeO)₂-Ph | H | O | H | CH₂ | 1-Ethyl-1H-pyrazol-4-yl | |
| 3.137 | 3-Br-5-CF₃-Ph | H | O | H | CH₂ | 1-Ethyl-1H-pyrazol-4-yl | |
| 3.138 | 3-Cl-5-Et-Ph | H | O | H | CH₂ | 1-Ethyl-1H-pyrazol-4-yl | |
| 3.139 | 3-F-5-MeO-Ph | H | O | H | CH₂ | 1-Ethyl-1H-pyrazol-4-yl | |
| 3.140 | 3-F-Ph | H | O | H | CH₂ | 1-Ethyl-1H-pyrazol-4-yl | |
| 3.141 | 3-Me-5-CF₃O-Ph | H | O | H | CH₂ | 1-Ethyl-1H-pyrazol-4-yl | |
| 3.142 | 3-Me-Ph | H | O | H | CH₂ | 1-Ethyl-1H-pyrazol-4-yl | |
| 3.143 | Ph | H | O | H | CH₂ | 1-Ethyl-1H-pyrazol-4-yl | |
| 3.144 | 3,5-Cl₂-Ph | H | O | H | CH₂ | 1-Ethyl-1H-pyrazol-5-yl | |
| 3.145 | 3,5-F₂-Ph | H | O | H | CH₂ | 1-Ethyl-1H-pyrazol-5-yl | |
| 3.146 | Ph | H | O | H | CH₂ | 1-Ethyl-1H-pyrazol-5-yl | |
| 3.147 | 2,3,4-F₃-Ph | H | O | H | CH₂ | 1-Ethyl-3-methyl-1H-pyrazol-4-yl | |
| 3.148 | 2,3,5-F₃-Ph | H | O | H | CH₂ | 1-Ethyl-3-methyl-1H-pyrazol-4-yl | |
| 3.149 | 2,3-F₂-Ph | H | O | H | CH₂ | 1-Ethyl-3-methyl-1H-pyrazol-4-yl | |
| 3.150 | 2,5-Cl₂-Ph | H | O | H | CH₂ | 1-Ethyl-3-methyl-1H-pyrazol-4-yl | |
| 3.151 | 2,5-F₂-Ph | H | O | H | CH₂ | 1-Ethyl-3-methyl-1H-pyrazol-4-yl | |
| 3.152 | 2,5-Me₂-Ph | H | O | H | CH₂ | 1-Ethyl-3-methyl-1H-pyrazol-4-yl | |
| 3.153 | 2-F-3-Me-Ph | H | O | H | CH₂ | 1-Ethyl-3-methyl-1H-pyrazol-4-yl | |
| 3.154 | 2-F-Ph | H | O | H | CH₂ | 1-Ethyl-3-methyl-1H-pyrazol-4-yl | |
| 3.155 | 3-CF₃O-Ph | H | O | H | CH₂ | 1-Ethyl-3-methyl-1H-pyrazol-4-yl | |
| 3.156 | 3,4,5-F₃-Ph | H | O | H | CH₂ | 1-Ethyl-3-methyl-1H-pyrazol-4-yl | |
| 3.157 | 3,4-F₂-Ph | H | O | H | CH₂ | 1-Ethyl-3-methyl-1H-pyrazol-4-yl | |
| 3.158 | 3,5-Br₂-Ph | H | O | H | CH₂ | 1-Ethyl-3-methyl-1H-pyrazol-4-yl | |
| 3.159 | 3,5-Cl₂-4-MeO-Ph | H | O | H | CH₂ | 1-Ethyl-3-methyl-1H-pyrazol-4-yl | |
| 3.160 | 3,5-Cl₂-Ph | CH 3 | O | H | CH₂ | 1-Ethyl-3-methyl-1H-pyrazol-4-yl | |
| 3.161 | 3,5-Cl₂-Ph | H | O | H | CH₂ | 1-Ethyl-3-methyl-1H-pyrazol-4-yl | [CDCl₃] 1.42 (t, 3H); 2.20 (s, 3H); 3.74 (d, 1H); 3.98 (d, 1H); 4.07 (q, 2H); 4.22 (dd, 1H); 4.49 (dd, 1H); 6.81 (m, 1H); 7.28 (s, 1H); 7.47 (m, 1H); 7.54 (m, 2H). |
| 3.162 | 3,5-Et₂-Ph | H | O | H | CH₂ | 1-Ethyl-3-methyl-1H-pyrazol-4-yl | |
| 3.163 | 3,5-F₂-Ph | H | O | H | CH₂ | 1-Ethyl-3-methyl-1H-pyrazol-4-yl | |
| 3.164 | 3,5-F₂-Ph | H | S | H | CH₂ | 1-Ethyl-3-methyl-1H-pyrazol-4-yl | |
| 3.165 | 3,5-Me₂-Ph | H | O | H | CH₂ | 1-Ethyl-3-methyl-1H-pyrazol-4-yl | |
| 3.166 | 3-CF₃S-Ph | H | O | H | CH₂ | 1-Ethyl-3-methyl-1H-pyrazol-4-yl | |
| 3.167 | 3-Br-5-CF₃-Ph | H | O | H | CH₂ | 1-Ethyl-3-methyl-1H-pyrazol-4-yl | |
| 3.168 | 3-Br-5-Cl-Ph | H | O | H | CH₂ | 1-Ethyl-3-methyl-1H-pyrazol-4-yl | |
| 3.169 | 3-Br-5-F-Ph | H | O | H | CH₂ | 1-Ethyl-3-methyl-1H-pyrazol-4-yl | |
| 3.170 | 3-Cl-4-F-Ph | H | O | H | CH₂ | 1-Ethyl-3-methyl-1H-pyrazol-4-yl | [CDCl₃] 1.44 (t, 3H); 1.70 (s, 3H); 3.77 (d, 1H); 4.00 (d, 1H); 4.06 (q, 2H); 4.23 (dd, 1H), 4.40 (dd, 1H); 6.86 (t br, 1H); 7.23 (m, 1H); 7.52 (m, 1H); 7.74 (m, 1H). |
| 3.171 | 3-Cl-5-CF₃-Ph | H | O | H | CH₂ | 1-Ethyl-3-methyl-1H-pyrazol-4-yl | |
| 3.172 | 3-Cl-5-CN-Ph | H | O | H | CH₂ | 1-Ethyl-3-methyl-1H-pyrazol-4-yl | |
| 3.173 | 3-Cl-5-Et-Ph | H | O | H | CH₂ | 1-Ethyl-3-methyl-1H-pyrazol-4-yl | |
| 3.174 | 3-Cl-5-F-Ph | H | O | H | CH₂ | 1-Ethyl-3-methyl-1H-pyrazol-4-yl | |
| 3.175 | 3-Cl-5-MeO-Ph | H | O | H | CH₂ | 1-Ethyl-3-methyl-1H-pyrazol-4-yl | |
| 3.176 | 3-Cl-5-Me-Ph | H | O | H | CH₂ | 1-Ethyl-3-methyl-1H-pyrazol-4-yl | |
| 3.177 | 3-Cl-5-CF₃O-Ph | H | O | H | CH₂ | 1-Ethyl-3-methyl-1H-pyrazol-4-yl | |
| 3.178 | 3-Cl-Ph | H | O | H | CH₂ | 1-Ethyl-3-methyl-1H-pyrazol-4-yl | |
| 3.179 | 3-CN-5-F-Ph | H | O | H | CH₂ | 1-Ethyl-3-methyl-1H-pyrazol-4-yl | |
| 3.180 | 3-CN-Ph | H | O | H | CH₂ | 1-Ethyl-3-methyl-1H-pyrazol-4-yl | |
| 3.181 | 3-cPr-5-F-Ph | H | O | H | CH₂ | 1-Ethyl-3-methyl-1H-pyrazol-4-yl | |
| 3.182 | 3-EtO-Ph | H | O | H | CH₂ | 1-Ethyl-3-methyl-1H-pyrazol-4-yl | |
| 3.183 | 3-Et-5-F-Ph | H | O | H | CH₂ | 1-Ethyl-3-methyl-1H-pyrazol-4-yl | |
| 3.184 | 3-F-5-CF₃-Ph | H | O | H | CH₂ | 1-Ethyl-3-methyl-1H-pyrazol-4-yl | |
| 3.185 | 3-F-5-MeO-Ph | H | O | H | CH₂ | 1-Ethyl-3-methyl-1H-pyrazol-4-yl | |
| 3.186 | 3-F-5-Me-Ph | H | O | H | CH₂ | 1-Ethyl-3-methyl-1H-pyrazol-4-yl | |
| 3.187 | 3-F-Ph | H | O | H | CH₂ | 1-Ethyl-3-methyl-1H-pyrazol-4-yl | |
| 3.188 | 3-MeO-Ph | H | O | H | CH₂ | 1-Ethyl-3-methyl-1H-pyrazol-4-yl | |
| 3.189 | 3-Me-Ph | H | O | H | CH₂ | 1-Ethyl-3-methyl-1H-pyrazol-4-yl | |
| 3.190 | Ph | H | O | H | CH₂ | 1-Ethyl-3-methyl-1H-pyrazol-4-yl | |
| 3.191 | 3,5-Cl₂-Ph | H | O | H | CH₂ | 1-Ethyl-3-methyl-1H-pyrazol-5-yl | |
| 3.192 | 3,5-F₂-Ph | H | O | H | CH₂ | 1-Ethyl-3-methyl-1H-pyrazol-5-yl | |
| 3.193 | 3-F-Ph | H | O | H | CH₂ | 1-Ethyl-3-methyl-1H-pyrazol-5-yl | |
| 3.194 | 3,5-F₂-Ph | H | O | H | CH₂ | 1H-1,2,3-Triazol-5-yl | |
| 3.195 | 3,5-Cl₂-Ph | H | O | H | CH₂ | 1H-Pyrazol-4-yl | |
| 3.196 | 3,5-F₂-Ph | H | O | H | CH₂ | 1H-Pyrazol-4-yl | |
| 3.197 | 3-F-Ph | H | O | H | CH₂ | 1H-Pyrazol-4-yl | |
| 3.198 | 3,5-Cl₂-Ph | H | O | H | CH₂ | 1-Isobutyl-1H-pyrazol-4-yl | |
| 3.199 | 3,5-F₂-Ph | H | O | H | CH₂ | 1-Isobutyl-1H-pyrazol-4-yl | |
| 3.200 | 3-F-Ph | H | O | H | CH₂ | 1-Isobutyl-1H-pyrazol-4-yl | |
| 3.201 | Ph | H | O | H | CH₂ | 1-Isobutyl-1H-pyrazol-4-yl | |
| 3.202 | 3,5-Cl₂-Ph | H | O | H | CH₂ | 1-Isopropyl-1H-pyrazol-4-yl | |
| 3.203 | 3,5-F₂-Ph | H | O | H | CH₂ | 1-Isopropyl-1H-pyrazol-4-yl | |
| 3.204 | 3-F-Ph | H | O | H | CH₂ | 1-Isopropyl-1H-pyrazol-4-yl | |
| 3.205 | Ph | H | O | H | CH₂ | 1-Isopropyl-1H-pyrazol-4-yl | |
| 3.206 | 3,5-(CF₃)₂-Ph | H | O | H | CH₂ | 1-Methyl-1H-pyrazol-4-yl | |
| 3.207 | 3,5-Cl₂-Ph | H | O | H | CH₂ | 1-Methyl-1H-pyrazol-4-yl | |
| 3.208 | 3,5-F₂-Ph | H | O | H | CH₂ | 1-Methyl-1H-pyrazol-4-yl | |
| 3.209 | 3-F-5-MeO-Ph | H | O | H | CH₂ | 1-Methyl-1H-pyrazol-4-yl | |
| 3.210 | 3-F-Ph | H | O | H | CH₂ | 1-Methyl-1H-pyrazol-4-yl | |
| 3.211 | 3-Me-Ph | H | O | H | CH₂ | 1-Methyl-1H-pyrazol-4-yl | |
| 3.212 | Ph | H | O | H | CH₂ | 1-Methyl-1H-pyrazol-4-yl | |
| 3.213 | 3,5-Cl₂-Ph | H | O | H | CH₂ | 1-Methyl-1H-pyrazol-5-yl | |
| 3.214 | 3,5-F₂-Ph | H | O | H | CH₂ | 1-Methyl-1H-pyrazol-5-yl | |
| 3.215 | 3-F-Ph | H | O | H | CH₂ | 1-Methyl-1H-pyrazol-5-yl | |
| 3.216 | Ph | H | O | H | CH₂ | 1-Methyl-1H-pyrazol-5-yl | |
| 3.217 | 3,5-Cl₂-Ph | H | O | H | CH₂ | 1-Methyl-3-(trifluormethyl)-1H-pyrazol-4-yl | D1 |
| 3.218 | 3,5-Cl₂-Ph | H | O | H | CH₂ | 1-Methyl-3-(trifluormethyl)-1H-pyrazol-4-yl | D2 |
| 3.219 | 3,5-F₂-Ph | H | O | H | CH₂ | 1-Methyl-5-(trifluormethyl)-1H-pyrazol-4-yl | |
| 3.220 | 3,5-Cl₂-Ph | H | O | H | CH₂ | 1-Propyl-1H-pyrazol-3-yl | |
| 3.221 | 3,5-F₂-Ph | H | O | H | CH₂ | 1-Propyl-1H-pyrazol-3-yl | |
| 3.222 | 3,5-Cl₂-Ph | H | O | H | CH₂ | 1-Propyl-1H-pyrazol-4-yl | |
| 3.223 | 3,5-F₂-Ph | H | O | H | CH₂ | 1-Propyl-1H-pyrazol-4-yl | |
| 3.224 | 3-F-Ph | H | O | H | CH₂ | 1-Propyl-1H-pyrazol-4-yl | |
| 3.225 | Ph | H | O | H | CH₂ | 1-Propyl-1H-pyrazol-4-yl | |
| 3.226 | 3,5-Cl₂-Ph | H | O | H | CH₂ | 1-Tert-butyl-1H-pyrazol-4-yl | |
| 3.227 | 3,5-F₂-Ph | H | O | H | CH₂ | 1-Tert-butyl-1H-pyrazol-4-yl | |
| 3.228 | Ph | H | O | H | CH₂ | 1-Tert-butyl-1H-pyrazol-4-yl | |
| 3.229 | 3,5-F₂-Ph | H | O | H | CH₂ | 2-Chlorpyridin-4-yl | |
| 3.230 | 3-F-Ph | H | O | H | CH₂ | 2-Chlorpyridin-4-yl | |
| 3.231 | 3,5-Cl₂-Ph | H | O | H | CH₂ | 2-Methyl-1,3-thiazol-4-yl | |
| 3.232 | 3,5-F₂-Ph | H | O | H | CH₂ | 2-Methyl-1,3-thiazol-4-yl | |
| 3.233 | 3,5-Cl₂-Ph | H | O | H | CH₂ | 3-(Cyclopropylcarbamo yl)-1,2-oxazol-5-yl | |
| 3.234 | 3,5-Cl₂-Ph | H | O | H | CH₂ | 3-(Ethoxycarbonyl)-1,2-oxazol-5-yl | |
| 3.235 | 3,5-F₂-Ph | H | O | H | CH₂ | 3-(Methoxycarbonyl)phenyl | |
| 3.236 | 3,5-F₂-Ph | H | O | H | CH₂ | 3,5-Difluorpyridin-2-yl | |
| 3.237 | 3,5-Cl₂-Ph | H | O | H | CH₂ | 3-Carboxy-1,2-oxazol-5-yl | |
| 3.238 | 3,5-Cl₂-Ph | H | O | H | CH₂ | 3-Chlor-1-ethyl-1H-pyrazol-4-yl | |
| 3.239 | 3,5-F₂-Ph | H | O | H | CH₂ | 3-Chlor-1-ethyl-1H-pyrazol-4-yl | |
| 3.240 | 3-F-Ph | H | O | H | CH₂ | 3-Chlor-1-ethyl-1H-pyrazol-4-yl | |
| 3.241 | Ph | H | O | H | CH₂ | 3-Chlor-1-ethyl-1H-pyrazol-4-yl | |
| 3.242 | 3,5-Cl₂-Ph | H | O | H | CH₂ | 3-Chlor-1-isopropyl-1 H-pyrazol-4-yl | |
| 3.243 | 3,5-F₂-Ph | H | O | H | CH₂ | 3-Chlor-1-isopropyl-1 H-pyrazol-4-yl | |
| 3.244 | 3,5-F₂-Ph | H | S | H | CH₂ | 3-Chlor-1-isopropyl-1 H-pyrazol-4-yl | |
| 3.245 | 3-F-Ph | H | O | H | CH₂ | 3-Chlor-1-isopropyl-1 H-pyrazol-4-yl | |
| 3.246 | 3,5-Cl₂-Ph | H | O | H | CH₂ | 3-Chlor-1-methyl-1H-pyrazol-4-yl | |
| 3.247 | 3,5-F₂-Ph | H | O | H | CH₂ | 3-Chlor-1-methyl-1H-pyrazol-4-yl | |
| 3.248 | 3,5-F₂-Ph | H | O | H | CH₂ | 3-Chlorpyridin-4-yl | |
| 3.249 | 3-F-Ph | H | O | H | CH₂ | 3-Chlorpyridin-4-yl | |
| 3.250 | 3,5-Cl₂-Ph | H | O | H | CH₂ | 3-Cyclopropyl-1,2,4-oxad iazol-5-yl | |
| 3.251 | 3,5-F₂-Ph | H | O | H | CH₂ | 3-Cyclopropyl-1,2,4-oxad iazol-5-yl | |
| 3.252 | 3,5-F₂-Ph | H | O | H | CH₂ | 3-Furyl | |
| 3.253 | 3,5-F₂-Ph | H | O | H | CH₂ | 3-Methyl-1H-pyrazol-4-yl | |
| 3.254 | 3-CF₃O-Ph | H | O | H | CH₂ | 3-Methyl-1-propyl-1H-pyrazol-4-yl | |
| 3.255 | 3,5-Cl₂-Ph | H | O | H | CH₂ | 3-Methyl-1-propyl-1H-pyrazol-4-yl | |
| 3.256 | 3,5-F₂-Ph | H | O | H | CH₂ | 3-Methyl-1-propyl-1H-pyrazol-4-yl | |
| 3.257 | 3-CI-4-F-Ph | H | O | H | CH₂ | 3-Methyl-1-propyl-1H-pyrazol-4-yl | [CDCl₃] 0.89 (t, 3H); 1.83 (q, 2H); 2.19 (s, 3H); 3.77 (d, 1H); 3.96 (m, 3H); 3.97 (d, 1H); 4.23 (dd, 1H), 4.40 (dd, 1H); 6.88 (m br, 1H); 7.21 (m, 1H); 7.52 (m, 1H); 7.73 (m, 1H) |
| 3.258 | 3-Cl-5-CF₃O-Ph | H | O | H | CH₂ | 3-Methyl-1-propyl-1H-pyrazol-4-yl | |
| 3.259 | 3-Cl-5-F-Ph | H | O | H | CH₂ | 3-Methyl-1-propyl-1H-pyrazol-4-yl | |
| 3.260 | 3-Cl-5-Me-Ph | H | O | H | CH₂ | 3-Methyl-1-propyl-1H-pyrazol-4-yl | |
| 3.261 | 3-F-5-Me-Ph | H | O | H | CH₂ | 3-Methyl-1-propyl-1H-pyrazol-4-yl | |
| 3.262 | 3-F-Ph | H | O | H | CH₂ | 3-Methyl-1-propyl-1H-pyrazol-4-yl | |
| 3.263 | 3-Me-Ph | H | O | H | CH₂ | 3-Methyl-1-propyl-1H-pyrazol-4-yl | |
| 3.264 | 4-Cl-3,5-F₂-Ph | H | O | H | CH₂ | 3-Methyl-1-propyl-1H-pyrazol-4-yl | |
| 3.265 | Ph | H | O | H | CH₂ | 3-Methyl-1-propyl-1H-pyrazol-4-yl | |
| 3.266 | 3,5-Cl₂-Ph | H | O | H | CH₂ | 4-(Methoxycarbonyl)phenyl | |
| 3.267 | 3,5-Cl₂-Ph | H | O | H | CH₂ | 4-(Trifluormethyl)-1,3-thiazol-2-yl | |
| 3.268 | 3,5-F₂-Ph | H | O | H | CH₂ | 4-(Trifluormethyl)-1,3-thiazol-2-yl | |
| 3.269 | 3-F-Ph | H | O | H | CH₂ | 4-(Trifluormethyl)-1,3-thiazol-2-yl | |
| 3.270 | 3,5-Cl₂-Ph | H | O | H | CH₂ | 4-Chlor-1-methyl-1H-pyrazol-3-yl | |
| 3.271 | 3,5-F₂-Ph | H | O | H | CH₂ | 4-Chlor-1-methyl-1H-pyrazol-3-yl | |
| 3.272 | 3-F-Ph | H | O | H | CH₂ | 4-Chlor-1-methyl-1H-pyrazol-3-yl | |
| 3.273 | 3,5-F₂-Ph | H | O | H | CH₂ | 4-Chlor-1-methyl-1H-pyrazol-5-yl | |
| 3.274 | 3,5-Cl₂-Ph | H | O | H | CH₂ | 5-Chlor-1-methyl-1H-pyrazol-4-yl | |
| 3.275 | 3,5-F₂-Ph | H | O | H | CH₂ | 5-Chlor-1-methyl-1H-pyrazol-4-yl | |
| 3.276 | 3,5-F₂-Ph | H | S | H | CH₂ | 5-Chlor-1-methyl-1H-pyrazol-4-yl | |
| 3.277 | 3-F-Ph | H | O | H | CH₂ | 5-Chlor-1-methyl-1H-pyrazol-4-yl | |
| 3.278 | Ph | H | O | H | CH₂ | 5-Chlor-1-methyl-1H-pyrazol-4-yl | |
| 3.279 | 3,5-Cl₂-Ph | H | O | H | CH₂ | 5-Methoxy-1,3-dimethyl-1H-pyrazol-4-yl | |
| 3.280 | 3,5-F₂-Ph | H | O | H | CH₂ | 5-Methoxy-1,3-dimethyl-1H-pyrazol-4-yl | |
| 3.281 | 3-F-Ph | H | O | H | CH₂ | 5-Methoxy-1,3-dimethyl-1H-pyrazol-4-yl | |
| 3.282 | 3,5-Cl₂-Ph | H | O | H | CH₂ | 5-Methyl-1,2,4-oxadiazol-3-yl | |
| 3.283 | 3,5-F₂-Ph | H | O | H | CH₂ | 5-Methyl-1,2,4-oxadiazol-3-yl | |
| 3.284 | 3,5-Cl₂-Ph | H | O | H | CH₂ | 5-Methyl-1,2-oxazol-3-yl | |
| 3.285 | 3,5-Cl₂-Ph | H | O | H | CH₂ | 5-Methyl-1-propyl-1H-pyrazol-4-yl | |
| 3.286 | 3,5-F₂-Ph | H | O | H | CH₂ | 5-Methyl-1-propyl-1H-pyrazol-4-yl | |
| 3.287 | 3-F-Ph | H | O | H | CH₂ | 5-Methyl-1-propyl-1H-pyrazol-4-yl | |
| 3.288 | Ph | H | O | H | CH₂ | 5-Methyl-1-propyl-1H-pyrazol-4-yl | |
| 3.289 | 3,5-F₂-Ph | H | O | H | CH₂ | 6-Chlorpyridin-3-yl | |
| 3.290 | 3,5-F₂-Ph | H | O | H | CH₂ | Furan-2-yl | |
| 3.291 | 3,5-Cl₂-Ph | H | O | H | CH₂ | Pyridin-2-yl | |
| 3.292 | 3,5-F₂-Ph | H | O | H | CH₂ | Pyridin-2-yl | |
| 3.293 | 3,5-F₂-Ph | H | O | H | CH₂ | Pyridin-3-yl | |
| 3.294 | 3-F-Ph | H | O | H | CH₂ | Pyridin-3-yl | |
| 3.295 | 3,5-F₂-Ph | H | O | H | CH₂ | Pyridin-4-yl | |
| 3.296 | 3-F-Ph | H | O | H | CH₂ | Pyridin-4-yl | |
| 3.297 | 3,5-Cl₂-Ph | H | O | H | CH₂ | Pyrimidin-2-yl | |
| 3.298 | 3,5-F₂-Ph | H | O | H | CH₂CH₂ | 1-(2,2,2-Trifluorethyl)-1H-pyrazol-4-yl | |
| 3.299 | 3,5-F₂-Ph | H | O | H | CH₂CH₂ | 1-(2-Chlorethyl)-1H-pyrazol-4-yl | |
| 3.300 | 3,5-Cl₂-Ph | H | O | H | CH₂CH₂ | 1-(2-Methoxy-2-oxoethyl)-1H-pyrazol-4-yl | |
| 3.301 | 3,5-F₂-Ph | H | O | H | CH₂CH₂ | 1-(3-Carboxypropyl)-1H-pyrazol-4-yl | |
| 3.302 | 3,5-F₂-Ph | H | O | H | CH₂CH₂ | 1-(3-Methoxy-3-oxopropyl)-1H-pyrazol-4-yl | |
| 3.303 | 3,5-F₂-Ph | H | O | H | CH₂CH₂ | 1-(4-Methoxy-4-oxobutyl)-1H-pyrazol-4-yl | |
| 3.304 | 3,5-F₂-Ph | H | O | H | CH₂CH₂ | 1-(Cyanomethyl)-1H-pyrazol-4-yl | |
| 3.305 | 3,5-Cl₂-Ph | H | O | H | CH₂CH₂ | 1-(Cyanomethyl)-1H-pyrazol-4-yl | |
| 3.306 | 3-F-Ph | H | O | H | CH₂CH₂ | 1-(Cyanomethyl)-1H-pyrazol-4-yl | |
| 3.307 | 3,5-F₂-Ph | H | O | H | CH₂CH₂ | 1,3-Dimethyl-1H-pyrazol-4-yl | |
| 3.308 | 3-F-Ph | H | O | H | CH₂CH₂ | 1,3-Dimethyl-1H-pyrazol-4-yl | |
| 3.309 | 3-Me-Ph | H | O | H | CH₂CH₂ | 1,3-Dimethyl-1H-pyrazol-4-yl | |
| 3.310 | Ph | H | O | H | CH₂CH₂ | 1,3-Dimethyl-1H-pyrazol-4-yl | |
| 3.311 | 3-Cl-Ph | H | O | H | CH₂CH₂ | 1,3-Dimethyl-1H-pyrazol-4-yl | |
| 3.312 | 3,5-Cl₂-Ph | H | O | H | CH₂CH₂ | 1,3-Dimethyl-1H-pyrazol-4-yl | |
| 3.313 | 3,5-(CF₃)₂-Ph | H | O | H | CH₂CH₂ | 1,3-Dimethyl-1H-pyrazol-4-yl | |
| 3.314 | 3,5-F₂-Ph | H | O | H | CH₂CH₂ | 1,3-Dimethyl-1H-pyrazol-5-yl | |
| 3.315 | Ph | H | O | H | CH₂CH₂ | 1,3-Dimethyl-1H-pyrazol-5-yl | |
| 3.316 | 3,5-Cl₂-Ph | H | O | H | CH₂CH₂ | 1,3-Dimethyl-1H-pyrazol-5-yl | |
| 3.317 | 3,5-(CF₃)₂-Ph | H | O | H | CH₂CH₂ | 1,3-Dimethyl-1H-pyrazol-5-yl | |
| 3.318 | 3,5-F₂-Ph | H | O | H | CH₂CH₂ | 1,3-Oxazol-4-yl | |
| 3.319 | 3,5-Cl₂-Ph | H | O | H | CH₂CH₂ | 1,3-Oxazol-4-yl | |
| 3.320 | 3-F-Ph | H | O | H | CH₂CH₂ | 1,3-Oxazol-4-yl | |
| 3.321 | 3,5-Cl₂-Ph | H | O | H | CH₂CH₂ | 1,3-Thiazol-2-yl | |
| 3.322 | 3,5-F₂-Ph | H | O | H | CH₂CH₂ | 1,3-Thiazol-2-yl | |
| 3.323 | 3,5-Cl₂-Ph | H | O | H | CH₂CH₂ | 1,4-Dimethyl-5-oxo-4,5-dihydro-1H-1,2,4-triazol-3-yl | |
| 3.324 | 3,5-F₂-Ph | H | O | H | CH₂CH₂ | 1,5-Dimethyl-1H-pyrazol-3-yl | |
| 3.325 | 3,5-F₂-Ph | H | O | H | CH₂CH₂ | 1,5-Dimethyl-1H-pyrazol-4-yl | |
| 3.326 | 3-F-Ph | H | O | H | CH₂CH₂ | 1,5-Dimethyl-1H-pyrazol-4-yl | |
| 3.327 | 3,5-Cl₂-Ph | H | O | H | CH₂CH₂ | 1,5-Dimethyl-1H-pyrazol-4-yl | |
| 3.328 | Ph | H | O | H | CH₂CH₂ | 1,5-Dimethyl-1H-pyrazol-4-yl | |
| 3.329 | 3,5-F₂-Ph | H | O | H | CH₂CH₂ | 1-Acetyl-1 H-pyrazol-4-yl | |
| 3.330 | 3,5-F₂-Ph | H | O | H | CH₂CH₂ | 1-Butyl-3-methyl-1H-pyrazol-4-yl | |
| 3.331 | 3,5-F₂-Ph | H | O | H | CH₂CH₂ | 1-Butyl-5-methyl-1H-pyrazol-4-yl | |
| 3.332 | 3,5-F₂-Ph | H | O | H | CH₂CH₂ | 1-Cyclopropyl-1H-pyrazol-4-yl | |
| 3.333 | Ph | H | O | H | CH₂CH₂ | 1-Cyclopropyl-1H-pyrazol-4-yl | |
| 3.334 | 3-F-Ph | H | O | H | CH₂CH₂ | 1-Cyclopropyl-1H-pyrazol-4-yl | |
| 3.335 | 3,5-Cl₂-Ph | H | O | H | CH₂CH₂ | 1-Cyclopropyl-1H-pyrazol-4-yl | |
| 3.336 | 3,5-Cl₂-Ph | H | O | H | CH₂CH₂ | 1-Ethyl-1H-pyrazol-4-yl | |
| 3.337 | 3-F-Ph | H | O | H | CH₂CH₂ | 1-Ethyl-1H-pyrazol-4-yl | |
| 3.338 | 3,5-F₂-Ph | H | O | H | CH₂CH₂ | 1-Ethyl-1H-pyrazol-4-yl | |
| 3.339 | 3-Br-5-CF₃-Ph | H | O | H | CH₂CH₂ | 1-Ethyl-1H-pyrazol-4-yl | |
| 3.340 | 3-Me-Ph | H | O | H | CH₂CH₂ | 1-Ethyl-1H-pyrazol-4-yl | |
| 3.341 | 3-F-5-MeO-Ph | H | O | H | CH₂CH₂ | 1-Ethyl-1H-pyrazol-4-yl | |
| 3.342 | 3-Cl-5-Et-Ph | H | O | H | CH₂CH₂ | 1-Ethyl-1H-pyrazol-4-yl | |
| 3.343 | 3,5-(MeO)₂-Ph | H | O | H | CH₂CH₂ | 1-Ethyl-1H-pyrazol-4-yl | |
| 3.344 | 3-Me-5-CF₃O-Ph | H | O | H | CH₂CH₂ | 1-Ethyl-1H-pyrazol-4-yl | |
| 3.345 | 3,5-Cl₂-4-MeO-Ph | H | O | H | CH₂CH₂ | 1-Ethyl-1H-pyrazol-4-yl | |
| 3.346 | Ph | H | O | H | CH₂CH₂ | 1-Ethyl-1H-pyrazol-4-yl | |
| 3.347 | 3,5-F₂-Ph | H | O | H | CH₂CH₂ | 1-Ethyl-1H-pyrazol-5-yl | |
| 3.348 | 3,5-Cl₂-Ph | H | O | H | CH₂CH₂ | 1-Ethyl-1H-pyrazol-5-yl | |
| 3.349 | Ph | H | O | H | CH₂CH₂ | 1-Ethyl-1H-pyrazol-5-yl | |
| 3.350 | 3,5-F₂-Ph | H | O | H | CH₂CH₂ | 1-Ethyl-3-methyl-1H-pyrazol-4-yl | |
| 3.351 | 3-Me-Ph | H | O | H | CH₂CH₂ | 1-Ethyl-3-methyl-1H-pyrazol-4-yl | |
| 3.352 | 3-Br-5-F-Ph | H | O | H | CH₂CH₂ | 1-Ethyl-3-methyl-1H-pyrazol-4-yl | |
| 3.353 | 3,4,5-F₃-Ph | H | O | H | CH₂CH₂ | 1-Ethyl-3-methyl-1H-pyrazol-4-yl | |
| 3.354 | 3,4-F₂-Ph | H | O | H | CH₂CH₂ | 1-Ethyl-3-methyl-1H-pyrazol-4-yl | |
| 3.355 | 3,5-F₂-Ph | H | S | H | CH₂CH₂ | 1-Ethyl-3-methyl-1H-pyrazol-4-yl | |
| 3.356 | 3-F-5-Me-Ph | H | O | H | CH₂CH₂ | 1-Ethyl-3-methyl-1H-pyrazol-4-yl | |
| 3.357 | 3-Cl-5-CN-Ph | H | O | H | CH₂CH₂ | 1-Ethyl-3-methyl-1H-pyrazol-4-yl | |
| 3.358 | 3-F-Ph | H | O | H | CH₂CH₂ | 1-Ethyl-3-methyl-1H-pyrazol-4-yl | |
| 3.359 | 3-cPr-5-F-Ph | H | O | H | CH₂CH₂ | 1-Ethyl-3-methyl-1H-pyrazol-4-yl | |
| 3.360 | 3-Cl-5-Me-Ph | H | O | H | CH₂CH₂ | 1-Ethyl-3-methyl-1H-pyrazol-4-yl | |
| 3.361 | 3-F-5-MeO-Ph | H | O | H | CH₂CH₂ | 1-Ethyl-3-methyl-1H-pyrazol-4-yl | |
| 3.362 | 2,5-Me₂-Ph | H | O | H | CH₂CH₂ | 1-Ethyl-3-methyl-1H-pyrazol-4-yl | |
| 3.363 | 3-CF₃O-Ph | H | O | H | CH₂CH₂ | 1-Ethyl-3-methyl-1H-pyrazol-4-yl | |
| 3.364 | 3-Cl-5-Et-Ph | H | O | H | CH₂CH₂ | 1-Ethyl-3-methyl-1H-pyrazol-4-yl | |
| 3.365 | 2,3,5-F₃-Ph | H | O | H | CH₂CH₂ | 1-Ethyl-3-methyl-1H-pyrazol-4-yl | |
| 3.366 | 3-Et-5-F-Ph | H | O | H | CH₂CH₂ | 1-Ethyl-3-methyl-1H-pyrazol-4-yl | |
| 3.367 | 3-F-5-CF₃-Ph | H | O | H | CH₂CH₂ | 1-Ethyl-3-methyl-1H-pyrazol-4-yl | |
| 3.368 | 3-Br-5-CF₃-Ph | H | O | H | CH₂CH₂ | 1-Ethyl-3-methyl-1H-pyrazol-4-yl | |
| 3.369 | 3-Cl-5-MeO-Ph | H | O | H | CH₂CH₂ | 1-Ethyl-3-methyl-1H-pyrazol-4-yl | |
| 3.370 | 3-Cl-4-F-Ph | H | O | H | CH₂CH₂ | 1-Ethyl-3-methyl-1H-pyrazol-4-yl | |
| 3.371 | 2,3-F₂-Ph | H | O | H | CH₂CH₂ | 1-Ethyl-3-methyl-1H-pyrazol-4-yl | |
| 3.372 | 3-Cl-5-CF₃-Ph | H | O | H | CH₂CH₂ | 1-Ethyl-3-methyl-1H-pyrazol-4-yl | |
| 3.373 | 3,5-Me₂-Ph | H | O | H | CH₂CH₂ | 1-Ethyl-3-methyl-1H-pyrazol-4-yl | |
| 3.374 | 3-Cl-Ph | H | O | H | CH₂CH₂ | 1-Ethyl-3-methyl-1H-pyrazol-4-yl | |
| 3.375 | 3-MeO-Ph | H | O | H | CH₂CH₂ | 1-Ethyl-3-methyl-1H-pyrazol-4-yl | |
| 3.376 | 3-Cl-5-F-Ph | H | O | H | CH₂CH₂ | 1-Ethyl-3-methyl-1H-pyrazol-4-yl | |
| 3.377 | 3-CN-5-F-Ph | H | O | H | CH₂CH₂ | 1-Ethyl-3-methyl-1H-pyrazol-4-yl | |
| 3.378 | 3-CN-Ph | H | O | H | CH₂CH₂ | 1-Ethyl-3-methyl-1H-pyrazol-4-yl | |
| 3.379 | 3-Br-5-Cl-Ph | H | O | H | CH₂CH₂ | 1-Ethyl-3-methyl-1H-pyrazol-4-yl | |
| 3.380 | 2,3,4-F₃-Ph | H | O | H | CH₂CH₂ | 1-Ethyl-3-methyl-1H-pyrazol-4-yl | |
| 3.381 | 3,5-Cl₂-4-MeO-Ph | H | O | H | CH₂CH₂ | 1-Ethyl-3-methyl-1H-pyrazol-4-yl | |
| 3.382 | Ph | H | O | H | CH₂CH₂ | 1-Ethyl-3-methyl-1H-pyrazol-4-yl | |
| 3.383 | 2,5-F₂-Ph | H | O | H | CH₂CH₂ | 1-Ethyl-3-methyl-1H-pyrazol-4-yl | |
| 3.384 | 3,5-Br₂-Ph | H | O | H | CH₂CH₂ | 1-Ethyl-3-methyl-1H-pyrazol-4-yl | |
| 3.385 | 2-F-Ph | H | O | H | CH₂CH₂ | 1-Ethyl-3-methyl-1H-pyrazol-4-yl | |
| 3.386 | 2,5-Cl₂-Ph | H | O | H | CH₂CH₂ | 1-Ethyl-3-methyl-1H-pyrazol-4-yl | |
| 3.387 | 3,5-Et₂-Ph | H | O | H | CH₂CH₂ | 1-Ethyl-3-methyl-1H-pyrazol-4-yl | |
| 3.388 | 3-CF₃S-Ph | H | O | H | CH₂CH₂ | 1-Ethyl-3-methyl-1H-pyrazol-4-yl | |
| 3.389 | 3-EtO-Ph | H | O | H | CH₂CH₂ | 1-Ethyl-3-methyl-1H-pyrazol-4-yl | |
| 3.390 | 3,5-Cl₂-Ph | CH 3 | O | H | CH₂CH₂ | 1-Ethyl-3-methyl-1H-pyrazol-4-yl | |
| 3.391 | 2-F-3-Me-Ph | H | O | H | CH₂CH₂ | 1-Ethyl-3-methyl-1H-pyrazol-4-yl | |
| 3.392 | 3-Cl-5-CF₃O-Ph | H | O | H | CH₂CH₂ | 1-Ethyl-3-methyl-1H-pyrazol-4-yl | |
| 3.393 | 3,5-F₂-Ph | H | O | H | CH₂CH₂ | 1-Ethyl-3-methyl-1H-pyrazol-5-yl | |
| 3.394 | 3,5-Cl₂-Ph | H | O | H | CH₂CH₂ | 1-Ethyl-3-methyl-1H-pyrazol-5-yl | |
| 3.395 | 3-F-Ph | H | O | H | CH₂CH₂ | 1-Ethyl-3-methyl-1H-pyrazol-5-yl | |
| 3.396 | 3,5-F₂-Ph | H | O | H | CH₂CH₂ | 1H-1,2,3-Triazol-5-yl | |
| 3.397 | 3,5-Cl₂-Ph | H | O | H | CH₂CH₂ | 1 H-Pyrazol-4-yl | |
| 3.398 | 3,5-F₂-Ph | H | O | H | CH₂CH₂ | 1 H-Pyrazol-4-yl | |
| 3.399 | 3-F-Ph | H | O | H | CH₂CH₂ | 1 H-Pyrazol-4-yl | |
| 3.400 | 3,5-F₂-Ph | H | O | H | CH₂CH₂ | 1-Isobutyl-1H-pyrazol-4-yl | |
| 3.401 | 3,5-Cl₂-Ph | H | O | H | CH₂CH₂ | 1-Isobutyl-1H-pyrazol-4-yl | |
| 3.402 | 3-F-Ph | H | O | H | CH₂CH₂ | 1-Isobutyl-1H-pyrazol-4-yl | |
| 3.403 | Ph | H | O | H | CH₂CH₂ | 1-Isobutyl-1H-pyrazol-4-yl | |
| 3.404 | 3,5-F₂-Ph | H | O | H | CH₂CH₂ | 1-Isopropyl-1H-pyrazol-4-yl | |
| 3.405 | 3-F-Ph | H | O | H | CH₂CH₂ | 1-Isopropyl-1H-pyrazol-4-yl | |
| 3.406 | 3,5-Cl₂-Ph | H | O | H | CH₂CH₂ | 1-Isopropyl-1H-pyrazol-4-yl | |
| 3.407 | Ph | H | O | H | CH₂CH₂ | 1-Isopropyl-1H-pyrazol-4-yl | |
| 3.408 | 3,5-F₂-Ph | H | O | H | CH₂CH₂ | 1-Methyl-1H-pyrazol-4-yl | |
| 3.409 | 3-F-5-MeO-Ph | H | O | H | CH₂CH₂ | 1-Methyl-1H-pyrazol-4-yl | |
| 3.410 | 3-F-Ph | H | O | H | CH₂CH₂ | 1-Methyl-1H-pyrazol-4-yl | |
| 3.411 | Ph | H | O | H | CH₂CH₂ | 1-Methyl-1H-pyrazol-4-yl | |
| 3.412 | 3,5-(CF₃)₂-Ph | H | O | H | CH₂CH₂ | 1-Methyl-1H-pyrazol-4-yl | |
| 3.413 | 3,5-Cl₂-Ph | H | O | H | CH₂CH₂ | 1-Methyl-1H-pyrazol-4-yl | |
| 3.414 | 3-Me-Ph | H | O | H | CH₂CH₂ | 1-Methyl-1H-pyrazol-4-yl | |
| 3.415 | 3,5-F₂-Ph | H | O | H | CH₂CH₂ | 1-Methyl-1H-pyrazol-5-yl | |
| 3.416 | 3-F-Ph | H | O | H | CH₂CH₂ | 1-Methyl-1H-pyrazol-5-yl | |
| 3.417 | 3,5-Cl₂-Ph | H | O | H | CH₂CH₂ | 1-Methyl-1H-pyrazol-5-yl | |
| 3.418 | Ph | H | O | H | CH₂CH₂ | 1-Methyl-1H-pyrazol-5-yl | |
| 3.419 | 3,5-Cl₂-Ph | H | O | H | CH₂CH₂ | 1-Methyl-3-(trifluormethyl)-1H-pyrazol-4-yl | D1 |
| 3.420 | 3,5-Cl₂-Ph | H | O | H | CH₂CH₂ | 1-Methyl-3-(trifluormethyl)-1H-pyrazol-4-yl | D2 |
| 3.421 | 3,5-F₂-Ph | H | O | H | CH₂CH₂ | 1-Methyl-5-(trifluormethyl)-1H-pyrazol-4-yl | |
| 3.422 | 3,5-F₂-Ph | H | O | H | CH₂CH₂ | 1-Propyl-1H-pyrazol-3-yl | |
| 3.423 | 3,5-Cl₂-Ph | H | O | H | CH₂CH₂ | 1-Propyl-1H-pyrazol-3-yl | |
| 3.424 | 3,5-F₂-Ph | H | O | H | CH₂CH₂ | 1-Propyl-1H-pyrazol-4-yl | |
| 3.425 | 3,5-Cl₂-Ph | H | O | H | CH₂CH₂ | 1-Propyl-1H-pyrazol-4-yl | |
| 3.426 | 3-F-Ph | H | O | H | CH₂CH₂ | 1-Propyl-1H-pyrazol-4-yl | |
| 3.427 | Ph | H | O | H | CH₂CH₂ | 1-Propyl-1H-pyrazol-4-yl | |
| 3.428 | 3,5-F₂-Ph | H | O | H | CH₂CH₂ | 1-Tert-butyl-1H-pyrazol-4-yl | |
| 3.429 | 3,5-Cl₂-Ph | H | O | H | CH₂CH₂ | 1-Tert-butyl-1H-pyrazol-4-yl | |
| 3.430 | Ph | H | O | H | CH₂CH₂ | 1-Tert-butyl-1H-pyrazol-4-yl | |
| 3.431 | 3,5-F₂-Ph | H | O | H | CH₂CH₂ | 2-Chlorpyridin-4-yl | |
| 3.432 | 3-F-Ph | H | O | H | CH₂CH₂ | 2-Chlorpyridin-4-yl | |
| 3.433 | 3,5-F₂-Ph | H | O | H | CH₂CH₂ | 2-Methyl-1,3-thiazol-4-yl | |
| 3.434 | 3,5-Cl₂-Ph | H | O | H | CH₂CH₂ | 2-Methyl-1,3-thiazol-4-yl | |
| 3.435 | 3,5-Cl₂-Ph | H | O | H | CH₂CH₂ | 3-(Cyclopropylcarbamo yl)-1,2-oxazol-5-yl | |
| 3.436 | 3,5-Cl₂-Ph | H | O | H | CH₂CH₂ | 3-(Ethoxycarbonyl)-1,2-oxazol-5-yl | |
| 3.437 | 3,5-F₂-Ph | H | O | H | CH₂CH₂ | 3-(Methoxycarbonyl)ph enyl | |
| 3.438 | 3,5-F₂-Ph | H | O | H | CH₂CH₂ | 3,5-Difluorpyridin-2-yl | |
| 3.439 | 3,5-Cl₂-Ph | H | O | H | CH₂CH₂ | 3-Carboxy-1,2-oxazol-5-yl | |
| 3.440 | 3,5-F₂-Ph | H | O | H | CH₂CH₂ | 3-Chlor-1-ethyl-1H-pyrazol-4-yl | |
| 3.441 | 3-F-Ph | H | O | H | CH₂CH₂ | 3-Chlor-1-ethyl-1H-pyrazol-4-yl | |
| 3.442 | Ph | H | O | H | CH₂CH₂ | 3-Chlor-1-ethyl-1H-pyrazol-4-yl | |
| 3.443 | 3,5-Cl₂-Ph | H | O | H | CH₂CH₂ | 3-Chlor-1-ethyl-1H-pyrazol-4-yl | |
| 3.444 | 3,5-F₂-Ph | H | O | H | CH₂CH₂ | 3-Chlor-1-isopropyl-1H-pyrazol-4-yl | |
| 3.445 | 3-F-Ph | H | O | H | CH₂CH₂ | 3-Chlor-1-isopropyl-1H-pyrazol-4-yl | |
| 3.446 | 3,5-F₂-Ph | H | S | H | CH₂CH₂ | 3-Chlor-1-isopropyl-1H-pyrazol-4-yl | |
| 3.447 | 3,5-Cl₂-Ph | H | O | H | CH₂CH₂ | 3-Chlor-1-isopropyl-1H-pyrazol-4-yl | |
| 3.448 | 3,5-Cl₂-Ph | H | O | H | CH₂CH₂ | 3-Chlor-1-methyl-1H-pyrazol-4-yl | |
| 3.449 | 3,5-F₂-Ph | H | O | H | CH₂CH₂ | 3-Chlor-1-methyl-1H-pyrazol-4-yl | |
| 3.450 | 3,5-F₂-Ph | H | O | H | CH₂CH₂ | 3-Chlorpyridin-4-yl | |
| 3.451 | 3-F-Ph | H | O | H | CH₂CH₂ | 3-Chlorpyridin-4-yl | |
| 3.452 | 3,5-F₂-Ph | H | O | H | CH₂CH₂ | 3-Cyclopropyl-1,2,4-oxadiazol-5-yl | |
| 3.453 | 3,5-Cl₂-Ph | H | O | H | CH₂CH₂ | 3-Cyclopropyl-1,2,4-oxadiazol-5-yl | |
| 3.454 | 3,5-F₂-Ph | H | O | H | CH₂CH₂ | 3-Furyl | |
| 3.455 | 3,5-F₂-Ph | H | O | H | CH₂CH₂ | 3-Methyl-1H-pyrazol-4-yl | |
| 3.456 | 3-F-Ph | H | O | H | CH₂CH₂ | 3-Methyl-1-propyl-1H-pyrazol-4-yl | |
| 3.457 | 3,5-F₂-Ph | H | O | H | CH₂CH₂ | 3-Methyl-1-propyl-1H-pyrazol-4-yl | |
| 3.458 | 3-CI-5-F-Ph | H | O | H | CH₂CH₂ | 3-Methyl-1-propyl-1H-pyrazol-4-yl | |
| 3.459 | 3-Cl-5-CF₃O-Ph | H | O | H | CH₂CH₂ | 3-Methyl-1-propyl-1H-pyrazol-4-yl | |
| 3.460 | 3-F-5-Me-Ph | H | O | H | CH₂CH₂ | 3-Methyl-1-propyl-1H-pyrazol-4-yl | |
| 3.461 | 3-Me-Ph | H | O | H | CH₂CH₂ | 3-Methyl-1-propyl-1H-pyrazol-4-yl | |
| 3.462 | 3-Cl-5-Me-Ph | H | O | H | CH₂CH₂ | 3-Methyl-1-propyl-1H-pyrazol-4-yl | |
| 3.463 | 3-CI-4-F-Ph | H | O | H | CH₂CH₂ | 3-Methyl-1-propyl-1H-pyrazol-4-yl | |
| 3.464 | 3-CF₃O-Ph | H | O | H | CH₂CH₂ | 3-Methyl-1-propyl-1H-pyrazol-4-yl | |
| 3.465 | 4-Cl-3,5-F₂-Ph | H | O | H | CH₂CH₂ | 3-Methyl-1-propyl-1H-pyrazol-4-yl | |
| 3.466 | 3,5-Cl₂-Ph | H | O | H | CH₂CH₂ | 3-Methyl-1-propyl-1H-pyrazol-4-yl | |
| 3.467 | Ph | H | O | H | CH₂CH₂ | 3-Methyl-1-propyl-1H-pyrazol-4-yl | |
| 3.468 | 3,5-Cl₂-Ph | H | O | H | CH₂CH₂ | 4-(Methoxycarbonyl)ph enyl | |
| 3.469 | 3,5-F₂-Ph | H | O | H | CH₂CH₂ | 4-(Trifluormethyl)-1,3-thiazol-2-yl | |
| 3.470 | 3-F-Ph | H | O | H | CH₂CH₂ | 4-(Trifluormethyl)-1,3-thiazol-2-yl | |
| 3.471 | 3,5-Cl₂-Ph | H | O | H | CH₂CH₂ | 4-(Trifluormethyl)-1,3-thiazol-2-yl | |
| 3.472 | 3,5-F₂-Ph | H | O | H | CH₂CH₂ | 4-Chlor-1-methyl-1H-pyrazol-3-yl | |
| 3.473 | 3,5-Cl₂-Ph | H | O | H | CH₂CH₂ | 4-Chlor-1-methyl-1H-pyrazol-3-yl | |
| 3.474 | 3-F-Ph | H | O | H | CH₂CH₂ | 4-Chlor-1-methyl-1H-pyrazol-3-yl | |
| 3.475 | 3,5-F₂-Ph | H | O | H | CH₂CH₂ | 4-Chlor-1-methyl-1H-pyrazol-5-yl | |
| 3.476 | 3,5-F₂-Ph | H | O | H | CH₂CH₂ | 5-Chlor-1-methyl-1H-pyrazol-4-yl | |
| 3.477 | 3-F-Ph | H | O | H | CH₂CH₂ | 5-Chlor-1-methyl-1H-pyrazol-4-yl | |
| 3.478 | 3,5-F₂-Ph | H | S | H | CH₂CH₂ | 5-Chlor-1-methyl-1H-pyrazol-4-yl | |
| 3.479 | 3,5-Cl₂-Ph | H | O | H | CH₂CH₂ | 5-Chlor-1-methyl-1H-pyrazol-4-yl | |
| 3.480 | Ph | H | O | H | CH₂CH₂ | 5-Chlor-1-methyl-1H-pyrazol-4-yl | |
| 3.481 | 3,5-F₂-Ph | H | O | H | CH₂CH₂ | 5-Methoxy-1,3-dimethyl-1H-pyrazol-4-yl | |
| 3.482 | 3,5-Cl₂-Ph | H | O | H | CH₂CH₂ | 5-Methoxy-1,3-dimethyl-1H-pyrazol-4-yl | |
| 3.483 | 3-F-Ph | H | O | H | CH₂CH₂ | 5-Methoxy-1,3-dimethyl-1H-pyrazol-4-yl | |
| 3.484 | 3,5-Cl₂-Ph | H | O | H | CH₂CH₂ | 5-Methyl-1,2,4-oxadiazol-3-yl | |
| 3.485 | 3,5-F₂-Ph | H | O | H | CH₂CH₂ | 5-Methyl-1,2,4-oxadiazol-3-yl | |
| 3.486 | 3,5-Cl₂-Ph | H | O | H | CH₂CH₂ | 5-Methyl-1,2-oxazol-3-yl | |
| 3.487 | 3-F-Ph | H | O | H | CH₂CH₂ | 5-Methyl-1-propyl-1H-pyrazol-4-yl | |
| 3.488 | 3,5-F₂-Ph | H | O | H | CH₂CH₂ | 5-Methyl-1-propyl-1H-pyrazol-4-yl | |
| 3.489 | 3,5-Cl₂-Ph | H | O | H | CH₂CH₂ | 5-Methyl-1-propyl-1H-pyrazol-4-yl | |
| 3.490 | Ph | H | O | H | CH₂CH₂ | 5-Methyl-1-propyl-1H-pyrazol-4-yl | |
| 3.491 | 3,5-F₂-Ph | H | O | H | CH₂CH₂ | 6-Chlorpyridin-3-yl | |
| 3.492 | 3,5-F₂-Ph | H | O | H | CH₂CH₂ | Furan-2-yl | |
| 3.493 | 3,5-Cl₂-Ph | H | O | H | CH₂CH₂ | Pyridin-2-yl | |
| 3.494 | 3,5-F₂-Ph | H | O | H | CH₂CH₂ | Pyridin-2-yl | |
| 3.495 | 3,5-F₂-Ph | H | O | H | CH₂CH₂ | Pyridin-3-yl | |
| 3.496 | 3-F-Ph | H | O | H | CH₂CH₂ | Pyridin-3-yl | |
| 3.497 | 3,5-F₂-Ph | H | O | H | CH₂CH₂ | Pyridin-4-yl | |
| 3.498 | 3-F-Ph | H | O | H | CH₂CH₂ | Pyridin-4-yl | |
| 3.499 | 3,5-Cl₂-Ph | H | O | H | CH₂CH₂ | Pyrimidin-2-yl | |
| 3.500 | 3,5-F₂-Ph | H | O | H | CHCH₃ | 1-(2,2,2-Trifluorethyl)-1H-pyrazol-4-yl | |
| 3.501 | 3,5-F₂-Ph | H | O | H | CHCH₃ | 1-(2-Chlorethyl)-1H-pyrazol-4-yl | |
| 3.502 | 3,5-Cl₂-Ph | H | O | H | CHCH₃ | 1-(2-Methoxy-2-oxoethyl)-1H-pyrazol-4-yl | |
| 3.503 | 3,5-F₂-Ph | H | O | H | CHCH₃ | 1-(3-Carboxypropyl)-1H-pyrazol-4-yl | |
| 3.504 | 3,5-F₂-Ph | H | O | H | CHCH₃ | 1-(3-Methoxy-3-oxopropyl)-1H-pyrazol-4-yl | |
| 3.505 | 3,5-F₂-Ph | H | O | H | CHCH₃ | 1-(4-Methoxy-4-oxobutyl)-1H-pyrazol-4-yl | |
| 3.506 | 3,5-F₂-Ph | H | O | H | CHCH₃ | 1-(Cyanomethyl)-1H-pyrazol-4-yl | |
| 3.507 | 3,5-Cl₂-Ph | H | O | H | CHCH₃ | 1-(Cyanomethyl)-1H-pyrazol-4-yl | |
| 3.508 | 3-F-Ph | H | O | H | CHCH₃ | 1-(Cyanomethyl)-1H-pyrazol-4-yl | |
| 3.509 | 3,5-F₂-Ph | H | O | H | CHCH₃ | 1,3-Dimethyl-1H-pyrazol-4-yl | |
| 3.510 | 3-F-Ph | H | O | H | CHCH₃ | 1,3-Dimethyl-1H-pyrazol-4-yl | |
| 3.511 | 3-Me-Ph | H | O | H | CHCH₃ | 1,3-Dimethyl-1H-pyrazol-4-yl | |
| 3.512 | Ph | H | O | H | CHCH₃ | 1,3-Dimethyl-1H-pyrazol-4-yl | |
| 3.513 | 3-CI-Ph | H | O | H | CHCH₃ | 1,3-Dimethyl-1H-pyrazol-4-yl | |
| 3.514 | 3,5-Cl₂-Ph | H | O | H | CHCH₃ | 1,3-Dimethyl-1H-pyrazol-4-yl | |
| 3.515 | 3,5-(CF₃)₂-Ph | H | O | H | CHCH₃ | 1,3-Dimethyl-1H-pyrazol-4-yl | |
| 3.516 | 3,5-F₂-Ph | H | O | H | CHCH₃ | 1,3-Dimethyl-1H-pyrazol-5-yl | |
| 3.517 | Ph | H | O | H | CHCH₃ | 1,3-Dimethyl-1H-pyrazol-5-yl | |
| 3.518 | 3,5-Cl₂-Ph | H | O | H | CHCH₃ | 1,3-Dimethyl-1H-pyrazol-5-yl | |
| 3.519 | 3,5-(CF₃)₂-Ph | H | O | H | CHCH₃ | 1,3-Dimethyl-1H-pyrazol-5-yl | |
| 3.520 | 3,5-F₂-Ph | H | O | H | CHCH₃ | 1,3-Oxazol-4-yl | |
| 3.521 | 3,5-Cl₂-Ph | H | O | H | CHCH₃ | 1,3-Oxazol-4-yl | |
| 3.522 | 3-F-Ph | H | O | H | CHCH₃ | 1,3-Oxazol-4-yl | |
| 3.523 | 3,5-Cl₂-Ph | H | O | H | CHCH₃ | 1,3-Thiazol-2-yl | |
| 3.524 | 3,5-F₂-Ph | H | O | H | CHCH₃ | 1,3-Thiazol-2-yl | |
| 3.525 | 3,5-Cl₂-Ph | H | O | H | CHCH₃ | 1,4-Dimethyl-5-oxo-4,5-dihydro-1H-1,2,4-triazol-3-yl | |
| 3.526 | 3,5-F₂-Ph | H | O | H | CHCH₃ | 1,5-Dimethyl-1H-pyrazol-3-yl | |
| 3.527 | 3,5-F₂-Ph | H | O | H | CHCH₃ | 1,5-Dimethyl-1H-pyrazol-4-yl | |
| 3.528 | 3-F-Ph | H | O | H | CHCH₃ | 1,5-Dimethyl-1H-pyrazol-4-yl | |
| 3.529 | 3,5-Cl₂-Ph | H | O | H | CHCH₃ | 1,5-Dimethyl-1H-pyrazol-4-yl | |
| 3.530 | Ph | H | O | H | CHCH₃ | 1,5-Dimethyl-1H-pyrazol-4-yl | |
| 3.531 | 3,5-F₂-Ph | H | O | H | CHCH₃ | 1-Acetyl-1H-pyrazol-4-yl | |
| 3.532 | 3,5-F₂-Ph | H | O | H | CHCH₃ | 1-Butyl-3-methyl-1H-pyrazol-4-yl | |
| 3.533 | 3,5-F₂-Ph | H | O | H | CHCH₃ | 1-Butyl-5-methyl-1H-pyrazol-4-yl | |
| 3.534 | 3,5-F₂-Ph | H | O | H | CHCH₃ | 1-Cyclopropyl-1H-pyrazol-4-yl | |
| 3.535 | Ph | H | O | H | CHCH₃ | 1-Cyclopropyl-1H-pyrazol-4-yl | |
| 3.536 | 3-F-Ph | H | O | H | CHCH₃ | 1-Cyclopropyl-1H-pyrazol-4-yl | |
| 3.537 | 3,5-Cl₂-Ph | H | O | H | CHCH₃ | 1-Cyclopropyl-1H-pyrazol-4-yl | |
| 3.538 | 3,5-Cl₂-Ph | H | O | H | CHCH₃ | 1-Ethyl-1H-pyrazol-4-yl | |
| 3.539 | 3-F-Ph | H | O | H | CHCH₃ | 1-Ethyl-1H-pyrazol-4-yl | |
| 3.540 | 3,5-F₂-Ph | H | O | H | CHCH₃ | 1-Ethyl-1H-pyrazol-4-yl | |
| 3.541 | 3-Br-5-CF₃-Ph | H | O | H | CHCH₃ | 1-Ethyl-1H-pyrazol-4-yl | |
| 3.542 | 3-Me-Ph | H | O | H | CHCH₃ | 1-Ethyl-1H-pyrazol-4-yl | |
| 3.543 | 3-F-5-MeO-Ph | H | O | H | CHCH₃ | 1-Ethyl-1H-pyrazol-4-yl | |
| 3.544 | 3-CI-5-Et-Ph | H | O | H | CHCH₃ | 1-Ethyl-1H-pyrazol-4-yl | |
| 3.545 | 3,5-(MeO)₂-Ph | H | O | H | CHCH₃ | 1-Ethyl-1H-pyrazol-4-yl | |
| 3.546 | 3-Me-5-CF₃O-Ph | H | O | H | CHCH₃ | 1-Ethyl-1H-pyrazol-4-yl | |
| 3.547 | 3,5-Cl₂-4-MeO-Ph | H | O | H | CHCH₃ | 1-Ethyl-1H-pyrazol-4-yl | |
| 3.548 | Ph | H | O | H | CHCH₃ | 1-Ethyl-1H-pyrazol-4-yl | |
| 3.549 | 3,5-F₂-Ph | H | O | H | CHCH₃ | 1-Ethyl-1H-pyrazol-5-yl | |
| 3.550 | 3,5-Cl₂-Ph | H | O | H | CHCH₃ | 1-Ethyl-1H-pyrazol-5-yl | |
| 3.551 | Ph | H | O | H | CHCH₃ | 1-Ethyl-1H-pyrazol-5-yl | |
| 3.552 | 3,5-F₂-Ph | H | O | H | CHCH₃ | 1-Ethyl-3-methyl-1H-pyrazol-4-yl | |
| 3.553 | 3-Me-Ph | H | O | H | CHCH₃ | 1-Ethyl-3-methyl-1H-pyrazol-4-yl | |
| 3.554 | 3-Br-5-F-Ph | H | O | H | CHCH₃ | 1-Ethyl-3-methyl-1H-pyrazol-4-yl | |
| 3.555 | 3,4,5-F₃-Ph | H | O | H | CHCH₃ | 1-Ethyl-3-methyl-1H-pyrazol-4-yl | |
| 3.556 | 3,4-F₂-Ph | H | O | H | CHCH₃ | 1-Ethyl-3-methyl-1H-pyrazol-4-yl | |
| 3.557 | 3,5-F₂-Ph | H | S | H | CHCH₃ | 1-Ethyl-3-methyl-1H-pyrazol-4-yl | |
| 3.558 | 3-F-5-Me-Ph | H | O | H | CHCH₃ | 1-Ethyl-3-methyl-1H-pyrazol-4-yl | |
| 3.559 | 3-Cl-5-CN-Ph | H | O | H | CHCH₃ | 1-Ethyl-3-methyl-1H-pyrazol-4-yl | |
| 3.560 | 3-F-Ph | H | O | H | CHCH₃ | 1-Ethyl-3-methyl-1H-pyrazol-4-yl | |
| 3.561 | 3-cPr-5-F-Ph | H | O | H | CHCH₃ | 1-Ethyl-3-methyl-1H-pyrazol-4-yl | |
| 3.562 | 3-Cl-5-Me-Ph | H | O | H | CHCH₃ | 1-Ethyl-3-methyl-1H-pyrazol-4-yl | |
| 3.563 | 3-F-5-MeO-Ph | H | O | H | CHCH₃ | 1-Ethyl-3-methyl-1H-pyrazol-4-yl | |
| 3.564 | 2,5-Me₂-Ph | H | O | H | CHCH₃ | 1-Ethyl-3-methyl-1H-pyrazol-4-yl | |
| 3.565 | 3-CF₃O-Ph | H | O | H | CHCH₃ | 1-Ethyl-3-methyl-1H-pyrazol-4-yl | |
| 3.566 | 3-Cl-5-Et-Ph | H | O | H | CHCH₃ | 1-Ethyl-3-methyl-1H-pyrazol-4-yl | |
| 3.567 | 2,3,5-F₃-Ph | H | O | H | CHCH₃ | 1-Ethyl-3-methyl-1H-pyrazol-4-yl | |
| 3.568 | 3-Et-5-F-Ph | H | O | H | CHCH₃ | 1-Ethyl-3-methyl-1H-pyrazol-4-yl | |
| 3.569 | 3-F-5-CF₃-Ph | H | O | H | CHCH₃ | 1-Ethyl-3-methyl-1H-pyrazol-4-yl | |
| 3.570 | 3-Br-5-CF₃-Ph | H | O | H | CHCH₃ | 1-Ethyl-3-methyl-1H-pyrazol-4-yl | |
| 3.571 | 3-Cl-5-MeO-Ph | H | O | H | CHCH₃ | 1-Ethyl-3-methyl-1H-pyrazol-4-yl | |
| 3.572 | 3-Cl-4-F-Ph | H | O | H | CHCH₃ | 1-Ethyl-3-methyl-1H-pyrazol-4-yl | |
| 3.573 | 2,3-F₂-Ph | H | O | H | CHCH₃ | 1-Ethyl-3-methyl-1H-pyrazol-4-yl | |
| 3.574 | 3-Cl-5-CF₃-Ph | H | O | H | CHCH₃ | 1-Ethyl-3-methyl-1H-pyrazol-4-yl | |
| 3.575 | 3,5-Me₂-Ph | H | O | H | CHCH₃ | 1-Ethyl-3-methyl-1H-pyrazol-4-yl | |
| 3.576 | 3-Cl-Ph | H | O | H | CHCH₃ | 1-Ethyl-3-methyl-1H-pyrazol-4-yl | |
| 3.577 | 3-MeO-Ph | H | O | H | CHCH₃ | 1-Ethyl-3-methyl-1H-pyrazol-4-yl | |
| 3.578 | 3-Cl-5-F-Ph | H | O | H | CHCH₃ | 1-Ethyl-3-methyl-1H-pyrazol-4-yl | |
| 3.579 | 3-CN-5-F-Ph | H | O | H | CHCH₃ | 1-Ethyl-3-methyl-1H-pyrazol-4-yl | |
| 3.580 | 3-CN-Ph | H | O | H | CHCH₃ | 1-Ethyl-3-methyl-1H-pyrazol-4-yl | |
| 3.581 | 3-Br-5-CI-Ph | H | O | H | CHCH₃ | 1-Ethyl-3-methyl-1H-pyrazol-4-yl | |
| 3.582 | 2,3,4-F₃-Ph | H | O | H | CHCH₃ | 1-Ethyl-3-methyl-1H-pyrazol-4-yl | |
| 3.583 | 3,5-Cl₂-4-MeO-Ph | H | O | H | CHCH₃ | 1-Ethyl-3-methyl-1H-pyrazol-4-yl | |
| 3.584 | Ph | H | O | H | CHCH₃ | 1-Ethyl-3-methyl-1H-pyrazol-4-yl | |
| 3.585 | 2,5-F₂-Ph | H | O | H | CHCH₃ | 1-Ethyl-3-methyl-1H-pyrazol-4-yl | |
| 3.586 | 3,5-Br₂-Ph | H | O | H | CHCH₃ | 1-Ethyl-3-methyl-1H-pyrazol-4-yl | |
| 3.587 | 2-F-Ph | H | O | H | CHCH₃ | 1-Ethyl-3-methyl-1H-pyrazol-4-yl | |
| 3.588 | 2,5-Cl₂-Ph | H | O | H | CHCH₃ | 1-Ethyl-3-methyl-1H-pyrazol-4-yl | |
| 3.589 | 3,5-Et₂-Ph | H | O | H | CHCH₃ | 1-Ethyl-3-methyl-1H-pyrazol-4-yl | |
| 3.590 | 3-CF₃S-Ph | H | O | H | CHCH₃ | 1-Ethyl-3-methyl-1H-pyrazol-4-yl | |
| 3.591 | 3-EtO-Ph | H | O | H | CHCH₃ | 1-Ethyl-3-methyl-1H-pyrazol-4-yl | |
| 3.592 | 3,5-Cl₂-Ph | CH 3 | O | H | CHCH₃ | 1-Ethyl-3-methyl-1H-pyrazol-4-yl | |
| 3.593 | 2-F-3-Me-Ph | H | O | H | CHCH₃ | 1-Ethyl-3-methyl-1H-pyrazol-4-yl | |
| 3.594 | 3-Cl-5-CF₃O-Ph | H | O | H | CHCH₃ | 1-Ethyl-3-methyl-1H-pyrazol-4-yl | |
| 3.595 | 3,5-F₂-Ph | H | O | H | CHCH₃ | 1-Ethyl-3-methyl-1H-pyrazol-5-yl | |
| 3.596 | 3,5-Cl₂-Ph | H | O | H | CHCH₃ | 1-Ethyl-3-methyl-1H-pyrazol-5-yl | |
| 3.597 | 3-F-Ph | H | O | H | CHCH₃ | 1-Ethyl-3-methyl-1H-pyrazol-5-yl | |
| 3.598 | 3,5-F₂-Ph | H | O | H | CHCH₃ | 1H-1,2,3-Triazol-5-yl | |
| 3.599 | 3,5-Cl₂-Ph | H | O | H | CHCH₃ | 1H-Pyrazol-4-yl | |
| 3.600 | 3,5-F₂-Ph | H | O | H | CHCH₃ | 1H-Pyrazol-4-yl | |
| 3.601 | 3-F-Ph | H | O | H | CHCH₃ | 1H-Pyrazol-4-yl | |
| 3.602 | 3,5-F₂-Ph | H | O | H | CHCH₃ | 1-Isobutyl-1H-pyrazol-4-yl | |
| 3.603 | 3,5-Cl₂-Ph | H | O | H | CHCH₃ | 1-Isobutyl-1H-pyrazol-4-yl | |
| 3.604 | 3-F-Ph | H | O | H | CHCH₃ | 1-Isobutyl-1H-pyrazol-4-yl | |
| 3.605 | Ph | H | O | H | CHCH₃ | 1-Isobutyl-1H-pyrazol-4-yl | |
| 3.606 | 3,5-F₂-Ph | H | O | H | CHCH₃ | 1-Isopropyl-1H-pyrazol-4-yl | |
| 3.607 | 3-F-Ph | H | O | H | CHCH₃ | 1-Isopropyl-1H-pyrazol-4-yl | |
| 3.608 | 3,5-Cl₂-Ph | H | O | H | CHCH₃ | 1-Isopropyl-1H-pyrazol-4-yl | |
| 3.609 | Ph | H | O | H | CHCH₃ | 1-Isopropyl-1H-pyrazol-4-yl | |
| 3.610 | 3,5-F₂-Ph | H | O | H | CHCH₃ | 1-Methyl-1H-pyrazol-4-yl | |
| 3.611 | 3-F-5-MeO-Ph | H | O | H | CHCH₃ | 1-Methyl-1H-pyrazol-4-yl | |
| 3.612 | 3-F-Ph | H | O | H | CHCH₃ | 1-Methyl-1H-pyrazol-4-yl | |
| 3.613 | Ph | H | O | H | CHCH₃ | 1-Methyl-1H-pyrazol-4-yl | |
| 3.614 | 3,5-(CF₃)₂-Ph | H | O | H | CHCH₃ | 1-Methyl-1H-pyrazol-4-yl | |
| 3.615 | 3,5-Cl₂-Ph | H | O | H | CHCH₃ | 1-Methyl-1H-pyrazol-4-yl | |
| 3.616 | 3-Me-Ph | H | O | H | CHCH₃ | 1-Methyl-1H-pyrazol-4-yl | |
| 3.617 | 3,5-F₂-Ph | H | O | H | CHCH₃ | 1-Methyl-1H-pyrazol-5-yl | |
| 3.618 | 3-F-Ph | H | O | H | CHCH₃ | 1-Methyl-1H-pyrazol-5-yl | |
| 3.619 | 3,5-Cl₂-Ph | H | O | H | CHCH₃ | 1-Methyl-1H-pyrazol-5-yl | |
| 3.620 | Ph | H | O | H | CHCH₃ | 1-Methyl-1H-pyrazol-5-yl | |
| 3.621 | 3,5-Cl₂-Ph | H | O | H | CHCH₃ | 1-Methyl-3-(trifluormethyl)-1H-pyrazol-4-yl | D1 |
| 3.622 | 3,5-Cl₂-Ph | H | O | H | CHCH₃ | 1-Methyl-3-(trifluormethyl)-1H-pyrazol-4-yl | D2 |
| 3.623 | 3,5-F₂-Ph | H | O | H | CHCH₃ | 1-Methyl-5-(trifluormethyl)-1H-pyrazol-4-yl | |
| 3.624 | 3,5-F₂-Ph | H | O | H | CHCH₃ | 1-Propyl-1H-pyrazol-3-yl | |
| 3.625 | 3,5-Cl₂-Ph | H | O | H | CHCHs | 1-Propyl-1H-pyrazol-3-yl | |
| 3.626 | 3,5-F₂-Ph | H | O | H | CHCH₃ | 1-Propyl-1H-pyrazol-4-yl | |
| 3.627 | 3,5-Cl₂-Ph | H | O | H | CHCH₃ | 1-Propyl-1H-pyrazol-4-yl | |
| 3.628 | 3-F-Ph | H | O | H | CHCH₃ | 1-Propyl-1H-pyrazol-4-yl | |
| 3.629 | Ph | H | O | H | CHCH₃ | 1-Propyl-1H-pyrazol-4-yl | |
| 3.630 | 3,5-F₂-Ph | H | O | H | CHCH₃ | 1-Tert-butyl-1H-pyrazol-4-yl | |
| 3.631 | 3,5-Cl₂-Ph | H | O | H | CHCH₃ | 1-Tert-butyl-1H-pyrazol-4-yl | |
| 3.632 | Ph | H | O | H | CHCH₃ | 1-Tert-butyl-1H-pyrazol-4-yl | |
| 3.633 | 3,5-F₂-Ph | H | O | H | CHCH₃ | 2-Chlorpyridin-4-yl | |
| 3.634 | 3-F-Ph | H | O | H | CHCH₃ | 2-Chlorpyridin-4-yl | |
| 3.635 | 3,5-F₂-Ph | H | O | H | CHCH₃ | 2-Methyl-1,3-thiazol-4-yl | |
| 3.636 | 3,5-Cl₂-Ph | H | O | H | CHCH₃ | 2-Methyl-1,3-thiazol-4-yl | |
| 3.637 | 3,5-Cl₂-Ph | H | O | H | CHCH₃ | 3-(Cyclopropylcarbamo yl)-1,2-oxazol-5-yl | |
| 3.638 | 3,5-Cl₂-Ph | H | O | H | CHCH₃ | 3-(Ethoxycarbonyl)-1,2-oxazol-5-yl | |
| 3.639 | 3,5-F₂-Ph | H | O | H | CHCH₃ | 3-(Methoxycarbonyl)ph enyl | |
| 3.640 | 3,5-F₂-Ph | H | O | H | CHCH₃ | 3,5-Difluorpyridin-2-yl | |
| 3.641 | 3,5-Cl₂-Ph | H | O | H | CHCH₃ | 3-Carboxy-1,2-oxazol-5-yl | |
| 3.642 | 3,5-F₂-Ph | H | O | H | CHCH₃ | 3-Chlor-1-ethyl-1H-pyrazol-4-yl | |
| 3.643 | 3-F-Ph | H | O | H | CHCH₃ | 3-Chlor-1-ethyl-1H-pyrazol-4-yl | |
| 3.644 | Ph | H | O | H | CHCH₃ | 3-Chlor-1-ethyl-1H-pyrazol-4-yl | |
| 3.645 | 3,5-Cl₂-Ph | H | O | H | CHCH₃ | 3-Chlor-1-ethyl-1H-pyrazol-4-yl | |
| 3.646 | 3,5-F₂-Ph | H | O | H | CHCH₃ | 3-Chlor-1-isopropyl-1H-pyrazol-4-yl | |
| 3.647 | 3-F-Ph | H | O | H | CHCH₃ | 3-Chlor-1-isopropyl-1H-pyrazol-4-yl | |
| 3.648 | 3,5-F₂-Ph | H | S | H | CHCH₃ | 3-Chlor-1-isopropyl-1H-pyrazol-4-yl | |
| 3.649 | 3,5-Cl₂-Ph | H | O | H | CHCH₃ | 3-Chlor-1-isopropyl-1H-pyrazol-4-yl | |
| 3.650 | 3,5-Cl₂-Ph | H | O | H | CHCH₃ | 3-Chlor-1-methyl-1H-pyrazol-4-yl | |
| 3.651 | 3,5-F₂-Ph | H | O | H | CHCH₃ | 3-Chlor-1-methyl-1H-pyrazol-4-yl | |
| 3.652 | 3,5-F₂-Ph | H | O | H | CHCH₃ | 3-Chlorpyridin-4-yl | |
| 3.653 | 3-F-Ph | H | O | H | CHCH₃ | 3-Chlorpyridin-4-yl | |
| 3.654 | 3,5-F₂-Ph | H | O | H | CHCH₃ | 3-c-Pr-1,2,4-oxadiazol-5-yl | |
| 3.655 | 3,5-Cl₂-Ph | H | O | H | CHCH₃ | 3-c-Pr-1,2,4-oxadiazol-5-yl | |
| 3.656 | 3,5-F₂-Ph | H | O | H | CHCH₃ | 3-Furyl | |
| 3.657 | 3,5-F₂-Ph | H | O | H | CHCH₃ | 3-Methyl-1H-pyrazol-4-yl | |
| 3.658 | 3-F-Ph | H | O | H | CHCH₃ | 3-Methyl-1-propyl-1H-pyrazol-4-yl | |
| 3.659 | 3,5-F₂-Ph | H | O | H | CHCH₃ | 3-Methyl-1-propyl-1H-pyrazol-4-yl | |
| 3.660 | 3-CI-5-F-Ph | H | O | H | CHCH₃ | 3-Methyl-1-propyl-1H-pyrazol-4-yl | |
| 3.661 | 3-Cl-5-CF₃O-Ph | H | O | H | CHCH₃ | 3-Methyl-1-propyl-1H-pyrazol-4-yl | |
| 3.662 | 3-F-5-Me-Ph | H | O | H | CHCH₃ | 3-Methyl-1-propyl-1H-pyrazol-4-yl | |
| 3.663 | 3-Me-Ph | H | O | H | CHCH₃ | 3-Methyl-1-propyl-1H-pyrazol-4-yl | |
| 3.664 | 3-Cl-5-Me-Ph | H | O | H | CHCH₃ | 3-Methyl-1-propyl-1H-pyrazol-4-yl | |
| 3.665 | 3-CI-4-F-Ph | H | O | H | CHCH₃ | 3-Methyl-1-propyl-1H-pyrazol-4-yl | |
| 3.666 | 3-CF₃O-Ph | H | O | H | CHCH₃ | 3-Methyl-1-propyl-1H-pyrazol-4-yl | |
| 3.667 | 4-Cl-3,5-F₂-Ph | H | O | H | CHCH₃ | 3-Methyl-1-propyl-1H-pyrazol-4-yl | |
| 3.668 | 3,5-Cl₂-Ph | H | O | H | CHCH₃ | 3-Methyl-1-propyl-1H-pyrazol-4-yl | |
| 3.669 | Ph | H | O | H | CHCH₃ | 3-Methyl-1-propyl-1H-pyrazol-4-yl | |
| 3.670 | 3,5-Cl₂-Ph | H | O | H | CHCH₃ | 4-(Methoxycarbonyl)ph enyl | |
| 3.671 | 3,5-F₂-Ph | H | O | H | CHCH₃ | 4-(Trifluormethyl)-1,3-thiazol-2-yl | |
| 3.672 | 3-F-Ph | H | O | H | CHCH₃ | 4-(Trifluormethyl)-1,3-thiazol-2-yl | |
| 3.673 | 3,5-Cl₂-Ph | H | O | H | CHCH₃ | 4-(Trifluormethyl)-1,3-thiazol-2-yl | |
| 3.674 | 3,5-F₂-Ph | H | O | H | CHCH₃ | 4-Chlor-1-methyl-1H-pyrazol-3-yl | |
| 3.675 | 3,5-Cl₂-Ph | H | O | H | CHCH₃ | 4-Chlor-1-methyl-1H-pyrazol-3-yl | |
| 3.676 | 3-F-Ph | H | O | H | CHCH₃ | 4-Chlor-1-methyl-1H-pyrazol-3-yl | |
| 3.677 | 3,5-F₂-Ph | H | O | H | CHCH₃ | 4-Chlor-1-methyl-1H-pyrazol-5-yl | |
| 3.678 | 3,5-F₂-Ph | H | O | H | CHCH₃ | 5-Chlor-1-methyl-1H-pyrazol-4-yl | |
| 3.679 | 3-F-Ph | H | O | H | CHCH₃ | 5-Chlor-1-methyl-1H-pyrazol-4-yl | |
| 3.680 | 3,5-F₂-Ph | H | S | H | CHCH₃ | 5-Chlor-1-methyl-1H-pyrazol-4-yl | |
| 3.681 | 3,5-Cl₂-Ph | H | O | H | CHCH₃ | 5-Chlor-1-methyl-1H-pyrazol-4-yl | |
| 3.682 | Ph | H | O | H | CHCH₃ | 5-Chlor-1-methyl-1H-pyrazol-4-yl | |
| 3.683 | 3,5-F₂-Ph | H | O | H | CHCH₃ | 5-Methoxy-1,3-dimethyl-1H-pyrazol-4-yl | |
| 3.684 | 3,5-Cl₂-Ph | H | O | H | CHCH₃ | 5-Methoxy-1,3-dimethyl-1H-pyrazol-4-yl | |
| 3.685 | 3-F-Ph | H | O | H | CHCH₃ | 5-Methoxy-1,3-dimethyl-1H-pyrazol-4-yl | |
| 3.686 | 3,5-Cl₂-Ph | H | O | H | CHCH₃ | 5-Methyl-1,2,4-oxadiazol-3-yl | |
| 3.687 | 3,5-F₂-Ph | H | O | H | CHCH₃ | 5-Methyl-1,2,4-oxadiazol-3-yl | |
| 3.688 | 3,5-Cl₂-Ph | H | O | H | CHCH₃ | 5-Methyl-1,2-oxazol-3-yl | |
| 3.689 | 3-F-Ph | H | O | H | CHCH₃ | 5-Methyl-1-propyl-1H-pyrazol-4-yl | |
| 3.690 | 3,5-F₂-Ph | H | O | H | CHCH₃ | 5-Methyl-1-propyl-1H-pyrazol-4-yl | |
| 3.691 | 3,5-Cl₂-Ph | H | O | H | CHCH₃ | 5-Methyl-1-propyl-1H-pyrazol-4-yl | |
| 3.692 | Ph | H | O | H | CHCH₃ | 5-Methyl-1-propyl-1H-pyrazol-4-yl | |
| 3.693 | 3,5-F₂-Ph | H | O | H | CHCH₃ | 6-Chlorpyridin-3-yl | |
| 3.694 | 3,5-F₂-Ph | H | O | H | CHCH₃ | Furan-2-yl | |
| 3.695 | 3,5-Cl₂-Ph | H | O | H | CHCH₃ | Pyridin-2-yl | |
| 3.696 | 3,5-F₂-Ph | H | O | H | CHCH₃ | Pyridin-2-yl | |
| 3.697 | 3,5-F₂-Ph | H | O | H | CHCH₃ | Pyridin-3-yl | |
| 3.698 | 3-F-Ph | H | O | H | CHCH₃ | Pyridin-3-yl | |
| 3.699 | 3,5-F₂-Ph | H | O | H | CHCH₃ | Pyridin-4-yl | |
| 3.700 | 3-F-Ph | H | O | H | CHCH₃ | Pyridin-4-yl | |
| 3.701 | 3,5-Cl₂-Ph | H | O | H | CHCH₃ | Pyrimidin-2-yl | |

| Tabelle 4: Erfindungsgemäße Verbindungen der allgemeinen Formel (I), worin R² für Wasserstoff und R³ für Cyano stehen, und Aryl den Rest 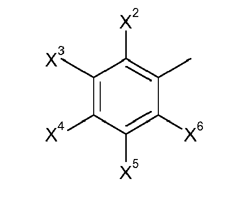 bedeutet. | | | | | | | |
|---|---|---|---|---|---|---|---|
| 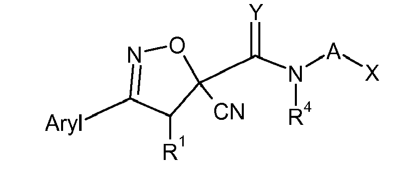 | | | | | | | |

| Nr. | Aryl | R¹ | Y | R⁴ | A | X | Physikalische Daten |
|---|---|---|---|---|---|---|---|
| 4.001 | 3,5-(CF₃)₂-Ph | H | O | H | Bindung | 1,3-Dimethyl-1H-pyrazol-5-yl | |
| 4.002 | 3,5-Cl₂-Ph | H | O | H | Bindung | 1,3-Dimethyl-1H-pyrazol-5-yl | |
| 4.003 | 3,5-Cl₂-Ph | H | O | H | Bindung | 1,5-Dimethyl-1H-pyrazol-4-yl | |
| 4.004 | 3,5-F₂-Ph | H | O | H | Bindung | 1,5-Dimethyl-1H-pyrazol-4-yl | |
| 4.005 | Ph | H | O | H | Bindung | 1,5-Dimethyl-1H-pyrazol-4-yl | |
| 4.006 | 3-F-Ph | H | O | H | Bindung | 1,5-Dimethyl-1H-pyrazol-4-yl | |
| 4.007 | 3-Me-Ph | H | O | H | Bindung | 1,5-Dimethyl-1H-pyrazol-4-yl | |
| 4.008 | 3,5-F₂-Ph | H | O | H | Bindung | 1-Ethyl-3-(methoxycarbonyl )-1H-pyrazol-4-yl | |
| 4.009 | 3,5-Cl₂-Ph | H | O | H | Bindung | 1-Ethyl-3-(methoxycarbonyl )-1H-pyrazol-4-yl | |
| 4.010 | 3-F-Ph | H | O | H | Bindung | 1-Ethyl-3-(methoxycarbonyl )-1H-pyrazol-4-yl | |
| 4.011 | 3,5-F₂-Ph | H | O | H | Bindung | 1-Ethyl-3-methyl-1H-pyrazol-4-yl | |
| 4.012 | 3-F-Ph | H | O | H | Bindung | 1-Ethyl-3-methyl-1H-pyrazol-4-yl | |
| 4.013 | 3,5-Cl₂-Ph | H | O | H | Bindung | 1-Methyl-1H-1,2,4-triazol-3-yl | |
| 4.014 | 3,5-Cl₂-Ph | H | O | H | Bindung | 1-Methyl-1H-imidazol-2-yl | |
| 4.015 | 3,5-F₂-Ph | H | O | H | Bindung | 1-Methyl-1H-pyrazol-4-yl | |
| 4.016 | 3,5-Cl₂-Ph | H | O | H | Bindung | 1-Methyl-1H-pyrazol-4-yl | |
| 4.017 | 3-F-Ph | H | O | H | Bindung | 1-Methyl-1H-pyrazol-4-yl | |
| 4.018 | Ph | H | O | H | Bindung | 1-Methyl-1H-pyrazol-4-yl | |
| 4.019 | 3,5-Cl₂-Ph | H | O | H | Bindung | 1-Propyl-1H-1,2,4-triazol-3-yl | |
| 4.020 | 3,5-F₂-Ph | H | O | H | Bindung | 1-Propyl-1H-1,2,4-triazol-3-yl | |
| 4.021 | 3,5-Cl₂-Ph | H | O | H | Bindung | 2,4-Difluorphenyl | |
| 4.022 | 3,5-Cl₂-Ph | H | O | H | Bindung | 2-Chlorpyridin-3-yl | |
| 4.023 | 3,5-Cl₂-Ph | H | O | H | Bindung | 3-(2-Methoxy-2-oxoethyl)phenyl | |
| 4.024 | 3,5-Cl₂-Ph | H | O | H | Bindung | 3-(Carboxymethyl)phenyl | |
| 4.025 | 3,5-Cl₂-Ph | H | O | H | Bindung | 3-(Methoxycarbonyl)phenyl | |
| 4.026 | 3-F-Ph | H | O | H | Bindung | 3-(Methoxycarbonyl)phenyl | |
| 4.027 | 3,5-Cl₂-Ph | H | O | H | Bindung | 3,5-Dimethoxyphenyl | |
| 4.028 | Ph | H | O | H | Bindung | 3,5-Dimethyl-1H-pyrazol-4-yl | |
| 4.029 | 3,5-Cl₂-Ph | H | O | H | Bindung | 3,5-Dimethyl-1H-pyrazol-4-yl | |
| 4.030 | 3,5-Cl₂-Ph | H | O | H | Bindung | 3-[2-(Methylamino)-2-oxoethyl]phenyl | |
| 4.031 | 3,5-Cl₂-Ph | H | O | H | Bindung | 3-Carboxyphenyl | |
| 4.032 | 3,5-Cl₂-Ph | H | O | H | Bindung | 3-Chlorpyridin-2-yl | |
| 4.033 | 3,5-Cl₂-Ph | H | O | H | Bindung | 3-Methyl-1,2-oxazol-5-yl | |
| 4.034 | 3,5-Cl₂-Ph | H | O | H | Bindung | 3-Methyl-1-propyl-1H-pyrazol-4-yl | |
| 4.035 | 3,5-Cl₂-Ph | H | O | H | Bindung | 4-(2-Ethoxy-2-oxoethyl)phenyl | |
| 4.036 | 3,5-Cl₂-Ph | H | O | H | Bindung | 4-(Carboxymethyl)phenyl | |
| 4.037 | 3,5-F₂-Ph | H | O | H | Bindung | 4-(Methoxycarbonyl)-3-thienyl | |
| 4.038 | 3,5-Cl₂-Ph | H | O | H | Bindung | 4-(Methoxycarbonyl)phenyl | |
| 4.039 | 3,5-Cl₂-Ph | H | O | H | Bindung | 4,5-Dimethyl-4H-1,2,4-triazol-3-yl | |
| 4.040 | 3,5-Cl₂-Ph | H | O | H | Bindung | 4-Carboxyphenyl | |
| 4.041 | 3,5-Cl₂-Ph | H | O | H | Bindung | 4-Chlor-2-(methoxycarbonyl)-6-methylphenyl | |
| 4.042 | 3,5-F₂-Ph | H | O | H | Bindung | 4-Cyano-1-ethyl-1H-pyrazol-3-yl | |
| 4.043 | 3,5-F₂-Ph | H | O | H | Bindung | 4-Formyl-3-(1-methylc-Pr)-1,2-oxazol-5-yl | |
| 4.044 | 3,5-Cl₂-Ph | H | O | H | Bindung | 4-Methyl-1,2,5-oxadiazol-3-yl | |
| 4.045 | 3,5-F₂-Ph | H | O | H | Bindung | 4-Methyl-1,3-thiazol-2-yl | |
| 4.046 | 3,5-Cl₂-Ph | H | O | H | Bindung | 5-(Methoxycarbonyl)-1,3-thiazol-4-yl | |
| 4.047 | 3,5-F₂-Ph | H | O | H | Bindung | 5-(Methoxycarbonyl)-1,3-thiazol-4-yl | |
| 4.048 | 3,5-Cl₂-Ph | H | O | H | Bindung | 5-Methoxy-1-methyl-1H-1,2,4-triazol-3-yl | |
| 4.049 | 3,5-F₂-Ph | H | O | H | Bindung | 5-Methoxy-1-methyl-1H-1,2,4-triazol-3-yl | |
| 4.050 | 3,5-Cl₂-Ph | H | O | H | Bindung | 6-Chlorpyridin-3-yl | |
| 4.051 | 3,5-Cl₂-Ph | H | O | H | Bindung | Pyridin-4-yl | |
| 4.052 | 3,5-F₂-Ph | H | O | H | CH₂ | 1-(2,2,2-Trifluorethyl)-1H-pyrazol-4-yl | |
| 4.053 | 3,5-F₂-Ph | H | O | H | CH₂ | 1-(2-Chlorethyl)-1H-pyrazol-4-yl | |
| 4.054 | 3,5-F₂-Ph | H | O | H | CH₂ | 1,3-Dimethyl-1H-pyrazol-4-yl | |
| 4.055 | 3-F-Ph | H | O | H | CH₂ | 1,3-Dimethyl-1H-pyrazol-4-yl | |
| 4.056 | 3,5-F₂-Ph | H | O | H | CH₂ | 1,3-Dimethyl-1H-pyrazol-5-yl | |
| 4.057 | 3,5-F₂-Ph | H | O | H | CH₂ | 1,5-Dimethyl-1H-pyrazol-4-yl | |
| 4.058 | 3,5-F₂-Ph | H | O | H | CH₂ | 1-Butyl-3-methyl-1H-pyrazol-4-yl | |
| 4.059 | 3,5-F₂-Ph | H | O | H | CH₂ | 1-Butyl-5-methyl-1H-pyrazol-4-yl | |
| 4.060 | 3,5-Cl₂-Ph | H | O | H | CH₂ | 1-c-Pr-1H-pyrazol-4-yl | |
| 4.061 | 3,5-F₂-Ph | H | O | H | CH₂ | 1-c-Pr-1H-pyrazol-4-yl | |
| 4.062 | 3-F-Ph | H | O | H | CH₂ | 1-c-Pr-1H-pyrazol-4-yl | |
| 4.063 | Ph | H | O | H | CH₂ | 1-c-Pr-1H-pyrazol-4-yl | |
| 4.064 | 3,5-Cl₂-Ph | H | O | H | CH₂ | 1-Ethyl-1H-pyrazol-4-yl | |
| 4.065 | 3,5-F₂-Ph | H | O | H | CH₂ | 1-Ethyl-1 H-pyrazol-4-yl | [CDCl3] 1.48 (t,3H); 4.05 (AB,2H); 4.16 (q,2H); 4.32 (dd,1H); 4.43 (dd,1H); 6.88 (s,br,1H); 6.96 (m,1H); 7.16 (m,2H); 7.41 (s,1H); 7.45 (s,1H). |
| 4.066 | 3-Br-5-CF₃-Ph | H | O | H | CH₂ | 1-Ethyl-1H-pyrazol-4-yl | |
| 4.067 | 3-F-5-MeO-Ph | H | O | H | CH₂ | 1-Ethyl-1H-pyrazol-4-yl | |
| 4.068 | 3-F-Ph | H | O | H | CH₂ | 1-Ethyl-1H-pyrazol-4-yl | |
| 4.069 | 3-Me-Ph | H | O | H | CH₂ | 1-Ethyl-1H-pyrazol-4-yl | |
| 4.070 | 3,5-F₂-Ph | H | O | H | CH₂ | 1-Ethyl-1H-pyrazol-5-yl | |
| 4.071 | 3,4,5-F₃-Ph | H | O | H | CH₂ | 1-Ethyl-3-methyl-1H-pyrazol-4-yl | |
| 4.072 | 3,4-F₂-Ph | H | O | H | CH₂ | 1-Ethyl-3-methyl-1H-pyrazol-4-yl | |
| 4.073 | 3,5-Cl₂-Ph | H | O | H | CH₂ | 1-Ethyl-3-methyl-1H-pyrazol-4-yl | [CDCl₃] 7.52 5.51;7.51 8.63;7.50 2.81;7.49 2.71;7.49 1.24;7.31 3.77;7.26 20.34;6.74 0.60;5.30 2.35;4.42 0.82;4.40 0.82;4.38 1.45;4.36 1.46;4.29 1.48;4.28 1.53;4.25 0.86;4.24 0.84;4.13 2.06;4.10 1.28;4.09 4.27;4.08 4.18;4.06 4.01;4.05 1.35;4.01 3.82; 3.96 1.99; 2.22 16.00;2.04 0.35;1.57 10.25;1.47 4.98;1.46 10.40;1.44 4.91;1.26 0.46;0.01 0.39;0.00 13.40;-0.01 0.47;-0.01 0.60 |
| 4.074 | 3,5-F₂-Ph | H | O | H | CH₂ | 1-Ethyl-3-methyl-1H-pyrazol-4-yl | [CDCl₃] 7.31 3.63;7.26 12.71;7.19 0.34;7.18 1.53;7.17 2.09;7.17 1.29;7.16 1.17;7.16 1.93;7.15 1.72;6.99 0.40;6.99 0.70;6.98 0.38;6.97 0.82;6.96 1.44;6.96 0.72;6.95 0.43;6.94 0.74;6.94 0.37;6.76 0.52;4.42 0.87;4.40 0.85;4.38 1.57;4.37 1.54;4.29 1.52;4.28 1.54;4.26 0.87;4.24 0.84;4.13 1.95;4.10 1.32;4.09 0.47;4.08 6.27;4.08 0.83;4.06 4.05;4.05 1.34;4.01 3.93;3.96 1.91;2.28 0.46;2.25 1.37;2.21 16.00;1.61 0.39;1.60 0.43;1.47 4.84;1.47 0.50;1.45 9.90;1.44 4.80;1.43 2.00;1.41 0.84;1.39 0.42;1.26 0.91;0.00 6.04 |
| 4.075 | 3,5-F₂-Ph | H | S | H | CH₂ | 1-Ethyl-3-methyl-1H-pyrazol-4-yl | |
| 4.076 | 3-Br-5-F-Ph | H | O | H | CH₂ | 1-Ethyl-3-methyl-1H-pyrazol-4-yl | |
| 4.077 | 3-Cl-5-CN-Ph | H | O | H | CH₂ | 1-Ethyl-3-methyl-1H-pyrazol-4-yl | |
| 4.078 | 3-Cl-5-Me-Ph | H | O | H | CH₂ | 1-Ethyl-3-methyl-1H-pyrazol-4-yl | |
| 4.079 | 3-Cl-5-CF₃O-Ph | H | O | H | CH₂ | 1-Ethyl-3-methyl-1H-pyrazol-4-yl | |
| 4.080 | 3-cPr-5-F-Ph | H | O | H | CH₂ | 1-Ethyl-3-methyl-1H-pyrazol-4-yl | |
| 4.081 | 3-F-5-Me-Ph | H | O | H | CH₂ | 1-Ethyl-3-methyl-1H-pyrazol-4-yl | |
| 4.082 | 3-F-Ph | H | O | H | CH₂ | 1-Ethyl-3-methyl-1H-pyrazol-4-yl | |
| 4.083 | 3-Me-Ph | H | O | H | CH₂ | 1-Ethyl-3-methyl-1H-pyrazol-4-yl | |
| 4.084 | 3,5-F₂-Ph | H | O | H | CH₂ | 1-Ethyl-3-methyl-1H-pyrazol-5-yl | |
| 4.085 | 3,5-F₂-Ph | H | O | H | CH₂ | 1-Isobutyl-1H-pyrazol-4-yl | |
| 4.086 | 3,5-F₂-Ph | H | O | H | CH₂ | 1-Isopropyl-1H-pyrazol-4-yl | |
| 4.087 | 3-F-Ph | H | O | H | CH₂ | 1-Isopropyl-1H-pyrazol-4-yl | |
| 4.088 | 3-Me-Ph | H | O | H | CH₂ | 1-Methyl-1H-pyrazol-4-yl | |
| 4.089 | 3,5-F₂-Ph | H | O | H | CH₂ | 1-Methyl-1H-pyrazol-5-yl | |
| 4.090 | 3-F-Ph | H | O | H | CH₂ | 1-Methyl-1H-pyrazol-5-yl | |
| 4.091 | 3,5-F₂-Ph | H | O | H | CH₂ | 1-Propyl-1H-pyrazol-4-yl | |
| 4.092 | 3,5-F₂-Ph | H | O | H | CH₂ | 1-Tert-butyl-1H-pyrazol-4-yl | |
| 4.093 | 3,5-F₂-Ph | H | O | H | CH₂ | 2-Chlorpyridin-4-yl | [CDCl3] 4.09 (AB,2H); 4.51 (dd,1H); 4.47 (dd,1H); 7.00 (m,1H); 7.13 (m,2H); 7.19 (m,4H); 8.39 (d,1H). |
| 4.094 | 3-F-Ph | H | O | H | CH₂ | 2-Chlorpyridin-4-yl | |
| 4.095 | 3,5-F₂-Ph | H | O | H | CH₂ | 3-Chlor-1-ethyl-1H-pyrazol-4-yl | |
| 4.096 | 3-F-Ph | H | O | H | CH₂ | 3-Chlor-1-ethyl-1H-pyrazol-4-yl | |
| 4.097 | 3,5-F₂-Ph | H | O | H | CH₂ | 3-Chlor-1-isopropyl-1H-pyrazol-4-yl | |
| 4.098 | 3,5-F₂-Ph | H | O | H | CH₂ | 3-Methyl-1-propyl-1H-pyrazol-4-yl | |
| 4.099 | 3-Cl-5-CF₃O-Ph | H | O | H | CH₂ | 3-Methyl-1-propyl-1H-pyrazol-4-yl | |
| 4.100 | 3-CI-5-F-Ph | H | O | H | CH₂ | 3-Methyl-1-propyl-1H-pyrazol-4-yl | |
| 4.101 | 3-F-5-Me-Ph | H | O | H | CH₂ | 3-Methyl-1-propyl-1H-pyrazol-4-yl | |
| 4.102 | 3-F-Ph | H | O | H | CH₂ | 3-Methyl-1-propyl-1H-pyrazol-4-yl | |
| 4.103 | 3-Me-Ph | H | O | H | CH₂ | 3-Methyl-1-propyl-1H-pyrazol-4-yl | |
| 4.104 | 3-F-Ph | H | O | H | CH₂ | 5-Methyl-1-propyl-1H-pyrazol-4-yl | |
| 4.105 | 3,5-F₂-Ph | H | O | H | CH₂CH₂ | 1-(2,2,2-Trifluorethyl)-1H-pyrazol-4-yl | |
| 4.106 | 3,5-F₂-Ph | H | O | H | CH₂CH₂ | 1-(2-Chlorethyl)-1H-pyrazol-4-yl | |
| 4.107 | 3,5-F₂-Ph | H | O | H | CH₂CH₂ | 1,3-Dimethyl-1H-pyrazol-4-yl | |
| 4.108 | 3-F-Ph | H | O | H | CH₂CH₂ | 1,3-Dimethyl-1H-pyrazol-4-yl | |
| 4.109 | 3,5-F₂-Ph | H | O | H | CH₂CH₂ | 1,3-Dimethyl-1H-pyrazol-5-yl | |
| 4.110 | 3,5-F₂-Ph | H | O | H | CH₂CH₂ | 1,5-Dimethyl-1H-pyrazol-4-yl | |
| 4.111 | 3,5-F₂-Ph | H | O | H | CH₂CH₂ | 1-Butyl-3-methyl-1H-pyrazol-4-yl | |
| 4.112 | 3,5-F₂-Ph | H | O | H | CH₂CH₂ | 1-Butyl-5-methyl-1H-pyrazol-4-yl | |
| 4.113 | 3,5-F₂-Ph | H | O | H | CH₂CH₂ | 1-c-Pr-1H-pyrazol-4-yl | |
| 4.114 | Ph | H | O | H | CH₂CH₂ | 1-c-Pr-1H-pyrazol-4-yl | |
| 4.115 | 3-F-Ph | H | O | H | CH₂CH₂ | 1-c-Pr-1H-pyrazol-4-yl | |
| 4.116 | 3,5-Cl₂-Ph | H | O | H | CH₂CH₂ | 1-c-Pr-1H-pyrazol-4-yl | |
| 4.117 | 3,5-Cl₂-Ph | H | O | H | CH₂CH₂ | 1-Ethyl-1H-pyrazol-4-yl | |
| 4.118 | 3-F-Ph | H | O | H | CH₂CH₂ | 1-Ethyl-1H-pyrazol-4-yl | |
| 4.119 | 3,5-F₂-Ph | H | O | H | CH₂CH₂ | 1-Ethyl-1H-pyrazol-4-yl | |
| 4.120 | 3-Br-5-CF₃-Ph | H | O | H | CH₂CH₂ | 1-Ethyl-1H-pyrazol-4-yl | |
| 4.121 | 3-Me-Ph | H | O | H | CH₂CH₂ | 1-Ethyl-1H-pyrazol-4-yl | |
| 4.122 | 3-F-5-MeO-Ph | H | O | H | CH₂CH₂ | 1-Ethyl-1H-pyrazol-4-yl | |
| 4.123 | 3,5-F₂-Ph | H | O | H | CH₂CH₂ | 1-Ethyl-1H-pyrazol-5-yl | |
| 4.124 | 3,5-F₂-Ph | H | O | H | CH₂CH₂ | 1-Ethyl-3-methyl-1H-pyrazol-4-yl | |
| 4.125 | 3-Me-Ph | H | O | H | CH₂CH₂ | 1-Ethyl-3-methyl-1H-pyrazol-4-yl | |
| 4.126 | 3-Br-5-F-Ph | H | O | H | CH₂CH₂ | 1-Ethyl-3-methyl-1H-pyrazol-4-yl | |
| 4.127 | 3,4,5-F₃-Ph | H | O | H | CH₂CH₂ | 1-Ethyl-3-methyl-1H-pyrazol-4-yl | |
| 4.128 | 3,4-F₂-Ph | H | O | H | CH₂CH₂ | 1-Ethyl-3-methyl-1H-pyrazol-4-yl | |
| 4.129 | 3,5-F₂-Ph | H | S | H | CH₂CH₂ | 1-Ethyl-3-methyl-1H-pyrazol-4-yl | |
| 4.130 | 3-F-5-Me-Ph | H | O | H | CH₂CH₂ | 1-Ethyl-3-methyl-1H-pyrazol-4-yl | |
| 4.131 | 3-Cl-5-CN-Ph | H | O | H | CH₂CH₂ | 1-Ethyl-3-methyl-1H-pyrazol-4-yl | |
| 4.132 | 3-F-Ph | H | O | H | CH₂CH₂ | 1-Ethyl-3-methyl-1H-pyrazol-4-yl | |
| 4.133 | 3-cPr-5-F-Ph | H | O | H | CH₂CH₂ | 1-Ethyl-3-methyl-1H-pyrazol-4-yl | |
| 4.134 | 3-Cl-5-Me-Ph | H | O | H | CH₂CH₂ | 1-Ethyl-3-methyl-1H-pyrazol-4-yl | |
| 4.135 | 3-Cl-5-CF₃O-Ph | H | O | H | CH₂CH₂ | 1-Ethyl-3-methyl-1H-pyrazol-4-yl | |
| 4.136 | 3,5-F₂-Ph | H | O | H | CH₂CH₂ | 1-Ethyl-3-methyl-1H-pyrazol-5-yl | |
| 4.137 | 3,5-F₂-Ph | H | O | H | CH₂CH₂ | 1-Isobutyl-1H-pyrazol-4-yl | |
| 4.138 | 3,5-F₂-Ph | H | O | H | CH₂CH₂ | 1-Isopropyl-1H-pyrazol-4-yl | |
| 4.139 | 3-F-Ph | H | O | H | CH₂CH₂ | 1-Isopropyl-1H-pyrazol-4-yl | |
| 4.140 | 3-Me-Ph | H | O | H | CH₂CH₂ | 1-Methyl-1H-pyrazol-4-yl | |
| 4.141 | 3,5-F₂-Ph | H | O | H | CH₂CH₂ | 1-Methyl-1H-pyrazol-5-yl | |
| 4.142 | 3-F-Ph | H | O | H | CH₂CH₂ | 1-Methyl-1H-pyrazol-5-yl | |
| 4.143 | 3,5-F₂-Ph | H | O | H | CH₂CH₂ | 1-Propyl-1H-pyrazol-4-yl | |
| 4.144 | 3,5-F₂-Ph | H | O | H | CH₂CH₂ | 1-Tert-butyl-1H-pyrazol-4-yl | |
| 4.145 | 3,5-F₂-Ph | H | O | H | CH₂CH₂ | 2-Chlorpyridin-4-yl | |
| 4.146 | 3-F-Ph | H | O | H | CH₂CH₂ | 2-Chlorpyridin-4-yl | |
| 4.147 | 3,5-F₂-Ph | H | O | H | CH₂CH₂ | 3-Chlor-1-ethyl-1H-pyrazol-4-yl | |
| 4.148 | 3-F-Ph | H | O | H | CH₂CH₂ | 3-Chlor-1-ethyl-1H-pyrazol-4-yl | |
| 4.149 | 3,5-F₂-Ph | H | O | H | CH₂CH₂ | 3-Chlor-1-isopropyl-1H-pyrazol-4-yl | |
| 4.150 | 3-F-Ph | H | O | H | CH₂CH₂ | 3-Methyl-1-propyl-1H-pyrazol-4-yl | |
| 4.151 | 3,5-F₂-Ph | H | O | H | CH₂CH₂ | 3-Methyl-1-propyl-1H-pyrazol-4-yl | |
| 4.152 | 3-Cl-5-F-Ph | H | O | H | CH₂CH₂ | 3-Methyl-1-propyl-1H-pyrazol-4-yl | |
| 4.153 | 3-Cl-5-CF₃O-Ph | H | O | H | CH₂CH₂ | 3-Methyl-1-propyl-1H-pyrazol-4-yl | |
| 4.154 | 3-F-5-Me-Ph | H | O | H | CH₂CH₂ | 3-Methyl-1-propyl-1H-pyrazol-4-yl | |
| 4.155 | 3-Me-Ph | H | O | H | CH₂CH₂ | 3-Methyl-1-propyl-1H-pyrazol-4-yl | |
| 4.156 | 3-F-Ph | H | O | H | CH₂CH₂ | 5-Methyl-1-propyl-1H-pyrazol-4-yl | |
| 4.157 | 3,5-F₂-Ph | H | O | H | CHCH₃ | 1-(2,2,2-Trifluorethyl)-1H-pyrazol-4-yl | |
| 4.158 | 3,5-F₂-Ph | H | O | H | CHCH₃ | 1-(2-Chlorethyl)-1H-pyrazol-4-yl | |
| 4.159 | 3,5-F₂-Ph | H | O | H | CHCH₃ | 1,3-Dimethyl-1H-pyrazol-4-yl | |
| 4.160 | 3-F-Ph | H | O | H | CHCH₃ | 1,3-Dimethyl-1H-pyrazol-4-yl | |
| 4.161 | 3,5-F₂-Ph | H | O | H | CHCH₃ | 1,3-Dimethyl-1H-pyrazol-5-yl | |
| 4.162 | 3,5-F₂-Ph | H | O | H | CHCH₃ | 1,5-Dimethyl-1H-pyrazol-4-yl | |
| 4.163 | 3,5-F₂-Ph | H | O | H | CHCH₃ | 1-Butyl-3-methyl-1H-pyrazol-4-yl | |
| 4.164 | 3,5-F₂-Ph | H | O | H | CHCH₃ | 1-Butyl-5-methyl-1H-pyrazol-4-yl | |
| 4.165 | 3,5-F₂-Ph | H | O | H | CHCH₃ | 1-c-Pr-1H-pyrazol-4-yl | |
| 4.166 | Ph | H | O | H | CHCH₃ | 1-c-Pr-1H-pyrazol-4-yl | |
| 4.167 | 3-F-Ph | H | O | H | CHCH₃ | 1-c-Pr-1H-pyrazol-4-yl | |
| 4.168 | 3,5-Cl₂-Ph | H | O | H | CHCH₃ | 1-c-Pr-1H-pyrazol-4-yl | |
| 4.169 | 3,5-Cl₂-Ph | H | O | H | CHCH₃ | 1-Ethyl-1H-pyrazol-4-yl | |
| 4.170 | 3-F-Ph | H | O | H | CHCH₃ | 1-Ethyl-1H-pyrazol-4-yl | |
| 4.171 | 3,5-F₂-Ph | H | O | H | CHCH₃ | 1-Ethyl-1H-pyrazol-4-yl | |
| 4.172 | 3-Br-5-CF₃-Ph | H | O | H | CHCH₃ | 1-Ethyl-1H-pyrazol-4-yl | |
| 4.173 | 3-Me-Ph | H | O | H | CHCH₃ | 1-Ethyl-1H-pyrazol-4-yl | |
| 4.174 | 3-F-5-MeO-Ph | H | O | H | CHCH₃ | 1-Ethyl-1H-pyrazol-4-yl | |
| 4.175 | 3,5-F₂-Ph | H | O | H | CHCH₃ | 1-Ethyl-1H-pyrazol-5-yl | |
| 4.176 | 3,5-F₂-Ph | H | O | H | CHCH₃ | 1-Ethyl-3-methyl-1H-pyrazol-4-yl | |
| 4.177 | 3-Me-Ph | H | O | H | CHCH₃ | 1-Ethyl-3-methyl-1H-pyrazol-4-yl | |
| 4.178 | 3-Br-5-F-Ph | H | O | H | CHCH₃ | 1-Ethyl-3-methyl-1H-pyrazol-4-yl | |
| 4.179 | 3,4,5-F₃-Ph | H | O | H | CHCH₃ | 1-Ethyl-3-methyl-1H-pyrazol-4-yl | |
| 4.180 | 3,4-F₂-Ph | H | O | H | CHCH₃ | 1-Ethyl-3-methyl-1H-pyrazol-4-yl | |
| 4.181 | 3,5-F₂-Ph | H | S | H | CHCH₃ | 1-Ethyl-3-methyl-1H-pyrazol-4-yl | |
| 4.182 | 3-F-5-Me-Ph | H | O | H | CHCH₃ | 1-Ethyl-3-methyl-1H-pyrazol-4-yl | |
| 4.183 | 3-Cl-5-CN-Ph | H | O | H | CHCH₃ | 1-Ethyl-3-methyl-1H-pyrazol-4-yl | |
| 4.184 | 3-F-Ph | H | O | H | CHCH₃ | 1-Ethyl-3-methyl-1H-pyrazol-4-yl | |
| 4.185 | 3-cPr-5-F-Ph | H | O | H | CHCH₃ | 1-Ethyl-3-methyl-1H-pyrazol-4-yl | |
| 4.186 | 3-Cl-5-Me-Ph | H | O | H | CHCH₃ | 1-Ethyl-3-methyl-1H-pyrazol-4-yl | |
| 4.187 | 3-Cl-5-CF₃O-Ph | H | O | H | CHCH₃ | 1-Ethyl-3-methyl-1H-pyrazol-4-yl | |
| 4.188 | 3,5-F₂-Ph | H | O | H | CHCH₃ | 1-Ethyl-3-methyl-1H-pyrazol-5-yl | |
| 4.189 | 3,5-F₂-Ph | H | O | H | CHCH₃ | 1-Isobutyl-1H-pyrazol-4-yl | |
| 4.190 | 3,5-F₂-Ph | H | O | H | CHCH₃ | 1-Isopropyl-1H-pyrazol-4-yl | |
| 4.191 | 3-F-Ph | H | O | H | CHCH₃ | 1-Isopropyl-1H-pyrazol-4-yl | |
| 4.192 | 3-Me-Ph | H | O | H | CHCH₃ | 1-Methyl-1H-pyrazol-4-yl | |
| 4.193 | 3,5-F₂-Ph | H | O | H | CHCH₃ | 1-Methyl-1H-pyrazol-5-yl | |
| 4.194 | 3-F-Ph | H | O | H | CHCH₃ | 1-Methyl-1H-pyrazol-5-yl | |
| 4.195 | 3,5-F₂-Ph | H | O | H | CHCH₃ | 1-Propyl-1H-pyrazol-4-yl | |
| 4.196 | 3,5-F₂-Ph | H | O | H | CHCH₃ | 1-Tert-butyl-1H-pyrazol-4-yl | |
| 4.197 | 3,5-F₂-Ph | H | O | H | CHCH₃ | 2-Chlorpyridin-4-yl | |
| 4.198 | 3-F-Ph | H | O | H | CHCH₃ | 2-Chlorpyridin-4-yl | |
| 4.199 | 3,5-F₂-Ph | H | O | H | CHCH₃ | 3-Chlor-1-ethyl-1H-pyrazol-4-yl | |
| 4.200 | 3-F-Ph | H | O | H | CHCH₃ | 3-Chlor-1-ethyl-1H-pyrazol-4-yl | |
| 4.201 | 3,5-F₂-Ph | H | O | H | CHCH₃ | 3-Chlor-1-isopropyl-1H-pyrazol-4-yl | |
| 4.202 | 3-F-Ph | H | O | H | CHCH₃ | 3-Methyl-1-propyl-1H-pyrazol-4-yl | |
| 4.203 | 3,5-F₂-Ph | H | O | H | CHCH₃ | 3-Methyl-1-propyl-1H-pyrazol-4-yl | |
| 4.204 | 3-CI-5-F-Ph | H | O | H | CHCH₃ | 3-Methyl-1-propyl-1H-pyrazol-4-yl | |
| 4.205 | 3-Cl-5-CF₃O-Ph | H | O | H | CHCH₃ | 3-Methyl-1-propyl-1H-pyrazol-4-yl | |
| 4.206 | 3-F-5-Me-Ph | H | O | H | CHCH₃ | 3-Methyl-1-propyl-1H-pyrazol-4-yl | |
| 4.207 | 3-Me-Ph | H | O | H | CHCH₃ | 3-Methyl-1-propyl-1H-pyrazol-4-yl | |
| 4.208 | 3-F-Ph | H | O | H | CHCH₃ | 5-Methyl-1-propyl-1H-pyrazol-4-yl | |
| 4.209 | 3,5-F₂-Ph | H | O | H | CH₂ | 2,6-Dimethylpyridin-4-yl | |
| 4.210 | 3,5-F₂-Ph | H | O | H | CH₂ | 2-Chlorpyridin-3-yl | |
| 4.211 | 3,5-F₂-Ph | H | O | H | CH₂ | 2-Fluorpyridin-4-yl | [CDCl₃] 4.05 (d,1H); 4.16 (d,1H); 4.53 (dd,1H); 4.62 (dd,1H); 6.61 (s,1H); 6.98 (m,1H); 7.07 (d,1H); 7.17 (m,2H); 7.28 (s,br,1H); 8.23 (d,1H). |
| 4.212 | 3,5-F₂-Ph | H | O | H | CH₂ | 2-Methoxypyridin-4-yl | [CDCl₃] 3.92 (s,3H); 4.08 (AB,2H); 4.48 (AB d,2H); 6.59 (s,1H); 6.74 (d,1H); 6.98 (m,1H); 7.09 (t br,1H); 7.18 (m,2H); 8.14 (d,1H). |
| 4.213 | 3,5-F₂-Ph | H | O | H | CH₂ | 2-tert-Butylpyridin-4-yl | |
| 4.214 | 3,5-F₂-Ph | H | O | H | CH₂ | 3,5-Dimethyl-1,2-oxazol-4-yl | |
| 4.215 | 3,5-F₂-Ph | H | O | H | CH₂ | 6-Chlorpyridin-3-yl | |

| Tabelle 5: Erfindungsgemäße Verbindungen der allgemeinen Formel (I), worin R² für Wasserstoff steht, und Aryl den Rest 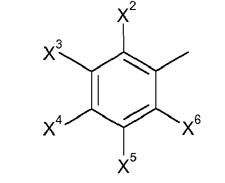 bedeutet. | | | | | | | | |
|---|---|---|---|---|---|---|---|---|
| 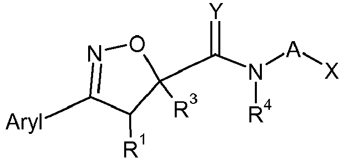 | | | | | | | | |

| Nr. | Aryl | R¹ | R³ | Y | R⁴ | A | X | Physikalische Daten |
|---|---|---|---|---|---|---|---|---|
| 5.001 | Ph | H | C(OH)CH(CH₃)₂ | O | H | CH₂ | 1-Ethyl-3-methyl-1H-pyrazol-4-yl | |
| 5.002 | 2,4-Cl₂-Ph | H | C(OH)CH(CH₃)₂ | O | H | CH₂ | 1-Ethyl-3-methyl-1H-pyrazol-4-yl | |
| 5.003 | 4-CI-Ph | H | C(OH)CH(CH₃)₂ | O | H | CH₂ | 1-Ethyl-3-methyl-1H-pyrazol-4-yl | |
| 5.004 | 3-CI-Ph | H | CH(CH₃)OH | O | H | CH₂ | 1-Ethyl-3-methyl-1H-pyrazol-4-yl | |
| 5.005 | 2,4-Cl₂-Ph | H | CH(CH₃)OH | O | H | CH₂ | 1-Ethyl-3-methyl-1H-pyrazol-4-yl | |
| 5.006 | Ph | H | CH(CH₃)OH | O | H | CH₂ | 1-Ethyl-3-methyl-1H-pyrazol-4-yl | |
| 5.007 | 2-CF₃-Ph | H | CH(CH₃)OH | O | H | CH₂ | 1-Ethyl-3-methyl-1H-pyrazol-4-yl | |
| 5.008 | 3,5-Cl₂-Ph | H | CH(CH₃)OH | O | H | CH₂ | 1-Ethyl-3-methyl-1H-pyrazol-4-yl | |
| 5.009 | 4-CI-Ph | H | CH(CH₃)OH | O | H | CH₂ | 1-Ethyl-3-methyl-1H-pyrazol-4-yl | |
| 5.010 | 3-CI-Ph | H | C(OCH₃)CH(C H₃)₂ | O | H | CH₂ | 1-Ethyl-3-methyl-1H-pyrazol-4-yl | |
| 5.011 | Ph | H | CH(CH₃)OCH₃ | O | H | CH₂ | 1-Ethyl-3-methyl-1H-pyrazol-4-yl | |
| 5.012 | 3-CI-Ph | H | CH(CH₃)OCH₃ | O | H | CH₂ | 1-Ethyl-3-methyl-1H-pyrazol-4-yl | |
| 5.013 | 4-CI-Ph | H | CH(CH₃)OCH₃ | O | H | CH₂ | 1-Ethyl-3-methyl-1H-pyrazol-4-yl | |
| 5.014 | 3,5-Cl₂-Ph | H | CH(CH₃)OCH₃ | O | H | CH₂ | 1-Ethyl-3-methyl-1H-pyrazol-4-yl | |
| 5.015 | 2-CF₃-Ph | H | CH(CH₃)OCH₃ | O | H | CH₂ | 1-Ethyl-3-methyl-1H-pyrazol-4-yl | |
| 5.016 | 2,4-Cl₂-Ph | H | CH(CH₃)OCH₃ | O | H | CH₂ | 1-Ethyl-3-methyl-1H-pyrazol-4-yl | |
| 5.017 | 3,5-Cl₂-Ph | H | (CH₂)₂OCH₃ | O | H | CH₂ | 1-Ethyl-3-methyl-1H-pyrazol-4-yl | [CDCl₃] 1.42 (t, 3H); 2.20 (s, 3H); 2.25 (m, 1H); 2.35 (m, 1H); 3.26 (s, 3H); 3.47 (d,1 H); 3.52 (m, 2H); 3.70 (d, 1H); 4.06 (q, 2H); 4.18 (dd, 1H); 4.31 (dd, 1H); 6.85 (t br, 1H); 7.43 (s, 1H); 7.52 (s, 2H). |
| 5.018 | 3,5-Cl₂-Ph | H | cPr | O | H | CH₂ | 1-Ethyl-3-methyl-1H-pyrazol-4-yl | [CDCl₃] 0.55 (m, 4H); 1.44 (t, 3H); 1.57 (m, 1 H), 2.21 (s, 3H); 3.35 (d, 1 H); 3.82 (d, 1H); 4.08 (q, 2H); 4.18 (dd, 1H); 4.35 (dd, 1 H); 6.74 (t br, 1 H); 7.42 (s, 1H); 7.50 (s, 2H) |
| 5.019 | 3,5-Cl₂-Ph | H | CH₂OH | O | H | CH₂ | 1-Ethyl-3-methyl-1H-pyrazol-4-yl | |
| 5.020 | 3-CI-Ph | H | CH₂OH | O | H | CH₂ | 1-Ethyl-3-methyl-1H-pyrazol-4-yl | |
| 5.021 | 4-CI-Ph | H | CH₂OH | O | H | CH₂ | 1-Ethyl-3-methyl-1H-pyrazol-4-yl | |
| 5.022 | Ph | H | CH₂OH | O | H | CH₂ | 1-Ethyl-3-methyl-1H-pyrazol-4-yl | |
| 5.023 | 2-CF₃-Ph | H | CH₂OH | O | H | CH₂ | 1-Ethyl-3-methyl-1H-pyrazol-4-yl | |
| 5.024 | 2,4-Cl₂-Ph | H | CH₂OH | O | H | CH₂ | 1-Ethyl-3-methyl-1H-pyrazol-4-yl | |
| 5.025 | 4-CI-Ph | H | CH₂OCH₃ | O | H | CH₂ | 1-Ethyl-3-methyl-1H-pyrazol-4-yl | |
| 5.026 | 2,4-Cl₂-Ph | H | CH₂OCH₃ | O | H | CH₂ | 1-Ethyl-3-methyl-1H-pyrazol-4-yl | |
| 5.027 | Ph | H | CH₂OCH₃ | O | H | CH₂ | 1-Ethyl-3-methyl-1H-pyrazol-4-yl | |
| 5.028 | 3-CI-Ph | H | CH₂OCH₃ | O | H | CH₂ | 1-Ethyl-3-methyl-1H-pyrazol-4-yl | |
| 5.029 | 3,5-Cl₂-Ph | H | CH₂OCH₃ | O | H | CH₂ | 1-Ethyl-3-methyl-1H-pyrazol-4-yl | |
| 5.030 | 3,5-Cl₂-Ph | H | iPr | O | H | CH₂ | 1-Ethyl-3-methyl-1H-pyrazol-4-yl | [CDCl₃] 1.00 (t, 3H); 1.44 (t, 3H); 2.20 (s, 3H); 2.32 (m, 1H); 3.30 (d, 1H), 3.66 (d, 1H); 4.06 (q, 2H); 4.17 (dd, 1H); 4.32 (dd, 1H); 6.84 (t br, 1H); 7.21 (m, 1H); 7.52 (m, 2H) |
| 5.031 | 3,5-F₂-Ph | H | CH₂F | O | H | CH₂ | 1-(2,2,2-Trifluorethyl)-1H-pyrazol-4-yl | [CDCl₃] 1.55 (s,3H); 3.43 (d,1H); 3.67 (d,1H); 4.37 (AB d,2H); 4.63 (d,0.5H); 4.65 (q,2H); 4.75 (m,1H); 4.87 (d,0.5H); 6.98 (m,1H); 7.13 (m,3H); 7.46 (s,1H); 7.51 (s,2H). |
| 5.032 | 3,5-F₂-Ph | H | F | O | H | CH₂ | 1-Ethyl-1H-pyrazol-4-yl | [CDCl₃] 1.49 (t,3H); 3.57 (dd,1H); 4.16 (q,2H); 4.21 (dd,1H); 4.42 (m,2H); 6.74 (s br,1H); 6.94 (m,1H); 7.22 (m,2H); 7.42 (s,1H); 7.48 (s,1H). |
| 5.033 | 3,5-Cl₂-Ph | H | F | O | H | CH₂ | 1-Ethyl-1H-pyrazol-4-yl | [CDCl₃] 1.49 (t,3H); 3.57 (dd,1H); 4.18 (q,2H); 4.20 (dd,1H); 4.42 (m,2H); 6.74 (s br,1H); 7.42 (s,1H); 7.47 (s,1H); 7.48 (s,1H); 7.57 (s,2H). |
| 5.034 | 3,5-F₂-Ph | H | CH₂F | O | H | CH₂ | 1-Ethyl-1H-pyrazol-4-yl | [CDCl₃] 1.47 (t,3H); 3.45 (d,1H); 3.68 (d,1H); 4.13 (q,2H); 2.26 (dd,1H); 4.40 (dd,1H); 4.64 |
| | | | | | | | | (d,0.5H);4.75 (m,1H); 4.86 (0.5H); 6.90 (m,1H); 7.05 (s br,1H); 7.18 (m,2H); 7.33 (s,1H); 7.42 (s,1H). |
| 5.035 | 3-F-Ph | H | CH₂F | O | H | CH₂ | 1-Ethyl-1H-pyrazol-4-yl | |
| 5.036 | 3,5-F₂-Ph | H | CH₂Cl | O | H | CH₂ | 1-Ethyl-1H-pyrazol-4-yl | [CDCl₃] 1.46 (t,3H); 3.55 (d,1H); 3.70 (d,1H); 3.94 (d,1H); 4.03 (d,1H); 4.13 (q,2H); 4.24 (dd,1H); 4.42 (dd,1H); 6.90 (m,1H); 7.05 (s br,1H); 7.16 (m,2H); 7.35 (s,1H); 7.42 (s,1H). |
| 5.037 | 3,5-Cl₂-Ph | H | CH₂Cl | O | H | CH₂ | 1-Ethyl-1H-pyrazol-4-yl | [CDCl₃] 1.47 (t,3H); 3.64 (AB,2H); 3.99 (AB,2H); 4.13 (q,2H); 4.34 (AB d,2H);7.04 (t br,1H); 7.36 (s,1H); 7.42 (s,1H); 7.43 (s,1H); 7.52 (s,2H). |
| 5.038 | 3,5-F₂-Ph | H | Vinyl | O | H | CH₂ | 1-Ethyl-1H-pyrazol-4-yl | [CDCl₃] 1.46 (t,3H); 3.32 (d,1H); 3.94 (d,1H); 4.13 (q,2H); 4.26 (dd,1H); 4.38 (dd,1H); 5.35 (d,1H); 5.53 (d,1H); 6.18 (dd,1H); 6.90 (m,1H); 6.94 (s br,1H); 7.18 (m,2H); 7.33 (s,1H); 7.41 (s,1H). |
| 5.039 | 3-F-Ph | H | CH₂Cl | O | H | CH₂ | 1-Ethyl-1H-pyrazol-4-yl | [CDCl₃] 1.46 (t,3H); 3.65 |
| | | | | | | | | (AB,2H); 4.01 (AB,2H); 4.14 (q,2H); 4.34 (AB,2H); 7.08 (s br,1H); 7.15 (m,1H); 7.39 (m,5H). |
| 5.040 | 3,5-F₂-Ph | H | F | O | H | CH₂ | 1-Ethyl-3-methyl-1H-pyrazol-4-yl | [CDCl₃] 1.47 (t,3H); 2.27 (s,3H); 3.56 (dd,1H); 4.08 (q,2H); 4.20 (dd,1H); 4.37 (m,2H); 6.61 (s br,1H); 6.95 (m,1H); 7.21 (m,2H); 7.34 (s,1H). |
| 5.041 | 3,5-Cl₂-Ph | H | F | O | H | CH₂ | 1-Ethyl-3-methyl-1H-pyrazol-4-yl | [CDCl₃] 1.47 (t,3H); 2.27 (s,3H); 3.57 (dd,1H); 4.08 (q,2H); 4.19 (dd,1H); 4.37 (m,2H); 6.59 (s br,1H); 7.34 (s,1H); 7.48 (s,1H); 7.57 (s,2H). |
| 5.042 | 3-F-Ph | H | CH₂F | O | H | CH₂ | 1-Ethyl-3-methyl-1H-pyrazol-4-yl | [CDCl₃] 1.43 (t,3H); 2.18 (s,3H); 3.45 (d,1H); 3.71 (d,1H); 4.04 (q,2H); 4.22 (dd,1H); 4.34 (dd,1H); 4.63 (d,0.5H); 4.73 (d,0.5H); 4.76 (d,0.5H); 4.88 (d,0.5H); 6.97 (s br,1H); 7.16 (m,1H); 7.25 (s,1H); 7.37 (m,3H). |
| 5.043 | 3,5-F₂-Ph | H | CH₂F | O | H | CH₂ | 1-Ethyl-3-methyl-1H-pyrazol-4-yl | [CDCl₃] 1.44 (t,3H); 2.19 (s,3H); 3.43 (d,1H); 3.68 (d,1H); 4.06 (q,2H); 4.21 (dd,1H); 4.36 |
| | | | | | | | | (dd,1H); 4.64 (d,0.5H); 4.75 (d,1H); 4.88 (d,0.5H); 6.43 (m,2H); 7.17 (m,2H). |
| 5.044 | 3,5-Cl₂-Ph | H | Acetyl | O | H | CH₂ | 1-Ethyl-3-methyl-1H-pyrazol-4-yl | |
| 5.045 | 3,5-F₂-Ph | H | Acetyl | O | H | CH₂ | 1-Ethyl-3-methyl-1H-pyrazol-4-yl | |
| 5.046 | 3,5-Cl₂-Ph | H | Prop-1-en-2-yl | O | H | CH₂ | 1-Ethyl-3-methyl-1H-pyrazol-4-yl | [CDCl₃] 1.44 (t,3H); 1.84 (s,3H); 2.19 (s,3H); 3.34 (d,1H); 4.02 (d,1H); 4.07 (q,2H); 4.19 (dd,1H); 4.32 (dd,1H); 5.08 (s,1H); 5.27 (s,1H); 6.76 (s br,1H); 7.23 (s,1H); 7.41 (s,1H); 7.53 (s,2H). |
| 5.047 | 3,5-F₂-Ph | H | Prop-1-en-2-yl | O | H | CH₂ | 1-Ethyl-3-methyl-1H-pyrazol-4-yl | [CDCl₃] 1.43 (t,3H); 1.89 (s,3H); 2.19 (s,3H); 3.35 (d,1H); 4.02 (d,1H); 4.06 (q,2H); 4.20 (dd,1H); 4.33 (dd,1H); 5.08 (s,1H); 5.27 (s,1H); 6.77 (t br,1H); 6.87 (m,1H); 7.17 (m,1H); 7.24 (s,1H); |
| 5.048 | 3,5-F₂-Ph | H | Vinyl | O | H | CH₂ | 1-Ethyl-3-methyl-1H-pyrazol-4-yl | [CDCl₃] 1.43 (t,3H); 2.19 (s,3H); 3.33 (d,1H); 3.94 (d,1H); 4.07 (q,2H); 4.20 (dd,1H); 4.33 (dd,1H); 5.34 (d,1H); 5.55 (d,1H); 6.20 (dd,1H); 6.81 (s br,1H); 6.89 |
| | | | | | | | | (m,1H); 7.17 (m,2H); 7.24 (s,1H). |
| 5.049 | 3,5-Cl₂-Ph | H | CH₂Cl | O | H | CH₂ | 1-Ethyl-3-methyl-1H-pyrazol-4-yl | [CDCl₃] 1.42 (t,3H); 2.20 (s,3H); 3.55 (d,1H); 3.71 (d,1H); 3.94 (d,1H); 4.03 (d,1H); 4.07 (q,2H); 4.20 (dd,1H); 4.34 (dd,1H); 6.91 (s br,1H); 7.44 (s,1H); 7.52 (s,2H). |
| 5.050 | 3,5-F₂-Ph | H | CH₂Cl | O | H | CH₂ | 1-Ethyl-3-methyl-1H-pyrazol-4-yl | [CDCl₃] 1.44 (t,3H); 2.20 (s,3H); 3.55 (d,1H); 3.72 (d,1H); 3.95 (d,1H); 4.05 (d,1H); 4.07 (q,2H); 4.21 (dd,1H); 4.36 (dd,1H); 6.92 (m,2H); 7.15 (m,2H); 7,27 (s,1H). |
| 5.051 | 3-F-Ph | H | CH₂Cl | O | H | CH₂ | 1-Ethyl-3-methyl-1H-pyrazol-4-yl | [CDCl₃] 1.44 (t,3H); 2.19 (s,3H); 3.57 (d,1H); 3.73 (d,1H); 3.84 (d,1H); 4.04 (d,1H); 4.05 (q,2H); 4.20 (dd,1H); 4.36 (dd,1H); 6.96 (s br,1H); 7.15 (m,1H); 7.38 (m,3H). |
| 5.052 | 3,5-Cl₂-Ph | H | Vinyl | O | H | CH₂ | 1-Ethyl-3-methyl-1H-pyrazol-4-yl | [CDCl₃] 1.44 (t,3H); 2.18 (s,3H); 3.32 (d,1H); 3.94 (d,1H); 4.03 (q,2H); 4.20 (dd,1H); 4.33 (dd,1H); 5.33 (d,1H); 5.52 (d,1H); 6.16 |
| | | | | | | | | (dd,1 H); 6.80 (t br,1H); 7.24 (s,1H); 7.42 (s,1H); 7.52 (s,2H). |
| 5.053 | 3,5-F₂-Ph | H | CH₂Cl | O | H | CH₂ | 2-Brompyridin-4-yl | [CDCl₃] 3.54 (d,1H); 3.73 (d,1H); 3.95 (d,1H); 4.11 (d,1H); 4.35 (dd,1H); 4.60 (dd,1H); 6.93 (m,1H); 7.18 (m,2H); 7.39 (m,2H); 8.33 (d,1 H). |
| 5.054 | 3,5-F₂-Ph | H | CH₂F | O | H | CH₂ | 2-Brompyridin-4-yl | [CDCl₃] 1.56 (s,3H); 3.42 (d,1H); 3.71 (d,1H); 4.47 (AB d,2H); 4.85 (AB,1H); 4.74 (AB,1H); 6.91 (m,1H); 7.12 (d,1H); 7.18 (m,2H); 7.35 (s,1H); 7.39 (t br,1H); 8.31 (d,1 H). |
| 5.055 | 3,5-Cl₂-Ph | H | CH₂Cl | O | H | CH₂ | 2-Brompyridin-4-yl | [CDCl₃] 3.54 (d,1H); 3.73 (d,1H); 3.93 (d,1H); 4.10 (d,1H); 4.33 (dd,1H); 4.58 (dd,1H); 7.14 (d,1H); 7.38 (s,2H); 7.45 (s,1H); 7.54 (s, 2H); 8.32 (d,1H). |
| 5.056 | 3-F-Ph | H | CH₂Cl | O | H | CH₂ | 2-Brompyridin-4-yl | [CDCl₃] 3.56 (d,1H); 3.76 (d,1H); 3.94 (d,1H); 4.12 (d,1H); 4.34 (dd,1H); 4.61 (dd,1H); 7.18 (m,2H); 7.40 (m,5H); 8.31 (d,1 H).. |
| 5.057 | 3,5-F₂-Ph | H | F | O | H | CH₂ | 2-Chlorpyridin-4-yl | [CDCl₃] 3.62 (dd,1H); 4.25 |
| | | | | | | | | (dd,1H); 4.57 (m,2H); 6.95 (m,1H); 7.03 (s br,1H); 7.15 (d,1H); 7.25 (m,3H); 8.40 (d,1 H). |
| 5.058 | 3,5-F₂-Ph | H | CH₂F | O | H | CH₂ | 2-Chlorpyridin-4-yl | [CDCl₃] 3.42 (d,1H); 3.72 (d,1H); 4.38 (dd,1H); 4.61 (dd,1H); 4.65 (d,0.5H); 4.76 (d,0.5H); 4.82 (d,0.5H); 4.94 (d,0.5H); 6.94 (m,1H); 7.08 (d,1H); 7.18 (m,2H); 7.27 (m,1H); 7.38 (s br,1H); 8.34 (d,1 H). |
| 5.059 | 3,5-Cl₂-Ph | H | Acetyl | O | H | CH₂ | 2-Chlorpyridin-4-yl | |
| 5.060 | 3,5-F₂-Ph | H | Acetyl | O | H | CH₂ | 2-Chlorpyridin-4-yl | |
| 5.061 | 3-F-Ph | H | CH₂F | O | H | CH₂ | 2-Chlorpyridin-4-yl | |
| 5.062 | 3,5-F₂-Ph | H | CH₂Cl | O | H | CH₂ | 2-Chlorpyridin-4-yl | [CDCl₃] 3.55 (d,1H); 3.74 (d,1H); 3.94 (d,1H); 4.10 (d,1H); 4.37 (dd,1H); 4.63 (dd,1H); 6.92 (m,1H); 7.12 (d,1H); 7.18 (m,2H); 7.23 (s,1H); 7.38 (t br,1H); 8.35 (d,1H). |
| 5.063 | 3,5-Cl₂-Ph | H | CH₂Cl | O | H | CH₂ | 2-Chlorpyridin-4-yl | |
| 5.064 | 3,5-F₂-Ph | H | Vinyl | O | H | CH₂ | 2-Chlorpyridin-4-yl | [CDCl₃] 3.38 (d,1H); 3.97 (d,1H); 4.40 (dd,1H); 4.54 (dd,1H); 5.42 (d,1H); 5.59 (d,1H); 6.20 (dd,1H); 6.91 (m,1H); 7.07 |
| | | | | | | | | (d,1H); 7.18 (m,2H); 7.29 (s br,1H); 8.33 (d,1 H). |
| 5.065 | 3-F-Ph | H | CH₂Cl | O | H | CH₂ | 2-Chlorpyridin-4-yl | |
| 5.066 | 3,5-F₂-Ph | H | CH₂F | O | H | CH₂ | 2-Cyclopropylpyridi n-4-yl | [CDCl₃] 0.99 (m,4H); 1.97 (m,1H); 3.44 (d,1H); 3.72 (d,1H); 4.34 (dd,1H); 4.56 (dd,1H); 4.67 (d, 0.5H); 4.78 (d,0,5H); 4.82 (d,0.5H); 4.93 (d,0.5H); 6.93 (m,3H); 7.18 (m,2H); 7.32 (t br,1h); 8.36 (d,1H). |
| 5.067 | 3,5-F₂-Ph | H | CH₂F | O | H | CH₂ | 2-Ethylpyridin-4-yl | [CDCl₃] 1.27 (t,3H); 2.78 (q,2H); 3.43 (d,1H); 3.73 (d,1H); 4.37 (dd,1H); 4.60 (dd,1H); 4.66 (d,0.5H); 4.78 (d,0.5H); 4.83 (d,0.5H); 4.94 (d,0.5H); 6.93 (m,1H); 6.98 (d,1H); 7.01 (s,1H); 7.19 (m,2H); 7.33 (s br,1H); 8.46 (d,1H). |
| 5.068 | 3,5-F₂-Ph | H | CH₂F | O | H | CH₂ | 2-Fluorpyridin-4-yl | [CDCl₃] 3.42 (d,1H); 3.71 (d,1H); 4.40 (dd,1H); 4,64 (dd,1H); 4.65 (d,0.5H); 4.78 (d,0.5H); 4.82 (d,0.5H); 4.94 (d,0.5H); 6.80 (s,1H); 6.92 (m,1H); 7.04 (d,1H); 7.18 (m,2H); 7.39 (s,br,1H); 8.18 |
| | | | | | | | | (d,1H). |
| 5.069 | 3-F-Ph | H | CH₂F | O | H | CH₂ | 2-Fluorpyridin-4-yl | |
| 5.070 | 3,5-F₂-Ph | H | CH₂F | O | H | CH₂ | 2-Methoxypyridin-4-yl | [CDCl₃] 3.45 (d,1H); 3.71 (d,1H); 3.91 (s,3H); 4.34 (dd,1H); 4.55 (dd,1H); 4.66 (d,0.5H);4.78 (d,0.5H); 4.80 (d,0.5H); 4.92 (d,0.5H); 6.57 (s,1H); 6.73 (d,1H); 6.92 (m,1H); 7.19 (m,2H); 7.29 (m,1H); 8.10 (d,1H). |
| 5.071 | 3,5-F₂-Ph | H | CH₂F | O | H | CH₂ | 2-Methylpyridin-4-yl | |
| 5.072 | 3,5-F₂-Ph | H | CH₂F | O | H | CH₂ | 3,5-Dimethyl-1,2-oxazol-4-yl | [CDCl₃] 2.20 (s,3H); 2.38 (s,3H); 3,41 (d,1H); 3.66 (d,1H); 4.13 (dd,1H); 4.30 (dd,1H); 4.61 (d,0.5H); 4.72 (d,0.5H); 4.75 (d,0.5H); 4.86 (d,0.5H); 6.91 (m,1H); 6.98 (s br,1H); 7.15 (m,2H). |
| 5.073 | 3,5-F₂-Ph | H | CH₂F | O | H | CH₂ | 3-Ethyl-5-methyl-1,2-oxazol-4-yl | |
| 5.074 | 3,5-F₂-Ph | H | CH₂Cl | O | H | CH₂ | 3-Ethyl-5-methyl-1,2-oxazol-4-yl | [CDCl₃] 1.24 (t,3H); 2.38 (s,3H); 2.61 (q,2H); 3.53 (d,1H); 3.68 (d,1H); 3.90 (d,1H); 4.02 (d,1H); 4.16 (dd,1H); 4.28 (dd,1H); 6.92 (m,2H); 7.15 (m,2H). |
| 5.075 | 3,5-F₂-Ph | H | CH₂F | O | H | CH₂ | 6-(Trifluormethyl)py ridin-3-yl | |

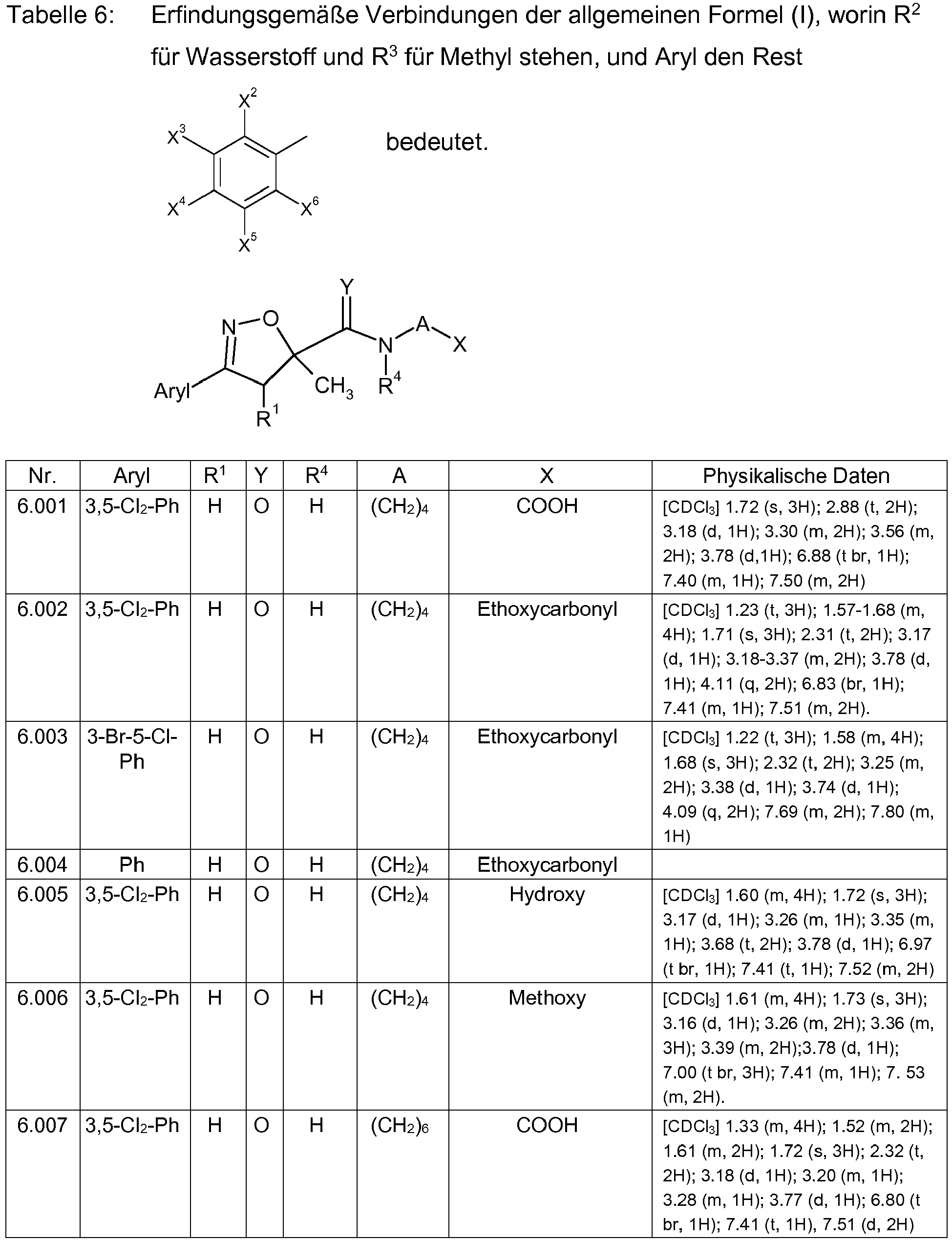

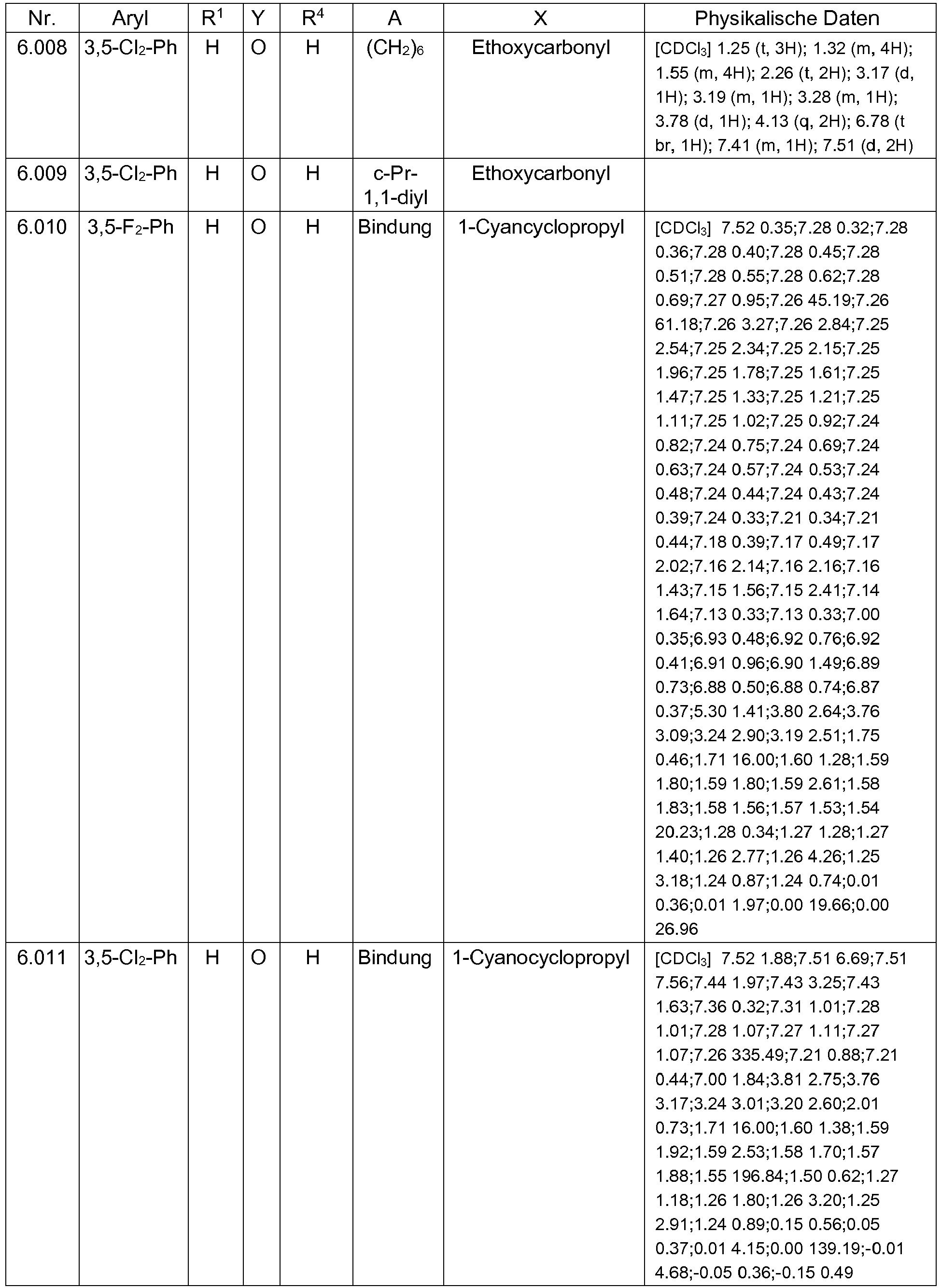

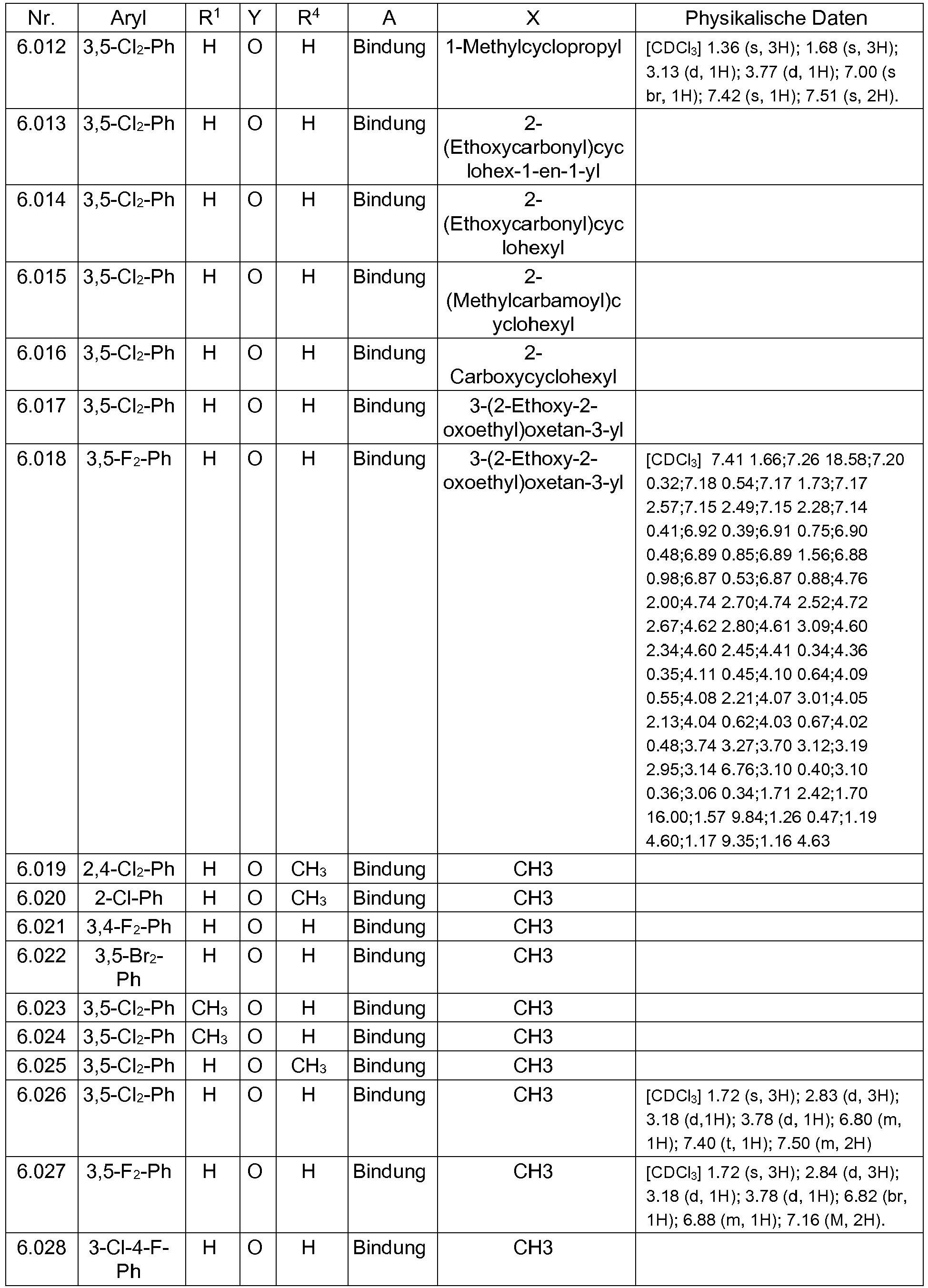

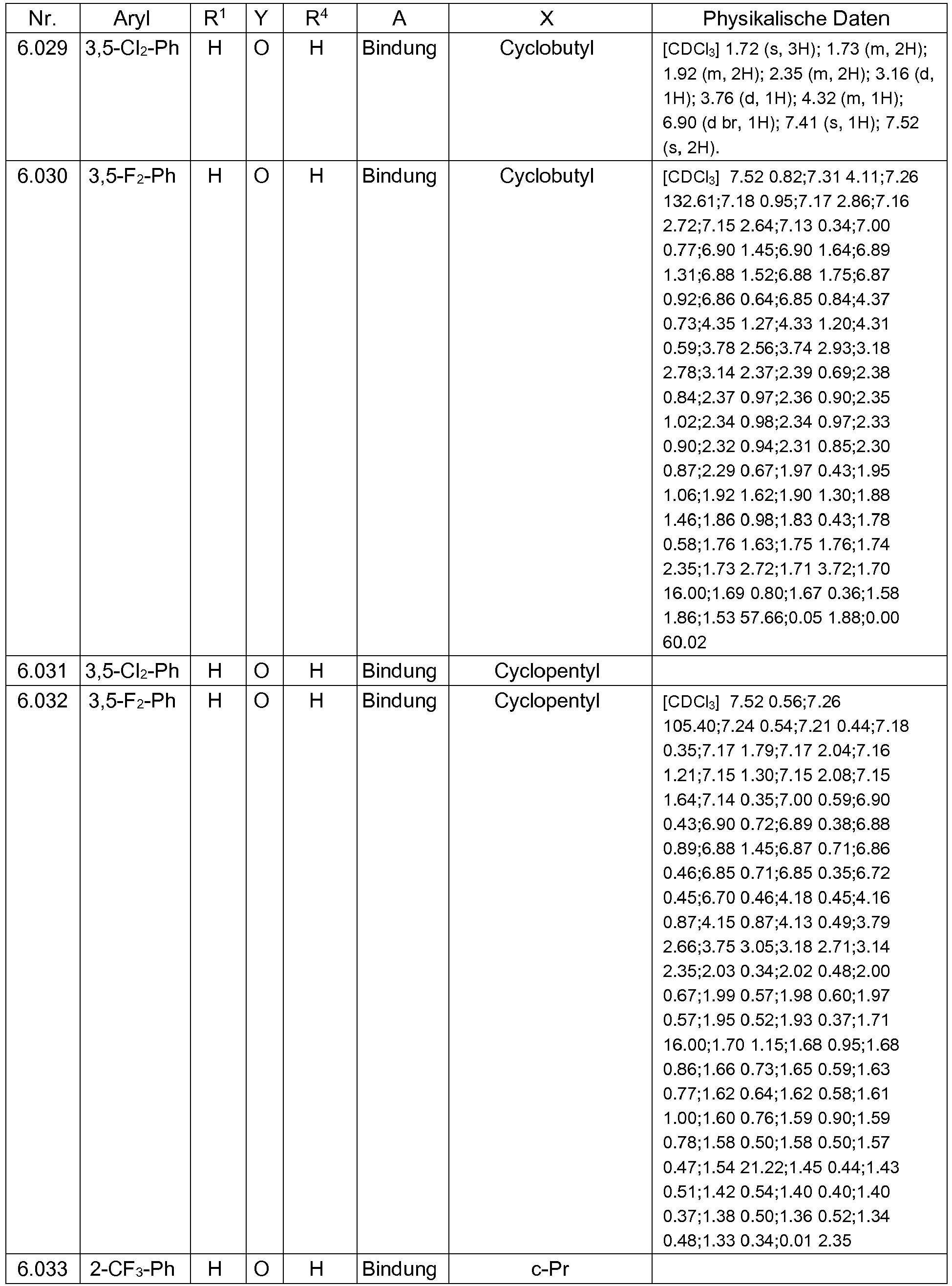

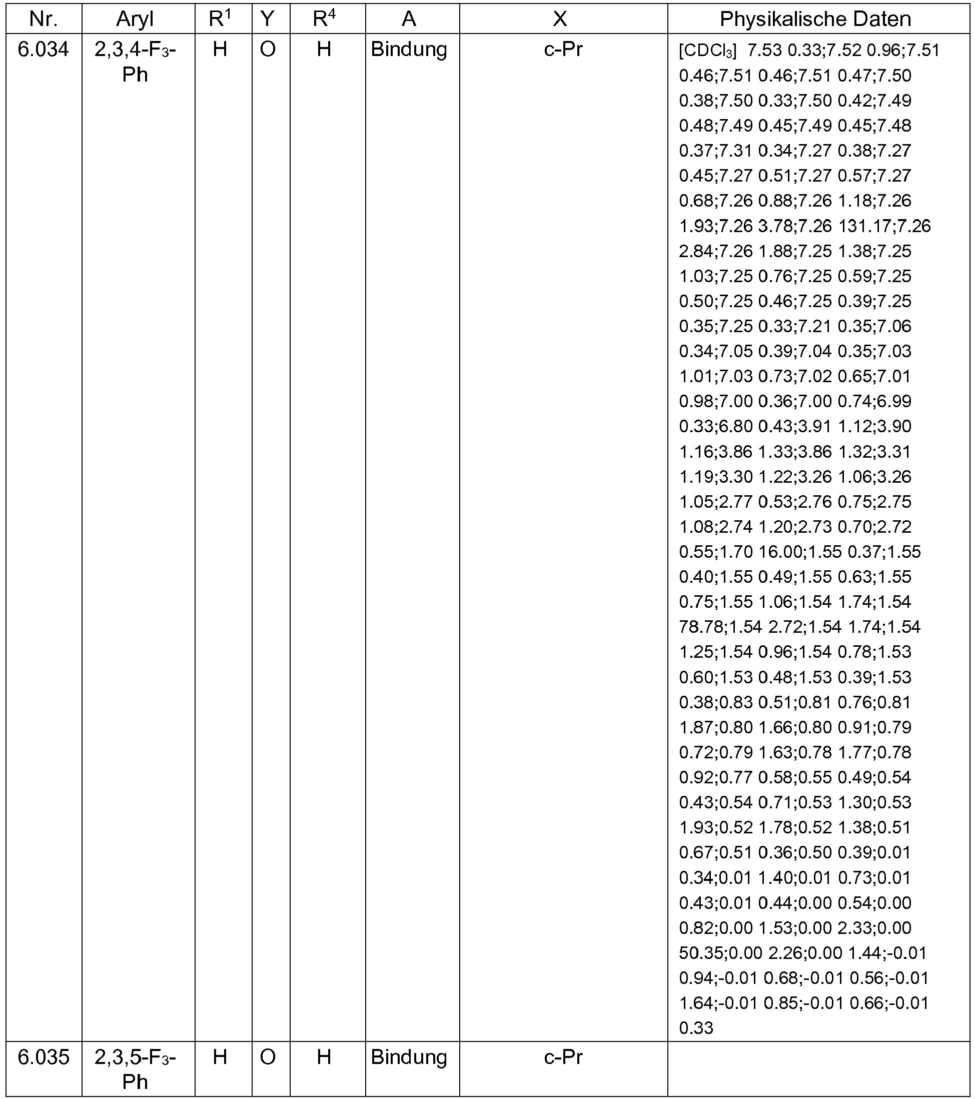

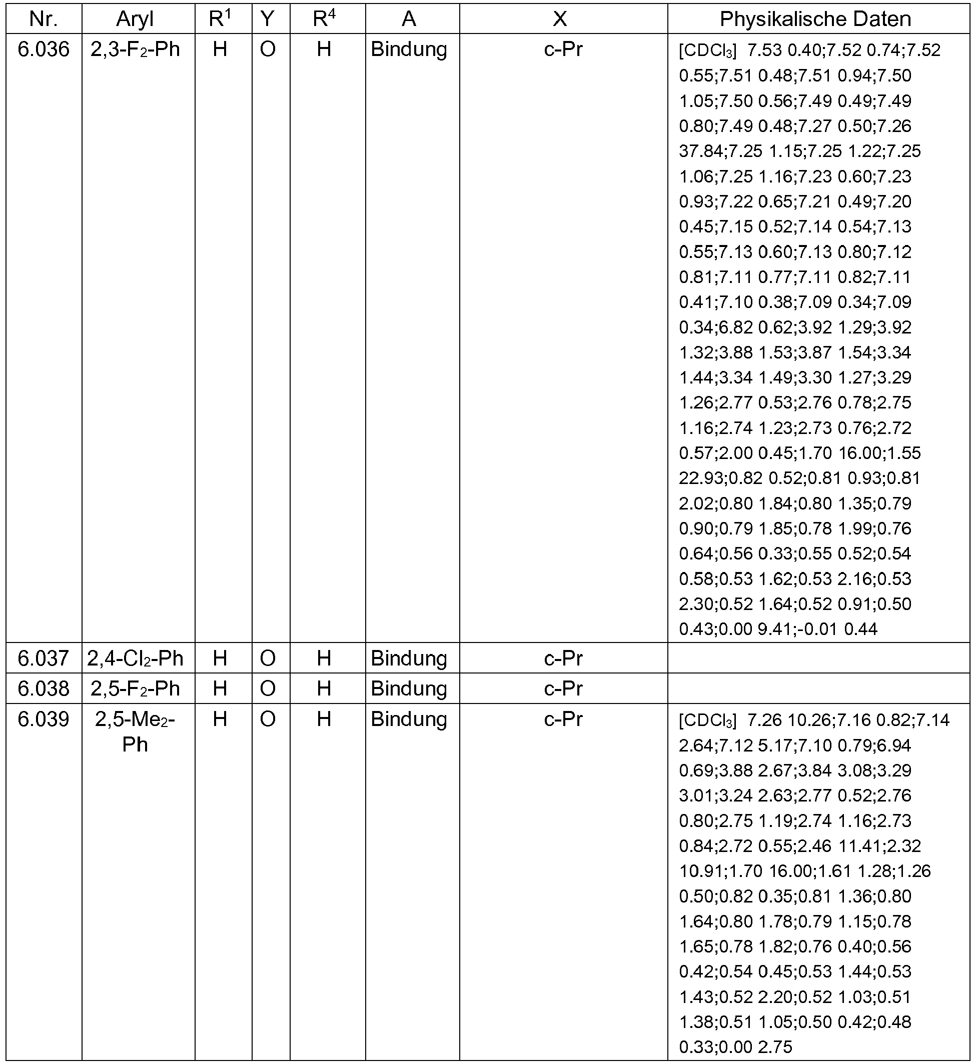

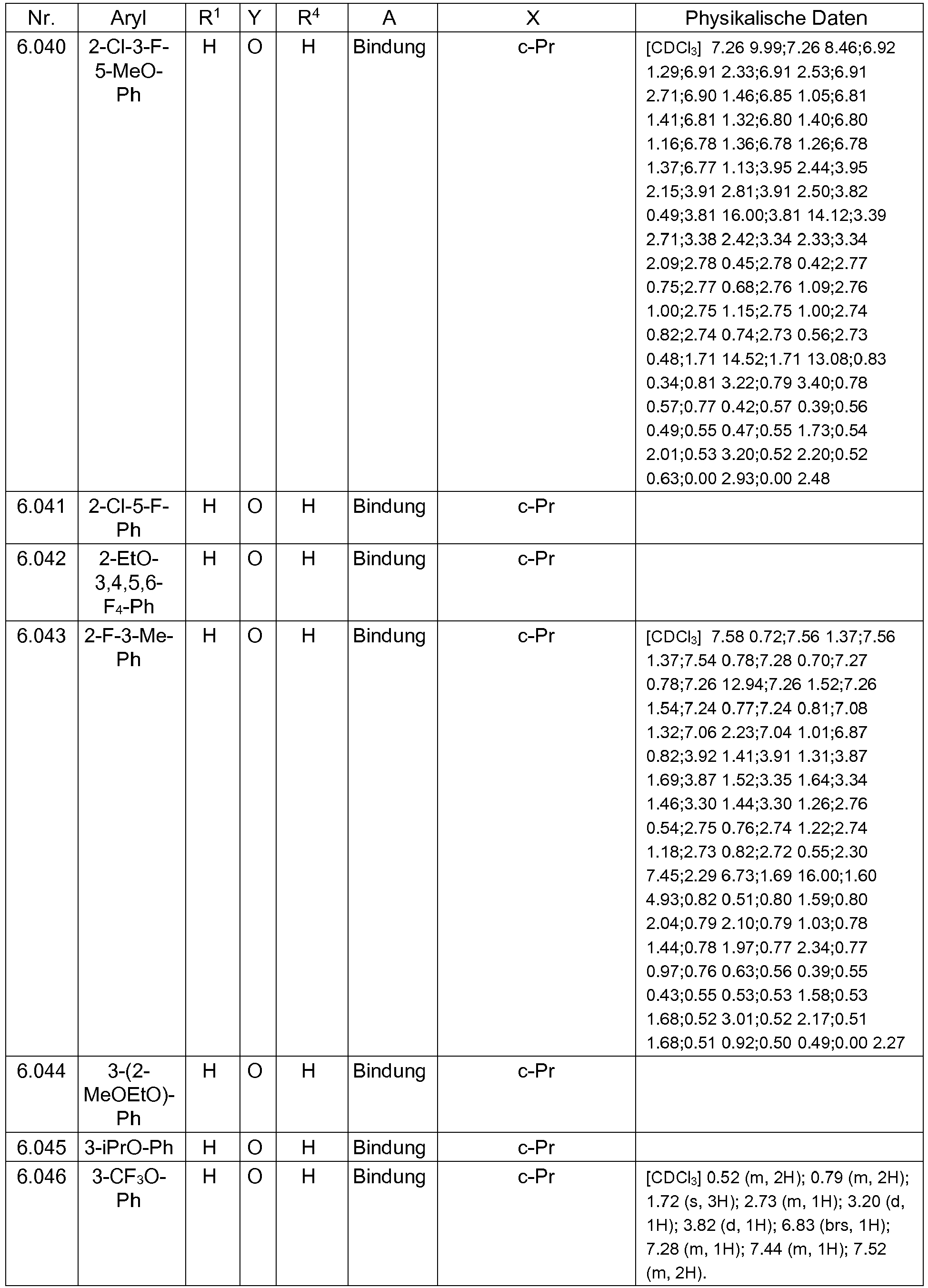

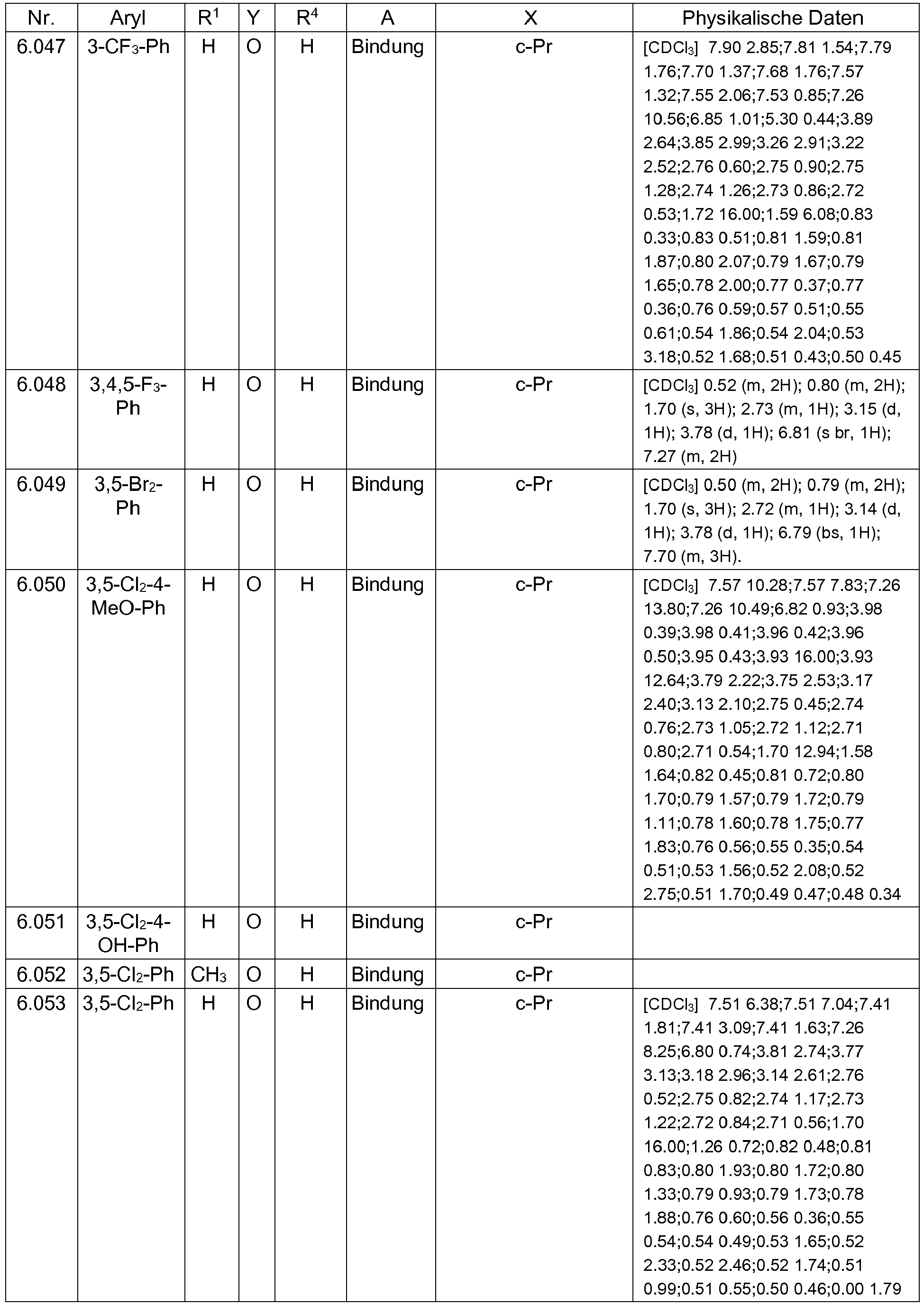

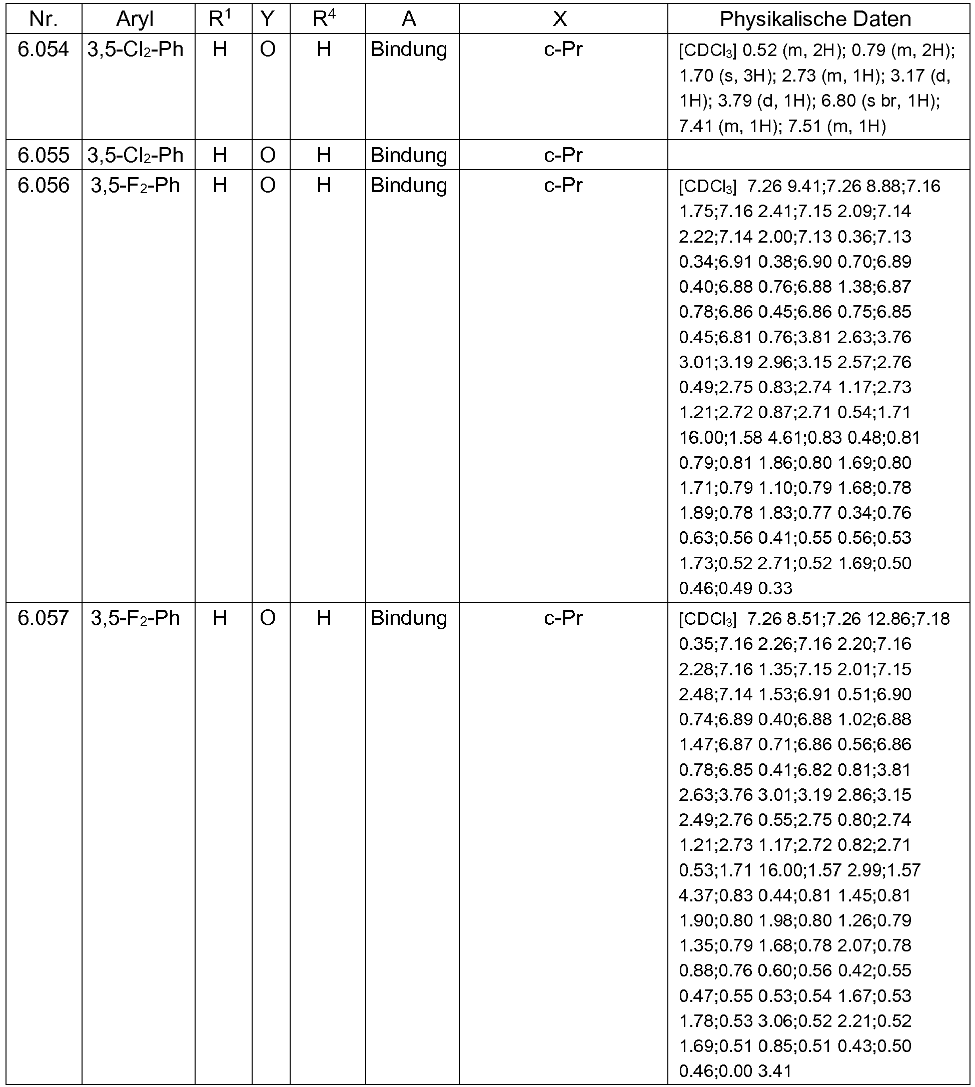

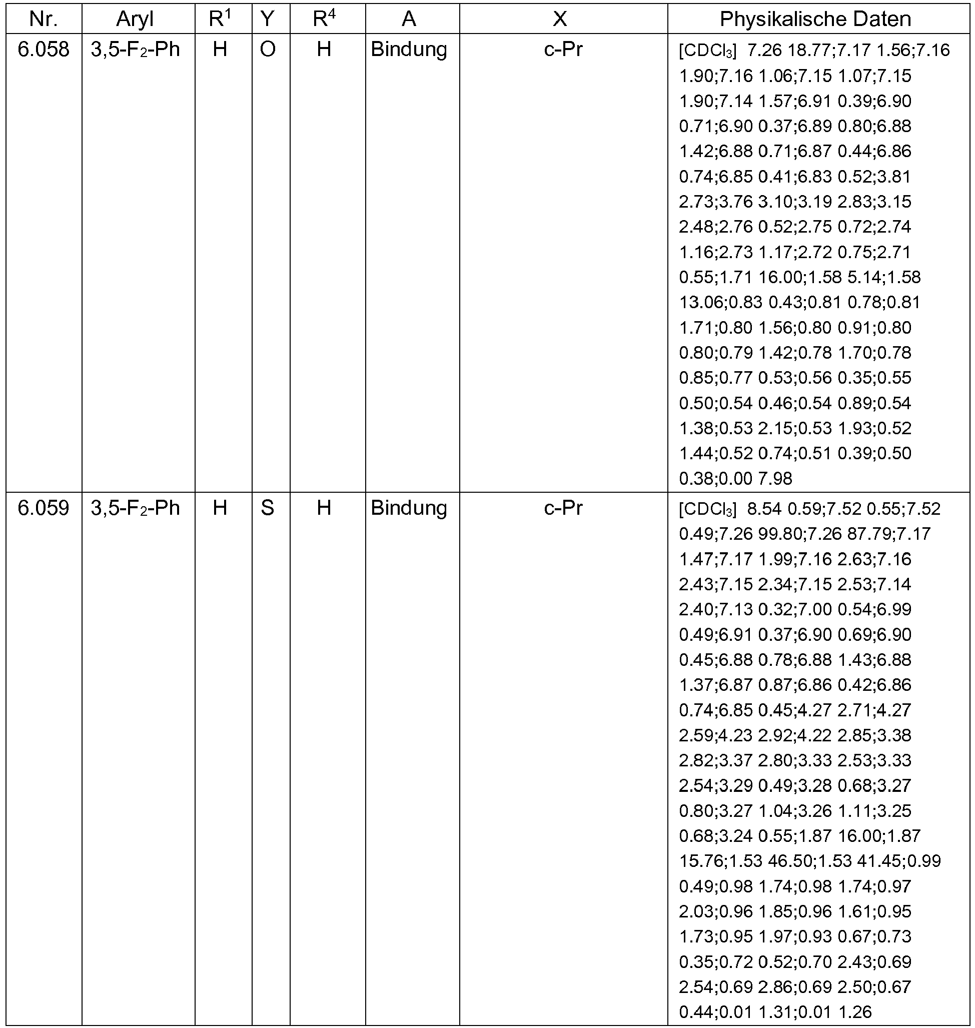

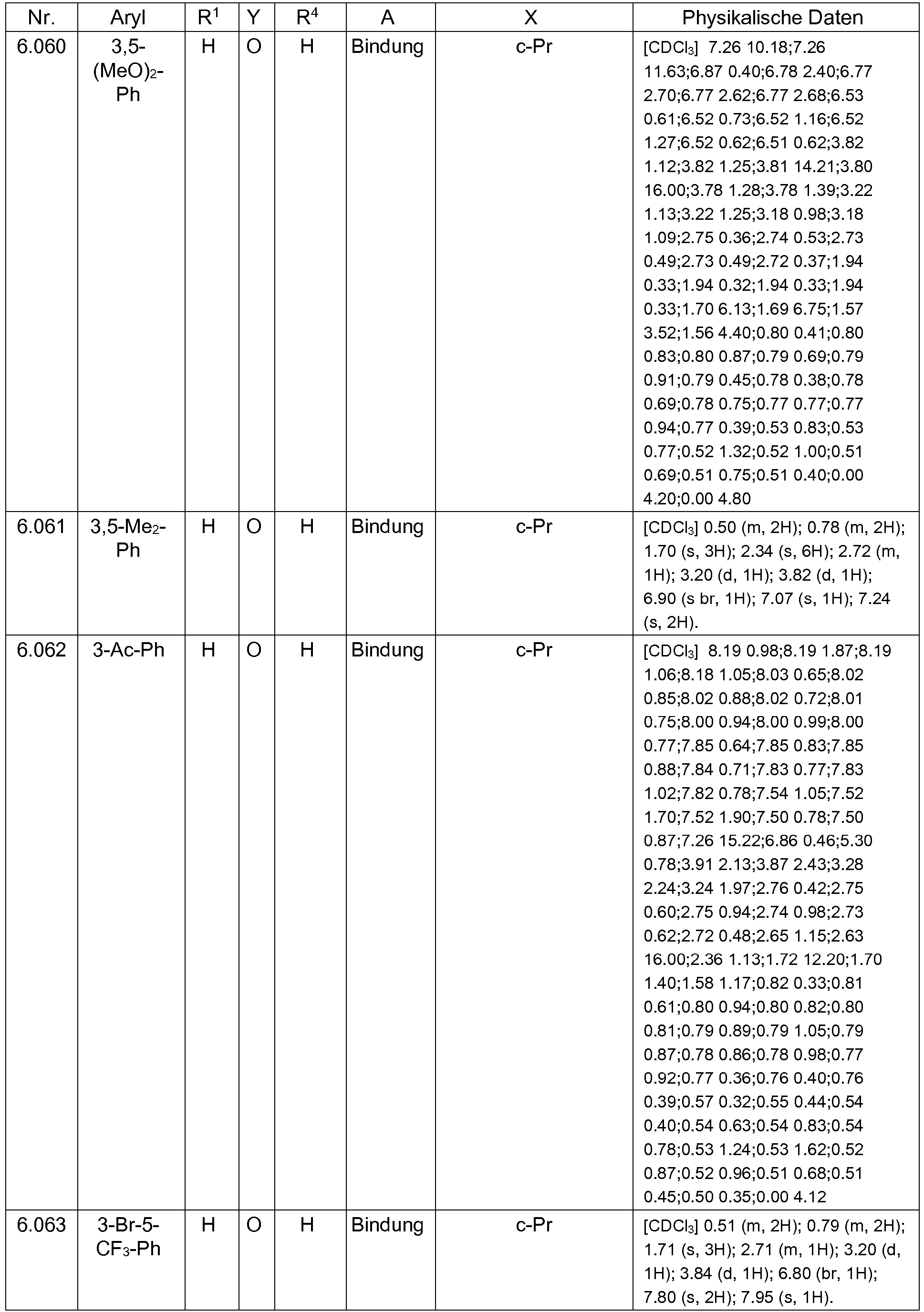

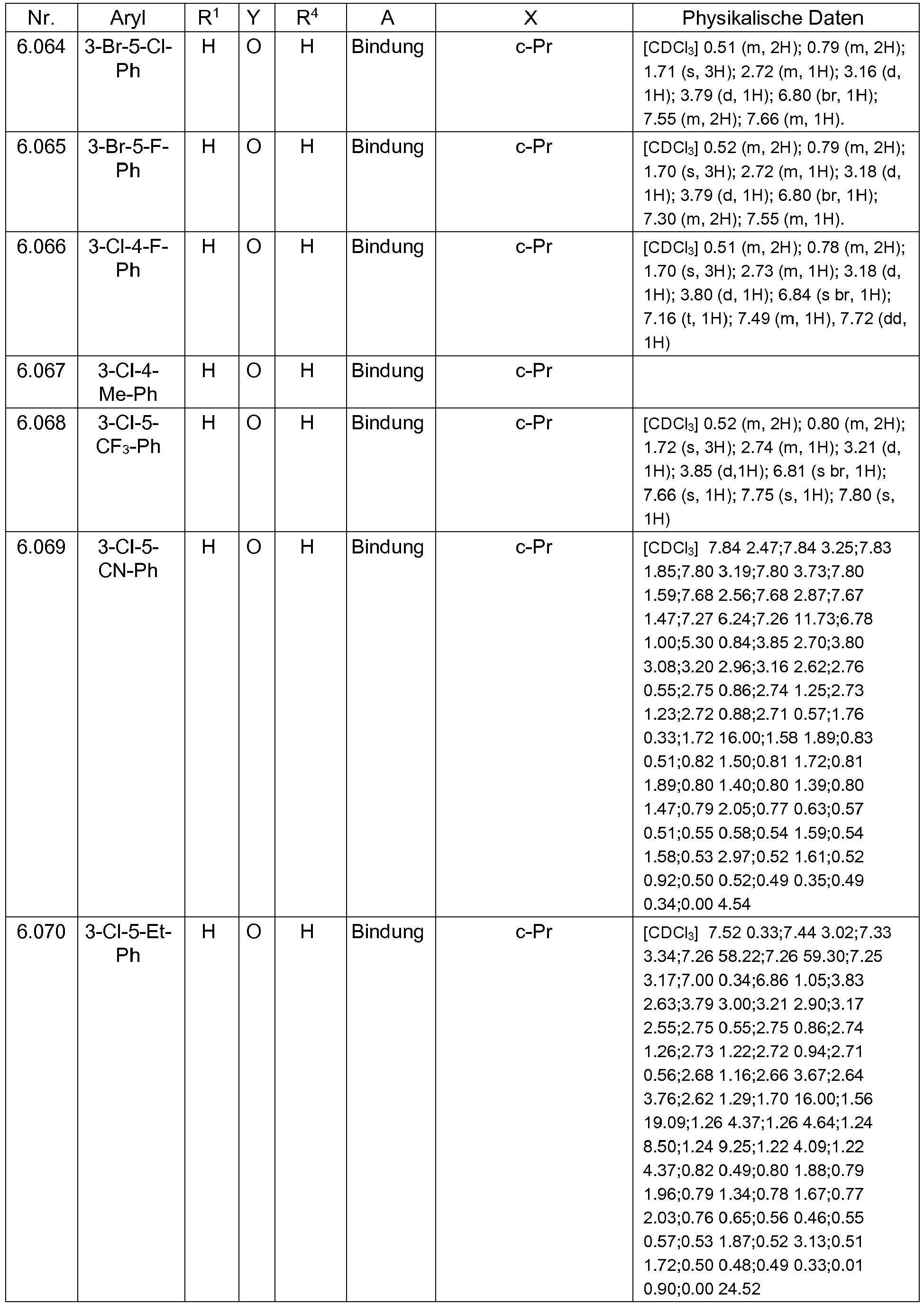

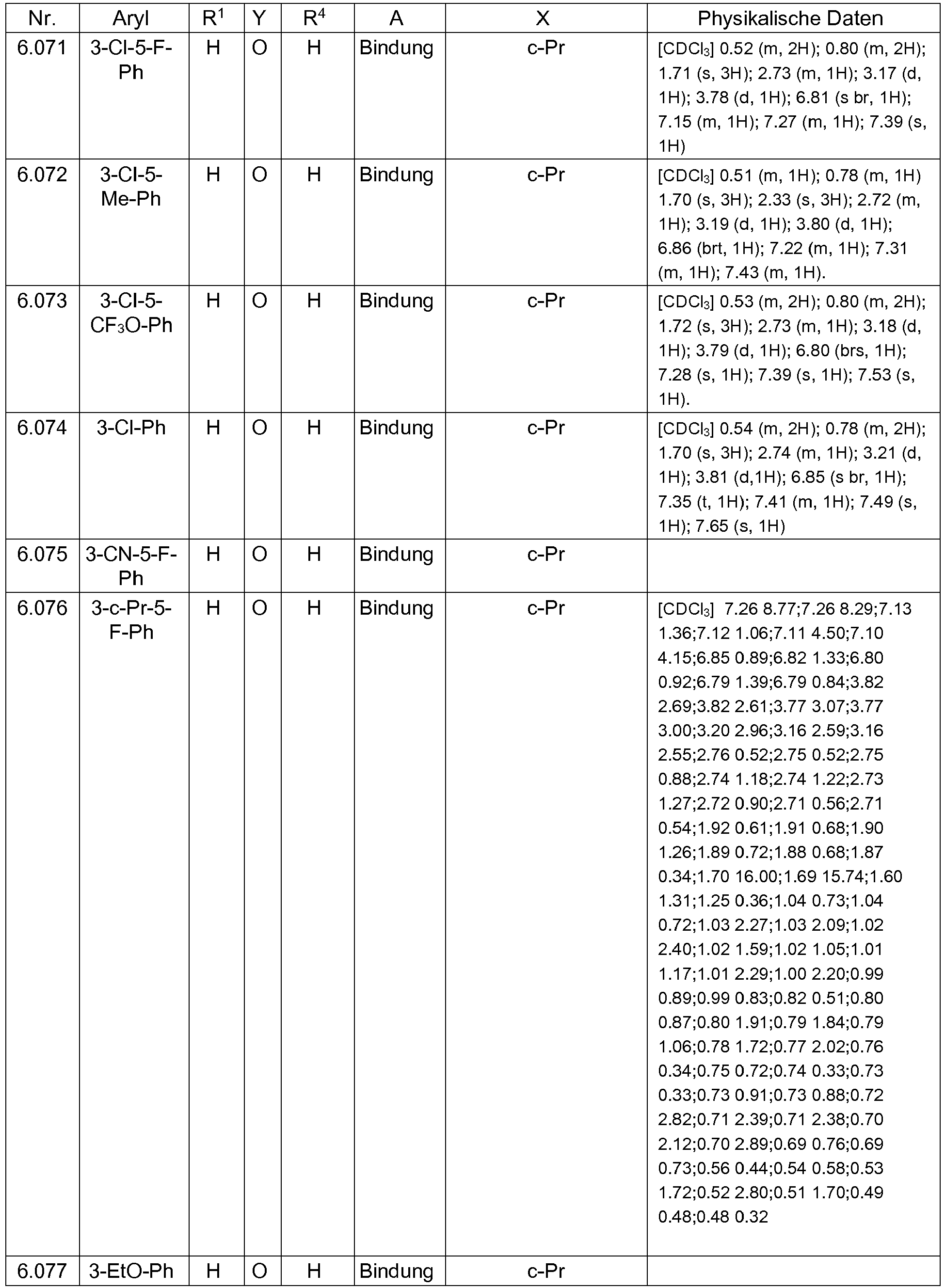

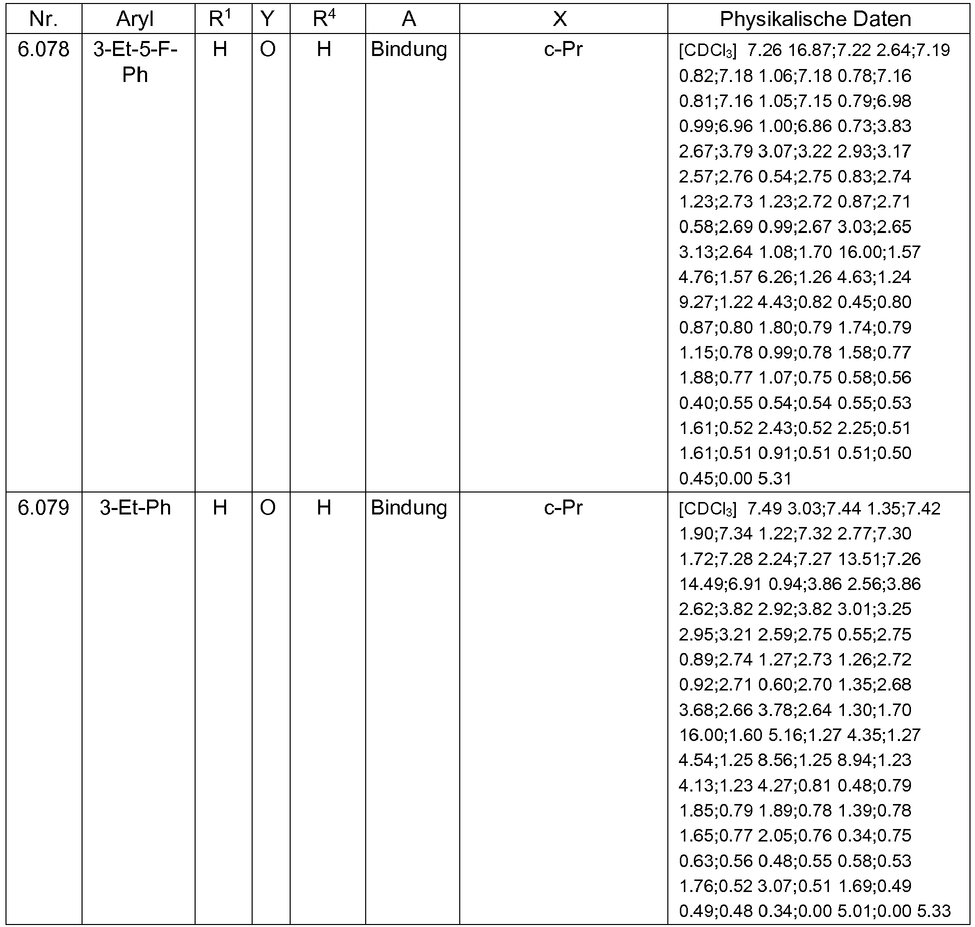

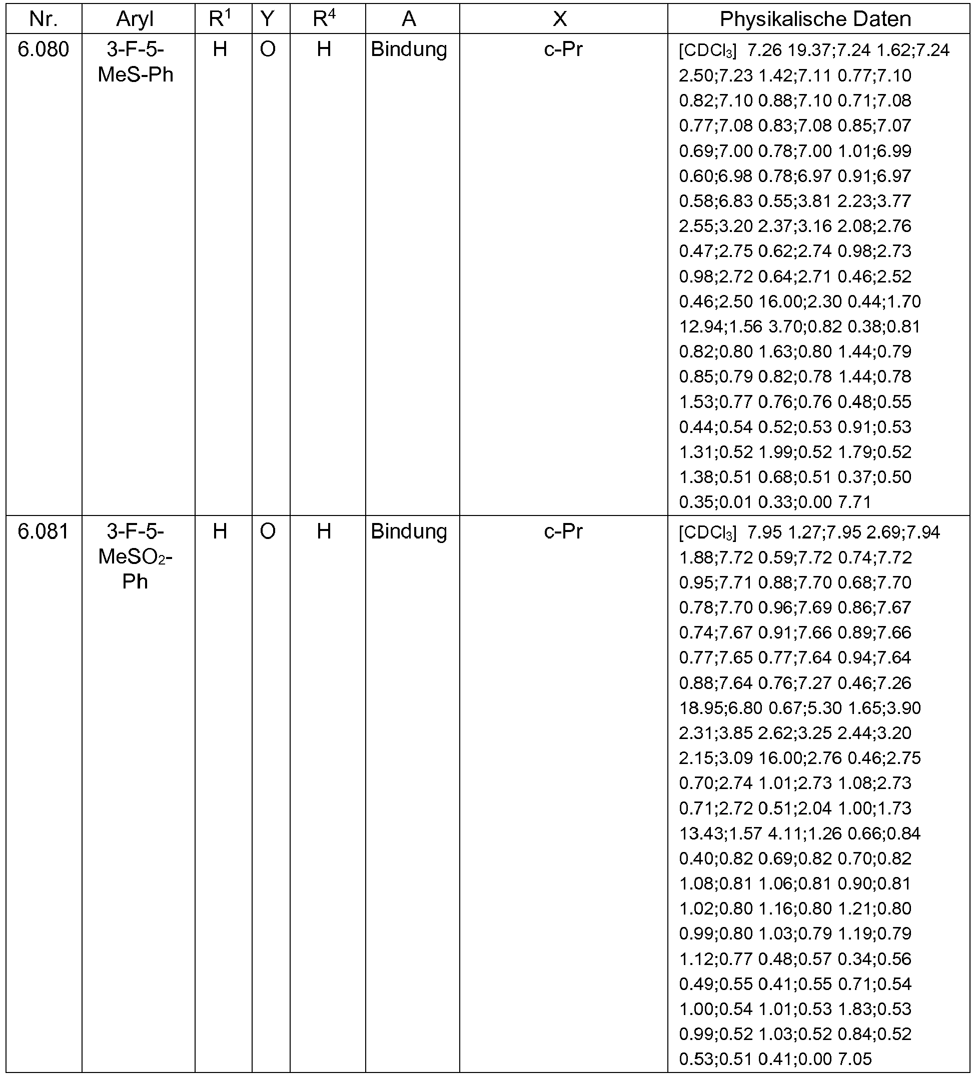

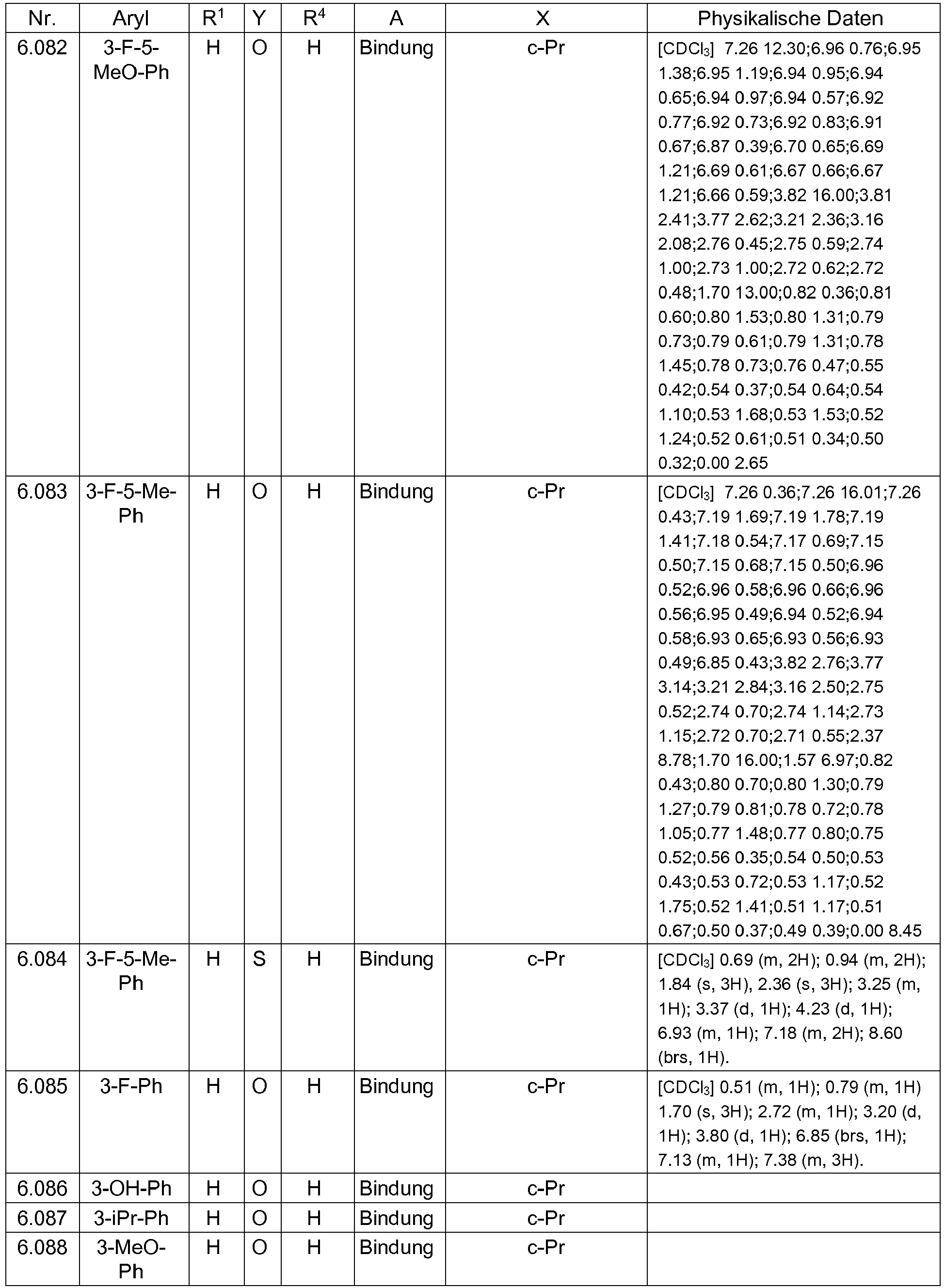

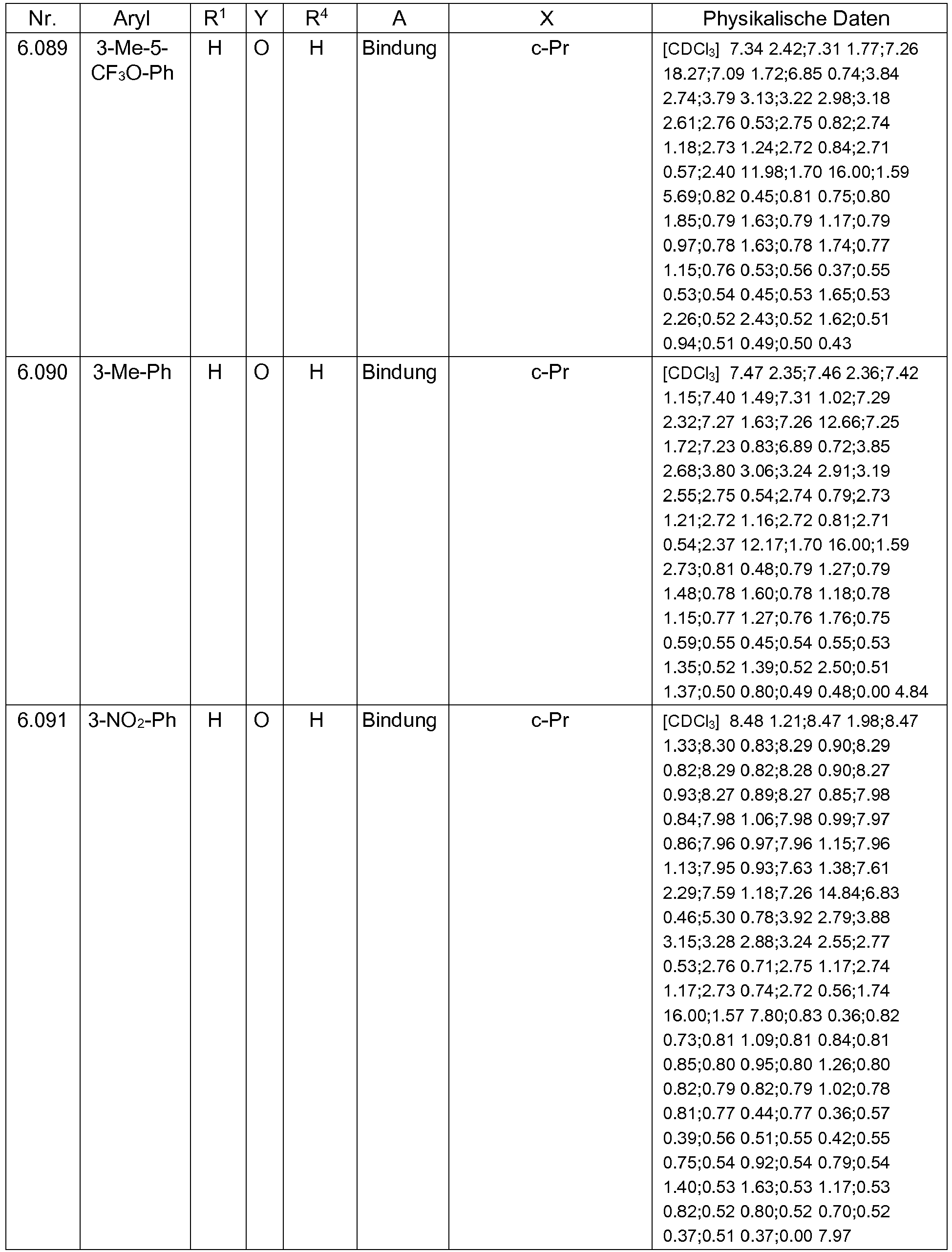

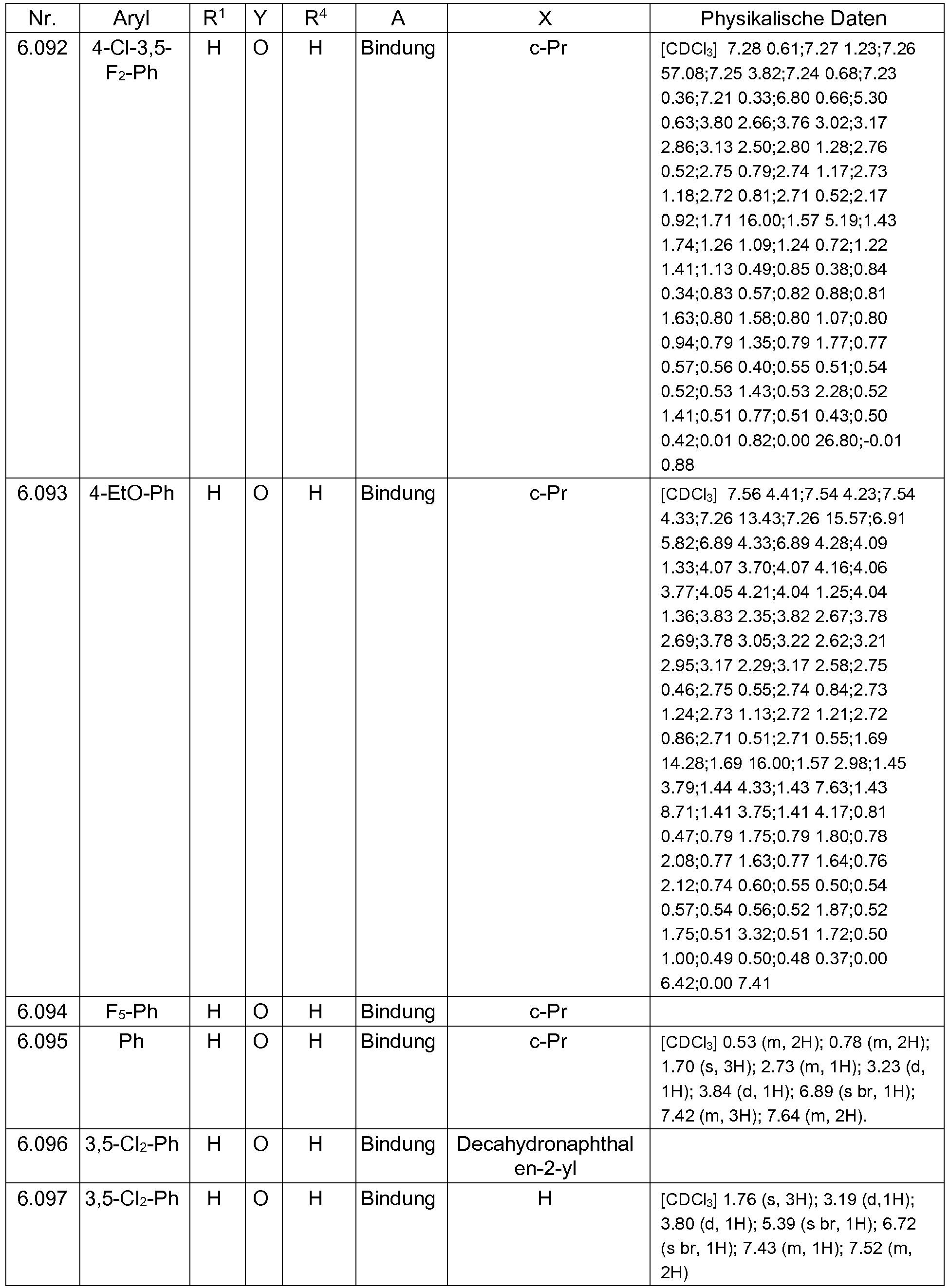

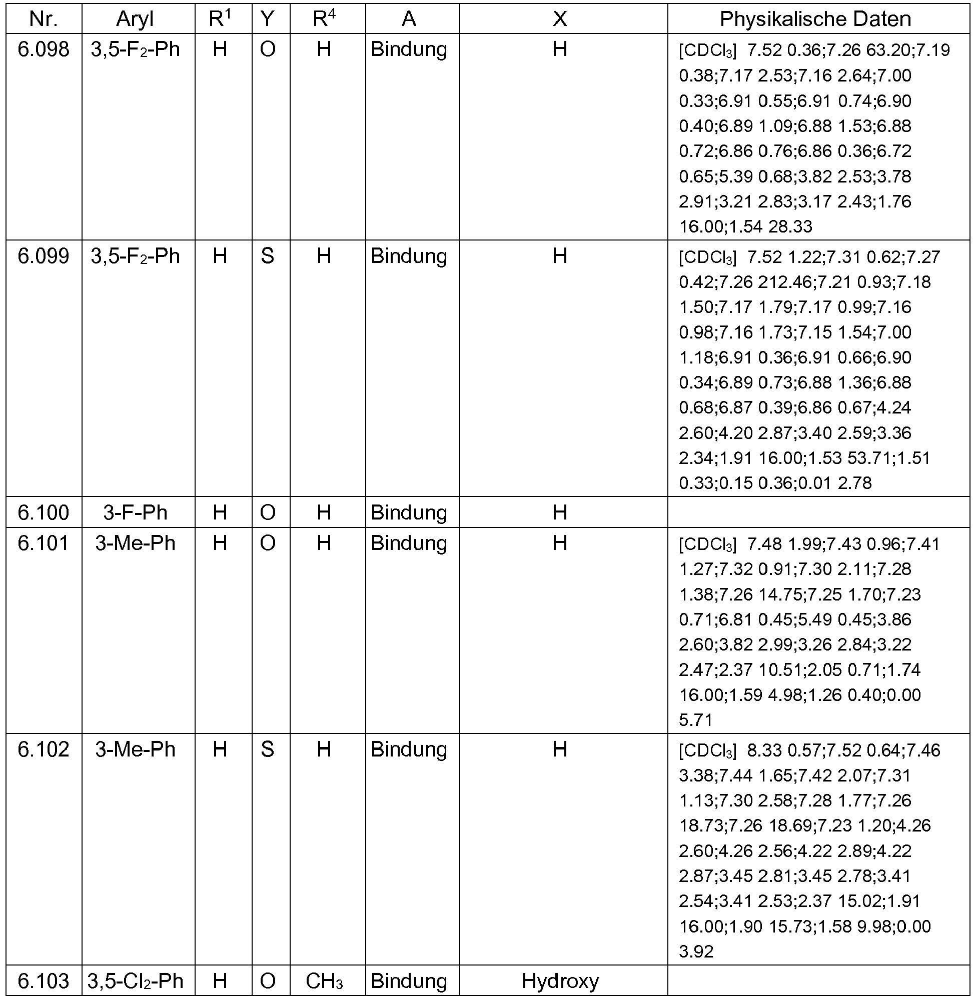

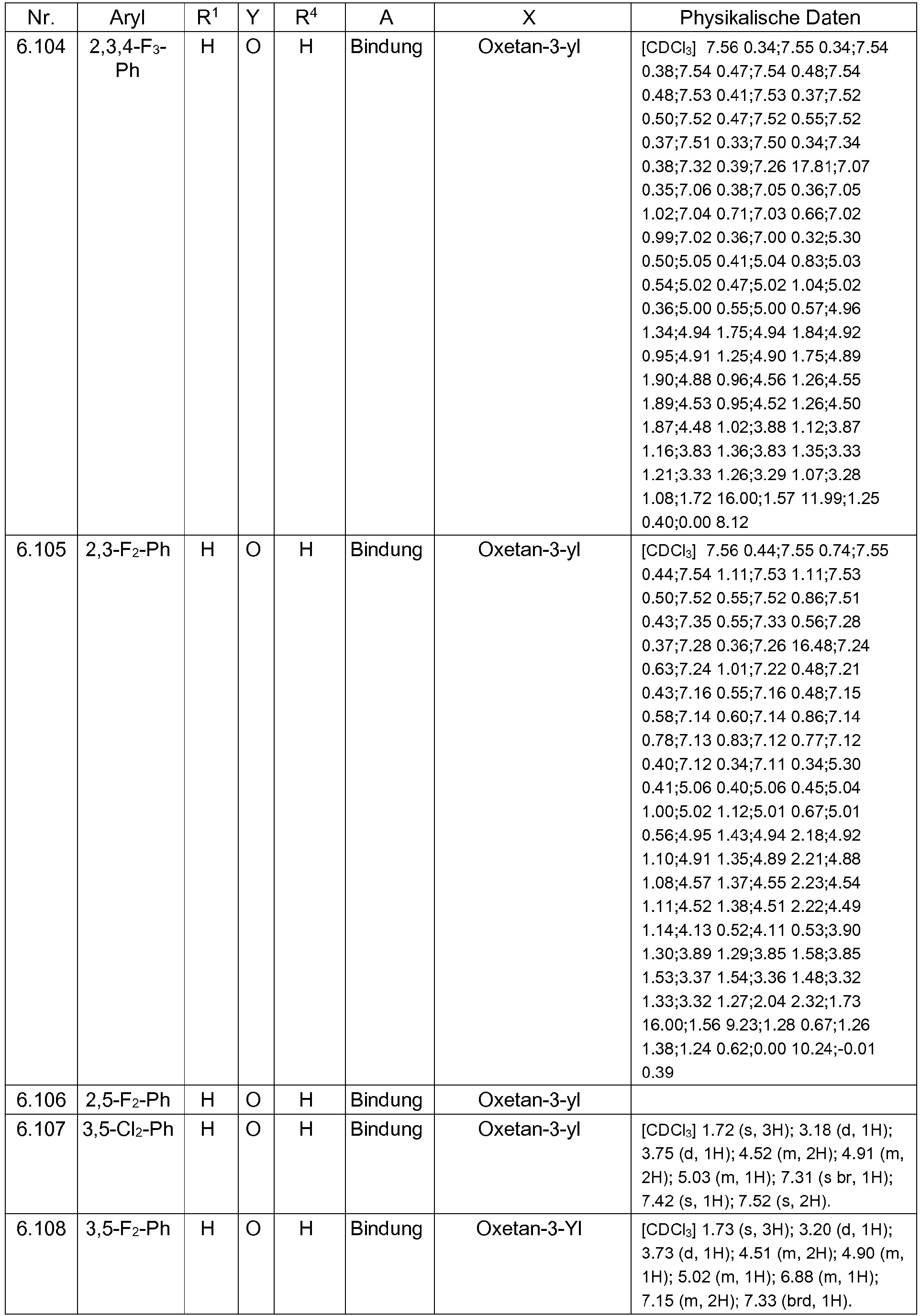

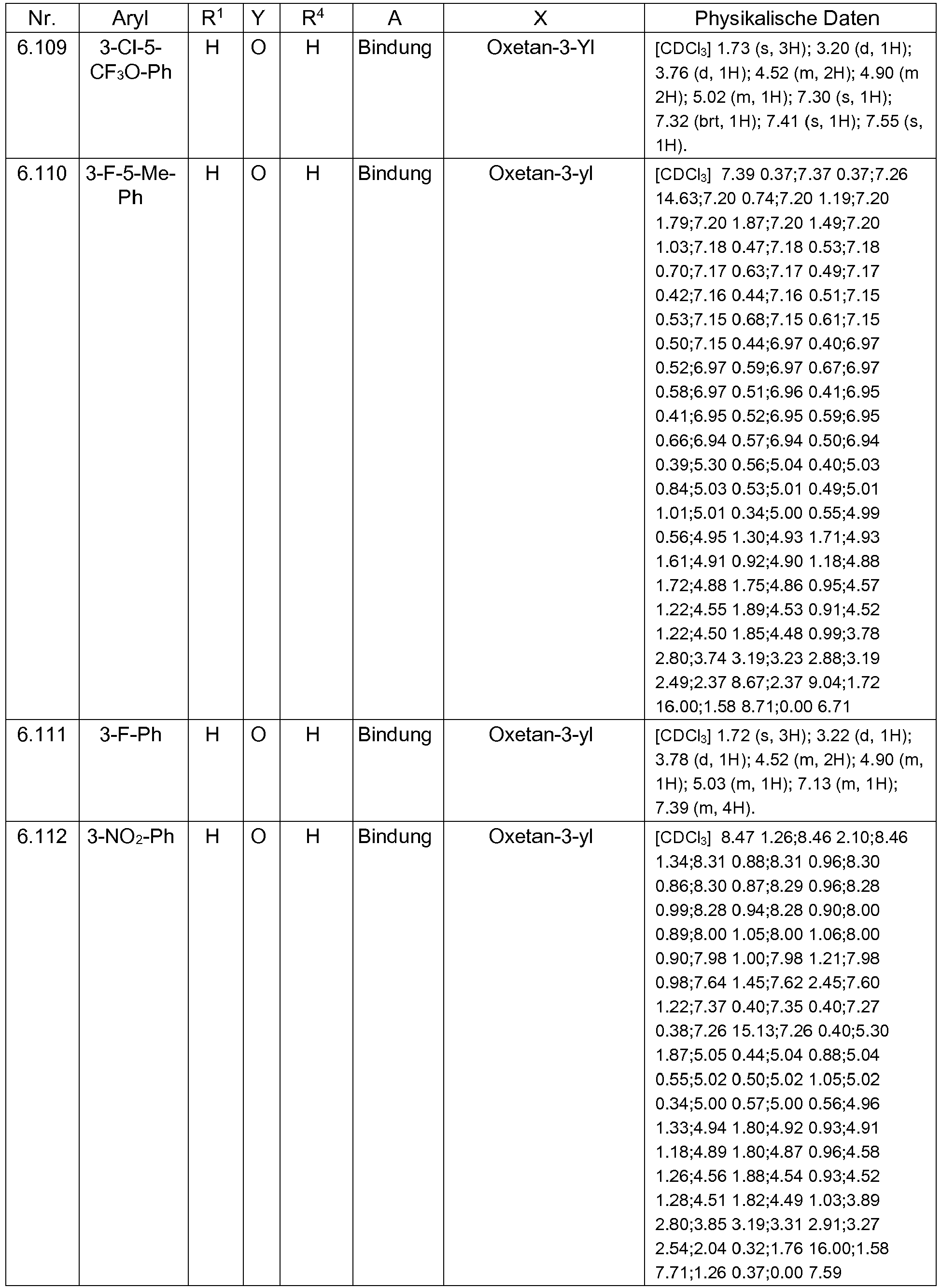

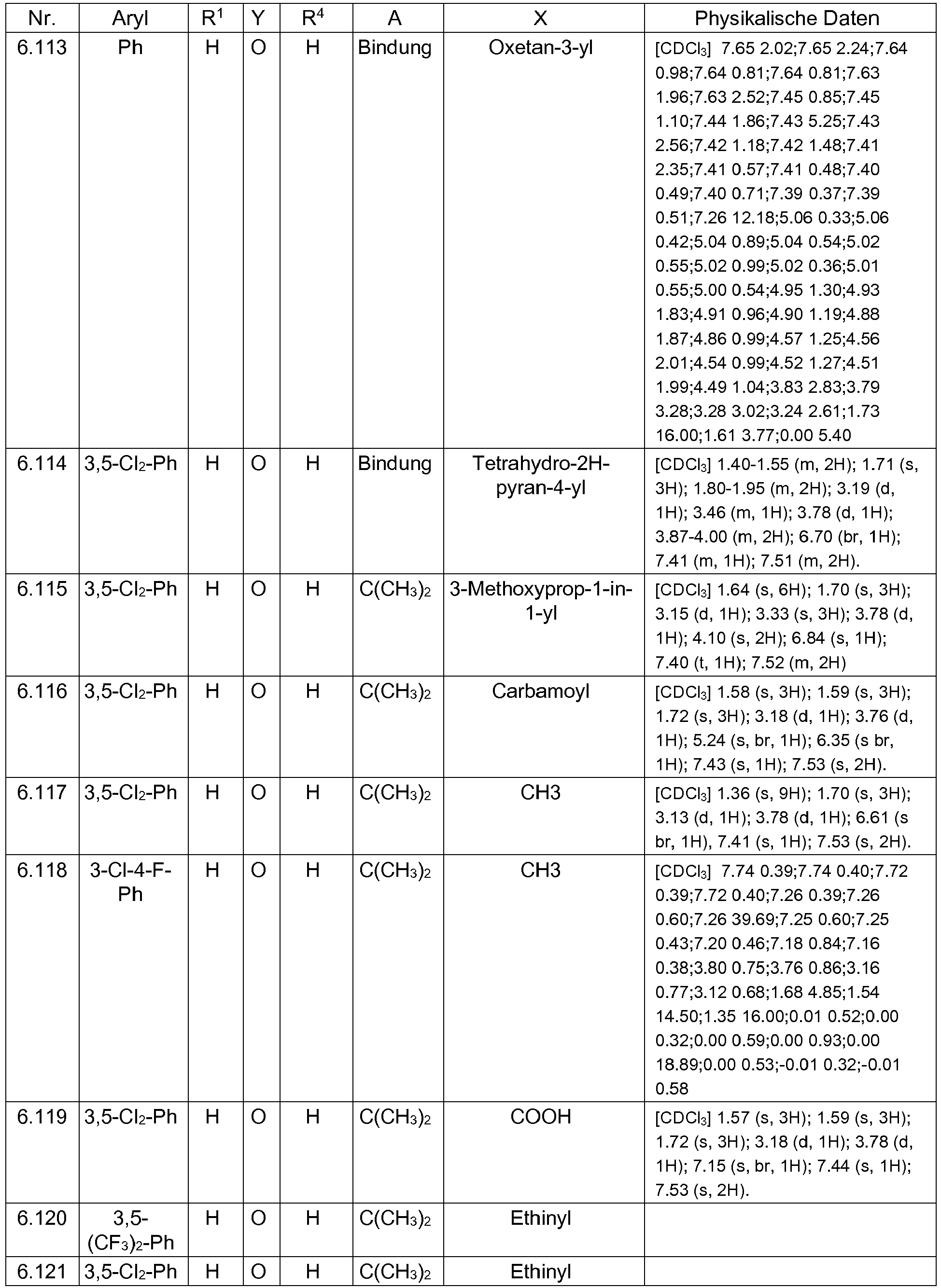

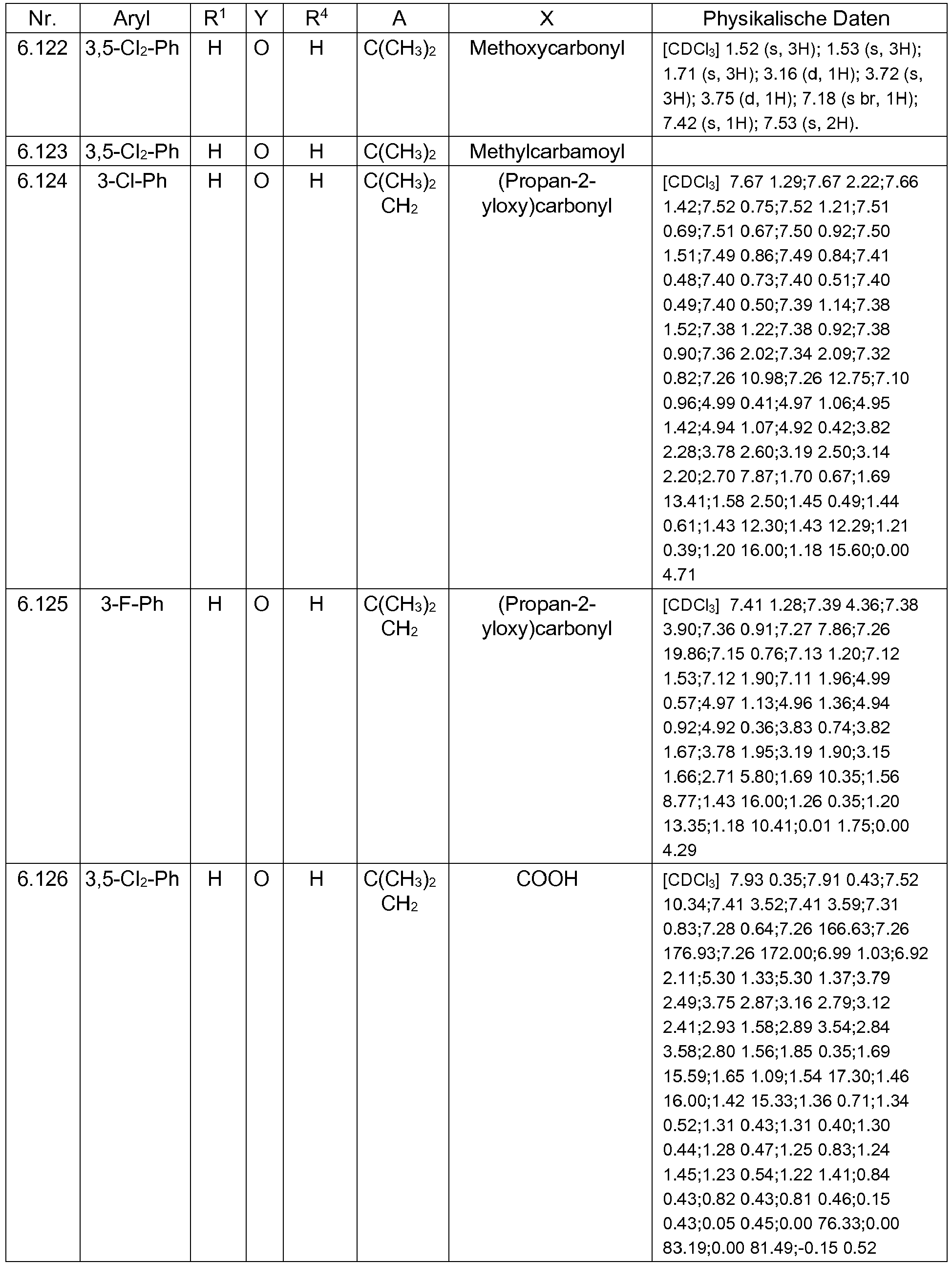

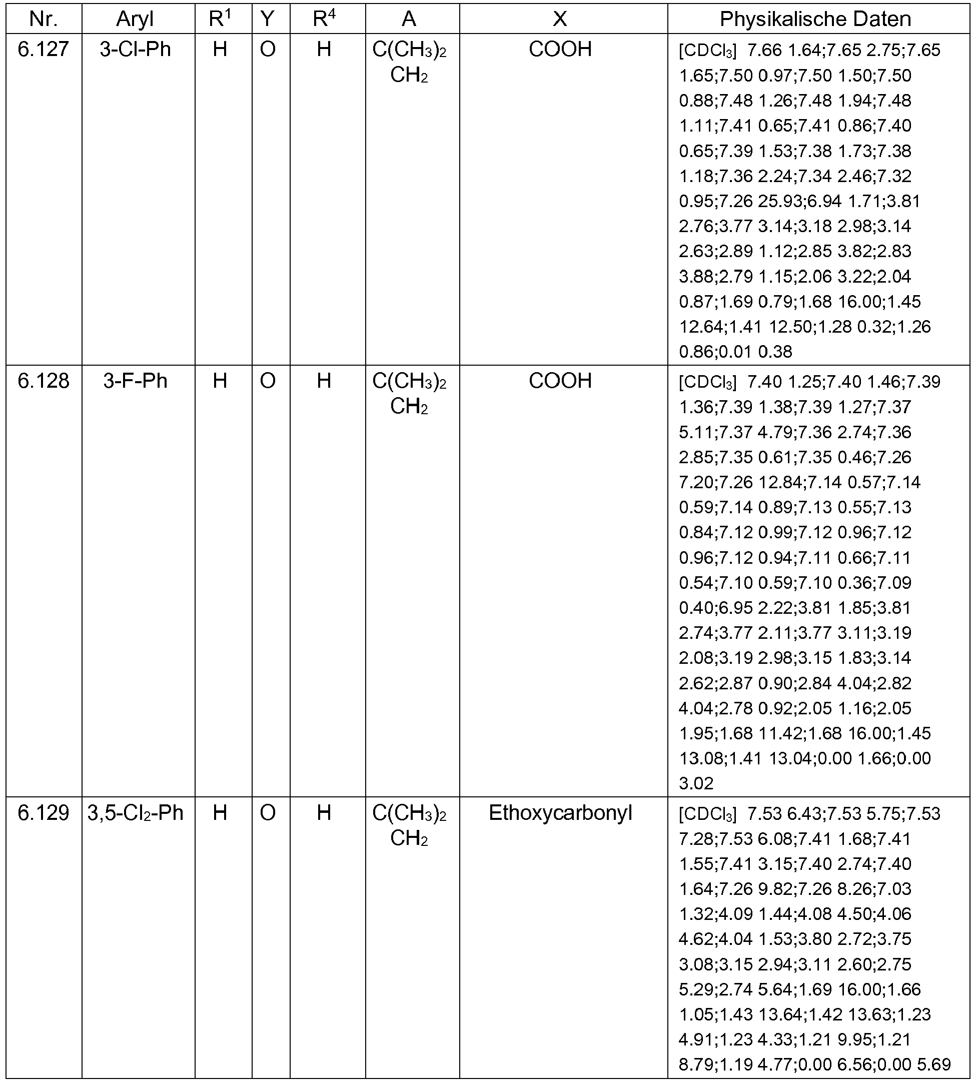

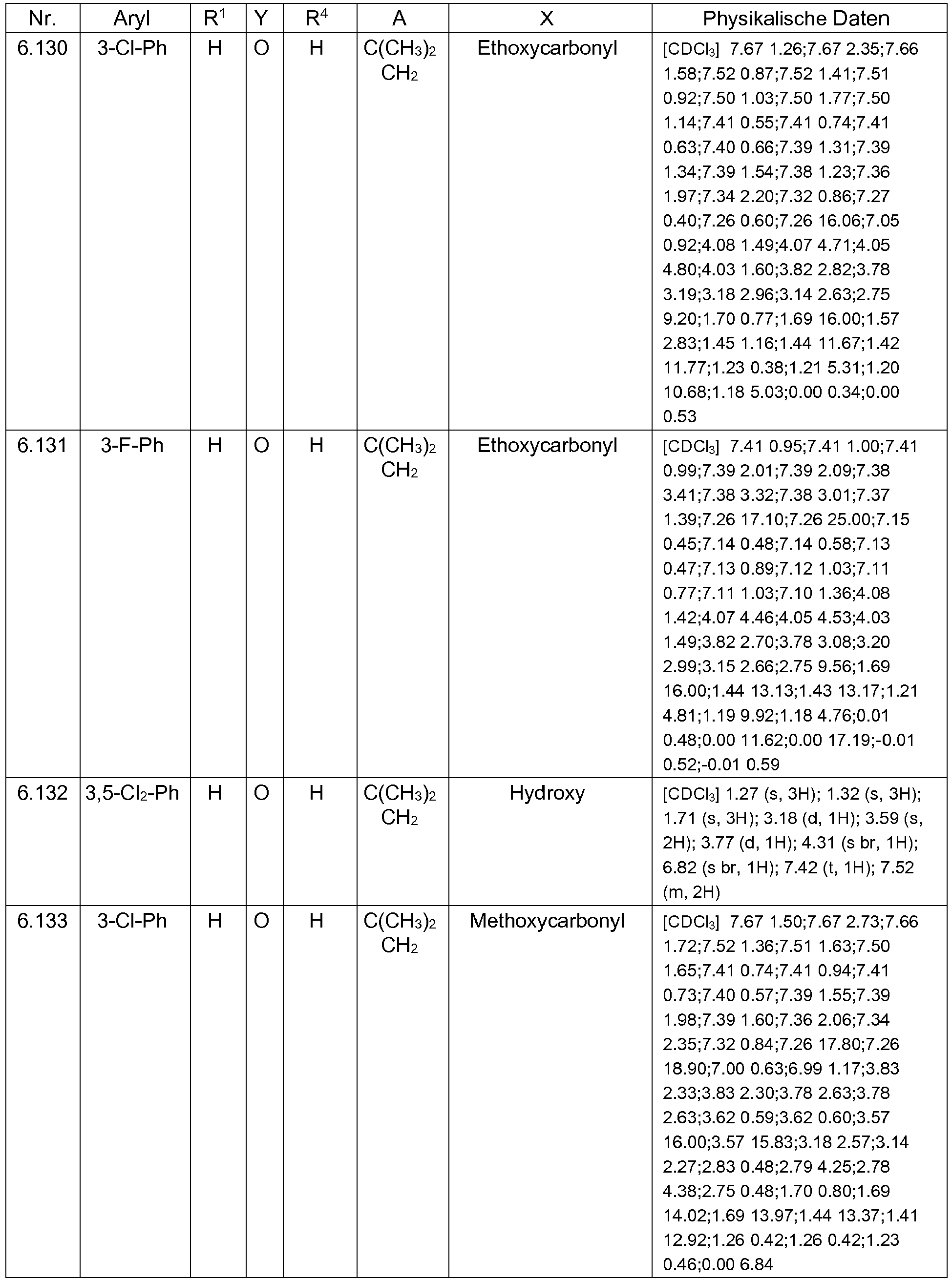

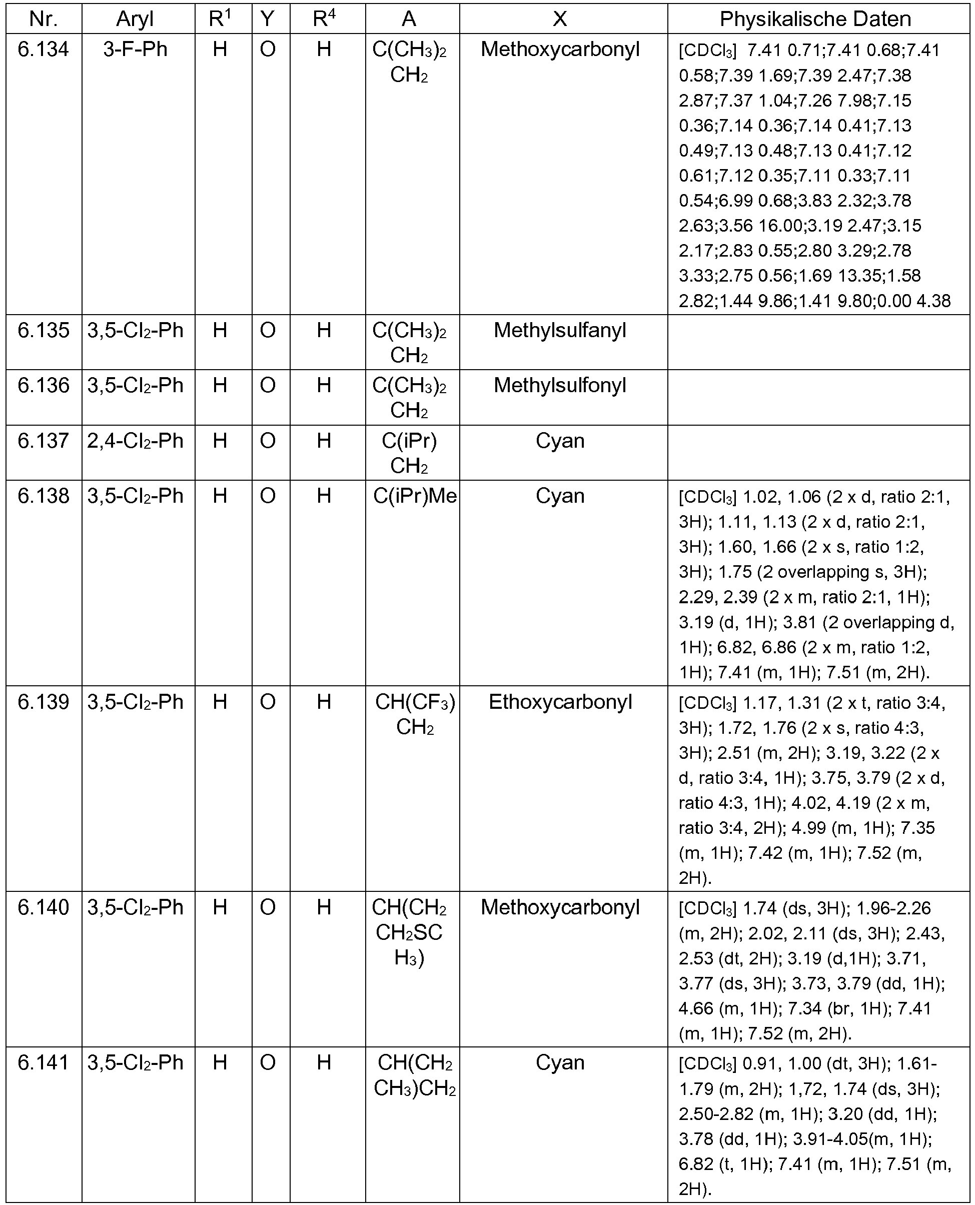

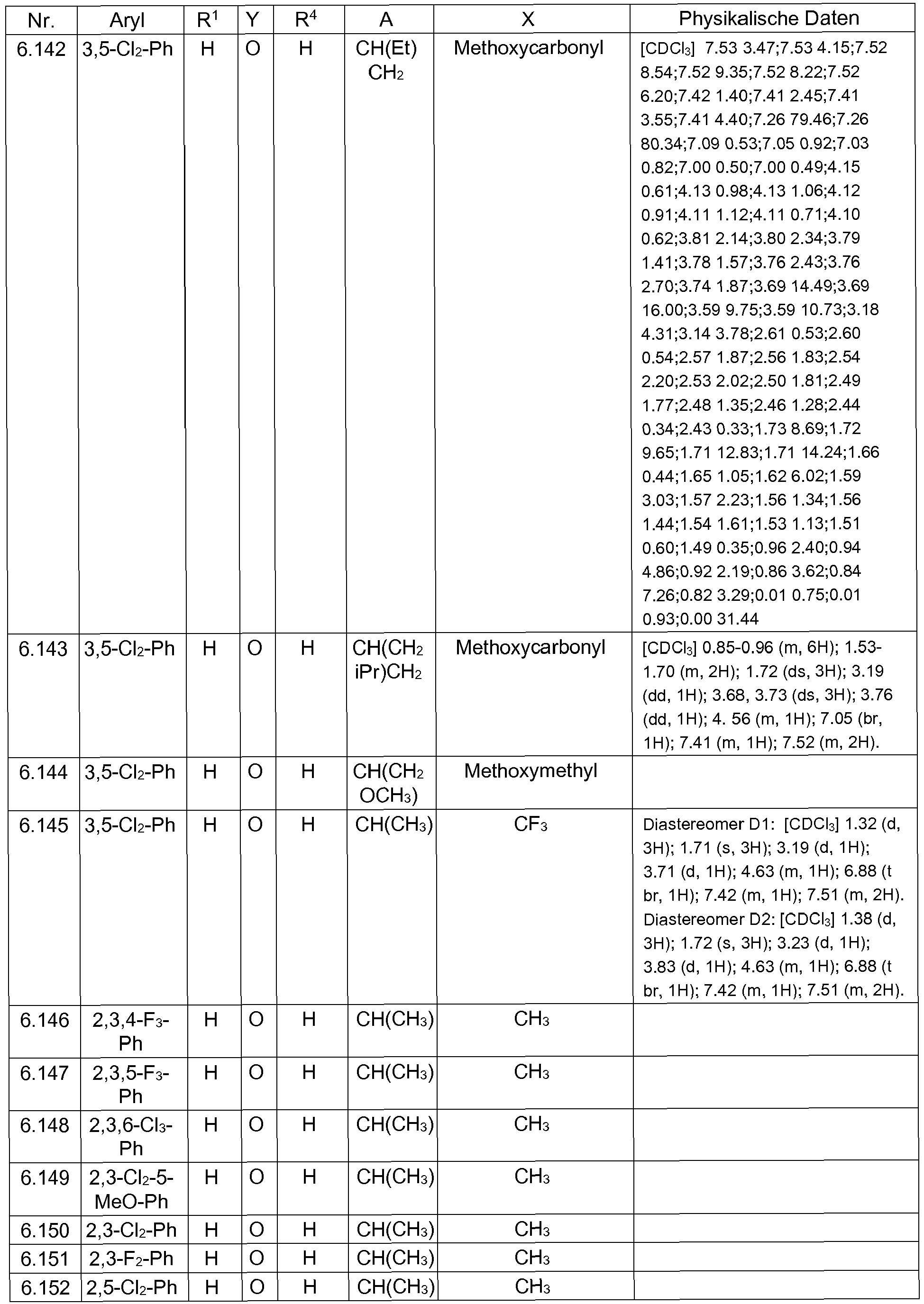

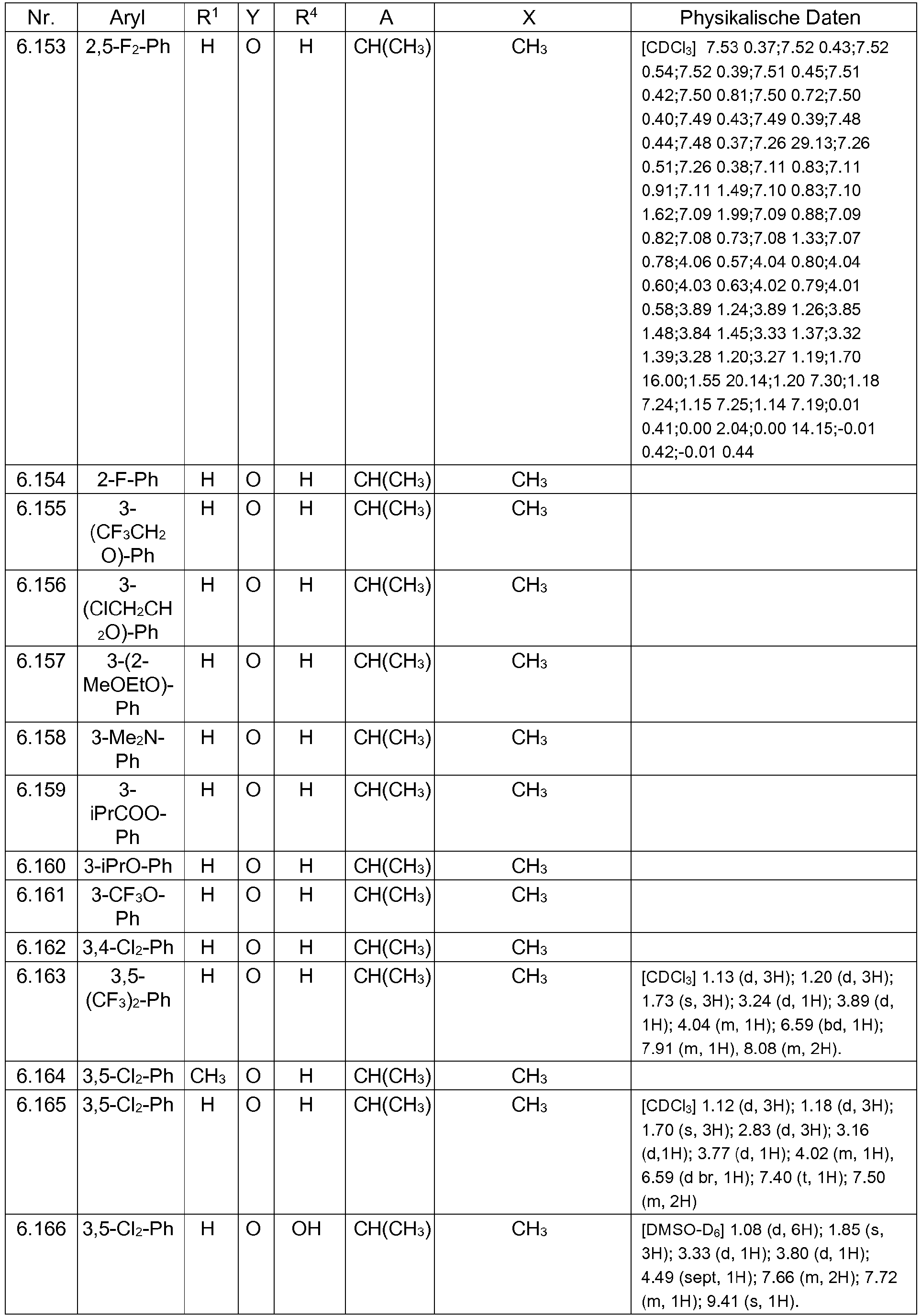

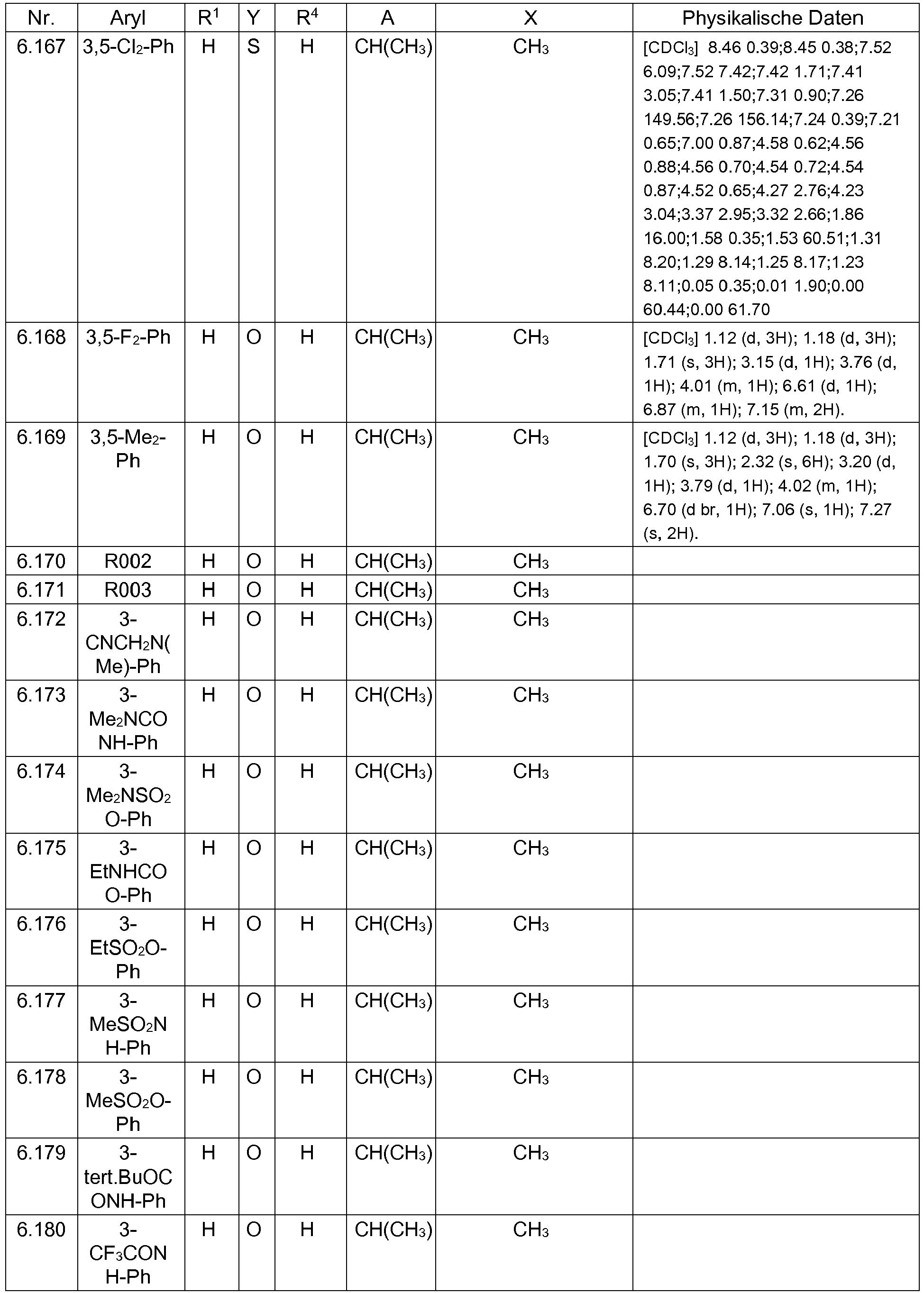

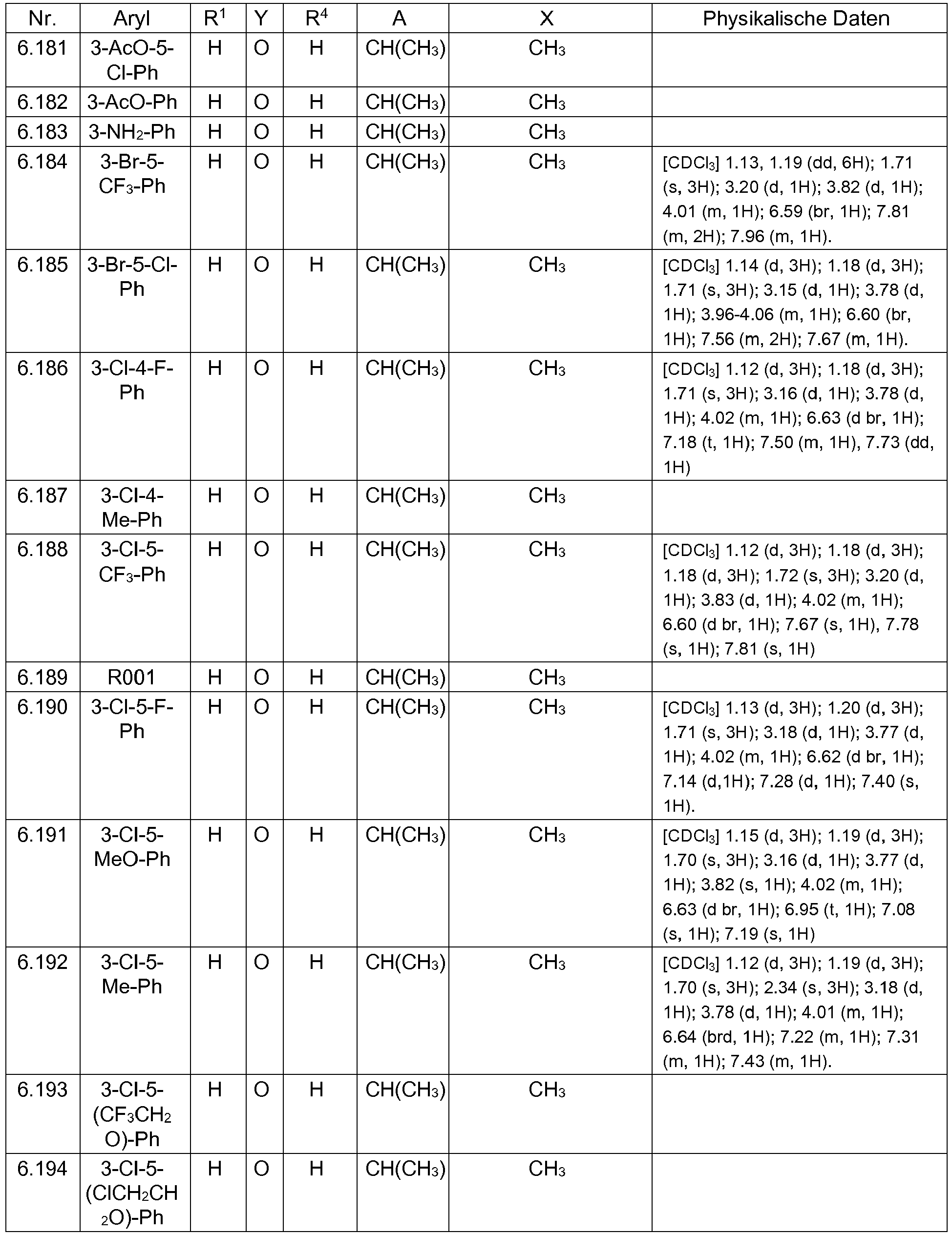

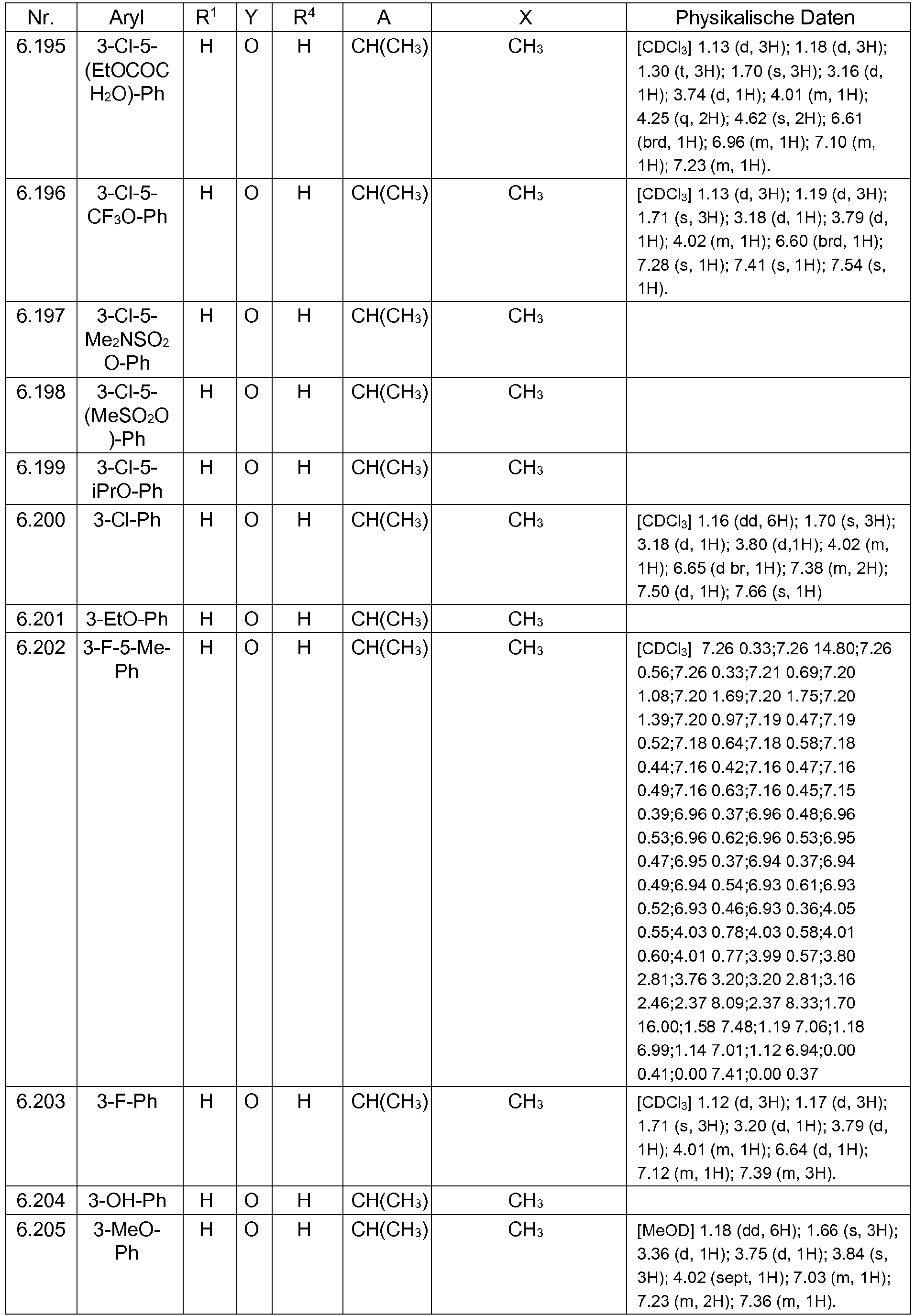

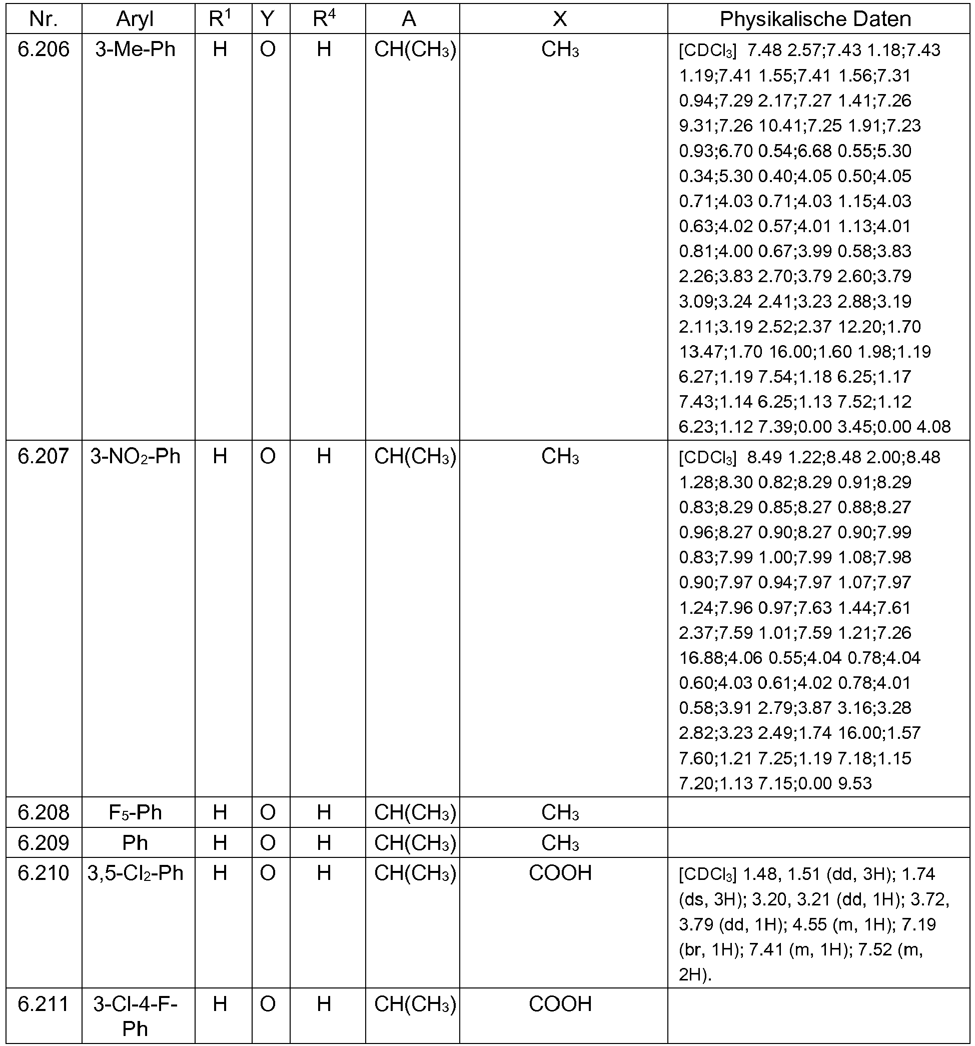

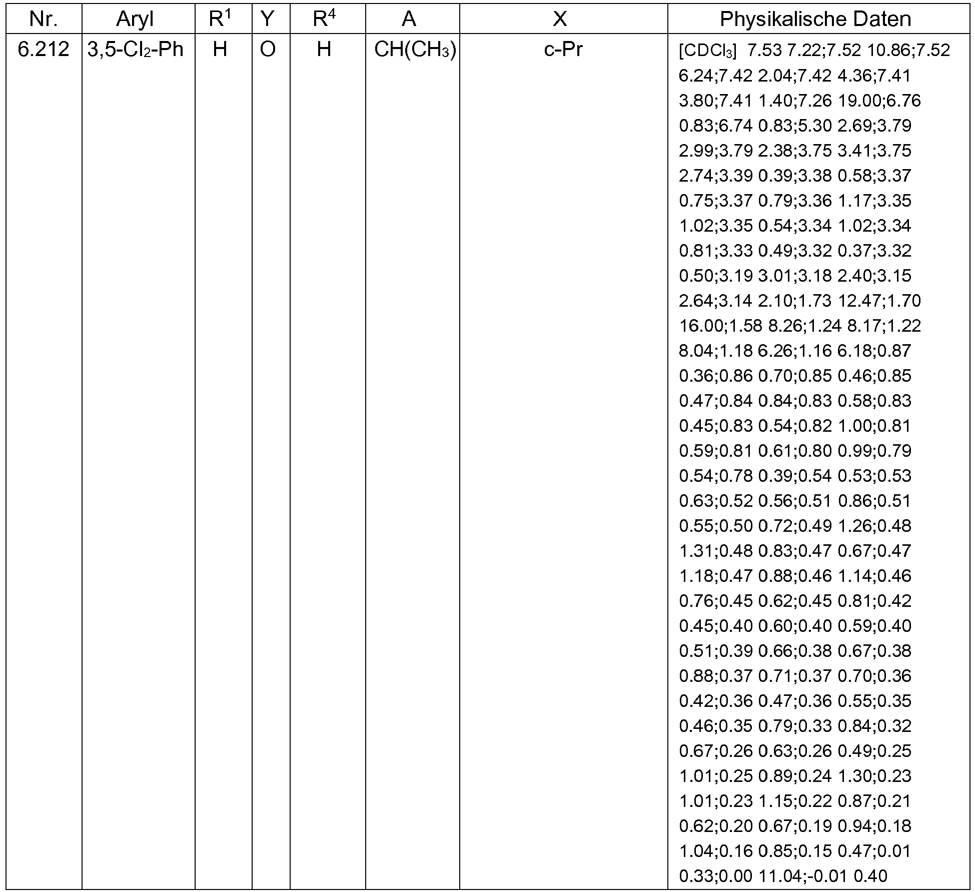

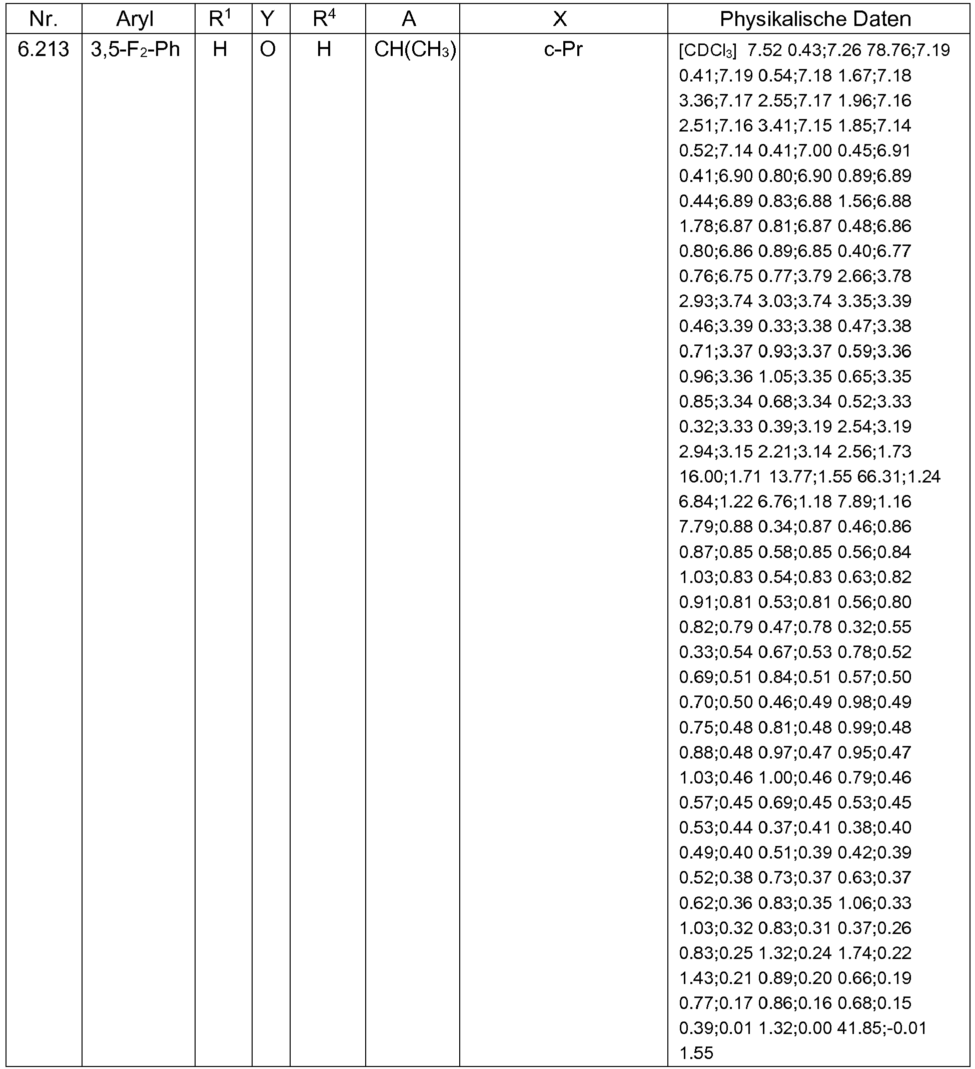

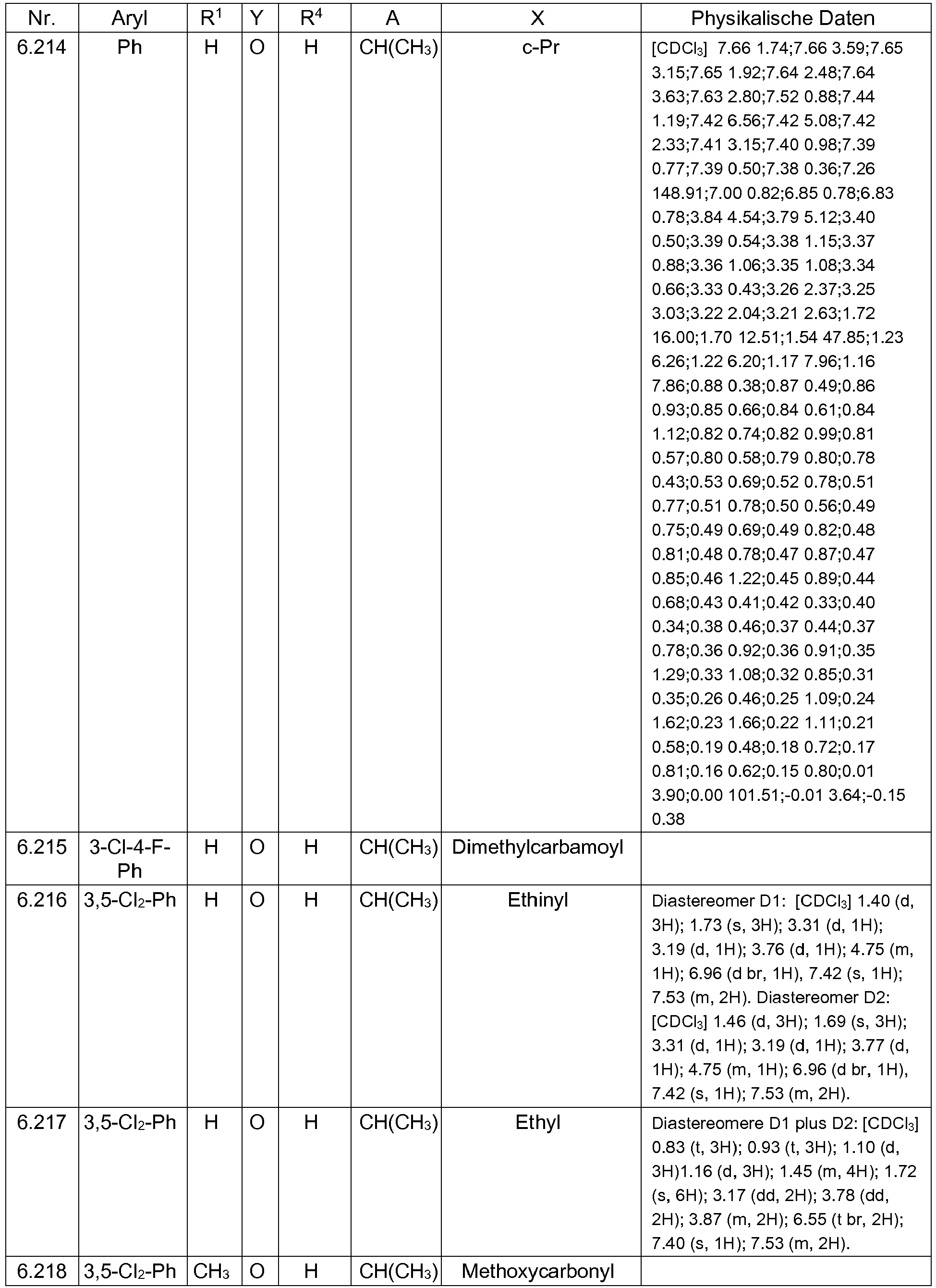

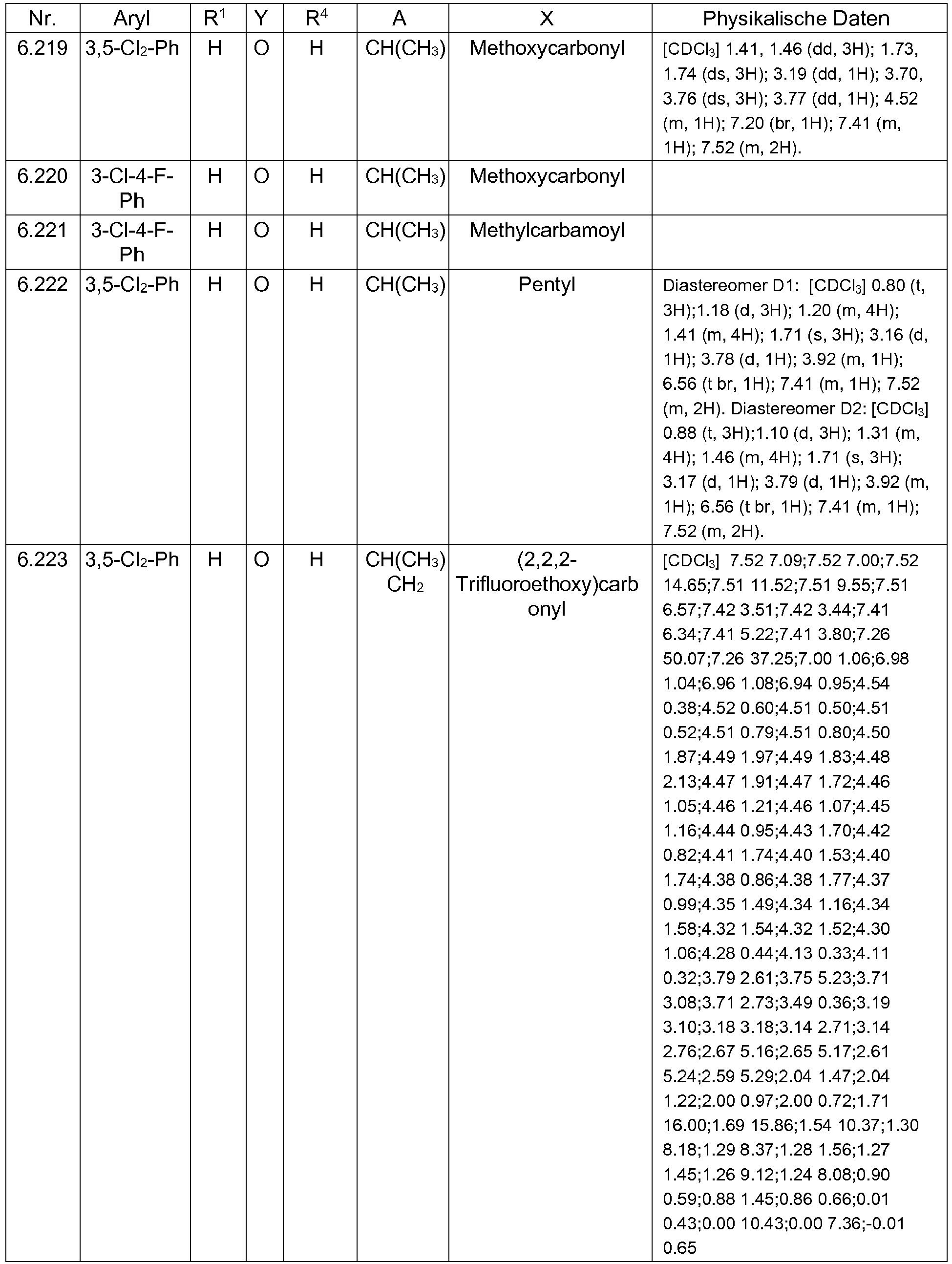

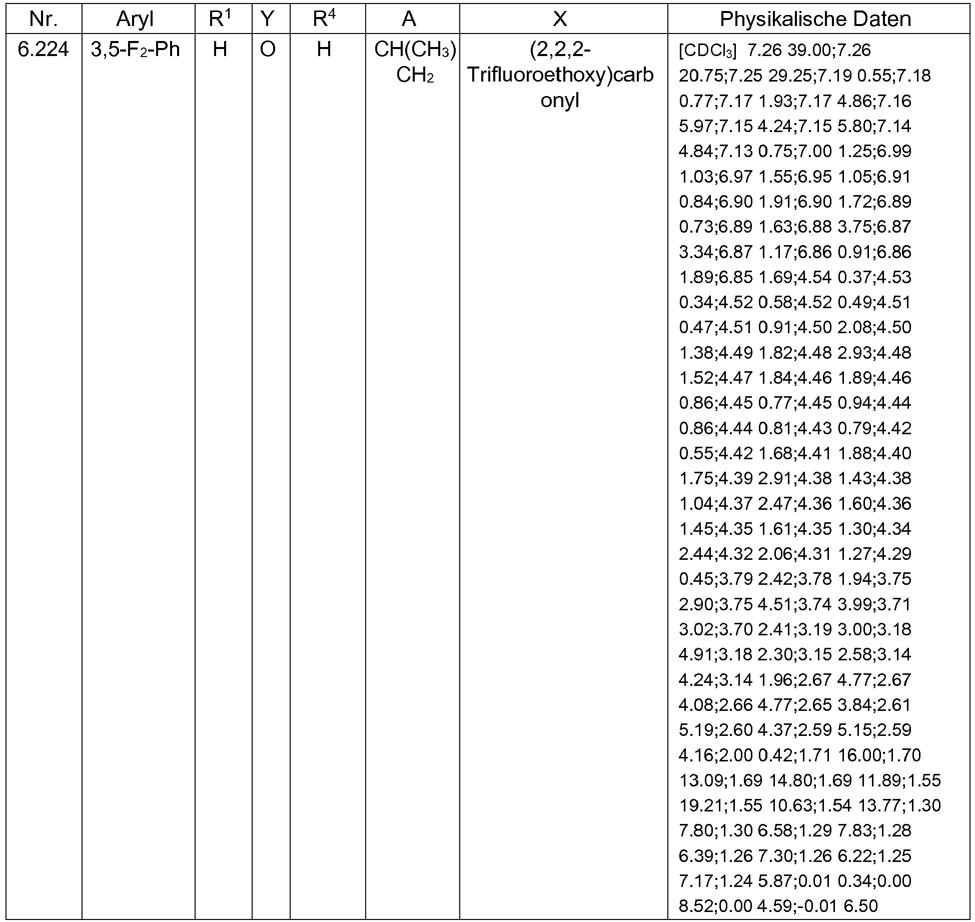

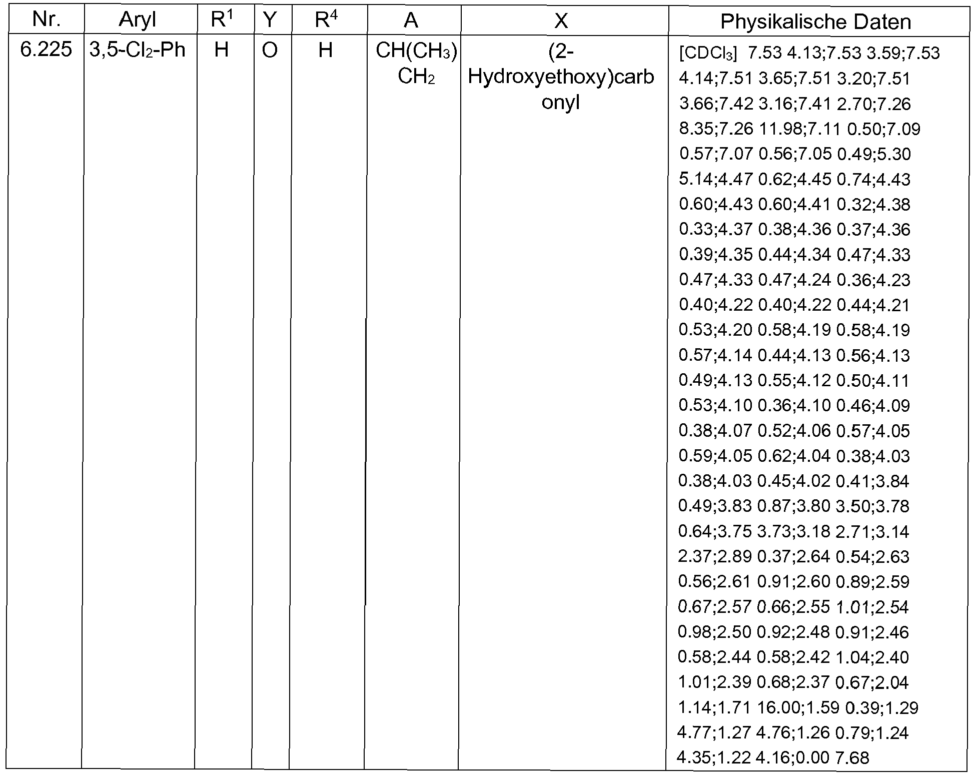

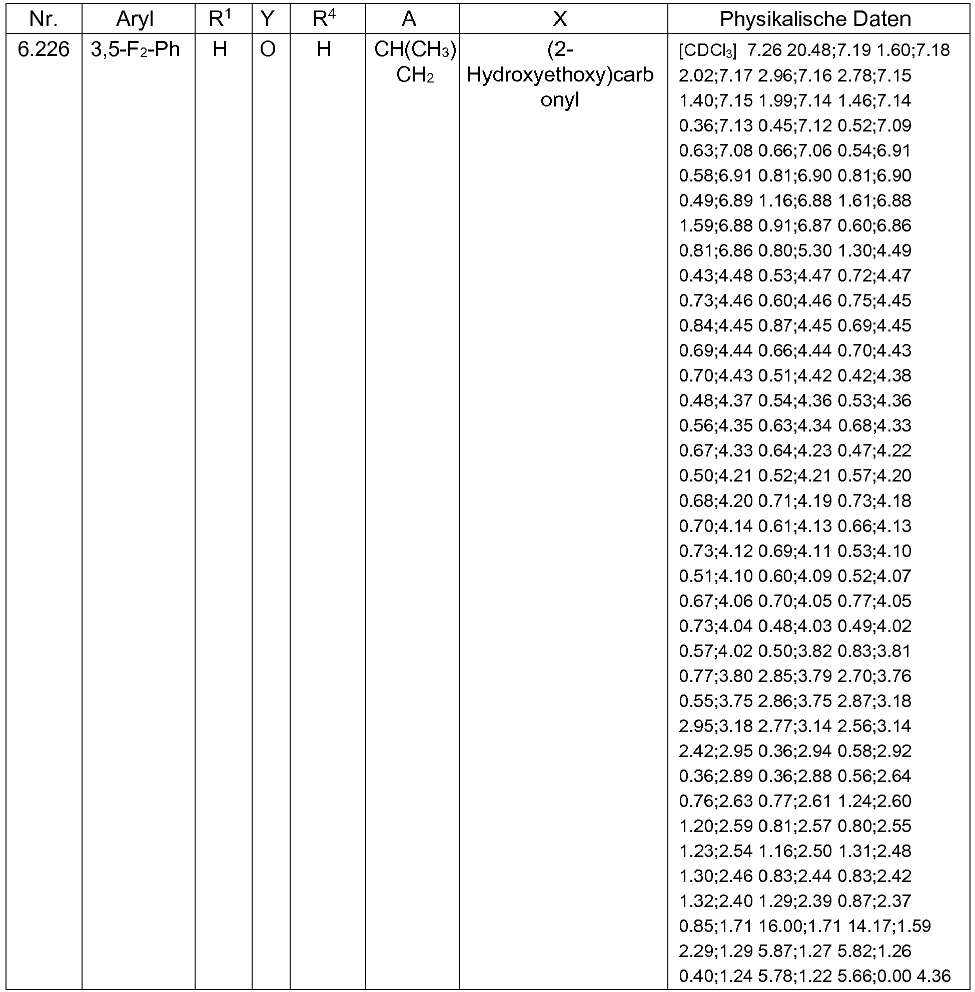

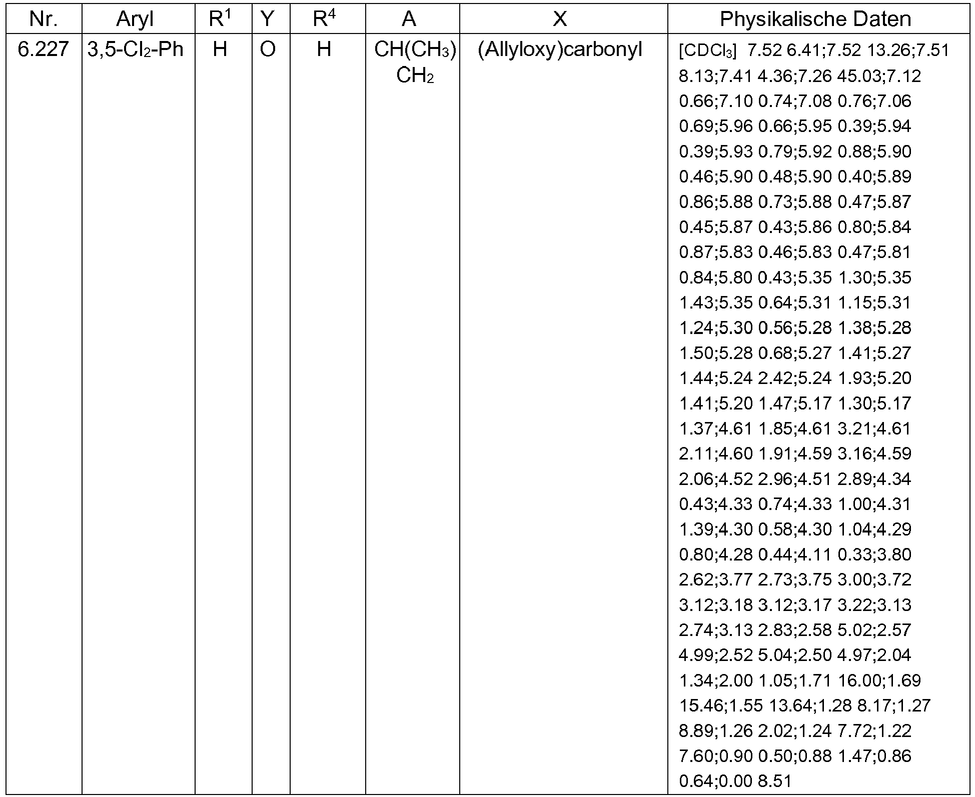

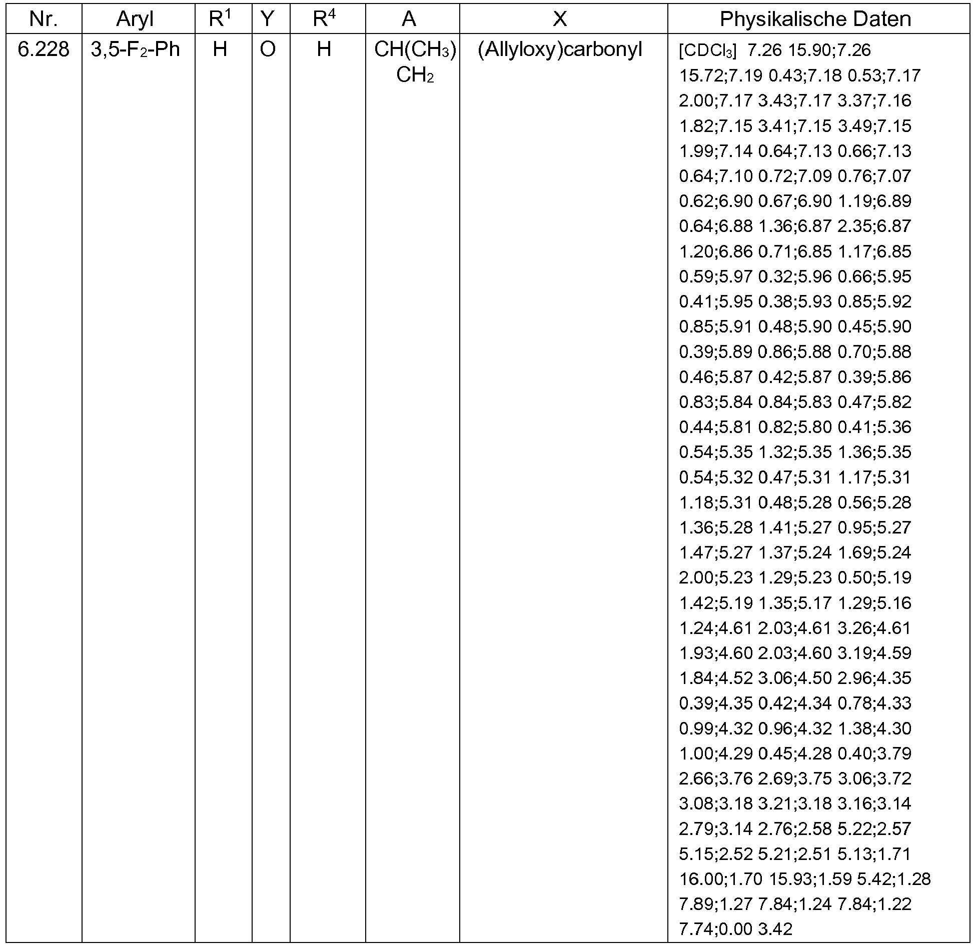

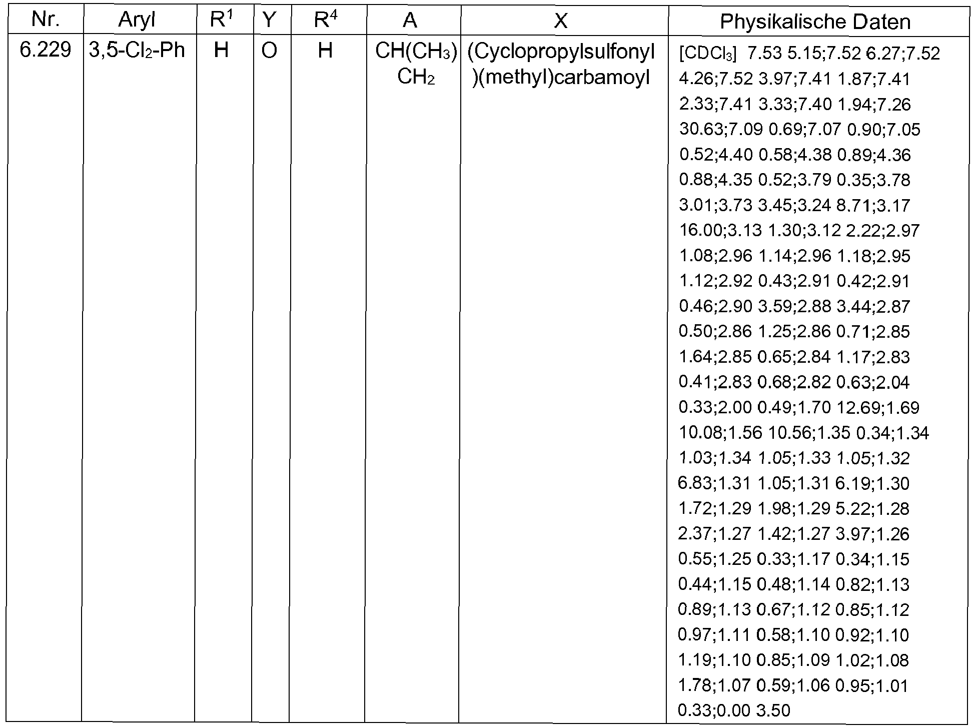

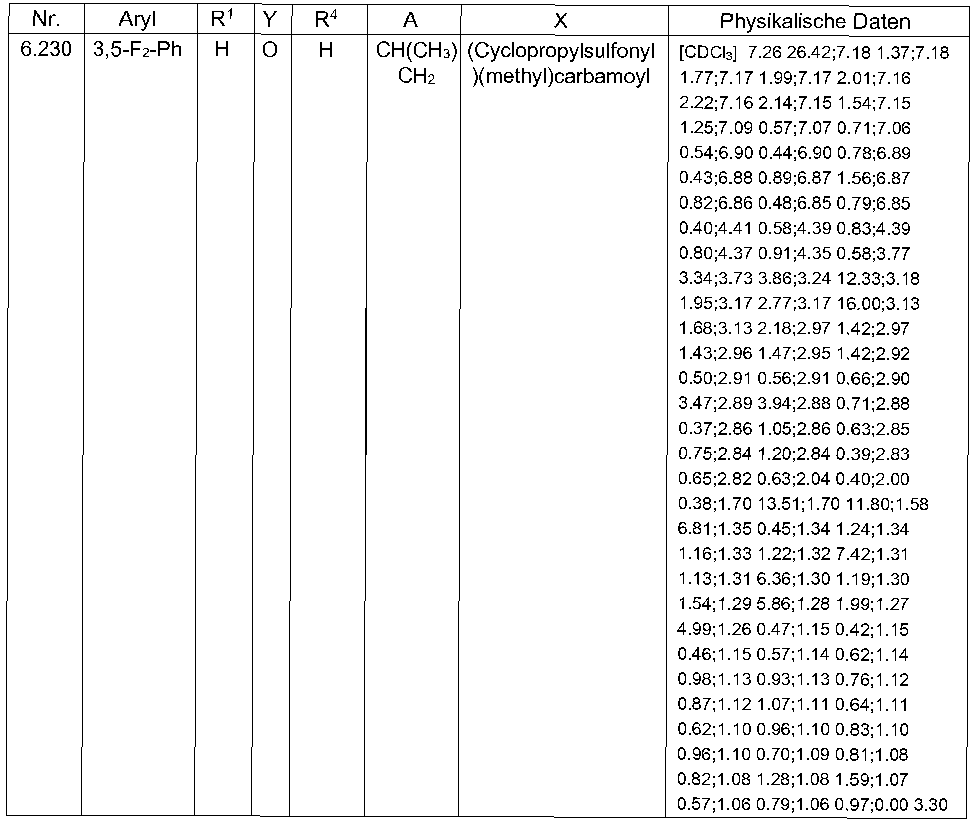

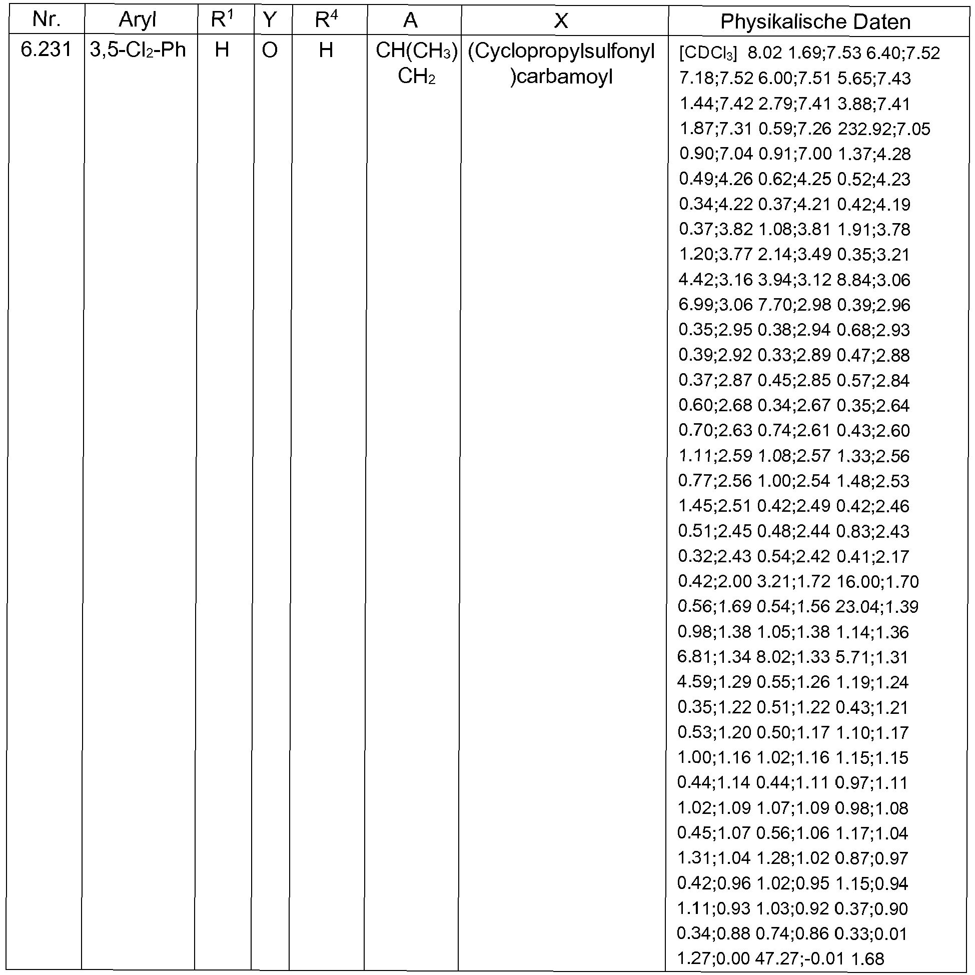

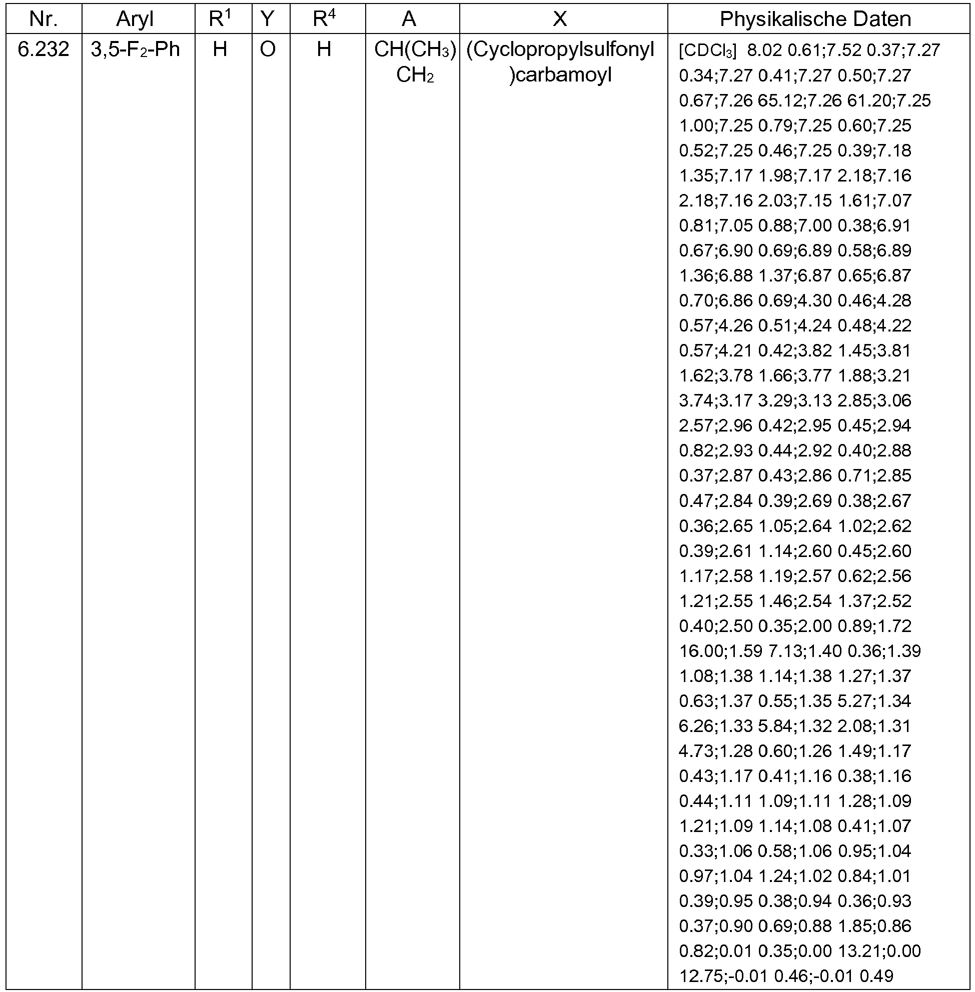

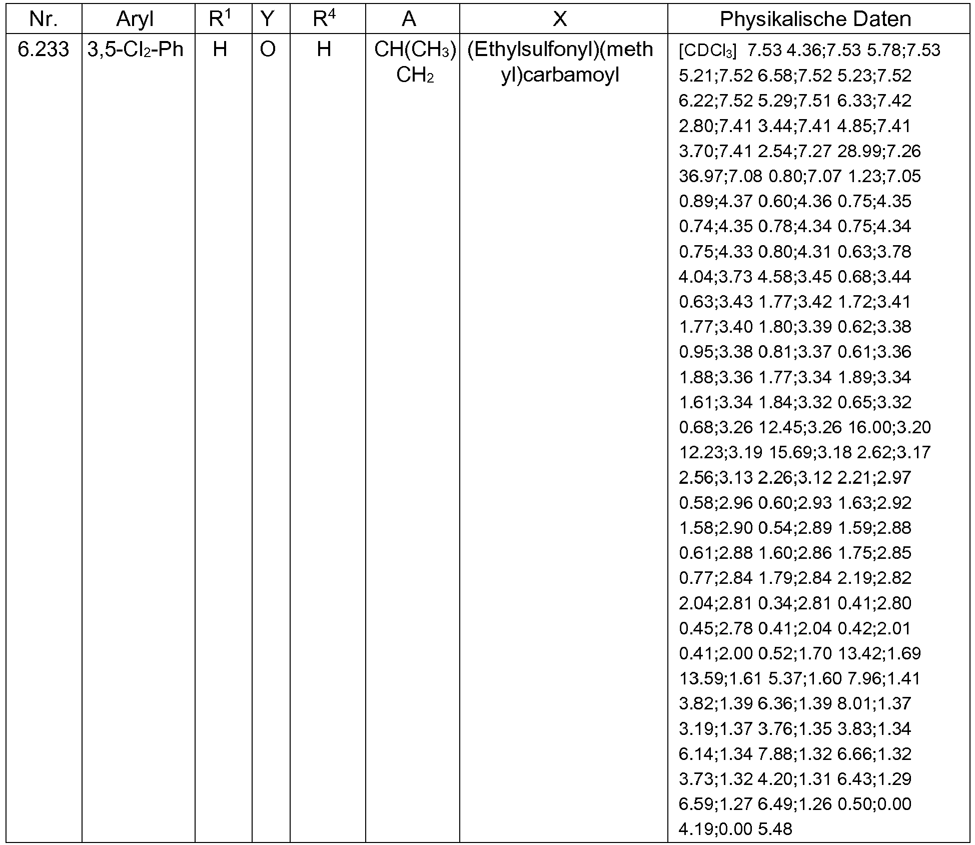

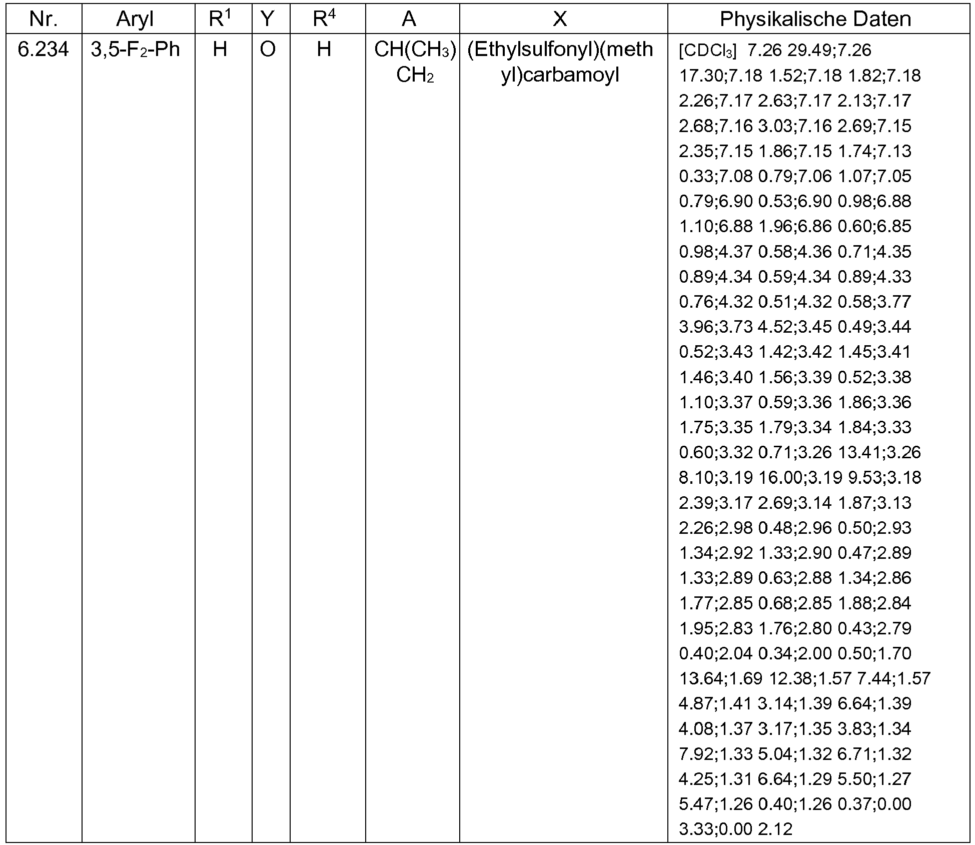

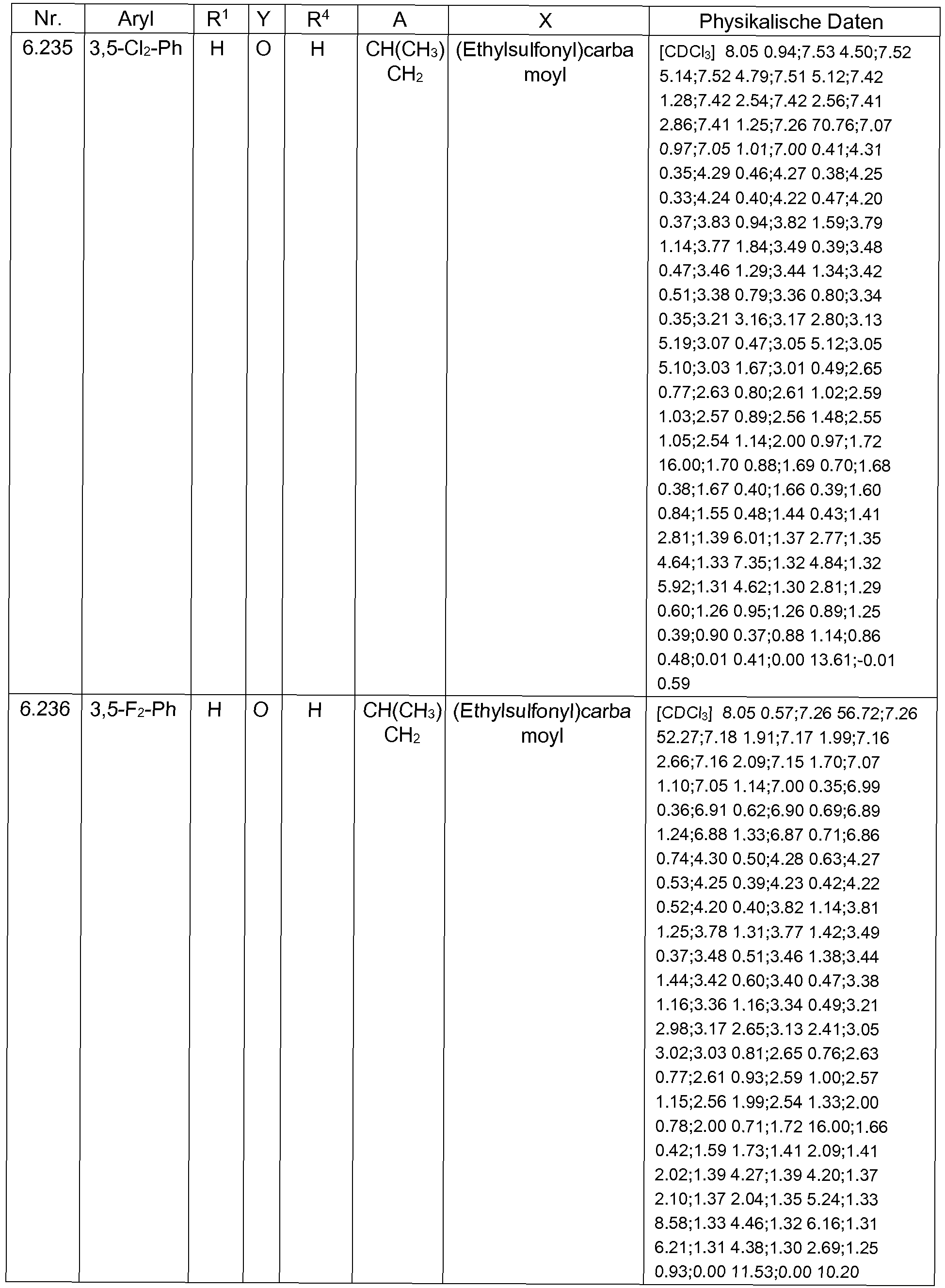

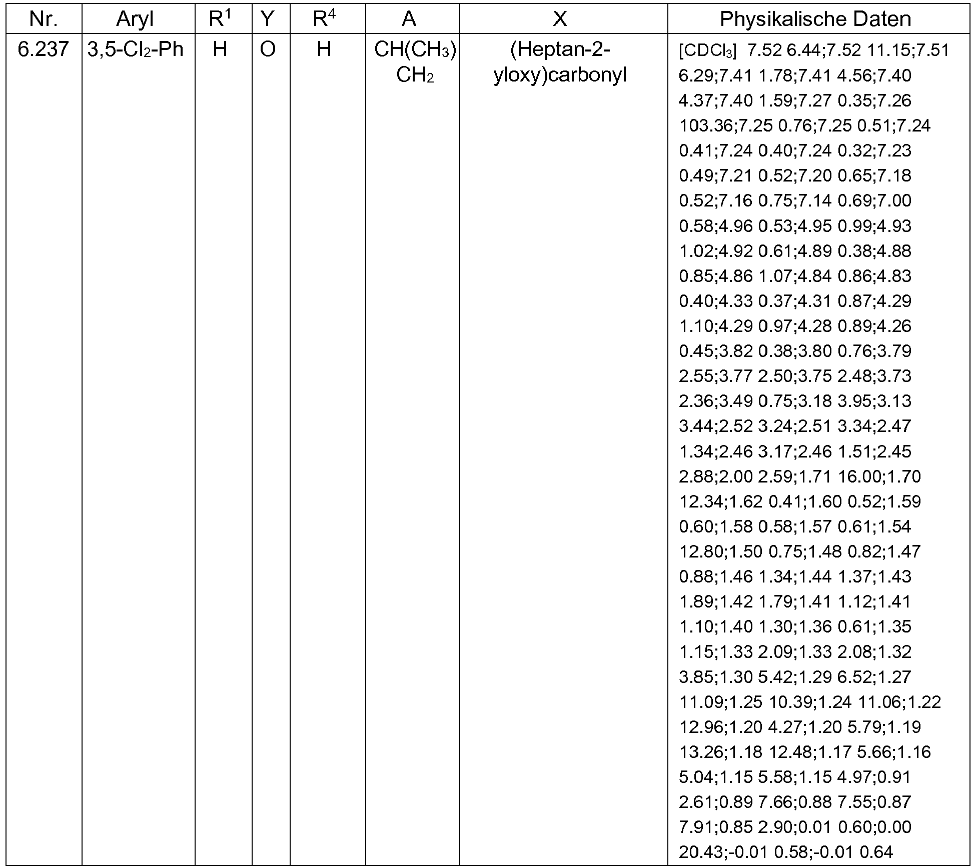

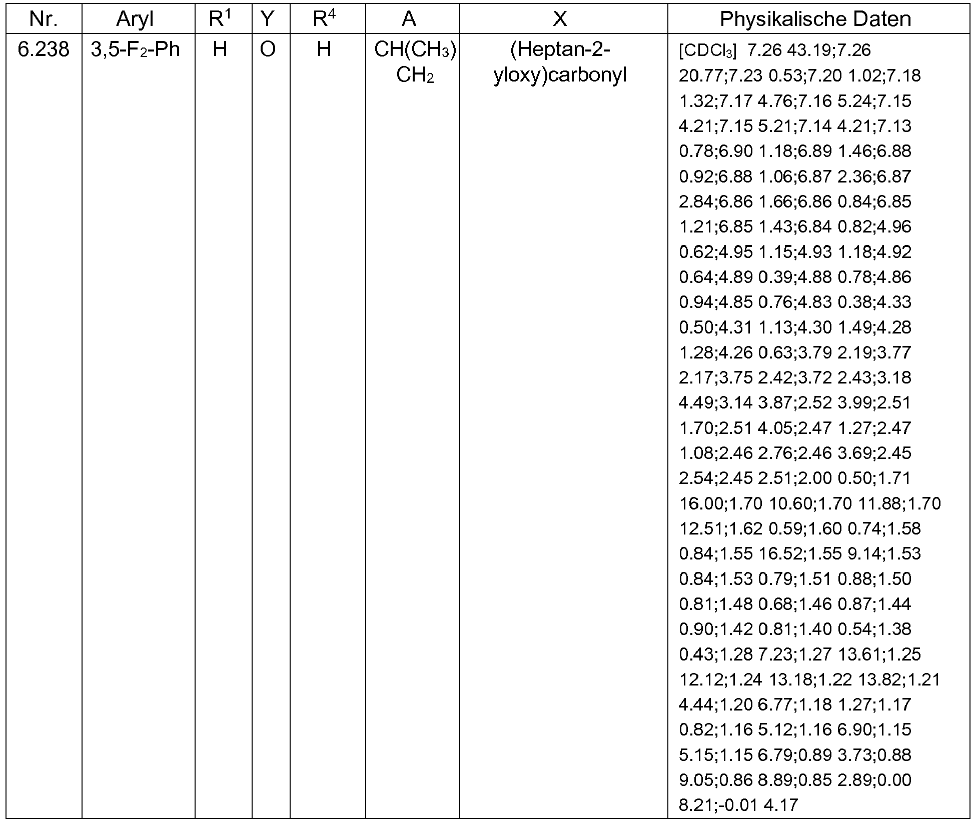

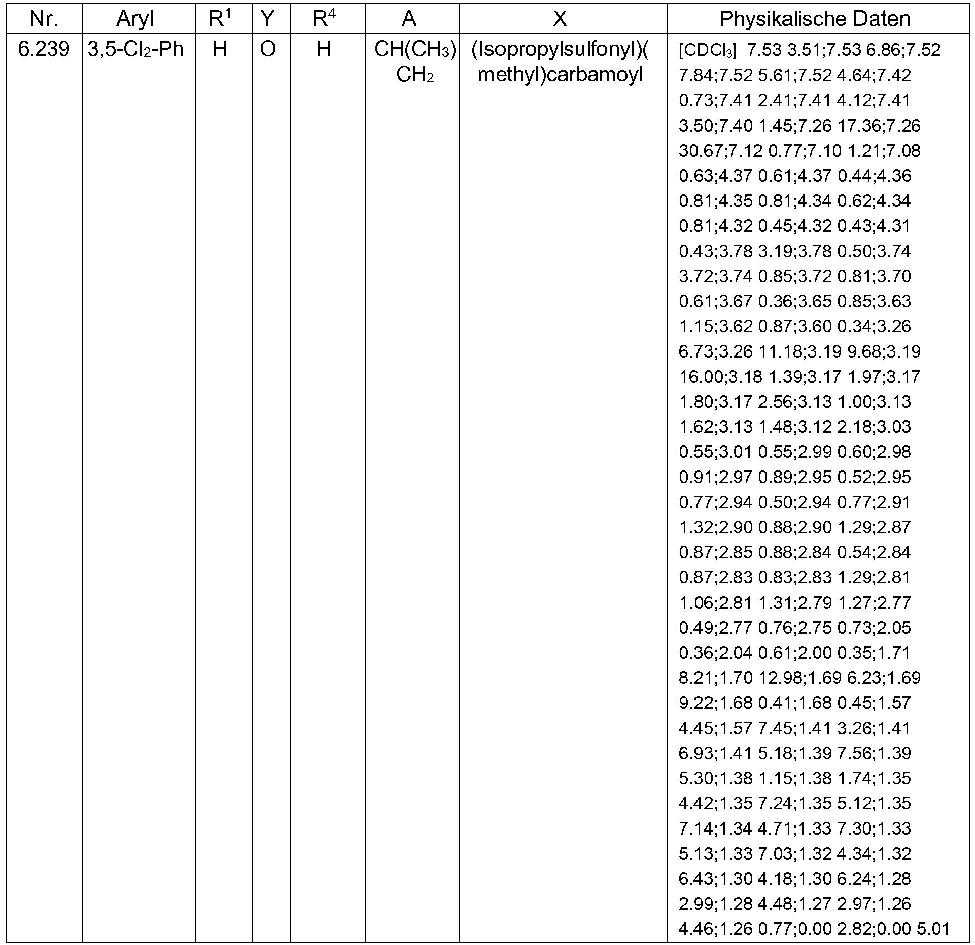

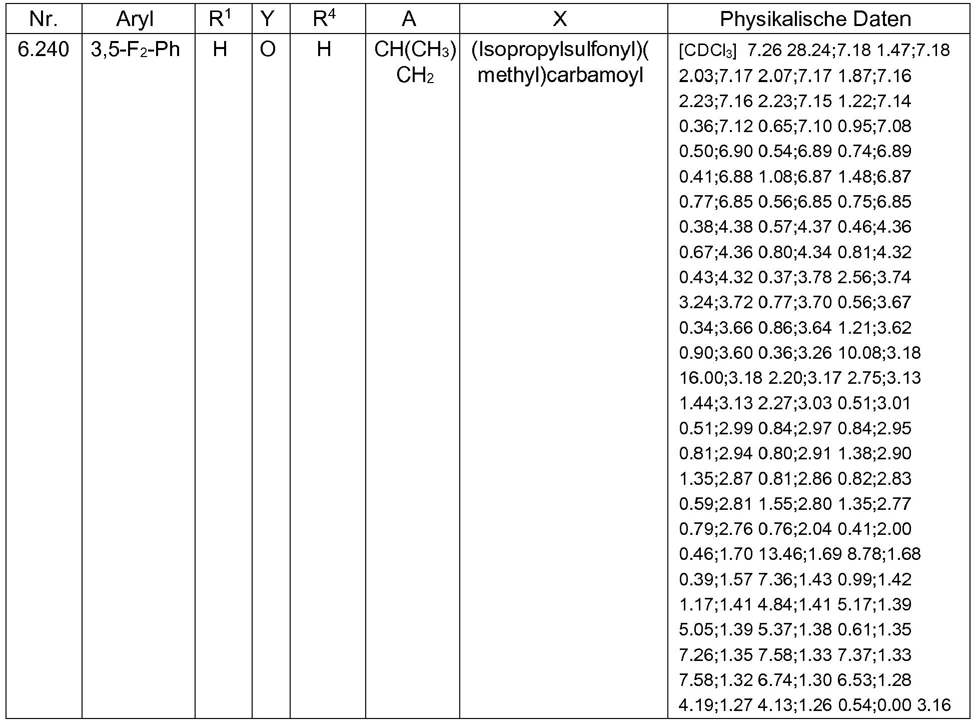

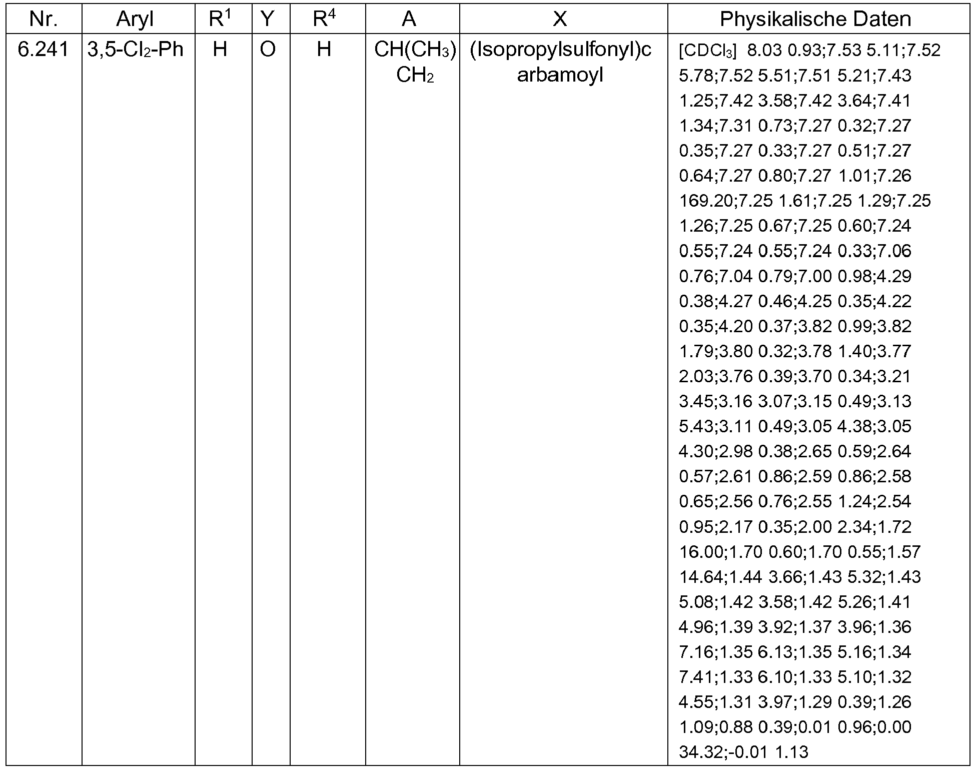

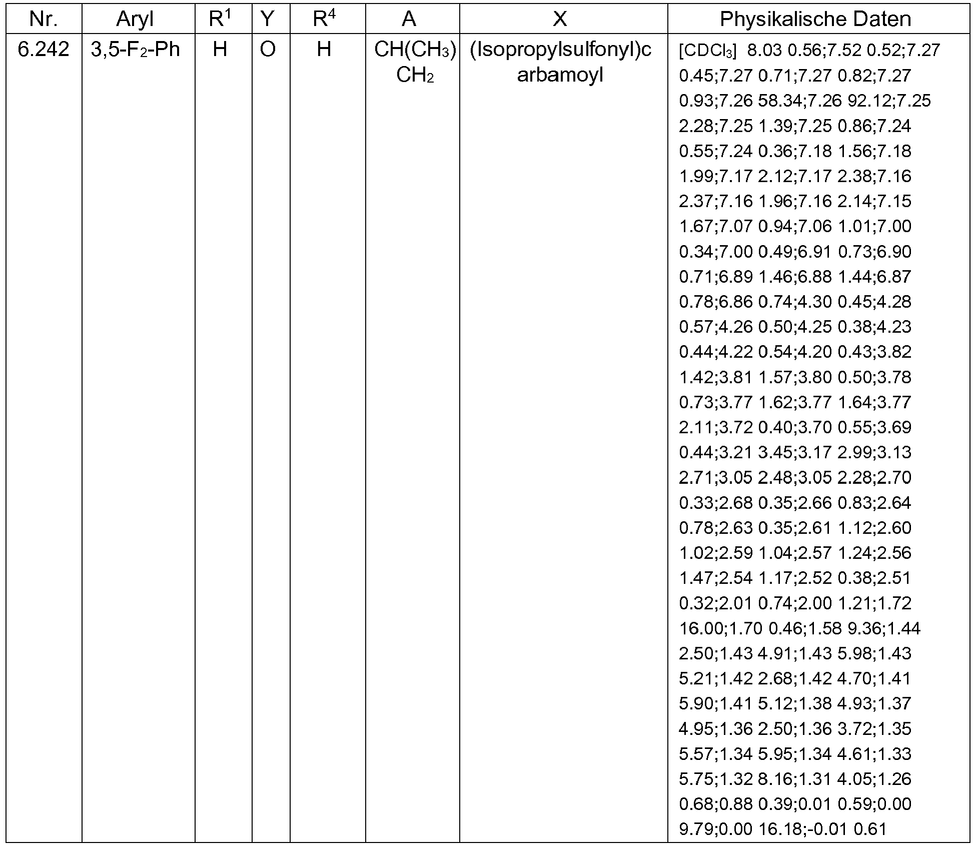

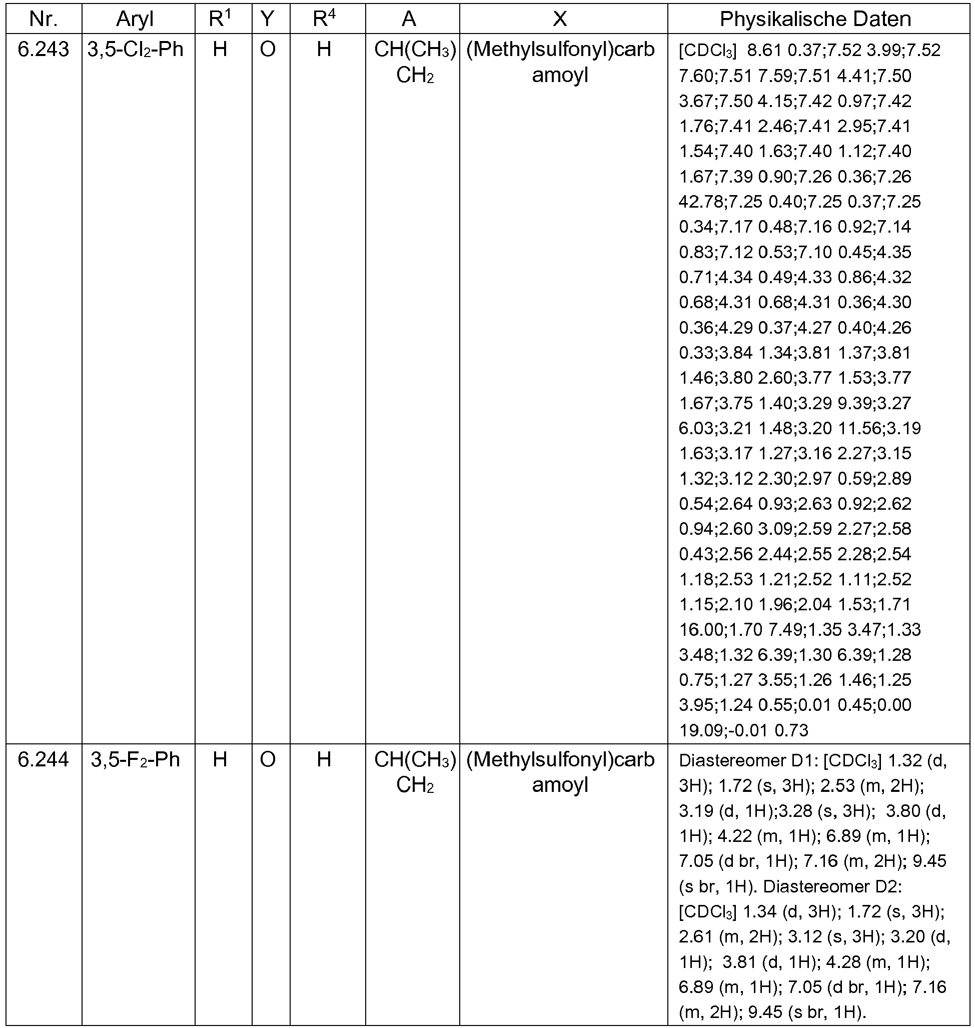

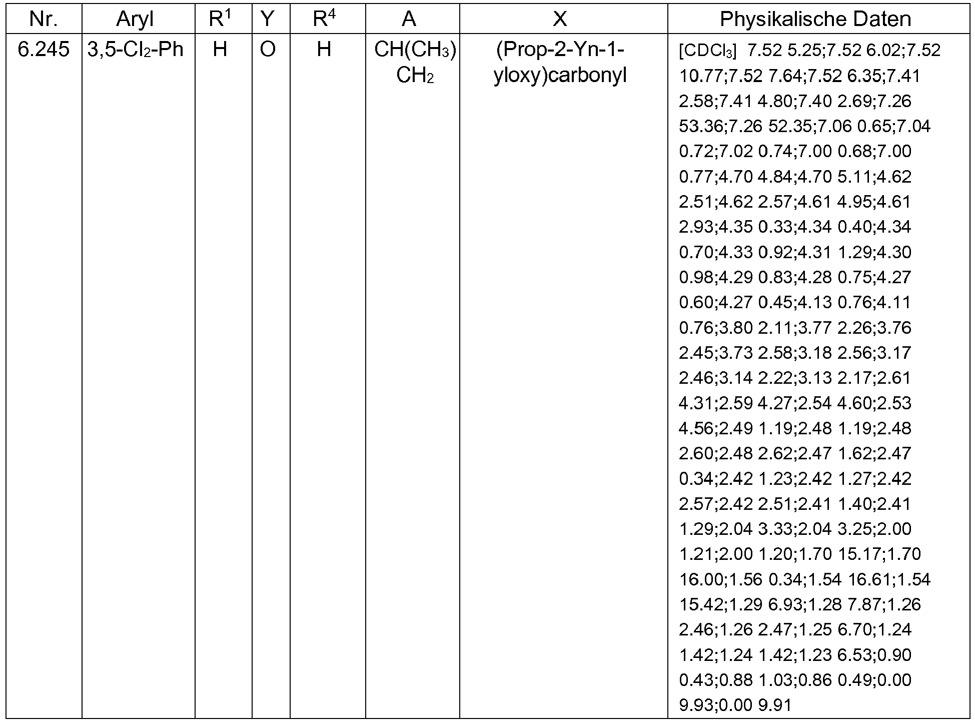

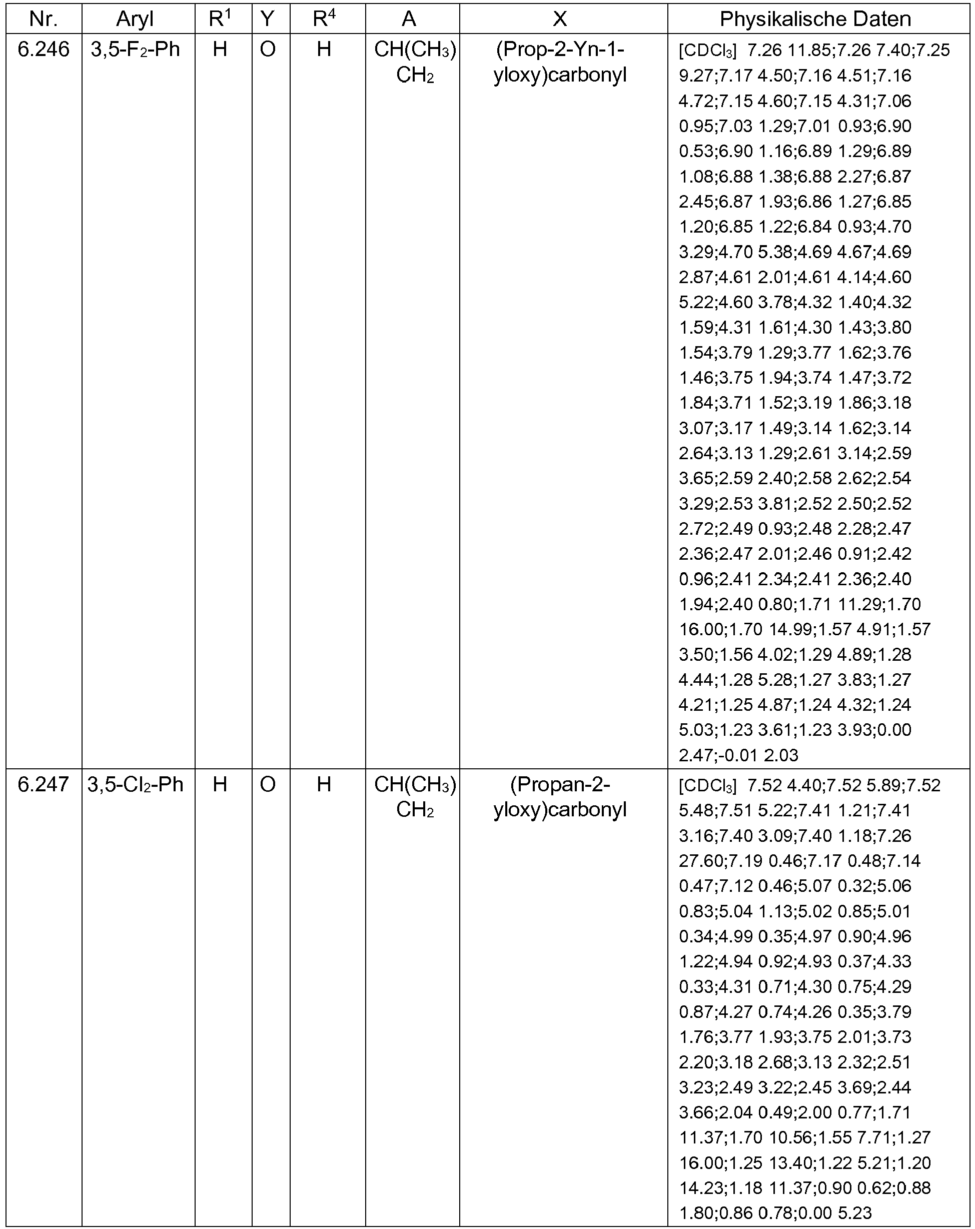

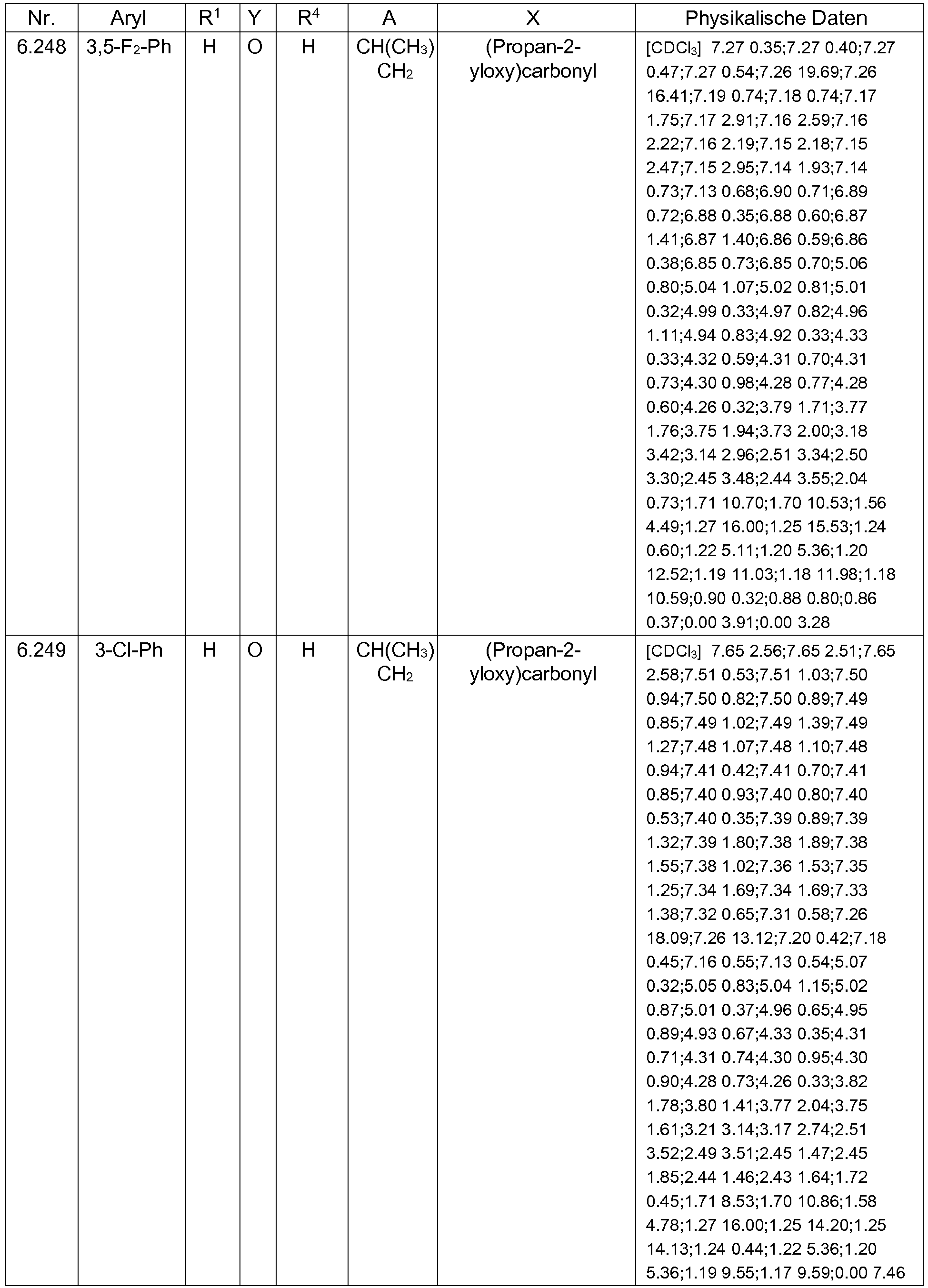

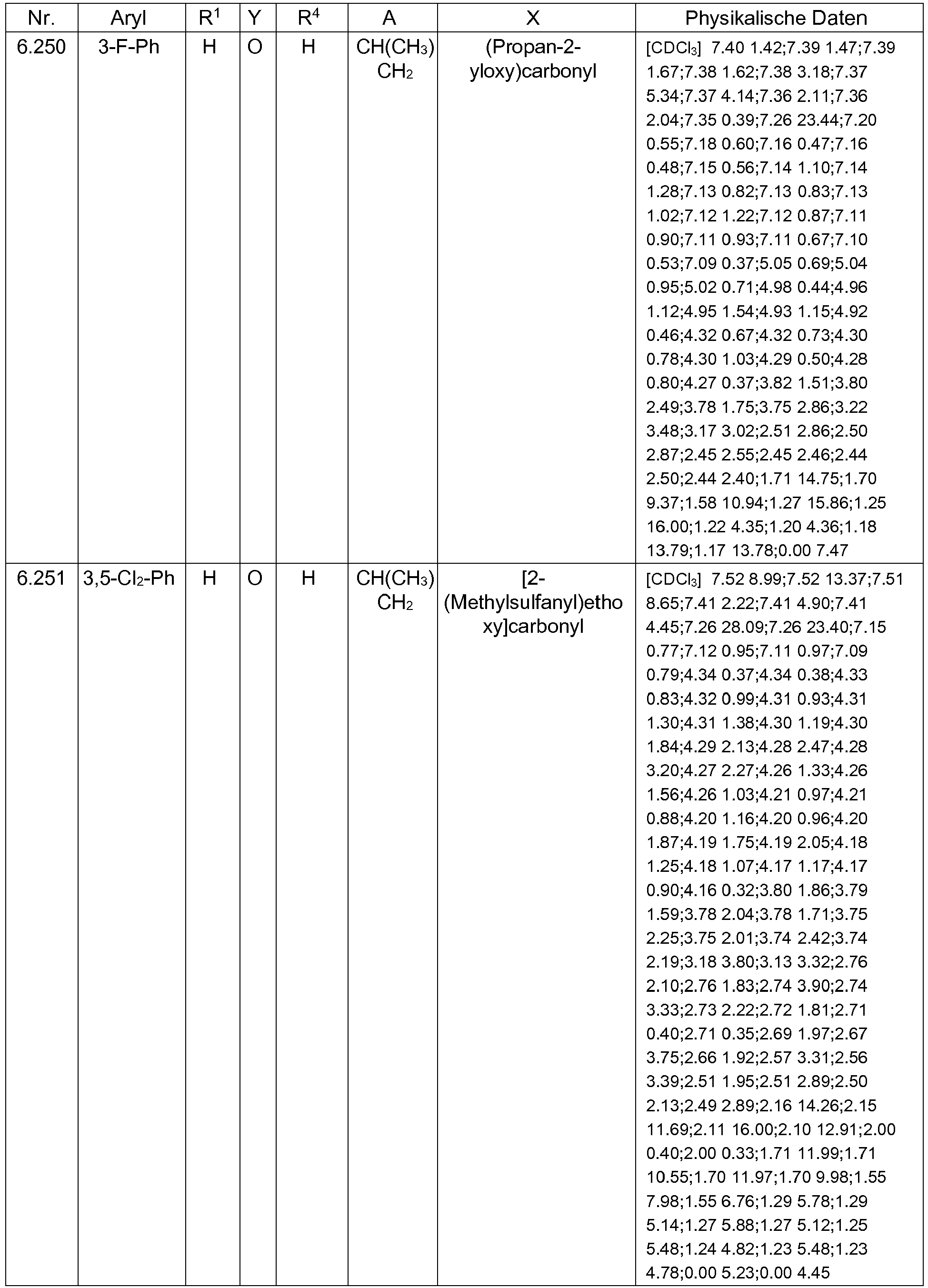

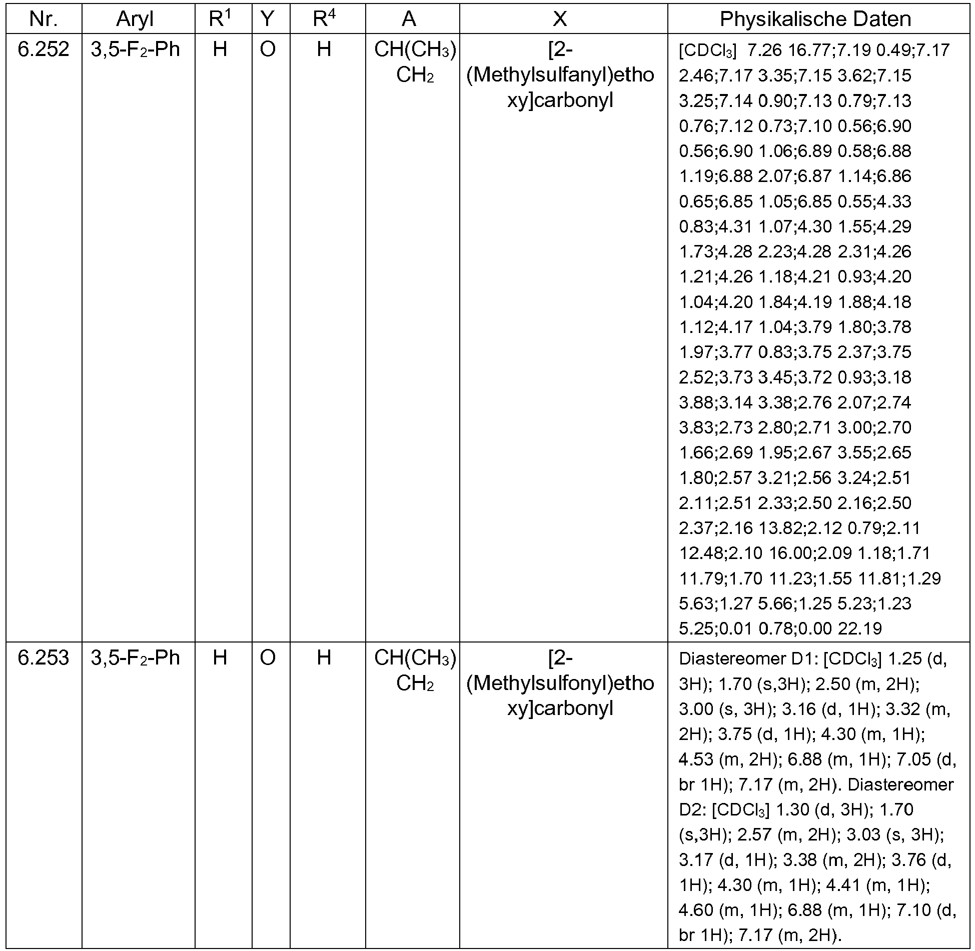

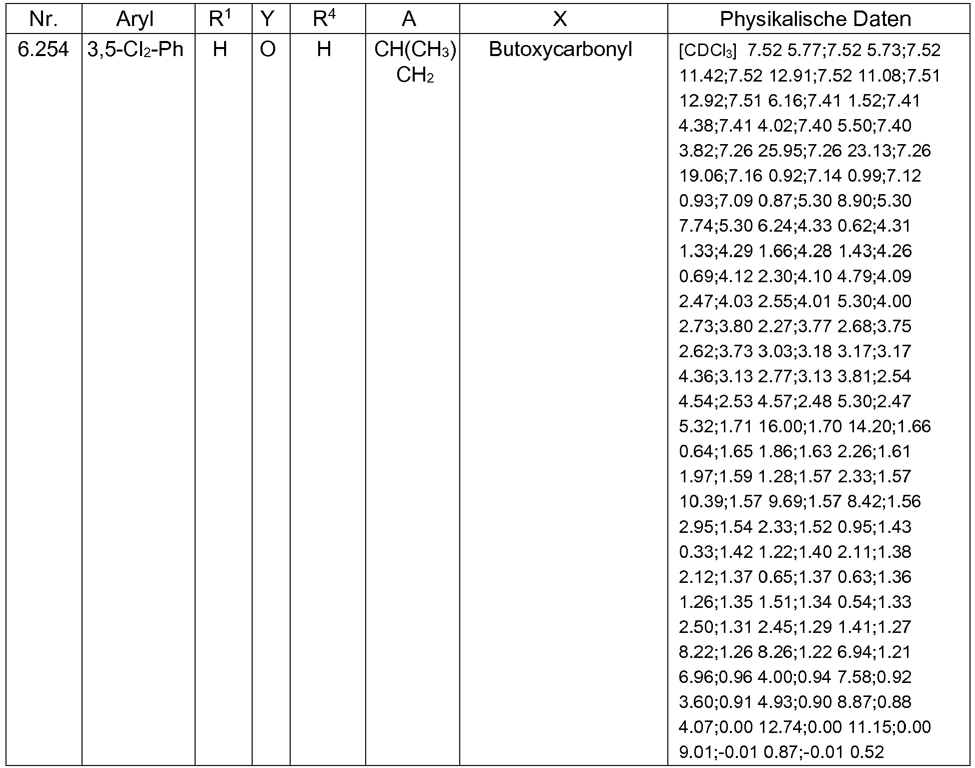

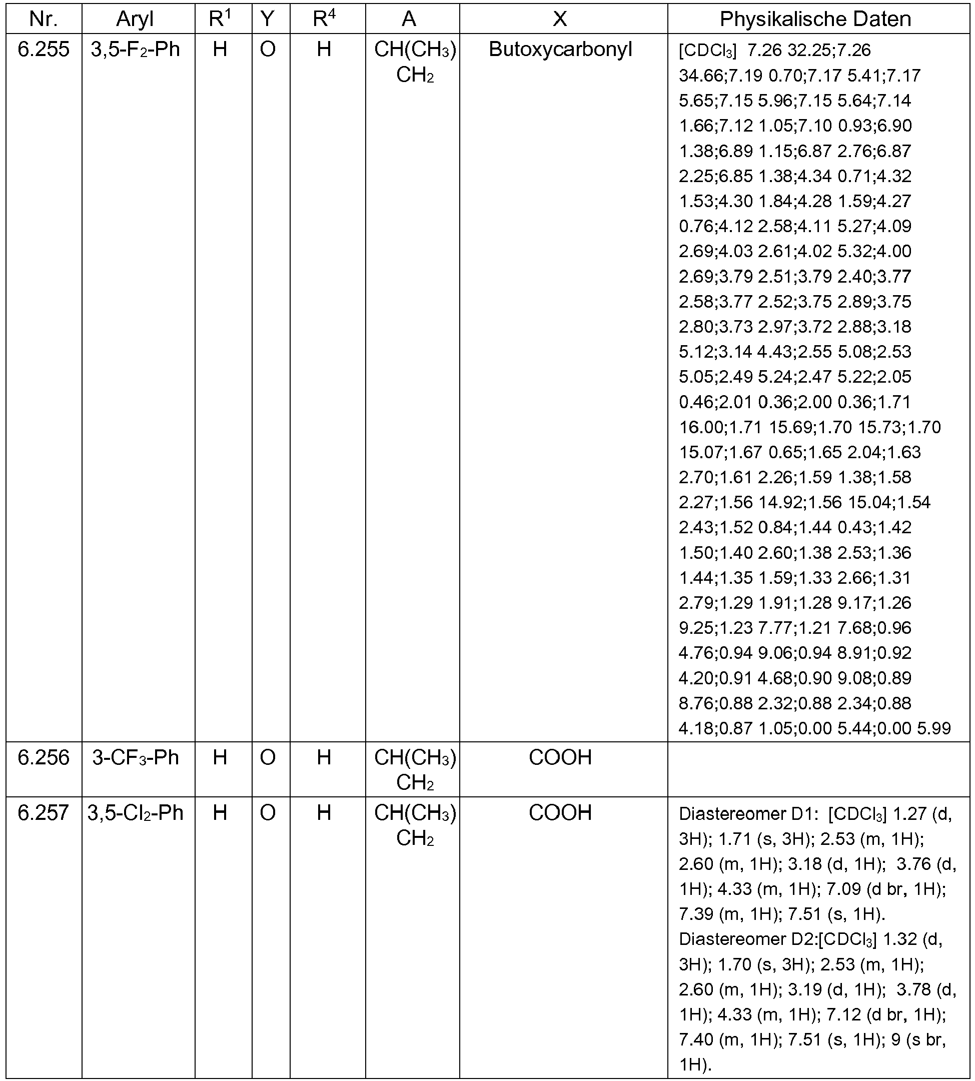

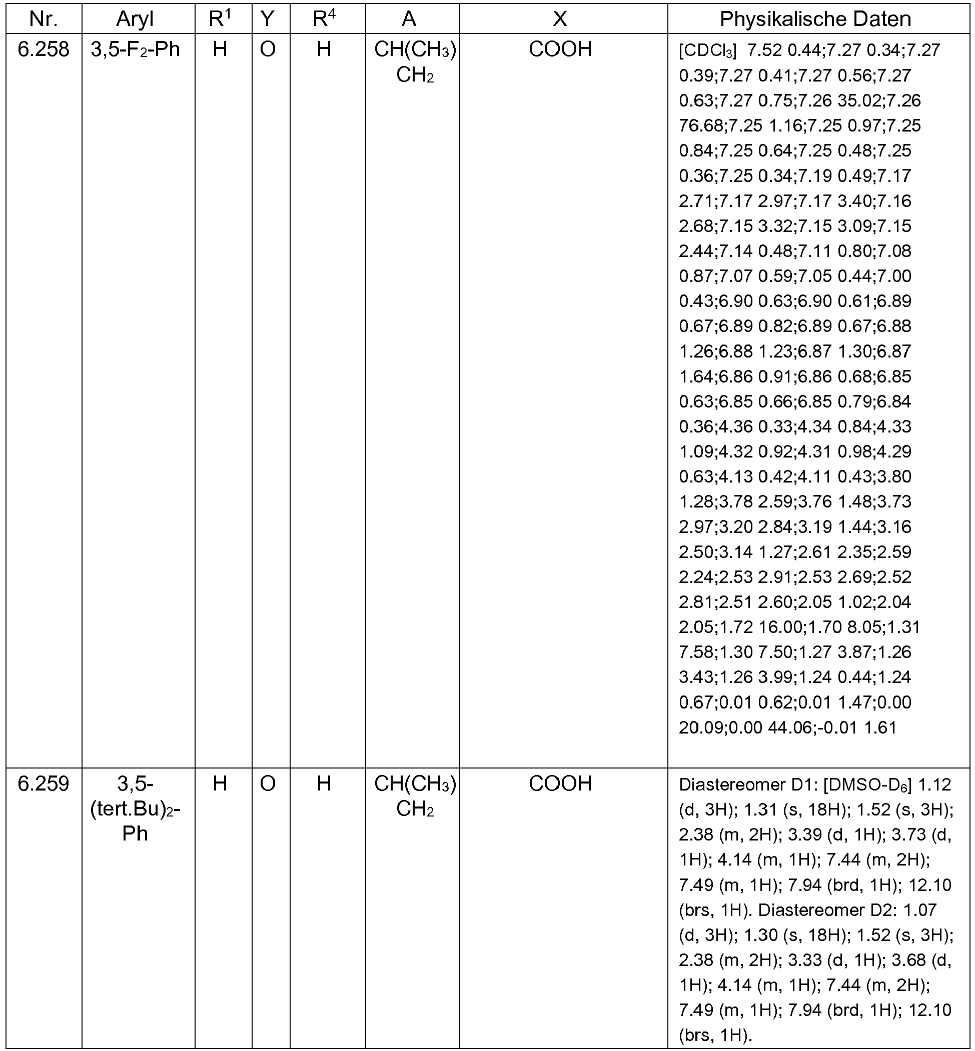

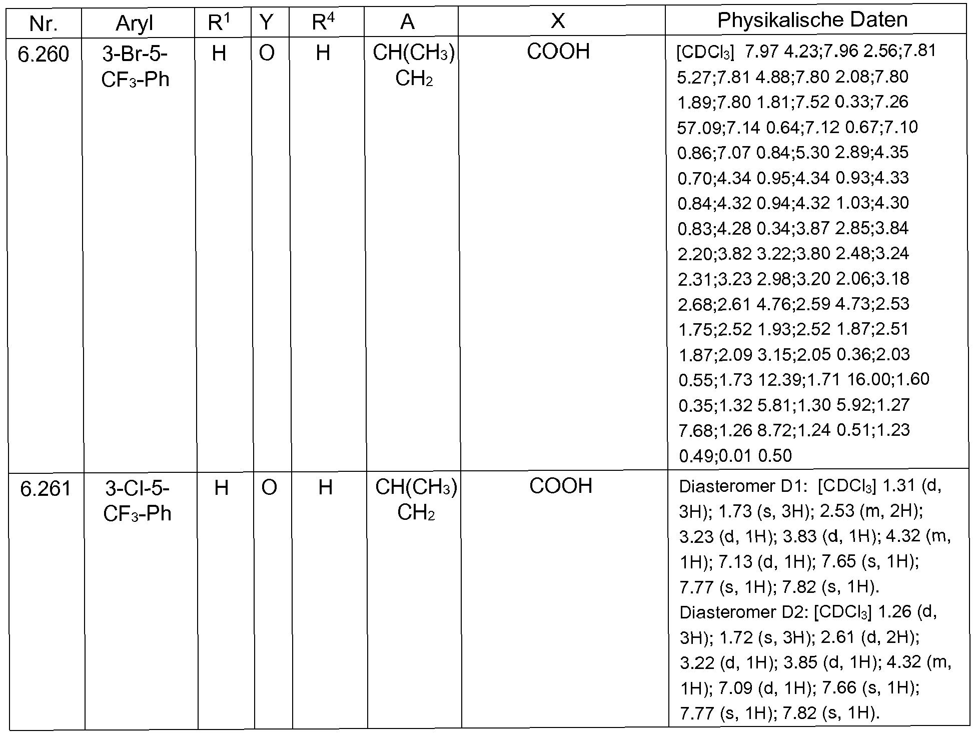

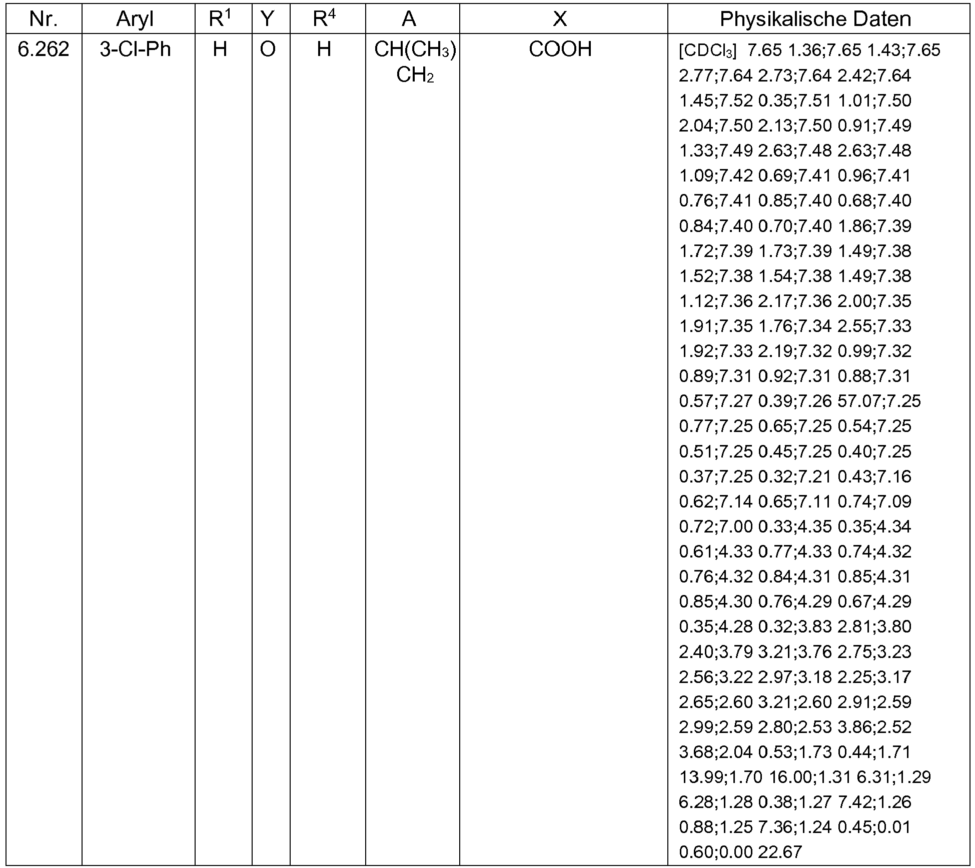

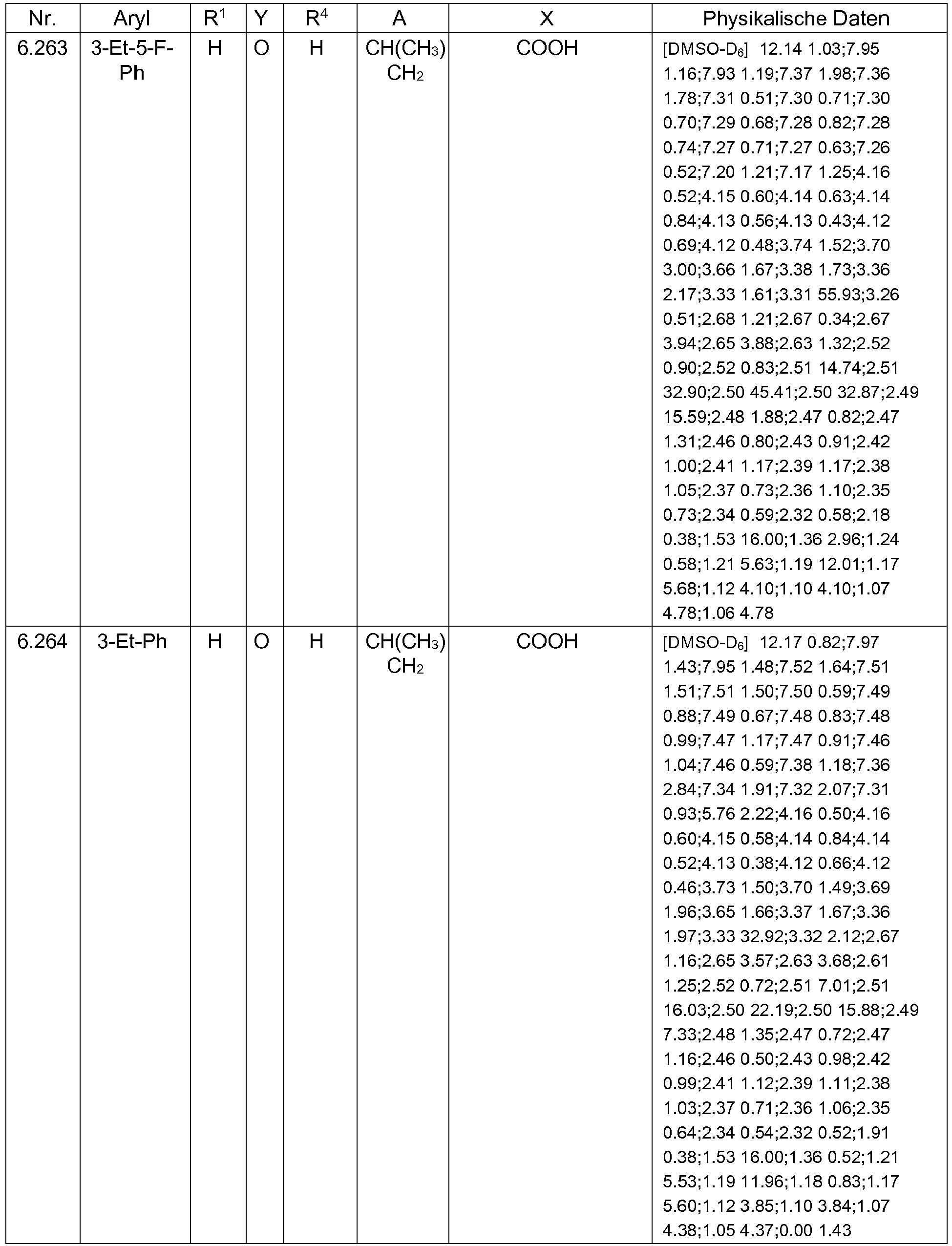

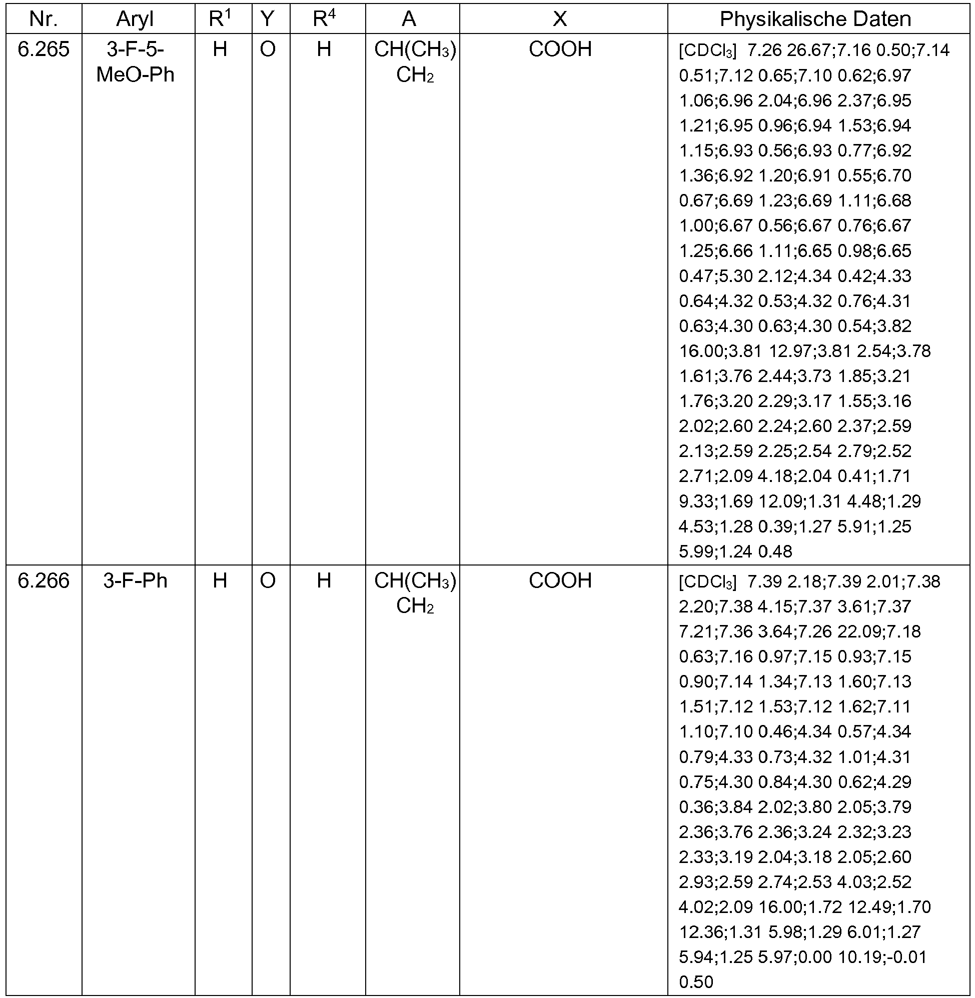

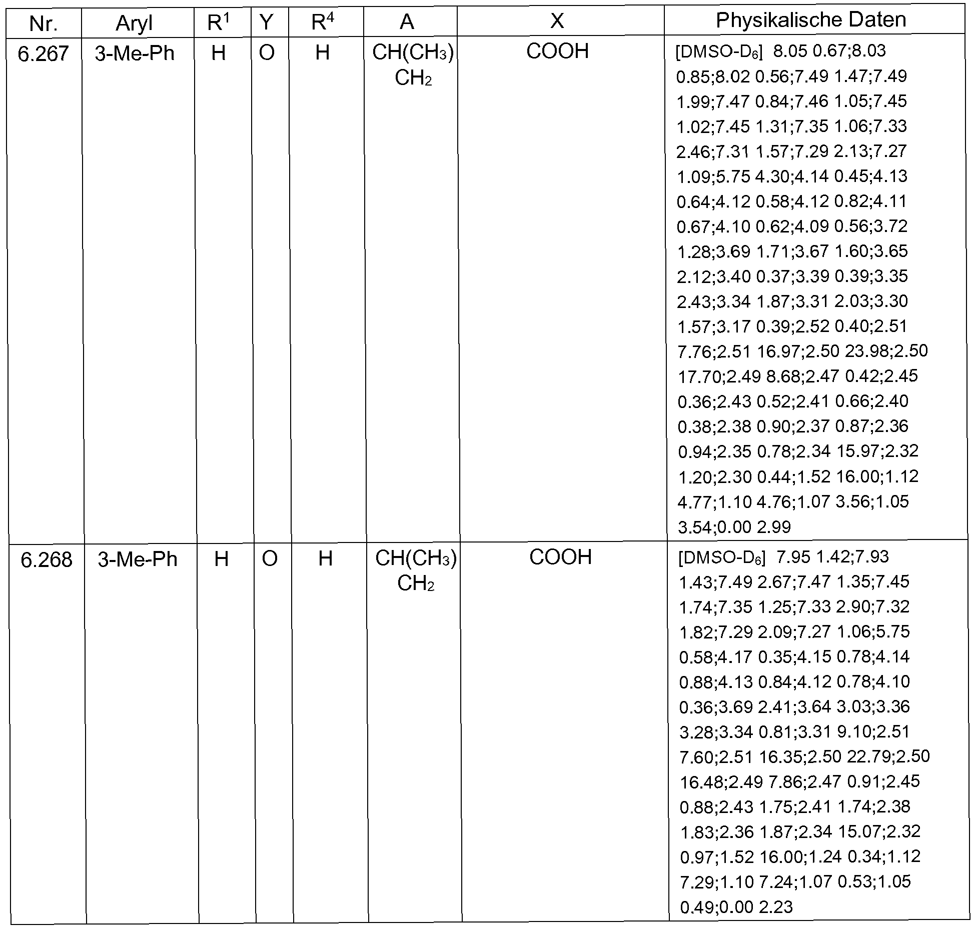

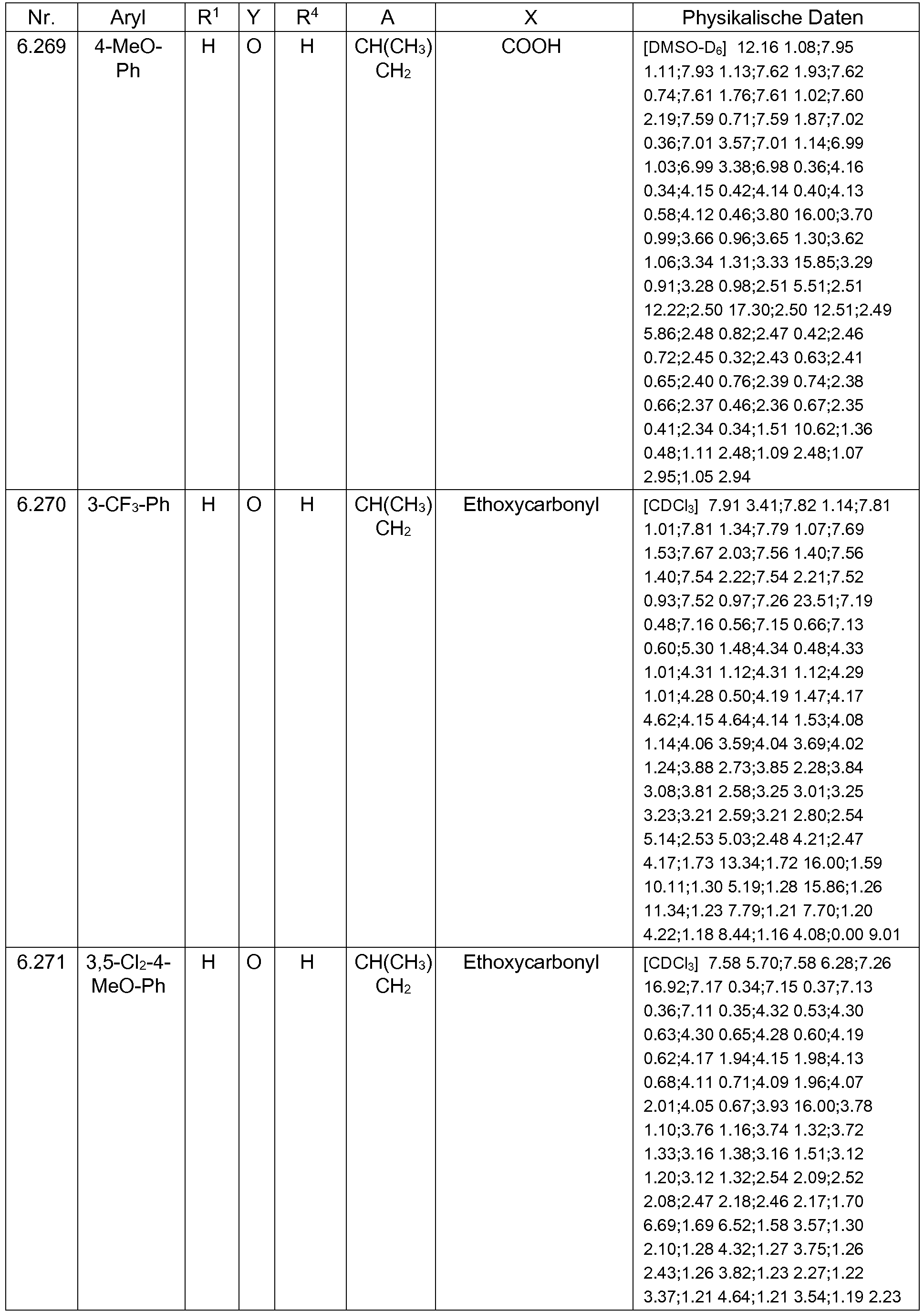

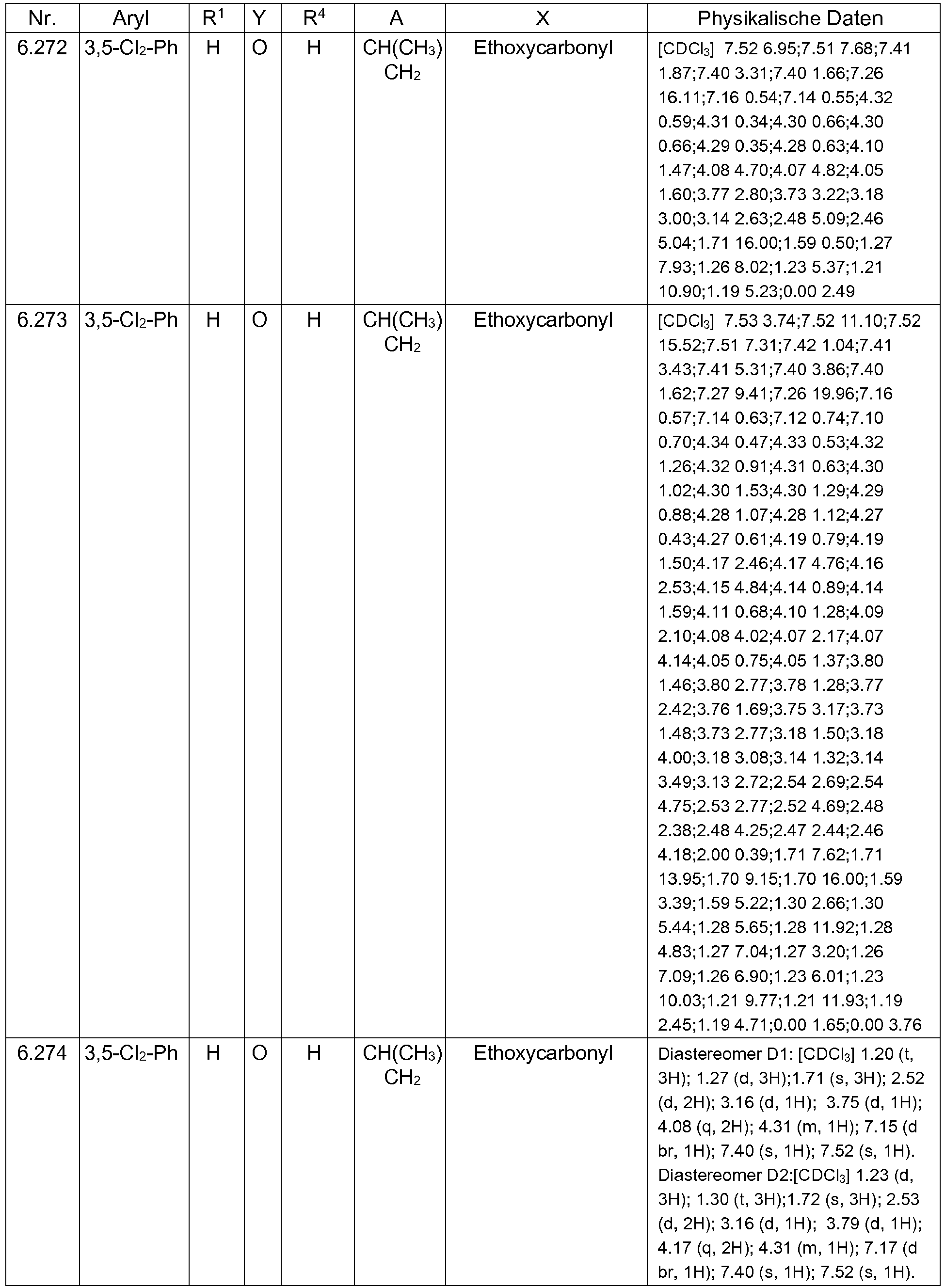

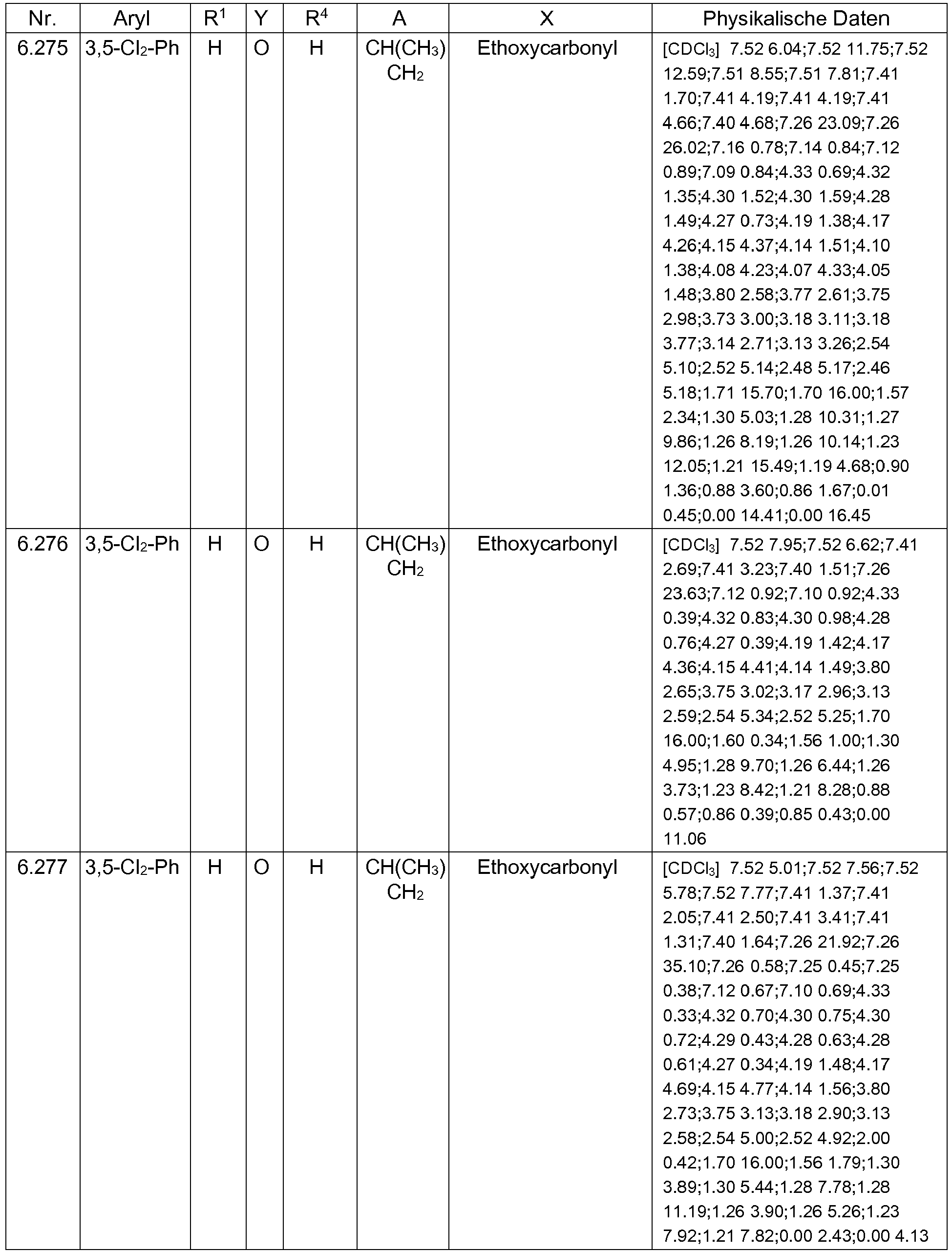

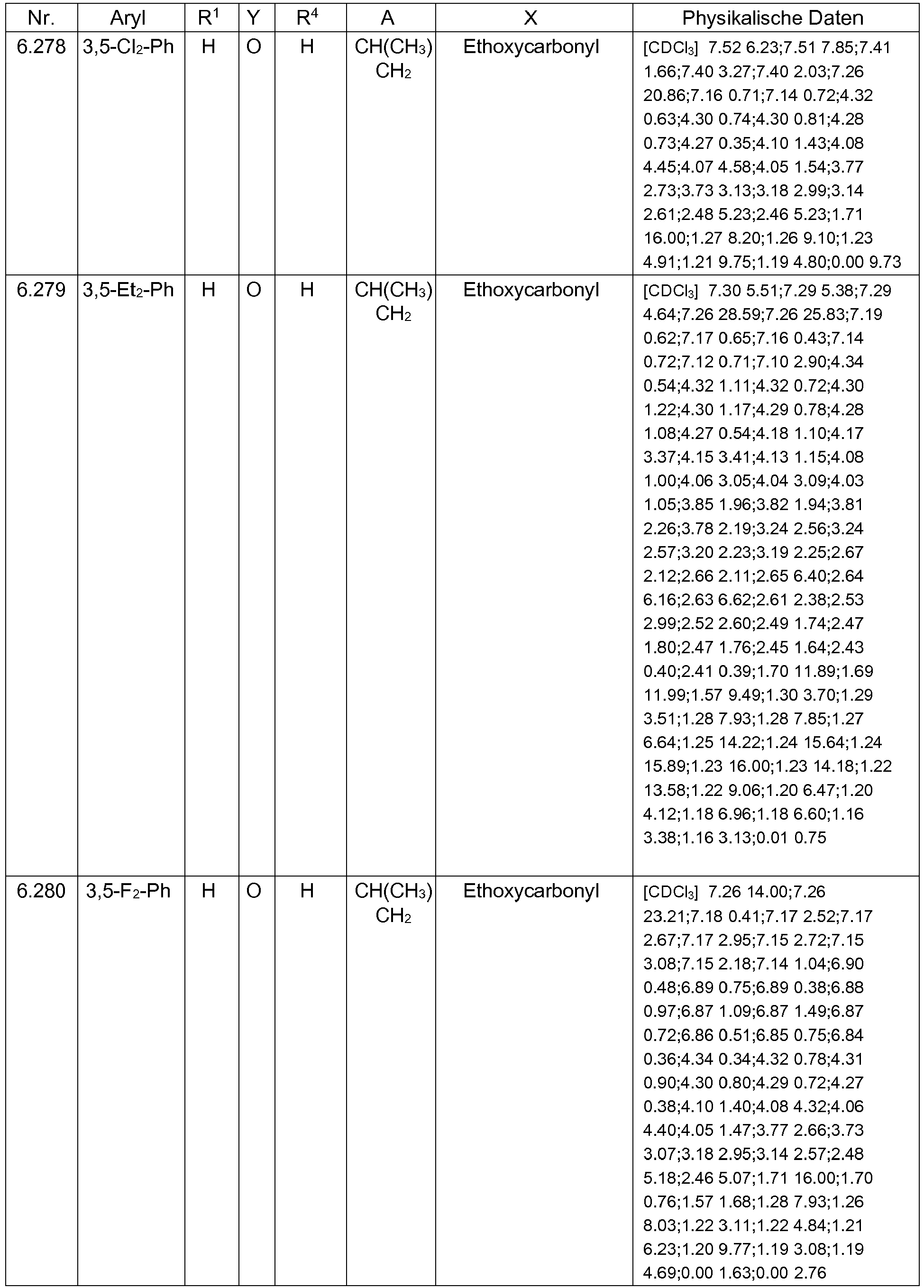

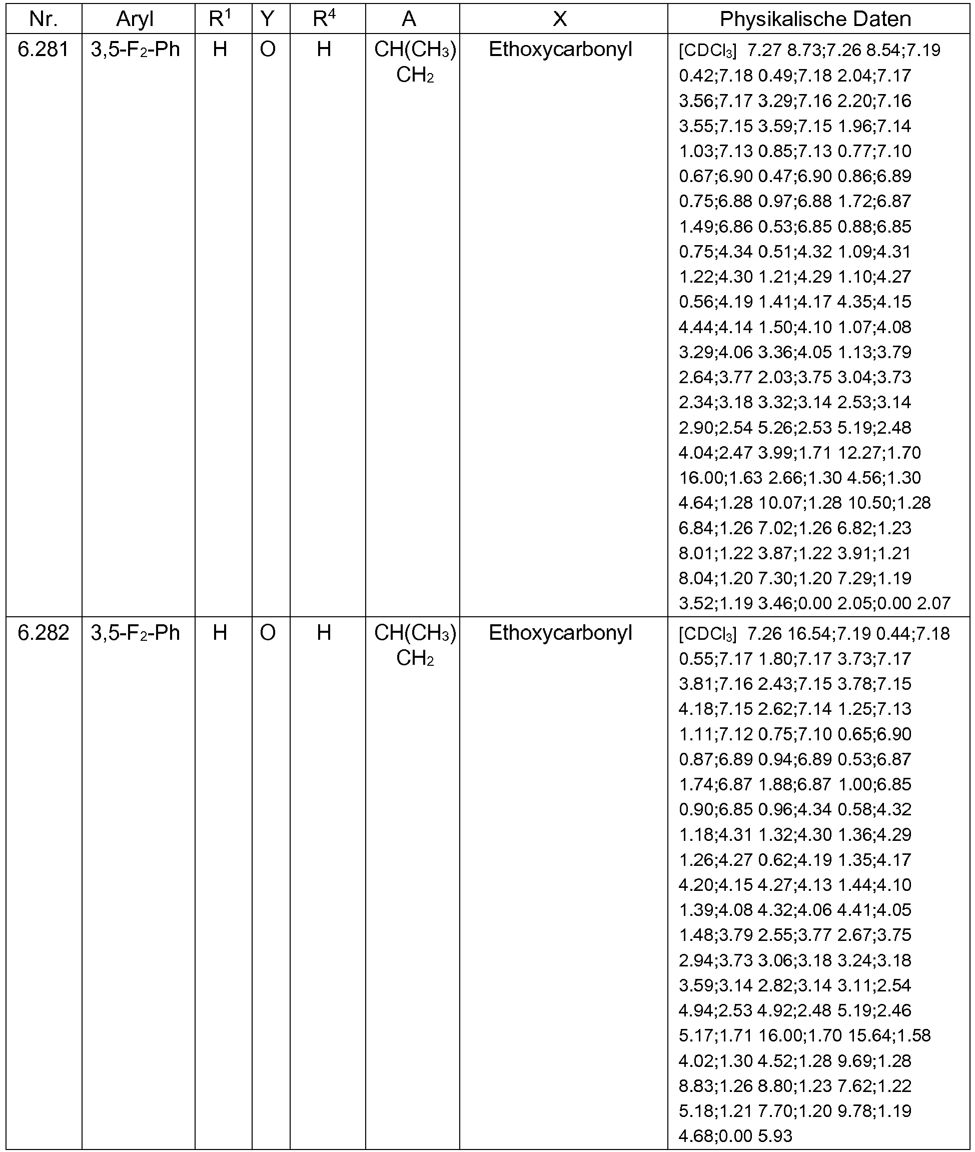

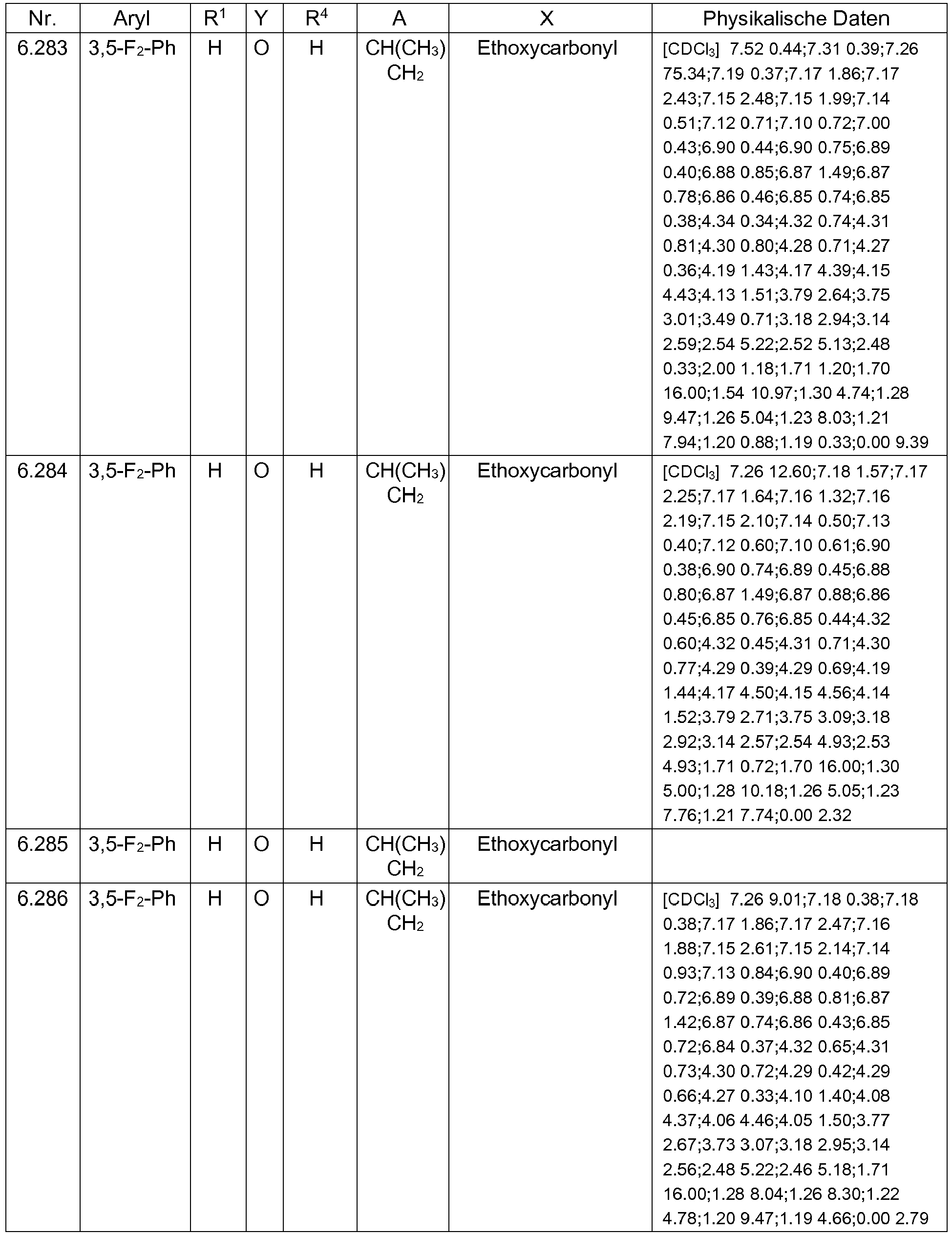

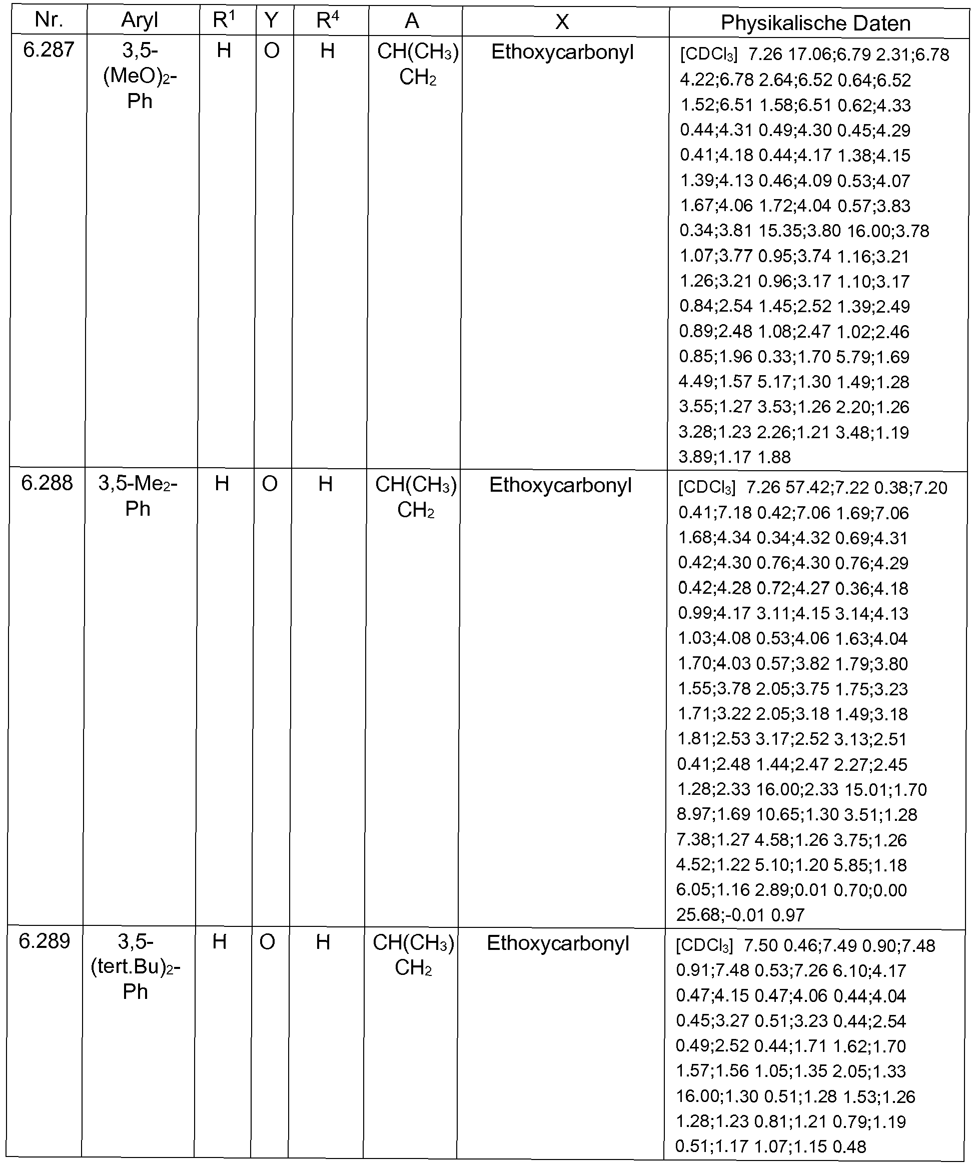

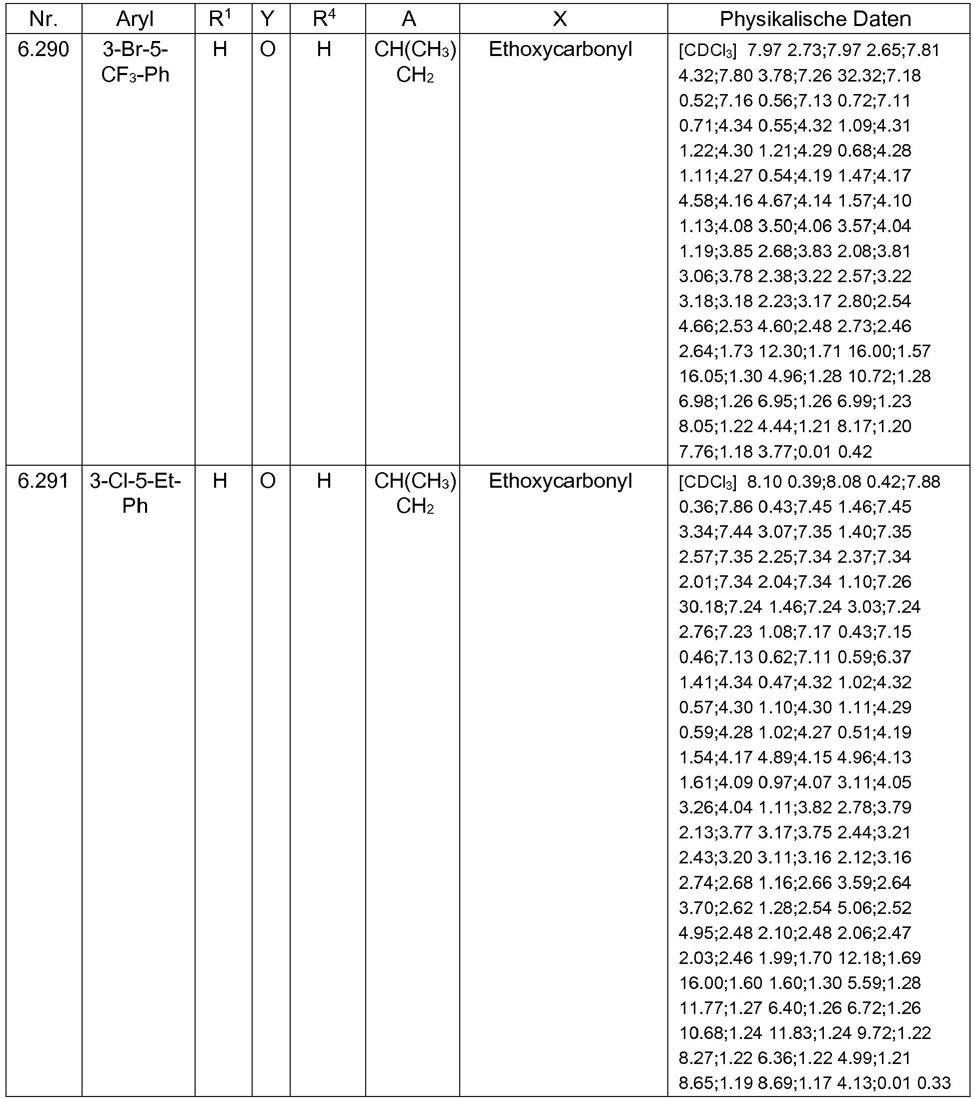

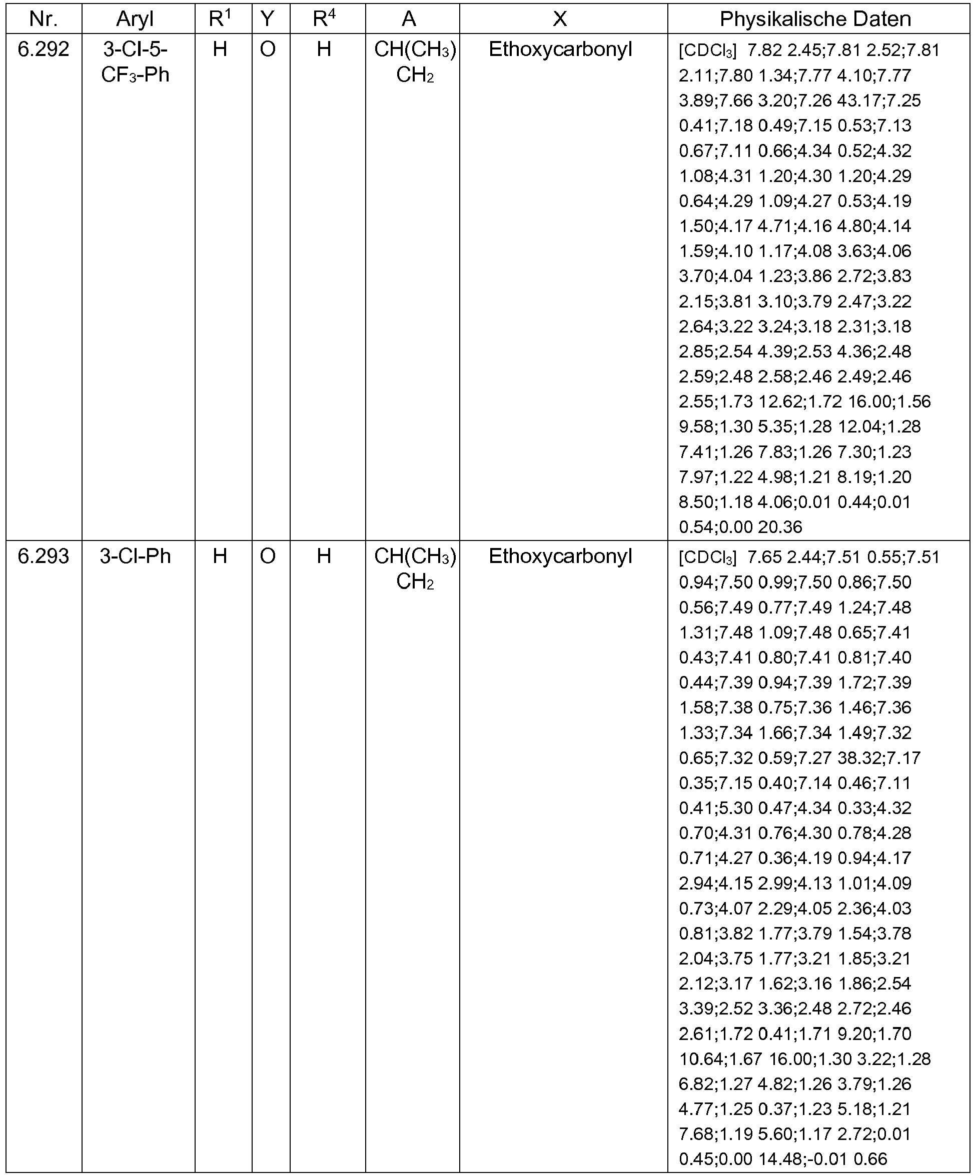

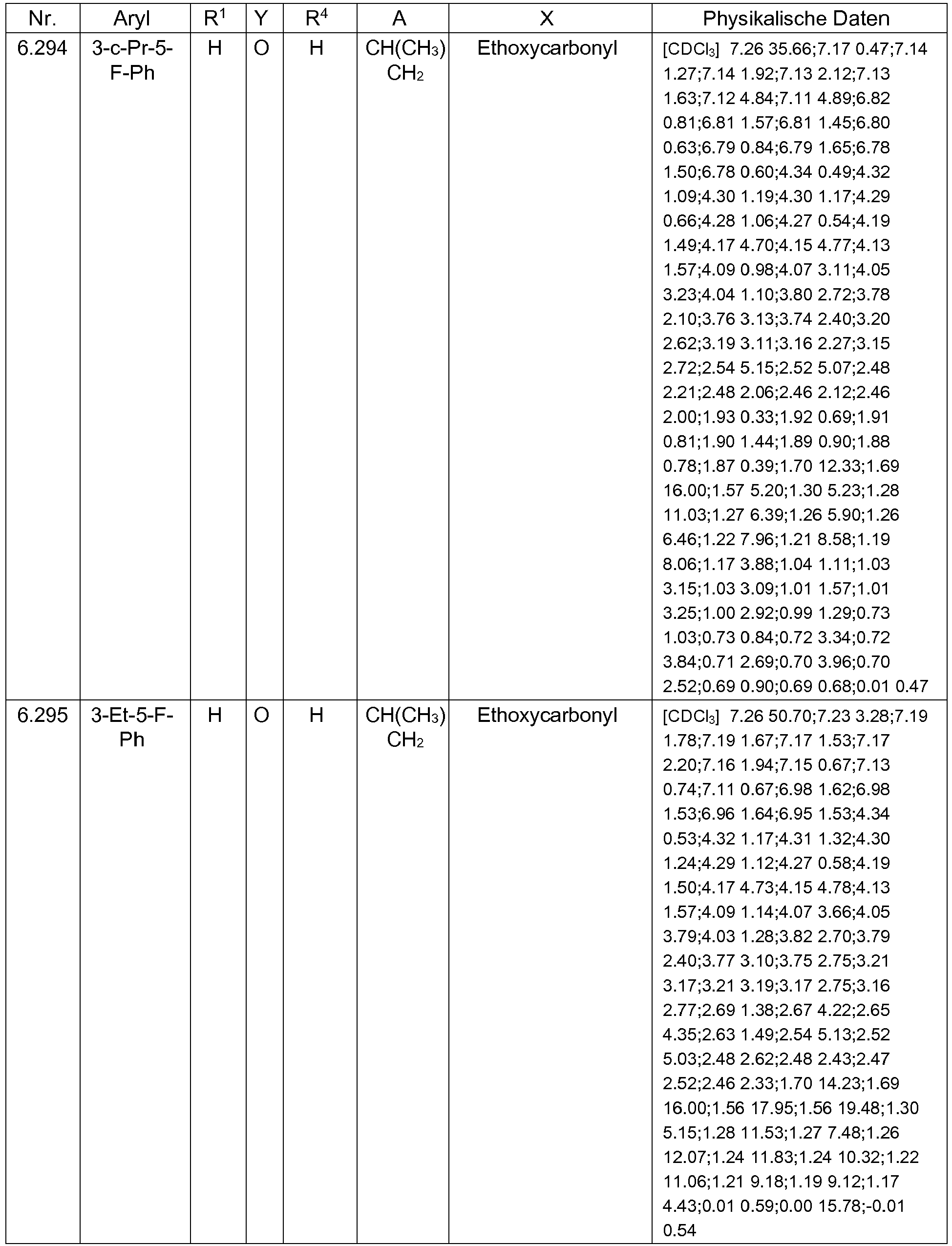

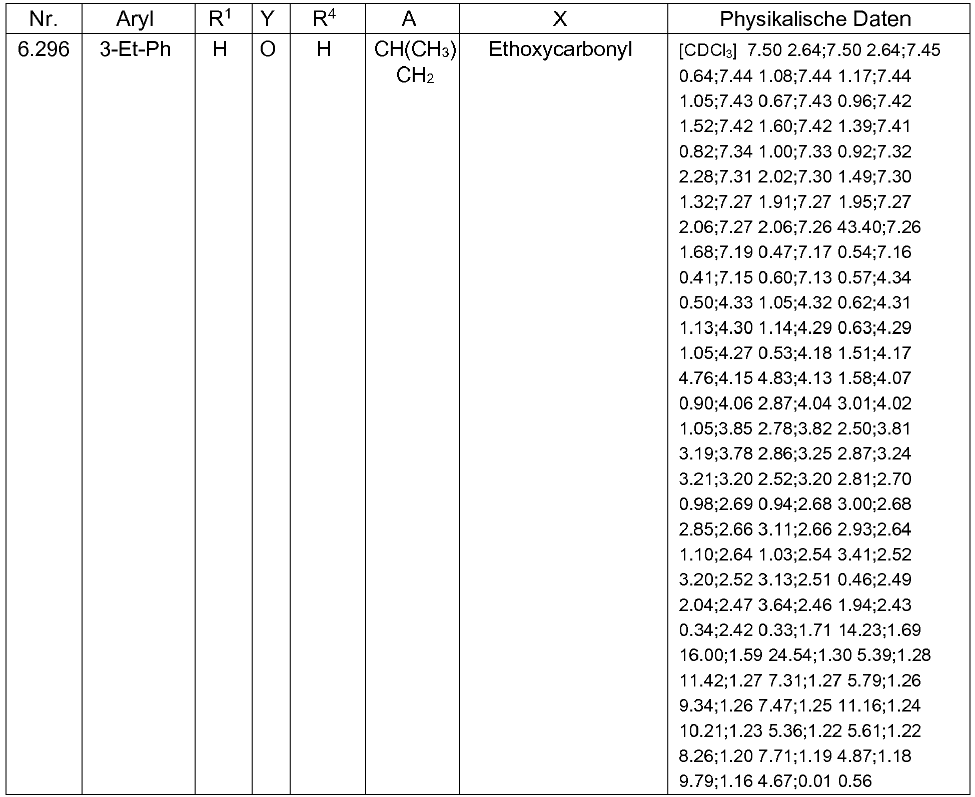

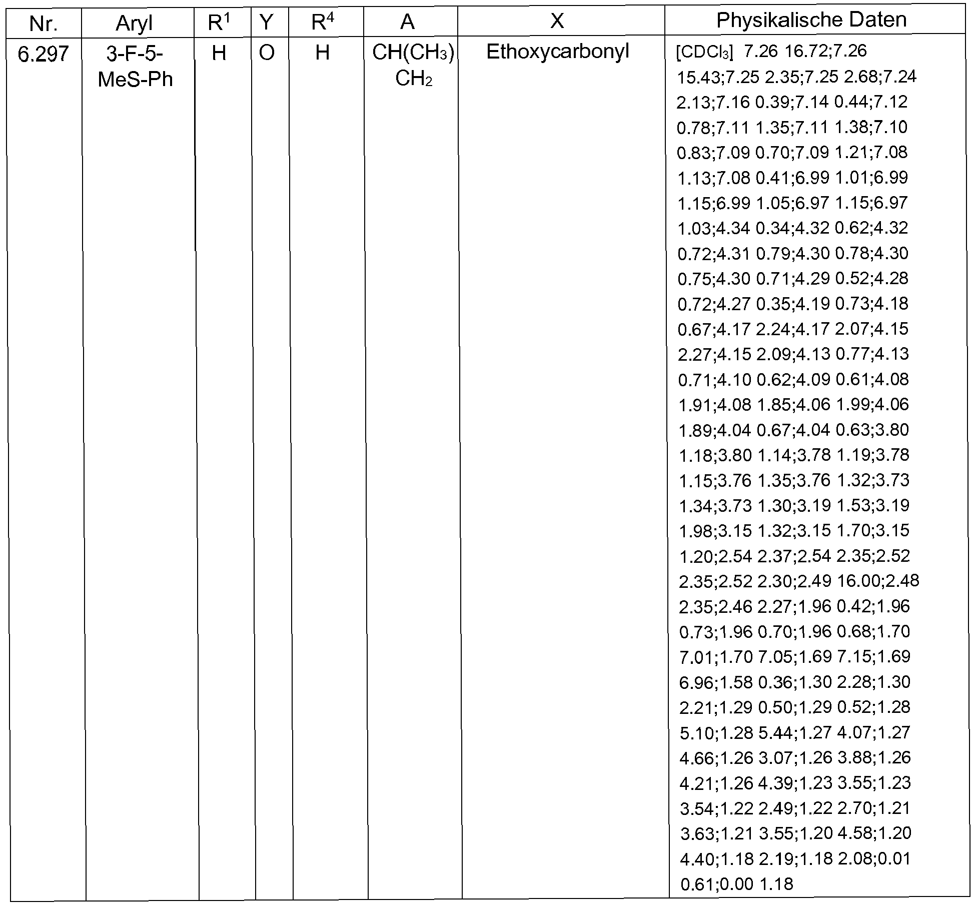

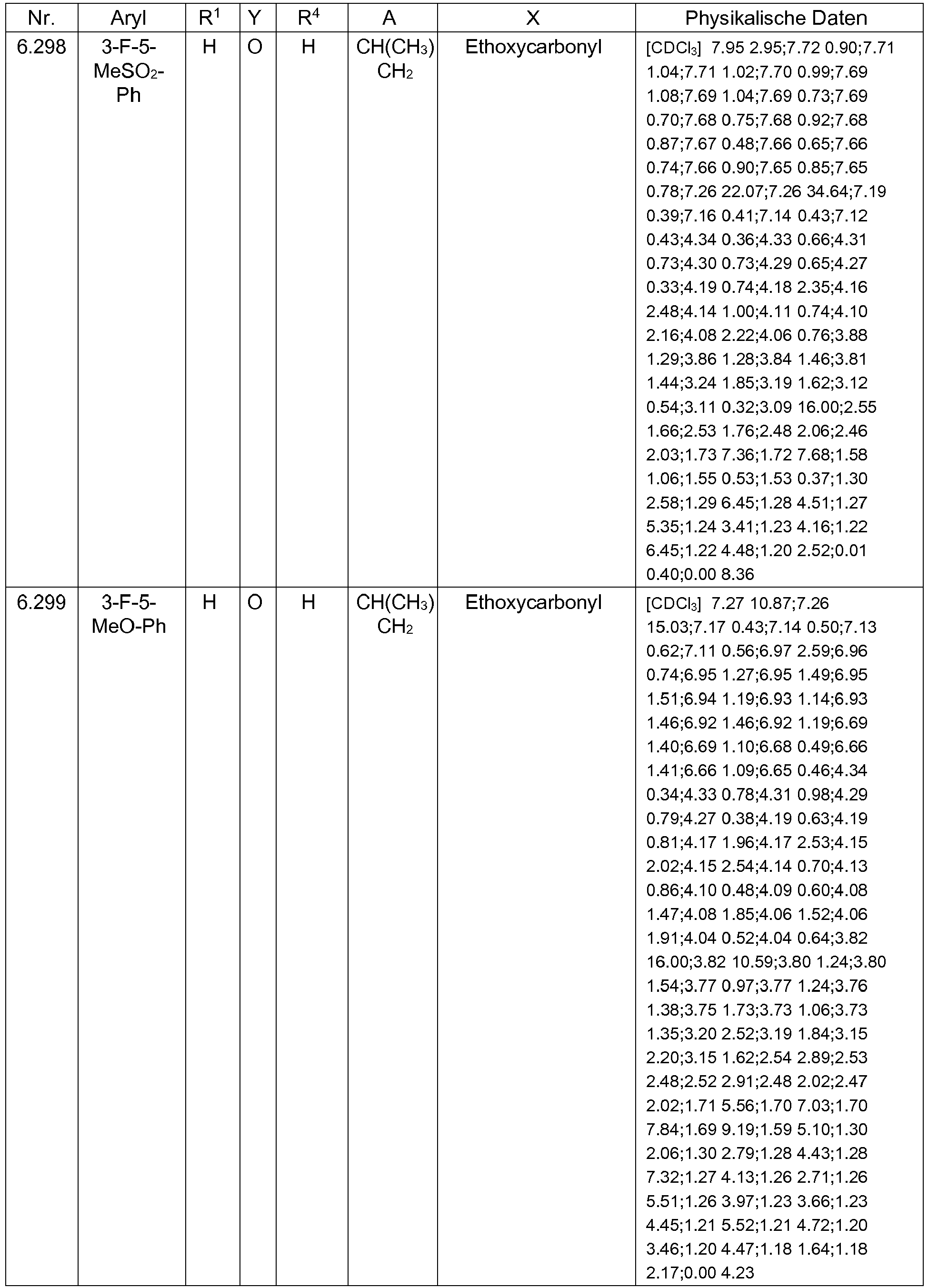

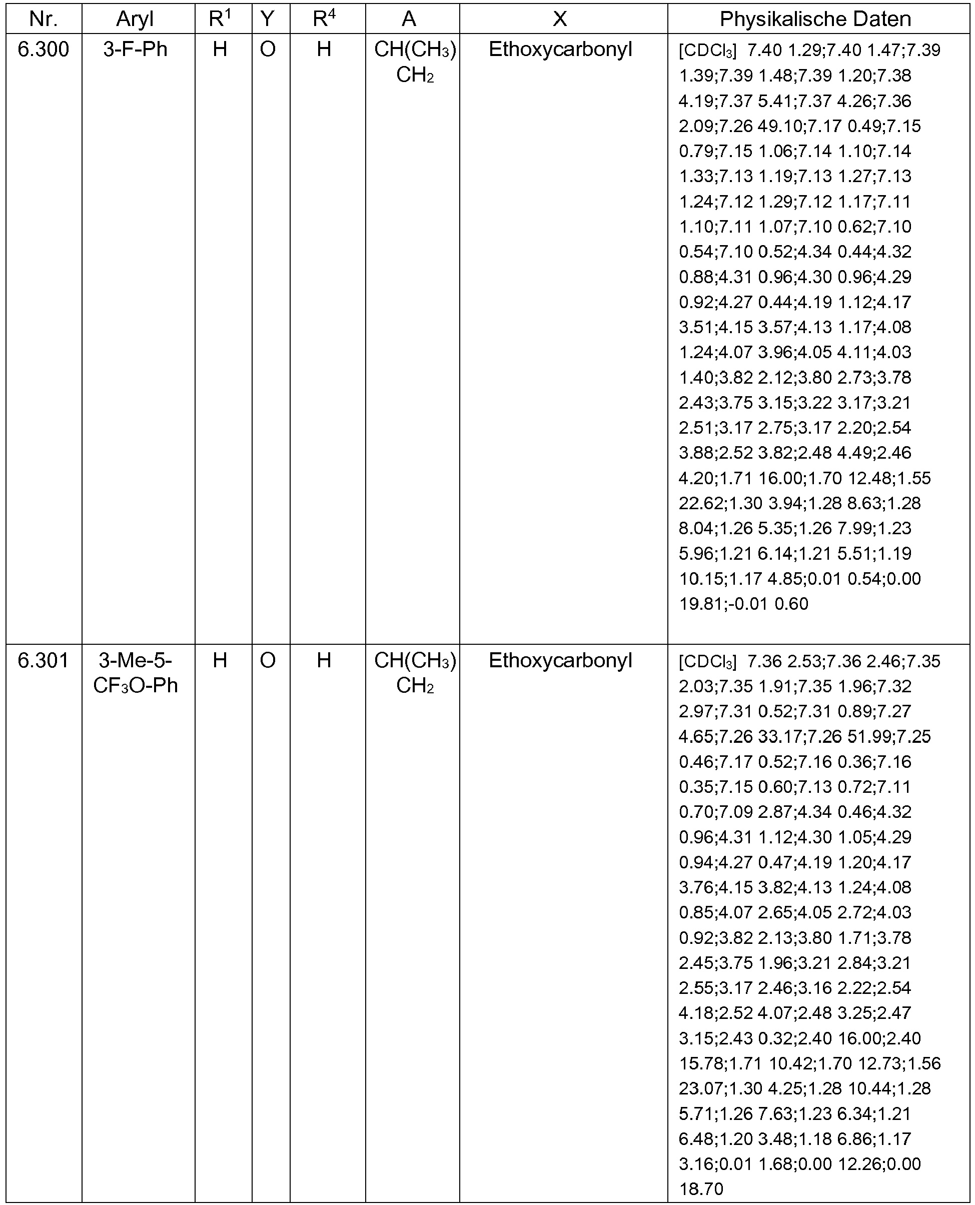

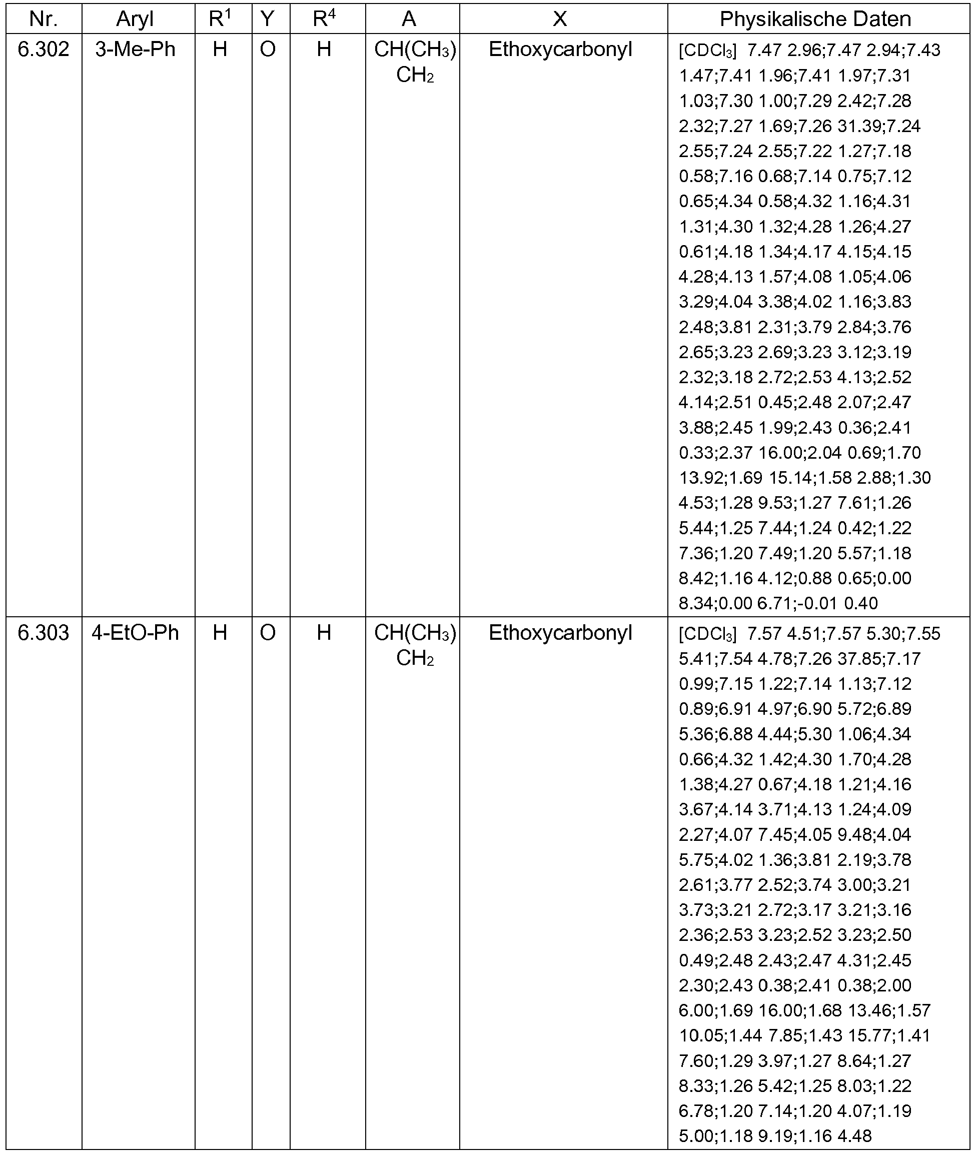

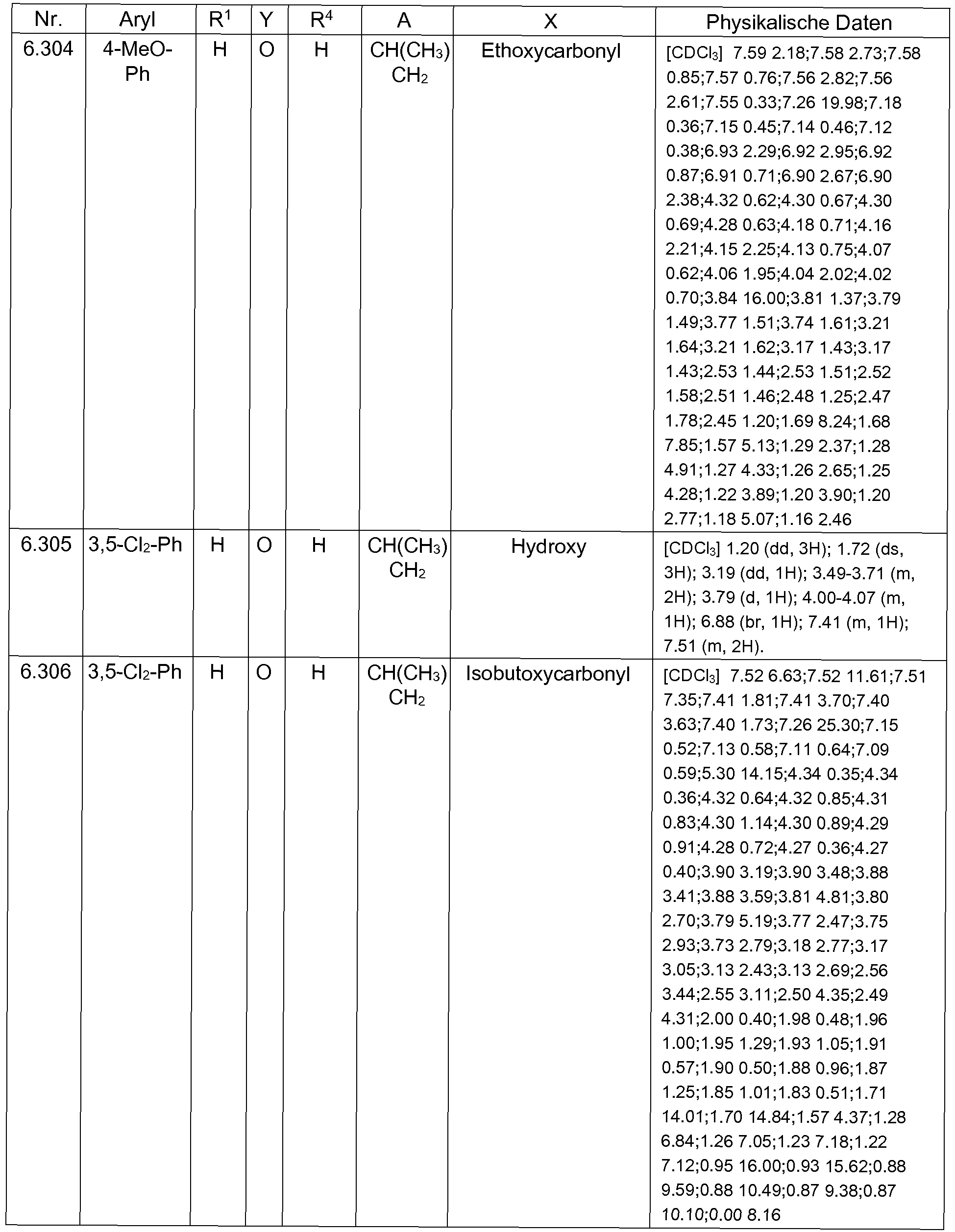

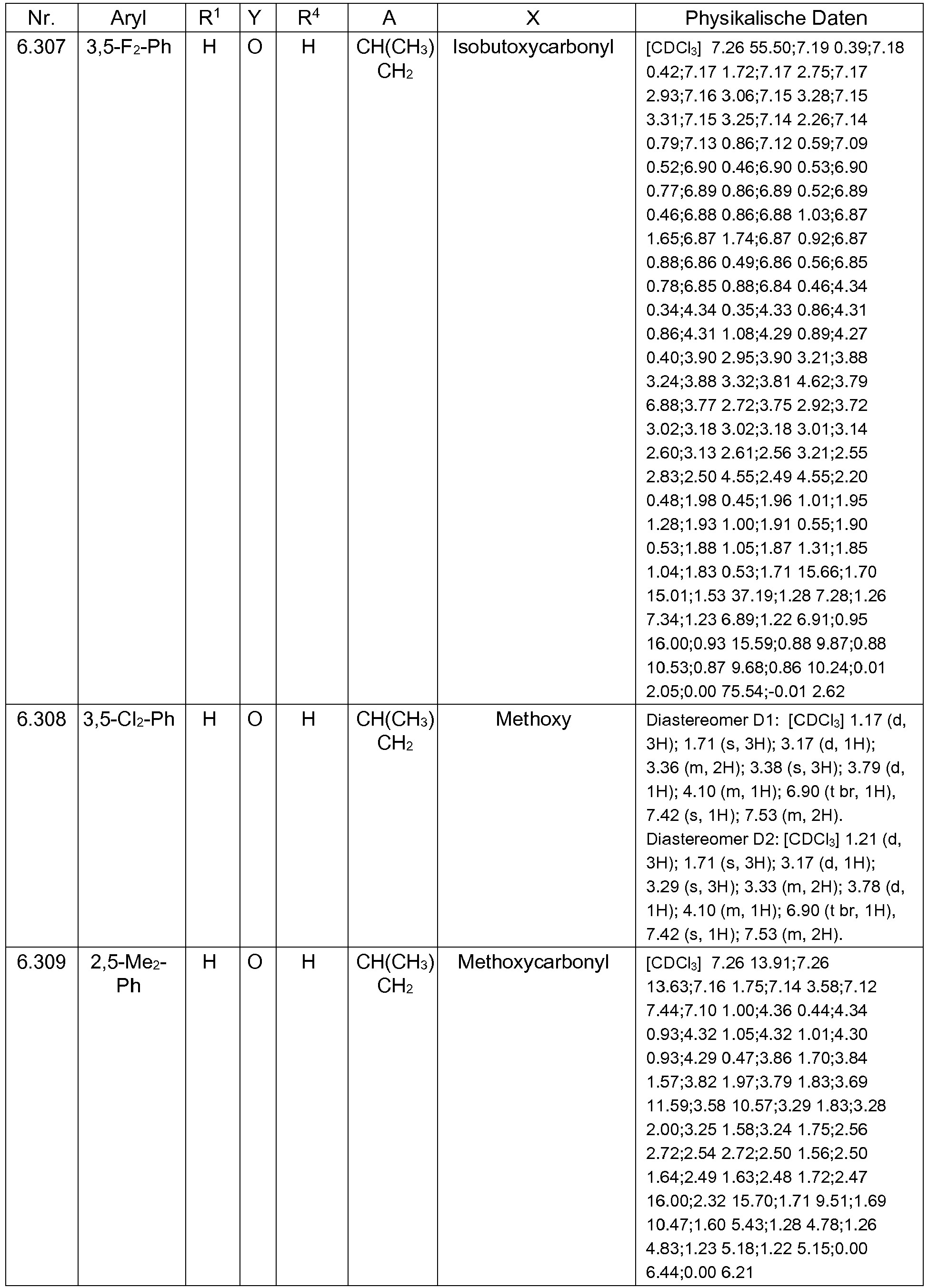

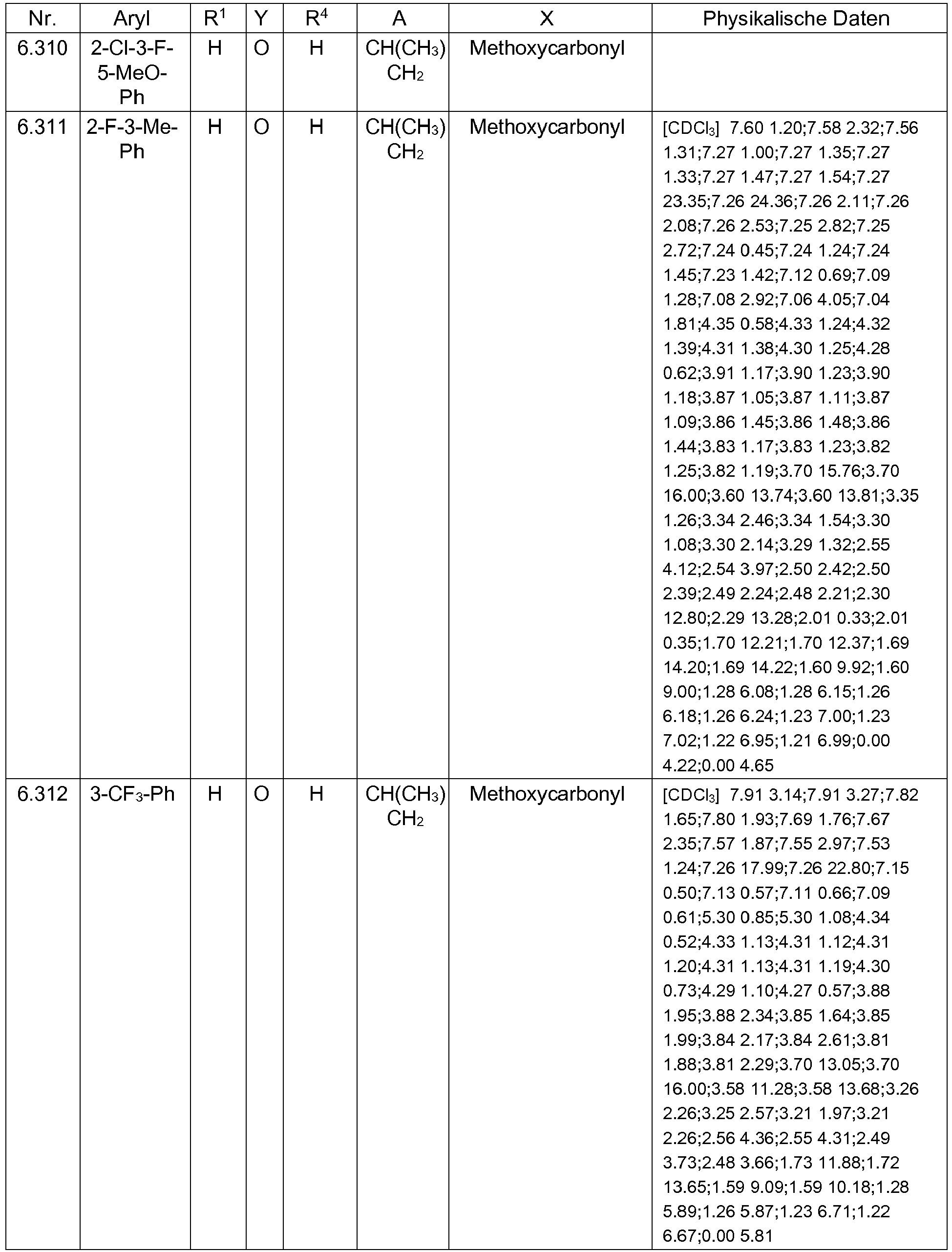

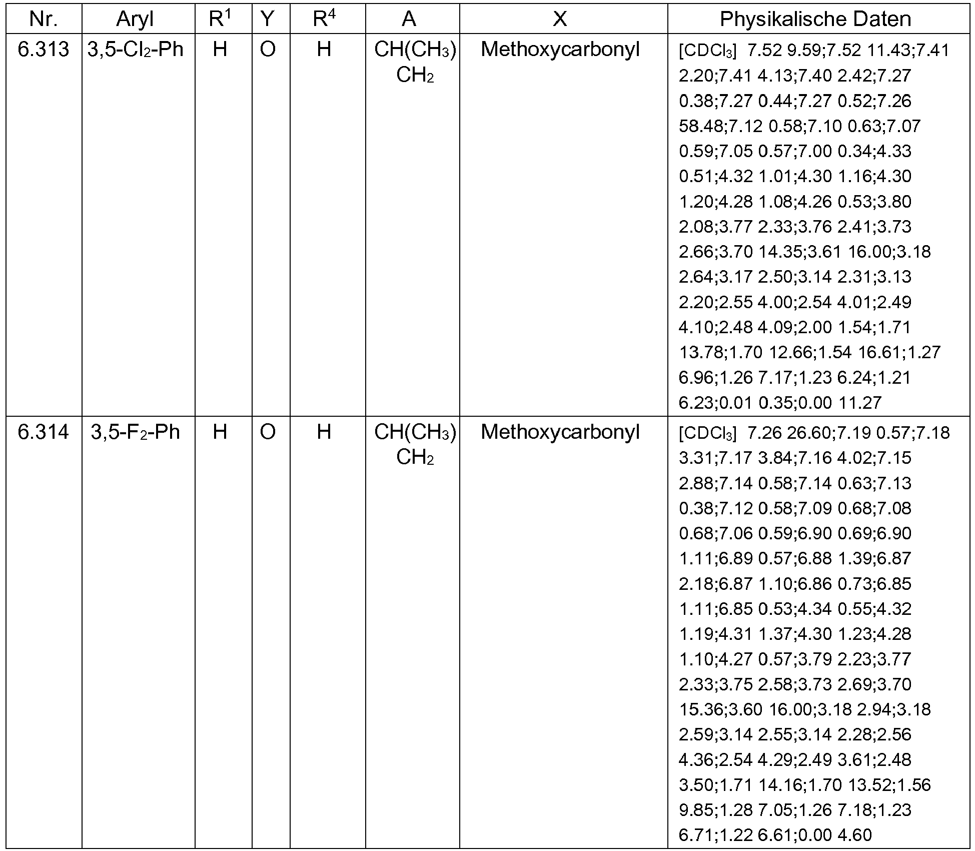

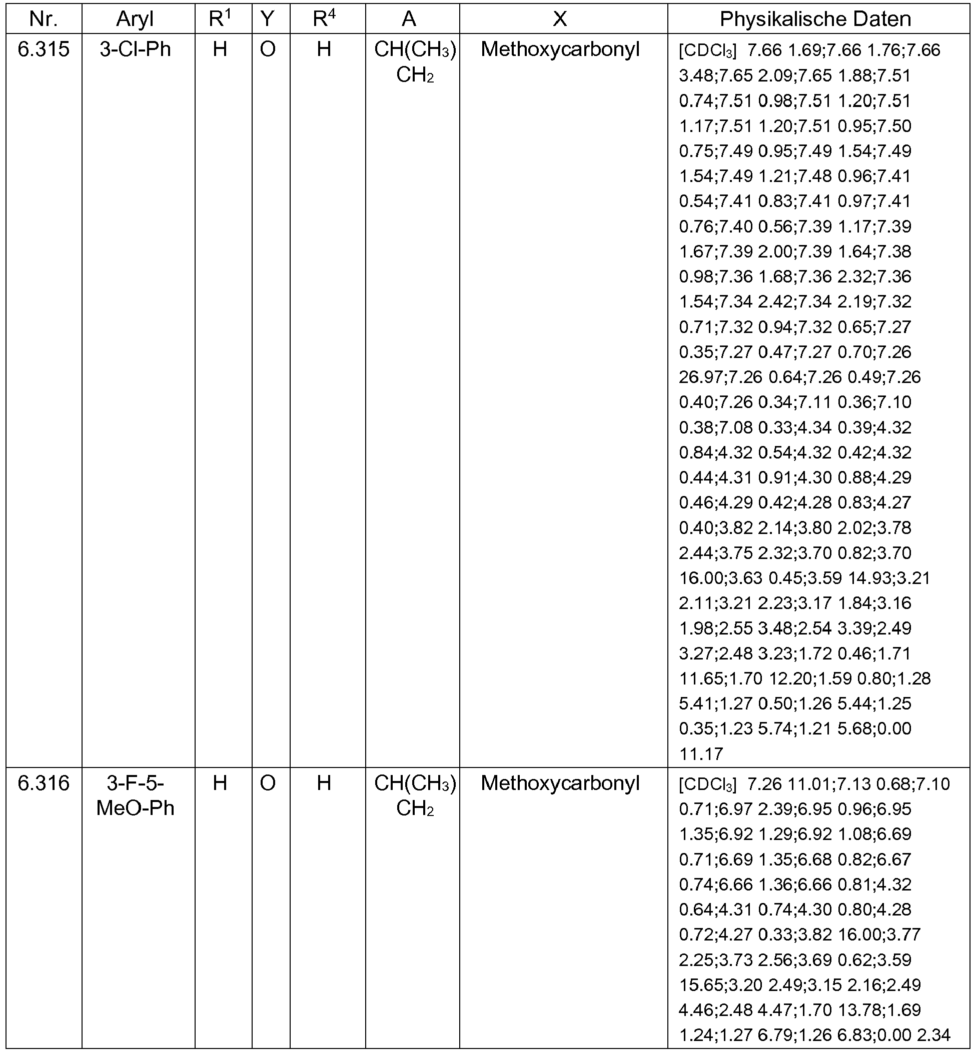

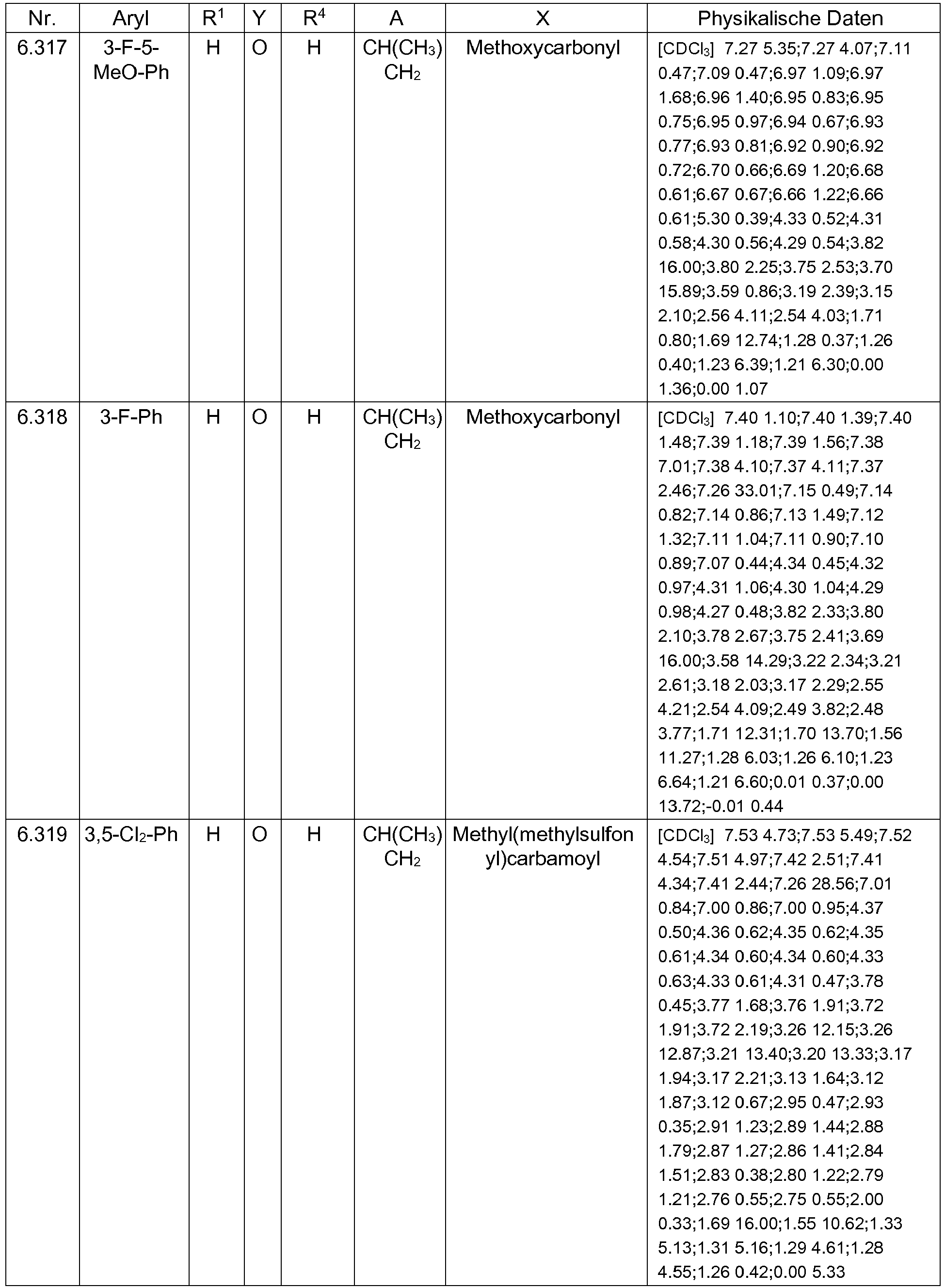

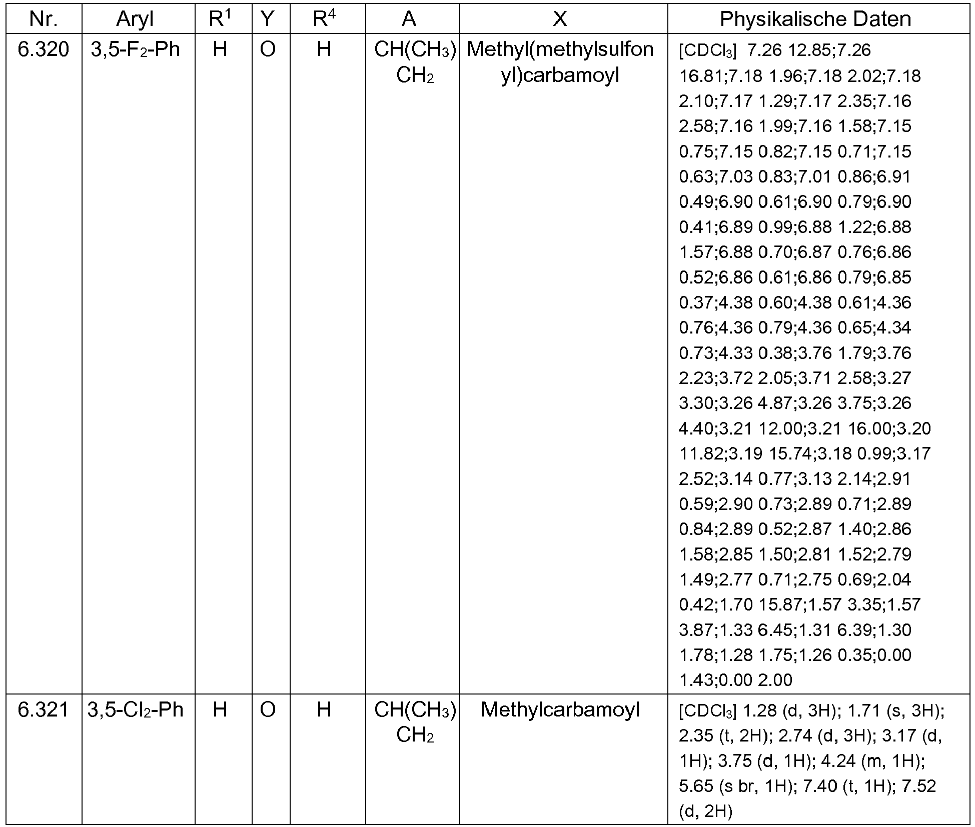

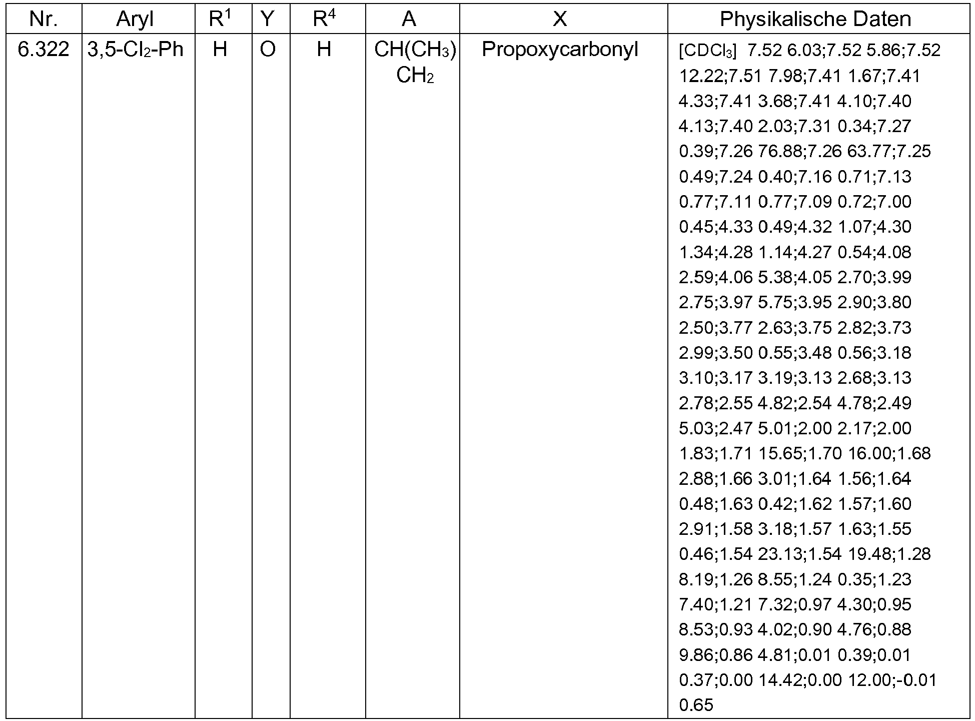

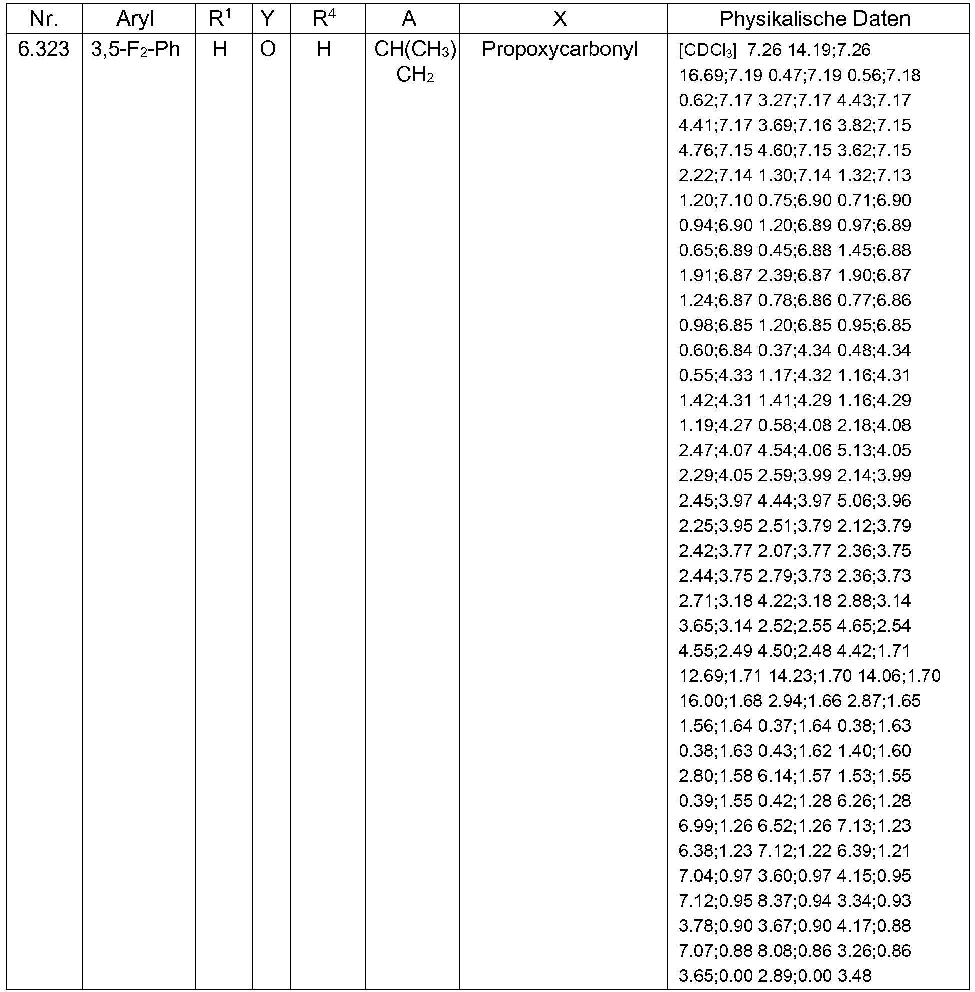

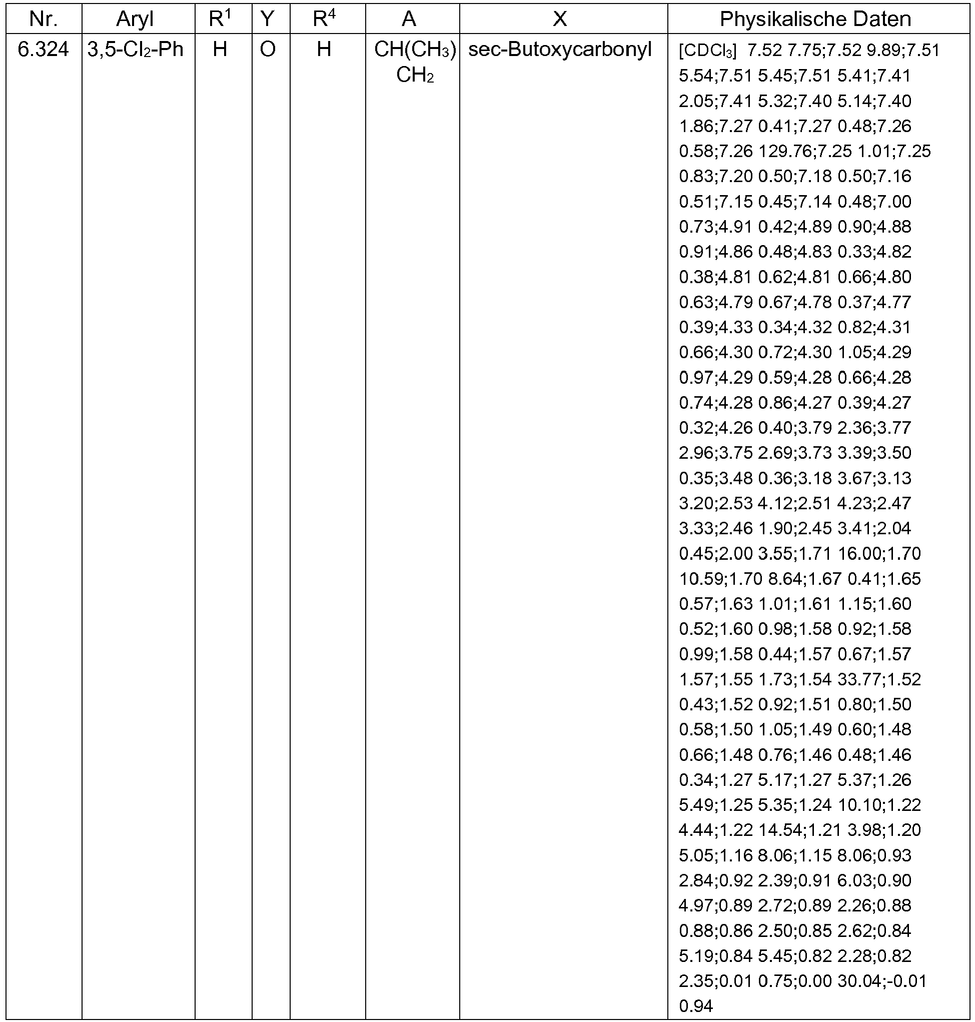

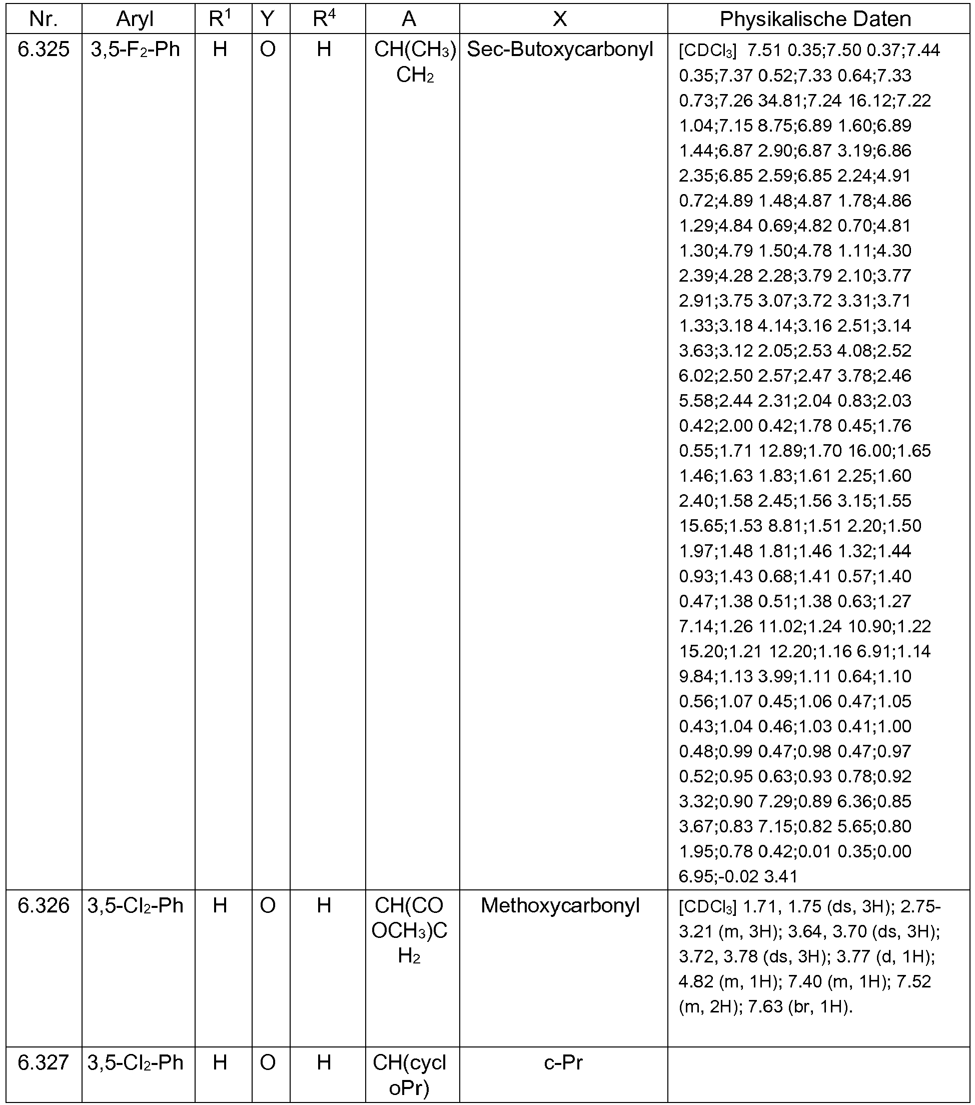

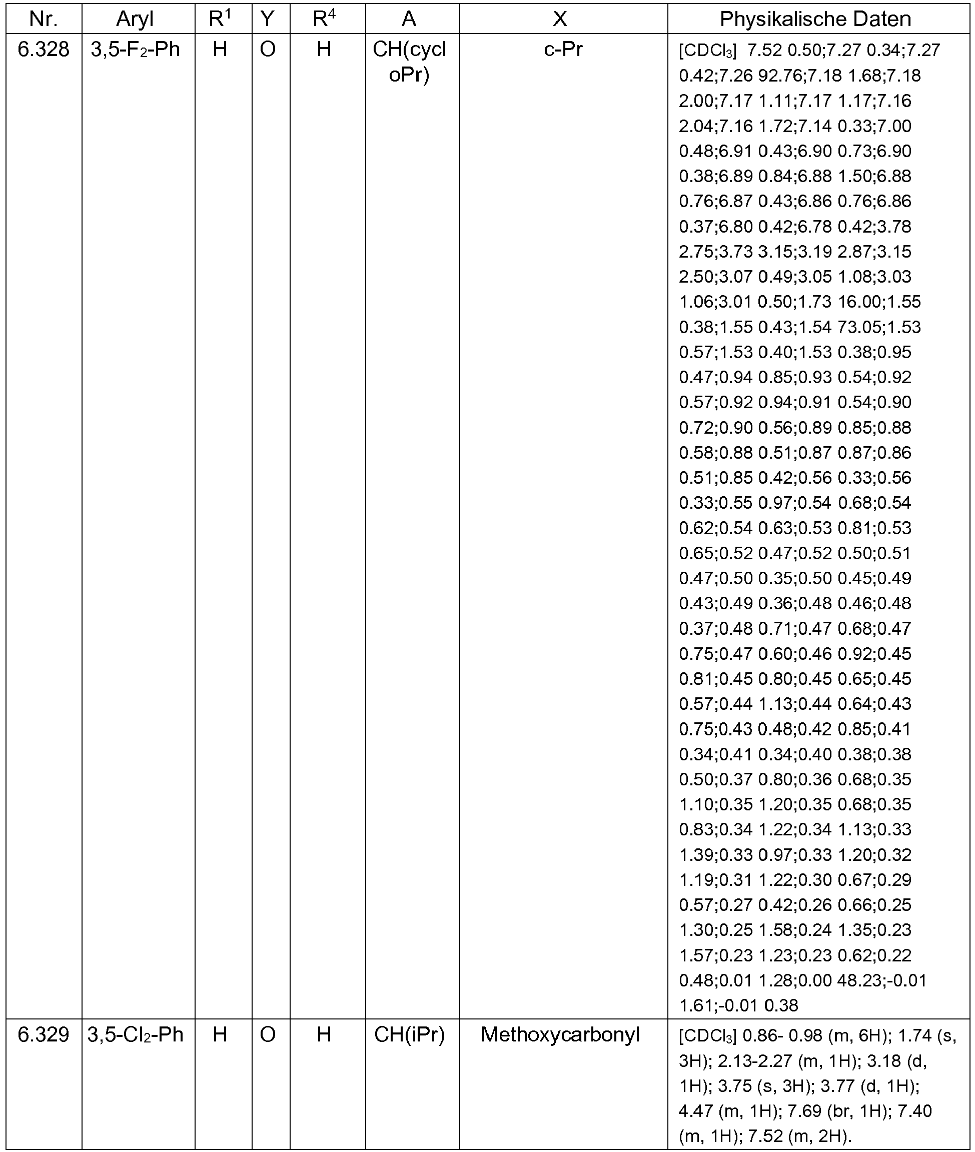

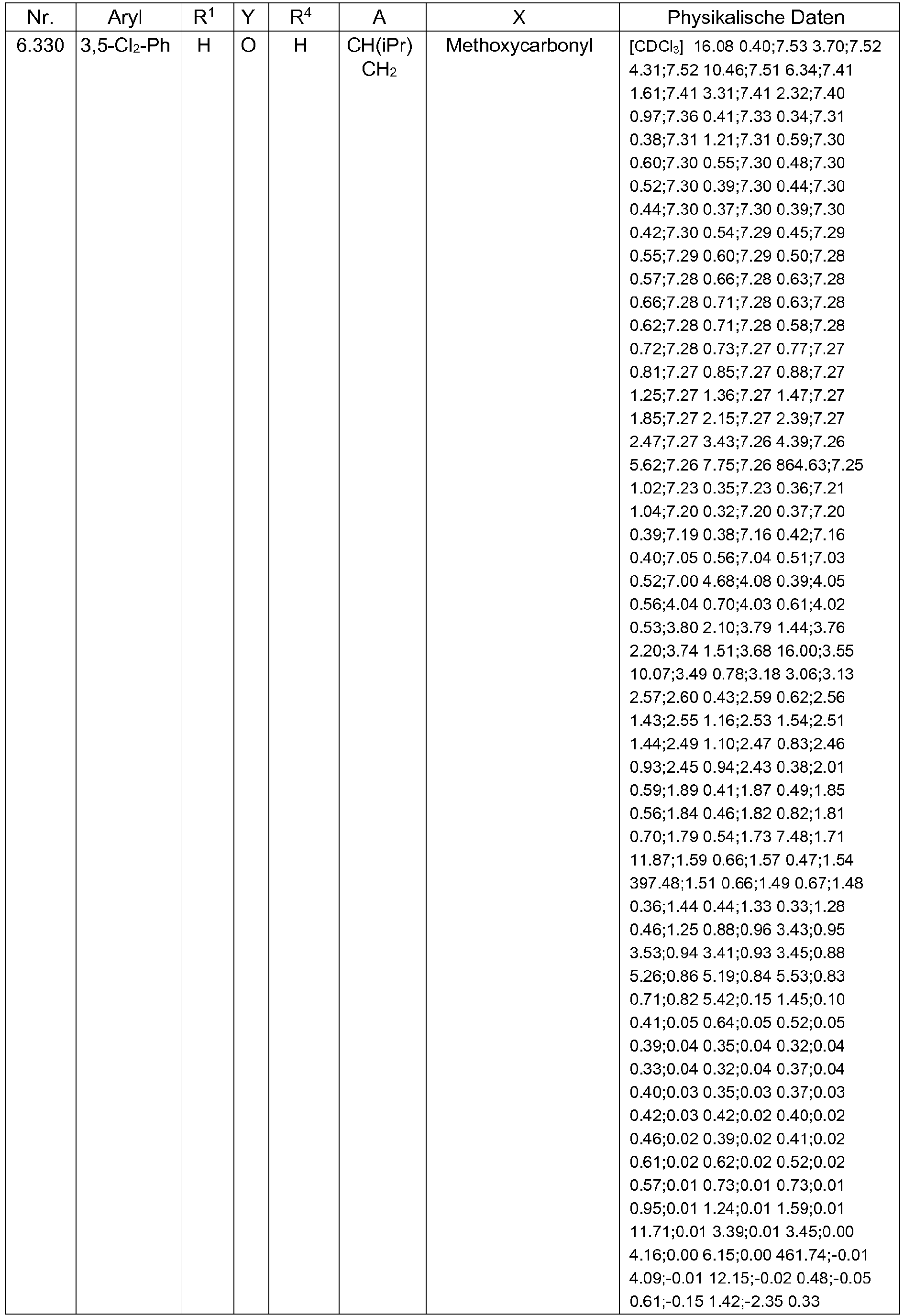

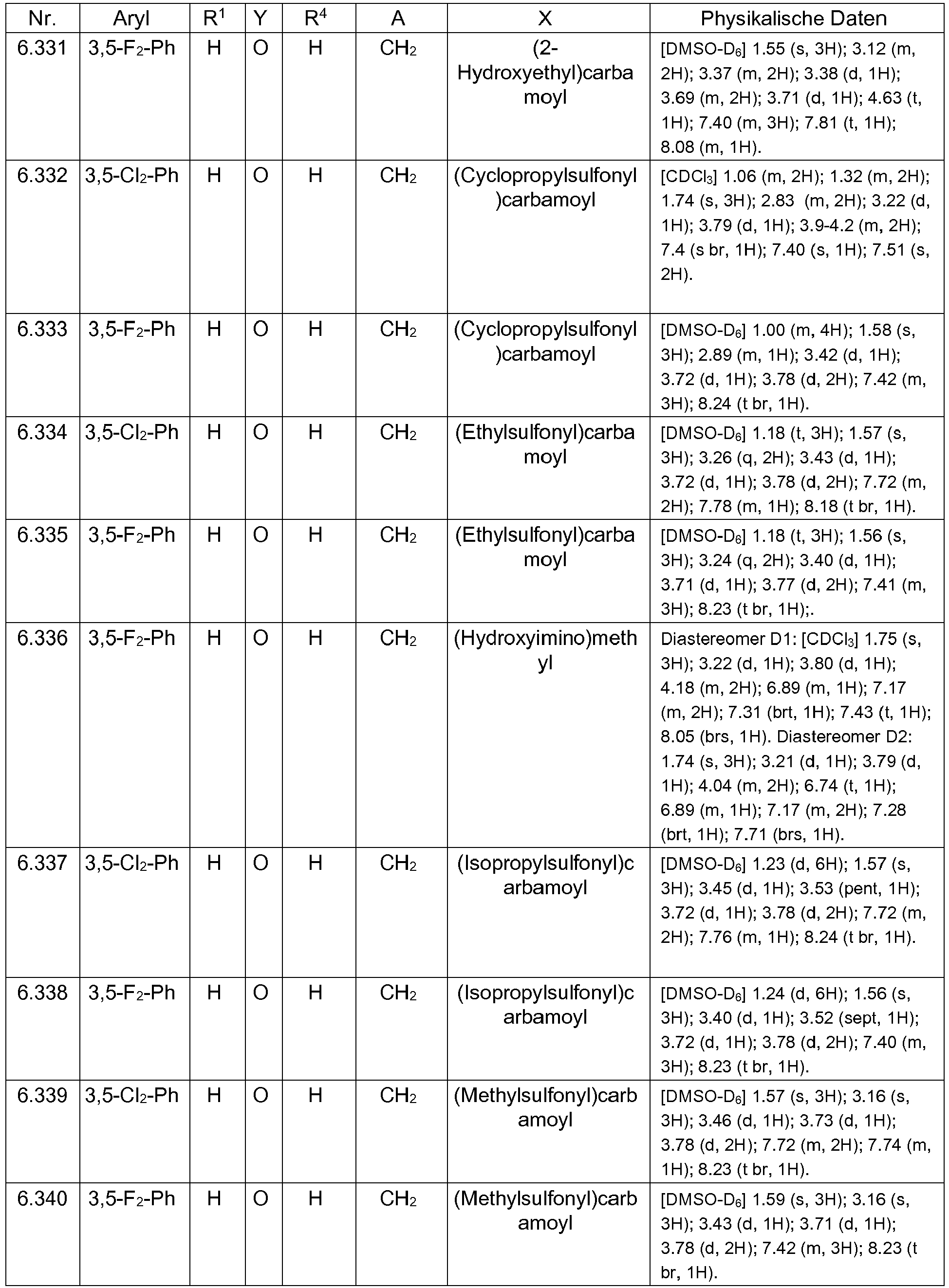

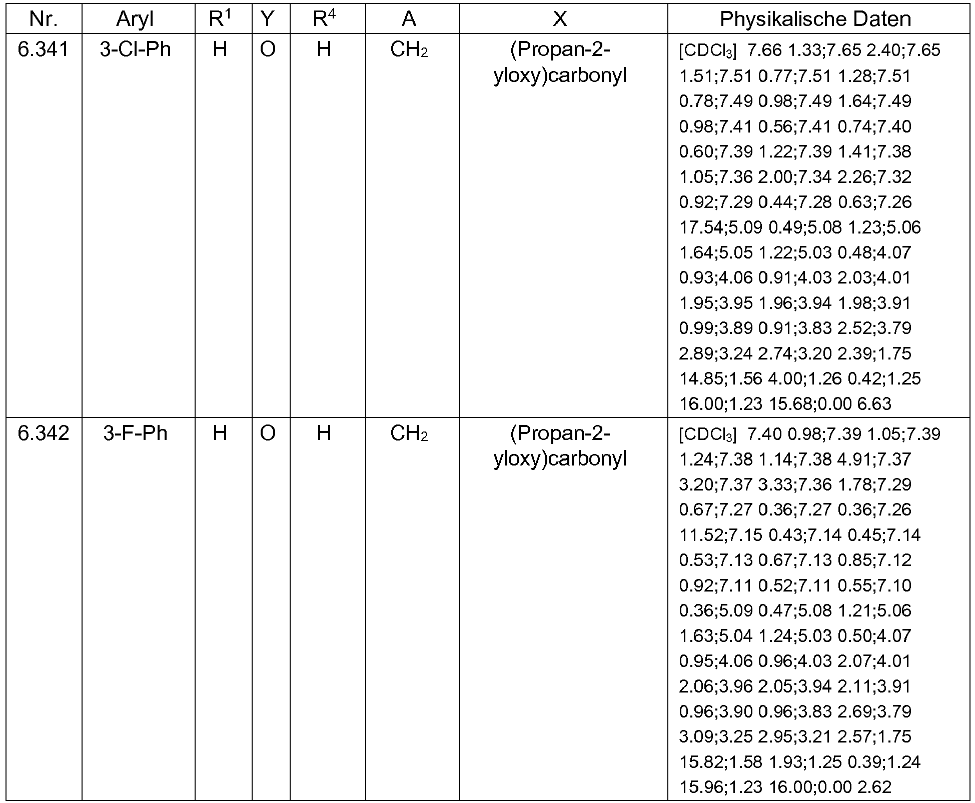

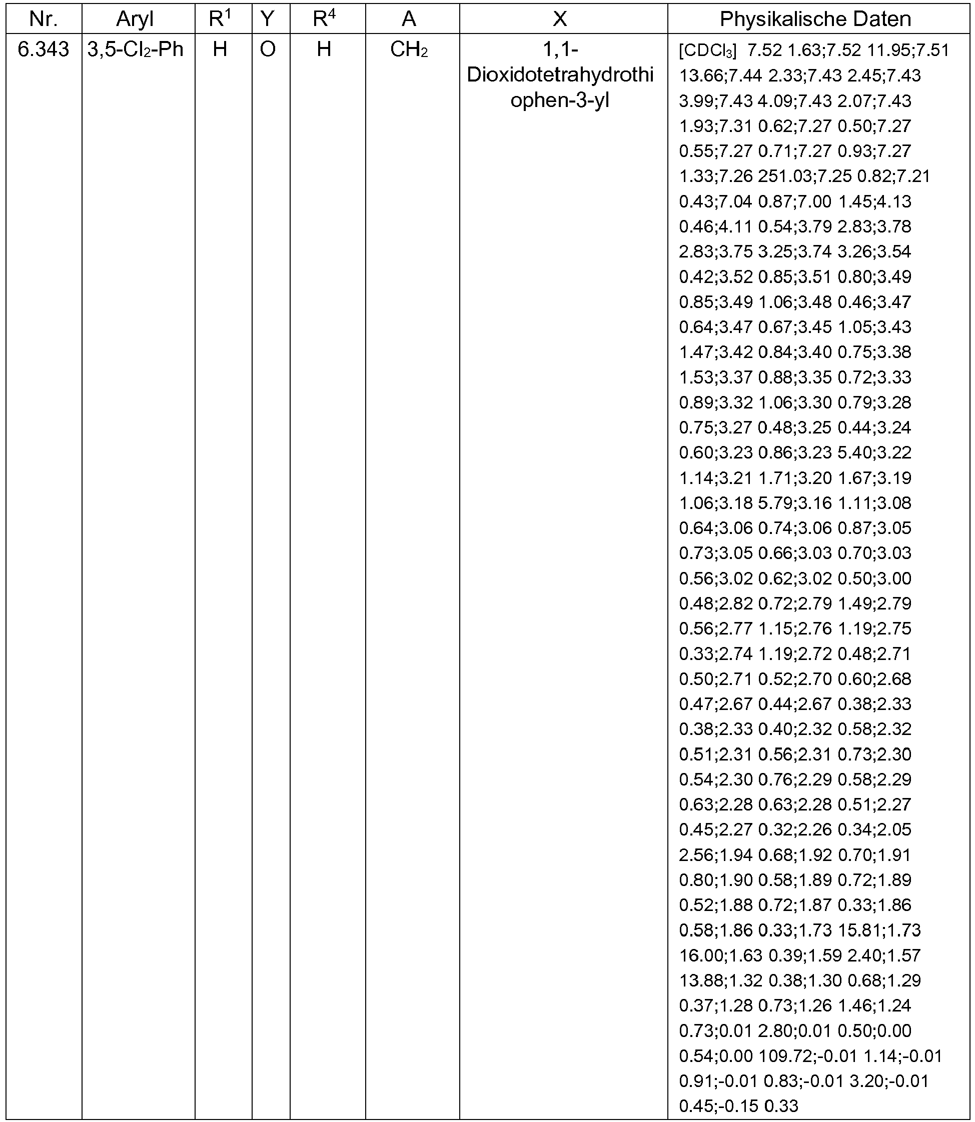

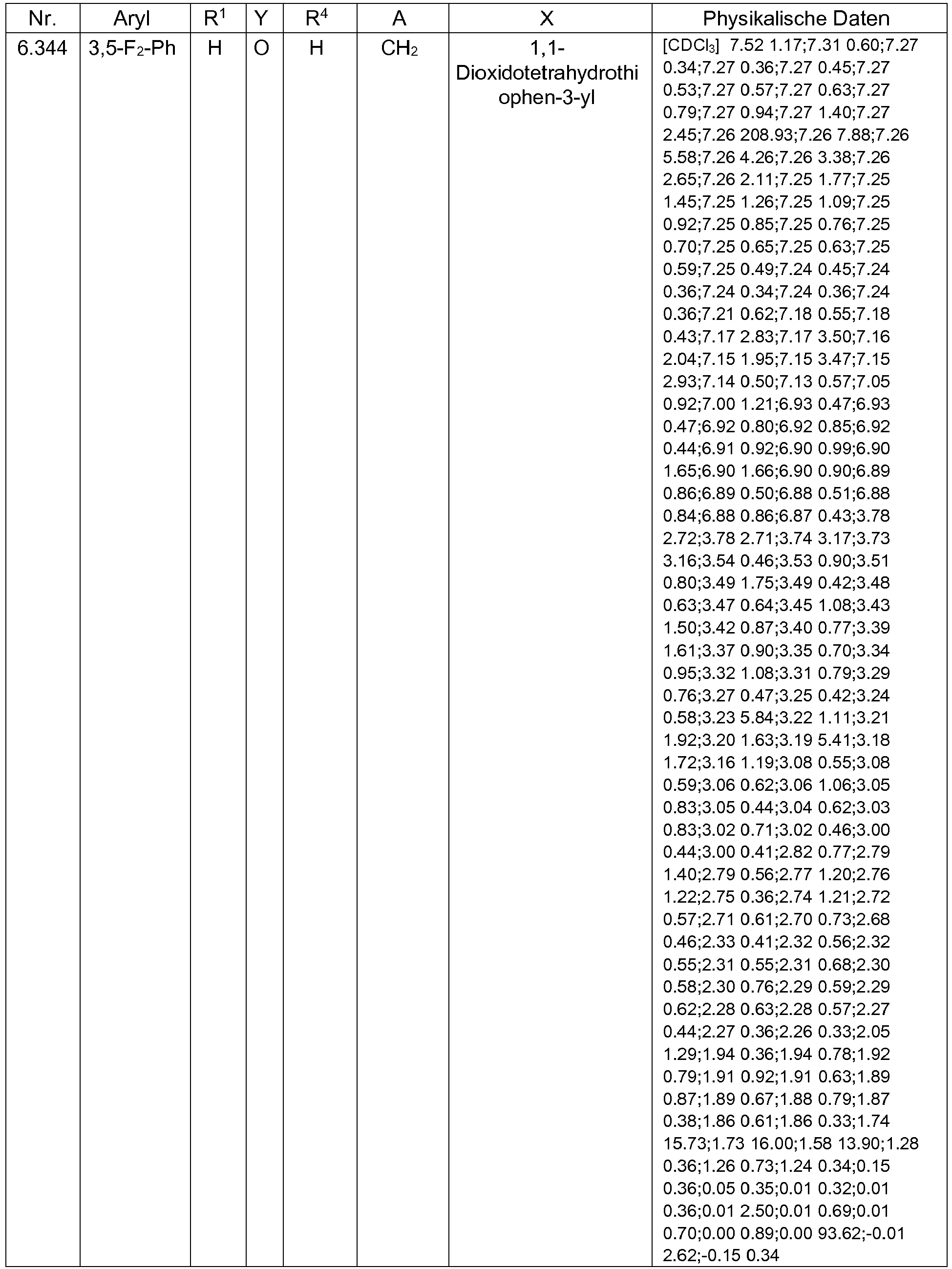

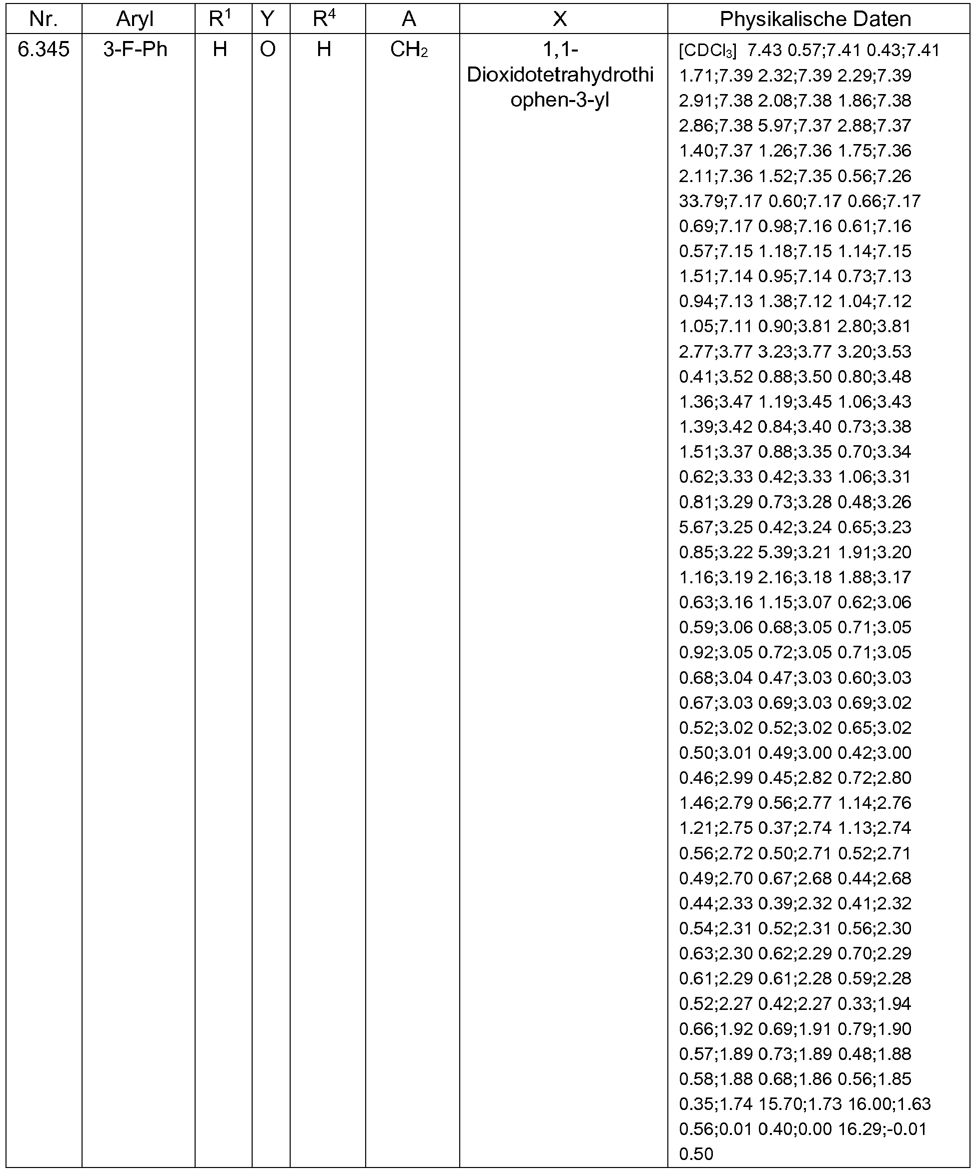

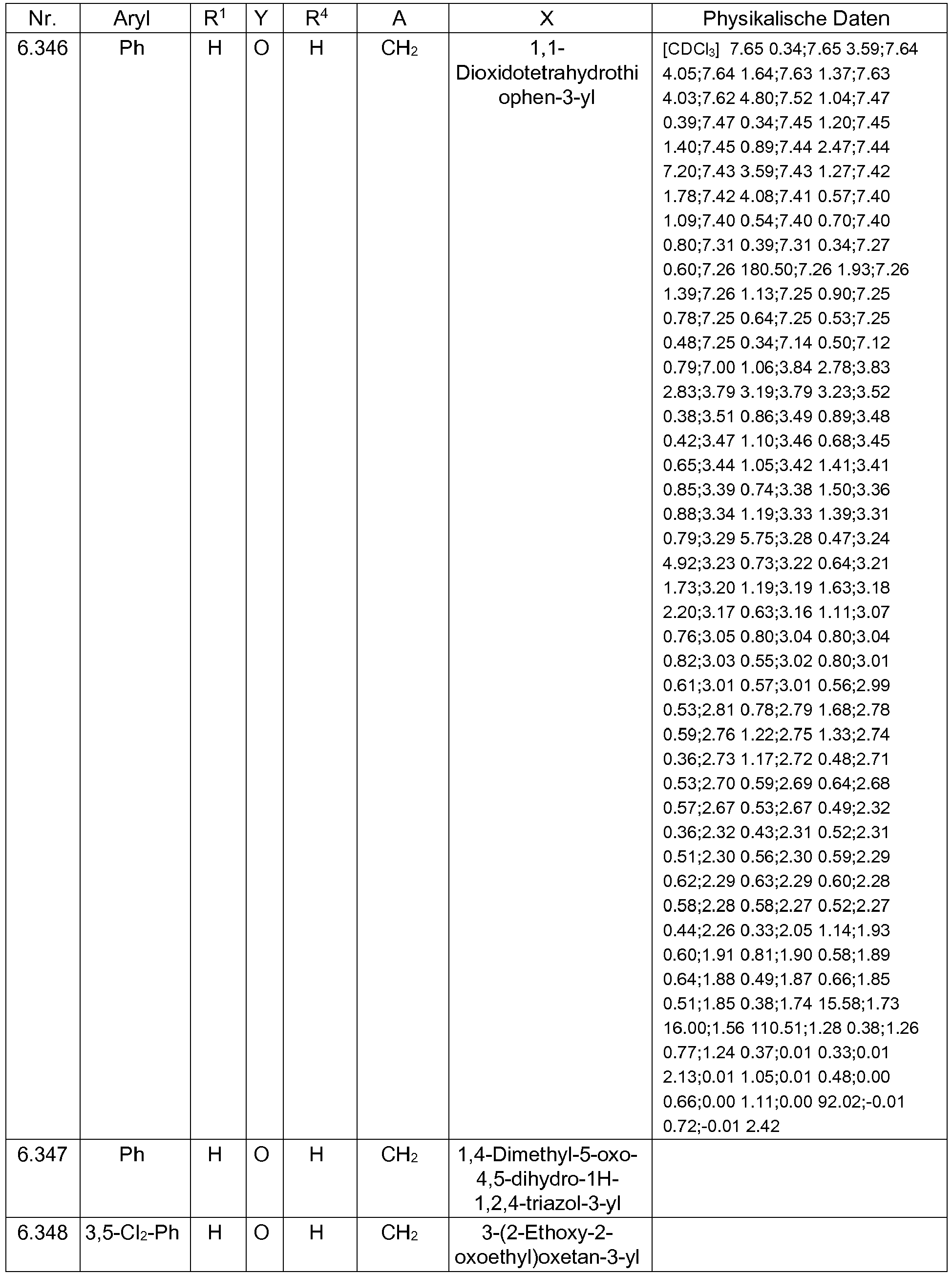

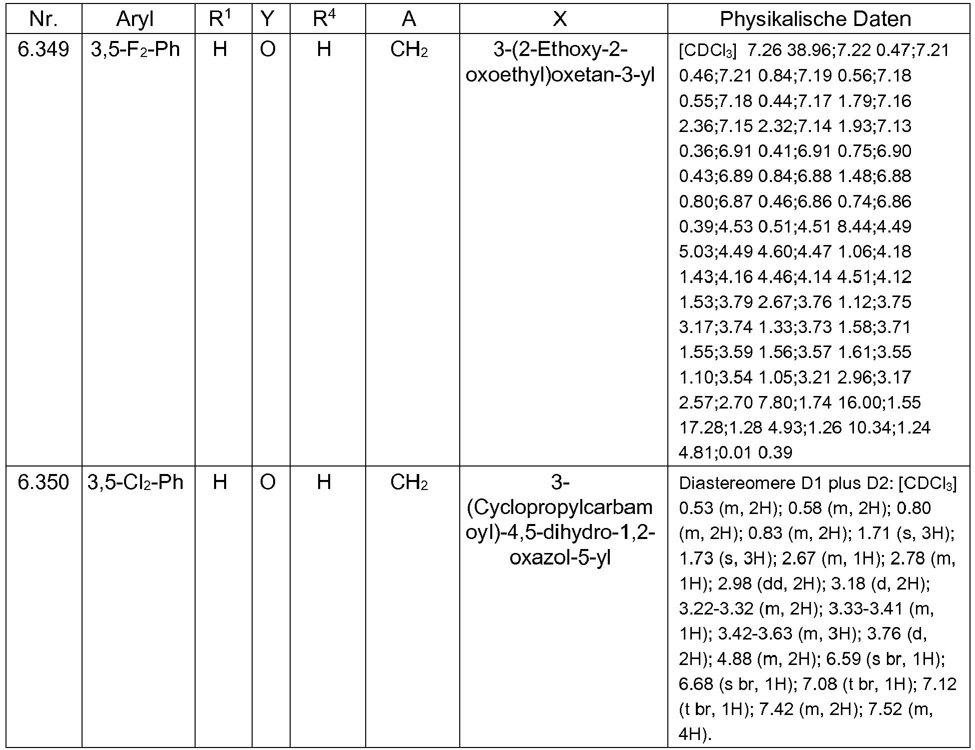

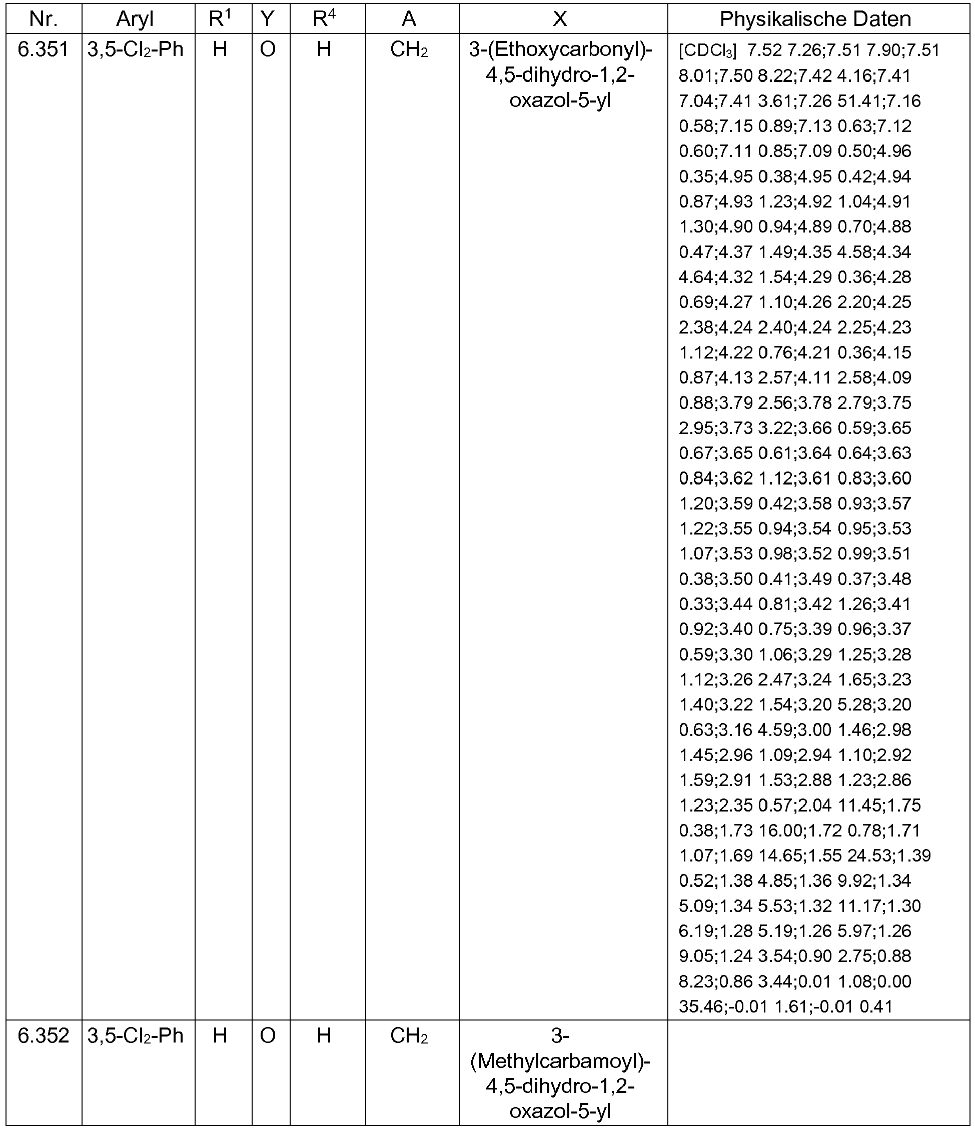

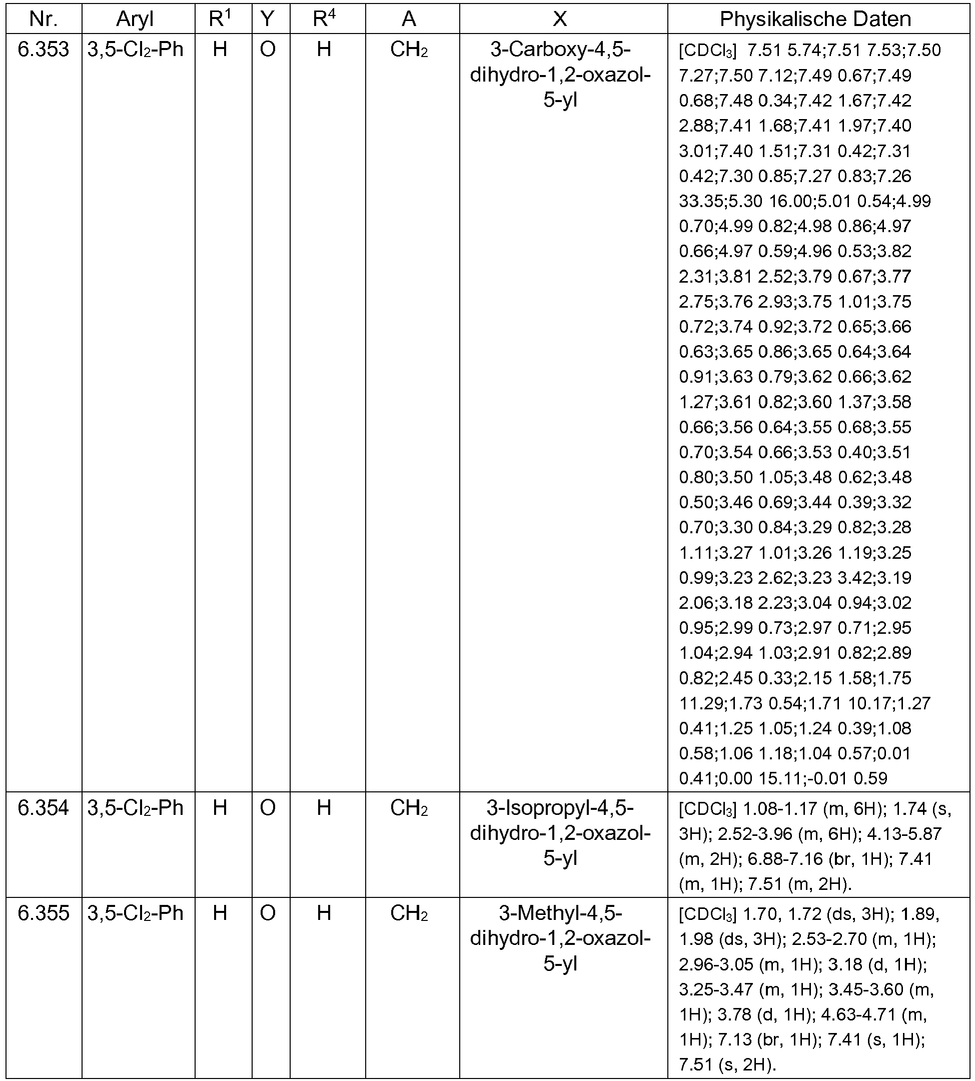

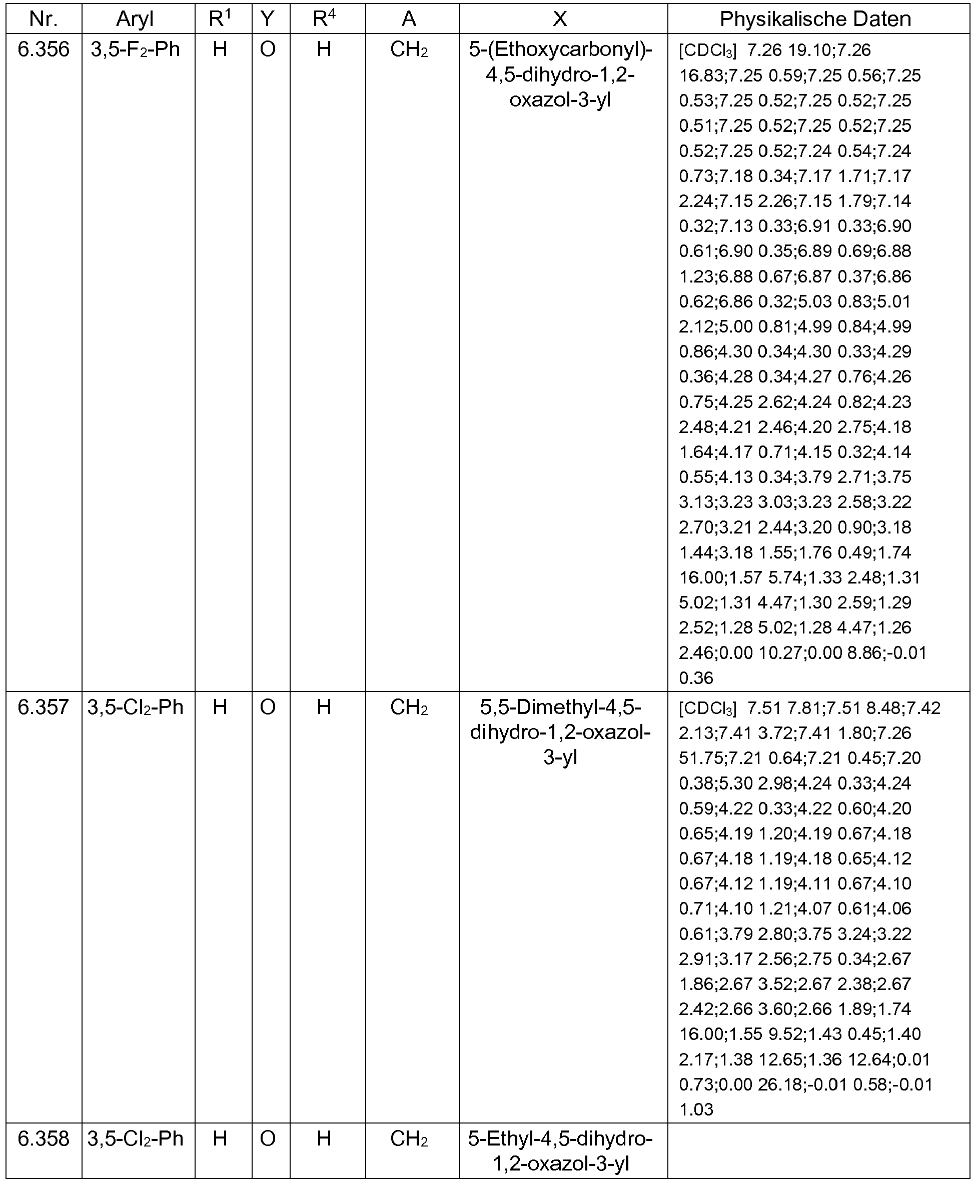

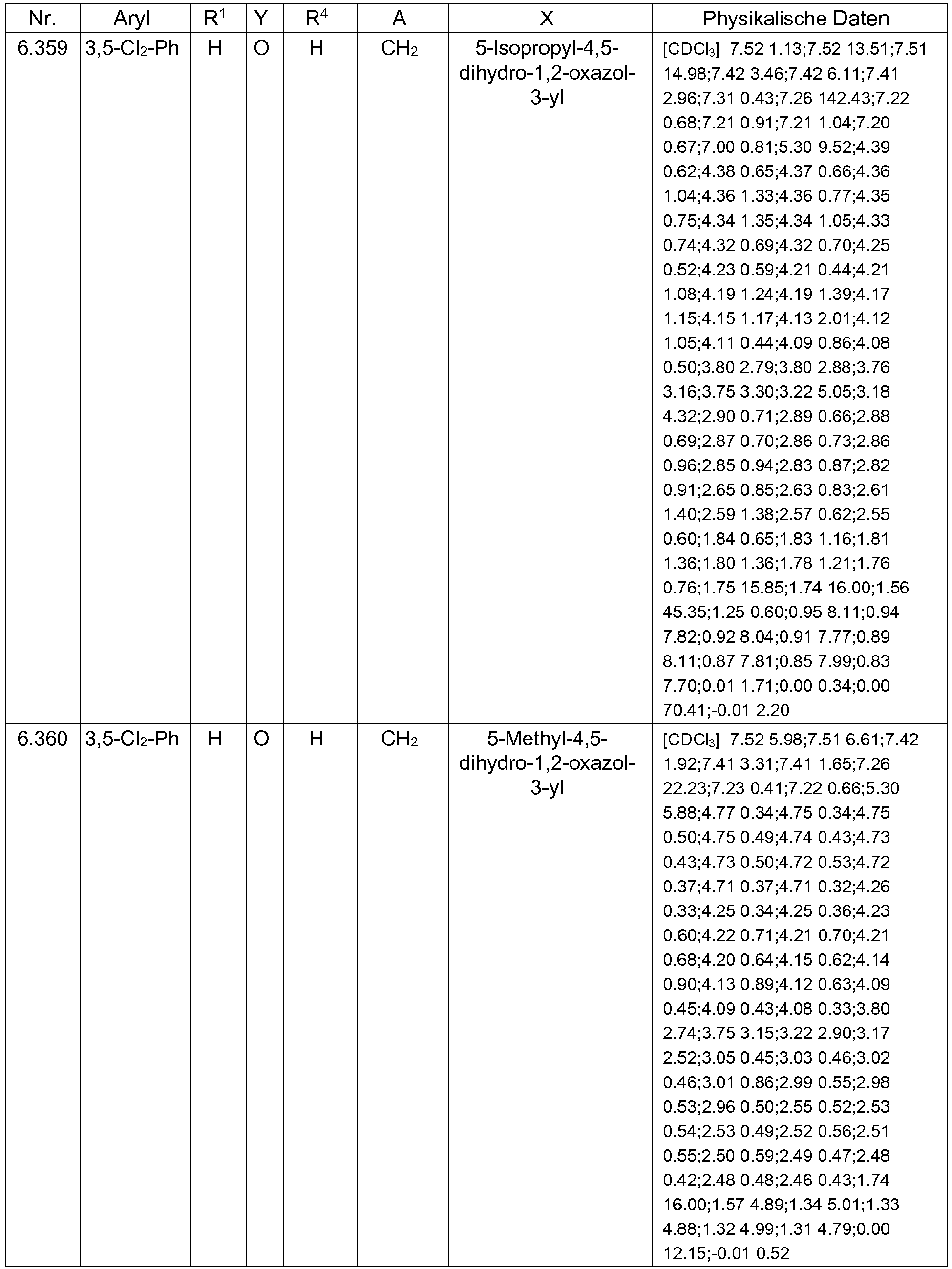

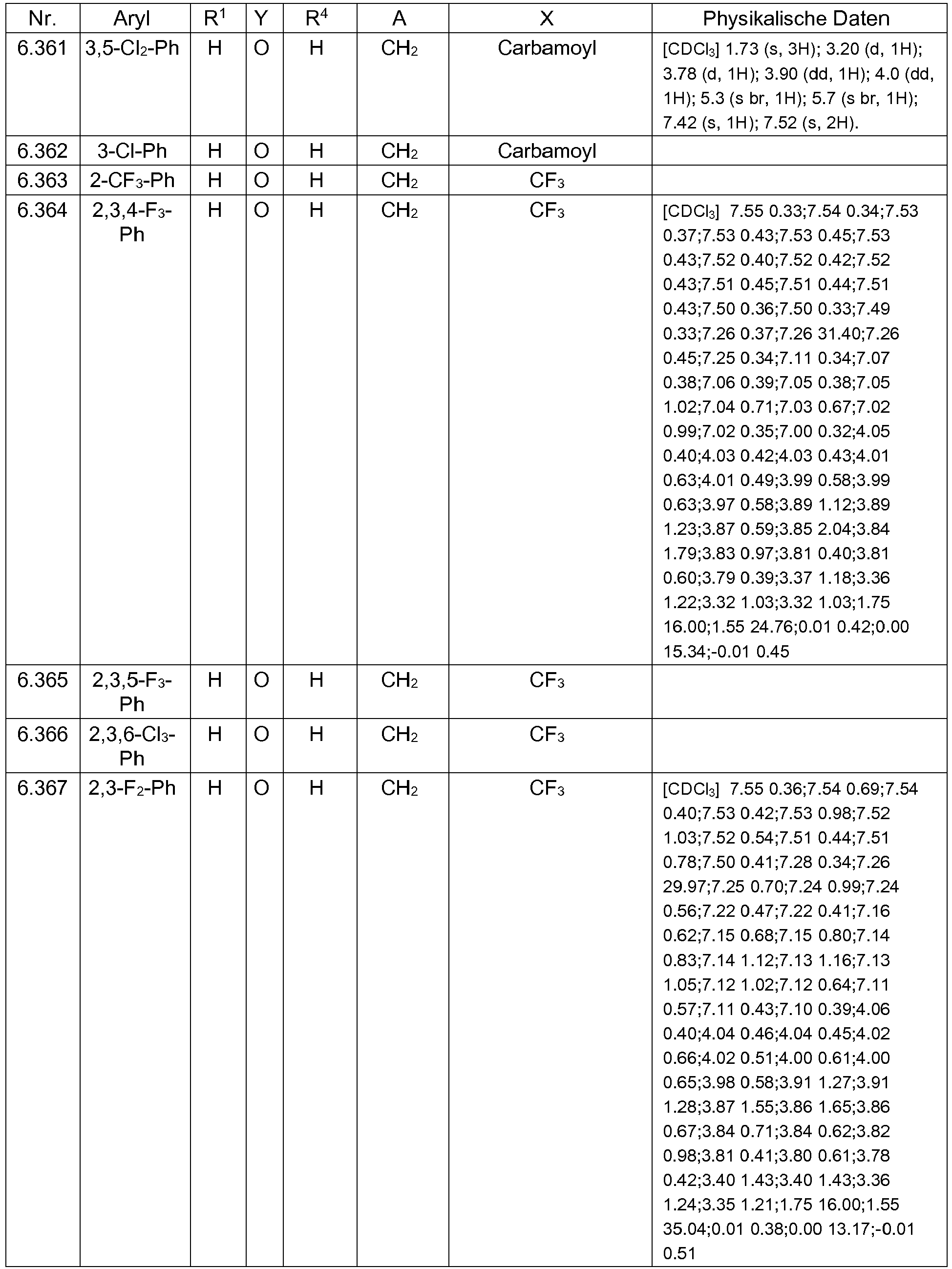

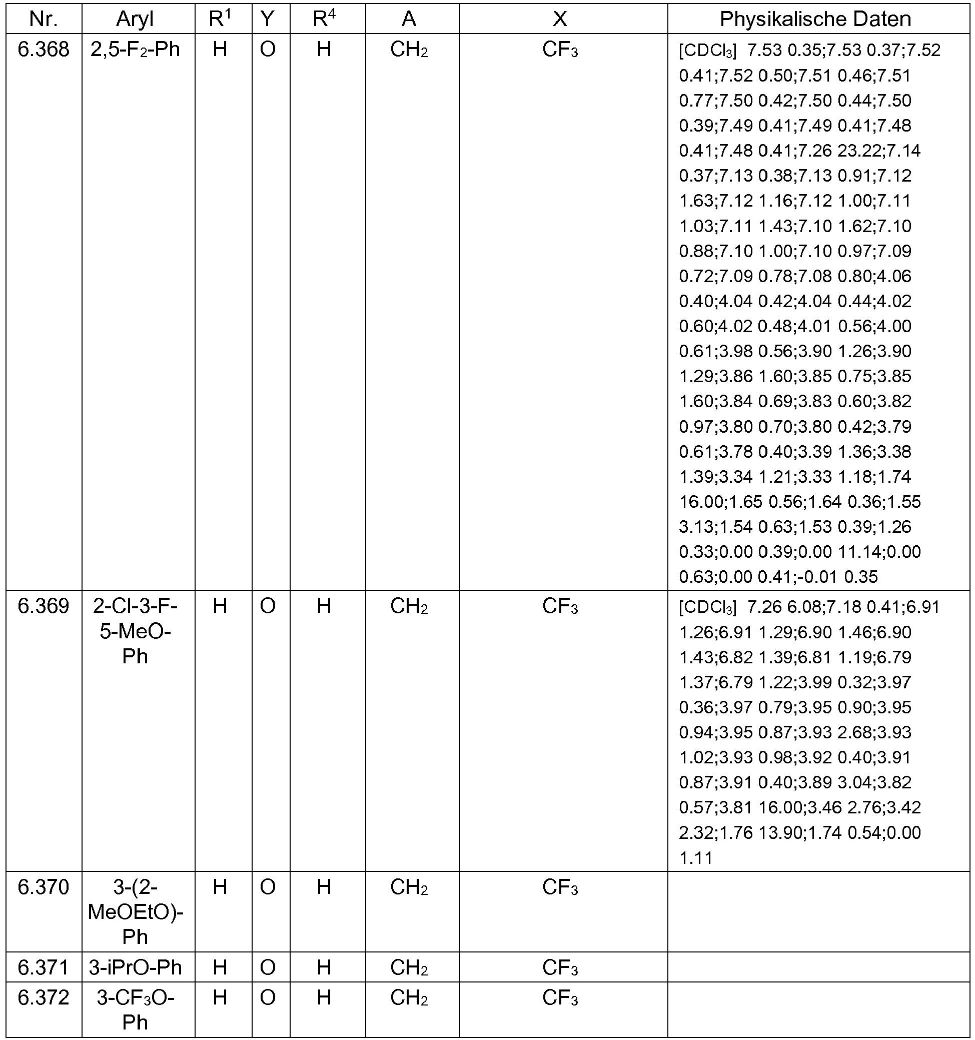

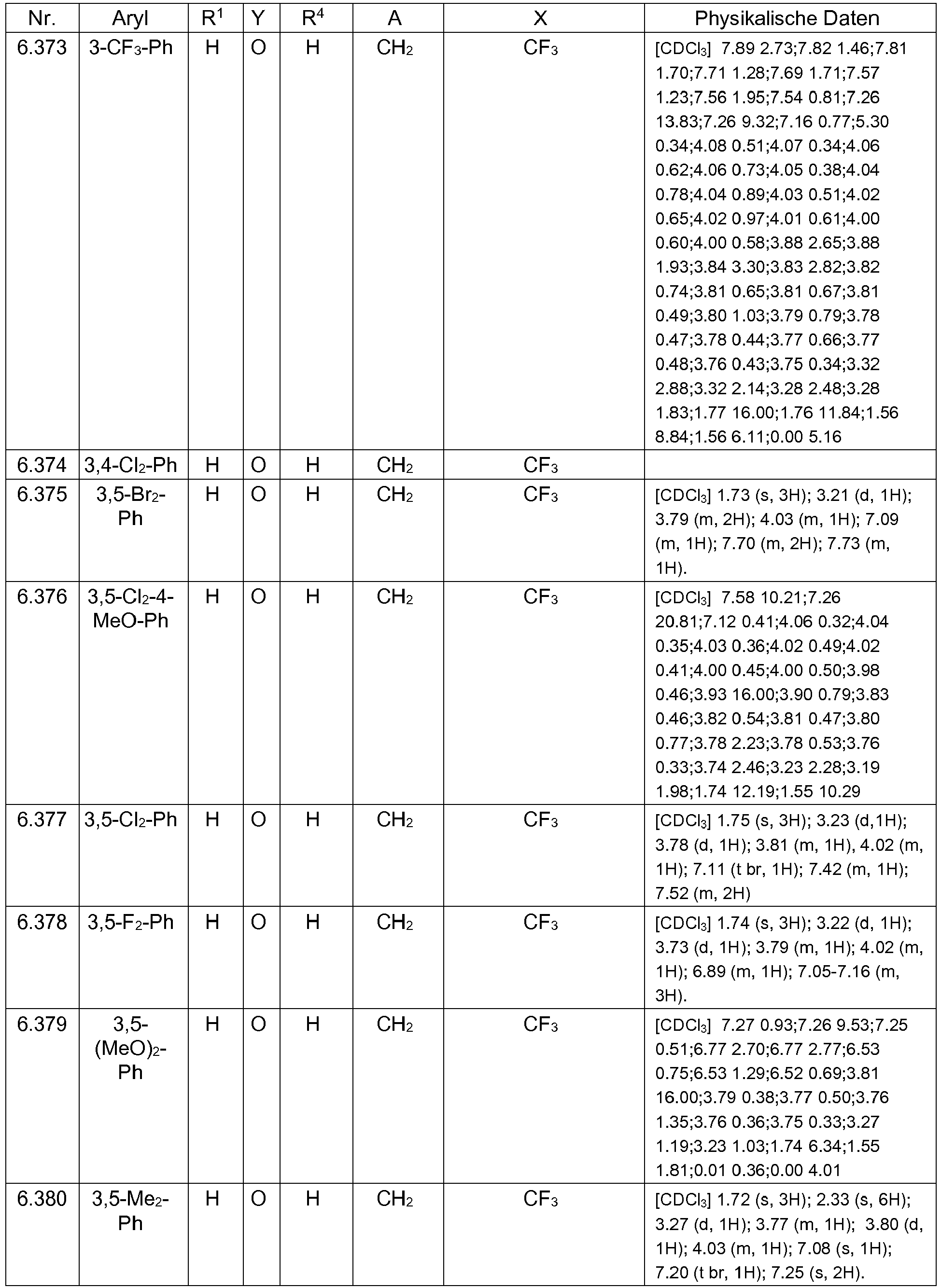

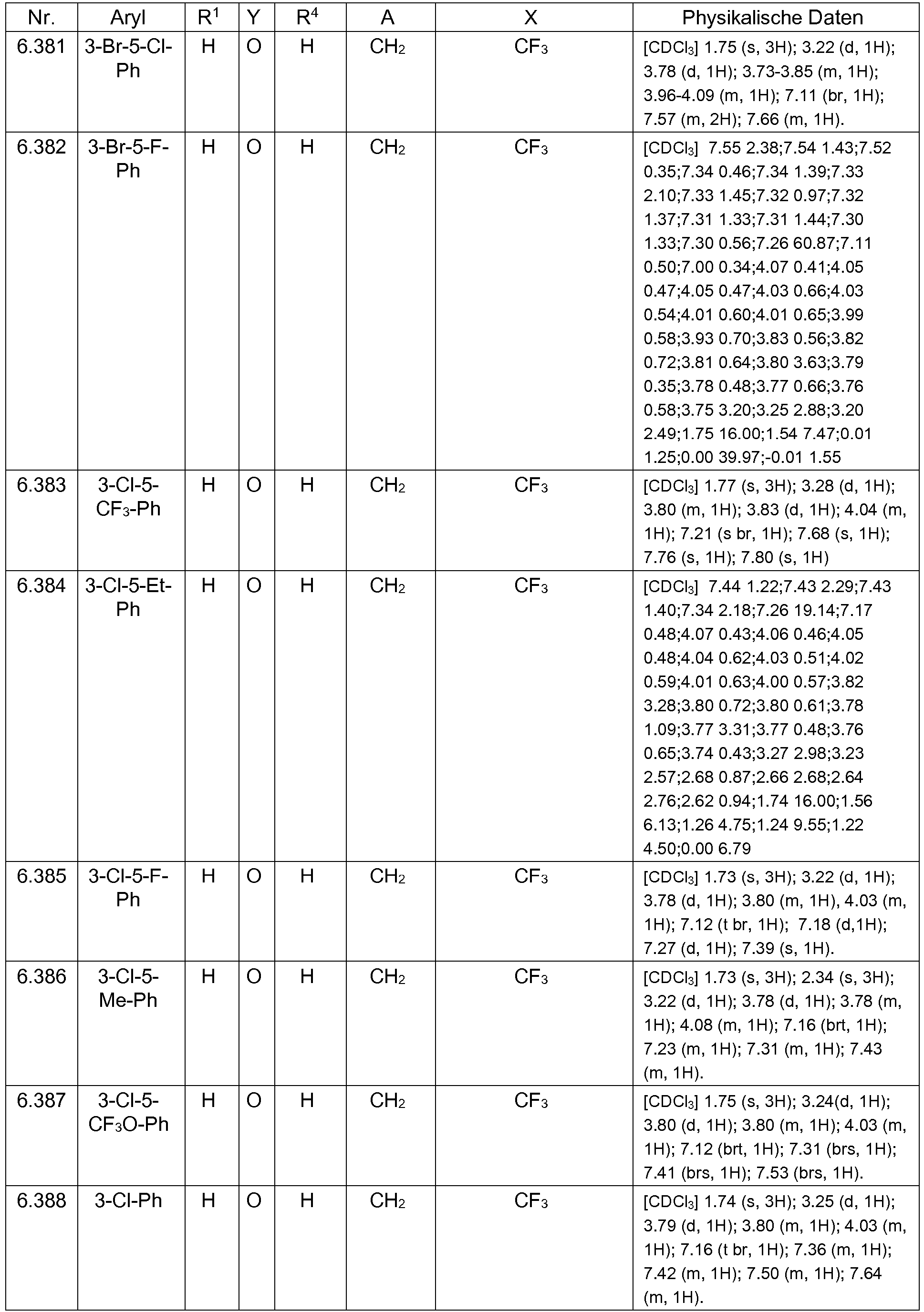

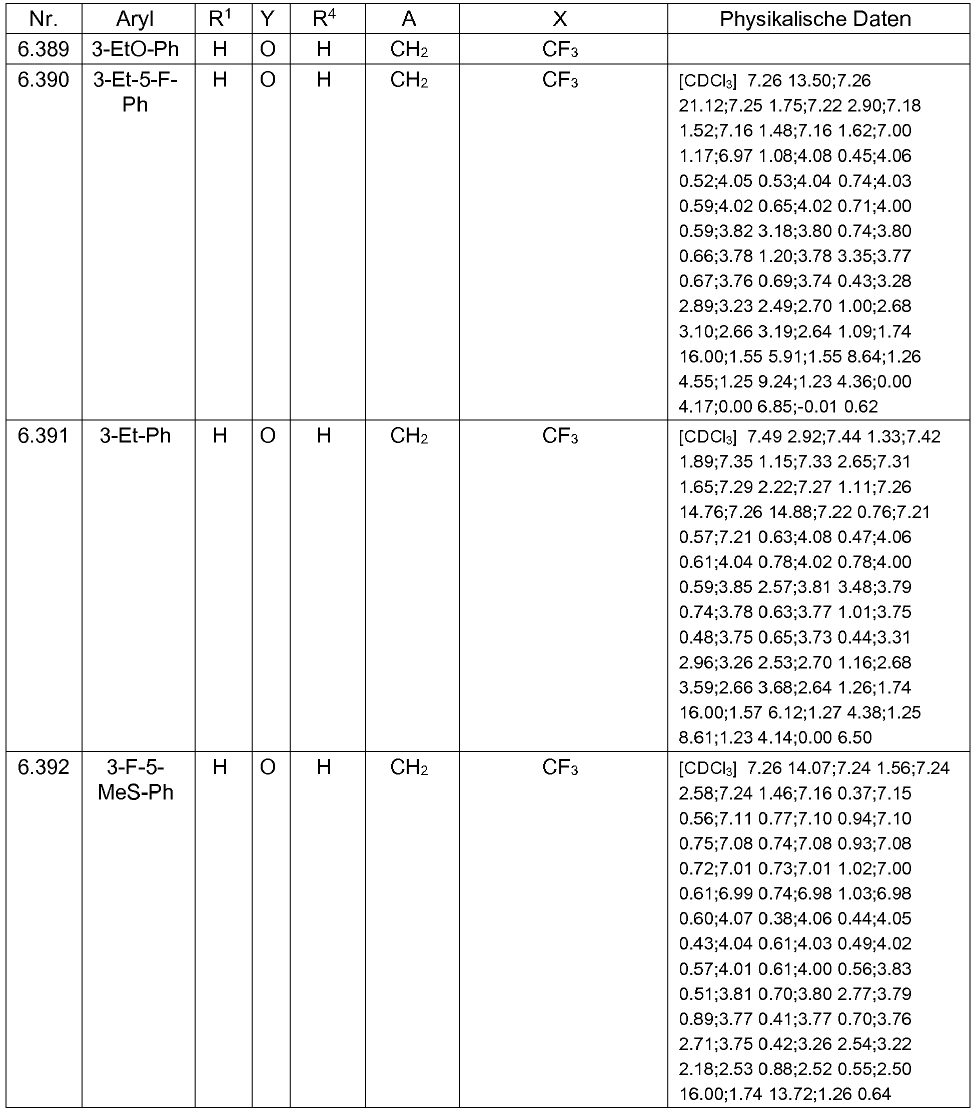

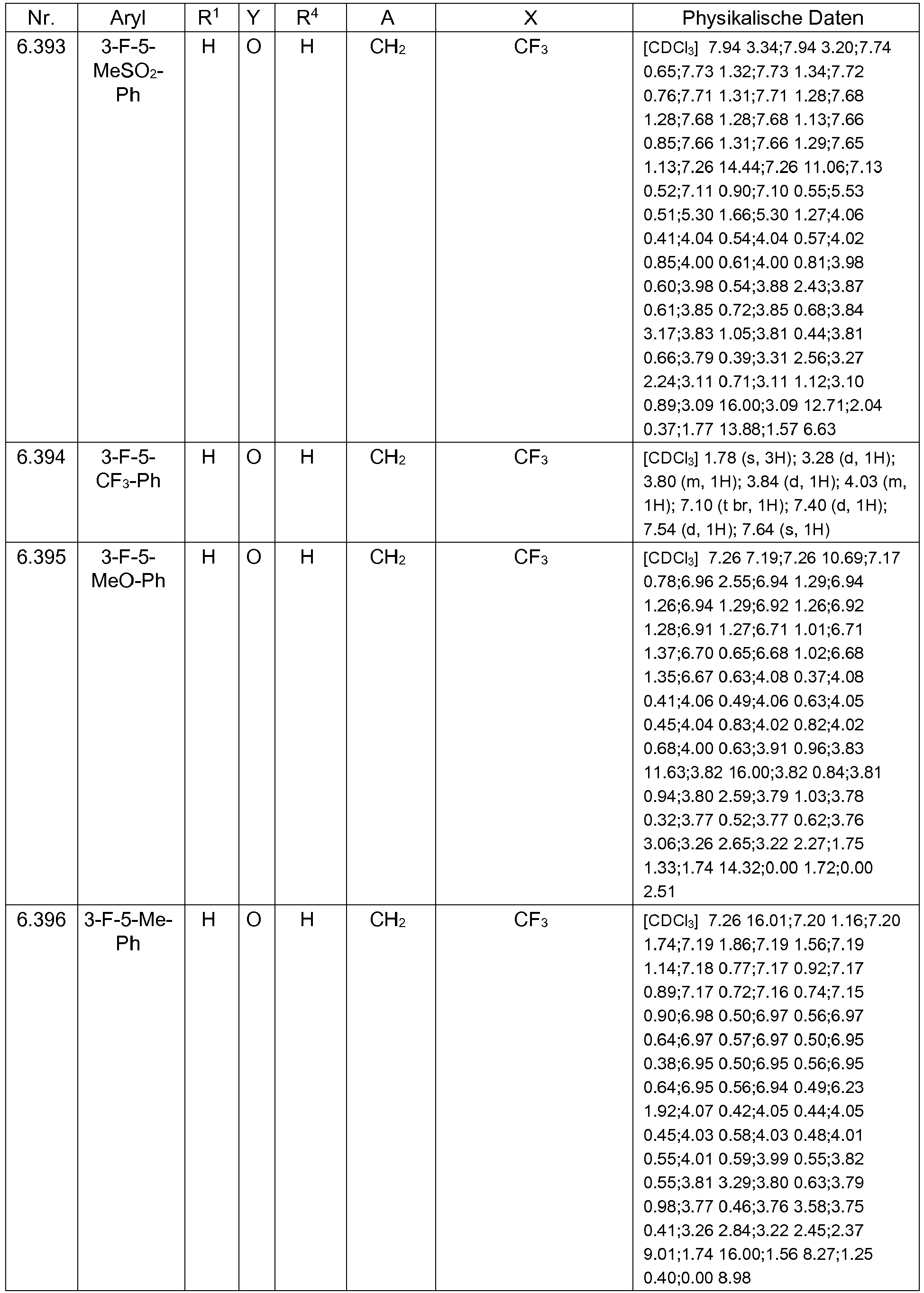

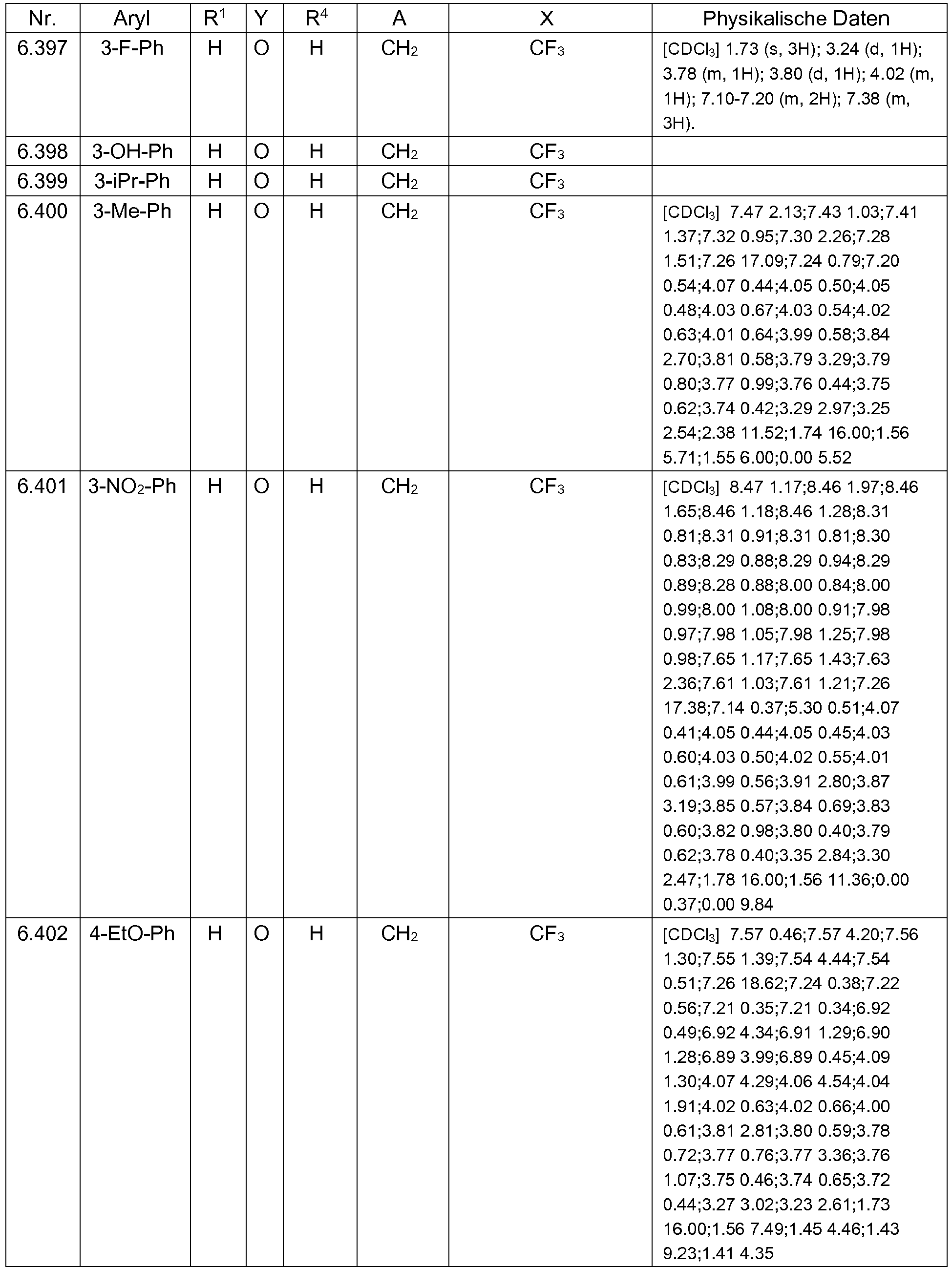

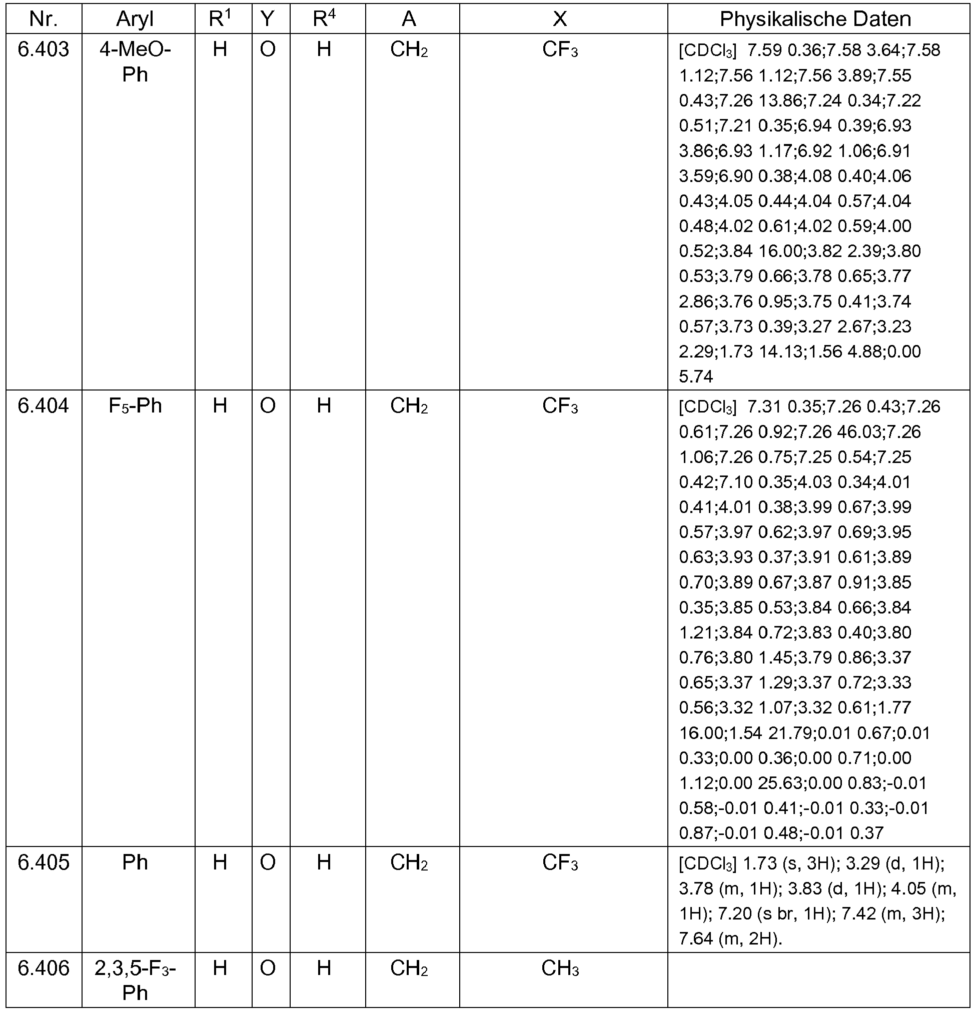

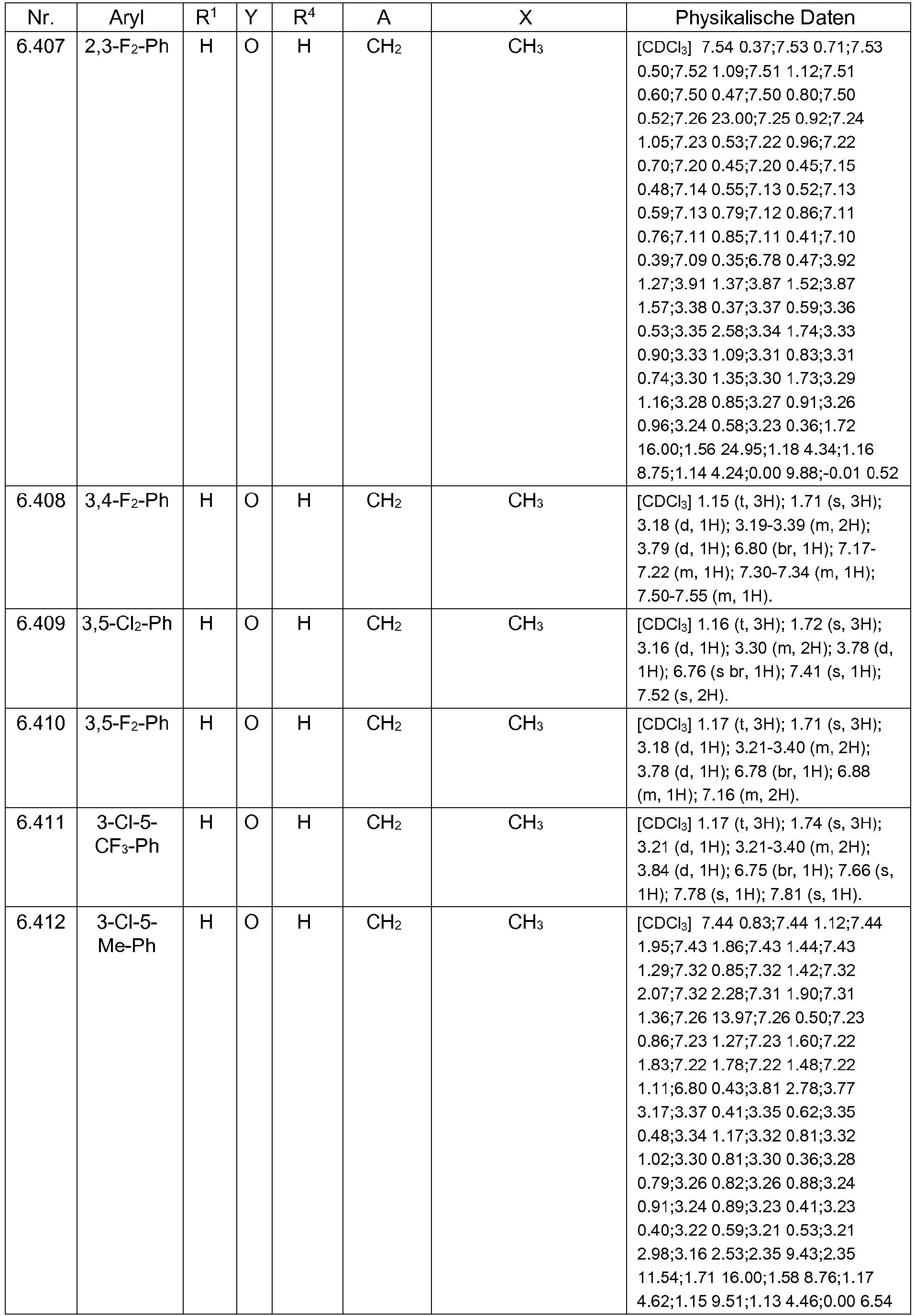

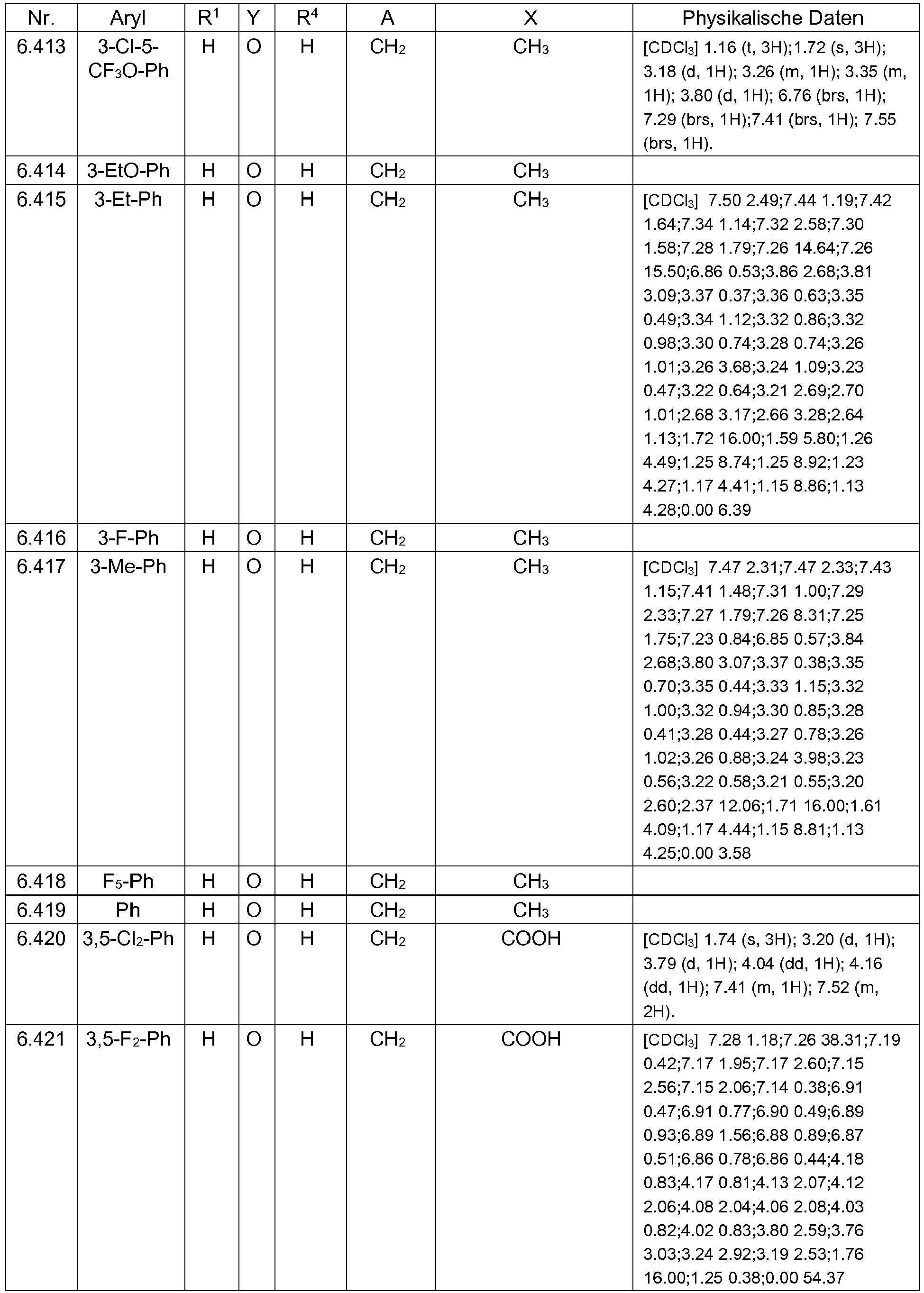

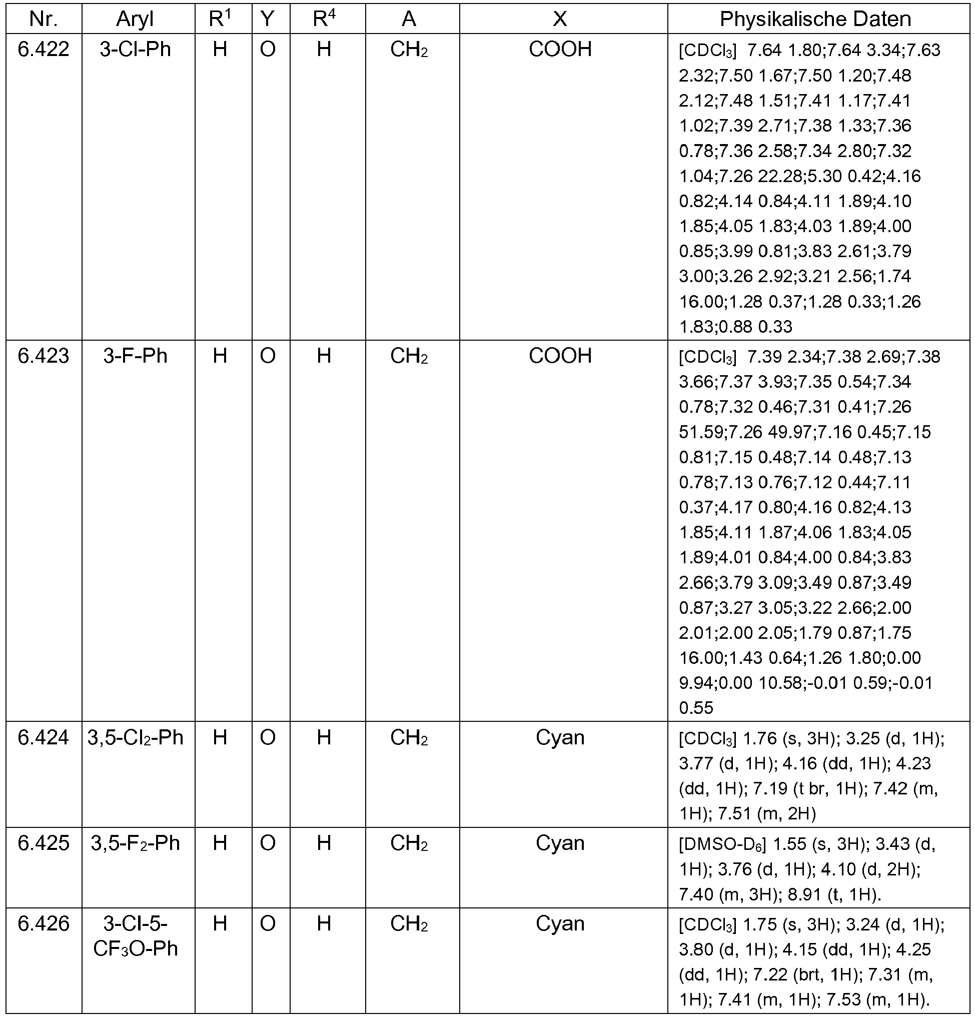

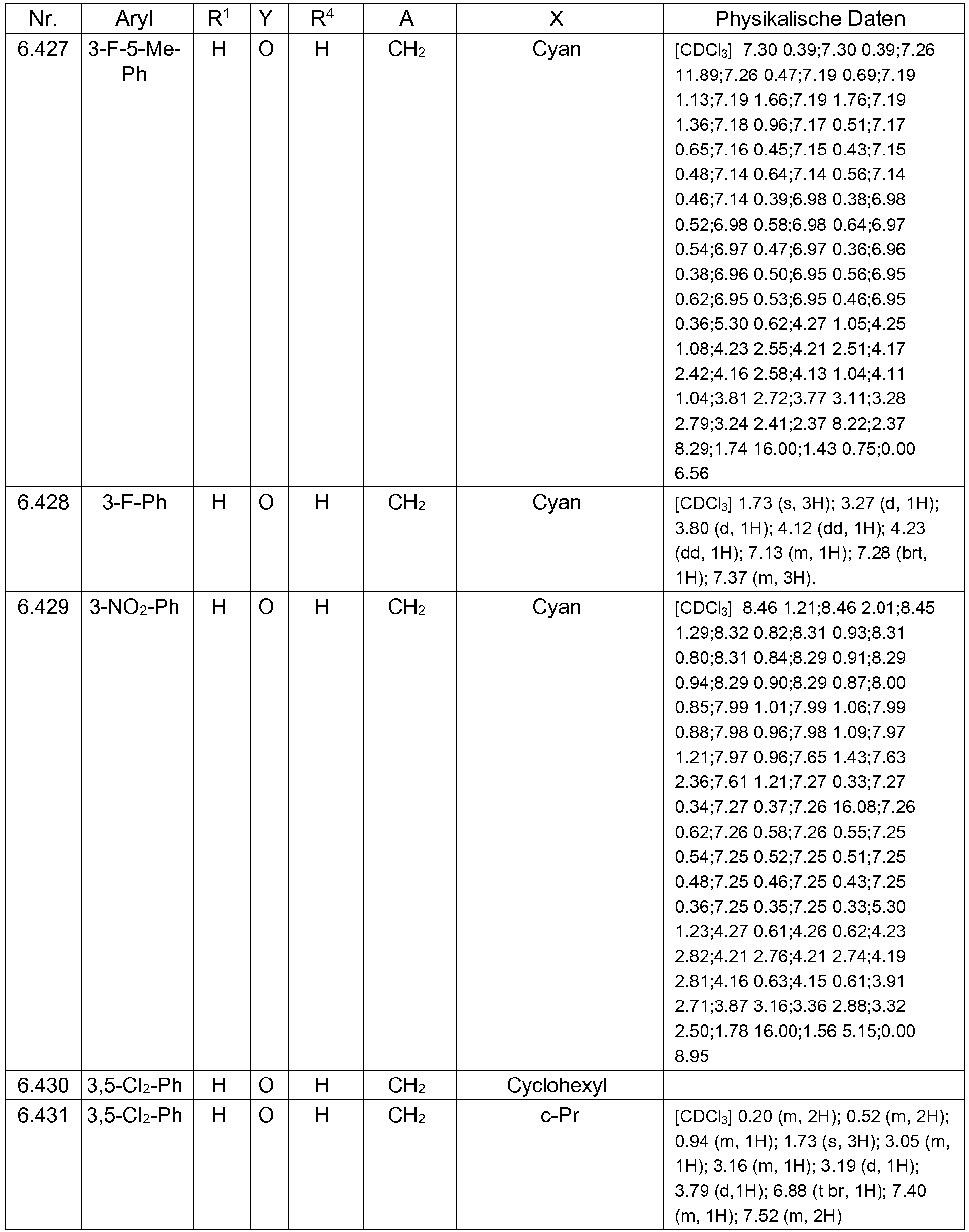

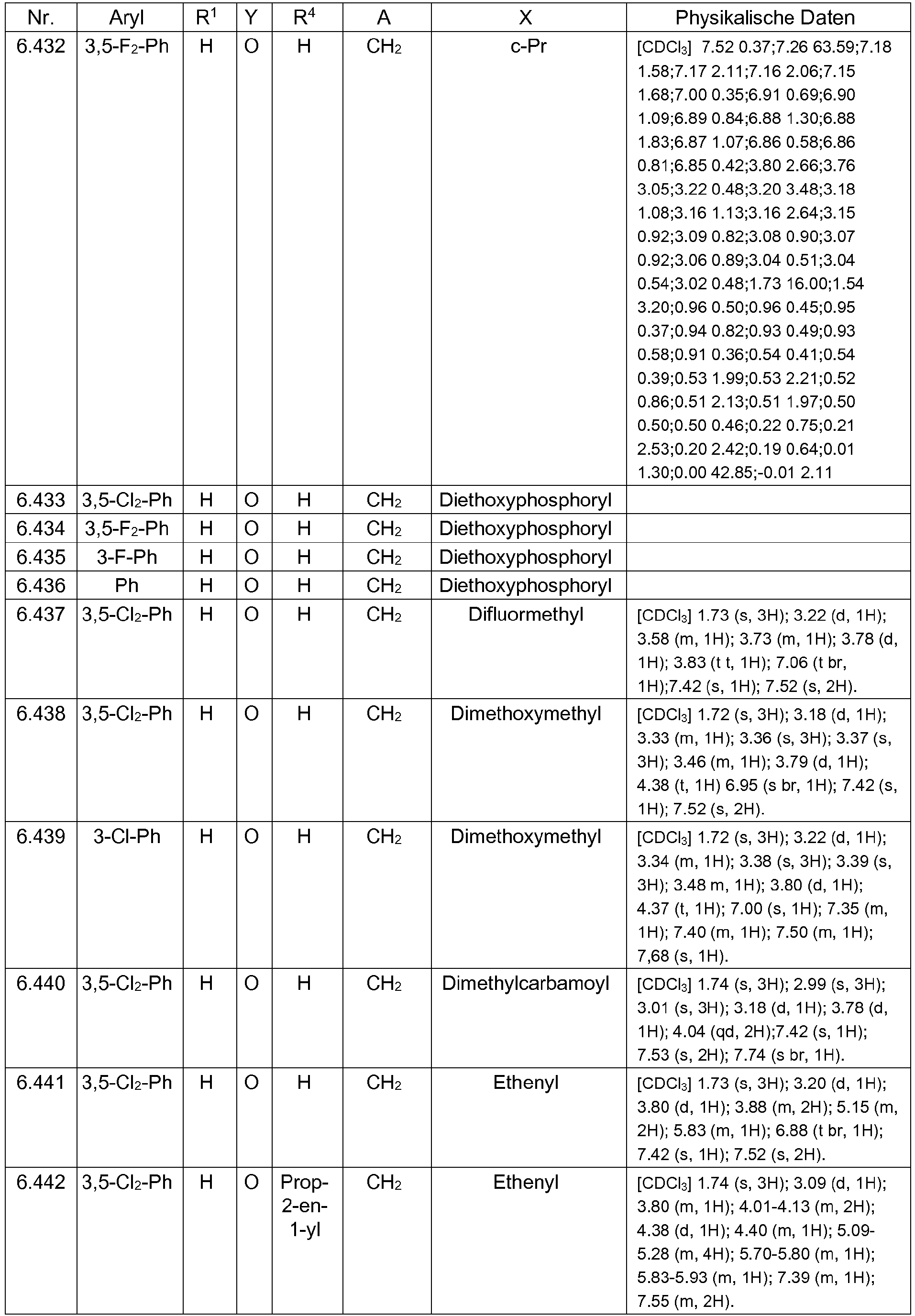

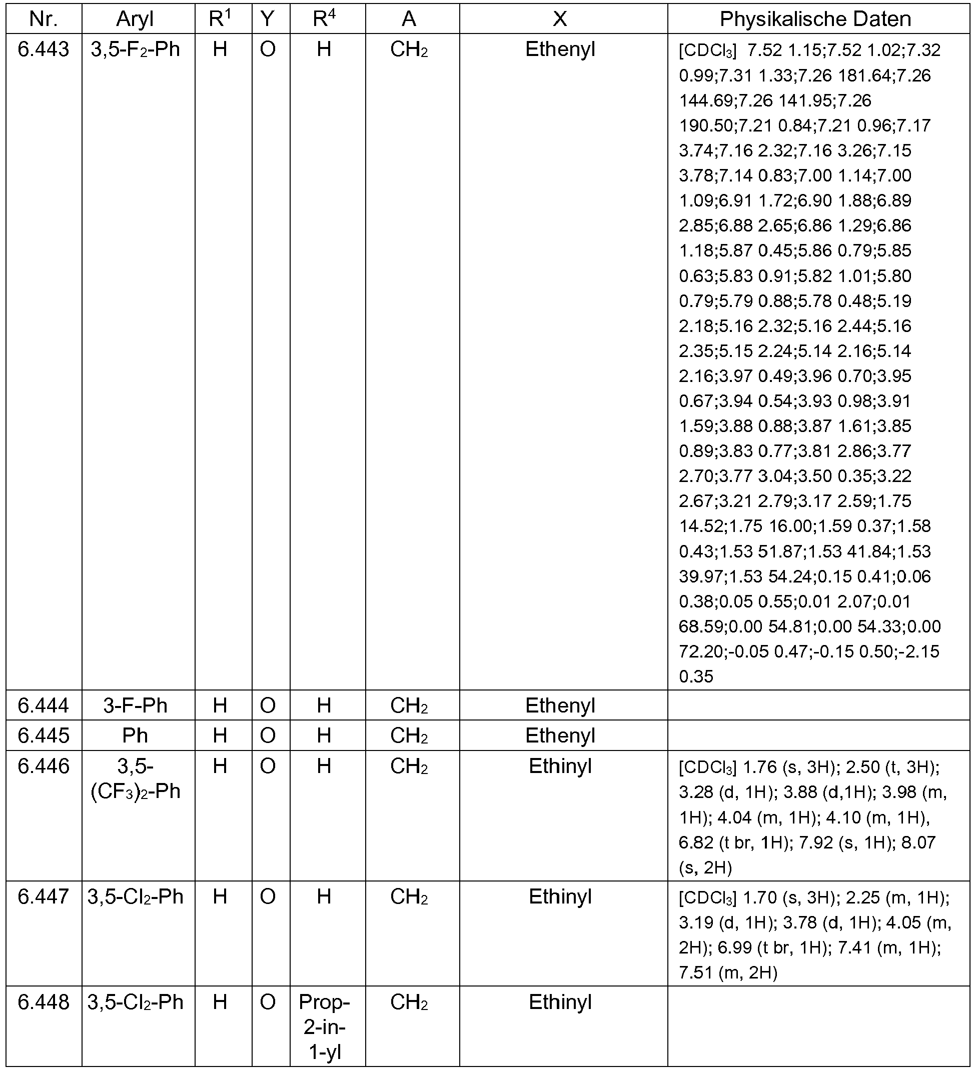

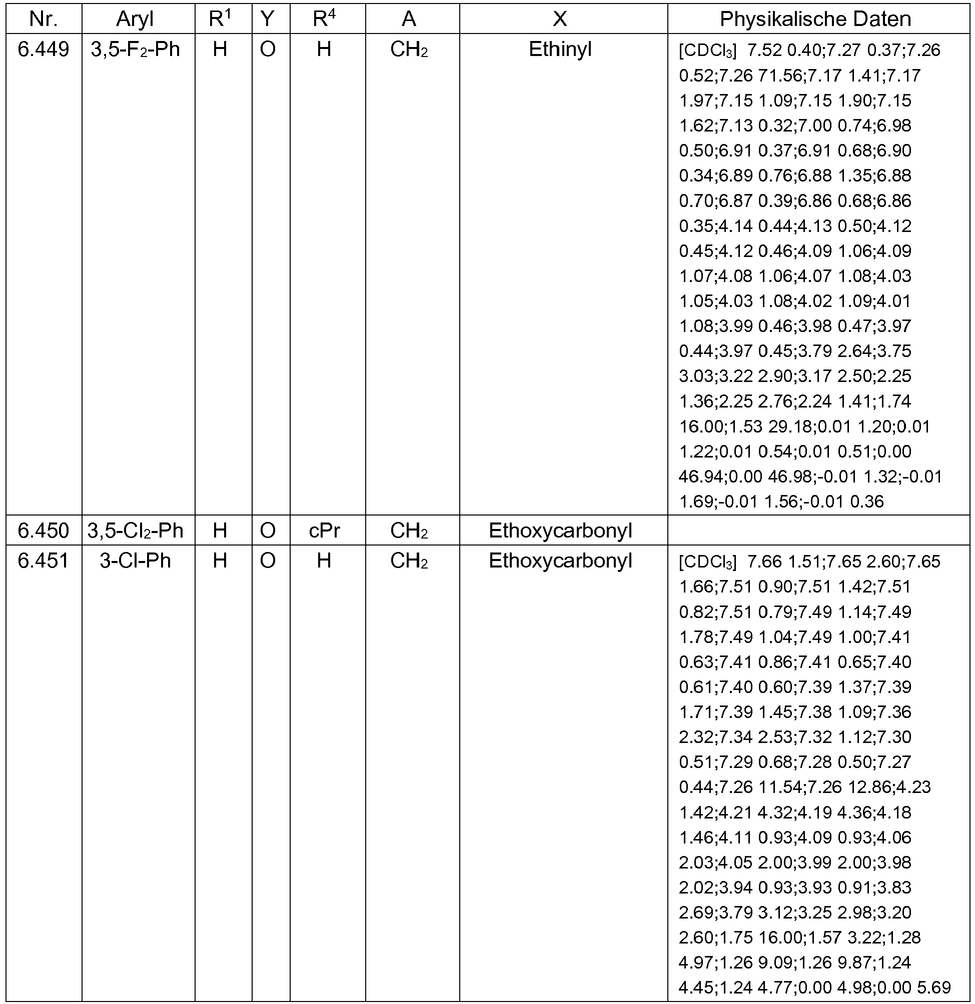

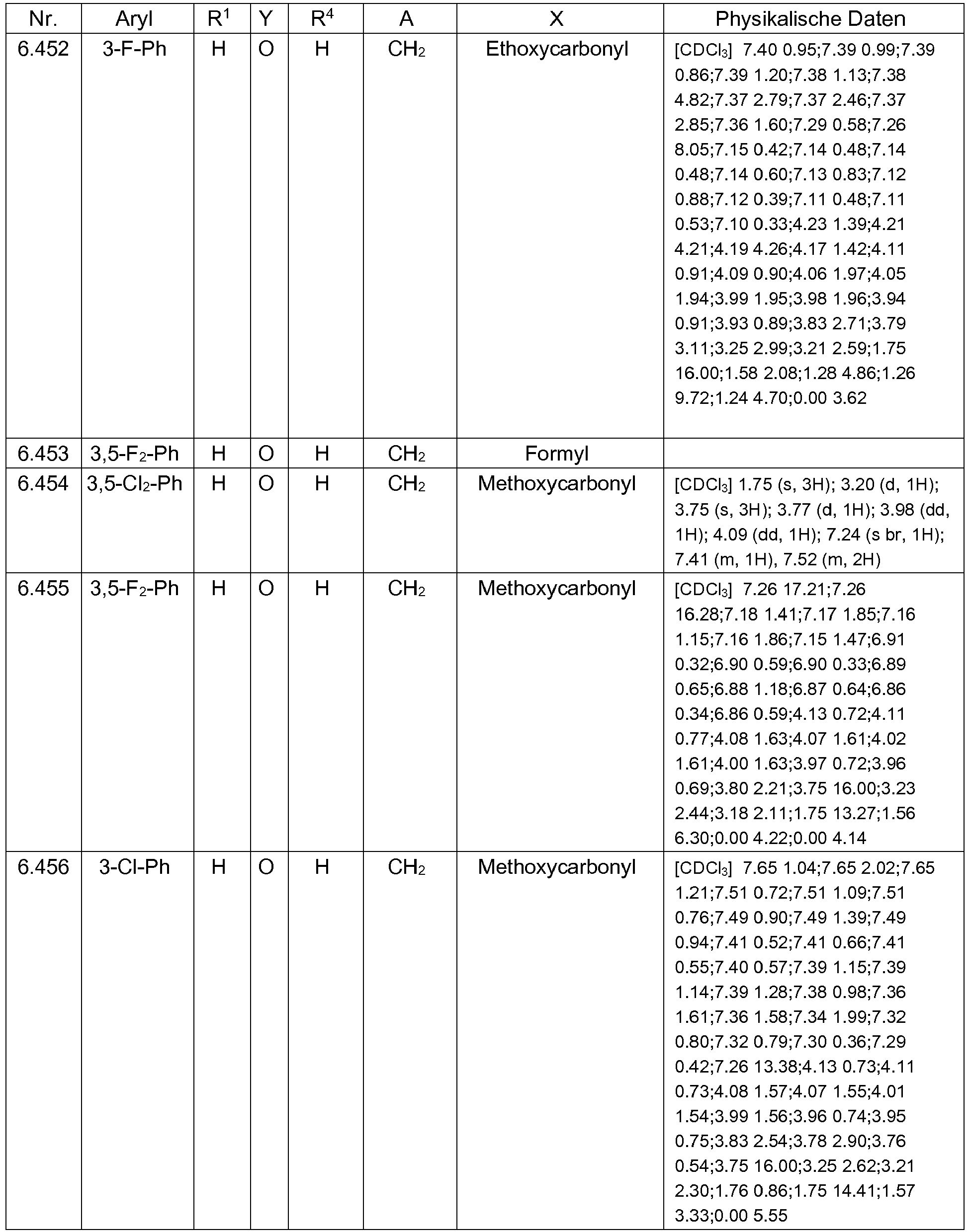

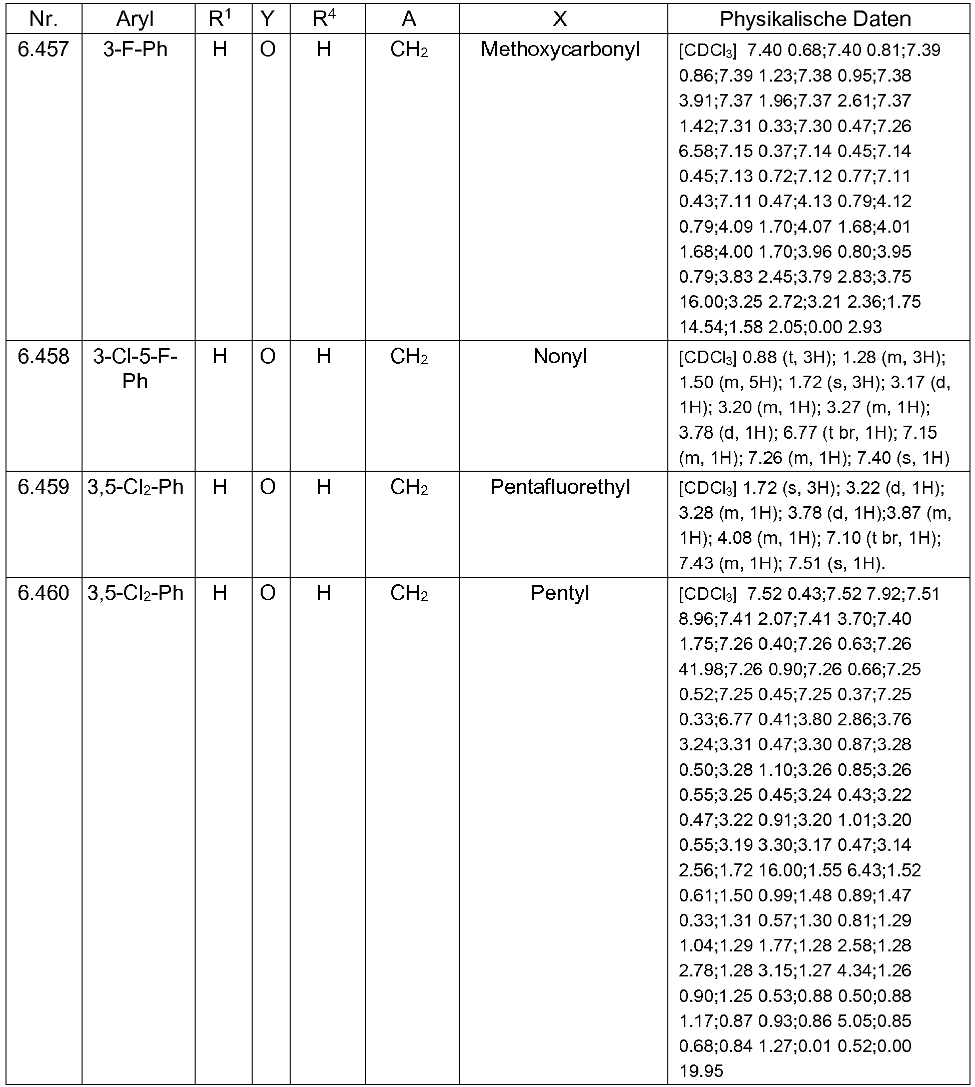

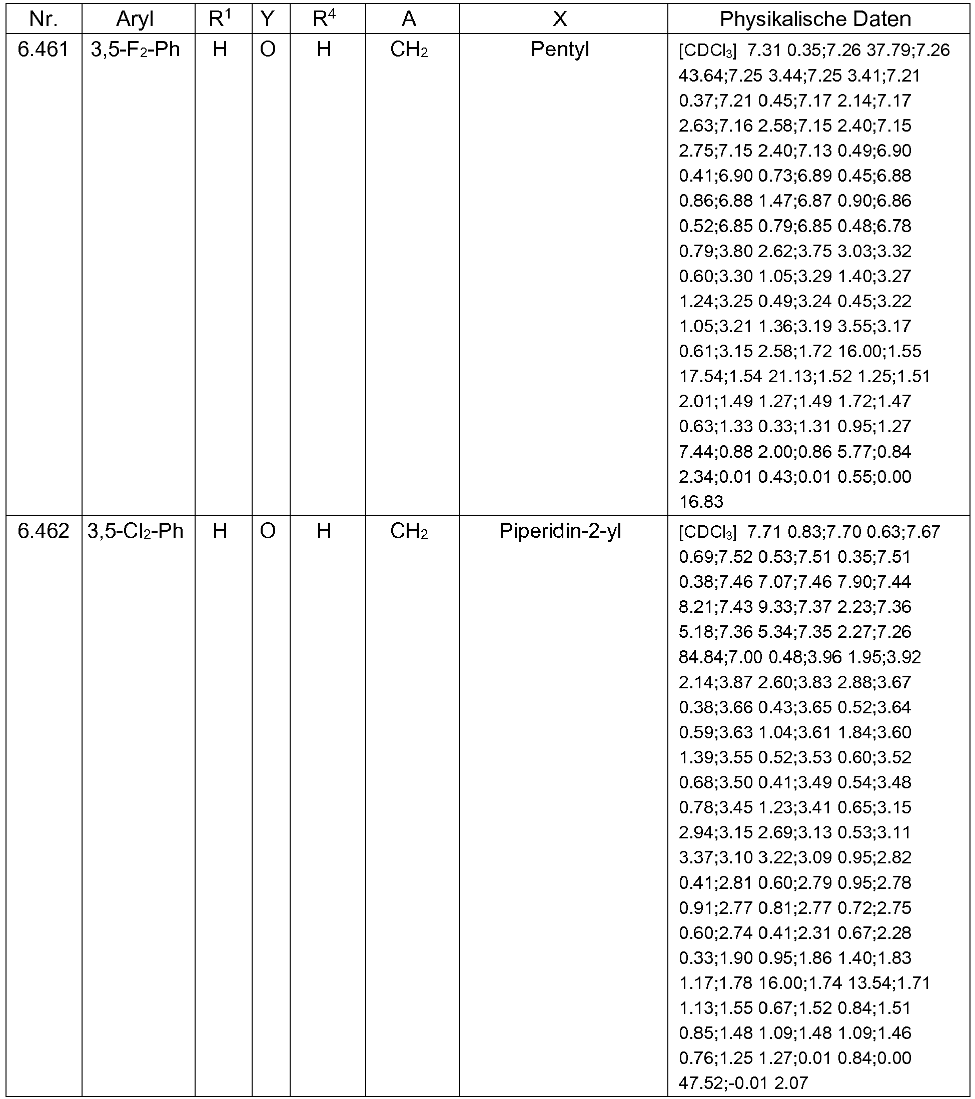

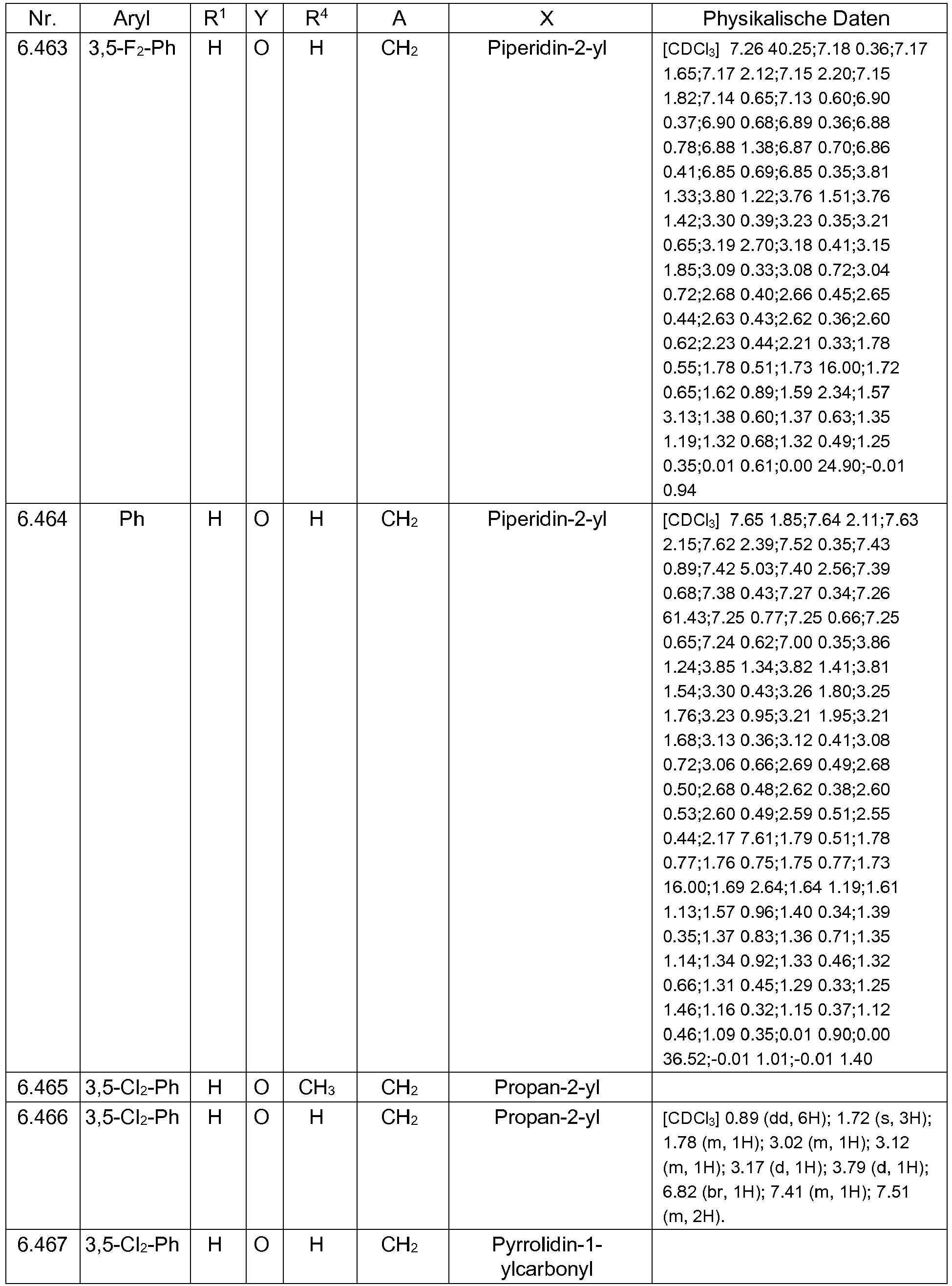

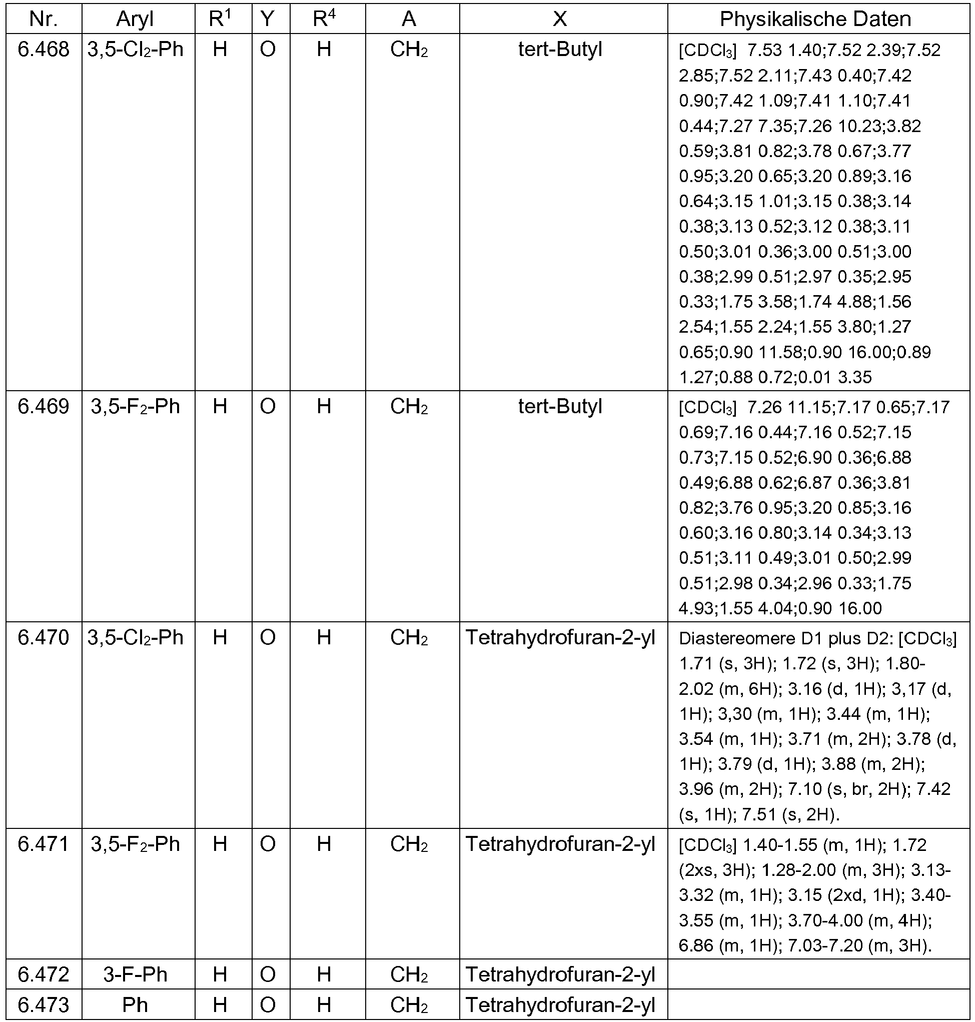

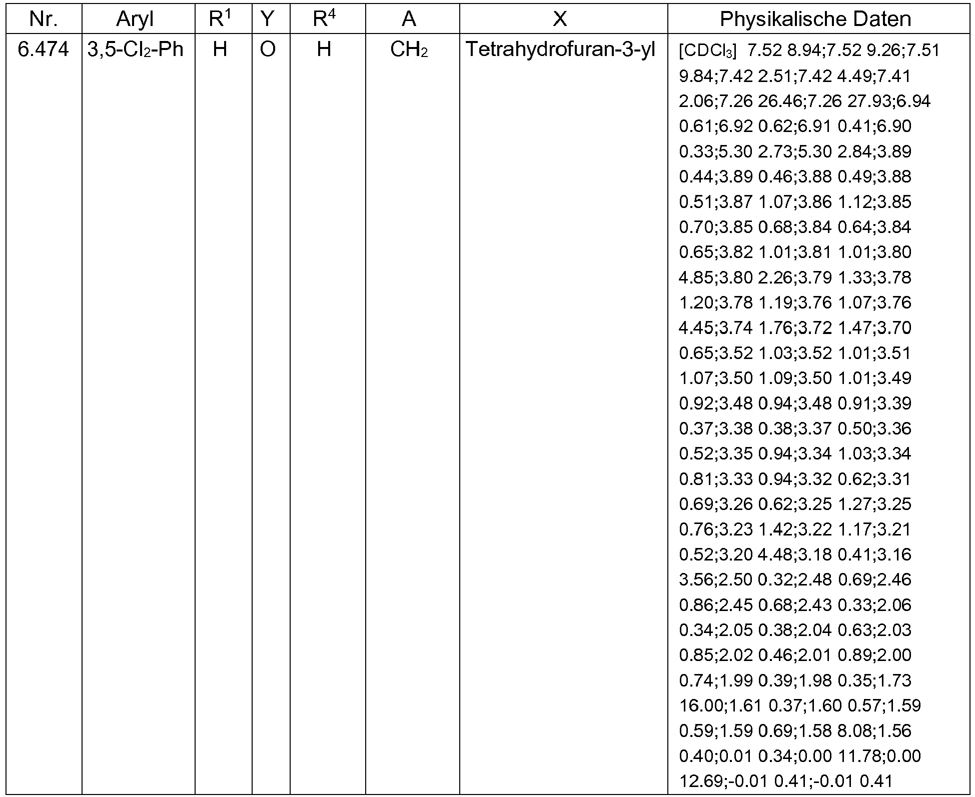

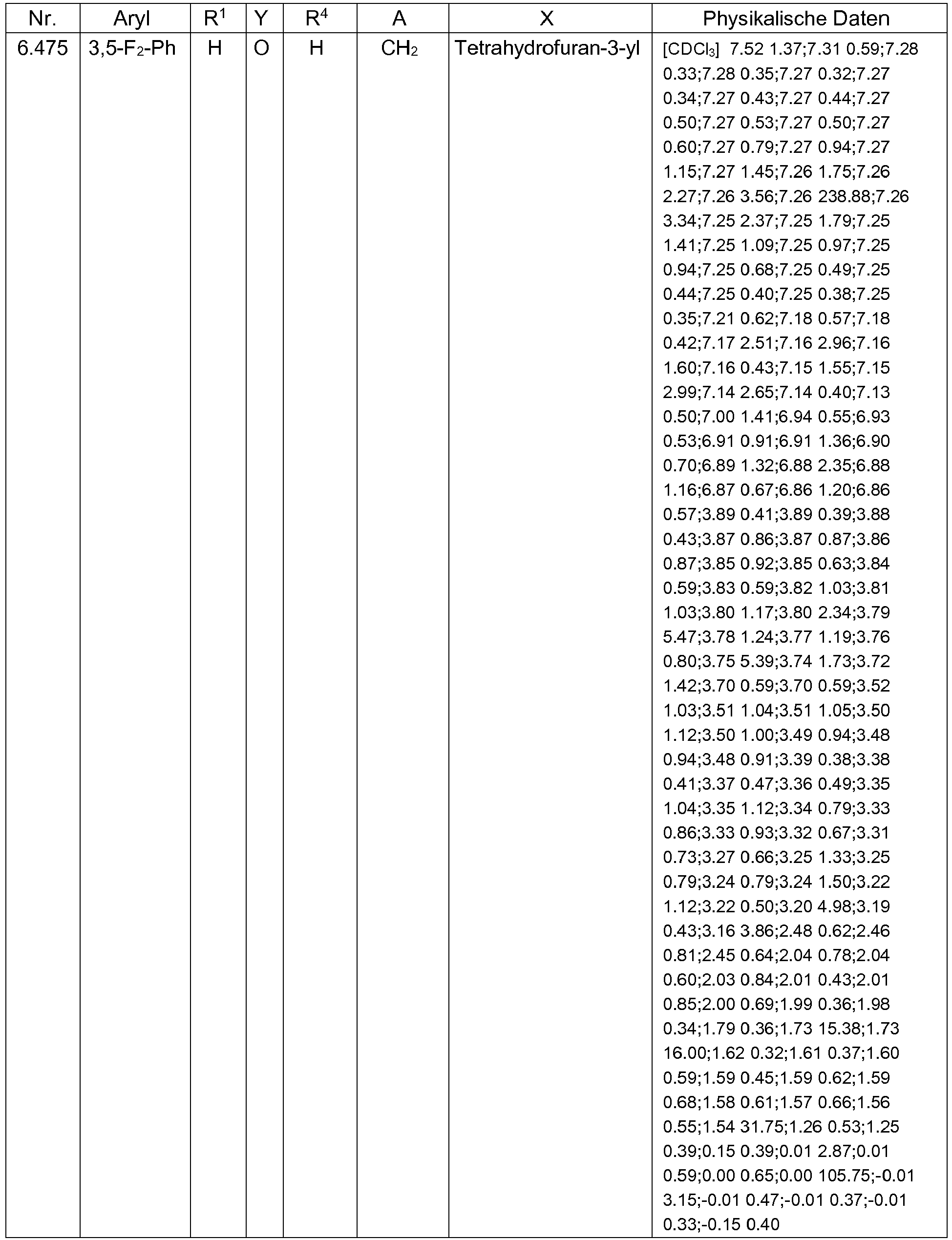

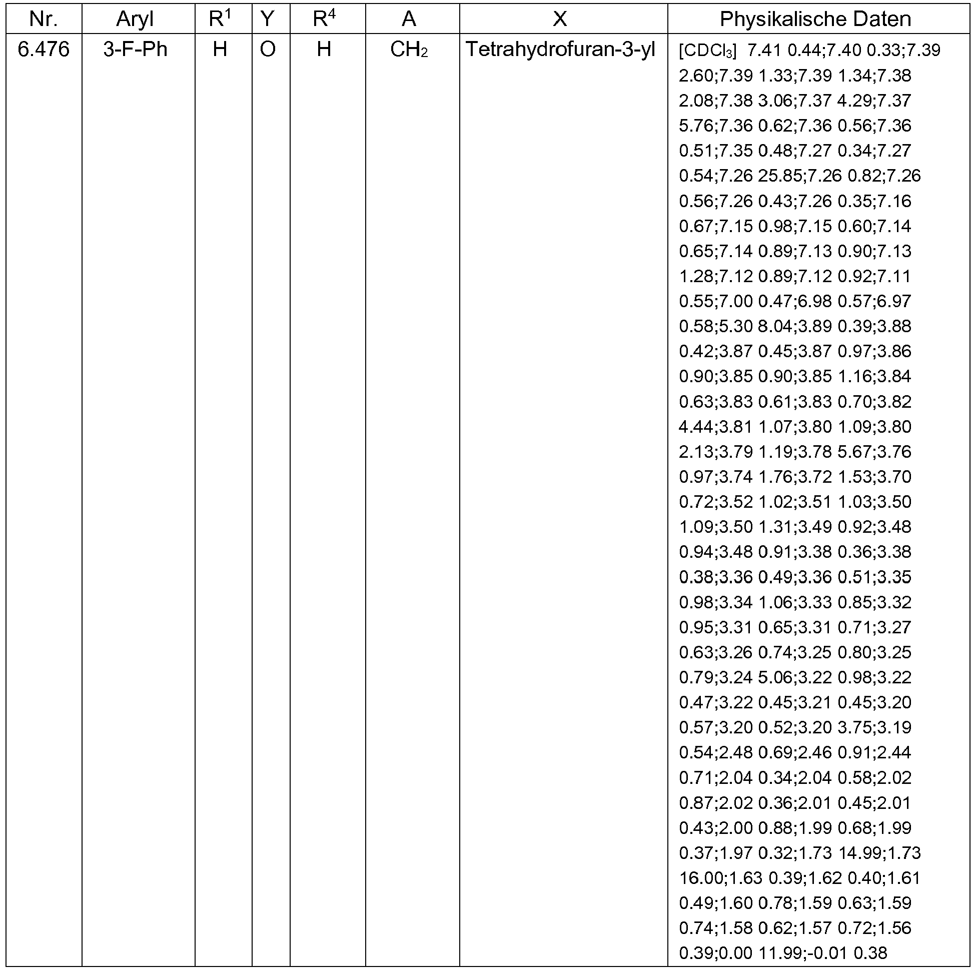

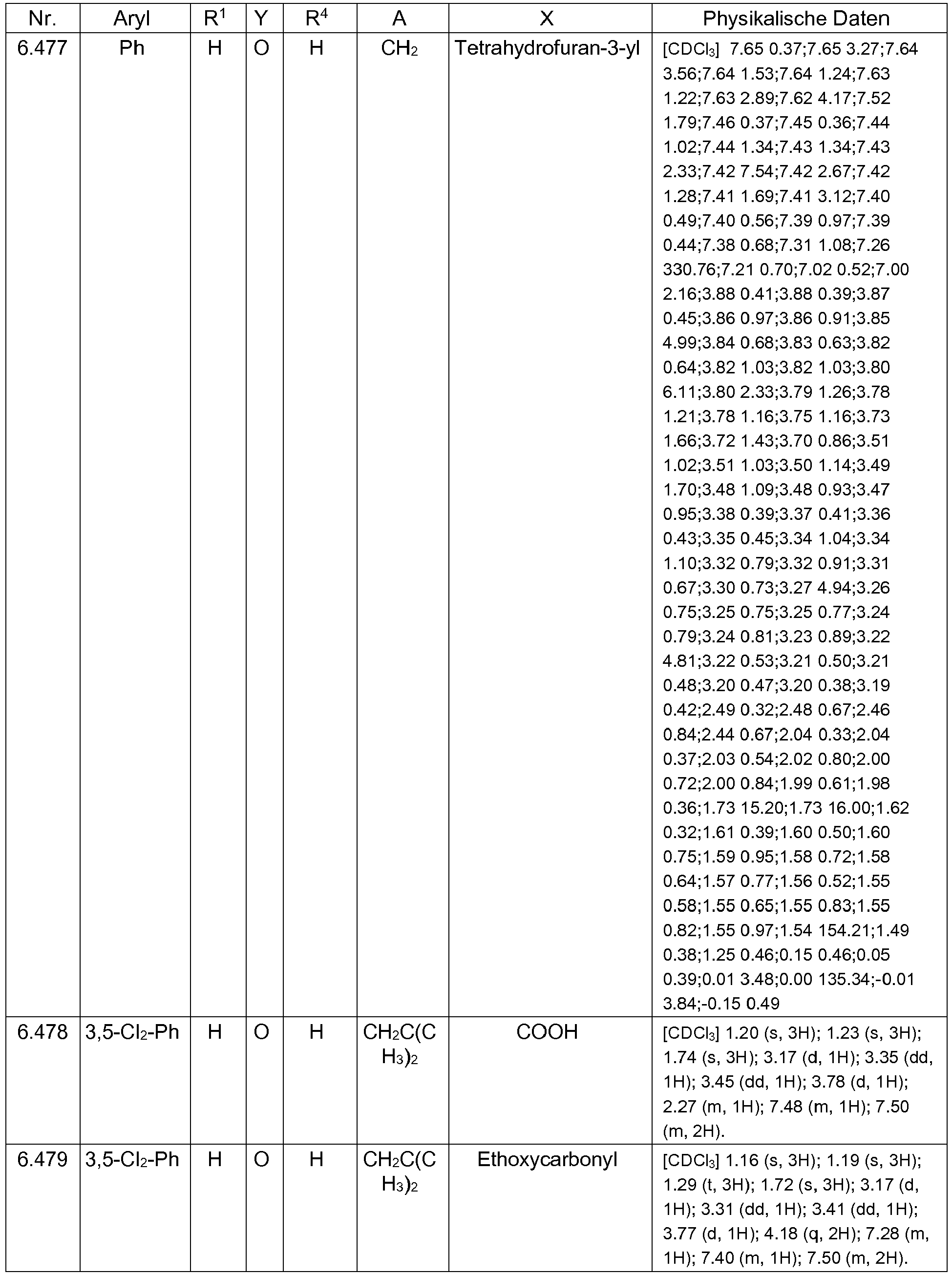

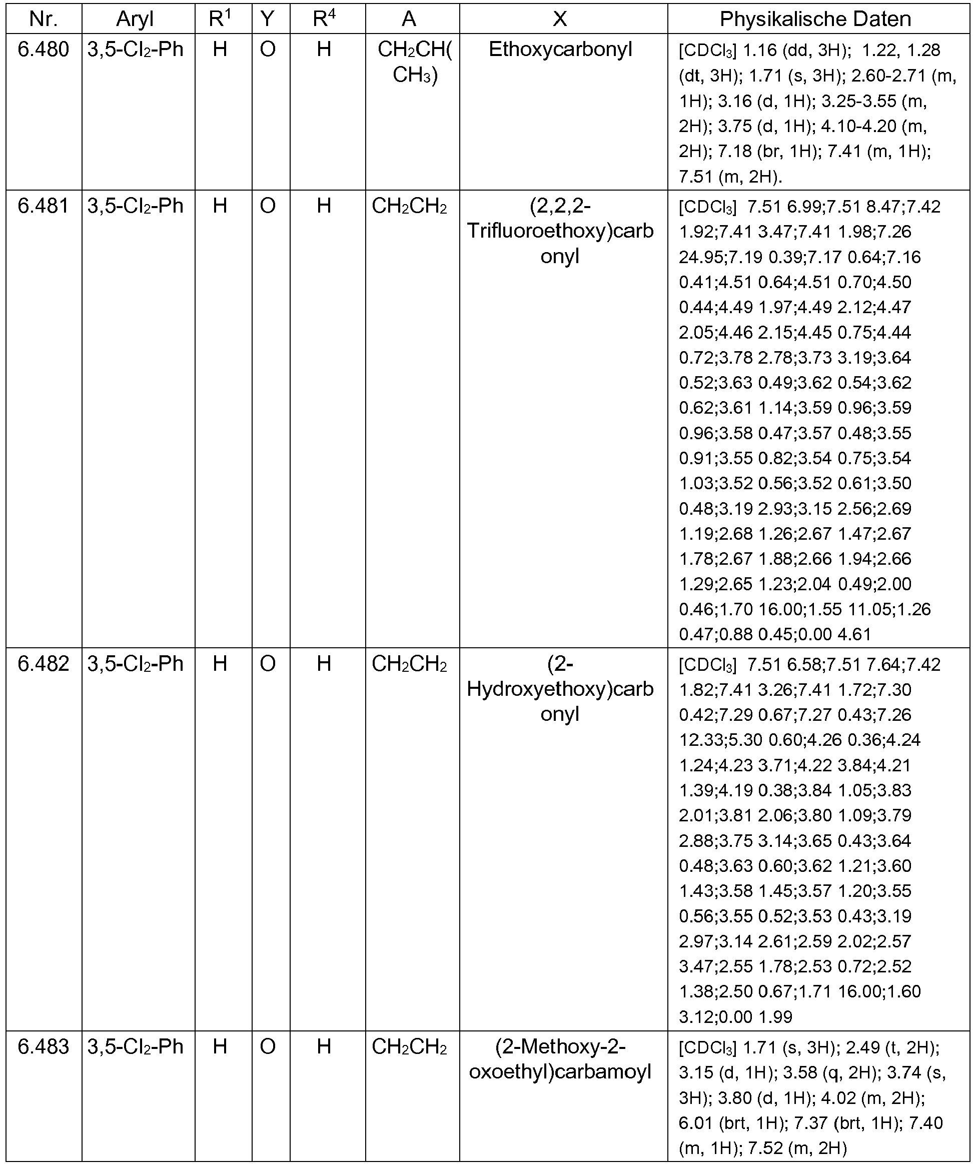

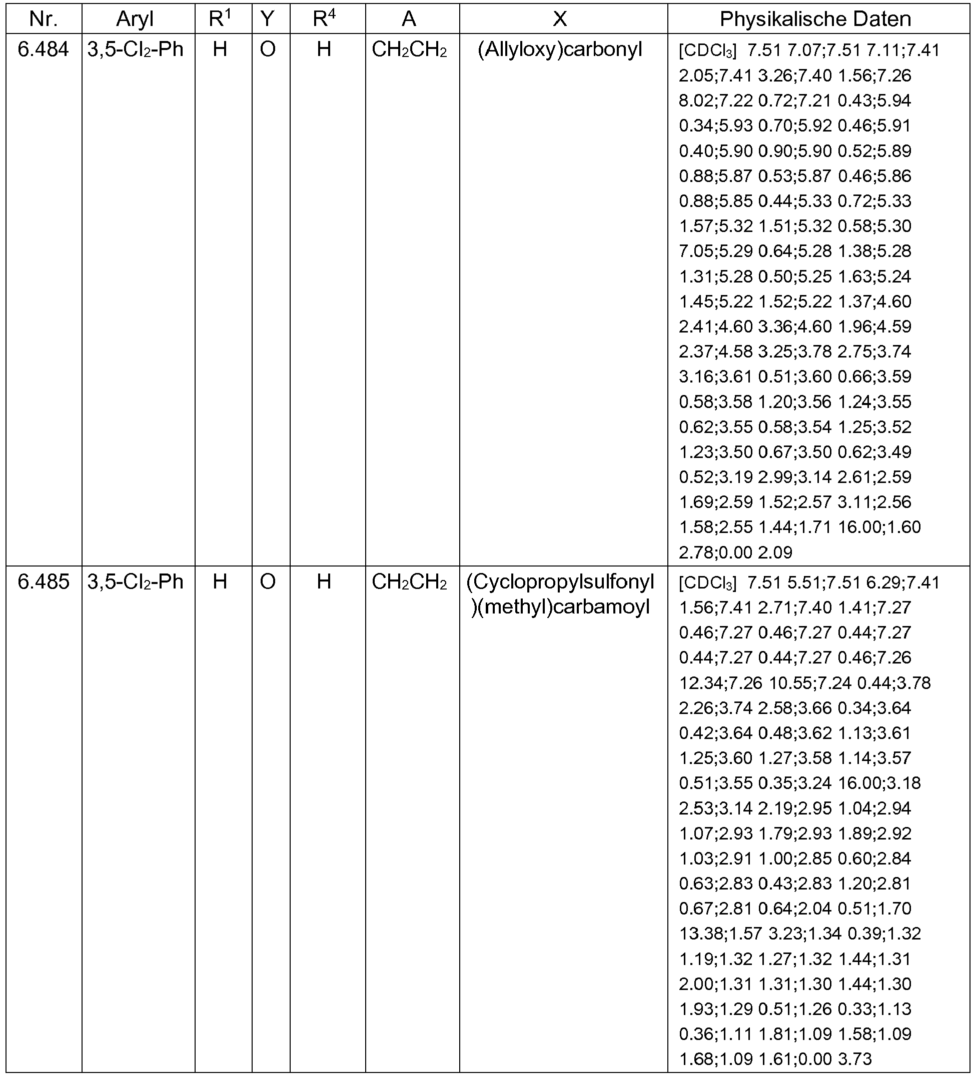

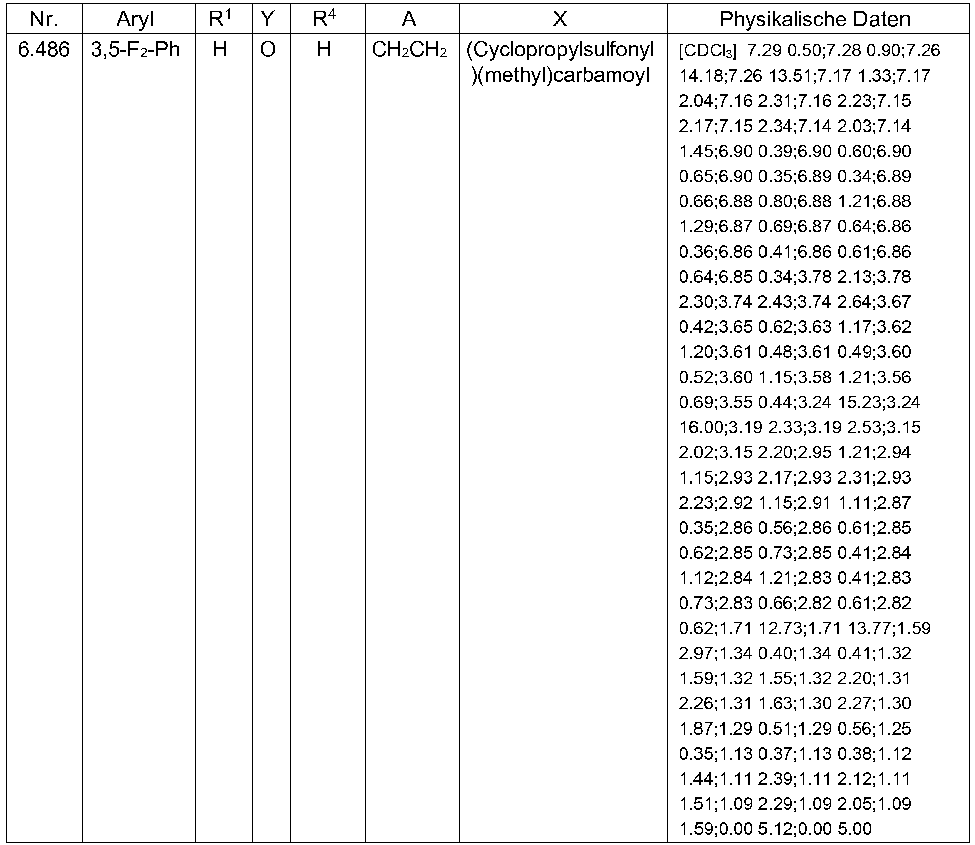

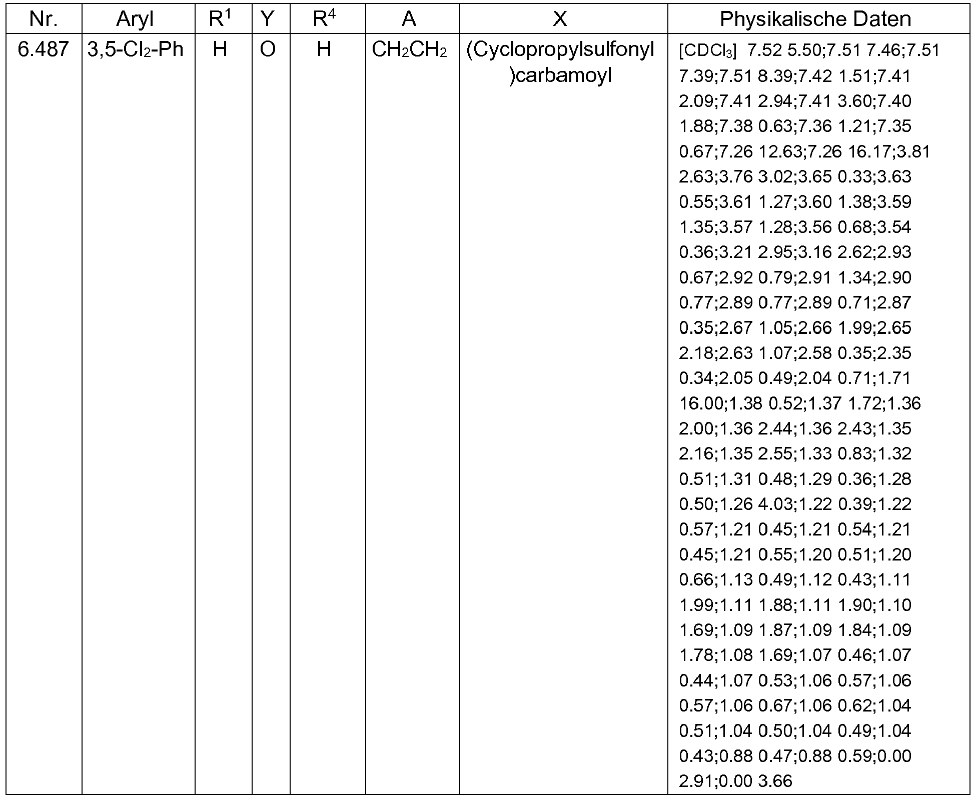

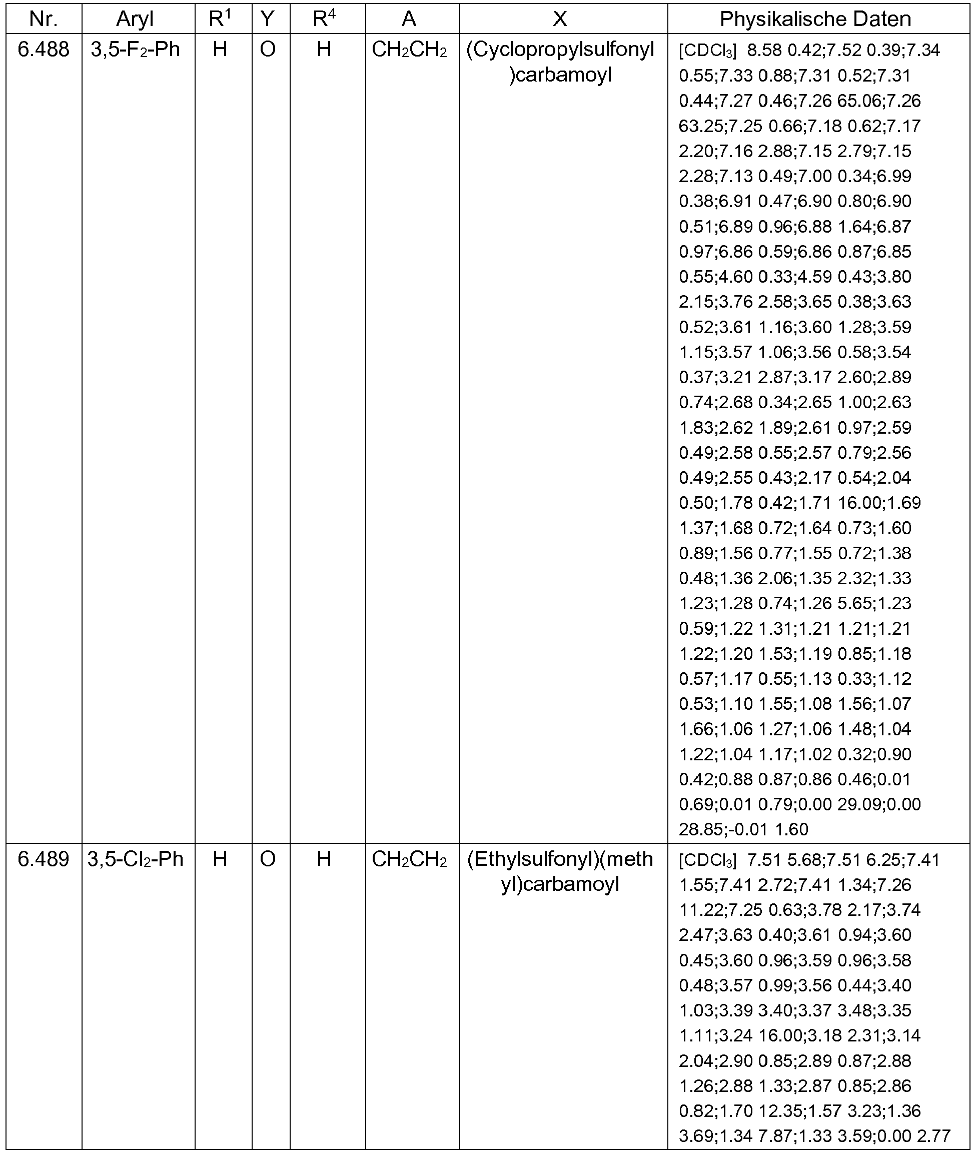

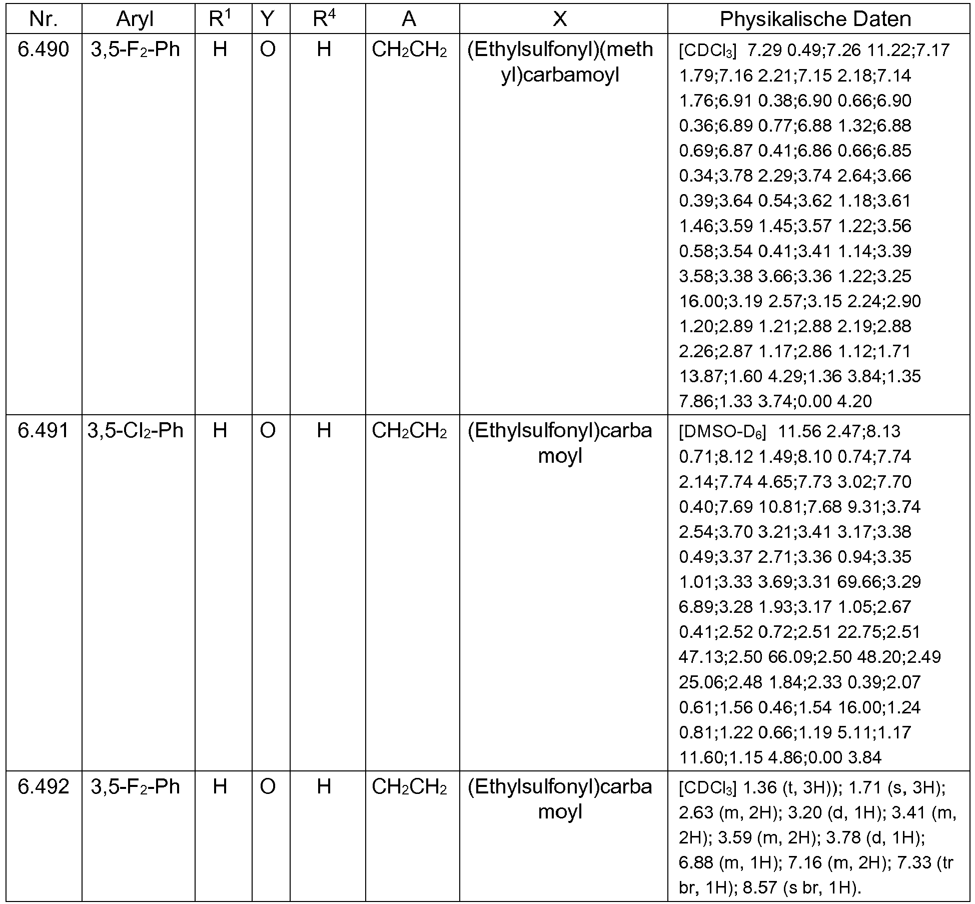

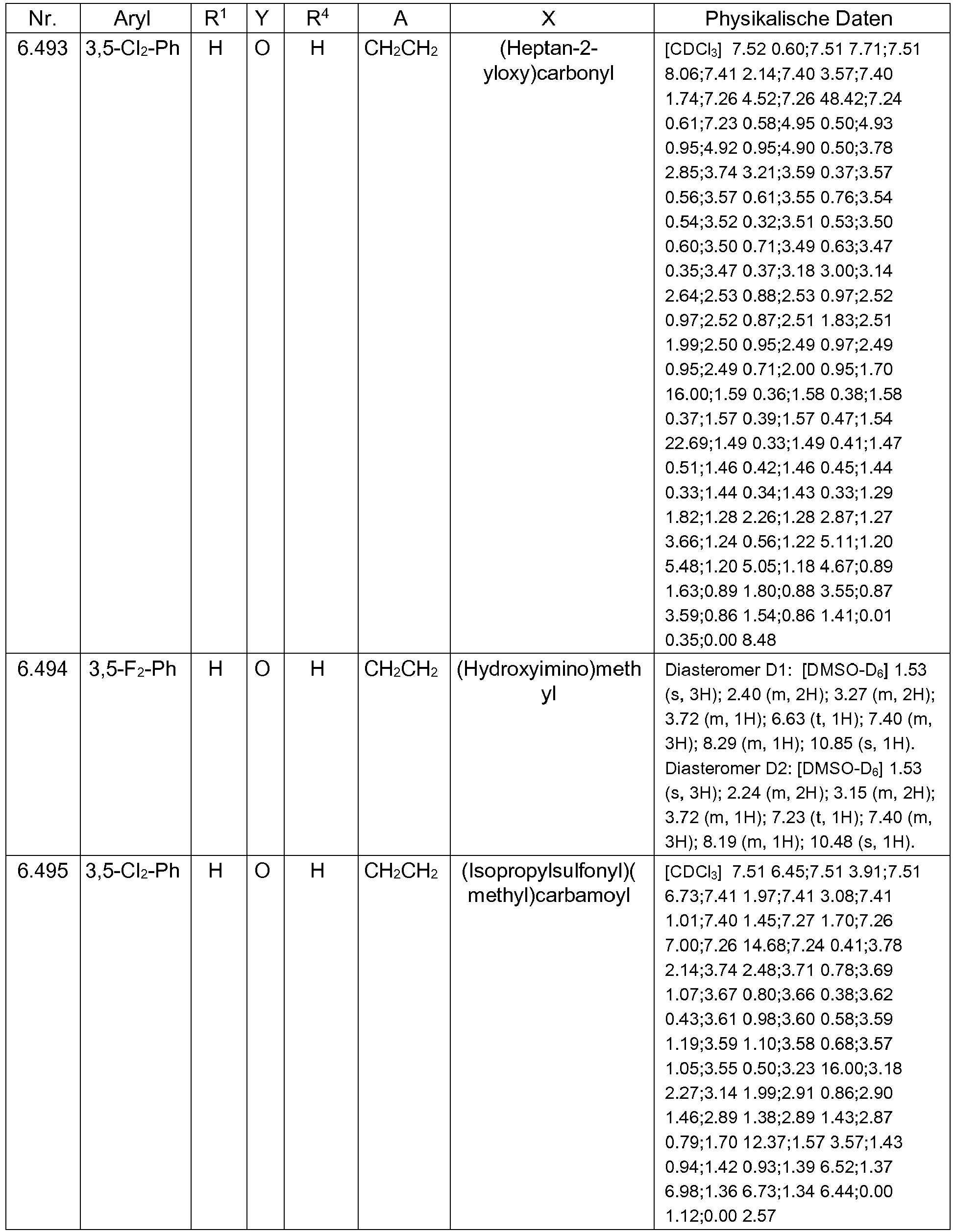

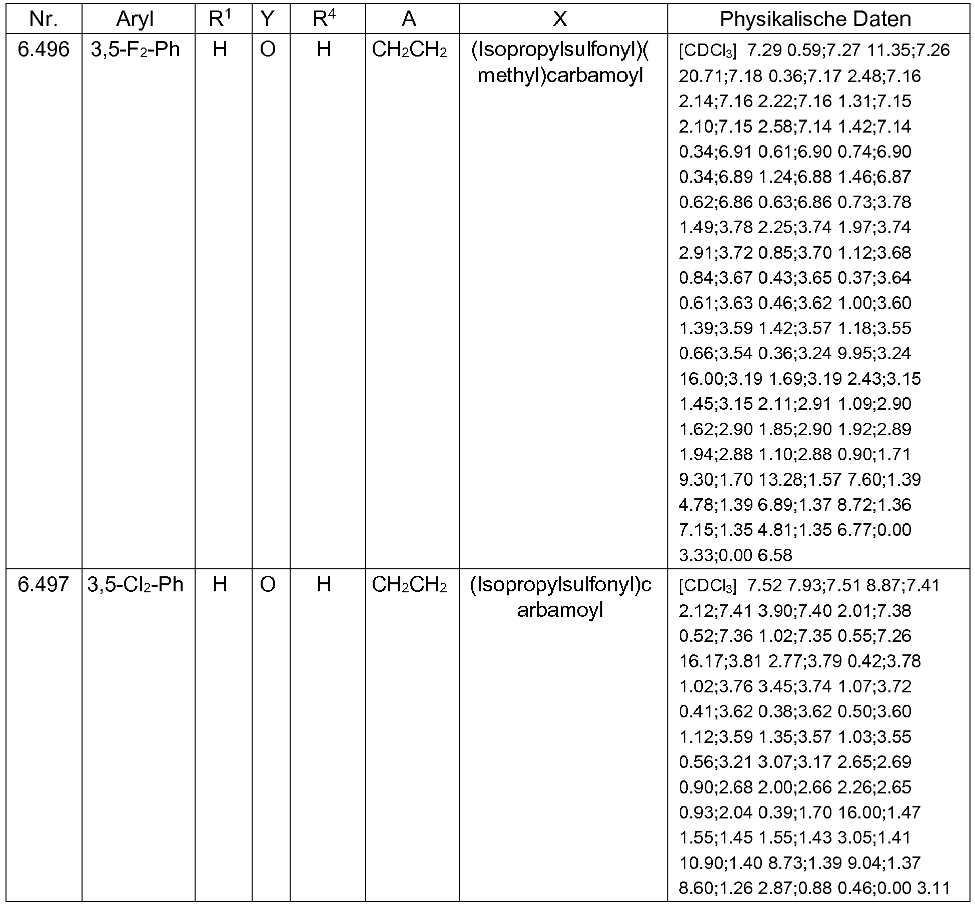

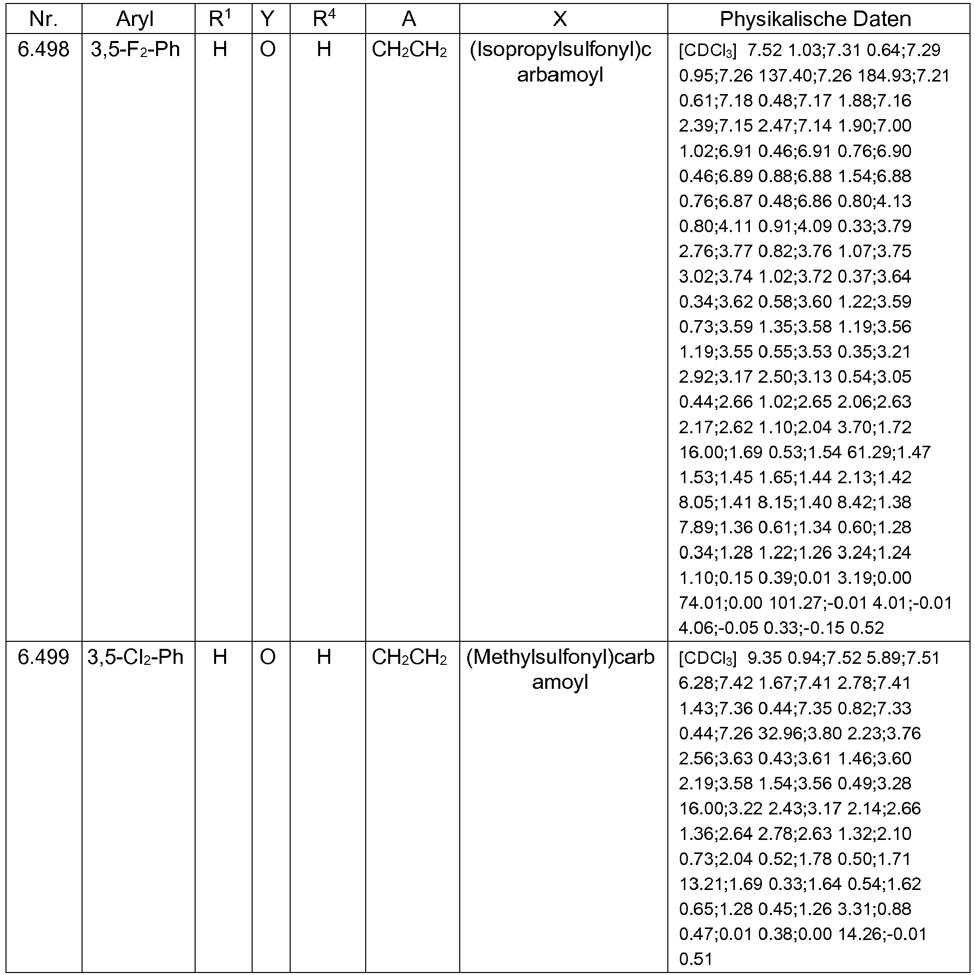

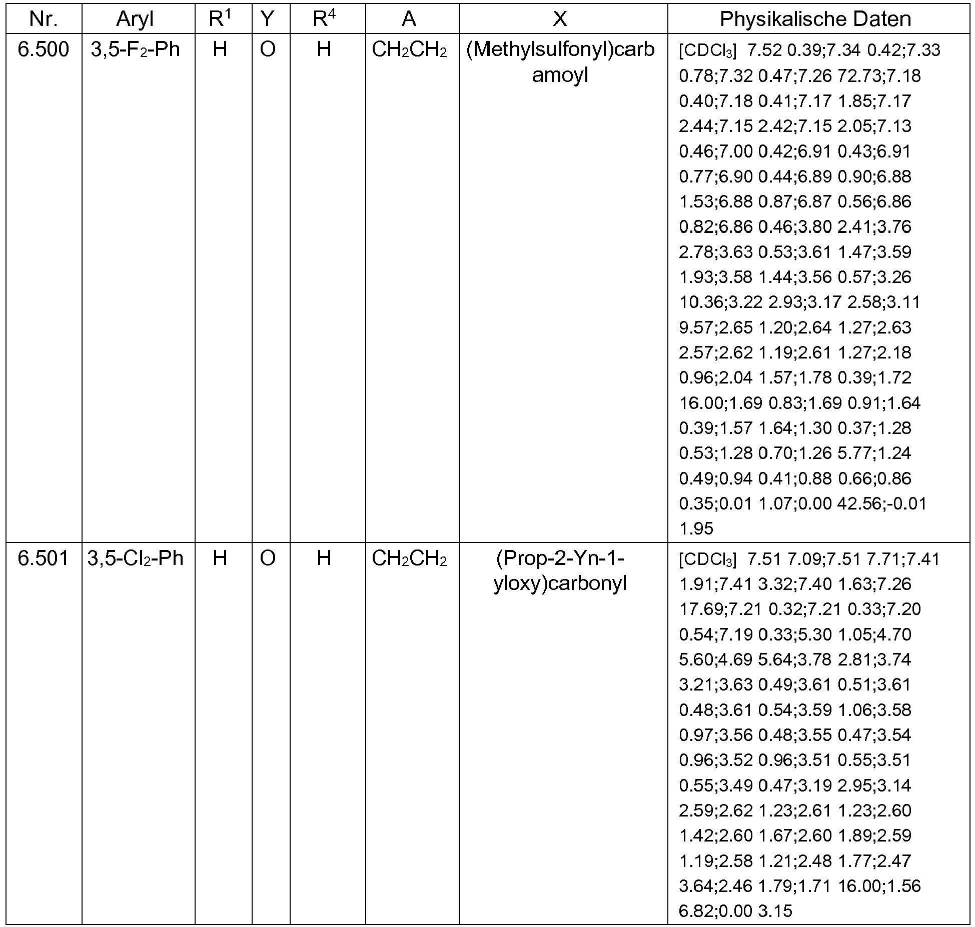

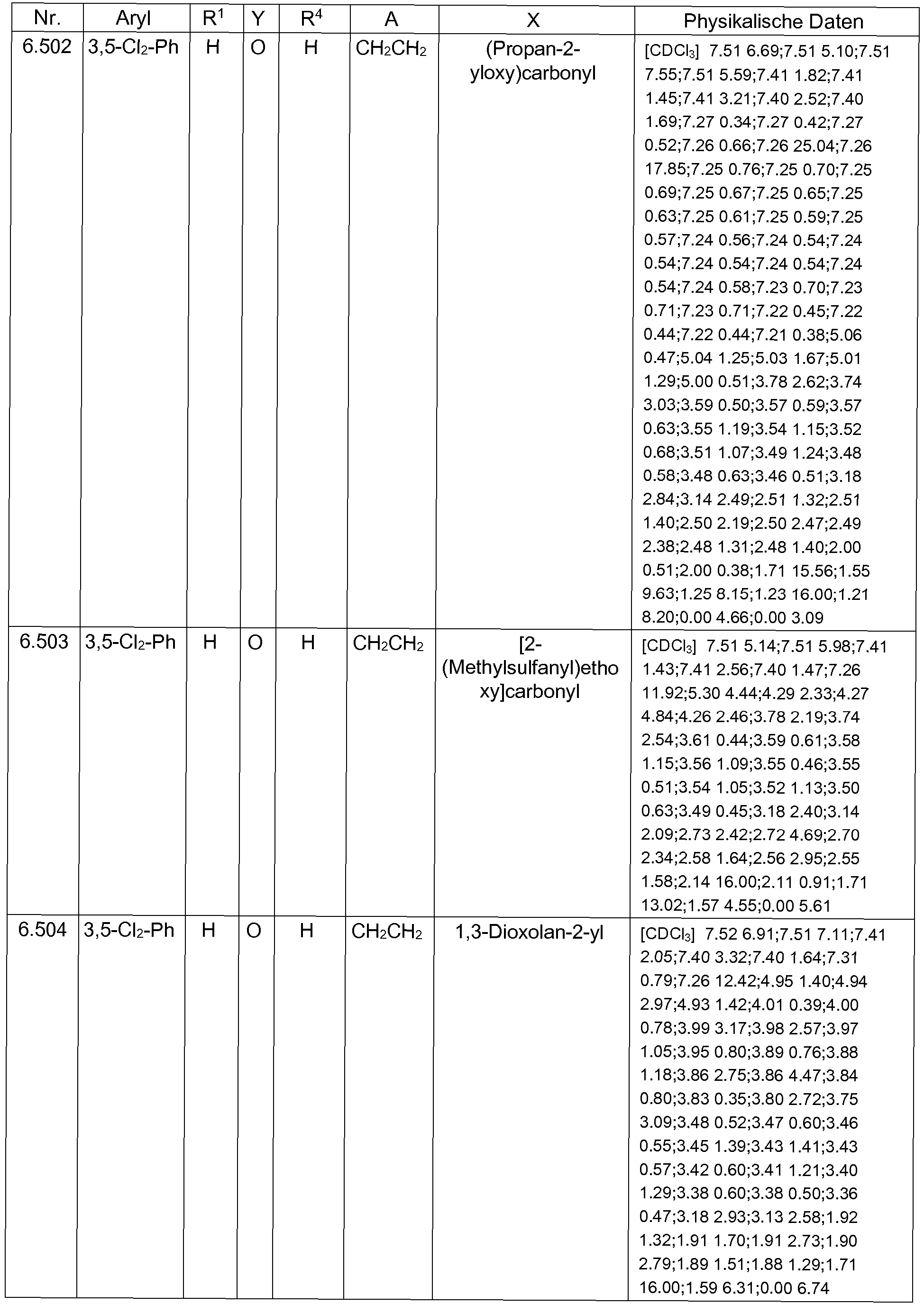

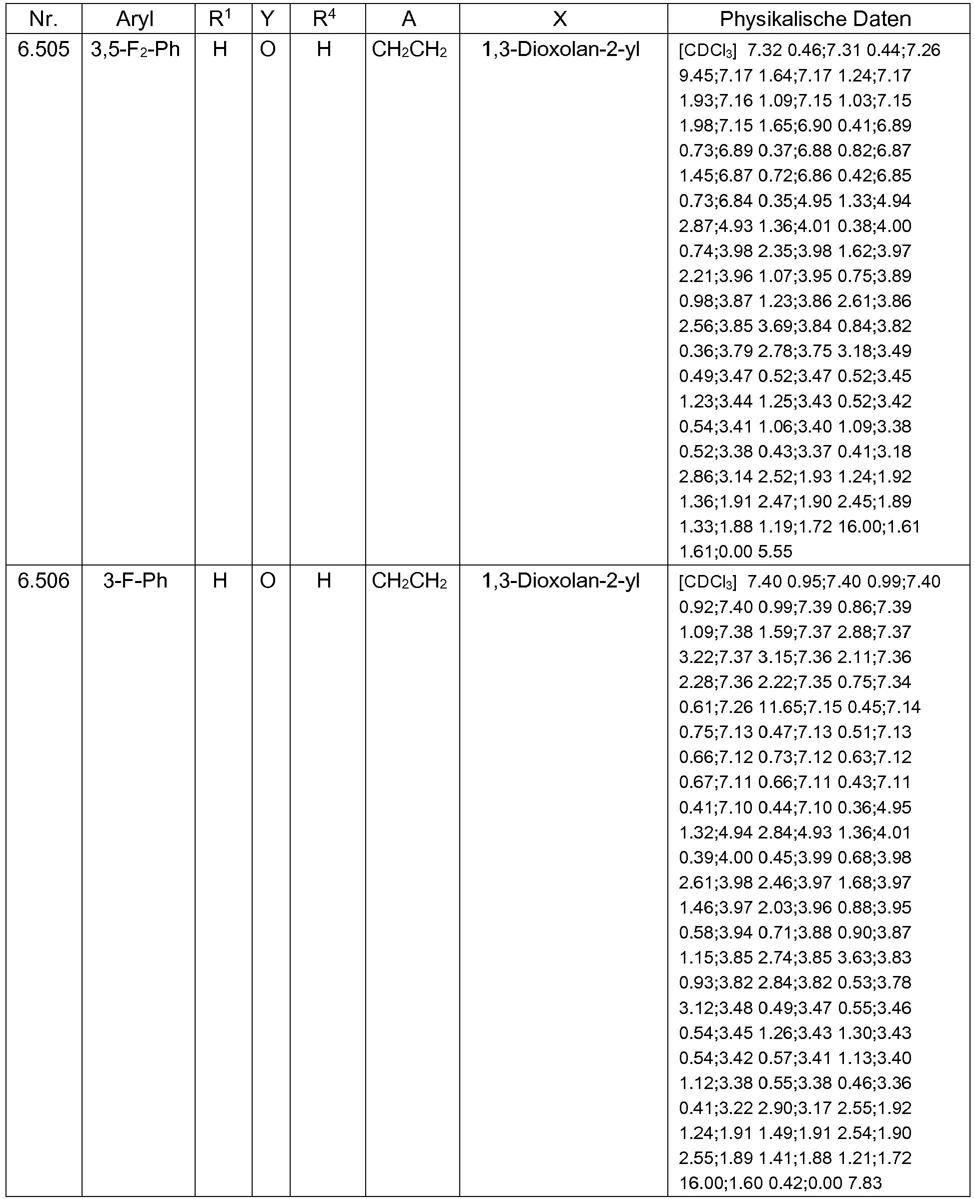

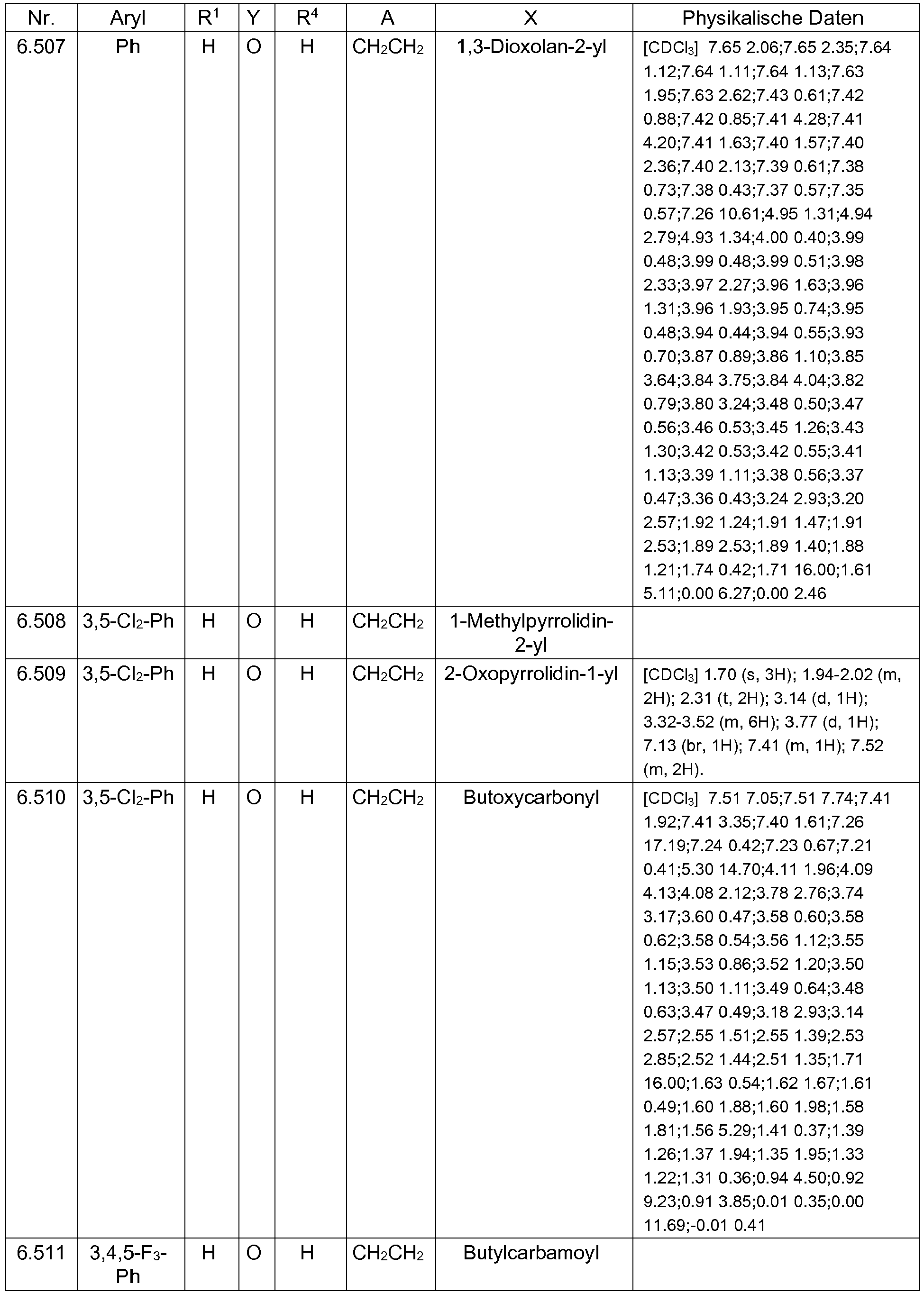

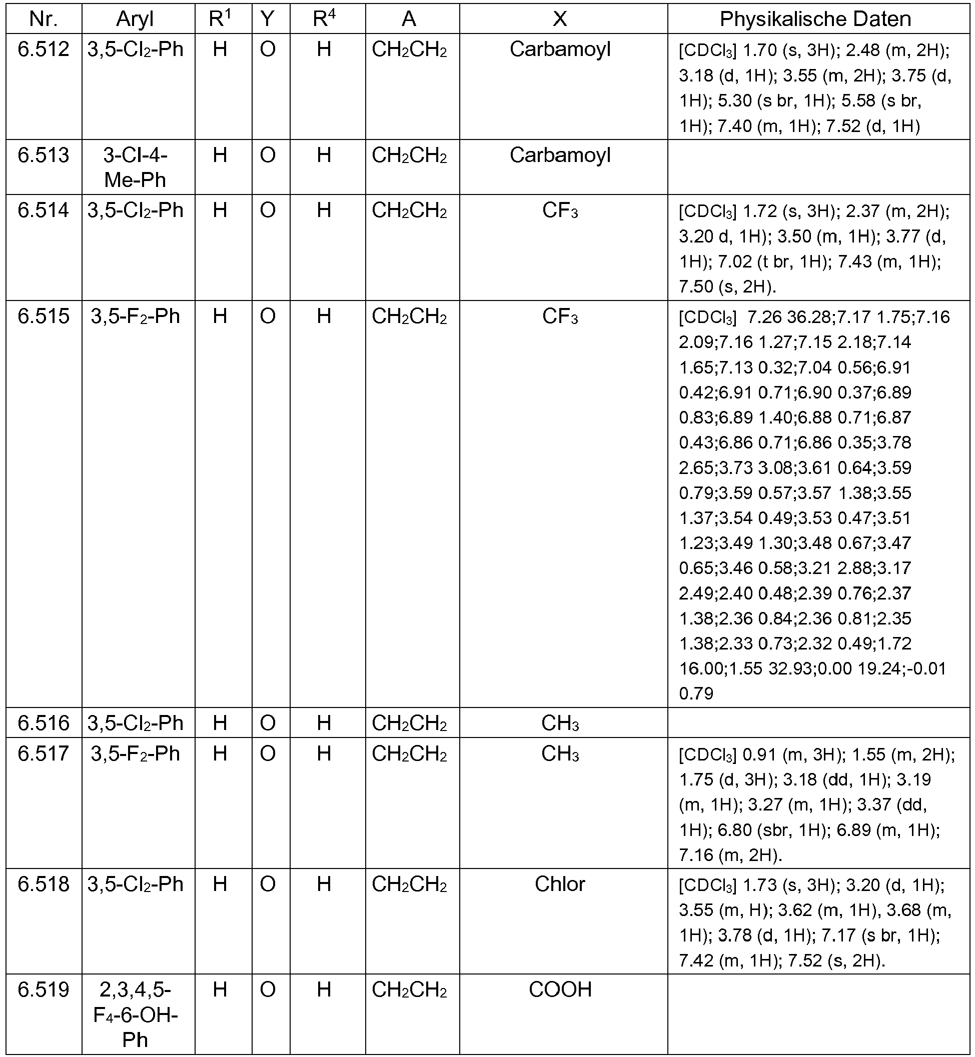

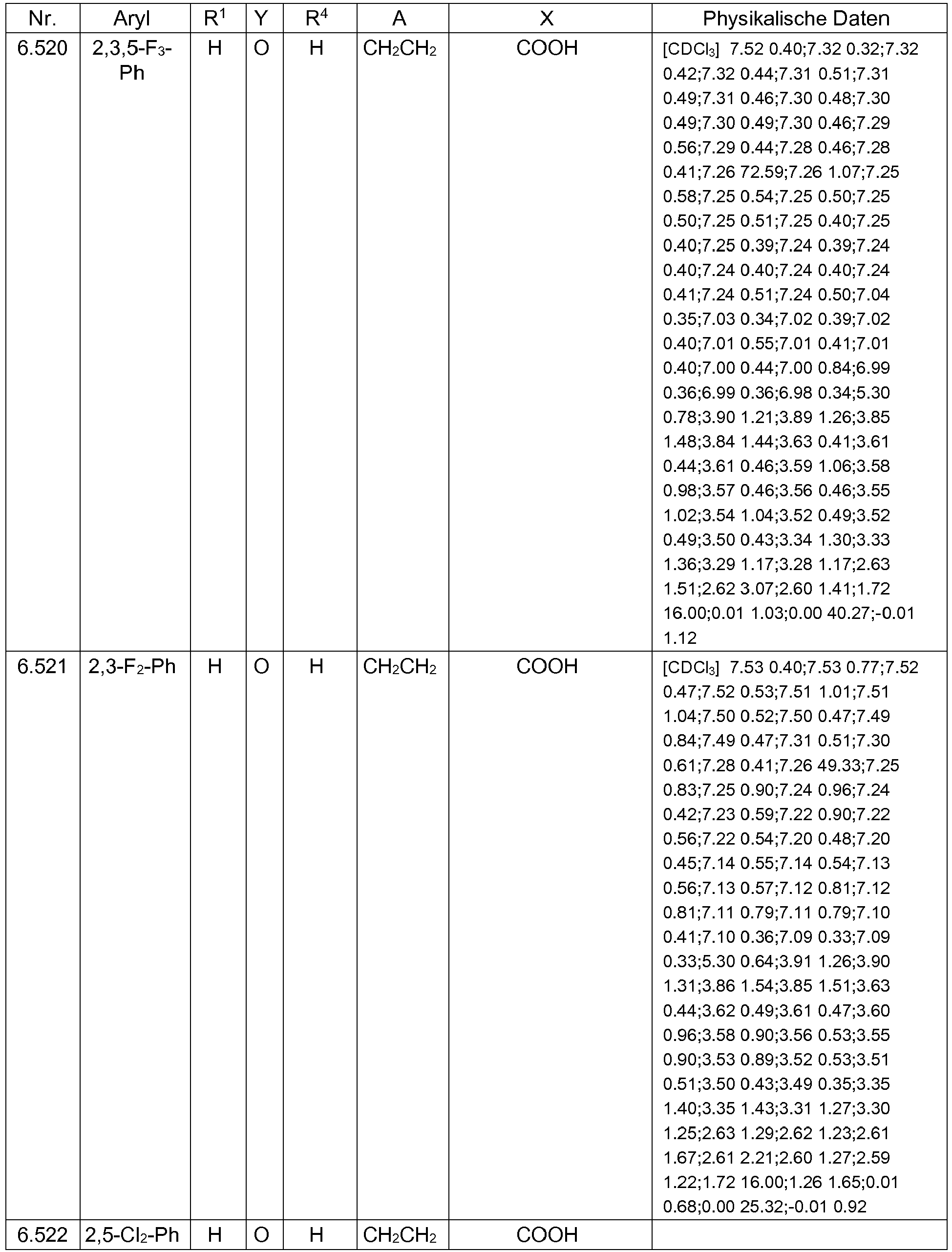

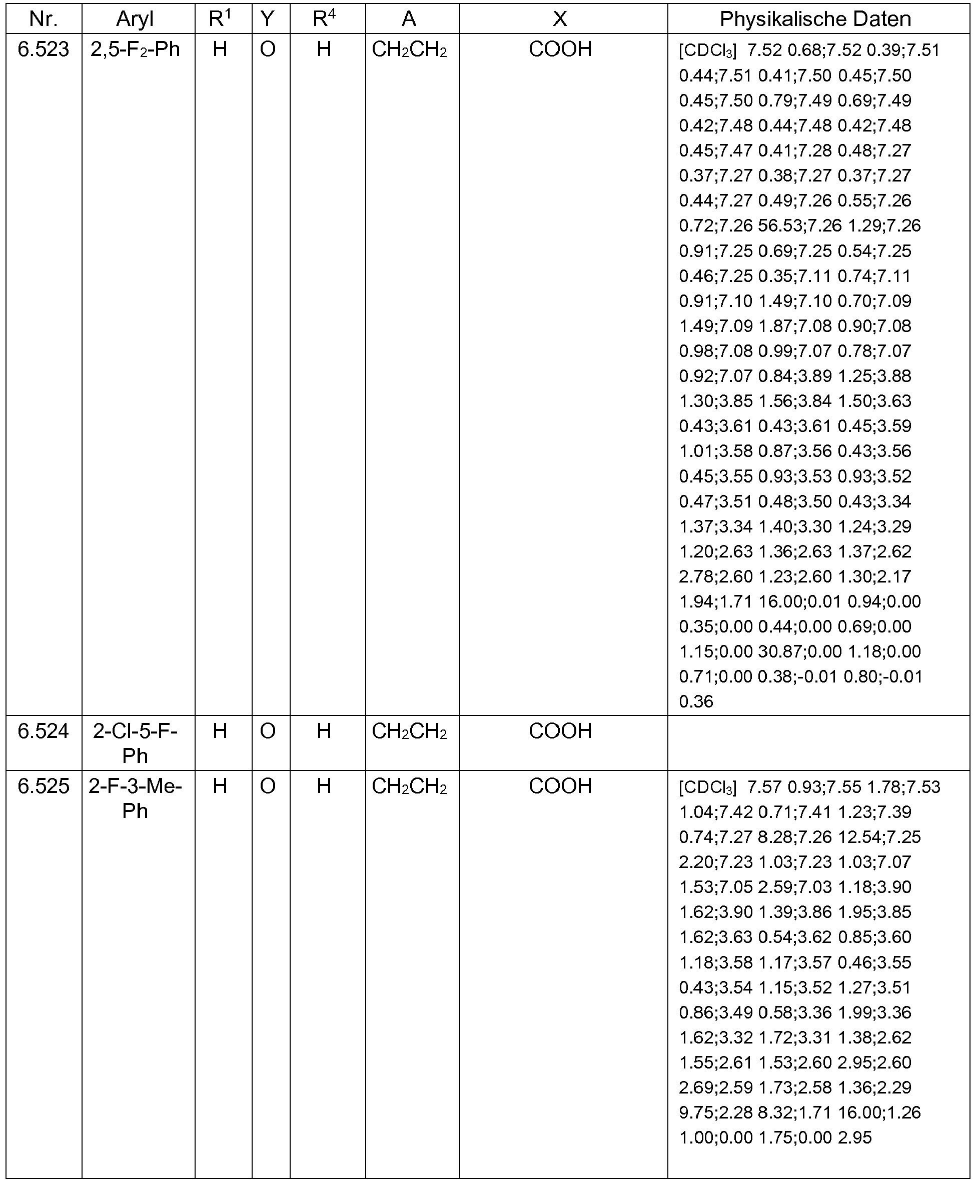

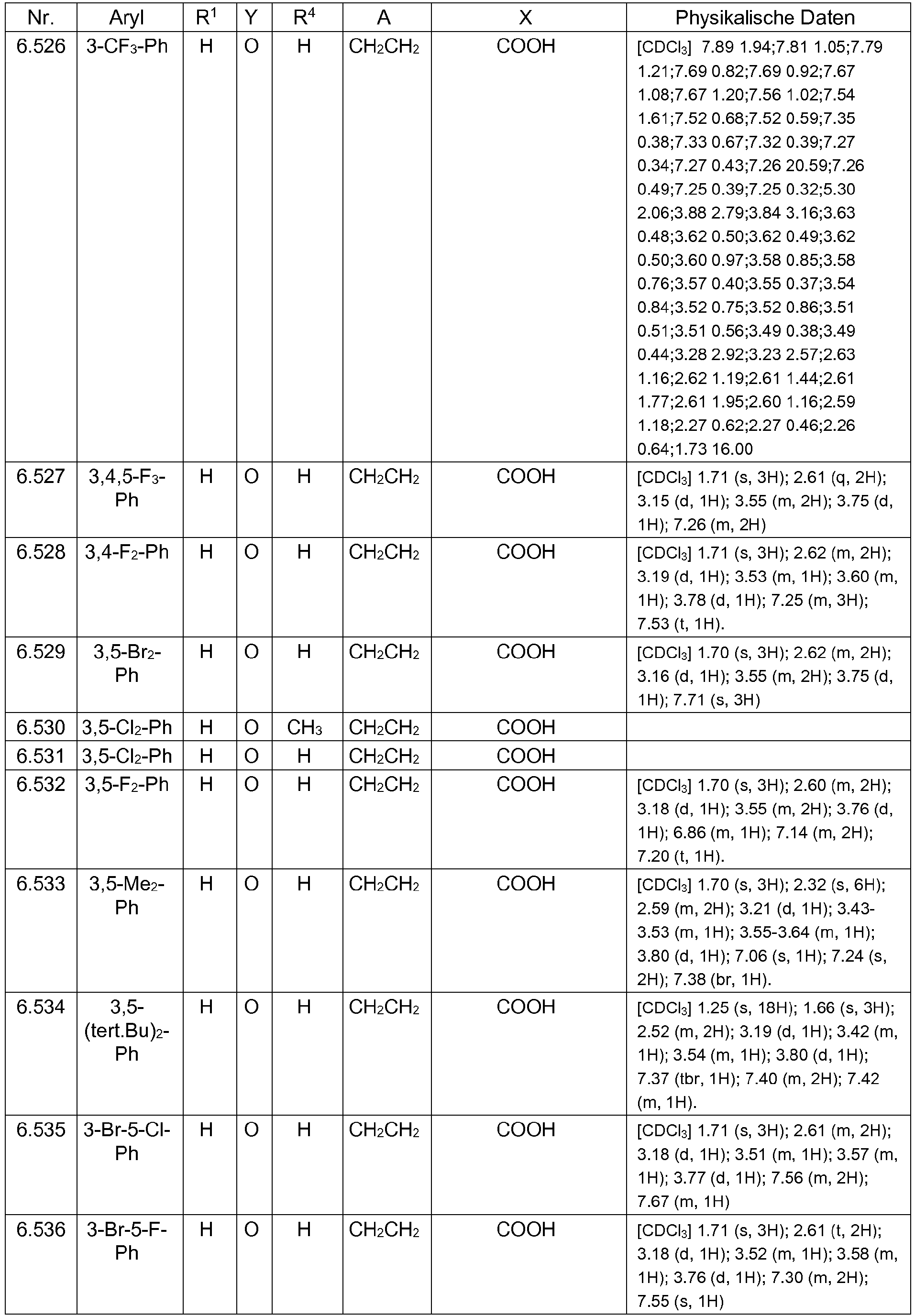

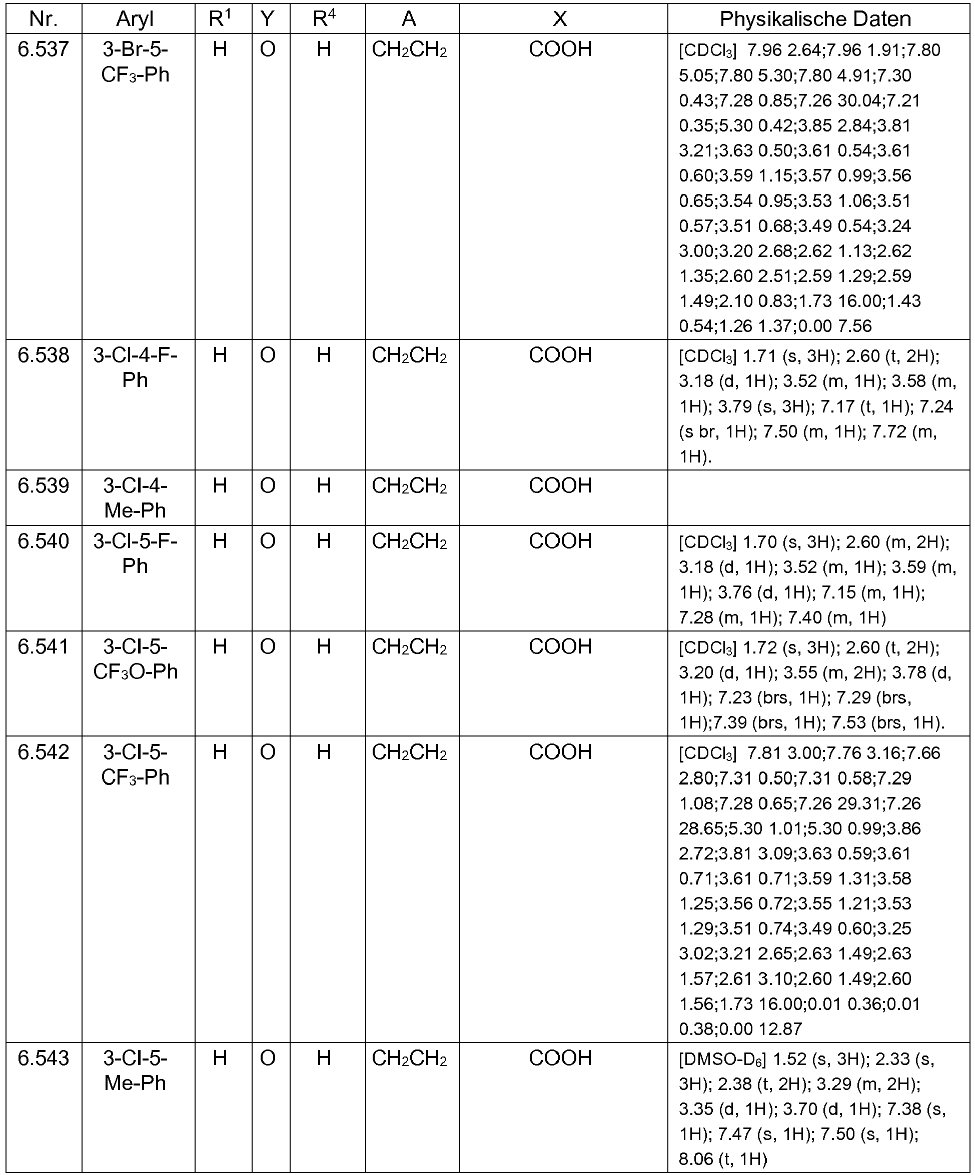

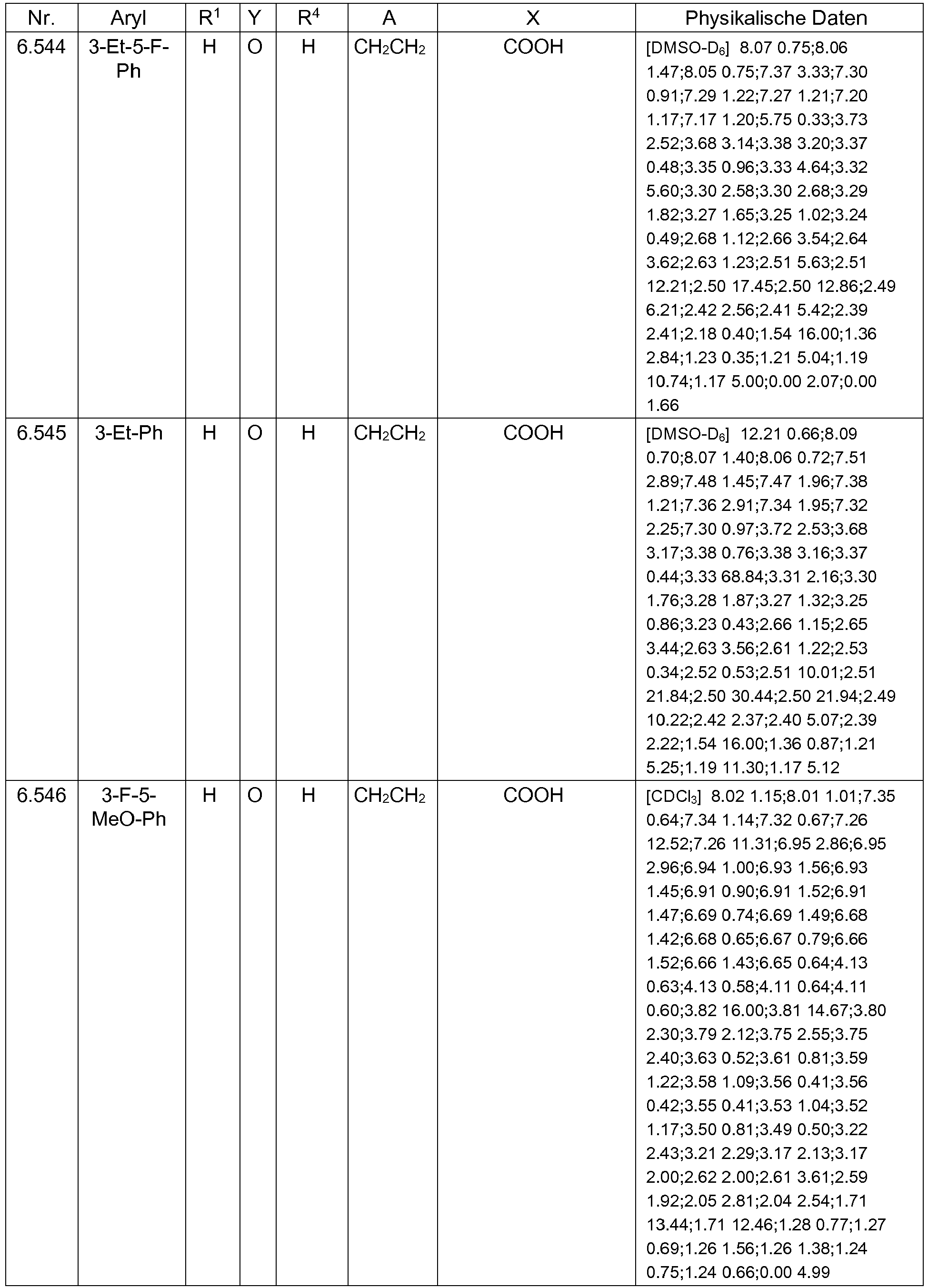

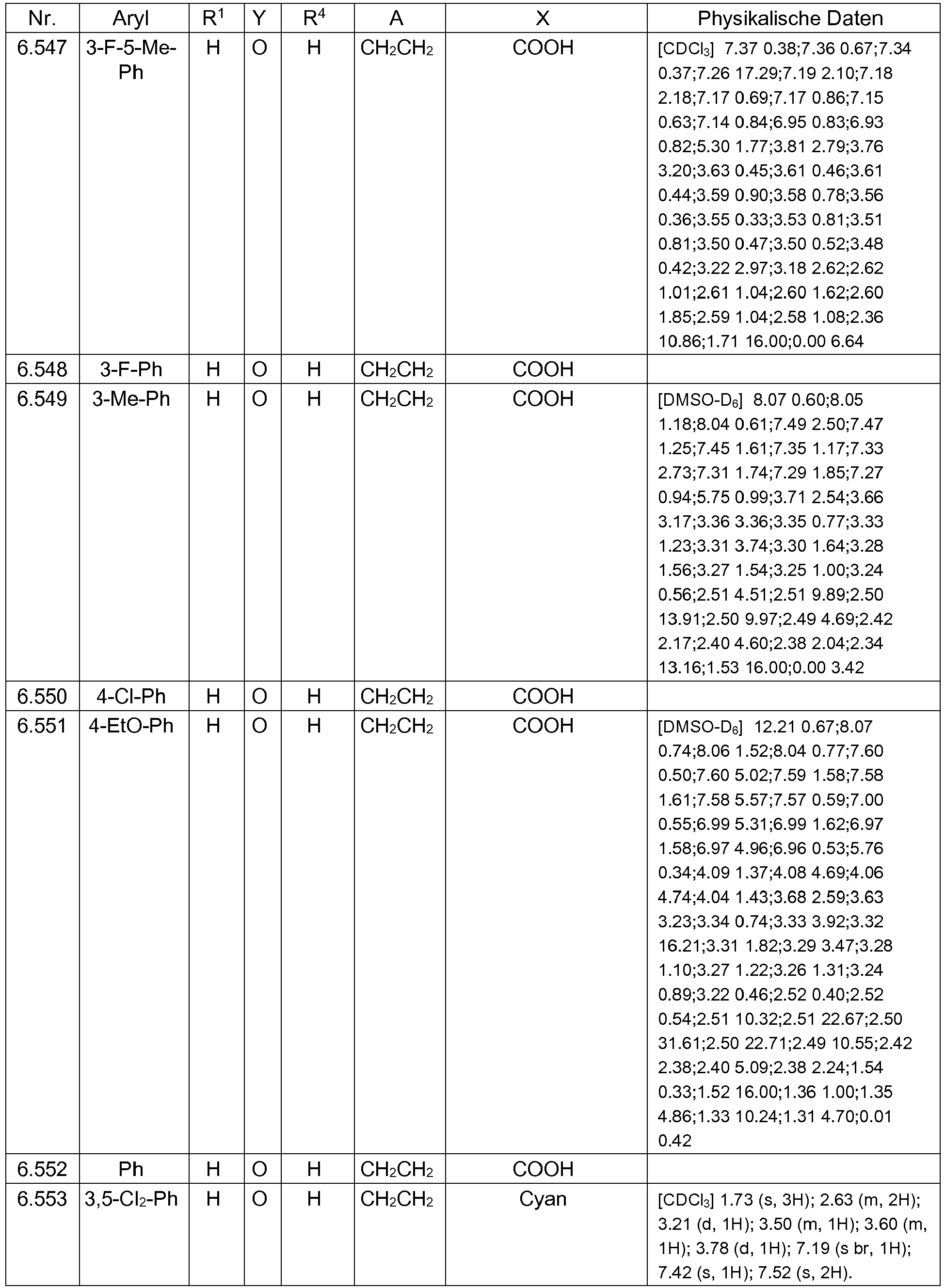

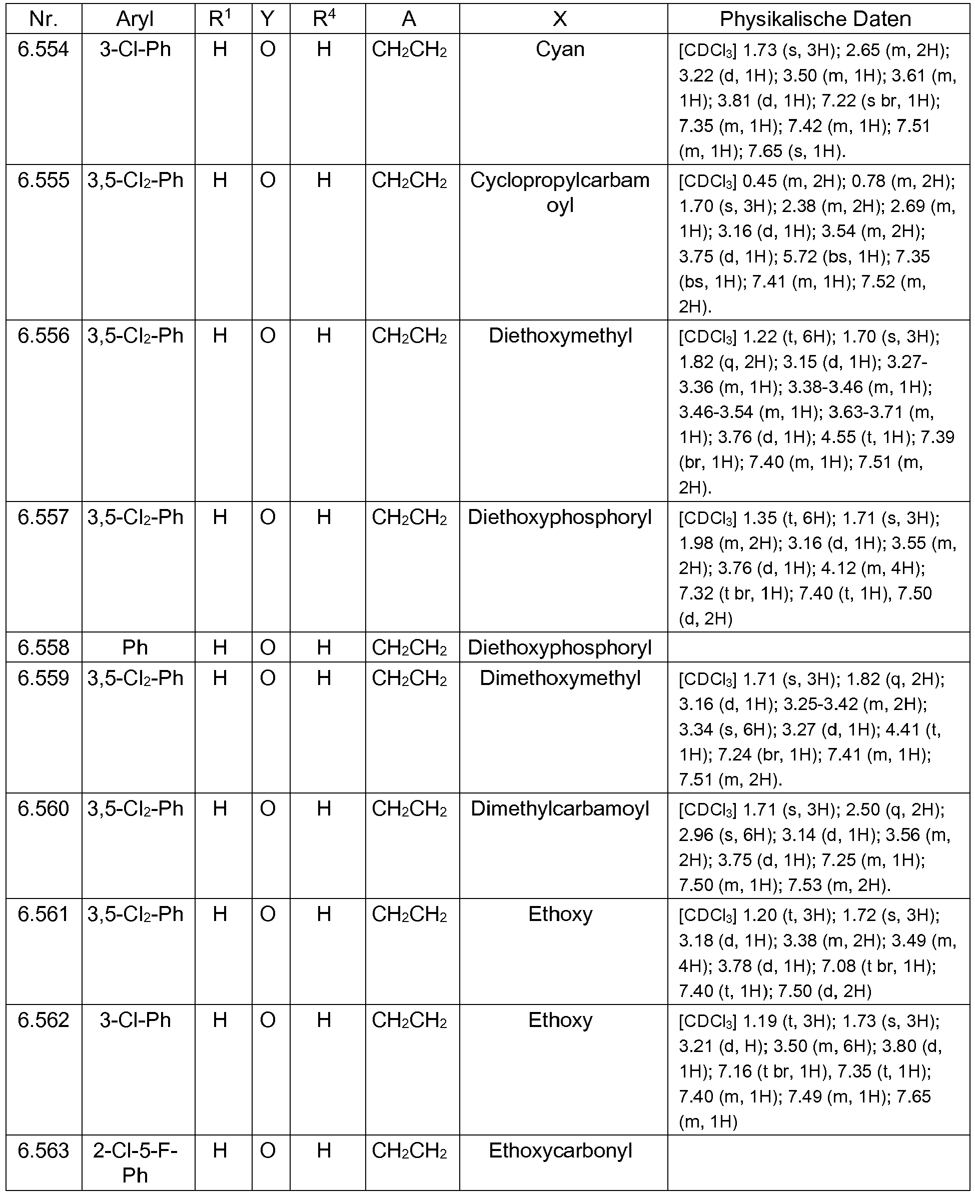

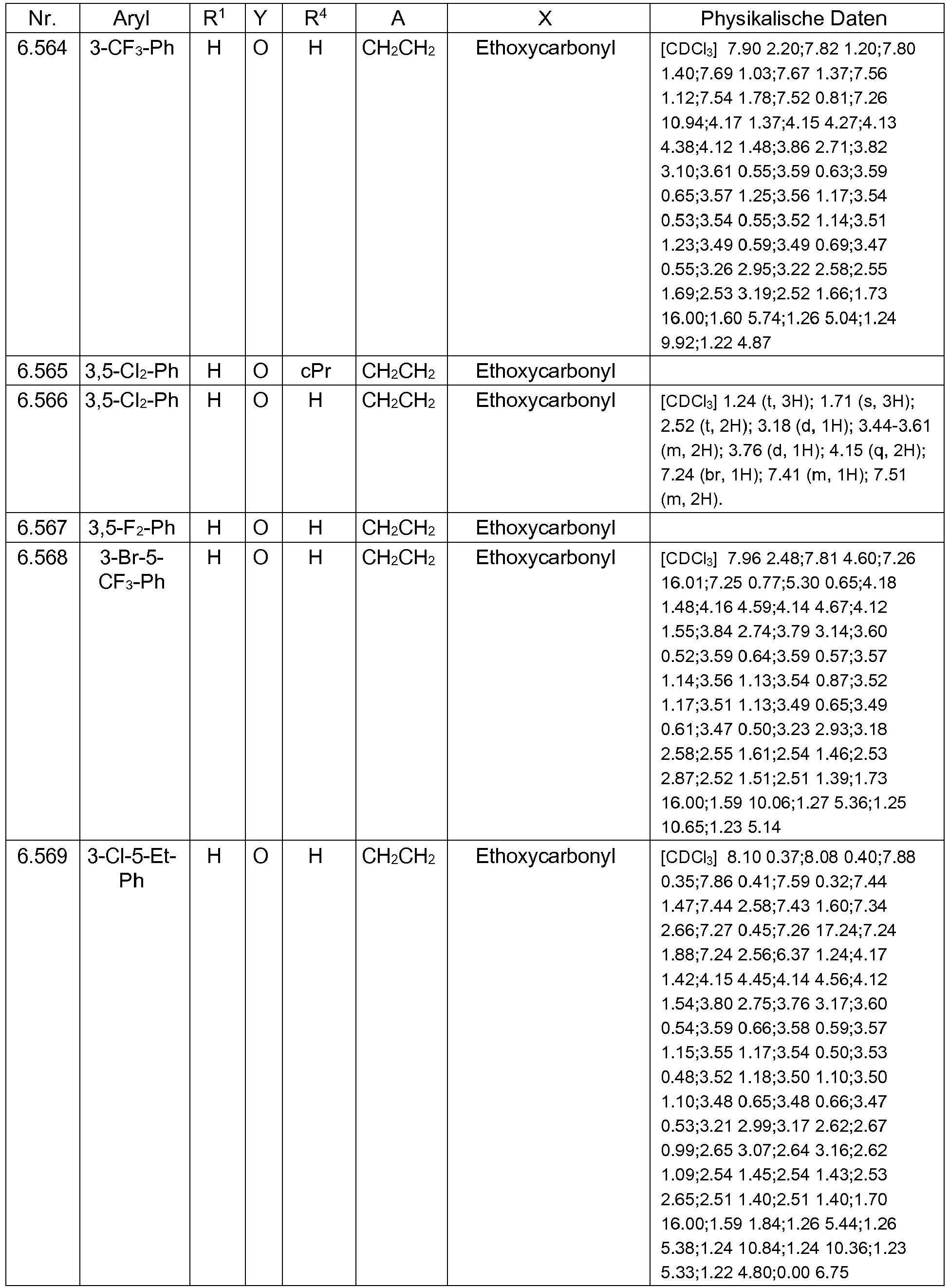

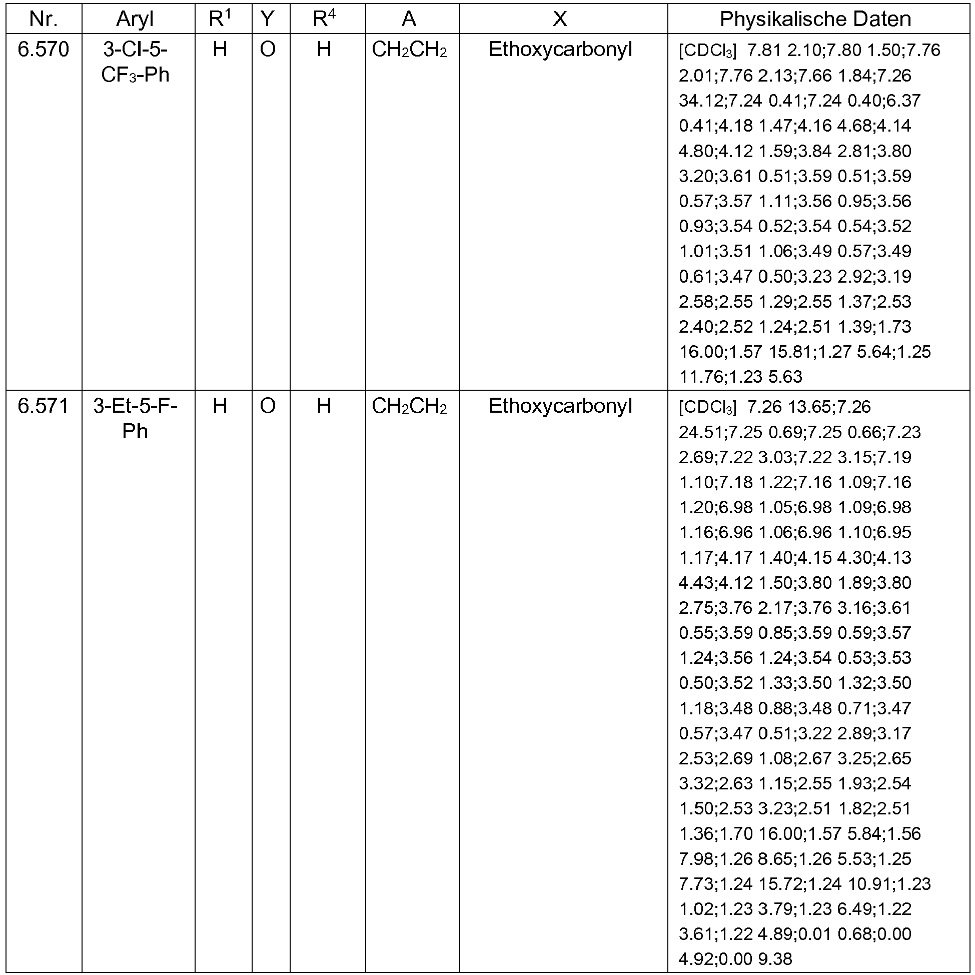

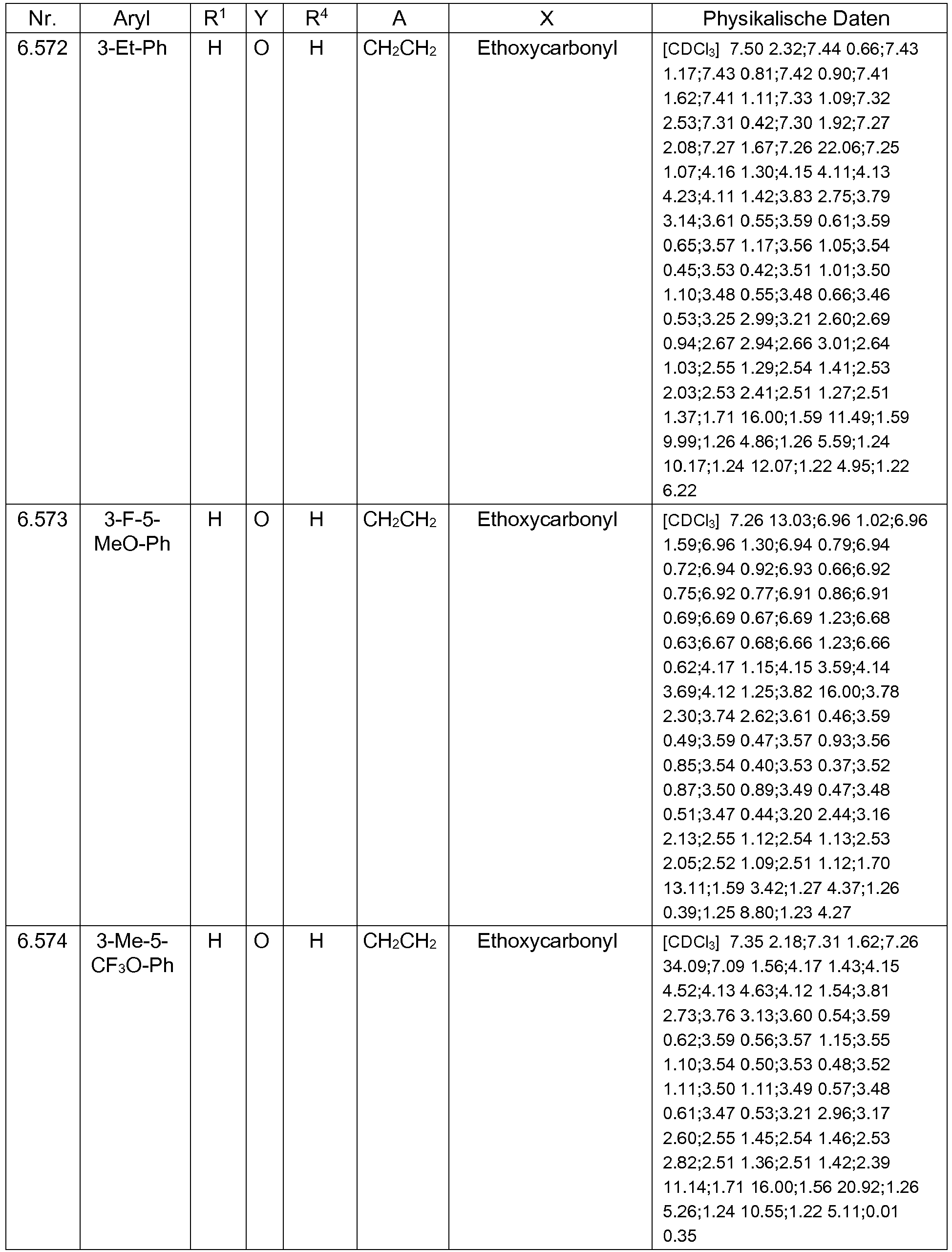

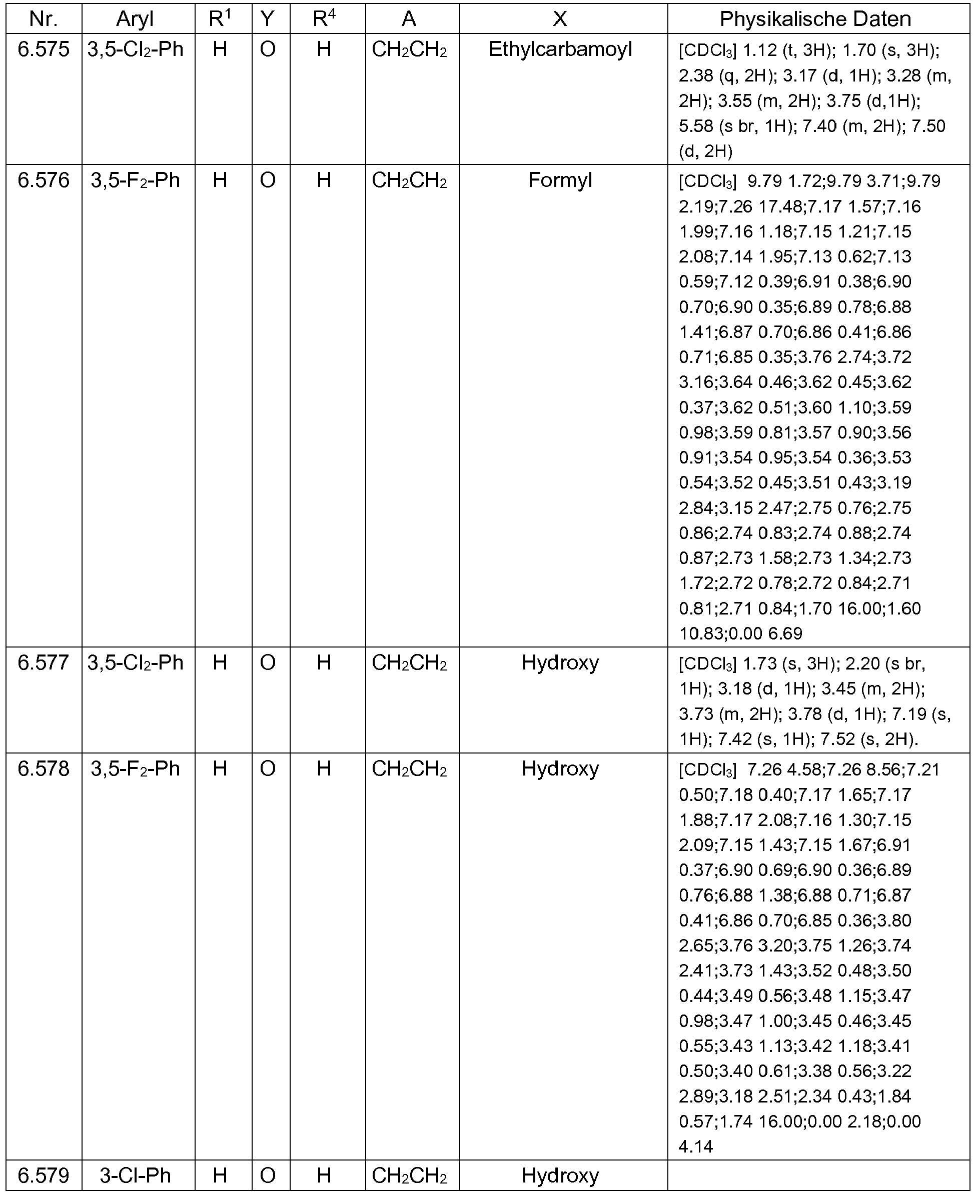

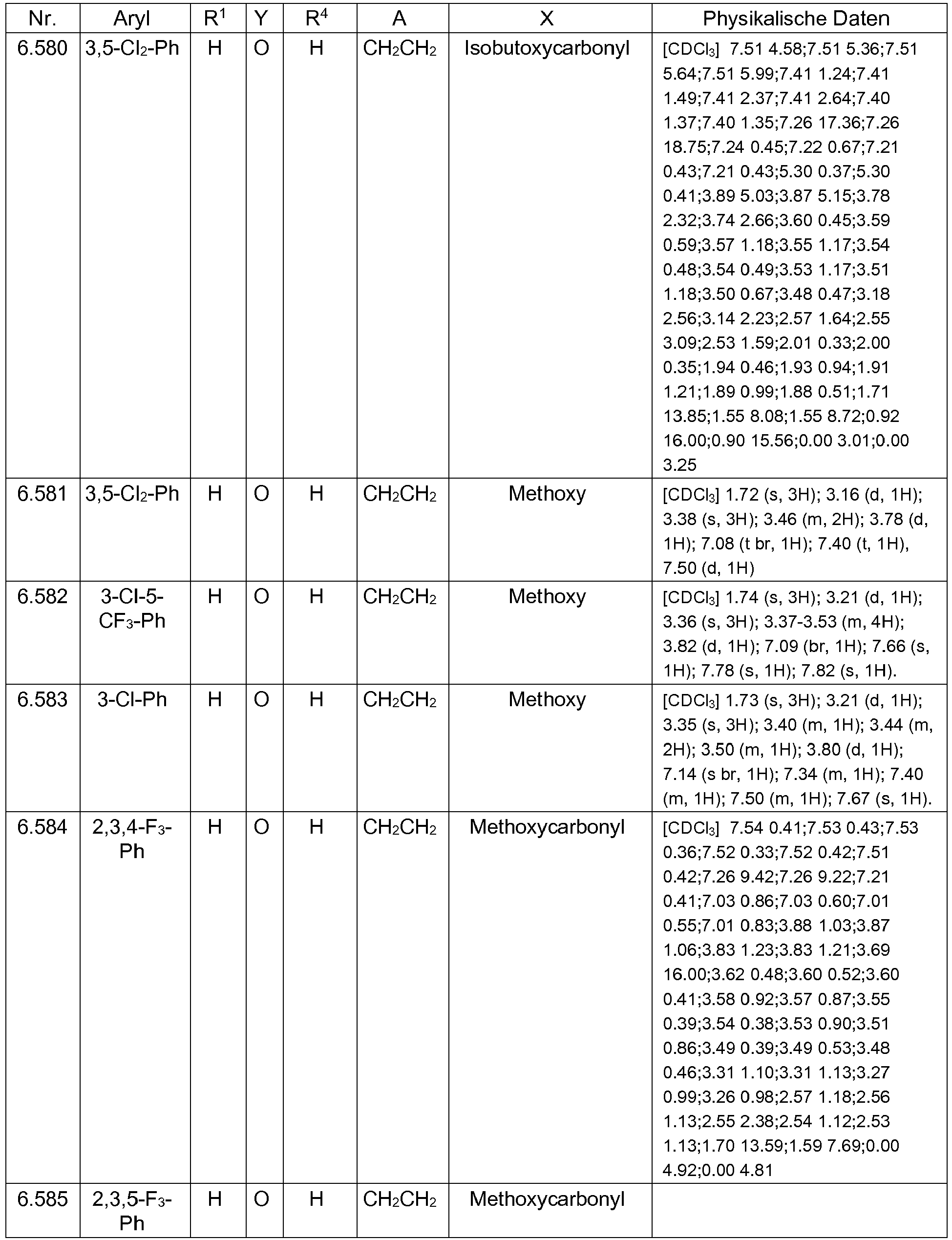

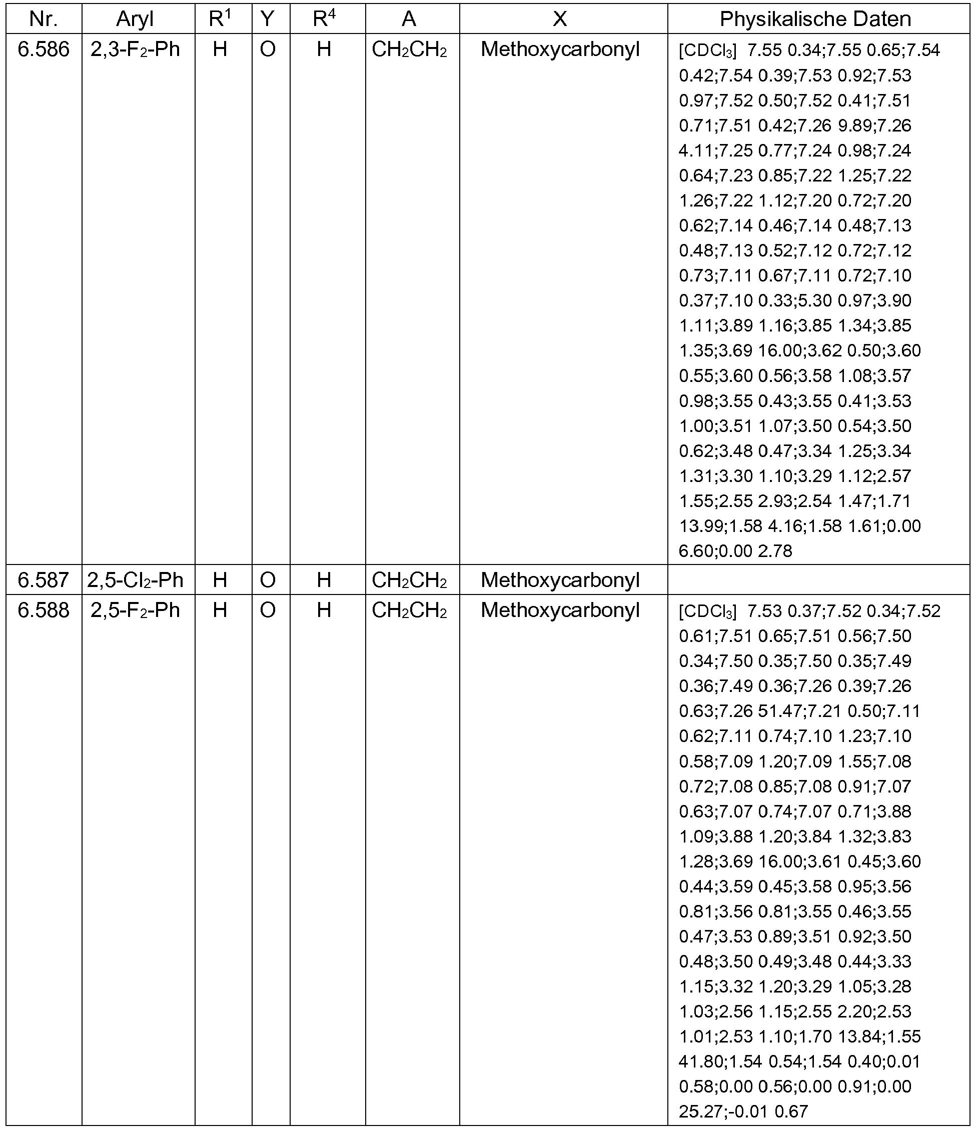

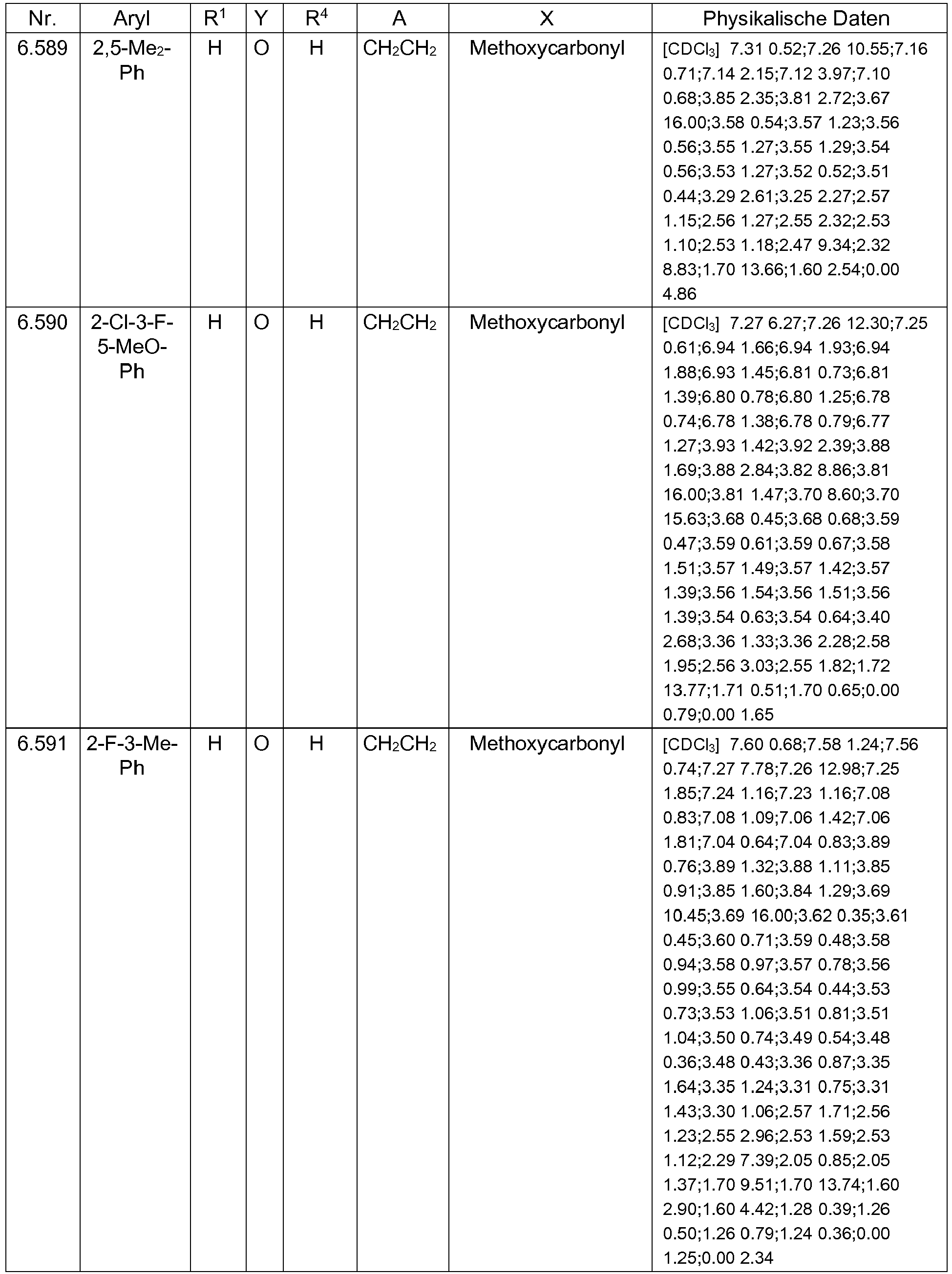

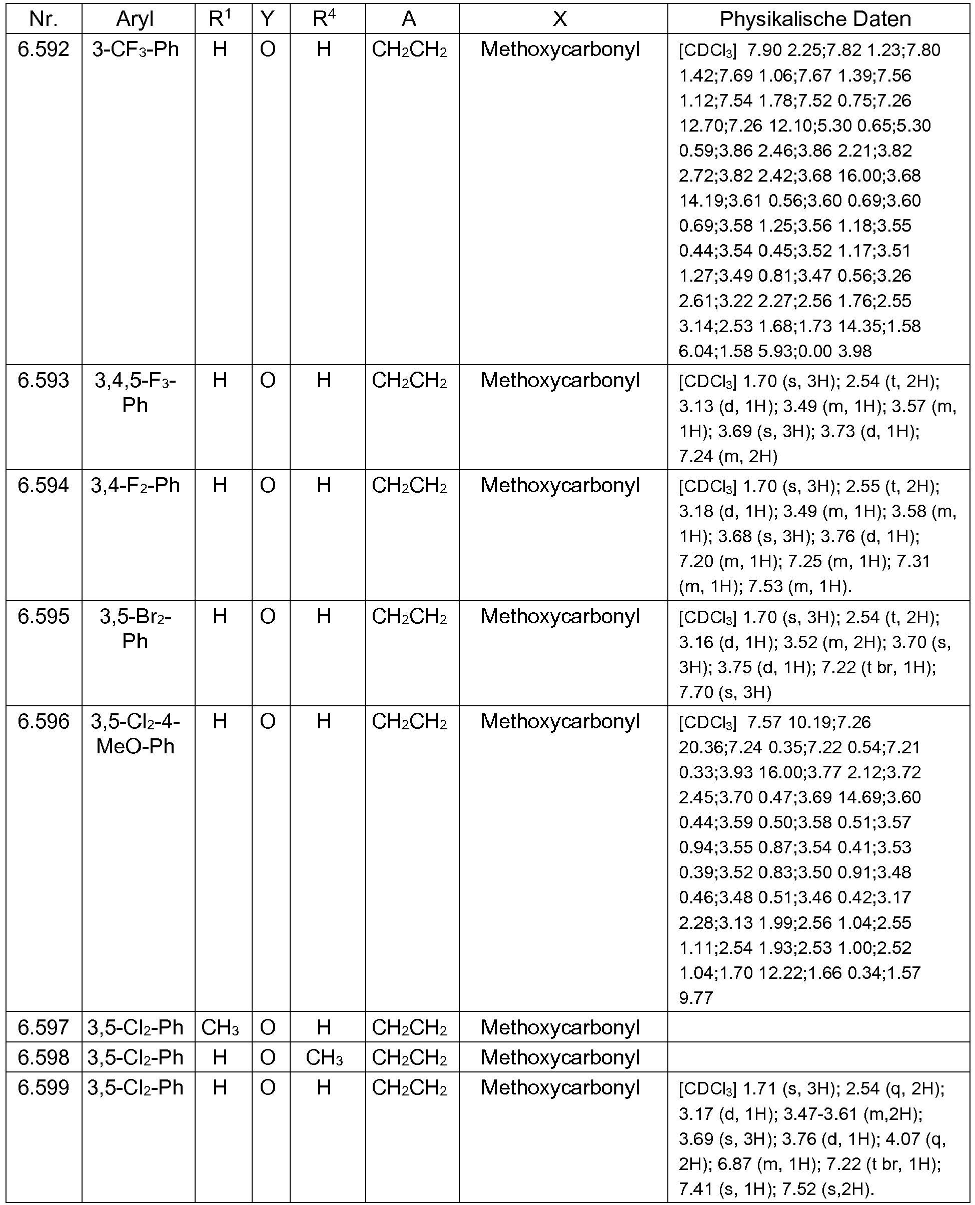

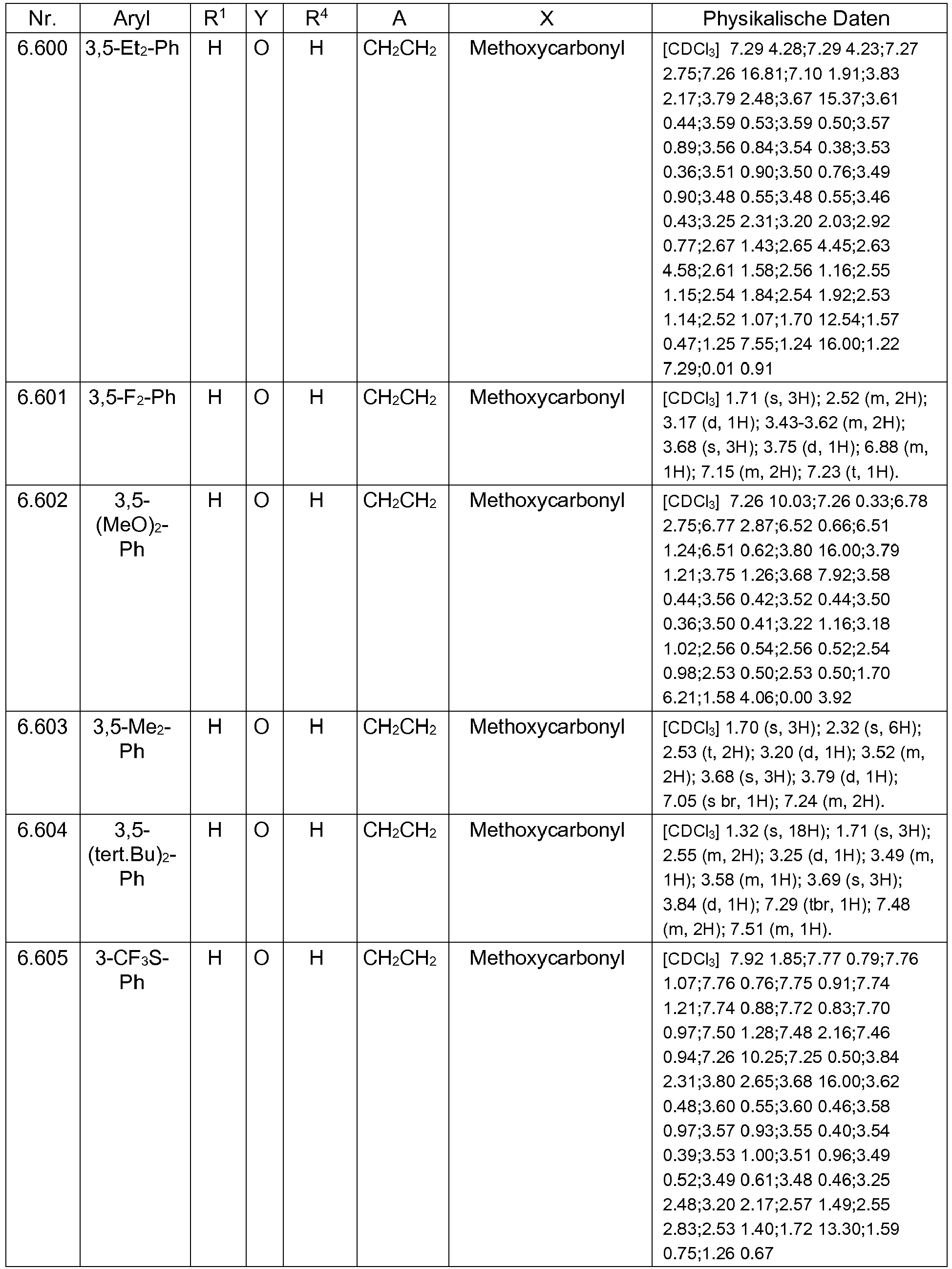

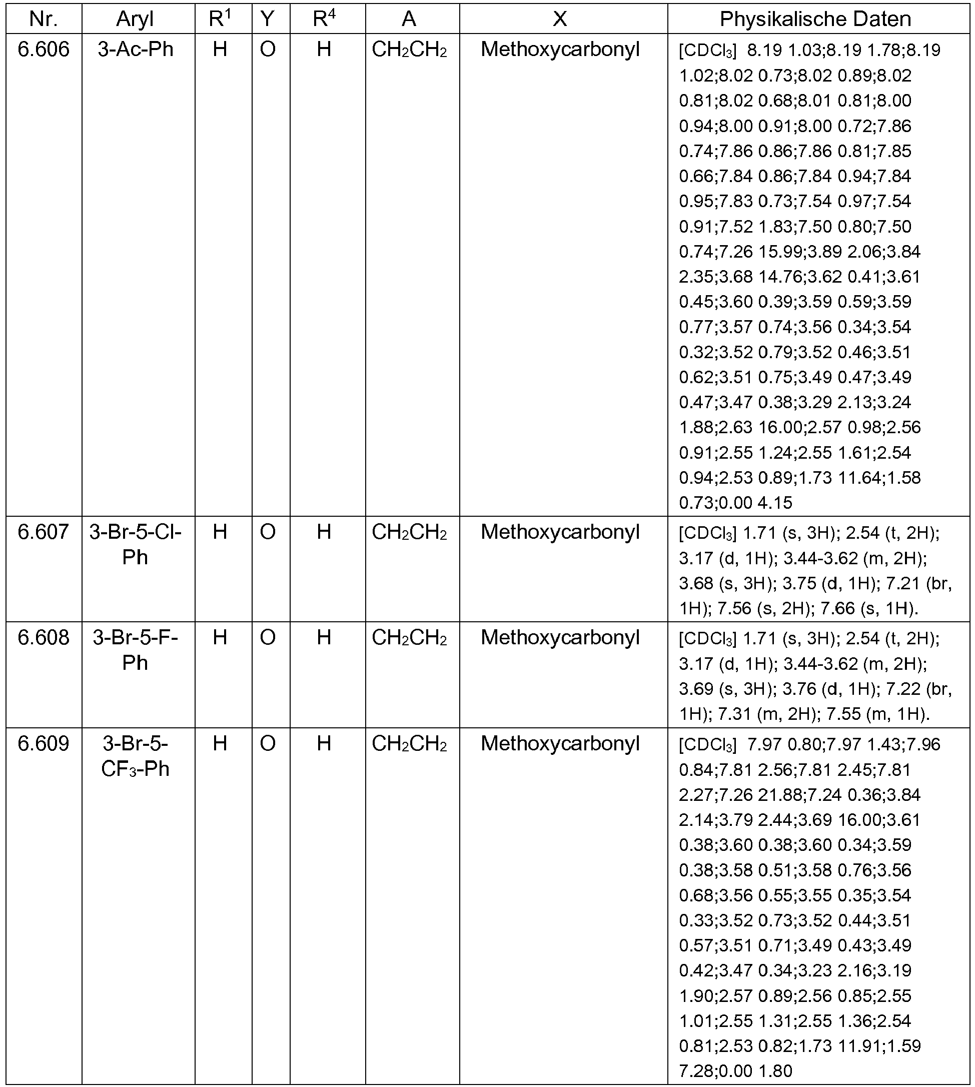

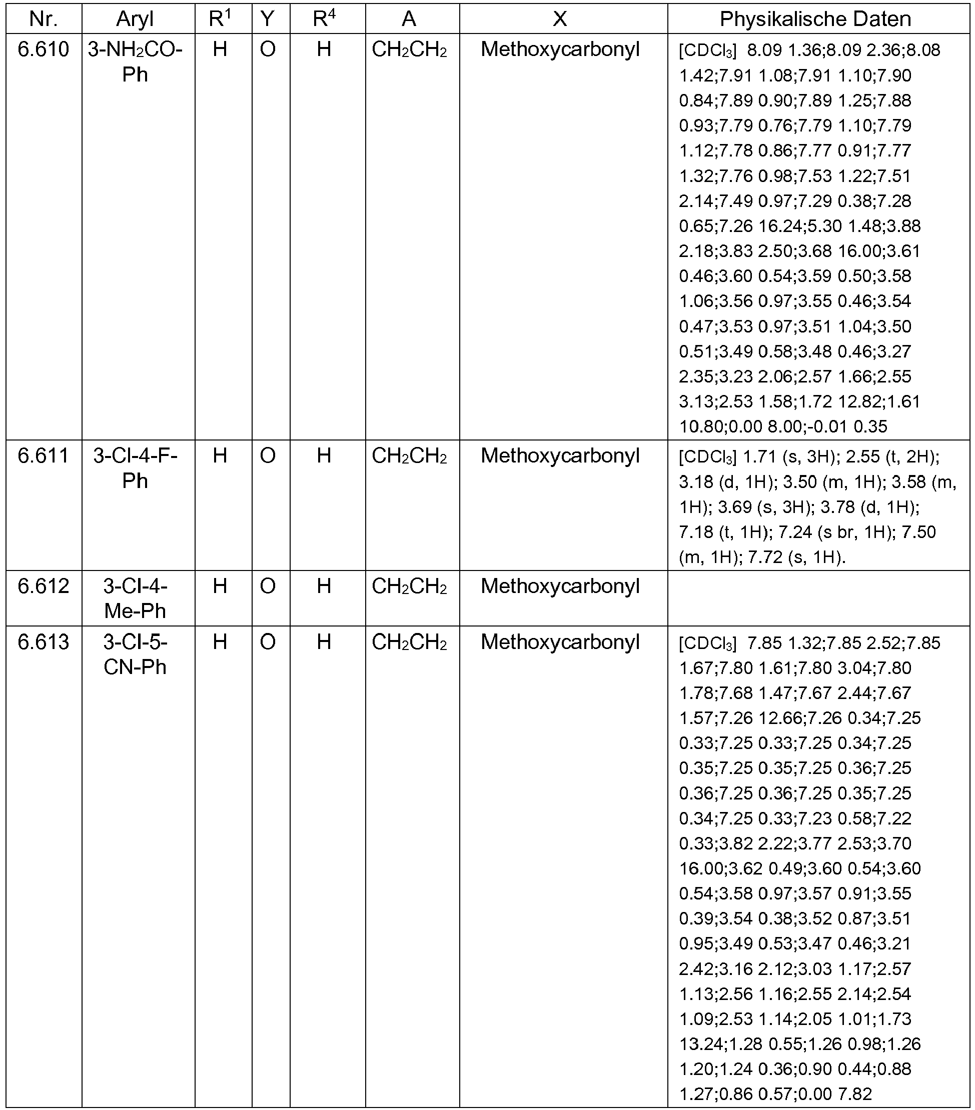

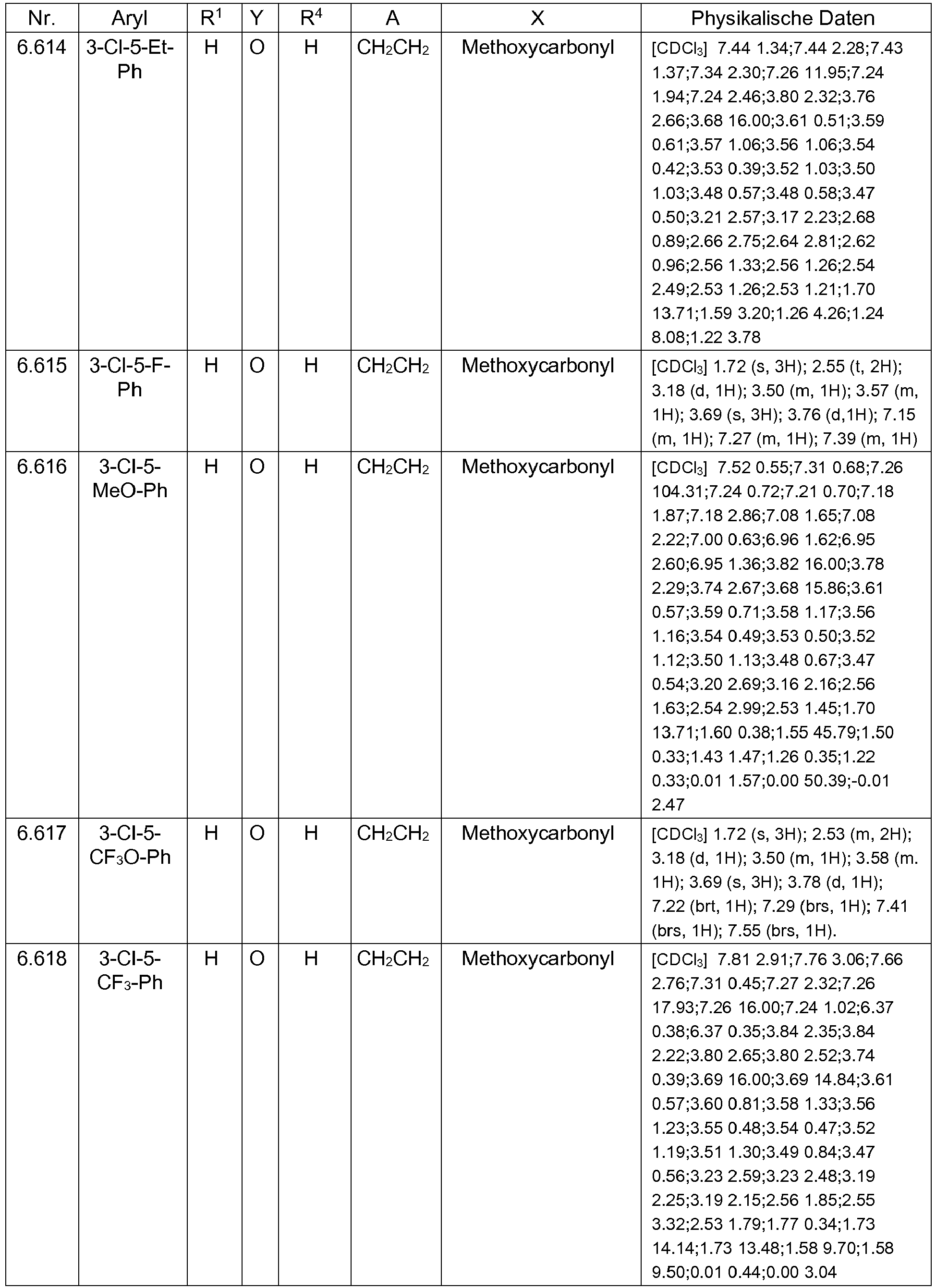

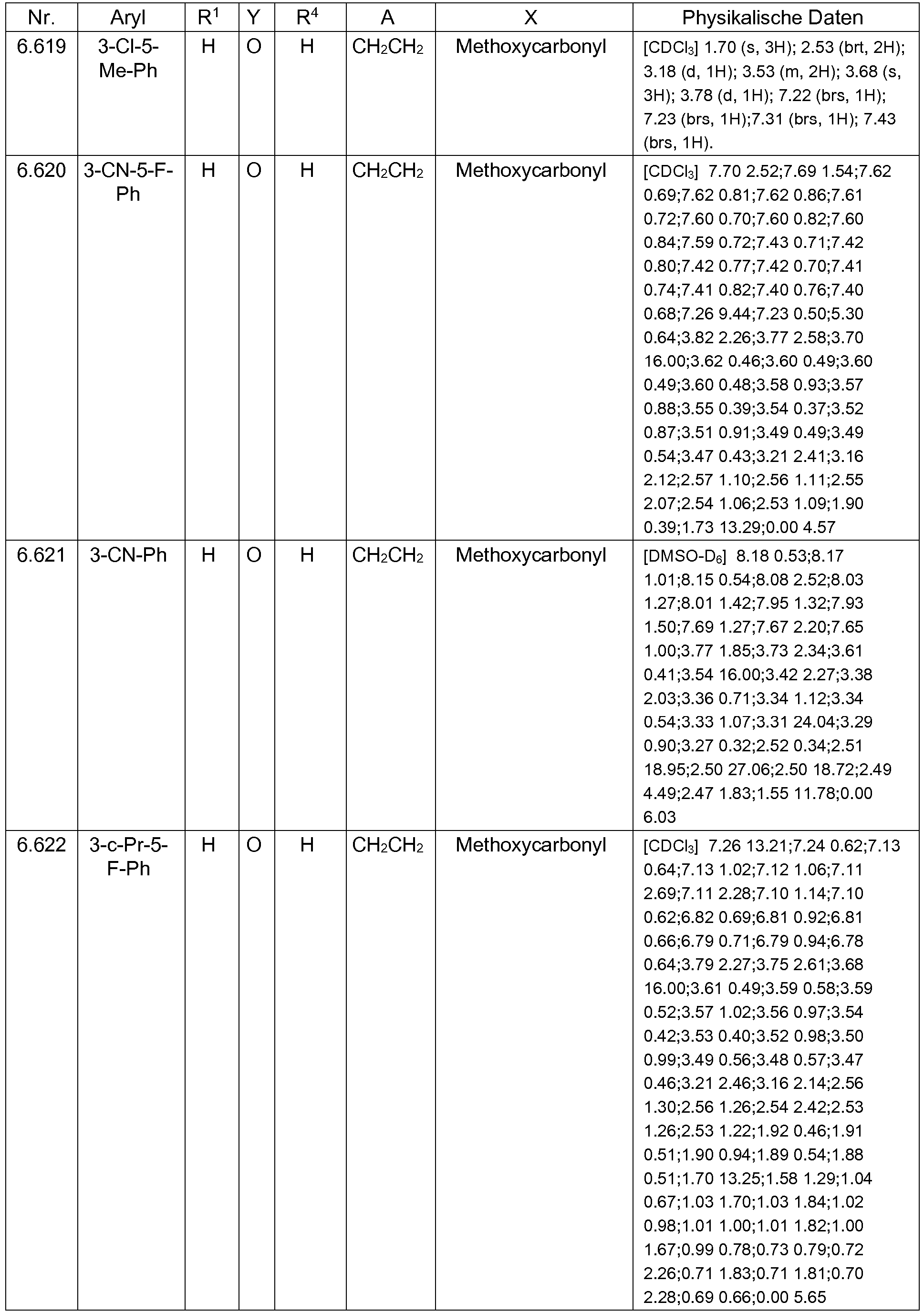

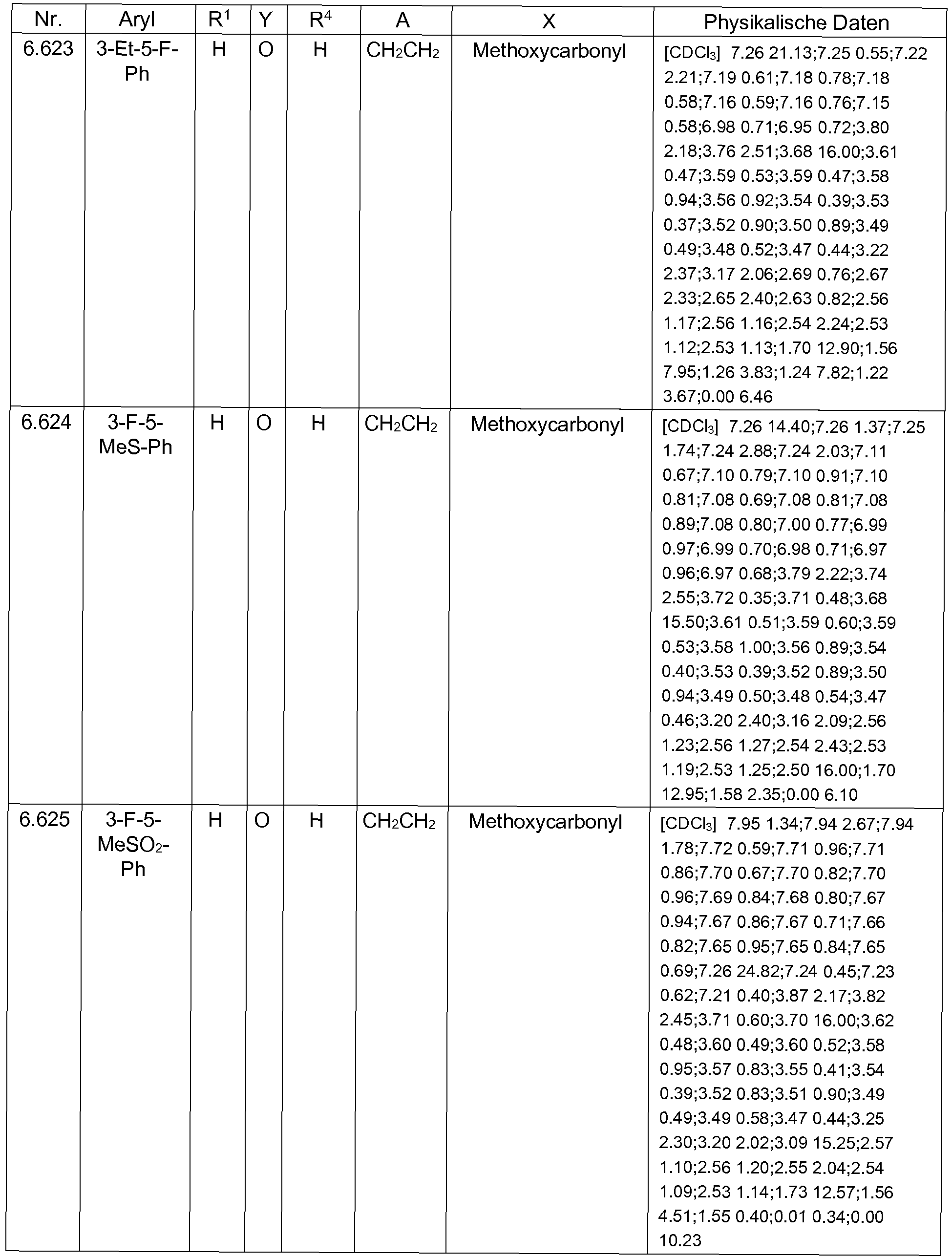

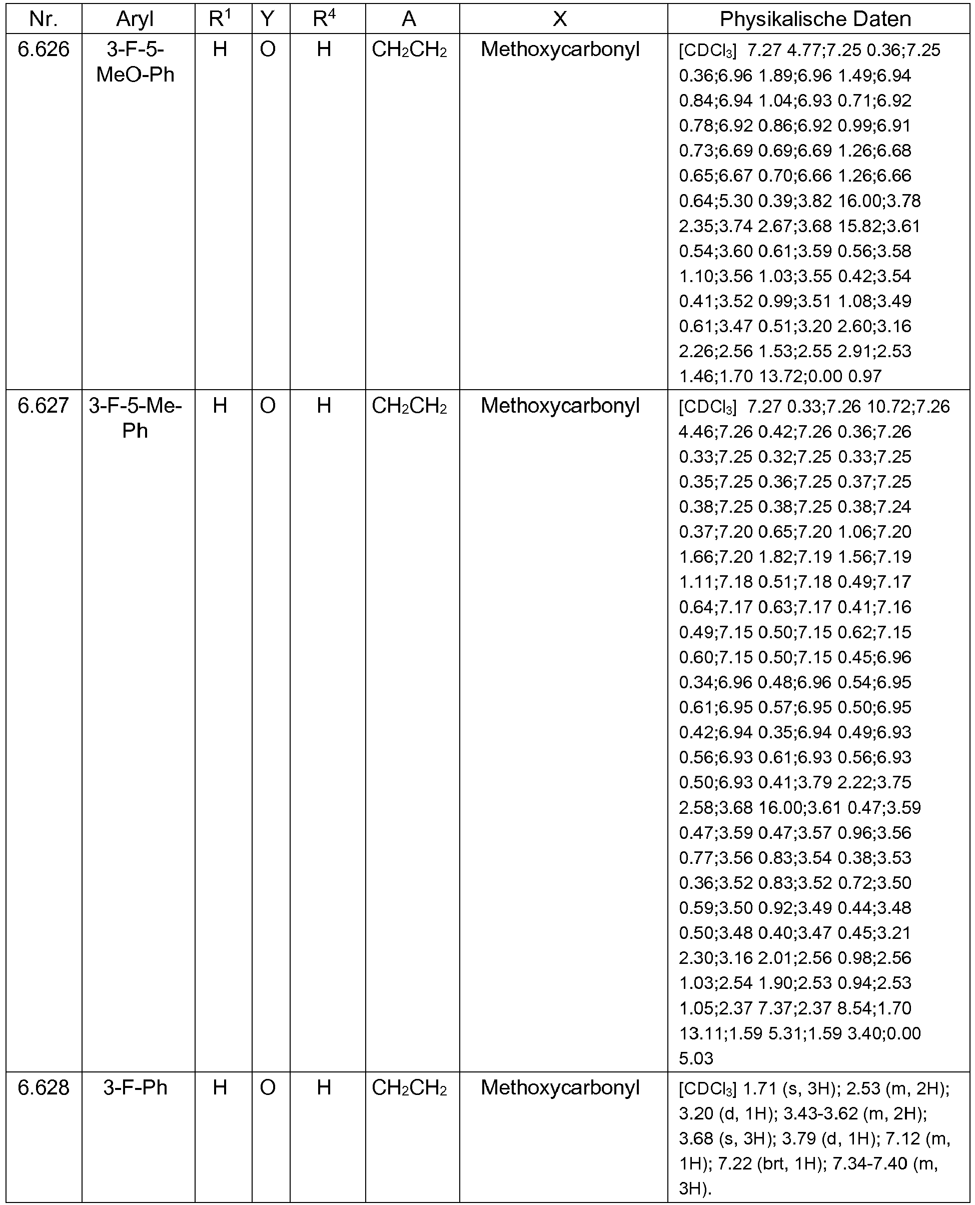

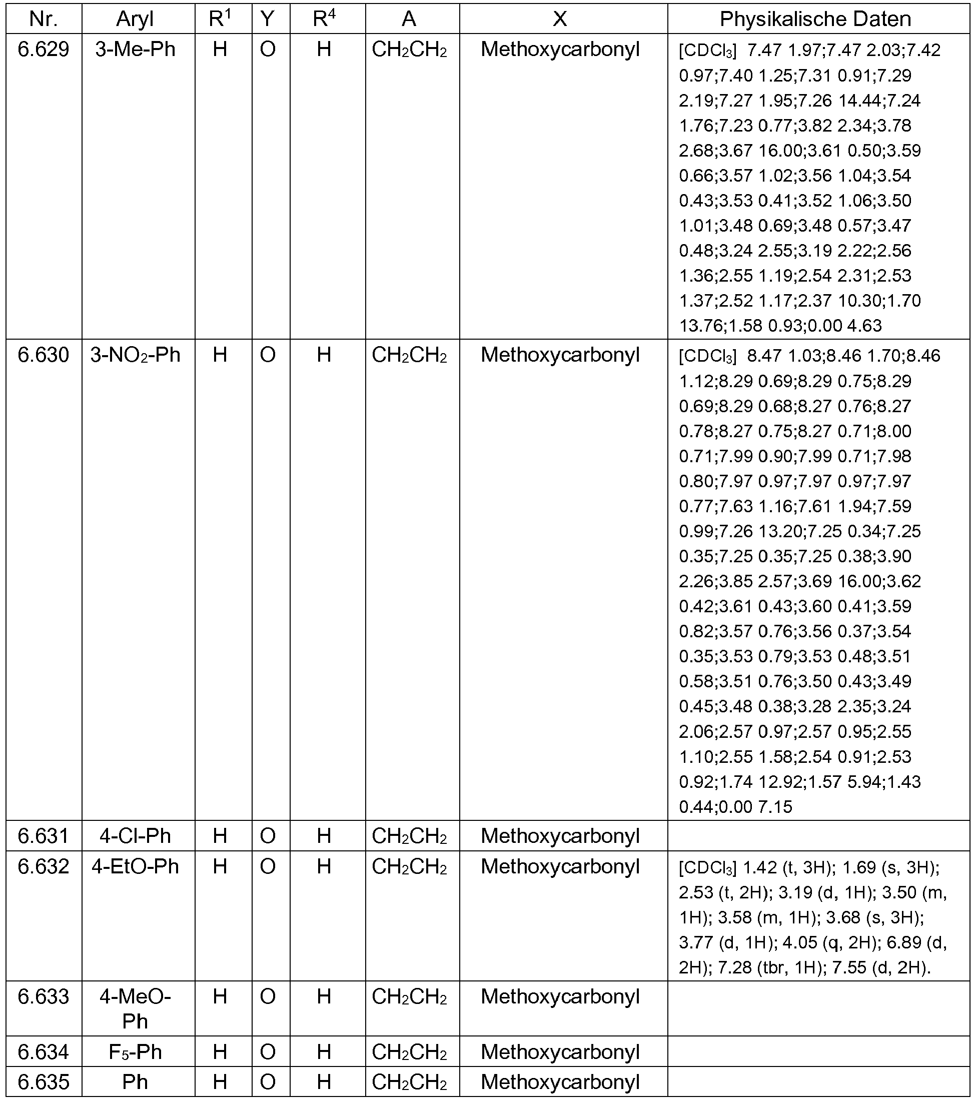

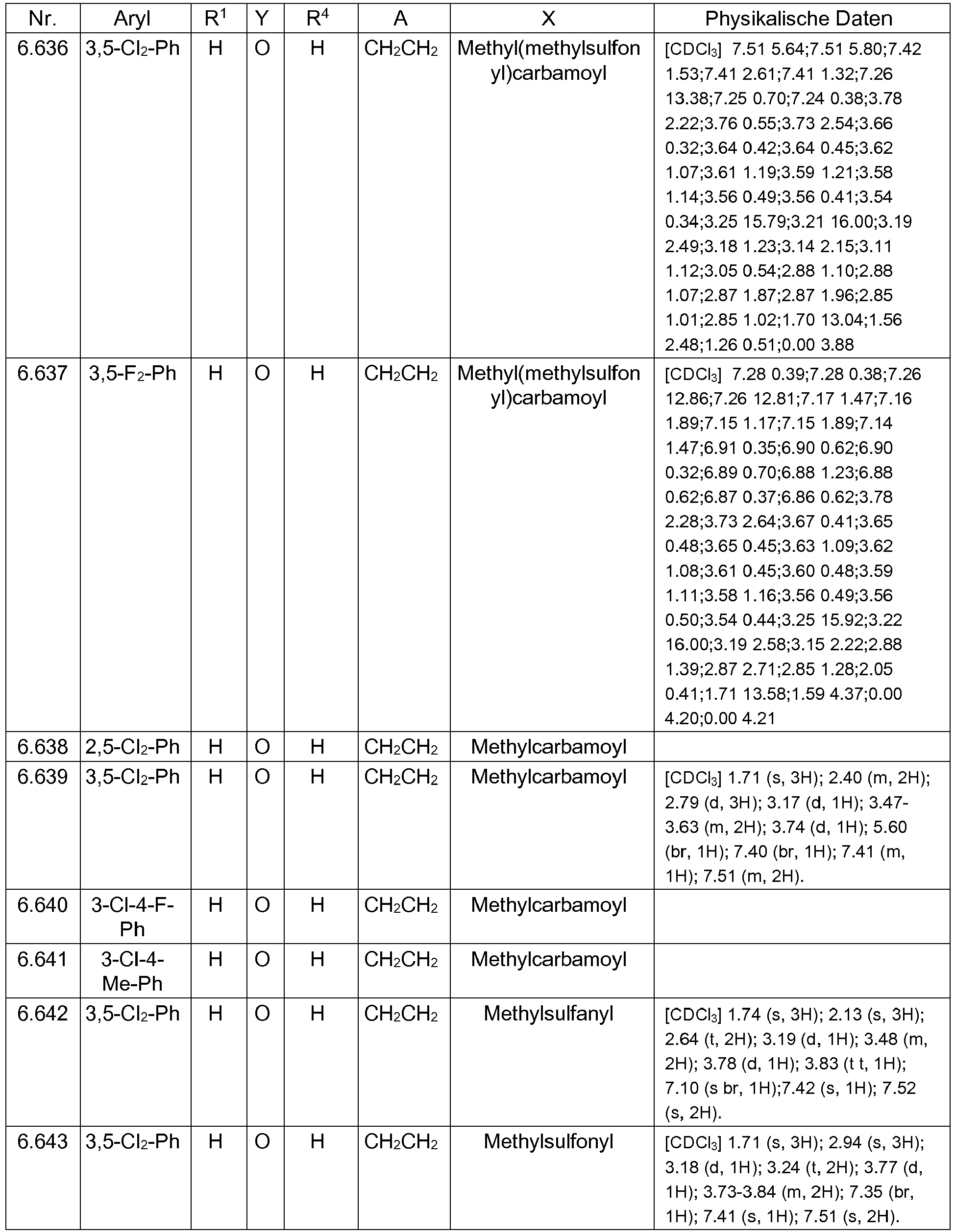

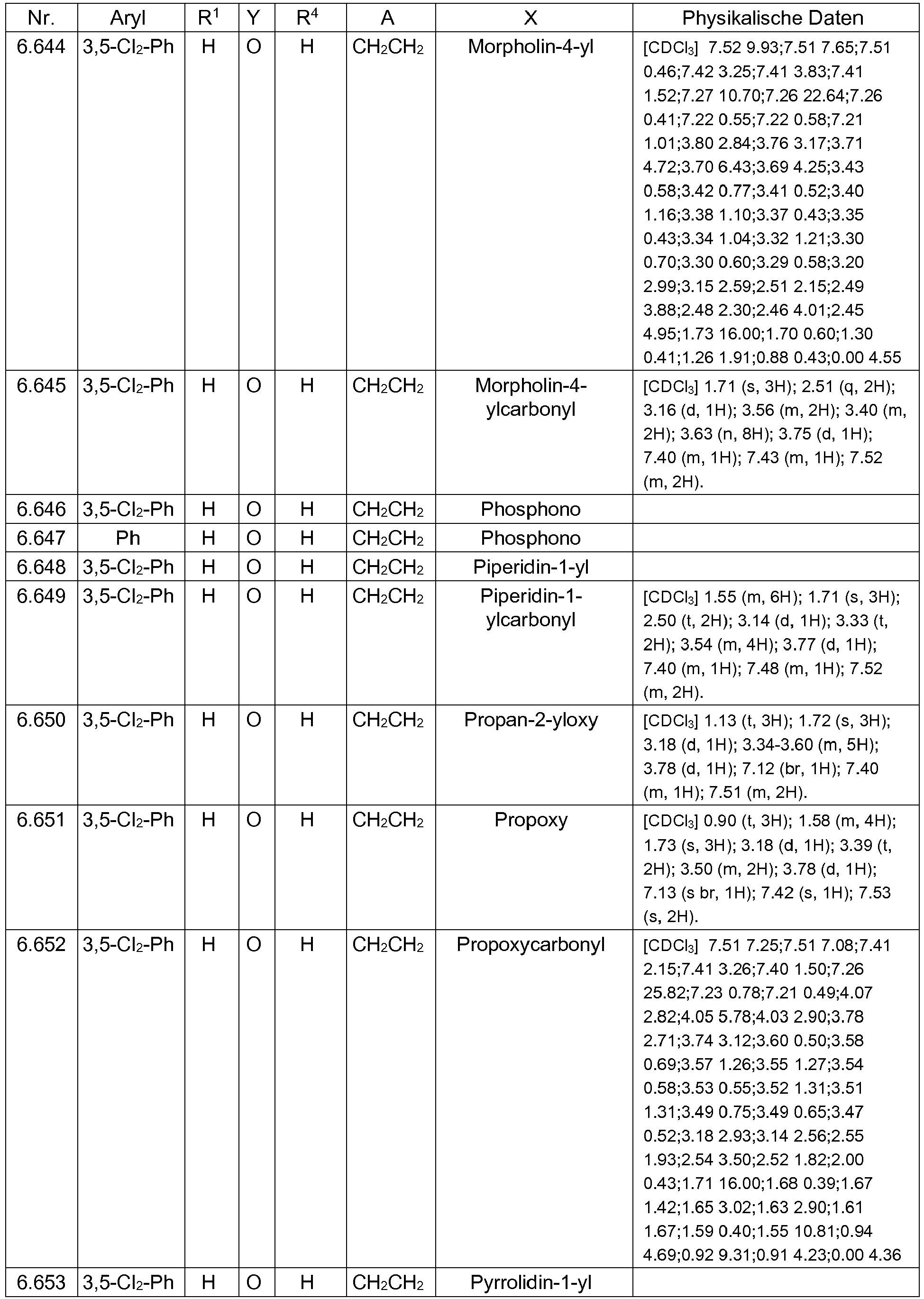

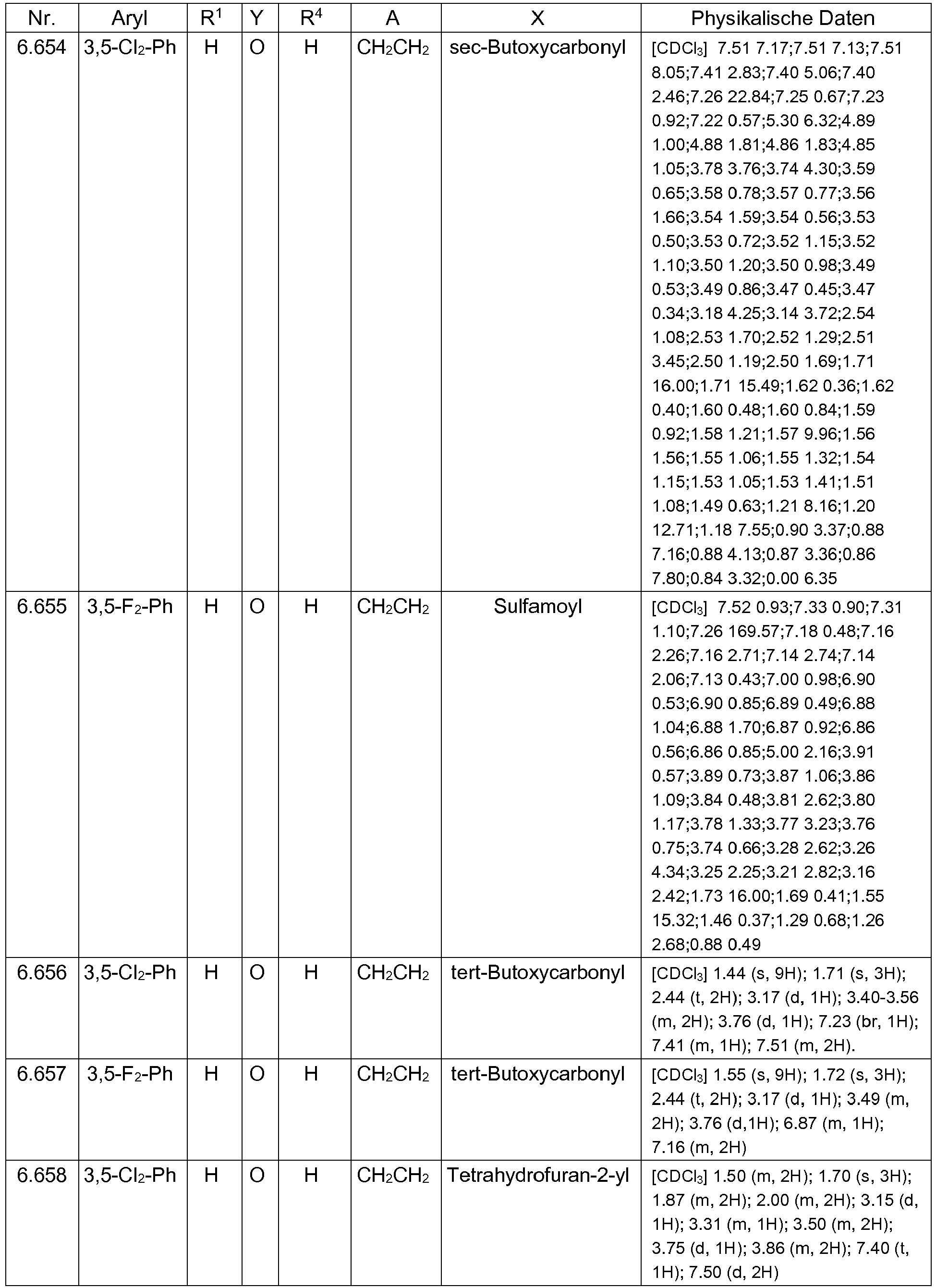

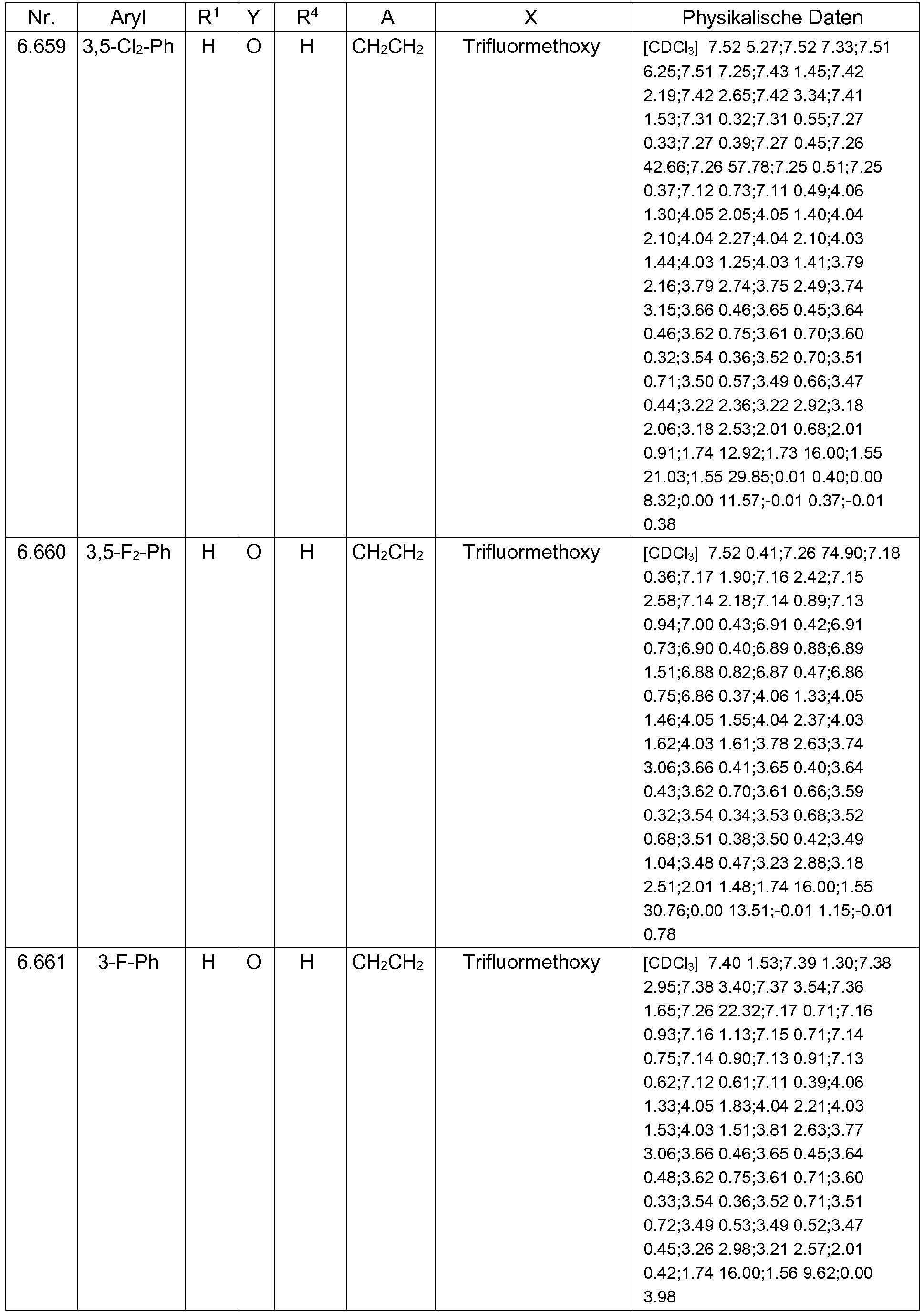

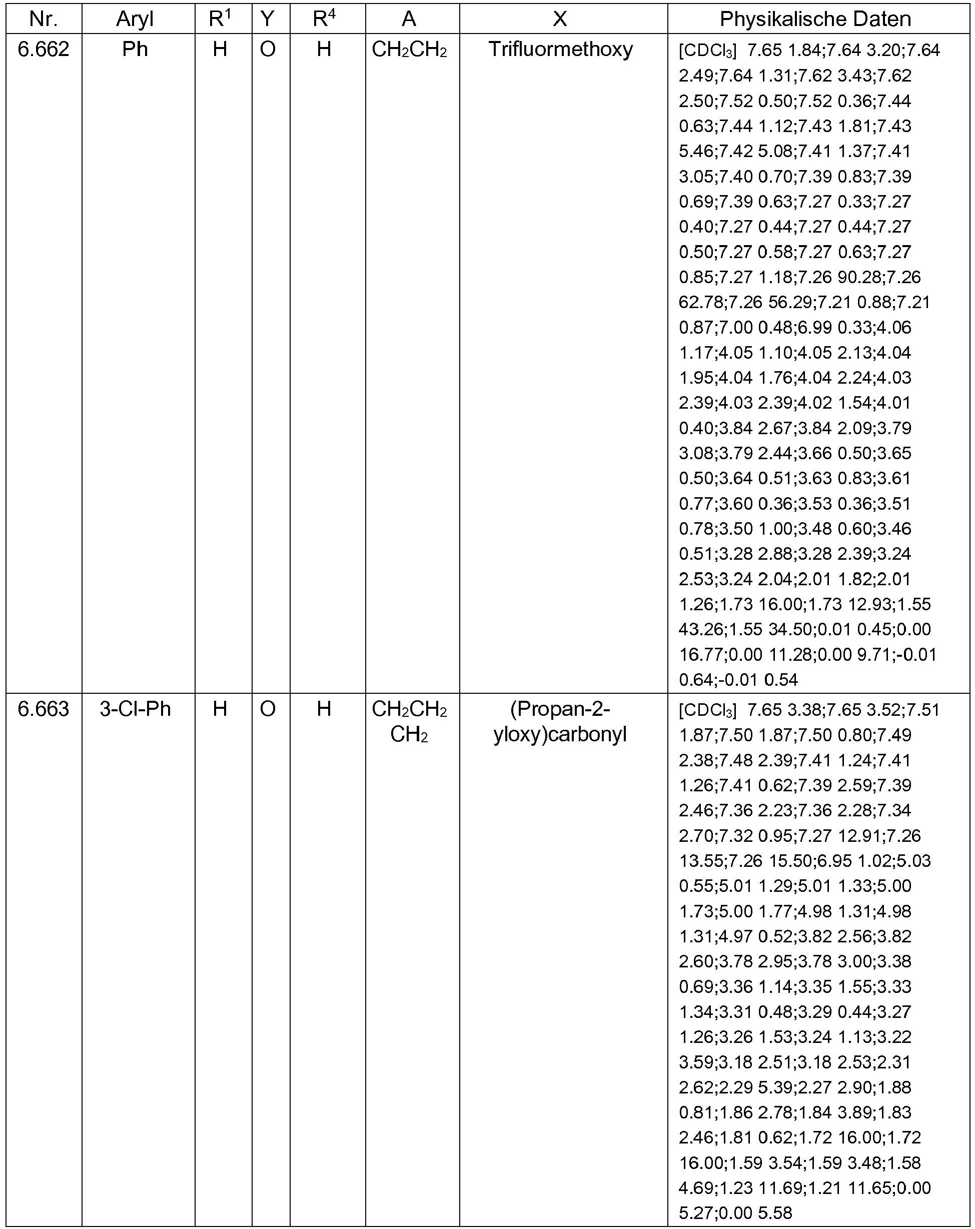

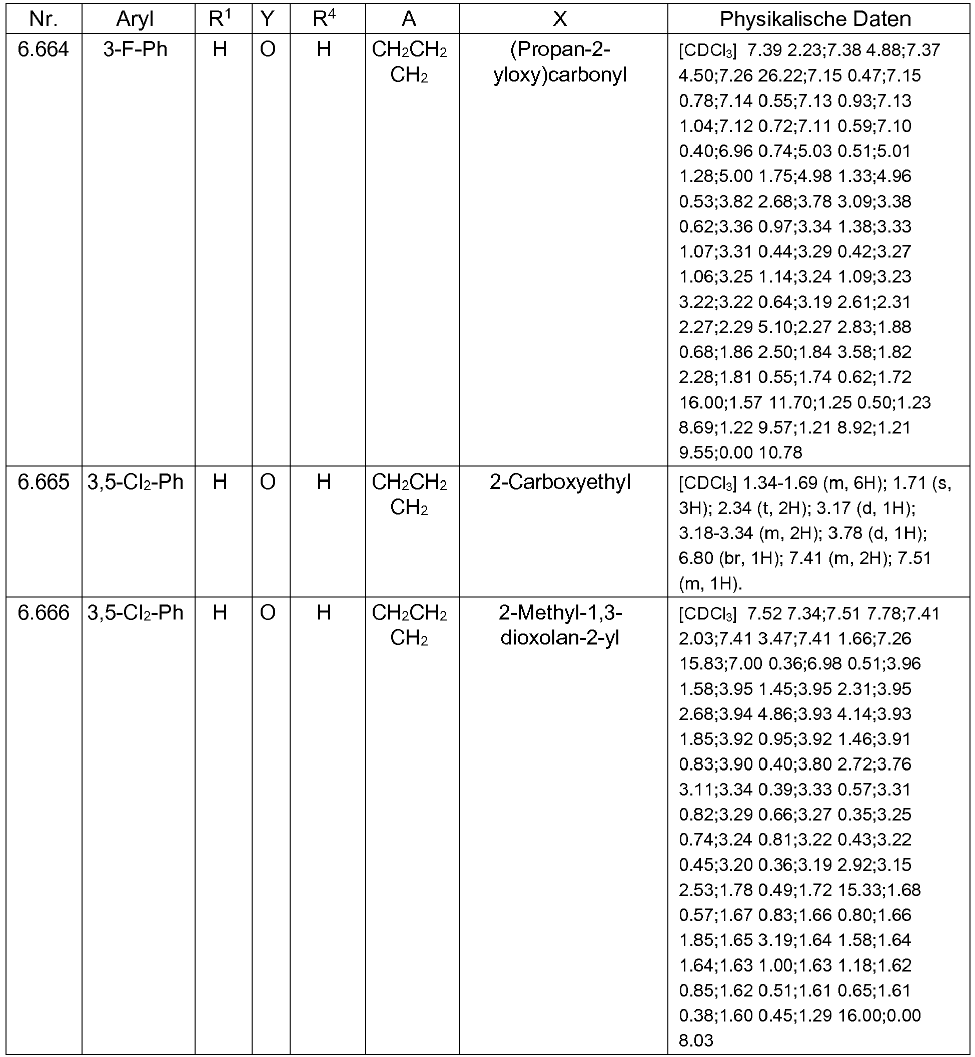

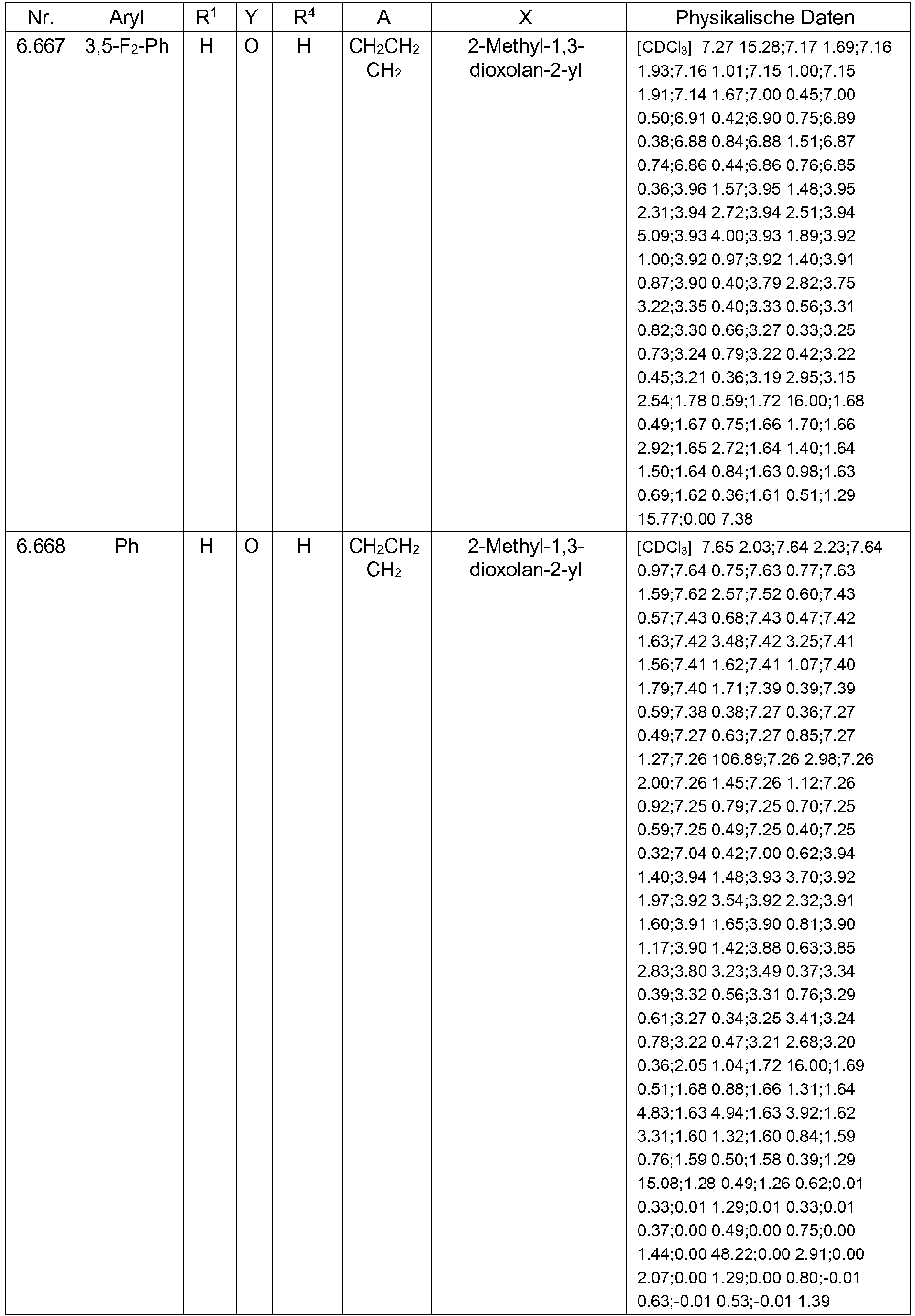

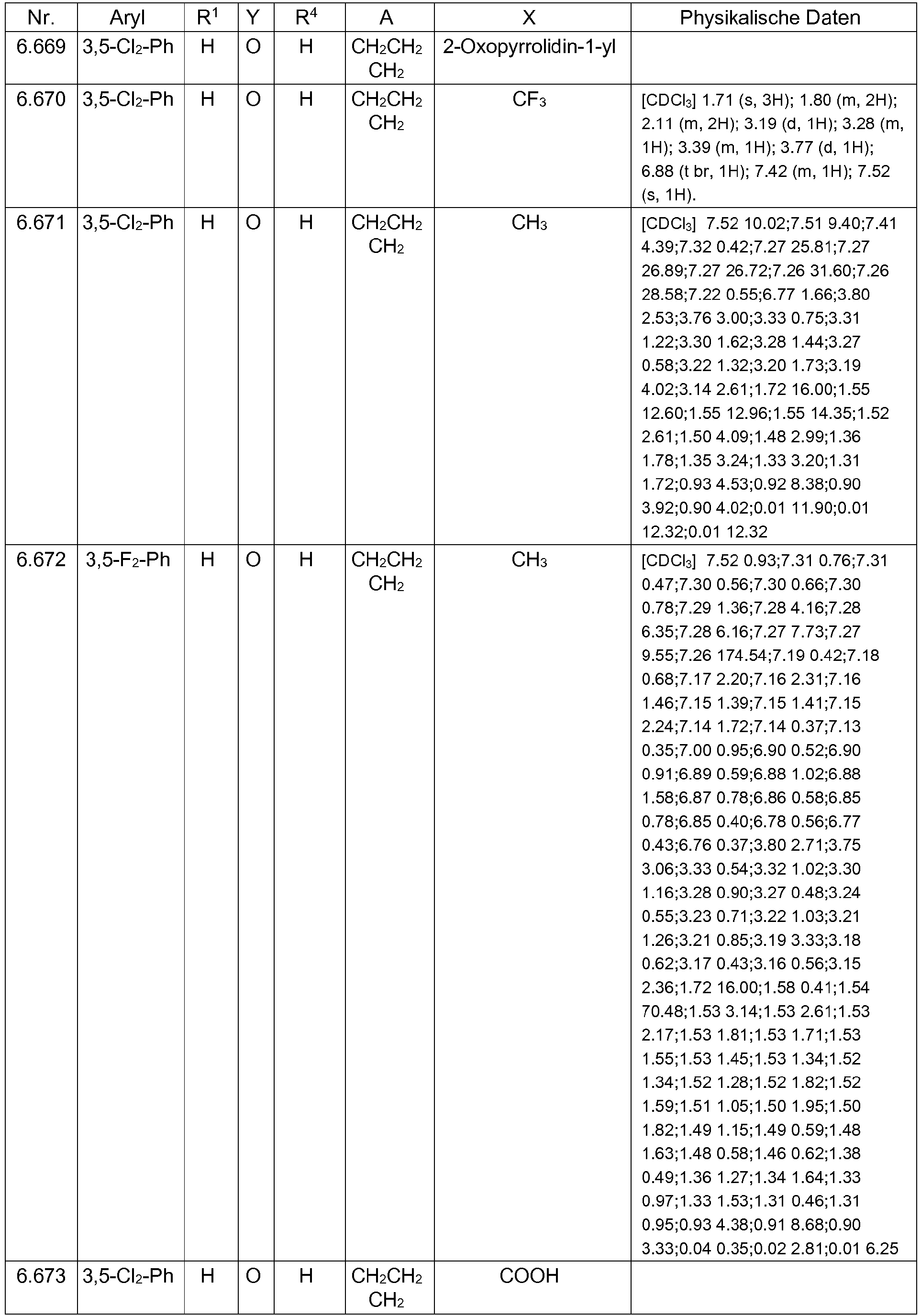

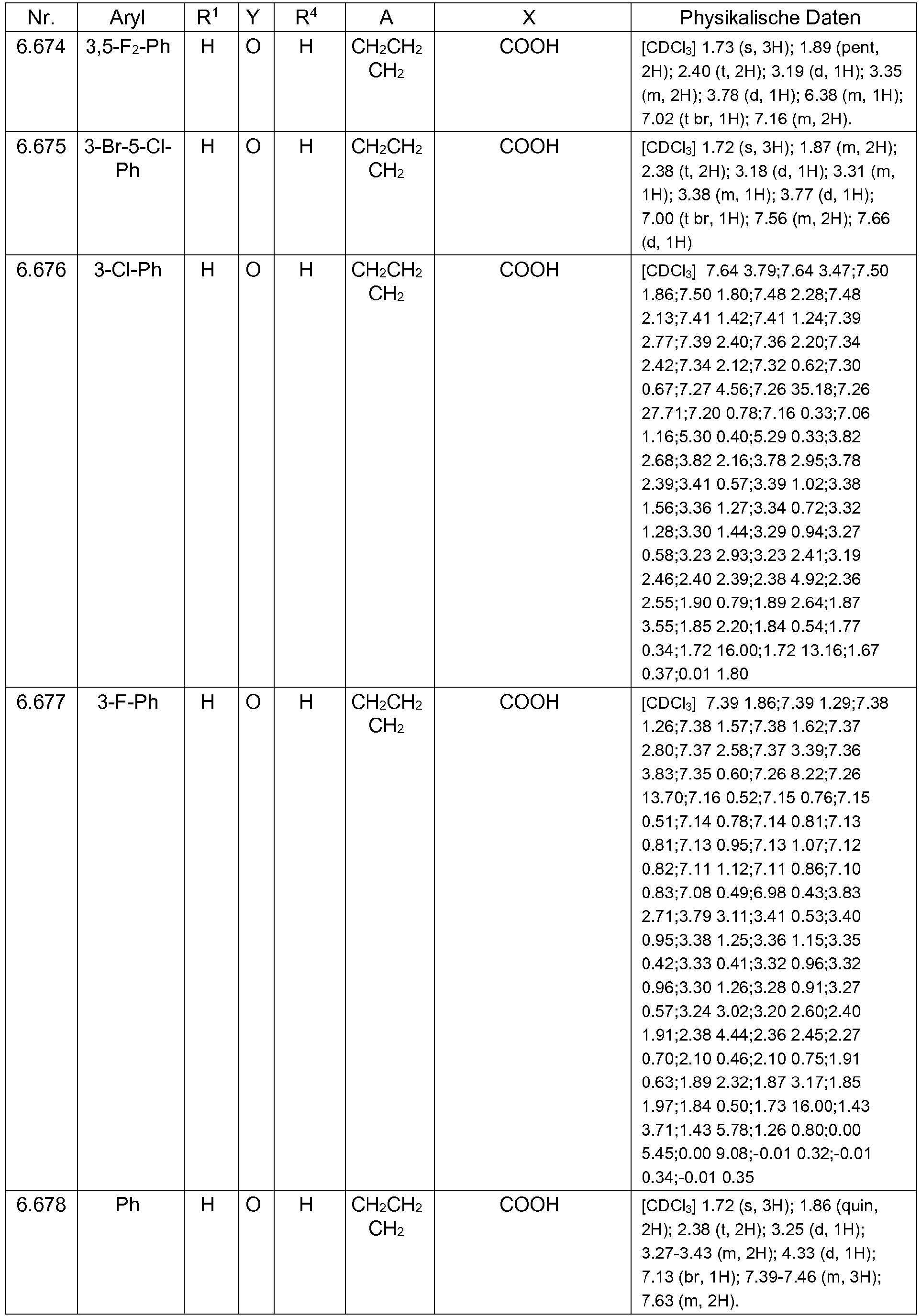

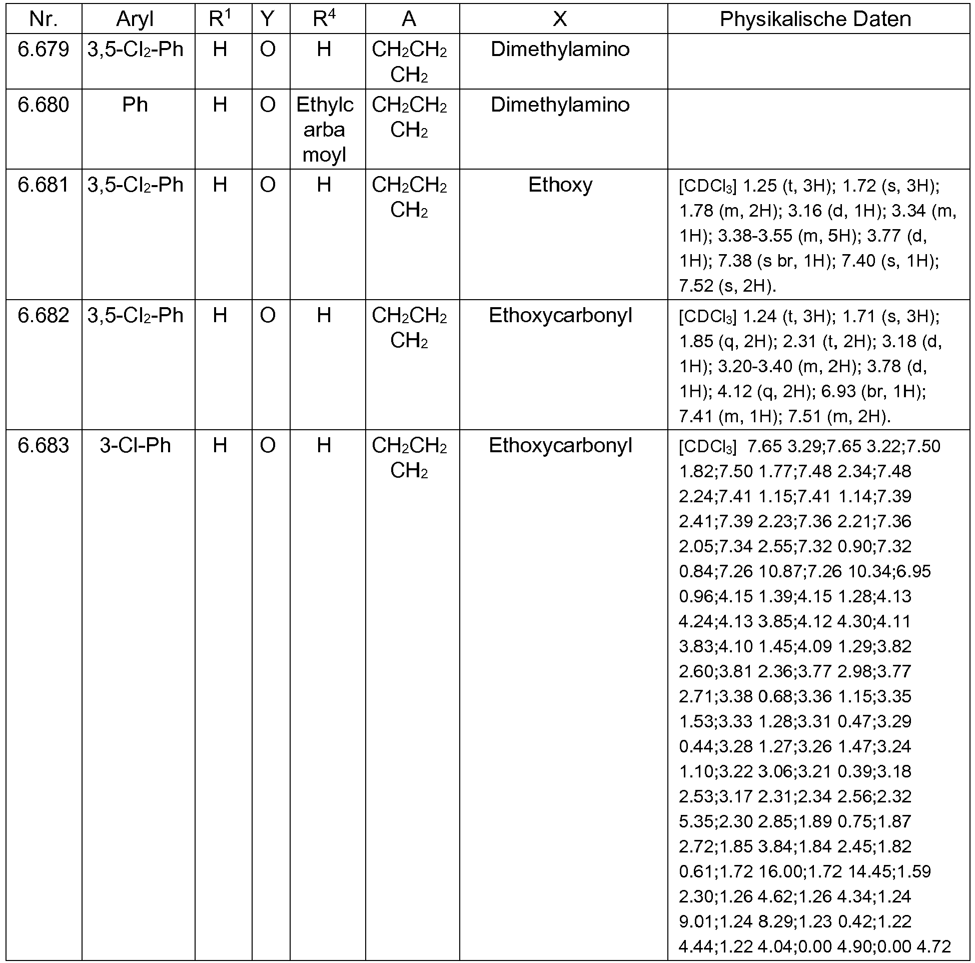

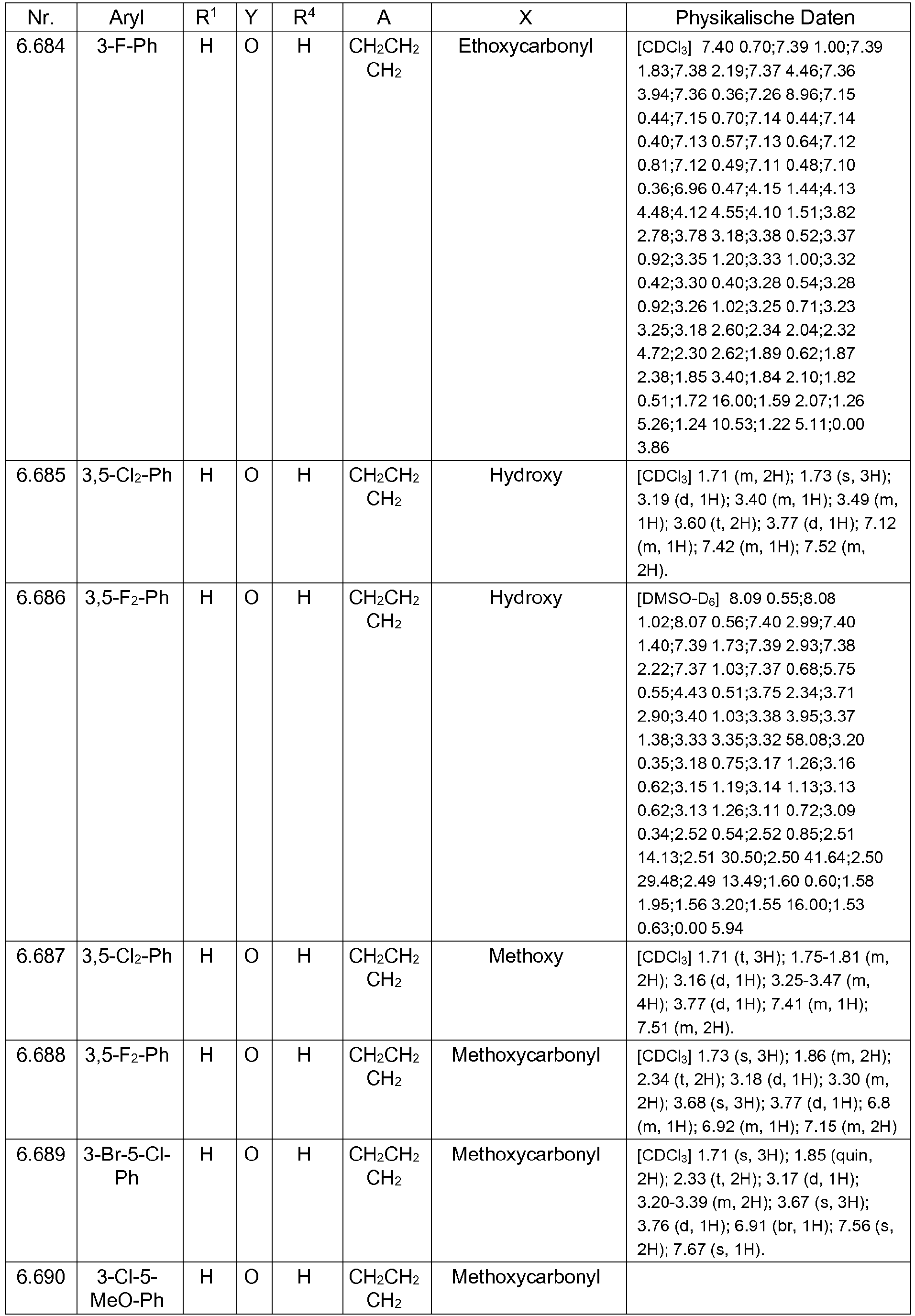

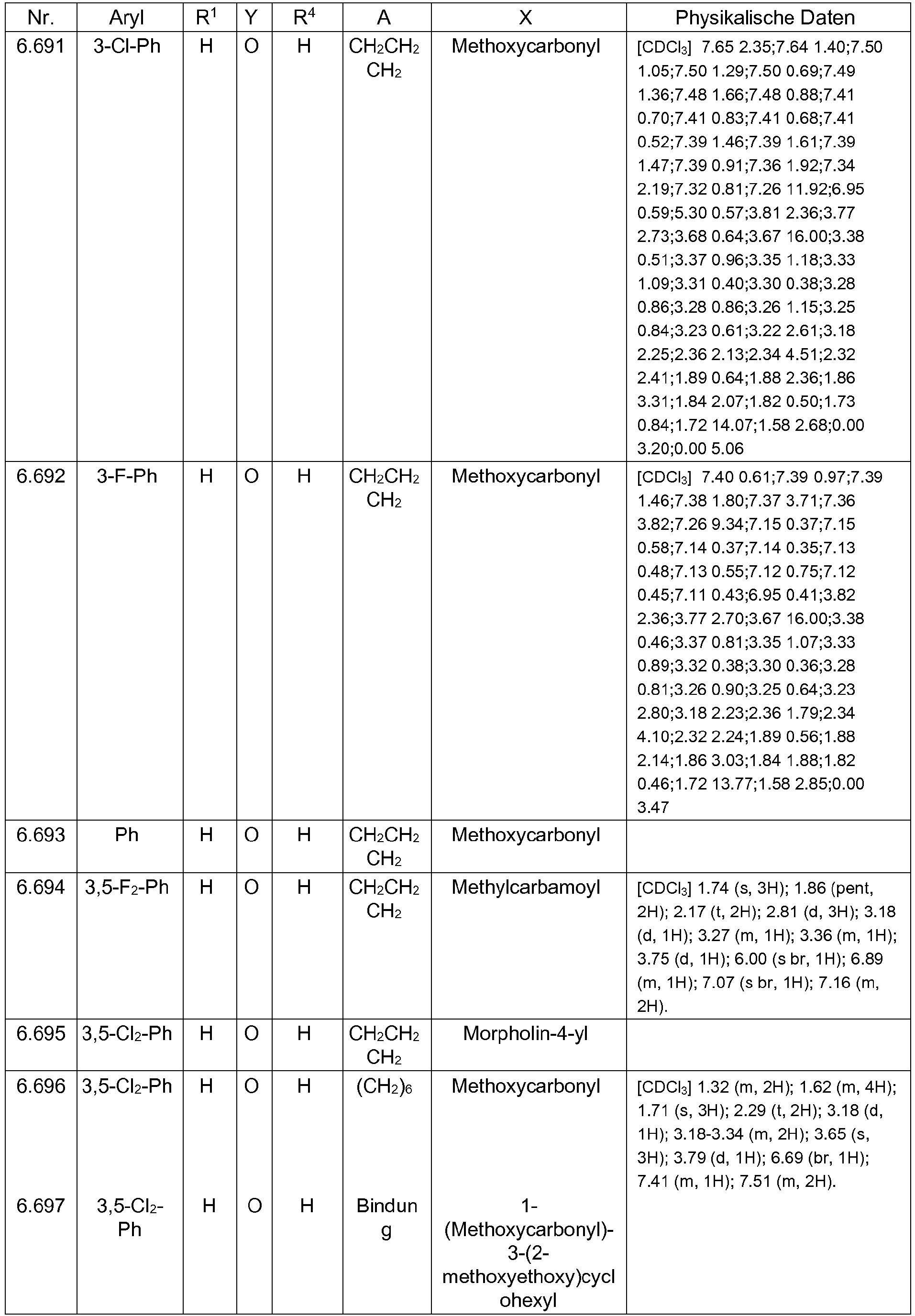

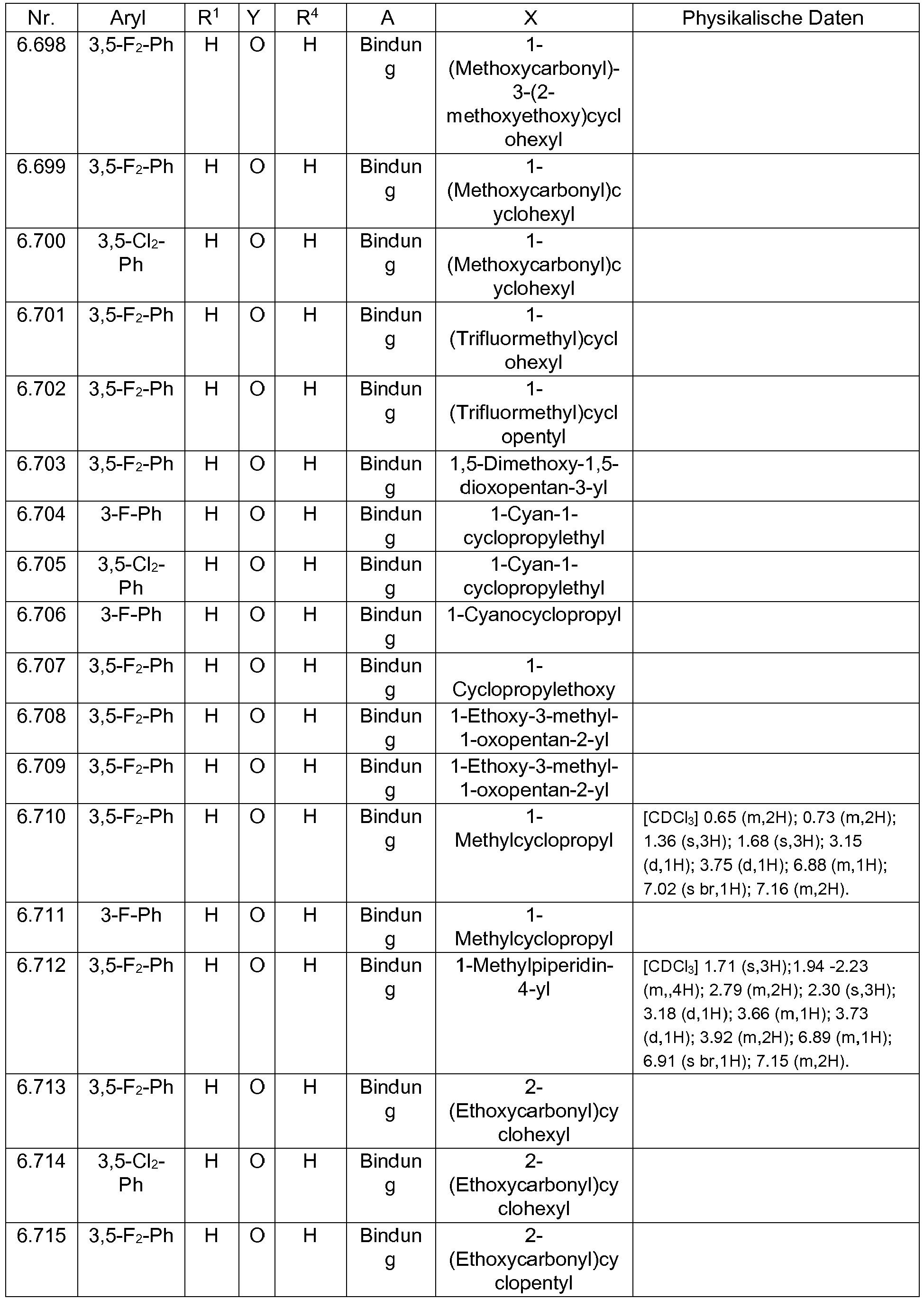

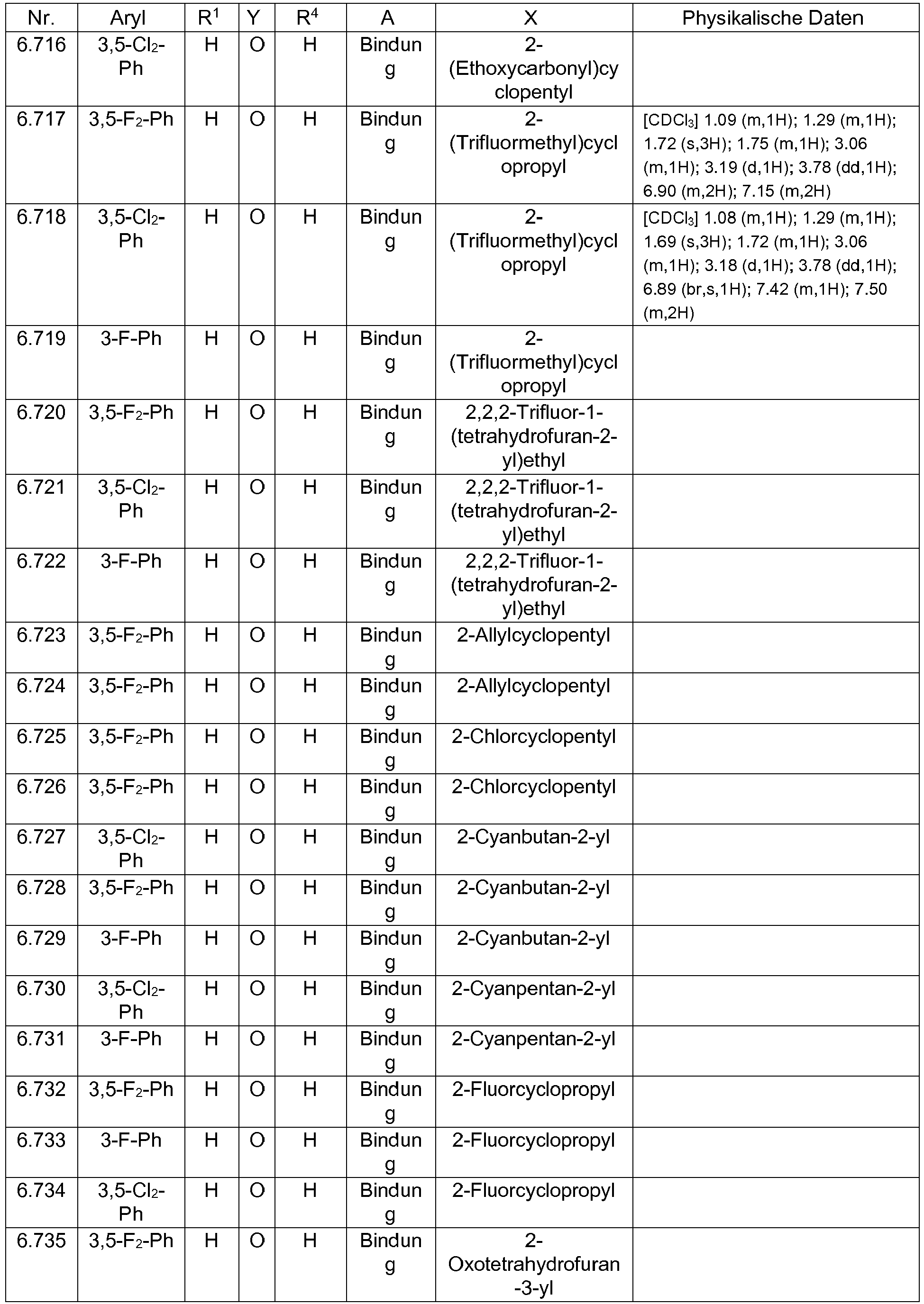

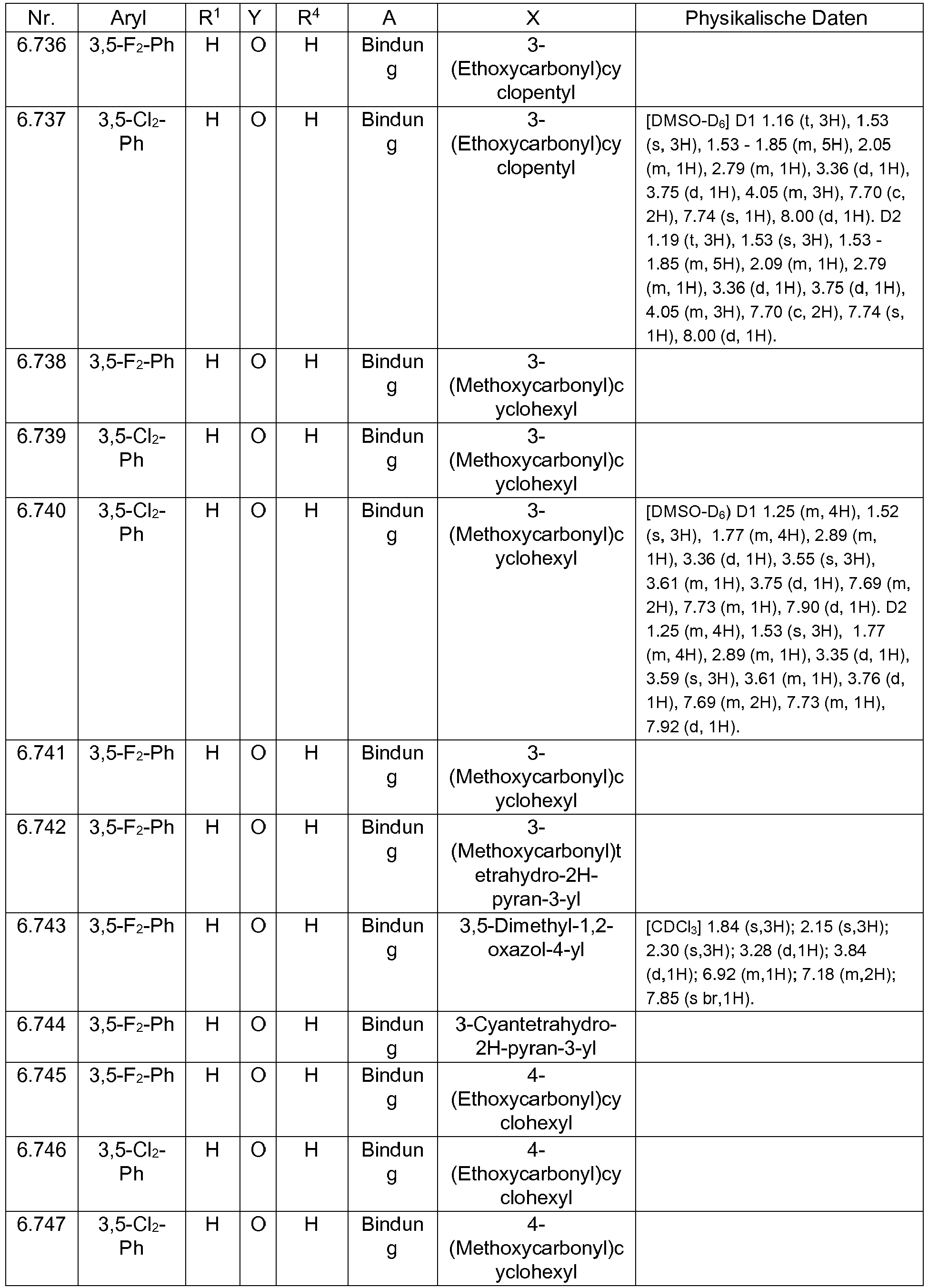

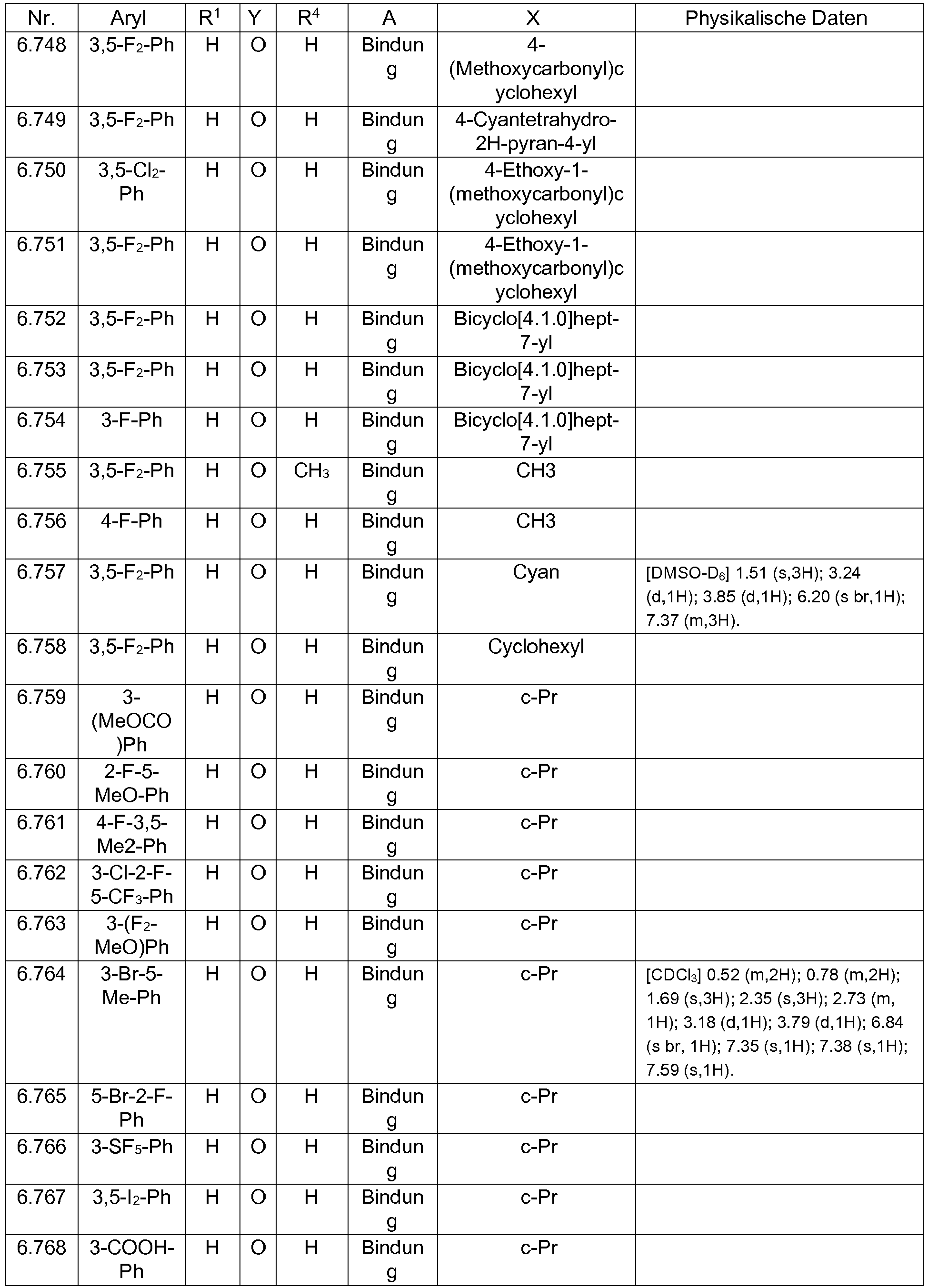

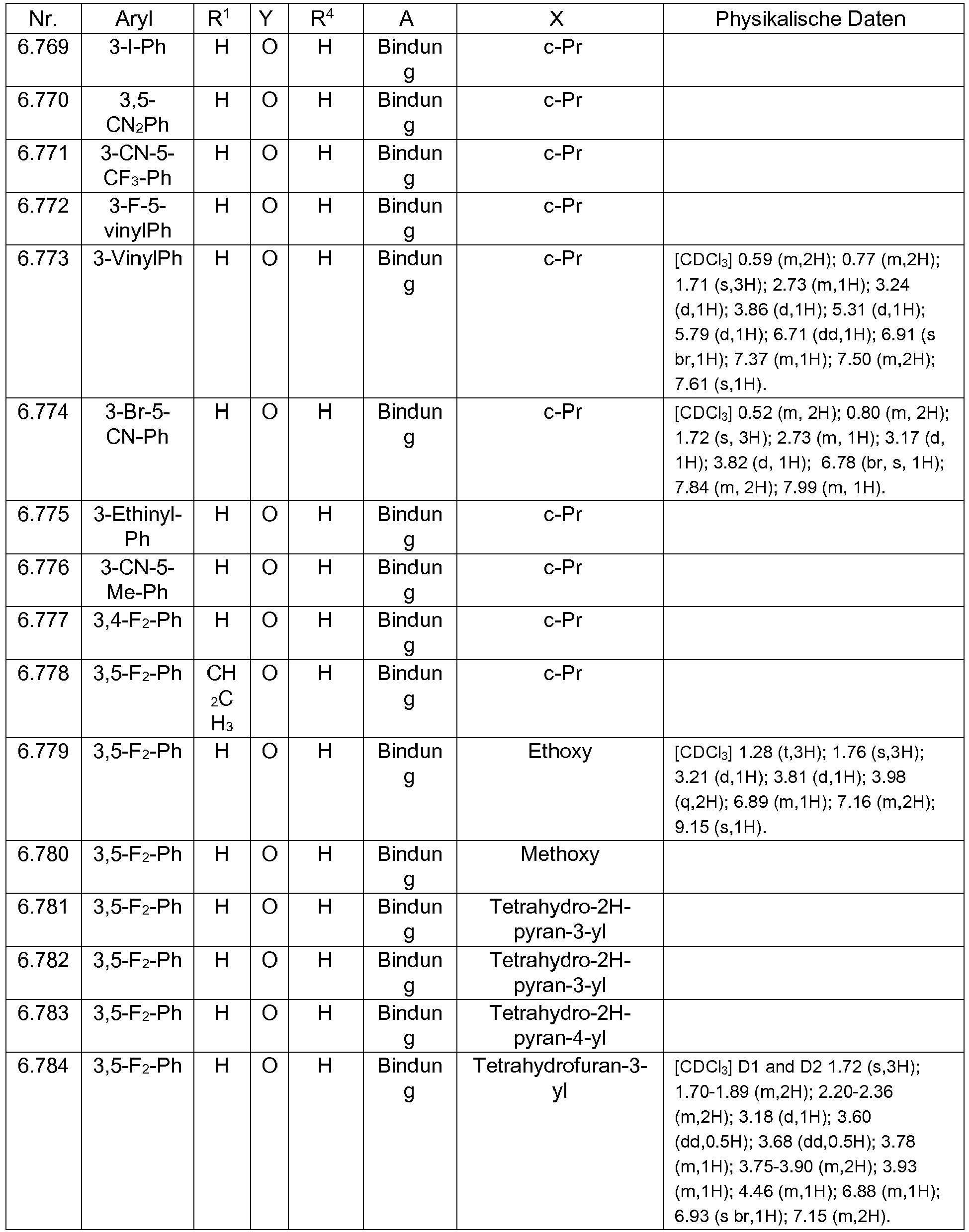

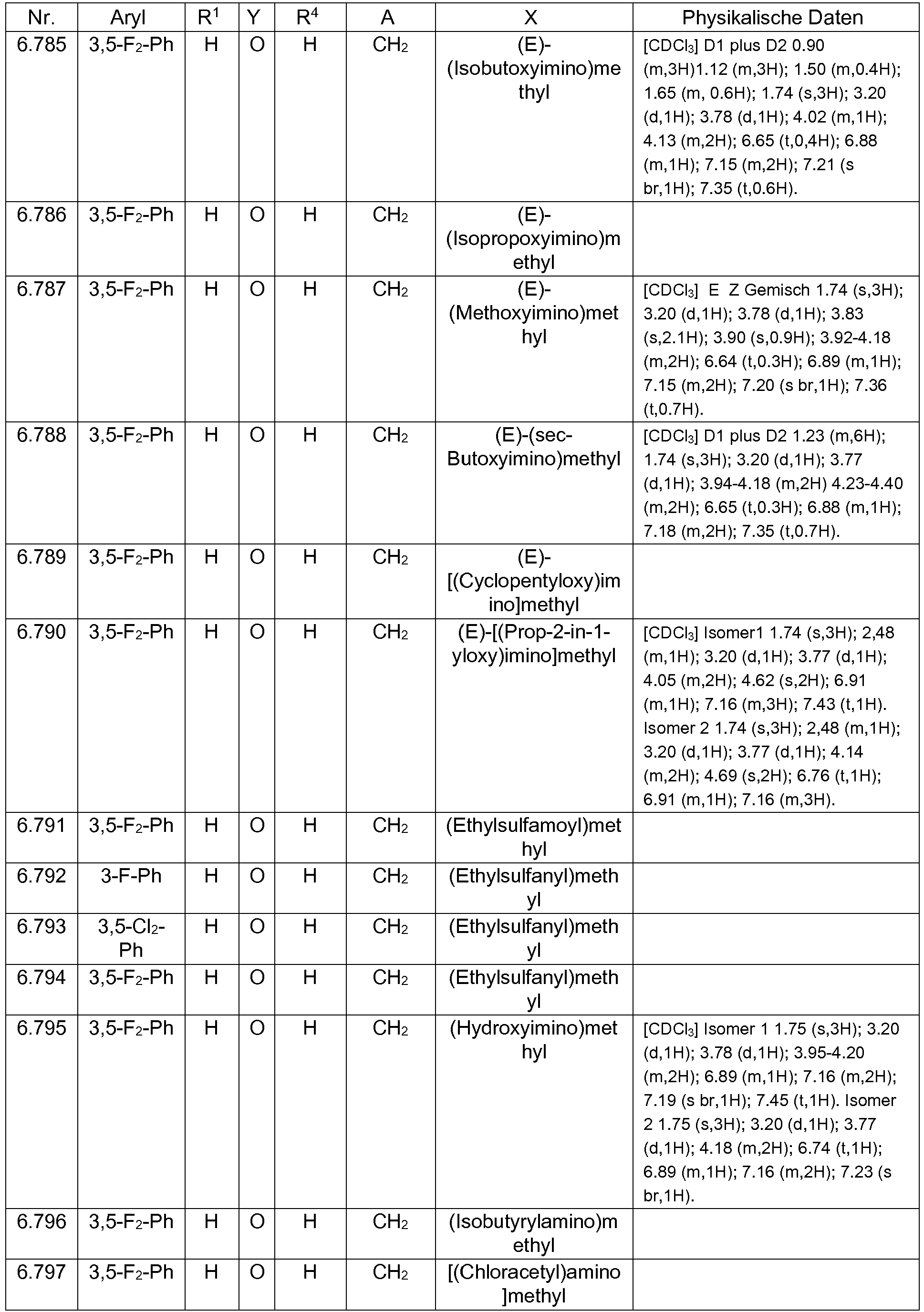

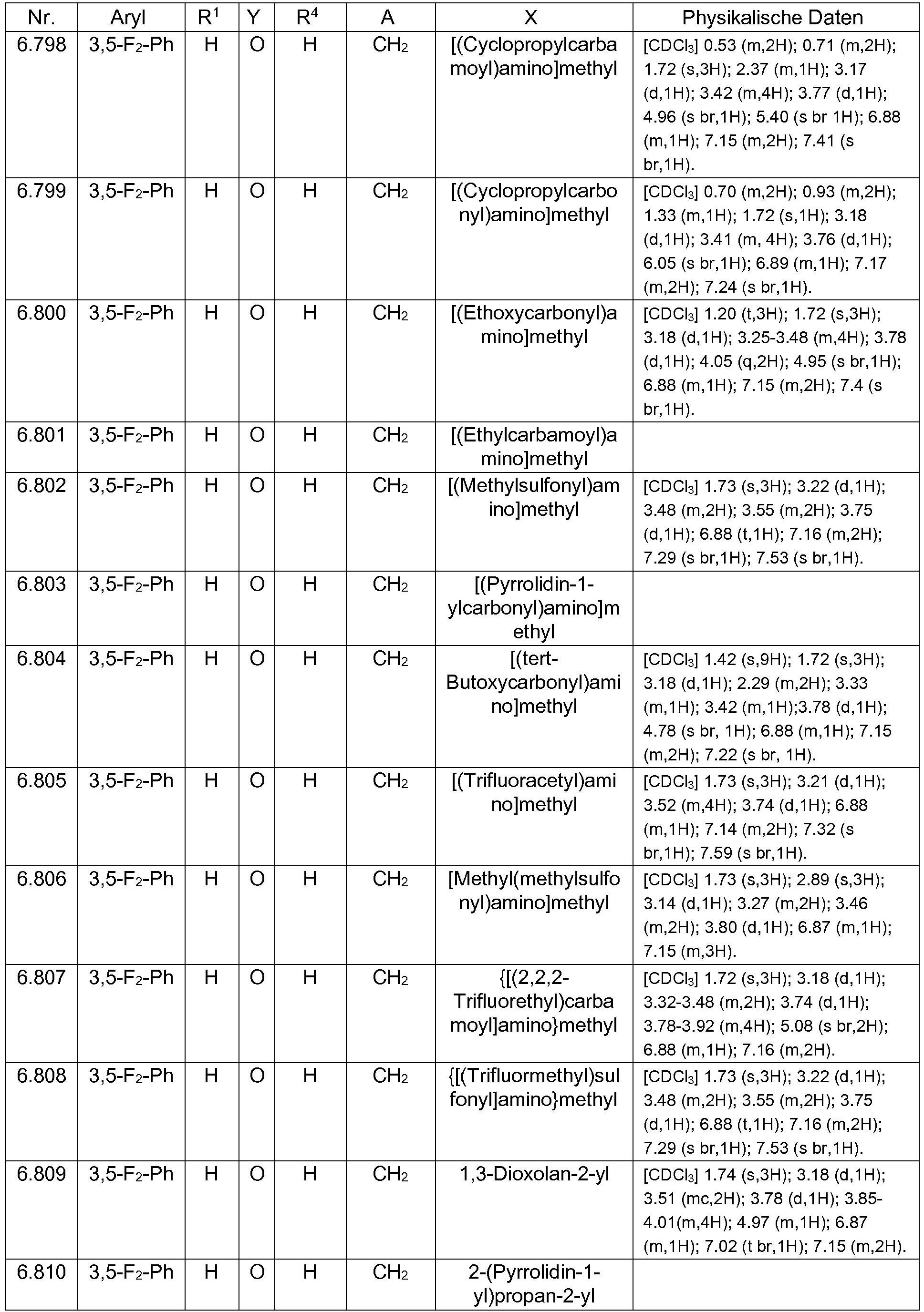

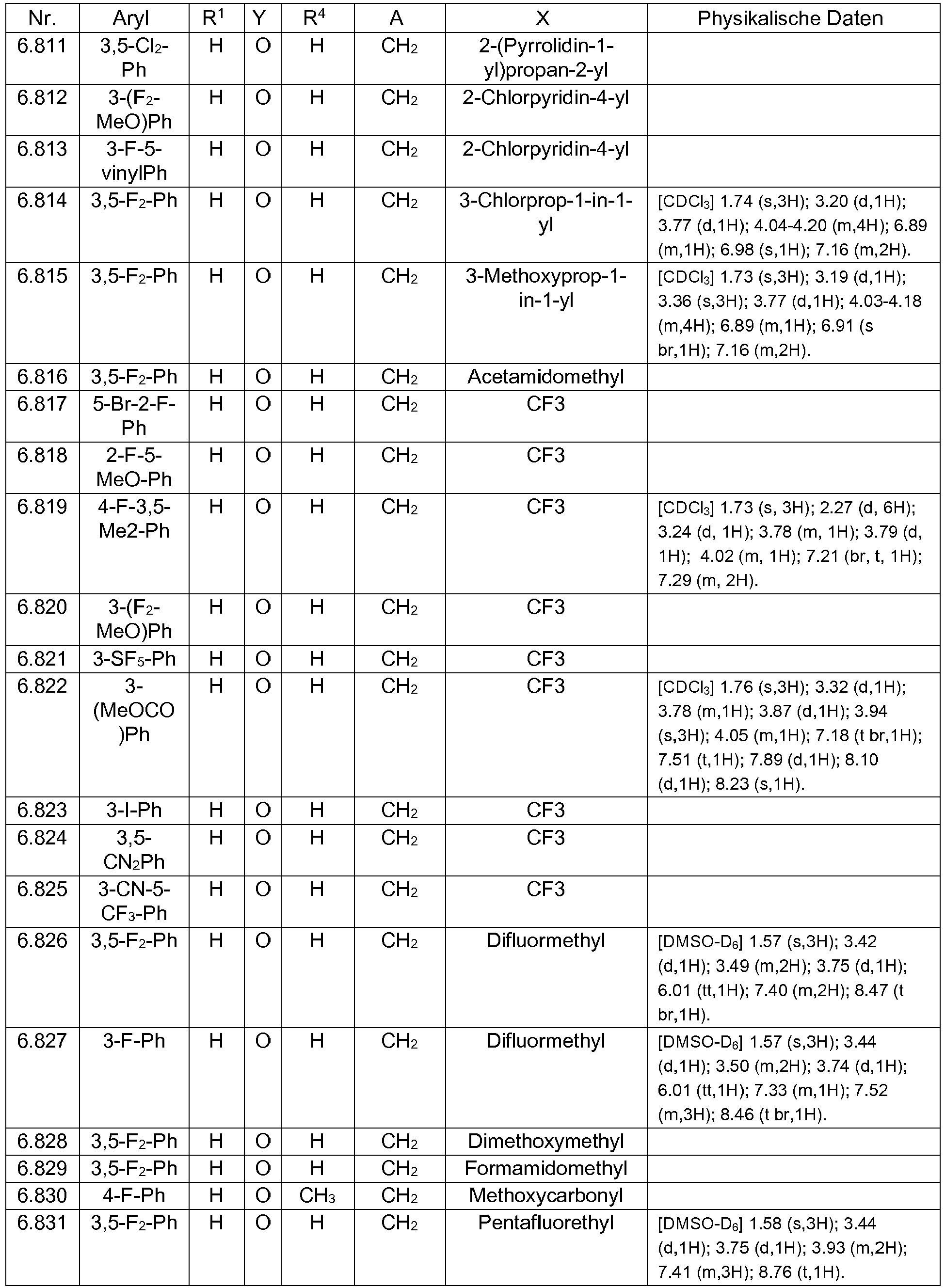

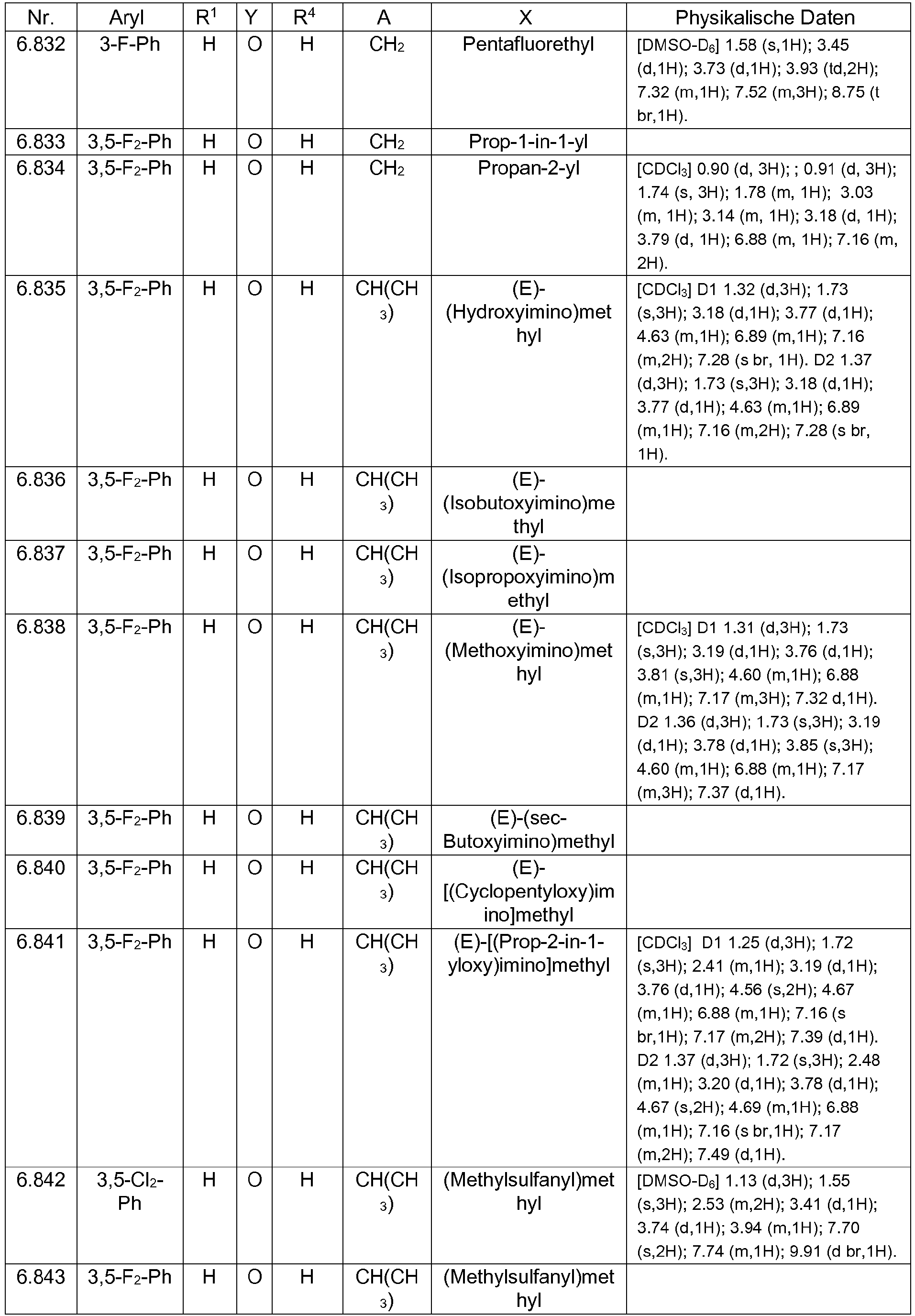

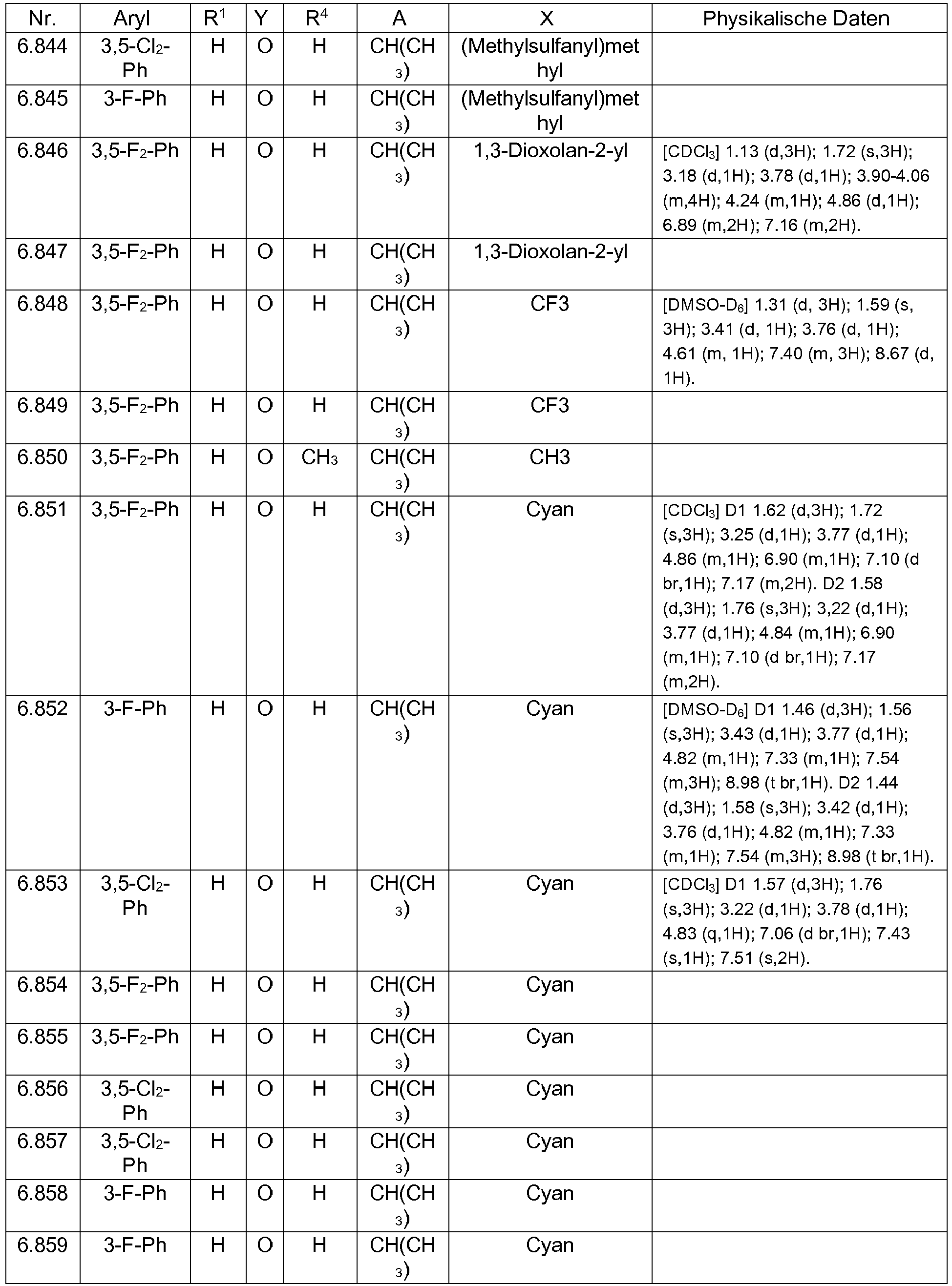

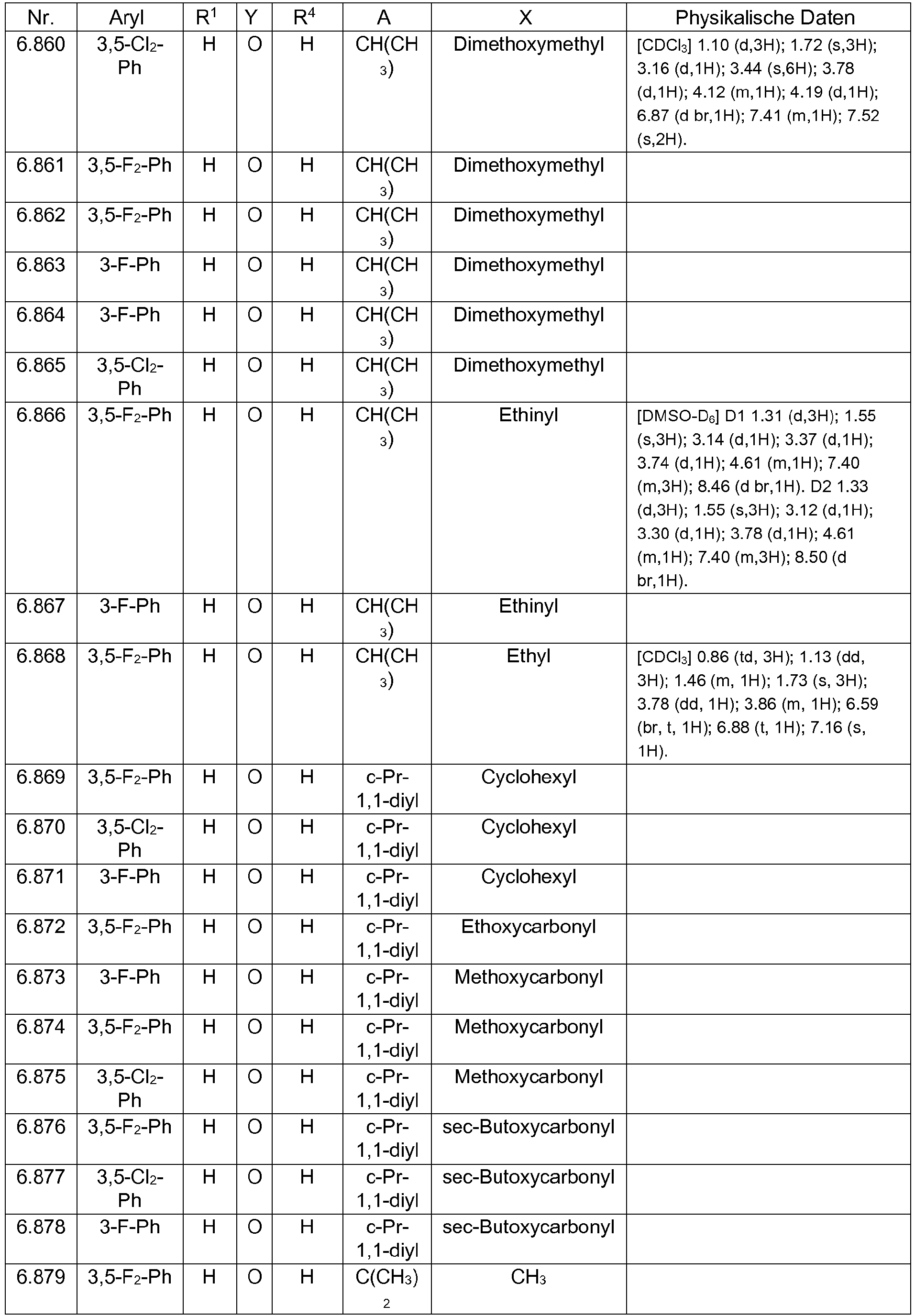

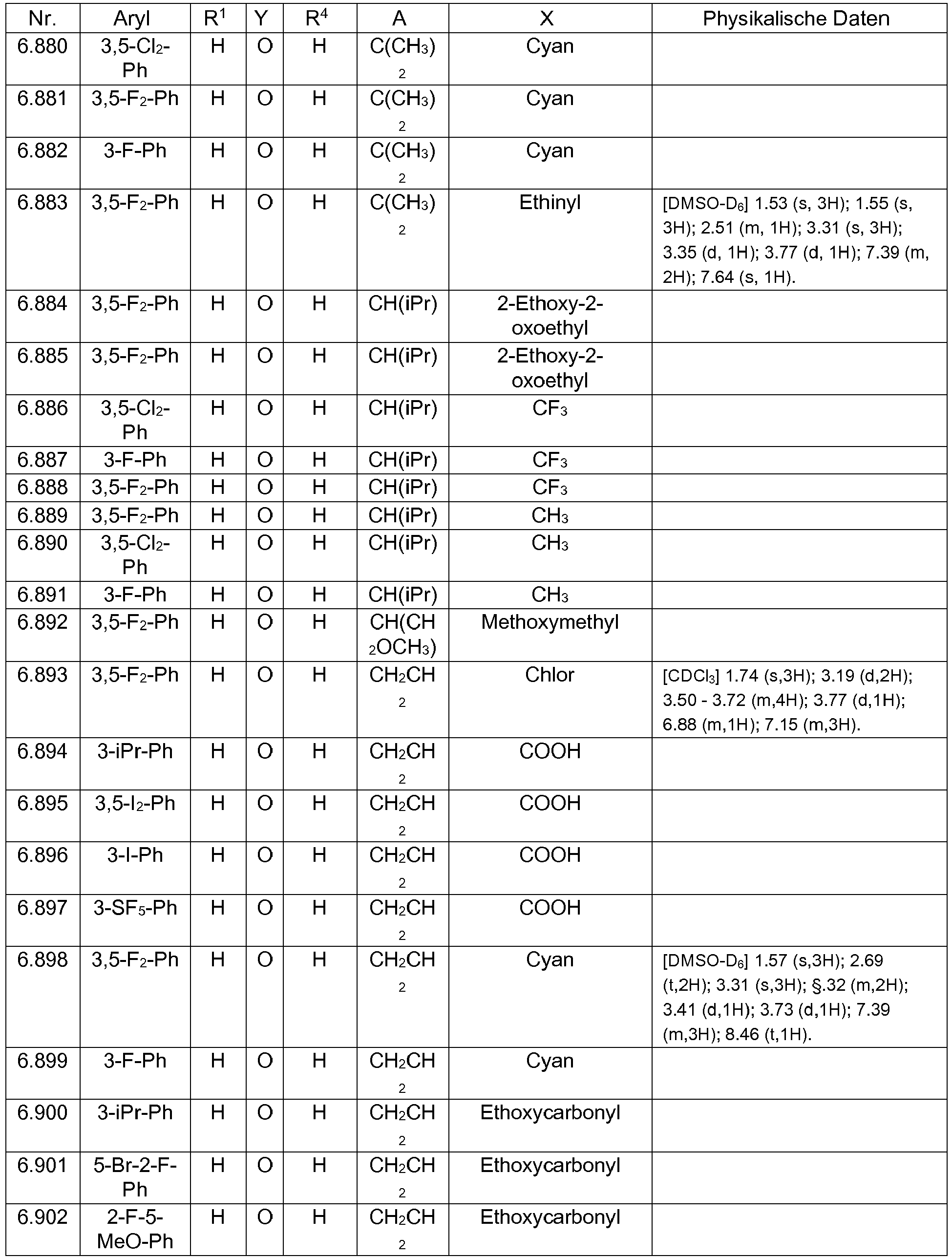

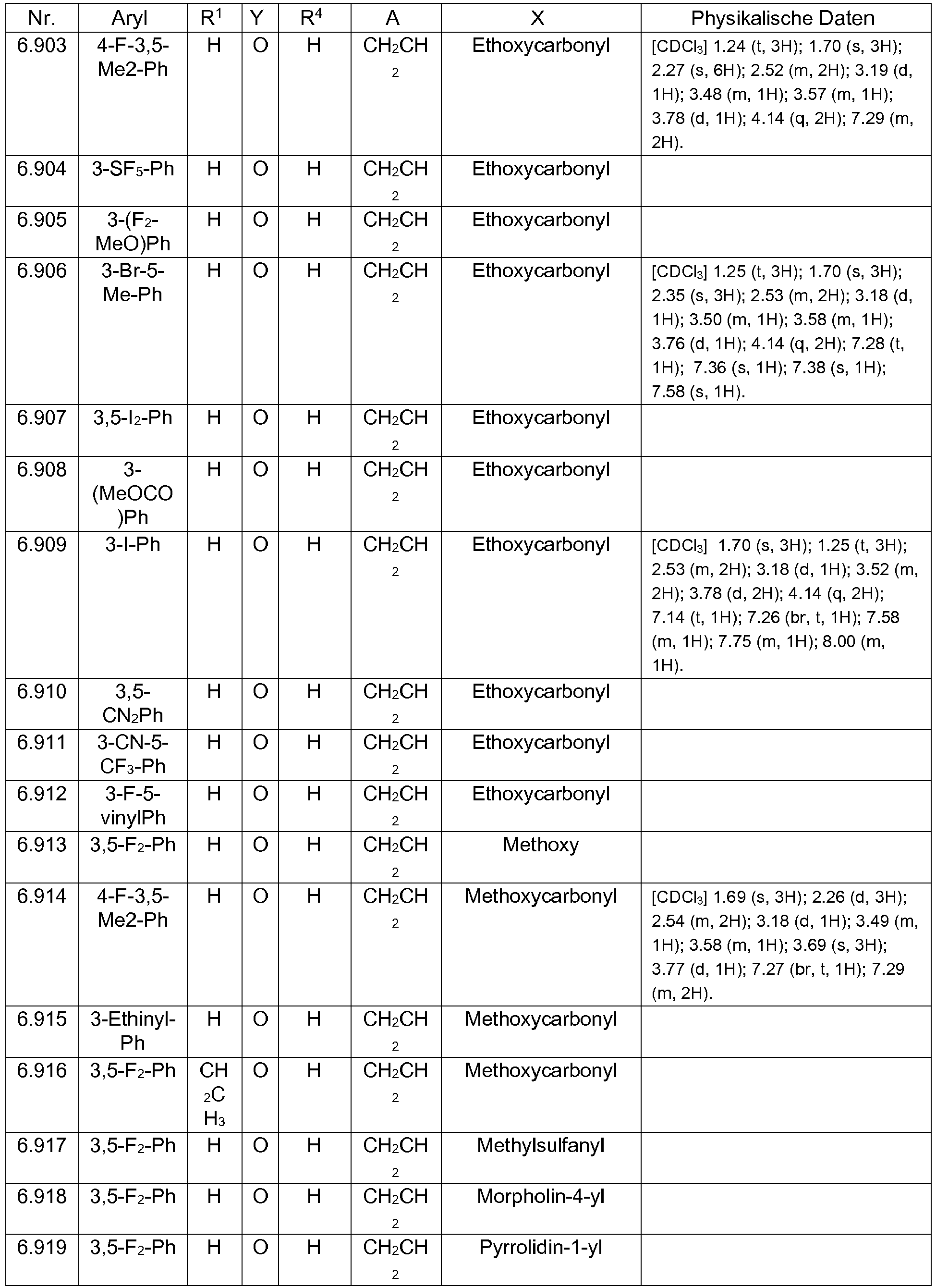

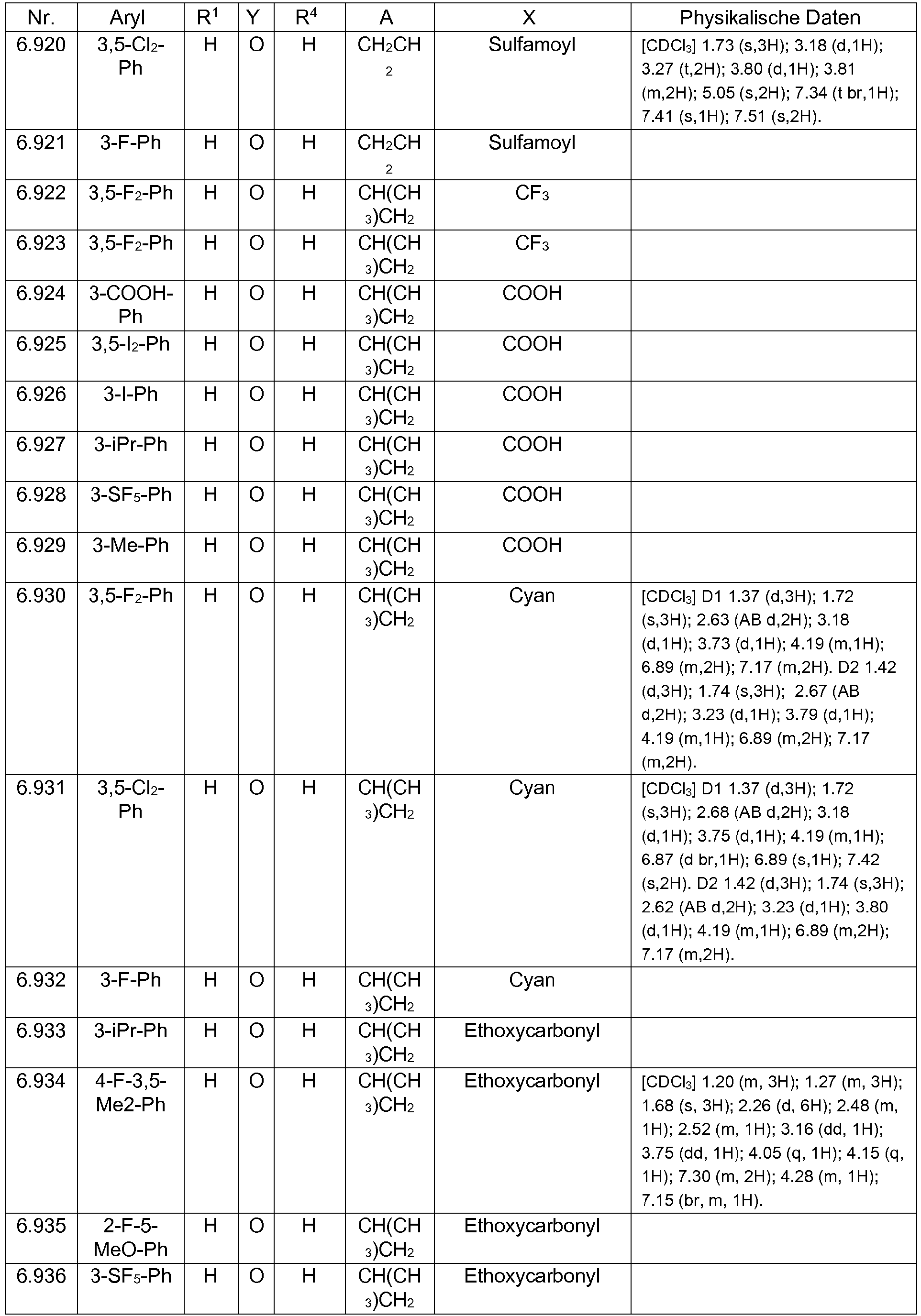

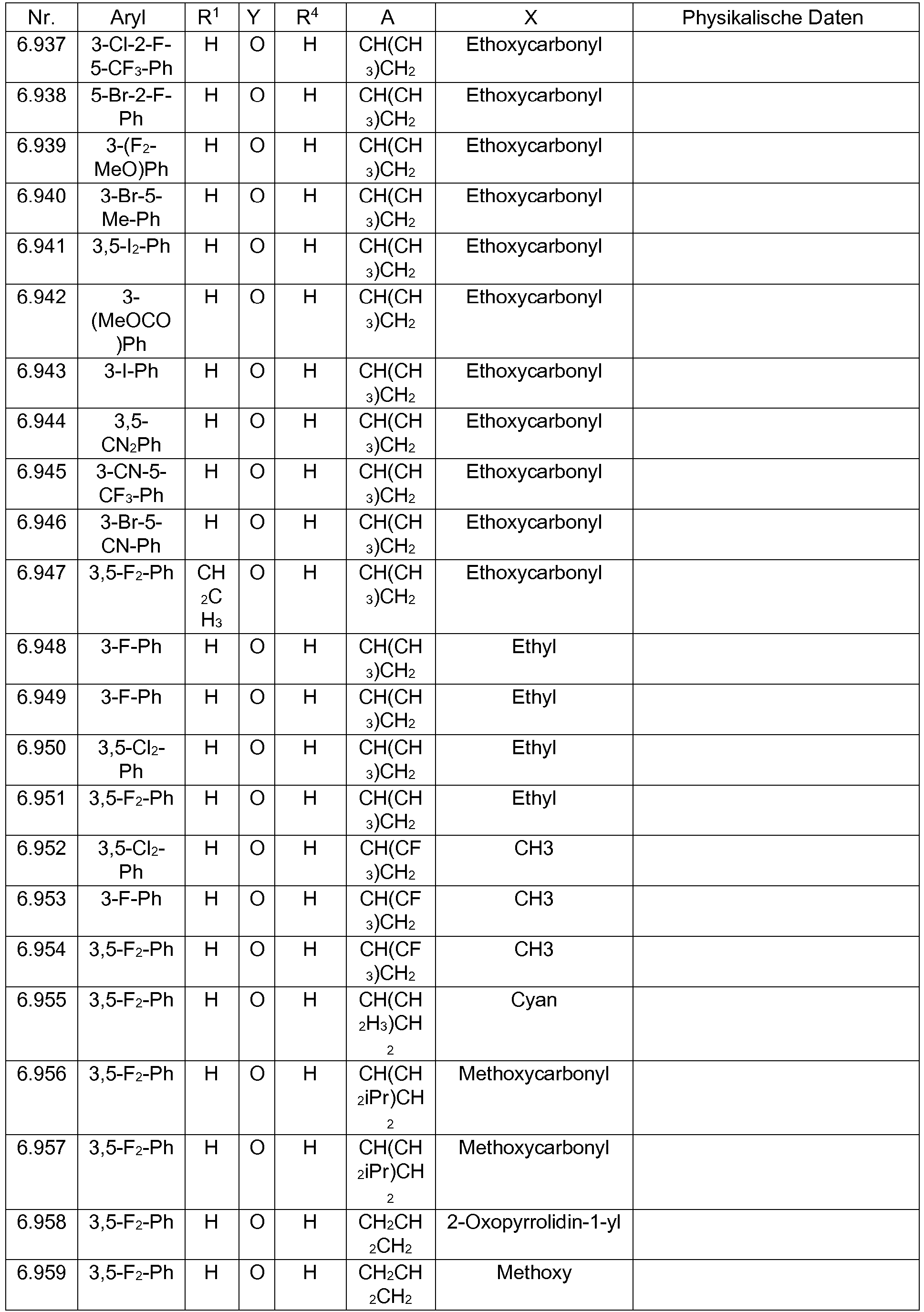

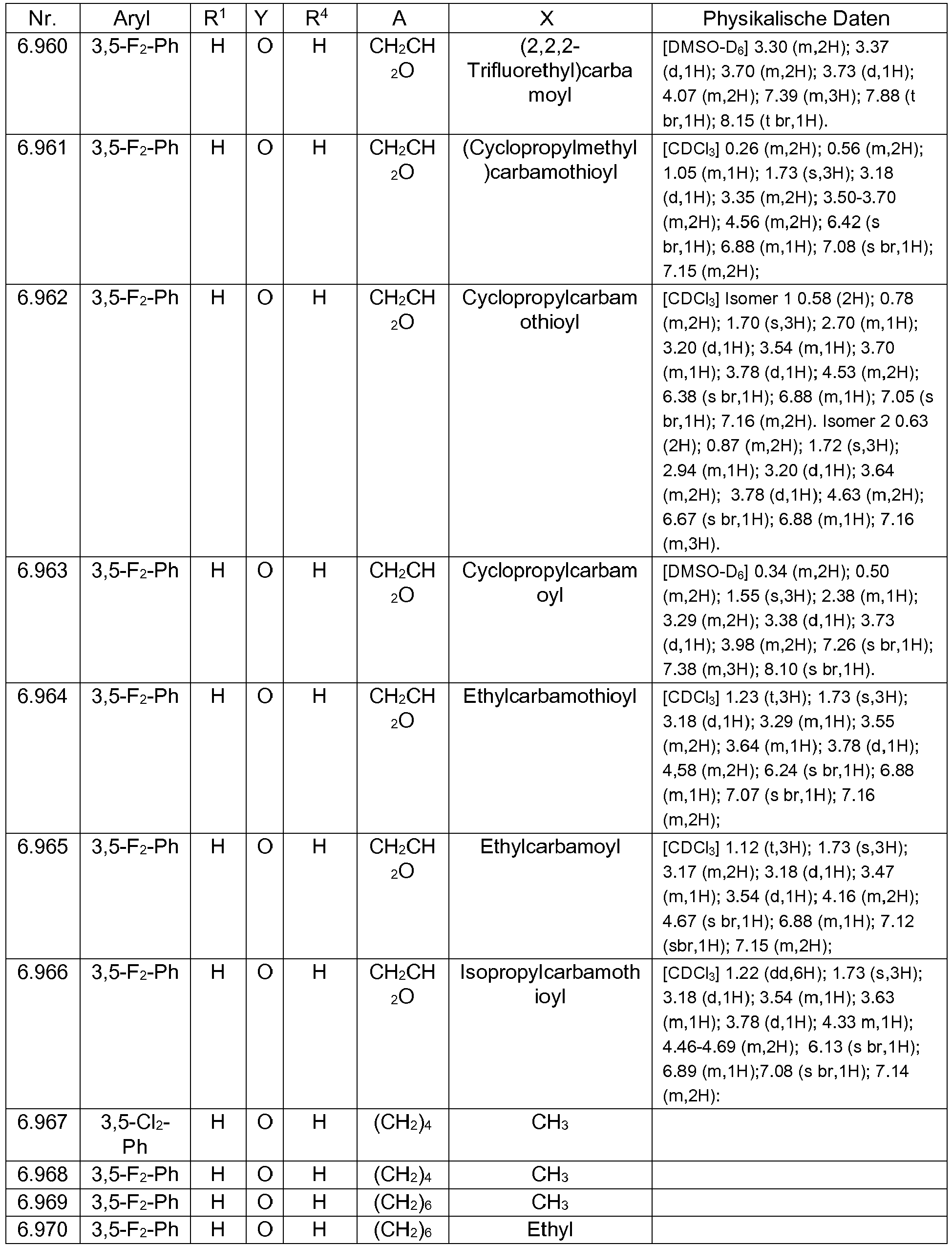

| Tabelle 7: Erfindungsgemäße Verbindungen der allgemeinen Formel (I), worin R² für Wasserstoff und R³ für Ethyl stehen, und Aryl den Rest 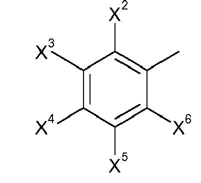 bedeutet. | | | | | | | |
|---|---|---|---|---|---|---|---|
| 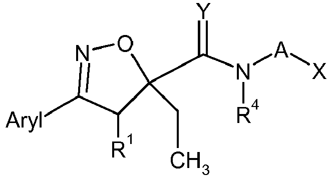 | | | | | | | |

| Nr. | Aryl | R¹ | Y | R⁴ | A | X | Physikalische Daten |
|---|---|---|---|---|---|---|---|
| 7.001 | 3,5-Cl₂-Ph | H | O | H | (CH₂)₄ | COOH | |
| 7.002 | 3,5-Cl₂-Ph | H | O | H | (CH₂)₄ | Ethoxycarbonyl | |
| 7.003 | 3-Br-5-CI-Ph | H | O | H | (CH₂)₄ | Ethoxycarbonyl | |
| 7.004 | Ph | H | O | H | (CH₂)₄ | Ethoxycarbonyl | |
| 7.005 | 3,5-Cl₂-Ph | H | O | H | (CH₂)₄ | Hydroxy | |
| 7.006 | 3,5-Cl₂-Ph | H | O | H | (CH₂)₄ | Methoxy | |
| 7.007 | 3,5-Cl₂-Ph | H | O | H | (CH₂)₆ | COOH | |
| 7.008 | 3,5-Cb-Ph | H | O | H | (CH₂)₆ | Ethoxycarbonyl | |
| 7.009 | 3,5-Cl₂-Ph | H | O | H | c-Pr-1,1-diyl | Ethoxycarbonyl | |
| 7.010 | 3,5-F₂-Ph | H | O | H | Bindung | 1-Cyancyclopropyl | |
| 7.011 | 3,5-Cl₂-Ph | H | O | H | Bindung | 1-Cyanocyclopropyl | |
| 7.012 | 3,5-Cl₂-Ph | H | O | H | Bindung | 1-Methylcyclopropyl | |
| 7.013 | 3,5-Cl₂-Ph | H | O | H | Bindung | 2-(Ethoxycarbonyl)cyclo hex-1-en-1-yl | |
| 7.014 | 3,5-Cl₂-Ph | H | O | H | Bindung | 2-(Ethoxycarbonyl)cyclo hexyl | |
| 7.015 | 3,5-Cl₂-Ph | H | O | H | Bindung | 2-(Methylcarbamoyl)cycl ohexyl | |
| 7.016 | 3,5-Cl₂-Ph | H | O | H | Bindung | 2-Carboxycyclohexyl | |
| 7.017 | 3,5-Cl₂-Ph | H | O | H | Bindung | 3-(2-Ethoxy-2-oxoethyl)oxetan-3-yl | |
| 7.018 | 3,5-F₂-Ph | H | O | H | Bindung | 3-(2-Ethoxy-2-oxoethyl)oxetan-3-yl | |
| 7.019 | 2,4-Cl₂-Ph | H | O | CH₃ | Bindung | CH3 | |
| 7.020 | 2-Cl-Ph | H | O | CH₃ | Bindung | CH3 | |
| 7.021 | 3,4-F₂-Ph | H | O | H | Bindung | CH3 | |
| 7.022 | 3,5-Br₂-Ph | H | O | H | Bindung | CH3 | |
| 7.023 | 3,5-Cl₂-Ph | CH₃ | O | H | Bindung | CH3 | |
| 7.024 | 3,5-Cl₂-Ph | H | O | CH₃ | Bindung | CH3 | |
| 7.025 | 3,5-Cl₂-Ph | H | O | H | Bindung | CH3 | |
| 7.026 | 3,5-F₂-Ph | H | O | H | Bindung | CH3 | |
| 7.027 | 3-CI-4-F-Ph | H | O | H | Bindung | CH3 | |
| 7.028 | 3,5-Cl₂-Ph | H | O | H | Bindung | Cyclobutyl | |
| 7.029 | 3,5-F₂-Ph | H | O | H | Bindung | Cyclobutyl | |
| 7.030 | 3,5-Cl₂-Ph | H | O | H | Bindung | Cyclopentyl | |
| 7.031 | 3,5-F₂-Ph | H | O | H | Bindung | Cyclopentyl | |
| 7.032 | 2-CF₃-Ph | H | O | H | Bindung | c-Pr | |
| 7.033 | 2,3,4-F₃-Ph | H | O | H | Bindung | c-Pr | |
| 7.034 | 2,3,5-F₃-Ph | H | O | H | Bindung | c-Pr | |
| 7.035 | 2,3-F₂-Ph | H | O | H | Bindung | c-Pr | |
| 7.036 | 2,4-Cl₂-Ph | H | O | H | Bindung | c-Pr | |
| 7.037 | 2,5-F₂-Ph | H | O | H | Bindung | c-Pr | |
| 7.038 | 2,5-Me₂-Ph | H | O | H | Bindung | c-Pr | |
| 7.039 | 2-CI-3-F-5-MeO-Ph | H | O | H | Bindung | c-Pr | |
| 7.040 | 2-CI-5-F-Ph | H | O | H | Bindung | c-Pr | |
| 7.041 | 2-EtO-3,4,5,6-F₄-Ph | H | O | H | Bindung | c-Pr | |
| 7.042 | 2-F-3-Me-Ph | H | O | H | Bindung | c-Pr | |
| 7.043 | 3-(2-MeOEtO)-Ph | H | O | H | Bindung | c-Pr | |
| 7.044 | 3-iPrO-Ph | H | O | H | Bindung | c-Pr | |
| 7.045 | 3-CF₃O-Ph | H | O | H | Bindung | c-Pr | |
| 7.046 | 3-CF₃-Ph | H | O | H | Bindung | c-Pr | |
| 7.047 | 3,4,5-F₃-Ph | H | O | H | Bindung | c-Pr | |
| 7.048 | 3,5-Br₂-Ph | H | O | H | Bindung | c-Pr | |
| 7.049 | 3,5-Cl₂-4-MeO-Ph | H | O | H | Bindung | c-Pr | |
| 7.050 | 3,5-Cl₂-4-OH-Ph | H | O | H | Bindung | c-Pr | |
| 7.051 | 3,5-Cl₂-Ph | CH₃ | O | H | Bindung | c-Pr | |
| 7.052 | 3,5-Cl₂-Ph | H | O | H | Bindung | c-Pr | [CDCl₃] 0.52 (m, 2H); 0.80 (m, 2H); 0.98 (t, 3H); 1.94 (m, 1H); 2.14 (m, 1H); 2.23 (m, 1H); 3.18 (d, 1H); 3.70 (d, 1H); 6.82 (s, 1H); 7.40 (t, 1H), 7.50 (d, 1H) |
| 7.053 | 3,5-F₂-Ph | H | O | H | Bindung | c-Pr | |
| 7.054 | 3,5-F₂-Ph | H | O | H | Bindung | c-Pr | |
| 7.055 | 3,5-F₂-Ph | H | S | H | Bindung | c-Pr | |
| 7.056 | 3,5-(MeO)₂-Ph | H | O | H | Bindung | c-Pr | |
| 7.057 | 3,5-Me₂-Ph | H | O | H | Bindung | c-Pr | |
| 7.058 | 3-Ac-Ph | H | O | H | Bindung | c-Pr | |
| 7.059 | 3-Br-5-CF₃-Ph | H | O | H | Bindung | c-Pr | |
| 7.060 | 3-Br-5-CI-Ph | H | O | H | Bindung | c-Pr | |
| 7.061 | 3-Br-5-F-Ph | H | O | H | Bindung | c-Pr | |
| 7.062 | 3-CI-4-F-Ph | H | O | H | Bindung | c-Pr | |
| 7.063 | 3-Cl-4-Me-Ph | H | O | H | Bindung | c-Pr | |
| 7.064 | 3-Cl-5-CF₃-Ph | H | O | H | Bindung | c-Pr | |
| 7.065 | 3-Cl-5-CN-Ph | H | O | H | Bindung | c-Pr | |
| 7.066 | 3-CI-5-Et-Ph | H | O | H | Bindung | c-Pr | |
| 7.067 | 3-CI-5-F-Ph | H | O | H | Bindung | c-Pr | |
| 7.068 | 3-Cl-5-Me-Ph | H | O | H | Bindung | c-Pr | |
| 7.069 | 3-CI-5-CF₃O-Ph | H | O | H | Bindung | c-Pr | |
| 7.070 | 3-CI-Ph | H | O | H | Bindung | c-Pr | |
| 7.071 | 3-CN-5-F-Ph | H | O | H | Bindung | c-Pr | |
| 7.072 | 3-c-Pr-5-F-Ph | H | O | H | Bindung | c-Pr | |
| 7.073 | 3-EtO-Ph | H | O | H | Bindung | c-Pr | |
| 7.074 | 3-Et-5-F-Ph | H | O | H | Bindung | c-Pr | |
| 7.075 | 3-Et-Ph | H | O | H | Bindung | c-Pr | |
| 7.076 | 3-F-5-MeS-Ph | H | O | H | Bindung | c-Pr | |
| 7.077 | 3-F-5-MeSO₂-Ph | H | O | H | Bindung | c-Pr | |
| 7.078 | 3-F-5-MeO-Ph | H | O | H | Bindung | c-Pr | |
| 7.079 | 3-F-5-Me-Ph | H | O | H | Bindung | c-Pr | |
| 7.080 | 3-F-5-Me-Ph | H | S | H | Bindung | c-Pr | |
| 7.081 | 3-F-Ph | H | O | H | Bindung | c-Pr | [CDCl3] 0.54 (m,2H); 0.80 (m,2H); 1.01 (t,3H); 1.94 (m,1H); 2.13 (m,1H); 2.75 (m,1H); 3.23 (d,1H); 3.73 (d,1H); 6.87 (s br,1H); 7.14 (m,1H); 7.39 (m,3H). |
| 7.082 | 3-OH-Ph | H | O | H | Bindung | c-Pr | |
| 7.083 | 3-iPr-Ph | H | O | H | Bindung | c-Pr | |
| 7.084 | 3-MeO-Ph | H | O | H | Bindung | c-Pr | |
| 7.085 | 3-Me-5-CF₃O-Ph | H | O | H | Bindung | c-Pr | |
| 7.086 | 3-Me-Ph | H | O | H | Bindung | c-Pr | |
| 7.087 | 3-NO₂-Ph | H | O | H | Bindung | c-Pr | |
| 7.088 | 4-Cl-3,5-F₂-Ph | H | O | H | Bindung | c-Pr | |
| 7.089 | 4-EtO-Ph | H | O | H | Bindung | c-Pr | |
| 7.090 | F₅-Ph | H | O | H | Bindung | c-Pr | |
| 7.091 | Ph | H | O | H | Bindung | c-Pr | |
| 7.092 | 3,5-Cl₂-Ph | H | O | H | Bindung | Decahydronaphthalen -2-yl | |
| 7.093 | 3,5-Cl₂-Ph | H | O | H | Bindung | H | |
| 7.094 | 3,5-F₂-Ph | H | O | H | Bindung | H | |
| 7.095 | 3,5-F₂-Ph | H | S | H | Bindung | H | |
| 7.096 | 3-F-Ph | H | O | H | Bindung | H | |
| 7.097 | 3-Me-Ph | H | O | H | Bindung | H | |
| 7.098 | 3-Me-Ph | H | S | H | Bindung | H | |
| 7.099 | 3,5-Cl₂-Ph | H | O | CH₃ | Bindung | Hydroxy | |
| 7.100 | 2,3,4-F₃-Ph | H | O | H | Bindung | Oxetan-3-yl | |
| 7.101 | 2,3-F₂-Ph | H | O | H | Bindung | Oxetan-3-yl | |
| 7.102 | 2,5-F₂-Ph | H | O | H | Bindung | Oxetan-3-yl | |
| 7.103 | 3,5-Cl₂-Ph | H | O | H | Bindung | Oxetan-3-yl | |
| 7.104 | 3,5-F₂-Ph | H | O | H | Bindung | Oxetan-3-Yl | |
| 7.105 | 3-CI-5-CF₃O-Ph | H | O | H | Bindung | Oxetan-3-Yl | |
| 7.106 | 3-F-5-Me-Ph | H | O | H | Bindung | Oxetan-3-yl | |
| 7.107 | 3-F-Ph | H | O | H | Bindung | Oxetan-3-yl | |
| 7.108 | 3-NO₂-Ph | H | O | H | Bindung | Oxetan-3-yl | |
| 7.109 | Ph | H | O | H | Bindung | Oxetan-3-yl | |
| 7.110 | 3,5-Cl₂-Ph | H | O | H | Bindung | Tetrahydro-2H-pyran-4-yl | |
| 7.111 | 3,5-Cl₂-Ph | H | O | H | C(CH₃)₂ | 3-Methoxyprop-1-in-1-yl | |
| 7.112 | 3,5-Cl₂-Ph | H | O | H | C(CH₃)₂ | Carbamoyl | |
| 7.113 | 3,5-Cl₂-Ph | H | O | H | C(CH₃)₂ | CH3 | |
| 7.114 | 3-CI-4-F-Ph | H | O | H | C(CH₃)₂ | CH3 | |
| 7.115 | 3,5-Cl₂-Ph | H | O | H | C(CH₃)₂ | COOH | |
| 7.116 | 3,5-(CF₃)₂-Ph | H | O | H | C(CH₃)₂ | Ethinyl | |
| 7.117 | 3,5-Cl₂-Ph | H | O | H | C(CH₃)₂ | Ethinyl | |
| 7.118 | 3,5-Cl₂-Ph | H | O | H | C(CH₃)₂ | Methoxycarbonyl | |
| 7.119 | 3,5-Cl₂-Ph | H | O | H | C(CH₃)₂ | Methylcarbamoyl | |
| 7.120 | 3-CI-Ph | H | O | H | C(CH₃)₂CH₂ | (Propan-2-yloxy)carbonyl | |
| 7.121 | 3-F-Ph | H | O | H | C(CH₃)₂CH₂ | (Propan-2-yloxy)carbonyl | |
| 7.122 | 3,5-Cl₂-Ph | H | O | H | C(CH₃)₂CH₂ | COOH | |
| 7.123 | 3-CI-Ph | H | O | H | C(CH₃)₂CH₂ | COOH | |
| 7.124 | 3-F-Ph | H | O | H | C(CH₃)₂CH₂ | COOH | |
| 7.125 | 3,5-Cl₂-Ph | H | O | H | C(CH₃)₂CH₂ | Ethoxycarbonyl | |
| 7.126 | 3-CI-Ph | H | O | H | C(CH₃)₂CH₂ | Ethoxycarbonyl | |
| 7.127 | 3-F-Ph | H | O | H | C(CH₃)₂CH₂ | Ethoxycarbonyl | |
| 7.128 | 3,5-Cl₂-Ph | H | O | H | C(CH₃)₂CH₂ | Hydroxy | |
| 7.129 | 3-CI-Ph | H | O | H | C(CH₃)₂CH₂ | Methoxycarbonyl | |
| 7.130 | 3-F-Ph | H | O | H | C(CH₃)₂CH₂ | Methoxycarbonyl | |
| 7.131 | 3,5-Cl₂-Ph | H | O | H | C(CH₃)₂CH₂ | Methylsulfanyl | |
| 7.132 | 3,5-Cl₂-Ph | H | O | H | C(CH₃)₂CH₂ | Methylsulfonyl | |
| 7.133 | 2,4-Cl₂-Ph | H | O | H | C(iPr)CH₃ | Cyan | |
| 7.134 | 3,5-Cl₂-Ph | H | O | H | C(iPr)CH₃ | Cyan | |
| 7.135 | 3,5-Cl₂-Ph | H | O | H | CH(CF₃)CH₂ | Ethoxycarbonyl | |
| 7.136 | 3,5-Cl₂-Ph | H | O | H | CH(CH₂CH₂S CH₃) | Methoxycarbonyl | |
| 7.137 | 3,5-Cl₂-Ph | H | O | H | CH(CH₂CH₃) CH₂ | Cyan | |
| 7.138 | 3,5-Cl₂-Ph | H | O | H | CH(CH₂CH₃) CH₂ | Methoxycarbonyl | |
| 7.139 | 3,5-Cl₂-Ph | H | O | H | CH(CH₂iPr)C H₂ | Methoxycarbonyl | |
| 7.140 | 3,5-Cl₂-Ph | H | O | H | CH(CH₂OCH₃ ) | Methoxymethyl | |
| 7.141 | 3,5-Cl₂-Ph | H | O | H | CH(CH₃) | CF3 | |
| 7.142 | 2,3,4-F₃-Ph | H | O | H | CH(CH₃) | CH3 | |
| 7.143 | 2,3,5-F₃-Ph | H | O | H | CH(CH₃) | CH3 | |
| 7.144 | 2,3,6-Cl₃-Ph | H | O | H | CH(CH₃) | CH3 | |
| 7.145 | 2,3-Cl₂-5-MeO-Ph | H | O | H | CH(CH₃) | CH3 | |
| 7.146 | 2,3-Cl₂-Ph | H | O | H | CH(CH₃) | CH3 | |
| 7.147 | 2,3-F₂-Ph | H | O | H | CH(CH₃) | CH3 | |
| 7.148 | 2,5-Cl₂-Ph | H | O | H | CH(CH₃) | CH3 | |
| 7.149 | 2,5-F₂-Ph | H | O | H | CH(CH₃) | CH3 | |
| 7.150 | 2-F-Ph | H | O | H | CH(CH₃) | CH3 | |
| 7.151 | 3-(CF₃CH₂O)-Ph | H | O | H | CH(CH₃) | CH3 | |
| 7.152 | 3-(ClCH₂CH₂ O)-Ph | H | O | H | CH(CH₃) | CH3 | |
| 7.153 | 3-(2-MeOEtO)-Ph | H | O | H | CH(CH₃) | CH3 | |
| 7.154 | 3-Me₂N-Ph | H | O | H | CH(CH₃) | CH3 | |
| 7.155 | 3-iPrCOO-Ph | H | O | H | CH(CH₃) | CH3 | |
| 7.156 | 3-iPrO-Ph | H | O | H | CH(CH₃) | CH3 | |
| 7.157 | 3-CF₃O-Ph | H | O | H | CH(CH₃) | CH3 | |
| 7.158 | 3,4-Cl₂-Ph | H | O | H | CH(CH₃) | CH3 | |
| 7.159 | 3,5-(CF₃)₂-Ph | H | O | H | CH(CH₃) | CH3 | |
| 7.160 | 3,5-Cl₂-Ph | CH₃ | O | H | CH(CH₃) | CH3 | |
| 7.161 | 3,5-Cl₂-Ph | H | O | H | CH(CH₃) | CH3 | [COCl₃] 1.00 (t, 3H); 1.13 (d, 3H); 1.18 (d, 3H); 1.93 (m, 1H); 2.14 (m, 1H); 3.18 (d, 1H); 3.69 (d, 1H); 4.05 (m, 1H); 6.62 (d, 1H); 7.40 (t, 1H); 7.50 (d, 1H) |
| 7.162 | 3,5-Cl2-Ph | H | O | OH | CH(CH₃) | CH3 | |
| 7.163 | 3,5-Cl₂-Ph | H | S | H | CH(CH₃) | CH3 | |
| 7.164 | 3,5-F₂-Ph | H | O | H | CH(CH₃) | CH3 | |
| 7.165 | 3,5-Me₂-Ph | H | O | H | CH(CH₃) | CH3 | |
| 7.166 | R002 | H | O | H | CH(CH₃) | CH3 | |
| 7.167 | R003 | H | O | H | CH(CH₃) | CH3 | |
| 7.168 | 3-CNCH₂N(M e)-Ph | H | O | H | CH(CH₃) | CH3 | |
| 7.169 | 3-Me₂NCONH -Ph | H | O | H | CH(CH₃) | CH3 | |
| 7.170 | 3-Me₂NSO₂O-Ph | H | O | H | CH(CH₃) | CH3 | |
| 7.171 | 3-EtNHCOO-Ph | H | O | H | CH(CH₃) | CH3 | |
| 7.172 | 3-EtSO₂O-Ph | H | O | H | CH(CH₃) | CH3 | |
| 7.173 | 3-MeSO₂NH-Ph | H | O | H | CH(CH₃) | CH3 | |
| 7.174 | 3-MeSO₂O-Ph | H | O | H | CH(CH₃) | CH3 | |
| 7.175 | 3-tert.BuOCO NH-Ph | H | O | H | CH(CH₃) | CH3 | |
| 7.176 | 3-CF₃CONH-Ph | H | O | H | CH(CH₃) | CH3 | |
| 7.177 | 3-AcO-5-Cl-Ph | H | O | H | CH(CH₃) | CH3 | |
| 7.178 | 3-AcO-Ph | H | O | H | CH(CH₃) | CH3 | |
| 7.179 | 3-NH₂-Ph | H | O | H | CH(CH₃) | CH3 | |
| 7.180 | 3-Br-5-CF₃-Ph | H | O | H | CH(CH₃) | CH3 | |
| 7.181 | 3-Br-5-CI-Ph | H | O | H | CH(CH₃) | CH3 | |
| 7.182 | 3-CI-4-F-Ph | H | O | H | CH(CH₃) | CH3 | |
| 7.183 | 3-Cl-4-Me-Ph | H | O | H | CH(CH₃) | CH3 | |
| 7.184 | 3-Cl-5-CF₃-Ph | H | O | H | CH(CH₃) | CH3 | |
| 7.185 | R001 | H | O | H | CH(CH₃) | CH3 | |
| 7.186 | 3-CI-5-F-Ph | H | O | H | CH(CH₃) | CH3 | |
| 7.187 | 3-CI-5-MeO-Ph | H | O | H | CH(CH₃) | CH3 | |
| 7.188 | 3-Cl-5-Me-Ph | H | O | H | CH(CH₃) | CH3 | |
| 7.189 | 3-CI-5-(CF₃CH₂O)-Ph | H | O | H | CH(CH₃) | CH3 | |
| 7.190 | 3-CI-5-(ClCH₂CH₂ O)-Ph | H | O | H | CH(CH₃) | CH3 | |
| 7.191 | 3-CI-5-(EtOCOCH₂ O)-Ph | H | O | H | CH(CH₃) | CH3 | |
| 7.192 | 3-CI-5-CF₃O-Ph | H | O | H | CH(CH₃) | CH3 | |
| 7.193 | 3-CI-5-Me₂NSO₂O-Ph | H | O | H | CH(CH₃) | CH3 | |
| 7.194 | 3-CI-5-(MeSO₂O)-Ph | H | O | H | CH(CH₃) | CH3 | |
| 7.195 | 3-Cl-5-iPrO-Ph | H | O | H | CH(CH₃) | CH3 | |
| 7.196 | 3-CI-Ph | H | O | H | CH(CH₃) | CH3 | |
| 7.197 | 3-EtO-Ph | H | O | H | CH(CH₃) | CH3 | |
| 7.198 | 3-F-5-Me-Ph | H | O | H | CH(CH₃) | CH3 | |
| 7.199 | 3-F-Ph | H | O | H | CH(CH₃) | CH3 | |
| 7.200 | 3-OH-Ph | H | O | H | CH(CH₃) | CH3 | |
| 7.201 | 3-MeO-Ph | H | O | H | CH(CH₃) | CH3 | |
| 7.202 | 3-Me-Ph | H | O | H | CH(CH₃) | CH3 | |
| 7.203 | 3-NO₂-Ph | H | O | H | CH(CH₃) | CH3 | |
| 7.204 | F₅-Ph | H | O | H | CH(CH₃) | CH3 | |
| 7.205 | Ph | H | O | H | CH(CH₃) | CH3 | |
| 7.206 | 3,5-Cl₂-Ph | H | O | H | CH(CH₃) | COOH | |
| 7.207 | 3-CI-4-F-Ph | H | O | H | CH(CH₃) | COOH | |
| 7.208 | 3,5-Cl₂-Ph | H | O | H | CH(CH₃) | c-Pr | |
| 7.209 | 3,5-F₂-Ph | H | O | H | CH(CH₃) | c-Pr | |
| 7.210 | Ph | H | O | H | CH(CH₃) | c-Pr | |
| 7.211 | 3-CI-4-F-Ph | H | O | H | CH(CH₃) | Dimethylcarbamoyl | |
| 7.212 | 3,5-Cl₂-Ph | H | O | H | CH(CH₃) | Ethinyl | |
| 7.213 | 3,5-Cl₂-Ph | H | O | H | CH(CH₃) | Ethyl | |
| 7.214 | 3,5-Cl₂-Ph | CH₃ | O | H | CH(CH₃) | Methoxycarbonyl | |
| 7.215 | 3,5-Cl₂-Ph | H | O | H | CH(CH₃) | Methoxycarbonyl | |
| 7.216 | 3-CI-4-F-Ph | H | O | H | CH(CH₃) | Methoxycarbonyl | |
| 7.217 | 3-CI-4-F-Ph | H | O | H | CH(CH₃) | Methylcarbamoyl | |
| 7.218 | 3,5-Cl₂-Ph | H | O | H | CH(CH₃) | Pentyl | |
| 7.219 | 3,5-Cl₂-Ph | H | O | H | CH(CH₃)CH₂ | (2,2,2-Trifluoroethoxy)carbonyl | |
| 7.220 | 3,5-F₂-Ph | H | O | H | CH(CH₃)CH₂ | (2,2,2-Trifluoroethoxy)carbonyl | |
| 7.221 | 3,5-Cl₂-Ph | H | O | H | CH(CH₃)CH₂ | (2-Hydroxyethoxy)carbonyl | |
| 7.222 | 3,5-F₂-Ph | H | O | H | CH(CH₃)CH₂ | (2-Hydroxyethoxy)carbonyl | |
| 7.223 | 3,5-Cl₂-Ph | H | O | H | CH(CH₃)CH₂ | (Allyloxy)carbonyl | |
| 7.224 | 3,5-F₂-Ph | H | O | H | CH(CH₃)CH₂ | (Allyloxy)carbonyl | |
| 7.225 | 3,5-Cl₂-Ph | H | O | H | CH(CH₃)CH₂ | (Cyclopropylsulfonyl)(methyl)carbamoyl | |
| 7.226 | 3,5-F₂-Ph | H | O | H | CH(CH₃)CH₂ | (Cyclopropylsulfonyl)(methyl)carbamoyl | |
| 7.227 | 3,5-Cl₂-Ph | H | O | H | CH(CH₃)CH₂ | (Cyclopropylsulfonyl)carbamoyl | |
| 7.228 | 3,5-F₂-Ph | H | O | H | CH(CH₃)CH₂ | (Cyclopropylsulfonyl)carbamoyl | |
| 7.229 | 3,5-Cl₂-Ph | H | O | H | CH(CH₃)CH₂ | (Ethylsulfonyl)(methyl)carbamoyl | |
| 7.230 | 3,5-F₂-Ph | H | O | H | CH(CH₃)CH₂ | (Ethylsulfonyl)(methyl)carbamoyl | |
| 7.231 | 3,5-Cl₂-Ph | H | O | H | CH(CH₃)CH₂ | (Ethylsulfonyl)carbamoyl | |
| 7.232 | 3,5-F₂-Ph | H | O | H | CH(CH₃)CH₂ | (Ethylsulfonyl)carbamoyl | |
| 7.233 | 3,5-Cl₂-Ph | H | O | H | CH(CH₃)CH₂ | (Heptan-2-yloxy)carbonyl | |
| 7.234 | 3,5-F₂-Ph | H | O | H | CH(CH₃)CH₂ | (Heptan-2-yloxy)carbonyl | |
| 7.235 | 3,5-Cl₂-Ph | H | O | H | CH(CH₃)CH₂ | (Isopropylsulfonyl)(methyl)carbamoyl | |
| 7.236 | 3,5-F₂-Ph | H | O | H | CH(CH₃)CH₂ | (Isopropylsulfonyl)(methyl)carbamoyl | |
| 7.237 | 3,5-Cl₂-Ph | H | O | H | CH(CH₃)CH₂ | (Isopropylsulfonyl)carbamoyl | |
| 7.238 | 3,5-F₂-Ph | H | O | H | CH(CH₃)CH₂ | (Isopropylsulfonyl)carbamoyl | |
| 7.239 | 3,5-Cl₂-Ph | H | O | H | CH(CH₃)CH₂ | (Methylsulfonyl)carbamoyl | |
| 7.240 | 3,5-F₂-Ph | H | O | H | CH(CH₃)CH₂ | (Methylsulfonyl)carbamoyl | |
| 7.241 | 3,5-Cl₂-Ph | H | O | H | CH(CH₃)CH₂ | (Prop-2-Yn-1-yloxy)carbonyl | |
| 7.242 | 3,5-F₂-Ph | H | O | H | CH(CH₃)CH₂ | (Prop-2-Yn-1-yloxy)carbonyl | |
| 7.243 | 3,5-Cl₂-Ph | H | O | H | CH(CH₃)CH₂ | (Propan-2-yloxy)carbonyl | |
| 7.244 | 3,5-F₂-Ph | H | O | H | CH(CH₃)CH₂ | (Propan-2-yloxy)carbonyl | |
| 7.245 | 3-CI-Ph | H | O | H | CH(CH₃)CH₂ | (Propan-2-yloxy)carbonyl | |
| 7.246 | 3-F-Ph | H | O | H | CH(CH₃)CH₂ | (Propan-2-yloxy)carbonyl | |
| 7.247 | 3,5-Cl₂-Ph | H | O | H | CH(CH₃)CH₂ | [2-(Methylsulfanyl)ethoxy]carbonyl | |
| 7.248 | 3,5-F₂-Ph | H | O | H | CH(CH₃)CH₂ | [2-(Methylsulfanyl)ethoxy]carbonyl | |
| 7.249 | 3,5-F₂-Ph | H | O | H | CH(CH₃)CH₂ | [2-(Methylsulfonyl)ethoxy]carbonyl | |
| 7.250 | 3,5-Cl₂-Ph | H | O | H | CH(CH₃)CH₂ | Butoxycarbonyl | |
| 7.251 | 3,5-F₂-Ph | H | O | H | CH(CH₃)CH₂ | Butoxycarbonyl | |
| 7.252 | 3-CF₃-Ph | H | O | H | CH(CH₃)CH₂ | COOH | |
| 7.253 | 3,5-Cl₂-Ph | H | O | H | CH(CH₃)CH₂ | COOH | |
| 7.254 | 3,5-F₂-Ph | H | O | H | CH(CH₃)CH₂ | COOH | |
| 7.255 | 3,5-(tert.Bu)2-Ph | H | O | H | CH(CH₃)CH₂ | COOH | |
| 7.256 | 3-Br-5-CF₃-Ph | H | O | H | CH(CH₃)CH₂ | COOH | |
| 7.257 | 3-Cl-5-CF₃-Ph | H | O | H | CH(CH₃)CH₂ | COOH | |
| 7.258 | 3-CI-Ph | H | O | H | CH(CH₃)CH₂ | COOH | |
| 7.259 | 3-Et-5-F-Ph | H | O | H | CH(CH₃)CH₂ | COOH | |
| 7.260 | 3-Et-Ph | H | O | H | CH(CH₃)CH₂ | COOH | |
| 7.261 | 3-F-5-MeO-Ph | H | O | H | CH(CH₃)CH₂ | COOH | |
| 7.262 | 3-F-Ph | H | O | H | CH(CH₃)CH₂ | COOH | |
| 7.263 | 3-Me-Ph | H | O | H | CH(CH₃)CH₂ | COOH | |
| 7.264 | 4-MeO-Ph | H | O | H | CH(CH₃)CH₂ | COOH | |
| 7.265 | 3-CF₃-Ph | H | O | H | CH(CH₃)CH₂ | Ethoxycarbonyl | |
| 7.266 | 3,5-Cl₂-4-MeO-Ph | H | O | H | CH(CH₃)CH₂ | Ethoxycarbonyl | |
| 7.267 | 3,5-Cl₂-Ph | H | O | H | CH(CH₃)CH₂ | Ethoxycarbonyl | |
| 7.268 | 3,5-Et₂-Ph | H | O | H | CH(CH₃)CH₂ | Ethoxycarbonyl | |
| 7.269 | 3,5-F₂-Ph | H | O | H | CH(CH₃)CH₂ | Ethoxycarbonyl | |
| 7.270 | 3,5-(MeO)₂-Ph | H | O | H | CH(CH₃)CH₂ | Ethoxycarbonyl | |
| 7.271 | 3,5-Me₂-Ph | H | O | H | CH(CH₃)CH₂ | Ethoxycarbonyl | |
| 7.272 | 3,5-(tert.Bu)2-Ph | H | O | H | CH(CH₃)CH₂ | Ethoxycarbonyl | |
| 7.273 | 3-Br-5-CF₃-Ph | H | O | H | CH(CH₃)CH₂ | Ethoxycarbonyl | |
| 7.274 | 3-CI-5-Et-Ph | H | O | H | CH(CH₃)CH₂ | Ethoxycarbonyl | |
| 7.275 | 3-Cl-5-CF₃-Ph | H | O | H | CH(CH₃)CH₂ | Ethoxycarbonyl | |
| 7.276 | 3-CI-Ph | H | O | H | CH(CH₃)CH₂ | Ethoxycarbonyl | |
| 7.277 | 3-c-Pr-5-F-Ph | H | O | H | CH(CH₃)CH₂ | Ethoxycarbonyl | |
| 7.278 | 3-Et-5-F-Ph | H | O | H | CH(CH₃)CH₂ | Ethoxycarbonyl | |
| 7.279 | 3-Et-Ph | H | O | H | CH(CH₃)CH₂ | Ethoxycarbonyl | |
| 7.280 | 3-F-5-MeS-Ph | H | O | H | CH(CH₃)CH₂ | Ethoxycarbonyl | |
| 7.281 | 3-F-5-MeSO₂-Ph | H | O | H | CH(CH₃)CH₂ | Ethoxycarbonyl | |
| 7.282 | 3-F-5-MeO-Ph | H | O | H | CH(CH₃)CH₂ | Ethoxycarbonyl | |
| 7.283 | 3-F-Ph | H | O | H | CH(CH₃)CH₂ | Ethoxycarbonyl | D1 [CDCl3] 0.99 (t,3H); 1.22 (t,3H); 1.27 (d,3H); 1.94 (m,1H); 2.14 (m,1H); 2.48 (d,2H); 3.20 (d,1H); 3.71 (d,1H); 4.05 (q,2H); 4.33 (m,1H); 7.12 (m,2H); 7.38 (m,3H). D2 [CDCl3] 1.00 (t,3H); 1.23 (t,3H); 1.28 (d,3H); 1.94 (m,1H); 2.14 (m,13H); 2.53 (m,2H); 3.20 (d,1H); 3.72 (d,1H); 4.15 (q,2H); 4.33 (m,1H); 7.12 (m,1H); 7.21 (d br,1H); 7.38 (m,3H). |
| 7.284 | 3-Me-5-CF₃O-Ph | H | O | H | CH(CH₃)CH₂ | Ethoxycarbonyl | |
| 7.285 | 3-Me-Ph | H | O | H | CH(CH₃)CH₂ | Ethoxycarbonyl | |
| 7.286 | 4-EtO-Ph | H | O | H | CH(CH₃)CH₂ | Ethoxycarbonyl | |
| 7.287 | 4-MeO-Ph | H | O | H | CH(CH₃)CH₂ | Ethoxycarbonyl | |
| 7.288 | 3,5-Cl₂-Ph | H | O | H | CH(CH₃)CH₂ | Hydroxy | |
| 7.289 | 3,5-Cl₂-Ph | H | O | H | CH(CH₃)CH₂ | Isobutoxycarbonyl | |
| 7.290 | 3,5-F₂-Ph | H | O | H | CH(CH₃)CH₂ | Isobutoxycarbonyl | |
| 7.291 | 3,5-Cl₂-Ph | H | O | H | CH(CH₃)CH₂ | Methoxy | |
| 7.292 | 2,5-Me₂-Ph | H | O | H | CH(CH₃)CH₂ | Methoxycarbonyl | |
| 7.293 | 2-CI-3-F-5-MeO-Ph | H | O | H | CH(CH₃)CH₂ | Methoxycarbonyl | |
| 7.294 | 2-F-3-Me-Ph | H | O | H | CH(CH₃)CH₂ | Methoxycarbonyl | |
| 7.295 | 3-CF₃-Ph | H | O | H | CH(CH₃)CH₂ | Methoxycarbonyl | |
| 7.296 | 3,5-Cl₂-Ph | H | O | H | CH(CH₃)CH₂ | Methoxycarbonyl | |
| 7.297 | 3,5-F₂-Ph | H | O | H | CH(CH₃)CH₂ | Methoxycarbonyl | |
| 7.298 | 3-CI-Ph | H | O | H | CH(CH₃)CH₂ | Methoxycarbonyl | |
| 7.299 | 3-F-5-MeO-Ph | H | O | H | CH(CH₃)CH₂ | Methoxycarbonyl | |
| 7.300 | 3-F-Ph | H | O | H | CH(CH₃)CH₂ | Methoxycarbonyl | |
| 7.301 | 3,5-Cl₂-Ph | H | O | H | CH(CH₃)CH₂ | Methyl(methylsulfonyl) carbamoyl | |
| 7.302 | 3,5-F₂-Ph | H | O | H | CH(CH₃)CH₂ | Methyl(methylsulfonyl) carbamoyl | |
| 7.303 | 3,5-Cl₂-Ph | H | O | H | CH(CH₃)CH₂ | Methylcarbamoyl | |
| 7.304 | 3,5-Cl₂-Ph | H | O | H | CH(CH₃)CH₂ | Propoxycarbonyl | |
| 7.305 | 3,5-F₂-Ph | H | O | H | CH(CH₃)CH₂ | Propoxycarbonyl | |
| 7.306 | 3,5-Cl₂-Ph | H | O | H | CH(CH₃)CH₂ | Sec-Butoxycarbonyl | |
| 7.307 | 3,5-F₂-Ph | H | O | H | CH(CH₃)CH₂ | Sec-Butoxycarbonyl | |
| 7.308 | 3,5-Cl₂-Ph | H | O | H | CH(COOCH₃) CH₂ | Methoxycarbonyl | |
| 7.309 | 3,5-Cl₂-Ph | H | O | H | CH(cycloPr) | c-Pr | |
| 7.310 | 3,5-F₂-Ph | H | O | H | CH(cycloPr) | c-Pr | |
| 7.311 | 3,5-Cl₂-Ph | H | O | H | CH(iPr) | Methoxycarbonyl | |
| 7.312 | 3,5-Cl₂-Ph | H | O | H | CH(iPr)CH₂ | Methoxycarbonyl | |
| 7.313 | 3,5-F₂-Ph | H | O | H | CH₂ | (2-Hydroxyethyl)carbamoyl | |
| 7.314 | 3,5-Cl₂-Ph | H | O | H | CH₂ | (Cyclopropylsulfonyl)carbamoyl | |
| 7.315 | 3,5-F₂-Ph | H | O | H | CH₂ | (Cyclopropylsulfonyl)carbamoyl | |
| 7.316 | 3,5-Cl₂-Ph | H | O | H | CH₂ | (Ethylsulfonyl)carbamoyl | |
| 7.317 | 3,5-F₂-Ph | H | O | H | CH₂ | (Ethylsulfonyl)carbamoyl | |
| 7.318 | 3,5-F₂-Ph | H | O | H | CH₂ | (Hydroxyimino)methyl | |
| 7.319 | 3,5-Cl₂-Ph | H | O | H | CH₂ | (Isopropylsulfonyl)carbamoyl | |
| 7.320 | 3,5-F₂-Ph | H | O | H | CH₂ | (Isopropylsulfonyl)carbamoyl | |
| 7.321 | 3,5-Cl₂-Ph | H | O | H | CH₂ | (Methylsulfonyl)carbamoyl | |
| 7.322 | 3,5-F₂-Ph | H | O | H | CH₂ | (Methylsulfonyl)carbamoyl | |
| 7.323 | 3-CI-Ph | H | O | H | CH₂ | (Propan-2-yloxy)carbonyl | |
| 7.324 | 3-F-Ph | H | O | H | CH₂ | (Propan-2-yloxy)carbonyl | |
| 7.325 | 3,5-Cl₂-Ph | H | O | H | CH₂ | 1,1-Dioxidotetrahydrothiophen-3-yl | |
| 7.326 | 3,5-F₂-Ph | H | O | H | CH₂ | 1,1-Dioxidotetrahydrothiophen-3-yl | |
| 7.327 | 3-F-Ph | H | O | H | CH₂ | 1,1-Dioxidotetrahydrothiophen-3-yl | |
| 7.328 | Ph | H | O | H | CH₂ | 1,1-Dioxidotetrahydrothio phen-3-yl | |
| 7.329 | Ph | H | O | H | CH₂ | 1,4-Dimethyl-5-oxo-4,5-dihydro-1H-1,2,4-triazol-3-yl | |
| 7.330 | 3,5-Cl₂-Ph | H | O | H | CH₂ | 3-(2-Ethoxy-2-oxoethyl)oxetan-3-yl | |
| 7.331 | 3,5-F₂-Ph | H | O | H | CH₂ | 3-(2-Ethoxy-2-oxoethyl)oxetan-3-yl | |
| 7.332 | 3,5-Cl₂-Ph | H | O | H | CH₂ | 3-(Cyclopropylcarbamoy l)-4,5-dihydro-1,2-oxazol-5-yl | |
| 7.333 | 3,5-Cl₂-Ph | H | O | H | CH₂ | 3-(Ethoxycarbonyl)-4,5-dihydro-1,2-oxazol-5-yl | |
| 7.334 | 3,5-Cl₂-Ph | H | O | H | CH₂ | 3-(Methylcarbamoyl)-4,5-dihydro-1,2-oxazol-5-yl | |
| 7.335 | 3,5-Cl₂-Ph | H | O | H | CH₂ | 3-Carboxy-4,5-dihydro-1,2-oxazol-5-yl | |
| 7.336 | 3,5-Cl₂-Ph | H | O | H | CH₂ | 3-Isopropyl-4,5-dihydro-1,2-oxazol-5-yl | |
| 7.337 | 3,5-Cl₂-Ph | H | O | H | CH₂ | 3-Methyl-4,5-dihydro-1,2-oxazol-5-yl | |
| 7.338 | 3,5-F₂-Ph | H | O | H | CH₂ | 5-(Ethoxycarbonyl)-4,5-dihydro-1,2-oxazol-3-yl | |
| 7.339 | 3,5-Cl₂-Ph | H | O | H | CH₂ | 5,5-Dimethyl-4,5-dihydro-1,2-oxazol-3-yl | |
| 7.340 | 3,5-Cl₂-Ph | H | O | H | CH₂ | 5-Ethyl-4,5-dihydro-1,2-oxazol-3-yl | |
| 7.341 | 3,5-Cl₂-Ph | H | O | H | CH₂ | 5-Isopropyl-4,5-dihydro-1,2-oxazol-3-yl | |
| 7.342 | 3,5-Cl₂-Ph | H | O | H | CH₂ | 5-Methyl-4,5-dihydro-1,2-oxazol-3-yl | |
| 7.343 | 3,5-Cl₂-Ph | H | O | H | CH₂ | Carbamoyl | |
| 7.344 | 3-CI-Ph | H | O | H | CH₂ | Carbamoyl | |
| 7.345 | 2-CF₃-Ph | H | O | H | CH₂ | CF₃ | |
| 7.346 | 2,3,4-F₃-Ph | H | O | H | CH₂ | CF₃ | |
| 7.347 | 2,3,5-F₃-Ph | H | O | H | CH₂ | CF₃ | |
| 7.348 | 2,3,6-Cl₃-Ph | H | O | H | CH₂ | CF₃ | |
| 7.349 | 2,3-F₂-Ph | H | O | H | CH₂ | CF₃ | |
| 7.350 | 2,5-F₂-Ph | H | O | H | CH₂ | CF₃ | |
| 7.351 | 2-CI-3-F-5-MeO-Ph | H | O | H | CH₂ | CF₃ | |
| 7.352 | 3-(2-MeOEtO)-Ph | H | O | H | CH₂ | CF₃ | |
| 7.353 | 3-iPrO-Ph | H | O | H | CH₂ | CF₃ | |
| 7.354 | 3-CF₃O-Ph | H | O | H | CH₂ | CF₃ | |
| 7.355 | 3-CF₃-Ph | H | O | H | CH₂ | CF₃ | |
| 7.356 | 3,4-Cl₂-Ph | H | O | H | CH₂ | CF₃ | |
| 7.357 | 3,5-Br₂-Ph | H | O | H | CH₂ | CF₃ | |
| 7.358 | 3,5-Cl₂-4-MeO-Ph | H | O | H | CH₂ | CF₃ | |
| 7.359 | 3,5-Cl₂-Ph | H | O | H | CH₂ | CF₃ | |
| 7.360 | 3,5-F₂-Ph | H | O | H | CH₂ | CF₃ | |
| 7.361 | 3,5-(MeO)₂-Ph | H | O | H | CH₂ | CF₃ | |
| 7.362 | 3,5-Me₂-Ph | H | O | H | CH₂ | CF₃ | |
| 7.363 | 3-Br-5-CI-Ph | H | O | H | CH₂ | CF₃ | |
| 7.364 | 3-Br-5-F-Ph | H | O | H | CH₂ | CF₃ | |
| 7.365 | 3-Cl-5-CF₃-Ph | H | O | H | CH₂ | CF₃ | |
| 7.366 | 3-CI-5-Et-Ph | H | O | H | CH₂ | CF₃ | |
| 7.367 | 3-CI-5-F-Ph | H | O | H | CH₂ | CF₃ | |
| 7.368 | 3-Cl-5-Me-Ph | H | O | H | CH₂ | CF₃ | |
| 7.369 | 3-CI-5-CF₃O-Ph | H | O | H | CH₂ | CF₃ | |
| 7.370 | 3-CI-Ph | H | O | H | CH₂ | CF₃ | |
| 7.371 | 3-EtO-Ph | H | O | H | CH₂ | CF₃ | |
| 7.372 | 3-Et-5-F-Ph | H | O | H | CH₂ | CF₃ | |
| 7.373 | 3-Et-Ph | H | O | H | CH₂ | CF₃ | |
| 7.374 | 3-F-5-MeS-Ph | H | O | H | CH₂ | CF₃ | |
| 7.375 | 3-F-5-MeSO₂-Ph | H | O | H | CH₂ | CF₃ | |
| 7.376 | 3-F-5-CF₃-Ph | H | O | H | CH₂ | CF₃ | |
| 7.377 | 3-F-5-MeO-Ph | H | O | H | CH₂ | CF₃ | |
| 7.378 | 3-F-5-Me-Ph | H | O | H | CH₂ | CF₃ | |
| 7.379 | 3-F-Ph | H | O | H | CH₂ | CF₃ | [CDCl3] 1.02 (t,3H); 1.96 (m,1H); 2.19 (m,1H); 3.28 (d,1H); 3.70 (m,1H); 3.72 (d,1H); 4.13 (m,1H); 7.13 (m,1H); 7.18 (s br,1H); 7.38 (m,3H). |
| 7.380 | 3-OH-Ph | H | O | H | CH₂ | CF₃ | |
| 7.381 | 3-iPr-Ph | H | O | H | CH₂ | CF₃ | |
| 7.382 | 3-Me-Ph | H | O | H | CH₂ | CF₃ | |
| 7.383 | 3-NO₂-Ph | H | O | H | CH₂ | CF₃ | |
| 7.384 | 4-EtO-Ph | H | O | H | CH₂ | CF₃ | |
| 7.385 | 4-MeO-Ph | H | O | H | CH₂ | CF₃ | |
| 7.386 | F₅-Ph | H | O | H | CH₂ | CF₃ | |
| 7.387 | Ph | H | O | H | CH₂ | CF₃ | |
| 7.388 | 2,3,5-F₃-Ph | H | O | H | CH₂ | CH₃ | |
| 7.389 | 2,3-F₂-Ph | H | O | H | CH₂ | CH₃ | |
| 7.390 | 3,4-F₂-Ph | H | O | H | CH₂ | CH₃ | |
| 7.391 | 3,5-Cl₂-Ph | H | O | H | CH₂ | CH₃ | |
| 7.392 | 3,5-F₂-Ph | H | O | H | CH₂ | CH₃ | |
| 7.393 | 3-Cl-5-CF₃-Ph | H | O | H | CH₂ | CH₃ | |
| 7.394 | 3-Cl-5-Me-Ph | H | O | H | CH₂ | CH₃ | |
| 7.395 | 3-Cl-5-CF₃O-Ph | H | O | H | CH₂ | CH₃ | |
| 7.396 | 3-EtO-Ph | H | O | H | CH₂ | CH₃ | |
| 7.397 | 3-Et-Ph | H | O | H | CH₂ | CH₃ | |
| 7.398 | 3-F-Ph | H | O | H | CH₂ | CH₃ | |
| 7.399 | 3-Me-Ph | H | O | H | CH₂ | CH₃ | |
| 7.400 | F₅-Ph | H | O | H | CH₂ | CH₃ | |
| 7.401 | Ph | H | O | H | CH₂ | CH₃ | |
| 7.402 | 3,5-Cl₂-Ph | H | O | H | CH₂ | COOH | |
| 7.403 | 3,5-F₂-Ph | H | O | H | CH₂ | COOH | |
| 7.404 | 3-CI-Ph | H | O | H | CH₂ | COOH | |
| 7.405 | 3-F-Ph | H | O | H | CH₂ | COOH | |
| 7.406 | 3,5-Cl₂-Ph | H | O | H | CH₂ | Cyan | |
| 7.407 | 3,5-F₂-Ph | H | O | H | CH₂ | Cyan | |
| 7.408 | 3-CI-5-CF₃O-Ph | H | O | H | CH₂ | Cyan | |
| 7.409 | 3-F-5-Me-Ph | H | O | H | CH₂ | Cyan | |
| 7.410 | 3-F-Ph | H | O | H | CH₂ | Cyan | |
| 7.411 | 3-NO₂-Ph | H | O | H | CH₂ | Cyan | |
| 7.412 | 3,5-Cl₂-Ph | H | O | H | CH₂ | Cyclohexyl | |
| 7.413 | 3,5-Cl₂-Ph | H | O | H | CH₂ | c-Pr | |
| 7.414 | 3,5-F₂-Ph | H | O | H | CH₂ | c-Pr | |
| 7.415 | 3,5-Cl₂-Ph | H | O | H | CH₂ | Diethoxyphosphoryl | |
| 7.416 | 3,5-F₂-Ph | H | O | H | CH₂ | Diethoxyphosphoryl | |
| 7.417 | 3-F-Ph | H | O | H | CH₂ | Diethoxyphosphoryl | |
| 7.418 | Ph | H | O | H | CH₂ | Diethoxyphosphoryl | |
| 7.419 | 3,5-Cl₂-Ph | H | O | H | CH₂ | Difluormethyl | |
| 7.420 | 3,5-Cl₂-Ph | H | O | H | CH₂ | Dimethoxymethyl | |
| 7.421 | 3-Cl-Ph | H | O | H | CH₂ | Dimethoxymethyl | |
| 7.422 | 3,5-Cl₂-Ph | H | O | H | CH₂ | Dimethylcarbamoyl | |
| 7.423 | 3,5-Cl₂-Ph | H | O | H | CH₂ | Ethenyl | |
| 7.424 | 3,5-Cl₂-Ph | H | O | Pro p-2-en-1-yl | CH₂ | Ethenyl | |
| 7.425 | 3,5-F₂-Ph | H | O | H | CH₂ | Ethenyl | |
| 7.426 | 3-F-Ph | H | O | H | CH₂ | Ethenyl | |
| 7.427 | Ph | H | O | H | CH₂ | Ethenyl | |
| 7.428 | 3,5-(CF₃)₂-Ph | H | O | H | CH₂ | Ethinyl | |
| 7.429 | 3,5-Cl₂-Ph | H | O | H | CH₂ | Ethinyl | |
| 7.430 | 3,5-Cl₂-Ph | H | O | Pro p-2-in-1-yl | CH₂ | Ethinyl | |
| 7.431 | 3,5-F₂-Ph | H | O | H | CH₂ | Ethinyl | |
| 7.432 | 3,5-Cl₂-Ph | H | O | cPr | CH₂ | Ethoxycarbonyl | |
| 7.433 | 3-CI-Ph | H | O | H | CH₂ | Ethoxycarbonyl | |
| 7.434 | 3-F-Ph | H | O | H | CH₂ | Ethoxycarbonyl | |
| 7.435 | 3,5-F₂-Ph | H | O | H | CH₂ | Formyl | |
| 7.436 | 3,5-Cl₂-Ph | H | O | H | CH₂ | Methoxycarbonyl | |
| 7.437 | 3,5-F₂-Ph | H | O | H | CH₂ | Methoxycarbonyl | |
| 7.438 | 3-CI-Ph | H | O | H | CH₂ | Methoxycarbonyl | |
| 7.439 | 3-F-Ph | H | O | H | CH₂ | Methoxycarbonyl | |
| 7.440 | 3-Cl-5-F-Ph | H | O | H | CH₂ | ony | |
| 7.441 | 3,5-Cl₂-Ph | H | O | H | CH₂ | Pentafluorethyl | |
| 7.442 | 3,5-Cl₂-Ph | H | O | H | CH₂ | Pentyl | |
| 7.443 | 3,5-F₂-Ph | H | O | H | CH₂ | Pentyl | |
| 7.444 | 3,5-Cl₂-Ph | H | O | H | CH₂ | Piperidin-2-yl | |
| 7.445 | 3,5-F₂-Ph | H | O | H | CH₂ | Piperidin-2-yl | |
| 7.446 | Ph | H | O | H | CH₂ | Piperidin-2-yl | |
| 7.447 | 3,5-Cl₂-Ph | H | O | CH₃ | CH₂ | Propan-2-yl | |
| 7.448 | 3,5-Cl₂-Ph | H | O | H | CH₂ | Propan-2-yl | |
| 7.449 | 3,5-Cl₂-Ph | H | O | H | CH₂ | Pyrrolidin-1-ylcarbonyl | |
| 7.450 | 3,5-Cl₂-Ph | H | O | H | CH₂ | tert-Butyl | |
| 7.451 | 3,5-F₂-Ph | H | O | H | CH₂ | tert-Butyl | |
| 7.452 | 3,5-Cl₂-Ph | H | O | H | CH₂ | Tetrahydrofuran-2-yl | |
| 7.453 | 3,5-F₂-Ph | H | O | H | CH₂ | Tetrahydrofuran-2-yl | |
| 7.454 | 3-F-Ph | H | O | H | CH₂ | Tetrahydrofuran-2-yl | |
| 7.455 | Ph | H | O | H | CH₂ | Tetrahydrofuran-2-yl | |
| 7.456 | 3,5-Cl₂-Ph | H | O | H | CH₂ | Tetrahydrofuran-3-yl | |
| 7.457 | 3,5-F₂-Ph | H | O | H | CH₂ | Tetrahydrofuran-3-yl | |
| 7.458 | 3-F-Ph | H | O | H | CH₂ | Tetrahydrofuran-3-yl | |
| 7.459 | Ph | H | O | H | CH₂ | Tetrahydrofuran-3-yl | |
| 7.460 | 3,5-Cl₂-Ph | H | O | H | CH₂C(CH₃)₂ | COOH | |
| 7.461 | 3,5-Cl₂-Ph | H | O | H | CH₂C(CH₃)₂ | Ethoxycarbonyl | |
| 7.462 | 3,5-Cl₂-Ph | H | O | H | CH₂CH(CH₃) | Ethoxycarbonyl | |
| 7.463 | 3,5-Cl₂-Ph | H | O | H | CH₂CH₂ | (2,2,2-Trifluoroethoxy)carbonyl | |
| 7.464 | 3,5-Cl₂-Ph | H | O | H | CH₂CH₂ | (2-Hydroxyethoxy)carbon yl | |
| 7.465 | 3,5-Cl₂-Ph | H | O | H | CH₂CH₂ | (2-Methoxy-2-oxoethyl)carbamoyl | |
| 7.466 | 3,5-Cl₂-Ph | H | O | H | CH₂CH₂ | (Allyloxy)carbonyl | |
| 7.467 | 3,5-Cl₂-Ph | H | O | H | CH₂CH₂ | (Cyclopropylsulfonyl)(methyl)carbamoyl | |
| 7.468 | 3,5-F₂-Ph | H | O | H | CH₂CH₂ | (Cyclopropylsulfonyl)( methyl)carbamoyl | |
| 7.469 | 3,5-Cl₂-Ph | H | O | H | CH₂CH₂ | (Cyclopropylsulfonyl)carbamoyl | |
| 7.470 | 3,5-F₂-Ph | H | O | H | CH₂CH₂ | (Cyclopropylsulfonyl)carbamoyl | |
| 7.471 | 3,5-Cl₂-Ph | H | O | H | CH₂CH₂ | (Ethylsulfonyl)(methyl)carbamoyl | |
| 7.472 | 3,5-F₂-Ph | H | O | H | CH₂CH₂ | (Ethylsulfonyl)(methyl)carbamoyl | |
| 7.473 | 3,5-Cl₂-Ph | H | O | H | CH₂CH₂ | (Ethylsulfonyl)carbamoyl | |
| 7.474 | 3,5-F₂-Ph | H | O | H | CH₂CH₂ | (Ethylsulfonyl)carbamoyl | |
| 7.475 | 3,5-Cl₂-Ph | H | O | H | CH₂CH₂ | (Heptan-2-yloxy)carbonyl | |
| 7.476 | 3,5-F₂-Ph | H | O | H | CH₂CH₂ | (Hydroxyimino)methyl | |
| 7.477 | 3,5-Cl₂-Ph | H | O | H | CH₂CH₂ | (Isopropylsulfonyl)(methyl)carbamoyl | |
| 7.478 | 3,5-F₂-Ph | H | O | H | CH₂CH₂ | (Isopropylsulfonyl)(methyl)carbamoyl | |
| 7.479 | 3,5-Cl₂-Ph | H | O | H | CH₂CH₂ | (Isopropylsulfonyl)carbamoyl | |
| 7.480 | 3,5-F₂-Ph | H | O | H | CH₂CH₂ | (Isopropylsulfonyl)carbamoyl | |
| 7.481 | 3,5-Cl₂-Ph | H | O | H | CH₂CH₂ | (Methylsulfonyl)carbamoyl | |
| 7.482 | 3,5-F₂-Ph | H | O | H | CH₂CH₂ | (Methylsulfonyl)carbamoyl | |
| 7.483 | 3,5-Cl₂-Ph | H | O | H | CH₂CH₂ | (Prop-2-Yn-1-yloxy)carbonyl | |
| 7.484 | 3,5-Cl₂-Ph | H | O | H | CH₂CH₂ | (Propan-2-yloxy)carbonyl | |
| 7.485 | 3,5-Cl₂-Ph | H | O | H | CH₂CH₂ | [2-(Methylsulfanyl)ethoxy]carbonyl | |
| 7.486 | 3,5-Cl₂-Ph | H | O | H | CH₂CH₂ | 1,3-Dioxolan-2-yl | |
| 7.487 | 3,5-F₂-Ph | H | O | H | CH₂CH₂ | 1,3-Dioxolan-2-yl | |
| 7.488 | 3-F-Ph | H | O | H | CH₂CH₂ | 1,3-Dioxolan-2-yl | |
| 7.489 | Ph | H | O | H | CH₂CH₂ | 1,3-Dioxolan-2-yl | |
| 7.490 | 3,5-Cl₂-Ph | H | O | H | CH₂CH₂ | 1-Methylpyrrolidin-2-yl | |
| 7.491 | 3,5-Cl₂-Ph | H | O | H | CH₂CH₂ | 2-Oxopyrrolidin-1-yl | |
| 7.492 | 3,5-Cl₂-Ph | H | O | H | CH₂CH₂ | Butoxycarbonyl | |
| 7.493 | 3,4,5-F₃-Ph | H | O | H | CH₂CH₂ | Butylcarbamoyl | |
| 7.494 | 3,5-Cl₂-Ph | H | O | H | CH₂CH₂ | Carbamoyl | |
| 7.495 | 3-Cl-4-Me-Ph | H | O | H | CH₂CH₂ | Carbamoyl | |
| 7.496 | 3,5-Cl₂-Ph | H | O | H | CH₂CH₂ | CF3 | |
| 7.497 | 3,5-F₂-Ph | H | O | H | CH₂CH₂ | CF3 | |
| 7.498 | 3,5-Cl₂-Ph | H | O | H | CH₂CH₂ | CH3 | |
| 7.499 | 3,5-F₂-Ph | H | O | H | CH₂CH₂ | CH3 | |
| 7.500 | 3,5-Cl₂-Ph | H | O | H | CH₂CH₂ | Chlor | |
| 7.501 | 2,3,4,5-F₄-6-OH-Ph | H | O | H | CH₂CH₂ | COOH | |
| 7.502 | 2,3,5-F₃-Ph | H | O | H | CH₂CH₂ | COOH | |
| 7.503 | 2,3-F₂-Ph | H | O | H | CH₂CH₂ | COOH | |
| 7.504 | 3,5-Cl₂-Ph | H | O | H | CH₂CH₂ | COOH | |
| 7.505 | 2,5-F₂-Ph | H | O | H | CH₂CH₂ | COOH | |
| 7.506 | 2-CI-5-F-Ph | H | O | H | CH₂CH₂ | COOH | |
| 7.507 | 2-F-3-Me-Ph | H | O | H | CH₂CH₂ | COOH | |
| 7.508 | 3-CF₃-Ph | H | O | H | CH₂CH₂ | COOH | |
| 7.509 | 3,4,5-F₃-Ph | H | O | H | CH₂CH₂ | COOH | |
| 7.510 | 3,4-F₂-Ph | H | O | H | CH₂CH₂ | COOH | |
| 7.511 | 3,5-Br₂-Ph | H | O | H | CH₂CH₂ | COOH | |
| 7.512 | 3,5-Cl₂-Ph | H | O | CH₃ | CH₂CH₂ | COOH | |
| 7.513 | 3,5-Cl₂-Ph | H | O | H | CH₂CH₂ | COOH | [COCl₃] 1.00 (t, 3H); 1.95 (m, 1H); 2.15 (m, 1H); 2.62 (m, 2H); 3.20 (d, 1H); 3.51 (m, 1H); 3.62 (m, 1H); 3.69 (d, 1H); 7.41 (s, 1H); 7,52 (s, 2H). |
| 7.514 | 3,5-F₂-Ph | H | O | H | CH₂CH₂ | COOH | [COCl₃] 1.00 (t, 3H); 1.95 (m, 1H); 2.14 (m, 1H); 2.61 (m, 2H); 3.20 (d, 1H); 3.50 (m, 1H); 3.61 (m, 1H); 3.69 (d, 1H); 6.88 (m, 1H); 7.14 (m, 2H) |
| 7.515 | 3,5-Me₂-Ph | H | O | H | CH₂CH₂ | COOH | |
| 7.516 | 3,5-(tert.Bu)2-Ph | H | O | H | CH₂CH₂ | COOH | |
| 7.517 | 3-Br-5-CI-Ph | H | O | H | CH₂CH₂ | COOH | |
| 7.518 | 3-Br-5-F-Ph | H | O | H | CH₂CH₂ | COOH | |
| 7.519 | 3-Br-5-CF₃-Ph | H | O | H | CH₂CH₂ | COOH | |
| 7.520 | 3-Cl-4-F-Ph | H | O | H | CH₂CH₂ | COOH | |
| 7.521 | 3-Cl-4-Me-Ph | H | O | H | CH₂CH₂ | COOH | |
| 7.522 | 3-Cl-5-F-Ph | H | O | H | CH₂CH₂ | COOH | |
| 7.523 | 3-Cl-5-CF₃O-Ph | H | O | H | CH₂CH₂ | COOH | |
| 7.524 | 3-Cl-5-CF₃-Ph | H | O | H | CH₂CH₂ | COOH | |
| 7.525 | 3-Cl-5-Me-Ph | H | O | H | CH₂CH₂ | COOH | |
| 7.526 | 3-Et-5-F-Ph | H | O | H | CH₂CH₂ | COOH | |
| 7.527 | 3-Et-Ph | H | O | H | CH₂CH₂ | COOH | |
| 7.528 | 3-F-5-MeO-Ph | H | O | H | CH₂CH₂ | COOH | |
| 7.529 | 3-F-5-Me-Ph | H | O | H | CH₂CH₂ | COOH | |
| 7.530 | 3-F-Ph | H | O | H | CH₂CH₂ | COOH | |
| 7.531 | 3-Me-Ph | H | O | H | CH₂CH₂ | COOH | |
| 7.532 | 4-Cl-Ph | H | O | H | CH₂CH₂ | COOH | |
| 7.533 | 4-EtO-Ph | H | O | H | CH₂CH₂ | COOH | |
| 7.534 | Ph | H | O | H | CH₂CH₂ | COOH | |
| 7.535 | 3,5-Cl₂-Ph | H | O | H | CH₂CH₂ | Cyan | |
| 7.536 | 3-Cl-Ph | H | O | H | CH₂CH₂ | Cyan | |
| 7.537 | 3,5-Cl₂-Ph | H | O | H | CH₂CH₂ | Cyclopropylcarbamoyl | |
| 7.538 | 3,5-Cl₂-Ph | H | O | H | CH₂CH₂ | Diethoxymethyl | |
| 7.539 | 3,5-Cl₂-Ph | H | O | H | CH₂CH₂ | Diethoxyphosphoryl | |
| 7.540 | Ph | H | O | H | CH₂CH₂ | Diethoxyphosphoryl | |
| 7.541 | 3,5-Cl₂-Ph | H | O | H | CH₂CH₂ | Dimethoxymethyl | |
| 7.542 | 3,5-Cl₂-Ph | H | O | H | CH₂CH₂ | Dimethylcarbamoyl | |
| 7.543 | 3,5-Cl₂-Ph | H | O | H | CH₂CH₂ | Ethoxy | |
| 7.544 | 3-Cl-Ph | H | O | H | CH₂CH₂ | Ethoxy | |
| 7.545 | 2-Cl-5-F-Ph | H | O | H | CH₂CH₂ | Ethoxycarbonyl | |
| 7.546 | 3-CF₃-Ph | H | O | H | CH₂CH₂ | Ethoxycarbonyl | |
| 7.547 | 3,5-Cl₂-Ph | H | O | cPr | CH₂CH₂ | Ethoxycarbonyl | |
| 7.548 | 3,5-Cl₂-Ph | H | O | H | CH₂CH₂ | Ethoxycarbonyl | |
| 7.549 | 3,5-F₂-Ph | H | O | H | CH₂CH₂ | Ethoxycarbonyl | |
| 7.550 | 3-Br-5-CF₃-Ph | H | O | H | CH₂CH₂ | Ethoxycarbonyl | |
| 7.551 | 3-CI-5-Et-Ph | H | O | H | CH₂CH₂ | Ethoxycarbonyl | |
| 7.552 | 3-Cl-5-CF₃-Ph | H | O | H | CH₂CH₂ | Ethoxycarbonyl | |
| 7.553 | 3-Et-5-F-Ph | H | O | H | CH₂CH₂ | Ethoxycarbonyl | |
| 7.554 | 3-Et-Ph | H | O | H | CH₂CH₂ | Ethoxycarbonyl | |
| 7.555 | 3-F-5-MeO-Ph | H | O | H | CH₂CH₂ | Ethoxycarbonyl | |
| 7.556 | 3-Me-5-CF₃O-Ph | H | O | H | CH₂CH₂ | Ethoxycarbonyl | |
| 7.557 | 3,5-Cl₂-Ph | H | O | H | CH₂CH₂ | Ethylcarbamoyl | |
| 7.558 | 3,5-F₂-Ph | H | O | H | CH₂CH₂ | Formyl | |
| 7.559 | 3,5-Cl₂-Ph | H | O | H | CH₂CH₂ | Hydroxy | |
| 7.560 | 3,5-F₂-Ph | H | O | H | CH₂CH₂ | Hydroxy | |
| 7.561 | 3-Cl-Ph | H | O | H | CH₂CH₂ | Hydroxy | |
| 7.562 | 3,5-Cl₂-Ph | H | O | H | CH₂CH₂ | Isobutoxycarbonyl | |
| 7.563 | 3,5-Cl₂-Ph | H | O | H | CH₂CH₂ | Methoxy | |
| 7.564 | 3-Cl-5-CF₃-Ph | H | O | H | CH₂CH₂ | Methoxy | |
| 7.565 | 3-CI-Ph | H | O | H | CH₂CH₂ | Methoxy | |
| 7.566 | 2,3,4-F₃-Ph | H | O | H | CH₂CH₂ | Methoxycarbonyl | |
| 7.567 | 2,3,5-F₃-Ph | H | O | H | CH₂CH₂ | Methoxycarbonyl | |
| 7.568 | 2,3-F₂-Ph | H | O | H | CH₂CH₂ | Methoxycarbonyl | |
| 7.569 | 3,5-Cl₂-Ph | H | O | H | CH₂CH₂ | Methoxycarbonyl | |
| 7.570 | 2,5-F₂-Ph | H | O | H | CH₂CH₂ | Methoxycarbonyl | |
| 7.571 | 2,5-Me₂-Ph | H | O | H | CH₂CH₂ | Methoxycarbonyl | |
| 7.572 | 2-CI-3-F-5-MeO-Ph | H | O | H | CH₂CH₂ | Methoxycarbonyl | |
| 7.573 | 2-F-3-Me-Ph | H | O | H | CH₂CH₂ | Methoxycarbonyl | |
| 7.574 | 3-CF₃-Ph | H | O | H | CH₂CH₂ | Methoxycarbonyl | |
| 7.575 | 3,4,5-F₃-Ph | H | O | H | CH₂CH₂ | Methoxycarbonyl | |
| 7.576 | 3,4-F₂-Ph | H | O | H | CH₂CH₂ | Methoxycarbonyl | |
| 7.577 | 3,5-Br₂-Ph | H | O | H | CH₂CH₂ | Methoxycarbonyl | |
| 7.578 | 3,5-Cl₂-4-MeO-Ph | H | O | H | CH₂CH₂ | Methoxycarbonyl | |
| 7.579 | 3,5-Cl₂-Ph | CH₃ | O | H | CH₂CH₂ | Methoxycarbonyl | |
| 7.580 | 3,5-Cl₂-Ph | H | O | CH₃ | CH₂CH₂ | Methoxycarbonyl | |
| 7.581 | 3,5-Cl₂-Ph | H | O | H | CH₂CH₂ | Methoxycarbonyl | [CDCl₃] 0.97 (t, 3H); 1.87-1.97 (m, 1H), 2.09-2.19 (m, 1H); 2.53-2.58 (m, 2H); 3.19 (d, 1H); 3.43-3.52 (m, 1H); 3.58-3.66 (m, 1H); 3.68 (d, 1H); 3.69 (s, 3H); 7.23 (br, 1H); 7.41 (m, 1H); 7.51 (m, 2H). |
| 7.582 | 3,5-Et₂-Ph | H | O | H | CH₂CH₂ | Methoxycarbonyl | |
| 7.583 | 3,5-F₂-Ph | H | O | H | CH₂CH₂ | Methoxycarbonyl | [CDCl₃] 7.26 29.42;7.24 0.35;7.23 0.51;7.21 0.36;7.17 1.39;7.17 1.80;7.16 1.07;7.15 1.74;7.15 1.43;6.90 0.34;6.89 0.61;6.88 0.68;6.87 1.21;6.87 0.62;6.86 0.36;6.85 0.62;3.69 2.41;3.69 16.00;3.65 2.73;3.63 0.56;3.63 0.53;3.61 0.87;3.60 0.80;3.58 0.38;3.52 0.35;3.51 0.78;3.49 0.82;3.48 0.64;3.46 0.48;3.22 2.37;3.17 2.01;2.57 1.05;2.57 1.07;2.56 1.22;2.56 1.34;2.55 1.78;2.54 1.05;2.54 1.05;2.17 0.66;2.15 0.85;2.13 1.06;2.11 0.96;1.96 0.90;1.94 1.10;1.92 0.91;1.91 0.68;1.55 8.93;1.43 1.50;1.22 0.43;1.00 3.33;0.99 6.94;0.97 3.11;0.01 0.56;0.00 19.10;-0.01 0.72 |
| 7.584 | 3,5-(MeO)₂-Ph | H | O | H | CH₂CH₂ | Methoxycarbonyl | |
| 7.585 | 3,5-Me₂-Ph | H | O | H | CH₂CH₂ | Methoxycarbonyl | |
| 7.586 | 3,5-(tert.Bu)2-Ph | H | O | H | CH₂CH₂ | Methoxycarbonyl | |
| 7.587 | 3-CF₃S-Ph | H | O | H | CH₂CH₂ | Methoxycarbonyl | |
| 7.588 | 3-Ac-Ph | H | O | H | CH₂CH₂ | Methoxycarbonyl | |
| 7.589 | 3-Br-5-CI-Ph | H | O | H | CH₂CH₂ | Methoxycarbonyl | |
| 7.590 | 3-Br-5-F-Ph | H | O | H | CH₂CH₂ | Methoxycarbonyl | |
| 7.591 | 3-Br-5-CF₃-Ph | H | O | H | CH₂CH₂ | Methoxycarbonyl | |
| 7.592 | 3-NH₂CO-Ph | H | O | H | CH₂CH₂ | Methoxycarbonyl | |
| 7.593 | 3-CI-4-F-Ph | H | O | H | CH₂CH₂ | Methoxycarbonyl | |
| 7.594 | 3-Cl-4-Me-Ph | H | O | H | CH₂CH₂ | Methoxycarbonyl | |
| 7.595 | 3-Cl-5-CN-Ph | H | O | H | CH₂CH₂ | Methoxycarbonyl | |
| 7.596 | 3-CI-5-Et-Ph | H | O | H | CH₂CH₂ | Methoxycarbonyl | |
| 7.597 | 3-CI-5-F-Ph | H | O | H | CH₂CH₂ | Methoxycarbonyl | |
| 7.598 | 3-CI-5-MeO-Ph | H | O | H | CH₂CH₂ | Methoxycarbonyl | |
| 7.599 | 3-CI-5-CF₃O-Ph | H | O | H | CH₂CH₂ | Methoxycarbonyl | |
| 7.600 | 3-Cl-5-CF₃-Ph | H | O | H | CH₂CH₂ | Methoxycarbonyl | |
| 7.601 | 3-Cl-5-Me-Ph | H | O | H | CH₂CH₂ | Methoxycarbonyl | |
| 7.602 | 3-CN-5-F-Ph | H | O | H | CH₂CH₂ | Methoxycarbonyl | |
| 7.603 | 3-CN-Ph | H | O | H | CH₂CH₂ | Methoxycarbonyl | |
| 7.604 | 3-c-Pr-5-F-Ph | H | O | H | CH₂CH₂ | Methoxycarbonyl | |
| 7.605 | 3-Et-5-F-Ph | H | O | H | CH₂CH₂ | Methoxycarbonyl | |
| 7.606 | 3-F-5-MeS-Ph | H | O | H | CH₂CH₂ | Methoxycarbonyl | |
| 7.607 | 3-F-5-MeSO₂-Ph | H | O | H | CH₂CH₂ | Methoxycarbonyl | |
| 7.608 | 3-F-5-MeO-Ph | H | O | H | CH₂CH₂ | Methoxycarbonyl | |
| 7.609 | 3-F-5-Me-Ph | H | O | H | CH₂CH₂ | Methoxycarbonyl | |
| 7.610 | 3-F-Ph | H | O | H | CH₂CH₂ | Methoxycarbonyl | |
| 7.611 | 3-Me-Ph | H | O | H | CH₂CH₂ | Methoxycarbonyl | |
| 7.612 | 3-NO₂-Ph | H | O | H | CH₂CH₂ | Methoxycarbonyl | |
| 7.613 | 4-CI-Ph | H | O | H | CH₂CH₂ | Methoxycarbonyl | |
| 7.614 | 4-EtO-Ph | H | O | H | CH₂CH₂ | Methoxycarbonyl | |
| 7.615 | 4-MeO-Ph | H | O | H | CH₂CH₂ | Methoxycarbonyl | |
| 7.616 | F₅-Ph | H | O | H | CH₂CH₂ | Methoxycarbonyl | |
| 7.617 | Ph | H | O | H | CH₂CH₂ | Methoxycarbonyl | |
| 7.618 | 3,5-Cl₂-Ph | H | O | H | CH₂CH₂ | Methyl(methylsulfonyl) carbamoyl | |
| 7.619 | 3,5-F₂-Ph | H | O | H | CH₂CH₂ | Methyl(methylsulfonyl) carbamoyl | |
| 7.620 | 3,5-Cl₂-Ph | H | O | H | CH₂CH₂ | Methylcarbamoyl | |
| 7.621 | 3,5-Cl₂-Ph | H | O | H | CH₂CH₂ | Methylcarbamoyl | |
| 7.622 | 3-Cl-4-F-Ph | H | O | H | CH₂CH₂ | Methylcarbamoyl | |
| 7.623 | 3-Cl-4-Me-Ph | H | O | H | CH₂CH₂ | Methylcarbamoyl | |
| 7.624 | 3,5-Cl₂-Ph | H | O | H | CH₂CH₂ | Methylsulfanyl | |
| 7.625 | 3,5-Cl₂-Ph | H | O | H | CH₂CH₂ | Methylsulfonyl | |
| 7.626 | 3,5-Cl₂-Ph | H | O | H | CH₂CH₂ | Morpholin-4-yl | |
| 7.627 | 3,5-Cl₂-Ph | H | O | H | CH₂CH₂ | Morpholin-4-ylcarbonyl | |
| 7.628 | 3,5-Cl₂-Ph | H | O | H | CH₂CH₂ | Phosphono | |
| 7.629 | Ph | H | O | H | CH₂CH₂ | Phosphono | |
| 7.630 | 3,5-Cl₂-Ph | H | O | H | CH₂CH₂ | Piperidin-1-yl | |
| 7.631 | 3,5-Cl₂-Ph | H | O | H | CH₂CH₂ | Piperidin-1-ylcarbonyl | |
| 7.632 | 3,5-Cl₂-Ph | H | O | H | CH₂CH₂ | Propan-2-yloxy | |
| 7.633 | 3,5-Cl₂-Ph | H | O | H | CH₂CH₂ | Propoxy | |
| 7.634 | 3,5-Cl₂-Ph | H | O | H | CH₂CH₂ | Propoxycarbonyl | |
| 7.635 | 3,5-Cl₂-Ph | H | O | H | CH₂CH₂ | Pyrrolidin-1-yl | |
| 7.636 | 3,5-Cl₂-Ph | H | O | H | CH₂CH₂ | Sec-Butoxycarbonyl | |
| 7.637 | 3,5-F₂-Ph | H | O | H | CH₂CH₂ | Sulfamoyl | |
| 7.638 | 3,5-Cl₂-Ph | H | O | H | CH₂CH₂ | tert-Butoxycarbonyl | |
| 7.639 | 3,5-F₂-Ph | H | O | H | CH₂CH₂ | tert-Butoxycarbonyl | |
| 7.640 | 3,5-Cl₂-Ph | H | O | H | CH₂CH₂ | Tetrahydrofuran-2-yl | |
| 7.641 | 3,5-Cl₂-Ph | H | O | H | CH₂CH₂ | Trifluormethoxy | |
| 7.642 | 3,5-F₂-Ph | H | O | H | CH₂CH₂ | Trifluormethoxy | |
| 7.643 | 3-F-Ph | H | O | H | CH₂CH₂ | Trifluormethoxy | |
| 7.644 | Ph | H | O | H | CH₂CH₂ | Trifluormethoxy | |
| 7.645 | 3-CI-Ph | H | O | H | CH₂CH₂CH₂ | (Propan-2-yloxy)carbonyl | |
| 7.646 | 3-F-Ph | H | O | H | CH₂CH₂CH₂ | (Propan-2-yloxy)carbonyl | |
| 7.647 | 3,5-Cl₂-Ph | H | O | H | CH₂CH₂CH₂ | 2-Carboxyethyl | |
| 7.648 | 3,5-Cl₂-Ph | H | O | H | CH₂CH₂CH₂ | 2-Methyl-1,3-dioxolan-2-yl | |
| 7.649 | 3,5-F₂-Ph | H | O | H | CH₂CH₂CH₂ | 2-Methyl-1,3-dioxolan-2-yl | |
| 7.650 | Ph | H | O | H | CH₂CH₂CH₂ | 2-Methyl-1,3-dioxolan-2-yl | |
| 7.651 | 3,5-Cl₂-Ph | H | O | H | CH₂CH₂CH₂ | 2-Oxopyrrolidin-1-yl | |
| 7.652 | 3,5-Cl₂-Ph | H | O | H | CH₂CH₂CH₂ | CF3 | |
| 7.653 | 3,5-Cl₂-Ph | H | O | H | CH₂CH₂CH₂ | CH3 | |
| 7.654 | 3,5-F₂-Ph | H | O | H | CH₂CH₂CH₂ | CH3 | |
| 7.655 | 3,5-Cl₂-Ph | H | O | H | CH₂CH₂CH₂ | COOH | |
| 7.656 | 3,5-F₂-Ph | H | O | H | CH₂CH₂CH₂ | COOH | |
| 7.657 | 3-Br-5-CI-Ph | H | O | H | CH₂CH₂CH₂ | COOH | |
| 7.658 | 3-CI-Ph | H | O | H | CH₂CH₂CH₂ | COOH | |
| 7.659 | 3-F-Ph | H | O | H | CH₂CH₂CH₂ | COOH | |
| 7.660 | Ph | H | O | H | CH₂CH₂CH₂ | COOH | |
| 7.661 | 3,5-Cl₂-Ph | H | O | H | CH₂CH₂CH₂ | Dimethylamino | |
| 7.662 | Ph | H | O | Eth ylca rba moy I | CH₂CH₂CH₂ | Dimethylamino | |
| 7.663 | 3,5-Cl₂-Ph | H | O | H | CH₂CH₂CH₂ | Ethoxy | |
| 7.664 | 3,5-Cl₂-Ph | H | O | H | CH₂CH₂CH₂ | Ethoxycarbonyl | |
| 7.665 | 3-CI-Ph | H | O | H | CH₂CH₂CH₂ | Ethoxycarbonyl | |
| 7.666 | 3-F-Ph | H | O | H | CH₂CH₂CH₂ | Ethoxycarbonyl | |
| 7.667 | 3,5-Cl₂-Ph | H | O | H | CH₂CH₂CH₂ | Hydroxy | |
| 7.668 | 3,5-F₂-Ph | H | O | H | CH₂CH₂CH₂ | Hydroxy | |
| 7.669 | 3,5-Cl₂-Ph | H | O | H | CH₂CH₂CH₂ | Methoxy | |
| 7.670 | 3,5-F₂-Ph | H | O | H | CH₂CH₂CH₂ | Methoxycarbonyl | |
| 7.671 | 3-Br-5-CI-Ph | H | O | H | CH₂CH₂CH₂ | Methoxycarbonyl | |
| 7.672 | 3-CI-5-MeO-Ph | H | O | H | CH₂CH₂CH₂ | Methoxycarbonyl | |
| 7.673 | 3-CI-Ph | H | O | H | CH₂CH₂CH₂ | Methoxycarbonyl | |
| 7.674 | 3-F-Ph | H | O | H | CH₂CH₂CH₂ | Methoxycarbonyl | |
| 7.675 | Ph | H | O | H | CH₂CH₂CH₂ | Methoxycarbonyl | |
| 7.676 | 3,5-F₂-Ph | H | O | H | CH₂CH₂CH₂ | Methylcarbamoyl | |
| 7.677 | 3,5-Cl₂-Ph | H | O | H | CH₂CH₂CH₂ | Morpholin-4-yl | |
| 7.678 | 3,5-Cl₂-Ph | H | O | H | (CH₂)₆ | Methoxycarbonyl | |
| 7.679 | 3,5-F₂-Ph | H | O | H | CH(CH₃) | Cyan | [CDCl₃] D1 1.00 (t,3H); 1.58 (d,3H); 2.01 (m,1 H); 2.17 (m,1H); 3.25 (d,1H); 3.69 (d, 1H); 4.87 (m,1H); 6.88 (m,1H); 7.12 (d br,1H); 7.15 (m,2H). D2 1.05 (t,3H); 1.62 (d,3H); 2.01 (m,1H); 2.17 (m,1H); 3.28 (d,1H); 3.69 (d,1H); 4.87 (m,1H); 6.88 (m,1H); 7.12 (d br,1H); 7.15 (m,2H) |
| 7.680 | 3,5-F₂-Ph | H | O | H | CH(CH₃)CH₂ | Ethoxycarbonyl | [CDCl₃] D1 0.97 (t,3H); 1.20 (t,3H); 1.28 (d,3H); 1.93 (m,2H); 2.50 (d,2H); 3.19 (d,1H); 3.67 (d,1H); 4.08 (q,2H); 4.33 (m,1H); 6.88 (m,1H); 7.18 (m,3H). D2 1.00 (t,3H); 1.22 (t,3H); 1.29 (d,3H); 2.14 (m,2H); 2.55 (m,2H); 3.19 (d,1H); 3.70 (d,1H); 4.16 (q,2H); 4.33 (m,1H); 6.88 (m,1H); 7.18 (m,3H). |

| Tabelle 8: Erfindungsgemäße Verbindungen der allgemeinen Formel (I), worin R² für Wasserstoff und R³ für Trifluormethyl stehen, und Aryl den Rest 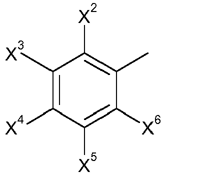 bedeutet. | | | | | | | |
|---|---|---|---|---|---|---|---|
| 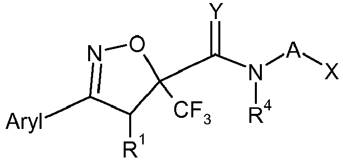 | | | | | | | |

| Nr. | Aryl | R¹ | Y | R⁴ | A | X | Physikalische Daten |
|---|---|---|---|---|---|---|---|
| 8.001 | 3,5-Cl₂-Ph | H | O | H | (CH₂)₄ | COOH | |
| 8.002 | 3,5-Cl₂-Ph | H | O | H | (CH₂)₄ | Ethoxycarbonyl | |
| 8.003 | 3-Br-5-Cl-Ph | H | O | H | (CH₂)₄ | Ethoxycarbonyl | |
| 8.004 | Ph | H | O | H | (CH₂)₄ | Ethoxycarbonyl | |
| 8.005 | 3,5-Cl₂-Ph | H | O | H | (CH₂)₄ | Hydroxy | |
| 8.006 | 3,5-Cl₂-Ph | H | O | H | (CH₂)₄ | Methoxy | |
| 8.007 | 3,5-Cl₂-Ph | H | O | H | (CH₂)₆ | COOH | |
| 8.008 | 3,5-Cl₂-Ph | H | O | H | (CH₂)₆ | Ethoxycarbonyl | |
| 8.009 | 3,5-Cl₂-Ph | H | O | H | c-Pr-1,1-diyl | Ethoxycarbonyl | |
| 8.010 | 3,5-F₂-Ph | H | O | H | Bindung | 1-Cyancyclopropyl | |
| 8.011 | 3,5-Cl₂-Ph | H | O | H | Bindung | 1-Cyanocyclopropyl | |
| 8.012 | 3,5-Cl₂-Ph | H | O | H | Bindung | 1-Methylcyclopropyl | |
| 8.013 | 3,5-Cl₂-Ph | H | O | H | Bindung | 2-(Ethoxycarbonyl)cyclohex-1-en-1-yl | |
| 8.014 | 3,5-Cl₂-Ph | H | O | H | Bindung | 2-(Ethoxycarbonyl)cyclohexyl | |
| 8.015 | 3,5-Cl₂-Ph | H | O | H | Bindung | 2-(Methylcarbamoyl)cyclohexyl | |
| 8.016 | 3,5-Cl₂-Ph | H | O | H | Bindung | 2-Carboxycyclohexyl | |
| 8.017 | 3,5-Cl₂-Ph | H | O | H | Bindung | 3-(2-Ethoxy-2-oxoethyl)oxetan-3-yl | |
| 8.018 | 3,5-F₂-Ph | H | O | H | Bindung | 3-(2-Ethoxy-2-oxoethyl)oxetan-3-yl | |
| 8.019 | 2,4-Cl₂-Ph | H | O | CH₃ | Bindung | CH3 | |
| 8.020 | 2-Cl-Ph | H | O | CH₃ | Bindung | CH3 | |
| 8.021 | 3,4-F₂-Ph | H | O | H | Bindung | CH3 | |
| 8.022 | 3,5-Br₂-Ph | H | O | H | Bindung | CH3 | |
| 8.023 | 3,5-Cl₂-Ph | CH₃ | O | H | Bindung | CH3 | |
| 8.024 | 3,5-Cl₂-Ph | H | O | CH₃ | Bindung | CH3 | |
| 8.025 | 3,5-Cl₂-Ph | H | O | H | Bindung | CH3 | [CDCl₃] 2.91 (d, 3H); 3.72 (d, 1H); 3.96 (d, 1H); 6.82 (bs, 1H); 7.47 (m, 1H); 7.54 (m, 2H). |
| 8.026 | 3,5-F₂-Ph | H | O | H | Bindung | CH3 | |
| 8.027 | 3-Cl-4-F-Ph | H | O | H | Bindung | CH3 | |
| 8.028 | 3,5-Cl₂-Ph | H | O | H | Bindung | Cyclobutyl | |
| 8.029 | 3,5-F₂-Ph | H | O | H | Bindung | Cyclobutyl | |
| 8.030 | 3,5-Cl₂-Ph | H | O | H | Bindung | Cyclopentyl | |
| 8.031 | 3,5-F₂-Ph | H | O | H | Bindung | Cyclopentyl | |
| 8.032 | 2-CF₃-Ph | H | O | H | Bindung | c-Pr | |
| 8.033 | 2,3,4-F₃-Ph | H | O | H | Bindung | c-Pr | |
| 8.034 | 2,3,5-F₃-Ph | H | O | H | Bindung | c-Pr | |
| 8.035 | 2,3-F₂-Ph | H | O | H | Bindung | c-Pr | |
| 8.036 | 2,4-Cl₂-Ph | H | O | H | Bindung | c-Pr | |
| 8.037 | 2,5-F₂-Ph | H | O | H | Bindung | c-Pr | |
| 8.038 | 2,5-Me₂-Ph | H | O | H | Bindung | c-Pr | |
| 8.039 | 2-Cl-3-F-5-MeO-Ph | H | O | H | Bindung | c-Pr | |
| 8.040 | 2-Cl-5-F-Ph | H | O | H | Bindung | c-Pr | |
| 8.041 | 2-EtO-3,4,5,6-F₄-Ph | H | O | H | Bindung | c-Pr | |
| 8.042 | 2-F-3-Me-Ph | H | O | H | Bindung | c-Pr | |
| 8.043 | 3-(2-MeOEtO)-Ph | H | O | H | Bindung | c-Pr | |
| 8.044 | 3-iPrO-Ph | H | O | H | Bindung | c-Pr | |
| 8.045 | 3-CF₃O-Ph | H | O | H | Bindung | c-Pr | |
| 8.046 | 3-CF₃-Ph | H | O | H | Bindung | c-Pr | |
| 8.047 | 3,4,5-F₃-Ph | H | O | H | Bindung | c-Pr | |
| 8.048 | 3,5-Br₂-Ph | H | O | H | Bindung | c-Pr | |
| 8.049 | 3,5-Cl₂-4-MeO-Ph | H | O | H | Bindung | c-Pr | |
| 8.050 | 3,5-Cl₂-4-OH-Ph | H | O | H | Bindung | c-Pr | |
| 8.051 | 3,5-Cl₂-Ph | CH₃ | O | H | Bindung | c-Pr | |
| 8.052 | 3,5-Cl₂-Ph | H | O | H | Bindung | c-Pr | [CDCl₃] 0.60 (m, 2H); 0.85 (m, 2H); 2.78 (m, 1H); 3.72 (d, 1H); 3.97 (d, 1H); 6.82 (s br, 1H); 7.48 (t, 1H); 7.53 (m, 2H) |
| 8.053 | 3,5-F₂-Ph | H | O | H | Bindung | c-Pr | |
| 8.054 | 3,5-F₂-Ph | H | O | H | Bindung | c-Pr | |
| 8.055 | 3,5-F₂-Ph | H | S | H | Bindung | c-Pr | |
| 8.056 | 3,5-(MeO)₂-Ph | H | O | H | Bindung | c-Pr | |
| 8.057 | 3,5-Me₂-Ph | H | O | H | Bindung | c-Pr | |
| 8.058 | 3-Ac-Ph | H | O | H | Bindung | c-Pr | |
| 8.059 | 3-Br-5-CF₃-Ph | H | O | H | Bindung | c-Pr | |
| 8.060 | 3-Br-5-Cl-Ph | H | O | H | Bindung | c-Pr | |
| 8.061 | 3-Br-5-F-Ph | H | O | H | Bindung | c-Pr | |
| 8.062 | 3-Cl-4-F-Ph | H | O | H | Bindung | c-Pr | [CDCl₃] 0.59 (m, 2H); 0.84 (m, 2H); 2.78 (m, 1H); 3.74 (d, 1H); 3.99 (d,1H); 6.85 (s br, 1H); 7.21 (m, 1H); 7.53 (m, 1H); 7.73 (m, 1H) |
| 8.063 | 3-Cl-4-Me-Ph | H | O | H | Bindung | c-Pr | |
| 8.064 | 3-Cl-5-CF₃-Ph | H | O | H | Bindung | c-Pr | |
| 8.065 | 3-Cl-5-CN-Ph | H | O | H | Bindung | c-Pr | |
| 8.066 | 3-Cl-5-Et-Ph | H | O | H | Bindung | c-Pr | |
| 8.067 | 3-Cl-5-F-Ph | H | O | H | Bindung | c-Pr | |
| 8.068 | 3-Cl-5-Me-Ph | H | O | H | Bindung | c-Pr | |
| 8.069 | 3-Cl-5-CF₃O-Ph | H | O | H | Bindung | c-Pr | |
| 8.070 | 3-Cl-Ph | H | O | H | Bindung | c-Pr | |
| 8.071 | 3-CN-5-F-Ph | H | O | H | Bindung | c-Pr | |
| 8.072 | 3-c-Pr-5-F-Ph | H | O | H | Bindung | c-Pr | |
| 8.073 | 3-EtO-Ph | H | O | H | Bindung | c-Pr | |
| 8.074 | 3-Et-5-F-Ph | H | O | H | Bindung | c-Pr | |
| 8.075 | 3-Et-Ph | H | O | H | Bindung | c-Pr | |
| 8.076 | 3-F-5-MeS-Ph | H | O | H | Bindung | c-Pr | |
| 8.077 | 3-F-5-MeSO₂-Ph | H | O | H | Bindung | c-Pr | |
| 8.078 | 3-F-5-MeO-Ph | H | O | H | Bindung | c-Pr | |
| 8.079 | 3-F-5-Me-Ph | H | O | H | Bindung | c-Pr | |
| 8.080 | 3-F-5-Me-Ph | H | S | H | Bindung | c-Pr | |
| 8.081 | 3-F-Ph | H | O | H | Bindung | c-Pr | |
| 8.082 | 3-OH-Ph | H | O | H | Bindung | c-Pr | |
| 8.083 | 3-iPr-Ph | H | O | H | Bindung | c-Pr | |
| 8.084 | 3-MeO-Ph | H | O | H | Bindung | c-Pr | |
| 8.085 | 3-Me-5-CF₃O-Ph | H | O | H | Bindung | c-Pr | |
| 8.086 | 3-Me-Ph | H | O | H | Bindung | c-Pr | |
| 8.087 | 3-NO₂-Ph | H | O | H | Bindung | c-Pr | |
| 8.088 | 4-Cl-3,5-F₂-Ph | H | O | H | Bindung | c-Pr | |
| 8.089 | 4-EtO-Ph | H | O | H | Bindung | c-Pr | |
| 8.090 | F₅-Ph | H | O | H | Bindung | c-Pr | |
| 8.091 | Ph | H | O | H | Bindung | c-Pr | |
| 8.092 | 3,5-Cl₂-Ph | H | O | H | Bindung | Decahydronaphthalen-2-yl | |
| 8.093 | 3,5-Cl₂-Ph | H | O | H | Bindung | H | |
| 8.094 | 3,5-F₂-Ph | H | O | H | Bindung | H | |
| 8.095 | 3,5-F₂-Ph | H | S | H | Bindung | H | |
| 8.096 | 3-F-Ph | H | O | H | Bindung | H | |
| 8.097 | 3-Me-Ph | H | O | H | Bindung | H | |
| 8.098 | 3-Me-Ph | H | S | H | Bindung | H | |
| 8.099 | 3,5-Cl₂-Ph | H | O | CH₃ | Bindung | Hydroxy | |
| 8.100 | 2,3,4-F₃-Ph | H | O | H | Bindung | Oxetan-3-yl | |
| 8.101 | 2,3-F₂-Ph | H | O | H | Bindung | Oxetan-3-yl | |
| 8.102 | 2,5-F₂-Ph | H | O | H | Bindung | Oxetan-3-yl | |
| 8.103 | 3,5-Cl₂-Ph | H | O | H | Bindung | Oxetan-3-yl | |
| 8.104 | 3,5-F₂-Ph | H | O | H | Bindung | Oxetan-3-Yl | |
| 8.105 | 3-Cl-5-CF₃O-Ph | H | O | H | Bindung | Oxetan-3-Yl | |
| 8.106 | 3-F-5-Me-Ph | H | O | H | Bindung | Oxetan-3-yl | |
| 8.107 | 3-F-Ph | H | O | H | Bindung | Oxetan-3-yl | |
| 8.108 | 3-NO₂-Ph | H | O | H | Bindung | Oxetan-3-yl | |
| 8.109 | Ph | H | O | H | Bindung | Oxetan-3-yl | |
| 8.110 | 3,5-Cl₂-Ph | H | O | H | Bindung | Tetrahydro-2H-pyran-4-yl | |
| 8.111 | 3,5-Cl₂-Ph | H | O | H | C(CH₃)₂ | 3-Methoxyprop-1-in-1-yl | |
| 8.112 | 3,5-Cl₂-Ph | H | O | H | C(CH₃)₂ | Carbamoyl | |
| 8.113 | 3,5-Cl₂-Ph | H | O | H | C(CH₃)₂ | CH3 | |
| 8.114 | 3-Cl-4-F-Ph | H | O | H | C(CH₃)₂ | CH3 | |
| 8.115 | 3,5-Cl₂-Ph | H | O | H | C(CH₃)₂ | COOH | |
| 8.116 | 3,5-(CF₃)₂-Ph | H | O | H | C(CH₃)₂ | Ethinyl | |
| 8.117 | 3,5-Cl₂-Ph | H | O | H | C(CH₃)₂ | Ethinyl | |
| 8.118 | 3,5-Cl₂-Ph | H | O | H | C(CH₃)₂ | Methoxycarbonyl | |
| 8.119 | 3,5-Cl₂-Ph | H | O | H | C(CH₃)₂ | Methylcarbamoyl | |
| 8.120 | 3-Cl-Ph | H | O | H | C(CH₃)₂C H₂ | (Propan-2-yloxy)carbonyl | |
| 8.121 | 3-F-Ph | H | O | H | C(CH₃)₂C H₂ | (Propan-2-yloxy)carbonyl | |
| 8.122 | 3,5-Cl₂-Ph | H | O | H | C(CH₃)₂C H₂ | COOH | |
| 8.123 | 3-CI-Ph | H | O | H | C(CH₃)₂C H₂ | COOH | |
| 8.124 | 3-F-Ph | H | O | H | C(CH₃)₂C H₂ | COOH | |
| 8.125 | 3,5-Cl₂-Ph | H | O | H | C(CH₃)₂C H₂ | Ethoxycarbonyl | |
| 8.126 | 3-Cl-Ph | H | O | H | C(CH₃)₂C H₂ | Ethoxycarbonyl | |
| 8.127 | 3-F-Ph | H | O | H | C(CH₃)₂C H₂ | Ethoxycarbonyl | |
| 8.128 | 3,5-Cl₂-Ph | H | O | H | C(CH₃)₂C H₂ | Hydroxy | |
| 8.129 | 3-Cl-Ph | H | O | H | C(CH₃)₂C H₂ | Methoxycarbonyl | |
| 8.130 | 3-F-Ph | H | O | H | C(CH₃)₂C H₂ | Methoxycarbonyl | |
| 8.131 | 3,5-Cl₂-Ph | H | O | H | C(CH₃)₂C H₂ | Methylsulfanyl | |
| 8.132 | 3,5-Cl₂-Ph | H | O | H | C(CH₃)₂C H₂ | Methylsulfonyl | |
| 8.133 | 2,4-Cl₂-Ph | H | O | H | C(iPr)CH₃ | Cyan | |
| 8.134 | 3,5-Cl₂-Ph | H | O | H | C(iPr)CH₃ | Cyan | |
| 8.135 | 3,5-Cl₂-Ph | H | O | H | CH(CF₃)C H₂ | Ethoxycarbonyl | |
| 8.136 | 3,5-Cl₂-Ph | H | O | H | CH(CH₂C H₂SCH₃) | Methoxycarbonyl | |
| 8.137 | 3,5-Cl₂-Ph | H | O | H | CH(CH₂C H₃)CH₂ | Cyan | |
| 8.138 | 3,5-Cl₂-Ph | H | O | H | CH(CH₂C H₃)CH₂ | Methoxycarbonyl | |
| 8.139 | 3,5-Cl₂-Ph | H | O | H | CH(CH₂iP r)CH₂ | Methoxycarbonyl | |
| 8.140 | 3,5-Cl₂-Ph | H | O | H | CH(CH₂O CH₃) | Methoxymethyl | |
| 8.141 | 3,5-Cl₂-Ph | H | O | H | CH(CH₃) | CF3 | |
| 8.142 | 2,3,4-F₃-Ph | H | O | H | CH(CH₃) | CH₃ | |
| 8.143 | 2,3,5-F₃-Ph | H | O | H | CH(CH₃) | CH₃ | |
| 8.144 | 2,3,6-Cl₃-Ph | H | O | H | CH(CH₃) | CH₃ | |
| 8.145 | 2,3-Cl₂-5-MeO-Ph | H | O | H | CH(CH₃) | CH₃ | |
| 8.146 | 2,3-Cl₂-Ph | H | O | H | CH(CH₃) | CH₃ | |
| 8.147 | 2,3-F₂-Ph | H | O | H | CH(CH₃) | CH₃ | |
| 8.148 | 2,5-Cl₂-Ph | H | O | H | CH(CH₃) | CH₃ | |
| 8.149 | 2,5-F₂-Ph | H | O | H | CH(CH₃) | CH₃ | |
| 8.150 | 2-F-Ph | H | O | H | CH(CH₃) | CH₃ | |
| 8.151 | 3-(CF₃CH₂O)-Ph | H | O | H | CH(CH₃) | CH₃ | |
| 8.152 | 3-(ClCH₂CH₂O)-Ph | H | O | H | CH(CH₃) | CH₃ | |
| 8.153 | 3-(2-MeOEtO)-Ph | H | O | H | CH(CH₃) | CH₃ | |
| 8.154 | 3-Me₂N-Ph | H | O | H | CH(CH₃) | CH₃ | |
| 8.155 | 3-iPrCOO-Ph | H | O | H | CH(CH₃) | CH₃ | |
| 8.156 | 3-iPrO-Ph | H | O | H | CH(CH₃) | CH₃ | |
| 8.157 | 3-CF₃O-Ph | H | O | H | CH(CH₃) | CH₃ | |
| 8.158 | 3,4-Cl₂-Ph | H | O | H | CH(CH₃) | CH₃ | |
| 8.159 | 3,5-(CF₃)₂-Ph | H | O | H | CH(CH₃) | CH₃ | |
| 8.160 | 3,5-Cl₂-Ph | CH₃ | O | H | CH(CH₃) | CH₃ | |
| 8.161 | 3,5-Cl₂-Ph | H | O | H | CH(CH₃) | CH₃ | |
| 8.162 | 3,5-Cl₂-Ph | H | O | OH | CH(CH₃) | CH₃ | |
| 8.163 | 3,5-Cl₂-Ph | H | S | H | CH(CH₃) | CH₃ | |
| 8.164 | 3,5-F₂-Ph | H | O | H | CH(CH₃) | CH₃ | |
| 8.165 | 3,5-Me₂-Ph | H | O | H | CH(CH₃) | CH₃ | |
| 8.166 | R002 | H | O | H | CH(CH₃) | CH₃ | |
| 8.167 | R003 | H | O | H | CH(CH₃) | CH₃ | |
| 8.168 | 3-CNCH₂ N(Me)-Ph | H | O | H | CH(CH₃) | CH₃ | |
| 8.169 | 3-Me₂NCONH-Ph | H | O | H | CH(CH₃) | CH₃ | |
| 8.170 | 3-Me₂NSO₂O-Ph | H | O | H | CH(CH₃) | CH₃ | |
| 8.171 | 3-EtNHCOO-Ph | H | O | H | CH(CH₃) | CH₃ | |
| 8.172 | 3-EtSO₂O-Ph | H | O | H | CH(CH₃) | CH₃ | |
| 8.173 | 3-MeSO₂NH Ph | H | O | H | CH(CH₃) | CH₃ | |
| 8.174 | 3-MeSO₂O-Ph | H | O | H | CH(CH₃) | CH₃ | |
| 8.175 | 3-tert.BuOCON H-Ph | H | O | H | CH(CH₃) | CH₃ | |
| 8.176 | 3-CF₃CONH-Ph | H | O | H | CH(CH₃) | CH₃ | |
| 8.177 | 3-AcO-5-Cl-Ph | H | O | H | CH(CH₃) | CH₃ | |
| 8.178 | 3-AcO-Ph | H | O | H | CH(CH₃) | CH₃ | |
| 8.179 | 3-NH₂-Ph | H | O | H | CH(CH₃) | CH₃ | |
| 8.180 | 3-Br-5-CF₃-Ph | H | O | H | CH(CH₃) | CH₃ | |
| 8.181 | 3-Br-5-CI-Ph | H | O | H | CH(CH₃) | CH₃ | |
| 8.182 | 3-Cl-4-F-Ph | H | O | H | CH(CH₃) | CH₃ | |
| 8.183 | 3-Cl-4-Me-Ph | H | O | H | CH(CH₃) | CH₃ | |
| 8.184 | 3-Cl-5-CF₃-Ph | H | O | H | CH(CH₃) | CH₃ | |
| 8.185 | R001 | H | O | H | CH(CH₃) | CH₃ | |
| 8.186 | 3-CI-5-F-Ph | H | O | H | CH(CH₃) | CH₃ | |
| 8.187 | 3-Cl-5-MeO-Ph | H | O | H | CH(CH₃) | CH₃ | |
| 8.188 | 3-Cl-5-Me-Ph | H | O | H | CH(CH₃) | CH₃ | |
| 8.189 | 3-Cl-5-(CF₃CH₂O)-Ph | H | O | H | CH(CH₃) | CH₃ | |
| 8.190 | 3-Cl-5-(ClCH₂CH₂O)-Ph | H | O | H | CH(CH₃) | CH₃ | |
| 8.191 | 3-Cl-5-(EtO COCH₂O)-Ph | H | O | H | CH(CH₃) | CH₃ | |
| 8.192 | 3-Cl-5-CF₃O-Ph | H | O | H | CH(CH₃) | CH₃ | |
| 8.193 | 3-Cl-5-Me₂NSO₂O-Ph | H | O | H | CH(CH₃) | CH₃ | |
| 8.194 | 3-Cl-5-(MeSO₂O)-Ph | H | O | H | CH(CH₃) | CH₃ | |
| 8.195 | 3-Cl-5-iPrO-Ph | H | O | H | CH(CH₃) | CH₃ | |
| 8.196 | 3-Cl-Ph | H | O | H | CH(CH₃) | CH₃ | |
| 8.197 | 3-EtO-Ph | H | O | H | CH(CH₃) | CH₃ | |
| 8.198 | 3-F-5-Me-Ph | H | O | H | CH(CH₃) | CH₃ | |
| 8.199 | 3-F-Ph | H | O | H | CH(CH₃) | CH₃ | |
| 8.200 | 3-OH-Ph | H | O | H | CH(CH₃) | CH₃ | |
| 8.201 | 3-MeO-Ph | H | O | H | CH(CH₃) | CH₃ | |
| 8.202 | 3-Me-Ph | H | O | H | CH(CH₃) | CH₃ | |
| 8.203 | 3-NO₂-Ph | H | O | H | CH(CH₃) | CH₃ | |
| 8.204 | F₅-Ph | H | O | H | CH(CH₃) | CH₃ | |
| 8.205 | Ph | H | O | H | CH(CH₃) | CH₃ | |
| 8.206 | 3,5-Cl₂-Ph | H | O | H | CH(CH₃) | COOH | |
| 8.207 | 3-Cl-4-F-Ph | H | O | H | CH(CH₃) | COOH | |
| 8.208 | 3,5-Cl₂-Ph | H | O | H | CH(CH₃) | c-Pr | |
| 8.209 | 3,5-F₂-Ph | H | O | H | CH(CH₃) | c-Pr | |
| 8.210 | Ph | H | O | H | CH(CH₃) | c-Pr | |
| 8.211 | 3-Cl-4-F-Ph | H | O | H | CH(CH₃) | Dimethylcarbamoyl | |
| 8.212 | 3,5-Cl₂-Ph | H | O | H | CH(CH₃) | Ethinyl | |
| 8.213 | 3,5-Cl₂-Ph | H | O | H | CH(CH₃) | Ethyl | |
| 8.214 | 3,5-Cl₂-Ph | CH₃ | O | H | CH(CH₃) | Methoxycarbonyl | |
| 8.215 | 3,5-Cl₂-Ph | H | O | H | CH(CH₃) | Methoxycarbonyl | |
| 8.216 | 3-Cl-4-F-Ph | H | O | H | CH(CH₃) | Methoxycarbonyl | |
| 8.217 | 3-Cl-4-F-Ph | H | O | H | CH(CH₃) | Methylcarbamoyl | |
| 8.218 | 3,5-Cl₂-Ph | H | O | H | CH(CH₃) | Pentyl | |
| 8.219 | 3,5-Cl₂-Ph | H | O | H | CH(CH₃) CH₂ | (2,2,2-Trifluoroethoxy)carbonyl | |
| 8.220 | 3,5-F₂-Ph | H | O | H | CH(CH₃) CH₂ | (2,2,2-Trifluoroethoxy)carbonyl | |
| 8.221 | 3,5-Cl₂-Ph | H | O | H | CH(CH₃) CH₂ | (2-Hydroxyethoxy)carbonyl | |
| 8.222 | 3,5-F₂-Ph | H | O | H | CH(CH₃) CH₂ | (2-Hydroxyethoxy)carbonyl | |
| 8.223 | 3,5-Cl₂-Ph | H | O | H | CH(CH₃) CH₂ | (Allyloxy)carbonyl | |
| 8.224 | 3,5-F₂-Ph | H | O | H | CH(CH₃) CH₂ | (Allyloxy)carbonyl | |
| 8.225 | 3,5-Cl₂-Ph | H | O | H | CH(CH₃) CH₂ | (Cyclopropylsulfonyl)(methyl)carbamoyl | |
| 8.226 | 3,5-F₂-Ph | H | O | H | CH(CH₃) CH₂ | (Cyclopropylsulfonyl)(methyl)carbamoyl | |
| 8.227 | 3,5-Cl₂-Ph | H | O | H | CH(CH₃) CH₂ | (Cyclopropylsulfonyl)carbamoyl | |
| 8.228 | 3,5-F₂-Ph | H | O | H | CH(CH₃) CH₂ | (Cyclopropylsulfonyl)carbamoyl | |
| 8.229 | 3,5-Cl₂-Ph | H | O | H | CH(CH₃) CH₂ | (Ethylsulfonyl)(methyl)carbamoyl | |
| 8.230 | 3,5-F₂-Ph | H | O | H | CH(CH₃) CH₂ | (Ethylsulfonyl)(methyl)carbamoyl | |
| 8.231 | 3,5-Cl₂-Ph | H | O | H | CH(CH₃) CH₂ | (Ethylsulfonyl)carbamoyl | |
| 8.232 | 3,5-F₂-Ph | H | O | H | CH(CH₃) CH₂ | (Ethylsulfonyl)carbamoyl | |
| 8.233 | 3,5-Cl₂-Ph | H | O | H | CH(CH₃) CH₂ | (Heptan-2-yloxy)carbonyl | |
| 8.234 | 3,5-F₂-Ph | H | O | H | CH(CH₃) CH₂ | (Heptan-2-yloxy)carbonyl | |
| 8.235 | 3,5-Cl₂-Ph | H | O | H | CH(CH₃) CH₂ | (Isopropylsulfonyl)(methyl)carbamoyl | |
| 8.236 | 3,5-F₂-Ph | H | O | H | CH(CH₃) CH₂ | (Isopropylsulfonyl)(methyl)carbamoyl | |
| 8.237 | 3,5-Cl₂-Ph | H | O | H | CH(CH₃) CH₂ | (Isopropylsulfonyl)carbamoyl | |
| 8.238 | 3,5-F₂-Ph | H | O | H | CH(CH₃) CH₂ | (Isopropylsulfonyl)carbamoyl | |
| 8.239 | 3,5-Cl₂-Ph | H | O | H | CH(CH₃) CH₂ | (Methylsulfonyl)carbamoyl | |
| 8.240 | 3,5-F₂-Ph | H | O | H | CH(CH₃) CH₂ | (Methylsulfonyl)carbamoyl | |
| 8.241 | 3,5-Cl₂-Ph | H | O | H | CH(CH₃) CH₂ | (Prop-2-Yn-1-yloxy)carbonyl | |
| 8.242 | 3,5-F₂-Ph | H | O | H | CH(CH₃) CH₂ | (Prop-2-Yn-1-yloxy)carbonyl | |
| 8.243 | 3,5-Cl₂-Ph | H | O | H | CH(CH₃) CH₂ | (Propan-2-yloxy)carbonyl | |
| 8.244 | 3,5-F₂-Ph | H | O | H | CH(CH₃) CH₂ | (Propan-2-yloxy)carbonyl | |
| 8.245 | 3-Cl-Ph | H | O | H | CH(CH₃) CH₂ | (Propan-2-yloxy)carbonyl | |
| 8.246 | 3-F-Ph | H | O | H | CH(CH₃) CH₂ | (Propan-2-yloxy)carbonyl | |
| 8.247 | 3,5-Cl₂-Ph | H | O | H | CH(CH₃) CH₂ | [2-(Methylsulfanyl)ethoxy]carbonyl | |
| 8.248 | 3,5-F₂-Ph | H | O | H | CH(CH₃) CH₂ | [2-(Methylsulfanyl)ethoxy]carbonyl | |
| 8.249 | 3,5-F₂-Ph | H | O | H | CH(CH₃) CH₂ | [2-(Methylsulfonyl)ethoxy]carbonyl | |
| 8.250 | 3,5-Cl₂-Ph | H | O | H | CH(CH₃) CH₂ | Butoxycarbonyl | |
| 8.251 | 3,5-F₂-Ph | H | O | H | CH(CH₃) CH₂ | Butoxycarbonyl | |
| 8.252 | 3-CF₃-Ph | H | O | H | CH(CH₃) CH₂ | COOH | |
| 8.253 | 3,5-Cl₂-Ph | H | O | H | CH(CH₃) CH₂ | COOH | |
| 8.254 | 3,5-F₂-Ph | H | O | H | CH(CH₃) CH₂ | COOH | |
| 8.255 | 3,5-(tert.Bu)₂-Ph | H | O | H | CH(CH₃) CH₂ | COOH | |
| 8.256 | 3-Br-5-CF₃-Ph | H | O | H | CH(CH₃) CH₂ | COOH | |
| 8.257 | 3-Cl-5-CF₃-Ph | H | O | H | CH(CH₃) CH₂ | COOH | |
| 8.258 | 3-Cl-Ph | H | O | H | CH(CH₃) CH₂ | COOH | |
| 8.259 | 3-Et-5-F-Ph | H | O | H | CH(CH₃) CH₂ | COOH | |
| 8.260 | 3-Et-Ph | H | O | H | CH(CH₃) CH₂ | COOH | |
| 8.261 | 3-F-5-MeO-Ph | H | O | H | CH(CH₃) CH₂ | COOH | |
| 8.262 | 3-F-Ph | H | O | H | CH(CH₃) CH₂ | COOH | |
| 8.263 | 3-Me-Ph | H | O | H | CH(CH₃) CH₂ | COOH | |
| 8.264 | 4-MeO-Ph | H | O | H | CH(CH₃) CH₂ | COOH | |
| 8.265 | 3-CF₃-Ph | H | O | H | CH(CH₃) CH₂ | Ethoxycarbonyl | |
| 8.266 | 3,5-Cl₂-4-MeO-Ph | H | O | H | CH(CH₃) CH₂ | Ethoxycarbonyl | |
| 8.267 | 3,5-Cl₂-Ph | H | O | H | CH(CH₃) CH₂ | Ethoxycarbonyl | |
| 8.268 | 3,5-Et₂-Ph | H | O | H | CH(CH₃) CH₂ | Ethoxycarbonyl | |
| 8.269 | 3,5-F₂-Ph | H | O | H | CH(CH₃) CH₂ | Ethoxycarbonyl | |
| 8.270 | 3,5-(MeO)₂-Ph | H | O | H | CH(CH₃) CH₂ | Ethoxycarbonyl | |
| 8.271 | 3,5-Me₂-Ph | H | O | H | CH(CH₃) CH₂ | Ethoxycarbonyl | |
| 8.272 | 3,5-(tert.Bu)₂-Ph | H | O | H | CH(CH₃) CH₂ | Ethoxycarbonyl | |
| 8.273 | 3-Br-5-CF₃-Ph | H | O | H | CH(CH₃) CH₂ | Ethoxycarbonyl | |
| 8.274 | 3-CI-5-Et-Ph | H | O | H | CH(CH₃) CH₂ | Ethoxycarbonyl | |
| 8.275 | 3-Cl-5-CF₃-Ph | H | O | H | CH(CH₃) CH₂ | Ethoxycarbonyl | |
| 8.276 | 3-Cl-Ph | H | O | H | CH(CH₃) CH₂ | Ethoxycarbonyl | |
| 8.277 | 3-c-Pr-5-F-Ph | H | O | H | CH(CH₃) CH₂ | Ethoxycarbonyl | |
| 8.278 | 3-Et-5-F-Ph | H | O | H | CH(CH₃) CH₂ | Ethoxycarbonyl | |
| 8.279 | 3-Et-Ph | H | O | H | CH(CH₃) CH₂ | Ethoxycarbonyl | |
| 8.280 | 3-F-5-MeS-Ph | H | O | H | CH(CH₃) CH₂ | Ethoxycarbonyl | |
| 8.281 | 3-F-5-MeSO₂-Ph | H | O | H | CH(CH₃) CH₂ | Ethoxycarbonyl | |
| 8.282 | 3-F-5-MeO-Ph | H | O | H | CH(CH₃) CH₂ | Ethoxycarbonyl | |
| 8.283 | 3-F-Ph | H | O | H | CH(CH₃) CH₂ | Ethoxycarbonyl | |
| 8.284 | 3-Me-5-CF₃O-Ph | H | O | H | CH(CH₃) CH₂ | Ethoxycarbonyl | |
| 8.285 | 3-Me-Ph | H | O | H | CH(CH₃) CH₂ | Ethoxycarbonyl | |
| 8.286 | 4-EtO-Ph | H | O | H | CH(CH₃) CH₂ | Ethoxycarbonyl | |
| 8.287 | 4-MeO-Ph | H | O | H | CH(CH₃) CH₂ | Ethoxycarbonyl | |
| 8.288 | 3,5-Cl₂-Ph | H | O | H | CH(CH₃) CH₂ | Hydroxy | |
| 8.289 | 3,5-Cl₂-Ph | H | O | H | CH(CH₃) CH₂ | Isobutoxycarbonyl | |
| 8.290 | 3,5-F₂-Ph | H | O | H | CH(CH₃) CH₂ | Isobutoxycarbonyl | |
| 8.291 | 3,5-Cl₂-Ph | H | O | H | CH(CH₃) CH₂ | Methoxy | |
| 8.292 | 2,5-Me₂-Ph | H | O | H | CH(CH₃) CH₂ | Methoxycarbonyl | |
| 8.293 | 2-Cl-3-F-5-MeO-Ph | H | O | H | CH(CH₃) CH₂ | Methoxycarbonyl | |
| 8.294 | 2-F-3-Me-Ph | H | O | H | CH(CH₃) CH₂ | Methoxycarbonyl | |
| 8.295 | 3-CF₃-Ph | H | O | H | CH(CH₃) CH₂ | Methoxycarbonyl | |
| 8.296 | 3,5-Cl₂-Ph | H | O | H | CH(CH₃) CH₂ | Methoxycarbonyl | |
| 8.297 | 3,5-F₂-Ph | H | O | H | CH(CH₃) CH₂ | Methoxycarbonyl | |
| 8.298 | 3-Cl-Ph | H | O | H | CH(CH₃) CH₂ | Methoxycarbonyl | |
| 8.299 | 3-F-5-MeO-Ph | H | O | H | CH(CH₃) CH₂ | Methoxycarbonyl | |
| 8.300 | 3-F-Ph | H | O | H | CH(CH₃) CH₂ | Methoxycarbonyl | |
| 8.301 | 3,5-Cl₂-Ph | H | O | H | CH(CH₃) CH₂ | Methyl(methylsulfonyl)carbamoyl | |
| 8.302 | 3,5-F₂-Ph | H | O | H | CH(CH₃) CH₂ | Methyl(methylsulfonyl)carbamoyl | |
| 8.303 | 3,5-Cl₂-Ph | H | O | H | CH(CH₃) CH₂ | Methylcarbamoyl | |
| 8.304 | 3,5-Cl₂-Ph | H | O | H | CH(CH₃) CH₂ | Propoxycarbonyl | |
| 8.305 | 3,5-F₂-Ph | H | O | H | CH(CH₃) CH₂ | Propoxycarbonyl | |
| 8.306 | 3,5-Cl₂-Ph | H | O | H | CH(CH₃) CH₂ | Sec-Butoxycarbonyl | |
| 8.307 | 3,5-F₂-Ph | H | O | H | CH(CH₃) CH₂ | Sec-Butoxycarbonyl | |
| 8.308 | 3,5-Cl₂-Ph | H | O | H | CH(COO CH₃)CH₂ | Methoxycarbonyl | |
| 8.309 | 3,5-Cl₂-Ph | H | O | H | CH(cyclo Pr) | c-Pr | |
| 8.310 | 3,5-F₂-Ph | H | O | H | CH(cyclo Pr) | c-Pr | |
| 8.311 | 3,5-Cl₂-Ph | H | O | H | CH(iPr) | Methoxycarbonyl | |
| 8.312 | 3,5-Cl₂-Ph | H | O | H | CH(iPr)C H₂ | Methoxycarbonyl | |
| 8.313 | 3,5-F₂-Ph | H | O | H | CH₂ | (2-Hydroxyethyl)carbamoyl | |
| 8.314 | 3,5-Cl₂-Ph | H | O | H | CH₂ | (Cyclopropylsulfonyl)carbamoyl | |
| 8.315 | 3,5-F₂-Ph | H | O | H | CH₂ | (Cyclopropylsulfonyl)carbamoyl | |
| 8.316 | 3,5-Cl₂-Ph | H | O | H | CH₂ | (Ethylsulfonyl)carbamoyl | |
| 8.317 | 3,5-F₂-Ph | H | O | H | CH₂ | (Ethylsulfonyl)carbamoyl | |
| 8.318 | 3,5-F₂-Ph | H | O | H | CH₂ | (Hydroxyimino)methyl | |
| 8.319 | 3,5-Cl₂-Ph | H | O | H | CH₂ | (Isopropylsufonyl)carbamoyl | |
| 8.320 | 3,5-F₂-Ph | H | O | H | CH₂ | (Isopropylsulfonyl)carbamoyl | |
| 8.321 | 3,5-Cl₂-Ph | H | O | H | CH₂ | (Methylsulfonyl)carbamoyl | |
| 8.322 | 3,5-F₂-Ph | H | O | H | CH₂ | (Methylsulfonyl)carbamoyl | |
| 8.323 | 3-Cl-Ph | H | O | H | CH₂ | (Propan-2-yloxy)carbonyl | |
| 8.324 | 3-F-Ph | H | O | H | CH₂ | (Propan-2-yloxy)carbonyl | |
| 8.325 | 3,5-Cl₂-Ph | H | O | H | CH₂ | 1,1-Dioxidotetrahydrothiophen-3-yl | |
| 8.326 | 3,5-F₂-Ph | H | O | H | CH₂ | 1,1-Dioxidotetrahydrothiophen-3-yl | |
| 8.327 | 3-F-Ph | H | O | H | CH₂ | 1,1-Dioxidotetrahydrothiophen-3-yl | |
| 8.328 | Ph | H | O | H | CH₂ | 1,1-Dioxidotetrahydrothiophen-3-yl | |
| 8.329 | Ph | H | O | H | CH₂ | 1,4-Dimethyl-5-oxo-4,5-dihydro-1H-1,2,4-triazol-3-yl | |
| 8.330 | 3,5-Cl₂-Ph | H | O | H | CH₂ | 3-(2-Ethoxy-2-oxoethyl)oxetan-3-yl | |
| 8.331 | 3,5-F₂-Ph | H | O | H | CH₂ | 3-(2-Ethoxy-2-oxoethyl)oxetan-3-yl | |
| 8.332 | 3,5-Cl₂-Ph | H | O | H | CH₂ | 3-(Cyclopropylcarbamoyl)-4,5-dihydro-1,2-oxazol-5-yl | |
| 8.333 | 3,5-Cl₂-Ph | H | O | H | CH₂ | 3-(Ethoxycarbonyl)-4,5-dihydro-1,2-oxazol-5-yl | |
| 8.334 | 3,5-Cl₂-Ph | H | O | H | CH₂ | 3-(Methylcarbamoyl)-4,5-dihydro-1,2-oxazol-5-yl | |
| 8.335 | 3,5-Cl₂-Ph | H | O | H | CH₂ | 3-Carboxy-4,5-dihydro-1,2-oxazol-5-yl | |
| 8.336 | 3,5-Cl₂-Ph | H | O | H | CH₂ | 3-Isopropyl-4,5-dihydro-1,2-oxazol-5-yl | |
| 8.337 | 3,5-Cl₂-Ph | H | O | H | CH₂ | 3-Methyl-4,5-dihydro-1,2-oxazol-5-yl | |
| 8.338 | 3,5-F₂-Ph | H | O | H | CH₂ | 5-(Ethoxycarbonyl)-4,5-dihydro-1,2-oxazol-3-yl | |
| 8.339 | 3,5-Cl₂-Ph | H | O | H | CH₂ | 5,5-Dimethyl-4,5-dihydro-1,2-oxazol-3-yl | |
| 8.340 | 3,5-Cl₂-Ph | H | O | H | CH₂ | 5-Ethyl-4,5-dihydro-1,2-oxazol-3-yl | |
| 8.341 | 3,5-Cl₂-Ph | H | O | H | CH₂ | 5-Isopropyl-4,5-dihydro-1,2-oxazol-3-yl | |
| 8.342 | 3,5-Cl₂-Ph | H | O | H | CH₂ | 5-Methyl-4,5-dihydro-1,2-oxazol-3-yl | |
| 8.343 | 3,5-Cl₂-Ph | H | O | H | CH₂ | Carbamoyl | |
| 8.344 | 3-Cl-Ph | H | O | H | CH₂ | Carbamoyl | |
| 8.345 | 2-CF₃-Ph | H | O | H | CH₂ | CF₃ | |
| 8.346 | 2,3,4-F₃-Ph | H | O | H | CH₂ | CF₃ | |
| 8.347 | 2,3,5-F₃-Ph | H | O | H | CH₂ | CF₃ | |
| 8.348 | 2,3,6-Cl₃-Ph | H | O | H | CH₂ | CF₃ | |
| 8.349 | 2,3-F₂-Ph | H | O | H | CH₂ | CF₃ | |
| 8.350 | 2,5-F₂-Ph | H | O | H | CH₂ | CF₃ | |
| 8.351 | 2-Cl-3-F-5-MeO-Ph | H | O | H | CH₂ | CF₃ | |
| 8.352 | 3-(2-MeOEtO)-Ph | H | O | H | CH₂ | CF₃ | |
| 8.353 | 3-iPrO-Ph | H | O | H | CH₂ | CF₃ | |
| 8.354 | 3-CF₃O-Ph | H | O | H | CH₂ | CF₃ | |
| 8.355 | 3-CF₃-Ph | H | O | H | CH₂ | CF₃ | |
| 8.356 | 3,4-Cl₂-Ph | H | O | H | CH₂ | CF₃ | |
| 8.357 | 3,5-Br₂-Ph | H | O | H | CH₂ | CF₃ | |
| 8.358 | 3,5-Cl₂-4-MeO-Ph | H | O | H | CH₂ | CF₃ | |
| 8.359 | 3,5-Cl₂-Ph | H | O | H | CH₂ | CF₃ | [CDCl₃] 3.78 (d, 1H); 4.00 (m, 3H); 7.10 (m, 1H); 7.50 (m, 1H); 7.56 (m, 2H). |
| 8.360 | 3,5-F₂-Ph | H | O | H | CH₂ | CF₃ | |
| 8.361 | 3,5-(MeO)₂-Ph | H | O | H | CH₂ | CF₃ | |
| 8.362 | 3,5-Me₂-Ph | H | O | H | CH₂ | CF₃ | |
| 8.363 | 3-Br-5-Cl-Ph | H | O | H | CH₂ | CF₃ | |
| 8.364 | 3-Br-5-F-Ph | H | O | H | CH₂ | CF₃ | |
| 8.365 | 3-Cl-5-CF₃-Ph | H | O | H | CH₂ | CF₃ | |
| 8.366 | 3-Cl-5-Et-Ph | H | O | H | CH₂ | CF₃ | |
| 8.367 | 3-Cl-5-F-Ph | H | O | H | CH₂ | CF₃ | |
| 8.368 | 3-Cl-5-Me-Ph | H | O | H | CH₂ | CF₃ | |
| 8.369 | 3-Cl-5-CF₃O-Ph | H | O | H | CH₂ | CF₃ | |
| 8.370 | 3-Cl-Ph | H | O | H | CH₂ | CF₃ | |
| 8.371 | 3-EtO-Ph | H | O | H | CH₂ | CF₃ | |
| 8.372 | 3-Et-5-F-Ph | H | O | H | CH₂ | CF₃ | |
| 8.373 | 3-Et-Ph | H | O | H | CH₂ | CF₃ | |
| 8.374 | 3-F-5-MeS-Ph | H | O | H | CH₂ | CF₃ | |
| 8.375 | 3-F-5-MeSO₂-Ph | H | O | H | CH₂ | CF₃ | |
| 8.376 | 3-F-5-CF₃-Ph | H | O | H | CH₂ | CF₃ | |
| 8.377 | 3-F-5-MeO-Ph | H | O | H | CH₂ | CF₃ | |
| 8.378 | 3-F-5-Me-Ph | H | O | H | CH₂ | CF₃ | |
| 8.379 | 3-F-Ph | H | O | H | CH₂ | CF₃ | |
| 8.380 | 3-OH-Ph | H | O | H | CH₂ | CF₃ | |
| 8.381 | 3-iPr-Ph | H | O | H | CH₂ | CF₃ | |
| 8.382 | 3-Me-Ph | H | O | H | CH₂ | CF₃ | |
| 8.383 | 3-NO₂-P | H | O | H | CH₂ | CF₃ | |
| 8.384 | 4-EtO-Ph | H | O | H | CH₂ | CF₃ | |
| 8.385 | 4-MeO-Ph | H | O | H | CH₂ | CF₃ | |
| 8.386 | F₅-Ph | H | O | H | CH₂ | CF₃ | |
| 8.387 | Ph | H | O | H | CH₂ | CF₃ | |
| 8.388 | 2,3,5-F₃-Ph | H | O | H | CH₂ | CH₃ | |
| 8.389 | 2,3-F₂-Ph | H | O | H | CH₂ | CH₃ | |
| 8.390 | 3,4-F₂-Ph | H | O | H | CH₂ | CH₃ | |
| 8.391 | 3,5-Cl₂-Ph | H | O | H | CH₂ | CH₃ | |
| 8.392 | 3,5-F₂-Ph | H | O | H | CH₂ | CH₃ | |
| 8.393 | 3-Cl-5-CF₃-Ph | H | O | H | CH₂ | CH₃ | |
| 8.394 | 3-Cl-5-Me-Ph | H | O | H | CH₂ | CH₃ | |
| 8.395 | 3-Cl-5-CF₃O-Ph | H | O | H | CH₂ | CH₃ | |
| 8.396 | 3-EtO-Ph | H | O | H | CH₂ | CH₃ | |
| 8.397 | 3-Et-Ph | H | O | H | CH₂ | CH₃ | |
| 8.398 | 3-F-Ph | H | O | H | CH₂ | CH₃ | |
| 8.399 | 3-Me-Ph | H | O | H | CH₂ | CH₃ | |
| 8.400 | F₅-Ph | H | O | H | CH₂ | CH₃ | |
| 8.401 | Ph | H | O | H | CH₂ | CH₃ | |
| 8.402 | 3,5-Cl₂-Ph | H | O | H | CH₂ | COOH | |
| 8.403 | 3,5-F₂-Ph | H | O | H | CH₂ | COOH | |
| 8.404 | 3-Cl-Ph | H | O | H | CH₂ | COOH | |
| 8.405 | 3-F-Ph | H | O | H | CH₂ | COOH | |
| 8.406 | 3,5-Cl₂-Ph | H | O | H | CH₂ | Cyan | |
| 8.407 | 3,5-F₂-Ph | H | O | H | CH₂ | Cyan | |
| 8.408 | 3-Cl-5-CF₃O-Ph | H | O | H | CH₂ | Cyan | |
| 8.409 | 3-F-5-Me-Ph | H | O | H | CH₂ | Cyan | |
| 8.410 | 3-F-Ph | H | O | H | CH₂ | Cyan | |
| 8.411 | 3-NO₂-Ph | H | O | H | CH₂ | Cyan | |
| 8.412 | 3,5-Cl₂-Ph | H | O | H | CH₂ | Cyclohexyl | |
| 8.413 | 3,5-Cl₂-Ph | H | O | H | CH₂ | c-Pr | |
| 8.414 | 3,5-F₂-Ph | H | O | H | CH₂ | c-Pr | |
| 8.415 | 3,5-Cl₂-Ph | H | O | H | CH₂ | Diethoxyphosphoryl | |
| 8.416 | 3,5-F₂-Ph | H | O | H | CH₂ | Diethoxyphosphoryl | |
| 8.417 | 3-F-Ph | H | O | H | CH₂ | Diethoxyphosphoryl | |
| 8.418 | Ph | H | O | H | CH₂ | Diethoxyphosphoryl | |
| 8.419 | 3,5-Cl₂-Ph | H | O | H | CH₂ | Difluormethyl | |
| 8.420 | 3,5-Cl₂-Ph | H | O | H | CH₂ | Dimethoxymethyl | |
| 8.421 | 3-Cl-Ph | H | O | H | CH₂ | Dimethoxymethyl | |
| 8.422 | 3,5-Cl₂-Ph | H | O | H | CH₂ | Dimethylcarbamoyl | |
| 8.423 | 3,5-Cl₂-Ph | H | O | H | CH₂ | Ethenyl | |
| 8.424 | 3,5-Cl₂-Ph | H | O | Prop-2-en-1-yl | CH₂ | Ethenyl | |
| 8.425 | 3,5-F₂-Ph | H | O | H | CH₂ | Ethenyl | |
| 8.426 | 3-F-Ph | H | O | H | CH₂ | Ethenyl | |
| 8.427 | Ph | H | O | H | CH₂ | Ethenyl | |
| 8.428 | 3,5-(CF₃)₂-Ph | H | O | H | CH₂ | Ethinyl | |
| 8.429 | 3,5-Cl₂-Ph | H | O | H | CH₂ | Ethinyl | |
| 8.430 | 3,5-Cl₂-Ph | H | O | Prop-2-in-1-yl | CH₂ | Ethinyl | |
| 8.431 | 3,5-F₂-Ph | H | O | H | CH₂ | Ethinyl | |
| 8.432 | 3,5-Cl₂-Ph | H | O | cPr | CH₂ | Ethoxycarbonyl | |
| 8.433 | 3-Cl-Ph | H | O | H | CH₂ | Ethoxycarbonyl | |
| 8.434 | 3-F-Ph | H | O | H | CH₂ | Ethoxycarbonyl | |
| 8.435 | 3,5-F₂-Ph | H | O | H | CH₂ | Formyl | |
| 8.436 | 3,5-Cl₂-Ph | H | O | H | CH₂ | Methoxycarbonyl | |
| 8.437 | 3,5-F₂-Ph | H | O | H | CH₂ | Methoxycarbonyl | |
| 8.438 | 3-Cl-Ph | H | O | H | CH₂ | Methoxycarbonyl | |
| 8.439 | 3-F-Ph | H | O | H | CH₂ | Methoxycarbonyl | |
| 8.440 | 3-CI-5-F-Ph | H | O | H | CH₂ | ony | |
| 8.441 | 3,5-Cl₂-Ph | H | O | H | CH₂ | Pentafluorethyl | |
| 8.442 | 3,5-Cl₂-Ph | H | O | H | CH₂ | Pentyl | |
| 8.443 | 3,5-F₂-Ph | H | O | H | CH₂ | Pentyl | |
| 8.444 | 3,5-Cl₂-Ph | H | O | H | CH₂ | Piperidin-2-yl | |
| 8.445 | 3,5-F₂-Ph | H | O | H | CH₂ | Piperidin-2-yl | |
| 8.446 | Ph | H | O | H | CH₂ | Piperidin-2-yl | |
| 8.447 | 3,5-Cl₂-Ph | H | O | CH₃ | CH₂ | Propan-2-yl | |
| 8.448 | 3,5-Cl₂-Ph | H | O | H | CH₂ | Propan-2-yl | |
| 8.449 | 3,5-Cl₂-Ph | H | O | H | CH₂ | Pyrrolidin-1-ylcarbonyl | |
| 8.450 | 3,5-Cl₂-Ph | H | O | H | CH₂ | tert-Butyl | |
| 8.451 | 3,5-F₂-Ph | H | O | H | CH₂ | tert-Butyl | |
| 8.452 | 3,5-Cl₂-Ph | H | O | H | CH₂ | Tetrahydrofuran-2-yl | |
| 8.453 | 3,5-F₂-Ph | H | O | H | CH₂ | Tetrahydrofuran-2-yl | |
| 8.454 | 3-F-Ph | H | O | H | CH₂ | Tetrahydrofuran-2-yl | |
| 8.455 | Ph | H | O | H | CH₂ | Tetrahydrofuran-2-yl | |
| 8.456 | 3,5-Cl₂-Ph | H | O | H | CH₂ | Tetrahydrofuran-3-yl | |
| 8.457 | 3,5-F₂-Ph | H | O | H | CH₂ | Tetrahydrofuran-3-yl | |
| 8.458 | 3-F-Ph | H | O | H | CH₂ | Tetrahydrofuran-3-yl | |
| 8.459 | Ph | H | O | H | CH₂ | Tetrahydrofuran-3-yl | |
| 8.460 | 3,5-Cl₂-Ph | H | O | H | CH₂C(CH ₃)₂ | COOH | |
| 8.461 | 3,5-Cl₂-Ph | H | O | H | CH₂C(CH ₃)₂ | Ethoxycarbonyl | |
| 8.462 | 3,5-Cl₂-Ph | H | O | H | CH₂CH(C H₃) | Ethoxycarbonyl | |
| 8.463 | 3,5-Cl₂-Ph | H | O | H | CH₂CH₂ | (2,2,2-Trifluoroethoxy)carbonyl | |
| 8.464 | 3,5-Cl₂-Ph | H | O | H | CH₂CH₂ | (2-Hydroxyethoxy)carbonyl | |
| 8.465 | 3,5-Cl₂-Ph | H | O | H | CH₂CH₂ | (2-Methoxy-2-oxoethyl)carbamoyl | |
| 8.466 | 3,5-Cl₂-Ph | H | O | H | CH₂CH₂ | (Allyloxy)carbonyl | |
| 8.467 | 3,5-Cl₂-Ph | H | O | H | CH₂CH₂ | (Cyclopropylsulfonyl)(methyl)carbamoyl | |
| 8.468 | 3,5-F₂-Ph | H | O | H | CH₂CH₂ | (Cyclopropylsulfonyl)(methyl)carbamoyl | |
| 8.469 | 3,5-Cl₂-Ph | H | O | H | CH₂CH₂ | (Cyclopropylsulfonyl)carbamoyl | |
| 8.470 | 3,5-F₂-Ph | H | O | H | CH₂CH₂ | (Cyclopropylsulfonyl)carbamoyl | |
| 8.471 | 3,5-Cl₂-Ph | H | O | H | CH₂CH₂ | (Ethylsulfonyl)(methyl)carbamoyl | |
| 8.472 | 3,5-F₂-Ph | H | O | H | CH₂CH₂ | (Ethylsulfonyl)(methyl)carbamoyl | |
| 8.473 | 3,5-Cl₂-Ph | H | O | H | CH₂CH₂ | (Ethylsulfonyl)carbamoyl | |
| 8.474 | 3,5-F₂-Ph | H | O | H | CH₂CH₂ | (Ethylsulfonyl)carbamoyl | |
| 8.475 | 3,5-Cl₂-Ph | H | O | H | CH₂CH₂ | (Heptan-2-yloxy)carbonyl | |
| 8.476 | 3,5-F₂-Ph | H | O | H | CH₂CH₂ | (Hydroxyimino)methyl | |
| 8.477 | 3,5-Cl₂-Ph | H | O | H | CH₂CH₂ | (Isopropylsulfonyl)(methyl)carbamoyl | |
| 8.478 | 3,5-F₂-Ph | H | O | H | CH₂CH₂ | (Isopropylsulfonyl)(methyl)carbamoyl | |
| 8.479 | 3,5-Cl₂-Ph | H | O | H | CH₂CH₂ | (Isopropylsulfonyl)carbamoyl | |
| 8.480 | 3,5-F₂-Ph | H | O | H | CH₂CH₂ | (Isopropylsulfonyl)carbamoyl | |
| 8.481 | 3,5-Cl₂-Ph | H | O | H | CH₂CH₂ | (Methylsulfonyl)carbamoyl | |
| 8.482 | 3,5-F₂-Ph | H | O | H | CH₂CH₂ | (Methylsulfonyl)carbamoyl | |
| 8.483 | 3,5-Cl₂-Ph | H | O | H | CH₂CH₂ | (Prop-2-Yn-1-yloxy)carbonyl | |
| 8.484 | 3,5-Cl₂-Ph | H | O | H | CH₂CH₂ | (Propan-2-yloxy)carbonyl | |
| 8.485 | 3,5-Cl₂-Ph | H | O | H | CH₂CH₂ | [2-(Methylsulfanyl)ethoxy]carbonyl | |
| 8.486 | 3,5-Cl₂-Ph | H | O | H | CH₂CH₂ | 1,3-Dioxolan-2-yl | |
| 8.487 | 3,5-F₂-Ph | H | O | H | CH₂CH₂ | 1,3-Dioxolan-2-yl | |
| 8.488 | 3-F-Ph | H | O | H | CH₂CH₂ | 1,3-Dioxolan-2-yl | |
| 8.489 | Ph | H | O | H | CH₂CH₂ | 1,3-Dioxolan-2-yl | |
| 8.490 | 3,5-Cl₂-Ph | H | O | H | CH₂CH₂ | 1-Methylpyrrolidin-2-yl | |
| 8.491 | 3,5-Cl₂-Ph | H | O | H | CH₂CH₂ | 2-Oxopyrrolidin-1-yl | |
| 8.492 | 3,5-Cl₂-Ph | H | O | H | CH₂CH₂ | Butoxycarbonyl | |
| 8.493 | 3,4,5-F₃-Ph | H | O | H | CH₂CH₂ | Butylcarbamoyl | |
| 8.494 | 3,5-Cl₂-Ph | H | O | H | CH₂CH₂ | Carbamoyl | |
| 8.495 | 3-Cl-4-Me-Ph | H | O | H | CH₂CH₂ | Carbamoyl | |
| 8.496 | 3,5-Cl₂-Ph | H | O | H | CH₂CH₂ | CF3 | |
| 8.497 | 3,5-F₂-Ph | H | O | H | CH₂CH₂ | CF3 | |
| 8.498 | 3,5-Cl₂-Ph | H | O | H | CH₂CH₂ | CH3 | |
| 8.499 | 3,5-F₂-Ph | H | O | H | CH₂CH₂ | CH3 | |
| 8.500 | 3,5-Cl₂-Ph | H | O | H | CH₂CH₂ | Chlor | |
| 8.501 | 2,3,4,5-F₄-6-OH-Ph | H | O | H | CH₂CH₂ | COOH | |
| 8.502 | 2,3,5-F₃-Ph | H | O | H | CH₂CH₂ | COOH | |
| 8.503 | 2,3-F₂-Ph | H | O | H | CH₂CH₂ | COOH | |
| 8.504 | 2,5-Cl₂-Ph | H | O | H | CH₂CH₂ | COOH | |
| 8.505 | 2,5-F₂-Ph | H | O | H | CH₂CH₂ | COOH | |
| 8.506 | 2-Cl-5-F-Ph | H | O | H | CH₂CH₂ | COOH | |
| 8.507 | 2-F-3-Me-Ph | H | O | H | CH₂CH₂ | COOH | |
| 8.508 | 3-CF₃-Ph | H | O | H | CH₂CH₂ | COOH | |
| 8.509 | 3,4,5-F₃-Ph | H | O | H | CH₂CH₂ | COOH | |
| 8.510 | 3,4-F₂-Ph | H | O | H | CH₂CH₂ | COOH | |
| 8.511 | 3,5-Br₂-Ph | H | O | H | CH₂CH₂ | COOH | |
| 8.512 | 3,5-Cl₂-Ph | H | O | CH₃ | CH₂CH₂ | COOH | |
| 8.513 | 3,5-Cl₂-Ph | H | O | H | CH₂CH₂ | COOH | |
| 8.514 | 3,5-F₂-Ph | H | O | H | CH₂CH₂ | COOH | |
| 8.515 | 3,5-Me₂-Ph | H | O | H | CH₂CH₂ | COOH | |
| 8.516 | 3,5-(tert.Bu)₂-Ph | H | O | H | CH₂CH₂ | COOH | |
| 8.517 | 3-Br-5-Cl-Ph | H | O | H | CH₂CH₂ | COOH | |
| 8.518 | 3-Br-5-F-Ph | H | O | H | CH₂CH₂ | COOH | |
| 8.519 | 3-Br-5-CF₃-Ph | H | O | H | CH₂CH₂ | COOH | |
| 8.520 | 3-Cl-4-F-Ph | H | O | H | CH₂CH₂ | COOH | |
| 8.521 | 3-Cl-4-Me-Ph | H | O | H | CH₂CH₂ | COOH | |
| 8.522 | 3-Cl-5-F-Ph | H | O | H | CH₂CH₂ | COOH | |
| 8.523 | 3-Cl-5-CF₃O-Ph | H | O | H | CH₂CH₂ | COOH | |
| 8.524 | 3-Cl-5-CF₃-Ph | H | O | H | CH₂CH₂ | COOH | |
| 8.525 | 3-Cl-5-Me-Ph | H | O | H | CH₂CH₂ | COOH | |
| 8.526 | 3-Et-5-F-Ph | H | O | H | CH₂CH₂ | COOH | |
| 8.527 | 3-Et-Ph | H | O | H | CH₂CH₂ | COOH | |
| 8.528 | 3-F-5-MeO-Ph | H | O | H | CH₂CH₂ | COOH | |
| 8.529 | 3-F-5-Me-Ph | H | O | H | CH₂CH₂ | COOH | |
| 8.530 | 3-F-Ph | H | O | H | CH₂CH₂ | COOH | |
| 8.531 | 3-Me-Ph | H | O | H | CH₂CH₂ | COOH | |
| 8.532 | 4-Cl-Ph | H | O | H | CH₂CH₂ | COOH | |
| 8.533 | 4-EtO-Ph | H | O | H | CH₂CH₂ | COOH | |
| 8.534 | Ph | H | O | H | CH₂CH₂ | COOH | |
| 8.535 | 3,5-Cl₂-Ph | H | O | H | CH₂CH₂ | Cyan | |
| 8.536 | 3-Cl-Ph | H | O | H | CH₂CH₂ | Cyan | |
| 8.537 | 3,5-Cl₂-Ph | H | O | H | CH₂CH₂ | Cyclopropylcarbamoyl | |
| 8.538 | 3,5-Cl₂-Ph | H | O | H | CH₂CH₂ | Diethoxymethyl | |
| 8.539 | 3,5-Cl₂-Ph | H | O | H | CH₂CH₂ | Diethoxyphosphoryl | |
| 8.540 | Ph | H | O | H | CH₂CH₂ | Diethoxyphosphoryl | |
| 8.541 | 3,5-Cl₂-Ph | H | O | H | CH₂CH₂ | Dimethoxymethyl | |
| 8.542 | 3,5-Cl₂-Ph | H | O | H | CH₂CH₂ | Dimethylcarbamoyl | |
| 8.543 | 3,5-Cl₂-Ph | H | O | H | CH₂CH₂ | Ethoxy | |
| 8.544 | 3-Cl-Ph | H | O | H | CH₂CH₂ | Ethoxy | |
| 8.545 | 2-Cl-5-F-Ph | H | O | H | CH₂CH₂ | Ethoxycarbonyl | |
| 8.546 | 3-CF₃-Ph | H | O | H | CH₂CH₂ | Ethoxycarbonyl | |
| 8.547 | 3,5-Cl₂-Ph | H | O | cPr | CH₂CH₂ | Ethoxycarbonyl | |
| 8.548 | 3,5-Cl₂-Ph | H | O | H | CH₂CH₂ | Ethoxycarbonyl | |
| 8.549 | 3,5-F₂-Ph | H | O | H | CH₂CH₂ | Ethoxycarbonyl | |
| 8.550 | 3-Br-5-CF₃-Ph | H | O | H | CH₂CH₂ | Ethoxycarbonyl | |
| 8.551 | 3-Cl-5-Et-Ph | H | O | H | CH₂CH₂ | Ethoxycarbonyl | |
| 8.552 | 3-Cl-5-CF₃-Ph | H | O | H | CH₂CH₂ | Ethoxycarbonyl | |
| 8.553 | 3-Et-5-F-Ph | H | O | H | CH₂CH₂ | Ethoxycarbonyl | |
| 8.554 | 3-Et-Ph | H | O | H | CH₂CH₂ | Ethoxycarbonyl | |
| 8.555 | 3-F-5-MeO-Ph | H | O | H | CH₂CH₂ | Ethoxycarbonyl | |
| 8.556 | 3-Me-5-CF₃O-Ph | H | O | H | CH₂CH₂ | Ethoxycarbonyl | |
| 8.557 | 3,5-Cl₂-Ph | H | O | H | CH₂CH₂ | Ethylcarbamoyl | |
| 8.558 | 3,5-F₂-Ph | H | O | H | CH₂CH₂ | Formyl | |
| 8.559 | 3,5-Cl₂-Ph | H | O | H | CH₂CH₂ | Hydroxy | |
| 8.560 | 3,5-F₂-Ph | H | O | H | CH₂CH₂ | Hydroxy | |
| 8.561 | 3-Cl-Ph | H | O | H | CH₂CH₂ | Hydroxy | |
| 8.562 | 3,5-Cl₂-Ph | H | O | H | CH₂CH₂ | Isobutoxycarbonyl | |
| 8.563 | 3,5-Cl₂-Ph | H | O | H | CH₂CH₂ | Methoxy | |
| 8.564 | 3-Cl-5-CF₃-Ph | H | O | H | CH₂CH₂ | Methoxy | |
| 8.565 | 3-Cl-Ph | H | O | H | CH₂CH₂ | Methoxy | |
| 8.566 | 2,3,4-F₃-Ph | H | O | H | CH₂CH₂ | Methoxycarbonyl | |
| 8.567 | 2,3,5-F₃-Ph | H | O | H | CH₂CH₂ | Methoxycarbonyl | |
| 8.568 | 2,3-F₂-Ph | H | O | H | CH₂CH₂ | Methoxycarbonyl | |
| 8.569 | 2,5-Cl₂-Ph | H | O | H | CH₂CH₂ | Methoxycarbonyl | |
| 8.570 | 2,5-F₂-Ph | H | O | H | CH₂CH₂ | Methoxycarbonyl | |
| 8.571 | 2,5-Me₂-Ph | H | O | H | CH₂CH₂ | Methoxycarbonyl | |
| 8.572 | 2-Cl-3-F-5-MeO-Ph | H | O | H | CH₂CH₂ | Methoxycarbonyl | |
| 8.573 | 2-F-3-Me-Ph | H | O | H | CH₂CH₂ | Methoxycarbonyl | |
| 8.574 | 3-CF₃-Ph | H | O | H | CH₂CH₂ | Methoxycarbonyl | |
| 8.575 | 3,4,5-F₃-Ph | H | O | H | CH₂CH₂ | Methoxycarbonyl | |
| 8.576 | 3,4-F₂-Ph | H | O | H | CH₂CH₂ | Methoxycarbonyl | |
| 8.577 | 3,5-Br₂-Ph | H | O | H | CH₂CH₂ | Methoxycarbonyl | |
| 8.578 | 3,5-Cl₂-4-MeO-Ph | H | O | H | CH₂CH₂ | Methoxycarbonyl | |
| 8.579 | 3,5-Cl₂-Ph | CH₃ | O | H | CH₂CH₂ | Methoxycarbonyl | |
| 8.580 | 3,5-Cl₂-Ph | H | O | CH₃ | CH₂CH₂ | Methoxycarbonyl | |
| 8.581 | 3,5-Cl₂-Ph | H | O | H | CH₂CH₂ | Methoxycarbonyl | |
| 8.582 | 3,5-Et₂-Ph | H | O | H | CH₂CH₂ | Methoxycarbonyl | |
| 8.583 | 3,5-F₂-Ph | H | O | H | CH₂CH₂ | Methoxycarbonyl | |
| 8.584 | 3,5-(MeO)₂-Ph | H | O | H | CH₂CH₂ | Methoxycarbonyl | |
| 8.585 | 3,5-Me₂-Ph | H | O | H | CH₂CH₂ | Methoxycarbonyl | |
| 8.586 | 3,5-(tert.Bu)₂-Ph | H | O | H | CH₂CH₂ | Methoxycarbonyl | |
| 8.587 | 3-CF₃S-Ph | H | O | H | CH₂CH₂ | Methoxycarbonyl | |
| 8.588 | 3-Ac-Ph | H | O | H | CH₂CH₂ | Methoxycarbonyl | |
| 8.589 | 3-Br-5-Cl-Ph | H | O | H | CH₂CH₂ | Methoxycarbonyl | |
| 8.590 | 3-Br-5-F-Ph | H | O | H | CH₂CH₂ | Methoxycarbonyl | |
| 8.591 | 3-Br-5-CF₃-Ph | H | O | H | CH₂CH₂ | Methoxycarbonyl | |
| 8.592 | 3-NH₂CO-Ph | H | O | H | CH₂CH₂ | Methoxycarbonyl | |
| 8.593 | 3-Cl-4-F-Ph | H | O | H | CH₂CH₂ | Methoxycarbonyl | |
| 8.594 | 3-Cl-4-Me-Ph | H | O | H | CH₂CH₂ | Methoxycarbonyl | |
| 8.595 | 3-Cl-5-CN-Ph | H | O | H | CH₂CH₂ | Methoxycarbonyl | |
| 8.596 | 3-Cl-5-Et-Ph | H | O | H | CH₂CH₂ | Methoxycarbonyl | |
| 8.597 | 3-Cl-5-F-Ph | H | O | H | CH₂CH₂ | Methoxycarbonyl | |
| 8.598 | 3-Cl-5-MeO-Ph | H | O | H | CH₂CH₂ | Methoxycarbonyl | |
| 8.599 | 3-Cl-5-CF₃O-Ph | H | O | H | CH₂CH₂ | Methoxycarbonyl | |
| 8.600 | 3-Cl-5-CF₃-Ph | H | O | H | CH₂CH₂ | Methoxycarbonyl | |
| 8.601 | 3-Cl-5-Me-Ph | H | O | H | CH₂CH₂ | Methoxycarbonyl | |
| 8.602 | 3-CN-5-F-Ph | H | O | H | CH₂CH₂ | Methoxycarbonyl | |
| 8.603 | 3-CN-Ph | H | O | H | CH₂CH₂ | Methoxycarbonyl | |
| 8.604 | 3-c-Pr-5-F-Ph | H | O | H | CH₂CH₂ | Methoxycarbonyl | |
| 8.605 | 3-Et-5-F-Ph | H | O | H | CH₂CH₂ | Methoxycarbonyl | |
| 8.606 | 3-F-5-MeS-Ph | H | O | H | CH₂CH₂ | Methoxycarbonyl | |
| 8.607 | 3-F-5-MeSO₂-Ph | H | O | H | CH₂CH₂ | Methoxycarbonyl | |
| 8.608 | 3-F-5-MeO-Ph | H | O | H | CH₂CH₂ | Methoxycarbonyl | |
| 8.609 | 3-F-5-Me-Ph | H | O | H | CH₂CH₂ | Methoxycarbonyl | |
| 8.610 | 3-F-Ph | H | O | H | CH₂CH₂ | Methoxycarbonyl | |
| 8.611 | 3-Me-Ph | H | O | H | CH₂CH₂ | Methoxycarbonyl | |
| 8.612 | 3-NO₂-Ph | H | O | H | CH₂CH₂ | Methoxycarbonyl | |
| 8.613 | 4-Cl-Ph | H | O | H | CH₂CH₂ | Methoxycarbonyl | |
| 8.614 | 4-EtO-Ph | H | O | H | CH₂CH₂ | Methoxycarbonyl | |
| 8.615 | 4-MeO-Ph | H | O | H | CH₂CH₂ | Methoxycarbonyl | |
| 8.616 | F₅-Ph | H | O | H | CH₂CH₂ | Methoxycarbonyl | |
| 8.617 | Ph | H | O | H | CH₂CH₂ | Methoxycarbonyl | |
| 8.618 | 3,5-Cl₂-Ph | H | O | H | CH₂CH₂ | Methyl(methylsulfonyl)carbamoyl | |
| 8.619 | 3,5-F₂-Ph | H | O | H | CH₂CH₂ | Methyl(methylsulfonyl)carbamoyl | |
| 8.620 | 2,5-Cl₂-Ph | H | O | H | CH₂CH₂ | Methylcarbamoyl | |
| 8.621 | 3,5-Cl₂-Ph | H | O | H | CH₂CH₂ | Methylcarbamoyl | |
| 8.622 | 3-Cl-4-F-Ph | H | O | H | CH₂CH₂ | Methylcarbamoyl | |
| 8.623 | 3-Cl-4-Me-Ph | H | O | H | CH₂CH₂ | Methylcarbamoyl | |
| 8.624 | 3,5-Cl₂-Ph | H | O | H | CH₂CH₂ | Methylsulfanyl | |
| 8.625 | 3,5-Cl₂-Ph | H | O | H | CH₂CH₂ | Methylsulfonyl | |
| 8.626 | 3,5-Cl₂-Ph | H | O | H | CH₂CH₂ | Morpholin-4-yl | |
| 8.627 | 3,5-Cl₂-Ph | H | O | H | CH₂CH₂ | Morpholin-4-ylcarbonyl | |
| 8.628 | 3,5-Cl₂-Ph | H | O | H | CH₂CH₂ | Phosphono | |
| 8.629 | Ph | H | O | H | CH₂CH₂ | Phosphono | |
| 8.630 | 3,5-Cl₂-Ph | H | O | H | CH₂CH₂ | Piperidin-1-yl | |
| 8.631 | 3,5-Cl₂-Ph | H | O | H | CH₂CH₂ | Piperidin-1-ylcarbonyl | |
| 8.632 | 3,5-Cl₂-Ph | H | O | H | CH₂CH₂ | Propan-2-yloxy | |
| 8.633 | 3,5-Cl₂-Ph | H | O | H | CH₂CH₂ | Propoxy | |
| 8.634 | 3,5-Cl₂-Ph | H | O | H | CH₂CH₂ | Propoxycarbonyl | |
| 8.635 | 3,5-Cl₂-Ph | H | O | H | CH₂CH₂ | Pyrrolidin-1-yl | |
| 8.636 | 3,5-Cl₂-Ph | H | O | H | CH₂CH₂ | Sec-Butoxycarbonyl | |
| 8.637 | 3,5-F₂-Ph | H | O | H | CH₂CH₂ | Sulfamoyl | |
| 8.638 | 3,5-Cl₂-Ph | H | O | H | CH₂CH₂ | tert-Butoxycarbonyl | |
| 8.639 | 3,5-F₂-Ph | H | O | H | CH₂CH₂ | tert-Butoxycarbonyl | |
| 8.640 | 3,5-Cl₂-Ph | H | O | H | CH₂CH₂ | Tetrahydrofuran-2-yl | |
| 8.641 | 3,5-Cl₂-Ph | H | O | H | CH₂CH₂ | Trifluormethoxy | |
| 8.642 | 3,5-F₂-Ph | H | O | H | CH₂CH₂ | Trifluormethoxy | |
| 8.643 | 3-F-Ph | H | O | H | CH₂CH₂ | Trifluormethoxy | |
| 8.644 | Ph | H | O | H | CH₂CH₂ | Trifluormethoxy | |
| 8.645 | 3-CI-Ph | H | O | H | (CH₂)₃ | (Propan-2-yloxy)carbonyl | |
| 8.646 | 3-F-Ph | H | O | H | (CH₂)₃ | (Propan-2-yloxy)carbonyl | |
| 8.647 | 3,5-Cl₂-Ph | H | O | H | (CH₂)₃ | 2-Carboxyethyl | |
| 8.648 | 3,5-Cl₂-Ph | H | O | H | (CH₂)₃ | 2-Methyl-1,3-dioxolan-2-yl | |
| 8.649 | 3,5-F₂-Ph | H | O | H | (CH₂)₃ | 2-Methyl-1,3-dioxolan-2-yl | |
| 8.650 | Ph | H | O | H | (CH₂)₃ | 2-Methyl-1,3-dioxolan-2-yl | |
| 8.651 | 3,5-Cl₂-Ph | H | O | H | (CH₂)₃ | 2-Oxopyrrolidin-1-yl | |
| 8.652 | 3,5-Cl₂-Ph | H | O | H | (CH₂)₃ | CF3 | |
| 8.653 | 3,5-Cl₂-Ph | H | O | H | (CH₂)₃ | CH3 | |
| 8.654 | 3,5-F₂-Ph | H | O | H | (CH₂)₃ | CH3 | |
| 8.655 | 3,5-Cl₂-Ph | H | O | H | (CH₂)₃ | COOH | |
| 8.656 | 3,5-F₂-Ph | H | O | H | (CH₂)₃ | COOH | |
| 8.657 | 3-Br-5-Cl-Ph | H | O | H | (CH₂)₃ | COOH | |
| 8.658 | 3-Cl-Ph | H | O | H | (CH₂)₃ | COOH | |
| 8.659 | 3-F-Ph | H | O | H | (CH₂)₃ | COOH | |
| 8.660 | Ph | H | O | H | (CH₂)₃ | COOH | |
| 8.661 | 3,5-Cl₂-Ph | H | O | H | (CH₂)₃ | Dimethylamino | |
| 8.662 | Ph | H | O | Etcarbam oyl | (CH₂)₃ | Dimethylamino | |
| 8.663 | 3,5-Cl₂-Ph | H | O | H | (CH₂)₃ | Ethoxy | |
| 8.664 | 3,5-Cl₂-Ph | H | O | H | (CH₂)₃ | Ethoxycarbonyl | |
| 8.665 | 3-Cl-Ph | H | O | H | (CH₂)₃ | Ethoxycarbonyl | |
| 8.666 | 3-F-Ph | H | O | H | (CH₂)₃ | Ethoxycarbonyl | |
| 8.667 | 3,5-Cl₂-Ph | H | O | H | (CH₂)₃ | Hydroxy | |
| 8.668 | 3,5-F₂-Ph | H | O | H | (CH₂)₃ | Hydroxy | |
| 8.669 | 3,5-Cl₂-Ph | H | O | H | (CH₂)₃ | Methoxy | |
| 8.670 | 3,5-F₂-Ph | H | O | H | (CH₂)₃ | Methoxycarbonyl | |
| 8.671 | 3-Br-5-Cl-Ph | H | O | H | (CH₂)₃ | Methoxycarbonyl | |
| 8.672 | 3-Cl-5-MeO-Ph | H | O | H | (CH₂)₃ | Methoxycarbonyl | |
| 8.673 | 3-Cl-Ph | H | O | H | (CH₂)₃ | Methoxycarbonyl | |
| 8.674 | 3-F-Ph | H | O | H | (CH₂)₃ | Methoxycarbonyl | |
| 8.675 | Ph | H | O | H | (CH₂)₃ | Methoxycarbonyl | |
| 8.676 | 3,5-F₂-Ph | H | O | H | (CH₂)₃ | Methylcarbamoyl | |
| 8.677 | 3,5-Cl₂-Ph | H | O | H | (CH₂)₃ | Morpholin-4-yl | |
| 8.678 | 3,5-Cl₂-Ph | H | O | H | (CH₂)₆ | Methoxycarbonyl | |

| Tabelle 9: Erfindungsgemäße Verbindungen der allgemeinen Formel (I), worin R² für Wasserstoff und R³ für Cyano stehen, und Aryl den Rest 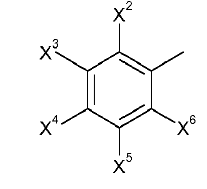 bedeutet. | | | | | | | |
|---|---|---|---|---|---|---|---|
| 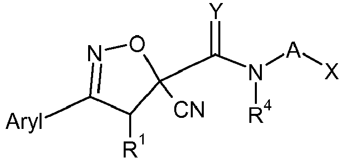 | | | | | | | |

| Nr. | Aryl | R¹ | Y | R⁴ | A | X | Physikalische Daten |
|---|---|---|---|---|---|---|---|
| 9.001 | 3,5-Cl₂-Ph | H | O | H | (CH₂)₄ | COOH | |
| 9.002 | 3,5-Cl₂-Ph | H | O | H | (CH₂)₄ | Ethoxycarbonyl | |
| 9.003 | 3-Br-5-Cl-Ph | H | O | H | (CH₂)₄ | Ethoxycarbonyl | |
| 9.004 | Ph | H | O | H | (CH₂)₄ | Ethoxycarbonyl | |
| 9.005 | 3,5-Cl₂-Ph | H | O | H | (CH₂)₄ | Hydroxy | |
| 9.006 | 3,5-Cl₂-Ph | H | O | H | (CH₂)₄ | Methoxy | |
| 9.007 | 3,5-Cl₂-Ph | H | O | H | (CH₂)₆ | COOH | |
| 9.008 | 3,5-Cl₂-Ph | H | O | H | (CH₂)₆ | Ethoxycarbonyl | |
| 9.009 | 3,5-Cl₂-Ph | H | O | H | c-Pr-1,1-diyl | Ethoxycarbonyl | |
| 9.010 | 3,5-F₂-Ph | H | O | H | Bindung | 1-Cyancyclopropyl | |
| 9.011 | 3,5-Cl₂-Ph | H | O | H | Bindung | 1-Cyanocyclopropyl | |
| 9.012 | 3,5-Cl₂-Ph | H | O | H | Bindung | 1-Methylcyclopropyl | |
| 9.013 | 3,5-Cl₂-Ph | H | O | H | Bindung | 2-(Ethoxycarbonyl)cyclohe x-1-en-1-yl | |
| 9.014 | 3,5-Cl₂-Ph | H | O | H | Bindung | 2-(Ethoxycarbonyl)cyclohe xyl | |
| 9.015 | 3,5-Cl₂-Ph | H | O | H | Bindung | 2-(Methylcarbamoyl)cyclo hexyl | |
| 9.016 | 3,5-Cl₂-Ph | H | O | H | Bindung | 2-Carboxycyclohexyl | |
| 9.017 | 3,5-Cl₂-Ph | H | O | H | Bindung | 3-(2-Ethoxy-2-oxoethyl)oxetan-3-yl | |
| 9.018 | 3,5-F₂-Ph | H | O | H | Bindung | 3-(2-Ethoxy-2-oxoethyl)oxetan-3-yl | |
| 9.019 | 2,4-Cl₂-Ph | H | O | CH₃ | Bindung | CH3 | |
| 9.020 | 2-Cl-Ph | H | O | CH₃ | Bindung | CH3 | |
| 9.021 | 3,4-F₂-Ph | H | O | H | Bindung | CH3 | |
| 9.022 | 3,5-Br₂-Ph | H | O | H | Bindung | CH3 | |
| 9.023 | 3,5-Cl₂-Ph | CH₃ | O | H | Bindung | CH3 | |
| 9.024 | 3,5-Cl₂-Ph | H | O | CH₃ | Bindung | CH3 | |
| 9.025 | 3,5-Cl₂-Ph | H | O | H | Bindung | CH3 | |
| 9.026 | 3,5-F₂-Ph | H | O | H | Bindung | CH3 | |
| 9.027 | 3-Cl-4-F-Ph | H | O | H | Bindung | CH3 | |
| 9.028 | 3,5-Cl₂-Ph | H | O | H | Bindung | Cyclobutyl | |
| 9.029 | 3,5-F₂-Ph | H | O | H | Bindung | Cyclobutyl | |
| 9.030 | 3,5-Cl₂-Ph | H | O | H | Bindung | Cyclopentyl | |
| 9.031 | 3,5-F₂-Ph | H | O | H | Bindung | Cyclopentyl | |
| 9.032 | 2-CF₃-Ph | H | O | H | Bindung | c-Pr | |
| 9.033 | 2,3,4-F₃-Ph | H | O | H | Bindung | c-Pr | |
| 9.034 | 2,3,5-F₃-Ph | H | O | H | Bindung | c-Pr | |
| 9.035 | 2,3-F₂-Ph | H | O | H | Bindung | c-Pr | |
| 9.036 | 2,4-Cl₂-Ph | H | O | H | Bindung | c-Pr | |
| 9.037 | 2,5-F₂-Ph | H | O | H | Bindung | c-Pr | |
| 9.038 | 2,5-Me₂-Ph | H | O | H | Bindung | c-Pr | |
| 9.039 | 2-Cl-3-F-5-MeO-Ph | H | O | H | Bindung | c-Pr | |
| 9.040 | 2-Cl-5-F-Ph | H | O | H | Bindung | c-Pr | |
| 9.041 | 2-EtO-3,4,5,6-F₄-Ph | H | O | H | Bindung | c-Pr | |
| 9.042 | 2-F-3-Me-Ph | H | O | H | Bindung | c-Pr | |
| 9.043 | 3-(2-MeOEtO)-Ph | H | O | H | Bindung | c-Pr | |
| 9.044 | 3-iPrO-Ph | H | O | H | Bindung | c-Pr | |
| 9.045 | 3-CF₃O-Ph | H | O | H | Bindung | c-Pr | |
| 9.046 | 3-CF₃-Ph | H | O | H | Bindung | c-Pr | |
| 9.047 | 3,4,5-F₃-Ph | H | O | H | Bindung | c-Pr | |
| 9.048 | 3,5-Br₂-Ph | H | O | H | Bindung | c-Pr | |
| 9.049 | 3,5-Cl₂-4-MeO-Ph | H | O | H | Bindung | c-Pr | |
| 9.050 | 3,5-Cl₂-4-OH-Ph | H | O | H | Bindung | c-Pr | |
| 9.051 | 3,5-Cl₂-Ph | CH₃ | O | H | Bindung | c-Pr | |
| 9.052 | 3,5-Cl₂-Ph | H | O | H | Bindung | c-Pr | [CDCl₃] 0.63 (m, 2H); 0.87 (m, 2H); 2.79 (m, 1H); 3.97 (d, 1H); 4.11 (d, 1H); 6.77 (brs, 1H); 7.47 (m, 1H); 7.52 (m, 2H). |
| 9.053 | 3,5-F₂-Ph | H | O | H | Bindung | c-Pr | |
| 9.054 | 3,5-F₂-Ph | H | O | H | Bindung | c-Pr | |
| 9.055 | 3,5-F₂-Ph | H | S | H | Bindung | c-Pr | |
| 9.056 | 3,5-(MeO)₂-Ph | H | O | H | Bindung | c-Pr | |
| 9.057 | 3,5-Me₂-Ph | H | O | H | Bindung | c-Pr | |
| 9.058 | 3-Ac-Ph | H | O | H | Bindung | c-Pr | |
| 9.059 | 3-Br-5-CF₃-Ph | H | O | H | Bindung | c-Pr | |
| 9.060 | 3-Br-5-Cl-Ph | H | O | H | Bindung | c-Pr | |
| 9.061 | 3-Br-5-F-Ph | H | O | H | Bindung | c-Pr | |
| 9.062 | 3-CI-4-F-Ph | H | O | H | Bindung | c-Pr | |
| 9.063 | 3-Cl-4-Me-Ph | H | O | H | Bindung | c-Pr | |
| 9.064 | 3-Cl-5-CF₃-Ph | H | O | H | Bindung | c-Pr | |
| 9.065 | 3-Cl-5-CN-Ph | H | O | H | Bindung | c-Pr | |
| 9.066 | 3-Cl-5-Et-Ph | H | O | H | Bindung | c-Pr | |
| 9.067 | 3-Cl-5-F-Ph | H | O | H | Bindung | c-Pr | |
| 9.068 | 3-Cl-5-Me-Ph | H | O | H | Bindung | c-Pr | |
| 9.069 | 3-C-5-CF₃O-Ph | H | O | H | Bindung | c-Pr | |
| 9.070 | 3-Cl-Ph | H | O | H | Bindung | c-Pr | |
| 9.071 | 3-CN-5-F-Ph | H | O | H | Bindung | c-Pr | |
| 9.072 | 3-c-Pr-5-F-Ph | H | O | H | Bindung | c-Pr | |
| 9.073 | 3-EtO-Ph | H | O | H | Bindung | c-Pr | |
| 9.074 | 3-Et-5-F-Ph | H | O | H | Bindung | c-Pr | |
| 9.075 | 3-Et-Ph | H | O | H | Bindung | c-Pr | |
| 9.076 | 3-F-5-MeS-Ph | H | O | H | Bindung | c-Pr | |
| 9.077 | 3-F-5-MeSO₂-Ph | H | O | H | Bindung | c-Pr | |
| 9.078 | 3-F-5-MeO-Ph | H | O | H | Bindung | c-Pr | |
| 9.079 | 3-F-5-Me-Ph | H | O | H | Bindung | c-Pr | |
| 9.080 | 3-F-5-Me-Ph | H | S | H | Bindung | c-Pr | |
| 9.081 | 3-F-Ph | H | O | H | Bindung | c-Pr | |
| 9.082 | 3-OH-Ph | H | O | H | Bindung | c-Pr | |
| 9.083 | 3-iPr-Ph | H | O | H | Bindung | c-Pr | |
| 9.084 | 3-MeO-Ph | H | O | H | Bindung | c-Pr | |
| 9.085 | 3-Me-5-CF₃O-Ph | H | O | H | Bindung | c-Pr | |
| 9.086 | 3-Me-Ph | H | O | H | Bindung | c-Pr | |
| 9.087 | 3-NO₂-Ph | H | O | H | Bindung | c-Pr | |
| 9.088 | 4-Cl-3,5-F₂-Ph | H | O | H | Bindung | c-Pr | |
| 9.089 | 4-EtO-Ph | H | O | H | Bindung | c-Pr | |
| 9.090 | F₅-Ph | H | O | H | Bindung | c-Pr | |
| 9.091 | Ph | H | O | H | Bindung | c-Pr | |
| 9.092 | 3,5-Cl₂-Ph | H | O | H | Bindung | c-Pr | |
| 9.093 | 3,5-Cl₂-Ph | H | O | H | Bindung | Decahydronaphthalen-2-yl | |
| 9.094 | 3,5-F₂-Ph | H | O | H | Bindung | H | |
| 9.095 | 3,5-F₂-Ph | H | S | H | Bindung | H | |
| 9.096 | 3-F-Ph | H | O | H | Bindung | H | |
| 9.097 | 3-Me-Ph | H | O | H | Bindung | H | |
| 9.098 | 3-Me-Ph | H | S | H | Bindung | H | |
| 9.099 | 3,5-Cl₂-Ph | H | O | CH₃ | Bindung | H | |
| 9.100 | 2,3,4-F₃-Ph | H | O | H | Bindung | Hydroxy | |
| 9.101 | 2,3-F₂-Ph | H | O | H | Bindung | Oxetan-3-yl | |
| 9.102 | 2,5-F₂-Ph | H | O | H | Bindung | Oxetan-3-yl | |
| 9.103 | 3,5-Cl₂-Ph | H | O | H | Bindung | Oxetan-3-yl | |
| 9.104 | 3,5-F₂-Ph | H | O | H | Bindung | Oxetan-3-yl | |
| 9.105 | 3-Cl-5-CF₃O-Ph | H | O | H | Bindung | Oxetan-3-Yl | |
| 9.106 | 3-F-5-Me-Ph | H | O | H | Bindung | Oxetan-3-Yl | |
| 9.107 | 3-F-Ph | H | O | H | Bindung | Oxetan-3-yl | |
| 9.108 | 3-NO₂-Ph | H | O | H | Bindung | Oxetan-3-yl | |
| 9.109 | Ph | H | O | H | Bindung | Oxetan-3-yl | |
| 9.110 | 3,5-Cl₂-Ph | H | O | H | Bindung | Oxetan-3-yl | |
| 9.111 | 3,5-Cl₂-Ph | H | O | H | C(CH₃)₂ | Tetrahydro-2H-pyran-4-yl | |
| 9.112 | 3,5-Cl₂-Ph | H | O | H | C(CH₃)₂ | 3-Methoxyprop-1-in-1-yl | |
| 9.113 | 3,5-Cl₂-Ph | H | O | H | C(CH₃)₂ | Carbamoyl | |
| 9.114 | 3-Cl-4-F-Ph | H | O | H | C(CH₃)₂ | CH3 | |
| 9.115 | 3,5-Cl₂-Ph | H | O | H | C(CH₃)₂ | CH3 | |
| 9.116 | 3,5-(CF₃)₂-Ph | H | O | H | C(CH₃)₂ | COOH | |
| 9.117 | 3,5-Cl₂-Ph | H | O | H | C(CH₃)₂ | Ethinyl | |
| 9.118 | 3,5-Cl₂-Ph | H | O | H | C(CH₃)₂ | Ethinyl | |
| 9.119 | 3,5-Cl₂-Ph | H | O | H | C(CH₃)₂ | Methoxycarbonyl | |
| 9.120 | 3-Cl-Ph | H | O | H | C(CH₃)₂ CH₂ | Methylcarbamoyl | |
| 9.121 | 3-F-Ph | H | O | H | C(CH₃)₂ CH₂ | (Propan-2-yloxy)carbonyl | |
| 9.122 | 3,5-Cl₂-Ph | H | O | H | C(CH₃)₂ CH₂ | (Propan-2-yloxy)carbonyl | |
| 9.123 | 3-CI-Ph | H | O | H | C(CH₃)₂ CH₂ | COOH | |
| 9.124 | 3-F-Ph | H | O | H | C(CH₃)₂ CH₂ | COOH | |
| 9.125 | 3,5-Cl₂-Ph | H | O | H | C(CH₃)₂ CH₂ | COOH | |
| 9.126 | 3-Cl-Ph | H | O | H | C(CH₃)₂ CH₂ | Ethoxycarbonyl | |
| 9.127 | 3-F-Ph | H | O | H | C(CH₃)₂ CH₂ | Ethoxycarbonyl | |
| 9.128 | 3,5-Cl₂-Ph | H | O | H | C(CH₃)₂ CH₂ | Ethoxycarbonyl | |
| 9.129 | 3-Cl-Ph | H | O | H | C(CH₃)₂ CH₂ | Hydroxy | |
| 9.130 | 3-F-Ph | H | O | H | C(CH₃)₂ CH₂ | Methoxycarbonyl | |
| 9.131 | 3,5-Cl₂-Ph | H | O | H | C(CH₃)₂ CH₂ | Methoxycarbonyl | |
| 9.132 | 3,5-Cl₂-Ph | H | O | H | C(CH₃)₂ CH₂ | Methylsulfanyl | |
| 9.133 | 2,4-Cl₂-Ph | H | O | H | C(iPr)C H₃ | Methylsulfonyl | |
| 9.134 | 3,5-Cl₂-Ph | H | O | H | C(iPr)C H₃ | Cyan | |
| 9.135 | 3,5-Cl₂-Ph | H | O | H | CH(CF₃) CH₂ | Cyan | |
| 9.136 | 3,5-Cl₂-Ph | H | O | H | CH(CH₂ CH₂SC H₃) | Ethoxycarbonyl | |
| 9.137 | 3,5-Cl₂-Ph | H | O | H | CH(CH₂ CH₃)CH₂ | Methoxycarbonyl | |
| 9.138 | 3,5-Cl₂-Ph | H | O | H | CH(CH₂ CH₃)CH 2 | Cyan | |
| 9.139 | 3,5-Cl₂-Ph | H | O | H | CH(CH₂ iPr)CH₂ | Methoxycarbonyl | |
| 9.140 | 3,5-Cl₂-Ph | H | O | H | CH(CH₂ OCH₃) | Methoxycarbonyl | |
| 9.141 | 3,5-Cl₂-Ph | H | O | H | CH(CH₃) | Methoxymethyl | |
| 9.142 | 2,3,4-F₃-Ph | H | O | H | CH(CH₃) | CF₃ | |
| 9.143 | 2,3,5-F₃-Ph | H | O | H | CH(CH₃) | CH₃ | |
| 9.144 | 2,3,6-Cl₂-Ph | H | O | H | CH(CH₃) | CH₃ | |
| 9.145 | 2,3-Cl₂-5-MeO-Ph | H | O | H | CH(CH₃) | CH₃ | |
| 9.146 | 2,3-Cl₂-Ph | H | O | H | CH(CH₃) | CH₃ | |
| 9.147 | 2,3-F₂-Ph | H | O | H | CH(CH₃) | CH₃ | |
| 9.148 | 2,5-Cl₂-Ph | H | O | H | CH(CH₃) | CH₃ | |
| 9.149 | 2,5-F₂-Ph | H | O | H | CH(CH₃) | CH₃ | |
| 9.150 | 2-F-Ph | H | O | H | CH(CH₃) | CH₃ | |
| 9.151 | 3-(CF₃CH₂O)-Ph | H | O | H | CH(CH₃) | CH₃ | |
| 9.152 | 3-(ClCH₂CH₂O)-Ph | H | O | H | CH(CH₃) | CH₃ | |
| 9.153 | 3-(2-MeOEtO)-Ph | H | O | H | CH(CH₃) | CH₃ | |
| 9.154 | 3-Me₂N-Ph | H | O | H | CH(CH₃) | CH₃ | |
| 9.155 | 3-iPrCOO-Ph | H | O | H | CH(CH₃) | CH₃ | |
| 9.156 | 3-iPrO-Ph | H | O | H | CH(CH₃) | CH₃ | |
| 9.157 | 3-CF₃O-Ph | H | O | H | CH(CH₃) | CH₃ | |
| 9.158 | 3,4-Cl₂-Ph | H | O | H | CH(CH₃) | CH₃ | |
| 9.159 | 3,5-(CF₃)₂-Ph | H | O | H | CH(CH₃) | CH₃ | |
| 9.160 | 3,5-Cl₂-Ph | CH₃ | O | H | CH(CH₃) | CH₃ | |
| 9.161 | 3,5-Cl₂-Ph | H | O | H | CH(CH₃) | CH₃ | |
| 9.162 | 3,5-Cl₂-Ph | H | O | OH | CH(CH₃) | CH₃ | |
| 9.163 | 3,5-Cl₂-Ph | H | S | H | CH(CH₃) | CH₃ | |
| 9.164 | 3,5-F₂-Ph | H | O | H | CH(CH₃) | CH₃ | |
| 9.165 | 3,5-Me₂-Ph | H | O | H | CH(CH₃) | CH₃ | |
| 9.166 | R002 | H | O | H | CH(CH₃) | CH₃ | |
| 9.167 | R003 | H | O | H | CH(CH₃) | CH₃ | |
| 9.168 | 3-CNCH₂N(Me)-Ph | H | O | H | CH(CH₃) | CH₃ | |
| 9.169 | 3-Me₂NCONH-Ph | H | O | H | CH(CH₃) | CH₃ | |
| 9.170 | 3-Me₂NSO₂O-Ph | H | O | H | CH(CH₃) | CH₃ | |
| 9.171 | 3-EtNHCOO-Ph | H | O | H | CH(CH₃) | CH₃ | |
| 9.172 | 3-EtSO₂O-Ph | H | O | H | CH(CH₃) | CH₃ | |
| 9.173 | 3-MeSO₂NH-Ph | H | O | H | CH(CH₃) | CH₃ | |
| 9.174 | 3-MeSO₂O-Ph | H | O | H | CH(CH₃) | CH₃ | |
| 9.175 | 3-tert.BuOCON H-Ph | H | O | H | CH(CH₃) | CH₃ | |
| 9.176 | 3-CF₃CONH-Ph | H | O | H | CH(CH₃) | CH₃ | |
| 9.177 | 3-AcO-5-Cl-Ph | H | O | H | CH(CH₃) | CH₃ | |
| 9.178 | 3-AcO-Ph | H | O | H | CH(CH₃) | CH₃ | |
| 9.179 | 3-NH₂-Ph | H | O | H | CH(CH₃) | CH₃ | |
| 9.180 | 3-Br-5-CF₃-Ph | H | O | H | CH(CH₃) | CH₃ | |
| 9.181 | 3-Br-5-Cl-Ph | H | O | H | CH(CH₃) | CH₃ | |
| 9.182 | 3-Cl-4-F-Ph | H | O | H | CH(CH₃) | CH₃ | |
| 9.183 | 3-Cl-4-Me-Ph | H | O | H | CH(CH₃) | CH₃ | |
| 9.184 | 3-Cl-5-CF₃-Ph | H | O | H | CH(CH₃) | CH₃ | |
| 9.185 | R001 | H | O | H | CH(CH₃) | CH₃ | |
| 9.186 | 3-Cl-5-F-Ph | H | O | H | CH(CH₃) | CH₃ | |
| 9.187 | 3-Cl-5-MeO-Ph | H | O | H | CH(CH₃) | CH₃ | |
| 9.188 | 3-Cl-5-Me-Ph | H | O | H | CH(CH₃) | CH₃ | |
| 9.189 | 3-Cl-5-(CF₃CH₂O)-Ph | H | O | H | CH(CH₃) | CH₃ | |
| 9.190 | 3-Cl-5-(ClCH₂CH₂O)-Ph | H | O | H | CH(CH₃) | CH₃ | |
| 9.191 | 3-Cl-5-(EtOCOCH₂O )-Ph | H | O | H | CH(CH₃) | CH₃ | |
| 9.192 | 3-Cl-5-CF₃O-Ph | H | O | H | CH(CH₃) | CH₃ | |
| 9.193 | 3-Cl-5-Me₂NSO₂O-Ph | H | O | H | CH(CH₃) | CH₃ | |
| 9.194 | 3-CI-5-(MeSO₂O)-Ph | H | O | H | CH(CH₃) | CH₃ | |
| 9.195 | 3-Cl-5-iPrO-Ph | H | O | H | CH(CH₃) | CH₃ | |
| 9.196 | 3-Cl-Ph | H | O | H | CH(CH₃) | CH₃ | |
| 9.197 | 3-EtO-Ph | H | O | H | CH(CH₃) | CH₃ | |
| 9.198 | 3-F-5-Me-Ph | H | O | H | CH(CH₃) | CH₃ | |
| 9.199 | 3-F-Ph | H | O | H | CH(CH₃) | CH₃ | |
| 9.200 | 3-OH-Ph | H | O | H | CH(CH₃) | CH₃ | |
| 9.201 | 3-MeO-Ph | H | O | H | CH(CH₃) | CH₃ | |
| 9.202 | 3-Me-Ph | H | O | H | CH(CH₃) | CH₃ | |
| 9.203 | 3-NO₂-Ph | H | O | H | CH(CH₃) | CH₃ | |
| 9.204 | F₅-Ph | H | O | H | CH(CH₃) | CH₃ | |
| 9.205 | Ph | H | O | H | CH(CH₃) | CH₃ | |
| 9.206 | 3,5-Cl₂-Ph | H | O | H | CH(CH₃) | CH₃ | |
| 9.207 | 3-Cl-4-F-Ph | H | O | H | CH(CH₃) | COOH | |
| 9.208 | 3,5-Cl₂-Ph | H | O | H | CH(CH₃) | COOH | |
| 9.209 | 3,5-F₂-Ph | H | O | H | CH(CH₃) | c-Pr | |
| 9.210 | Ph | H | O | H | CH(CH₃) | c-Pr | |
| 9.211 | 3-Cl-4-F-Ph | H | O | H | CH(CH₃) | c-Pr | |
| 9.212 | 3,5-Cl₂-Ph | H | O | H | CH(CH₃) | Dimethylcarbamoyl | |
| 9.213 | 3,5-Cl₂-Ph | H | O | H | CH(CH₃) | Ethinyl | |
| 9.214 | 3,5-Cl₂-Ph | CH₃ | O | H | CH(CH₃) | Ethyl | |
| 9.215 | 3,5-Cl₂-Ph | H | O | H | CH(CH₃) | Methoxycarbonyl | |
| 9.216 | 3-Cl-4-F-Ph | H | O | H | CH(CH₃) | Methoxycarbonyl | |
| 9.217 | 3-Cl-4-F-Ph | H | O | H | CH(CH₃) | Methoxycarbonyl | |
| 9.218 | 3,5-Cl₂-Ph | H | O | H | CH(CH₃) | Methylcarbamoyl | |
| 9.219 | 3,5-Cl₂-Ph | H | O | H | CH(CH₃) CH₂ | Pentyl | |
| 9.220 | 3,5-F₂-Ph | H | O | H | CH(CH₃) CH₂ | (2,2,2-Trifluoroethoxy)carbonyl | |
| 9.221 | 3,5-Cl₂-Ph | H | O | H | CH(CH₃) CH₂ | (2,2,2-Trifluoroethoxy)carbonyl | |
| 9.222 | 3,5-F₂-Ph | H | O | H | CH(CH₃) CH₂ | (2-Hydroxyethoxy)carbonyl | |
| 9.223 | 3,5-Cl₂-Ph | H | O | H | CH(CH₃) CH₂ | (2-Hydroxyethoxy)carbonyl | |
| 9.224 | 3,5-F₂-Ph | H | O | H | CH(CH₃) CH₂ | (Allyloxy)carbonyl | |
| 9.225 | 3,5-Cl₂-Ph | H | O | H | CH(CH₃) CH₂ | (Allyloxy)carbonyl | |
| 9.226 | 3,5-F₂-Ph | H | O | H | CH(CH₃) CH₂ | (Cyclopropylsulfonyl)(methyl)carbamoyl | |
| 9.227 | 3,5-Cl₂-Ph | H | O | H | CH(CH₃) CH₂ | (Cyclopropylsulfonyl)(methyl)carbamoyl | |
| 9.228 | 3,5-F₂-Ph | H | O | H | CH(CH₃) CH₂ | (Cyclopropylsulfonyl)carbamoyl | |
| 9.229 | 3,5-Cl₂-Ph | H | O | H | CH(CH₃) CH₂ | (Cyclopropylsulfonyl)carbamoyl | |
| 9.230 | 3,5-F₂-Ph | H | O | H | CH(CH₃) CH₂ | (Ethylsulfonyl)(methyl)carbamoyl | |
| 9.231 | 3,5-Cl₂-Ph | H | O | H | CH(CH₃) CH₂ | (Ethylsulfonyl)(methyl)carbamoyl | |
| 9.232 | 3,5-F₂-Ph | H | O | H | CH(CH₃) CH₂ | (Ethylsulfonyl)carbamoyl | |
| 9.233 | 3,5-Cl₂-Ph | H | O | H | CH(CH₃) CH₂ | (Ethylsulfonyl)carbamoyl | |
| 9.234 | 3,5-F₂-Ph | H | O | H | CH(CH₃) CH₂ | (Heptan-2-yloxy)carbonyl | |
| 9.235 | 3,5-Cl₂-Ph | H | O | H | CH(CH₃) CH₂ | (Heptan-2-yloxy)carbonyl | |
| 9.236 | 3,5-F₂-Ph | H | O | H | CH(CH₃) CH₂ | (Isopropylsulfonyl)(methyl)carbamoyl | |
| 9.237 | 3,5-Cl₂-Ph | H | O | H | CH(CH₃) CH₂ | (Isopropylsulfonyl)(methyl)carbamoyl | |
| 9.238 | 3,5-F₂-Ph | H | O | H | CH(CH₃) CH₂ | (Isopropylsulfonyl)carbamoyl | |
| 9.239 | 3,5-Cl₂-Ph | H | O | H | CH(CH₃) CH₂ | (Isopropylsulfonyl)carbamoyl | |
| 9.240 | 3,5-F₂-Ph | H | O | H | CH(CH₃) CH₂ | (Methylsulfonyl)carbamoyl | |
| 9.241 | 3,5-Cl₂-Ph | H | O | H | CH(CH₃) CH₂ | (Methylsulfonyl)carbamoyl | |
| 9.242 | 3,5-F₂-Ph | H | O | H | CH(CH₃) CH₂ | (Prop-2-Yn-1-yloxy)carbonyl | |
| 9.243 | 3,5-Cl₂-Ph | H | O | H | CH(CH₃) CH₂ | (Prop-2-Yn-1-yloxy)carbonyl | |
| 9.244 | 3,5-F₂-Ph | H | O | H | CH(CH₃) CH₂ | (Propan-2-yloxy)carbonyl | |
| 9.245 | 3-Cl-Ph | H | O | H | CH(CH₃) CH₂ | (Propan-2-yloxy)carbonyl | |
| 9.246 | 3-F-Ph | H | O | H | CH(CH₃) CH₂ | (Propan-2-yloxy)carbonyl | |
| 9.247 | 3,5-Cl₂-Ph | H | O | H | CH(CH₃) CH₂ | (Propan-2-yloxy)carbonyl | |
| 9.248 | 3,5-F₂-Ph | H | O | H | CH(CH₃) CH₂ | [2-(Methylsulfanyl)ethoxy]carbonyl | |
| 9.249 | 3,5-F₂-Ph | H | O | H | CH(CH₃) CH₂ | [2-(Methylsulfanyl)ethoxy]carbonyl | |
| 9.250 | 3,5-Cl₂-Ph | H | O | H | CH(CH₃) CH₂ | [2-(Methylsulfonyl)ethoxy]carbonyl | |
| 9.251 | 3,5-F₂-Ph | H | O | H | CH(CH₃) CH₂ | Butoxycarbonyl | |
| 9.252 | 3-CF₃-Ph | H | O | H | CH(CH₃) CH₂ | Butoxycarbonyl | |
| 9.253 | 3,5-Cl₂-Ph | H | O | H | CH(CH₃) CH₂ | COOH | |
| 9.254 | 3,5-F₂-Ph | H | O | H | CH(CH₃) CH₂ | COOH | |
| 9.255 | 3,5-(tert.Bu)₂-Ph | H | O | H | CH(CH₃) CH₂ | COOH | |
| 9.256 | 3-Br-5-CF₃-Ph | H | O | H | CH(CH₃) CH₂ | COOH | |
| 9.257 | 3-Cl-5-CF₃-Ph | H | O | H | CH(CH₃) CH₂ | COOH | |
| 9.258 | 3-Cl-Ph | H | O | H | CH(CH₃) CH₂ | COOH | |
| 9.259 | 3-Et-5-F-Ph | H | O | H | CH(CH₃) CH₂ | COOH | |
| 9.260 | 3-Et-Ph | H | O | H | CH(CH₃) CH₂ | COOH | |
| 9.261 | 3-F-5-MeO-Ph | H | O | H | CH(CH₃) CH₂ | COOH | |
| 9.262 | 3-F-Ph | H | O | H | CH(CH₃) CH₂ | COOH | |
| 9.263 | 3-Me-Ph | H | O | H | CH(CH₃) CH₂ | COOH | |
| 9.264 | 4-MeO-Ph | H | O | H | CH(CH₃) CH₂ | COOH | |
| 9.265 | 3-CF₃-Ph | H | O | H | CH(CH₃) CH₂ | COOH | |
| 9.266 | 3,5-Cl₂-4-MeO-Ph | H | O | H | CH(CH₃) CH₂ | Ethoxycarbonyl | |
| 9.267 | 3,5-Cl₂-Ph | H | O | H | CH(CH₃) CH₂ | Ethoxycarbonyl | |
| 9.268 | 3,5-Et₂-Ph | H | O | H | CH(CH₃) CH₂ | Ethoxycarbonyl | |
| 9.269 | 3,5-F₂-Ph | H | O | H | CH(CH₃) CH₂ | Ethoxycarbonyl | |
| 9.270 | 3,5-(MeO)₂-Ph | H | O | H | CH(CH₃) CH₂ | Ethoxycarbonyl | |
| 9.271 | 3,5-Me₂-Ph | H | O | H | CH(CH₃) CH₂ | Ethoxycarbonyl | |
| 9.272 | 3,5-(tert.Bu)₂-Ph | H | O | H | CH(CH₃) CH₂ | Ethoxycarbonyl | |
| 9.273 | 3-Br-5-CF₃-Ph | H | O | H | CH(CH₃) CH₂ | Ethoxycarbonyl | |
| 9.274 | 3-Cl-5-Et-Ph | H | O | H | CH(CH₃) CH₂ | Ethoxycarbonyl | |
| 9.275 | 3-Cl-5-CF₃-Ph | H | O | H | CH(CH₃) CH₂ | Ethoxycarbonyl | |
| 9.276 | 3-Cl-Ph | H | O | H | CH(CH₃) CH₂ | Ethoxycarbonyl | |
| 9.277 | 3-c-Pr-5-F-Ph | H | O | H | CH(CH₃) CH₂ | Ethoxycarbonyl | |
| 9.278 | 3-Et-5-F-Ph | H | O | H | CH(CH₃) CH₂ | Ethoxycarbonyl | |
| 9.279 | 3-Et-Ph | H | O | H | CH(CH₃) CH₂ | Ethoxycarbonyl | |
| 9.280 | 3-F-5-MeS-Ph | H | O | H | CH(CH₃) CH₂ | Ethoxycarbonyl | |
| 9.281 | 3-F-5-MeSO₂-Ph | H | O | H | CH(CH₃) CH₂ | Ethoxycarbonyl | |
| 9.282 | 3-F-5-MeO-Ph | H | O | H | CH(CH₃) CH₂ | Ethoxycarbonyl | |
| 9.283 | 3-F-Ph | H | O | H | CH(CH₃) CH₂ | Ethoxycarbonyl | |
| 9.284 | 3-Me-5-CF₃O-Ph | H | O | H | CH(CH₃) CH₂ | Ethoxycarbonyl | |
| 9.285 | 3-Me-Ph | H | O | H | CH(CH₃) CH₂ | Ethoxycarbonyl | |
| 9.286 | 4-EtO-Ph | H | O | H | CH(CH₃) CH₂ | Ethoxycarbonyl | |
| 9.287 | 4-MeO-Ph | H | O | H | CH(CH₃) CH₂ | Ethoxycarbonyl | |
| 9.288 | 3,5-Cl₂-Ph | H | O | H | CH(CH₃) CH₂ | Ethoxycarbonyl | |
| 9.289 | 3,5-Cl₂-Ph | H | O | H | CH(CH₃) CH₂ | Hydroxy | |
| 9.290 | 3,5-F₂-Ph | H | O | H | CH(CH₃) CH₂ | Isobutoxycarbonyl | |
| 9.291 | 3,5-Cl₂-Ph | H | O | H | CH(CH₃) CH₂ | Isobutoxycarbonyl | |
| 9.292 | 2,5-Me₂-Ph | H | O | H | CH(CH₃) CH₂ | Methoxy | |
| 9.293 | 2-Cl-3-F-5-MeO-Ph | H | O | H | CH(CH₃) CH₂ | Methoxycarbonyl | |
| 9.294 | 2-F-3-Me-Ph | H | O | H | CH(CH₃) CH₂ | Methoxycarbonyl | |
| 9.295 | 3-CF₃-Ph | H | O | H | CH(CH₃) CH₂ | Methoxycarbonyl | |
| 9.296 | 3,5-Cl₂-Ph | H | O | H | CH(CH₃) CH₂ | Methoxycarbonyl | |
| 9.297 | 3,5-F₂-Ph | H | O | H | CH(CH₃) CH₂ | Methoxycarbonyl | |
| 9.298 | 3-Cl-Ph | H | O | H | CH(CH₃) CH₂ | Methoxycarbonyl | |
| 9.299 | 3-F-5-MeO-Ph | H | O | H | CH(CH₃) CH₂ | Methoxycarbonyl | |
| 9.300 | 3-F-Ph | H | O | H | CH(CH₃) CH₂ | Methoxycarbonyl | |
| 9.301 | 3,5-Cl₂-Ph | H | O | H | CH(CH₃) CH₂ | Methoxycarbonyl | |
| 9.302 | 3,5-F₂-Ph | H | O | H | CH(CH₃) CH₂ | Methyl(methylsulfonyl)carbamoyl | |
| 9.303 | 3,5-Cl₂-Ph | H | O | H | CH(CH₃) CH₂ | Methyl(methylsulfonyl)carbamoyl | |
| 9.304 | 3,5-Cl₂-Ph | H | O | H | CH(CH₃) CH₂ | Methylcarbamoyl | |
| 9.305 | 3,5-F₂-Ph | H | O | H | CH(CH₃) CH₂ | Propoxycarbonyl | |
| 9.306 | 3,5-Cl₂-Ph | H | O | H | CH(CH₃) CH₂ | Propoxycarbonyl | |
| 9.307 | 3,5-F₂-Ph | H | O | H | CH(CH₃) CH₂ | Sec-Butoxycarbonyl | |
| 9.308 | 3,5-Cl₂-Ph | H | O | H | CH(COO CH₃)CH₂ | Sec-Butoxycarbonyl | |
| 9.309 | 3,5-Cl₂-Ph | H | O | H | CH(cycl oPr) | Methoxycarbonyl | |
| 9.310 | 3,5-F₂-Ph | H | O | H | CH(cycl oPr) | c-Pr | |
| 9.311 | 3,5-Cl₂-Ph | H | O | H | CH(iPr) | c-Pr | |
| 9.312 | 3,5-Cl₂-Ph | H | O | H | CH(iPr) CH₂ | Methoxycarbonyl | |
| 9.313 | 3,5-F₂-Ph | H | O | H | CH₂ | Methoxycarbonyl | |
| 9.314 | 3,5-Cl₂-Ph | H | O | H | CH₂ | (2-Hydroxyethyl)carbamoyl | |
| 9.315 | 3,5-F₂-Ph | H | O | H | CH₂ | (Cyclopropylsulfonyl)carbamoyl | |
| 9.316 | 3,5-Cl₂-Ph | H | O | H | CH₂ | (Cyclopropylsulfonyl)carbamoyl | |
| 9.317 | 3,5-F₂-Ph | H | O | H | CH₂ | (Ethylsulfonyl)carbamoyl | |
| 9.318 | 3,5-F₂-Ph | H | O | H | CH₂ | (Ethylsulfonyl)carbamoyl | |
| 9.319 | 3,5-Cl₂-Ph | H | O | H | CH₂ | (Hydroxyimino)methyl | |
| 9.320 | 3,5-F₂-Ph | H | O | H | CH₂ | (Isopropylsulfonyl)carbamoyl | |
| 9.321 | 3,5-Cl₂-Ph | H | O | H | CH₂ | (Isopropylsulfonyl)carbamoyl | |
| 9.322 | 3,5-F₂-Ph | H | O | H | CH₂ | (Methylsulfonyl)carbamoyl | |
| 9.323 | 3-Cl-Ph | H | O | H | CH₂ | (Methylsulfonyl)carbamoyl | |
| 9.324 | 3-F-Ph | H | O | H | CH₂ | (Propan-2-yloxy)carbonyl | |
| 9.325 | 3,5-Cl₂-Ph | H | O | H | CH₂ | (Propan-2-yloxy)carbonyl | |
| 9.326 | 3,5-F₂-Ph | H | O | H | CH₂ | 1,1-Dioxidotetrahydrothiophen-3-yl | |
| 9.327 | 3-F-Ph | H | O | H | CH₂ | 1,1-Dioxidotetrahydrothiophen-3-yl | |
| 9.328 | Ph | H | O | H | CH₂ | 1,1-Dioxidotetrahydrothiophen-3-yl | |
| 9.329 | Ph | H | O | H | CH₂ | 1,1-Dioxidotetrahydrothiophen-3-yl | |
| 9.330 | 3,5-Cl₂-Ph | H | O | H | CH₂ | 1,4-Dimethyl-5-oxo-4,5-dihydro-1H-1,2,4-triazol-3-yl | |
| 9.331 | 3,5-F₂-Ph | H | O | H | CH₂ | 3-(2-Ethoxy-2-oxoethyl)oxetan-3-yl | |
| 9.332 | 3,5-Cl₂-Ph | H | O | H | CH₂ | 3-(2-Ethoxy-2-oxoethyl)oxetan-3-yl | |
| 9.333 | 3,5-Cl₂-Ph | H | O | H | CH₂ | 3-(Cyclopropylcarbamoyl)-4,5-dihydro-1,2-oxazol-5-yl | |
| 9.334 | 3,5-Cl₂-Ph | H | O | H | CH₂ | 3-(Ethoxycarbonyl)-4,5-dihydro-1,2-oxazol-5-yl | |
| 9.335 | 3,5-Cl₂-Ph | H | O | H | CH₂ | 3-(Methylcarbamoyl)-4,5-dihydro-1,2-oxazol-5-yl | |
| 9.336 | 3,5-Cl₂-Ph | H | O | H | CH₂ | 3-Carboxy-4,5-dihydro-1,2-oxazol-5-yl | |
| 9.337 | 3,5-Cl₂-Ph | H | O | H | CH₂ | 3-Isopropyl-4,5-dihydro-1,2-oxazol-5-yl | |
| 9.338 | 3,5-F₂-Ph | H | O | H | CH₂ | 3-Methyl-4,5-dihydro-1,2-oxazol-5-yl | |
| 9.339 | 3,5-Cl₂-Ph | H | O | H | CH₂ | 5-(Ethoxycarbonyl)-4,5-dihydro-1,2-oxazol-3-yl | |
| 9.340 | 3,5-Cl₂-Ph | H | O | H | CH₂ | 5,5-Dimethyl-4,5-dihydro-1,2-oxazol-3-yl | |
| 9.341 | 3,5-Cl₂-Ph | H | O | H | CH₂ | 5-Ethyl-4,5-dihydro-1,2-oxazol-3-yl | |
| 9.342 | 3,5-Cl₂-Ph | H | O | H | CH₂ | 5-Isopropyl-4,5-dihydro-1,2-oxazol-3-yl | |
| 9.343 | 3,5-Cl₂-Ph | H | O | H | CH₂ | 5-Methyl-4,5-dihydro-1,2-oxazol-3-yl | |
| 9.344 | 3-Cl-Ph | H | O | H | CH₂ | Carbamoyl | |
| 9.345 | 2-CF₃-Ph | H | O | H | CH₂ | Carbamoyl | |
| 9.346 | 2,3,4-F₃-Ph | H | O | H | CH₂ | CF₃ | |
| 9.347 | 2,3,5-F₃-Ph | H | O | H | CH₂ | CF₃ | |
| 9.348 | 2,3,6-Cl₂-Ph | H | O | H | CH₂ | CF₃ | |
| 9.349 | 2,3-F₂-Ph | H | O | H | CH₂ | CF₃ | |
| 9.350 | 2,5-F₂-Ph | H | O | H | CH₂ | CF₃ | |
| 9.351 | 2-Cl-3-F-5-MeO-Ph | H | O | H | CH₂ | CF₃ | |
| 9.352 | 3-(2-MeOEtO)-Ph | H | O | H | CH₂ | CF₃ | |
| 9.353 | 3-iPrO-Ph | H | O | H | CH₂ | CF₃ | |
| 9.354 | 3-CF₃O-Ph | H | O | H | CH₂ | CF₃ | |
| 9.355 | 3-CF₃-Ph | H | O | H | CH₂ | CF₃ | |
| 9.356 | 3,4-Cl₂-Ph | H | O | H | CH₂ | CF₃ | |
| 9.357 | 3,5-Br₂-Ph | H | O | H | CH₂ | CF₃ | |
| 9.358 | 3,5-Cl₂-4-MeO-Ph | H | O | H | CH₂ | CF₃ | |
| 9.359 | 3,5-Cl₂-Ph | H | O | H | CH₂ | CF₃ | |
| 9.360 | 3,5-F₂-Ph | H | O | H | CH₂ | CF₃ | |
| 9.361 | 3,5-(MeO)₂-Ph | H | O | H | CH₂ | CF₃ | |
| 9.362 | 3,5-Me₂-Ph | H | O | H | CH₂ | CF₃ | |
| 9.363 | 3-Br-5-Cl-Ph | H | O | H | CH₂ | CF₃ | |
| 9.364 | 3-Br-5-F-Ph | H | O | H | CH₂ | CF₃ | |
| 9.365 | 3-Cl-5-CF₃-Ph | H | O | H | CH₂ | CF₃ | |
| 9.366 | 3-Cl-5-Et-Ph | H | O | H | CH₂ | CF₃ | |
| 9.367 | 3-Cl-5-F-Ph | H | O | H | CH₂ | CF₃ | |
| 9.368 | 3-Cl-5-Me-Ph | H | O | H | CH₂ | CF₃ | |
| 9.369 | 3-Cl-5-CF₃O-Ph | H | O | H | CH₂ | CF₃ | |
| 9.370 | 3-Cl-Ph | H | O | H | CH₂ | CF₃ | |
| 9.371 | 3-EtO-Ph | H | O | H | CH₂ | CF₃ | |
| 9.372 | 3-Et-5-F-Ph | H | O | H | CH₂ | CF₃ | |
| 9.373 | 3-Et-Ph | H | O | H | CH₂ | CF₃ | |
| 9.374 | 3-F-5-MeS-Ph | H | O | H | CH₂ | CF₃ | |
| 9.375 | 3-F-5-MeSO₂-Ph | H | O | H | CH₂ | CF₃ | |
| 9.376 | 3-F-5-CF₃-Ph | H | O | H | CH₂ | CF₃ | |
| 9.377 | 3-F-5-MeO-Ph | H | O | H | CH₂ | CF₃ | |
| 9.378 | 3-F-5-Me-Ph | H | O | H | CH₂ | CF₃ | |
| 9.379 | 3-F-Ph | H | O | H | CH₂ | CF₃ | |
| 9.380 | 3-OH-Ph | H | O | H | CH₂ | CF₃ | |
| 9.381 | 3-iPr-Ph | H | O | H | CH₂ | CF₃ | |
| 9.382 | 3-Me-Ph | H | O | H | CH₂ | CF₃ | |
| 9.383 | 3-NO₂-Ph | H | O | H | CH₂ | CF₃ | |
| 9.384 | 4-EtO-Ph | H | O | H | CH₂ | CF₃ | |
| 9.385 | 4-MeO-Ph | H | O | H | CH₂ | CF₃ | |
| 9.386 | F₅-Ph | H | O | H | CH₂ | CF₃ | |
| 9.387 | Ph | H | O | H | CH₂ | CF₃ | |
| 9.388 | 2,3,5-F₃-Ph | H | O | H | CH₂ | CF₃ | |
| 9.389 | 2,3-F₂-Ph | H | O | H | CH₂ | CH₃ | |
| 9.390 | 3,4-F₂-Ph | H | O | H | CH₂ | CH₃ | |
| 9.391 | 3,5-Cl₂-Ph | H | O | H | CH₂ | CH₃ | |
| 9.392 | 3,5-F₂-Ph | H | O | H | CH₂ | CH₃ | |
| 9.393 | 3-Cl-5-CF₃-Ph | H | O | H | CH₂ | CH₃ | |
| 9.394 | 3-Cl-5-Me-Ph | H | O | H | CH₂ | CH₃ | |
| 9.395 | 3-Cl-5-CF₃O-Ph | H | O | H | CH₂ | CH₃ | |
| 9.396 | 3-EtO-Ph | H | O | H | CH₂ | CH₃ | |
| 9.397 | 3-Et-Ph | H | O | H | CH₂ | CH₃ | |
| 9.398 | 3-F-Ph | H | O | H | CH₂ | CH₃ | |
| 9.399 | 3-Me-Ph | H | O | H | CH₂ | CH₃ | |
| 9.400 | F₅-Ph | H | O | H | CH₂ | CH₃ | |
| 9.401 | Ph | H | O | H | CH₂ | CH₃ | |
| 9.402 | 3,5-Cl₂-Ph | H | O | H | CH₂ | CH₃ | |
| 9.403 | 3,5-F₂-Ph | H | O | H | CH₂ | COOH | |
| 9.404 | 3-Cl-Ph | H | O | H | CH₂ | COOH | |
| 9.405 | 3-F-Ph | H | O | H | CH₂ | COOH | |
| 9.406 | 3,5-Cl₂-Ph | H | O | H | CH₂ | COOH | |
| 9.407 | 3,5-F₂-Ph | H | O | H | CH₂ | Cyan | |
| 9.408 | 3-Cl-5-CF₃O-Ph | H | O | H | CH₂ | Cyan | |
| 9.409 | 3-F-5-Me-Ph | H | O | H | CH₂ | Cyan | |
| 9.410 | 3-F-Ph | H | O | H | CH₂ | Cyan | |
| 9.411 | 3-NO₂-Ph | H | O | H | CH₂ | Cyan | |
| 9.412 | 3,5-Cl₂-Ph | H | O | H | CH₂ | Cyan | |
| 9.413 | 3,5-Cl₂-Ph | H | O | H | CH₂ | Cyclohexyl | |
| 9.414 | 3,5-F₂-Ph | H | O | H | CH₂ | c-Pr | |
| 9.415 | 3,5-Cl₂-Ph | H | O | H | CH₂ | c-Pr | |
| 9.416 | 3,5-F₂-Ph | H | O | H | CH₂ | Diethoxyphosphoryl | |
| 9.417 | 3-F-Ph | H | O | H | CH₂ | Diethoxyphosphoryl | |
| 9.418 | Ph | H | O | H | CH₂ | Diethoxyphosphoryl | |
| 9.419 | 3,5-Cl₂-Ph | H | O | H | CH₂ | Diethoxyphosphoryl | |
| 9.420 | 3,5-Cl₂-Ph | H | O | H | CH₂ | Difluormethyl | |
| 9.421 | 3-Cl-Ph | H | O | H | CH₂ | Dimethoxymethyl | |
| 9.422 | 3,5-Cl₂-Ph | H | O | H | CH₂ | Dimethoxymethyl | |
| 9.423 | 3,5-Cl₂-Ph | H | O | H | CH₂ | Dimethylcarbamoyl | |
| 9.424 | 3,5-Cl₂-Ph | H | O | Prop-2-en-1-yl | CH₂ | Ethenyl | |
| 9.425 | 3,5-F₂-Ph | H | O | H | CH₂ | Ethenyl | |
| 9.426 | 3-F-Ph | H | O | H | CH₂ | Ethenyl | |
| 9.427 | Ph | H | O | H | CH₂ | Ethenyl | |
| 9.428 | 3,5-(CF₃)₂-Ph | H | O | H | CH₂ | Ethenyl | |
| 9.429 | 3,5-Cl₂-Ph | H | O | H | CH₂ | Ethinyl | |
| 9.430 | 3,5-Cl₂-Ph | H | O | Prop-2-in-1-yl | CH₂ | Ethinyl | |
| 9.431 | 3,5-F₂-Ph | H | O | H | CH₂ | Ethinyl | |
| 9.432 | 3,5-Cl₂-Ph | H | O | cPr | CH₂ | Ethinyl | |
| 9.433 | 3-Cl-Ph | H | O | H | CH₂ | Ethoxycarbonyl | |
| 9.434 | 3-F-Ph | H | O | H | CH₂ | Ethoxycarbonyl | |
| 9.435 | 3,5-F₂-Ph | H | O | H | CH₂ | Ethoxycarbonyl | |
| 9.436 | 3,5-Cl₂-Ph | H | O | H | CH₂ | Formyl | |
| 9.437 | 3,5-F₂-Ph | H | O | H | CH₂ | Methoxycarbonyl | |
| 9.438 | 3-Cl-Ph | H | O | H | CH₂ | Methoxycarbonyl | |
| 9.439 | 3-F-Ph | H | O | H | CH₂ | Methoxycarbonyl | |
| 9.440 | 3-Cl-5-F-Ph | H | O | H | CH₂ | Methoxycarbonyl | |
| 9.441 | 3,5-Cl₂-Ph | H | O | H | CH₂ | Nonyl | |
| 9.442 | 3,5-Cl₂-Ph | H | O | H | CH₂ | Pentafluorethyl | |
| 9.443 | 3,5-F₂-Ph | H | O | H | CH₂ | Pentyl | |
| 9.444 | 3,5-Cl₂-Ph | H | O | H | CH₂ | Pentyl | |
| 9.445 | 3,5-F₂-Ph | H | O | H | CH₂ | Piperidin-2-yl | |
| 9.446 | Ph | H | O | H | CH₂ | Piperidin-2-yl | |
| 9.447 | 3,5-Cl₂-Ph | H | O | CH₃ | CH₂ | Piperidin-2-yl | |
| 9.448 | 3,5-Cl₂-Ph | H | O | H | CH₂ | Propan-2-yl | |
| 9.449 | 3,5-Cl₂-Ph | H | O | H | CH₂ | Propan-2-yl | |
| 9.450 | 3,5-Cl₂-Ph | H | O | H | CH₂ | Pyrrolidin-1-ylcarbonyl | |
| 9.451 | 3,5-F₂-Ph | H | O | H | CH₂ | tert-Butyl | |
| 9.452 | 3,5-Cl₂-Ph | H | O | H | CH₂ | tert-Butyl | |
| 9.453 | 3,5-F₂-Ph | H | O | H | CH₂ | Tetrahydrofuran-2-yl | |
| 9.454 | 3-F-Ph | H | O | H | CH₂ | Tetrahydrofuran-2-yl | |
| 9.455 | Ph | H | O | H | CH₂ | Tetrahydrofuran-2-yl | |
| 9.456 | 3,5-Cl₂-Ph | H | O | H | CH₂ | Tetrahydrofuran-2-yl | |
| 9.457 | 3,5-F₂-Ph | H | O | H | CH₂ | Tetrahydrofuran-3-yl | |
| 9.458 | 3-F-Ph | H | O | H | CH₂ | Tetrahydrofuran-3-yl | |
| 9.459 | Ph | H | O | H | CH₂ | Tetrahydrofuran-3-yl | |
| 9.460 | 3,5-Cl₂-Ph | H | O | H | CH₂C(C H₃)₂ | Tetrahydrofuran-3-yl | |
| 9.461 | 3,5-Cl₂-Ph | H | O | H | CH₂C(C H₃)₂ | COOH | |
| 9.462 | 3,5-Cl₂-Ph | H | O | H | CH₂CH( CH₃) | Ethoxycarbonyl | |
| 9.463 | 3,5-Cl₂-Ph | H | O | H | CH₂CH₂ | Ethoxycarbonyl | |
| 9.464 | 3,5-Cl₂-Ph | H | O | H | CH₂CH₂ | (2,2,2-Trifluoroethoxy)carbonyl | |
| 9.465 | 3,5-Cl₂-Ph | H | O | H | CH₂CH₂ | (2-Hydroxyethoxy)carbonyl | |
| 9.466 | 3,5-Cl₂-Ph | H | O | H | CH₂CH₂ | (2-Methoxy-2-oxoethyl)carbamoyl | |
| 9.467 | 3,5-Cl₂-Ph | H | O | H | CH₂CH₂ | (Allyloxy)carbonyl | |
| 9.468 | 3,5-F₂-Ph | H | O | H | CH₂CH₂ | (Cyclopropylsulfonyl)(methyl)carbamoyl | |
| 9.469 | 3,5-Cl₂-Ph | H | O | H | CH₂CH₂ | (Cyclopropylsulfonyl)(methyl)carbamoyl | |
| 9.470 | 3,5-F₂-Ph | H | O | H | CH₂CH₂ | (Cyclopropylsulfonyl)carbamoyl | |
| 9.471 | 3,5-Cl₂-Ph | H | O | H | CH₂CH₂ | (Cyclopropylsulfonyl)carbamoyl | |
| 9.472 | 3,5-F₂-Ph | H | O | H | CH₂CH₂ | (Ethylsulfonyl)(methyl)carbamoyl | |
| 9.473 | 3,5-Cl₂-Ph | H | O | H | CH₂CH₂ | (Ethylsulfonyl)(methyl)carbamoyl | |
| 9.474 | 3,5-F₂-Ph | H | O | H | CH₂CH₂ | (Ethylsulfonyl)carbamoyl | |
| 9.475 | 3,5-Cl₂-Ph | H | O | H | CH₂CH₂ | (Ethylsulfonyl)carbamoyl | |
| 9.476 | 3,5-F₂-Ph | H | O | H | CH₂CH₂ | (Heptan-2-yloxy)carbonyl | |
| 9.477 | 3,5-Cl₂-Ph | H | O | H | CH₂CH₂ | (Hydroxyimino)methyl | |
| 9.478 | 3,5-F₂-Ph | H | O | H | CH₂CH₂ | (Isopropylsulfonyl)(methyl)carbamoyl | |
| 9.479 | 3,5-Cl₂-Ph | H | O | H | CH₂CH₂ | (Isopropylsulfonyl)(methyl)carbamoyl | |
| 9.480 | 3,5-F₂-Ph | H | O | H | CH₂CH₂ | (Isopropylsulfonyl)carbamoyl | |
| 9.481 | 3,5-Cl₂-Ph | H | O | H | CH₂CH₂ | (Isopropylsulfonyl)carbamoyl | |
| 9.482 | 3,5-F₂-Ph | H | O | H | CH₂CH₂ | (Methylsulfonyl)carbamoyl | |
| 9.483 | 3,5-Cl₂-Ph | H | O | H | CH₂CH₂ | (Methylsulfonyl)carbamoyl | |
| 9.484 | 3,5-Cl₂-Ph | H | O | H | CH₂CH₂ | (Prop-2-Yn-1-yloxy)carbonyl | |
| 9.485 | 3,5-Cl₂-Ph | H | O | H | CH₂CH₂ | (Propan-2-yloxy)carbonyl | |
| 9.486 | 3,5-Cl₂-Ph | H | O | H | CH₂CH₂ | [2-(Methylsulfanyl)ethoxy]carbonyl | |
| 9.487 | 3,5-F₂-Ph | H | O | H | CH₂CH₂ | 1,3-Dioxolan-2-yl | |
| 9.488 | 3-F-Ph | H | O | H | CH₂CH₂ | 1,3-Dioxolan-2-yl | |
| 9.489 | Ph | H | O | H | CH₂CH₂ | 1,3-Dioxolan-2-yl | |
| 9.490 | 3,5-Cl₂-Ph | H | O | H | CH₂CH₂ | 1,3-Dioxolan-2-yl | |
| 9.491 | 3,5-Cl₂-Ph | H | O | H | CH₂CH₂ | 1-Methylpyrrolidin-2-yl | |
| 9.492 | 3,5-Cl₂-Ph | H | O | H | CH₂CH₂ | 2-Oxopyrrolidin-1-yl | |
| 9.493 | 3,4,5-F₃-Ph | H | O | H | CH₂CH₂ | Butoxycarbonyl | |
| 9.494 | 3,5-Cl₂-Ph | H | O | H | CH₂CH₂ | Butylcarbamoyl | |
| 9.495 | 3-Cl-4-Me-Ph | H | O | H | CH₂CH₂ | Carbamoyl | |
| 9.496 | 3,5-Cl₂-Ph | H | O | H | CH₂CH₂ | Carbamoyl | |
| 9.497 | 3,5-F₂-Ph | H | O | H | CH₂CH₂ | CF₃ | |
| 9.498 | 3,5-Cl₂-Ph | H | O | H | CH₂CH₂ | CF₃ | |
| 9.499 | 3,5-F₂-Ph | H | O | H | CH₂CH₂ | CH₃ | |
| 9.500 | 3,5-Cl₂-Ph | H | O | H | CH₂CH₂ | CH₃ | |
| 9.501 | 2,3,4,5-F₄-6-OH-Ph | H | O | H | CH₂CH₂ | Chlor | |
| 9.502 | 2,3,5-F₃-Ph | H | O | H | CH₂CH₂ | COOH | |
| 9.503 | 2,3-F₂-Ph | H | O | H | CH₂CH₂ | COOH | |
| 9.504 | 2,5-Cl₂-Ph | H | O | H | CH₂CH₂ | COOH | |
| 9.505 | 2,5-F₂-Ph | H | O | H | CH₂CH₂ | COOH | |
| 9.506 | 2-Cl-5-F-Ph | H | O | H | CH₂CH₂ | COOH | |
| 9.507 | 2-F-3-Me-Ph | H | O | H | CH₂CH₂ | COOH | |
| 9.508 | 3-CF₃-Ph | H | O | H | CH₂CH₂ | COOH | |
| 9.509 | 3,4,5-F₃-Ph | H | O | H | CH₂CH₂ | COOH | |
| 9.510 | 3,4-F₂-Ph | H | O | H | CH₂CH₂ | COOH | |
| 9.511 | 3,5-Br₂-Ph | H | O | H | CH₂CH₂ | COOH | |
| 9.512 | 3,5-Cl₂-Ph | H | O | CH₃ | CH₂CH₂ | COOH | |
| 9.513 | 3,5-Cl₂-Ph | H | O | H | CH₂CH₂ | COOH | |
| 9.514 | 3,5-F₂-Ph | H | O | H | CH₂CH₂ | COOH | |
| 9.515 | 3,5-Me₂-Ph | H | O | H | CH₂CH₂ | COOH | |
| 9.516 | 3,5-(tert.Bu)₂-Ph | H | O | H | CH₂CH₂ | COOH | |
| 9.517 | 3-Br-5-Cl-Ph | H | O | H | CH₂CH₂ | COOH | |
| 9.518 | 3-Br-5-F-Ph | H | O | H | CH₂CH₂ | COOH | |
| 9.519 | 3-Br-5-CF₃-Ph | H | O | H | CH₂CH₂ | COOH | |
| 9.520 | 3-Cl-4-F-Ph | H | O | H | CH₂CH₂ | COOH | |
| 9.521 | 3-Cl-4-Me-Ph | H | O | H | CH₂CH₂ | COOH | |
| 9.522 | 3-Cl-5-F-Ph | H | O | H | CH₂CH₂ | COOH | |
| 9.523 | 3-Cl-5-CF₃O-Ph | H | O | H | CH₂CH₂ | COOH | |
| 9.524 | 3-Cl-5-CF₃-Ph | H | O | H | CH₂CH₂ | COOH | |
| 9.525 | 3-Cl-5-Me-Ph | H | O | H | CH₂CH₂ | COOH | |
| 9.526 | 3-Et-5-F-Ph | H | O | H | CH₂CH₂ | COOH | |
| 9.527 | 3-Et-Ph | H | O | H | CH₂CH₂ | COOH | |
| 9.528 | 3-F-5-MeO-Ph | H | O | H | CH₂CH₂ | COOH | |
| 9.529 | 3-F-5-Me-Ph | H | O | H | CH₂CH₂ | COOH | |
| 9.530 | 3-F-Ph | H | O | H | CH₂CH₂ | COOH | |
| 9.531 | 3-Me-Ph | H | O | H | CH₂CH₂ | COOH | |
| 9.532 | 4-CI-Ph | H | O | H | CH₂CH₂ | COOH | |
| 9.533 | 4-EtO-Ph | H | O | H | CH₂CH₂ | COOH | |
| 9.534 | Ph | H | O | H | CH₂CH₂ | COOH | |
| 9.535 | 3,5-Cl₂-Ph | H | O | H | CH₂CH₂ | COOH | |
| 9.536 | 3-Cl-Ph | H | O | H | CH₂CH₂ | Cyan | |
| 9.537 | 3,5-Cl₂-Ph | H | O | H | CH₂CH₂ | Cyan | |
| 9.538 | 3,5-Cl₂-Ph | H | O | H | CH₂CH₂ | Cyclopropylcarbamoyl | |
| 9.539 | 3,5-Cl₂-Ph | H | O | H | CH₂CH₂ | Diethoxymethyl | |
| 9.540 | Ph | H | O | H | CH₂CH₂ | Diethoxyphosphoryl | |
| 9.541 | 3,5-Cl₂-Ph | H | O | H | CH₂CH₂ | Diethoxyphosphoryl | |
| 9.542 | 3,5-Cl₂-Ph | H | O | H | CH₂CH₂ | Dimethoxymethyl | |
| 9.543 | 3,5-Cl₂-Ph | H | O | H | CH₂CH₂ | Dimethylcarbamoyl | |
| 9.544 | 3-Cl-Ph | H | O | H | CH₂CH₂ | Ethoxy | |
| 9.545 | 2-Cl-5-F-Ph | H | O | H | CH₂CH₂ | Ethoxy | |
| 9.546 | 3-CF₃-Ph | H | O | H | CH₂CH₂ | Ethoxycarbonyl | |
| 9.547 | 3,5-Cl₂-Ph | H | O | cPr | CH₂CH₂ | Ethoxycarbonyl | |
| 9.548 | 3,5-Cl₂-Ph | H | O | H | CH₂CH₂ | Ethoxycarbonyl | |
| 9.549 | 3,5-F₂-Ph | H | O | H | CH₂CH₂ | Ethoxycarbonyl | |
| 9.550 | 3-Br-5-CF₃-Ph | H | O | H | CH₂CH₂ | Ethoxycarbonyl | |
| 9.551 | 3-Cl-5-Et-Ph | H | O | H | CH₂CH₂ | Ethoxycarbonyl | |
| 9.552 | 3-Cl-5-CF₃-Ph | H | O | H | CH₂CH₂ | Ethoxycarbonyl | |
| 9.553 | 3-Et-5-F-Ph | H | O | H | CH₂CH₂ | Ethoxycarbonyl | |
| 9.554 | 3-Et-Ph | H | O | H | CH₂CH₂ | Ethoxycarbonyl | |
| 9.555 | 3-F-5-MeO-Ph | H | O | H | CH₂CH₂ | Ethoxycarbonyl | |
| 9.556 | 3-Me-5-CF₃O-Ph | H | O | H | CH₂CH₂ | Ethoxycarbonyl | |
| 9.557 | 3,5-Cl₂-Ph | H | O | H | CH₂CH₂ | Ethoxycarbonyl | |
| 9.558 | 3,5-F₂-Ph | H | O | H | CH₂CH₂ | Ethylcarbamoyl | |
| 9.559 | 3,5-Cl₂-Ph | H | O | H | CH₂CH₂ | Formyl | |
| 9.560 | 3,5-F₂-Ph | H | O | H | CH₂CH₂ | Hydroxy | |
| 9.561 | 3-Cl-Ph | H | O | H | CH₂CH₂ | Hydroxy | |
| 9.562 | 3,5-Cl₂-Ph | H | O | H | CH₂CH₂ | Hydroxy | |
| 9.563 | 3,5-Cl₂-Ph | H | O | H | CH₂CH₂ | Isobutoxycarbonyl | |
| 9.564 | 3-Cl-5-CF₃-Ph | H | O | H | CH₂CH₂ | Methoxy | |
| 9.565 | 3-Cl-Ph | H | O | H | CH₂CH₂ | Methoxy | |
| 9.566 | 2,3,4-F₃-Ph | H | O | H | CH₂CH₂ | Methoxy | |
| 9.567 | 2,3,5-F₃-Ph | H | O | H | CH₂CH₂ | Methoxycarbonyl | |
| 9.568 | 2,3-F₂-Ph | H | O | H | CH₂CH₂ | Methoxycarbonyl | |
| 9.569 | 2,5-Cl₂-Ph | H | O | H | CH₂CH₂ | Methoxycarbonyl | |
| 9.570 | 2,5-F₂-Ph | H | O | H | CH₂CH₂ | Methoxycarbonyl | |
| 9.571 | 2,5-Me₂-Ph | H | O | H | CH₂CH₂ | Methoxycarbonyl | |
| 9.572 | 2-Cl-3-F-5-MeO-Ph | H | O | H | CH₂CH₂ | Methoxycarbonyl | |
| 9.573 | 2-F-3-Me-Ph | H | O | H | CH₂CH₂ | Methoxycarbonyl | |
| 9.574 | 3-CF₃-Ph | H | O | H | CH₂CH₂ | Methoxycarbonyl | |
| 9.575 | 3,4,5-F₃-Ph | H | O | H | CH₂CH₂ | Methoxycarbonyl | |
| 9.576 | 3,4-F₂-Ph | H | O | H | CH₂CH₂ | Methoxycarbonyl | |
| 9.577 | 3,5-Br₂-Ph | H | O | H | CH₂CH₂ | Methoxycarbonyl | |
| 9.578 | 3,5-Cl₂-4-MeO-Ph | H | O | H | CH₂CH₂ | Methoxycarbonyl | |
| 9.579 | 3,5-Cl₂-Ph | CH₃ | O | H | CH₂CH₂ | Methoxycarbonyl | |
| 9.580 | 3,5-Cl₂-Ph | H | O | CH₃ | CH₂CH₂ | Methoxycarbonyl | |
| 9.581 | 3,5-Cl₂-Ph | H | O | H | CH₂CH₂ | Methoxycarbonyl | [CDCl₃] 7.71 0.67;7.71 0.71;7.53 4.91;7.52 6.49;7.49 1.78;7.49 2.59;7.48 1.17;7.48 0.34;7.34 0.42;7.26 18.46;7.19 0.71;5.30 0.41;4.10 1.44;4.06 3.46;4.00 3.43;3.96 1.43; 3.75 2.10;3.74 0.69;3.72 16.00;3.69 0.42;3.67 0.38;3.67 0.40;3.66 0.49;3.65 0.84;3.64 0.77;3.64 0.53;3.62 0.39; 3.61 0.36; 3.60 0.72;3.59 0.47;3.58 0.52;3.58 0.72;3.56 0.44;3.56 0.42;3.54 0.36;2.68 0.32;2.63 0.98;2.62 0.98;2.61 1.05;2.61 1.53;2.59 0.96;2.59 0.97;1.58 8.31;0.00 7.16 |
| 9.582 | 3,5-Et₂-Ph | H | O | H | CH₂CH₂ | Methoxycarbonyl | |
| 9.583 | 3,5-F₂-Ph | H | O | H | CH₂CH₂ | Methoxycarbonyl | [CDCl₃] 7.34 0.32;7.34 0.32;7.32 0.49;7.31 0.38;7.31 0.39;7.26 14.30;7.26 11.16;7.19 1.35;7.18 1.33; 7.18 1.98;7.17 1.24;7.17 1.77;7.17 1.86;7.16 1.58;6.99 0.35;6.98 0.61;6.98 0.34;6.97 0.71;6.96 1.24;6.95 0.66;6.94 0.38;6.94 0.64;6.93 0.34;4.10 1.51;4.05 3.62;4.00 3.65;3.95 1.48;3.75 0.55;3.72 16.00;3.71 0.33;3.69 0.50;3.67 0.50;3.67 0.53;3.66 0.99;3.64 0.86;3.63 0.42;3.61 0.40;3.60 0.84;3.58 0.57;3.58 0.87;3.57 0.48;3.57 0.48;3.56 0.52;3.55 0.44;2.62 1.14;2.62 1.18;2.61 1.27;2.61 2.20;2.59 1.16;2.59 1.19;2.04 0.47;1.56 3.55;1.26 0.43;1.26 0.42;0.00 6.65;0.00 5.26 |
| 9.584 | 3,5-(MeO)₂-Ph | H | O | H | CH₂CH₂ | Methoxycarbonyl | |
| 9.585 | 3,5-Me₂-Ph | H | O | H | CH₂CH₂ | Methoxycarbonyl | |
| 9.586 | 3,5-(tert.Bu)₂-Ph | H | O | H | CH₂CH₂ | Methoxycarbonyl | |
| 9.587 | 3-CF₃S-Ph | H | O | H | CH₂CH₂ | Methoxycarbonyl | |
| 9.588 | 3-Ac-Ph | H | O | H | CH₂CH₂ | Methoxycarbonyl | |
| 9.589 | 3-Br-5-Cl-Ph | H | O | H | CH₂CH₂ | Methoxycarbonyl | |
| 9.590 | 3-Br-5-F-Ph | H | O | H | CH₂CH₂ | Methoxycarbonyl | |
| 9.591 | 3-Br-5-CF₃-Ph | H | O | H | CH₂CH₂ | Methoxycarbonyl | |
| 9.592 | 3-NH₂CO-Ph | H | O | H | CH₂CH₂ | Methoxycarbonyl | |
| 9.593 | 3-Cl-4-F-Ph | H | O | H | CH₂CH₂ | Methoxycarbonyl | |
| 9.594 | 3-Cl-4-Me-Ph | H | O | H | CH₂CH₂ | Methoxycarbonyl | |
| 9.595 | 3-Cl-5-CN-Ph | H | O | H | CH₂CH₂ | Methoxycarbonyl | |
| 9.596 | 3-Cl-5-Et-Ph | H | O | H | CH₂CH₂ | Methoxycarbonyl | |
| 9.597 | 3-Cl-5-F-Ph | H | O | H | CH₂CH₂ | Methoxycarbonyl | |
| 9.598 | 3-Cl-5-MeO-Ph | H | O | H | CH₂CH₂ | Methoxycarbonyl | |
| 9.599 | 3-Cl-5-CF₃O-Ph | H | O | H | CH₂CH₂ | Methoxycarbonyl | |
| 9.600 | 3-Cl-5-CF₃-Ph | H | O | H | CH₂CH₂ | Methoxycarbonyl | |
| 9.601 | 3-Cl-5-Me-Ph | H | O | H | CH₂CH₂ | Methoxycarbonyl | |
| 9.602 | 3-CN-5-F-Ph | H | O | H | CH₂CH₂ | Methoxycarbonyl | |
| 9.603 | 3-CN-Ph | H | O | H | CH₂CH₂ | Methoxycarbonyl | |
| 9.604 | 3-c-Pr-5-F-Ph | H | O | H | CH₂CH₂ | Methoxycarbonyl | |
| 9.605 | 3-Et-5-F-Ph | H | O | H | CH₂CH₂ | Methoxycarbonyl | |
| 9.606 | 3-F-5-MeS-Ph | H | O | H | CH₂CH₂ | Methoxycarbonyl | |
| 9.607 | 3-F-5-MeSO₂-Ph | H | O | H | CH₂CH₂ | Methoxycarbonyl | |
| 9.608 | 3-F-5-MeO-Ph | H | O | H | CH₂CH₂ | Methoxycarbonyl | |
| 9.609 | 3-F-5-Me-Ph | H | O | H | CH₂CH₂ | Methoxycarbonyl | |
| 9.610 | 3-F-Ph | H | O | H | CH₂CH₂ | Methoxycarbonyl | |
| 9.611 | 3-Me-Ph | H | O | H | CH₂CH₂ | Methoxycarbonyl | |
| 9.612 | 3-NO₂-Ph | H | O | H | CH₂CH₂ | Methoxycarbonyl | |
| 9.613 | 4-Cl-Ph | H | O | H | CH₂CH₂ | Methoxycarbonyl | |
| 9.614 | 4-EtO-Ph | H | O | H | CH₂CH₂ | Methoxycarbonyl | |
| 9.615 | 4-MeO-Ph | H | O | H | CH₂CH₂ | Methoxycarbonyl | |
| 9.616 | F₅-Ph | H | O | H | CH₂CH₂ | Methoxycarbonyl | |
| 9.617 | Ph | H | O | H | CH₂CH₂ | Methoxycarbonyl | |
| 9.618 | 3,5-Cl₂-Ph | H | O | H | CH₂CH₂ | Methoxycarbonyl | |
| 9.619 | 3,5-F₂-Ph | H | O | H | CH₂CH₂ | Methoxycarbonyl | |
| 9.620 | 2,5-Cl₂-Ph | H | O | H | CH₂CH₂ | Methoxycarbonyl | |
| 9.621 | 3,5-Cl₂-Ph | H | O | H | CH₂CH₂ | Methyl(methylsulfonyl)carbamoyl | |
| 9.622 | 3-Cl-4-F-Ph | H | O | H | CH₂CH₂ | Methyl(methylsulfonyl)carbamoyl | |
| 9.623 | 3-Cl-4-Me-Ph | H | O | H | CH₂CH₂ | Methylcarbamoyl | |
| 9.624 | 3,5-Cl₂-Ph | H | O | H | CH₂CH₂ | Methylcarbamoyl | |
| 9.625 | 3,5-Cl₂-Ph | H | O | H | CH₂CH₂ | Methylcarbamoyl | |
| 9.626 | 3,5-Cl₂-Ph | H | O | H | CH₂CH₂ | Methylcarbamoyl | |
| 9.627 | 3,5-Cl₂-Ph | H | O | H | CH₂CH₂ | Methylsulfanyl | |
| 9.628 | 3,5-Cl₂-Ph | H | O | H | CH₂CH₂ | Methylsulfonyl | |
| 9.629 | Ph | H | O | H | CH₂CH₂ | Morpholin-4-yl | |
| 9.630 | 3,5-Cl₂-Ph | H | O | H | CH₂CH₂ | Morpholin-4-ylcarbonyl | |
| 9.631 | 3,5-Cl₂-Ph | H | O | H | CH₂CH₂ | Phosphono | |
| 9.632 | 3,5-Cl₂-Ph | H | O | H | CH₂CH₂ | Phosphono | |
| 9.633 | 3,5-Cl₂-Ph | H | O | H | CH₂CH₂ | Piperidin-1-yl | |
| 9.634 | 3,5-Cl₂-Ph | H | O | H | CH₂CH₂ | Piperidin-1-ylcarbonyl | |
| 9.635 | 3,5-Cl₂-Ph | H | O | H | CH₂CH₂ | Propan-2-yloxy | |
| 9.636 | 3,5-Cl₂-Ph | H | O | H | CH₂CH₂ | Propoxy | |
| 9.637 | 3,5-F₂-Ph | H | O | H | CH₂CH₂ | Propoxycarbonyl | |
| 9.638 | 3,5-Cl₂-Ph | H | O | H | CH₂CH₂ | Pyrrolidin-1-yl | |
| 9.639 | 3,5-F₂-Ph | H | O | H | CH₂CH₂ | Sec-Butoxycarbonyl | |
| 9.640 | 3,5-Cl₂-Ph | H | O | H | CH₂CH₂ | Sulfamoyl | |
| 9.641 | 3,5-Cl₂-Ph | H | O | H | CH₂CH₂ | tert-Butoxycarbonyl | |
| 9.642 | 3,5-F₂-Ph | H | O | H | CH₂CH₂ | tert-Butoxycarbonyl | |
| 9.643 | 3-F-Ph | H | O | H | CH₂CH₂ | Tetrahydrofuran-2-yl | |
| 9.644 | Ph | H | O | H | CH₂CH₂ | Trifluormethoxy | |
| 9.645 | 3-Cl-Ph | H | O | H | (CH₂)₃ | Trifluormethoxy | |
| 9.646 | 3-F-Ph | H | O | H | (CH₂)₃ | Trifluormethoxy | |
| 9.647 | 3,5-Cl₂-Ph | H | O | H | (CH₂)₃ | Trifluormethoxy | |
| 9.648 | 3,5-Cl₂-Ph | H | O | H | (CH₂)₃ | (Propan-2-yloxy)carbonyl | |
| 9.649 | 3,5-F₂-Ph | H | O | H | (CH₂)₃ | (Propan-2-yloxy)carbonyl | |
| 9.650 | Ph | H | O | H | (CH₂)₃ | 2-Carboxyethyl | |
| 9.651 | 3,5-Cl₂-Ph | H | O | H | (CH₂)₃ | 2-Methyl-1,3-dioxolan-2-yl | |
| 9.652 | 3,5-Cl₂-Ph | H | O | H | (CH₂)₃ | 2-Methyl-1,3-dioxolan-2-yl | |
| 9.653 | 3,5-Cl₂-Ph | H | O | H | (CH₂)₃ | 2-Methyl-1,3-dioxolan-2-yl | |
| 9.654 | 3,5-F₂-Ph | H | O | H | (CH₂)₃ | 2-Oxopyrrolidin-1-yl | |
| 9.655 | 3,5-Cl₂-Ph | H | O | H | (CH₂)₃ | CF3 | |
| 9.656 | 3,5-F₂-Ph | H | O | H | (CH₂)₃ | CH3 | |
| 9.657 | 3-Br-5-Cl-Ph | H | O | H | (CH₂)₃ | CH3 | |
| 9.658 | 3-Cl-Ph | H | O | H | (CH₂)₃ | COOH | |
| 9.659 | 3-F-Ph | H | O | H | (CH₂)₃ | COOH | |
| 9.660 | Ph | H | O | H | (CH₂)₃ | COOH | |
| 9.661 | 3,5-Cl₂-Ph | H | O | H | (CH₂)₃ | COOH | |
| 9.662 | Ph | H | O | Etcarba moyl | (CH₂)₃ | COOH | |
| 9.663 | 3,5-Cl₂-Ph | H | O | H | (CH₂)₃ | COOH | |
| 9.664 | 3,5-Cl₂-Ph | H | O | H | (CH₂)₃ | Dimethylamino | |
| 9.665 | 3-Cl-Ph | H | O | H | (CH₂)₃ | Dimethylamino | |
| 9.666 | 3-F-Ph | H | O | H | (CH₂)₃ | Ethoxy | |
| 9.667 | 3,5-Cl₂-Ph | H | O | H | (CH₂)₃ | Ethoxycarbonyl | |
| 9.668 | 3,5-F₂-Ph | H | O | H | (CH₂)₃ | Ethoxycarbonyl | |
| 9.669 | 3,5-Cl₂-Ph | H | O | H | (CH₂)₃ | Ethoxycarbonyl | |
| 9.670 | 3,5-F₂-Ph | H | O | H | (CH₂)₃ | Hydroxy | |
| 9.671 | 3-Br-5-Cl-Ph | H | O | H | (CH₂)₃ | Hydroxy | |
| 9.672 | 3-Cl-5-MeO-Ph | H | O | H | (CH₂)₃ | Methoxy | |
| 9.673 | 3-Cl-Ph | H | O | H | (CH₂)₃ | Methoxycarbonyl | |
| 9.674 | 3-F-Ph | H | O | H | (CH₂)₃ | Methoxycarbonyl | |
| 9.675 | Ph | H | O | H | (CH₂)₃ | Methoxycarbonyl | |
| 9.676 | 3,5-F₂-Ph | H | O | H | (CH₂)₃ | Methoxycarbonyl | |
| 9.677 | 3,5-Cl₂-Ph | H | O | H | (CH₂)₃ | Methoxycarbonyl | |
| 9.678 | 3,5-Cl₂-Ph | H | O | H | (CH₂)₆ | Methoxycarbonyl | |
| | | | | | | Methylcarbamoyl | |
| | | | | | | Morpholin-4-yl | |
| | | | | | | Methoxycarbonyl | |

| Tabelle 10: Erfindungsgemäße Verbindungen der allgemeinen Formel (I), worin R² für Wasserstoff steht, und Aryl den Rest 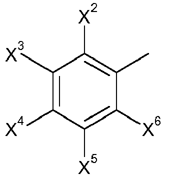 bedeutet. | | | | | | | | |
|---|---|---|---|---|---|---|---|---|
| 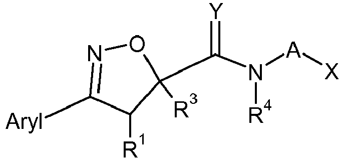 | | | | | | | | |

| Nr. | Aryl | R¹ | R³ | Y | R⁴ | A | X | Daten |
|---|---|---|---|---|---|---|---|---|
| 10.001 | 3,5-Cl₂-Ph | H | (Methylsulfan yl)methyl | O | H | Bindung | CH₃ | |
| 10.002 | 3,5-Cl₂-Ph | H | (Methylsulfan yl)methyl | O | H | Bindung | c-Pr | |
| 10.003 | 3,5-Cl₂-Ph | H | (Methylsulfan yl)methyl | O | H | CH₂CH₂ | Methoxycarbonyl | |
| 10.004 | 3,5-Cl₂-Ph | H | (Methylsulfon yl)methyl | O | H | Bindung | CH₃ | |
| 10.005 | 2-CF₃-Ph | H | C(OH)CH(CH 3)2 | O | H | Bindung | CH₃ | |
| 10.006 | 2,4-Cl₂-Ph | H | C(OH)CH(CH 3)2 | O | H | Bindung | CH₃ | |
| 10.007 | 3-Cl-Ph | H | C(OH)CH(CH 3)2 | O | H | Bindung | CH₃ | |
| 10.008 | 4-Cl-Ph | H | C(OH)CH(CH 3)2 | O | H | Bindung | CH₃ | |
| 10.009 | Ph | H | C(OH)CH(CH 3)2 | O | H | Bindung | CH₃ | |
| 10.010 | 2,4-Cl₂-Ph | H | C(OH)CH(CH 3)2 | O | H | Bindung | c-Pr | |
| 10.011 | 3-CF₃-Ph | H | C(OH)CH(CH 3)2 | O | H | Bindung | c-Pr | |
| 10.012 | 3,5-Cl₂-Ph | H | C(OH)CH(CH 3)2 | O | H | Bindung | c-Pr | |
| 10.013 | 3-Cl-Ph | H | C(OH)CH(CH 3)2 | O | H | Bindung | c-Pr | |
| 10.014 | 4-Cl-Ph | H | C(OH)CH(CH 3)2 | O | H | Bindung | c-Pr | |
| 10.015 | Ph | H | C(OH)CH(CH 3)2 | O | H | Bindung | c-Pr | |
| 10.016 | 2-CF₃-Ph | H | 1-Hydroxyethyl | O | H | Bindung | CH₃ | |
| 10.017 | 2,4-Cl₂-Ph | H | CH(CH₃)OH | O | H | Bindung | CH₃ | |
| 10.018 | 3,5-Cl₂-Ph | H | CH(CH₃)OH | O | H | Bindung | CH₃ | |
| 10.019 | 3-Cl-Ph | H | CH(CH₃)OH | O | H | Bindung | CH₃ | |
| 10.020 | 4-Cl-Ph | H | CH(CH₃)OH | O | H | Bindung | CH₃ | |
| 10.021 | Ph | H | CH(CH₃)OH | O | H | Bindung | CH₃ | |
| 10.022 | 2-Cl-Ph | H | CH(CH₃)OH | O | H | Bindung | c-Pr | |
| 10.023 | 3,5-Cl₂-Ph | H | CH(CH₃)OH | O | H | Bindung | c-Pr | |
| 10.024 | 3-Cl-Ph | H | CH(CH₃)OH | O | H | Bindung | c-Pr | |
| 10.025 | 4-Cl-Ph | H | CH(CH₃)OH | O | H | Bindung | c-Pr | |
| 10.026 | Ph | H | CH(CH₃)OH | O | H | Bindung | c-Pr | |
| 10.027 | 2,4-Cl₂-Ph | H | CH(CH₃)OH | O | H | Bindung | c-Pr | |
| 10.028 | 3-Cl-Ph | H | CH(CH₃)OH | O | H | Bindung | CH₃ | |
| 10.029 | Ph | H | CH(CH₃)OH | O | H | Bindung | CH₃ | |
| 10.030 | 3,5-Cl₂-Ph | H | CH(CH₃)OH | O | H | Bindung | c-Pr | |
| 10.031 | 3,5-Cl₂-Ph | H | Brommethyl | O | H | CH₂ | CF₃ | |
| 10.032 | 3,5-Cl₂-Ph | H | cPr | O | H | CH(CH₃) | CH₃ | |
| 10.033 | 3,5-Cl₂-Ph | H | cPr | O | H | CH₂ | CF₃ | |
| 10.034 | 2-CF₃-Ph | H | CH₂OH | O | H | Bindung | CH₃ | |
| 10.035 | 2,4-Cl₂-Ph | H | CH₂OH | O | H | Bindung | CH₃ | |
| 10.036 | 3,5-Cl₂-Ph | H | CH₂OH | O | H | Bindung | CH₃ | |
| 10.037 | 3-Cl-Ph | H | CH₂OH | O | H | Bindung | CH₃ | |
| 10.038 | 4-Cl-Ph | H | CH₂OH | O | H | Bindung | CH₃ | |
| 10.039 | Ph | H | CH₂OH | O | H | Bindung | CH₃ | |
| 10.040 | 2-CF₃-Ph | H | CH₂OH | O | H | Bindung | c-Pr | |
| 10.041 | 2,4-Cl₂**-**Ph | H | CH₂OH | O | H | Bindung | c-Pr | |
| 10.042 | 3,5-Cl₂-Ph | H | CH₂OH | O | H | Bindung | c-Pr | |
| 10.043 | 3-Cl-Ph | H | CH₂OH | O | H | Bindung | c-Pr | |
| 10.044 | 4-Cl-Ph | H | CH₂OH | O | H | Bindung | c-Pr | |
| 10.045 | Ph | H | CH₂OH | O | H | Bindung | c-Pr | |
| 10.046 | 2-CF₃-Ph | H | CH₂OH | O | H | Bindung | CH3 | |
| 10.047 | 3,5-Cl₂-Ph | H | CH₂OH | O | H | Bindung | CH3 | |
| 10.048 | 3-Cl-Ph | H | CH₂OH | O | H | Bindung | CH₃ | |
| 10.049 | 4-Cl-Ph | H | CH₂OH | O | H | Bindung | CH₃ | |
| 10.050 | Ph | H | CH₂OH | O | H | Bindung | CH₃ | |
| 10.051 | 3,5-Cl₂-Ph | H | CH₂OH | O | H | Bindung | c-Pr | |
| 10.052 | 3,5-Cl₂-Ph | H | iPr | O | H | Bindung | H | |
| 10.053 | 3,5-Cl₂-Ph | H | iPr | O | H | C(CH₃)₂ | Cyan | |
| 10.054 | 3,5-Cl₂-Ph | H | iPr | O | H | C(CH₃)₂ | Ethinyl | |
| 10.055 | 3,5-Cl₂-Ph | H | iPr | O | H | CH(CH₃) | CH₃ | |
| 10.056 | 3,5-Cl₂-Ph | H | iPr | O | H | CH₂ | CF₃ | |
| 10.057 | 3,5-Cl₂-Ph | H | Acetyl | O | CH 3 | Bindung | CH₃ | |
| 10.058 | 3,5-F₂-Ph | H | Acetyl | O | CH 3 | Bindung | CH₃ | |
| 10.059 | 3,5-F₂-Ph | H | F | O | H | Bindung | c-Pr | [CDCl₃] 0.66 (m,2H); 0.90 (m,2H); 2.33 (m,1H); 3.55 (dd,1H); 4.20 (dd,1H); 6.61 (s br,1H); 6.95 (m,1H); 7.20 (m,2H). |
| 10.060 | 3,5-Cl₂-Ph | H | F | O | H | Bindung | c-Pr | [CDCl₃] 0.65 (m,2H); 0.90 (m,2H); 2.85 (m,1H); 3.55 (dd,1H); 4.20 (dd,1H); 6.61 (s br,1H); 7.48 (s,1H); 7.58 (s,2H). |
| 10.061 | 3,5-F₂-Ph | H | CH₂F | O | H | Bindung | c-Pr | [CDCl₃] 0.55 (m,2H); 0.83 (m, 2H); 2.76 (m,1H); 3.43 (d,1H); 3.68 (d,1H); 4.62 (d,1H); 4.63 (d,0.5H); 4.72 (d,0.5H); 4.74 (d,0.5H); 4.84 (d,0.5H); 6.88 (m,2H); 7.16 (m,2H). |
| 10.062 | 3,5-Cl₂-Ph | H | Acetyl | O | H | Bindung | c-Pr | [CDCl₃] 0.59 (m,2H); 0.86 (m,2H); 2.23 (s,3H); 2.81 (m,1H); 3.53 (d,1H); 4.15 (d,1H); 6.94 (s br,1H); 7.44 (s,1H); 7.54 (s,2H). |
| 10.063 | 3-F-Ph | H | CH₂F | O | H | Bindung | c-Pr | |
| 10.064 | 3,5-F₂-Ph | H | Acetyl | O | H | Bindung | c-Pr | |
| 10.065 | 3,5-F₂-Ph | H | CH₂OH | O | H | Bindung | c-Pr | |
| 10.066 | 3,5-F₂-Ph | H | Prop-1-en-2-yl | O | H | Bindung | c-Pr | [CDCl₃] 0.54 (m,2H); 0.80 (m,2H); 1.78 (s,3H); 2.74 (m,1H); 3.32 (d,1H); 4.01 (d,1H); 5.07 (s,1H); 5.26 (s,1H); 6.75 (s br,1H); 6.88 (m,1H); 7.19 (m,2H). |
| 10.067 | 3,5-F₂-Ph | H | CH₂Cl | O | H | Bindung | c-Pr | |
| 10.068 | 3,5-Cl₂-Ph | H | Prop-1-en-2-yl | O | H | Bindung | c-Pr | [CDCl₃] 0.51 (m,2H); 0.81 (m,2H); 1.88 (s,3H); 2,74 (m,1H); 3.31 (d,1H); 4.02 (d,1H); 5.06 (s,1H); 5.25 (s,1 H); 6.72 (s br,1H); 7.41 (s,1H); 7.53 (s,2H). |
| 10.069 | 3,5-Cl₂-Ph | H | CH₂Cl | O | H | Bindung | c-Pr | [CDCl₃] 0.55 (m,2H); 0.83 (m,2H); 2.75 (m,1H); 3.54 (d,1H); 3.71 (d,1H); 3.97 (AB,2H); 6.99 (s br,1H); 7.43 (s,1H); 7.53 (s,2H); |
| 10.070 | 3,5-F₂-Ph | H | Vinyl | O | H | Bindung | c-Pr | [CDCl₃] 0.55 (m,2H); 0.82 (m,2H); 2.74 (m,1H); 3.30 (d,1H); 3.93 (d,1H); 5.34 (d,1H); 5.51 (d,1H); 6.15 (dd,1H); 6.78 (s br,1H); 6.88 (m,1H); 7.15 (m,2H); |
| 10.071 | 3-F-Ph | H | CH₂Cl | O | H | Bindung | c-Pr | |
| 10.072 | 3,5-Cl₂-Ph | H | Vinyl | O | H | Bindung | c-Pr | [CDCl₃] 0.54 (m,2H); 0.80 |
| | | | | | | | | (m,2H); 2.73 (m,1H); 3.29 (d,1H); 3.94 (d,1H); 5.32 (d,1H); 5.51 (d,1H); 6.15 (dd,1H); 7.00 (s br,1H); 7.42 (s,1H); 7.52 (s,2H). |
| 10.073 | 3-F-Ph | H | CH₂F | O | H | CH₂ | 1-c-Pr-1H-pyrazol-4-yl | [CDCl₃] 0.98 (m,2H); 1.07 (m,2H); 3.47 (d,1H); 3.55 (m,1H); 3.71 (d,1H); 4.24 (dd,1H); 4.38 (dd,1H); 4.64 (d,0.5H); 4.74 (d,0.5H); 4.75 (d,0.5H); 4.87 (d,0.5H); 7.07 (s br,1H); 7.16 (m,1H); 7.40 (m,3H). |
| 10.074 | 3,5-F₂-Ph | H | CH₂F | O | H | CH₂ | CF₃ | [CDCl₃] 3.48 (d,1H); 3.68 (d,1H); 3.83 - 4.06 (m,2H); 4.63 (d,0.5H); 4.75 (d,1H); 4.86 (d,0.5H); 6.91 (m,1H); 7.18 (m,3H). |
| 10.075 | 3,5-Cl₂-Ph | H | Acetyl | O | H | CH₂ | CF₃ | |
| 10.076 | 3,5-F₂-Ph | H | Acetyl | O | H | CH₂ | CF₃ | |
| 10.077 | 3-F-Ph | H | CH₂F | O | H | CH₂ | CF₃ | |
| 10.078 | 3,5-F₂-Ph | H | CH₂Cl | O | H | CH₂ | CF₃ | |
| 10.079 | 3,5-Cl₂-Ph | H | CH₂Cl | O | H | CH₂ | CF₃ | |
| 10.080 | 3-F-Ph | H | CH₂Cl | O | H | CH₂ | CF₃ | |
| 10.081 | 3,5-F₂-Ph | H | CH₂F | O | H | CH(CH₃) | CH₃ | [CDCl₃] 1.16 (d,3H); 1.20 (d,3H); 3.44 (d,1H); 3.67 (d,1H); 4.05 (m,1H); 4.62 (d,0.5H); 4.73 (m,2H); 4.83 (d,0.5H); 6.69 (d br,1H); 6.92 (m,1H); 7.17 (m,2H). |
| 10.082 | 3-F-Ph | H | CH₂F | O | H | CH(CH₃) | CH₃ | |
| 10.083 | 3,5-F₂-Ph | H | CH₂Cl | O | H | CH(CH₃) | CH₃ | [CDCl₃] 1.17 (d,3H); 1.22 (d,3H); 3.53 (d,1H); 3.69 (d,1H); 3.93 (d,1H); 4.02 (d,1H); 4.07 (m,1H); 6.70 (d br, 1 H); 6.91 (m,1H); 7.18 (m,2H). |
| 10.084 | 3,5-Cl₂-Ph | H | CH₂Cl | O | H | CH(CH₃) | CH₃ | [CDCl₃] 1.70 (dd,6H); 3.52 (d,1H); 3.71 (d,1H); 3.98 (AB,2H);4.06 (m,1H); 6.70 (d br,1H); 6.90 (m,1H); 7.16 (m,2H). |
| 10.085 | 3-F-Ph | H | CH₂Cl | O | H | CH(CH₃) | CH₃ | |
| 10.086 | 3,5-F₂-Ph | H | CH₂F | O | H | CH(CH₃) | Cyan | |
| 10.087 | 3-F-Ph | H | CH₂F | O | H | CH₂CH₂ | COOH | |
| 10.088 | 3,5-Cl₂-Ph | H | CH(CH₃)OH | O | H | CH₂CH₂ | COOH | |
| 10.089 | 3,5-F₂-Ph | H | CH(CH₃)OH | O | H | CH₂CH₂ | COOH | |
| 10.090 | 3,5-Cl₂-Ph | H | CH(CH₃)OH | O | H | CH₂CH₂ | Ethoxycarbonyl | |
| 10.091 | 3,5-F₂-Ph | H | CH(CH₃)OH | O | H | CH₂CH₂ | Ethoxycarbonyl | |
| 10.092 | 3,5-F₂-Ph | H | F | O | H | CH₂CH₂ | Methoxycarbonyl | [CDCl₃] 2,64 (m,2H); 3.56 (dd,1H); 3.67 (m,2H); 3.74 (s,3H); 4.18 (dd,1H); 6.94 (m,1H); 7.21 (m,3H). |
| 10.093 | 3,5-Cl₂-Ph | H | F | O | H | CH₂CH₂ | Methoxycarbonyl | [CDCl₃] 2,63 (m,2H); 3.56 (dd,1H); 3.67 (m,2H); 3.74 (s,3H); 4.18 (dd,1H); 7.17 (s br,1H); 7.47 (s,1H); 7.56 (s,2H). |
| 10.094 | 3,5-F₂-Ph | H | CH₂F | O | H | CH₂CH₂ | Methoxycarbonyl | [CDCl₃] 2.56 (t,2H); 3.44 (d,1H); 3.52 (m,1H); 6.62 (m,1H); 3.65 (d,1H); 4.62 (d,0.5H), 4.73 (m,1H); 4.84 (d,0.5H); 6.90 (m,1H); 7.18 (m,2H); 7.35 (s |
| | | | | | | | | br,1H). |
| 10.095 | 3,5-Cl₂-Ph | H | Acetyl | O | H | CH₂CH₂ | Methoxycarbonyl | [CDCl₃] 2.33 (s,3H); 2.59 (m,2H); 3.51 (d,1H); 3.56 (m,1H); 3.64 (m,1H); 3.71 (s,3H); 4.14 (d,1H); 7.41 (t br,1H); 7.44 (s,1H); 7.54 (s,2H). |
| 10.096 | 3,5-F₂-Ph | H | Acetyl | O | H | CH₂CH₂ | Methoxycarbonyl | |
| 10.097 | 3-F-Ph | H | CH₂F | O | H | CH₂CH₂ | Methoxycarbonyl | |
| 10.098 | 3,5-F₂-Ph | H | CH₂Cl | O | H | CH₂CH₂ | Methoxycarbonyl | [CDCl₃] 2.57 (m,2H); 3.52 (m,1H); 3,53 (d,1H); 3.63 (m,1H); 3.68 (d,1H); 3.69 (s,3H); 3.97 (AB,2H); 6.89 (m,1H); 7.18 (m,2H); 7.33 (t br,1H). |
| 10.099 | 3,5-Cl₂-Ph | H | CH₂Cl | O | H | CH₂CH₂ | Methoxycarbonyl | [CDCl₃] 2.57 (m,2H); 3.50 (m,1H); 3.53 (d,1H); 3.62 (m,1H); 3.68 (d,1H); 3.71 (s,3H); 3.92 (d,1H); 4.01 (d,1H); 7.33 (s br,1H); 7.43 (s,1H); 7.53 (s,2H). |
| 10.100 | 3,5-Cl₂-Ph | H | Prop-1-en-2-yl | O | H | CH₂CH₂ | Methoxycarbonyl | [CDCl₃] 1.86 (s,3H); 2.55 (m,2H); 3.32 (d,1H); 3.52 (m,1H); 3.58 (m,1H); 3.69 (s,3H); 3.97 (d,1H); 5.07 (s,1H); 5.26 (s,1H); 7.15 (t br,1H); 7.40 (s,1H); 7.55 (s,2H). |
| 10.101 | 3,5-F₂-Ph | H | Prop-1-en-2-yl | O | H | CH₂CH₂ | Methoxycarbonyl | [CDCl₃] 1.86 (s,3H); 2.55 (m,2H); 3.32 (d,1H); 3.52 (m,1H); 3.58 (m,1H); 3.79 |
| | | | | | | | | (s,3H); 3.98 (d,1H); 5.09 (s,1H); 5.26 (s,1H); 6.87 (m,1H); 7.18 (m,3H). |
| 10.102 | 3,5-F₂-Ph | H | Vinyl | O | H | CH₂CH₂ | Methoxycarbonyl | [CDCl₃] 2.55 (t,3H); 3.31 (d,1H); 3.56 (m,2H); 3.71 (s,3H); 3.92 (d,1H); 5.34 (d,1H); 5.54 (d,1H); 6.15 (dd,1H); 6.88 (m,1H); 7.17 (m,2H); 7.2 (d br,1H). |
| 10.103 | 3-F-Ph | H | CH₂Cl | O | H | CH₂CH₂ | Methoxycarbonyl | |
| 10.104 | 3,5-Cl₂-Ph | H | Vinyl | O | H | CH₂CH₂ | Methoxycarbonyl | [CDCl₃] 2.55 (t,3H); 3.20 (d,1H); 3.55 (m,2H); 3.69 (s,3H); 3.90 (d,1H); 5.30 (d,1H); 5.52 (d,1H); 6.15 (dd,1H); 7.20 (s br,1H); 7.41 (s,1H); 7.52 (s,1H). |
| 10.105 | 3-F-Ph | H | CH₂F | O | H | CH(CH₃)CH₂ | COOH | |
| 10.106 | 3,5-F₂-Ph | H | CH₂F | O | H | CH(CH₃)CH₂ | Ethoxycarbonyl | |
| 10.107 | 3-F-Ph | H | CH₂F | O | H | CH(CH₃)CH₂ | Ethoxycarbonyl | |
| 10.108 | 3,5-F₂-Ph | H | CH₂Cl | O | H | CH(CH₃)CH₂ | Ethoxycarbonyl | [CDCl₃] D1 plus D2 1.17-1.32 (m,5H); 2.48 (AB,0.8H); 2.56 (d,1.2H); 3.60 (m,2H); 3.97 (AB,2H); 4.08 (q,0.8H); 4.15 (q,1.2H); 4.33 (m,1H); 6.88 (m,1H); 7.18 (m,2H); 7.26 (s br,1 H). |
| 10.109 | 3,5-Cl₂-Ph | H | CH₂Cl | O | H | CH(CH₃)CH₂ | Ethoxycarbonyl | [CDCl₃] D1 [CDCl₃] 1.23 (t,3H); 1.28 (t,3H); 1.29 (d,3H); 2.49 (m,2H); 3.51 (d,2H); 3.69 (d,1H); 3.93 (dd,1H); 4.04 |
| | | | | | | | | (dd,1H); 4.10 (q,2H); 4.33 (m,1H); 7.26 (s br,1H); 7.43 (s, 1H); 7.53 (s,2H). D2 [CDCl₃] 1.23 (t,3H); 1.28 (t,3H); 1.29 (d,3H); 2.56 (d,2H); 3.52 (d,2H); 3.70 (d,1H); 3.93 (dd,1H); 4.04 (dd,1H); 4.18 (q,2H); 4.33 (m,1H); 7.26 (s br,1H); 7.43 (s,1H); 7.53 (s,2H). |
| 10.110 | 3-F-Ph | H | CH₂Cl | O | H | CH(CH₃)CH₂ | Ethoxycarbonyl | |
| 10.111 | 3,5-F₂-Ph | H | F | O | H | CH(CH₃)CH₂ | Methoxycarbonyl | [CDCl₃] 1.32 (d,3H); 2.61 (m,2H); 3.56 (dd,1H); 3.73 (s,3H); 4.18 (dd,1H); 4.43 (m,1H); 6.93 (m,1H); 7.24 (m,3H). |
| 10.112 | 3-F-Ph | H | CH₂F | O | H | CH₂CH₂CH₂ | COOH | |
| 10.113 | 3,5-F₂-Ph | H | F | O | H | CH₂CH₂CH₂ | Ethoxycarbonyl | [CDCl₃] 1.27 (t,3H); 1.95 (pent, 2H); 2,42 (t,2H); 3.44 (q,2H); 3.55 (dd,1H); 4.17 (q,2H); 4.20 (dd,1H); 6.86 (s br,1H); 6.93 (m,1H); 7.20 (m,2H). |
| 10.114 | 3,5-Cl₂-Ph | H | F | O | H | CH₂CH₂CH₂ | Ethoxycarbonyl | [CDCl₃] 1.27 (t,3H); 1.94 (pent,2H); 3.41 (t,2H); 3.45 (q,2H); 3.56 (dd,1H); 4.16 (q,2H); 4.20 (dd,1H); 6.88 (s br,1H); 7,47 (s,1H); 7.57 s,2H). |
| 10.115 | 3,5-F₂-Ph | H | CH₂F | O | H | CH₂CH₂CH₂ | Methoxycarbonyl | [CDCl₃] 1.88 (pent, 2H); 2,35 (t,2H); 3.28 (m,1H); 3.36 (m,1H); 3.43 (d,1 H); 3.65 (d,1H); 3.67 |
| | | | | | | | | (s,3H); 4.62 (d,0.5H); 4.74 (m,1H); 4.85 (d,0.5H); 6.90 (m,1H); 7.04 (s br,1H); 7.18 (m,2H). |
| 10.116 | 3-F-Ph | H | CH₂F | O | H | CH₂CH₂CH₂ | Methoxycarbonyl | |

| Tabelle 11: Erfindungsgemäße Verbindungen der allgemeinen Formel (I), worin R² für Wasserstoff steht, R⁴-N-A-X einen Ring bilden, und Aryl den Rest 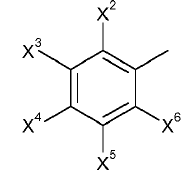 bedeutet. | | | | | | |
|---|---|---|---|---|---|---|
| 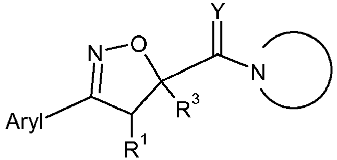 | | | | | | |

| Nr. | Aryl | R¹ | R³ | Y | R⁴-N-A-X | Daten |
|---|---|---|---|---|---|---|
| 11.001 | 3,5-Cl₂-Ph | H | CF₃ | O | 1,2-Oxazolidin-2-yl | |
| 11.002 | 3,5-Cl₂-Ph | H | CF₃ | O | 1,3-Thiazolidin-3-yl | |
| 11.003 | 3,5-Cl₂-Ph | H | CF₃ | O | 2-(Ethoxycarbonyl)piperidin-1-yl | |
| 11.004 | 3,5-Cl₂-Ph | H | CF₃ | O | 2-(Methoxycarbonyl)pyrrolidin-1-yl | |
| 11.005 | 3,5-Cl₂-Ph | H | CF₃ | O | 3-(Ethoxycarbonyl)piperidin-1-yl | |
| 11.006 | 3,5-Cl₂-Ph | H | CF₃ | O | 4-(Ethoxycarbonyl)piperidin-1-yl | |
| 11.007 | 3,5-Cl₂-Ph | H | CF₃ | O | 4-(THF-2-ylcarbonyl)piperazin-1-yl | |
| 11.008 | 3,5-Cl₂-Ph | H | CF₃ | O | 4-Acetylpiperazin-1-yl | |
| 11.009 | 3,5-Cl₂-Ph | H | CF₃ | O | Decahydronaphthalen-1-ylamino | |
| 11.010 | 3,5-Cl₂-Ph | H | CF₃ | O | Morpholin-4-yl | |
| 11.011 | 3,5-Cl₂-Ph | H | CF₃ | O | Pyrrolidin-1-yl | |
| 11.012 | 3-F-Ph | H | CF₃ | O | Pyrrolidin-1-yl | |
| 11.013 | 3-OH-Ph | H | CF₃ | O | Pyrrolidin-1-yl | |
| 11.014 | 3-iPrO-Ph | H | CF₃ | O | Pyrrolidin-1-yl | |
| 11.015 | 3-(2-MeOEtO)-Ph | H | CF₃ | O | Pyrrolidin-1-yl | |
| 11.016 | 3-EtO-Ph | H | CF₃ | O | Pyrrolidin-1-yl | |
| 11.017 | 3-CF₃O-Ph | H | CF₃ | O | Pyrrolidin-1-yl | |
| 11.018 | 3,5-F₂-Ph | H | CF₃ | O | Pyrrolidin-1-yl | |
| 11.019 | 3-Cl-5-Me-Ph | H | CF₃ | O | Pyrrolidin-1-yl | |
| 11.020 | 3,5-Cl₂-Ph | H | C₂H₅ | O | 1,2-Oxazolidin-2-yl | |
| 11.021 | 3,5-Cl₂-Ph | H | C₂H₅ | O | 1,3-Thiazolidin-3-yl | |
| 11.022 | 3,5-Cl₂-Ph | H | C₂H₅ | O | 2-(Ethoxycarbonyl)piperidin-1-yl | |
| 11.023 | 3,5-Cl₂-Ph | H | C₂H₅ | O | 2-(Methoxycarbonyl)pyrrolidin-1-yl | |
| 11.024 | 3,5-Cl₂-Ph | H | C₂H₅ | O | 3-(Ethoxycarbonyl)piperidin-1-yl | |
| 11.025 | 3,5-Cl₂-Ph | H | C₂H₅ | O | 4-(Ethoxycarbonyl)piperidin-1-yl | |
| 11.026 | 3,5-Cl₂-Ph | H | C₂H₅ | O | 4-(THF-2-ylcarbonyl)piperazin-1-yl | |
| 11.027 | 3,5-Cl₂-Ph | H | C₂H₅ | O | 4-Acetylpiperazin-1-yl | |
| 11.028 | 3,5-Cl₂-Ph | H | C₂H₅ | O | Decahydronaphthalen-1-ylamino | |
| 11.029 | 3,5-Cl₂-Ph | H | C₂H₅ | O | Morpholin-4-yl | |
| 11.030 | 3,5-Cl₂-Ph | H | C₂H₅ | O | Pyrrolidin-1-yl | |
| 11.031 | 3-F-Ph | H | C₂H₅ | O | Pyrrolidin-1-yl | |
| 11.032 | 3-OH-Ph | H | C₂H₅ | O | Pyrrolidin-1-yl | |
| 11.033 | 3-iPrO-Ph | H | C₂H₅ | O | Pyrrolidin-1-yl | |
| 11.034 | 3-(2-MeOEtO)-Ph | H | C₂H₅ | O | Pyrrolidin-1-yl | |
| 11.035 | 3-EtO-Ph | H | C₂H₅ | O | Pyrrolidin-1-yl | |
| 11.036 | 3-CF₃O-Ph | H | C₂H₅ | O | Pyrrolidin-1-yl | |
| 11.037 | 3,5-F₂-Ph | H | C₂H₅ | O | Pyrrolidin-1-yl | |
| 11.038 | 3-Cl-5-Me-Ph | H | C₂H₅ | O | Pyrrolidin-1-yl | |
| 11.039 | 3,5-Cl₂-Ph | H | CH₃ | O | 1,2-Oxazolidin-2-yl | |
| 11.040 | 3,5-Cl₂-Ph | H | CH₃ | O | 1,3-Thiazolidin-3-yl | |
| 11.041 | 3,5-Cl₂-Ph | H | CH₃ | O | 2-(Ethoxycarbonyl)piperidin-1-yl | |
| 11.042 | 3,5-Cl₂-Ph | H | CH₃ | O | 2-(Methoxycarbonyl)pyrrolidin-1-yl | |
| 11.043 | 3,5-Cl₂-Ph | H | CH₃ | O | 3-(Ethoxycarbonyl)piperidin-1-yl | |
| 11.044 | 3,5-Cl₂-Ph | H | CH₃ | O | 4-(Ethoxycarbonyl)piperidin-1-yl | |
| 11.045 | 3,5-Cl₂-Ph | H | CH₃ | O | 4-(THF-2-ylcarbonyl)piperazin-1-yl | |
| 11.046 | 3,5-Cl₂-Ph | H | CH₃ | O | 4-Acetylpiperazin-1-yl | |
| 11.047 | 3,5-Cl₂-Ph | H | CH₃ | O | Decahydronaphthalen-1-ylamino | |
| 11.048 | 3,5-Cl₂-Ph | H | CH₃ | O | Morpholin-4-yl | |
| 11.049 | 3,5-Cl₂-Ph | H | CH₃ | O | Pyrrolidin-1-yl | |
| 11.050 | 3-F-Ph | H | CH₃ | O | Pyrrolidin-1-yl | |
| 11.051 | 3-OH-Ph | H | CH₃ | O | Pyrrolidin-1-yl | |
| 11.052 | 3-iPrO-Ph | H | CH₃ | O | Pyrrolidin-1-yl | |
| 11.053 | 3-(2-MeOEtO)-Ph | H | CH₃ | O | Pyrrolidin-1-yl | |
| 11.054 | 3-EtO-Ph | H | CH₃ | O | Pyrrolidin-1-yl | |
| 11.055 | 3-CF₃O-Ph | H | CH₃ | O | Pyrrolidin-1-yl | |
| 11.056 | 3,5-F₂-Ph | H | CH₃ | O | Pyrrolidin-1-yl | [CDCl₃] 7.27 |
| | | | | | | 11.63;7.26 |
| | | | | | | 11.98;7.20 |
| | | | | | | 1.52;7.20 |
| | | | | | | 2.03;7.20 |
| | | | | | | 2.41;7.19 |
| | | | | | | 2.18;7.18 |
| | | | | | | 2.11;7.18 |
| | | | | | | 2.44;7.18 |
| | | | | | | 1.96;7.17 |
| | | | | | | 1.52;7.17 |
| | | | | | | 0.34;6.89 |
| | | | | | | 0.42;6.88 |
| | | | | | | 0.66;6.88 |
| | | | | | | 0.68;6.88 |
| | | | | | | 0.38;6.87 |
| | | | | | | 0.35;6.87 |
| | | | | | | 0.75;6.86 |
| | | | | | | 0.84;6.86 |
| | | | | | | 1.33;6.86 |
| | | | | | | 1.35;6.85 |
| | | | | | | 0.72;6.85 |
| | | | | | | 0.67;6.84 |
| | | | | | | 0.39;6.84 |
| | | | | | | 0.43;6.84 |
| | | | | | | 0.68;6.84 |
| | | | | | | 0.68;6.83 |
| | | | | | | 0.36;6.83 |
| | | | | | | 0.33;5.30 |
| | | | | | | 0.92;5.30 |
| | | | | | | 0.97;4.25 |
| | | | | | | 2.58;4.25 |
| | | | | | | 2.60;4.21 |
| | | | | | | 2.74;4.21 |
| | | | | | | 2.79;3.90 |
| | | | | | | 0.34;3.88 |
| | | | | | | 0.78;3.87 |
| | | | | | | 0.63;3.86 |
| | | | | | | 0.55;3.85 |
| | | | | | | 1.32;3.83 |
| | | | | | | 0.71;3.78 |
| | | | | | | 0.52;3.77 |
| | | | | | | 0.86;3.76 |
| | | | | | | 0.80;3.75 |
| | | | | | | 1.02;3.74 |
| | | | | | | 0.57;3.72 |
| | | | | | | 0.44;3.53 |
| | | | | | | 1.14;3.53 |
| | | | | | | 1.20;3.51 |
| | | | | | | 2.65;3.51 |
| | | | | | | 1.40;3.49 |
| | | | | | | 1.54;3.12 |
| | | | | | | 2.70;3.12 |
| | | | | | | 2.71;3.07 |
| | | | | | | 2.51;3.07 |
| | | | | | | 2.55;2.02 |
| | | | | | | 0.58;2.01 |
| | | | | | | 0.81;1.99 |
| | | | | | | 1.03;1.97 |
| | | | | | | 0.69;1.96 |
| | | | | | | 0.70;1.94 |
| | | | | | | 0.81;1.92 |
| | | | | | | 0.83;1.91 |
| | | | | | | 0.46;1.90 |
| | | | | | | 0.76;1.89 |
| | | | | | | 0.78;1.87 |
| | | | | | | 1.11;1.86 |
| | | | | | | 1.24;1.84 |
| | | | | | | 0.99;1.82 |
| | | | | | | 0.41;1.82 |
| | | | | | | 0.96;1.81 |
| | | | | | | 0.39;1.80 |
| | | | | | | 0.67;1.79 |
| | | | | | | 0.43;1.71 |
| | | | | | | 15.61;1.71 |
| | | | | | | 16.00;1.60 |
| | | | | | | 4.26;1.59 |
| | | | | | | 4.34;0.00 5.42 |
| 11.057 | 3-Cl-5-Me-Ph | H | CH₃ | O | Pyrrolidin-1-yl | |
| 11.058 | 3,5-Cl₂-Ph | H | CN | O | 1,2-Oxazolidin-2-yl | |
| 11.059 | 3,5-Cl₂-Ph | H | CN | O | 1,3-Thiazolidin-3-yl | |
| 11.060 | 3,5-Cl₂-Ph | H | CN | O | 2-(Ethoxycarbonyl)piperidin-1-yl | |
| 11.061 | 3,5-Cl₂-Ph | H | CN | O | 2-(Methoxycarbonyl)pyrrolidin-1-yl | |
| 11.062 | 3,5-Cl₂-Ph | H | CN | O | 3-(Ethoxycarbonyl)piperidin-1-yl | |
| 11.063 | 3,5-Cl₂-Ph | H | CN | O | 4-(Ethoxycarbonyl)piperidin-1-yl | |
| 11.064 | 3,5-Cl₂-Ph | H | CN | O | 4-(THF-2-ylcarbonyl)piperazin-1-yl | |
| 11.065 | 3,5-Cl₂-Ph | H | CN | O | 4-Acetylpiperazin-1-yl | |
| 11.066 | 3,5-Cl₂-Ph | H | CN | O | Decahydronaphthalen-1-ylamino | |
| 11.067 | 3,5-Cl₂-Ph | H | CN | O | Morpholin-4-yl | |
| 11.068 | 3,5-Cl₂-Ph | H | CN | O | Pyrrolidin-1-yl | |
| 11.069 | 3-F-Ph | H | CN | O | Pyrrolidin-1-yl | |
| 11.070 | 3-OH-Ph | H | CN | O | Pyrrolidin-1-yl | |
| 11.071 | 3-iPrO-Ph | H | CN | O | Pyrrolidin-1-yl | |
| 11.072 | 3-(2-MeOEtO)-Ph | H | CN | O | Pyrrolidin-1-yl | |
| 11.073 | 3-EtO-Ph | H | CN | O | Pyrrolidin-1-yl | |
| 11.074 | 3-CF₃O-Ph | H | CN | O | Pyrrolidin-1-yl | |
| 11.075 | 3,5-F₂-Ph | H | CN | O | Pyrrolidin-1-yl | |
| 11.076 | 3-Cl-5-Me-Ph | H | CN | O | Pyrrolidin-1-yl | |
| 11.077 | 3,5-F₂-Ph | H | CH₃ | O | 2-Oxopyrrolidin-1-yl | |
| 11.078 | 3,5-F₂-Ph | H | CH₃ | O | 3-(Ethoxycarbonyl)piperidin-1-yl | |
| 11.079 | 3,5-F₂-Ph | H | CH₃ | O | 4-(Ethoxycarbonyl)piperidin-1-yl | |
| 11.080 | 3,5-F₂-Ph | H | CH₃ | O | 4,4-Difluorpiperidin-1-yl | |
| 11.081 | 3,5-F₂-Ph | H | CH₃ | O | 4-Methylpiperazin-1-yl | |
| 11.082 | 3,5-F₂-Ph | H | CH₃ | O | Morpholin-4-yl | |

Die verwendeten Abkürzungen bedeuten:

| | | | | | | | |
|---|---|---|---|---|---|---|---|
| Ac | Acetoxy | Bu | Butyl | Et | Ethyl | Me | Methyl |
| Pr | Propyl | Pen | Pentyl | Hex | Hexyl | Ph | Phenyl |
| c | cyclo | s | sekundär | i | iso | t | tertiär |
| THF | Tetrahydrofuran | | | | | | |

R001 steht für den Rest 3-Chlor-5-{[1-methyl-3-(trifluormethyl)-1 H-pyrazol-5-yl]oxy}phenyl.
R002 steht für den Rest 3-[(2-Brom-1-fluorvinyl)oxy]-5-chlorphenyl.
R003 steht für den Rest 3-[(2-Brom-1-fluorvinyl)oxy]phenyl.

Mit E1, E2, E3, E4 werden enantiomerenreine Verbindungen bezeichnet. D1, D2 bezeichnen Diastereomere eines Diastereomerenpaares, die als Racemate zweier Enantiomere vorliegen.

### B. Formulierungsbeispiele

### 1. Stäubemittel

Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der Formel (I),
10 " ligninsulfonsaures Calcium,
5 " Natriumlaurylsulfat,
3 " Polyvinylalkohol und
7 " Kaolin
mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man
25 Gew.-Teile einer Verbindung der Formel (I),
5 " 2,2'-dinaphthylmethan-6,6'-disulfonsaures Natrium,
2 " oleoylmethyltaurinsaures Natrium,
1 " Polyvinylalkohol,
17 " Calciumcarbonat und
50 " Wasser
auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha in unterschiedlichen Dosierungen auf die Oberfläche der Abdeckerde appliziert. Anschließend werden die Töpfe zur weiteren Kultivierung der Pflanzen im Gewächshaus unter optimalen Bedingungen gehalten. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. 1.130, 1.175, 1.183, 1.189, 1.204, 1.250, 1.373, 1.421, 1.676, 2.189, 4.074, 6.058, 6.115, 6.163, 6.321, 6.431, 6.470, 6.504, 6.248, 6.288, 6.666, 6.669 und 6.687 bei einer Aufwandmenge von 320 Gramm pro Hektar jeweils eine mindestens 90%-ige Wirkung gegen Echinochloa crus galli, Lolium multiflorum und Setaria viridis. Die Verbindungen der Nr. 1.140, 1.270, 6.202, 6.122, 6.357, 6.372, 6.433, 6.485, 6.617 und 8.359 zeigen bei einer Aufwandmenge von 320 Gramm pro Hektar jeweils eine mindestens 90%-ige Wirkung gegen Alopecurus myosuroides, Lolium multiflorum und Veronica persica. Die Verbindungen der Nr. 1.139, 1.201, 1.262, 1.394, 6.082, 6.195, 6.351 und 9.581 zeigen bei einer Aufwandmenge von 320 Gramm pro Hektar jeweils eine mindestens 90%-ige Wirkung gegen Fallopia convolvulus und Stellaria media.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. 1.111, 1.252, 1.903, 2.189, 6.485, 6.504, 6.510, 6.559 und 6.599 bei einer Aufwandmenge von 320 Gramm pro Hektar jeweils eine mindestens 90%-ige Wirkung gegen Lolium multiflorum, Stellaria media und Veronica persica. Die Verbindungen der Nr. 6.248, 6.279, 6.420, 6.504, 6.491, 6.616 und 6.617 zeigen bei einer Aufwandmenge von 320 Gramm pro Hektar jeweils eine mindestens 90%-ige Wirkung gegen Alopecurus myosuroides und Avena fatua. Die Verbindungen der Nr. 1.373, 1.183, 1.250, 1.344, 1.338, 1.676, 6.305, 6.541, 6.556 und 6.608 zeigen bei einer Aufwandmenge von 320 Gramm pro Hektar jeweils eine mindestens 90%-ige Wirkung gegen Echinochloa crus galli und Veronica persica.

## Patentansprüche

1. 3-Phenylisoxazolin-5-carboxamide und 3-Phenylisoxazolin-5-thioamide der Formel (I), oder deren Salze 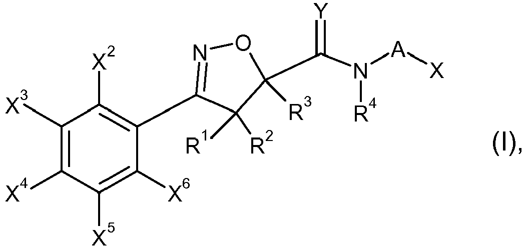 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 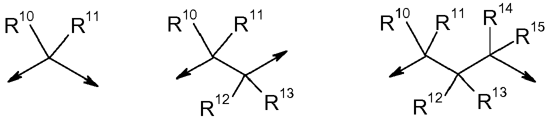 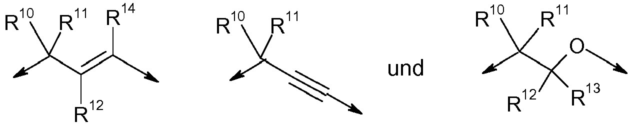 R¹⁰, R¹¹, R¹², R¹³, R¹⁴ und R¹⁵ bedeuten unabhängig voneinanderjeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen fünf- oder sechsgliedrigen gesättigten, teilweise ungesättigten, vollständig ungesättigten oder aromatischen Ring, der aus r Kohlenstoffatomen, s Stickstoffatomen, n Schwefelatomen und n Sauerstoffatomen aufgebaut ist, und der durch n Reste aus der Gruppe bestehend aus R⁶, R^{6a} , R⁸ und R⁹ substituiert ist;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO²R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy,
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₃-C₆)-Alkenyloxy oder (C₃-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
r bedeutet 1, 2, 3, 4 oder 5;
s bedeutet 0, 1, 2, 3 oder 4;
mit der Maßgabe, daß X³ und X⁴ nicht gleichzeitig substituiertes oder unsubstituiertes Alkoxy bedeuten.

2. 3-Phenylisoxazolin-5-carboxamide und 3-Phenylisoxazolin-5-thioamide der Formel (I), oder deren Salze 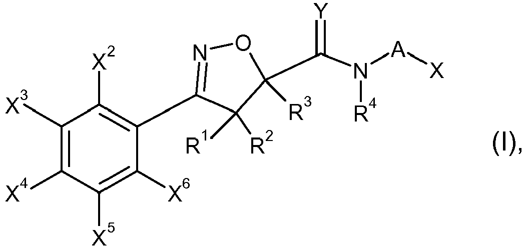 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 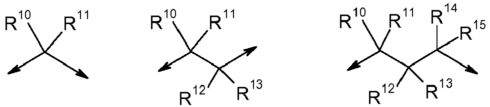 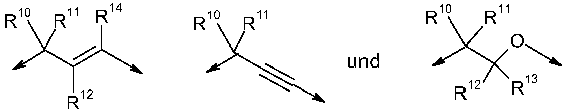 R¹⁰, R¹¹, R¹², R¹³, R¹⁴ und R¹⁵ bedeuten unabhängig voneinanderjeweils Wasserstoff, Fluor, Chlor, Brom, Iod, Hydroxy, Cyano, CO₂R⁸, CONR⁶R⁸, R⁵,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷,X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch n Reste aus der Gruppe bestehend aus R⁶, R^{6a} , R⁸ und R⁹ substituierten Ring aus der Gruppe bestehend aus 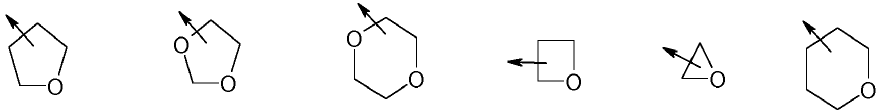 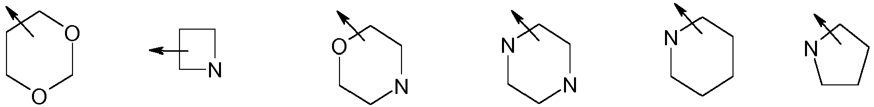 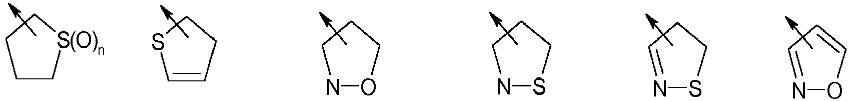 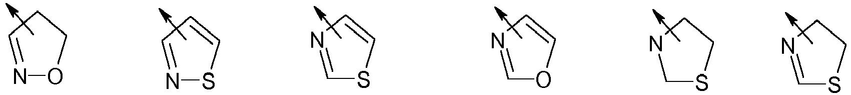 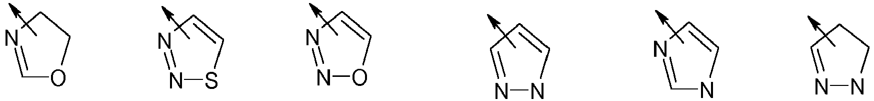 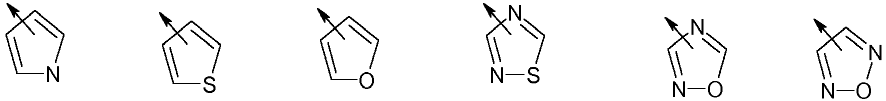 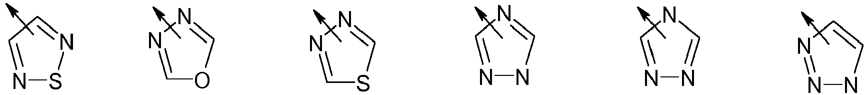 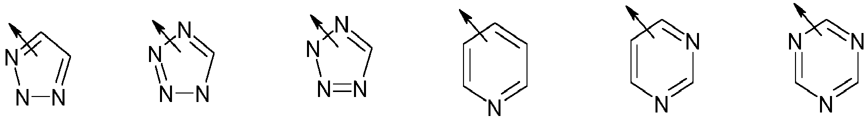 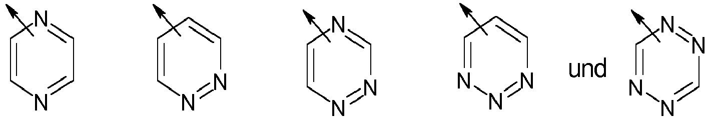 X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO2R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy,
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl,
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
mit der Maßgabe, daß X³ und X⁴ nicht gleichzeitig substituiertes oder unsubstituiertes Alkoxy bedeuten.

3. 3-Phenylisoxazolin-5-carboxamide und 3-Phenylisoxazolin-5-thioamide nach Anspruch 1 oder 2, worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl,
R³ bedeutet Fluor, Chlor, oder Cyano,
oder durch jeweils m Reste aus der Gruppe bestehend aus Fluor und Chlor substituiertes (C₁-C₆)-Alkyl, (C₃-C₆)-Cycloalkyl, (C₂-C₆)-Alkenyl oder (C₂-C₆)-Alkinyl, oder durch m Reste aus der Gruppe bestehend aus Fluor und Chlor substituiertes (C₁-C₆)-Alkylcarbonyl;
A bedeutet eine Bindung oder eine divalente Einheit aus der Gruppe bestehend aus CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C=C, CH(CF₃)CH₂, CH(CH₃)CH₂O, CH₂CH₂O, CH(cPr)CH₂O, CH(CH₂OCH₃),CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ und CH₂CH₂CONHCH₂;
R⁴ bedeutet Wasserstoff oder (C₁-C₈)-Alkyl,
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Cyano, Hydroxy, OR⁷, X¹, OX¹, NHX¹, S(O)ₙ R⁵, CO₂R⁸, CONR⁶R⁸, CONR⁸SO₂R⁵ und POR⁹R⁹ substituiertes (C₁-C₁₂)-Alkyl, (C₃-C₈)-Cycloalkyl, (C₂-C₁₂)-Alkenyl oder (C₂-C₁₂)-Alkinyl;
X¹ bedeutet einen durch n Reste aus der Gruppe bestehend aus R⁶, R^{6a} , R⁸ und R⁹ substituierten Ring aus der Gruppe bestehend aus 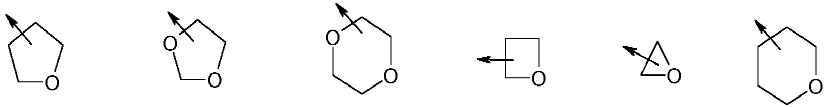 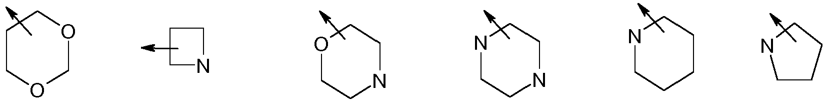 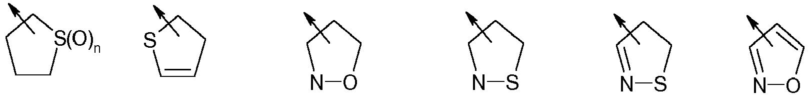 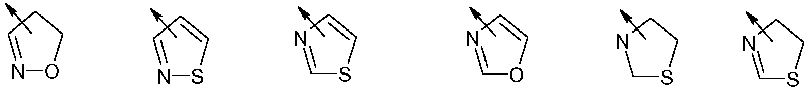 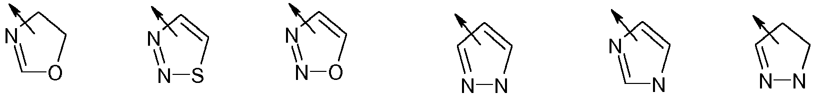 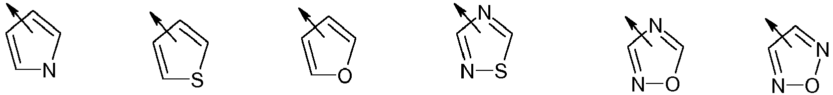 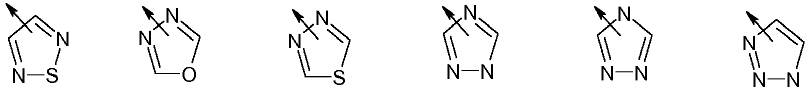 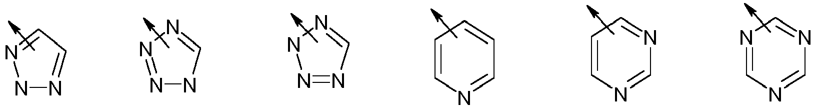 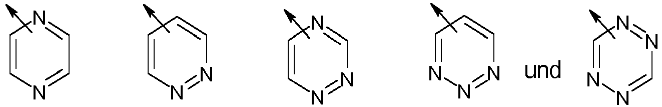 X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy;
X³ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
X⁵ bedeutet Wasserstoff oder X³;
R⁵ bedeutet Methyl oder Ethyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor und Chlor substituiertes (C₁-C₆)-Alkyl,
R⁸ bedeutet R⁷,
R⁹ bedeutet (C₁-C₃)-Alkoxy,
m bedeutet 0, 1, 2 oder 3;
n bedeutet 0, 1 oder 2;
mit der Maßgabe, daß X³ und X⁴ nicht gleichzeitig substituiertes oder unsubstituiertes Alkoxy bedeuten.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksame Stoffen aus der Gruppe Insektizide, Akarizide, Herbizide, Fungizide, Safenern und Wachstumsregulatoren.

7. Herbizide Mittel nach Anspruch 6 enthaltend einen Safener.

8. Herbizide Mittel nach Anspruch 7, worin der Safener ausgewählt ist aus der Gruppe bestehend aus Mefenpyr-diethyl, Cyprosulfamid, Isoxadifen-ethyl, Cloquintocet-mexyl, Benoxacor und Dichlormid.

9. Herbizide Mittel nach einem der Ansprüche Anspruch 6 bis 8 enthaltend ein weiteres Herbizid.

10. Verwendung von 3-Phenylisoxazolin-5-carboxamiden und 3-Phenylisoxazolin-5-thioamiden der Formel (Ia) 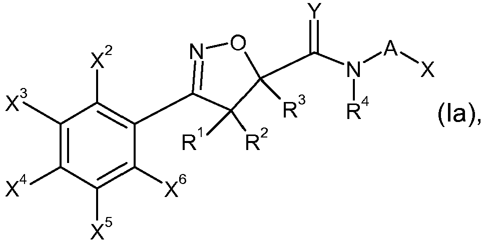 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano, Hydroxy,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 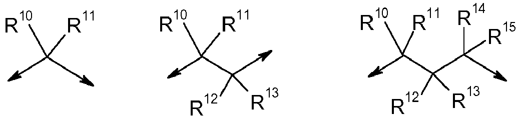 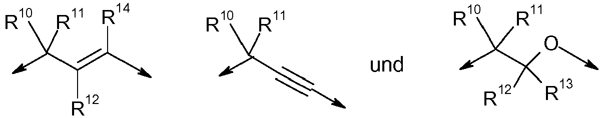 R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR₆SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen fünf- oder sechsgliedrigen gesättigten, teilweise ungesättigten, vollständig ungesättigten oder aromatischen Ring, der aus r Kohlenstoffatomen, s Stickstoffatomen, n Schwefelatomen und n Sauerstoffatomen aufgebaut ist, und der durch n Reste aus der Gruppe bestehend aus R⁶, R^{6a} , R⁸ und R⁹ substituiert ist, oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
r bedeutet 1, 2, 3, 4 oder 5;
s bedeutet 0, 1, 2, 3 oder 4
zur Bekämpfung unerwünschter Pflanzen.

11. Verwendung von 3-Phenylisoxazolin-5-carboxamiden und 3-Phenylisoxazolin-5-thioamiden der Formel (Ia) 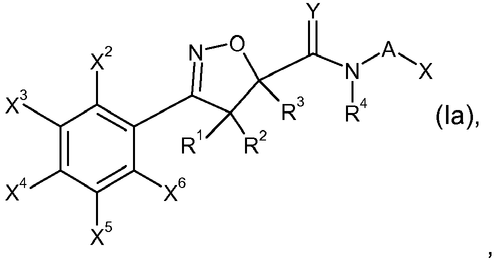 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙ R⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁴ bedeutet Wasserstoff, Cyano, Hydroxy,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 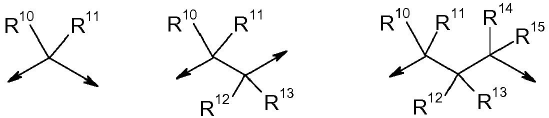 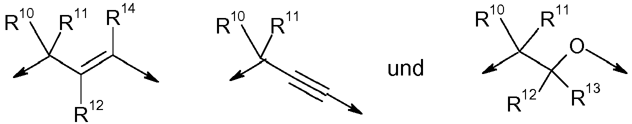 R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R 6)=NOR⁸substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch n Reste aus der Gruppe bestehend aus R⁶, R^{6a} , R⁸ und R⁹ substituierten Ring aus der Gruppe bestehend aus 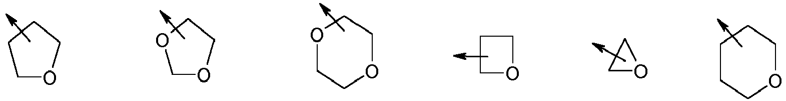 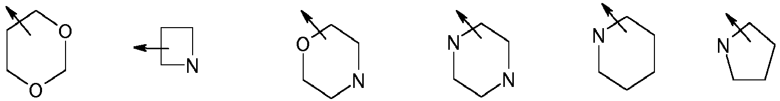 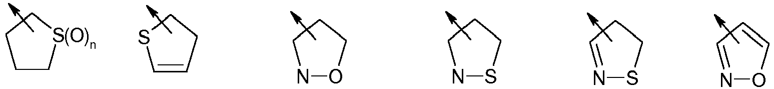 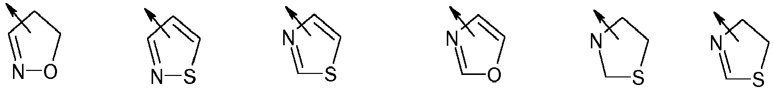 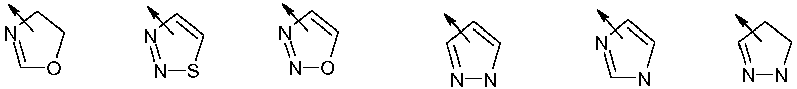 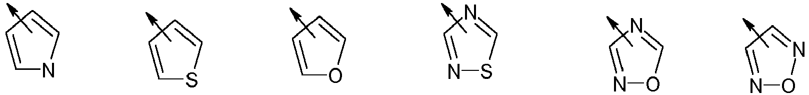 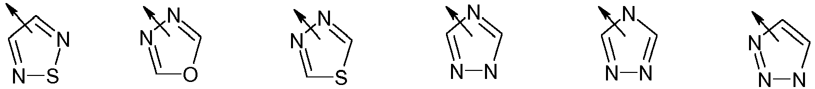 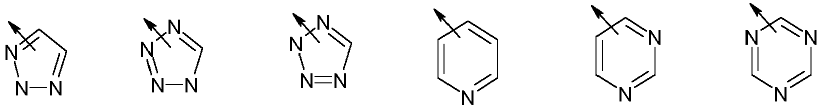 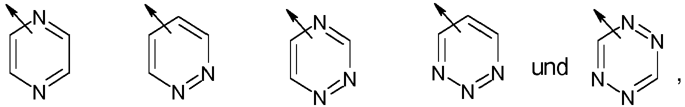 oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff oder X³;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5
zur Bekämpfung unerwünschter Pflanzen.

12. Verwendung von 3-Phenylisoxazolin-5-carboxamiden und 3-Phenylisoxazolin-5-thioamiden nach Anspruch 10 oder 11, worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl,
R³ bedeutet Fluor, Chlor, oder Cyano,
oder durch jeweils m Reste aus der Gruppe bestehend aus Fluor und Chlor substituiertes (C₁-C₆)-Alkyl, (C₃-C₆)-Cycloalkyl, (C₂-C₆)-Alkenyl oder (C₂-C₆)-Alkinyl, oder durch m Reste aus der Gruppe bestehend aus Fluor und Chlor substituiertes (C₁-C₆)-Alkylcarbonyl,
R⁴ bedeutet Wasserstoff, Hydroxy oder (C₁-C₈)-Alkyl,
A bedeutet eine Bindung oder eine divalente Einheit aus der Gruppe bestehend aus CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH(CH₂OCH₃),CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ und CH₂CH₂CONHCH₂;
Y bedeutet O oder S;
X bedeutet Wasserstoff, Cyano, Hydroxy, X¹,
oder
durch jeweils m Reste aus der Gruppe bestehend aus Fluor, Chlor, Cyano, Hydroxy, X¹, OX¹, NHX¹, S(O)ₙ R⁵, CO₂R⁸, CONR⁶R⁸, CONR⁸SO₂R⁵ und POR⁹R⁹ substituiertes (C₁-C₁₂)-Alkyl, (C₃-C₈)-Cycloalkyl, (C₂-C₁₂)-Alkenyl oder (C₂-C₁₂)-Alkinyl;
X¹ bedeutet einen durch n Reste aus der Gruppe bestehend aus R⁶, R^{6a} , R⁸ und R⁹ substituierten Ring aus der Gruppe bestehend aus 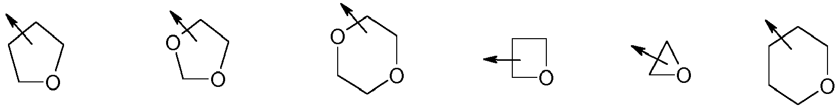 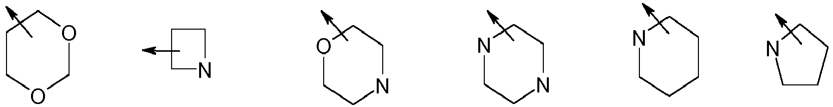 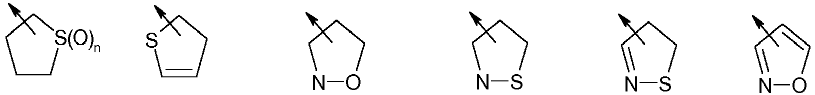 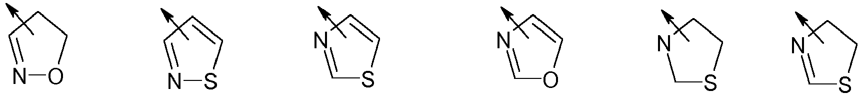 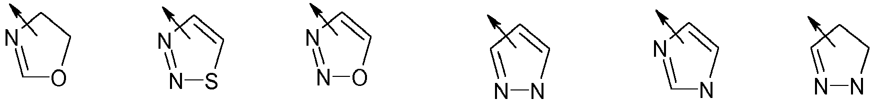 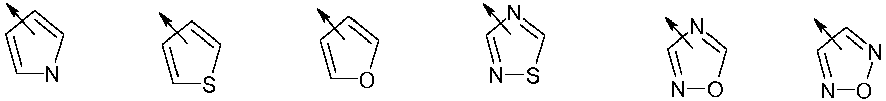 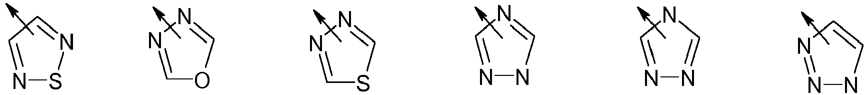 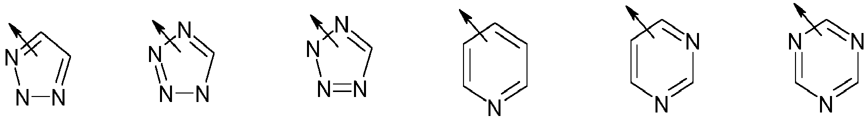 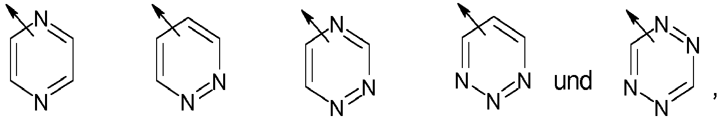 oder durch n Reste aus der Gruppe bestehend aus R⁶, R⁸ und R⁹ substituiertes Phenyl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy,
X³ bedeutet Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy,
X⁵ bedeutet Wasserstoff oder X³;
R⁵ bedeutet Methyl oder Ethyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor und Chlor substituiertes (C₁-C₆)-Alkyl;
R⁸ bedeutet R⁷;
R⁹ bedeutet (C₁-C₃)-Alkoxy,
m bedeutet 0, 1, 2 oder 3;
n bedeutet 0, 1 oder 2.

13. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder mindestens einer Verbindung der Formel (Ia) gemäß einem der Ansprüche 10 bis 12 oder eines herbiziden Mittels nach einem der Ansprüche 4 bis 9 auf die Pflanzen oder auf den Ort des unerwünschten Pflanzenwachstums appliziert.

14. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

15. Verwendung nach einem der Ansprüche 10 bis 12 oder 14, **dadurch gekennzeichnet, daß** die Verbindungen der Formel (I) zur Bekämpfung unerwünschter Pflanzen in Kulturen von Nutzpflanzen eingesetzt werden.

16. Verwendung nach Anspruch 15, **dadurch gekennzeichnet, daß** die Nutzpflanzen transgene Nutzpflanzen sind.

## Claims

1. 3-Phenylisoxazoline-5-carboxamides or 3-phenylisoxazoline-5-thioamides of the formula (I) or salts thereof 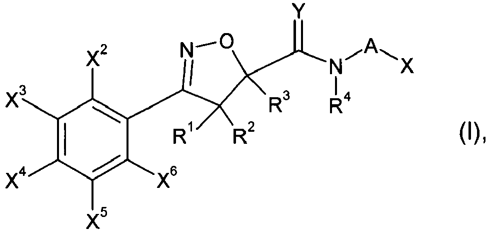 in which
R¹ and R² independently of one another are each hydrogen, fluorine, chlorine, bromine, iodine, cyano, or (C₁-C₄)-alkyl or (C₁-C₄)-alkoxy substituted by in each case m radicals from the group consisting of fluorine, chlorine, bromine, iodine and cyano,
or
R¹ and R² together with the carbon atom to which they are attached form a saturated, partially or fully unsaturated three-, four- or 5-membered ring which is constructed of q carbon atoms and p oxygen atoms;
R³ is fluorine, chlorine, cyano, (C₁-C₃)-alkylcarbonyloxy or S(O)ₙ R⁵,
or (C₁-C₆)-alkyl, (C₃-C₆) -cycloalkyl, (C₂-C₆) -alkenyl or (C₂-C₆)-alkynyl substituted by in each case m radicals from the group consisting of fluorine, chlorine, bromine, iodine, cyano, (C₁-C₄)-alkoxy and hydroxy,
or (C₁-C₆) -alkylcarbonyl, (C₂-C₆) -alkenylcarbonyl or (C₃-C₆)-cycloalkylcarbonyl substituted by in each case m radicals from the group consisting of fluorine, chlorine, bromine, iodine, cyano and (C₁-C₆)-alkoxy;
R⁴ is hydrogen, cyano,
or (C₁-C₈)-alkyl or (C₃-C₈)-cycloalkyl substituted by in each case m radicals from the group consisting of fluorine, chlorine, bromine, iodine, cyano, hydroxy and (C₁-C₆)-alkoxy;
A is a bond or a divalent unit from the group consisting of 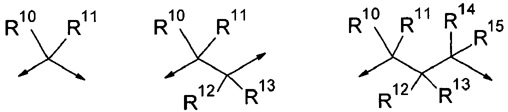 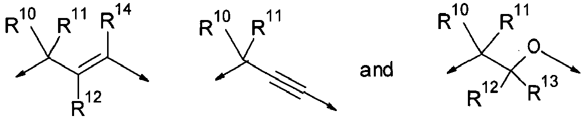 R¹⁰, R¹¹, R¹², R¹³, R¹⁴ and R¹⁵ are each independently of one another
hydrogen, fluorine, chlorine, bromine, iodine, hydroxy, cyano, CO₂R⁸, CONR⁶R⁸, R⁵,
or (C₁-C₆)-alkyl, (C₃-C₅)-cycloalkyl, (C₂-C₆) -alkenyl, (C₂-C₆)-alkynyl, each of which is substituted by m radicals from the group consisting of fluorine, chlorine, bromine, iodine, hydroxy and cyano ;
or (C₁-C₆)-alkoxy, (C₃-C₆) -cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
Y is oxygen or sulfur;
X is hydrogen, cyano, hydroxy, X¹,
or
(C₁-C₁₂) -alkyl, (C₃-C₈) -cycloalkyl, (C₂-C₁₂) -alkenyl or (C₂-C₁₂) -alkynyl substituted by in each case m radicals from the group consisting of fluorine, chlorine, bromine, iodine, cyano, hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ and C (R⁶) =NOR⁸,
or
X, A and R⁴ together with the nitrogen atom to which they are attached form a saturated, partially or fully unsaturated five-, six- or seven-membered ring which, in addition to this nitrogen atom, contains k carbon atoms, n oxygen atoms, p sulphur atoms and p elements from the group consisting of NR⁷ and NCOR⁷ as ring atoms, where a carbon atom carries p oxo groups;
X¹ is a 5- or 6-membered saturated, partially unsaturated, fully unsaturated or aromatic ring which is constructed of r carbon atoms, s nitrogen atoms, n sulphur atoms and n oxygen atoms and which is substituted by n radicals from the group consisting of R⁶_{,} R^{6a}, R⁸ and R⁹;
X², X⁴ and X⁶ independently of one another are each hydrogen, fluorine, chlorine, bromine, iodine, cyano, nitro,
or (C₁-C₄)-alkyl, (C₃-C₅) -cycloalkyl, (C₂-C₄)-alkenyl, (C₂-C₄)-alkynyl, (C₁-C₄)-alkoxy, (C₂-C₄) -alkenyloxy, (C₂-C₄)-alkynyloxy or (C₁-C₄)-alkylcarbonyl, each of which is substituted by m radicals from the group consisting of fluorine, chlorine, bromine, iodine, cyano and (C₁-C₄)-alkoxy;
X³ is fluorine, chlorine, bromine, iodine, hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵ , SO₂NR⁶R⁸, OSO₂NR⁶R⁸,
or (C₁-C₆)-alkyl, (C₃-C₅) -cycloalkyl, (C₂-C₆) -alkenyl, (C₂-C₆)-alkynyl, each of which is substituted by m radicals from the group consisting of fluorine, chlorine, bromine, iodine, hydroxy and cyano,
or (C₁-C₆)-alkoxy, (C₃-C₆) -cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
X⁵ is hydrogen or X³;
R⁵ is (C₁-C₆)-alkyl or (C₃-C₆)-cycloalkyl, each of which is substituted by m radicals from the group consisting of fluorine, chlorine, bromine, iodine, cyano and hydroxy;
R⁶ is hydrogen or R⁵;
R^{6a} is fluorine, chlorine, bromine, iodine, cyano, hydroxy, S(O)ₙR⁵ or (C₁-C₆) -alkoxy, (C₂-C₆) -alkenyloxy or (C₃-C₆)-alkynyloxy, each of which is substituted by m radicals from the group consisting of fluorine, chlorine, bromine, cyano and (C₁-C₂)-alkoxy;
R⁷ is hydrogen or (C₁-C₆)-alkyl, (C₃-C₆) -cycloalkyl, (C₂-C₄)-alkenyl or (C₂-C₄)-alkynyl, each of which is substituted by m radicals from the group consisting of fluorine, chlorine, bromine, cyano and (C₁-C₂)-alkoxy;
R⁸ is R⁷;
R⁹ is (C₁-C₃)-alkyl or (C₁-C₃)-alkoxy;
k is 3, 4, 5 or 6;
m is 0, 1, 2, 3, 4 or 5;
n is 0, 1 or 2;
p is 0 or 1;
q is 3, 4 or 5;
r is 1, 2, 3, 4 or 5;
s is 0, 1, 2, 3 or 4;
with the proviso that X³ and X⁴ are not both substituted or unsubstituted alkoxy.

2. 3-Phenylisoxazoline-5-carboxamides or 3-phenylisoxazoline-5-thioamides of the formula (I), or salts thereof 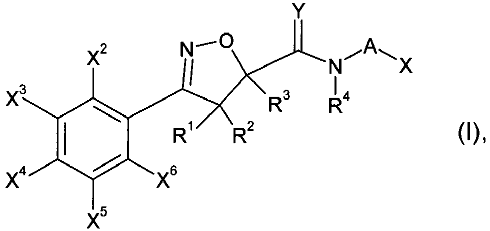 in which
R¹ and R² independently of one another are each hydrogen, fluorine, chlorine, bromine, iodine, cyano, or (C₁-C₄)-alkyl or (C₁-C₄)-alkoxy substituted by in each case m radicals from the group consisting of fluorine, chlorine, bromine, iodine and cyano,
or
R¹ and R² together with the carbon atom to which they are attached form a saturated, partially or fully unsaturated three-, four- or 5-membered ring which is constructed of q carbon atoms and p oxygen atoms;
R³ is fluorine, chlorine, cyano, (C₁-C₃)-alkylcarbonyloxy or S(O)ₙR⁵,
or (C₁-C₆)-alkyl, (C₃-C₆) -cycloalkyl, (C₂-C₆) -alkenyl or (C₂-C₆)-alkynyl substituted by in each case m radicals from the group consisting of fluorine, chlorine, bromine, iodine, cyano, (C₁-C₄)-alkoxy and hydroxy,
or (C₁-C₆)-alkylcarbonyl, (C₂-C₆) -alkenylcarbonyl or (C₃-C₆)-cycloalkylcarbonyl substituted by in each case m radicals from the group consisting of fluorine, chlorine, bromine, iodine, cyano and (C₁-C₆)-alkoxy;
R⁴ is hydrogen, cyano,
or (C₁-C₈)-alkyl or (C₃-C₈)-cycloalkyl substituted by in each case m radicals from the group consisting of fluorine, chlorine, bromine, iodine, cyano, hydroxy and (C₁-C₆)-alkoxy;
A is a bond or a divalent unit from the group consisting of 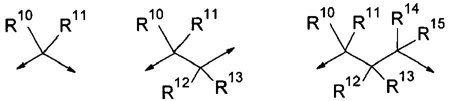 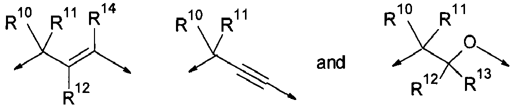 R¹⁰, R¹¹, R¹², R¹³, R¹⁴ and R¹⁵ are each independently of one another
hydrogen, fluorine, chlorine, bromine, iodine, hydroxy, cyano, CO₂R⁸, CONR⁶R⁸, R⁵,
or (C₁-C₆) -alkyl, (C₃-C₅) -cycloalkyl, (C₂-C₆) -alkenyl, (C₂-C₆)-alkynyl, each of which is substituted by m radicals from the group consisting of fluorine, chlorine, bromine, iodine, hydroxy and cyano;
or (C₁-C₆)-alkoxy, (C₃-C₆)-cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
Y is oxygen or sulfur;
X is hydrogen, cyano, hydroxy, X¹,
or
(C₁-C₁₂)-alkyl, (C₃-C₈)-cycloalkyl, (C₂-C₁₂) -alkenyl or (C₂-C₁₂) -alkynyl substituted by in each case m radicals from the group consisting of fluorine, chlorine, bromine, iodine, cyano, hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ and C(R⁶)=NOR⁸,
or
X, A and R⁴ together with the nitrogen atom to which they are attached form a saturated, partially or fully unsaturated five-, six- or seven-membered ring which, in addition to this nitrogen atom, contains k carbon atoms, n oxygen atoms, p sulphur atoms and p elements from the group consisting of NR⁷ and NCOR⁷ as ring atoms, where a carbon atom carries p oxo groups;
X¹ is a ring, substituted by n radicals from the group consisting of R⁶, R^{6a}, R⁸ and R⁹, from the group consisting of 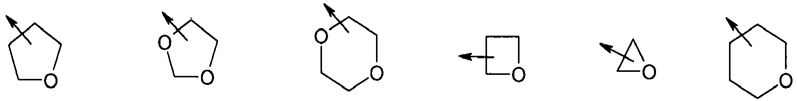 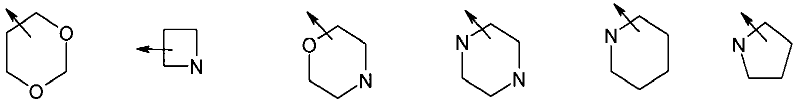 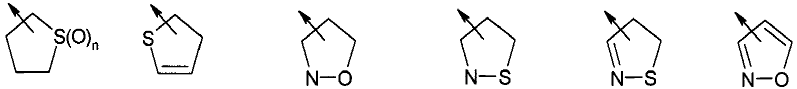 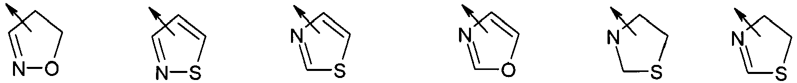 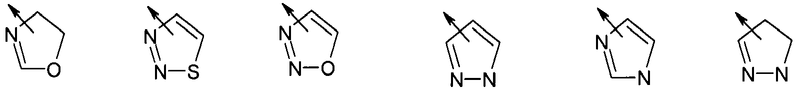 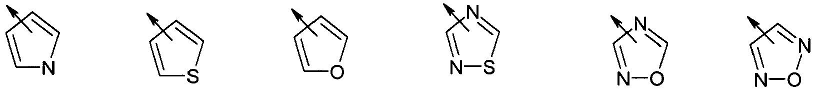 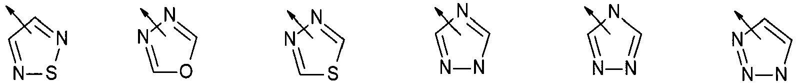 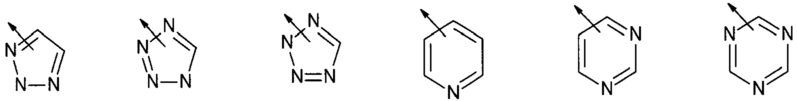 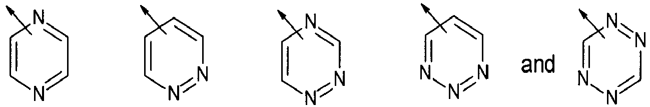 X², X⁴ and X⁶ independently of one another are each hydrogen, fluorine, chlorine, bromine, iodine, cyano, nitro,
or (C₁-C₄) -alkyl, (C₃-C₅) -cycloalkyl, (C₂-C₄) -alkenyl, (C₂-C₄)-alkynyl, (C₁-C₄)-alkoxy, (C₂-C₄) -alkenyloxy, (C₂-C₄)-alkynyloxy or (C₁-C₄)-alkylcarbonyl, each of which is substituted by m radicals from the group consisting of fluorine, chlorine, bromine, iodine, cyano and (C₁-C₄)-alkoxy;
X³ is fluorine, chlorine, bromine, iodine, hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵ , SO₂NR⁶R⁸, OSO₂NR⁶R⁸,
or (C₁-C₆)-alkyl, (C₃-C₅)-cycloalkyl, (C₂-C₆) -alkenyl, (C₂-C₆)-alkynyl, each of which is substituted by m radicals from the group consisting of fluorine, chlorine, bromine, iodine, hydroxy and cyano,
or (C₁-C₆) -alkoxy, (C₃-C₆) -cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
X⁵ is hydrogen or X³;
R⁵ is (C₁-C₆) -alkyl or (C₃-C₆) -cycloalkyl, each of which is substituted by m radicals from the group consisting of fluorine, chlorine, bromine, iodine, cyano and hydroxy;
R⁶ is hydrogen or R⁵;
R^{6a} is fluorine, chlorine, bromine, iodine, cyano, hydroxy, S(O)ₙR⁵ or (C₁-C₆)-alkoxy, (C₂-C₆)-alkenyloxy or (C₂-C₆)-alkynyloxy, each of which is substituted by m radicals from the group consisting of fluorine, chlorine, bromine, cyano and (C₁-C₂)-alkoxy;
R⁷ is hydrogen or (C₁-C₆)-alkyl, (C₃-C₆)-cycloalkyl, (C₂-C₄)-alkenyl or (C₂-C₄)-alkynyl, each of which is substituted by m radicals from the group consisting of fluorine, chlorine, bromine, cyano and (C₁-C₂)-alkoxy;
R⁸ is R⁷;
R⁹ is (C₁-C₃)-alkyl or (C₁-C₃)-alkoxy;
k is 3, 4, 5 or 6;
m is 0, 1, 2, 3, 4 or 5;
n is 0, 1 or 2;
p is 0 or 1;
q is 3, 4 or 5;
with the proviso that X³ and X⁴ are not both substituted or unsubstituted alkoxy.

3. 3-Phenylisoxazoline-5-carboxamides or 3-phenylisoxazoline-5-thioamides according to Claim 1 or 2 in which
R¹ and R² independently of one another are each hydrogen, fluorine, chlorine, bromine, iodine, cyano, or (C₁-C₄)-alkyl substituted by in each case m radicals from the group consisting of fluorine, chlorine, bromine, iodine and cyano,
R³ is fluorine, chlorine or cyano,
or (C₁-C₆)-alkyl, (C₃-C₆) -cycloalkyl, (C₂-C₆) -alkenyl, (C₂-C₆)-alkynyl, each of which is substituted by m radicals from the group consisting of fluorine and chlorine,
or (C₁-C₆)-alkylcarbonyl which is substituted by m radicals from the group consisting of fluorine and chlorine;
A is a bond or a divalent unit from the group consisting of CH₂, CH₂CH₂, CHCH₃, CH₂CH₂CH₂, CH (CH₂CH₃), CH(CH₃) CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O,
CH(cPr)CH₂O, CH(CH₂OCH₃), CH(CH₂CH₂SCH₃), CH(COOH), CH(COOCH₃), CH(COOH)CH₂, CH(COOCH₃)CH₂, CH₂COH (CF₃), CH(CONHCH₃), CH (CONHCH₃) CH₂ and CH₂CH₂CONHCH₂;
R⁴ is hydrogen or (C₁-C₈)-alkyl;
Y is oxygen or sulfur;
X is hydrogen, cyano, hydroxy, X¹,
or
(C₁-C₁₂)-alkyl, (C₃-C₈) -cycloalkyl, (C₂-C₁₂) -alkenyl or (C₂-C₁₂)-alkynyl, each of which is substituted by m radicals from the group consisting of fluorine, chlorine, cyano, hydroxy, OR⁷, X¹, OX¹, NHX¹, S(O)ₙ R⁵, CO₂R⁸, CONR⁶R⁸, CONR⁸SO₂R⁵ and POR⁹R⁹;
X¹ is a ring, substituted by n radicals from the group consisting of R⁶, R^{6a}, R⁸ and R⁹, from the group consisting of 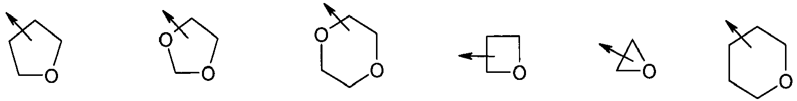 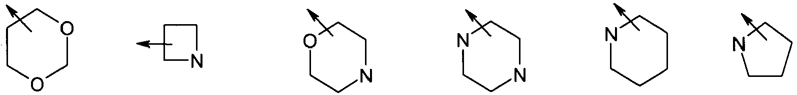 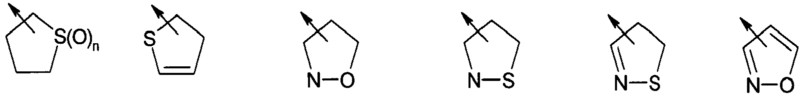 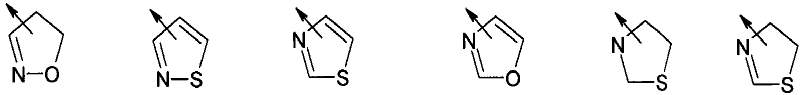 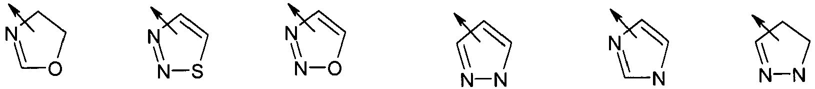 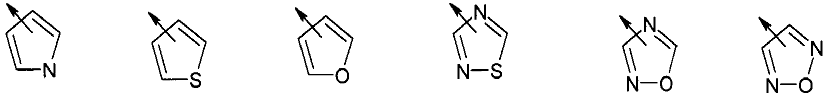 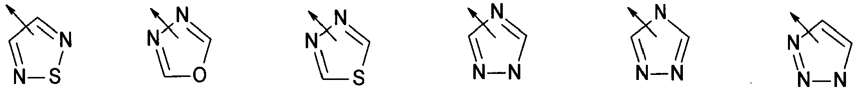 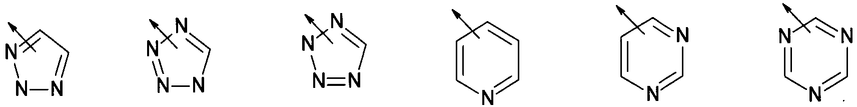 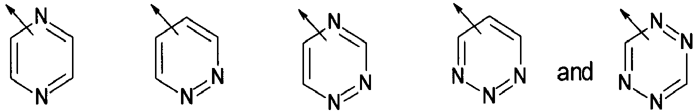 X², X⁴ and X⁶ independently of one another are each hydrogen, fluorine or chlorine,
or (C₁-C₄)-alkyl or (C₁-C₄)-alkoxy, each of which is substituted by m radicals from the group consisting of fluorine, chlorine, cyano and (C₁-C₄)-alkoxy;
X³ is fluorine, chlorine, bromine, cyano,
or (C₁-C₆)-alkyl which is in each case substituted by m radicals from the group consisting of fluorine and chlorine;
or (C₁-C₆)-alkoxy which is substituted by m radicals from the group consisting of fluorine and chlorine;
X⁵ is hydrogen or X³;
R⁵ is methyl or ethyl;
R⁶ is hydrogen or R⁵;
R^{6a} is fluorine, chlorine, bromine, iodine, cyano, hydroxy, S(O)ₙR⁵ or (C₁-C₆)-alkoxy, (C₂-C₆) -alkenyloxy or (C₂-C₆)-alkynyloxy, each of which is substituted by m radicals from the group consisting of fluorine, chlorine, bromine, cyano and (C₁-C₂)-alkoxy;
R⁷ is hydrogen or (C₁-C₆)-alkyl which is substituted by in each case m radicals from the group consisting of fluorine and chlorine;
R⁸ is R⁷;
R⁹ is (C₁-C₃)-alkoxy;
m is 0, 1, 2 or 3;
n is 0, 1 or 2;
with the proviso that X³ and X⁴ are not both substituted or unsubstituted alkoxy.

4. Herbicidal composition which comprises a herbicidally effective amount of at least one compound of the formula (I) according to any of Claims 1 to 3.

5. Herbicidal composition according to Claim 4 in a mixture with formulation auxiliaries.

6. Herbicidal composition according to Claim 4 or 5, comprising at least one further pesticidally active compound from the group of the insecticides, acaricides, herbicides, fungicides, safeners and growth regulators.

7. Herbicidal composition according to Claim 6, comprising a safener.

8. Herbicidal composition according to Claim 7 where the safener is selected from the group consisting of mefenpyr-diethyl, cyprosulfamide, isoxadifen-ethyl, cloquintocet-mexyl, benoxacor and dichlormid.

9. Herbicidal composition according to any of Claims 6 to 8, comprising a further herbicide.

10. Use of 3-phenylisoxazoline-5-carboxamides or 3-phenylisoxazoline-5-thioamides of the formula (Ia) 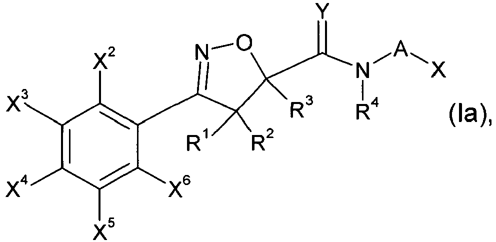 in which
R¹ and R² independently of one another are each hydrogen, fluorine, chlorine, bromine, iodine, cyano, or (C₁-C₄)-alkyl or (C₁-C₄)-alkoxy substituted by in each case m radicals from the group consisting of fluorine, chlorine, bromine, iodine and cyano,
or
R¹ and R² together with the carbon atom to which they are attached form a saturated, partially or fully unsaturated three-, four- or 5-membered ring which is constructed of q carbon atoms and p oxygen atoms;
R³ is fluorine, chlorine, cyano, (C₁-C₃)-alkylcarbonyloxy or S(O)ₙR⁵,
or (C₁-C₆)-alkyl, (C₃-C₆) -cycloalkyl, (C₂-C₆) -alkenyl or (C₂-C₆)-alkynyl substituted by in each case m radicals from the group consisting of fluorine, chlorine, bromine, iodine, cyano, (C₁-C₄)-alkoxy and hydroxy,
or (C₁-C₆)-alkylcarbonyl, (C₂-C₆)-alkenylcarbonyl or (C₃-C₆)-cycloalkylcarbonyl substituted by in each case m radicals from the group consisting of fluorine, chlorine, bromine, iodine, cyano and (C₁-C₆) -alkoxy;
R⁴ is hydrogen, cyano, hydroxy,
or (C₁-C₈)-alkyl or (C₃-C₈)-cycloalkyl substituted by in each case m radicals from the group consisting of fluorine, chlorine, bromine, iodine, cyano, hydroxy and
(C₁-C₆)-alkoxy;
A is a bond or a divalent unit from the group consisting of 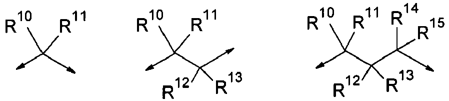 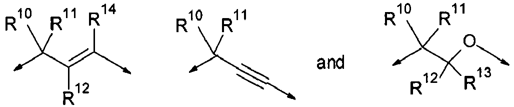 R¹⁰, R¹¹, R¹², R¹³, R¹⁴ and R¹⁵ are each independently of one another
hydrogen, fluorine, chlorine, bromine, iodine, hydroxy, cyano, CO₂R⁸, CONR⁶R⁸, R⁵,
or (C₁-C₆)-alkyl, (C₃-C₅) -cycloalkyl, (C₂-C₆) -alkenyl, (C₂-C₆)-alkynyl, each of which is substituted by m radicals from the group consisting of fluorine, chlorine, bromine, iodine, hydroxy and cyano ;
or (C₁-C₆)-alkoxy, (C₃-C₆) -cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
Y is oxygen or sulfur;
X is hydrogen, cyano, hydroxy, X¹,
or
(C₁-C₁₂)-alkyl, (C₃-C₈)-cycloalkyl, (C₂-C₁₂)-alkenyl or (C₂-C₁₂)-alkynyl substituted by in each case m radicals from the group consisting of fluorine, chlorine, bromine, iodine, cyano, hydroxy, OR⁷, X¹, OX¹, NHX¹, S(O)ₙ R⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ and C (R⁶)=NOR⁸,
or
X, A and R⁴ together with the nitrogen atom to which they are attached form a saturated, partially or fully unsaturated five-, six- or seven-membered ring which, in addition to this nitrogen atom, contains k carbon atoms, n oxygen atoms, p sulphur atoms and p elements from the group consisting of NR⁷ and NCOR⁷ as ring atoms, where a carbon atom carries p oxo groups;
X¹ is a 5- or 6-membered saturated, partially unsaturated, fully unsaturated or aromatic ring which is constructed of r carbon atoms, s nitrogen atoms, n sulphur atoms and n oxygen atoms and which is substituted by n radicals from the group consisting of R⁶, R^{6a}, R⁸ and R⁹;
or phenyl which is substituted by n radicals from the group consisting of R⁶, R⁸ and R⁹;
X², X⁴ and X⁶ independently of one another are each hydrogen, fluorine, chlorine, bromine, iodine, cyano, nitro,
or (C₁-C₄)-alkyl, (C₃-C₅) -cycloalkyl, (C₂-C₄) -alkenyl, (C₂-C₄)-alkynyl, (C₁-C₄)-alkoxy, (C₂-C₄) -alkenyloxy, (C₂-C₄)-alkynyloxy or (C₁-C₄) -alkylcarbonyl, each of which is substituted by m radicals from the group consisting of fluorine, chlorine, bromine, iodine, cyano and (C₁-C₄)-alkoxy;
X³ is hydrogen, fluorine, chlorine, bromine, iodine, hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙ R⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl, each of which is substituted by m radicals from the group consisting of fluorine, chlorine, bromine, iodine, hydroxy and cyano,
or (C₁-C₆)-alkoxy, (C₃-C₆)-cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
X⁵ is hydrogen or X³;
R⁵ is (C₁-C₆)-alkyl or (C₃-C₆)-cycloalkyl, each of which is substituted by m radicals from the group consisting of fluorine, chlorine, bromine, iodine, cyano and hydroxy;
R⁶ is hydrogen or R⁵;
R^{6a} is fluorine, chlorine, bromine, iodine, cyano, hydroxy, S(O)ₙR⁵ or (C₁-C₆)-alkoxy, (C₂-C₆)-alkenyloxy or (C₂-C₆)-alkynyloxy, each of which is substituted by m radicals from the group consisting of fluorine, chlorine, bromine, cyano and (C₁-C₂)-alkoxy;
R⁷ is hydrogen or (C₁-C₆)-alkyl, (C₃-C₆)-cycloalkyl, (C₂-C₄)-alkenyl or (C₂-C₄)-alkynyl, each of which is substituted by m radicals from the group consisting of fluorine, chlorine, bromine, cyano and (C₁-C₂)-alkoxy;
R⁸ is R⁷;
R⁹ is (C₁-C₃)-alkyl or (C₁-C₃)-alkoxy;
k is 3, 4, 5 or 6;
m is 0, 1, 2, 3, 4 or 5;
n is 0, 1 or 2;
p is 0 or 1;
q is 3, 4 or 5;
r is 1, 2, 3, 4 or 5;
s is 0, 1, 2, 3 or 4;
for controlling unwanted plants.

11. Use of 3-phenylisoxazoline-5-carboxamides or 3-phenylisoxazoline-5-thioamides of the formula (Ia) 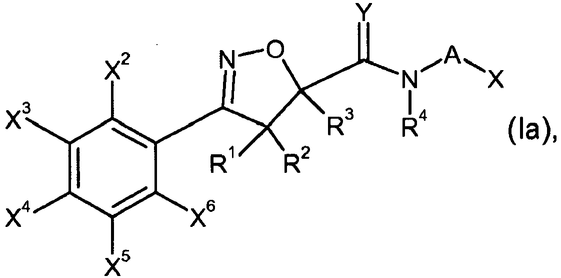 in which
R¹ and R² independently of one another are each hydrogen, fluorine, chlorine, bromine, iodine, cyano, or (C₁-C₉)-alkyl or (C₁-C₄)-alkoxy substituted by in each case m radicals from the group consisting of fluorine, chlorine, bromine, iodine and cyano,
or
R¹ and R² together with the carbon atom to which they are attached form a saturated, partially or fully unsaturated three-, four- or 5-membered ring which is constructed of q carbon atoms and p oxygen atoms;
R³ is fluorine, chlorine, cyano, (C₁-C₃)-alkylcarbonyloxy or S(O)ₙR⁵,
or (C₁-C₆)-alkyl, (C₃-C₆)-cycloalkyl, (C₂-C₆)-alkenyl or (C₂-C₆)-alkynyl substituted by in each case m radicals from the group consisting of fluorine, chlorine, bromine, iodine, cyano, (C₁-C₄)-alkoxy and hydroxy,
or (C₁-C₆)-alkylcarbonyl, (C₂-C₆)-alkenylcarbonyl or (C₃-C₆)-cycloalkylcarbonyl substituted by in each case m radicals from the group consisting of fluorine, chlorine, bromine, iodine, cyano and (C₁-C₆)-alkoxy;
R⁴ is hydrogen, cyano, hydroxy,
or (C₁-C₈)-alkyl or (C₃-C₈)-cycloalkyl substituted by in each case m radicals from the group consisting of fluorine, chlorine, bromine, iodine, cyano, hydroxy and (C₁-C₆)-alkoxy;
A is a bond or a divalent unit from the group consisting of 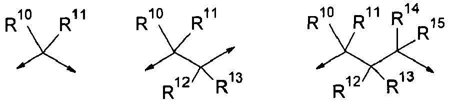 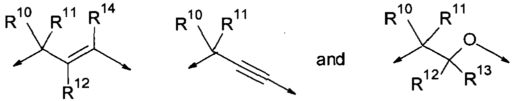 R¹⁰, R¹¹, R¹², R¹³, R¹⁴ and R¹⁵ are each independently of one another
hydrogen, fluorine, chlorine, bromine, iodine, hydroxy, cyano, CO₂R⁸, CONR⁶R⁸, R⁵,
or (C₁-C₆)-alkyl, (C₃-C₅)-cycloalkyl, (C₂-C₆)-alkenyl, (C₂-C₆)-alkynyl, each of which is substituted by m radicals from the group consisting of fluorine, chlorine, bromine, iodine, hydroxy and cyano ;
or (C₁-C₆)-alkoxy, (C₃-C₆)-cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
Y is oxygen or sulfur;
X is hydrogen, cyano, hydroxy, X¹,
or
(C₁-C₁₂)-alkyl, (C₃-C₈)-cycloalkyl, (C₂-C₁₂)-alkenyl or (C₂-C₁₂)-alkynyl substituted by in each case m radicals from the group consisting of fluorine, chlorine, bromine, iodine, cyano, hydroxy, X¹, OX¹, NHX¹, S(O)ₙR⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ and C(R⁶)=NOR⁸,
or
X, A and R⁴ together with the nitrogen atom to which they are attached form a saturated, partially or fully unsaturated five-, six- or seven-membered ring which, in addition to this nitrogen atom, contains k carbon atoms, n oxygen atoms, p sulphur atoms and p elements from the group consisting of NR⁷ and NCOR⁷ as ring atoms, where a carbon atom carries p oxo groups;
X¹ is a ring, substituted by n radicals from the group consisting of R⁶, R^{6a}, R⁸ and R⁹, from the group consisting of 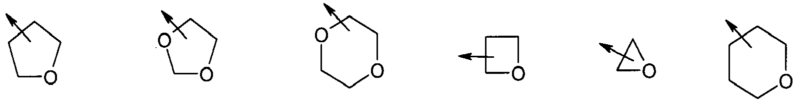 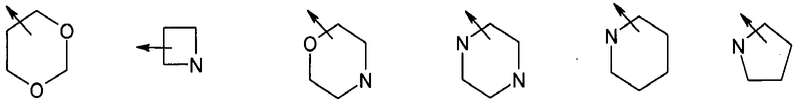 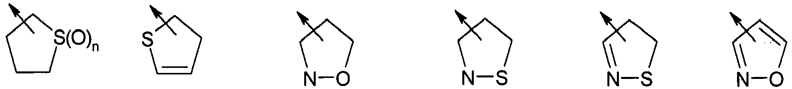 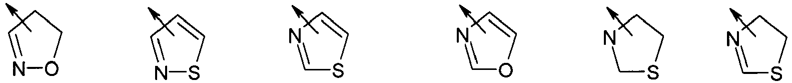 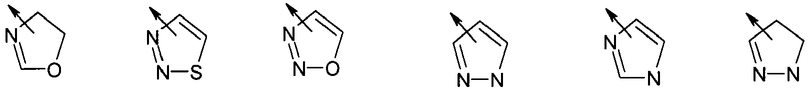 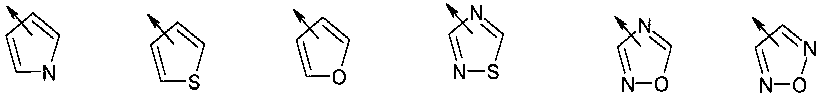 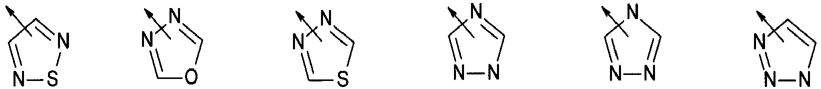 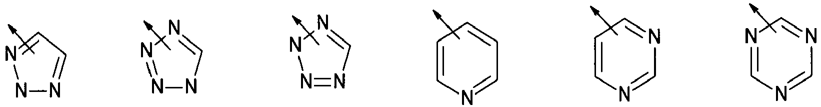 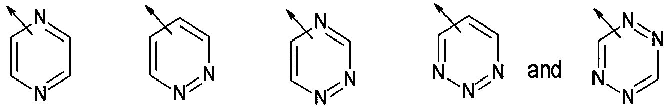 or phenyl which is substituted by n radicals from the group consisting of R⁶, R⁸ and R⁹;
X², X⁴ and X⁶ independently of one another are each hydrogen, fluorine, chlorine, bromine, iodine, cyano, nitro,
or (C₁-C₄)-alkyl, (C₃-C₅)-cycloalkyl, (C₂-C₄)-alkenyl, (C₂-C₄)-alkynyl, (C₁-C₄)-alkoxy, (C₂-C₄)-alkenyloxy, (C₂-C₄)-alkynyloxy or (C₁-C₄)-alkylcarbonyl, each of which is substituted by m radicals from the group consisting of fluorine, chlorine, bromine, iodine, cyano and (C₁-C₄)-alkoxy;
X³ is hydrogen, fluorine, chlorine, bromine, iodine, hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl, each of which is substituted by m radicals from the group consisting of fluorine, chlorine, bromine, iodine, hydroxy and cyano,
or (C₁-C₆)-alkoxy, (C₃-C₆)-cycloalkoxy, (C₂-C₆)-alkenyloxy or (C₂-C₆)-alkynyloxy, each of which is substituted by m radicals from the group consisting of fluorine, chlorine, bromine, iodine, cyano and (C₁-C₂)-alkoxy,
X⁵ is hydrogen or X³;
R⁵ is (C₁-C₆)-alkyl or (C₃-C₆)-cycloalkyl, each of which is substituted by m radicals from the group consisting of fluorine, chlorine, bromine, iodine, cyano and hydroxy;
R⁶ is hydrogen or R⁵;
R^{6a} is fluorine, chlorine, bromine, iodine, cyano, hydroxy, S(O)ₙR⁵ or (C₁-C₆)-alkoxy, (C₂-C₆)-alkenyloxy or (C₂-C₆)-alkynyloxy, each of which is substituted by m radicals from the group consisting of fluorine, chlorine, bromine, cyano and (C₁-C₂)-alkoxy;
R⁷ is hydrogen or (C₁-C₆)-alkyl, (C₃-C₆)-cycloalkyl, (C₂-C₄)-alkenyl or (C₂-C₄)-alkynyl, each of which is substituted by m radicals from the group consisting of fluorine, chlorine, bromine, cyano and (C₁-C₂)-alkoxy;
R⁸ is R⁷;
R⁹ is (C₁-C₃)-alkyl or (C₁-C₃)-alkoxy;
k is 3, 4, 5 or 6;
m is 0, 1, 2, 3, 4 or 5;
n is 0, 1 or 2;
p is 0 or 1;
q is 3, 4 or 5 for controlling unwanted plants.

12. Use of 3-phenylisoxazoline-5-carboxamides or 3-phenylisoxazoline-5-thioamides according to Claim 10 or 11 in which
R¹ and R² independently of one another are each hydrogen, fluorine, chlorine, bromine, iodine, cyano, or (C₁-C₄)-alkyl substituted by in each case m radicals from the group consisting of fluorine, chlorine, bromine, iodine and cyano,
R³ is fluorine, chlorine or cyano,
or (C₁-C₆)-alkyl, (C₃-C₆)-cycloalkyl, (C₂-C₆)-alkenyl, (C₂-C₆)-alkynyl, each of which is substituted by m radicals from the group consisting of fluorine and chlorine,
or (C₁-C₆)-alkylcarbonyl which is substituted by m radicals from the group consisting of fluorine and chlorine;
R⁴ is hydrogen, hydroxy or (C₁-C₈)-alkyl;
A is a bond or a divalent unit from the group consisting of CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH (CH₂OCH₃), CH (CH₂CH₂SCH₃), CH(COOH), CH(COOCH₃), CH(COOH)CH₂, CH(COOCH₃)CH₂, CH₂COH(CF₃), CH(CONHCH₃), CH (CONHCH₃)CH₂ and CH₂CH₂CONHCH₂;
Y is 0 or S;
X is hydrogen, cyano, hydroxy, X¹,
or
(C₁-C₁₂)-alkyl, (C₃-C₈)-cycloalkyl, (C₂-C₁₂)-alkenyl or (C₂-C₁₂)-alkynyl, each of which is substituted by m radicals from the group consisting of fluorine, chlorine, cyano, hydroxy, X¹, OX¹, NHX¹, S(O)ₙR⁵, CO₂R⁸, CONR⁶R⁸, CONR⁸SO₂R⁵ and POR⁹R⁹;
X¹ is a ring, substituted by n radicals from the group consisting of R⁶, R^{6a}, R⁸ and R⁹, from the group consisting of 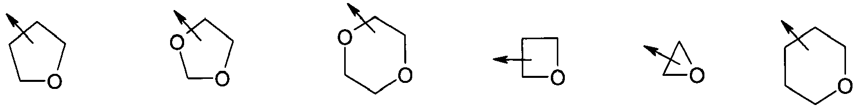 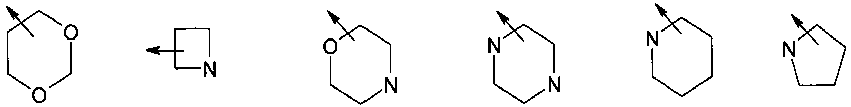 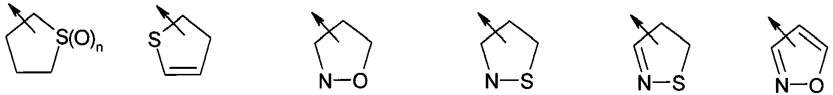 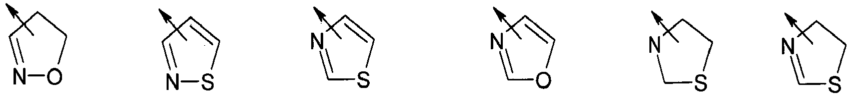 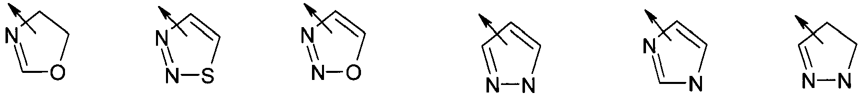 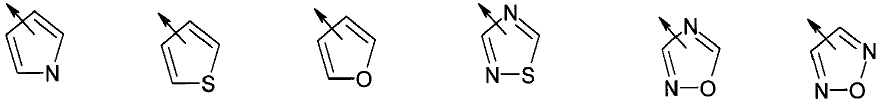 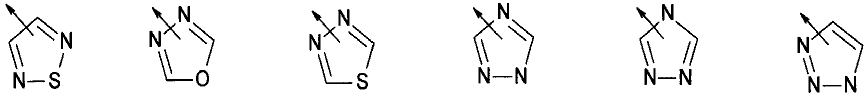 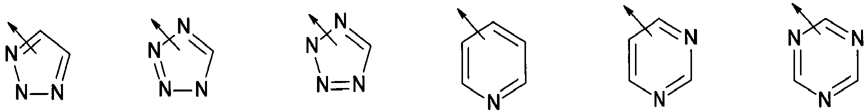 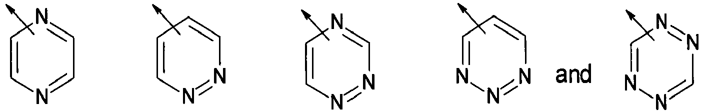 or phenyl which is substituted by n radicals from the group consisting of R⁶, R⁸ and R⁹;
X², X⁴ and X⁶ independently of one another are each hydrogen, fluorine or chlorine,
or (C₁-C₄)-alkyl or (C₁-C₄)-alkoxy, each of which is substituted by m radicals from the group consisting of fluorine, chlorine, cyano and (C₁-C₄)-alkoxy;
X³ is hydrogen, fluorine, chlorine, bromine, cyano,
or (C₁-C₆)-alkyl which is in each case substituted by m radicals from the group consisting of fluorine and chlorine;
or (C₁-C₆)-alkoxy which is substituted by m radicals from the group consisting of fluorine and chlorine;
X⁵ is hydrogen or X³;
R⁵ is methyl or ethyl;
R⁶ is hydrogen or R⁵;
R^{6a} is fluorine, chlorine, bromine, iodine, cyano, hydroxy, S(O)ₙR⁵ or (C₁-C₆)-alkoxy, (C₂-C₆)-alkenyloxy or (C₂-C₆)-alkynyloxy, each of which is substituted by m radicals from the group consisting of fluorine, chlorine, bromine, cyano and (C₁-C₂)-alkoxy;
R⁷ is hydrogen or (C₁-C₆)-alkyl which is substituted by in each case m radicals from the group consisting of fluorine and chlorine;
R⁸ is R⁷;
R⁹ is (C₁-C₃)-alkoxy;
m is 0, 1, 2 or 3;
n is 0, 1 or 2.

13. Method for controlling unwanted plants, **characterized in that** an effective amount of at least one compound of the formula (I) according to any of Claims 1 to 3 or of at least one compound of the formula (Ia) according to any of Claims 10 to 12 or of a herbicidal composition according to any of Claims 4 to 9 is applied to the plants or the site of the unwanted vegetation.

14. Use of compounds of the formula (I) according to any of Claims 1 to 3 or of a herbicidal composition according to any of Claims 4 to 9 for controlling unwanted plants.

15. Use according to any of Claims 10 to 12 or 14, **characterized in that** the compounds of the formula (I) are used for controlling unwanted plants in crops of useful plants.

16. Use according to Claim 15, **characterized in that** the useful plants are transgenic useful plants.

## Revendications

1. 3-phénylisoxazoline-5-carboxamides et 3-phényl-isoxazoline-5-thioamides de formule (I), ou leurs sels 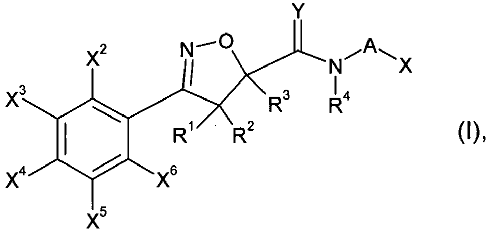 formule dans laquelle
R¹ et R² représentent chacun indépendamment l'un de l'autre un atome d'hydrogène, de fluor, de chlore, de brome, d'iode, un groupe cyano, ou un groupe alkyle en C₁-C₄ ou alcoxy en C₁-C₄, substitué dans chaque cas par m radicaux choisis dans le groupe constitué par fluoro, chloro, bromo, iodo et cyano,
ou
R¹ et R² forment ensemble avec l'atome de carbone, auquel ils sont liés, un cycle à trois, quatre ou cinq chaînons, saturé ou partiellement ou totalement insaturé, qui est constitué de q atomes de carbone et p atomes d'oxygène ;
R³ représente un atome de fluor, de chlore, un groupe cyano, alkyl(C₁-C₃)carbonyloxy ou S(O)ₙR⁵,
ou un groupe alkyle en C₁-C₆, cycloalkyle en C₃-C₆, alcényle en C₂-C₆ ou alcynyle en C₂-C₆, substitué dans chaque cas par m radicaux choisis dans le groupe constitué par fluoro, chloro, bromo, iodo, cyano, alcoxy en C₁-C₄ et hydroxy,
ou un groupe alkyl(C₁-C₆)carbonyle, alcényl(C₂-C₆)-carbonyle ou cycloalkyl(C₃-C₆)carbonyle, substitué dans chaque cas par m radicaux choisis dans le groupe constitué par fluoro, chloro, bromo, iodo, cyano et alcoxy en C₁-C₆ ;
R⁴ représente un atome d'hydrogène ou un groupe cyano,
ou un groupe alkyle en C₁-C₈ ou cycloalkyle en C₃-C₈, substitué dans chaque cas par m radicaux choisis dans le groupe constitué par fluoro, chloro, bromo, iodo, cyano, hydroxy et alcoxy en C₁-C₆ ;
A représente une liaison ou une unité divalente choisie dans le groupe constitué par 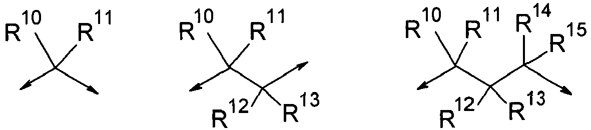 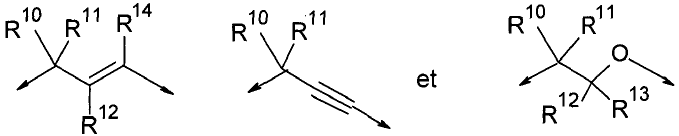 R¹⁰, R¹¹, R¹², R¹³, R¹⁴ et R¹⁵ représentent chacun indépendamment les uns des autres un atome d'hydrogène, de fluor, de chlore, de brome, d'iode, un groupe hydroxy, cyano, CO₂R⁸, CONR⁶R⁸, R⁵,
ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
Y représente un atome d'oxygène ou de soufre ;
X représente un atome d'hydrogène, un groupe cyano, hydroxy, X¹,
ou
un groupe alkyle en C₁-C₁₂, cycloalkyle en C₃-C₈, alcényle en C₂-C₁₂ ou alcynyle en C₂-C₁₂, substitué dans chaque cas par m radicaux choisis dans le groupe constitué par fluoro, chloro, bromo, iodo, cyano, hydroxy, OR⁷, X¹, OX¹, NHX¹, S(O)ₙR⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ et C(R⁶)=NOR⁸,
ou
X, A et R⁴ forment avec l'atome d'azote, auquel ils sont liés, un cycle à cinq, six ou sept chaînons, saturé ou partiellement ou totalement insaturé, qui en plus de cet atome d'azote contient, en tant qu'atomes formant le cycle, k atomes de carbone, n atomes d'oxygène, p atomes de soufre et p éléments choisis dans le groupe constitué par NR⁷ et NCOR⁷, un atome de carbone portant p groupes oxo ;
X¹ représente un cycle à cinq ou six chaînons, saturé, partiellement insaturé, totalement insaturé ou aromatique, qui est constitué de r atomes de carbone, s atomes d'azote, n atomes de soufre et n atomes d'oxygène, et qui est substitué par n radicaux choisis dans le groupe constitué par R⁶, R^{6a}, R⁸ et R⁹ ;
X², X⁴ et X⁶ représentent chacun indépendamment un atome d'hydrogène, de fluor, de chlore, de brome, d'iode, un groupe cyano, nitro,
ou un groupe alkyle en C₁-C₄, cycloalkyle en C₃-C₅, alcényle en C₂-C₄, alcynyle en C₂-C₄, alcoxy en C₁-C₄, alcényloxy en C₂-C₄, alcynyloxy en C₂-C₄ ou alkyl(C₁-C₄)-carbonyle, substitué dans chaque cas par m radicaux choisis dans le groupe constitué par fluoro, chloro, bromo, iodo, cyano et alcoxy en C₁-C₄ ;
X³ représente un atome de fluor, de chlore, de brome, d'iode, un groupe hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
X⁵ représente un atome d'hydrogène ou X³ ;
R⁵ représente un groupe alkyle en C₁-C₆ ou cycloalkyle en C₃-C₆, substitué dans chaque cas par m radicaux choisis dans le groupe constitué par fluoro, chloro, bromo, iodo, cyano et hydroxy ;
R⁶ représente un atome d'hydrogène ou R⁵ ;
R^{6a} représente un atome de fluor, de chlore, de brome, d'iode, un groupe cyano, hydroxy, S(O)ₙR⁵ ou un groupe alcoxy en C₁-C₆, alcényloxy en C₃-C₆ ou alcynyloxy en C₃-C₆, substitué dans chaque cas par m radicaux choisis dans le groupe constitué par fluoro, chloro, bromo, cyano et alcoxy en C₁-C₂ ;
R⁷ représente un atome d'hydrogène ou un groupe alkyle en C₁-C₆, cycloalkyle en C₃-C₆, alcényle en C₂-C₄ ou alcynyle en C₂-C₄, substitué dans chaque cas par m radicaux choisis dans le groupe constitué par fluoro, chloro, bromo, cyano et alcoxy en C₁-C₂ ;
R⁸ représente R⁷ ;
R⁹ représente un groupe alkyle en C₁-C₃ ou alcoxy en C₁-C₃ ;
k représente 3, 4, 5 ou 6 ;
m représente 0, 1, 2, 3, 4 ou 5 ;
n représente 0, 1 ou 2 ;
p représente 0 ou 1 ;
q représente 3, 4 ou 5 ;
r représente 1, 2, 3, 4 ou 5 ;
s représente 0, 1, 2, 3 ou 4 ;
étant entendu que X³ et X⁴ ne représentent pas simultanément un groupe alcoxy substitué ou non substitué.

2. 3-phénylisoxazoline-5-carboxamides et 3-phényl-isoxazoline-5-thioamides de formule (I), ou leurs sels 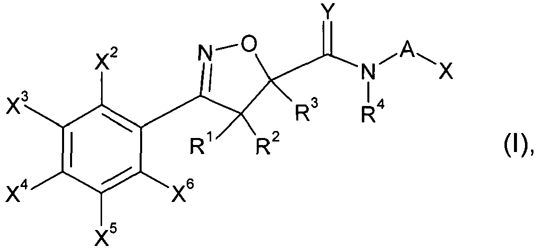 formule dans laquelle
R¹ et R² représentent chacun indépendamment l'un de l'autre un atome d'hydrogène, de fluor, de chlore, de brome, d'iode, un groupe cyano, ou un groupe alkyle en C₁-C₄ ou alcoxy en C₁-C₄, substitué dans chaque cas par m radicaux choisis dans le groupe constitué par fluoro, chloro, bromo, iodo et cyano,
ou
R¹ et R² forment ensemble avec l'atome de carbone, auquel ils sont liés, un cycle à trois, quatre ou cinq chaînons, saturé ou partiellement ou totalement insaturé, qui est constitué de q atomes de carbone et p atomes d'oxygène ;
R³ représente un atome de fluor, de chlore, un groupe cyano, alkyl(C₁-C₃)carbonyloxy ou S(O)ₙR⁵,
ou un groupe alkyle en C₁-C₆, cycloalkyle en C₃-C₆, alcényle en C₂-C₆ ou alcynyle en C₂-C₆, substitué dans chaque cas par m radicaux choisis dans le groupe constitué par fluoro, chloro, bromo, iodo, cyano, alcoxy en C₁-C₄ et hydroxy,
ou un groupe alkyl(C₁-C₆)carbonyle, alcényl(C₂-C₆)-carbonyle ou cycloalkyl(C₃-C₆)carbonyle, substitué dans chaque cas par m radicaux choisis dans le groupe constitué par fluoro, chloro, bromo, iodo, cyano et alcoxy en C₁-C₆ ;
R⁴ représente un atome d'hydrogène ou un groupe cyano,
ou un groupe alkyle en C₁-C₈ ou cycloalkyle en C₃-C₈, substitué dans chaque cas par m radicaux choisis dans le groupe constitué par fluoro, chloro, bromo, iodo, cyano, hydroxy et alcoxy en C₁-C₆ ;
A représente une liaison ou une unité divalente choisie dans le groupe constitué par 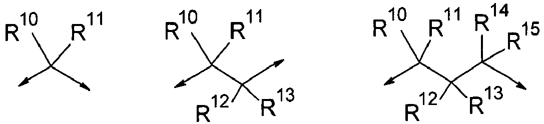 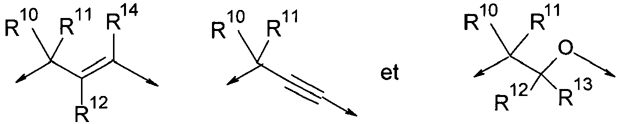 R¹⁰, R¹¹, R¹², R¹³, R¹⁴ et R¹⁵ représentent chacun indépendamment les uns des autres un atome d'hydrogène, de fluor, de chlore, de brome, d'iode, un groupe hydroxy, cyano, CO₂R⁸, CONR⁶R⁸, R⁵, ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
Y représente un atome d'oxygène ou de soufre ;
X représente un atome d'hydrogène, un groupe cyano, hydroxy, X¹,
ou
un groupe alkyle en C₁-C₁₂, cycloalkyle en C₃-C₈, alcényle en C₂-C₁₂ ou alcynyle en C₂-C₁₂, substitué dans chaque cas par m radicaux choisis dans le groupe constitué par fluoro, chloro, bromo, iodo, cyano, hydroxy, OR⁷, X¹, OX¹, NHX¹, S(O)ₙR⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ et C(R⁶)=NOR⁸,
ou
X, A et R⁴ forment avec l'atome d'azote, auquel ils sont liés, un cycle à cinq, six ou sept chaînons, saturé ou partiellement ou totalement insaturé, qui en plus de cet atome d'azote contient, en tant qu'atomes formant le cycle, k atomes de carbone, n atomes d'oxygène, p atomes de soufre et p éléments choisis dans le groupe constitué par NR⁷ et NCOR⁷, un atome de carbone portant p groupes oxo ;
X¹ représente un cycle substitué par n radicaux choisis dans le groupe constitué par R⁶, R^{6a}, R⁸ et R⁹, choisi dans le groupe constitué par 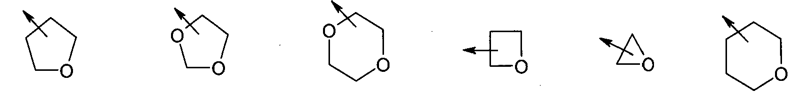 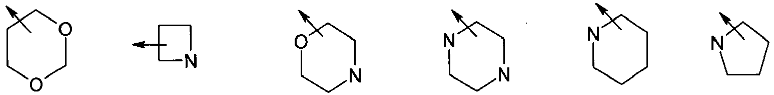 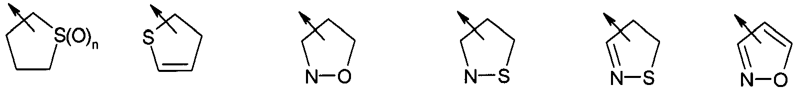 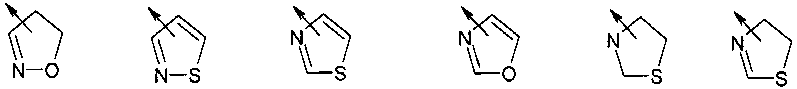 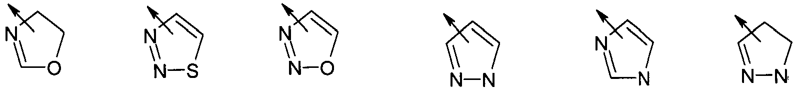 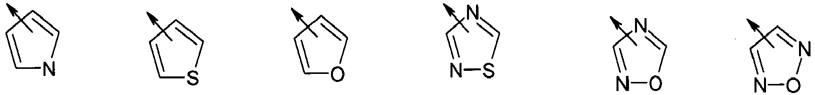 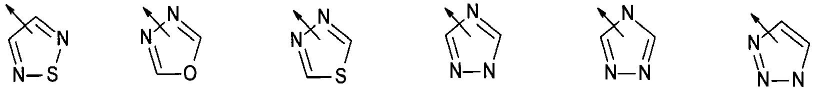 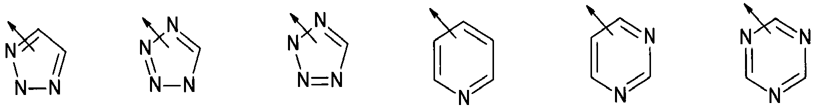 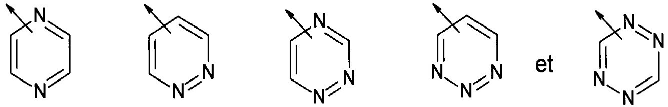 X², X⁴ et X⁶ représentent chacun indépendamment un atome d'hydrogène, de fluor, de chlore, de brome, d'iode, un groupe cyano, nitro,
ou un groupe alkyle en C₁-C₄, cycloalkyle en C₃-C₅, alcényle en C₂-C₄, alcynyle en C₂-C₄, alcoxy en C₁-C₄, alcényloxy en C₂-C₄, alcynyloxy en C₂-C₄ ou alkyl(C₁-C₄)-carbonyle, substitué dans chaque cas par m radicaux choisis dans le groupe constitué par fluoro, chloro, bromo, iodo, cyano et alcoxy en C₁-C₄ ;
X³ représente un atome de fluor, de chlore, de brome, d'iode, un groupe hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
X⁵ représente un atome d'hydrogène ou X³ ;
R⁵ représente un groupe alkyle en C₁-C₆ ou cycloalkyle en C₃-C₆, substitué dans chaque cas par m radicaux choisis dans le groupe constitué par fluoro, chloro, bromo, iodo, cyano et hydroxy ;
R⁶ représente un atome d'hydrogène ou R⁵ ;
R^{6a} représente un atome de fluor, de chlore, de brome, d'iode, un groupe cyano, hydroxy, S(O)ₙR⁵ ou un groupe alcoxy en C₁-C₆, alcényloxy en C₂-C₆ ou alcynyloxy en C₂-C₆, substitué dans chaque cas par m radicaux choisis dans le groupe constitué par fluoro, chloro, bromo, cyano et alcoxy en C₁-C₂ ;
R⁷ représente un atome d'hydrogène ou un groupe alkyle en C₁-C₆, cycloalkyle en C₃-C₆, alcényle en C₂-C₄ ou alcynyle en C₂-C₄, substitué dans chaque cas par m radicaux choisis dans le groupe constitué par fluoro, chloro, bromo, cyano et alcoxy en C₁-C₂ ;
R⁸ représente R⁷ ;
R⁹ représente un groupe alkyle en C₁-C₃ ou alcoxy en C₁-C₃ ;
k représente 3, 4, 5 ou 6 ;
m représente 0, 1, 2, 3, 4 ou 5 ;
n représente 0, 1 ou 2 ;
p représente 0 ou 1 ;
q représente 3, 4 ou 5 ;
étant entendu que X³ et X⁴ ne représentent pas simultanément un groupe alcoxy substitué ou non substitué.

3. 3-phénylisoxazoline-5-carboxamides et 3-phényl-isoxazoline-5-thioamides selon la revendication 1 ou 2, dans lesquels
R¹ et R² représentent chacun indépendamment l'un de l'autre un atome d'hydrogène, de fluor, de chlore, de brome, d'iode, un groupe cyano, ou un groupe alkyle en C₁-C₄ substitué dans chaque cas par m radicaux choisis dans le groupe constitué par fluoro, chloro, bromo, iodo et cyano ;
R³ représente un atome de fluor, de chlore ou un groupe cyano,
ou un groupe alkyle en C₁-C₆, cycloalkyle en C₃-C₆, alcényle en C₂-C₆ ou alcynyle en C₂-C₆, substitué dans chaque cas par m radicaux choisis dans le groupe constitué par fluoro et chloro,
ou un groupe alkyl(C₁-C₆)carbonyle substitué par m radicaux choisis dans le groupe constitué par fluoro et chloro ;
A représente une liaison ou une unité divalente choisie dans le groupe constitué par CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH(CH₂OCH₃), CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ et CH₂CH₂CONHCH₂ ;
R⁴ représente un atome d'hydrogène ou un groupe alkyle en C₁-C₈ ;
Y représente un atome d'oxygène ou de soufre ;
X représente un atome d'hydrogène, un groupe cyano, hydroxy, X¹,
ou
un groupe alkyle en C₁-C₁₂, cycloalkyle en C₃-C₈, alcényle en C₂-C₁₂ ou alcynyle en C₂-C₁₂, substitué dans chaque cas par m radicaux choisis dans le groupe constitué par fluoro, chloro, cyano, hydroxy, OR⁷, X¹, OX¹, NHX¹, S(O)ₙR⁵, CO₂R⁸, CONR⁶R⁸, CONR⁸SO₂R⁵ et POR⁹R⁹ ;
X¹ représente un cycle substitué par n radicaux choisis dans le groupe constitué par R⁶, R^{6a}, R⁸ et R⁹, choisi dans le groupe constitué par 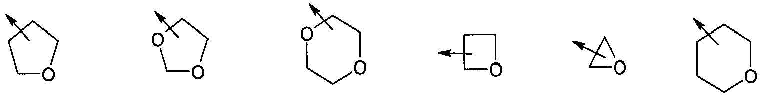 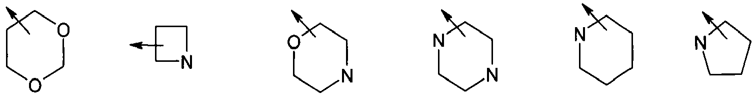 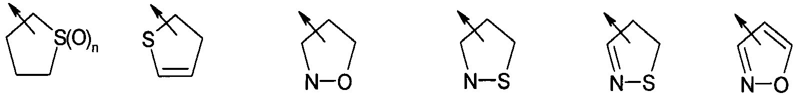 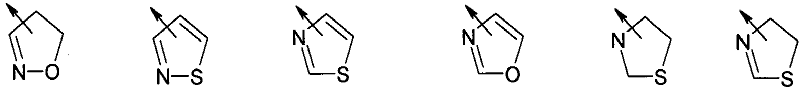 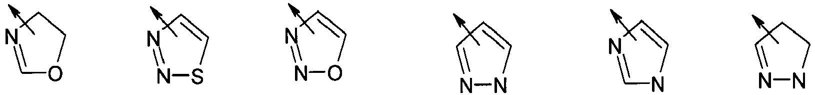 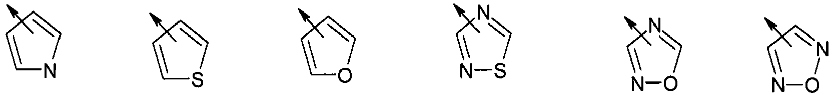 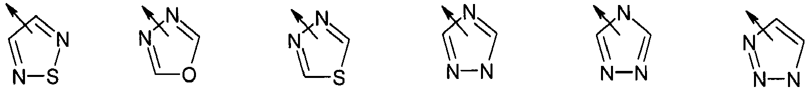 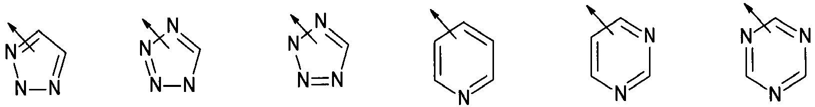 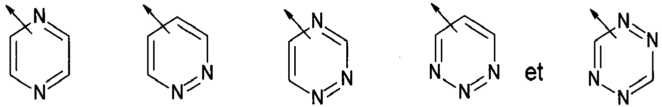 X², X⁴ et X⁶ représentent chacun indépendamment un atome d'hydrogène, de fluor ou de chlore,
ou un groupe alkyle en C₁-C₄ ou alcoxy en C₁-C₄, substitué dans chaque cas par m radicaux choisis dans le groupe constitué par fluoro, chloro, cyano et alcoxy en C₁-C₄ ;
X³ représente un atome de fluor, de chlore, de brome, un groupe cyano,
ou un groupe alkyle en C₁-C₆ substitué par m radicaux choisis dans le groupe constitué par fluoro et chloro, ou un groupe alcoxy en C₁-C₆ substitué par m radicaux choisis dans le groupe constitué par fluoro et chloro ;
X⁵ représente un atome d'hydrogène ou X³ ;
R⁵ représente le groupe méthyle ou éthyle ;
R⁶ représente un atome d'hydrogène ou R⁵ ;
R^{6a} représente un atome de fluor, de chlore, de brome, d'iode, un groupe cyano, hydroxy, S(O)ₙR⁵ ou un groupe alcoxy en C₁-C₆, alcényloxy en C₂-C₆ ou alcynyloxy en C₂-C₆, substitué dans chaque cas par m radicaux choisis dans le groupe constitué par fluoro, chloro, bromo, cyano et alcoxy en C₁-C₂ ;
R⁷ représente un atome d'hydrogène ou un groupe alkyle en C₁-C₆ substitué dans chaque cas par m radicaux choisis dans le groupe constitué par fluoro et chloro ;
R⁸ représente R⁷;
R⁹ représente un groupe alcoxy en C₁-C₃ ;
m représente 0, 1, 2 ou 3 ;
n représente 0, 1 ou 2 ;
étant entendu que X³ et X⁴ ne représentent pas simultanément un groupe alcoxy substitué ou non substitué.

4. Compositions herbicides, **caractérisées par** une teneur à activité herbicide en au moins un composé de formule (I) selon l'une quelconque des revendications 1 à 3.

5. Compositions herbicides selon la revendication 4, en mélange avec des adjuvants de formulation.

6. Compositions herbicides selon la revendication 4 ou 5, contenant au moins une autre substance à activité pesticide, choisie dans le groupe constitué par les insecticides, acaricides, herbicides, fongicides, phytoprotecteurs et régulateurs de croissance.

7. Compositions herbicides selon la revendication 6, contenant un phytoprotecteur.

8. Compositions herbicides selon la revendication 7, dans lesquelles le phytoprotecteur est choisi dans le groupe constitué par le méfenpyrdiéthyle, le cyprosulfamide, l'isoxadifène-éthyle, le cloquintocet-mexyle, le bénoxacor et le dichlormide.

9. Compositions herbicides selon l'une quelconque des revendications 6 à 8, contenant un autre herbicide.

10. Utilisation de 3-phénylisoxazoline-5-carboxamides et 3-phénylisoxazoline-5-thioamides de formule (Ia) 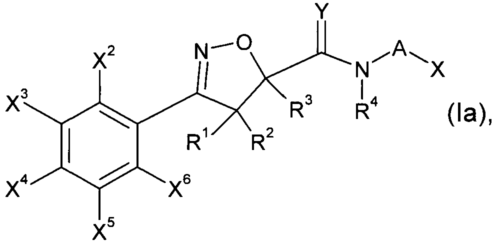 dans laquelle
R¹ et R² représentent chacun indépendamment l'un de l'autre un atome d'hydrogène, de fluor, de chlore, de brome, d'iode, un groupe cyano, ou un groupe alkyle en C₁-C₄ ou alcoxy en C₁-C₄, substitué dans chaque cas par m radicaux choisis dans le groupe constitué par fluoro, chloro, bromo, iodo et cyano,
ou
R¹ et R² forment ensemble avec l'atome de carbone, auquel ils sont liés, un cycle à trois, quatre ou cinq chaînons, saturé ou partiellement ou totalement insaturé, qui est constitué de q atomes de carbone et p atomes d'oxygène ;
R³ représente un atome de fluor, de chlore, un groupe cyano, alkyl(C₁-C₃)carbonyloxy ou S(O)ₙR⁵,
ou un groupe alkyle en C₁-C₆, cycloalkyle en C₃-C₆, alcényle en C₂-C₆ ou alcynyle en C₂-C₆, substitué dans chaque cas par m radicaux choisis dans le groupe constitué par fluoro, chloro, bromo, iodo, cyano, alcoxy en C₁-C₄ et hydroxy,
ou un groupe alkyl(C₁-C₆)carbonyle, alcényl(C₂-C₆)-carbonyle ou cycloalkyl(C₃-C₆)carbonyle, substitué dans chaque cas par m radicaux choisis dans le groupe constitué par fluoro, chloro, bromo, iodo, cyano et alcoxy en C₁-C₆ ;
R⁴ représente un atome d'hydrogène, un groupe cyano, hydroxy,
ou un groupe alkyle en C₁-C₈ ou cycloalkyle en C₃-C₈, substitué dans chaque cas par m radicaux choisis dans le groupe constitué par fluoro, chloro, bromo, iodo, cyano, hydroxy et alcoxy en C₁-C₆ ;
A représente une liaison ou une unité divalente choisie dans le groupe constitué par 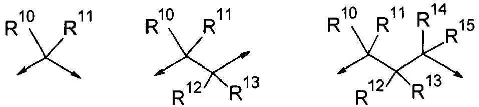 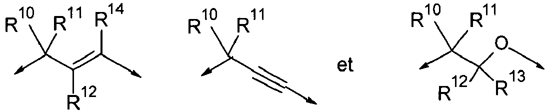 R¹⁰, R¹¹, R¹², R¹³, R¹⁴ et R¹⁵ représentent chacun indépendamment les uns des autres un atome d'hydrogène, de fluor, de chlore, de brome, d'iode, un groupe hydroxy, cyano, CO₂R⁸, CONR⁶R⁸, R⁵,
ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
Y représente un atome d'oxygène ou de soufre ;
X représente un atome d'hydrogène, un groupe cyano, hydroxy, X¹,
ou
un groupe alkyle en C₁-C₁₂, cycloalkyle en C₃-C₈, alcényle en C₂-C₁₂ ou alcynyle en C₂-C₁₂, substitué dans chaque cas par m radicaux choisis dans le groupe constitué par fluoro, chloro, bromo, iodo, cyano, hydroxy, OR⁷, X¹, OX¹, NHX¹, S(O)ₙR⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸ OCONR⁶R⁸, OCSNR⁶R⁸, POR⁹R⁹ et C(R⁶)=NOR⁸,
ou
X, A et R⁴ forment avec l'atome d'azote, auquel ils sont liés, un cycle à cinq, six ou sept chaînons, saturé ou partiellement ou totalement insaturé, qui en plus de cet atome d'azote contient, en tant qu'atomes formant le cycle, k atomes de carbone, n atomes d'oxygène, p atomes de soufre et p éléments choisis dans le groupe constitué par NR⁷ et NCOR⁷, un atome de carbone portant p groupes oxo ;
X¹ représente un cycle à cinq ou six chaînons, saturé, partiellement insaturé, totalement insaturé ou aromatique, qui est constitué de r atomes de carbone, s atomes d'azote, n atomes de soufre et n atomes d'oxygène, et qui est substitué par n radicaux choisis dans le groupe constitué par R⁶, R^{6a}, R⁸ et R⁹,
ou un groupe phényle substitué par n radicaux choisis dans le groupe constitué par R⁶, R⁸ et R⁹ ;
X², X⁴ et X⁶ représentent chacun indépendamment un atome d'hydrogène, de fluor, de chlore, de brome, d'iode, un groupe cyano, nitro,
ou un groupe alkyle en C₁-C₄, cycloalkyle en C₃-C₅, alcényle en C₂-C₄, alcynyle en C₂-C₄, alcoxy en C₁-C₄, alcényloxy en C₂-C₄, alcynyloxy en C₂-C₄ ou alkyl(C₁-C₄)-carbonyle, substitué dans chaque cas par m radicaux choisis dans le groupe constitué par fluoro, chloro, bromo, iodo, cyano et alcoxy en C₁-C₄ ;
X³ représente un atome d'hydrogène, de fluor, de chlore, de brome, d'iode, un groupe hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
X⁵ représente un atome d'hydrogène ou X³ ;
R⁵ représente un groupe alkyle en C₁-C₆ ou cycloalkyle en C₃-C₆, substitué dans chaque cas par m radicaux choisis dans le groupe constitué par fluoro, chloro, bromo, iodo, cyano et hydroxy ;
R⁶ représente un atome d'hydrogène ou R⁵ ;
R^{6a} représente un atome de fluor, de chlore, de brome, d'iode, un groupe cyano, hydroxy, S(O)ₙR⁵ ou un groupe alcoxy en C₁-C₆, alcényloxy en C₂-C₆ ou alcynyloxy en C₂-C₆, substitué dans chaque cas par m radicaux choisis dans le groupe constitué par fluoro, chloro, bromo, cyano et alcoxy en C₁-C₂ ;
R⁷ représente un atome d'hydrogène ou un groupe alkyle en C₁-C₆, cycloalkyle en C₃-C₆, alcényle en C₂-C₄ ou alcynyle en C₂-C₄, substitué dans chaque cas par m radicaux choisis dans le groupe constitué par fluoro, chloro, bromo, cyano et alcoxy en C₁-C₂ ;
R⁸ représente R⁷ ;
R⁹ représente un groupe alkyle en C₁-C₃ ou alcoxy en C₁-C₃ ;
k représente 3, 4, 5 ou 6 ;
m représente 0, 1, 2, 3, 4 ou 5 ;
n représente 0, 1 ou 2 ;
p représente 0 ou 1 ;
q représente 3, 4 ou 5 ;
r représente 1, 2, 3, 4 ou 5 ;
s représente 0, 1, 2, 3 ou 4 ;
pour la lutte contre des plantes indésirables.

11. Utilisation de 3-phénylisoxazoline-5-carboxamides et 3-phénylisoxazoline-5-thioamides de formule (Ia) 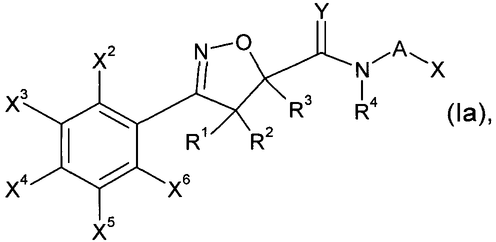 dans laquelle
R¹ et R² représentent chacun indépendamment l'un de l'autre un atome d'hydrogène, de fluor, de chlore, de brome, d'iode, un groupe cyano, ou un groupe alkyle en C₁-C₄ ou alcoxy en C₁-C₄, substitué dans chaque cas par m radicaux choisis dans le groupe constitué par fluoro, chloro, bromo, iodo et cyano,
ou
R¹ et R² forment ensemble avec l'atome de carbone, auquel ils sont liés, un cycle à trois, quatre ou cinq chaînons, saturé ou partiellement ou totalement insaturé, qui est constitué de q atomes de carbone et p atomes d'oxygène ;
R³ représente un atome de fluor, de chlore, un groupe cyano, alkyl(C₁-C₃)carbonyloxy ou S(O)ₙR⁵,
ou un groupe alkyle en C₁-C₆, cycloalkyle en C₃-C₆, alcényle en C₂-C₆ ou alcynyle en C₂-C₆, substitué dans chaque cas par m radicaux choisis dans le groupe constitué par fluoro, chloro, bromo, iodo, cyano, alcoxy en C₁-C₄ et hydroxy,
ou un groupe alkyl(C₁-C₆)carbonyle, alcényl(C₂-C₆)-carbonyle ou cycloalkyl(C₃-C₆)carbonyle, substitué dans chaque cas par m radicaux choisis dans le groupe constitué par fluoro, chloro, bromo, iodo, cyano et alcoxy en C₁-C₆ ;
R⁴ représente un atome d'hydrogène ou un groupe cyano, hydroxy,
ou un groupe alkyle en C₁-C₈ ou cycloalkyle en C₃-C₈, substitué dans chaque cas par m radicaux choisis dans le groupe constitué par fluoro, chloro, bromo, iodo, cyano, hydroxy et alcoxy en C₁-C₆ ;
A représente une liaison ou une unité divalente choisie dans le groupe constitué par 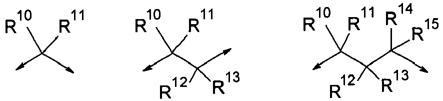 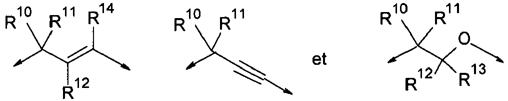 R¹⁰, R¹¹, R¹², R¹³, R¹⁴ et R¹⁵ représentent chacun indépendamment les uns des autres un atome d'hydrogène, de fluor, de chlore, de brome, d'iode, un groupe hydroxy, cyano, CO₂R⁸, CONR⁶R⁸, R⁵, ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
Y représente un atome d'oxygène ou de soufre ;
X représente un atome d'hydrogène, un groupe cyano, hydroxy, X¹,
ou
un groupe alkyle en C₁-C₁₂, cycloalkyle en C₃-C₈, alcényle en C₂-C₁₂ ou alcynyle en C₂-C₁₂, substitué dans chaque cas par m radicaux choisis dans le groupe constitué par fluoro, chloro, bromo, iodo, cyano, hydroxy, X¹, OX¹, NHX¹, S(O)ₙR⁵, SO₂NR⁶R⁷, SO₂NR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ et C(R⁶)=NOR⁸,
ou
X, A et R⁴ forment avec l'atome d'azote, auquel ils sont liés, un cycle à cinq, six ou sept chaînons, saturé ou partiellement ou totalement insaturé, qui en plus de cet atome d'azote contient, en tant qu'atomes formant le cycle, k atomes de carbone, n atomes d'oxygène, p atomes de soufre et p éléments choisis dans le groupe constitué par NR⁷ et NCOR⁷, un atome de carbone portant p groupes oxo ;
X¹ représente un cycle substitué par n radicaux choisis dans le groupe constitué par R⁶, R^{6a}, R⁸ et R⁹, choisi dans le groupe constitué par 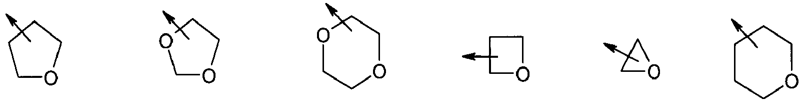 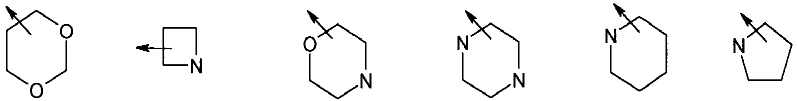 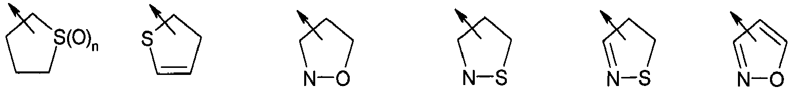 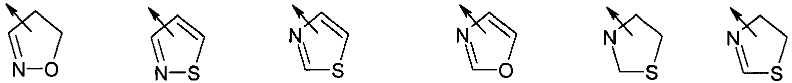 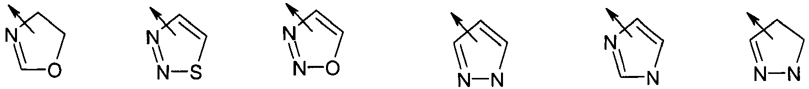 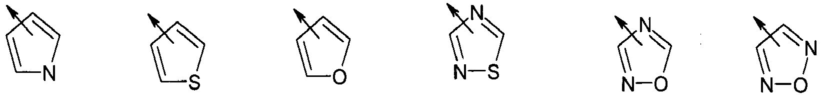 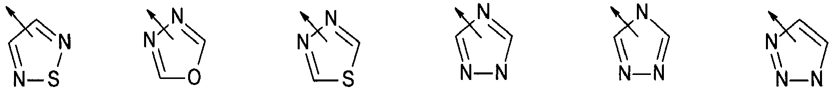 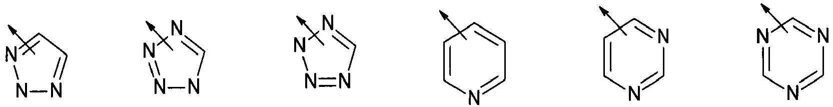 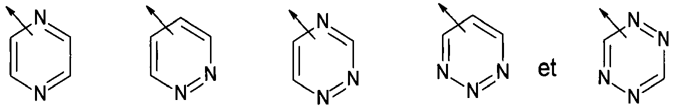 ou un groupe phényle substitué par n radicaux choisis dans le groupe constitué par R⁶, R⁸ et R⁹ ;
X², X⁴ et X⁶ représentent chacun indépendamment un atome d'hydrogène, de fluor, de chlore, de brome, d'iode, un groupe cyano, nitro,
ou un groupe alkyle en C₁-C₄, cycloalkyle en C₃-C₅, alcényle en C₂-C₄, alcynyle en C₂-C₄, alcoxy en C₁-C₄, alcényloxy en C₂-C₄, alcynyloxy en C₂-C₄ ou alkyl(C₁-C₄)-carbonyle, substitué dans chaque cas par m radicaux choisis dans le groupe constitué par fluoro, chloro, bromo, iodo, cyano et alcoxy en C₁-C₄ ;
X³ représente un atome d'hydrogène, de fluor, de chlore, de brome, d'iode, un groupe hydroxy, cyano, nitro, SF₅, CONR⁸SO₂R⁵, CONR⁶R⁸, COR⁶, CO₂R⁸, CONR⁶R⁸,
C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou un groupe alkyle en C₁-C₆, cycloalkyle en C₃-C₅, alcényle en C₂-C₆, alcynyle en C₂-C₆, substitué dans chaque cas par m radicaux choisis dans le groupe constitué par fluoro, chloro, bromo, iodo, hydroxy et cyano,
ou un groupe alcoxy en C₁-C₆, cycloalcoxy en C₃-C₆, alcényloxy en C₂-C₆ ou alcynyloxy en C₂-C₆, substitué dans chaque cas par m radicaux choisis dans le groupe constitué par fluoro, chloro, bromo, iodo, cyano et alcoxy en C₁-C₂ ;
X⁵ représente un atome d'hydrogène ou X³ ;
R⁵ représente un groupe alkyle en C₁-C₆ ou cycloalkyle en C₃-C₆, substitué dans chaque cas par m radicaux choisis dans le groupe constitué par fluoro, chloro, bromo, iodo, cyano et hydroxy ;
R⁶ représente un atome d'hydrogène ou R⁵ ;
R^{6a} représente un atome de fluor, de chlore, de brome, d'iode, un groupe cyano, hydroxy, S(O)ₙR⁵ ou un groupe alcoxy en C₁-C₆, alcényloxy en C₂-C₆ ou alcynyloxy en C₂-C₆, substitué dans chaque cas par m radicaux choisis dans le groupe constitué par fluoro, chloro, bromo, cyano et alcoxy en C₁-C₂ ;
R⁷ représente un atome d'hydrogène ou un groupe alkyle en C₁-C₆, cycloalkyle en C₃-C₆, alcényle en C₂-C₄ ou alcynyle en C₂-C₄, substitué dans chaque cas par m radicaux choisis dans le groupe constitué par fluoro, chloro, bromo, cyano et alcoxy en C₁-C₂ ;
R⁸ représente R⁷ ;
R⁹ représente un groupe alkyle en C₁-C₃ ou alcoxy en C₁-C₃ ;
k représente 3, 4, 5 ou 6 ;
m représente 0, 1, 2, 3, 4 ou 5 ;
n représente 0, 1 ou 2 ;
p représente 0 ou 1 ;
q représente 3, 4 ou 5 ;
pour la lutte contre des plantes indésirables.

12. Utilisation de 3-phénylisoxazoline-5-carboxamides et 3-phénylisoxazoline-5-thioamides selon la revendication 10 ou 11, dans lesquels R¹ et R² représentent chacun indépendamment l'un de l'autre un atome d'hydrogène, de fluor, de chlore, de brome, d'iode, un groupe cyano, ou un groupe alkyle en C₁-C₄ substitué dans chaque cas par m radicaux choisis dans le groupe constitué par fluoro, chloro, bromo, iodo et cyano ;
R³ représente un atome de fluor, de chlore ou un groupe cyano,
ou un groupe alkyle en C₁-C₆, cycloalkyle en C₃-C₆, alcényle en C₂-C₆ ou alcynyle en C₂-C₆, substitué dans chaque cas par m radicaux choisis dans le groupe constitué par fluoro et chloro,
ou un groupe alkyl(C₁-C₆)carbonyle substitué par m radicaux choisis dans le groupe constitué par fluoro et chloro ;
R⁴ représente un atome d'hydrogène ou un groupe alkyle en C₁-C₈ ;
A représente une liaison ou une unité divalente choisie dans le groupe constitué par CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH(CH₂OCH₃), CH (CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ et CH₂CH₂CONHCH₂ ;
Y représente un atome d'oxygène ou de soufre ;
X représente un atome d'hydrogène, un groupe cyano, hydroxy, X¹,
ou
un groupe alkyle en C₁-C₁₂, cycloalkyle en C₃-C₈, alcényle en C₂-C₁₂ ou alcynyle en C₂-C₁₂, substitué dans chaque cas par m radicaux choisis dans le groupe constitué par fluoro, chloro, cyano, hydroxy, X¹, OX¹, NHX¹, S(O)ₙR⁵, CO₂R⁸, CONR⁶R⁸, CONR⁸SO₂R⁵ et POR⁹R⁹ ;
X¹ représente un cycle substitué par n radicaux choisis dans le groupe constitué par R⁶, R^{6a}, R⁸ et R⁹, choisi dans le groupe constitué par 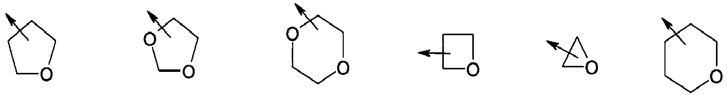 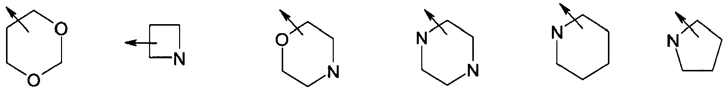 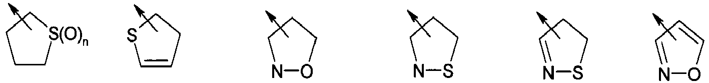 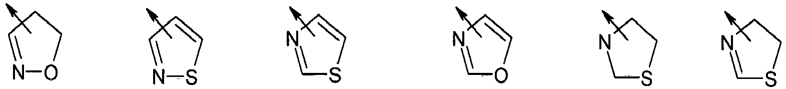 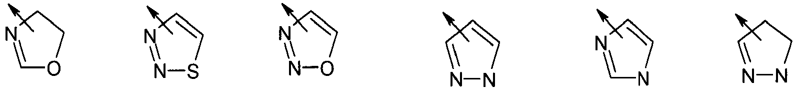 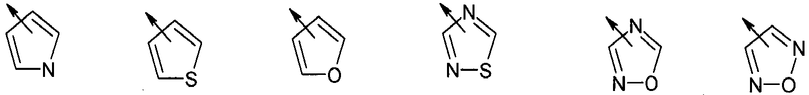 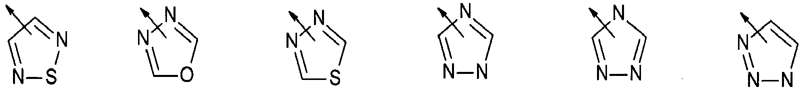 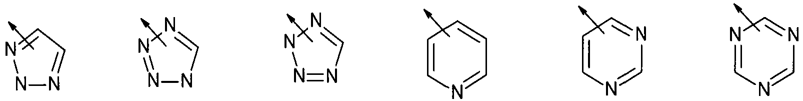 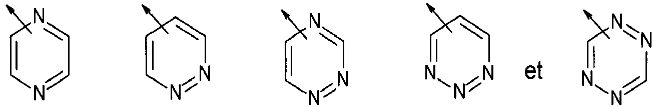 ou un groupe phényle substitué par n radicaux choisis dans le groupe constitué par R⁶, R⁸ et R⁹ ;
X², X⁴ et X⁶ représentent chacun indépendamment un atome d'hydrogène, de fluor ou de chlore,
ou un groupe alkyle en C₁-C₄ ou alcoxy en C₁-C₄, substitué dans chaque cas par m radicaux choisis dans le groupe constitué par fluoro, chloro, cyano et alcoxy en C₁-C₄ ;
X³ représente un atome d'hydrogène, de fluor, de chlore, de brome, un groupe cyano,
ou un groupe alkyle en C₁-C₆ substitué par m radicaux choisis dans le groupe constitué par fluoro et chloro, ou un groupe alcoxy en C₁-C₆ substitué par m radicaux choisis dans le groupe constitué par fluoro et chloro ;
X⁵ représente un atome d'hydrogène ou X³ ;
X⁵ représente un atome d'hydrogène ou X³ ;
R⁵ représente le groupe méthyle ou éthyle ;
R⁶ représente un atome d'hydrogène ou R⁵ ;
R^{6a} représente un atome de fluor, de chlore, de brome, d'iode, un groupe cyano, hydroxy, S(O)ₙR⁵ ou un groupe alcoxy en C₁-C₆, alcényloxy en C₂-C₆ ou alcynyloxy en C₂-C₆, substitué dans chaque cas par m radicaux choisis dans le groupe constitué par fluoro, chloro, bromo, cyano et alcoxy en C₁-C₂ ;
R⁷ représente un atome d'hydrogène ou un groupe alkyle en C₁-C₆ substitué dans chaque cas par m radicaux choisis dans le groupe constitué par fluoro et chloro ;
R⁸ représente R⁷ ;
R⁹ représente un groupe alcoxy en C₁-C₃ ;
m représente 0, 1, 2 ou 3 ;
n représente 0, 1 ou 2.

13. Procédé pour la lutte contre des plantes indésirables, **caractérisé en ce qu'**on applique sur les plantes ou sur le lieu de la croissance de plantes indésirables une quantité efficace d'au moins un composé de formule (I) selon l'une quelconque des revendication 1 à 3 ou d'au moins un composé de formule (Ia) selon l'une quelconque des revendications 10 à 12 ou d'une composition herbicide selon l'une quelconque des revendications 4 à 9.

14. Utilisation de composés de formule (I) selon l'une quelconque des revendications 1 à 3 ou de compositions herbicides selon l'une quelconque des revendications 4 à 9, pour la lutte contre des plantes indésirables.

15. Utilisation selon l'une quelconque des revendications 10 à 12 ou 14, **caractérisée en ce qu'**on utilise les composés de formule (I) pour la lutte contre des plantes indésirables dans des cultures de plantes utiles.

16. Utilisation selon la revendication 15, **caractérisée en ce que** les plantes utiles sont des plantes utiles transgéniques.
